# EUROPEAN PATENT SPECIFICATION

(11) **EP 3 468 996 B1**
(45) Date of publication and mention of the grant of the patent: **04.05.2022**
(21) Application number: 17731394.7
(22) Date of filing: 07.06.2017
(51) Int. Cl.: C07K 16/28, A61K 47/68, A61P 35/00, A61P 43/00

(54) **ANTI-EGFR ANTIBODY DRUG CONJUGATES**
ANTI-EGFR-ANTIKÖRPER-ARZNEIMITTELKONJUGATE
CONJUGUÉ ANTICORPS-MÉDICAMENT ANTI-EGFR

(30) Priority: 08.06.2016 US 201662347416 P
(43) Date of publication of application: 17.04.2019
(60) Divisional application: 22165662.2
(73) Proprietor: AbbVie Inc., North Chicago, IL 60064 (US)
(72) Inventor: BOGHAERT, Erwin, R., Pleasant Prairie, WI 53158 (US); SOUERS, Andrew, J., Libertyville, IL 60048 (US); PHILLIPS, Andrew, C., Libertyville, IL 60048 (US); JUDD, Andrew, S., Grayslakc, IL 60030 (US); BRUNCKO, Milan, Green Oaks, IL 60048 (US)
(74) Representative: Schlich, George
(86) International application number: PCT/US2017/036368
(87) International publication number: WO 2017/214282

(56) References cited:
- WO-A1-2013/055897
- WO-A1-2015/143382
- WO-A1-2016/094505
- WO-A1-2016/094509
- WO-A1-2016/094517
- HEIDI L. PEREZ ET AL: "Antibody-drug conjugates: current status and future directions", DRUG DISCOVERY TODAY, vol. 19, no. 7, 1 July 2014 (2014-07-01), pages 869-881, XP55184320, ISSN: 1359-6446, DOI: 10.1016/j.drudis.2013.11.004

## Description

### RELATED APPLICATIONS

This application claims priority to U.S. Provisional Application No. 62/347,416, filed on June 8, 2016.

### SEQUENCE LISTING

The instant application contains a Sequence Listing which has been submitted electronically in ASCII format. Said ASCII copy, created on June 2, 2017, is named 117813-13402_SL.txt and is 139,240 bytes in size.

### BACKGROUND OF THE INVENTION

The human epidermal growth factor receptor (also known as HER-1 or Erb-B1, and referred to herein as "EGFR") is a 170 kDa transmembrane receptor encoded by the c-erbB protooncogene, and exhibits intrinsic tyrosine kinase activity (Modjtahedi et al., Br. J. Cancer 73:228-235 (1996); Herbst and Shin, Cancer 94:1593-1611 (2002)). SwissProt database entry P00533 provides the sequence of human EGFR. EGFR regulates numerous cellular processes via tyrosine-kinase mediated signal transduction pathways, including, but not limited to, activation of signal transduction pathways that control cell proliferation, differentiation, cell survival, apoptosis, angiogenesis, mitogenesis, and metastasis (Atalay et al., Ann. Oncology 14:1346-1363 (2003); Tsao and Herbst, Signal 4:4-9 (2003); Herbst and Shin, Cancer 94:1593-1611 (2002); Modjtahedi et al., Br. J. Cancer 73:228-235 (1996)).

Known ligands of EGFR include EGF, TGFA/TGF-alpha, amphiregulin, epigen/EPGN, BTC/betacellulin, epiregulin/EREG and HBEGF/heparin-binding EGF. Ligand binding by EGFR triggers receptor homo- and/or heterodimerization and autophosphorylation of key cytoplasmic residues. The phosphorylated EGFR recruits adapter proteins like GRB2 which in turn activate complex downstream signaling cascades, including at least the following major downstream signaling cascades: the RAS-RAF-MEK-ERK, PI3 kinase-AKT, PLCgamma-PKC, and STATs modules. This autophosphorylation also elicits downstream activation and signaling by several other proteins that associate with the phosphorylated tyrosines through their own phosphotyrosine-binding SH2 domains. These downstream signaling proteins initiate several signal transduction cascades, principally the MAPK, Akt and JNK pathways, leading to cell proliferation. Ligand binding by EGFR may also activate the NF-kappa-B signaling cascade. Ligand binding also directly phosphorylates other proteins like RGS16, activating its GTPase activity and potentially coupling the EGF receptor signaling to G protein-coupled receptor signaling. Ligand binding also phosphorylates MUC1 and increases its interaction with SRC and CTNNB1/beta-catenin.

Overexpression of EGFR has been reported in numerous human malignant conditions, including cancers of the bladder, brain, head and neck, pancreas, lung, breast, ovary, colon, prostate, and kidney. (Atalay et al., Ann. Oncology 14:1346-1363 (2003); Herbst and Shin, Cancer 94:1593-1611 (2002); and Modjtahedi et al., Br. J. Cancer 73:228-235 (1996)). In many of these conditions, the overexpression of EGFR correlates or is associated with poor prognosis of the patients. (Herbst and Shin, Cancer 94:1593-1611 (2002); and Modjtahedi et al., Br. J. Cancer 73:228-235 (1996)). EGFR is also expressed in the cells of normal tissues, particularly the epithelial tissues of the skin, liver, and gastrointestinal tract, although at generally lower levels than in malignant cells (Herbst and Shin, Cancer 94:1593-1611 (2002)).

A significant proportion of tumors containing amplifications of the EGFR gene also co-express a truncated version of the receptor (Wikstrand et al. (1998) J. Neurovirol. 4, 148-158) known as de2-7 EGFR, ΔEGFR, EGFRvIII, or Δ2-7 (terms used interchangeably herein) (Olapade-Olaopa et al. (2000) Br. J. Cancer. 82, 186-94). The rearrangement seen in the de2-7 EGFR results in an in-frame mature mRNA lacking 801 nucleotides spanning exons 2-7 (Wong et al. (1992) Proc. Natl. Acad. Sci. U.S.A. 89, 2965-9; Yamazaki et al. (1990) Jpn. J. Cancer Res. 81, 773-9; Yamazaki et al. (1988) Mol. Cell. Biol. 8, 1816-20; and Sugawa et al. (1990) Proc. Natl. Acad. Sci. U.S.A. 87, 8602-6). The corresponding EGFR protein has a 267 amino acid deletion comprising residues 6-273 of the extracellular domain and a novel glycine residue at the fusion junction (Sugawa et al., 1990). This deletion, together with the insertion of a glycine residue, produces a unique junctional peptide at the deletion interface (Sugawa et al., 1990).

EGFRvIII has been reported in a number of tumor types including glioma, breast, lung, ovarian and prostate (Wikstrand et al. (1997) Cancer Res. 57, 4130-40; Olapade-Olaopa et al. (2000) Br. J. Cancer. 82, 186-94; Wikstrand, et al. (1995) Cancer Res. 55, 3140-8; Garcia de Palazzo et al. (1993) Cancer Res. 53, 3217-20). While this truncated receptor does not bind ligand, it possesses low constitutive activity and imparts a significant growth advantage to glioma cells grown as tumor xenografts in nude mice (Nishikawa et al. (1994) Proc. Natl. Acad. Sci. U.S.A. 91, 7727-31) and is able to transform NIH3T3 cells (Batra et al. (1995) Cell Growth Differ. 6, 1251-9) and MCF-7 cells. The cellular mechanisms utilized by the de2-7 EGFR in glioma cells are not fully defined but are reported to include a decrease in apoptosis (Nagane et al. (1996) Cancer Res. 56, 5079-86) and a small enhancement of proliferation (Nagane et al., 1996). As expression of this truncated receptor is restricted to tumor cells it represents a highly specific target for antibody therapy.

Antibody drug conjugates (ADC) represent a new class of therapeutics comprising an antibody conjugated to a cytotoxic drug via a chemical linker. The therapeutic concept of ADCs is to combine binding capabilities of an antibody with a drug, where the antibody is used to deliver the drug to a tumor cell by means of binding to a target surface antigen. Given the role of EGFR in cancer, there remains a need in the art for anti-EGFR ADCs that can be used for treatment of cancer.

ADCs are described in WO 2016/094505, WO 2016/094509 and WO 2016/094517, available under EPC Art. 54(3) EPC.

### SUMMARY OF THE CLAIMED INVENTION

It has been discovered that small molecule inhibitors of Bcl-xL are efficacious when administered in the form of antibody drug conjugates (ADCs) that bind to antigens expressed on the surface of cells, *e.g.* cells that express EGFR, where inhibition of Bcl-xL and consequent induction of apoptosis would be beneficial. This discovery provides the ability to target Bcl-xL inhibitory therapies to specific cells and/or tissues that express EGFR, such that the Bcl-xL inhibitor is delivered internally to a transformed cancer cell expressing EGFR. One advantage of the invention is the potential for lowering serum levels necessary to achieve desired therapeutic benefit and/or avoiding and/or ameliorating potential side effects associated with systemic administration of the small molecule Bcl-xL inhibitors *per se.*

ADCs may increase the therapeutic efficacy of antibodies in treating disease, *e.g.*, cancer, due to the ability of the ADC to selectively deliver one or more drug moiety(s) to target tissues, such as a tumor-associated antigen, *e.g.*, EGFR expressing tumors. Thus, in certain embodiments, the invention provides the specific anti-EGFR ADCs noted immediately below for therapeutic use, *e.g.,* treatment of cancer. Any references to methods of treatment in the subsequent paragraphs of this description are to be interpreted as references to the compounds, pharmaceutical compositions and medicaments of the present invention for use in a method for treatment of the human (or animal) body by therapy (or for diagnosis).

The disclosure as claimed hence provides an anti-human Epidermal Growth Factor Receptor (hEGFR) antibody drug conjugate comprising the following structure: 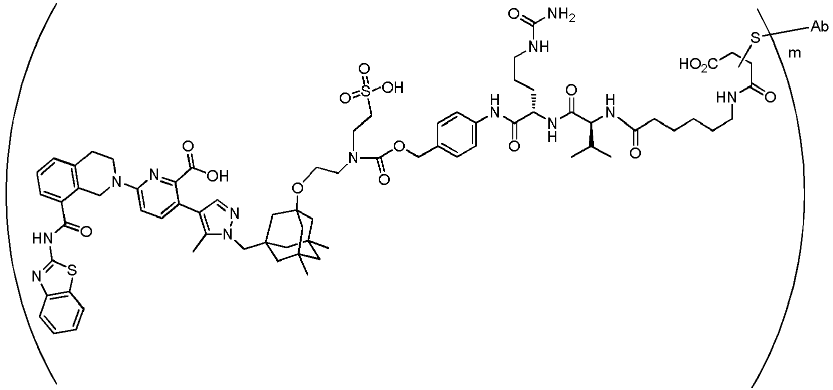 wherein Ab is an IgG1 anti-hEGFR antibody comprising
a heavy chain variable region comprising a heavy chain CDR3 domain comprising the amino acid sequence set forth in SEQ ID NO: 12, a heavy chain CDR2 domain comprising the amino acid sequence set forth in SEQ ID NO: 11, and a heavy chain CDR1 domain comprising the amino acid sequence set forth in SEQ ID NO: 10; and
a light chain variable region comprising a light chain CDR3 domain comprising the amino acid sequence set forth in SEQ ID NO: 8, a light chain CDR2 domain comprising the amino acid sequence set forth in SEQ ID NO: 7, and a light chain CDR1 domain comprising the amino acid sequence set forth in SEQ ID NO: 6; and wherein m is 2.

The anti-hEGFR antibody may comprise a heavy chain variable region comprising the amino acid sequence set forth in SEQ ID NO: 9 and a light chain variable region comprising the amino acid sequence set forth in SEQ ID NO: 5.

The anti-hEGFR antibody may comprises a heavy chain comprising the amino acid sequence set forth in SEQ ID NO: 15, and a light chain comprising the amino acid sequence set forth in SEQ ID NO: 13.

### SUMMARY AND DETAILS OF THE DISCLOSURE

The invention as claimed is discussed and illustrated with reference to the disclosure below, in which references to aspects and embodiments are references to aspects of the disclosure.

In one aspect, the disclosure features an anti-human Epidermal Growth Factor Receptor (hEGFR) antibody drug conjugate (ADC) comprising an anti-hEGFR antibody, *i.e.,* an antibody that specifically binds to human EGFR, linked to one or more Bcl-xL inhibitor(s).

In another aspect, the disclosure features an anti-human Epidermal Growth Factor Receptor (hEGFR) antibody drug conjugate (ADC) comprising a drug linked to an anti-human Epidermal Growth Factor (hEGFR) antibody by way of a linker, wherein the drug is a Bcl-xL inhibitor according to structural formula (IIa), (IIb), (IIc), or (IId): 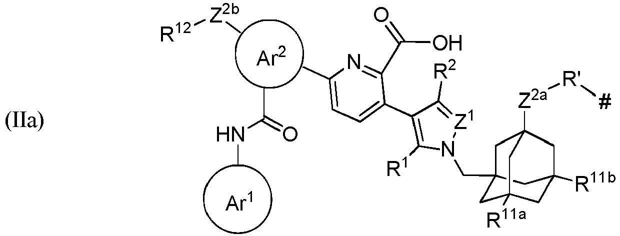 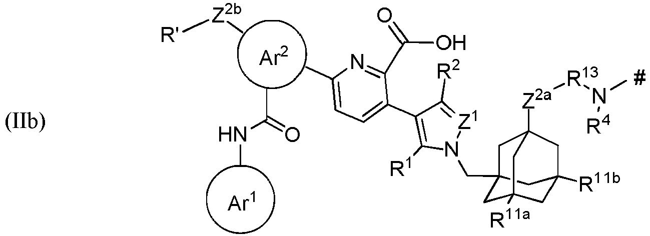 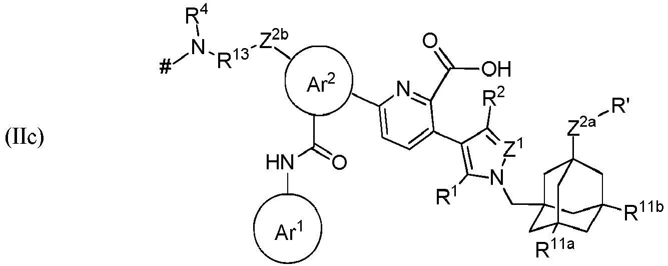 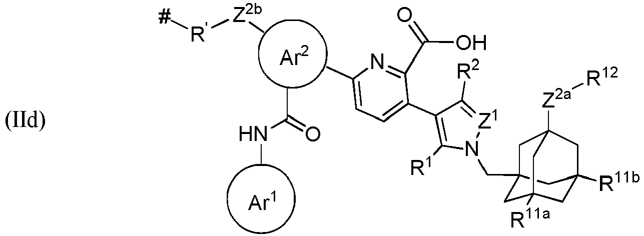 wherein:
Ar¹ is selected from 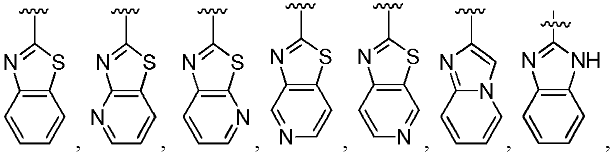 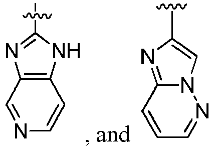 and is optionally substituted with one or more substituents independently selected from halo, hydroxy, nitro, lower alkyl, lower heteroalkyl, C₁₋₄alkoxy, amino, cyano and halomethyl;
Ar² is selected from 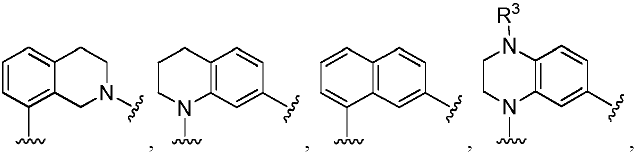 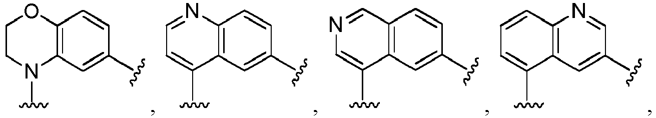 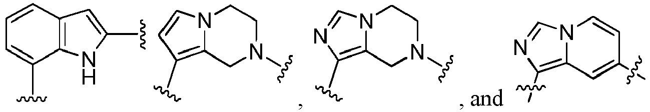 or an N-oxide thereof, and is optionally substituted with one or more substituents independently selected from halo, hydroxy, nitro, lower alkyl, lower heteroalkyl, C₁₋₄alkoxy, amino, cyano and halomethyl, wherein the R¹²-Z^{2b}-, R'-Z^{2b}-, #-N(R⁴)-R¹³-Z^{2b}-, or #-R'-Z^{2b}- substituents are attached to Ar² at any Ar² atom capable of being substituted;
Z¹ is selected from N, CH, C-halo, C-CH₃ and C-CN;
Z^{2a} and Z^{2b} are each , independently from one another, selected from a bond, NR⁶, CR^{6a}R^{6b}, O, S, S(O), S(O)₂, -NR⁶C(O)-,-NR^{6a}C(O)NR^{6b}-, and -NR⁶C(O)O-;
R' is 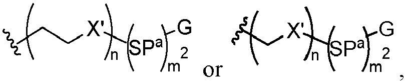 wherein #, where attached to R', is attached
to R' at any R' atom capable of being substituted;
X' is selected at each occurrence from -N(R¹⁰)-, -N(R¹⁰)C(O)-, -N(R¹⁰)S(O)₂-, -S(O)₂N(R¹⁰)-, and -O-;
n is selected from 0-3;
R¹⁰ is independently selected at each occurrence from hydrogen, lower alkyl, heterocycle, aminoalkyl, G-alkyl, and -(CH₂)₂-O-(CH₂)₂-O-(CH₂)₂-NH₂;
G at each occurrence is independently selected from a polyol, a polyethylene glycol with between 4 and 30 repeating units, a salt and a moiety that is charged at physiological pH;
SP^{a} is independently selected at each occurrence from oxygen, -S(O)₂N(H)-, -N(H)S(O)₂-, -N(H)C(O)-, -C(O)N(H) -, -N(H)- , arylene, heterocyclene, and optionally substituted methylene; wherein methylene is optionally substituted with one or more of -NH(CH₂)₂G, NH₂, C₁₋₈alkyl, and carbonyl;
m² is selected from 0-12;
R¹ is selected from hydrogen, methyl, halo, halomethyl, ethyl, and cyano;
R² is selected from hydrogen, methyl, halo, halomethyl and cyano;
R³ is selected from hydrogen, methyl, ethyl, halomethyl and haloethyl;
R⁴ is selected from hydrogen, lower alkyl and lower heteroalkyl or is taken together with an atom of R¹³ to form a cycloalkyl or heterocyclyl ring having between 3 and 7 ring atoms;
R⁶, R^{6a} and R^{6b} are each, independent from one another, selected from hydrogen, optionally substituted lower alkyl, optionally substituted lower heteroalkyl, optionally substituted cycloalkyl and optionally substituted heterocyclyl, or are taken together with an atom from R⁴ and an atom from R¹³ to form a cycloalkyl or heterocyclyl ring having between 3 and 7 ring atoms;
R^{11a} and R^{11b} are each, independently of one another, selected from hydrogen, halo, methyl, ethyl, halomethyl, hydroxyl, methoxy, CN, and SCH₃;
R¹² is optionally R' or is selected from hydrogen, halo, cyano, optionally substituted alkyl, optionally substituted heteroalkyl, optionally substituted heterocyclyl, and optionally substituted cycloalkyl;
R¹³ is selected from optionally substituted C₁₋₈ alkylene, optionally substituted heteroalkylene, optionally substituted heterocyclene, and optionally substituted cycloalkylene; and
# represents the point of attachment to a linker L;
wherein the hEGFR antibody has the following characteristics:
   binds to an epitope within the amino acid sequence CGADSYEMEEDGVRKC (SEQ ID NO: 45) or competes with a second anti-hEGFR antibody for binding to epidermal growth factor receptor variant III (EGFRvIII) (SEQ ID NO: 33) in a competitive binding assay, wherein the second anti-EGFR antibody comprises a heavy chain variable domain comprising the amino acid sequence set forth in SEQ ID NO: 1 and a light chain variable domain comprising the amino acid sequence set forth in SEQ ID NO: 5; and
   binds to EGFR(1-525) (SEQ ID NO: 47) with a dissociation constant (K_{d}) of about 1 x 10⁻⁶ M or less, as determined by surface plasmon resonance.

In one embodiment, the ADC is a compound according to structural formula (I): 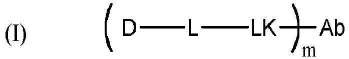 wherein:
D is the Bcl-xL inhibitor drug of formula (IIa), (IIb), (IIc) or (IId);
L is the linker;
Ab is the anti-hEGFR antibody;
LK represents a covalent linkage linking the linker (L) to the anti-hEGFR antibody (Ab); and
m is an integer ranging from 1 to 20.

In one embodiment, G at each occurrence is a salt or a moiety that is charged at physiological pH. In another embodiment, G at each occurrence is a salt of a carboxylate, a sulfonate, a phosphonate, or ammonium. In another embodiment, G at each occurrence is a moiety that is charged at physiological pH selected from the group consisting of carboxylate, a sulfonate, a phosphonate, and an amine. In another embodiment, G at each occurrence is a moiety containing a polyethylene glycol with between 4 and 30 repeating units, or a polyol. In a further embodiment, the polyol is a sugar.

In one embodiment of any one of the aspects and embodiments herein, the disclosure features the ADC of formula (IIa) or formula (IId), in which R' includes at least one substitutable nitrogen suitable for attachment to a linker.

In another embodiment, G is selected at each occurrence from: 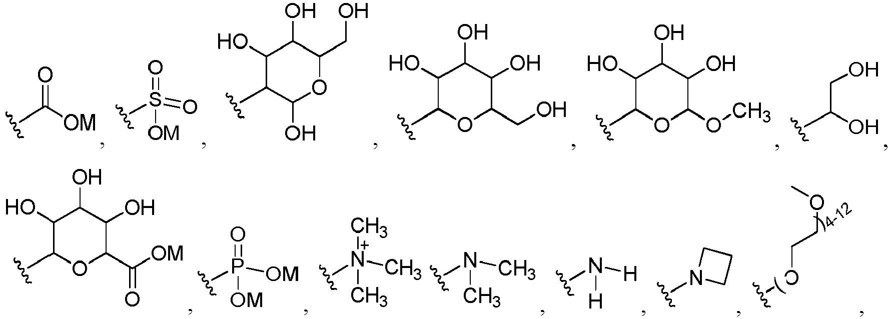 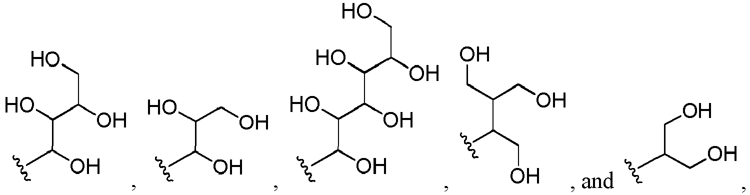 wherein M is hydrogen or a positively charged counterion.

In one embodiment of any one of the aspects and embodiments herein, R' is selected from 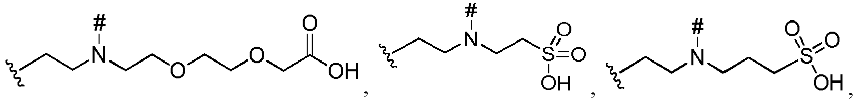 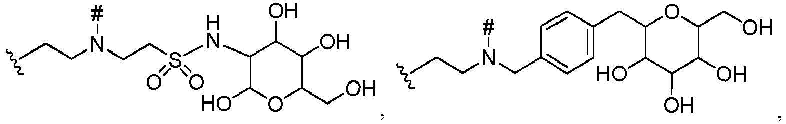 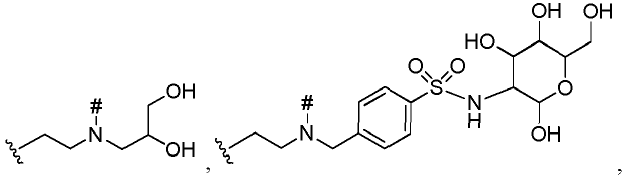 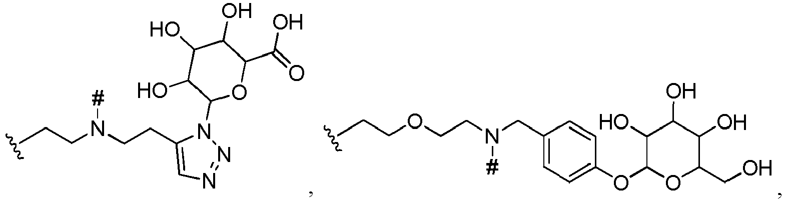 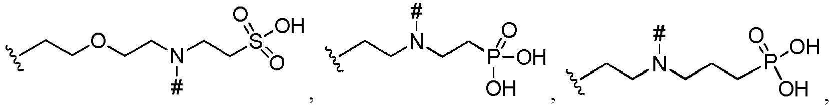 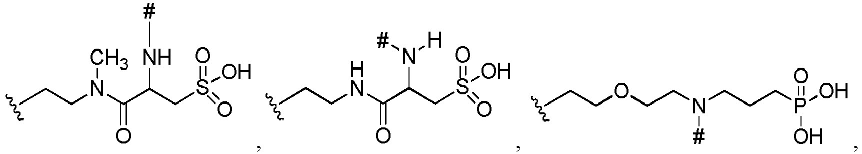 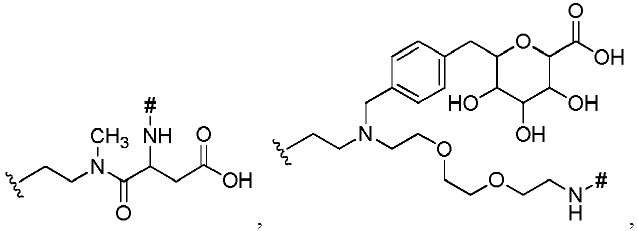 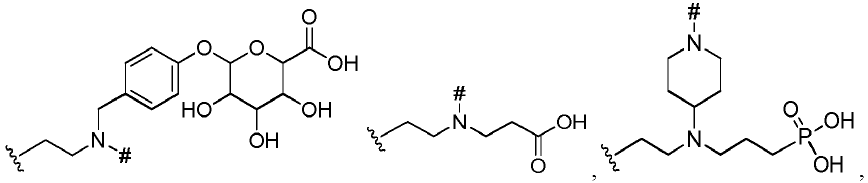 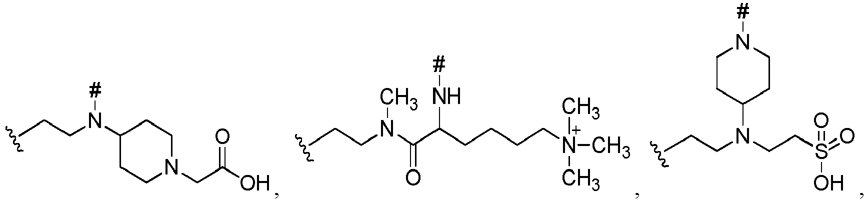 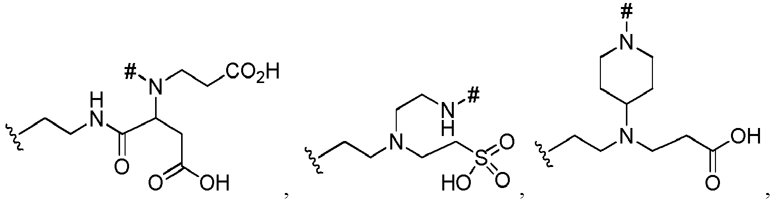 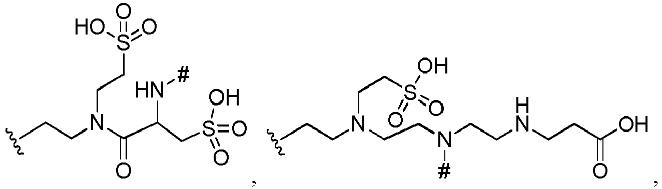 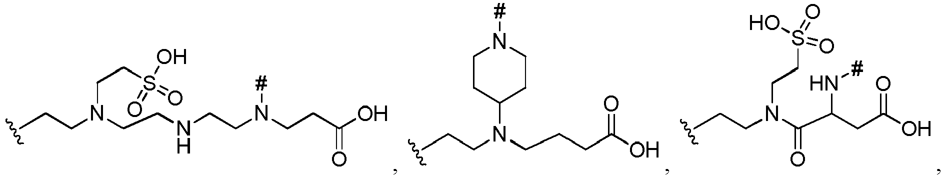 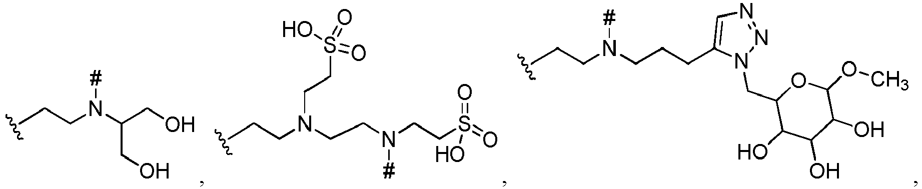 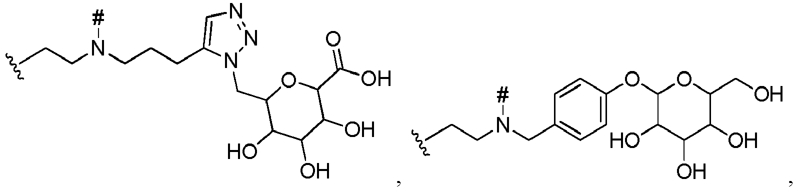 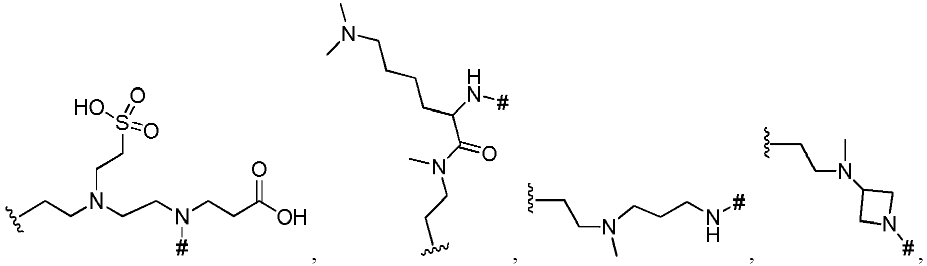 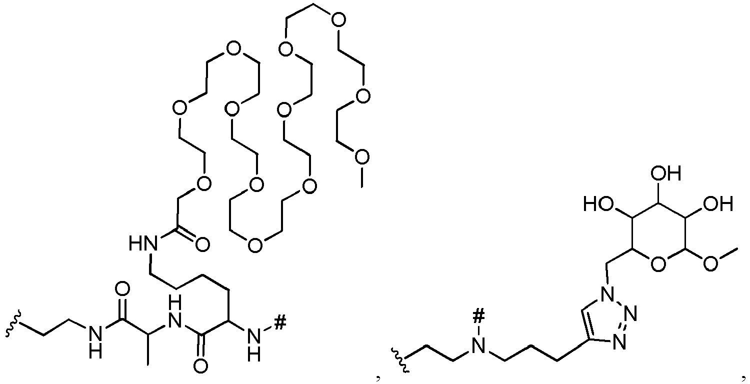 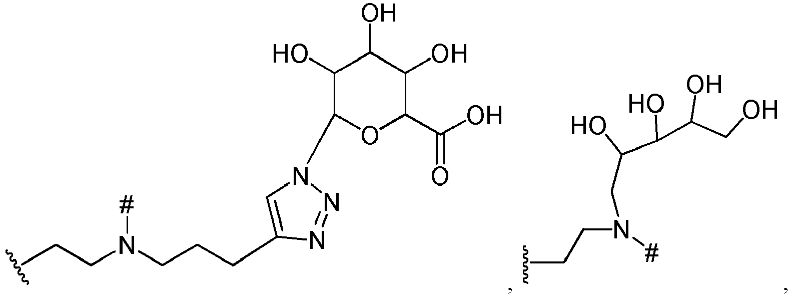 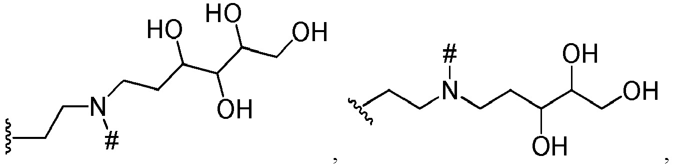 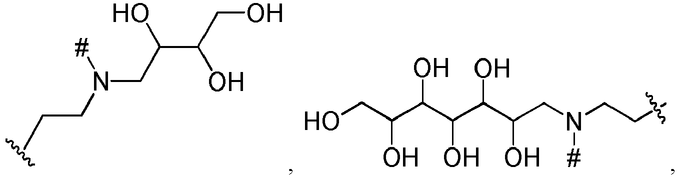 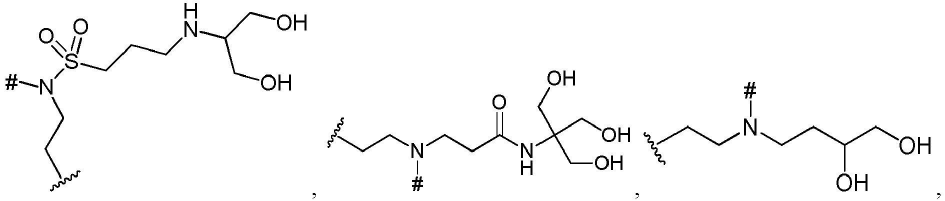 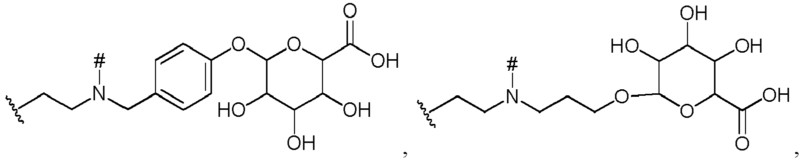 and 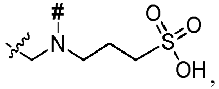 wherein # represents either a hydrogen atom in the Bcl-xL inhibitor drug of the ADCs of formula (IIb) or (IIc) or the point of attachment in the Bcl-xL inhibitor drug of the ADCs of formula (IIa) or (IId) to a linker L.

In one embodiment of any one of the aspects and embodiments herein, Ar¹ is selected from 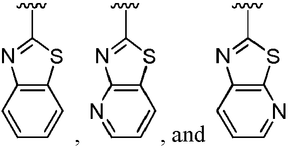 and is optionally substituted with one or more substituents independently selected from halo, cyano, methyl, and halomethyl.

In a further embodiment, Ar¹ is

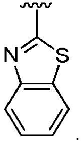

In one embodiment of any one of the aspects and embodiments herein, Ar² is 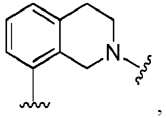 optionally substituted with one or more substituents.

In one embodiment of any one of the aspects and embodiments herein, Ar² is selected from 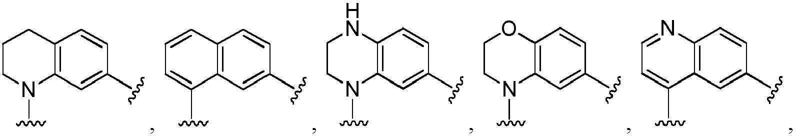 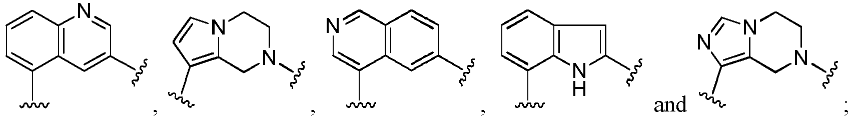 and is optionally substituted with one or more substituents.

In another embodiment, Ar² is substituted with one or more solubilizing groups.

In a further embodiment, each solubilizing group is, independently of the others, selected from a moiety containing a polyol, a polyethylene glycol with between 4 and 30 repeating units, a salt, or a moiety that is charged at physiological pH.

In another embodiment, Ar² is substituted with one or more solubilizing groups.

In a further embodiment, each solubilizing group is, independently of the others, selected from a moiety containing a polyol, a polyethylene glycol with between 4 and 30 repeating units, a salt, or a moiety that is charged at physiological pH.

In one embodiment of any one of the aspects and embodiments herein, Z¹ is N.

In one embodiment of any one of the aspects and embodiments herein, Z^{2a} is O.

In one embodiment of any one of the aspects and embodiments herein, R¹ is methyl or chloro.

In one embodiment of any one of the aspects and embodiments herein, R² is hydrogen or methyl.

In one embodiment of any one of the aspects and embodiments herein, R² is hydrogen.

In one embodiment of any one of the aspects and embodiments herein, Z^{2b} is O.

In one embodiment of any one of the aspects and embodiments herein, Z^{2b} is NH or CH₂.

In one embodiment of any one of the aspects and embodiments herein, the ADC is a compound according to structural formula (IIa).

In a further embodiment, the ADC includes a core selected from structures (C.1)-(C.21):

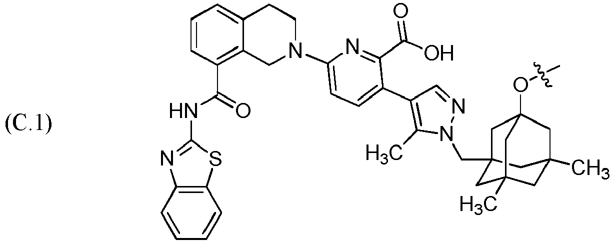

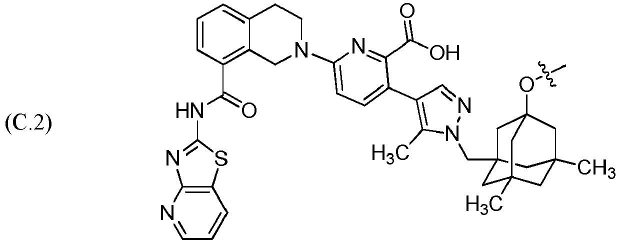

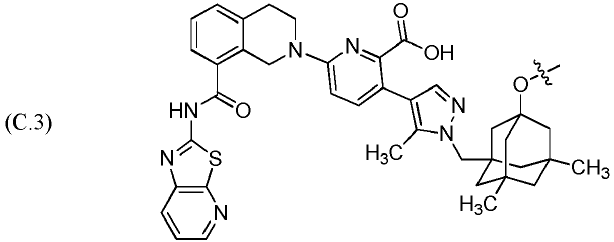

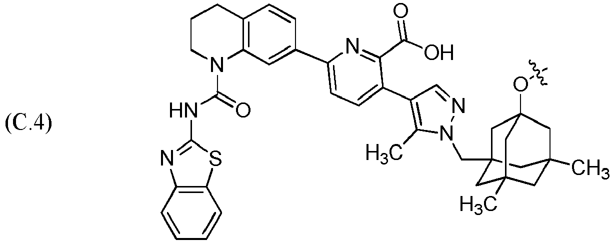

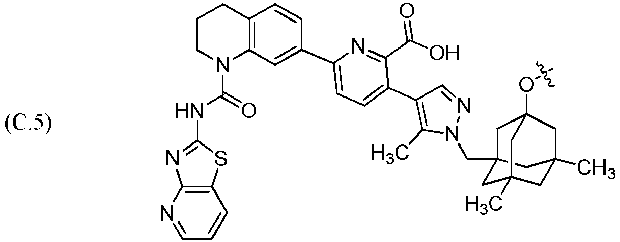

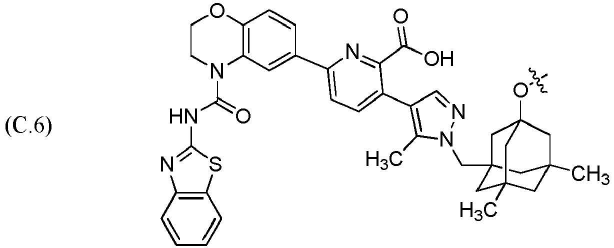

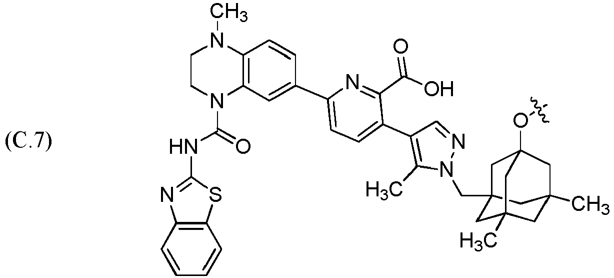

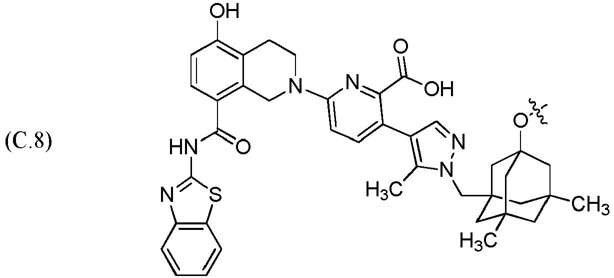

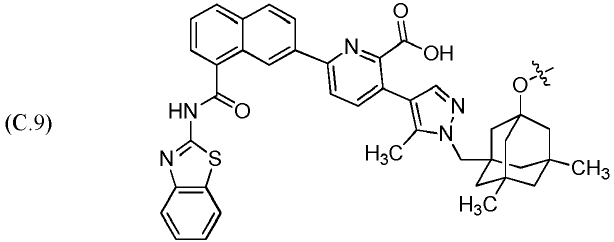

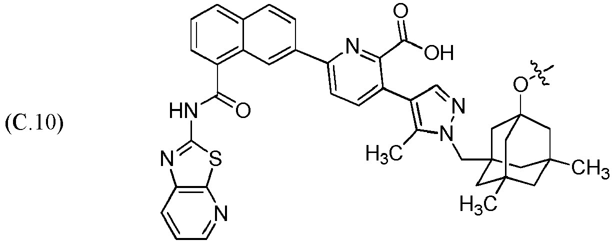

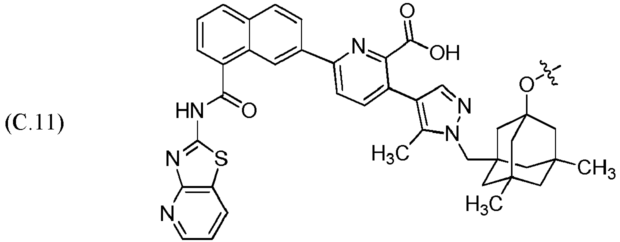

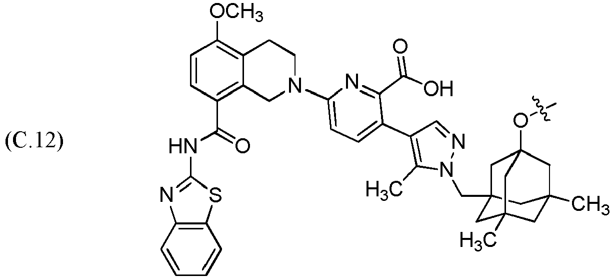

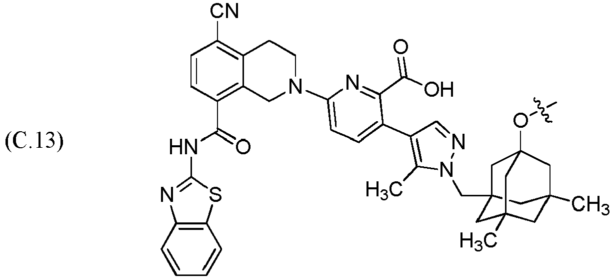

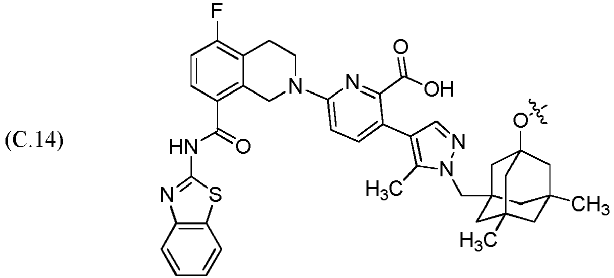

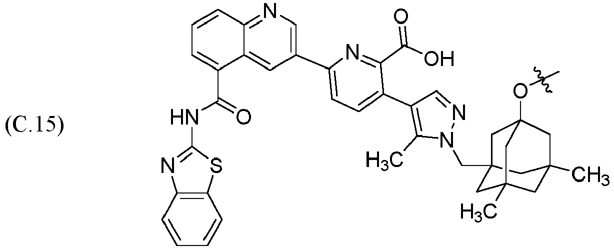

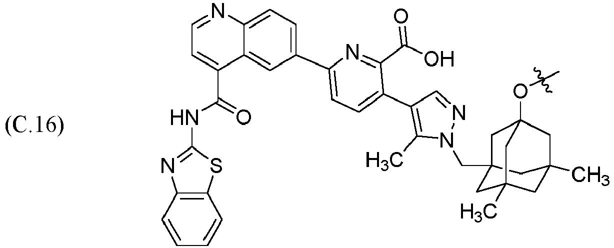

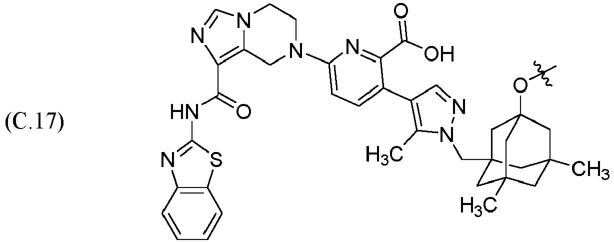

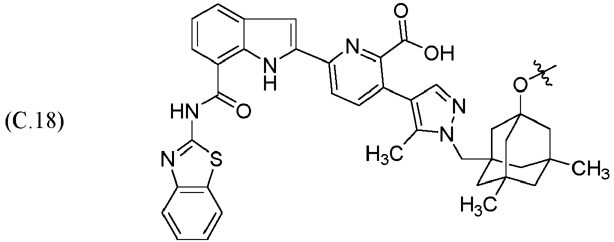

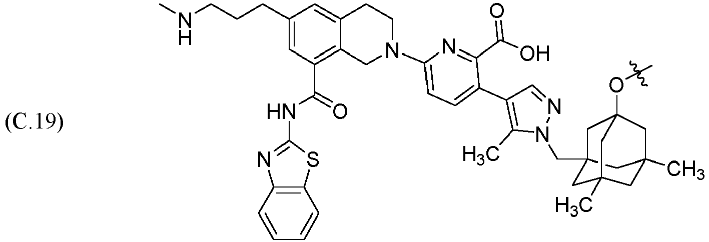

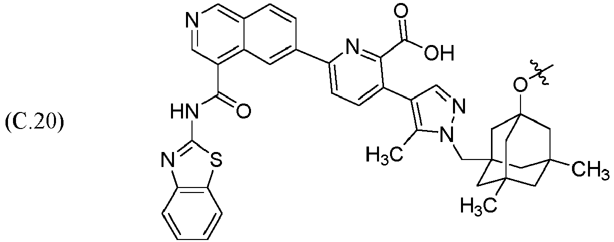

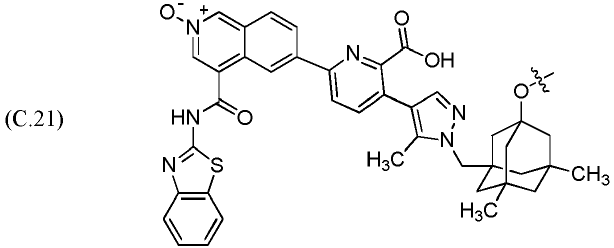

In another further embodiment, the ADC is a compound according to structural formula (IIa.1): 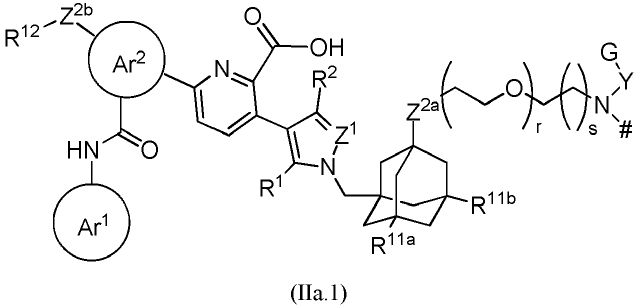 wherein:
Y is optionally substituted C₁-C₈ alkylene;
r is 0 or 1; and
s is 1, 2 or 3.

In another further embodiment, the ADC is a compound according to structural formula (IIa.2): 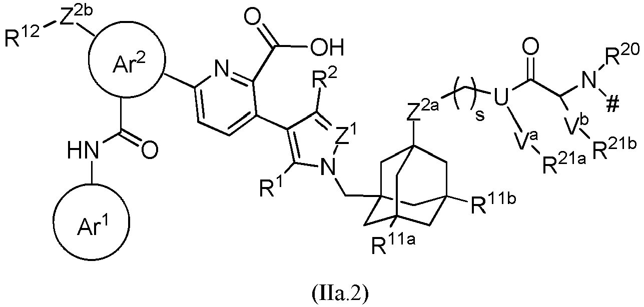 wherein:
U is selected from N, O and CH, with the proviso that when U is O, then V^{a} and R^{21a} are absent;
R²⁰ is selected from H and C₁-C₄ alkyl;
R^{21a} and R^{21b} are each, independently from one another, absent or selected from H, C₁-C₄ alkyl and G, where G is selected from a polyol, PEG4-30, a salt and a moiety that is charged at physiological pH;
V^{a} and V^{b} are each, independently from one another, absent or selected from a bond, and an optionally substituted alkylene;
R²⁰ is selected from H and C₁-C₄ alkyl; and
s is 1, 2 or 3.

In another further embodiment, the ADC is a compound according to structural formula (IIa.3): 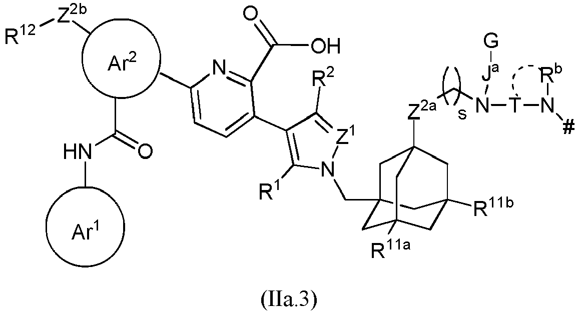 wherein:
R^{b} is selected from H, C₁-C₄ alkyl and J^{b}-G or is optionally taken together with an atom of T to form a ring having between 3 and 7 atoms;
J^{a} and J^{b} are each, independently from one another, selected from optionally substituted C₁-C₈ alkylene and optionally substituted phenylene;
T is selected from optionally substituted C₁-C₈ alkylene, CH₂CH₂OCH₂CH₂OCH₂CH₂, CH₂CH₂OCH₂CH₂OCH₂CH₂OCH₂ and a polyethylene glycol containing from 4 to 10 ethylene glycol units;
G is selected from a polyol, PEG4-30, a salt and a moiety that is charged at physiological pH; and
s is 1, 2 or 3.

In one embodiment of any one of the aspects and embodiments herein, the ADC is a compound according to structural formula (IIb).

In a further embodiment, the ADC is a compound according to structural formula (IIb.1): 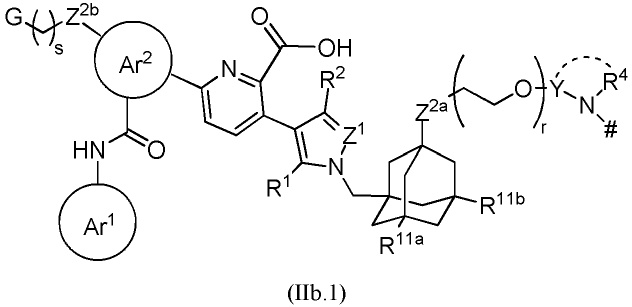 wherein:
Y is optionally substituted C₁-C₈ alkylene;
G is selected from a polyol, PEG4-30, a salt and a moiety that is charged at physiological pH;
r is 0 or 1; and
s is 1, 2 or 3.

In one embodiment of any one of the aspects and embodiments herein, the ADC is a compound according to structural formula (IIc).

In a further embodiment, the ADC is a compound according to structural formula (IIc.1): 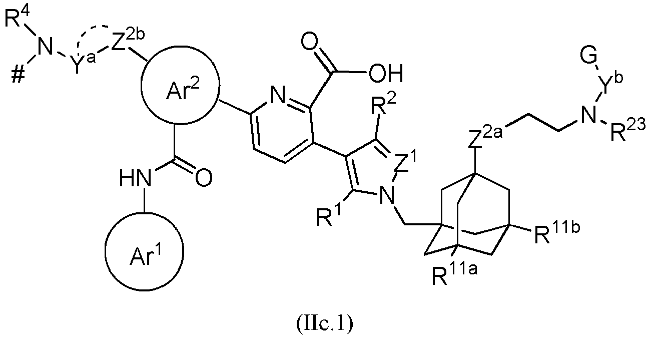 wherein:
Y^{a} is optionally substituted C₁-C₈ alkylene;
Y^{b} is optionally substituted C₁-C₈ alkylene;
R²³ is selected from H and C₁-C₄ alkyl; and
G is selected from a polyol, PEG4-30, a salt and a moiety that is charged at physiological pH.

In another further embodiment, the ADC is a compound according to structural formula (IIc.2): 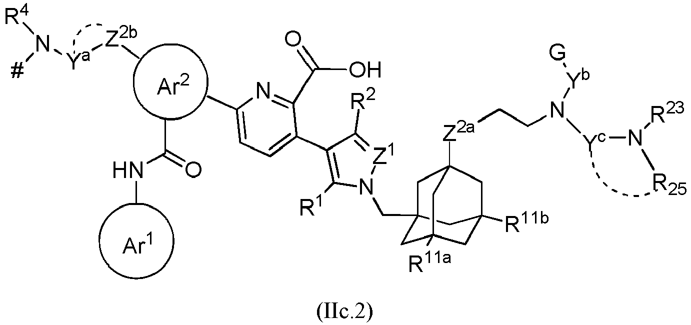 wherein:
Y^{a} is optionally substituted C₁-C₈ alkylene;
Y^{b} is optionally substituted C₁-C₈ alkylene;
Y^{c} is optionally substituted C₁-C₈ alkylene;
R²³ is selected from H and C₁-C₄ alkyl;
R²⁵ is Y^{b}-G or is taken together with an atom of Y^{c} to form a ring having 4-6 ring atoms; and
G is selected from a polyol, PEG4-30, a salt and a moiety that is charged at physiological pH.

In one embodiment of any one of the aspects and embodiments herein, the Bcl-xL inhibitor is selected from the group consisting of the following compounds modified in that the hydrogen corresponding to the # position of structural formula (IIa), (IIb), (IIc), or (IId) is not present forming a monoradical:
6-[8-(1,3-benzothiazol-2-ylcarbamoyl)-3,4-dihydroisoquinolin-2(1H)-yl]-3-[1-({3-[2-({2-[2-(carboxymethoxy)ethoxy]ethyl}amino)ethoxy]-5,7-dimethyltricyclo[3.3.1.1^{3,7}]dec-1-yl}methyl)-5-methyl-1H-pyrazol-4-yl]pyridine-2-carboxylic acid;
6-[8-(1,3-benzothiazol-2-ylcarbamoyl)-3,4-dihydroisoquinolin-2(1H)-yl]-3-{1-[(3,5-dimethyl-7-{2-[(2-sulfoethyl)amino]ethox}tricyclo[3.3.1.1^{3,7}]dec-1-yl)methyl]-5-methyl-1H-pyrazol-4-yl}pyridine-2-carboxylic acid;
2-{[(2-{[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl]amino}ethyl)sulfonyl]amino } -2-deoxy-D-glucopyranose;
6-[8-(1,3-benzothiazol-2-ylcarbamoyl)-3,4-dihydroisoquinolin-2(1H)-yl]-3-{1-[(3,5-dimethyl-7-{2-[(4-{[(3R,4R,5S,6R)-3,4,5-trihydroxy-6-(hydroxymethyl)tetrahydro-2H-pyran-2-yl]methyl}benzyl)amino]ethoxy}tricyclo[3.3.1.13,7]dec-1-yl)methyl]-5-methyl-1H-pyrazol-4-yl}pyridine-2-carboxylic acid;
6-[8-(1,3-benzothiazol-2-ylcarbamoyl)-3,4-dihydroisoquinolin-2(1H)-yl]-3-{1-[(3,5-dimethyl-7-{2-[(3-sulfopropyl)amino]ethoxy}tricyclo[3.3.1.1^{3,7}]dec-1-yl)methyl]-5-methyl-1H-pyrazol-4-yl}pyridine-2-carboxylic acid;
6-[8-(1,3-benzothiazol-2-ylcarbamoyl)-3,4-dihydroisoquinolin-2(1H)-yl]-3-{1-[(3-{2-[(2,3-dihydroxypropyl)amino]ethoxy}-5,7-dimethyltricyclo[3.3.1.1^{3,7}]dec-1-yl)methyl]-5-methyl-1H-pyrazol-4-yl}pyridine-2-carboxylic acid;
2-({[4-({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl]amino}methyl)phenyl]sulfonyl}amino)-2-deoxy-beta-D-glucopyranose;
8-(1,3-benzothiazol-2-ylcarbamoyl)-2-{6-carboxy-5-[1-({3-[2-({2-[1-(beta-D-glucopyranuronosyl)-1H-1,2,3-triazol-4-yl]ethyl}amino)ethoxy]-5,7-dimethyltricyclo[3.3.1.1^{3,7}]dec-1-yl}methyl)-5-methyl-1H-pyrazol-4-yl]pyridin-2-yl}-1,2,3,4-tetrahydroisoquinoline;
3-[1-({3-[2-(2-{[4-(beta-D-allopyranosyloxy)benzyl]amino}ethoxy)ethoxy]-5,7-dimethyltricyclo[3.3.1.1^{3,7}]dec-1-yl}methyl)-5-methyl-1H-pyrazol-4-yl]-6-[8-(1,3-benzothiazol-2-ylcarbamoyl)-3,4-dihydroisoquinolin-2(1H)-yl]pyridine-2-carboxylic acid;
6-[8-(1,3-benzothiazol-2-ylcarbamoyl)-3,4-dihydroisoquinolin-2(1H)-yl]-3-(1-{[3,5-dimethyl-7-(2-{2-[(2-sulfoethyl)amino]ethoxy}ethoxy)tricyclo[3.3.1.1^{3,7}]dec-1-yl]methyl}-5-methyl-1H-pyrazol-4-yl)pyridine-2-carboxylic acid;
6-[8-(1,3-benzothiazol-2-ylcarbamoyl)-3,4-dihydroisoquinolin-2(1H)-yl]-3-{1-[(3,5-dimethyl-7-{2-[(2-phosphonoethyl)amino]ethoxy}tricyclo[3.3.1.1^{3,7}]dec-1-yl)methyl]-5-methyl-1H-pyrazol-4-yl}pyridine-2-carboxylic acid;
6-[8-(1,3-benzothiazol-2-ylcarbamoyl)-3,4-dihydroisoquinolin-2(1H)-yl]-3-{1-[(3,5-dimethyl-7-{2-[methyl(3-sulfo-L-alanyl)amino]ethoxy}tricyclo[3.3.1.1^{3,7}]dec-1-yl)methyl]-5-methyl-1H-pyrazol-4-yl}pyridine-2-carboxylic acid;
6-[8-(1,3-benzothiazol-2-ylcarbamoyl)-3,4-dihydroisoquinolin-2(1H)-yl]-3-{1-[(3,5-dimethyl-7-{2-[(3-phosphonopropyl)amino]ethoxy}tricyclo[3.3.1.1^{3,7}]dec-1-yl)methyl]-5-methyl-1H-pyrazol-4-yl}pyridine-2-carboxylic acid;
6-[8-(1,3-benzothiazol-2-ylcarbamoyl)-3,4-dihydroisoquinolin-2(1H)-yl]-3-{1-[(3,5-dimethyl-7-{2-[(3-sulfo-L-alanyl)amino]ethoxy}tricyclo[3.3.1.1^{3,7}]dec-1-yl)methyl]-5-methyl-1H-pyrazol-4-yl}pyridine-2-carboxylic acid;
6-[8-(1,3-benzothiazol-2-ylcarbamoyl)-3,4-dihydroisoquinolin-2(1H)-yl]-3-(1-{[3,5-dimethyl-7-(2-{2-[(3-phosphonopropyl)amino]ethoxy]ethoxy)tricyclo[3.3.1.1^{3,7}]dec-1-yl]methyl}-5-methyl-1H-pyrazol-4-yl)pyridine-2-carboxylic acid;
3-{1-[(3-{2-[L-alpha-aspartyl(methyl)amino]ethoxy}-5,7-dimethyltricyclo[3.3.1.1^{3,7}]dec-1-yl)methyl]-5-methyl-1H-pyrazol-4-yl}-6-[8-(1,3-benzothiazol-2-ylcarbamoyl)-3,4-dihydroisoquinolin-2(1H)-yl]pyridine-2-carboxylic acid;
6-{4-[({2-[2-(2-aminoethoxy)ethoxy]ethyl}[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl]amino)methyl]benzyl}-2,6-anhydro-L-gulonic acid;
4-({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl]amino}methyl)phenyl hexopyranosiduronic acid;
6-[1-(1,3-benzothiazol-2-ylcarbamoyl)-1,2,3,4-tetrahydroquinolin-7-yl]-3-{1-[(3,5-dimethyl-7-{2-[(2-phosphonoethyl)amino]ethoxy}tricyclo[3.3.1.1^{3,7}]dec-1-yl)methyl]-5-methyl-1H-pyrazol-4-yl}pyridine-2-carboxylic acid;
6-[1-(1,3-benzothiazol-2-ylcarbamoyl)-1,2,3,4-tetrahydroquinolin-7-yl]-3-{1-[(3,5-dimethyl-7-{2-[methyl(3-sulfo-L-alanyl)amino]ethoxy}tricyclo[3.3.1.1^{3,7}]dec-1-yl)methyl]-5-methyl-1H-pyrazol-4-yl}pyridine-2-carboxylic acid;
3-{1-[(3,5-dimethyl-7-{2-[(2-sulfoethyl)amino]ethoxy}tricyclo[3.3.1.1^{3,7}]dec-1-yl)methyl]-5-methyl-1H-pyrazol-4-yl}-6-[8-([1,3]thiazolo[5,4-b]pyridin-2-ylcarbamoyl)-3,4-dihydroisoquinolin-2(1H)-yl]pyridine-2-carboxylic acid;
3-{1-[(3,5-dimethyl-7-{2-[(2-sulfoethyl)amino]ethoxy}tricyclo[3.3.1.1^{3,7}]dec-1-yl)methyl]-5-methyl-1H-pyrazol-4-yl}-6-[8-([1,3]thiazolo[4,5-b]pyridin-2-ylcarbamoyl)-3,4-dihydroisoquinolin-2(1H)-yl]pyridine-2-carboxylic acid;
6-[1-(1,3-benzothiazol-2-ylcarbamoyl)-1,2,3,4-tetrahydroquinolin-7-yl]-3-{1-[(3,5-dimethyl-7-{2-[(2-sulfoethyl)amino]ethoxy}tricyclo[3.3.1.1^{3,7}]dec-1-yl)methyl]-5-methyl-1H-pyrazol-4-yl}pyridine-2-carboxylic acid;
6-[8-(1,3-benzothiazol-2-ylcarbamoyl)-3,4-dihydroisoquinolin-2(1H)-yl]-3-{1-[(3-{2-[(2-carboxyethyl)amino]ethoxy}-5,7-dimethyltricyclo[3.3.1.1^{3,7}]dec-1-yl)methyl]-5-methyl-1H-pyrazol-4-yl}pyridine-2-carboxylic acid;
6-[8-(1,3-benzothiazol-2-ylcarbamoyl)-3,4-dihydroisoquinolin-2(1H)-yl]-3-{1-[(3,5-dimethyl-7-{2-[(3-phosphonopropyl)(piperidin-4-yl)amino]ethoxy}tricyclo[3.3.1.1^{3,7}]dec-1-yl)methyl]-5-methyl-1H-pyrazol-4-yl}pyridine-2-carboxylic acid;
3-{1-[(3-{2-[D-alpha-aspartyl(methyl)amino]ethoxy}-5,7-dimethyltricyclo[3.3.1.1^{3,7}]dec-1-yl)methyl]-5-methyl-1H-pyrazol-4-yl}-6-[8-(1,3-benzothiazol-2-ylcarbamoyl)-3,4-dihydroisoquinolin-2(1H)-yl]pyridine-2-carboxylic acid;
6-[8-(1,3-benzothiazol-2-ylcarbamoyl)-3,4-dihydroisoquinolin-2(1H)-yl]-3-(1-{[3-(2-{[1-(carboxymethyl)piperidin-4-yl]amino}ethoxy)-5,7-dimethyltricyclo[3.3.1.1^{3,7}]dec-1-yl]methyl}-5-methyl-1H-pyrazol-4-yl)pyridine-2-carboxylic acid;
N-[(5S)-5-amino-6-{[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](methyl)amino}-6-oxohexyl]-N,N-dimethylmethanaminium;
6-[8-(1,3-benzothiazol-2-ylcarbamoyl)-3,4-dihydroisoquinolin-2(1H)-yl]-3-{1-[(3,5-dimethyl-7-{2-[piperidin-4-yl(2-sulfoethyl)amino]ethoxy}tricyclo[3.3.1.1^{3,7}]dec-1-yl)methyl]-5-methyl-1H-pyrazol-4-yl}pyridine-2-carboxylic acid;
6-[8-(1,3-benzothiazol-2-ylcarbamoyl)-5-(3-phosphonopropoxy)-3,4-dihydroisoquinolin-2(1H)-yl]-3-[1-({3,5-dimethyl-7-[2-(methylamino)ethoxy]tricyclo[3.3.1.1^{3,7}]dec-1-yl}methyl)-5-methyl-1H-pyrazol-4-yl]pyridine-2-carboxylic acid;
6-[8-(1,3-benzothiazol-2-ylcarbamoyl)-3,4-dihydroisoquinolin-2(1H)-yl]-3-(1-{[3-(2-{[N-(2-carboxyethyl)-L-alpha-aspartyl]amino}ethoxy)-5,7-dimethyltricyclo[3.3.1.1^{3,7}]dec-1-yl]methyl}-5-methyl-1H-pyrazol-4-yl)pyridine-2-carboxylic acid;
3-{1-[(3-{2-[(2-aminoethyl)(2-sulfoethyl)amino]ethoxy}-5,7-dimethyltricyclo[3.3.1.1^{3,7}]dec-1-yl)methyl]-5-methyl-1H-pyrazol-4-yl}-6-[8-(1,3-benzothiazol-2-ylcarbamoyl)-3,4-dihydroisoquinolin-2(1H)-yl]pyridine-2-carboxylic acid;
6-[5-(2-aminoethoxy)-8-(1,3-benzothiazol-2-ylcarbamoyl)-3,4-dihydroisoquinolin-2(1H)-yl]-3-[1-({3,5-dimethyl-7-[2-(methylarnino)ethoxy]tricyclo[3.3.1.1^{3,7}]dec-1-yl}methyl)-5-methyl-1H-pyrazol-4-yl]pyridine-2-carboxylic acid;
6-[8-(1,3-benzothiazol-2-ylcarbamoyl)naphthalen-2-yl]-3-{1-[(3,5-dimethyl-7-{2-[(3-sulfopropyl)amino]ethoxy}tricyclo[3.3.1.1^{3,7}]dec-1-yl)methyl]-5-methyl-1H-pyrazol-4-yl}pyridine-2-carboxylic acid;
6-[8-(1,3-benzothiazol-2-ylcarbamoyl)-3,4-dihydroisoquinolin-2(1H)-yl]-3-{1-[(3-{2-[(2-carboxyethyl)(piperidin-4-yl)amino]ethoxy}-5,7-dimethyltricyclo[3.3.1.1^{3,7}]dec-1-yl)methyl]-5-methyl-1H-pyrazol-4-yl}pyridine-2-carboxylic acid;
6-[8-(1,3-benzothiazol-2-ylcarbamoyl)-3,4-dihydroisoquinolin-2(1H)-yl]-3-{1-[(3,5-dimethyl-7-{2-[(3-sulfo-L-alanyl)(2-sulfoethyl)amino]ethoxy]tricyclo[3.3.1.1^{3,7}]dec-1-yl)methyl]-5-methyl-1H-pyrazol-4-yl}pyridine-2-carboxylic acid;
6-[8-(1,3-benzothiazol-2-ylcarbamoyl)-3,4-dihydroisoquinolin-2(1H)-yl]-3-{1-[(3-{2-[{2-[(2-carboxyethyl)amino]ethyl}(2-sulfoethyl)amino]ethoxy}-5,7-dimethyltricyclo[3.3.1.1^{3,7}]dec-1-yl)methyl]-5-methyl-1H-pyrazol-4-yl}pyridine-2-carboxylic acid;
3-{1-[(3,5-dimethyl-7-{2-[(3-phosphonopropyl)amino]ethoxy}tricyclo[3.3.1.1^{3,7}]dec-1-yl)methyl]-5-methyl-1H-pyrazol-4-yl}-6-[8-([1,3]thiazolo[4,5-b]pyridin-2-ylcarbamoyl)-3,4-dihydroisoquinolin-2(1H)-yl]pyridine-2-carboxylic acid;
3-{1-[(3,5-dimethyl-7-{2-[(3-phosphonopropyl)amino]ethoxy}tricyclo[3.3.1.1^{3,7}]dec-1-yl)methyl]-5-methyl-1H-pyrazol-4-yl}-6-[8-([1,3]thiazolo[5,4-b]pyridin-2-ylcarbamoyl)-3,4-dihydroisoquinolin-2(1H)-yl]pyridine-2-carboxylic acid;
6-[8-(1,3-benzothiazol-2-ylcarbamoyl)-5-(carboxymethoxy)-3,4-dihydroisoquinolin-2(1H)-yl]-3-[1-({3,5-dimethyl-7-[2-(methylamino)ethoxy]tricyclo[3.3.1.1^{3,7}]dec-1-yl}methyl)-5-methyl-1H-pyrazol-4-yl]pyridine-2-carboxylic acid;
6-[8-(1,3-benzothiazol-2-ylcarbamoyl)-3,4-dihydroisoquinolin-2(1H)-yl]-3-{1-[(3-{2-[(3-carboxypropyl)(piperidin-4-yl)amino]ethoxy}-5,7-dimethyltricyclo[3.3.1.1^{3,7}]dec-1-yl)methyl]-5-methyl-1H-pyrazol-4-yl}pyridine-2-carboxylic acid;
6-[8-(1,3-benzothiazol-2-ylcarbamoyl)naphthalen-2-yl]-3-{1-[(3,5-dimethyl-7-{2-[(2-sulfoethyl)amino]ethoxy}tricyclo[3.3.1.1^{3,7}]dec-1-yl)methyl]-5-methyl-1H-pyrazol-4-yl}pyridine-2-carboxylic acid;
3-{1-[(3-{2-[L-alpha-aspartyl(2-sulfoethyl)amino]ethoxy}-5,7-dimethyltricyclo[3.3.1.1^{3,7}]dec-1-yl)methyl]-5-methyl-1H-pyrazol-4-yl}-6-[8-(1,3-benzothiazol-2-ylcarbamoyl)-3,4-dihydroisoquinolin-2(1H)-yl]pyridine-2-carboxylic acid;
6-[8-(1,3-benzothiazol-2-ylcarbamoyl)-3,4-dihydroisoquinolin-2(1H)-yl]-3-{1-[(3-{2-[(1,3-dihydroxypropan-2-yl)amino]ethoxy}-5,7-dimethyltricyclo[3.3.1.1^{3,7}]dec-1-yl)methyl]-5-methyl-1H-pyrazol-4-yl}pyridine-2-carboxylic acid;
6-[5-(2-aminoethoxy)-8-(1,3-benzothiazol-2-ylcarbamoyl)-3,4-dihydroisoquinolin-2(1H)-yl]-3-{1-[(3,5-dimethyl-7-{2-[methyl(2-sulfoethyl)amino]ethoxy]tricyclo[3.3.1.1^{3,7}]dec-1-yl)methyl]-5-methyl-1H-pyrazol-4-yl}pyridine-2-carboxylic acid;
6-[8-(1,3-benzothiazol-2-ylcarbamoyl)-5-{2-[(2-sulfoethyl)amino]ethoxy}-3,4-dihydroisoquinolin-2(1H)-yl]-3-{1-[(3,5-dimethyl-7-{2-[methyl(2-sulfoethyl)amino]ethoxy}tricyclo[3.3.1.1^{1,7}]dec-1-yl)methyl]-5-methyl-1H-pyrazol-4-yl}pyridine-2-carboxylic acid;
6-[8-(1,3-benzothiazol-2-ylcarbamoyl)-3,4-dihydroisoquinolin-2(1H)-yl]-3-{1-[(3,5-dimethyl-7-{2-[(2-sulfoethyl){2-[(2-sulfoethyl)amino]ethyl}amino]ethoxy}tricyclo[3.3.1.1^{3,7}]dec-1-yl)methyl]-5-methyl-1H-pyrazol-4-yl}pyridine-2-carboxylic acid;
6-[8-(1,3-benzothiazol-2-ylcarbamoyl)-5-{2-[(2-carboxyethyl)amino]ethoxy}-3,4-dihydroisoquinolin-2(1H)-yl]-3-{1-[(3,5-dimethyl-7-{2-[methyl(2-sulfoethyl)amino]ethoxy}tricyclo[3.3.1.1^{3,7}]dec-1-yl)methyl]-5-methyl-1H-pyrazol-4-yl}pyridine-2-carboxylic acid;
3-{1-[(3,5-dimethyl-7-{2-[(3-phosphonopropyl)(piperidin-4-yl)amino]ethoxy}tricyclo[3.3.1.1^{3,7}]dec-1-yl)methyl]-5-methyl-1H-pyrazol-4-yl}-6-[8-([1,3]thiazolo[4,5-b]pyridin-2-ylcarbamoyl)-3,4-dihydroisoquinolin-2(1H)-yl]pyridine-2-carboxylic acid;
6-[4-(1,3-benzothiazol-2-ylcarbamoyl)-3,4-dihydro-2H-1,4-benzoxazin-6-yl]-3-{1-[(3,5-dimethyl-7-{2-[(2-sulfoethyl)amino]ethoxy}tricyclo[3.3.1.1^{3,7}]dec-1-yl)methyl]-5-methyl-1H-pyrazol-4-yl}pyridine-2-carboxylic acid;
6-[8-(1,3-benzothiazol-2-ylcarbamoyl)-5-(3-sulfopropoxy)-3,4-dihydroisoquinolin-2(1H)-yl]-3-[1-({3,5-dimethyl-7-[2-(methylamino)ethoxy]tricyclo[3.3.1.1^{3,7}]dec-1-yl}methyl)-5-methyl-1H-pyrazol-4-yl]pyridine-2-carboxylic acid;
3-{1-[(3,5-dimethyl-7-{2-[(2-sulfoethyl)amino]ethoxy}tricyclo[3.3.1.1^{3,7}]dec-1-yl)methyl]-5-methyl-1H-pyrazol-4-yl}-6-[1-([1,3]thiazolo[4,5-b]pyridin-2-ylcarbamoyl)-1,2,3,4-tetrahydroquinolin-7-yl]pyridine-2-carboxylic acid;
3-{1-[(3,5-dimethyl-7-{2-[(2-sulfoethyl)amino]ethoxy}tricyclo[3.3.1.1^{3,7}]dec-1-yl)methyl]-5-methyl-1H-pyrazol-4-yl}-6-[8-([1,3]thiazolo[4,5-b]pyridin-2-ylcarbamoyl)naphthalen-2-yl]pyridine-2-carboxylic acid;
(1ξ)-1-({2-[5-(1-{[3-(2-aminoethoxy)-5,7-dimethyltricyclo[3.3.1.1^{3,7}]dec-1-yl]methyl}-5-methyl-1*H*-pyrazol-4-yl)-6-carboxypyridin-2-yl]-8-(1,3-benzothiazol-2-ylcarbamoyl)-1,2,3,4-tetrahydroisoquinolin-5-yl}methyl)-1,5-anhydro-D-glucitol;
6-[8-(1,3-benzothiazol-2-ylcarbamoyl)-3,4-dihydroisoquinolin-2(1H)-yl]-3-{1-[(3-{2-[(3-carboxypropyl)amino]ethoxy}-5,7-dimethyltricyclo[3.3.1.1^{3,7}]dec-1-yl)methyl]-5-methyl-1H-pyrazol-4-yl}pyridine-2-carboxylic acid;
6-[8-(1,3-benzothiazol-2-ylcarbamoyl)naphthalen-2-yl]-3-{1-[(3,5-dimethyl-7-{2-[(3-phosphonopropyl)amino]ethoxy}tricyclo[3.3.1.1^{3,7}]dec-1-yl)methyl]-5-methyl-1H-pyrazol-4-yl}pyridine-2-carboxylic acid;
6-[8-(1,3-benzothiazol-2-ylcarbamoyl)-3,4-dihydroisoquinolin-2(1H)-yl]-3-(-(1-{[3-(2-{[4-(beta-D-glucopyranosyloxy)benzyl]amino}ethoxy)-5,7-dimethyltricyclo[3.3.1.1^{3,7}]dec-1-yl]methyl}-5-methyl-1H-pyrazol-4-yl)pyridine-2-carboxylic acid;
3-(1-{[3-(2-{[4-(beta-D-allopyranosyloxy)benzyl]amino}ethoxy)-5,7-dimethyltricyclo[3.3.1.1^{3,7}]dec-1-yl]methyl}-5-methyl-1H-pyrazol-4-yl)-6-[8-(1,3-benzothiazol-2-ylcarbamoyl)-3,4-dihydroisoquinolin-2(1H)-yl]pyridine-2-carboxylic acid;
3-{1-[(3-{2-[azetidin-3-yl(2-sulfoethyl)amino]ethoxy}-5,7-dimethyltricyclo[3.3.1.1^{3,7}]dec-1-yl)methyl]-5-methyl-1H-pyrazol-4-yl}-6-[8-(1,3-benzothiazol-2-ylcarbamoyl)-3,4-dihydroisoquinolin-2(1H)-yl]pyridine-2-carboxylic acid;
3-{1-[(3-{2-[(3-aminopropyl)(2-sulfoethyl)amnio]ethoxy}-5,7-dimethyltricyclo[3.3.1.1^{3,7}]dec-1-yl)methyl]-5-methyl-1H-pyrazol-4-yl}-6-[8-(1,3-benzothiazol-2-ylcarbamoyl)-3,4-dihydroisoquinolin-2(1H)-yl]pyridine-2-carboxylic acid;
6-[1-(1,3-benzothiazol-2-ylcarbamoyl)-1,2,3,4-tetrahydroquinolin-7-yl]-3-{1-[(3-{2-[(2-carboxyethyl)amino]ethoxy}-5,7-dimethyltricyclo[3.3.1.1^{3,7}]dec-1-yl)methyl]-5-methyl-1H-pyrazol-4-yl}pyridine-2-carboxylic acid;
6-[8-(1,3-benzothiazol-2-ylcarbamoyl)-3,4-dihydroisoquinolin-2(1H)-yl]-3-{1-[(3-{2-[(N⁶,N⁶-dimethyl-L-lysyl)(methyl)amino]ethoxy}-5,7-dimethyltricyclo[3.3.1.1^{3,7}]dec-1-yl)methyl]-5-methyl-1H-pyrazol-4-yl}pyridine-2-carboxylic acid;
3-{1-[(3-{2-[(3-aminopropyl)(methyl)amino]ethoxy}-5,7-dimethyltricyclo[3.3.1.1^{3,7}]dec-1-yl)methyl]-5-methyl-1H-pyrazol-4-yl}-6-[1-(1,3-benzothiazol-2-ylcarbamoyl)-1,2,3,4-tetrahydroquinolin-7-yl]pyridine-2-carboxylic acid;
3-{1-[(3-{2-[azetidin-3-yl(methyl)amino]ethoxy}-5,7-dimethyltricyclo[3.3.1.1^{3,7}]dec-1-yl)methyl]-5-methyl-1H-pyrazol-4-yl}-6-[1-(1,3-benzothiazol-2-ylcarbamoyl)-1,2,3,4-tetrahydroquinolin-7-yl]pyridine-2-carboxylic acid;
N⁶-(37-oxo-2,5,8,11,14,17,20,23,26,29,32,35-dodecaoxaheptatriacontan-37-yl)-L-lysyl-N-[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl]-L-alaninamide;
methyl 6-[4-(3-{[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl]amnio}propyl)-1H-1,2,3-triazol-1-yl]-6-deoxy-beta-L-glucopyranoside;
6-[8-(1,3-benzothiazol-2-ylcarbamoyl)naphthalen-2-yl]-3-{1-[(3-{2-[(2-carboxyethyl)amino]ethoxy}-5,7-dimethyltricyclo[3.3.1.1^{3,7}]dec-1-yl)methyl]-5-methyl-1H-pyrazol-4-yl}pyridine-2-carboxylic acid;
6-[5-(1,3-benzothiazol-2-ylcarbamoyl)quinolin-3-yl]-3-{1-[(3,5-dimethyl-7-{2-[(2-sulfoethyl)amino]ethoxy}tricyclo[3.3.1.1^{3,7}]dec-1-yl)methyl]-5-methyl-1H-pyrazol-4-yl}pyridine-2-carboxylic acid;
6-[4-(1,3-benzothiazol-2-ylcarbamoyl)quinolin-6-yl]-3-{1-[(3,5-dimethyl-7-{2-[(2-sulfoethyl)amino]ethoxy}tricyclo[3.3.1.1^{3,7}]dec-1-yl)methyl]-5-methyl-1H-pyrazol-4-yl}pyridine-2-carboxylic acid;
6-[5-(1,3-benzothiazol-2-ylcarbamoyl)quinolin-3-yl]-3-{1-[(3-{2-[(2-carboxyethyl)amino]ethoxy}-5,7-dimethyltricyclo[3.3.1.1^{3,7}]dec-1-yl)methyl]-5-methyl-1H-pyrazol-4-yl}pyridine-2-carboxylic acid;
6-[1-(1,3-benzothiazol-2-ylcarbamoyl)-5,6-dihydroimidazo[1,5-a]pyrazin-7(8H)-yl]-3-{1-[(3,5-dimethyl-7-{2-[(2-sulfoethyl)amino]ethoxy}tricyclo[3.3.1.1^{3,7}]dec-1-yl)methyl]-5-methyl-1H-pyrazol-4-yl}pyridine-2-carboxylic acid;
8-(1,3-benzothiazol-2-ylcarbamoyl)-2-{6-carboxy-5-[1-({3-[2-({3-[1-(beta-D-glucopyranuronosyl)-1H-1,2,3-triazol-4-yl]propyl}amino)ethoxy]-5,7-dimethyltricyclo[3.3.1.1^{3,7}]dec-1-yl}methyl)-5-methyl-1H-pyrazol-4-yl]pyridin-2-yl}-1,2,3,4-tetrahydroisoquinoline;
6-[7-(1,3-benzothiazol-2-ylcarbamoyl)-1H-indol-2-yl]-3-{1-[(3,5-dimethyl-7-{2-[(2-sulfoethyl)amino]ethoxy}tricyclo[3.3.1.1^{3,7}]dec-1-yl)methyl]-5-methyl-1H-pyrazol-4-yl}pyridine-2-carboxylic acid;
6-[8-(1,3-benzothiazol-2-ylcarbamoyl)-6-[3-(methylamino)propyl]-3,4-dihydroisoquinolin-2(1H)-yl]-3-{1-[(3,5-dimethyl-7-{2-[(2-sulfoethyl)amino]ethoxy}tricyclo[3.3.1.1^{3,7}]dec-1-yl)methyl]-5-methyl-1H-pyrazol-4-yl}pyridine-2-carboxylic acid;
5-{[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl]amino}-5-deoxy-D-arabinitol;
1-{[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl]amino}-1,2-dideoxy-D-arabino-hexitol;
6-[4-(1,3-benzothiazol-2-ylcarbamoyl)isoquinolin-6-yl]-3-{1-[(3,5-dimethyl-7-{2-[(2-sulfoethyl)amino]ethoxy}tricyclo[3.3.1.1^{3,7}]dec-1-yl)methyl]-5-methyl-1H-pyrazol-4-yl}pyridine-2-carboxylic acid;
6-[8-(1,3-benzothiazol-2-ylcarbamoyl)-3,4-dihydroisoquinolin-2(1H)-yl]-3-((1-{[3-(2-{[3-hydroxy-2-(hydroxymethyl)propyl]amino}ethoxy)-5,7-dimethyltricyclo[3.3.1.1^{3,7}]dec-1-yl]methyl}-5-methyl-1H-pyrazol-4-yl)pyridine-2-carboxylic acid;
1-{[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl} oxy)ethyl] amino }-1,2-dideoxy-D-erythro-pentitol;
6-[8-(1,3-benzothiazol-2-ylcarbamoyl)-3,4-dihydroisoquinolin-2(1H)-yl]-3-(1-{[3,5-dimethyl-7-(2-{[(2S,3S)-2,3,4-trihydroxybutyl]amino}ethoxy)tricyclo[3.3.1.1^{3,7}]dec-1-yl]methyl}-5-methyl-1H-pyrazol-4-yl)pyridine-2-carboxylic acid;
6-[8-(1,3-benzothiazol-2-ylcarbamoyl)-3,4-dihydroisoquinolin-2(1H)-yl]-3-(1-{[3-(2-{[(2S,3S,4R,5R,6R)-2,3,4,5,6,7-hexahydroxyheptyl]amino}ethoxy)-5,7-dimethyltricyclo[3.3.1.1^{3,7}]dec-1-yl]methyl}-5-methyl-1H-pyrazol-4-yl)pyridine-2-carboxylicacid;
6-[8-(1,3-benzothiazol-2-ylcarbamoyl)-3,4-dihydroisoquinolin-2(1H)-yl]-3-{1-[(3-{2-[({3-[(1,3-dihydroxypropan-2-yl)amino]propyl}sulfonyl)amino]ethoxy}-5,7-dimethyltricyclo[3.3.1.1^{3,7}]dec-1-yl)methyl]-5-methyl-1H-pyrazol-4-yl}pyridine-2-carboxylic acid;
6-[8-(1,3-benzothiazol-2-ylcarbamoyl)-3,4-dihydroisoquinolin-2(1H)-yl]-3-{1-[(3-{2-[(3-{[1,3-dihydroxy-2-(hydroxymethyl)propan-2-yl]amino}-3-oxopropyl)amino]ethoxy}-5,7-dimethyltricyclo[3.3.1.1^{3,7}]dec-1-yl)methyl]-5-methyl-1H-pyrazol-4-yl}pyridine-2-carboxylic acid;
6-[8-(1,3-benzothiazol-2-ylcarbamoyl)-3,4-dihydroisoquinolin-2(1H)-yl]-3-(1-{[3-(2-{[(3*S*)-3,4-dihydroxybutyl]amino}ethoxy)-5,7-dimethyltricyclo[3.3.1.1^{3,7}]dec-1-yl]methyl}-5-methyl-1*H-*pyrazol-4-yl)pyridine-2-carboxylic acid;
4-({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl]amino}methyl)phenyl beta-D-glucopyranosiduronic acid;
3-{[2-({3-[(4-{6-[8-(1,3-benzothiazol-2-ylcarbamoyl)naphthalen-2-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl]amino}propyl beta-D-glucopyranosiduronic acid;
6-[4-(1,3-benzothiazol-2-ylcarbamoyl)-2-oxidoisoquinolin-6-yl]-3-[1-({3,5-dimethyl-7-[2-(methylamino)ethoxy]tricyclo[3.3.1.1^{3,7}]dec-1-yl}methyl)-5-methyl-1H-pyrazol-4-yl]pyridine-2-carboxylic acid;
6-{8-[(1,3-benzothiazol-2-yl)carbamoyl]-3,4-dihydroisoquinolin-2(1H)-yl}-3-{1-[(3,5-dimethyl-7-{2-[(2-sulfoethyl)amino]acetamido}tricyclo[3.3.1.1^{3,7}]decan-1-yl)methyl]-5-methyl-1H-pyrazol-4-yl}pyridine-2-carboxylic acid;
6-[8-(1,3-benzothiazol-2-ylcarbamoyl)-3,4-dihydroisoquinolin-2(1*H*)-yl]-3-(1-{[3,5-dimethyl-7-({2-[(2-sulfoethyl)amino]ethyl}sulfanyl)tricyclo[3.3.1.1^{3,7}]dec-1-yl]methyl}-5-methyl-1*H*-pyrazol-4-yl)pyridine-2-carboxylic acid; and
6-{8-[(1,3-benzothiazol-2-yl)carbamoyl]-3,4-dihydroisoquinolin-2(1*H*)-yl}-3-{1-[(3,5-dimethyl-7-{3-[(2-sulfoethyl)amino]propyl}tricyclo[3.3.1.1^{3,7}]decan-1-yl)methyl]-5-methyl-1*H-*pyrazol-4-yl}pyridine-2-carboxylic acid.

In one embodiment of any one of the aspects and embodiments herein, the linker is cleavable by a lysosomal enzyme. In a further embodiment, the lysosomal enzyme is Cathepsin B.

In one embodiment of any one of the aspects and embodiments herein, the linker comprises a segment according to structural formula (IVa), (IVb), (IVc), or (IVd): 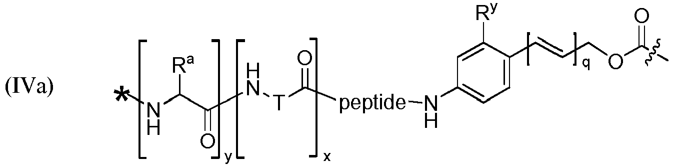 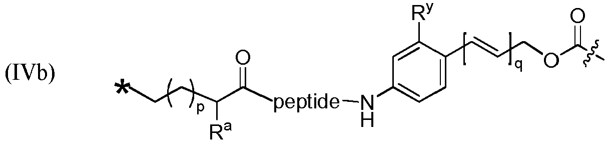 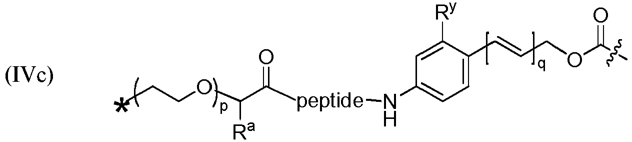 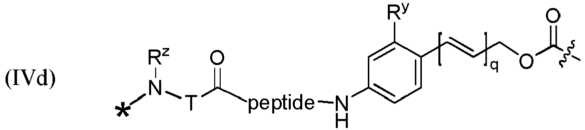 wherein:
peptide represents a peptide (illustrated N→C, wherein peptide includes the amino and carboxy "termini") a cleavable by a lysosomal enzyme;
T represents a polymer comprising one or more ethylene glycol units or an alkylene chain, or combinations thereof;
R^{a} is selected from hydrogen, C₁₋₆ alkyl, SO₃H and CH₂SO₃H;
R^{y} is hydrogen or C₁₋₄ alkyl-(O)ᵣ-(C₁₋₄ alkylene)ₛ-G¹ or C₁₋₄ alkyl-(N)-[(C₁₋₄ alkylene)-G¹]₂;
R^{z} is C₁₋₄ alkyl-(O)ᵣ-(C₁₋₄ alkylene)ₛ-G²;
G¹ is SO₃H, CO₂H, PEG 4-32, or sugar moiety;
G² is SO₃H, CO₂H, or PEG 4-32 moiety;
r is 0 or 1;
s is 0 or 1;
p is an integer ranging from 0 to 5;
q is 0 or 1;
x is 0 or 1;
y is 0 or 1;
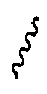 represents the point of attachment of the linker to the Bcl-xL inhibitor; and
* represents the point of attachment to the remainder of the linker.

In another embodiment, the peptide is selected from the group consisting of Val-Cit; Cit-Val; Ala-Ala; Ala-Cit; Cit-Ala; Asn-Cit; Cit-Asn; Cit-Cit; Val-Glu; Glu-Val; Ser-Cit; Cit-Ser; Lys-Cit; Cit-Lys; Asp-Cit; Cit-Asp; Ala-Val; Val-Ala; Phe-Lys; Lys-Phe; Val-Lys; Lys-Val; Ala-Lys; Lys-Ala; Phe-Cit; Cit-Phe; Leu-Cit; Cit-Leu; Ile-Cit; Cit-Ile; Phe-Arg; Arg-Phe; Cit-Trp; and Trp-Cit.

In another embodiment, the lysosomal enzyme is β-glucuronidase or β-galactosidase.

In one embodiment of any one of the aspects and embodiments herein, the linker comprises a segment according to structural formula (Va), (Vb), (Vc), (Vd), or (Ve): 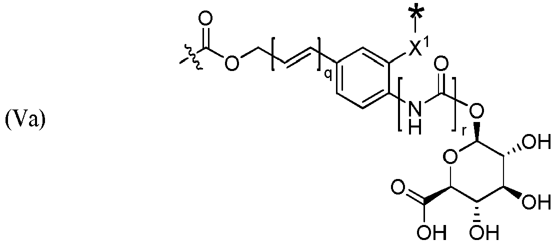 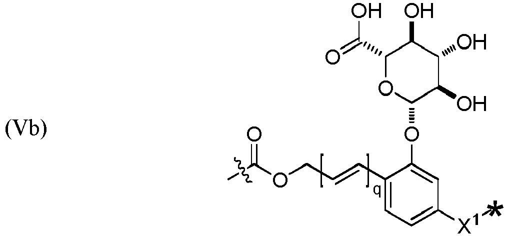 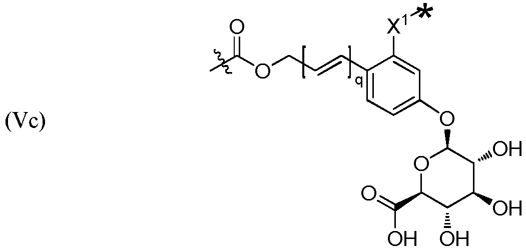 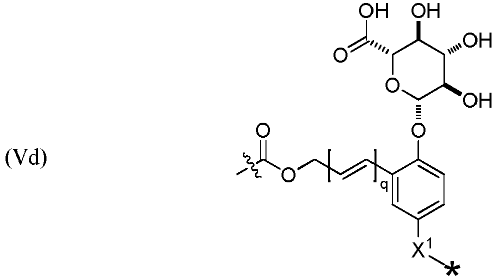 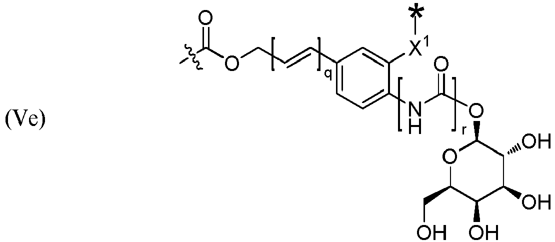 wherein:
q is 0 or 1;
r is 0 or 1;
X¹ is CH₂, O or NH;
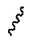 represents the point of attachment of the linker to the drug; and
^{∗} represents the point of attachment to the remainder of the linker.

In one embodiment of any one of the aspects and embodiments herein, the linker comprises a segment according to structural formulae (VIIIa), (VIIIb), or (VIIIc): 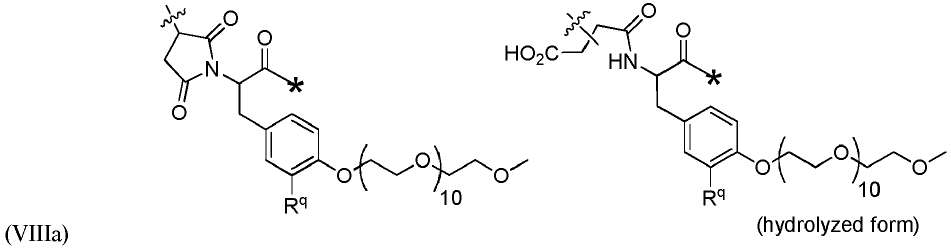 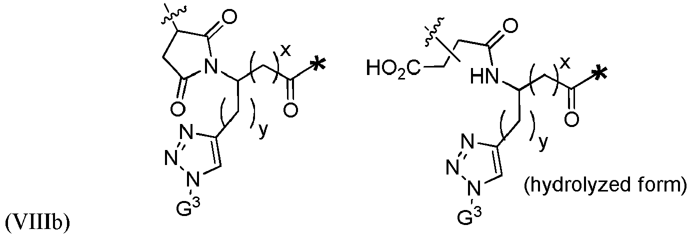 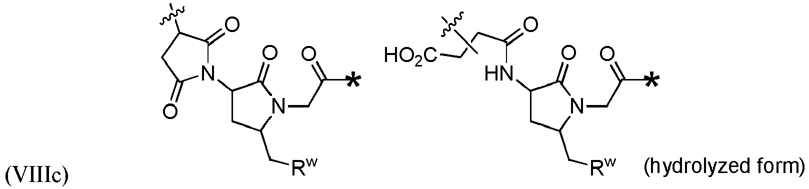 or a hydrolyzed derivative thereof, wherein:
R^{q} is H or -O-(CH₂CH₂O)₁₁-CH₃;
x is 0 or 1;
y is 0 or 1;
G³ is -CH₂CH₂CH₂SO₃H or -CH₂CH₂O-(CH₂CH₂O)₁₁-CH₃;
R^{w} is -O-CH₂CH₂SO₃H or -NH(CO)-CH₂CH₂O-(CH₂CH₂O)₁₂-CH₃;
^{∗} represents the point of attachment to the remainder of the linker; and
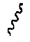 represents the point of attachment of the linker to the antibody.

In one embodiment of any one of the aspects and embodiments herein, the linker comprises a polyethylene glycol segment having from 1 to 6 ethylene glycol units.

In one embodiment of any one of the aspects and embodiments herein, m is 2, 3 or 4. In a further embodiment, linker L is selected from IVa or IVb.

In one embodiment of any one of the aspects and embodiments herein, the linker L is selected from the group consisting of IVa.1-IVa.8, IVb.1-IVb.19, IVc.1-IVc.7, IVd.1-IVd.4, Va.1-Va.12, Vb.1-Vb.10, Vc.1-Vc.11, Vd.1-Vd.6, Ve.1-Ve.2, VIa.1, VIc.1-V1c.2, VId.1-VId.4, VIIa.1-VIIa.4, VIIb.1-VIIb.8, VIIc.1-VIIc.6 in either the closed or open form.

In one embodiment of any one of the aspects and embodiments herein, the linker L is selected from the group consisting of IVb.2, IVc.5, IVc.6, IVc.7, IVd.4, Vb.9, VIIa.1, VIIa.3, VIIc.1, VIIc.4, and VIIc.5, wherein the maleimide of each linker has reacted with the antibody Ab, forming a covalent attachment as either a succinimide (closed form) or succinamide (open form).

In one embodiment of any one of the aspects and embodiments herein, the linker L is selected from the group consisting of IVb.2, IVc.5, IVc.6, IVd.4, VIIa.1, VIIa.3, VIIc.1, VIIc.4, VIIc.5, wherein the maleimide of each linker has reacted with the antibody Ab, forming a covalent attachment as either a succinimide (closed form) or succinamide (open form).

In one embodiment of any one of the aspects and embodiments herein, the linker L is selected from the group consisting of IVb.2, VIIa.3, IVc.6, and VIIc.1, wherein 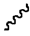 is the attachment point to drug D and @ is the attachment point to the LK, wherein when the linker is in the open form as shown below, @ can be either at the α-position or β-position of the carboxylic acid next to it: 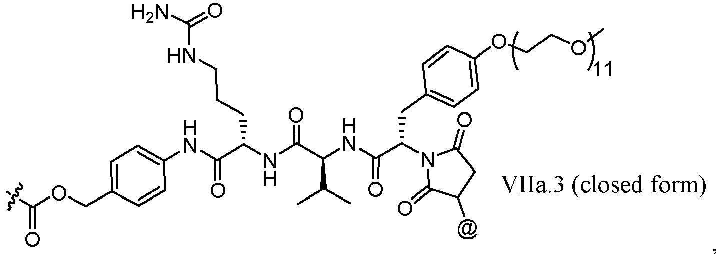 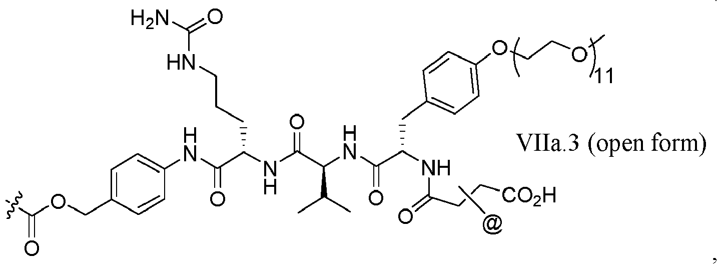 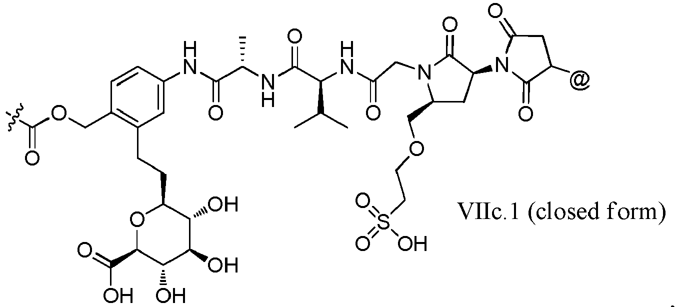 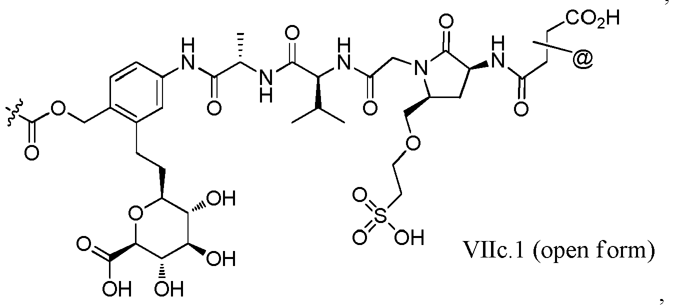 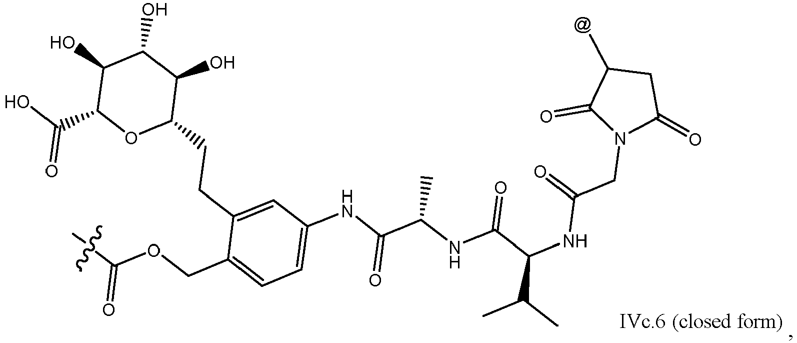 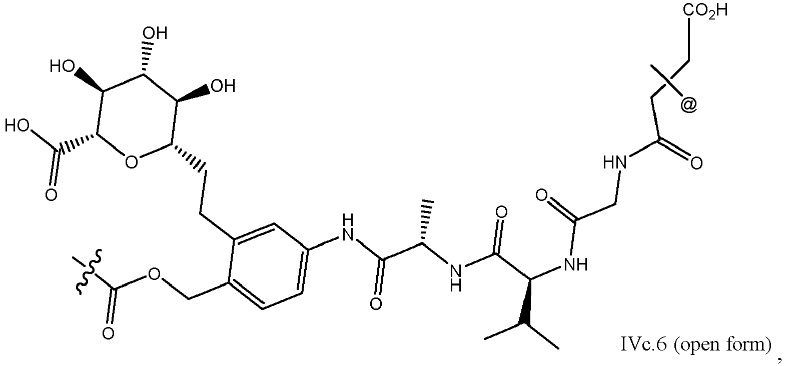 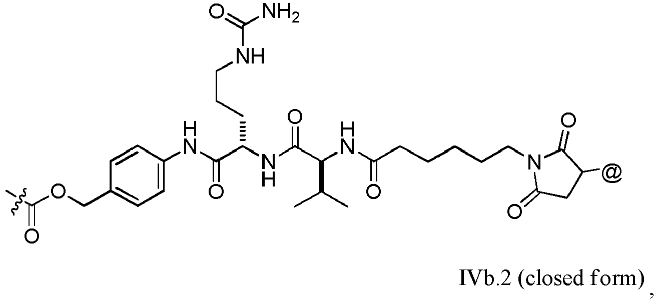 and

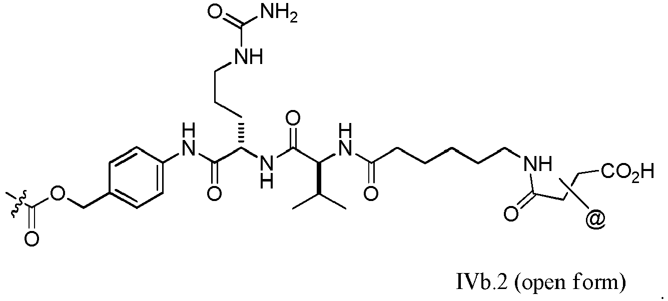

In one embodiment of any one of the aspects and embodiments herein, LK is a linkage formed with an amino group on the anti-hEGFR antibody Ab.

In another embodiment, LK is an amide or a thiourea.

In one embodiment of any one of the aspects and embodiments herein, LK is a linkage formed with a sulfhydryl group on the anti-hEGFR antibody Ab. In a further embodiment, LK is a thioether.

In one embodiment, the disclosure features an ADC of the aspects and embodiments described herein, in which:
LK is selected from the group consisting of amide, thiourea and thioether; and
m is an integer ranging from 1 to 8.

In one embodiment, the disclosure features an ADC of the aspects and embodiments described herein, in which:
D is the Bcl-xL inhibitor as defined in claim 35;
L is selected from the group consisting of linkers IVa.1-IVa.8, IVb.1-IVb.19, IVc.1-IVc.7, IVd.1-IVd.4, Va.1-Va.12, Vb.1-Vb.10, Vc.1-Vc.11, Vd.1-Vd.6, Ve.1-Ve.2, VIa.1, VIc.1-V1c.2, VId.1-VId.4, VIIa.1-VIIa.4, VIIb.1-VIIb.8, and VIIc.1-VIIc.6, wherein each linker has reacted with the antibody, Ab, forming a covalent attachment;
LK is thioether; and
m is an integer ranging from 1 to 8.

In one embodiment, the disclosure features an ADC of the aspects and embodiments described herein, in which:
D is the Bcl-xL inhibitor selected from the group consisting of the following compounds modified in that the hydrogen corresponding to the # position of structural formula (IIa), (IIb), (IIc), or (IId) is not present, forming a monoradical:
6-[8-(1,3-benzothiazol-2-ylcarbamoyl)-3,4-dihydroisoquinolin-2(1H)-yl]-3-{1-[(3,5-dimethyl-7-{2-[(2-sulfoethyl)amino]ethoxy}tricyclo[3.3.1.1^{3,7}]dec-1-yl)methyl]-5-methyl-1H-pyrazol-4-yl}pyridine-2-carboxylic acid;
6-[8-(1,3-benzothiazol-2-ylcarbamoyl)-3,4-dihydroisoquinolin-2(1H)-yl]-3-{1-[(3-{2-[(2-carboxyethyl)amino]ethoxy}-5,7-dimethyltricyclo[3.3.1.1^{3,7}]dec-1-yl)methyl]-5-methyl-1H-pyrazol-4-yl}pyridine-2-carboxylic acid;
6-[8-(1,3-benzothiazol-2-ylcarbamoyl)naphthalen-2-yl]-3-{1-[(3,5-dimethyl-7-{2-[(2-sulfoethyl)amino]ethoxy}tricyclo[3.3.1.1^{3,7}]dec-1-yl)methyl]-5-methyl-1H-pyrazol-4-yl}pyridine-2-carboxylic acid;
1-{[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl} oxy)ethyl]amino}-1,2-dideoxy-D-arabino-hexitol;
6-[8-(1,3-benzothiazol-2-ylcarbamoyl)-3,4-dihydroisoquinolin-2(1H)-yl]-3-(-(1-{[3-(2-{[3-hydroxy-2-(hydroxymethyl)propyl]amino}ethoxy)-5,7-dimethyltricyclo[3.3.1.1^{3,7}]dec-1-yl]methyl}-5-methyl-1H-pyrazol-4-yl)pyridine-2-carboxylic acid; and
6-[8-(1,3-benzothiazol-2-ylcarbamoyl)-3,4-dihydroisoquinolin-2(1*H*)-yl]-3-(1-{[3-(2-{[(3*S*)-3,4-dihydroxybutyl]amino}ethoxy)-5,7-dimethyltricyclo[3.3.1.1^{3,7}]dec-1-yl]methyl}-5-methyl-1*H-*pyrazol-4-yl)pyridine-2-carboxylic acid;
L is selected from the group consisting of linkers IVb.2, IVc.5, IVc.6, IVc.7, IVd.4, Vb.9, Vc.11, VIIa.1, VIIa.3, VIIc.1, VIIc.4, and VIIc.5 in either closed or open forms;
LK is thioether; and
m is an integer ranging from 2 to 4.

In one embodiment, the ADC is selected from the group consisting of AbA-CZ, AbA-TX, AbA-TV, AbA-YY, AbA-AAA, AbA-AAD, AbB-CZ, AbB-TX, AbB-TV, AbB-YY, AbB-AAD, AbG-CZ, AbG-TX, AbG-TV, AbG-YY, AbG-AAA, AbG-AAD, AbK-CZ, AbK-TX, AbK-TV, AbK-YY, AbK-AAA, AbK-AAD, wherein CZ, TX, TV, YY, AAA, and AAD are synthons disclosed in Table 5, and where in the synthons are either in open or closed form;.

In one embodiment, the ADCof the aspects and embodiments described herein, is selected from the group consisting of formulae i-vi: 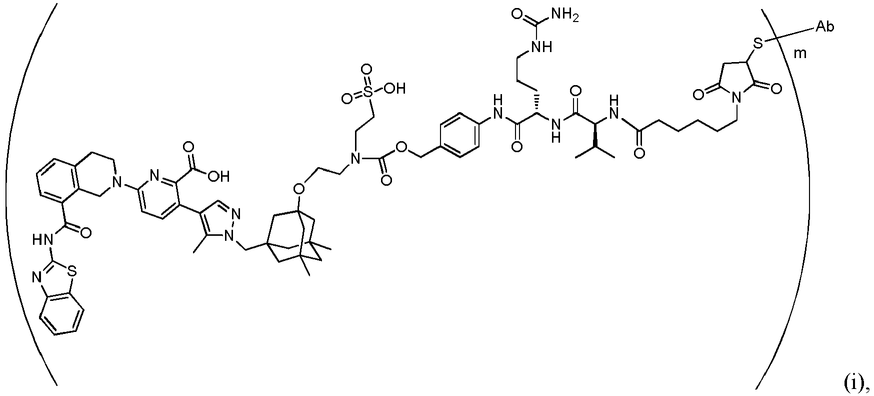 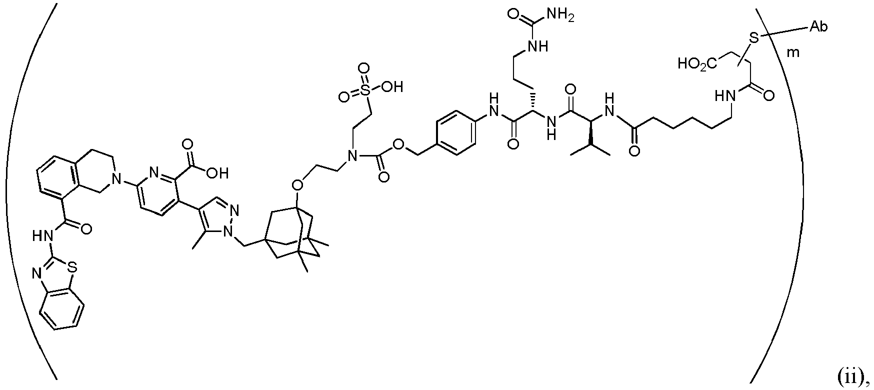 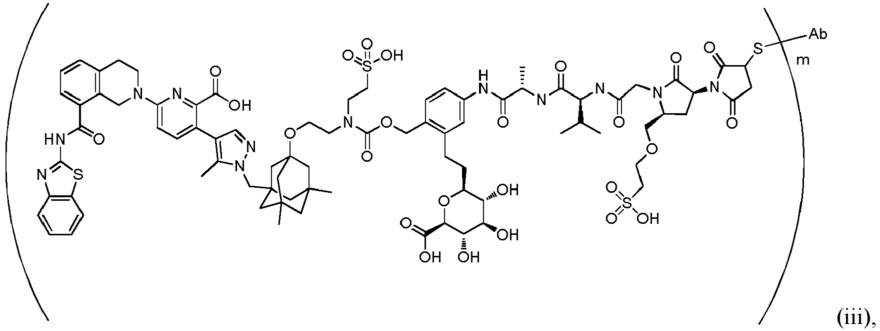 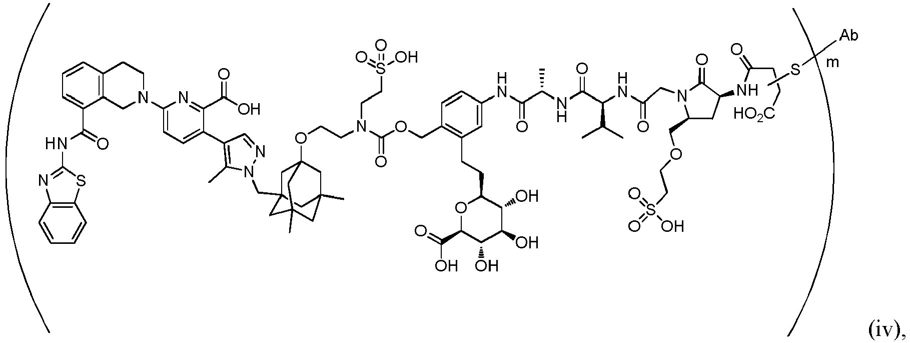 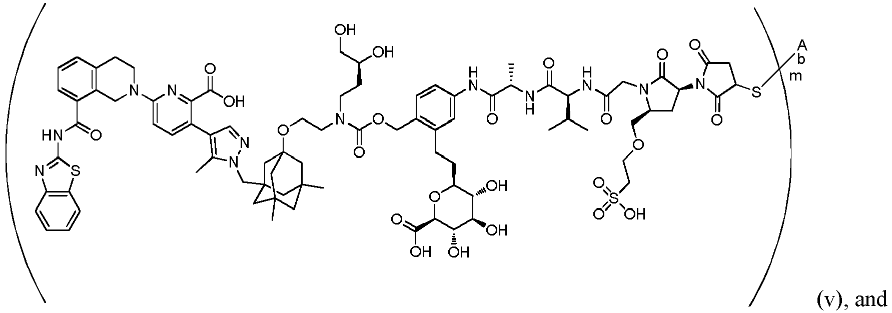 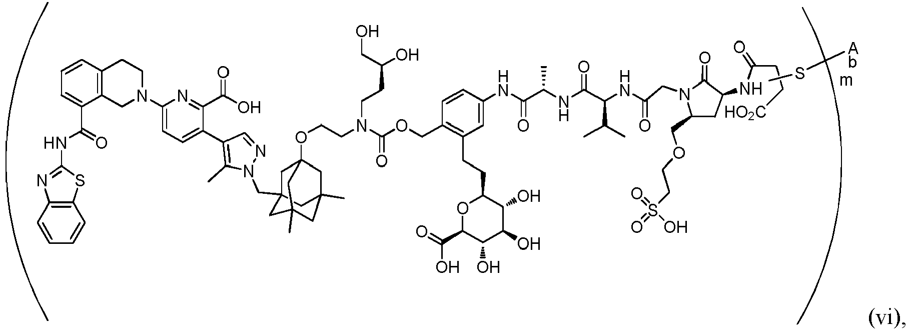 wherein m is an integer from 1 to 6; optionally 2 to 6. In a specific embodiment, m is 2. In a specific embodiment, Ab is the hEGFR antibody, wherein the hEGFR antibody comprises the heavy and light chain CDRs of AbA. In other embodiments, the hEGFR ADC comprises an antibody comprising a heavy chain CDR3 domain comprising the amino acid sequence set forth in SEQ ID NO: 12, a heavy chain CDR2 domain comprising the amino acid sequence set forth in SEQ ID NO: 11, and a heavy chain CDR1 domain comprising the amino acid sequence set forth in SEQ ID NO: 10; and a light chain CDR3 domain comprising the amino acid sequence set forth in SEQ ID NO: 8, a light chain CDR2 domain comprising the amino acid sequence set forth in SEQ ID NO: 7, and a light chain CDR1 domain comprising the amino acid sequence set forth in SEQ ID NO: 6. In yet another embodiment, the hEGFR ADC comprises an antibody comprising a heavy chain variable region comprising the amino acid sequence set forth in SEQ ID NO: 9, and a light chain variable region comprising the amino acid sequence set forth in SEQ ID NO: 5. In other embodiments, the hEGFR ADC comprises an antibody comprising a heavy chain constant region comprising the amino acid sequence set forth in SEQ ID NO: 41 and/or a light chain constant region comprising the amino acid sequence set forth in SEQ ID NO: 43. In a further embodiment, the hEGFR ADC comprises an antibody comprising a heavy chain comprising the amino acid sequence set forth in SEQ ID NO: 15, and a light chain comprising the amino acid sequence set forth in SEQ ID NO: 13. In a further embodiment, the hEGFR ADC comprises an antibody comprising a heavy chain comprising the amino acid sequence set forth in SEQ ID NO: 102, and a light chain comprising the amino acid sequence set forth in SEQ ID NO: 13. In another specific embodiment, Ab is the hEGFR antibody, wherein the hEGFR antibody comprises the heavy and light chain CDRs of AbG. In other embodiments, the hEGFR ADC comprises an antibody comprising a heavy chain CDR3 domain comprising the amino acid sequence set forth in SEQ ID NO: 18, a heavy chain CDR2 domain comprising the amino acid sequence set forth in SEQ ID NO: 17, and a heavy chain CDR1 domain comprising the amino acid sequence set forth in SEQ ID NO: 16; and a light chain CDR3 domain comprising the amino acid sequence set forth in SEQ ID NO: 25, a light chain CDR2 domain comprising the amino acid sequence set forth in SEQ ID NO: 24, and a light chain CDR1 domain comprising the amino acid sequence set forth in SEQ ID NO: 23. In yet another embodiment, the hEGFR ADC comprises an antibody comprising a heavy chain variable region comprising the amino acid sequence set forth in SEQ ID NO: 72, and a light chain variable region comprising the amino acid sequence set forth in SEQ ID NO: 73. In other embodiments, the hEGFR ADC comprises an antibody comprising a heavy chain constant region comprising the amino acid sequence set forth in SEQ ID NO: 41 and/or a light chain constant region comprising the amino acid sequence set forth in SEQ ID NO: 43. In a further embodiment, the hEGFR ADC comprises an antibody comprising a heavy chain comprising the amino acid sequence set forth in SEQ ID NO: 93, and a light chain comprising the amino acid sequence set forth in SEQ ID NO: 95. In a further embodiment, the hEGFR ADC comprises an antibody comprising a heavy chain comprising the amino acid sequence set forth in SEQ ID NO: 94, and a light chain comprising the amino acid sequence set forth in SEQ ID NO: 95.

In one embodiment of any one of the aspects and embodiments herein, the antibody binds to EGFR (1-525) (SEQ ID NO: 47) with a K_{d} of between about 1 x 10⁻⁶ M and about 1 x 10⁻¹⁰ M, as determined by surface plasmon resonance.

In one embodiment of any one of the aspects and embodiments herein, the antibody binds to EGFR (1-525) (SEQ ID NO: 47) with a K_{d} of between about 1 x 10⁻⁶ M and about 1 x 10⁻⁷ M, as determined by surface plasmon resonance.

In one embodiment of any one of the aspects and embodiments herein, the antibody binds to EGFRvIII (SEQ ID NO: 33) with a K_{d} of about 8.2 x 10⁻⁹ M or less, as determined by surface plasmon resonance.

In one embodiment of any one of the aspects and embodiments herein, the antibody binds to EGFRvIII (SEQ ID NO: 33) with a K_{d} of between about 8.2 x 10⁻⁹ M and about 6.3 x 10⁻¹⁰ M, as determined by surface plasmon resonance.

In one embodiment of any one of the aspects and embodiments herein, the antibody binds to EGFRvIII (SEQ ID NO: 33) with a K_{d} of between about 8.2 x 10⁻⁹ M and about 2.0 x 10⁻⁹ M, as determined by surface plasmon resonance.

In one embodiment of any one of the aspects and embodiments herein, the anti-hEGFR antibody comprises a heavy chain CDR3 domain comprising the amino acid sequence set forth in SEQ ID NO: 12, a heavy chain CDR2 domain comprising the amino acid sequence set forth in SEQ ID NO: 11, and a heavy chain CDR1 domain comprising the amino acid sequence set forth in SEQ ID NO: 10; a light chain CDR3 domain comprising the amino acid sequence set forth in SEQ ID NO: 8, a light chain CDR2 domain comprising the amino acid sequence set forth in SEQ ID NO: 7, and a light chain CDR1 domain comprising the amino acid sequence set forth in SEQ ID NO: 6;

In one embodiment of any one of the aspects and embodiments herein, the antibody comprises a heavy chain variable region comprising the amino acid sequence set forth in SEQ ID NO: 9, and a light chain variable region comprising the amino acid sequence set forth in SEQ ID NO: 5.

In one embodiment of any one of the aspects and embodiments herein, the antibody comprises a heavy chain comprising the amino acid sequence set forth in SEQ ID NO: 15, and a light chain comprising the amino acid sequence set forth in SEQ ID NO: 13.

In one embodiment of any one of the aspects and embodiments herein, the antibody comprises a light chain CDR3 domain comprising the amino acid sequence set forth in SEQ ID NO: 40, a light chain CDR2 domain comprising the amino acid sequence set forth in SEQ ID NO: 39, and a light chain CDR1 domain comprising the amino acid sequence set forth in SEQ ID NO: 38; and a heavy chain CDR3 domain comprising the amino acid sequence set forth in SEQ ID NO: 37, a heavy chain CDR2 domain comprising the amino acid sequence set forth in SEQ ID NO: 36, and a heavy chain CDR1 domain comprising the amino acid sequence set forth in SEQ ID NO: 35.

In one embodiment of any one of the aspects and embodiments herein, the antibody comprises a heavy chain variable region comprising an amino acid sequence selected from the group consisting of 50, 52, 54, 56, 58, 60, 62, 64, 66, 68, 70, 72, 74, 76, and 78; and a light chain variable region comprising an amino acid sequence selected from the group consisting of 51, 53, 55, 57, 59, 61, 63, 65, 67, 69, 71, 73, 75, 77, and 79.

In one embodiment of any one of the aspects and embodiments herein, the antibody comprises a heavy chain CDR set (CDR1, CDR2, and CDR3) selected from the group consisting of SEQ ID NOs: 10, 11, and 12; SEQ ID NOs: 16, 17, and 18; SEQ ID NOs: 10, 11, and 19; SEQ ID NOs: 20, 11, and 12; SEQ ID NOs: 21, 3, and 22; SEQ ID NOs: 16, 17, and 19; SEQ ID NOs: 2, 3, and 4; SEQ ID NOs: 10, 3, and 12; SEQ ID NOs: 80, 11, and 18; SEQ ID NOs: 80, 3, and 18; SEQ ID NOs: 20, 3, and 12; SEQ ID NOs: 80, 11, and 12; and SEQ ID NOs: 81, 11, and 22; and a light chain CDR set (CDR1, CDR2, and CDR3) selected from the group consisting of SEQ ID NOs: 6, 7, and 8; SEQ ID NOs: 23, 24, and 25; SEQ ID NOs: 26, 27, and 28; SEQ ID NOs: 29, 30, and 31; SEQ ID NOs: 6, 7, and 84; SEQ ID NOs: 82, 83, and 31; and SEQ ID NOs: 82, 27, and 85, wherein the antibody does not comprise both the heavy chain CDR set of SEQ ID NOs: 2, 3, and 4, and the light chain CDR set of SEQ ID NOs: 6, 7, and 8.

In one embodiment of any one of the aspects and embodiments herein, the antibody comprises a light chain CDR3 domain comprising the amino acid sequence set forth in SEQ ID NO: 8, a light chain CDR2 domain comprising the amino acid sequence set forth in SEQ ID NO: 7, and a light chain CDR1 domain comprising the amino acid sequence set forth in SEQ ID NO: 6; and a heavy chain CDR3 domain comprising the amino acid sequence set forth in SEQ ID NO: 19, a heavy chain CDR2 domain comprising the amino acid sequence set forth in SEQ ID NO: 17, and a heavy chain CDR1 domain comprising the amino acid sequence set forth in SEQ ID NO: 16.

In one embodiment of any one of the aspects and embodiments herein, the antibody comprises a light chain CDR3 domain comprising the amino acid sequence set forth in SEQ ID NO: 25, a light chain CDR2 domain comprising the amino acid sequence set forth in SEQ ID NO: 24, and a light chain CDR1 domain comprising the amino acid sequence set forth in SEQ ID NO: 23; and a heavy chain CDR3 domain comprising the amino acid sequence set forth in SEQ ID NO: 18, a heavy chain CDR2 domain comprising the amino acid sequence set forth in SEQ ID NO: 17, and a heavy chain CDR1 domain comprising the amino acid sequence set forth in SEQ ID NO: 16.

In one embodiment of any one of the aspects and embodiments herein, the antibody comprises a light chain CDR3 domain comprising the amino acid sequence set forth in SEQ ID NO: 28, a light chain CDR2 domain comprising the amino acid sequence set forth in SEQ ID NO: 27, and a light chain CDR1 domain comprising the amino acid sequence set forth in SEQ ID NO: 26; and a heavy chain CDR3 domain comprising the amino acid sequence set forth in SEQ ID NO: 19, a heavy chain CDR2 domain comprising the amino acid sequence set forth in SEQ ID NO: 11, and a heavy chain CDR1 domain comprising the amino acid sequence set forth in SEQ ID NO: 10.

In one embodiment of any one of the aspects and embodiments herein, the antibody comprises a heavy chain variable region comprising the amino acid sequence set forth in SEQ ID NO: 64, and a light chain variable region comprising the amino acid sequence set forth in SEQ ID NO: 65.

In one embodiment of any one of the aspects and embodiments herein, the antibody comprises a heavy chain variable region comprising the amino acid sequence set forth in SEQ ID NO: 72, and a light chain variable region comprising the amino acid sequence set forth in SEQ ID NO: 73.

In one embodiment of any one of the aspects and embodiments herein, the antibody comprises a heavy chain variable region comprising the amino acid sequence set forth in SEQ ID NO: 74, and a light chain variable region comprising the amino acid sequence set forth in SEQ ID NO: 75.

In another aspect, the disclosure features an anti-hEGFR ADC which is selected from the group consisting of Ab-CZ, Ab-TX, Ab-TV, Ab-YY, Ab-AAA, and Ab-AAD, wherein CZ, TX, TV, YY, AAA, and AAD are synthons disclosed in Table 5, and where in the synthons are either in open or closed form, and wherein the Ab is an IgG1 and comprises the variable light and heavy chain CDRs of AbA, AbB, AbG, or AbK.

In one embodiment, the Ab comprises a variable light chain region comprising an amino acid sequence as set forth in SEQ ID NO: 5, 65, 73, or 75, and a variable heavy chain region comprising an amino acid sequence as set forth in SEQ ID NO: 9, 64, 72, or 74.

In one embodiment, the antibody is an IgG, *e.g.,* an IgG1, having four polypeptide chains which are two heavy chains and two light chains.

In one embodiment, the antibody is a monoclonal antibody.

In another aspect, the disclosure features a pharmaceutical composition comprising an effective amount of an ADC according to any one of the aspects and embodiments herein, and a pharmaceutically acceptable carrier.

In another aspect, the disclosure features a pharmaceutical composition comprising an ADC mixture comprising a plurality of the ADC of any one of the aspects or embodiments herein, and a pharmaceutically acceptable carrier.

In one embodiment, the ADC mixture has an average drug to antibody ratio (DAR) of 2 to 4.

In one embodiment, the ADC mixture comprises ADCs each having a DAR of 2 to 8.

In another aspect, the disclosure features a use of the claimed ADC in a method for treating cancer, comprising administering a therapeutically effective amount of the ADC of any one of the aspects or embodiments herein, to a subject in need thereof.

In one embodiment, the cancer is selected from the group consisting of non small cell lung cancer, breast cancer, ovarian cancer, a glioblastoma, prostate cancer, pancreatic cancer, colon cancer, head and neck cancer, and kidney cancer.

In one embodiment, the cancer is a squamous cell carcinoma. In a further embodiment, the squamous cell carcinoma is squamous lung cancer or squamous head and neck cancer.

In one embodiment, the cancer is triple negative breast cancer.

In one embodiment, the cancer is non-small cell lung cancer. In a further embodiment, the ADC is administered with taxane.

In one embodiment, the cancer is non small cell lung cancer. In a further embodiment, the ADC is administered with venetoclax.

In one embodiment of any one of the aspects and embodiments herein, the cancer is characterized as having EGFR expression, or as being EGFRvIII positive.

In one embodiment of any one of the aspects and embodiments herein, the cancer is characterized as having EGFR overexpression or EGFR amplification.

In one embodiment of any one of the aspects and embodiments herein, the cancer is characterized as having an activating EGFR mutation. In a further embodiment, the EGFR mutation is selected from the group consisting of an exon 19 deletion mutation, a single-point substitution mutation L858R in exon 21, a T790M point mutation, and combinations thereof.

In another aspect, the disclosure features a method for inhibiting or decreasing solid tumor growth in a subject having a solid tumor, said method comprising administering an effective amount of the ADC of any one of the aspects or embodiments herein to the subject having the solid tumor, such that the solid tumor growth is inhibited or decreased.

In one embodiment, the solid tumor is selected from the group consisting of non-small cell lung carcinoma, breast cancer, ovarian cancer, and glioblastoma.

In one embodiment, the solid tumor is a squamous cell carcinoma.

In one embodiment of any one of the aspects and embodiments herein, the solid tumor is an EGFRvIII positive solid tumor, is a solid tumor characterized as having EGFR amplification, or is a solid tumor characterized as having EGFR overexpression.

In one embodiment of any one of the aspects and embodiments herein, the ADC is administered in combination with an additional agent or an additional therapy.

In a further embodiment, the additional agent is selected from the group consisting of an anti-PD1 antibody (e.g. pembrolizumab), an anti-PD-L1 antibody (atezolizumab), an anti-CTLA-4 antibody (e.g. ipilimumab), a MEK inhibitor (e.g. trametinib), an ERK inhibitor, a BRAF inhibitor (e.g. dabrafenib), osimertinib, erlotinib, gefitinib, sorafenib, a CDK9 inhibitor (e.g. dinaciclib), a MCL-1 inhibitor, temozolomide, a Bcl-2 inhibitor (e.g. venetoclax), a Bcl-xL inhibitor, ibrutinib, a mTOR inhibitor (e.g. everolimus), a PI3K inhibitor (e.g. buparlisib), duvelisib, idelalisib, an AKT inhibitor, a HER2 inhibitor (e.g. lapatinib), a taxane (e.g. docetaxel, paclitaxel, nab-paclitaxel), an ADC comprising an auristatin, an ADC comprising a PBD (e.g. rovalpituzumab tesirine), an ADC comprising a maytansinoid (e.g. TDM1), a TRAIL agonist, a proteasome inhibitor (e.g. bortezomib), and a nicotinamide phosphoribosyltransferase (NAMPT) inhibitor.

In another further embodiment, the additional therapy is radiation.

In another further embodiment, the additional agent is a chemotherapeutic agent.

In another aspect, the disclosure features a process for the preparation of an ADC according to structural formula (I): 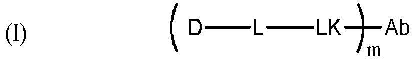 wherein:
D is the Bcl-xL inhibitor drug of formula (IIa), (IIb), (IIc), or (IId) as disclosed herein;
L is the linker as disclosed herein;
Ab is an hEGFR antibody, wherein the hEGFR antibody comprises the heavy and light chain CDRs of AbA; AbB; AbG; or AbK;
LK represents a covalent linkage linking linker L to antibody Ab; and
m is an integer ranging from 1 to 20;
the process comprising:
   treating an antibody in an aqueous solution with an effective amount of a disulfide reducing agent at 30-40 °C for at least 15 minutes, and then cooling the antibody solution to 20-27 °C;
   adding to the reduced antibody solution a solution of water/dimethyl sulfoxide comprising a synthon selected from the group of 2.1 to 2.176 (Table 5);
   adjusting the pH of the solution to a pH of 7.5 to 8.5;
   allowing the reaction to run for 48 to 80 hours to form the ADC;
   wherein the mass is shifted by 18 ± 2 amu for each hydrolysis of a succinimide to a succinamide as measured by electron spray mass spectrometry; and
   wherein the ADC is optionally purified by hydrophobic interaction chromatography.

In one embodiment, m is 2.

In another aspect, the disclosure features an ADC prepared by the process as described above.

### BRIEF DESCRIPTION OF THE DRAWINGS

**Figure 1** shows a schematic of EGFR and the regions bound by Ab1 and Ab2.
**Figure 2** provides the variable heavy (VH) and variable light (VL) chain region amino acid sequences of Ab1 (SEQ ID NOs: 1 and 5) and AbA (SEQ ID NOs: 9 and 5). CDR sequences within the VH and VL regions are boxed, and differences between the Ab1 VH sequence and the AbA VH sequence are shaded.
**Figure 3** describes the full length light and heavy chains for Ab1 (SEQ ID NOs: 13 and 14) and AbA (SEQ ID NOs: 13 and 15). Differences between the Ab1 sequence and the AbA sequence in the heavy chain are highlighted.
**Figure 4** shows a representation of antibody reduction, modification with a maleimide derivative to give a thiosuccinimide intermediate, and subesequent hydrolysis of thiosuccinimide moiety.
**Figure 5** shows mass spectrometry (MS) characterization of light chain and heavy chain of an exemplary antibody 1) prior to conjugation, 2) after conjugation to a maleimide derivative to give a thiosuccinimide intermediate and 3) post pH8-mediated hydrolysis of the thiosuccinimide ring.

### DETAILED DESCRIPTION OF THE DISCLOSURE

Numerous Bcl-xL inhibitors have been developed for treatment of diseases *(e.g.,* cancer) that involve dysregulated apoptotic pathways. However, Bcl-xL inhibitors can act on cells other than the target cells (*e.g.,* cancer cells). For instance, pre-clinical studies have shown that pharmacological inactivation of Bcl-xL reduces platelet half-life and causes thrombocytopenia (see Mason et al., 2007, Cell 128:1173-1186).

Given the importance of Bcl-xL in regulating apoptosis, there remains a need in the art for agents that inhibit Bcl-xL activity, either selectively or non-selectively, as an approach towards the treatment of diseases in which apoptosis is dysregulated via expression or over-expression of anti-apoptotic Bcl-2 family proteins, such as Bcl-xL. Accordingly, new Bcl-xL inhibitors with reduced dose-limiting toxicity are needed.

One potential means of delivering a drug to a cell which has not been explored for Bcl-xL inhibitors is delivery through the use of antibody drug conjugates (ADCs). Antibody drug conjugates (ADC) represent a new class of therapeutics comprising an antibody conjugated to a cytotoxic drug via a chemical linker. The therapeutic concept of ADCs is to combine binding capabilities of an antibody with a drug, where the antibody is used to deliver the drug to a tumor cell by means of binding to a target surface antigen.

Accordingly, the development of new ADCs that can selectively deliver Bcl-xL to target cancer cells, *e.g.*, EGFRvIII expressing cells, would be a significant discovery.

Various aspects of the disclosure relate to new anti-EGFR antibody drug conjugates (ADCs; also called immunoconjugates), and pharmaceutical compositions thereof. In particular, the present disclosure concerns new anti-EGFR ADCs comprising Bcl-xL inhibitors, synthons useful for synthesizing the ADCs, compositions comprising the ADCs, methods of making the ADCs, and various methods of using the ADCs.

As will be appreciated by skilled artisans, the ADCs disclosed herein are "modular" in nature. Throughout the instant disclosure, various specific embodiments of the various "modules" comprising the ADCs, as well as the synthons useful for synthesizing the ADCs, are described. As specific nonlimiting examples, specific embodiments of antibodies, linkers, and Bcl-xL inhibitors that may comprise the ADCs and synthons are described. It is intended that all of the specific embodiments described may be combined with each other as though each specific combination were explicitly described individually.

It will also be appreciated by skilled artisans that the various Bcl-xL inhibitors, ADCs and/or ADC synthons described herein may be in the form of salts, and in certain embodiments, particularly pharmaceutically acceptable salts. The compounds of the present disclosure that possess a sufficiently acidic, a sufficiently basic, or both functional groups, can react with any of a number of inorganic bases, and inorganic and organic acids, to form a salt. Alternatively, compounds that are inherently charged, such as those with a quaternary nitrogen, can form a salt with an appropriate counterion, e.g., a halide such as a bromide, chloride, or fluoride.

Acids commonly employed to form acid addition salts are inorganic acids such as hydrochloric acid, hydrobromic acid, hydroiodic acid, sulfuric acid, phosphoric acid, and the like, and organic acids such as p-toluenesulfonic acid, methanesulfonic acid, oxalic acid, p-bromophenylsulfonic acid, carbonic acid, succinic acid, citric acid, etc. Base addition salts include those derived from inorganic bases, such as ammonium and alkali or alkaline earth metal hydroxides, carbonates, bicarbonates, and the like.

In the disclosure below, if both structural diagrams and nomenclature are included and if the nomenclature conflicts with the structural diagram, the structural diagram controls.

### 1. Definitions

In order that the disclosure may be more readily understood, certain terms are first defined. In addition, it should be noted that whenever a value or range of values of a parameter are recited, it is intended that values and ranges intermediate to the recited values are also intended to be part of this disclosure. Further, unless otherwise defined herein, scientific and technical terms used in connection with the present disclosure shall have the meanings that are commonly understood by those of ordinary skill in the art.

Various chemical substituents are defined below. In some instances, the number of carbon atoms in a substituent (*e.g.*, alkyl, alkanyl, alkenyl, alkynyl, cycloalkyl, heterocyclyl, heteroaryl, and aryl) is indicated by the prefix "Cₓ-C_{y}" or "C_{x-y}" wherein x is the minimum and y is the maximum number of carbon atoms. Thus, for example, "C₁-C₆ alkyl" refers to an alkyl containing from 1 to 6 carbon atoms. Illustrating further, "C₃-C₈ cycloalkyl" means a saturated hydrocarbyl ring containing from 3 to 8 carbon ring atoms. If a substituent is described as being "substituted," a hydrogen atom on a carbon or nitrogen is replaced with a non-hydrogen group. For example, a substituted alkyl substituent is an alkyl substituent in which at least one hydrogen atom on the alkyl is replaced with a non-hydrogen group. To illustrate, monofluoroalkyl is alkyl substituted with a fluoro radical, and difluoroalkyl is alkyl substituted with two fluoro radicals. It should be recognized that if there is more than one substitution on a substituent, each substitution may be identical or different (unless otherwise stated). If a substituent is described as being "optionally substituted", the substituent may be either (1) not substituted or (2) substituted. Possible substituents include, but are not limited to, C₁-C₆ alkyl, C₂-C₆ alkenyl, C₂-C₆ alkynyl, aryl, cycloalkyl, heterocyclyl, heteroaryl, halogen, C₁-C₆ haloalkyl, oxo, -CN, NO₂, -OR^{xa}, -OC(O)R^{xz}, -OC(O)N(R^{xa})₂, -SR^{xa}, -S(O)₂R^{xa}, -S(O)₂N(R^{xa})₂, -C(O)R^{xa}, -C(O)OR^{xa}, -C(O)N(R^{xa})₂, -C(O)N(R^{xa})S(O)₂R^{xz}, -N(R^{xa})₂, -N(R^{xa})C(O)R^{xz}, -N(R^{xa})S(O)₂R^{xz}, -N(R^{xa})C(O)O(R^{xz}), -N(R^{xa})C(O)N(R^{xa})₂, -N(R^{xa})S(O)₂N(R^{xa})₂, -(C₁-C₆ alkylenyl)-CN, -(C₁-C₆ alkylenyl)-OR^{xa}, -(C₁-C₆ alkylenyl)-OC(O)R^{xz}, -(C₁-C₆ alkylenyl)-OC(O)N(R^{xa})₂, -(C₁-C₆ alkylenyl)-SR^{xa}, -(C₁-C₆ alkylenyl)-S(O)₂R^{xa}, -(C₁-C₆ alkylenyl)-S(O)₂N(R^{xa})₂, -(C₁-C₆ alkylenyl)-C(O)R^{xa}, -(C₁-C₆ alkylenyl)-C(O)OR^{xa}, -(C₁-C₆ alkylenyl)-C(O)N(R^{xa})₂, -(C₁-C₆ alkylenyl)-C(O)N(R^{xa})S(O)₂R^{xz}, -(C₁-C₆ alkylenyl)-N(R^{xa})₂, -(C₁-C₆ alkylenyl)-N(R^{xa})C(O)R^{xz}, -(C₁-C₆ alkylenyl)-N(R^{xa})S(O)₂R^{xz}, -(C₁-C₆ alkylenyl)-N(R^{xa})C(O)O(R^{xz}), -(C₁-C₆ alkylenyl)-N(R^{xa})C(O)N(R^{xa})₂, or -(C₁-C₆ alkylenyl)-N(R^{xa})S(O)₂N(R^{xa})₂; wherein R^{xa}, at each occurrence, is independently hydrogen, aryl, cycloalkyl, heterocyclyl, heteroaryl, C₁-C₆ alkyl, or C₁-C₆ haloalkyl; and R^{xz}, at each occurrence, is independently aryl, cycloalkyl, heterocyclyl, heteroaryl, C₁-C₆ alkyl or C₁-C₆ haloalkyl.

Various ADCs, synthons and Bcl-xL inhibitors comprising the ADCs and/or synthons are described in some embodiments herein by reference to structural formulae including substituents. It is to be understood that the various groups comprising substituents may be combined as valence and stability permit. Combinations of substituents and variables envisioned by this disclosure are only those that result in the formation of stable compounds. As used herein, the term "stable" refers to compounds that possess stability sufficient to allow manufacture and that maintain the integrity of the compound for a sufficient period of time to be useful for the purpose detailed herein.

As used herein, the following terms are intended to have the following meanings:
The term "alkoxy" refers to a group of the formula -OR^{xa}, where R^{xa} is an alkyl group. Representative alkoxy groups include methoxy, ethoxy, propoxy, tert-butoxy and the like.
The term "alkoxyalkyl" refers to an alkyl group substituted with an alkoxy group and may be represented by the general formula -R^{b}OR^{xa} where R^{b} is an alkylene group and R^{xa} is an alkyl group.
The term "alkyl" by itself or as part of another substituent refers to a saturated or unsaturated branched, straight-chain or cyclic monovalent hydrocarbon radical that is derived by the removal of one hydrogen atom from a single carbon atom of a parent alkane, alkene or alkyne. Typical alkyl groups include, but are not limited to, methyl; ethyls such as ethanyl, ethenyl, ethynyl; propyls such as propan-1-yl, propan-2-yl, cyclopropan-1-yl, prop-1-en-1-yl, prop-1-en-2-yl, prop-2-en-1-yl, cycloprop-1-en-1-yl; cycloprop-2-en-1-yl, prop-1-yn-1-yl , prop-2-yn-1-yl, *etc.;* butyls such as butan-1-yl, butan-2-yl, 2-methyl-propan-1-yl, 2-methyl-propan-2-yl, cyclobutan-1-yl, but-1-en-1-yl, but-1-en-2-yl, 2-methyl-prop-1-en-1-yl, but-2-en-1-yl , but-2-en-2-yl, buta-1,3-dien-1-yl, buta-1,3-dien-2-yl, cyclobut-1-en-1-yl, cyclobut-1-en-3-yl, cyclobuta-1,3-dien-1-yl, but-1-yn-1-yl, but-1-yn-3-yl, but-3-yn-1-yl, *etc.;* and the like. Where specific levels of saturation are intended, the nomenclature "alkanyl," "alkenyl" and/or "alkynyl" are used, as defined below. The term "lower alkyl" refers to alkyl groups with 1 to 6 carbons.
The term "alkanyl" by itself or as part of another substituent refers to a saturated branched, straight-chain or cyclic alkyl derived by the removal of one hydrogen atom from a single carbon atom of a parent alkane. Typical alkanyl groups include, but are not limited to, methyl; ethanyl; propanyls such as propan-1-yl, propan-2-yl (isopropyl), cyclopropan-1-yl, *etc.;* butanyls such as butan-1-yl, butan-2-yl (*sec*-butyl), 2-methyl-propan-1-yl (isobutyl), 2-methyl-propan-2-yl (*t*-butyl), cyclobutan-1-yl, *etc.;* and the like.
The term "alkenyl" by itself or as part of another substituent refers to an unsaturated branched, straight-chain or cyclic alkyl having at least one carbon-carbon double bond derived by the removal of one hydrogen atom from a single carbon atom of a parent alkene. Typical alkenyl groups include, but are not limited to, ethenyl; propenyls such as prop-1-en-1-yl , prop-1-en-2-yl, prop-2-en-1-yl, prop-2-en-2-yl, cycloprop-1-en-1-yl; cycloprop-2-en-1-yl ; butenyls such as but-1-en-1-yl, but-1-en-2-yl, 2-methyl-prop-1-en-1-yl, but-2-en-1-yl, but-2-en-2-yl, buta-1,3-dien-1-yl, buta-1,3-dien-2-yl, cyclobut-1-en-1-yl, cyclobut-1-en-3-yl, cyclobuta-1,3-dien-1-yl, etc.; and the like.
The term "alkynyl" by itself or as part of another substituent refers to an unsaturated branched, straight-chain or cyclic alkyl having at least one carbon-carbon triple bond derived by the removal of one hydrogen atom from a single carbon atom of a parent alkyne. Typical alkynyl groups include, but are not limited to, ethynyl; propynyls such as prop-1-yn-1-yl , prop-2-yn-1-yl, etc.; butynyls such as but-1-yn-1-yl, but-1-yn-3-yl, but-3-yn-1-yl , etc.; and the like.
The term "alkylamine" refers to a group of the formula -NHR^{xa} and "dialkylamine" refers to a group of the formula -NR^{xa}R^{xa}, where each R^{xa} is, independently of the others, an alkyl group.
The term "alkylene" refers to an alkane, alkene or alkyne group having two terminal monovalent radical centers derived by the removal of one hydrogen atom from each of the two terminal carbon atoms. Typical alkylene groups include, but are not limited to, methylene; and saturated or unsaturated ethylene; propylene; butylene; and the like. The term "lower alkylene" refers to alkylene groups with 1 to 6 carbons.
The term "heteroalkylene" refers to a divalent alkylene having one or more -CH₂- groups replaced with a thio, oxy, or -NR^{x3}- where R^{x3} is selected from hydrogen, lower alkyl and lower heteroalkyl. The heteroalkylene can be linear, branched, cyclic, bicyclic, or a combination thereof and can include up to 10 carbon atoms and up to 4 heteroatoms. The term "lower heteroalkylene" refers to alkylene groups with 1 to 4 carbon atoms and 1 to 3 heteroatoms.
The term "aryl" means an aromatic carbocyclyl containing from 6 to 14 carbon ring atoms. An aryl may be monocyclic or polycyclic (*i.e.,* may contain more than one ring). In the case of polycyclic aromatic rings, only one ring the polycyclic system is required to be aromatic while the remaining ring(s) may be saturated, partially saturated or unsaturated. Examples of aryls include phenyl, naphthalenyl, indenyl, indanyl, and tetrahydronaphthyl.
The term "arylene" refers to an aryl group having two monovalent radical centers derived by the removal of one hydrogen atom from each of the two ring carbons. An exemplary arylene group is a phenylene.

An alkyl group may be substituted by a "carbonyl" which means that two hydrogen atoms from a single alkanylene carbon atom are removed and replaced with a double bond to an oxygen atom.

The prefix "halo" indicates that the substituent which includes the prefix is substituted with one or more independently selected halogen radicals. For example, haloalkyl means an alkyl substituent in which at least one hydrogen radical is replaced with a halogen radical. Typical halogen radicals include chloro, fluoro, bromo and iodo. Examples of haloalkyls include chloromethyl, 1-bromoethyl, fluoromethyl, difluoromethyl, trifluoromethyl, and 1,1,1-trifluoroethyl. It should be recognized that if a substituent is substituted by more than one halogen radical, those halogen radicals may be identical or different (unless otherwise stated).

The term "haloalkoxy" refers to a group of the formula -OR^{c}, where R^{c} is a haloalkyl.

The terms "heteroalkyl," "heteroalkanyl," "heteroalkenyl," "heteroalkynyl," and "heteroalkylene" refer to alkyl, alkanyl, alkenyl, alkynyl, and alkylene groups, respectively, in which one or more of the carbon atoms, *e.g.*, 1, 2 or 3 carbon atoms, are each independently replaced with the same or different heteroatoms or heteroatomic groups. Typical heteroatoms and/or heteroatomic groups which can replace the carbon atoms include, but are not limited to, -O-, -S-, -S-O-, -NR^{c}-, -PH, -S(O)-, -S(O)₂-, -S(O)NR^{c}-, -S(O)₂NR^{c}-, and the like, including combinations thereof, where each R^{c} is independently hydrogen or C₁-C₆ alkyl. The term "lower heteroalkyl" refers to between 1 and 4 carbon atoms and between 1 and 3 heteroatoms.

The terms "cycloalkyl" and "heterocyclyl" refer to cyclic versions of "alkyl" and "heteroalkyl" groups, respectively. For heterocyclyl groups, a heteroatom can occupy the position that is attached to the remainder of the molecule. A cycloalkyl or heterocyclyl ring may be a single-ring (monocyclic) or have two or more rings (bicyclic or polycyclic).

Monocyclic cycloalkyl and heterocyclyl groups will typically contains from 3 to 7 ring atoms, more typically from 3 to 6 ring atoms, and even more typically 5 to 6 ring atoms. Examples of cycloalkyl groups include, but are not limited to, cyclopropyl; cyclobutyls such as cyclobutanyl and cyclobutenyl; cyclopentyls such as cyclopentanyl and cyclopentenyl; cyclohexyls such as cyclohexanyl and cyclohexenyl; and the like. Examples of monocyclic heterocyclyls include, but are not limited to, oxetane, furanyl, dihydrofuranyl, tetrahydrofuranyl, tetrahydropyranyl, thiophenyl (thiofuranyl), dihydrothiophenyl, tetrahydrothiophenyl, pyrrolyl, pyrrolinyl, pyrrolidinyl, imidazolyl, imidazolinyl, imidazolidinyl, pyrazolyl, pyrazolinyl, pyrazolidinyl, triazolyl, tetrazolyl, oxazolyl, oxazolidinyl, isoxazolidinyl, isoxazolyl, thiazolyl, isothiazolyl, thiazolinyl, isothiazolinyl, thiazolidinyl, isothiazolidinyl, thiodiazolyl, oxadiazolyl (including 1,2,3-oxadiazolyl, 1,2,4-oxadiazolyl, 1,2,5-oxadiazolyl (furazanyl), or 1,3,4-oxadiazolyl), oxatriazolyl (including 1,2,3,4-oxatriazolyl or 1,2,3,5-oxatriazolyl), dioxazolyl (including 1,2,3-dioxazolyl, 1,2,4-dioxazolyl, 1,3,2-dioxazolyl, or 1,3,4-dioxazolyl), 1,4-dioxanyl, dioxothiomorpholinyl, oxathiazolyl, oxathiolyl, oxathiolanyl, pyranyl, dihydropyranyl, thiopyranyl, tetrahydrothiopyranyl, pyridinyl (azinyl), piperidinyl, diazinyl (including pyridazinyl (1,2-diazinyl), pyrimidinyl (1,3-diazinyl), or pyrazinyl (1,4-diazinyl)), piperazinyl, triazinyl (including 1,3,5-triazinyl, 1,2,4-triazinyl, and 1,2,3-triazinyl)), oxazinyl (including 1,2-oxazinyl, 1,3-oxazinyl, or 1,4-oxazinyl)), oxathiazinyl (including 1,2,3-oxathiazinyl, 1,2,4-oxathiazinyl, 1,2,5-oxathiazinyl, or 1,2,6-oxathiazinyl)), oxadiazinyl (including 1,2,3-oxadiazinyl, 1,2,4-oxadiazinyl, 1,4,2-oxadiazinyl, or 1,3,5-oxadiazinyl)), morpholinyl, azepinyl, oxepinyl, thiepinyl, diazepinyl, pyridonyl (including pyrid-2(1H)-onyl and pyrid-4(1H)-onyl), furan-2(5H)-onyl, pyrimidonyl (including pyramid-2(1H)-onyl and pyramid-4(3H)-onyl), oxazol-2(3H)-onyl, 1H-imidazol-2(3H)-onyl, pyridazin-3(2H)-onyl, and pyrazin-2(1H)-onyl.

Polycyclic cycloalkyl and heterocyclyl groups contain more than one ring, and bicyclic cycloalkyl and heterocyclyl groups contain two rings. The rings may be in a bridged, fused or spiro orientation. Polycyclic cycloalkyl and heterocyclyl groups may include combinations of bridged, fused and/or spiro rings. In a spirocyclic cycloalkyl or heterocyclyl, one atom is common to two different rings. An example of a spirocycloalkyl is spiro[4.5]decane and an example of a spiroheterocyclyls is a spiropyrazoline.

In a bridged cycloalkyl or heterocyclyl, the rings share at least two common non-adjacent atoms. Examples of bridged cycloalkyls include, but are not limited to, adamantyl and norbornanyl rings. Examples of bridged heterocyclyls include, but are not limited to, 2-oxatricyclo[3.3.1.1^{3,7}]decanyl.

In a fused-ring cycloalkyl or heterocyclyl, two or more rings are fused together, such that two rings share one common bond. Examples of fused-ring cycloalkyls include decalin, naphthylene, tetralin, and anthracene. Examples of fused-ring heterocyclyls containing two or three rings include imidazopyrazinyl (including imidazo[1,2-a]pyrazinyl), imidazopyridinyl (including imidazo[1,2-a]pyridinyl), imidazopyridazinyl (including imidazo[1,2-b]pyridazinyl), thiazolopyridinyl (including thiazolo[5,4-c]pyridinyl, thiazolo[5,4-b]pyridinyl, thiazolo[4,5-b]pyridinyl, and thiazolo[4,5-c]pyridinyl), indolizinyl, pyranopyrrolyl, 4H-quinolizinyl, purinyl, naphthyridinyl, pyridopyridinyl (including pyrido[3,4-b]-pyridinyl, pyrido[3,2-b]-pyridinyl, or pyrido[4,3-b]-pyridinyl), and pteridinyl. Other examples of fused-ring heterocyclyls include benzo-fused heterocyclyls, such as dihydrochromenyl, tetrahydroisoquinolinyl, indolyl, isoindolyl (isobenzazolyl, pseudoisoindolyl), indoleninyl (pseudoindolyl), isoindazolyl (benzpyrazolyl), benzazinyl (including quinolinyl (1-benzazinyl) or isoquinolinyl (2-benzazinyl)), phthalazinyl, quinoxalinyl, quinazolinyl, benzodiazinyl (including cinnolinyl (1,2-benzodiazinyl) or quinazolinyl (1,3-benzodiazinyl)), benzopyranyl (including chromanyl or isochromanyl), benzoxazinyl (including 1,3,2-benzoxazinyl, 1,4,2-benzoxazinyl, 2,3,1-benzoxazinyl, or 3,1,4-benzoxazinyl), benzo[d]thiazolyl, and benzisoxazinyl (including 1,2-benzisoxazinyl or 1,4-benzisoxazinyl).

The term "cycloalkylene" refers to a cycoalkyl group having two monovalent radical centers derived by the removal of one hydrogen atom from each of two ring carbons. Exemplary cycloalkylene groups include: 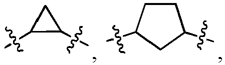 and

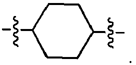

The term "heteroaryl" refers to an aromatic heterocyclyl containing from 5 to 14 ring atoms. A heteroaryl may be a single ring or 2 or 3 fused rings. Examples of heteroaryls include 6-membered rings such as pyridyl, pyrazyl, pyrimidinyl, pyridazinyl, and 1,3,5-, 1,2,4- or 1,2,3-triazinyl; 5-membered ring substituents such as triazolyl, pyrrolyl, imidazyl, furanyl, thiophenyl, pyrazolyl, oxazolyl, isoxazolyl, thiazolyl, 1,2,3-, 1,2,4-, 1,2,5-, or 1,3,4-oxadiazolyl and isothiazolyl; 6/5-membered fused ring substituents such as imidazopyrazinyl (including imidazo[1,2-a]pyrazinyl)imidazopyridinyl (including imidazo[1,2-a]pyridinyl), imidazopyridazinyl (including imidazo[1,2-b]pyridazinyl), thiazolopyridinyl (including thiazolo[5,4-c]pyridinyl, thiazolo[5,4-b]pyridinyl, thiazolo[4,5-b]pyridinyl, and thiazolo[4,5-c]pyridinyl), benzo[d]thiazolyl, benzothiofuranyl, benzisoxazolyl, benzoxazolyl, purinyl, and anthranilyl; and 6/6-membered fused rings such as benzopyranyl, quinolinyl, isoquinolinyl, cinnolinyl, quinazolinyl, and benzoxazinyl. Heteroaryls may also be heterocycles having aromatic (4N+2 pi electron) resonance contributors such as pyridonyl (including pyrid-2(1H)-onyl and pyrid-4(1H)-onyl), pyrimidonyl (including pyramid-2(1H)-onyl and pyramid-4(3H)-onyl), pyridazin-3(2H)-onyl and pyrazin-2(1H)-onyl.

The term "sulfonate" as used herein means a salt or ester of a sulfonic acid.

The term "methyl sulfonate" as used herein means a methyl ester of a sulfonic acid group.

The term "carboxylate" as used herein means a salt or ester of a carboxylic acid.

The term "polyol", as used herein, means a group containing more than two hydroxyl groups independently or as a portion of a monomer unit. Polyols include, but are not limited to, reduced C₂-C₆ carbohydrates, ethylene glycol, and glycerin.

The term "sugar" when used in context of "G¹" includes O-glycoside, *N*-glycoside, *S-*glycoside and *C*-glycoside (C-glycoslyl) carbohydrate derivatives of the monosaccharide and disaccharide classes and may originate from naturally-occurring sources or may be synthetic in origin. For example "sugar" when used in context of "G¹"includes derivatives such as but not limited to those derived from glucuronic acid, galacturonic acid, galactose, and glucose among others. Suitable sugar substitutions include but are not limited to hydroxyl, amine, carboxylic acid, sulfonic acid, phosphonic acid, esters, and ethers.

The term "NHS ester" means the N-hydroxysuccinimide ester derivative of a carboxylic acid.

The term "amine" includes primary, secondary and tertiary aliphatic amines, including cyclic versions.

The term salt when used in context of "or salt thereof" include salts commonly used to form alkali metal salts and to form addition salts of free acids or free bases. In general, these salts typically may be prepared by conventional means by reacting, for example, the appropriate acid or base with a compound of the disclosure

Where a salt is intended to be administered to a patient (as opposed to, for example, being in use in an *in vitro* context), the salt preferably is pharmaceutically acceptable and/or physiologically compatible. The term "pharmaceutically acceptable" is used adjectivally in this patent application to mean that the modified noun is appropriate for use as a pharmaceutical product or as a part of a pharmaceutical product. The term "pharmaceutically acceptable salt" includes salts commonly used to form alkali metal salts and to form addition salts of free acids or free bases. In general, these salts typically may be prepared by conventional means by reacting, for example, the appropriate acid or base with a compound of the disclosure.

The term "anti-Epidermal Growth Factor Receptor (EGFR) antibody" as used herein, refers to an antibody that specifically binds to EGFR. An antibody "which binds" an antigen of interest, *i.e.,* EGFR, is one capable of binding that antigen with sufficient affinity such that the antibody is useful in targeting a cell expressing the antigen. In a preferred embodiment, the antibody specifically binds to human EGFR (hEGFR). Examples of anti-EGFR antibodies are disclosed below. Unless otherwise indicated, the term "anti-EGFR antibody" is meant to refer to an antibody which binds to wild type EGFR or any variant of EGFR, such as EGFRvIII.

The amino acid sequence of wild type human EGFR is provided below as SEQ ID NO: 32, where the signal peptide (amino acid residues 1-24) is underlined, and the amino acid residues of the extracellular domain (ECD, amino acid residues 25-645) are highlighted in bold. A truncated wild type ECD of the EGFR (also referred to herein as EGFR(1-525)) corresponds to SEQ ID NO: 47 and is equivalent to amino acids 1-525 of SEQ ID NO: 32. The mature form of wild type EGFR corresponds to the protein without the signal peptide, *i.e.,* amino acid residues 25 to 1210 of SEQ ID NO: 32.

The amino acid sequence of the ECD of human EGFR is provided below as SEQ ID NO: 34, and includes the signal sequence (underlined).

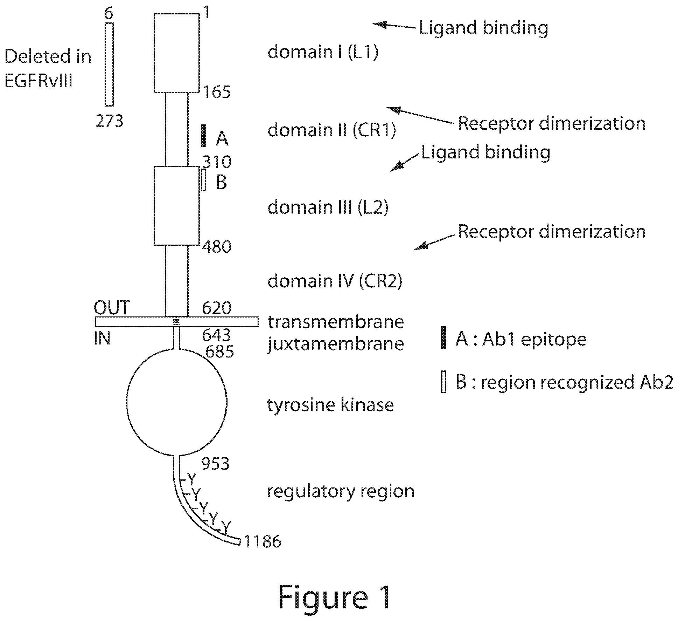

The overall structure of EGFR is described in Figure 1. The ECD of EGFR has four domains (Cochran et al. (2004) J. Immunol. Methods, 287, 147-158). Domains I and III have been suggested to contribute to the formation of high affinity binding sites for ligands. Domains II and IV are cysteine rich, laminin-like regions that stabilize protein folding and contain a possible EGFR dimerization interface.

EGFR variants may result from gene rearrangement accompanied by EGFR gene amplification.

EGFRvIII is the most commonly occurring variant of the EGFR in human cancers (Kuan et al. Endocr Relat Cancer. 8(2):83-96 (2001)). During the process of gene amplification, a 267 amino acid deletion occurs in the extracellular domain of EGFR with a glycine residue inserted at the fusion junction. Thus, EGFRvIII lacks amino acids 6-273 of the extracellular domain of wild type EGFR and includes a glycine residue insertion at the junction. The EGFRvIII variant of EGFR contains a deletion of 267 amino acid residues in the extracellular domain where a glycine is inserted at the deletion junction. The EGFRvIII amino acid sequence is shown below as SEQ ID NO: 33 (the ECD is highlighted in bold and corresponds to SEQ ID NO: 46 the signal sequence is underlined).

EGFRvIII contributes to tumor progression through constitutive signaling in a ligand independent manner. EGFRvIII is not known to be expressed in normal tissues (Wikstrand et al. Cancer Research 55(14): 3140-3148 (1995); Olapade-Olaopa et al. Br J Cancer. 82(1): 186-94 (2000)), but shows significant expression in tumor cells, including breast cancers, gliomas, NSCL cancers, ovarian cancers, and prostate cancers (Wikstrand et al. Cancer Research 55(14): 3140-3148 (1995); Ge et al. Int J Cancer. 98(3):357-61 (2002); Wikstrand et al. Cancer Research 55(14): 3140-3148 (1995); Moscatello et al. Cancer Res. 55(23):5536-9 (1995); Garcia de Palazzo et al. Cancer Res. 53(14):3217-20 (1993); Moscatello et al. Cancer Res. 55(23):5536-9 (1995); and Olapade-Olaopa et *al.* 2(1): 186-94 (2000)).

"Biological activity of EGFR " as used herein, refers to all inherent biological properties of the EGFR, including, but not limited to, binding to epidermal growth factor (EGF), binding to tumor growth factor α (TGFα), homodimerization, activation of JAK2 kinase activity, activation of MAPK kinase activity, and activation of transmembrane receptor protein tyrosine kinase activity.

The term "gene amplification", as used herein, refers to a cellular process characterized by the production of multiple copies of any particular piece of DNA. For example, a tumor cell may amplify, or copy, chromosomal segments as a result of cell signals and sometimes environmental events. The process of gene amplification leads to the production of additional copies of the gene. In one embodiment, the gene is EGFR, *i.e.,* "EGFR amplification." In one embodiment, the compositions and methods disclosed herein are used to treat a subject having EGFR amplified cancer.

The terms "specific binding" or "specifically binding", as used herein, in reference to the interaction of an antibody or an ADC with a second chemical species, mean that the interaction is dependent upon the presence of a particular structure (*e.g.*, an antigenic determinant or epitope) on the chemical species; for example, an antibody recognizes and binds to a specific protein structure rather than to proteins generally. If an antibody or ADC is specific for epitope "A", the presence of a molecule containing epitope A (or free, unlabeled A), in a reaction containing labeled "A" and the antibody, will reduce the amount of labeled A bound to the antibody or ADC.

The phrase "specifically binds to hEGFR" or "specific binding to hEGFR", as used herein, refers to the ability of an anti-EGFR antibody or ADC to bind to hEGFR with an Kd of at least about 1x10⁻⁶ M, 1×10⁻⁷ M, 1×10⁻⁸ M, 1x10⁻⁹ M, 1×10⁻¹⁰ M, 1×10⁻¹¹ M, 1×10⁻¹² M, or more, and/or bind to an antigen with an affinity that is at least two-fold greater than its affinity for a nonspecific antigen. It shall be understood, however, that the antibody or ADC may be capable of specifically binding to two or more antigens which are related in sequence. For example, in one embodiment, an antibody can specifically bind to both human and a non-human (e.g., mouse or non-human primate) orthologs of EGFR. In one embodiment, the antigen is EGFR(1-525).

The term "antibody" refers to an immunoglobulin molecule that specifically binds to an antigen and comprises a heavy (H) chain(s) and a light (L chain(s). Each heavy chain is comprised of a heavy chain variable region (abbreviated herein as HCVR or VH) and a heavy chain constant region. The heavy chain constant region is comprised of three domains, CH1, CH2 and CH3. Each light chain is comprised of a light chain variable region (abbreviated herein as LCVR or VL) and a light chain constant region. The light chain constant region is comprised of one domain, CL. The VH and VL regions can be further subdivided into regions of hypervariability, termed complementarity determining regions (CDR), interspersed with regions that are more conserved, termed framework regions (FR). Each VH and VL is composed of three CDRs and four FRs, arranged from amino-terminus to carboxy-terminus in the following order: FR1, CDR1, FR2, CDR2, FR3, CDR3, FR4. An antibody can be of any type (e.g., IgG, IgE, IgM, IgD, IgA and IgY) and class (e.g., IgG1, IgG2, IgG 3, IgG4, IgA1 and IgA2) or subclass. While the term "antibody" is not intended to include antigen binding portions of an antibody (defined below), it is intended, in certain embodiments, to include a small number of amino acid deletions from the carboxy end of the heavy chain(s). In one embodiment, an antibody comprises a heavy chain having 1-5 amino acid deletions the carboxy end of the heavy chain. In a one embodiment, an antibody is a monoclonal antibody which is an IgG, having four polypeptide chains, two heavy (H) chains, and two light (L chains) that can bind to hEGFR. In one embodiment, an antibody is a monoclonal IgG antibody comprising a lambda or a kappa light chain.

The term "antigen binding portion" of an antibody (or simply "antibody portion"), as used herein, refers to one or more fragments of an antibody that retain the ability to specifically bind to an antigen (*e.g.*, hEGFR). It has been shown that the antigen binding function of an antibody can be performed by fragments of a full-length antibody. Such antibody embodiments may also be bispecific, dual specific, or multi-specific formats; specifically binding to two or more different antigens. Examples of binding fragments encompassed within the term "antigen binding portion" of an antibody include (i) a Fab fragment, a monovalent fragment consisting of the VL, VH, CL and CH1 domains; (ii) a F(ab')₂ fragment, a bivalent fragment comprising two Fab fragments linked by a disulfide bridge at the hinge region; (iii) a Fd fragment consisting of the VH and CH1 domains; (iv) a Fv fragment consisting of the VL and VH domains of a single arm of an antibody, (v) a dAb fragment (Ward et al., (1989) Nature 341:544-546, Winter et al., PCT publication WO 90/05144 A1 which comprises a single variable domain; and (vi) an isolated complementarity determining region (CDR). Furthermore, although the two domains of the Fv fragment, VL and VH, are coded for by separate genes, they can be joined, using recombinant methods, by a synthetic linker that enables them to be made as a single protein chain in which the VL and VH regions pair to form monovalent molecules (known as single chain Fv (scFv); see *e.g.,* Bird et al. (1988) Science 242:423-426; and Huston et al. (1988) Proc. Natl. Acad. Sci. USA 85:5879-5883). Such single chain antibodies are also intended to be encompassed within the term "antigen binding portion" of an antibody. In certain embodiments of the disclosure, scFv molecules may be incorporated into a fusion protein. Other forms of single chain antibodies, such as diabodies are also encompassed. Diabodies are bivalent, bispecific antibodies in which VH and VL domains are expressed on a single polypeptide chain, but using a linker that is too short to allow for pairing between the two domains on the same chain, thereby forcing the domains to pair with complementary domains of another chain and creating two antigen binding sites (see *e.g.,* Holliger, P., et al. (1993) Proc. Natl. Acad. Sci. USA 90:6444-6448; Poljak, R.J., et al. (1994) Structure 2:1121-1123). Such antibody binding portions are known in the art (Kontermann and Dubel eds., Antibody Engineering (2001) Springer-Verlag. New York. 790 pp. (ISBN 3-540-41354-5).

An IgG is a class of antibody comprising two heavy chains and two light chains arranged in a Y-shape. Exemplary human IgG heavy chain and light chain constant domain amino acid sequences are known in the art and represented below in Table 1.

**Table 1: Sequence of human IgG heavy chain constant domain and light chain constant domain**

| Protein | Sequence Identifier | Sequence |
|---|---|---|
| Ig gamma-1 constant region | SEQ ID NO:41 | |
| Ig gamma-1 constant region mutant | SEQ ID NO:42 | |
| Ig Kappa constant region | SEQ ID NO:43 | |
| Ig Lambda constant region | SEQ ID NO:44 | |

An "isolated antibody", as used herein, is intended to refer to an antibody that is substantially free of other antibodies having different antigenic specificities *(e.g.,* an isolated antibody that specifically binds EGFR is substantially free of antibodies that specifically bind antigens other than EGFR). An isolated antibody that specifically binds EGFR may, however, have cross-reactivity to other antigens, such as EGFR molecules from other species. Moreover, an isolated antibody may be substantially free of other cellular material and/or chemicals.

The term "humanized antibody" refers to antibodies which comprise heavy and light chain variable region sequences from a nonhuman species (e.g., a mouse) but in which at least a portion of the VH and/or VL sequence has been altered to be more "human-like", i.e., more similar to human germline variable sequences. In particular, the term "humanized antibody" is an antibody or a variant, derivative, analog or fragment thereof which immunospecifically binds to an antigen of interest and which comprises a framework (FR) region having substantially the amino acid sequence of a human antibody and a complementary determining region (CDR) having substantially the amino acid sequence of a non-human antibody. As used herein, the term "substantially" in the context of a CDR refers to a CDR having an amino acid sequence at least 80%, preferably at least 85%, at least 90%, at least 95%, at least 98% or at least 99% identical to the amino acid sequence of a non-human antibody CDR. A humanized antibody comprises substantially all of at least one, and typically two, variable domains (Fab, Fab', F(ab')₂, FabC, Fv) in which all or substantially all of the CDR regions correspond to those of a non-human immunoglobulin (i.e., donor antibody) and all or substantially all of the framework regions are those of a human immunoglobulin consensus sequence. Preferably, a humanized antibody also comprises at least a portion of an immunoglobulin constant region (Fc), typically that of a human immunoglobulin. In some embodiments, a humanized antibody contains both the light chain as well as at least the variable domain of a heavy chain. The antibody also may include the CH1, hinge, CH2, CH3, and CH4 regions of the heavy chain. In some embodiments, a humanized antibody only contains a humanized light chain. In other embodiments, a humanized antibody only contains a humanized heavy chain. In specific embodiments, a humanized antibody only contains a humanized variable domain of a light chain and/or humanized heavy chain.

The humanized antibody can be selected from any class of immunoglobulins, including IgM, IgG, IgD, IgA and IgE, and any isotype, including without limitation IgG1, IgG2, IgG3 and IgG4. The humanized antibody may comprise sequences from more than one class or isotype, and particular constant domains may be selected to optimize desired effector functions using techniques well-known in the art.

The terms "Kabat numbering," "Kabat definitions," and "Kabat labeling" are used interchangeably herein. These terms, which are recognized in the art, refer to a system of numbering amino acid residues which are more variable *(i.e.,* hypervariable) than other amino acid residues in the heavy and light chain variable regions of an antibody, or an antigen binding portion thereof (Kabat et al. (1971) Ann. NY Acad, Sci. 190:382-391 and, Kabat, E.A., et al. (1991) Sequences of Proteins of Immunological Interest, Fifth Edition, U.S. Department of Health and Human Services, NIH Publication No. 91-3242). For the heavy chain variable region, the hypervariable region ranges from amino acid positions 31 to 35 for CDR1, amino acid positions 50 to 65 for CDR2, and amino acid positions 95 to 102 for CDR3. For the light chain variable region, the hypervariable region ranges from amino acid positions 24 to 34 for CDR1, amino acid positions 50 to 56 for CDR2, and amino acid positions 89 to 97 for CDR3.

As used herein, the term "CDR" refers to the complementarity determining region within antibody variable sequences. There are three CDRs in each of the variable regions of the heavy chain (HC) and the light chain (LC), which are designated CDR1, CDR2 and CDR3 (or specifically HC CDR1, HC CDR2, HC CDR3, LC CDR1, LC CDR2, and LC CDR3), for each of the variable regions. The term "CDR set" as used herein refers to a group of three CDRs that occur in a single variable region capable of binding the antigen. The exact boundaries of these CDRs have been defined differently according to different systems. The system described by Kabat (Kabat et al., Sequences of Proteins of Immunological Interest (National Institutes of Health, Bethesda, Md. (1987) and (1991)) not only provides an unambiguous residue numbering system applicable to any variable region of an antibody, but also provides precise residue boundaries defining the three CDRs. These CDRs may be referred to as Kabat CDRs. Chothia and coworkers (Chothia &Lesk, J. Mol. Biol. 196:901-917 (1987) and Chothia et al., Nature 342:877-883 (1989)) found that certain sub- portions within Kabat CDRs adopt nearly identical peptide backbone conformations, despite having great diversity at the level of amino acid sequence. These sub-portions were designated as L1, L2 and L3 or H1, H2 and H3 where the "L" and the "H" designates the light chain and the heavy chains regions, respectively. These regions may be referred to as Chothia CDRs, which have boundaries that overlap with Kabat CDRs. Other boundaries defining CDRs overlapping with the Kabat CDRs have been described by Padlan (FASEB J. 9:133-139 (1995)) and MacCallum (J Mol Biol 262(5):732-45 (1996)). Still other CDR boundary definitions may not strictly follow one of the above systems, but will nonetheless overlap with the Kabat CDRs, although they may be shortened or lengthened in light of prediction or experimental findings that particular residues or groups of residues or even entire CDRs do not significantly impact antigen binding. The methods used herein may utilize CDRs defined according to any of these systems, although preferred embodiments use Kabat or Chothia defined CDRs.

As used herein, the term "framework" or "framework sequence" refers to the remaining sequences of a variable region minus the CDRs. Because the exact definition of a CDR sequence can be determined by different systems, the meaning of a framework sequence is subject to correspondingly different interpretations. The six CDRs (CDR-L1, CDR-L2, and CDR-L3 of light chain and CDR-H1, CDR-H2, and CDR-H3 of heavy chain) also divide the framework regions on the light chain and the heavy chain into four sub-regions (FR1, FR2, FR3 and FR4) on each chain, in which CDR1 is positioned between FR1 and FR2, CDR2 between FR2 and FR3, and CDR3 between FR3 and FR4. Without specifying the particular sub-regions as FR1, FR2, FR3 or FR4, a framework region, as referred by others, represents the combined FR's within the variable region of a single, naturally occurring immunoglobulin chain. As used herein, a FR represents one of the four sub-regions, and FRs represents two or more of the four sub- regions constituting a framework region.

The framework and CDR regions of a humanized antibody need not correspond precisely to the parental sequences, *e.g.*, the donor antibody CDR or the consensus framework may be mutagenized by substitution, insertion and/or deletion of at least one amino acid residue so that the CDR or framework residue at that site does not correspond to either the donor antibody or the consensus framework. In a preferred embodiment, such mutations, however, will not be extensive. Usually, at least 80%, preferably at least 85%, more preferably at least 90%, and most preferably at least 95% of the humanized antibody residues will correspond to those of the parental FR and CDR sequences. As used herein, the term "consensus framework" refers to the framework region in the consensus immunoglobulin sequence. As used herein, the term "consensus immunoglobulin sequence" refers to the sequence formed from the most frequently occurring amino acids (or nucleotides) in a family of related immunoglobulin sequences (See *e.g*., Winnaker, From Genes to Clones (Verlagsgesellschaft, Weinheim, Germany 1987). In a family of immunoglobulins, each position in the consensus sequence is occupied by the amino acid occurring most frequently at that position in the family. If two amino acids occur equally frequently, either can be included in the consensus sequence.

"Percent (%) amino acid sequence identity" with respect to a peptide or polypeptide sequence is defined as the percentage of amino acid residues in a candidate sequence that are identical with the amino acid residues in the specific peptide or polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for measuring alignment, including any algorithms needed to achieve maximal alignment over the full length of the sequences being compared. In one embodiment, the disclosure includes an amino acid sequence having at least 80%, at least 85%, at least 90%, at least 95%, at least 96%, at least 97%, at least 98%, or at least 99% identity to an amino acid sequence set forth in any one of SEQ ID NOs: 1 to 31, 35-40, or 50 to 85.

The term "multivalent antibody" is used herein to denote an antibody comprising two or more antigen binding sites. In certain embodiments, the multivalent antibody may be engineered to have the three or more antigen binding sites, and is generally not a naturally occurring antibody.

The term "multispecific antibody" refers to an antibody capable of binding two or more unrelated antigens. In one embodiment, the multispecific antibody is a bispecific antibody that is capable of binding to two unrelated antigens, *e.g.,* a bispecific antibody, or antigen-binding portion thereof, that binds EGFR (*e.g.,* EGFRvIII) and CD3.

The term "activity" includes activities such as the binding specificity/affinity of an antibody or ADC for an antigen, for example, an anti-hEGFR antibody that binds to an hEGFR antigen and/or the neutralizing potency of an antibody, for example, an anti-hEGFR antibody whose binding to hEGFR inhibits the biological activity of hEGFR, *e.g.,* inhibition of phosphorylation of EGFR in an EGFR expressing cell line, *e.g.,* the human lung carcinoma cell line H292, or inhibition of proliferation of EGFR expressing cell lines, *e.g.,* human H292 lung carcinoma cells, human H1703 lung carcinoma cells, or human EBC1 lung carcinoma cells.

The term "non small-cell lung carcinoma (NSCLC) xenograft assay," as used herein, refers to an *in vivo* assay used to determine whether an anti-EGFR antibody or ADC, can inhibit tumor growth (*e.g.,* further growth) and/or decrease tumor growth resulting from the transplantation of NSCLC cells into an immunodeficient mouse. An NSCLC xenograft assay includes transplantation of NSCLC cells into an immunodeficient mouse such that a tumor grows to a desired size, *e.g.,* 200-250 mm³, whereupon the antibody or ADC is administered to the mouse to determine whether the antibody or ADC can inhibit and/or decrease tumor growth. In certain embodiments, the activity of the antibody or ADC is determined according to the percent tumor growth inhibition (%TGI) relative to a control antibody, *e.g.,* a human IgG antibody (or collection thereof) which does not specifically bind tumor cells, *e.g.,* is directed to an antigen not associated with cancer or is obtained from a source which is noncancerous (e.g., normal human serum). In such embodiments, the antibody (or ADC) and the control antibody are administered to the mouse at the same dose, with the same frequency, and via the same route. In one embodiment, the mouse used in the NSCLC xenograft assay is a severe combined immunodeficiency (SCID) mouse and/or an athymic CD-1 nude mouse. Examples of NSCLC cells that may be used in the NSCLC xenograft assay include, but are not limited to, H292 cells (*e.g.,* NCIH292 [H292] (ATCC CRL1848).

The term "epitope" refers to a region of an antigen that is bound by an antibody or ADC. In certain embodiments, epitope determinants include chemically active surface groupings of molecules such as amino acids, sugar side chains, phosphoryl, or sulfonyl, and, in certain embodiments, may have specific three dimensional structural characteristics, and/or specific charge characteristics. In certain embodiments, an antibody is said to specifically bind an antigen when it preferentially recognizes its target antigen in a complex mixture of proteins and/or macromolecules. In one embodiment, the antibodies of the disclosure bind to an epitope defined by the amino acid sequence CGADSYEMEEDGVRKC (SEQ ID NO: 45) (which corresponds to amino acid residues 287-302 of the mature form of hEGFR).

The term "surface plasmon resonance", as used herein, refers to an optical phenomenon that allows for the analysis of real-time biospecific interactions by detection of alterations in protein concentrations within a biosensor matrix, for example using the BIAcore system (Pharmacia Biosensor AB, Uppsala, Sweden and Piscataway, NJ). For further descriptions, see Jönsson, U., et al. (1993) Ann. Biol. Clin. 51:19-26; Jönsson, U., et al. (1991) Biotechniques 11:620-627; Johnsson, B., et al. (1995) J. Mol. Recognit. 8:125-131; and Johnnson, B., et al. (1991) Anal. Biochem. 198:268-277. In one embodiment, surface plasmon resonance is determined according to the methods described in Example 2.

The term " kₒₙ" or "kₐ", as used herein, is intended to refer to the on rate constant for association of an antibody to the antigen to form the antibody/antigen complex.

The term "k_{off}" or "k_{d}", as used herein, is intended to refer to the off rate constant for dissociation of an antibody from the antibody/antigen complex.

The term "K_{D}", as used herein, is intended to refer to the equilibrium dissociation constant of a particular antibody-antigen interaction (*e.g.,* AbA antibody and EGFR). K_{D} is calculated by kₐ / k_{d}.

The term "competitive binding", as used herein, refers to a situation in which a first antibody competes with a second antibody, for a binding site on a third molecule, *e.g.,* an antigen. In one embodiment, competitive binding between two antibodies is determined using FACS analysis.

The term "competitive binding assay" is an assay used to determine whether two or more antibodies bind to the same epitope. In one embodiment, a competitive binding assay is a competition fluorescent activated cell sorting (FACS) assay which is used to determine whether two or more antibodies bind to the same epitope by determining whether the fluorescent signal of a labeled antibody is reduced due to the introduction of a non-labeled antibody, where competition for the same epitope will lower the level of fluorescence.

The term "antibody-drug-conjugate" or "ADC" refers to a binding protein, such as an antibody or antigen binding fragment thereof, chemically linked to one or more chemical drug(s) (also referred to herein as agent(s), warhead(s), or payload(s)) that may optionally be therapeutic or cytotoxic agents. In a preferred embodiment, an ADC includes an antibody, a cytotoxic or therapeutic drug, and a linker that enables attachment or conjugation of the drug to the antibody. An ADC typically has anywhere from 1 to 8 drugs conjugated to the antibody, including drug loaded species of 2, 4, 6, or 8. In a preferred embodiment, the ADC of the disclosure comprises an anti-EGFR antibody conjugated via a linker to a Bcl-xL inhibitor.

The terms "anti-Epidermal Growth Factor antibody drug conjugate," "anti-EGFR antibody drug conjugate," or "anti-EGFR ADC", used interchangeably herein, refer to an ADC comprising an antibody that specifically binds to EGFR, whereby the antibody is conjugated to one or more chemical agent(s). In one embodiment, an anti-EGFR ADC comprises antibody AbA conjugated to a Bcl-xL inhibitor. In one embodiment, an anti-EGFR ADC comprises antibody AbB conjugated to a Bcl-xL inhibitor. In one embodiment, an anti-EGFR ADC comprises antibody AbK conjugated to a Bcl-xL inhibitor. In one embodiment, an anti-EGFR ADC comprises antibody AbG conjugated to a Bcl-xL inhibitor.

The term "drug-to-antibody ratio" or "DAR" refers to the number of drugs, *e.g.,* Bcl-xL inhibitor, attached to the antibody of the ADC. The DAR of an ADC can range from 1 to 8, although higher loads, *e.g.,* 20, are also possible depending on the number of linkage site on an antibody. The term DAR may be used in reference to the number of drugs loaded onto an individual antibody, or, alternatively, may be used in reference to the average or mean DAR of a group of ADCs.

The term "undesired ADC species", as used herein, refers to any drug loaded species which is to be separated from an ADC species having a different drug load. In one embodiment, the term undesired ADC species may refer to drug loaded species of 6 or more, *i.e..,* ADCs with a DAR of 6 or more, including DAR6, DAR7, DAR8, and DAR greater than 8 (*i.e.*, drug loaded species of 6, 7, 8, or greater than 8). In a separate embodiment, the term undesired ADC species may refer to drug loaded species of 8 or more, *i.e.,* ADCs with a DAR of 8 or more, including DAR8, and DAR greater than 8 (*i.e.*, drug loaded species of 8, or greater than 8).

The term "ADC mixture", as used herein, refers to a composition containing a heterogeneous DAR distribution of ADCs. In one embodiment, an ADC mixture contains ADCs having a distribution of DARs of 1 to 8, *e.g.,* 2, 4, 6, and 8 (*i.e.*, drug loaded species of 2, 4, 6, and 8). Notably, degradation products may result such that DARs of 1, 3, 5, and 7 may also be included in the mixture. Further, ADCs within the mixture may also have DARs greater than 8. The ADC mixture results from interchain disulfide reduction followed by conjugation. In one embodiment, the ADC mixture comprises both ADCs with a DAR of 4 or less (*i.e.*, a drug loaded species of 4 or less) and ADCs with a DAR of 6 or more (*i.e.,* a drug loaded species of 6 or more).

The term "cancer" is meant to refer to or describe the physiological condition in mammals that is typically characterized by unregulated cell growth. Examples of cancer include, but are not limited to, carcinoma, lymphoma, blastoma, sarcoma, and leukemia or lymphoid malignancies. More particular examples of such cancers include glioblastoma, non-small cell lung cancer, lung cancer, colon cancer, colorectal cancer, head and neck cancer, breast cancer (e.g., triple negative breast cancer), pancreatic cancer, squamous cell tumors, squamous cell carcinoma (e.g., squamous cell lung cancer or squamous cell head and neck cancer), anal cancer, skin cancer, and vulvar cancer. In one embodiment, the ADCs of the disclosure are administered to a patient having a tumor(s) containing amplifications of the EGFR gene, whereby the tumor expresses the truncated version of the EGFR, EGFRvIII. In one embodiment, the ADCs of the disclosure are administered to a patient having a solid tumor which is likely to over-express EGFR. In one embodiment, the ADCs of the disclosure are administered to a patient having squamous cell Non-Small Cell Lung Cancer (NSCLC). In one embodiment, the ADCs of the disclosure are administered to a patient having solid tumors, including advanced solid tumors.

The term "EGFR expressing tumor," as used herein, refers to a tumor which expresses EGFR protein. In one embodiment, EGFR expression in a tumor is determined using immunohistochemical staining of tumor cell membranes, where any immunohistochemical staining above background level in a tumor sample indicates that the tumor is an EGFR expressing tumor. Methods for detecting expression of EGFR in a tumor are known in the art, *e.g.,* the EGFR pharmDx^{™} Kit (Dako). In contrast, an "EGFR negative tumor" is defined as a tumor having an absence of EGFR membrane staining above background in a tumor sample as determined by immunohistochemical techniques.

The term "EGFRvIII positive tumor," as used herein, refers to a tumor which expresses EGFRvIII protein. In one embodiment, EGFRvIII expression in a tumor is determined using immunohistochemical staining of tumor cell membranes, where any immunohistochemical staining above background level in a tumor sample indicates that the tumor is an EGFRvIII expressing tumor. Methods for detecting expression of EGFR in a tumor are known in the art, and include immunohistochemical assays. In contrast, an "EGFRvIII negative tumor" is defined as a tumor having an absence of EGFRvIII membrane staining above background in a tumor sample as determined by immunohistochemical techniques.

The terms "overexpress," "overexpression," or "overexpressed" interchangeably refer to a gene that is transcribed or translated at a detectably greater level, usually in a cancer cell, in comparison to a normal cell. Overexpression therefore refers to both overexpression of protein and RNA (due to increased transcription, post transcriptional processing, translation, post translational processing, altered stability, and altered protein degradation), as well as local overexpression due to altered protein traffic patterns (increased nuclear localization), and augmented functional activity, e.g., as in an increased enzyme hydrolysis of substrate. Thus, overexpression refers to either protein or RNA levels. Overexpression can also be by 50%, 60%, 70%, 80%, 90% or more in comparison to a normal cell or comparison cell. In certain embodiments, the anti-EGFR ADCs of the disclosure are used to treat solid tumors likely to overexpress EGFR.

The term "administering" as used herein is meant to refer to the delivery of a substance (e.g., an anti-EGFR ADC) to achieve a therapeutic objective (e.g., the treatment of an EGFR- associated disorder). Modes of administration may be parenteral, enteral and topical. Parenteral administration is usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal and intrasternal injection and infusion.

The term "combination therapy", as used herein, refers to the administration of two or more therapeutic substances, e.g., an anti-EGFR ADC and an additional therapeutic agent. The additional therapeutic agent may be administered concomitant with, prior to, or following the administration of the anti-EGFR ADC.

As used herein, the term "effective amount" or "therapeutically effective amount" refers to the amount of a drug, *e.g.,* an antibody or ADC, which is sufficient to reduce or ameliorate the severity and/or duration of a disorder, e.g., cancer, or one or more symptoms thereof, prevent the advancement of a disorder, cause regression of a disorder, prevent the recurrence, development, onset or progression of one or more symptoms associated with a disorder, detect a disorder, or enhance or improve the prophylactic or therapeutic effect(s) of another therapy (*e.g.,* prophylactic or therapeutic agent). The effective amount of an antibody or ADC may, for example, inhibit tumor growth (e.g., inhibit an increase in tumor volume), decrease tumor growth (*e.g.,* decrease tumor volume), reduce the number of cancer cells, and/or relieve to some extent one or more of the symptoms associated with the cancer. The effective amount may, for example, improve disease free survival (DFS), improve overall survival (OS), or decrease likelihood of recurrence.

Various aspects of the disclosure are described in further detail in the following subsections.

### 2. Anti-EGFR Antibody Drug Conjugates (ADCs): Anti-EGFR Antibodies

One aspect of the disclosure features an anti-human Epidermal Growth Factor Receptor (anti-hEGFR) Antibody Drug Conjgate (ADC) comprising an anti-hEGFR antibody conjugated to a drug via a linker, wherein the drug is a Bcl-xL inhibitor. Exemplary anti-EGFR antibodies (and sequences thereof) that can be used in the ADCs set forth herein are described below, as well as in US 2015-0337042.

The anti-EGFR antibodies described herein provide the ADCs of the disclosure with the ability to bind to EGFR such that the cytotoxic Bcl-xL drug attached to the antibody may be delivered to the EGFR-expressing cell.

While the term "antibody" is used throughout, it should be noted that antibody fragments (*i.e.,* antigen-binding portions of an anti-EGFR antibody) may also be conjugated to the Bcl-xL inhibitors described herein. Thus, it is within the scope of the disclosure that in certain embodiments, antibody fragments of the anti-EGFR antibodies described herein are conjugated to Bcl-xL inhibitors (including those described below in Section 3) via linkers (including those described below in Section 4). In certain embodiments, the anti-EGFR antibody binding portion is a Fab, a Fab', a F(ab')2, a Fv, a disulfide linked Fv, an scFv, a single domain antibody, or a diabody.

Anti-EGFR antibodies that may be used in the ADCs of the disclosure have characteristics making them advantageous for use in an ADC. In one embodiment, an anti-EGFR antibody has characteristics including, but not limited to, binding to tumor cells expressing EGFRvIII, binding to wild type EGFR on tumor cells expressing EGFR, recognizing the epitope CGADSYEMEEDGVRKC (SEQ ID NO: 45) on EGFR, binding to EGFR on normal human epithelial keratinocytes, and decreasing or inhibiting xenograft tumor growth in a mouse model. In one embodiment, an anti-EGFR antibody which may be used in the ADC of the disclosure is capable of binding an epitope of human EGFR defined by SEQ ID NO: 45 and/or is able to compete with any antibody disclosed herein (*e.g.,* Ab1, AbA, AbB, AbC, AbD, AbE, AbF, AbG, AbH, AbJ, AbK) for binding to human EGFR. Binding of the antibody to EGFR may be assessed according to, e.g. competition assay analysis, as described in US 2015-0337042 A1. In one embodiment of the disclosure, an anti-EGFR antibody that may be used in an ADC of the disclosure has a dissociation constant (K_{d}) of between about 1 x 10⁻⁶ M and about 1 x 10⁻¹⁰ M, as determined by surface plasmon resonance, to 1-525 of EGFR (SEQ ID NO: 47). In other embodiments of the foregoing aspects, the ADC of the disclosure comprises an anti-EGFR antibody that binds EGFRvIII, binds EGFR on cells overexpressing EGFR, and recognizes the epitope CGADSYEMEEDGVRKC (SEQ ID NO: 45) on EGFR. In a further embodiment, the anti-EGFR antibody binds EGFRvIII at an epitope which is distinct from the EGFRvIII junctional peptide. In additional embodiments of the foregoing aspects, the anti-EGFR antibody used in an ADC of the disclosure, does not compete with cetuximab for binding to human EGFR.

In one embodiment, an ADC of the disclosure comprises an anti-EGFR antibody that binds to EGFR(1-525) (SEQ ID NO: 47) with a dissociation constant (K_{d}) of about 1 x 10⁻⁶ M or less, as determined by surface plasmon resonance. Alternatively, an anti-EGFR antibody may bind to EGFR (1-525) (SEQ ID NO: 47) with a K_{d} of between about 1 x 10⁻⁶ M and about 1 x 10⁻¹⁰ M, as determined by surface plasmon resonance. In a further alternative, an anti-EGFR antibody binds to EGFR (1-525) (SEQ ID NO: 47) with a K_{d} of between about 1 x 10⁻⁶ M and about 1 x 10⁻⁷ M, as determined by surface plasmon resonance. Alternatively, antibodies used in the disclosure may bind to EGFR (1-525) (SEQ ID NO: 47) with a K_{d} of between about 1 x 10⁻⁶ M and about 5 x 10⁻¹⁰ M; a K_{d} of between about 1 x 10⁻⁶ M and about 1 x 10⁻⁹ M; a K_{d} of between about 1 x 10⁻⁶ M and about 5 x 10⁻⁹ M; a K_{d} of between about 1 x 10⁻⁶ M and about 1 x 10⁻⁸ M; a K_{d} of between about 1 x 10⁻⁶ M and about 5 x 10⁻⁸ M; a K_{d} of between about 5.9 x 10⁻⁷ M and about 1.7 x 10⁻⁹M; a K_{d} of between about 5.9 x 10⁻⁷ M and about 2.2 x 10⁻⁷ M, as determined by surface plasmon resonance. In certain embodiments, the dissociation constant (K_{d}) of the anti-hEGFR antibody used in the ADC of the disclosure is lower than the dissociation constant for Ab1 but higher than the dissociation constant of anti-EGFR antibody cetuximab (*i.e.*, the antibody binds to EGFR more tightly than Ab1 but not as tightly as cetuximab).

One advantage of the anti-EGFR antibodies described herein, is that the antibodies are capable of binding to tumor cells expressing EGFRvIII, thus making the ADCs of the disclosure specific for malignant cells. While EGFRvIII is associated with certain types of cancer, many anti-EGFR antibodies known in the art, *e.g.,* cetuximab, are not effective at inhibiting or decreasing tumor growth in EGFRvIII expressing tumors. Thus, in one embodiment, an antibody used in an ADC of the disclosure binds to EGFRvIII (SEQ ID NO: 33) with a K_{d} of about 8.2 x 10⁻⁹ M or less, as determined by surface plasmon resonance. Alternatively, an antibody used in an ADC of the disclosure binds to EGFRvIII (SEQ ID NO: 33) with a K_{d} of between about 8.2 x 10⁻⁹ M and about 6.3 x 10⁻¹⁰ M; a K_{d} of between about 8.2 x 10⁻⁹ M and about 2.0 x 10⁻⁹ M; a K_{d} of between about 2.3 x 10⁻⁹ M and about 1.5 x 10⁻¹⁰ M, as determined by surface plasmon resonance.

An anti-EGFR antibody used in an ADC of the disclosure is able, in one embodiment, to inhibit or decrease tumor growth in an *in vivo* xenograft mouse model. For example, in certain embodiments, an anti-EGFR antibody is able to inhibit tumor growth by at least about 50% in an *in vivo* human non-small-cell lung carcinoma (NSCLC) xenograft assay relative to a human IgG antibody which is not specific for EGFR. In certain embodiments, an anti-EGFR antibody is able to inhibit or decrease tumor growth in an *in vivo* human non-small-cell lung carcinoma (NSCLC) xenograft assay relative to a human IgG antibody which is not specific for EGFR by at least about 55%, at least about 60%, at least about 65%, at least about 70%, at least about 75%, or at least about 80%, when administered at the same dose and dosing periodicity.

The term a "xenograft assay", as used herein, refers to a human tumor xenograft assay, wherein human tumor cells are transplanted, either under the skin or into the organ type in which the tumor originated, into immunocompromised mice that do not reject human cells.

It should be noted that anti-EGFR antibodies having combinations of the aforementioned characteristics are also considered to be embodiments of the disclosure. For example, an anti-EGFR antibody may bind to EGFR(1-525) (SEQ ID NO: 47) with a dissociation constant (K_{d}) of about 1 x 10⁻⁶ M or less, as determined by surface plasmon resonance, and bind to an epitope within the amino acid sequence CGADSYEMEEDGVRKC (SEQ ID NO: 45) and compete with Ab1 (or an anti-EGFR antibody comprising a heavy chain variable domain comprising the amino acid sequence set forth in SEQ ID NO: 1 and a light chain variable domain comprising the amino acid sequence set forth in SEQ ID NO: 5) for binding to EGFRvIII (SEQ ID NO: 33) in a competitive binding assay. In certain embodiments, an anti-EGFR ADC of the disclosure comprises an anti-EGFR antibody that binds to an epitope within the amino acid sequence CGADSYEMEEDGVRKC (SEQ ID NO: 45) and competes with Ab1 (or an anti-EGFR antibody comprises a heavy chain variable domain comprising the amino acid sequence set forth in SEQ ID NO: 1 and a light chain variable domain comprising the amino acid sequence set forth in SEQ ID NO: 5) for binding to EGFRvIII (SEQ ID NO: 33) in a competitive binding assay; and bind to EGFRvIII (SEQ ID NO: 33) with a K_{d} of about 8.2 x 10⁻⁹ M or less, as determined by surface plasmon resonance.

In one embodiment, anti-EGFR antibodies used in an ADC of the disclosure exhibits a high capacity to reduce or to neutralize EGFR activity, *e.g.,* as assessed by any one of several *in vitro* and *in vivo* assays known in the art. For example, inhibition of phosphorylation of EGFR in an EGFR expressing cell line, *e.g.,* the h292 cell line, can be measured. In certain embodiments, an anti-EGFR antibody binds human EGFR, wherein the antibody dissociates from human EGFR (EGFR 1-525) with a K_{D} rate constant of about 5.9 x 10⁻⁷ M or less, as determined by surface plasmon resonance. In a further embodiment, the antibody may dissociate from human EGFR (1-525) with a K_{D} rate constant of about 4.2 x 10⁻⁷ M, as determined by surface plasmon resonance. Alternatively, the antibody may dissociate from human EGFR (1-525) with a k_{off} rate constant of about K_{D} rate constant of about 2.5 x 10⁻⁷ M, as determined by surface plasmon resonance. In certain embodiments, the anti-EGFR antibodies of the disclosure have a K_{D} rate constant of between 5.9 x 10⁻⁷ M and 5 x 10⁻⁹ M. Alternatively, the antibody may dissociate from human EGFRvIII with a K_{D} rate constant of about 6.1 x 10⁻⁹ M or less, as determined by surface plasmon resonance. Alternatively, the antibody may dissociate from human EGFRvIII with a K_{D} rate constant of about 3.9 x 10⁻⁹ M or less, as determined by surface plasmon resonance. Alternatively, the antibody may dissociate from human EGFRvIII with a K_{D} rate constant of about 2.3 x 10⁻⁹M or less, as determined by surface plasmon resonance.

Exemplary anti-EGFR antibodies that may be used in the ADCs described herein include, but are not limited to, Antibody 1 (Ab1), Antibody A (AbA), Antibody B (AbB), Antibody C (AbC), Antibody D (AbD), Antibody E (AbE), Antibody F (AbF), Antibody G (AbG), Antibody H (AbH), Antibody J (AbJ), Antibody K (AbK), Antibody L (AbL), Antibody M (AbM), Antibody N (AbN), Antibody O (AbO), Antibody P (AbP), and Antibody Q (AbQ).

In one embodiment, the disclosure features an anti-EGFR ADC comprising Ab1 conjugated via a linker to a Bcl-xL inhibitor. Ab1 is a humanized anti-EGFR antibody. The light and heavy chain sequences of Ab1 are described in SEQ ID NO: 13 and SEQ ID NO: 14, respectively (see also US Patent Application Publication No. 20120183471). The light chain variable region of Ab1 is described in SEQ ID NO: 5, and comprises a CDR1 amino acid sequence set forth in SEQ ID NO: 6, a CDR2 amino acid sequence set forth in SEQ ID NO: 7, and a CDR3 amino acid sequence set forth in SEQ ID NO: 8. The heavy chain variable region of Ab1 is described in SEQ ID NO: 1, and comprises a CDR1 amino acid sequence set forth in SEQ ID NO: 2, a CDR2 amino acid sequence set forth in SEQ ID NO: 3, and a CDR3 amino acid sequence set forth in SEQ ID NO: 4. In one embodiment, an ADC of the disclosure comprises an anti-EGFR antibody that binds to an epitope within the amino acid sequence set forth in SEQ ID NO: 45 and competes with an anti-EGFR antibody comprising a heavy chain variable domain comprising the amino acid sequence set forth in SEQ ID NO: 1 and a light chain variable domain comprising the amino acid sequence set forth in SEQ ID NO: 5 for binding to EGFRvIII in a competitive binding assay.

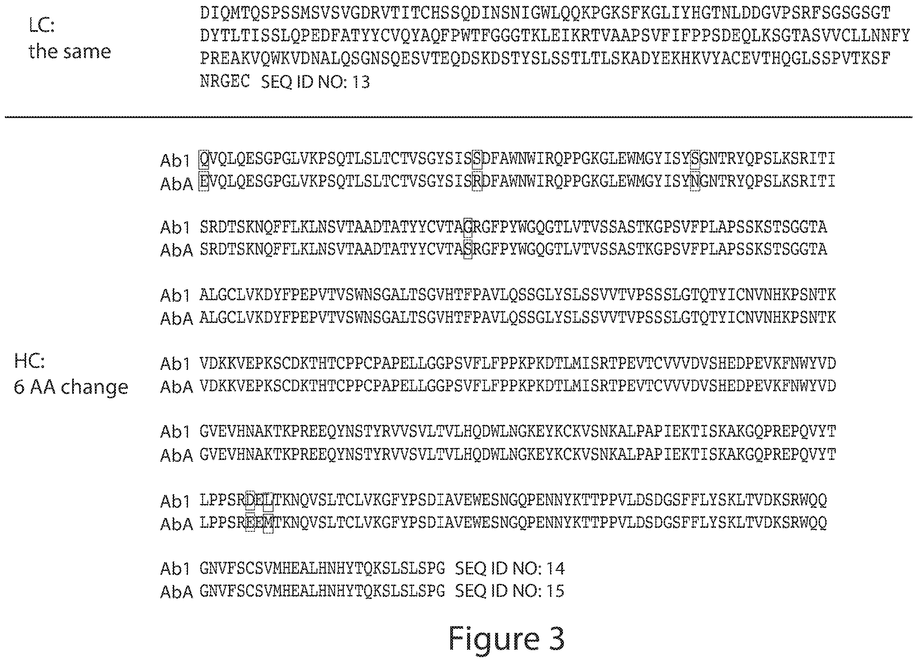

In one embodiment, the disclosure features an anti-hEGFR ADC comprising an anti-hEGFR antibody which is antibody AbA conjugated via a linker to a Bcl-xL inhibitor. The term "AbA" is meant to include an IgG antibody having at least the six CDRs of AbA. The AbA antibody has the same light chain as that of Ab1, but has a heavy chain containing six amino acid sequence changes relative to parental antibody Ab1 (four amino acid changes in the variable region and two changes in the constant region of the heavy chain). The AbA antibody comprises a heavy chain variable region comprising a CDR3 domain comprising the amino acid sequence of SEQ ID NO: 12, a CDR2 domain comprising the amino acid sequence of SEQ ID NO: 11, and a CDR1 domain comprising the amino acid sequence of SEQ ID NO: 10, and a light chain variable region comprising a CDR3 domain comprising the amino acid sequence of SEQ ID NO: 8, a CDR2 domain comprising the amino acid sequence of SEQ ID NO: 7, and a CDR1 domain comprising the amino acid sequence of SEQ ID NO: 6. The heavy chain variable region of AbA is defined by the amino acid sequence set forth in SEQ ID NO: 9, and a light chain variable region comprising the amino acid sequence of SEQ ID NO: 5. The full length heavy chain of antibody AbA is set forth in the amino acid sequence described in SEQ ID NO: 15, while the full length light chain of antibody AbA is set forth in the amino acid sequence described in SEQ ID NO: 13 (see Figure 3). The nucleic acid sequence of the heavy chain of AbA is provided below:
The nucleic acid sequence of the light chain of AbA is provided below:
The amino acid sequence of the heavy chain of AbA is provided below:
In another embodiment, the amino acid sequence of the heavy chain of AbA is provided below:
The amino acid sequence of the light chain of AbA is provided below:

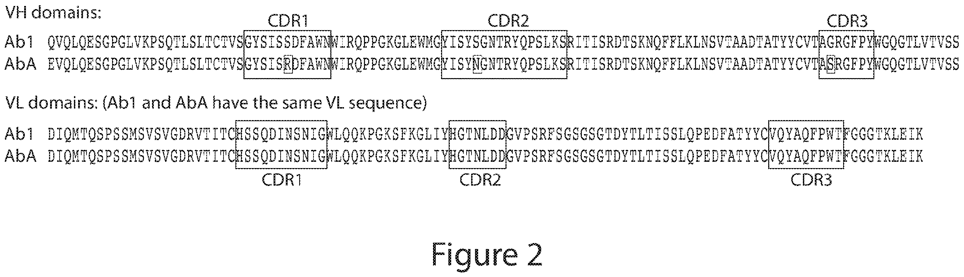

Figures 2 and 3 provide an alignment of the amino acid sequences of the VH and VL regions (Figure 2) and the complete heavy and light chains (Figure 3) of Ab1 and AbA. The light chain amino acid sequences of Ab1 and AbA are the same (SEQ ID NO: 13). The heavy chain amino acid sequences of Ab1 and AbA, however, have six amino acid differences between the two sequences, three of which are in the CDRs. Differences between the Ab1 VH amino acid sequence and the AbA VH amino acid sequence are shaded in Figure 2 and are found in each of the VH CDRs. The CDR1 domain of the variable heavy chain of AbA included an amino acid change from a serine (Ab1) to an arginine. The CDR2 domain of the variable heavy chain included an amino acid change from a serine in Ab1 to an asparagine in AbA. Finally, the CDR3 domain of the variable heavy chain included an amino acid change from a glycine in Ab1 to a serine in AbA. Two of the amino acid changes within AbA are in the constant region of the heavy chain (D354E and L356M). The Fc region amino acid mutations in AbA represent human IgG allotype changes from a z, a allotype to a z, non-a allotype. In addition to the other changes, the first amino acid was changed from a glutamine (Q) to a glutamic acid (E), as described, for example, in Figure 3.

Thus, in one embodiment, the disclosure features an ADC comprising an anti-hEGFR antibody conjugated via a linker to a Bcl-xL inhibitor wherein the antibody comprises a heavy chain variable region comprising a CDR3 domain comprising the amino acid sequence of SEQ ID NO: 12, a CDR2 domain comprising the amino acid sequence of SEQ ID NO: 11, and a CDR1 domain comprising the amino acid sequence of SEQ ID NO: 10, and a light chain variable region comprising a CDR3 domain comprising the amino acid sequence of SEQ ID NO: 8, a CDR2 domain comprising the amino acid sequence of SEQ ID NO: 7, and a CDR1 domain comprising the amino acid sequence of SEQ ID NO: 6. In one embodiment, the disclosure features an ADC comprising an anti-hEGFR antibody conjugated via a linker to a Bcl-xL inhibitor, wherein the antibody comprises a heavy chain variable region comprising the amino acid sequence set forth in SEQ ID NO: 9, and a light chain variable region comprising the amino acid sequence of SEQ ID NO: 5.

In one embodiment, the disclosure features an anti-EGFR ADC comprising antibody AbB conjugated via a linker to a Bcl-xL inhibitor. The AbB antibody comprises a heavy chain variable region comprising a CDR3 domain comprising the amino acid sequence of SEQ ID NO: 19, a CDR2 domain comprising the amino acid sequence of SEQ ID NO: 17, and a CDR1 domain comprising the amino acid sequence of SEQ ID NO: 16, and a light chain variable region comprising a CDR3 domain comprising the amino acid sequence of SEQ ID NO: 8, a CDR2 domain comprising the amino acid sequence of SEQ ID NO: 7, and a CDR1 domain comprising the amino acid sequence of SEQ ID NO: 6. In further embodiments, the disclosure provides an antibody having a heavy chain variable region comprising the amino acid sequence of SEQ ID NO: 64 and a light chain variable region comprising the amino acid sequence of SEQ ID NO: 65. Thus, in one embodiment, the ADC of the disclosure comprises an anti-hEGFR antibody having the CDR amino acid sequences of AbB. In a separate embodiment, the ADC of the disclosure comprises an anti-hEGFR antibody having heavy and light chain variable regions comprising the amino acid sequences of AbB.

In one embodiment, the disclosure features an anti-EGFR ADC comprising antibody AbC conjugated via a linker to a Bcl-xL inhibitor. The AbC antibody comprises a heavy chain variable region comprising a CDR3 domain comprising the amino acid sequence of SEQ ID NO: 4, a CDR2 domain comprising the amino acid sequence of SEQ ID NO: 3, and a CDR1 domain comprising the amino acid sequence of SEQ ID NO: 2, and a light chain variable region comprising a CDR3 domain comprising the amino acid sequence of SEQ ID NO: 84, a CDR2 domain comprising the amino acid sequence of SEQ ID NO: 7, and a CDR1 domain comprising the amino acid sequence of SEQ ID NO: 6. In further embodiments, the disclosure provides an antibody having a heavy chain variable region comprising the amino acid sequence of SEQ ID NO: 66 and a light chain variable region comprising the amino acid sequence of SEQ ID NO: 67. Thus, in one embodiment, the ADC of the disclosure comprises an anti-hEGFR antibody having the CDR amino acid sequences of AbC. In a separate embodiment, the ADC of the disclosure comprises an anti-hEGFR antibody having heavy and light chain variable regions comprising the amino acid sequences of AbC.

In one embodiment, the disclosure features an anti-EGFR ADC comprising antibody AbD conjugated via a linker to a Bcl-xL inhibitor. The AbD antibody comprises a heavy chain variable region comprising a CDR3 domain comprising the amino acid sequence of SEQ ID NO: 4, a CDR2 domain comprising the amino acid sequence of SEQ ID NO: 3, and a CDR1 domain comprising the amino acid sequence of SEQ ID NO: 2, and a light chain variable region comprising a CDR3 domain comprising the amino acid sequence of SEQ ID NO: 31, a CDR2 domain comprising the amino acid sequence of SEQ ID NO: 83, and a CDR1 domain comprising the amino acid sequence of SEQ ID NO: 82. In further embodiments, the disclosure provides an antibody having a heavy chain variable region comprising the amino acid sequence of SEQ ID NO: 68 and a light chain variable region comprising the amino acid sequence of SEQ ID NO: 69. Thus, in one embodiment, the ADC of the disclosure comprises an anti-hEGFR antibody having the CDR amino acid sequences of AbD. In a separate embodiment, the ADC of the disclosure comprises an anti-hEGFR antibody having heavy and light chain variable regions comprising the amino acid sequences of AbD.

In one embodiment, the disclosure features an anti-EGFR ADC comprising antibody AbE conjugated via a linker to a Bcl-xL inhibitor. The AbE antibody comprises a heavy chain variable region comprising a CDR3 domain comprising the amino acid sequence of SEQ ID NO: 4, a CDR2 domain comprising the amino acid sequence of SEQ ID NO: 3, and a CDR1 domain comprising the amino acid sequence of SEQ ID NO: 2, and a light chain variable region comprising a CDR3 domain comprising the amino acid sequence of SEQ ID NO: 85, a CDR2 domain comprising the amino acid sequence of SEQ ID NO: 27, and a CDR1 domain comprising the amino acid sequence of SEQ ID NO: 82. In further embodiments, the disclosure provides an antibody having a heavy chain variable region comprising the amino acid sequence of SEQ ID NO: 50 and a light chain variable region comprising the amino acid sequence of SEQ ID NO: 51. Thus, in one embodiment, the ADC of the disclosure comprises an anti-hEGFR antibody having the CDR amino acid sequences of AbE. In a separate embodiment, the ADC of the disclosure comprises an anti-hEGFR antibody having heavy and light chain variable regions comprising the amino acid sequences of AbE.

In one embodiment, the disclosure features an anti-EGFR ADC comprising antibody AbF conjugated via a linker to a Bcl-xL inhibitor. The AbF antibody comprises a heavy chain variable region comprising a CDR3 domain comprising the amino acid sequence of SEQ ID NO: 12, a CDR2 domain comprising the amino acid sequence of SEQ ID NO: 3, and a CDR1 domain comprising the amino acid sequence of SEQ ID NO: 10, and a light chain variable region comprising a CDR3 domain comprising the amino acid sequence of SEQ ID NO: 8, a CDR2 domain comprising the amino acid sequence of SEQ ID NO: 7, and a CDR1 domain comprising the amino acid sequence of SEQ ID NO: 6. In further embodiments, the disclosure provides an antibody having a heavy chain variable region comprising the amino acid sequence of SEQ ID NO: 52 and a light chain variable region comprising the amino acid sequence of SEQ ID NO: 53. Thus, in one embodiment, the ADC of the disclosure comprises an anti-hEGFR antibody having the CDR amino acid sequences of AbF. In a separate embodiment, the ADC of the disclosure comprises an anti-hEGFR antibody having heavy and light chain variable regions comprising the amino acid sequences of AbF.

In one embodiment, the disclosure features an anti-EGFR ADC comprising antibody AbG conjugated via a linker to a Bcl-xL inhibitor. The AbG antibody comprises a heavy chain variable region comprising a CDR3 domain comprising the amino acid sequence of SEQ ID NO: 18, a CDR2 domain comprising the amino acid sequence of SEQ ID NO: 17, and a CDR1 domain comprising the amino acid sequence of SEQ ID NO: 16, and a light chain variable region comprising a CDR3 domain comprising the amino acid sequence of SEQ ID NO: 25, a CDR2 domain comprising the amino acid sequence of SEQ ID NO: 24, and a CDR1 domain comprising the amino acid sequence of SEQ ID NO: 23. In further embodiments, the disclosure provides an antibody having a heavy chain variable region comprising the amino acid sequence of SEQ ID NO: 72 and a light chain variable region comprising the amino acid sequence of SEQ ID NO: 73. Thus, in one embodiment, the ADC of the disclosure comprises an anti-hEGFR antibody having the CDR amino acid sequences of AbG. In a separate embodiment, the ADC of the disclosure comprises an anti-hEGFR antibody having heavy and light chain variable regions comprising the amino acid sequences of AbG.

In one embodiment, the disclosure features an anti-EGFR ADC comprising antibody AbH conjugated via a linker to a Bcl-xL inhibitor. The AbH antibody comprises a heavy chain variable region comprising a CDR3 domain comprising the amino acid sequence of SEQ ID NO: 18, a CDR2 domain comprising the amino acid sequence of SEQ ID NO: 11, and a CDR1 domain comprising the amino acid sequence of SEQ ID NO: 80, and a light chain variable region comprising a CDR3 domain comprising the amino acid sequence of SEQ ID NO: 25, a CDR2 domain comprising the amino acid sequence of SEQ ID NO: 24, and a CDR1 domain comprising the amino acid sequence of SEQ ID NO: 23. In further embodiments, the disclosure provides an antibody having a heavy chain variable region comprising the amino acid sequence of SEQ ID NO: 54 and a light chain variable region comprising the amino acid sequence of SEQ ID NO: 55. Thus, in one embodiment, the ADC of the disclosure comprises an anti-hEGFR antibody having the CDR amino acid sequences of AbH. In a separate embodiment, the ADC of the disclosure comprises an anti-hEGFR antibody having heavy and light chain variable regions comprising the amino acid sequences of AbH.

In one embodiment, the disclosure features an anti-EGFR ADC comprising antibody AbJ conjugated via a linker to a Bcl-xL inhibitor. The AbJ antibody comprises a heavy chain variable region comprising a CDR3 domain comprising the amino acid sequence of SEQ ID NO: 18, a CDR2 domain comprising the amino acid sequence of SEQ ID NO: 3, and a CDR1 domain comprising the amino acid sequence of SEQ ID NO: 80, and a light chain variable region comprising a CDR3 domain comprising the amino acid sequence of SEQ ID NO: 25, a CDR2 domain comprising the amino acid sequence of SEQ ID NO: 24, and a CDR1 domain comprising the amino acid sequence of SEQ ID NO: 23. In further embodiments, the disclosure provides an antibody having a heavy chain variable region comprising the amino acid sequence of SEQ ID NO: 56 and a light chain variable region comprising the amino acid sequence of SEQ ID NO: 57. Thus, in one embodiment, the ADC of the disclosure comprises an anti-hEGFR antibody having the CDR amino acid sequences of AbJ. In a separate embodiment, the ADC of the disclosure comprises an anti-hEGFR antibody having heavy and light chain variable regions comprising the amino acid sequences of AbJ.

In one embodiment, the disclosure features an anti-EGFR ADC comprising antibody AbK conjugated via a linker to a Bcl-xL inhibitor. The AbK antibody comprises a heavy chain variable region comprising a CDR3 domain comprising the amino acid sequence of SEQ ID NO: 19, a CDR2 domain comprising the amino acid sequence of SEQ ID NO: 11, and a CDR1 domain comprising the amino acid sequence of SEQ ID NO: 10, and a light chain variable region comprising a CDR3 domain comprising the amino acid sequence of SEQ ID NO: 28, a CDR2 domain comprising the amino acid sequence of SEQ ID NO: 27, and a CDR1 domain comprising the amino acid sequence of SEQ ID NO: 26. In further embodiments, the disclosure provides an antibody having a heavy chain variable region comprising the amino acid sequence of SEQ ID NO: 74 and a light chain variable region comprising the amino acid sequence of SEQ ID NO: 75. Thus, in one embodiment, the ADC of the disclosure comprises an anti-hEGFR antibody having the CDR amino acid sequences of AbK. In a separate embodiment, the ADC of the disclosure comprises an anti-hEGFR antibody having heavy and light chain variable regions comprising the amino acid sequences of AbK.

In one embodiment, the disclosure features an anti-EGFR ADC comprising antibody AbL conjugated via a linker to a Bcl-xL inhibitor. The AbL antibody comprises a heavy chain variable region comprising a CDR3 domain comprising the amino acid sequence of SEQ ID NO: 18, a CDR2 domain comprising the amino acid sequence of SEQ ID NO: 11, and a CDR1 domain comprising the amino acid sequence of SEQ ID NO: 80, and a light chain variable region comprising a CDR3 domain comprising the amino acid sequence of SEQ ID NO: 28, a CDR2 domain comprising the amino acid sequence of SEQ ID NO: 27, and a CDR1 domain comprising the amino acid sequence of SEQ ID NO: 26. In further embodiments, the disclosure provides an antibody having a heavy chain variable region comprising the amino acid sequence of SEQ ID NO: 58 and a light chain variable region comprising the amino acid sequence of SEQ ID NO: 59. Thus, in one embodiment, the ADC of the disclosure comprises an anti-hEGFR antibody having the CDR amino acid sequences of AbL. In a separate embodiment, the ADC of the disclosure comprises an anti-hEGFR antibody having heavy and light chain variable regions comprising the amino acid sequences of AbL.

In one embodiment, the disclosure features an anti-EGFR ADC comprising antibody AbM conjugated via a linker to a Bcl-xL inhibitor. The AbM antibody comprises a heavy chain variable region comprising a CDR3 domain comprising the amino acid sequence of SEQ ID NO: 12, a CDR2 domain comprising the amino acid sequence of SEQ ID NO: 11, and a CDR1 domain comprising the amino acid sequence of SEQ ID NO: 20, and a light chain variable region comprising a CDR3 domain comprising the amino acid sequence of SEQ ID NO: 28, a CDR2 domain comprising the amino acid sequence of SEQ ID NO: 27, and a CDR1 domain comprising the amino acid sequence of SEQ ID NO: 26. In further embodiments, the disclosure provides an antibody having a heavy chain variable region comprising the amino acid sequence of SEQ ID NO: 76 and a light chain variable region comprising the amino acid sequence of SEQ ID NO: 77. Thus, in one embodiment, the ADC of the disclosure comprises an anti-hEGFR antibody having the CDR amino acid sequences of AbM. In a separate embodiment, the ADC of the disclosure comprises an anti-hEGFR antibody having heavy and light chain variable regions comprising the amino acid sequences of AbM.

In one embodiment, the disclosure features an anti-EGFR ADC comprising antibody AbN conjugated via a linker to a Bcl-xL inhibitor. The AbN antibody comprises a heavy chain variable region comprising a CDR3 domain comprising the amino acid sequence of SEQ ID NO: 12, a CDR2 domain comprising the amino acid sequence of SEQ ID NO: 3, and a CDR1 domain comprising the amino acid sequence of SEQ ID NO: 20, and a light chain variable region comprising a CDR3 domain comprising the amino acid sequence of SEQ ID NO: 28, a CDR2 domain comprising the amino acid sequence of SEQ ID NO: 27, and a CDR1 domain comprising the amino acid sequence of SEQ ID NO: 26. In further embodiments, the disclosure provides an antibody having a heavy chain variable region comprising the amino acid sequence of SEQ ID NO: 60 and a light chain variable region comprising the amino acid sequence of SEQ ID NO: 61. Thus, in one embodiment, the ADC of the disclosure comprises an anti-hEGFR antibody having the CDR amino acid sequences of AbN. In a separate embodiment, the ADC of the disclosure comprises an anti-hEGFR antibody having heavy and light chain variable regions comprising the amino acid sequences of AbN.

In one embodiment, the disclosure features an anti-EGFR ADC comprising antibody AbO conjugated via a linker to a Bcl-xL inhibitor. The AbO antibody comprises a heavy chain variable region comprising a CDR3 domain comprising the amino acid sequence of SEQ ID NO: 12, a CDR2 domain comprising the amino acid sequence of SEQ ID NO: 11, and a CDR1 domain comprising the amino acid sequence of SEQ ID NO: 80, and a light chain variable region comprising a CDR3 domain comprising the amino acid sequence of SEQ ID NO: 28, a CDR2 domain comprising the amino acid sequence of SEQ ID NO: 27, and a CDR1 domain comprising the amino acid sequence of SEQ ID NO: 26. In further embodiments, the disclosure provides an antibody having a heavy chain variable region comprising the amino acid sequence of SEQ ID NO: 62 and a light chain variable region comprising the amino acid sequence of SEQ ID NO: 63. Thus, in one embodiment, the ADC of the disclosure comprises an anti-hEGFR antibody having the CDR amino acid sequences of AbO. In a separate embodiment, the ADC of the disclosure comprises an anti-hEGFR antibody having heavy and light chain variable regions comprising the amino acid sequences of AbO.

In one embodiment, the disclosure features an anti-EGFR ADC comprising antibody AbP conjugated via a linker to a Bcl-xL inhibitor. The AbP antibody comprises a heavy chain variable region comprising a CDR3 domain comprising the amino acid sequence of SEQ ID NO: 22, a CDR2 domain comprising the amino acid sequence of SEQ ID NO: 3, and a CDR1 domain comprising the amino acid sequence of SEQ ID NO: 21, and a light chain variable region comprising a CDR3 domain comprising the amino acid sequence of SEQ ID NO: 31, a CDR2 domain comprising the amino acid sequence of SEQ ID NO: 30, and a CDR1 domain comprising the amino acid sequence of SEQ ID NO: 29. In further embodiments, the disclosure provides an antibody having a heavy chain variable region comprising the amino acid sequence of SEQ ID NO: 78 and a light chain variable region comprising the amino acid sequence of SEQ ID NO: 79. Thus, in one embodiment, the ADC of the disclosure comprises an anti-hEGFR antibody having the CDR amino acid sequences of AbP. In a separate embodiment, the ADC of the disclosure comprises an anti-hEGFR antibody having heavy and light chain variable regions comprising the amino acid sequences of AbP.

In one embodiment, the disclosure features an anti-EGFR ADC comprising antibody AbQ conjugated via a linker to a Bcl-xL inhibitor. The AbQ antibody comprises a heavy chain variable region comprising a CDR3 domain comprising the amino acid sequence of SEQ ID NO: 22, a CDR2 domain comprising the amino acid sequence of SEQ ID NO: 11, and a CDR1 domain comprising the amino acid sequence of SEQ ID NO: 81, and a light chain variable region comprising a CDR3 domain comprising the amino acid sequence of SEQ ID NO: 31, a CDR2 domain comprising the amino acid sequence of SEQ ID NO: 30, and a CDR1 domain comprising the amino acid sequence of SEQ ID NO: 29. In further embodiments, the disclosure provides an antibody having a heavy chain variable region comprising the amino acid sequence of SEQ ID NO: 70 and a light chain variable region comprising the amino acid sequence of SEQ ID NO: 71. Thus, in one embodiment, the ADC of the disclosure comprises an anti-hEGFR antibody having the CDR amino acid sequences of AbQ. In a separate embodiment, the ADC of the disclosure comprises an anti-hEGFR antibody having heavy and light chain variable regions comprising the amino acid sequences of AbQ.

As described in Table 2, shown below, the antibody sequences disclosed herein provide amino acid consensus sequences that represent CDR domains resulting in improved binding to the Ab1 EGFR epitope. Thus, in one embodiment, the disclosure features an anti-EGFR antibody comprising a light chain variable region comprising a CDR3 domain comprising the amino acid sequence set forth as SEQ ID NO: 40, a CDR2 domain comprising the amino acid sequence set forth as SEQ ID NO: 39, and a CDR1 domain comprising the amino acid sequence set forth as SEQ ID NO: 38; and a heavy chain variable region comprising a CDR3 domain comprising the amino acid sequence set forth as SEQ ID NO: 37, a CDR2 domain comprising the amino acid sequence set forth as SEQ ID NO: 36, and a CDR1 domain comprising the amino acid sequence set forth as SEQ ID NO: 35. In a further embodiment, the anti-EGFR antibody of the disclosure comprises a heavy chain variable region comprising a CDR3 domain comprising an amino acid sequence as set forth in SEQ ID NO: 12, 18, 19, and 22; a CDR2 domain comprising an amino acid sequence as set forth in SEQ ID NO: 11 or 17; and a CDR1 domain comprising an amino acid sequence as set forth in SEQ ID NO: 10, 16, 20, and 21; and a light chain variable region comprising a CDR3 domain comprising an amino acid sequence as set forth in SEQ ID NO: 8, 25, 28, and 31; a CDR2 domain comprising an amino acid sequence as set forth in SEQ ID NO: 7, 24, 27, and 30; and a CDR1 domain comprising an amino acid sequence as set forth in SEQ ID NO: 6, 23, 26, and 29.

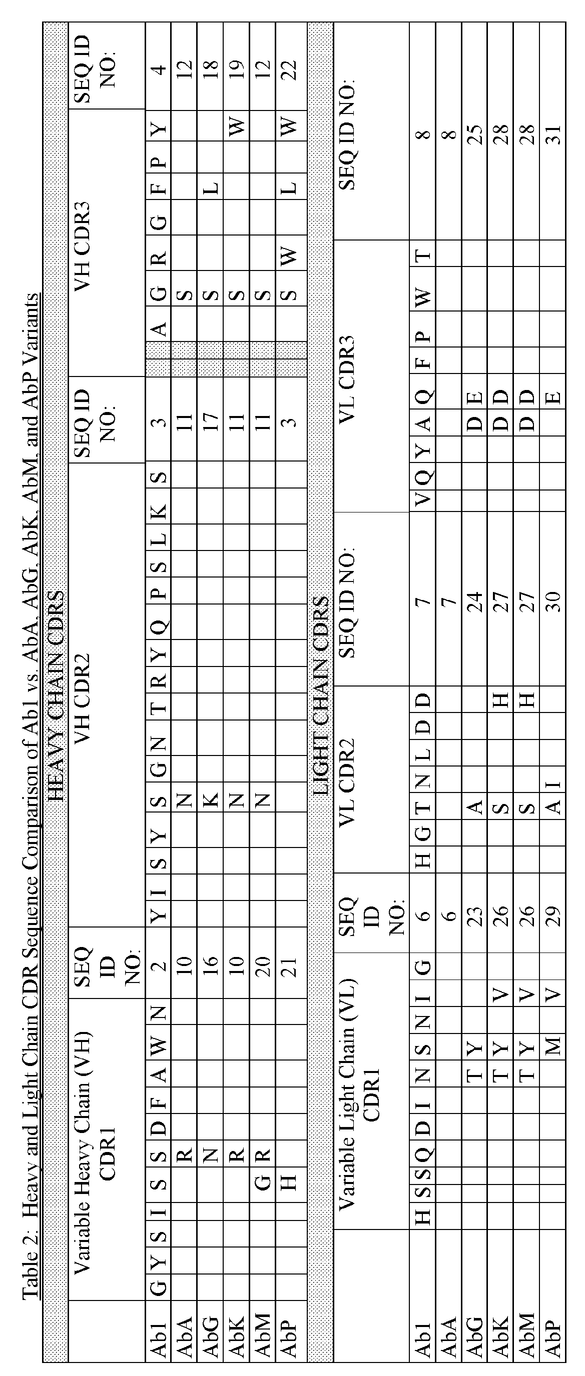

In one embodiment, the ADC of the disclosure includes an anti-hEGFR antibody comprises a heavy chain variable region comprising an amino acid sequence selected from the group consisting of 50, 52, 53, 56, 58, 60, 62, 64, 66, and 68; and a light chain variable region comprising an amino acid sequence selected from the group consisting of 51, 53, 55, 57, 59, 61, 63, 65, 67, and 69.

The foregoing anti-EGFR antibody CDR sequences establish a novel family of EGFR binding proteins, isolated in accordance with this disclosure, and comprising polypeptides that include the CDR sequences listed in Tables 2-4.

Table 2, above, provides an alignment of the amino acid sequences of the heavy and light chain CDRs for Ab1 variant antibodies AbA, AbG, AbK, AbM, and AbP in comparison to Ab1.

As described in Table 3, below, the Ab1 variant antibodies AbA, AbG, AbK, AbM, AbP each has a serine residue in the variable heavy chain of CDR3 in place of a glycine (shown in bold/underlined in Table 3).

**Table 3: CDR Consensus Sequences for Ab1 Variants from Table 2**

| CDR region | SEQ ID NO: | CDR Consensus Sequences for Ab1 Variants |
|---|---|---|
| VH CDR1 | SEQ ID NO:35 | G Y S I(S/G/H) (S/R/N)D FAWN |
| VH CDR2 | SEQ ID NO:36 | Y I S Y(S/N/K)G N T R Y Q P S L K S |
| VH CDR3 | SEQ ID NO:37 | A **S** (R/W) G (F/L) P (Y/W) |
| VL CDR1 | SEQ ID NO:38 | H S S Q D I(N/T)(Y/M/S)N(I/V)G |
| VL CDR2 | SEQ ID NO:39 | H G(T/A/S)(N/I)L D(D/H) |
| VL CDR3 | SEQ ID NO:40 | V Q Y (A/D) (Q/E/D) F P T |

A comparison of the VH and VL CDR sequences of Ab1 versus antibodies AbB, AbC, AbD, AbE, AbF, AbH, AbJ, AbL, AbN, AbO, and AbQ is described in Table 4. In addition to the CDR changes described in Table 4, below, AbG has an amino acid residue change within the framework 2 regions of the VH.

In one embodiment, the disclosure includes an anti-hEGFR antibody comprising a heavy chain variable region comprising an amino acid sequence selected from the group consisting of 50, 52, 54, 56, 58, 60, 62, 64, 66, 68, 70, 72, 74, 76, and 78; and a light chain variable region comprising an amino acid sequence selected from the group consisting of 51, 53, 55, 57, 59, 61, 63, 65, 67, 69, 71, 73, 75, 77, and 79.

In one embodiment, the disclosure includes an anti-hEGFR antibody comprising an HC CDR set (CDR1, CDR2, and CDR3) selected from the group consisting of SEQ ID NOs: 10, 11, and 12; SEQ ID NOs: 16, 17, and 18; SEQ ID NOs: 10, 11, and 19; SEQ ID NOs: 20, 11, and 12; SEQ ID NOs: 21, 3, and 22; SEQ ID NOs: 16, 17, and 19; SEQ ID NOs: 2, 3, and 4; SEQ ID NOs: 10, 3, and 12; SEQ ID NOs: 80, 11, and 18; SEQ ID NOs: 80, 3, and 18; SEQ ID NOs: 20, 3, and 12; SEQ ID NOs: 80, 11, and 12; and SEQ ID NOs: 81, 11, and 22; and an LC light chain CDR set (CDR1, CDR2, and CDR3) selected from the group consisting of SEQ ID NOs: 6, 7, and 8; SEQ ID NOs: 23, 24, and 25; SEQ ID NOs: 26, 27, and 28; SEQ ID NOs: 29, 30, and 31; SEQ ID NOs: 6, 7, and 84; SEQ ID NOs: 82, 83, and 31; and SEQ ID NOs: 82, 27, and 85, wherein the antibody, or antigen binding portion thereof, does not comprise both the HC CDR set of SEQ ID NOs: 2, 3, and 4, and the LC CDR set of SEQ ID NOs: 6, 7, and 8. In one embodiment, the disclosure includes an anti-hEGFR antibody comprising an LC CDR3 domain comprising the amino acid sequence set forth in SEQ ID NO: 40, an LC CDR2 domain comprising the amino acid sequence set forth in SEQ ID NO: 39, and an LC CDR1 domain comprising the amino acid sequence set forth in SEQ ID NO: 38; and an HC CDR3 domain comprising the amino acid sequence set forth in SEQ ID NO: 37, an HC CDR2 domain comprising the amino acid sequence set forth in SEQ ID NO: 36, and an HC CDR1 domain comprising the amino acid sequence set forth in SEQ ID NO: 35.

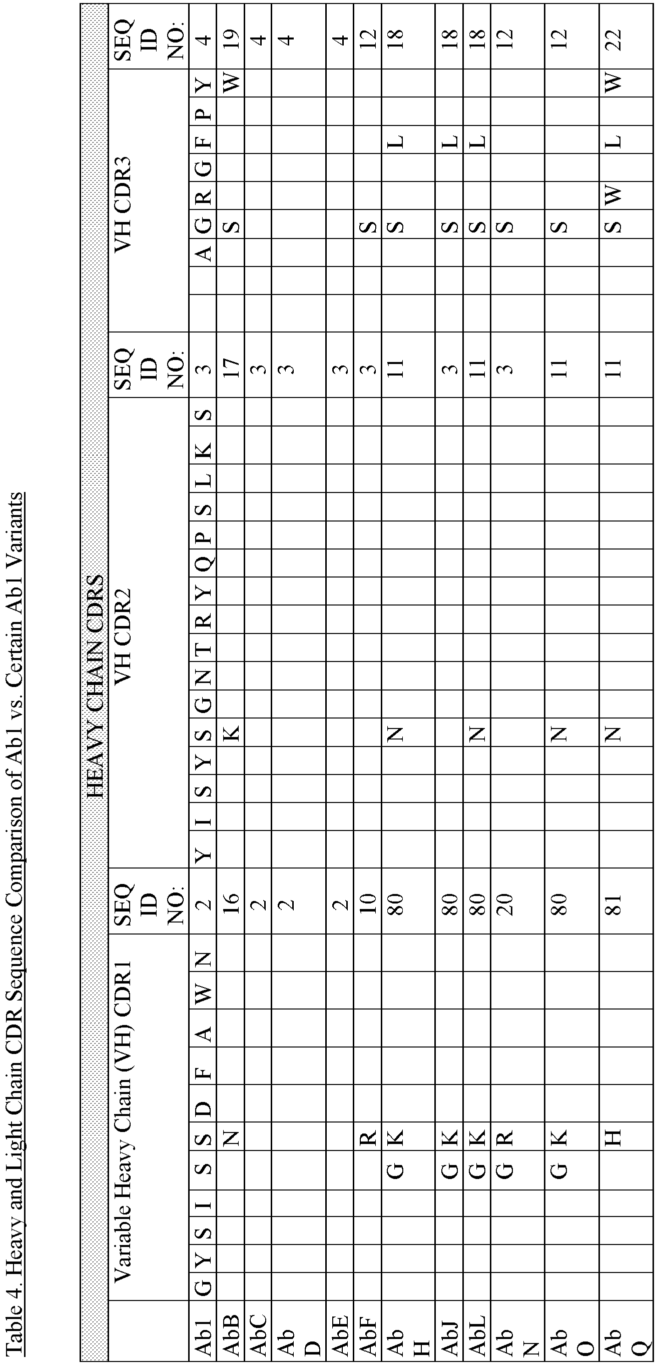

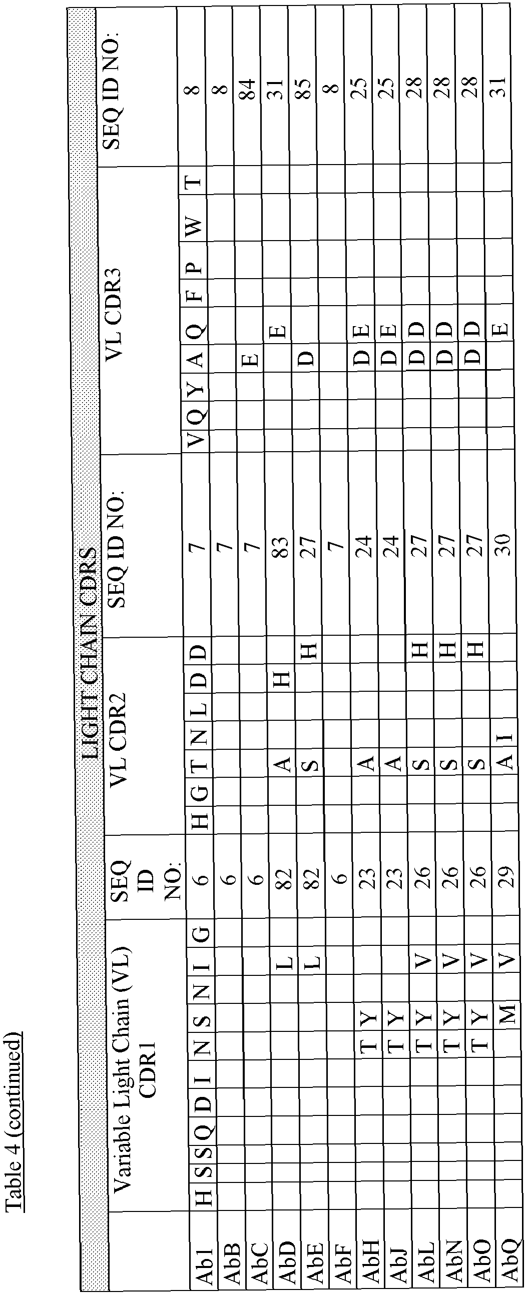

The full length heavy and light chain sequences of AbB are provided below:
AbB
Heavy chain

In one embodiment, the above AbB heavy chain sequence contains two alanine substitutions at the positions marked with two bold leucines (see also SEQ ID NO: 91).
AbB Light chain

In one embodiment, the ADC comprises an anti-EGFR antibody comprising a heavy chain comprising SEQ ID NO: 90 or 91 and a light chain comprising SEQ ID NO: 92.

The full length heavy and light chain sequences of AbG are provided below:
AbG
Heavy chain

In one embodiment, the above AbG heavy chain sequence contains two alanine substitutions at the positions marked with two bold leucines (see also SEQ ID NO: 94).
Light chain

In one embodiment, the ADC comprises an anti-EGFR antibody comprising a heavy chain comprising SEQ ID NO: 93 or 94 and a light chain comprising SEQ ID NO: 95.

The full length heavy and light chain sequences of AbK are provided below:
AbK
Heavy chain

In one embodiment, the above AbK heavy chain sequence contains two alanine substitutions at the positions marked with two bold leucines (see also SEQ ID NO: 97).
Light chain

In one embodiment, the ADC comprises an anti-EGFR antibody comprising a heavy chain comprising SEQ ID NO: 96 or 97 and a light chain comprising SEQ ID NO: 98.

To generate and to select CDRs having preferred EGFR binding and/or neutralizing activity with respect to hEGFR, standard methods known in the art for generating antibodies, or antigen binding portions thereof, and assessing the EGFR binding and/or neutralizing characteristics of those antibodies, or antigen binding portions thereof, may be used, including but not limited to those specifically described herein.

In certain embodiments, the antibody comprises a heavy chain constant region, such as an IgG1, IgG2, IgG3, IgG4, IgA, IgE, IgM, or IgD constant region. In certain embodiments, the anti-EGFR antibody comprises a heavy chain immunoglobulin constant domain selected from the group consisting of a human IgG constant domain, a human IgM constant domain, a human IgE constant domain, and a human IgA constant domain. In further embodiments, the antibody, or antigen binding portion thereof, has an IgG1 heavy chain constant region, an IgG2 heavy chain constant region, an IgG3 constant region, or an IgG4 heavy chain constant region. Preferably, the heavy chain constant region is an IgG1 heavy chain constant region or an IgG4 heavy chain constant region. Furthermore, the antibody can comprise a light chain constant region, either a kappa light chain constant region or a lambda light chain constant region. In one embodiment, the antibody comprises a kappa light chain constant region.

In certain embodiments, the anti-EGFR antibody is a multispecific antibody, e.g. a bispecific antibody.

In certain embodiments, the anti-EGFR antibody comprises a heavy chain constant region comprising the amino acid sequence set forth in SEQ ID NO: 41 and/or a light chain constant region comprising the amino acid sequence set forth in SEQ ID NO: 43.

Replacements of amino acid residues in the Fc portion to alter antibody effector function are have been described (Winter, et al. US Patent Nos. 5,648,260 and 5,624,821). The Fc portion of an antibody mediates several important effector functions e.g. cytokine induction, ADCC, phagocytosis, complement dependent cytotoxicity (CDC) and half-life/ clearance rate of antibody and antigen-antibody complexes. In some cases these effector functions are desirable for therapeutic antibody but in other cases might be unnecessary or even deleterious, depending on the therapeutic objectives. Certain human IgG isotypes, particularly IgG1 and IgG3, mediate ADCC and CDC via binding to FcyRs and complement C1q, respectively. Neonatal Fc receptors (FcRn) are the critical components determining the circulating half-life of antibodies. In still another embodiment at least one amino acid residue is replaced in the constant region of the antibody, for example the Fc region of the antibody, such that effector functions of the antibody are altered.

One embodiment of the disclosure includes a labeled anti-EGFR antibody where the antibody is derivatized or linked to one or more functional molecule(s) (e.g., another peptide or protein) in addition to the Bcl-xL inhibitors described below. For example, a labeled antibody can be derived by functionally linking an antibody or antibody portion of the disclosure (by chemical coupling, genetic fusion, noncovalent association or otherwise) to one or more other molecular entities, such as another antibody (e.g., a bispecific antibody or a diabody), a detectable agent, a pharmaceutical agent, a protein or peptide that can mediate the association of the antibody or antibody portion with another molecule (such as a streptavidin core region or a polyhistidine tag), and/or a cytotoxic or therapeutic agent selected from the group consisting of a mitotic inhibitor, an antitumor antibiotic, an immunomodulating agent, a vector for gene therapy, an alkylating agent, an antiangiogenic agent, an antimetabolite, a boron-containing agent, a chemoprotective agent, a hormone, an antihormone agent, a corticosteroid, a photoactive therapeutic agent, an oligonucleotide, a radionuclide agent, a topoisomerase inhibitor, a kinase inhibitor, a radiosensitizer, and a combination thereof.

Useful detectable agents with which an antibody or ADC may be derivatized include fluorescent compounds. Exemplary fluorescent detectable agents include fluorescein, fluorescein isothiocyanate, rhodamine, 5-dimethylamine-1-napthalenesulfonyl chloride, phycoerythrin and the like. An antibody may also be derivatized with detectable enzymes, such as alkaline phosphatase, horseradish peroxidase, glucose oxidase and the like. When an antibody is derivatized with a detectable enzyme, it is detected by adding additional reagents that the enzyme uses to produce a detectable reaction product. For example, when the detectable agent horseradish peroxidase is present the addition of hydrogen peroxide and diaminobenzidine leads to a colored reaction product, which is detectable. An antibody may also be derivatized with biotin, and detected through indirect measurement of avidin or streptavidin binding.

In one embodiment, the antibody or ADC is conjugated to an imaging agent. Examples of imaging agents that may be used in the compositions and methods described herein include, but are not limited to, a radiolabel (e.g., indium), an enzyme, a fluorescent label, a luminescent label, a bioluminescent label, a magnetic label, and biotin.

In one embodiment, the antibodies are linked to a radiolabel, such as, but not limited to, indium (¹¹¹In). ¹¹¹Indium may be used to label the antibodies and ADCs described herein for use in identifying EGFR positive tumors. In a certain embodiment, anti-EGFR antibodies (or ADCs) described herein are labeled with ¹¹¹I via a bifunctional chelator which is a bifunctional cyclohexyl diethylenetriaminepentaacetic acid (DTPA) chelate (see US Patent Nos. 5,124,471; 5,434,287; and 5,286,850).

Another embodiment of the disclosure provides a glycosylated binding protein wherein the anti-EGFR antibody comprises one or more carbohydrate residues. Nascent *in vivo* protein production may undergo further processing, known as post-translational modification. In particular, sugar (glycosyl) residues may be added enzymatically, a process known as glycosylation. The resulting proteins bearing covalently linked oligosaccharide side chains are known as glycosylated proteins or glycoproteins. Antibodies are glycoproteins with one or more carbohydrate residues in the Fc domain, as well as the variable domain. Carbohydrate residues in the Fc domain have important effect on the effector function of the Fc domain, with minimal effect on antigen binding or half-life of the antibody (R. Jefferis, Biotechnol. Prog. 21 (2005), pp. 11-16). In contrast, glycosylation of the variable domain may have an effect on the antigen binding activity of the antibody. Glycosylation in the variable domain may have a negative effect on antibody binding affinity, likely due to steric hindrance (Co, M.S., et al., Mol. Immunol. (1993) 30:1361- 1367), or result in increased affinity for the antigen (Wallick, S.C., et al., Exp. Med. (1988) 168:1099-1109; Wright, A., et al., EMBO J. (1991) 10:2717-2723).

One aspect of the disclosure is directed to generating glycosylation site mutants in which the O- or N-linked glycosylation site of the binding protein has been mutated. One skilled in the art can generate such mutants using standard well-known technologies. Glycosylation site mutants that retain the biological activity, but have increased or decreased binding activity, are another object of the disclosure.

In still another embodiment, the glycosylation of the anti-EGFR antibody is modified. For example, an aglycoslated antibody can be made (*i.e.,* the antibody lacks glycosylation). Glycosylation can be altered to, for example, increase the affinity of the antibody for antigen. Such carbohydrate modifications can be accomplished by, for example, altering one or more sites of glycosylation within the antibody sequence. For example, one or more amino acid substitutions can be made that result in elimination of one or more variable region glycosylation sites to thereby eliminate glycosylation at that site. Such aglycosylation may increase the affinity of the antibody for antigen. Such an approach is described in further detail in PCT Publication WO2003016466A2, and U.S. Pat. Nos. 5,714,350 and 6,350,861.

Additionally or alternatively, a modified anti-EGFR antibody can be made that has an altered type of glycosylation, such as a hypofucosylated antibody having reduced amounts of fucosyl residues or an antibody having increased bisecting GlcNAc structures. Such altered glycosylation patterns have been demonstrated to increase the ADCC ability of antibodies. Such carbohydrate modifications can be accomplished by, for example, expressing the antibody in a host cell with altered glycosylation machinery. Cells with altered glycosylation machinery have been described in the art and can be used as host cells in which to express recombinant antibodies of the disclosure to thereby produce an antibody with altered glycosylation. See, for example, Shields, R. L. et al. (2002) J. Biol. Chem. 277:26733-26740; Umana et al. (1999) Nat. Biotech. 17:176-1, as well as, European Patent No: EP 1,176,195; PCT Publications WO 03/035835; WO 99/54342.

Protein glycosylation depends on the amino acid sequence of the protein of interest, as well as the host cell in which the protein is expressed. Different organisms may produce different glycosylation enzymes (e.g., glycosyltransferases and glycosidases), and have different substrates (nucleotide sugars) available. Due to such factors, protein glycosylation pattern, and composition of glycosyl residues, may differ depending on the host system in which the particular protein is expressed. Glycosyl residues useful in the disclosure may include, but are not limited to, glucose, galactose, mannose, fucose, n-acetylglucosamine and sialic acid. Preferably the glycosylated binding protein comprises glycosyl residues such that the glycosylation pattern is human.

Differing protein glycosylation may result in differing protein characteristics. For instance, the efficacy of a therapeutic protein produced in a microorganism host, such as yeast, and glycosylated utilizing the yeast endogenous pathway may be reduced compared to that of the same protein expressed in a mammalian cell, such as a CHO cell line. Such glycoproteins may also be immunogenic in humans and show reduced half-life *in vivo* after administration. Specific receptors in humans and other animals may recognize specific glycosyl residues and promote the rapid clearance of the protein from the bloodstream. Other adverse effects may include changes in protein folding, solubility, susceptibility to proteases, trafficking, transport, compartmentalization, secretion, recognition by other proteins or factors, antigenicity, or allergenicity. Accordingly, a practitioner may prefer a therapeutic protein with a specific composition and pattern of glycosylation, for example glycosylation composition and pattern identical, or at least similar, to that produced in human cells or in the species-specific cells of the intended subject animal.

Expressing glycosylated proteins different from that of a host cell may be achieved by genetically modifying the host cell to express heterologous glycosylation enzymes. Using recombinant techniques, a practitioner may generate antibodies or antigen binding portions thereof exhibiting human protein glycosylation. For example, yeast strains have been genetically modified to express non-naturally occurring glycosylation enzymes such that glycosylated proteins (glycoproteins) produced in these yeast strains exhibit protein glycosylation identical to that of animal cells, especially human cells (U.S. patent Publication Nos. 20040018590 and 20020137134 and PCT publication WO2005100584 A2).

Antibodies may be produced by any of a number of techniques. For example, expression from host cells, wherein expression vector(s) encoding the heavy and light chains is (are) transfected into a host cell by standard techniques. The various forms of the term "transfection" are intended to encompass a wide variety of techniques commonly used for the introduction of exogenous DNA into a prokaryotic or eukaryotic host cell, *e.g.,* electroporation, calcium-phosphate precipitation, DEAE-dextran transfection and the like. Although it is possible to express antibodies in either prokaryotic or eukaryotic host cells, expression of antibodies in eukaryotic cells is preferable, and most preferable in mammalian host cells, because such eukaryotic cells (and in particular mammalian cells) are more likely than prokaryotic cells to assemble and secrete a properly folded and immunologically active antibody.

Preferred mammalian host cells for expressing the recombinant antibodies of the disclosure include Chinese Hamster Ovary (CHO cells) (including dhfr- CHO cells, described in Urlaub and Chasin, (1980) Proc. Natl. Acad. Sci. USA 77:4216-4220, used with a DHFR selectable marker, *e.g.,* as described in R.J. Kaufman and P.A. Sharp (1982) Mol. Biol. 159:601-621), NS0 myeloma cells, COS cells and SP2 cells. When recombinant expression vectors encoding antibody genes are introduced into mammalian host cells, the antibodies are produced by culturing the host cells for a period of time sufficient to allow for expression of the antibody in the host cells or, more preferably, secretion of the antibody into the culture medium in which the host cells are grown. Antibodies can be recovered from the culture medium using standard protein purification methods.

Host cells can also be used to produce functional antibody fragments, such as Fab fragments or scFv molecules. It will be understood that variations on the above procedure are within the scope of the disclosure. For example, it may be desirable to transfect a host cell with DNA encoding functional fragments of either the light chain and/or the heavy chain of an antibody of this disclosure. Recombinant DNA technology may also be used to remove some, or all, of the DNA encoding either or both of the light and heavy chains that is not necessary for binding to the antigens of interest. The molecules expressed from such truncated DNA molecules are also encompassed by the antibodies of the disclosure. In addition, bifunctional antibodies may be produced in which one heavy and one light chain are an antibody of the disclosure and the other heavy and light chain are specific for an antigen other than the antigens of interest by crosslinking an antibody of the disclosure to a second antibody by standard chemical crosslinking methods.

In a preferred system for recombinant expression of an antibody, or antigen binding portion thereof, a recombinant expression vector encoding both the antibody heavy chain and the antibody light chain is introduced into dhfr- CHO cells by calcium phosphate-mediated transfection. Within the recombinant expression vector, the antibody heavy and light chain genes are each operatively linked to CMV enhancer/AdMLP promoter regulatory elements to drive high levels of transcription of the genes. The recombinant expression vector also carries a DHFR gene, which allows for selection of CHO cells that have been transfected with the vector using methotrexate selection/amplification. The selected transformant host cells are cultured to allow for expression of the antibody heavy and light chains and intact antibody is recovered from the culture medium. Standard molecular biology techniques are used to prepare the recombinant expression vector, transfect the host cells, select for transformants, culture the host cells and recover the antibody from the culture medium. Still further the disclosure provides a method of synthesizing a recombinant antibody of the disclosure by culturing a host cell in a suitable culture medium until a recombinant antibody is synthesized. Recombinant antibodies of the disclosure may be produced using nucleic acid molecules corresponding to the amino acid sequences disclosed herein. In one embodiment, the nucleic acid molecules set forth in SEQ ID NOs: 86 and/or 87 are used in the production of a recombinant antibody. The method can further comprise isolating the recombinant antibody from the culture medium.

The antibodies and the sequences of the antibodies recited herein are also described in US Patent No. 9,493,568 (AbbVie Inc.).

### 3. Anti-EGFR Antibody Drug Conjugates (ADCs): Bcl-xL Inhibitors and Linkers

Dysregulated apoptotic pathways have also been implicated in the pathology of cancer. The implication that down-regulated apoptosis (and more particularly the Bcl-2 family of proteins) is involved in the onset of cancerous malignancy has revealed a novel way of targeting this still elusive disease. Research has shown, for example, the anti-apoptotic proteins, Bcl 2 and Bcl-xL, are overexpressed in many cancer cell types. See, Zhang, 2002, Nature Reviews/Drug Discovery 1:101; Kirkin et al., 2004, Biochimica Biophysica Acta 1644:229-249; and Amundson et al., 2000, Cancer Research 60:6101-6110. The effect of this deregulation is the survival of altered cells which would otherwise have undergone apoptosis in normal conditions. The repetition of these defects associated with unregulated proliferation is thought to be the starting point of cancerous evolution.

Aspects of the disclosure concern anti-hEGFR ADCs comprising an anti-hEGFR antibody conjugated to a drug via a linker, wherein the drug is a Bcl-xL inhibitor. In specific embodiments, the ADCs are compounds according to structural formula (I) below, or a pharmaceutically acceptable salt thereof, wherein Ab represents the anti-hEGFR antibody, D represents a Bcl-xL inhibitor drug (i.e., a compound of formula (IIa), (IIb), (IIc), or (IId) as shown below), L represents a linker, LK represents a covalent linkage linking the linker (L) to the anti-hEGFR antibody (Ab) and m represents the number of D-L-LK units linked to the antibody, which is an integer ranging from 1 to 20. In some embodiments, m ranges from 1 to 8, 1 to 7, 1 to 6, 2 to 6, 1 to 5, 1 to 4, 2 to 4, or 1 to 3. In certain embodiments, m is 2, 3 or 4.

In some embodiments, the ADC has the following formula (formula I): 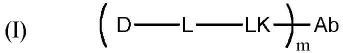 wherein Ab is the antibody, e.g., anti-EGFR antibody AbA, AbB, AbG, or AbK, and (D-L-LK) is a Drug-Linker-Covalent Linkage. The Drug-Linker moiety is made of L- which is a Linker, and -D, which is a drug moiety having, for example, cytostatic, cytotoxic, or otherwise therapeutic activity against a target cell, *e.g.,* a cell expressing EGFR; and m is an integer from 1 to 20. In some embodiments, m ranges from 1 to 8, 1 to 7, 1 to 6, 2 to 6, 1 to 5, 1 to 4, 1 to 3, 1 to 2, 1.5 to 8, 1.5 to 7, 1.5 to 6, 1.5 to 5, 1.5 to 4, 2 to 6, 1 to 5, 1 to 4, 1 to 3, 1 to 2, or 2 to 4. The DAR of an ADC is equivalent to the "m" referred to in Formula I. In one embodiment, the ADC has a formula of Ab-(LK-L-D)*ₘ*, wherein Ab is an anti-EGFR antibody, e.g. AbA, AbB, AbG, or AbK, L is a linker, D is a drug, *e.g.,* a Bcl-xL inhibitor, LK is a covalent linker, e.g. -S-, and m is 1 to 8 (or a DAR of 2-4). Additional details regarding drugs (D of Formula I) and linkers (L of Formula I) that may be used in the ADCs of the disclosure, as well as alternative ADC structures, are described below.

Specific embodiments of various Bcl-xL inhibitors per se, and various Bcl-xL inhibitors (D), linkers (L) and anti-EGFR antibodies (Ab) that can comprise the ADCs described herein, as well as the number of Bcl-xL inhibitors linked to the ADCs, are described in more detail below.

Examples of Bcl-xL inhibitors that may be used in the anti-EGFR ADC of the disclosure are provided below, as are linkers that may be used to conjugate the antibody and the one or more Bcl-xL inhibitor(s). The terms "linked" and "conjugated" are also used interchangeably herein and indicate that the antibody and moiety are covalently linked.

Bcl-xL inhibitors and linkers that may be used in the ADCs described herein and methods of making the same, are described in US 2016-0339117 (AbbVie Inc.).

### 3.1. Bcl-xL Inhibitors

One aspect of the instant disclosure concerns Bcl-xL inhibitors that have low cell permeability. The compounds are generally heterocyclic in nature and include one or more solubilizing groups that impart the compounds with high water solubility and low cell permeability. The solubilizing groups are generally groups that are capable of hydrogen bonding, forming dipole-dipole interactions, and/or that include a polyethylene glycol polymer containing from 1 to 30 units, one or more polyols, one or more salts, or one or more groups that are charged at physiological pH.

The Bcl-xL inhibitors may be used as compounds or salts *per se* in the various methods described herein, or may be included as a component part of an ADC.

Specific embodiments of Bcl-xL inhibitors that may be used in unconjugated form, or that may be included as part of an ADC include compounds according to structural formulae (IIa), (IIb), (IIc), or (IId). In the present disclosure, when the Bcl-xL inhibitors are included as part of an ADC, # shown in structural formula (IIa), (IIb), (IIc), or (IId) below represents a point of attachment to a linker, which indicates that they are represented in a monoradical form. 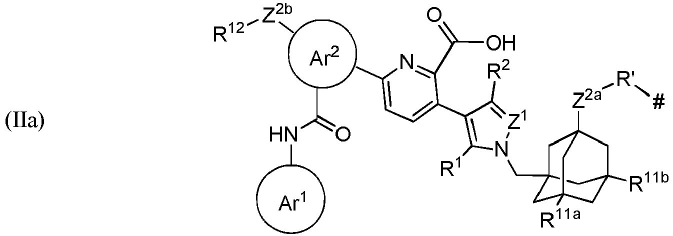 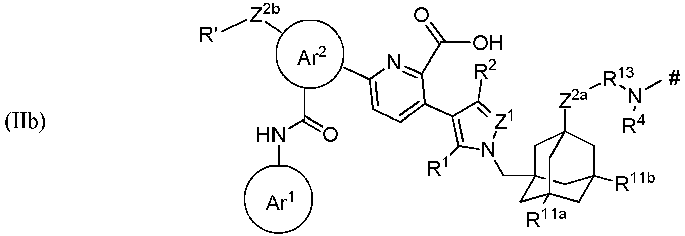 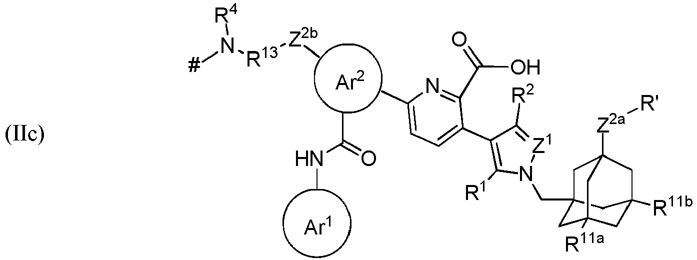 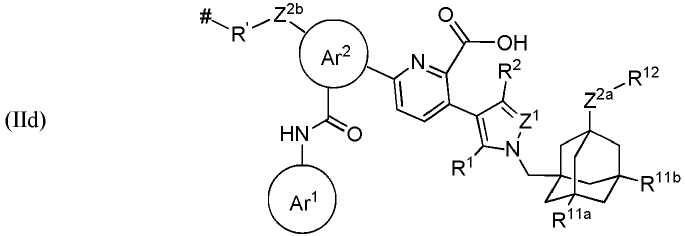 or a pharmaceutically acceptable salt thereof, wherein:
Ar¹ is selected from 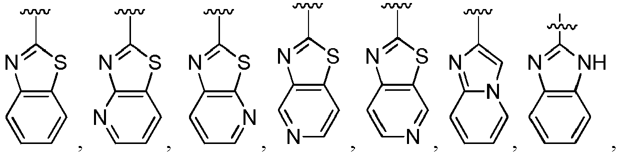 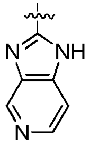 and 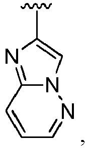 and is optionally substituted with one or more substituents independently selected from halo, hydroxy, nitro, lower alkyl, lower heteroalkyl, C₁₋₄alkoxy, amino, cyano and halomethyl;
Ar² is selected from 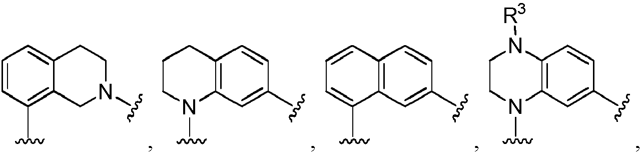 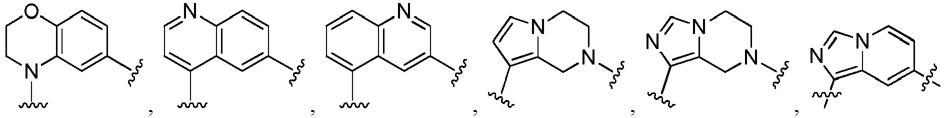 and is optionally substituted with one or more substituents independently selected from halo, hydroxy, nitro, lower alkyl, lower heteroalkyl, C₁₋₄alkoxy, amino, cyano and halomethyl, wherein the R¹²-Z^{2b}-, R'-Z^{2b}-, #-N(R⁴)-R¹³-Z^{2b}-, or #-R'-Z^{2b}- substituents are attached to Ar² at any Ar² atom capable of being substituted;
Z¹ is selected from N, CH, C-halo, C-CH₃ and C-CN;
Z^{2a} and Z^{2b}are each, independently from one another, selected from a bond, NR⁶, CR^{6a}R^{6b}, O, S, S(O), S(O)₂, -NR⁶C(O)-,-NR^{6a}C(O)NR^{6b}-, and -NR⁶C(O)O-;
R' is a alkylene, heteroalkylene, cycloalkylene, heterocyclene, aryl or heteroaryl independently substituted at one or more carbon or heteroatoms with a solubilizing moiety containing a group selected from a polyol, a polyethylene glycol containing from 4 to 30 ethylene glycol units, a salt, and a group that is charged at physiological pH and combinations thereof, wherein #, where attached to R', is attached to R' at any R' atom capable of being substituted;
R¹ is selected from hydrogen, methyl, halo, halomethyl, ethyl, and cyano;
R² is selected from hydrogen, methyl, halo, halomethyl and cyano;
R³ is selected from hydrogen, methyl, ethyl, halomethyl and haloethyl;
R⁴ is selected from hydrogen, lower alkyl and lower heteroalkyl or is taken together with an atom of R¹³ to form a cycloalkyl or heterocyclyl ring having between 3 and 7 ring atoms;
R⁶, R^{6a} and R^{6b} are each, independent from one another, selected from hydrogen, optionally substituted lower alkyl, optionally substituted lower heteroalkyl, optionally substituted cycloalkyl and optionally substituted heterocyclyl, or are taken together with an atom from R⁴ and an atom from R¹³ to form a cycloalkyl or heterocyclyl ring having between 3 and 7 ring atoms;
R^{11a} and R^{11b} are each, independently of one another, selected from hydrogen, halo, methyl, ethyl, halomethyl, hydroxyl, methoxy, CN, and SCH₃;
R¹² is optionally R' or is selected from hydrogen, halo, cyano, optionally substituted alkyl, optionally substituted heteroalkyl, optionally substituted heterocyclyl, and optionally substituted cycloalkyl;
R¹³ is selected from optionally substituted C₁₋₈ alkylene, optionally substituted heteroalkylene, optionally substituted heterocyclene, and optionally substituted cycloalkylene; and
# represents the point of attachment to a linker L.

One embodiment of Bcl-xL inhibitors that may be used in unconjugated form, or that may be included as part of an ADC include compounds according to structural formulae (IIa), (IIb), (IIc), or (IId): 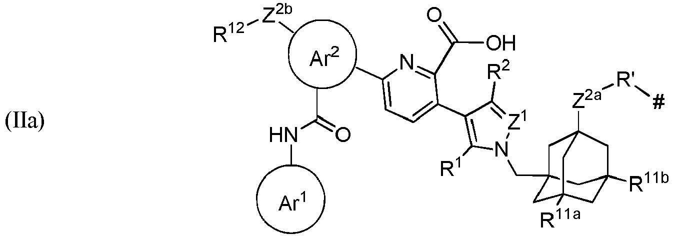 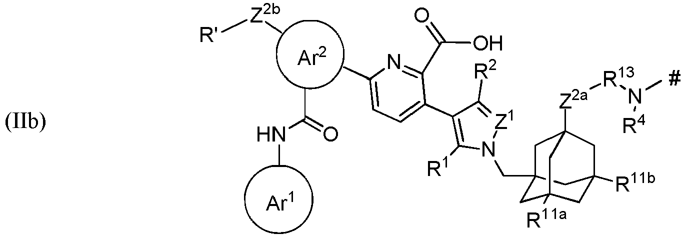 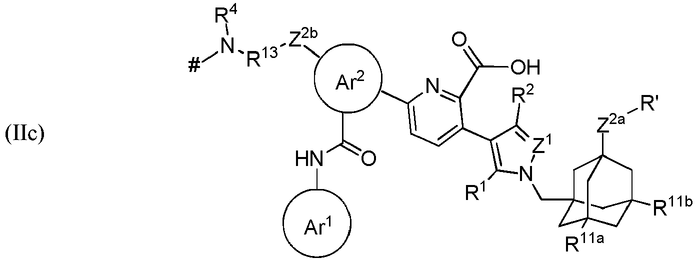 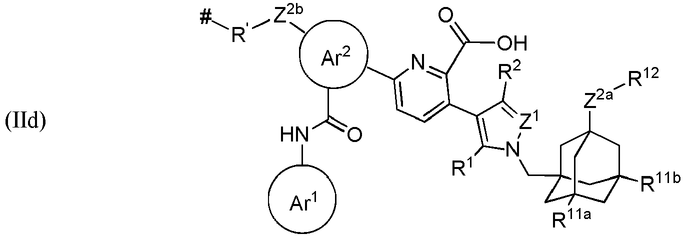 or a pharmaceutically acceptable salt thereof, wherein:
Ar¹ is selected from 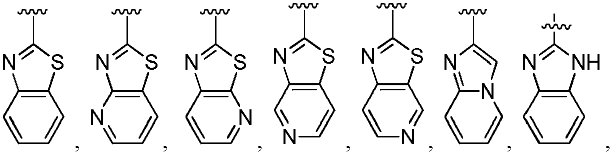 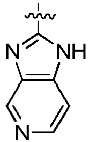 and 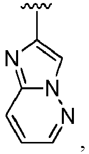 and is optionally substituted with one or more substituents independently selected from halo, hydroxy, nitro, lower alkyl, lower heteroalkyl, C₁₋₄alkoxy, amino, cyano and halomethyl;
Ar² is selected from 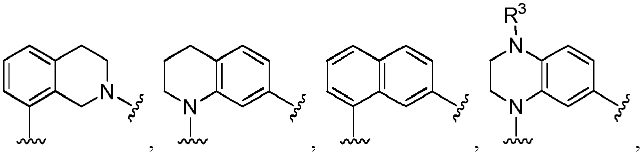 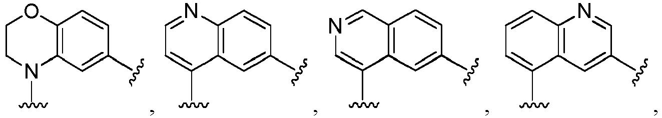 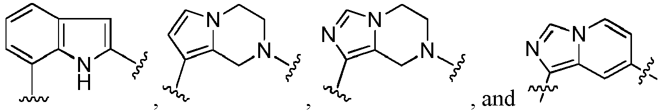 or an N-oxide thereof, and is optionally substituted with one or more substituents independently selected from halo, hydroxy, nitro, lower alkyl, lower heteroalkyl, C₁₋₄alkoxy, amino, cyano and halomethyl, wherein the R¹²-Z^{2b}-, R'-Z^{2b}-, #-N(R⁴)-R¹³-Z^{2b}-, or #-R'-Z^{2b}- substituents are attached to Ar² at any Ar² atom capable of being substituted;
Z¹ is selected from N, CH, C-halo, C-CH₃ and C-CN;
Z^{2a} and Z^{2b}are each, independently from one another, selected from a bond, NR⁶, CR^{6a}R^{6b}, O, S, S(O), S(O)₂, -NR⁶C(O)-,-NR^{6a}C(O)NR^{6b}-, and -NR⁶C(O)O-;
R' is 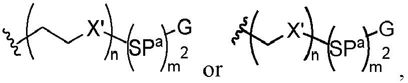 wherein #, where attached to R', is attached to R' at any R' atom capable of being substituted;
X' is selected at each occurrence from -N(R¹⁰)-, -N(R¹⁰)C(O)-, -N(R¹⁰)S(O)₂-, -S(O)₂N(R¹⁰)-, and -O-;
n is selected from 0-3;
R¹⁰ is independently selected at each occurrence from hydrogen, lower alkyl, heterocycle, aminoalkyl, G-alkyl, and -(CH₂)₂-O-(CH₂)₂-O-(CH₂)₂-NH₂;
G at each occurrence is independently selected from a polyol, a polyethylene glycol with between 4 and 30 repeating units, a salt and a moiety that is charged at physiological pH;
SP^{a} is independently selected at each occurrence from oxygen, -S(O)₂N(H)-, -N(H)S(O)₂-, -N(H)C(O)-, -C(O)N(H) -, -N(H)- , arylene, heterocyclene, and optionally substituted methylene; wherein methylene is optionally substituted with one or more of -NH(CH₂)₂G, NH₂, C₁₋₈alkyl, and carbonyl;
m² is selected from 0-12;
R¹ is selected from hydrogen, methyl, halo, halomethyl, ethyl, and cyano;
R² is selected from hydrogen, methyl, halo, halomethyl and cyano;
R³ is selected from hydrogen, methyl, ethyl, halomethyl and haloethyl;
R⁴ is selected from hydrogen, lower alkyl and lower heteroalkyl or is taken together with an atom of R¹³ to form a cycloalkyl or heterocyclyl ring having between 3 and 7 ring atoms;
R⁶, R^{6a} and R^{6b} are each, independent from one another, selected from hydrogen, optionally substituted lower alkyl, optionally substituted lower heteroalkyl, optionally substituted cycloalkyl and optionally substituted heterocyclyl, or are taken together with an atom from R⁴ and an atom from R¹³ to form a cycloalkyl or heterocyclyl ring having between 3 and 7 ring atoms;
R^{11a} and R^{11b} are each, independently of one another, selected from hydrogen, halo, methyl, ethyl, halomethyl, hydroxyl, methoxy, CN, and SCH₃;
R¹² is optionally R' or is selected from hydrogen, halo, cyano, optionally substituted alkyl, optionally substituted heteroalkyl, optionally substituted heterocyclyl, and optionally substituted cycloalkyl;
R¹³ is selected from optionally substituted C₁₋₈ alkylene, optionally substituted heteroalkylene, optionally substituted heterocyclene, and optionally substituted cycloalkylene; and
# represents the point of attachment to a linker L.

When a Bcl-xL inhibitor of structural formulae (IIa)-(IId) is not a component of an ADC, # in formulae (IIa)-(IId) represents the point of attachment to a hydrogen atom. When the Bcl-xL inhibitor is a component of an ADC, # in formulae (IIa)-(IId) represents the point of attachment to the linker. When a Bcl-xL inhibitor is a component of an ADC, the ADC may comprise one or more Bcl-xL inhibitors, which may be the same or different, but are typically the same.

In certain embodiments, R' is a C₂-C₈ heteroalkylene substituted with one or more moieties containing a salt and/or a group that is charged at physiological pH. The salt may be selected, for example, from the salt of a carboxylate, a sulfonate, a phosphonate, and an ammonium ion. For example, the salt may be the sodium or potassium salt of a carboxylate, sulfonate or phosphonate or the chloride salt of an ammonium ion. The group that is charged at physiological pH may be any group that is charged at a physiological pH, including, by way of example and not limitation, a zwitterionic group. In certain embodiments a group that is a salt is a dipolar moiety such as, but not limited to, N-oxides of amines including certain heterocyclyls such as, but not limited to, pyridine and quinoline. In specific embodiments the group that is charged at physiological pH is selected independently at each occurrence, from carboxylate, sulfonate, phosphonate, and amine.

In certain embodiments, R' is a C₂-C₈ heteroalkylene substituted with one or more moieties containing polyethylene glycol or a polyol such as a diol or a sugar moiety.

In certain embodiments, R' may be substituted with groups in addition to a solubilizing moiety. For example, R' may be substituted with one or more of the same or different alkyl, heteroalkyl, cycloalkyl, heterocyclyl, aryl, heteroaryl, or halo groups.

In certain embodiments, R' is represented by the formula: 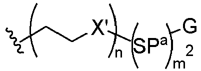 or a pharmaceutically acceptable salt thereof, wherein:
X' is selected at each occurrence from -N(R¹⁰)- and -O-;
n is selected from 1-3;
R¹⁰ is individually selected at each occurrence from hydrogen, alkyl, heterocycle, aminoalkyl, G-alkyl, heterocycle, and -(CH₂)₂-O-(CH₂)₂-O-(CH₂)₂-NH₂;
G at each occurrence is independently selected from a polyol, a polyethylene glycol with between 4 and 30 repeating unit (referred to herein as PEG4-30), a salt and a moiety that is charged at physiological pH;
SP^{a} is independently selected at each occurrence from oxygen, sulfonamide, arylene, heterocyclene, and optionally substituted methylene; wherein methylene is optionally substituted with one or more of
-NH(CH₂)₂G, amine and carbonyl; and
m² is selected from 0-6,
wherein there is at least one substitutable nitrogen in R' that is attached to a linker or a hydrogen atom at a substitutable nitrogen atom of R'.

In certain embodiments, R' is 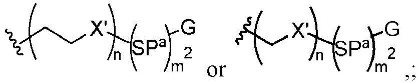
X' is selected at each occurrence from -N(R¹⁰)- , -N(R¹⁰)C(O)-, -N(R¹⁰)S(O)₂-, -S(O)₂N(R¹⁰)-, and -O-;
n is selected from 0-3;
R¹⁰ is independently selected at each occurrence from hydrogen, alkyl, heterocycle, aminoalkyl, G-alkyl, heterocycle, and -(CH₂)₂-O-(CH₂)₂-O-(CH₂)₂-NH₂;
G at each occurrence is independently selected from a polyol, a polyethylene glycol with between 4 and 30 repeating units, a salt and a moiety that is charged at physiological pH;
SP^{a} is independently selected at each occurrence from oxygen-S(O)₂N(H)-, -N(H)S(O)₂-, -N(H)C(O)-, -C(O)N(H)-, -N(H)- , arylene, heterocyclene, and optionally substituted methylene; wherein methylene is optionally substituted with one or more of -NH(CH₂)₂G, amine, alkyl, and carbonyl;
m² is selected from 0-12, and
#, where attached to R', is attached to R' at any R' atom capable of being substituted.

In certain embodiments, G at each occurrence is a salt or a moiety that is charged at physiological pH.

In certain embodiments, G at each occurrence is a salt of a carboxylate, a sulfonate, a phosphonate, or ammonium.

In certain embodiments, G at each occurrence is a moiety that is charged at physiological pH selected from the group consisting of carboxylate, a sulfonate, a phosphonate, and an amine.

In certain embodiments, G at each occurrence is a moiety containing a polyethylene glycol with between 4 and 30 repeating units, or a polyol.

In certain embodiments, the polyol is a sugar.

In certain embodiments, R' of formula (IIa) or (IId) includes at least one substitutable nitrogen suitable for attachment to a linker.

In certain embodiments, G is selected independently at each occurrence from: 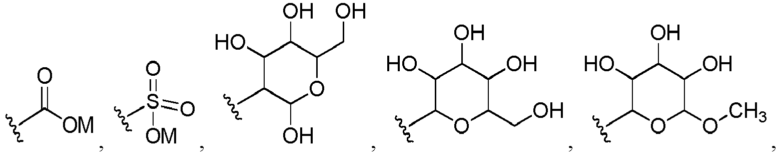 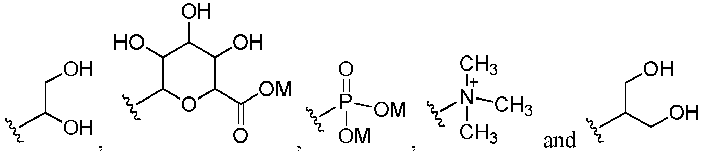 wherein M is hydrogen or a positively charged counterion. In certain embodiments, M is Na⁺, K⁺ or Li⁺. In certain embodiments, M is hydrogen. In particular embodiments, G is SO₃H.

In certain embodiments, G is selected independently at each occurrence from: 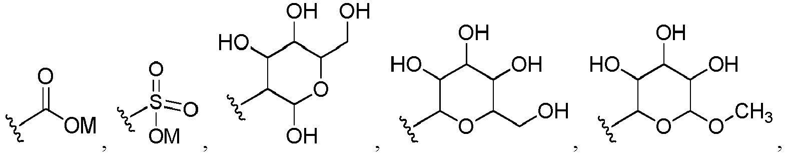 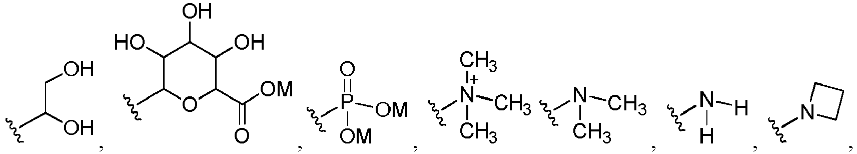 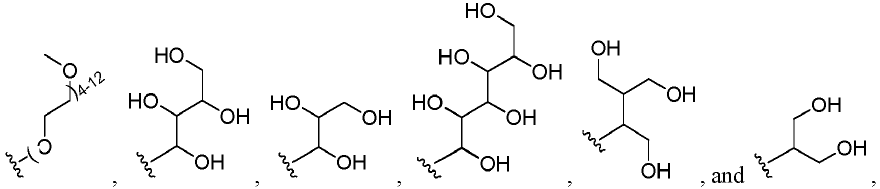 wherein M is hydrogen or a positively charged counterion. In certain embodiments, M is hydrogen. In particular embodiments, G is SO₃H.

In certain embodiments, R' is selected from: 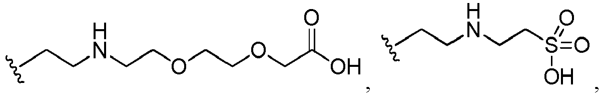 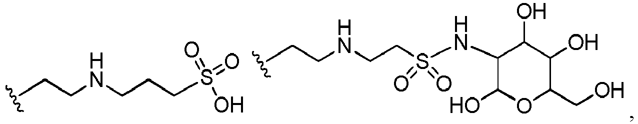 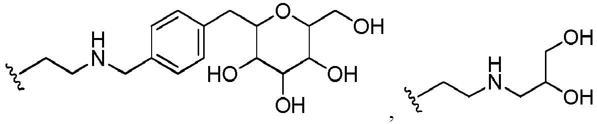 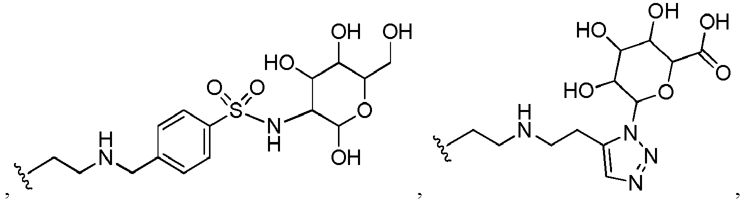 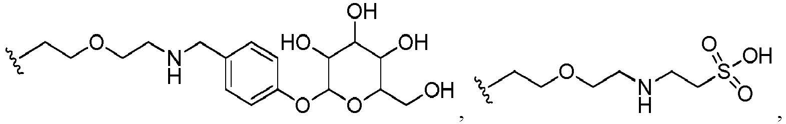 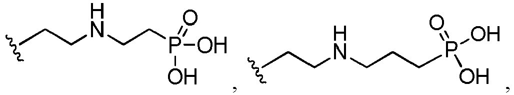 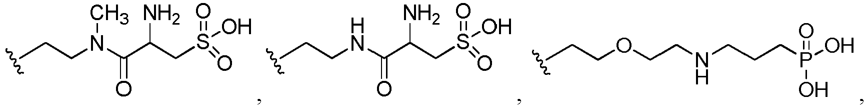 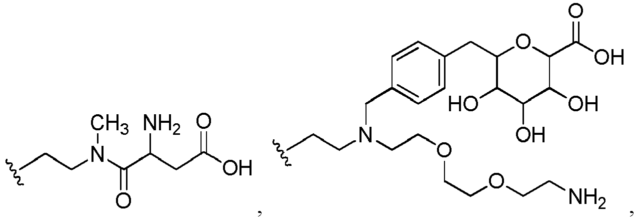 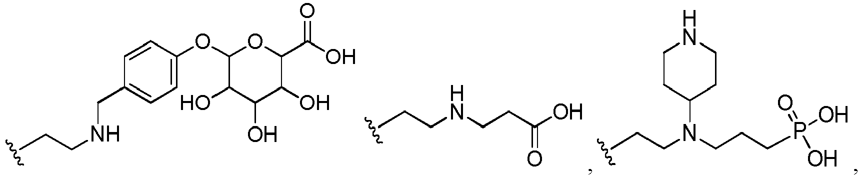 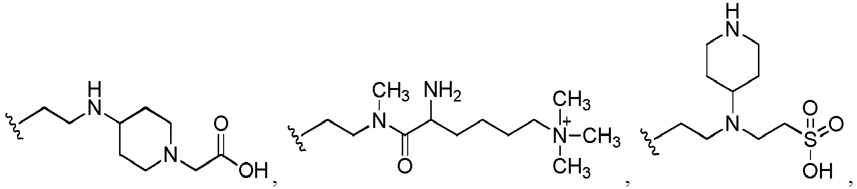 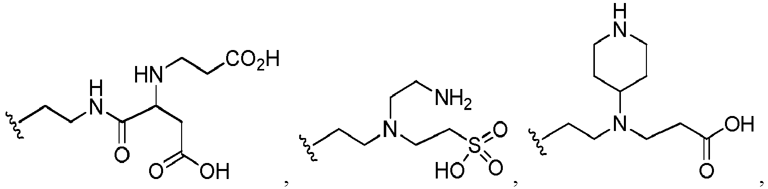 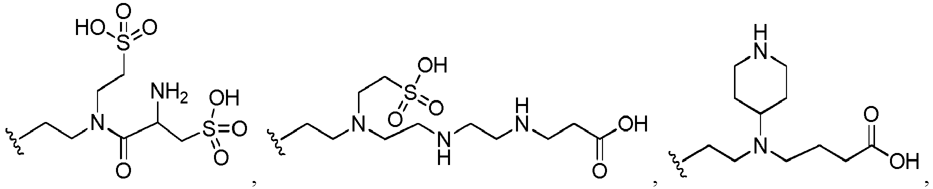 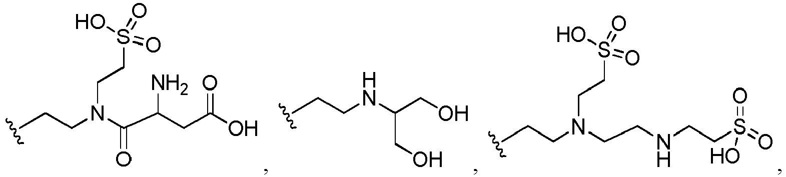 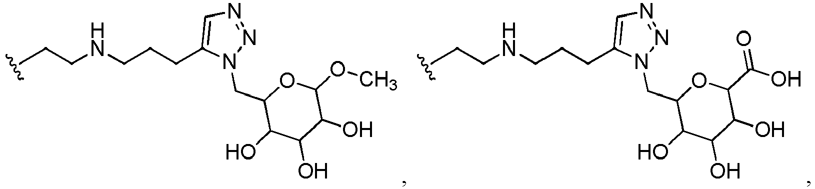 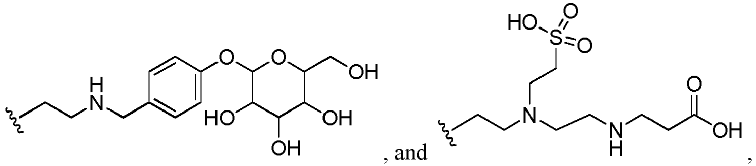 or a salt thereof. When Bcl-xL inhibitors of this embodiment are included in an ADC, the linker of the ADC is linked to the nitrogen atom of an available primary or secondary amine group.

In certain embodiments, R' is selected from: 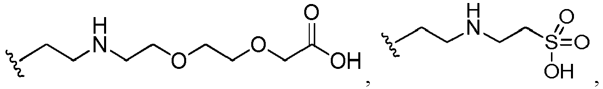 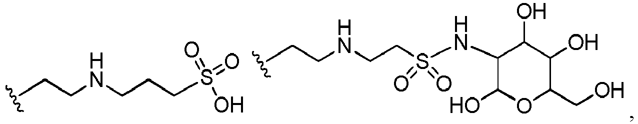 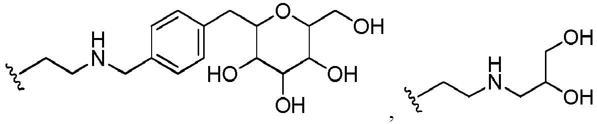 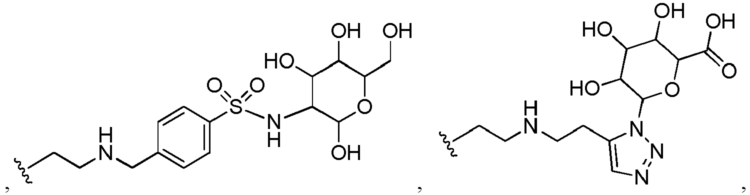 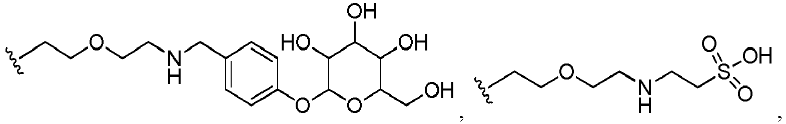 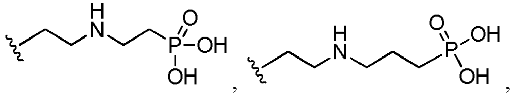 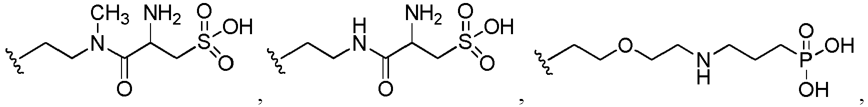 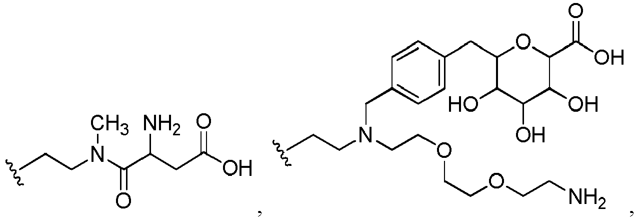 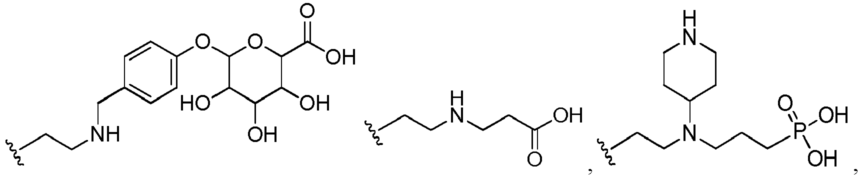 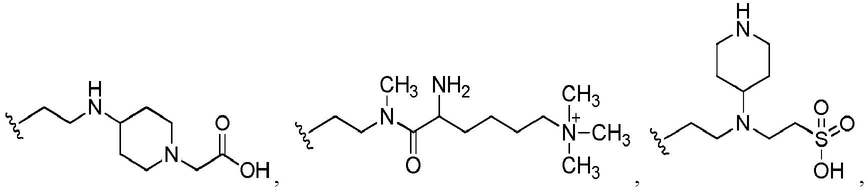 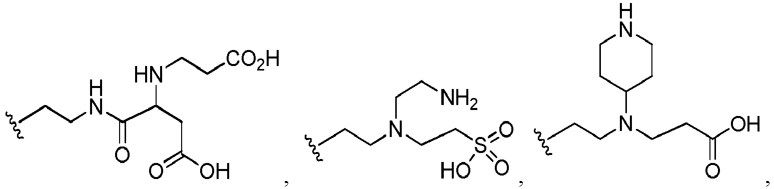 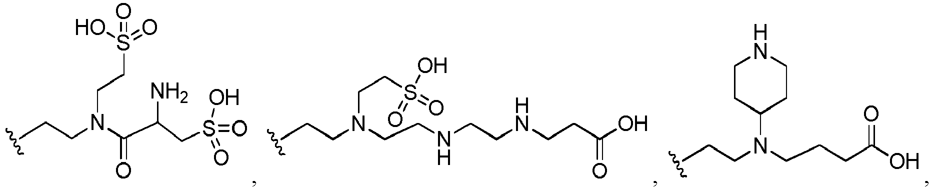 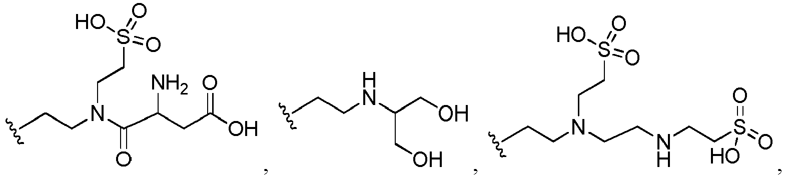 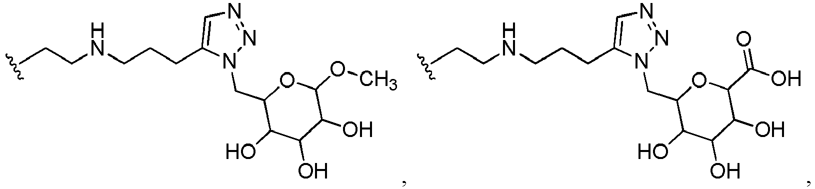 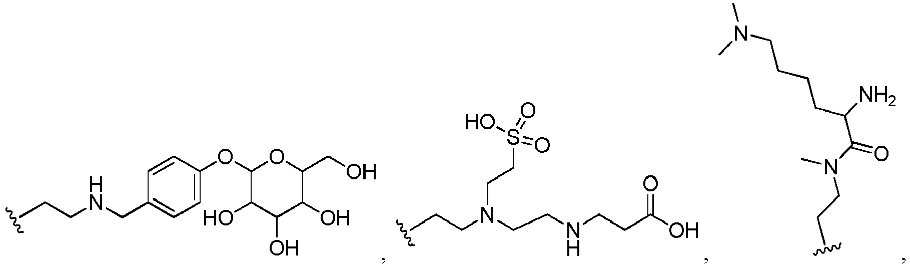 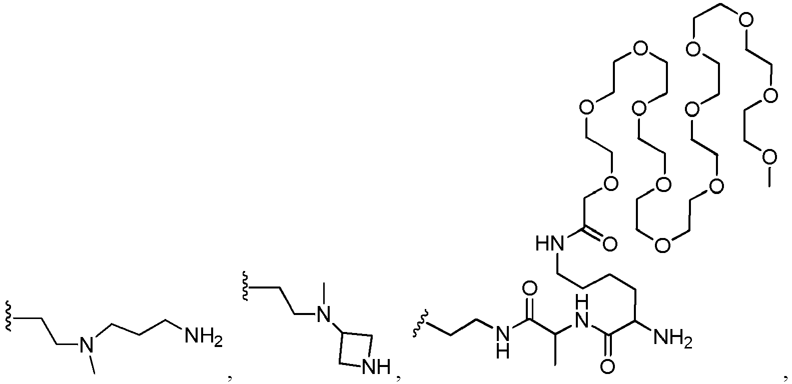 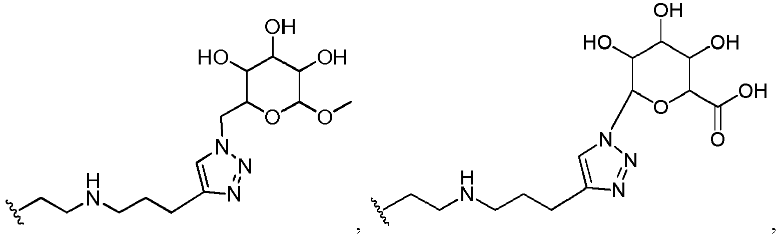 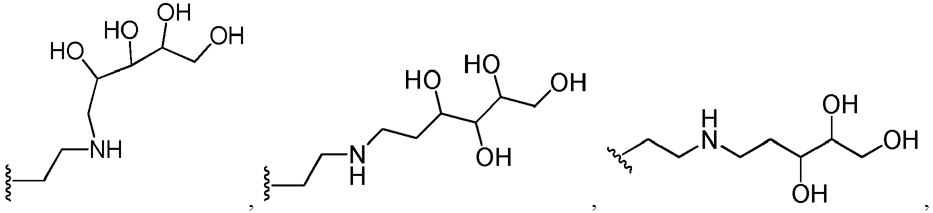 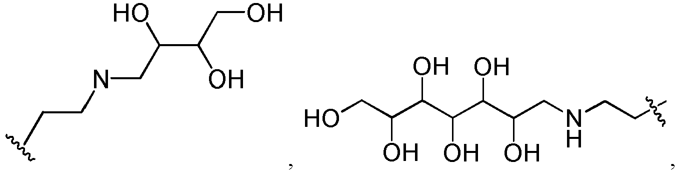 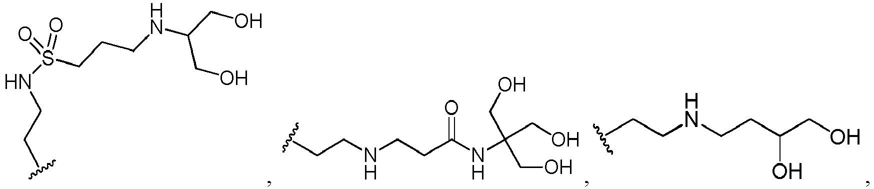 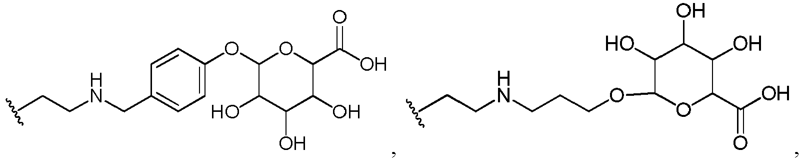 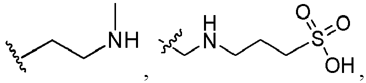 or a salt thereof. When Bcl-xL inhibitors of this embodiment are included in an ADC, the linker of the ADC is linked to the nitrogen atom of an available primary or secondary amine group.

In certain embodiments, R' is selected from 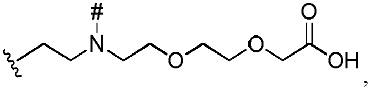 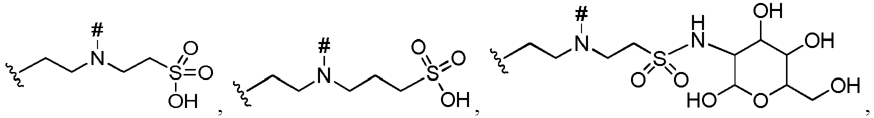 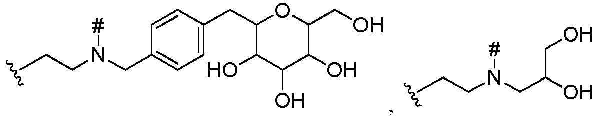 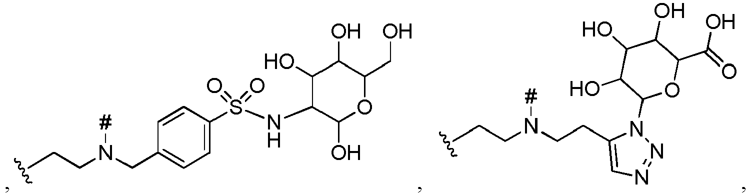 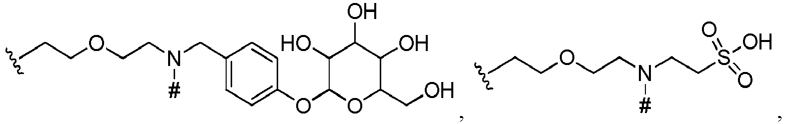 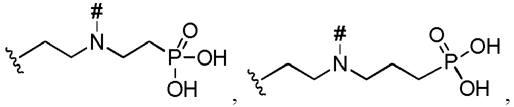 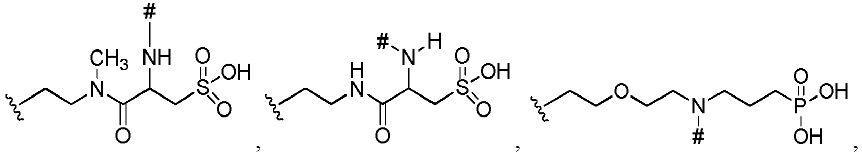 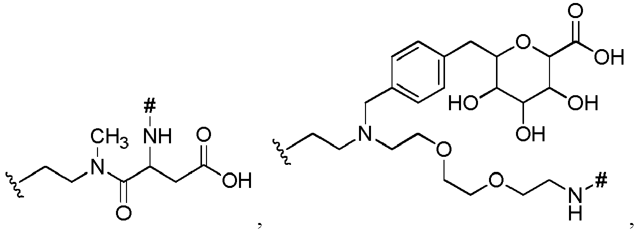 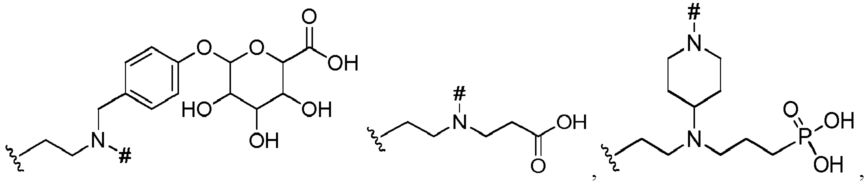 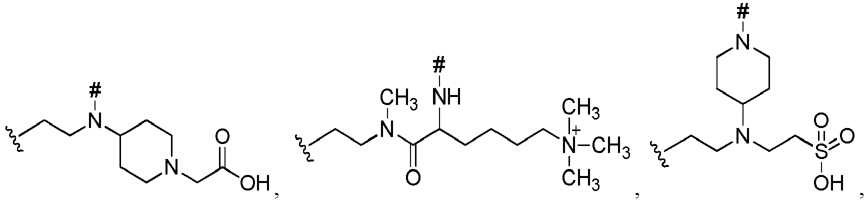 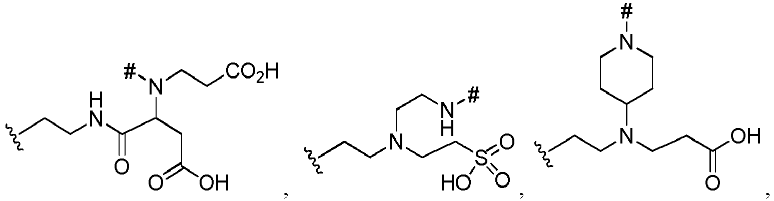 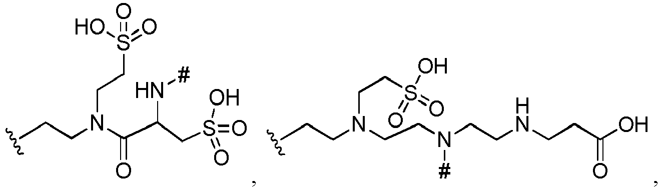 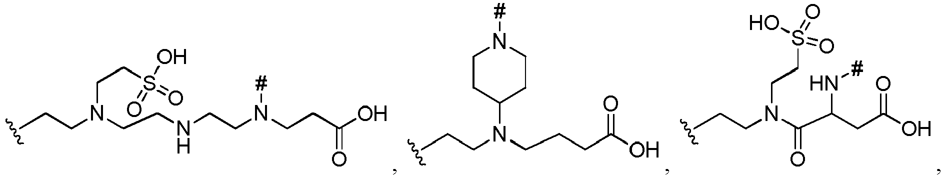 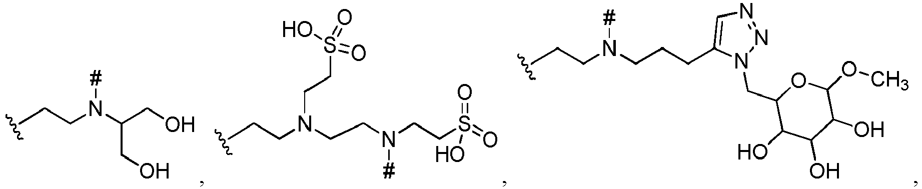 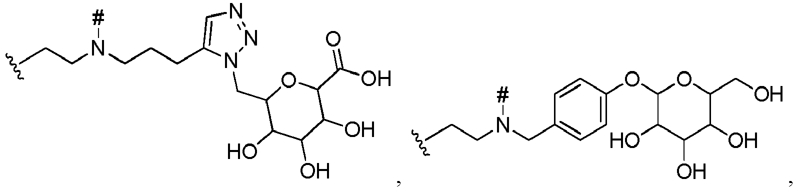 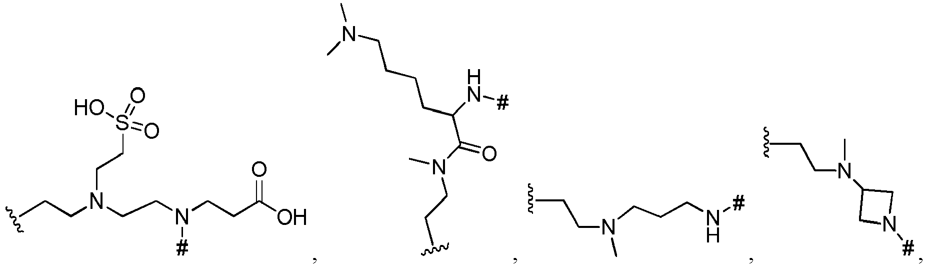 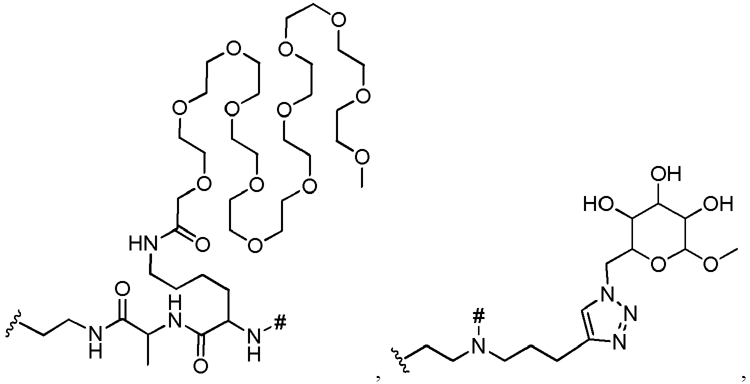 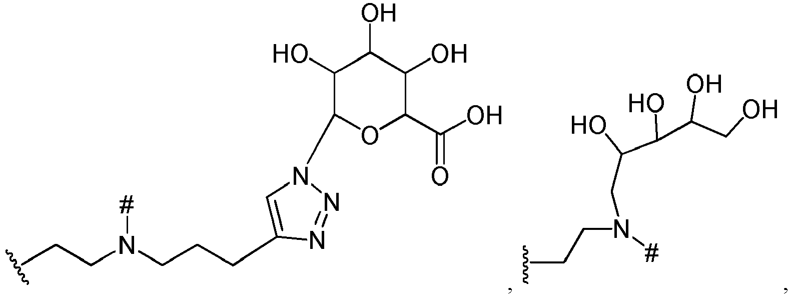 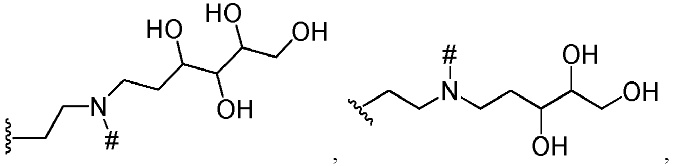 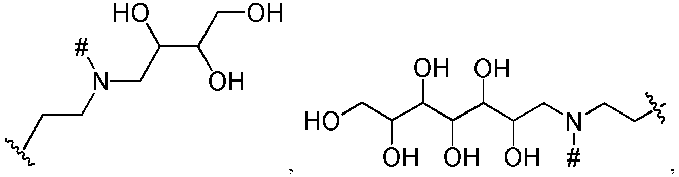 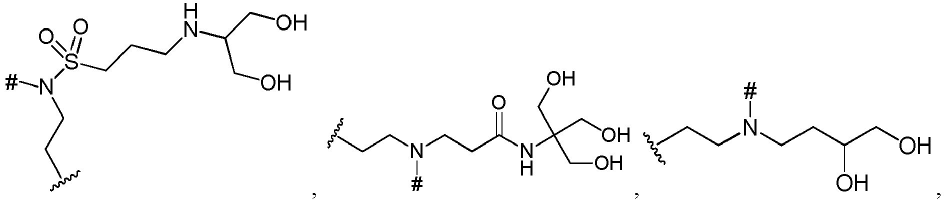 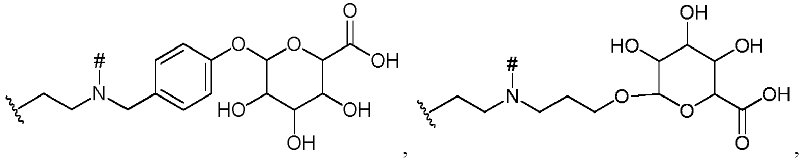 and 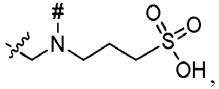 wherein # represents either a hydrogen atom in the Bcl-xL inhibitor drug of the ADCs of formula (IIb) or (IIc) or the point of attachment in the Bcl-xL inhibitor drug of the ADCs of formula (IIa) or (IId) to a linker L.

In certain embodiments, Ar¹ of formulae (IIa)-(IId) is selected from 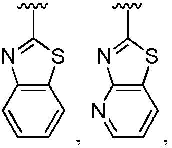 and 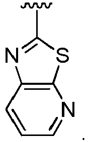 In certain embodiments, Ar¹ of formulae (IIa)-(IId) is selected from 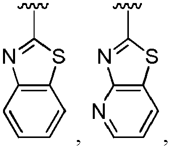 and 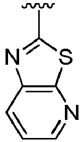 and is optionally substituted with one or more substituents independently selected from halo, cyano, methyl, and halomethyl. In particular embodiments, Ar¹ is

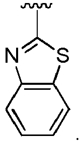

In certain embodiments, Ar² is 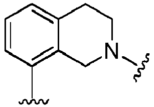 optionally substituted with one or more substituents, wherein the R¹²-Z^{2b}-, R'-Z^{2b}-, #-N(R⁴)-R¹³-Z^{2b}-, or #-R'-Z^{2b}- substituents are attached to Ar² at any Ar² atom capable of being substituted.

In certain embodiments, Ar² is selected from: 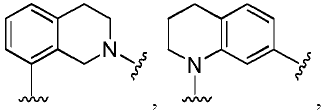 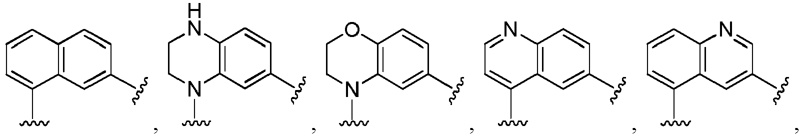 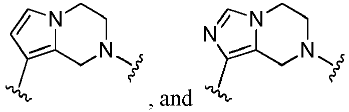 and is optionally substituted with one or more substituents, wherein the R¹²-Z^{2b}-, R'-Z^{2b}-, #-N(R⁴)-R¹³-Z^{2b}-, or #-R'-Z^{2b}- substituents are attached to Ar² at any Ar² atom capable of being substituted. In certain embodiments, Ar² is selected from: 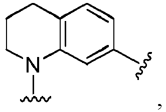 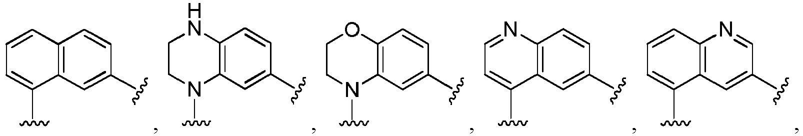 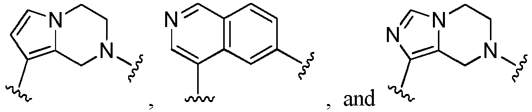 ; and is optionally substituted with one or more substituents, wherein the R¹²-Z^{2b}-, R'-Z^{2b}-, #-N(R⁴)-R¹³-Z^{2b}-, or #-R'-Z^{2b}- substituents are attached to Ar² at any Ar² atom capable of being substituted. In certain embodiments, Ar² is substituted with one or more solubilizing group. In certain embodiments, the each solubilizing group is, independently of the others, selected from a moiety containing a polyol, a polyethylene glycol with between 4 and 30 repeating units, a salt, or a moiety that is charged at physiological pH.

In certain embodiments, Z¹ of formulae (IIa)-(IId) is N.

In certain embodiments, Z^{2a} of formulae (IIa)-(IId) is O. In certain embodiments, Z^{2a} of formulae (IIa)-(IId) is CR^{6a}R^{6b}. In certain embodiments, Z^{2a} of formulae (IIa)-(IId) is S. In certain embodiments, Z^{2a} of formulae (IIa)-(IId) is -NR⁶C(O)-. In particular embodiments, R⁶ is hydrogen.

In certain embodiments, Z^{2b} of formulae (IIa)-(IId) is O. In certain embodiments, Z^{2b} of formulae (IIa)-(IId) is NH or CH₂.

In certain embodiments, R¹ of formulae (IIa)-(IId) is selected from methyl and chloro.

In certain embodiments, R² of formulae (IIa)-(IId) is selected from hydrogen and methyl. In particular embodiments, R² is hydrogen.

In certain embodiments the Bcl-xL inhibitor is a compound of formula (IIa). In certain embodiments in which the Bcl-xL inhibitor is a compound of formula (IIa), the compound has the structural formula (IIa.1), 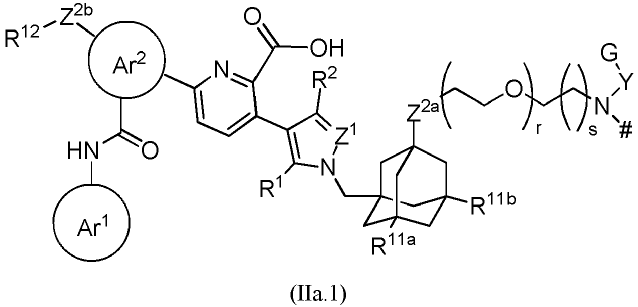 or salts thereof, wherein:
Ar¹, Ar², Z¹, Z^{2a}, Z^{2b}, R¹, R², R^{11a}, R^{11b}, R^{12,} G and # are defined as above;
Y is optionally substituted C₁-C₈ alkylene;
r is 0 or 1; and
s is 1, 2 or 3.

In certain embodiments in which the Bcl-xL inhibitor is a compound of formula (IIa.1), r is 0 and s is 1.

In certain embodiments in which the Bcl-xL inhibitor is a compound of formula (IIa.1), r is 0 and s is 2.

In certain embodiments in which the Bcl-xL inhibitor is a compound of formula (IIa.1), r is 1 and s is 2.

In certain embodiments in which the Bcl-xL inhibitor is a compound of formula (IIa.1), Z^{2a} is selected from O, NH, CH₂ and S. In particular embodiments, Z^{2a} is O. In certain embodiments, Z^{2a} of formula (IIa.1) is -CR^{6a}R^{6b}-. In certain embodiments, Z^{2a} of formula (IIa.1) is CH₂. In certain embodiments, Z^{2a} of formula (IIa.1) is S. In certain embodiments, Z^{2a} of formula (IIa.1) is -NR⁶C(O)-.

In certain embodiments in which the Bcl-xL inhibitor is a compound of formula (IIa.1), Y is selected from ethylene, propylene and butylene. In particular embodiments, Y is selected from ethylene and propylene.

In certain embodiments in which the Bcl-xL inhibitor is a compound of formula (IIa.1), G is selected from 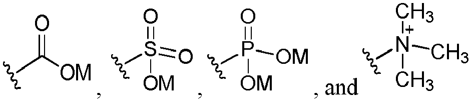 wherein M is hydrogen or a positively charged counterion. In particular embodiments, G is 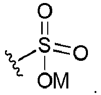 In particular embodiments, G is SO₃H.

In certain embodiments in which the Bcl-xL inhibitor is a compound of formula (IIa.1), Ar² is selected from 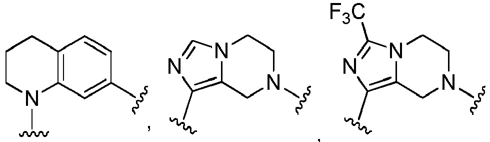 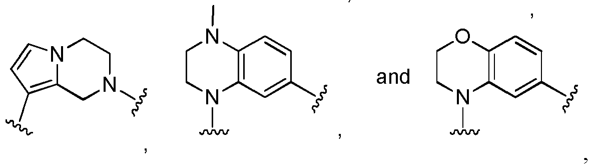 wherein the R¹²-Z^{2b}- substituent is attached to Ar² at any Ar² atom capable of being substituted.

In certain embodiments in which the Bcl-xL inhibitor is a compound of formula (IIa.1), Ar² is selected from 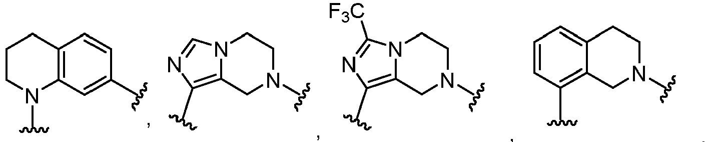 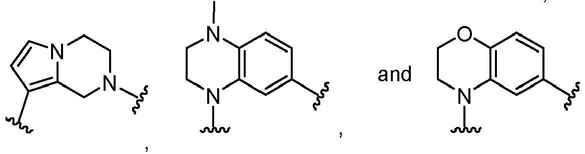 , wherein the R¹²-Z^{2b}-substituent is attached to Ar² at any Ar² atom capable of being substituted.

In particular embodiments in which the Bcl-xL inhibitor is a compound of formula (IIa.1), Ar² 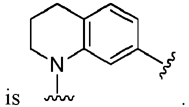 In particular embodiments in which the Bcl-xL inhibitor is a compound of formula (IIa.1), Ar² is

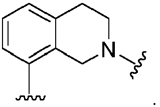

In certain embodiments in which the Bcl-xL inhibitor is a compound of formula (IIa.1), Z^{2b}-R¹² is selected from H, F, CN, OCH₃, OH, NH₂, OCH₂CH₂OCH₃, N(CH₃)C(=O)CH₃, CH₂N(CH₃)C(=O)CH₃SCH₃, C(=O)N(CH₃)₂ and OCH₂CH₂N(CH₃)(C(=O)CH₃). In particular embodiments, Z^{2b}-R¹² is selected from H, F and CN. In particular embodiments, Z^{2b}-R¹² is H.

In embodiments where Z^{2b}-R¹² is substituted with hydroxyl (OH), the oxygen can serve as the point of attachment to a linking group (*See* Section 4.4.1.1).

In certain embodiments in which the Bcl-xL inhibitor is a compound of formula (IIa.1), Ar¹ is

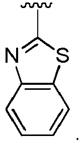

In certain embodiments in which the Bcl-xL inhibitor is a compound of formula (IIa.1), the group 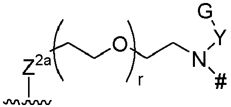 bonded to the adamantane ring is selected from:

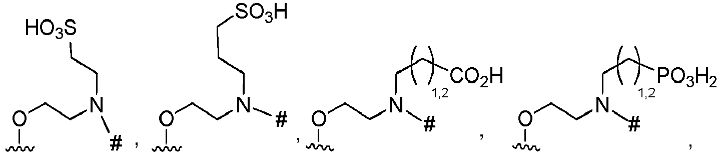

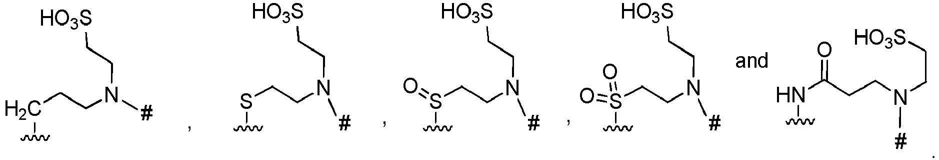

In certain embodiments, a compound of formula (IIa.1) may be converted into the compound of formula IIa.1.1, wherein n is selected from 1-3:

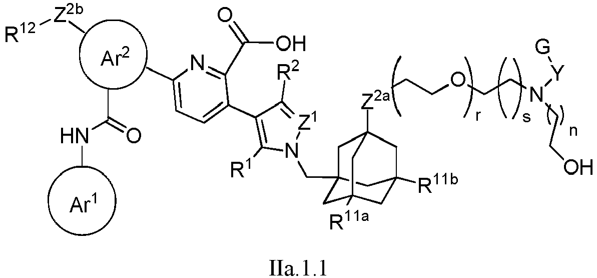

In certain embodiments, the compound of formula IIa.1.1 can be converted into a compound of formula IIa.1.2, wherein L represents a linker and LK represents a linkage formed between a reactive functional group on linker L and a complementary functional group on antibody.

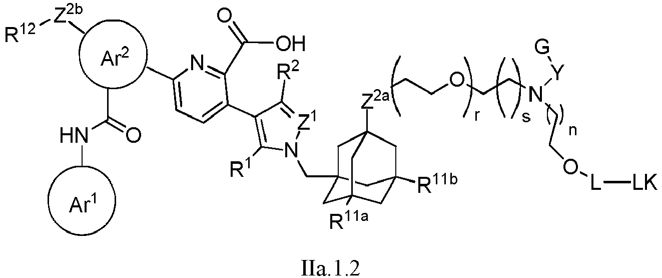

In certain embodiments in which the Bcl-xL inhibitor is a compound of formula (IIa), the compound has the structural formula (IIa.2), 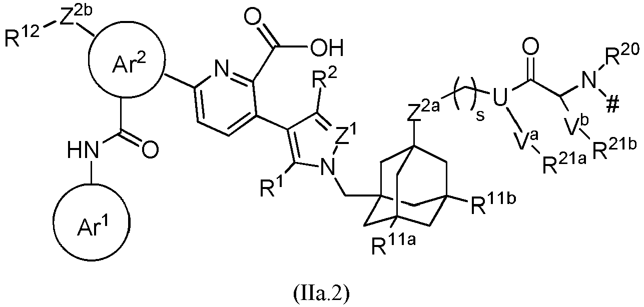 or salts thereof, wherein:
Ar¹, Ar², Z¹, Z^{2a}, Z^{2b}, R¹, R², R^{11a}, R^{11b}, R¹² and # are defined as above;
U is selected from N, O and CH, with the proviso that when U is O, then V^{a} and R^{21a} are absent;
   R²⁰ is selected from H and C₁-C₄ alkyl;
   R^{21a} and R^{21b} are each, independently from one another, absent or selected from H, C₁-C₄ alkyl and G, where G is selected from a polyol, PEG4-30, a salt and a moiety that is charged at physiological pH;
V^{a} and V^{b} are each, independently from one another, absent or selected from a bond, and an optionally substituted alkylene;
R²⁰ is selected from H and C₁-C₄ alkyl; and
s is 1, 2 or 3.

In certain embodiments in which the Bcl-xL inhibitor is a compound of formula (IIa.2), s is 2.

In certain embodiments in which the Bcl-xL inhibitor is a compound of formula (IIa.2), Z^{2a} is selected from O, NH, CH₂ and S. In particular embodiments, Z^{2a} is O. In certain embodiments, Z^{2a} of formula (IIa.2) is CR^{6a}R^{6b}. In certain embodiments, Z^{2a} of formula (IIa.2) is CH₂. In certain embodiments, Z^{2a} of formula (IIa.2) is S. In certain embodiments, Z^{2a} of formula (IIa.2) is -NR⁶C(O)-.

In certain embodiments in which the Bcl-xL inhibitor is a compound of formula (IIa.2), U is selected from N and O. In particular embodiments, U is O.

In certain embodiments in which the Bcl-xL inhibitor is a compound of formula (IIa.2), V^{a} is a bond, R^{21a} is a C₁-C₄ alkyl group, V^{b} is selected from methylene and ethylene and R^{21b} is G. In particular embodiments, V^{a} is a bond, R^{21a} is a methyl group and V^{b} is selected from methylene and ethylene and R^{21b} is G.

In certain embodiments in which the Bcl-xL inhibitor is a compound of formula (IIa.2), V^{a} is selected from methylene and ethylene, R^{21a} is G, V^{b} is selected from methylene and ethylene and R^{21b} is G. In particular embodiments, V^{a} is ethylene, R^{21a} is G, V^{b} is selected from methylene and ethylene and R^{21b} is G.

In certain embodiments in which the Bcl-xL inhibitor is a compound of formula (IIa.2), G is selected from 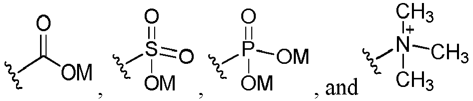 wherein M is hydrogen or a positively charged counterion. In particular embodiments, G is 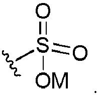 In particular embodiments, G is SO₃H.

In certain embodiments in which the Bcl-xL inhibitor is a compound of formula (IIa.2), R²⁰ is selected from hydrogen and a methyl group.

In certain embodiments in which the Bcl-xL inhibitor is a compound of formula (IIa.2), Ar² is selected from 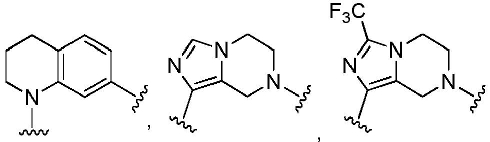 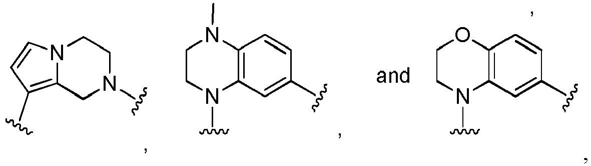 wherein the R¹²-Z^{2b}- substituent is attached to Ar² at any Ar² atom capable of being substituted.

In certain embodiments in which the Bcl-xL inhibitor is a compound of formula (IIa.2), Ar² is selected from 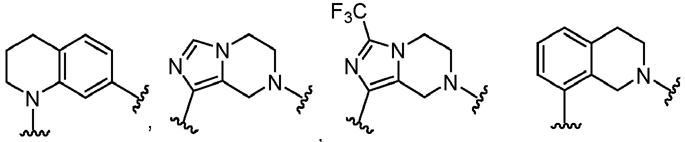 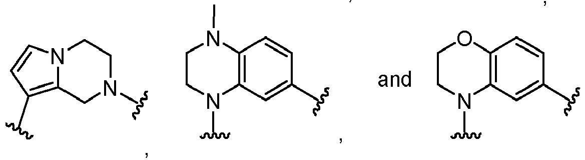 , , wherein the R¹²-Z^{2b}- substituent is attached to Ar² at any Ar² atom capable of being substituted. In particular embodiments in which the Bcl-xL inhibitor is a compound of formula (IIa.2), Ar² is 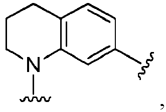 wherein the R¹²-Z^{2b}- substituent is attached to Ar² at any Ar² atom capable of being substituted.

In certain embodiments in which the Bcl-xL inhibitor is a compound of formula (IIa.2), Z^{2b}-R¹² is selected from H, F, CN, OCH₃, OH, NH₂, OCH₂CH₂OCH₃, N(CH₃)C(=O)CH₃, CH₂N(CH₃)C(=O)CH₃SCH₃, C(=O)N(CH₃)₂ and OCH₂CH₂N(CH₃)(C(=O)CH₃). In particular embodiments, Z^{2b}-R¹² is selected from H, F and CN. In particular embodiments, Z^{2b}-R¹² is H. In certain embodiments in which the Bcl-xL inhibitor is a compound of formula (IIa.2), Ar¹ is 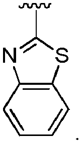 In particular embodiments in which the Bcl-xL inhibitor is a compound of formula (IIa.2), Ar² is 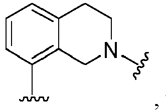 wherein the R¹²-Z^{2b}- substituent is attached to Ar² at any Ar² atom capable of being substituted.

In certain embodiments in which the Bcl-xL inhibitor is a compound of formula (IIa), the compound has the structural formula (IIa.3), 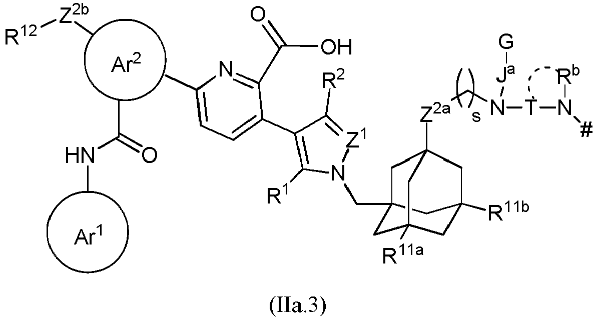 or salts thereof, wherein:
Ar¹, Ar², Z¹, Z^{2a}, Z^{2b}, R¹, R², R^{11a}, R^{11b}, R¹² and # are defined as above;
R^{b} is selected from H, C₁-C₄ alkyl and J^{b}-G or is optionally taken together with an atom of T to form a ring having between 3 and 7 atoms;
J^{a} and J^{b} are each, independently from one another, selected from optionally substituted C₁-C₈ alkylene and optionally substituted phenylene;
T is selected from optionally substituted C₁-C₈ alkylene, CH₂CH₂OCH₂CH₂OCH₂CH₂, CH₂CH₂OCH₂CH₂OCH₂CH₂OCH₂ and a polyethylene glycol containing from 4 to 10 ethylene glycol units;
G is selected from a polyol, PEG4-30, a salt and a moiety that is charged at physiological pH; and s is 1, 2 or 3.

In certain embodiments in which the Bcl-xL inhibitor is a compound of formula (IIa.3), s is 1. In certain embodiments in which the Bcl-xL inhibitor is a compound of formula (IIa.3), s is 2.

In certain embodiments in which the Bcl-xL inhibitor is a compound of formula (IIa.3), Z^{2a} is selected from O, CH₂ and S. In particular embodiments, Z^{2a} is O. In certain embodiments, Z^{2a} of formula (IIa.3) is CR^{6a}R^{6b}. In certain embodiments, Z^{2a} of formula (IIa.3) is CH₂. In certain embodiments, Z^{2a} of formula (IIa.3) is S. In certain embodiments, Z^{2a} of formula (IIa.3) is -NR⁶C(O)-

In certain embodiments in which the Bcl-xL inhibitor is a compound of formula (IIa.3), J^{a} is selected from methylene and ethylene and R^{b} is J^{b}-G, wherein J^{b} is methylene or ethylene. In some such embodiments, T is ethylene. In other such embodiments, T is CH₂CH₂OCH₂CH₂OCH₂CH₂. In other such embodiments, T is a polyethylene glycol containing from 4 to 10 ethylene glycol units.

In certain embodiments in which the Bcl-xL inhibitor is a compound of formula (IIa.3), J^{a} is selected from methylene and ethylene and R^{b} is taken together with an atom of T to form a ring having 4-6 ring atoms.

In certain embodiments in which the Bcl-xL inhibitor is a compound of formula (IIa.3), J^{a} is selected from methylene and ethylene and R^{b} is H or alkyl. In some such embodiments, T is ethylene. In other such embodiments, T is CH₂CH₂OCH₂CH₂OCH₂CH₂.

In certain embodiments in which the Bcl-xL inhibitor is a compound of formula (IIa.3), G is selected from 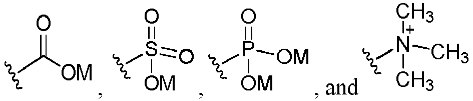 wherein M is hydrogen or a positively charged counterion. In particular embodiments, G is 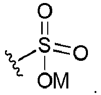 In particular embodiments, G is SO₃H.

In certain embodiments in which the Bcl-xL inhibitor is a compound of formula (IIa.3), R²⁰ is selected from hydrogen and a methyl group.

In certain embodiments in which the Bcl-xL inhibitor is a compound of formula (IIa.3), Ar² is selected from 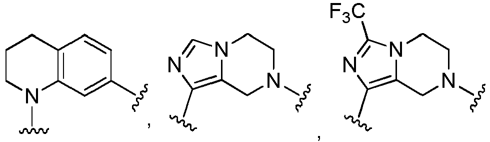 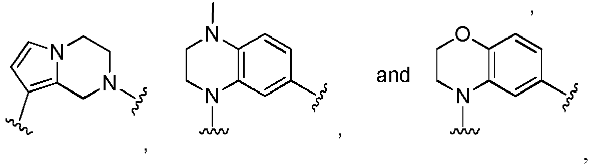 wherein the R¹²-Z^{2b}- substituent is attached to Ar² at any Ar² atom capable of being substituted.

In particular embodiments in which the Bcl-xL inhibitor is a compound of formula (IIa.3), Ar² is 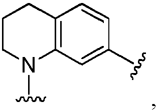 wherein the R¹²-Z^{2b}- substituent is attached to Ar² at any Ar² atom capable of being substituted. In certain embodiments in which the Bcl-xL inhibitor is a compound of formula (IIa.3), Ar² is selected from 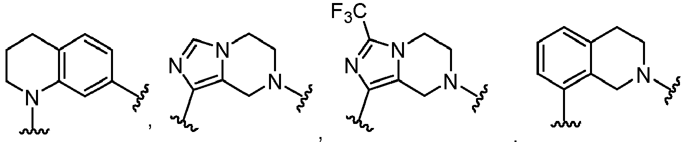 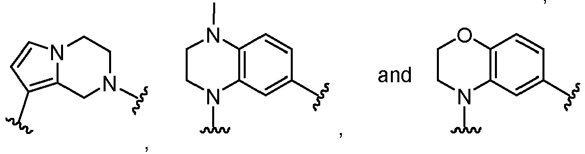 , , wherein the R¹²-Z^{2b}-substituent is attached to Ar² at any Ar² atom capable of being substituted. In particular embodiments in which the Bcl-xL inhibitor is a compound of formula (IIa.3), Ar² is 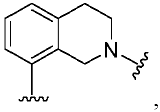 wherein the R¹²-Z^{2b}- substituent is attached to Ar² at any Ar² atom capable of being substituted.

In certain embodiments in which the Bcl-xL inhibitor is a compound of formula (IIa.3), Z^{2b}-R¹² is selected from H, F, CN, OCH₃, OH, NH₂, OCH₂CH₂OCH₃, N(CH₃)C(=O)CH₃, CH₂N(CH₃)C(=O)CH₃SCH₃, C(=O)N(CH₃)₂ and OCH₂CH₂N(CH₃)(C(=O)CH₃). In particular embodiments, Z^{2b}-R¹² is selected from H, F and CN. In particular embodiments, Z^{2b}-R¹² is H.

In certain embodiments in which the Bcl-xL inhibitor is a compound of formula (IIa.3), Ar¹ is

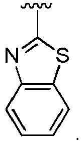

In certain embodiments in which the Bcl-xL inhibitor is a compound of formula (IIa.3), the group 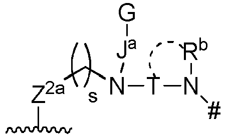 is selected from:

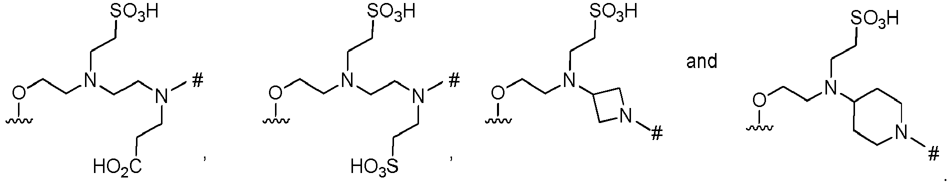

In certain embodiments in which the Bcl-xL inhibitor is a compound of formula (IIa.3), the group 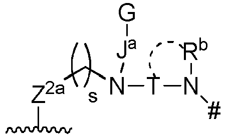 is selected from:

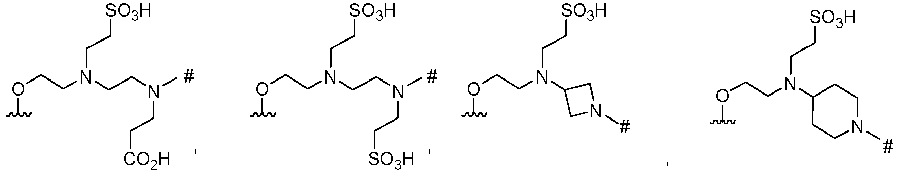

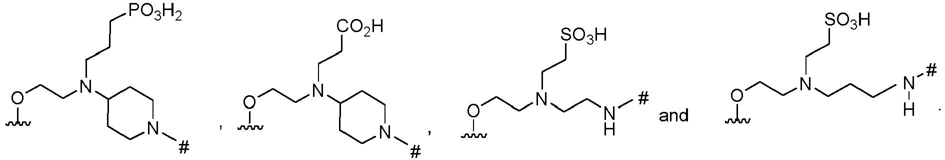

In certain embodiments the Bcl-xL inhibitor is a compound of formula (IIb). In certain embodiments in which the Bcl-xL inhibitor is a compound of formula (IIb), the compound has the structural formula (IIb.1), 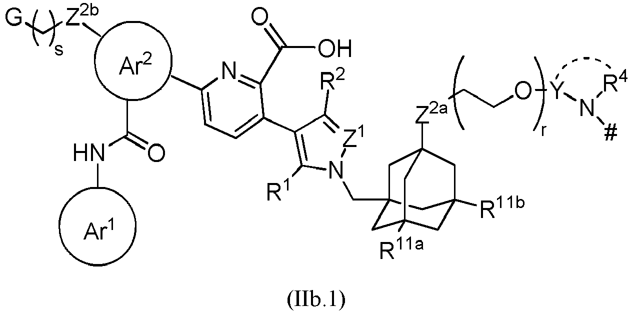 or salts thereof, wherein:
Ar¹, Ar², Z¹, Z^{2a}, Z^{2b}, R¹, R², R⁴, R^{11a}, R^{11b} and # are defined as above;
Y is optionally substituted C₁-C₈ alkylene;
G is selected from a polyol, PEG4-30, a salt and a moiety that is charged at physiological pH;
r is 0 or 1; and
s is 1, 2 or 3.

In certain embodiments in which the Bcl-xL inhibitor is a compound of formula (IIb.1), s is 1. In certain embodiments in which the Bcl-xL inhibitor is a compound of formula (IIb.1), s is 2. In certain embodiments in which the Bcl-xL inhibitor is a compound of formula (IIb.1), s is 3.

In certain embodiments in which the Bcl-xL inhibitor is a compound of formula (IIb.1), Z^{2a} is selected from O, CH₂, NH and S. In particular embodiments, Z^{2a} is O. In certain embodiments, Z^{2a} of formula (IIb.1) is CR^{6a}R^{6b}. In certain embodiments, Z^{2a} of formula (IIb.1) is CH₂. In certain embodiments, Z^{2a} of formula (IIb.1) is S. In certain embodiments, Z^{2a} of formula (IIb.1) is - NR⁶C(O)-.

In certain embodiments in which the Bcl-xL inhibitor is a compound of formula (IIb.1), Z^{2b} is selected from O, CH₂, NH, NCH₃ and S. In particular embodiments, Z^{2b} is O. In particular embodiments, Z^{2b} is NH. In particular embodiments, Z^{2b} is NCH₃.

In certain embodiments in which the Bcl-xL inhibitor is a compound of formula (IIb.1), Y is ethylene and r is 0.

In certain embodiments in which the Bcl-xL inhibitor is a compound of formula (IIb.1), Y is ethylene and r is 1.

In certain embodiments in which the Bcl-xL inhibitor is a compound of formula (IIb.1), R⁴ is H or methyl. In particular embodiments, R⁴ is methyl. In other embodiments, R⁴ is H.

In certain embodiments in which the Bcl-xL inhibitor is a compound of formula (IIb.1), R⁴ is taken together with an atom of Y to form a ring having 4-6 ring atoms. In particular embodiments, the ring is a cyclobutane ring. In other embodiments, the ring is a piperazine ring. In other embodiments, the ring is a morpholine ring.

In certain embodiments in which the Bcl-xL inhibitor is a compound of formula (IIb.1), G is selected from 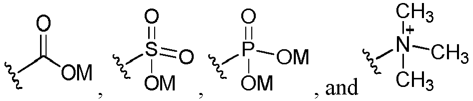 wherein M is hydrogen or a positively charged counterion. In particular embodiments, G is 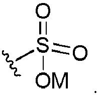 In other embodiments, G is SO₃H. In particular embodiments, G is NH₂. In other embodiments, G is PO₃H₂. In particular embodiments, G is NH₂. In particular embodiments, G is C(O)OH. In particular embodiments, G is polyol.

In certain embodiments in which the Bcl-xL inhibitor is a compound of formula (IIb.1), Ar² is selected from 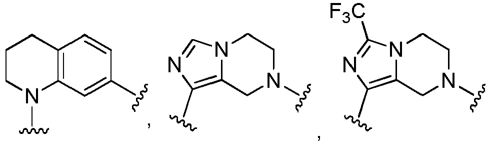 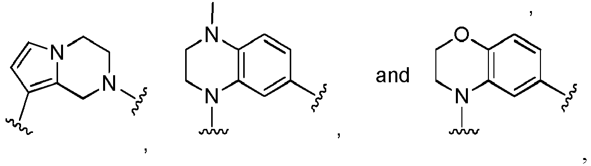 wherein the G-(CH₂)ₛ-Z^{2b}- substituent is attached to Ar² at any Ar² atom capable of being substituted.

In particular embodiments in which the Bcl-xL inhibitor is a compound of formula (IIb.1), Ar² is 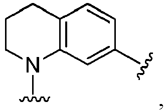 wherein the G-(CH₂)ₛ-Z^{2b}- substituent is attached to Ar² at any Ar² atom capable of being substituted. In certain embodiments in which the Bcl-xL inhibitor is a compound of formula (IIb.1), Ar² is selected from 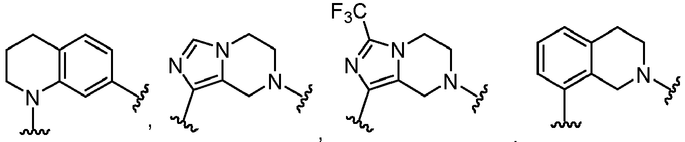 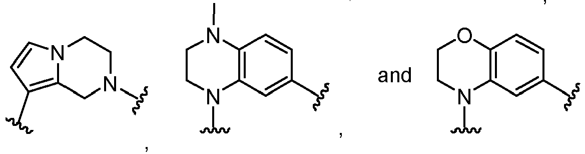 , , wherein the G-(CH₂)ₛ-Z^{2b}- substituent is attached to Ar² at any Ar² atom capable of being substituted. In particular embodiments in which the Bcl-xL inhibitor is a compound of formula (IIb.1), Ar² is 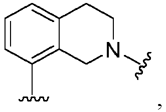 wherein the G-(CH₂)ₛ-Z^{2b}- substituent is attached to Ar² at any Ar² atom capable of being substituted.

In certain embodiments in which the Bcl-xL inhibitor is a compound of formula (IIb.1), Ar¹ is

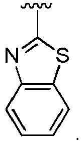

In certain embodiments in which the Bcl-xL inhibitor is a compound of formula (IIb.1), the group 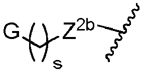 is selected from:

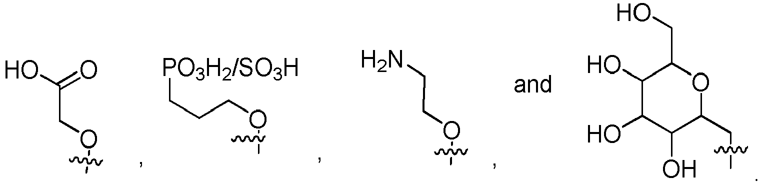

In certain embodiments in which the Bcl-xL inhibitor is a compound of formula (IIb.1), the group 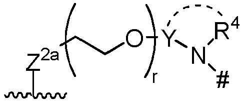 is selected from:

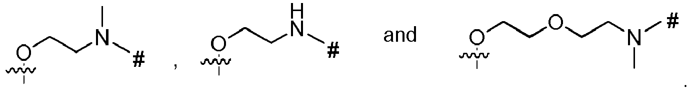

In certain embodiments in which the Bcl-xL inhibitor is a compound of formula (IIb.1), the group 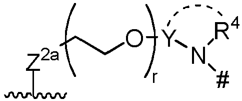 is selected from:

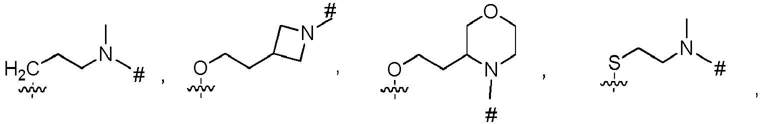

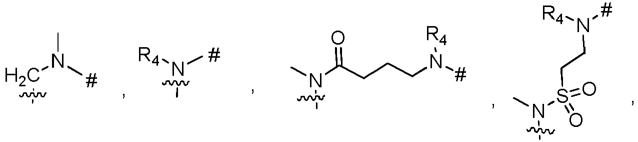

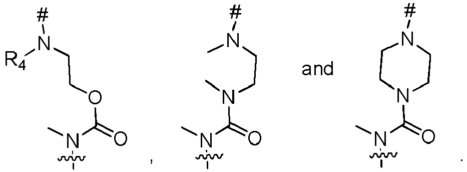

In certain embodiments the Bcl-xL inhibitor is a compound of formula (IIc). In certain embodiments in which the Bcl-xL inhibitor is a compound of formula (IIc), the compound has the structural formula (IIc.1) 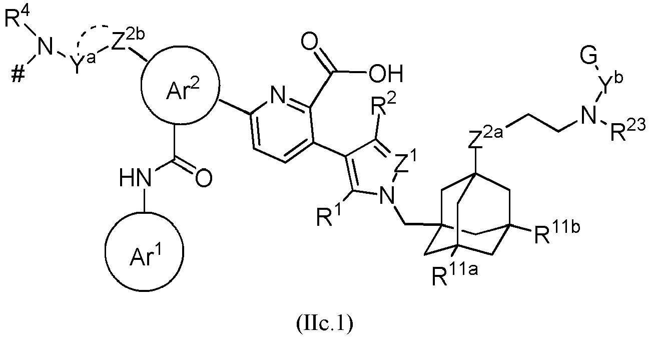 or salts thereof, wherein:
Ar¹, Ar², Z¹, Z^{2a}, Z^{2b}, R¹, R², R⁴, R^{11a,}R^{11b} and # are defined as above;
Y^{a} is optionally substituted C₁-C₈ alkylene;
Y^{b} is optionally substituted C₁-C₈ alkylene;
R²³ is selected from H and C₁-C₄ alkyl; and
G is selected from a polyol, PEG4-30, a salt and a moiety that is charged at physiological pH;

In certain embodiments in which the Bcl-xL inhibitor is a compound of formula (IIc.1), Z^{2a} is selected from O, CH₂, NH and S. In particular embodiments, Z^{2a} is O. In certain embodiments, Z^{2a} of formula (IIc.1) is CR^{6a}R^{6b}. In certain embodiments, Z^{2a} of formula (IIc.1) is S. In certain embodiments, Z^{2a} of formula (IIc.1) is -NR⁶C(O)-.

In certain embodiments in which the Bcl-xL inhibitor is a compound of formula (IIc.1), Z^{2b} is selected from O, CH₂, NH, NCH₃ and S. In particular embodiments, Z^{2b} is O. In particular embodiments, Z^{2b} is NH. In particular embodiments, Z^{2b} is NCH₃.

In certain embodiments in which the Bcl-xL inhibitor is a compound of formula (IIc.1), Z^{2b} is a bond. In some such embodiments Y^{a} is methylene or ethylene.

In certain embodiments in which the Bcl-xL inhibitor is a compound of formula (IIc.1), Z^{2b} is O. In some such embodiments Y^{a} is methylene, ethylene, or propylene.

In certain embodiments in which the Bcl-xL inhibitor is a compound of formula (IIc.1), Z^{2b} is NR⁶, where R⁶ is defined as above. In some such embodiments, R⁶ is taken together with an atom from Y^{a} to form a cycloalkyl or heterocyclyl ring having between 3 and 7 ring atoms. In some such embodiments, the ring has 5 atoms.

In certain embodiments in which the Bcl-xL inhibitor is a compound of formula (IIc.1), Y^{a} is ethylene.

In certain embodiments in which the Bcl-xL inhibitor is a compound of formula (IIc.1), Y^{a} is methylene.

In certain embodiments in which the Bcl-xL inhibitor is a compound of formula (IIc.1), Y^{a} is propylene.

In certain embodiments in which the Bcl-xL inhibitor is a compound of formula (IIc.1), R⁴ is H or methyl. In particular embodiments, R⁴ is H.

In certain embodiments in which the Bcl-xL inhibitor is a compound of formula (IIc.1), Y^{b} is ethylene or propylene. In particular embodiments, Y^{b} is ethylene.

In certain embodiments in which the Bcl-xL inhibitor is a compound of formula (IIc.1), R²³ is methyl.

In certain embodiments in which the Bcl-xL inhibitor is a compound of formula (IIc.1), R²³ is H.

In certain embodiments in which the Bcl-xL inhibitor is a compound of formula (IIc.1), G is selected from 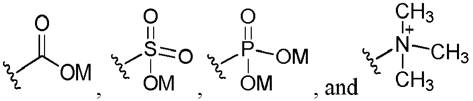 wherein M is hydrogen or a positively charged counterion. In particular embodiments, G is 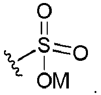 In particular embodiments, G isSO₃H.

In certain embodiments in which the Bcl-xL inhibitor is a compound of formula (IIc.1), Ar² is selected from 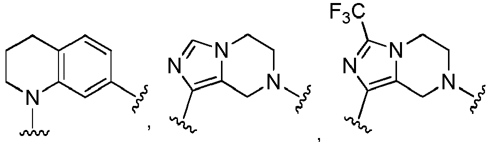 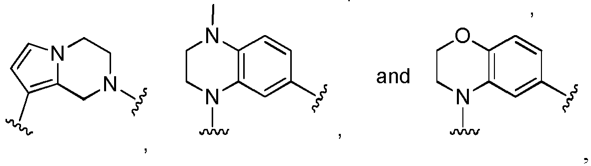 wherein the #-N(R⁴)-Y^{a}-Z^{2b}- substituent is attached to Ar² at any Ar² atom capable of being substituted.

In particular embodiments in which the Bcl-xL inhibitor is a compound of formula (IIc.1), Ar² is 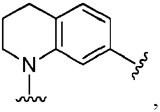 wherein the #-N(R⁴)-Y^{a}-Z^{2b}- substituent is attached to Ar² at any Ar² atom capable of being substituted. In certain embodiments in which the Bcl-xL inhibitor is a compound of formula (IIc.1), Ar² is selected from 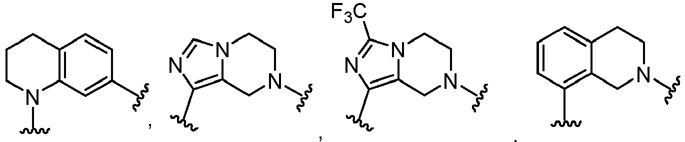 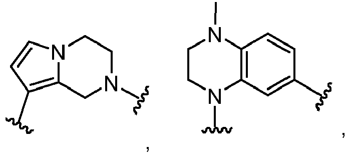 and 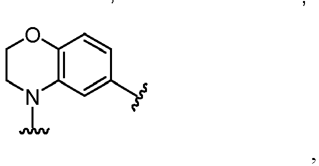 wherein the #-N(R⁴)-Y^{a}-Z^{2b}- substituent is attached to Ar² at any Ar² atom capable of being substituted. In particular embodiments in which the Bcl-xL inhibitor is a compound of formula (IIc.1), Ar² is 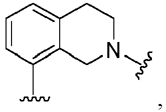 wherein the #-N(R⁴)-Y^{a}-Z^{2b}- substituent is attached to Ar² at any Ar² atom capable of being substituted.

In certain embodiments in which the Bcl-xL inhibitor is a compound of formula (IIc.1), Ar¹ is

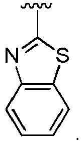

In certain embodiments in which the Bcl-xL inhibitor is a compound of formula (IIc.1), the group 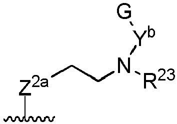 is selected from:

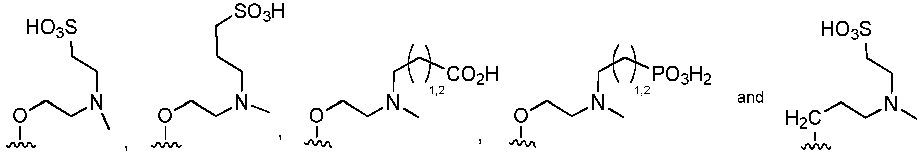

In other embodiments in which the Bcl-xL inhibitor is a compound of formula (IIc.1), the group 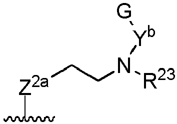 is selected from:

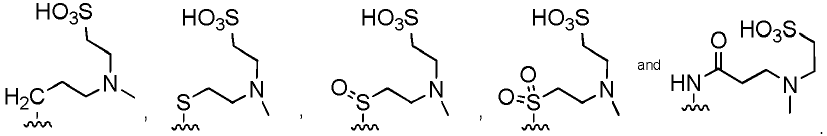

In certain embodiments in which the Bcl-xL inhibitor is a compound of formula (IIc), the compound has the structural formula (IIc.2), 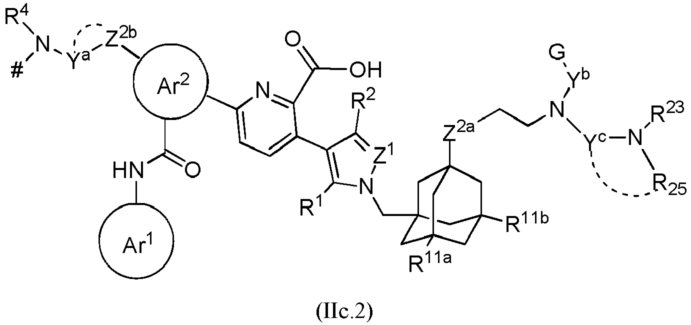 or salts thereof, wherein:
Ar¹, Ar², Z¹, Z^{2a}, Z^{2b}, R¹, R², R⁴, R^{11a,}R^{11b} and # are defined as above;
Y^{a} is optionally substituted C₁-C₈ alkylene;
Y^{b} is optionally substituted C₁-C₈ alkylene;
Y^{c} is optionally substituted C₁-C₈ alkylene;
R²³ is selected from H and C₁-C₄ alkyl;
R²⁵ is Y^{b}-G or is taken together with an atom of Y^{c} to form a ring having 4-6 ring atoms; and
G is selected from a polyol, PEG4-30, a salt and a moiety that is charged at physiological pH.

In certain embodiments in which the Bcl-xL inhibitor is a compound of formula (IIc.2), Z^{2a} is selected from O, CH₂, NH and S. In particular embodiments, Z^{2a} is O. In certain embodiments, Z^{2a} of formula (IIc.2) is CR^{6a}R^{6b}. In certain embodiments, Z^{2a} of formula (IIc.2) is S . In certain embodiments, Z^{2a} of formula (IIc.2) is -NR⁶C(O)-. In certain embodiments in which the Bcl-xL inhibitor is a compound of formula (IIc.2), Z^{2b} is selected from O, CH₂, NH, NCH₃ and S. In particular embodiments, Z^{2b} is O. In particular embodiments, Z^{2b} is NH. In particular embodiments, Z^{Zb} is NCH₃.

In certain embodiments in which the Bcl-xL inhibitor is a compound of formula (IIc.2), Z^{2b} is a bond. In some such embodiments Y^{a} is methylene or ethylene.

In certain embodiments in which the Bcl-xL inhibitor is a compound of formula (IIc.2), Z^{2b} is NR⁶, where R⁶ is defined as above. In some such embodiments, R⁶ is taken together with an atom from Y^{a} to form a cycloalkyl or heterocyclyl ring having between 3 and 7 ring atoms. In some such embodiments, the ring has 5 atoms.

In certain embodiments in which the Bcl-xL inhibitor is a compound of formula (IIc.2), Y^{a} is ethylene.

In certain embodiments in which the Bcl-xL inhibitor is a compound of formula (IIc.2), Y^{a} is methylene.

In certain embodiments in which the Bcl-xL inhibitor is a compound of formula (IIc.2), R⁴ is H or methyl.

In certain embodiments in which the Bcl-xL inhibitor is a compound of formula (IIc.2), Y^{b} is ethylene or propylene. In particular embodiments, Y^{b} is ethylene.

In certain embodiments in which the Bcl-xL inhibitor is a compound of formula (IIc.2), Y^{c} is ethylene or propylene. In particular embodiments, Y^{b} is ethylene.

In certain embodiments in which the Bcl-xL inhibitor is a compound of formula (IIc.2), R²⁵ is taken together with an atom of Y^{c} to form a ring having 4 or 5 ring atoms.

In certain embodiments in which the Bcl-xL inhibitor is a compound of formula (IIc.2), R²³ is methyl.

In certain embodiments in which the Bcl-xL inhibitor is a compound of formula (IIc.2), G is selected from 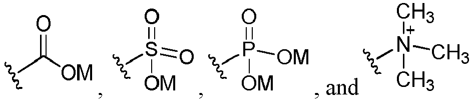 wherein M is hydrogen or a positively charged counterion. In particular embodiments, G is 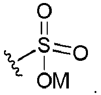 In particular embodiments, G is SO₃H.

In certain embodiments in which the Bcl-xL inhibitor is a compound of formula (IIc.2), Ar² is selected from 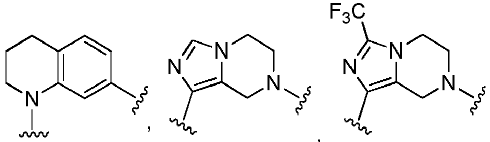 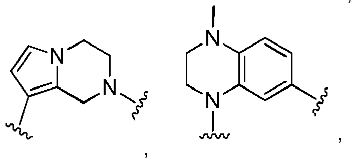 and 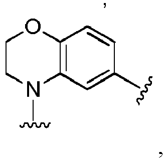 wherein the #-N(R⁴)-Y^{a}-Z^{2b}-substituent is attached to Ar² at any Ar² atom capable of being substituted.

In particular embodiments in which the Bcl-xL inhibitor is a compound of formula (IIc.2), Ar² is 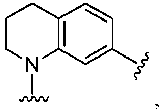 wherein the #-N(R⁴)-Y^{a}-Z^{2b}- substituent is attached to Ar² at any Ar² atom capable of being substituted. In certain embodiments in which the Bcl-xL inhibitor is a compound of formula (IIc.2), Ar² is selected from 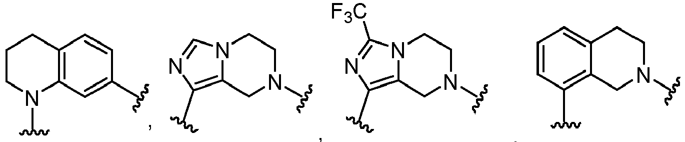 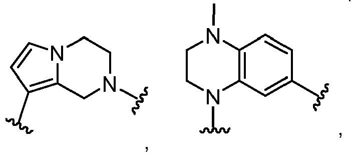 and 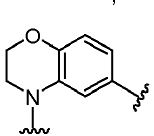 , , wherein the #-N(R⁴)-Y^{a}-Z^{2b}- substituent is attached to Ar² at any Ar² atom capable of being substituted. In particular embodiments in which the Bcl-xL inhibitor is a compound of formula (IIc.2), Ar² is 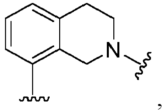 wherein the #-N(R⁴)-Y^{a}-Z^{2b}- substituent is attached to Ar² at any Ar² atom capable of being substituted.

In certain embodiments in which the Bcl-xL inhibitor is a compound of formula (IIc.2), Ar¹ is

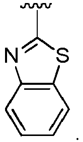

In certain embodiments in which the Bcl-xL inhibitor is a compound of formula (IIc.2), the group 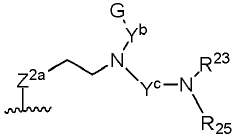 is selected from:

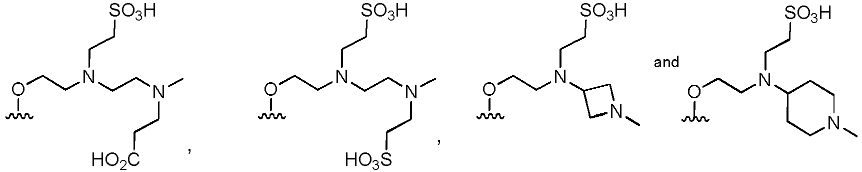

In certain embodiments in which the Bcl-xL inhibitor is a compound of formula (IId), the compound has the structural formula (IId.1), 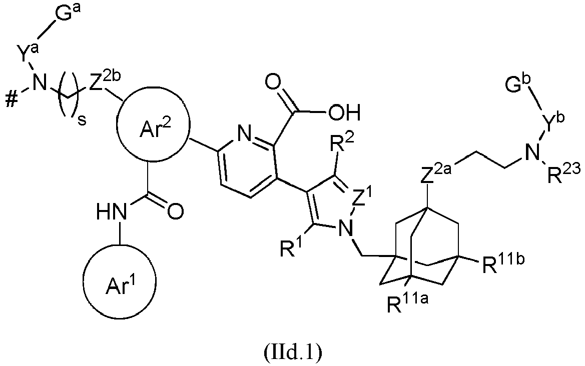 or salts thereof, wherein:
Ar¹, Ar², Z¹, Z^{2a}, Z^{2b}, R¹, R², R^{11a,}R^{11b} and # are defined as above;
   Y^{a} is optionally substituted alkylene;
   Y^{b} is optionally substituted alkylene;
R²³ is selected from H and C₁-C₄ alkyl;
G^{a} is selected from a polyol, PEG4-30, a salt and a moiety that is charged at physiological pH;
G^{b} is selected from a polyol, PEG4-30, a salt and a moiety that is charged at physiological pH;

In certain embodiments in which the Bcl-xL inhibitor is a compound of formula (IId.1), s is 1.

In certain embodiments in which the Bcl-xL inhibitor is a compound of formula (IId.1), s is 2.

In certain embodiments in which the Bcl-xL inhibitor is a compound of formula (IId.1), Z^{2a} is selected from O, NH, CH₂ and S. In particular embodiments, Z^{2a} is O. In certain embodiments, Z^{2a} of formula (IId.1) is CR^{6a}R^{6b}. In certain embodiments, Z^{2a} of formula (IId.1) is S. In certain embodiments, Z^{2a} of formula (IId.1) is -NR⁶C(O)-.

In certain embodiments in which the Bcl-xL inhibitor is a compound of formula (IId.1), Z^{2b} is selected from O, NH, CH₂ and S. In particular embodiments, Z^{2b} is O.

In certain embodiments in which the Bcl-xL inhibitor is a compound of formula (IId.1), Y^{a} is selected from ethylene, propylene and butylene. In particular embodiments, Y is ethylene.

In certain embodiments in which the Bcl-xL inhibitor is a compound of formula (IId.1), Y^{a} is selected from ethylene, propylene and butylene. In particular embodiments, Y is ethylene.

In certain embodiments in which the Bcl-xL inhibitor is a compound of formula (IId.1), G^{a} is selected from 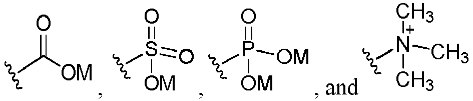 wherein M is hydrogen or a positively charged counterion. In particular embodiments, G^{a} is 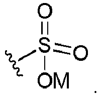 In particular embodiments, G^{a} is SO₃H. In particular embodiments, G^{a} is CO₂H.

In certain embodiments in which the Bcl-xL inhibitor is a compound of formula (IId.1), G^{b} is selected from 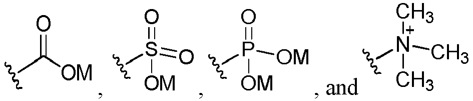 wherein M is hydrogen or a positively charged counterion. In particular embodiments, G^{b} is 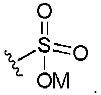 In particular embodiments, G^{b} is SO₃H. In particular embodiments, G^{b} is CO₂H.

In certain embodiments in which the Bcl-xL inhibitor is a compound of formula (IId.1), R²³ is methyl.

In certain embodiments in which the Bcl-xL inhibitor is a compound of formula (IId.1), Ar² is selected from 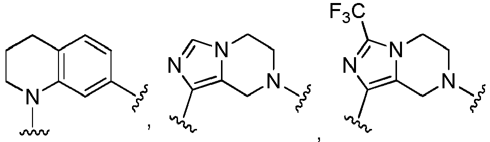 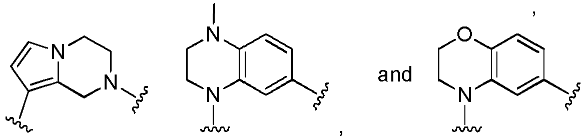 , , wherein the G^{a}-Y^{a}-N(#)-(CH₂)ₛ-Z^{2b}- substituent is attached to Ar² at any Ar² atom capable of being substituted.

In particular embodiments in which the Bcl-xL inhibitor is a compound of formula (IId.1), Ar² is 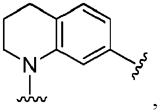 wherein the G^{a}-Y^{a}-N(#)-(CH₂)ₛ-Z^{2b}- substituent is attached to Ar² at any Ar² atom capable of being substituted. In certain embodiments in which the Bcl-xL inhibitor is a compound of formula (IId.1), Ar² is selected from 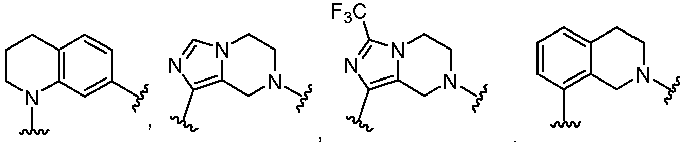 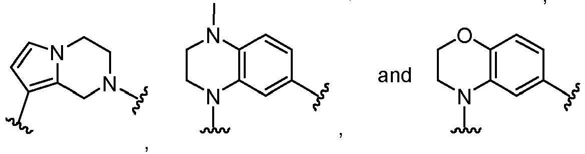 , , wherein the G^{a}-Y^{a}-N(#)-(CH₂)ₛ-Z^{2b}- substituent is attached to Ar² at any Ar² atom capable of being substituted. In particular embodiments in which the Bcl-xL inhibitor is a compound of formula (IId.1), Ar² is 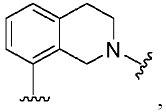 wherein the G^{a}-Y^{a}-N(#)-(CH₂)ₛ-Z^{2b}- substituent is attached to Ar² at any Ar² atom capable of being substituted.

In certain embodiments in which the Bcl-xL inhibitor is a compound of formula (IId.1), Ar¹ is

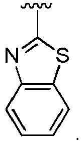

In certain embodiments, R^{11a} and R^{11b} of formulae (IIa)-(IId) are the same. In a particular embodiment, R^{11a} and R^{11b} are each methyl.

In certain embodiments, the compounds of formulae (IIa)-(IId) include one of the following cores (C.1)-(C.21):

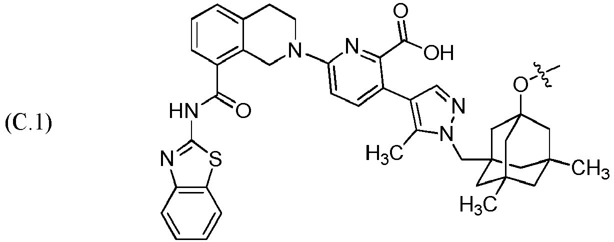

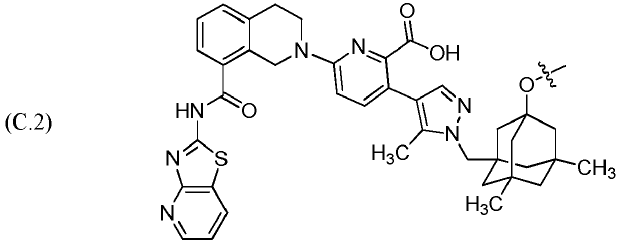

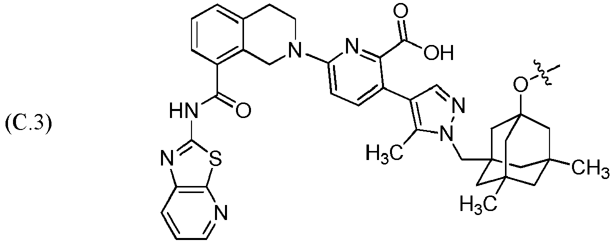

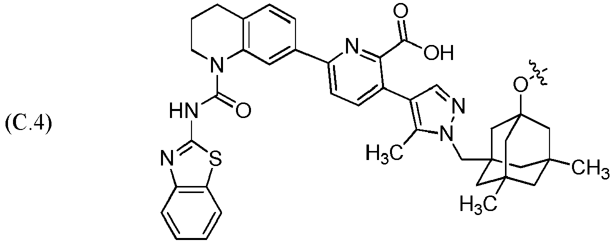

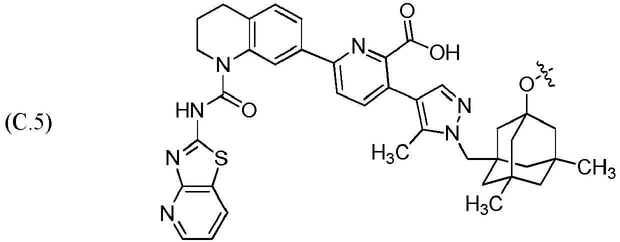

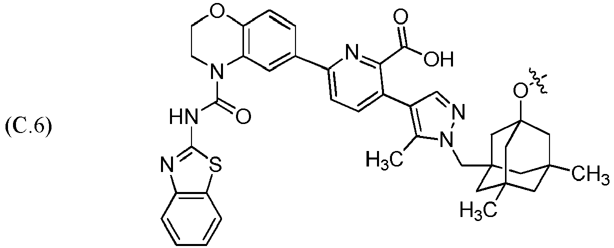

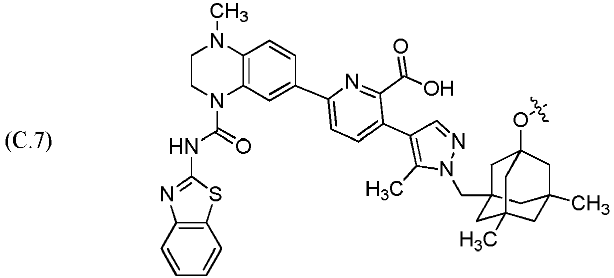

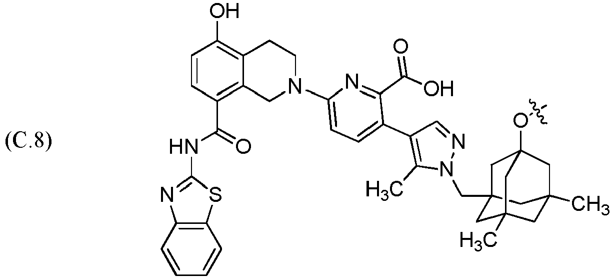

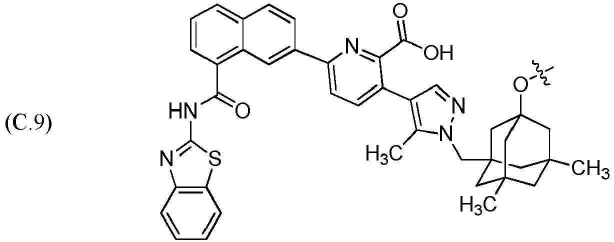

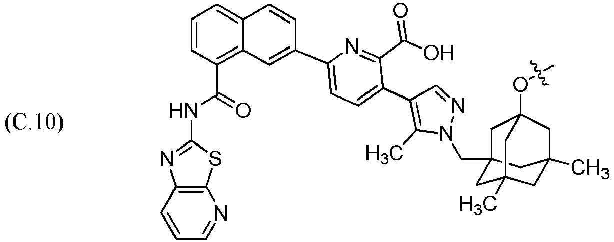

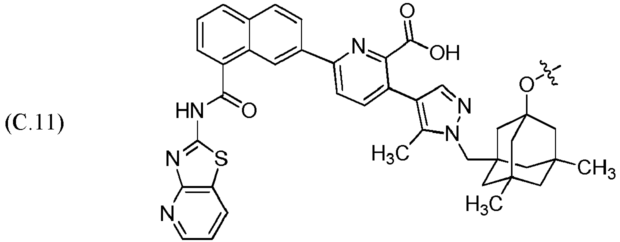

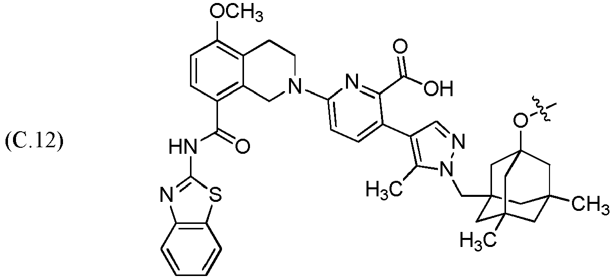

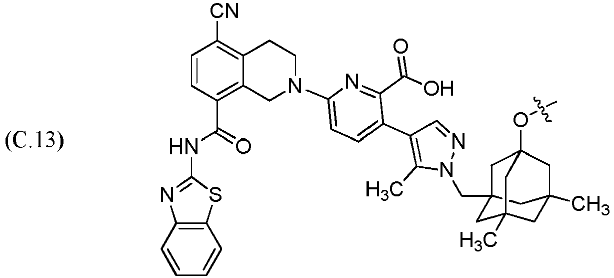

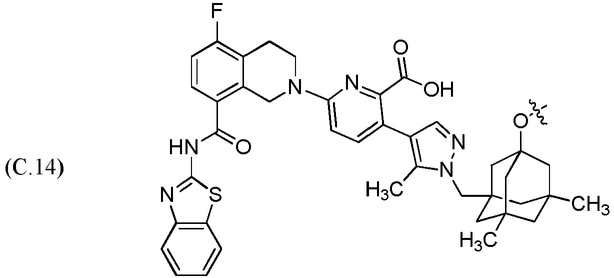

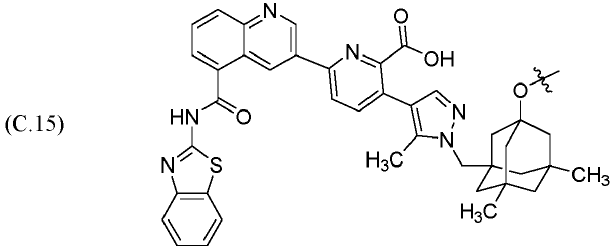

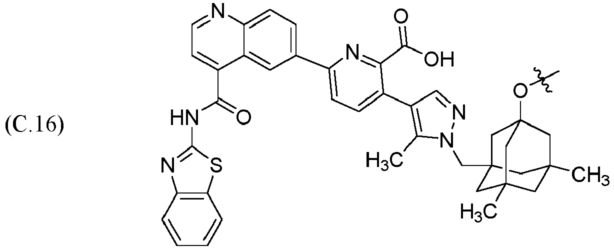

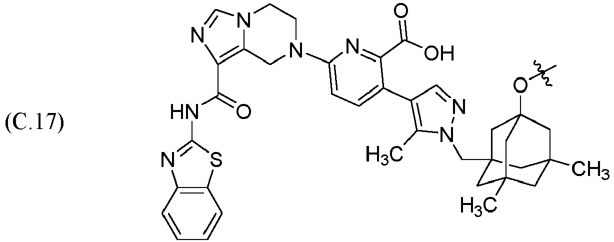

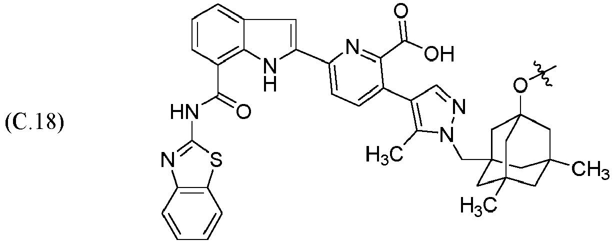

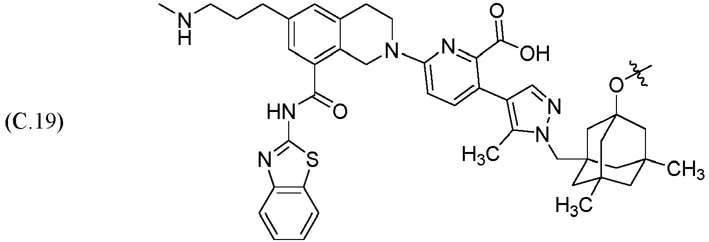

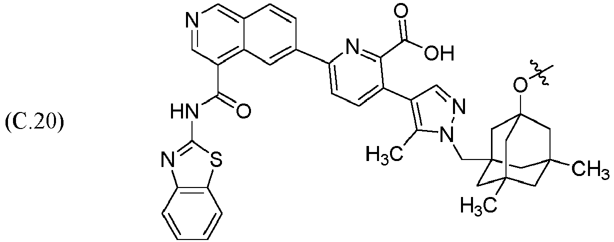

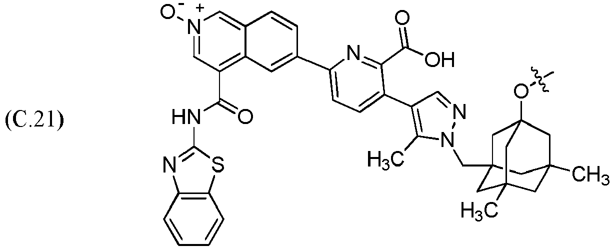

Exemplary Bcl-xL inhibitors according to structural formulae (IIa)-(IId) that may be used in the methods described herein in unconjugated form and/or included in the ADCs described herein include the following compounds, and/or salts thereof:

| **App Ex. No.** | **Bcl-xL Inhibitor Cmpd No** |
|---|---|
| 1.1 | W2.01 |
| 1.2 | W2.02 |
| 1.3 | W2.03 |
| 1.5 | W2.05 |
| 1.6 | W2.06 |
| 1.7 | W2.07 |
| 1.8 | W2.08 |
| 1.9 | W2.09 |
| 1.10 | W2.10 |
| 1.11 | W2.11 |
| 1.12 | W2.12 |
| 1.13 | W2.13 |
| 1.14 | W2.14 |
| 1.15 | W2.15 |
| 1.16 | W2.16 |
| 1.17 | W2.17 |
| 1.18 | W2.18 |
| 1.19 | W2.19 |
| 1.20 | W2.20 |
| 1.21 | W2.21 |
| 1.22 | W2.22 |
| 1.23 | W2.23 |
| 1.24 | W2.24 |
| 1.25 | W2.25 |
| 1.26 | W2.26 |
| 1.27 | W2.27 |
| 1.28 | W2.28 |
| 1.29 | W2.29 |
| 1.30 | W2.30 |
| 1.31 | W2.31 |
| 1.32 | W2.32 |
| 1.33 | W2.33 |
| 1.34 | W2.34 |
| 1.35 | W2.35 |
| 1.36 | W2.36 |
| 1.37 | W2.37 |
| 1.38 | W2.38 |
| 1.39 | W2.39 |
| 1.40 | W2.40 |
| 1.41 | W2.41 |
| 1.42 | W2.42 |
| 1.43 | W2.43 |
| 1.44 | W2.44 |
| 1.45 | W2.45 |
| 1.46 | W2.46 |
| 1.47 | W2.47 |
| 1.48 | W2.48 |
| 1.49 | W2.49 |
| 1.50 | W2.50 |
| 1.51 | W2.51 |
| 1.52 | W2.52 |
| 1.53 | W2.53 |
| 1.54 | W2.54 |
| 1.55 | W2.55 |
| 1.56 | W2.56 |
| 1.57 | W2.57 |
| 1.58 | W2.58 |
| 1.59 | W2.59 |
| 1.60 | W2.60 |
| 1.61 | W2.61 |
| 1.62 | W2.62 |
| 1.63 | W2.63 |
| 1.64 | W2.64 |
| 1.65 | W2.65 |
| 1.66 | W2.66 |
| 1.67 | W2.67 |
| 1.68 | W2.68 |
| 1.69 | W2.69 |
| 1.70 | W2.70 |
| 1.71 | W2.71 |
| 1.72 | W2.72 |
| 1.73 | W2.73 |
| 1.74 | W2.74 |
| 1.75 | W2.75 |
| 1.76 | W2.76 |
| 1.77 | W2.77 |
| 1.78 | W2.78 |
| 1.79 | W2.79 |
| 1.80 | W2.80 |
| 1.81 | W2.81 |
| 1.82 | W2.82 |
| 1.83 | W2.83 |
| 1.84 | W2.84 |
| 1.85 | W2.85 |
| 1.86 | W2.86 |
| 1.87 | W2.87 |
| 1.88 | W2.88 |
| 1.89 | W2.89 |
| 1.90 | W2.90 |
| 1.91 | W2.91 |

Notably, when the Bcl-xL inhibitor of the present application is in conjugated form, the hydrogen corresponding to the # position of structural formula (IIa) or (IIb) is not present, forming a monoradical. For example, compound W2.01 (Example 1.1) is 6-[8-(1,3-benzothiazol-2-ylcarbamoyl)-3,4-dihydroisoquinolin-2(1H)-yl]-3-[1-({3-[2-({2-[2-(carboxymethoxy)ethoxy]ethyl}amino)ethoxy]-5,7-dimethyltricyclo[3.3.1.13,7]dec-1-yl}methyl)-5-methyl-1H-pyrazol-4-yl]pyridine-2-carboxylic acid.

When it is in unconjugated form, it has the following structure:

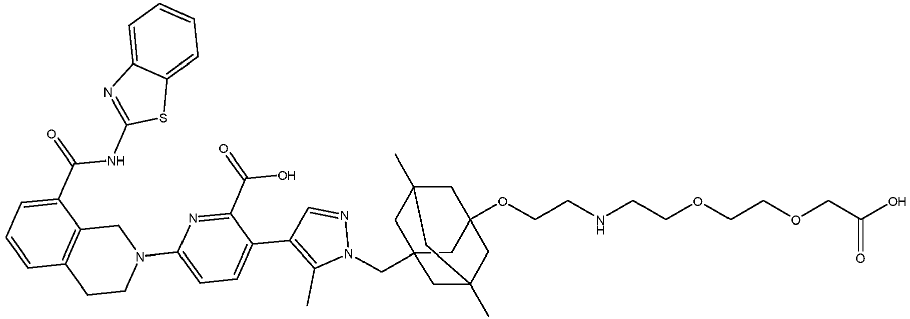

When the same compound is included in the ADCs as shown in structural formula (IIa) or (IIb), the hydrogen corresponding to the # position is not present, forming a monoradical.

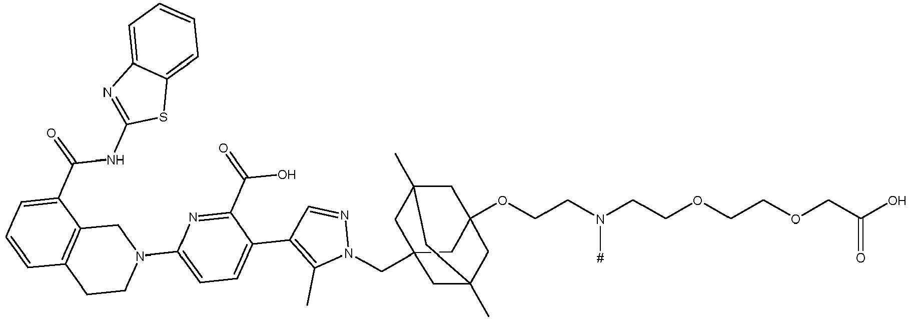

In certain embodiments, the Bcl-xL inhibitors according to structural formulae (IIa)-(IId) are selected from the group consisting of W2.01, W2.02, W2.03, W2.04, W2.05, W2.06, W2.07, W2.08, W2.09, W2.10, W2.11, W2.12, W2.13, W2.14, W2.15, W2.16, W2.17, W2.18, W2.19, W2.20, W2.21, W2.22, W2.23, W2.24, W2.25, W2.26, W2.27, W2.28, W2.29, W2.30, W2.31, W2.32, W2.33, W2.34, W2.35, W2.36, W2.37, W2.38, W2.39, W2.40, W2.41, W2.42, W2.43, W2.44, W2.45, W2.46, W2.47, W2.48, W2.49, W2.50, W2.51, W2.52, W2.53, W2.54, W2.55, W2.56, W2.57, W2.58, W2.59, W2.60, W2.61, W2.62, W2.63, W2.64, W2.65, W2.66, W2.67, W2.68, W2.69, W2.70, W2.71, W2.72, W2.73, W2.74, W2.75, W2.76, W2.77, W2.78, W2.79, W2.80, W2.81, W2.82, W2.83, W2.84, W2.85, W2.86, W2.87, W2.88, W2.89, W2.90, and W2.91, or pharmaceutically acceptable salts thereof.

In certain embodiments, the ADC, or a pharmaceutically acceptable salt thereof, comprises a drug linked to an antibody by way of a linker, wherein the drug is a Bcl-xL inhibitor selected from the group consisting of W2.01, W2.02, W2.03, W2.04, W2.05, W2.06, W2.07, W2.08, W2.09, W2.10, W2.11, W2.12, W2.13, W2.14, W2.15, W2.16, W2.17, W2.18, W2.19, W2.20, W2.21, W2.22, W2.23, W2.24, W2.25, W2.26, W2.27, W2.28, W2.29, W2.30, W2.31, W2.32, W2.33, W2.34, W2.35, W2.36, W2.37, W2.38, W2.39, W2.40, W2.41, W2.42, W2.43, W2.44, W2.45, W2.46, W2.47, W2.48, W2.49, W2.50, W2.51, W2.52, W2.53, W2.54, W2.55, W2.56, W2.57, W2.58, W2.59, W2.60, W2.61, W2.62, W2.63, W2.64, W2.65, W2.66, W2.67, W2.68, W2.69, W2.70, W2.71, W2.72, W2.73, W2.74, W2.75, W2.76, W2.77, W2.78, W2.79, W2.80, W2.81, W2.82, W2.83, W2.84, W2.85, W2.86, W2.87, W2.88, W2.89, W2.90, and W2.91.

In certain embodiments, the ADC, or a pharmaceutically acceptable salt thereof, the Bcl-xL inhibitor is selected from the group consisting of the following compounds modified in that the hydrogen corresponding to the # position of structural formula (IIa), (IIb),(IIc), or (IId) is not present forming a monoradical:
6-[8-(1,3-benzothiazol-2-ylcarbamoyl)-3,4-dihydroisoquinolin-2(1H)-yl]-3-[1-({3-[2-({2-[2-(carboxymethoxy)ethoxy]ethyl}amino)ethoxy]-5,7-dimethyltricyclo[3.3.1.1^{3,}]dec-1yl}methyl)-5-methyl-1H-pyrazol-4-yl]pyridine-2-carboxylic acid;
6-[8-(1,3-benzothiazol-2-ylcarbamoyl)-3,4-dihydroisoquinolin-2(1H)-yl]-3-{1-[(3,5-dimethyl-7-{2-[(2-sulfoethyl)amino]ethoxy}tricyclo[3.3.1.1^{3,7}]dec-1-yl)methyl]-5-methyl-1H-pyrazol-4-yl}pyridine-2-carboxylic acid;
2-{[(2-{[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl]amino}ethyl)sulfonyl]amino}-2-deoxy-D-glucopyranose;
6-[8-(1,3-benzothiazol-2-ylcarbamoyl)-3,4-dihydroisoquinolin-2(1H)-yl]-3-{1-[(3,5-dimethyl-7-{2-[(4-{[(3R,4R,5S,6R)-3,4,5-trihydroxy-6-(hydroxymethyl)tetrahydro-2*H*-pyran-2-yl]methyl}benzyl)amino]ethoxy}tricyclo[3.3.1.1^{3,7}]dec-1-yl)methyl]-5-methyl-1*H*-pyrazol-4-yl}pyridine-2-carboxylic acid
6-[8-(1,3-benzothiazol-2-ylcarbamoyl)-3,4-dihydroisoquinolin-2(1H)-yl]-3-{1-[(3,5-dimethyl-7-{2-[(3-sulfopropyl)amino]ethoxy}tricyclo[3.3.1.1^{3,7}]dec-1-yl)methyl]-5-methyl-1H-pyrazol-4-yl}pyridine-2-carboxylic acid;
6-[8-(1,3-benzothiazol-2-ylcarbamoyl)-3,4-dihydroisoquinolin-2(1H)-yl]-3-{1-[(3-{2-[(2,3-dihydroxypropyl)amino]ethoxy}-5,7-dimethyltricyclo[3.3.1.1^{3,7}]dec-1-yl)methyl]-5-methyl-1H-pyrazol-4-yl}pyridine-2-carboxylic acid;
2-({[4-({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl]amino}methyl)phenyl]sulfonyl}amino)-2-deoxy-beta-D-glucopyranose;
8-(1,3-benzothiazol-2-ylcarbamoyl)-2-{6-carboxy-5-[1-({3-[2-({2-[1-(beta-D-glucopyranuronosyl)-1H-1,2,3-triazol-4-yl]ethyl}amino)ethoxy]-5,7-dimethyltricyclo[3.3.1.1^{3,7}]dec-1-yl}methyl)-5-methyl-1H-pyrazol-4-yl]pyridin-2-yl}-1,2,3,4-tetrahydroisoquinoline;
3-[1-({3-[2-(2-{[4-(beta-D-allopyranosyloxy)benzyl]amino}ethoxy)ethoxy]-5,7-dimethyltricyclo[3.3.1.1^{3,7}]dec-1-yl}methyl)-5-methyl-1H-pyrazol-4-yl]-6-[8-(1,3-benzothiazol-2-ylcarbamoyl)-3,4-dihydroisoquinolin-2(1H)-yl]pyridine-2-carboxylic acid;
6-[8-(1,3-benzothiazol-2-ylcarbamoyl)-3,4-dihydroisoquinolin-2(1H)-yl]-3-(1-{[3,5-dimethyl-7-(2-{2-[(2-sulfoethyl)amino]ethoxy}ethoxy)tricyclo[3.3.1.1^{3,7}]dec-1-yl]methyl}-5-methyl-1H-pyrazol-4-yl)pyridine-2-carboxylic acid;
6-[8-(1,3-benzothiazol-2-ylcarbamoyl)-3,4-dihydroisoquinolin-2(1H)-yl]-3-{1-[(3,5-dimethyl-7-{2-[(2-phosphonoethyl)amino]ethoxy}tricyclo[3.3.1.1^{3,7}]dec-1-yl)methyl]-5-methyl-1H-pyrazol-4-yl}pyridine-2-carboxylic acid;
6-[8-(1,3-benzothiazol-2-ylcarbamoyl)-3,4-dihydroisoquinolin-2(1H)-yl]-3-{1-[(3,5-dimethyl-7-{2-[methyl(3-sulfo-L-alanyl)amino]ethoxy}tricyclo[3.3.1.1^{3,7}]dec-1-yl)methyl]-5-methyl-1H-pyrazol-4-yl}pyridine-2-carboxylic acid;
6-[8-(1,3-benzothiazol-2-ylcarbamoyl)-3,4-dihydroisoquinolin-2(1H)-yl]-3-{1-[(3,5-dimethyl-7-{2-[(3-phosphonopropyl)amino]ethoxy}tricyclo[3.3.1.1^{3,7}]dec-1-yl)methyl]-5-methyl-1H-pyrazol-4-yl}pyridine-2-carboxylic acid;
6-[8-(1,3-benzothiazol-2-ylcarbamoyl)-3,4-dihydroisoquinolin-2(1H)-yl]-3-{1-[(3,5-dimethyl-7-{2-[(3-sulfo-L-alanyl)amino]ethoxy}tricyclo[3.3.1.1^{3,7}]dec-1-yl)methyl]-5-methyl-1H-pyrazol-4-yl}pyridine-2-carboxylic acid;
6-[8-(1,3-benzothiazol-2-ylcarbamoyl)-3,4-dihydroisoquinolin-2(1H)-yl]-3-(1-{[3,5-dimethyl-7-(2-{2-[(3-phosphonopropyl)amino]ethoxy}ethoxy)tricyclo[3.3.1.1^{3,7}]dec-1-yl]methyl}-5-methyl-1H-pyrazol-4-yl)pyridine-2-carboxylic acid;
3-{1-[(3-{2-[L-alpha-aspartyl(methyl)amino]ethoxy}-5,7-dimethyltricyclo[3.3.1.1^{3,7}]dec-1-yl)methyl]-5-methyl-1H-pyrazol-4-yl}-6-[8-(1,3-benzothiazol-2-ylcarbamoyl)-3,4-dihydroisoquinolin-2(1H)-yl]pyridine-2-carboxylic acid;
6-{4-[({2-[2-(2-aminoethoxy)ethoxy]ethyl}[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl]amino)methyl]benzyl}-2,6-anhydro-L-gulonic acid;
4-({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl]amino}methyl)phenyl hexopyranosiduronic acid;
6-[1-(1,3-benzothiazol-2-ylcarbamoyl)-1,2,3,4-tetrahydroquinolin-7-yl]-3-{1-[(3,5-dimethyl-7-{2-[(2-phosphonoethyl)amino]ethoxy}tricyclo[3.3.1.1^{3,7}]dec-1-yl)methyl]-5-methyl-1H-pyrazol-4-yl}pyridine-2-carboxylic acid;
6-[1-(1,3-benzothiazol-2-ylcarbamoyl)-1,2,3,4-tetrahydroquinolin-7-yl]-3-{1-[(3,5-dimethyl-7-{2-[methyl(3-sulfo-L-alanyl)amino]ethoxy}tricyclo[3.3.1.1^{3,7}]dec-1-yl)methyl]-5-methyl-1H-pyrazol-4-yl}pyridine-2-carboxylic acid;
3-{1-[(3,5-dimethyl-7-{2-[(2-sulfoethyl)amino]ethoxy}tricyclo[3.3.1.1^{3,7}]dec-1-yl)methyl]-5-methyl-1H-pyrazol-4-yl}-6-[8-([1,3]thiazolo[5,4-b]pyridin-2-ylcarbamoyl)-3,4-dihydroisoquinolin-2(1H)-yl]pyridine-2-carboxylic acid;
3-{1-[(3,5-dimethyl-7-{2-[(2-sulfoethyl)amino]ethoxy}tricyclo[3.3.1.1^{3,7}]dec-1-yl)methyl]-5-methyl-1H-pyrazol-4-yl}-6-[8-([1,3]thiazolo[4,5-b]pyridin-2-ylcarbamoyl)-3,4-dihydroisoquinolin-2(1H)-yl]pyridine-2-carboxylic acid;
6-[1-(1,3-benzothiazol-2-ylcarbamoyl)-1,2,3,4-tetrahydroquinolin-7-yl]-3-{1-[(3,5-dimethyl-7-{2-[(2-sulfoethyl)amino]ethoxy}tricyclo[3.3.1.1^{3,7}]dec-1-yl)methyl]-5-methyl-1H-pyrazol-4-yl}pyridine-2-carboxylic acid;
6-[8-(1,3-benzothiazol-2-ylcarbamoyl)-3,4-dihydroisoquinolin-2(1H)-yl]-3-{1-[(3-{2-[(2-carboxyethyl)amino]ethoxy}-5,7-dimethyltricyclo[3.3.1.1^{3,7}]dec-1-yl)methyl]-5-methyl-1H-pyrazol-4-yl}pyridine-2-carboxylic acid;
6-[8-(1,3-benzothiazol-2-ylcarbamoyl)-3,4-dihydroisoquinolin-2H)-yl]-3-{1-[(3,5-dimethyl-7-{2-[(3-phosphonopropyl)(piperidin-4-yl)amino]ethoxy}tricyclo[3.3.1.1^{3,7}]dec-1-yl)methyl]-5-methyl-1H-pyrazol-4-yl}pyridine-2-carboxylic acid;
3-{1-[(3-{2-[D-alpha-aspartyl(methyl)amino]ethoxy}-5,7-dimethyltricyclo[3.3.1.1^{3,7}]dec-1-yl)methyl]-5-methyl-1H-pyrazol-4-yl}-6-[8-(1,3-benzothiazol-2-ylcarbamoyl)-3,4-dihydroisoquinolin-2(1H)-yl]pyridine-2-carboxylic acid;
6-[8-(1,3-benzothiazol-2-ylcarbamoyl)-3,4-dihydroisoquinolin-2(1H)-yl]-3-(1-{[3-(2-{[1-(carboxymethyl)piperidin-4-yl]amino}ethoxy)-5,7-dimethyltricyclo[3.3.1.1^{3,7}]dec-1-yl]methyl}-5-methyl-1H-pyrazol-4-yl)pyridine-2-carboxylic acid;
N-[(5S)-5-amino-6-{[2-({3-[(4-{6-[8-(1,3-benzothiazol-2-ylcarbamoyl)-3,4-dihydroisoquinolin-2(1H)-yl]-2-carboxypyridin-3 -yl} -5 -methyl- 1H-pyrazol-1-yl)methyl] -5,7-dimethyltricyclo[3.3.1.1^{3,7}]dec-1-yl}oxy)ethyl](methyl)amino}-6-oxohexyl]-N,N-dimethylmethanaminium;
6-[8-(1,3-benzothiazol-2-ylcarbamoyl)-3,4-dihydroisoquinolin-2(1H)-yl]-3-{1-[(3,5-dimethyl-7-{2-[piperidin-4-yl(2-sulfoethyl)amino]ethoxy}tricyclo[3.3.1.1^{3,7}]dec-1-yl)methyl]-5-methyl-1H-pyrazol-4-yl}pyridine-2-carboxylic acid;
6-[8-(1,3-benzothiazol-2-ylcarbamoyl)-5-(3-phosphonopropoxy)-3,4-dihydroisoquinolin-2(1H)-yl]-3-[1-({3,5-dimethyl-7-[2-(methylamino)ethoxy]tricyclo[3.3.1.1^{3,7}]dec-1-yl}methyl)-5-methyl-1H-pyrazol-4-yl]pyridine-2-carboxylic acid;
6-[8-(1,3-benzothiazol-2-ylcarbamoyl)-3,4-dihydroisoquinolin-2(1H)-yl]-3-(1-{[3-(2-{[N-(2-carboxyethyl)-L-alpha-aspartyl]amino}ethoxy)-5,7-dimethyltricyclo[3.3.1.1^{3,7}]dec-1-yl]methyl}-5-methyl-1H-pyrazol-4-yl)pyridine-2-carboxylic acid;
3-{1-[(3-{2-[(2-aminoethyl)(2-sulfoethyl)amino]ethoxy}-5,7-dimethyltricyclo[3.3.1.1^{3,7}]dec-1-yl)methyl]-5-methyl-1H-pyrazol-4-yl}-6-[8-(1,3-benzothiazol-2-ylcarbamoyl)-3,4-dihydroisoquinolin-2(1H)-yl]pyridine-2-carboxylic acid;
6-[5-(2-aminoethoxy)-8-(1,3-benzothiazol-2-ylcarbamoyl)-3,4-dihydroisoquinolin-2(1H)-yl]-3-[1-({3,5-dimethyl-7-[2-(methylamino)ethoxy]tricyclo[3.3.1.1^{3,7}]dec-1-yl}methyl)-5-methyl-1H-pyrazol-4-yl]pyridine-2-carboxylic acid;
6-[8-(1,3-benzothiazol-2-ylcarbamoyl)naphthalen-2-yl]-3-{1-[(3,5-dimethyl-7-{2-[(3-sulfopropyl)amino]ethoxy}tricyclo[3.3.1.1^{3,7}]dec-1-yl)methyl]-5-methyl-1H-pyrazol-4-yl}pyridine-2-carboxylic acid;
6-[8-(1,3-benzothiazol-2-ylcarbamoyl)-3,4-dihydroisoquinolin-2(1H)-yl]-3-{1-[(3-{2-[(2-carboxyethyl)(piperidin-4-yl)amino]ethoxy}-5,7-dimethyltricyclo[3.3.1.1^{3,7}]dec-1-yl)methyl]-5-methyl-1H-pyrazol-4-yl}pyridine-2-carboxylic acid;
6-[8-(1,3-benzothiazol-2-ylcarbamoyl)-3,4-dihydroisoquinolin-2(1H)-yl]-3-{1-[(3,5-dimethyl-7-{2-[(3-sulfo-L-alanyl)(2-sulfoethyl)amino]ethoxy}tricyclo[3.3.1.1^{3,7}]dec-1-yl)methyl]-5-methyl-1H-pyrazol-4-yl}pyridine-2-carboxylic acid;
6-[8-(1,3-benzothiazol-2-ylcarbamoyl)-3,4-dihydroisoquinolin-2(1H)-yl]-3-{1-[(3-{2-[{2-[(2-carboxyethyl)amino]ethyl}(2-sulfoethyl)amino]ethoxy}-5,7-dimethyltricyclo[3.3.1.1^{3,7}]dec-1-yl)methyl]-5-methyl-1H-pyrazol-4-yl}pyridine-2-carboxylic acid;
3-{1-[(3,5-dimethyl-7-{2-[(3-phosphonopropyl)amino]ethoxy}tricyclo[3.3.1.1^{3,7}]dec-1-yl)methyl]-5-methyl-1H-pyrazol-4-yl}-6-[8-([1,3]thiazolo[4,5-b]pyridin-2-ylcarbamoyl)-3,4-dihydroisoquinolin-2(1H)-yl]pyridine-2-carboxylic acid;
3-{1-[(3,5-dimethyl-7-{2-[(3-phosphonopropyl)amino]ethoxy}tricyclo[3.3.1.1^{3,7}]dec-1-yl)methyl]-5-methyl-1H-pyrazol-4-yl}-6-[8-([1,3]thiazolo[5,4-b]pyridin-2-ylcarbamoyl)-3,4-dihydroisoquinolin-2(1H)-yl]pyridine-2-carboxylic acid;
6-[8-(1,3-benzothiazol-2-ylcarbamoyl)-5-(carboxymethoxy)-3,4-dihydroisoquinolin-2(1H)-yl]-3-[1-({3,5-dimethyl-7-[2-(methylamino)ethoxy]tricyclo[3.3.1.1^{3,7}]dec-1-yl}methyl)-5-methyl-1H-pyrazol-4-yl]pyridine-2-carboxylic acid;
6-[8-(1,3-benzothiazol-2-ylcarbamoyl)-3,4-dihydroisoquinolin-2(1H)-yl]-3-{1-[(3-{2-[(3-carboxypropyl)(piperidin-4-yl)amino]ethoxy}-5,7-dimethyltricyclo[3.3.1.1^{3,7}]dec-1-yl)methyl]-5-methyl-1H-pyrazol-4-yl}pyridine-2-carboxylic acid;
6-[8-(1,3-benzothiazol-2-ylcarbamoyl)naphthalen-2-yl]-3-{1-[(3,5-dimethyl-7-{2-[(2-sulfoethyl)amino]ethoxy}tricyclo[3.3.1.1^{3,7}]dec-1-yl)methyl]-5-methyl-1H-pyrazol-4-yl}pyridine-2-carboxylic acid;
3-{1-[(3-{2-[L-alpha-aspartyl(2-sulfoethyl)amino]ethoxy}-5,7-dimethyltricyclo[3.3.1.1^{3,7}]dec-1-yl)methyl]-5-methyl-1H-pyrazol-4-yl}-6-[8-(1,3-benzothiazol-2-ylcarbamoyl)-3,4-dihydroisoquinolin-2(1H)-yl]pyridine-2-carboxylic acid;
6-[8-(1,3-benzothiazol-2-ylcarbamoyl)-3,4-dihydroisoquinolin-2(1H)-yl]-3-{1-[(3-{2-[(1,3-dihydroxypropan-2-yl)amino]ethoxy}-5,7-dimethyltricyclo[3.3.1.1^{3,7}]dec-1-yl)methyl]-5-methyl-1H-pyrazol-4-yl}pyridine-2-carboxylic acid;
6-[5-(2-aminoethoxy)-8-(1,3-benzothiazol-2-ylcarbamoyl)-3,4-dihydroisoquinolin-2(1H)-yl]-3-{1-[(3,5-dimethyl-7-{2-[methyl(2-sulfoethyl)amino]ethoxy}tricyclo[3.3.1.1^{3,7}]dec-1-yl)methyl]-5-methyl-1H-pyrazol-4-yl}pyridine-2-carboxylic acid;
6-[8-(1,3-benzothiazol-2-ylcarbamoyl)-5-{2-[(2-sulfoethyl)amino]ethoxy}-3,4-dihydroisoquinolin-2(1H)-yl]-3-{1-[(3,5-dimethyl-7-{2-[methyl(2-sulfoethyl)amino]ethoxy}tricyclo[3.3.1.1^{3,7}]dec-1-yl)methyl]-5-methyl-1H-pyrazol-4-yl}pyridine-2-carboxylic acid;
6-[8-(1,3-benzothiazol-2-ylcarbamoyl)-3,4-dihydroisoquinolin-2(1H)-yl]-3-{1-[(3,5-dimethyl-7-{2-[(2-sulfoethyl){2-[(2-sulfoethyl)amino]ethyl}amino]ethoxy}tricyclo[3.3.1.1^{3,7}]dec-1-yl)methyl]-5-methyl-1H-pyrazol-4-yl}pyridine-2-carboxylic acid;
6-[8-(1,3-benzothiazol-2-ylcarbamoyl)-5-{2-[(2-carboxyethyl)amino]ethoxy}-3,4-dihydroisoquinolin-2(1H)-yl]-3-{1-[(3,5-dimethyl-7-{2-[methyl(2-sulfoethyl)amino]ethoxy}tricyclo[3.3.1.1^{3,7}]dec-1-yl)methyl]-5-methyl-1H-pyrazol-4-yl}pyridine-2-carboxylic acid;
3-{1-[(3,5-dimethyl-7-{2-[(3-phosphonopropyl)(piperidin-4-yl)amino]ethoxy}tricyclo[3.3.1.1^{3,7}]dec-1-yl)methyl]-5-methyl-1H-pyrazol-4-yl}-6-[8-([1,3]thiazolo[4,5-b]pyridin-2-ylcarbamoyl)-3,4-dihydroisoquinolin-2(1H)-yl]pyridine-2-carboxylic acid;
6-[4-(1,3-benzothiazol-2-ylcarbamoyl)-3,4-dihydro-2H-1,4-benzoxazin-6-yl]-3-{1-[(3,5-dimethyl-7-{2-[(2-sulfoethyl)amino]ethoxy}tricyclo[3.3.1.1^{3,7}]dec-1-yl)methyl]-5-methyl-1H-pyrazol-4-yl}pyridine-2-carboxylic acid;
-[8-(1,3-benzothiazol-2-ylcarbamoyl)-5-(3-sulfopropoxy)-3,4-dihydroisoquinolin-2(1H)-yl]-3-[1-({3,5-dimethyl-7-[2-(methylamino)ethoxy]tricyclo[3.3.1.1^{3,7}]dec-1-yl}methyl)-5-methyl-1H-pyrazol-4-yl]pyridine-2-carboxylic acid;
3-{1-[(3,5-dimethyl-7-{2-[(2-sulfoethyl)amino]ethoxy}tricyclo[3.3.1.1^{3,7}]dec-1-yl)methyl]-5-methyl-1H-pyrazol-4-yl}-6-[1-([1,3]thiazolo[4,5-b]pyridin-2-ylcarbamoyl)-1,2,3,4-tetrahydroquinolin-7-yl]pyridine-2-carboxylic acid;
3-{1-[(3,5-dimethyl-7-{2-[(2-sulfoethyl)amino]ethoxy}tricyclo[3.3.1.1^{3,7}]dec-1-yl)methyl]-5-methyl-1H-pyrazol-4-yl}-6-[8-([1,3]thiazolo[4,5-b]pyridin-2-ylcarbamoyl)naphthalen-2-yl]pyridine-2-carboxylic acid;
(1ξ)-1-({2-[5-(1-{[3-(2-aminoethoxy)-5,7-dimethyltricyclo[3.3.1.1^{3,7}]dec-1-yl]methyl}-5-methyl-1*H*-pyrazol-4-yl)-6-carboxypyridin-2-yl]-8-(1,3-benzothiazol-2-ylcarbamoyl)-1,2,3,4-tetrahydroisoquinolin-5-yl}methyl)-1,5-anhydro-D-glucitol;
6-[8-(1,3-benzothiazol-2-ylcarbamoyl)-3,4-dihydroisoquinolin-2(1H)-yl]-3-{1-[(3-{2-[(3-carboxypropyl)amino]ethoxy}-5,7-dimethyltricyclo[3.3.1.1^{3,7}]dec-1-yl)methyl]-5-methyl-1H-pyrazol-4-yl}pyridine-2-carboxylic acid;
6-[8-(1,3-benzothiazol-2-ylcarbamoyl)naphthalen-2-yl]-3-{1-[(3,5-dimethyl-7-{2-[(3-phosphonopropyl)amino]ethoxy}tricyclo[3.3.1.1^{3,7}]dec-1-yl)methyl]-5-methyl-1H-pyrazol-4-yl}pyridine-2-carboxylic acid;
6-[8-(1,3-benzothiazol-2-ylcarbamoyl)-3,4-dihydroisoquinolin-2(1H)-yl]-3-(1-{[3-(2-{[4-(beta-D-glucopyranosyloxy)benzyl]amino}ethoxy)-5,7-dimethyltricyclo[3.3.1.1^{3,7}]dec-1-yl]methyl}-5-methyl-1H-pyrazol-4-yl)pyridine-2-carboxylic acid;
3-(1-{[3-(2-{[4-(beta-D-allopyranosyloxy)benzyl]amino}ethoxy)-5,7-dimethyltricyclo[3.3.1.1^{3,7}]dec-1-yl]methyl}-5-methyl-1H-pyrazol-4-yl)-6-[8-(1,3-benzothiazol-2-ylcarbamoyl)-3,4-dihydroisoquinolin-2(1H)-yl]pyridine-2-carboxylic acid;
3-{1-[(3-{2-[azetidin-3-yl(2-sulfoethyl)amino]ethoxy}-5,7-dimethyltricyclo[3.3.1.1^{3,7}]dec-1-yl)methyl]-5-methyl-1H-pyrazol-4-yl}-6-[8-(1,3-benzothiazol-2-ylcarbamoyl)-3,4-dihydroisoquinolin-2(1H)-yl]pyridine-2-carboxylic acid;
3-{1-[(3-{2-[(3-aminopropyl)(2-sulfoethyl)amino]ethoxy}-5,7-dimethyltricyclo[3.3.1.1^{3,7}]dec-1-yl)methyl]-5-methyl-1H-pyrazol-4-yl}-6-[8-(1,3-benzothiazol-2-ylcarbamoyl)-3,4-dihydroisoquinolin-2(1H)-yl]pyridine-2-carboxylic acid;
6-[1-(1,3-benzothiazol-2-ylcarbamoyl)-1,2,3,4-tetrahydroquinolin-7-yl]-3-11-[(3-{2-[(2-carboxyethyl)amino]ethoxy}-5,7-dimethyltricyclo[3.3.1.1^{3,7}]dec-1-yl)methyl]-5-methyl-1H-pyrazol-4-yl}pyridine-2-carboxylic acid;
6-[8-(1,3-benzothiazol-2-ylcarbamoyl)-3,4-dihydroisoquinolin-2(1H)-yl]-3-{1-[(3-{2-[(N⁶,N⁶-dimethyl-L-lysyl)(methyl)amino]ethoxy}-5,7-dimethyltricyclo[3.3.1.1^{3,7}]dec-1-yl)methyl]-5-methyl-1H-pyrazol-4-yl}pyridine-2-carboxylic acid;
3-{1-[(3-{2-[(3-aminopropyl)(methyl)amino]ethoxy}-5,7-dimethyltricyclo[3.3.1.1^{3,7}]dec-1-yl)methyl]-5-methyl-1H-pyrazol-4-yl}-6-[1-(1,3-benzothiazol-2-ylcarbamoyl)-1,2,3,4-tetrahydroquinolin-7-yl]pyridine-2-carboxylic acid;
3-{1-[(3-{2-[azetidin-3-yl(methyl)amino]ethoxy}-5,7-dimethyltricyclo[3.3.1.1^{3,7}]dec-1-yl)methyl]-5-methyl-1H-pyrazol-4-yl}-6-[1-(1,3-benzothiazol-2-ylcarbamoyl)-1,2,3,4-tetrahydroquinolin-7-yl]pyridine-2-carboxylic acid;
N⁶-(37-oxo-2,5,8,11,14,17,20,23,26,29,32,35-dodecaoxaheptatiracontan-37-yl)-L-lysyl-N-[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl]-L-alaninamide;
methyl 6-[4-(3-{[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl]amino}propyl)-1H-1,2,3-triazol-1-yl]-6-deoxy-beta-L-glucopyranoside;
6-[8-(1,3-benzothiazol-2-ylcarbamoyl)naphthalen-2-yl]-3-{1-[(3-{2-[(2-carboxyethyl)amino]ethoxy}-5,7-dimethyltricyclo[3.3.1.1^{3,7}]dec-1-yl)methyl]-5-methyl-1H-pyrazol-4-yl}pyridine-2-carboxylic acid;
6-[5-(1,3-benzothiazol-2-ylcarbamoyl)quinolin-3-yl]-3-{1-[(3,5-dimethyl-7-{2-[(2-sulfoethyl)amino]ethoxy}tricyclo[3.3.1.1^{3,7}]dec-1-yl)methyl]-5-methyl-1H-pyrazol-4-yl}pyridine-2-carboxylic acid;
6-[4-(1,3-benzothiazol-2-ylcarbamoyl)quinolin-6-yl]-3-{1-[(3,5-dimethyl-7-{2-[(2-sulfoethyl)amino]ethoxy}tricyclo[3.3.1.1^{3,7}]dec-1-yl)methyl]-5-methyl-1H-pyrazol-4-yl}pyridine-2-carboxylic acid;
6-[5-(1,3-benzothiazol-2-ylcarbamoyl)quinolin-3-yl]-3-{1-[(3-{2-[(2-carboxyethyl)amino]ethoxy}-5,7-dimethyltricyclo[3.3.1.1^{3,7}]dec-1-yl)methyl]-5-methyl-1H-pyrazol-4-yl}pyridine-2-carboxylic acid;
6-[1-(1,3-benzothiazol-2-ylcarbamoyl)-5,6-dihydroimidazo[1,5-a]pyrazin-7(8H)-yl]-3-{1-[(3,5-dimethyl-7-{2-[(2-sulfoethyl)amino]ethoxy}tricyclo[3.3.1.1^{3,7}]dec-1-yl)methyl]-5-methyl-1H-pyrazol-4-yl}pyridine-2-carboxylic acid;
8-(1,3-benzothiazol-2-ylcarbamoyl)-2-{6-carboxy-5-[1-({3-[2-({3-[1-(beta-D-glucopyranuronosyl)-1H-1,2,3-triazol-4-yl]propyl}amino)ethoxy]-5,7-dimethyltricyclo[3.3.1.1^{3,7}]dec-1-yl}methyl)-5-methyl-1H-pyrazol-4-yl]pyridin-2-yl}-1,2,3,4-tetrahydroisoquinoline;
6-[7-(1,3-benzothiazol-2-ylcarbamoyl)-1H-indol-2-yl]-3-{1-[(3,5-dimethyl-7-{2-[(2-sulfoethyl)amino]ethoxy}tricyclo[3.3.1.1^{3,7}]dec-1-yl)methyl]-5-methyl-1H-pyrazol-4-yl}pyridine-2-carboxylic acid;
6-[8-(1,3-benzothiazol-2-ylcarbarnoyl)-6-[3-(methylamino)propyl]-3,4-dihydroisoquinolin-2(1H)-yl]-3-{1-[(3,5-dimethyl-7-{2-[(2-sulfoethyl)amino]ethoxy}tricyclo[3.3.1.1^{3,7}]dec-1-yl)methyl]-5-methyl-1H-pyrazol-4-yl}pyridine-2-carboxylic acid;
5-{[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl] amino } -5 -deoxy-D-arabinitol;
1-{[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl]amino}-1,2-dideoxy-D-arabino-hexitol;
6-[4-(1,3-benzothiazol-2-ylcarbamoyl)isoquinolin-6-yl]-3-{1-[(3,5-dimethyl-7-{2-[(2-sulfoethyl)amino]ethoxy}tricyclo[3.3.1.1^{3,7}]dec-1-yl)methyl]-5-methyl-1H-pyrazol-4-yl}pyridine-2-carboxylic acid;
6-[8-(1,3-benzothiazol-2-ylcarbamoyl)-3,4-dihydroisoquinolin-2(1H)-yl]-3-(1-{[3-(2-{[3-hydroxy-2-(hydroxymethyl)propyl]amino}ethoxy)-5,7-dimethyltricyclo[3.3.1.1^{3,7}]dec-1-yl]methyl}-5-methyl-1H-pyrazol-4-yl)pyridine-2-carboxylic acid;
1-{[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl]amino}-1,2-dideoxy-D-erythro-pentitol;
6-[8-(1,3-benzothiazol-2-ylcarbamoyl)-3,4-dihydroisoquinolin-2(1H)-yl]-3-(1-{[3,5-dimethyl-7-(2-{[(2S,3S)-2,3,4-trihydroxybutyl]amino}ethoxy)tricyclo[3.3.1.1^{3,7}]dec-1-yl]methyl}-5-methyl-1H-pyrazol-4-yl)pyridine-2-carboxylic acid;
6-[8 -(1,3-benzothiazol-2-ylcarbamoyl)-3,4-dihydroisoquinolin-2(1H)-yl]-3-(1-{[3-(2-{[(2S,3S,4R,5R,6R)-2,3,4,5,6,7-hexahydroxyheptyl]amino}ethoxy)-5,7-dimethyltricyclo[3.3.1.1^{3,7}]dec-1-yl]methyl}-5-methyl-1H-pyrazol-4-yl)pyridine-2-carboxylicacid;
6-[8-(1,3-benzothiazol-2-ylcarbamoyl)-3,4-dihydroisoquinolin-2(1H)-yl]-3-{1-[(3-{2-[({3-[(1,3-dihydroxypropan-2-yl)amino]propyl}sulfonyl)amino]ethoxy}-5,7-dimethyltricyclo[3.3.1.1^{3,7}]dec-1-yl)methyl]-5-methyl-1H-pyrazol-4-yl}pyridine-2-carboxylic acid;
6-[8-(1,3-benzothiazol-2-ylcarbamoyl)-3,4-dihydroisoquinolin-2(1H)-yl]-3-{1-[(3-{2-[(3-{[1,3-dihydroxy-2-(hydroxymethyl)propan-2-yl]amino}-3-oxopropyl)amino]ethoxy}-5,7-dimethyltricyclo[3.3.1.1^{3,7}]dec-1-yl)methyl]-5-methyl-1H-pyrazol-4-yl}pyridine-2-carboxylic acid;
6-[8-(1,3-benzothiazol-2-ylcarbamoyl)-3,4-dihydroisoquinolin-2(1*H*)-yl]-3-(1-{[3-(2-{[(3*S*)-3,4-dihydroxybutyl]amino}ethoxy)-5,7-dimethyltricyclo[3.3.1.1^{3,7}]dec-1-yl]methyl}-5-methyl-1*H-*pyrazol-4-yl)pyridine-2-carboxylic acid;
4-({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl]amino}methyl)phenyl beta-D-glucopyranosiduronic acid;
3-{[2-({3-[(4-{6-[8-(1,3-benzothiazol-2-ylcarbamoyl)naphthalen-2-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl]amino}propyl beta-D-glucopyranosiduronic acid;
6-[4-(1,3-benzothiazol-2-ylcarbamoyl)-2-oxidoisoquinolin-6-yl]-3-[1-({3,5-dimethyl-7-[2-(methylamino)ethoxy]tricyclo[3.3.1.1^{3,7}]dec-1-yl}methyl)-5-methyl-1H-pyrazol-4-yl]pyridine-2-carboxylic acid;
6-{8-[(1,3-benzothiazol-2-yl)carbamoyl]-3,4-dihydroisoquinolin-2(1H)-yl}-3-{1-[(3,5-dimethyl-7-{2-[(2-sulfoethyl)amino]acetamido}tricyclo[3.3.1.1^{3,7}]decan-1-yl)methyl]-5-methyl-1H-pyrazol-4-yl}pyridine-2-carboxylic acid;
6-[8-(1,3-benzothiazol-2-ylcarbamoyl)-3,4-dihydroisoquinolin-2(1*H*)-yl]-3-(1-{[3,5-dimethyl-7-({2-[(2-sulfoethyl)amino]ethyl}sulfanyl)tricyclo[3.3.1.1^{3,7}]dec-1-yl]methyl}-5-methyl-1*H*-pyrazol-4-yl)pyridine-2-carboxylic acid; and
6-{8-[(1,3-benzothiazol-2-yl)carbamoyl]-3,4-dihydroisoquinolin-2(1*H*)-yl}-3-{1-[(3,5-dimethyl-7-{3-[(2-sulfoethyl)amino]propyl}tricyclo[3.3.1.1^{3,7}]decan-1-yl)methyl]-5-methyl-1*H-*pyrazol-4-yl}pyridine-2-carboxylic acid;
and a pharmaceutically acceptable salt thereof.

The Bcl-xL inhibitors bind to and inhibit anti-apoptotic Bcl-xL proteins, inducing apoptosis. The ability of specific Bcl-xL inhibitors according to structural formulae (IIa)-(IId) to bind to and inhibit Bcl-xL activity may be confirmed in standard binding and activity assays, including, for example, the TR-FRET Bcl-xL binding assays described in Tao et al., 2014, ACS Med. Chem. Lett., 5:1088-1093. A specific TR-FRET Bcl-xL binding assay that can be used to confirm Bcl-xL binding is provided in Example 4, below. Typically, Bcl-xL inhibitors useful as inhibitors *per se* and in the ADCs described herein will exhibit a Kᵢ in the binding assay of Example 5 of less than about 1 nM, but may exhibit a significantly lower Kᵢ, for example a Kᵢ of less than about 1, 0.1, or even 0.01 nM.

Bcl-xL inhibitory activity may also be confirmed in standard cell-based cytotoxicity assays, such as the FL5.12 cellular and Molt-4 cytotoxicity assays described in Tao et al., 2014, ACS Med. Chem. Lett., 5:1088-1093. A specific Molt-4 cellular cytotoxicity assay that may be used to confirm Bcl-xL inhibitory activity of specific Bcl-xL inhibitors that are able to permeate cell membranes is provided in Examples 5 and 6, below. Typically, such cell-permeable Bcl-xL inhibitors will exhibit an EC₅₀ of less than about 500 nM in the Molt-4 cytotoxicity assay of Examples 5 and 6, but may exhibit a significantly lower EC₅₀, for example an EC₅₀ of less than about 250, 100, 50, 20, 10 or even 5 nM.

Owing to the presence of solubilizing groups, many of the Bcl-xL inhibitors described herein are expected to exhibit low or very low cell permeability, and therefore will not yield significant activity in certain cellular assays due to the inability of the compound to traverse the cell membrane, including the Molt-4 cellular toxicity assay of Examples 5 and 6. Bcl-xL inhibitory activity of compounds that do not freely traverse cell membranes may be confirmed in cellular assays with permeabilized cells. The process of mitochondrial outer-membrane permeabilization (MOMP) is controlled by the Bcl-2 family proteins. Specifically, MOMP is promoted by the pro-apoptotic Bcl-2 family proteins Bax and Bak which, upon activation oligomerize on the outer mitochondrial membrane and form pores, leading to release of cytochrome c (cyt c). The release of cyt c triggers formulation of the apoptosome which, in turn, results in caspase activation and other events that commit the cell to undergo programmed cell death (*see,* Goldstein et al., 2005, Cell Death and Differentiation 12:453-462). The oligomerization action of Bax and Bak is antagonized by the anti-apoptotic Bcl-2 family members, including Bcl-2 and Bcl-xL. Bcl-xL inhibitors, in cells that depend upon Bcl-xL for survival, can cause activation of Bax and/or Bak, MOMP, release of cyt c and downstream events leading to apoptosis. The process of cyt c release can be measured via western blot of both mitochondrial and cytosolic fractions of cells and used as a proxy measurement of apoptosis in cells.

As a means of detecting Bcl-xL inhibitory activity and consequent release of cyt c for Bcl-xL inhibitors with low cell permeability, the cells can be treated with an agent that causes selective pore formation in the plasma, but not mitochondrial, membrane. Specifically, the cholesterol/phospholipid ratio is much higher in the plasma membrane than the mitochondrial membrane. As a result, short incubation with low concentrations of the cholesterol-directed detergent digitonin selectively permeabilizes the plasma membrane without significantly affecting the mitochondrial membrane. This agent forms insoluble complexes with cholesterol leading to the segregation of cholesterol from its normal phospholipid binding sites. This action, in turn, leads to the formation of holes about 40-50 Å wide in the lipid bilayer. Once the plasma membrane is permeabilized, cytosolic components able to pass over digitonin-formed holes can be washed out, including the cytochrome C that was released from mitochondria to cytosol in the apoptotic cells (Campos, 2006, Cytometry A 69(6):515-523).

Typically, Bcl-xL inhibitors will yield an EC₅₀ of less than about 10 nM in the Molt-4 cell permeabilized cyt c assay of Examples 5 and 6, although the compounds may exhibit significantly lower EC₅₀s, for example, less than about 5, 1, or even 0.5 nM. As demonstrated in Example 6, Bcl-xL inhibitors having low or very low cell permeability that do not exhibit activity in the standard Molt-4 cellular toxicity assay with non-permeabilized cells exhibit potent functional activity, as measured by release of cyt c, in cellular cytotoxicity assays with permeabilized cells. In addition to cytochrome c release, mitochondria undergoing apoptosis frequently lose their transmembrane mitochondrial membrane potential (Bouchier-Hayes et al., 2008, Methods 44(3): 222-228). JC-1 is a cationic carbocyanine dye that accumulates in mitochondria and fluoresces red when mitochondria are healthy and is lost when the mitochondrial membrane is compromised (percentage depolarization; Smiley et al., 1991, Proc. Natl. Acad. Sci. USA, 88: 3671-3675; Reers et al., 1991: Biochemistry, 30: 4480-4486). This loss in signal can be detected in permeabilized cells using a fluorimeter (excitation 545 nm and emission of 590 nm) and is therefore fully quantitative, enhancing both reproducibility and throughput. Typically, Bcl-xL inhibitors will yield an EC₅₀ of less than about 10 nM in the Molt-4 cell permeabilized JC-1 assay of Examples 5 and 6, although the compounds may exhibit significantly lower EC₅₀s, for example, less than about 5, 1, 0.5 or even 0.05 nM. As demonstrated in Example 6, Bcl-xL inhibitors having low or very low cell permeability that do not exhibit activity in the standard Molt-4 cellular toxicity assay with non-permeablized cells exhibit potent functional activity, as measured by their loss of transmembrane mitochondrial membrane potential in the JC-1 assay, in cellular cytotoxicity assays with permeabilized cells. Low permeability Bcl-xL inhibitors also exhibit potent activity when administered to cells in the form of ADCs (*see, e.g.,* Example 8).

Although many of the Bcl-xL inhibitors of structural formulae (IIa)-(IId) selectively or specifically inhibit Bcl-xL over other anti-apoptotic Bcl-2 family proteins, selective and/or specific inhibition of Bcl-xL is not necessary. The Bcl-xL inhibitors and ADCs comprising the compounds may also, in addition to inhibiting Bcl-xL, inhibit one or more other anti-apoptotic Bcl-2 family proteins, such as, for example, Bcl-2. In some embodiments, the Bcl-xL inhibitors and/or ADCs are selective and/or specific for Bcl-xL. By specific or selective is meant that the particular Bcl-xL inhibitor and/or ADC binds or inhibits Bcl-xL to a greater extent than Bcl-2 under equivalent assay conditions. In specific embodiments, the Bcl-xL inhibitors and/or ADCs exhibit in the range of about 10-fold, 100-fold, or even greater specificity or selectivity for Bcl-xL than Bcl-2 in binding assays.

### 3.2. Linkers

In the ADCs described herein, the Bcl-xL inhibitors are linked to the antibody by way of linkers. The linker linking a Bcl-xL inhibitor to the antibody of an ADC may be short, long, hydrophobic, hydrophilic, flexible or rigid, or may be composed of segments that each independently has one or more of the above-mentioned properties such that the linker may include segments having different properties. The linkers may be polyvalent such that they covalently link more than one Bcl-xL inhibitor to a single site on the antibody, or monovalent such that covalently they link a single Bcl-xL inhibitor to a single site on the antibody.

As will be appreciated by skilled artisans, the linkers link the Bcl-xL inhibitors to the antibody by forming a covalent linkage to the Bcl-xL inhibitor at one location and a covalent linkage to antibody at another. The covalent linkages are formed by reaction between functional groups on the linker and functional groups on the inhibitors and antibody. As used herein, the expression "linker" is intended to include (i) unconjugated forms of the linker that include a functional group capable of covalently linking the linker to a Bcl-xL inhibitor and a functional group capable of covalently linking the linker to an antibody; (ii) partially conjugated forms of the linker that include a functional group capable of covalently linking the linker to an antibody and that is covalently linked to a Bcl-xL inhibitor, or *vice versa;* and (iii) fully conjugated forms of the linker that is covalently linked to both a Bcl-xL inhibitor and an antibody. In some specific embodiments of intermediate synthons and ADCs described herein, moieties comprising the functional groups on the linker and covalent linkages formed between the linker and antibody are specifically illustrated as R*^{x}* and LK, respectively.

The linkers are preferably, but need not be, chemically stable to conditions outside the cell, and may be designed to cleave, immolate and/or otherwise specifically degrade inside the cell. Alternatively, linkers that are not designed to specifically cleave or degrade inside the cell may be used. A wide variety of linkers useful for linking drugs to antibodies in the context of ADCs are known in the art. Any of these linkers, as well as other linkers, may be used to link the Bcl-xL inhibitors to the antibody of the ADCs described herein.

Exemplary polyvalent linkers that may be used to link many Bcl-xL inhibitors to an antibody are described, for example, in U.S. Patent No 8,399,512; U.S. Published Application No. 2010/0152725; U.S. Patent No. 8,524,214; U.S. Patent No. 8,349,308; U.S. Published Application No. 2013/189218; U.S. Published Application No. 2014/017265; WO 2014/093379; WO 2014/093394; WO 2014/093640. For example, the Fleximer^{®} linker technology developed by Mersana *et al.* has the potential to enable high-DAR ADCs with good physicochemical properties. As shown below, the Fleximer^{®} linker technology is based on incorporating drug molecules into a solubilizing poly-acetal backbone via a sequence of ester bonds. The methodology renders highly-loaded ADCs (DAR up to 20) whilst maintaining good physicochemical properties. This methodology could be utilized with Bcl-xL inhibitors as shown in the Scheme below.

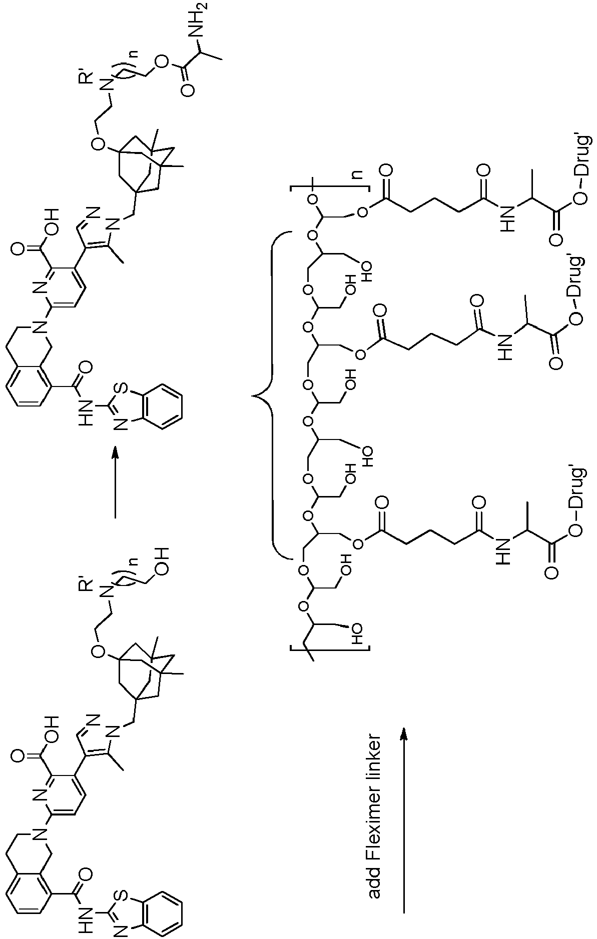

To utilize the Fleximer^{®} linker technology depicted in the scheme above, an aliphatic alcohol can be present or introduced into the Bcl-xL inhibitor. The alcohol moiety is then conjugated to an alanine moiety, which is then synthetically incorporated into the Fleximer^{®} linker. Liposomal processing of the ADC *in vitro* releases the parent alcohol -containing drug.

Additional examples of dendritic type linkers can be found in US 2006/116422; US 2005/271615; de Groot et al., (2003) Angew. Chem. Int. Ed. 42:4490-4494; Amir et al., (2003) Angew. Chem. Int. Ed. 42:4494-4499; Shamis et al., (2004) J. Am. Chem. Soc. 126:1726-1731 ; Sun et al., (2002) Bioorganic & Medicinal Chemistry Letters 12:2213-2215; Sun et al., (2003) Bioorganic & Medicinal Chemistry 11:1761-1768; King et al., (2002) Tetrahedron Letters 43:1987-1990.

Exemplary monovalent linkers that may be used are described, for example, in Nolting, 2013, Antibody-Drug Conjugates, Methods in Molecular Biology 1045:71-100; Kitson et al., 2013, CROs/CMOs - Chemica Oggi - Chemistry Today 31(4): 30-36; Ducry et al., 2010, Bioconjugate Chem. 21:5-13; Zhao et al., 2011, J. Med. Chem. 54:3606-3623; U.S. Patent No. 7,223,837; U.S. Patent No. 8,568,728; U.S. Patent No. 8,535,678; and WO2004010957.

By way of example and not limitation, some cleavable and noncleavable linkers that may be included in the ADCs described herein are described below.

### 3.2.1 Cleavable Linkers

In certain embodiments, the linker selected is cleavable *in vitro* and *in vivo.* Cleavable linkers may include chemically or enzymatically unstable or degradable linkages. Cleavable linkers generally rely on processes inside the cell to liberate the drug, such as reduction in the cytoplasm, exposure to acidic conditions in the lysosome, or cleavage by specific proteases or other enzymes within the cell. Cleavable linkers generally incorporate one or more chemical bonds that are either chemically or enzymatically cleavable while the remainder of the linker is noncleavable.

In certain embodiments, a linker comprises a chemically labile group such as hydrazone and/or disulfide groups. Linkers comprising chemically labile groups exploit differential properties between the plasma and some cytoplasmic compartments. The intracellular conditions to facilitate drug release for hydrazone containing linkers are the acidic environment of endosomes and lysosomes, while the disulfide containing linkers are reduced in the cytosol, which contains high thiol concentrations, e.g., glutathione. In certain embodiments, the plasma stability of a linker comprising a chemically labile group may be increased by introducing steric hindrance using substituents near the chemically labile group.

Acid-labile groups, such as hydrazone, remain intact during systemic circulation in the blood's neutral pH environment (pH 7.3-7.5) and undergo hydrolysis and release the drug once the ADC is internalized into mildly acidic endosomal (pH 5.0-6.5) and lysosomal (pH 4.5-5.0) compartments of the cell. This pH dependent release mechanism has been associated with nonspecific release of the drug. To increase the stability of the hydrazone group of the linker, the linker may be varied by chemical modification, e.g., substitution, allowing tuning to achieve more efficient release in the lysosome with a minimized loss in circulation.

Hydrazone-containing linkers may contain additional cleavage sites, such as additional acid-labile cleavage sites and/or enzymatically labile cleavage sites. ADCs including exemplary hydrazone-containing linkers include the following structures: 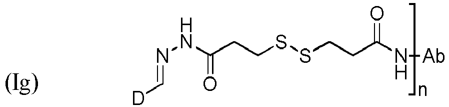 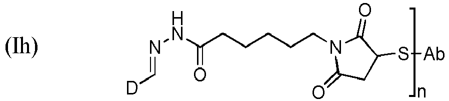 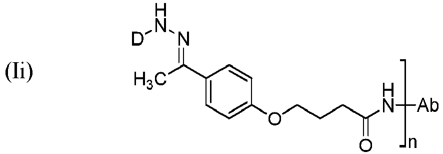 wherein D and Ab represent the drug and Ab, respectively, and n represents the number of drug-linkers linked to the antibody. In certain linkers such as linker (Ig), the linker comprises two cleavable groups - a disulfide and a hydrazone moiety. For such linkers, effective release of the unmodified free drug requires acidic pH or disulfide reduction and acidic pH. Linkers such as (Ih) and (Ii) have been shown to be effective with a single hydrazone cleavage site.

Other acid-labile groups that may be included in linkers include *cis*-aconityl-containing linkers. *cis*-Aconityl chemistry uses a carboxylic acid juxtaposed to an amide bond to accelerate amide hydrolysis under acidic conditions.

Cleavable linkers may also include a disulfide group. Disulfides are thermodynamically stable at physiological pH and are designed to release the drug upon internalization inside cells, wherein the cytosol provides a significantly more reducing environment compared to the extracellular environment. Scission of disulfide bonds generally requires the presence of a cytoplasmic thiol cofactor, such as (reduced) glutathione (GSH), such that disulfide-containing linkers are reasonable stable in circulation, selectively releasing the drug in the cytosol. The intracellular enzyme protein disulfide isomerase, or similar enzymes capable of cleaving disulfide bonds, may also contribute to the preferential cleavage of disulfide bonds inside cells. GSH is reported to be present in cells in the concentration range of 0.5-10 mM compared with a significantly lower concentration of GSH or cysteine, the most abundant low-molecular weight thiol, in circulation at approximately 5 µM. Tumor cells, where irregular blood flow leads to a hypoxic state, result in enhanced activity of reductive enzymes and therefore even higher glutathione concentrations. In certain embodiments, the *in vivo* stability of a disulfide-containing linker may be enhanced by chemical modification of the linker, *e.g.,* use of steric hindrance adjacent to the disulfide bond.

ADCs including exemplary disulfide-containing linkers include the following structures: 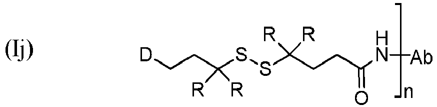 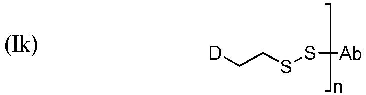 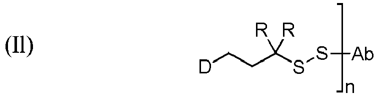 wherein D and Ab represent the drug and antibody, respectively, n represents the number of drug-linkers linked to the antibody and R is independently selected at each occurrence from hydrogen or alkyl, for example. In certain embodiments, increasing steric hindrance adjacent to the disulfide bond increases the stability of the linker. Structures such as (Ij) and (Il) show increased *in vivo* stability when one or more R groups is selected from a lower alkyl such as methyl.

Another type of linker that may be used is a linker that is specifically cleaved by an enzyme. Such linkers are typically peptide-based or include peptidic regions that act as substrates for enzymes. Peptide based linkers tend to be more stable in plasma and extracellular milieu than chemically labile linkers. Peptide bonds generally have good serum stability, as lysosomal proteolytic enzymes have very low activity in blood due to endogenous inhibitors and the unfavorably high pH value of blood compared to lysosomes. Release of a drug from an antibody occurs specifically due to the action of lysosomal proteases, e.g., cathepsin and plasmin. These proteases may be present at elevated levels in certain tumor tissues. In certain embodiments, the linker is cleavable by a lysosomal enzyme. In certain embodiments, the linker is cleavable by a lysosomal enzyme, and the lysosomal enzyme is Cathepsin B.. In certain embodiments, the linker is cleavable by a lysosomal enzyme, and the lysosomal enzyme is β-glucuronidase or β-galactosidase. In certain embodiments, the linker is cleavable by a lysosomal enzyme, and the lysosomal enzyme is β-glucuronidase. In certain embodiments, the linker is cleavable by a lysosomal enzyme, and the lysosomal enzyme is β-galactosidase.

Those skilled in the art recognize the importance of cleavable linkers that are stable to plasma, yet are readily cleaved by a lysosomal enzyme. Disclosed herein, in certain embodiments, are linkers, cleavable by the lysosomal enzymes β-glucuronidase or β-galactosidase, that show improved plasma stability and reduced non-specific release of small molecule drug.

In exemplary embodiments, the cleavable peptide is selected from tetrapeptides such as Gly-Phe-Leu-Gly, Ala-Leu-Ala-Leu or dipeptides such as Val-Cit, Val-Ala, and Phe-Lys. In certain embodiments, dipeptides are preferred over longer polypeptides due to hydrophobicity of the longer peptides.

A variety of dipeptide-based cleavable linkers useful for linking drugs such as doxorubicin, mitomycin, camptothecin, tallysomycin and auristatin/auristatin family members to antibodies have been described (*see,* Dubowchik et al., 1998, J. Org. Chem. 67:1866-1872; Dubowchik et al., 1998, Bioorg. Med. Chem. Lett. 8:3341-3346; Walker et al., 2002, Bioorg. Med. Chem. Lett. 12:217-219; Walker et al., 2004, Bioorg. Med. Chem. Lett. 14:4323-4327; and Francisco et al., 2003, Blood 102:1458-1465). All of these dipeptide linkers, or modified versions of these dipeptide linkers, may be used in the ADCs described herein. Other dipeptide linkers that may be used include those found in ADCs such as Seattle Genetics' Brentuximab Vendotin SGN-35 (Adcetris^{™}), Seattle Genetics SGN-75 (anti-CD-70, MC-monomethyl auristatin F(MMAF), Celldex Therapeutics glembatumumab (CDX-011) (anti-NMB, Val-Cit- monomethyl auristatin E(MMAE), and Cytogen PSMA-ADC (PSMA-ADC-1301) (anti-PSMA, Val-Cit-MMAE).

Enzymatically cleavable linkers may include a self-immolative spacer to spatially separate the drug from the site of enzymatic cleavage. The direct attachment of a drug to a peptide linker can result in proteolytic release of an amino acid adduct of the drug, thereby impairing its activity. The use of a self-immolative spacer allows for the elimination of the fully active, chemically unmodified drug upon amide bond hydrolysis.

One self-immolative spacer is the bifunctional *para*-aminobenzyl alcohol group, which is linked to the peptide through the amino group, forming an amide bond, while amine containing drugs may be attached through carbamate functionalities to the benzylic hydroxyl group of the linker (to give a *p*-amidobenzylcarbamate, PABC). The resulting prodrugs are activated upon protease-mediated cleavage, leading to a 1,6-elimination reaction releasing the unmodified drug, carbon dioxide, and remnants of the linker group. The following scheme depicts the fragmentation of *p-*amidobenzyl carbamate and release of the drug: 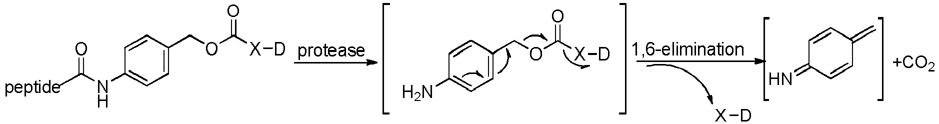 wherein X-D represents the unmodified drug. Heterocyclic variants of this self-immolative group have also been described. *See* U.S. Patent No. 7,989,434.

In certain embodiments, the enzymatically cleavable linker is a β-glucuronic acid-based linker. Facile release of the drug may be realized through cleavage of the β-glucuronide glycosidic bond by the lysosomal enzyme β-glucuronidase. This enzyme is present abundantly within lysosomes and is overexpressed in some tumor types, while the enzyme activity outside cells is low. β-Glucuronic acid-based linkers may be used to circumvent the tendency of an ADC to undergo aggregation due to the hydrophilic nature of β-glucuronides. In certain embodiments, β-glucuronic acid-based linkers are preferred as linkers for ADCs linked to hydrophobic drugs. The following scheme depicts the release of the drug from and ADC containing a β-glucuronic acid-based linker:

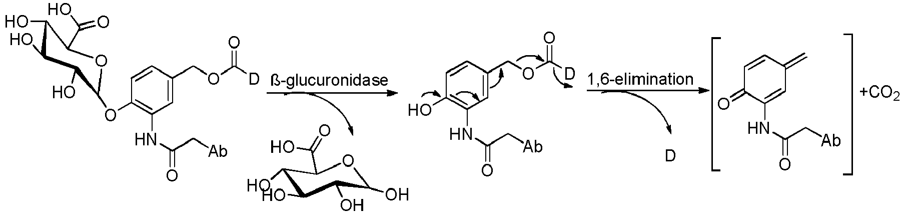

A variety of cleavable β-glucuronic acid-based linkers useful for linking drugs such as auristatins, camptothecin and doxorubicin analogues, CBI minor-groove binders, and psymberin to antibodies have been described (*see,* Jeffrey et al., 2006, Bioconjug. Chem. 17:831-840; Jeffrey et al., Bioorg. Med. Chem. Lett. 17:2278-2280; and Jiang et al., 2005, J. Am. Chem. Soc. 127:11254-11255). All of these β-glucuronic acid-based linkers may be used in the ADCs described herein. In certain embodiments, the enzymatically cleavable linker is a β-galactoside-based linker. β-Galactoside is present abundantly within lysosomes, while the enzyme activity outside cells is low. Additionally, Bcl-xL inhibitors containing a phenol group can be covalently bonded to a linker through the phenolic oxygen. One such linker, described in U.S. Published App. No. 2009/0318668, relies on a methodology in which a diamino-ethane "SpaceLink" is used in conjunction with traditional "PABO"-based self-immolative groups to deliver phenols. The cleavage of the linker is depicted schematically below using a Bcl-xL inhibitor of the disclosure.

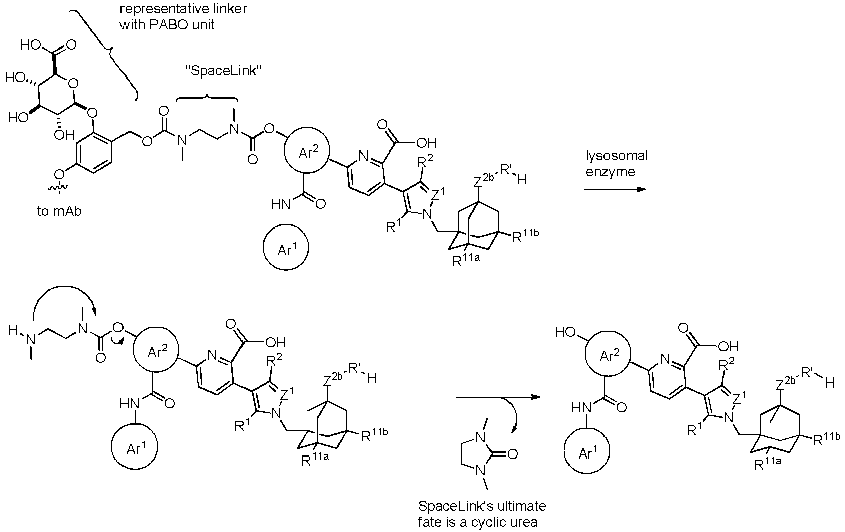

Cleavable linkers may include noncleavable portions or segments, and/or cleavable segments or portions may be included in an otherwise non-cleavable linker to render it cleavable. By way of example only, polyethylene glycol (PEG) and related polymers may include cleavable groups in the polymer backbone. For example, a polyethylene glycol or polymer linker may include one or more cleavable groups such as a disulfide, a hydrazone or a dipeptide.

Other degradable linkages that may be included in linkers include ester linkages formed by the reaction of PEG carboxylic acids or activated PEG carboxylic acids with alcohol groups on a biologically active agent, wherein such ester groups generally hydrolyze under physiological conditions to release the biologically active agent. Hydrolytically degradable linkages include, but are not limited to, carbonate linkages; imine linkages resulting from reaction of an amine and an aldehyde; phosphate ester linkages formed by reacting an alcohol with a phosphate group; acetal linkages that are the reaction product of an aldehyde and an alcohol; orthoester linkages that are the reaction product of a formate and an alcohol; and oligonucleotide linkages formed by a phosphoramidite group, including but not limited to, at the end of a polymer, and a 5' hydroxyl group of an oligonucleotide.

In certain embodiments, the linker comprises an enzymatically cleavable peptide moiety, for example, a linker comprising structural formula (IVa), (IVb), (IVc) or (IVd): 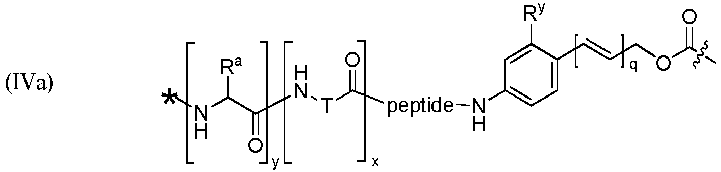 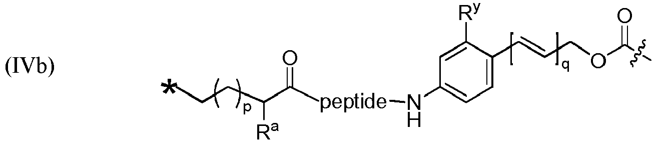 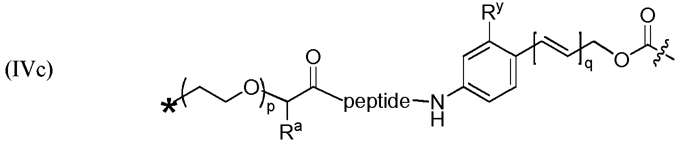 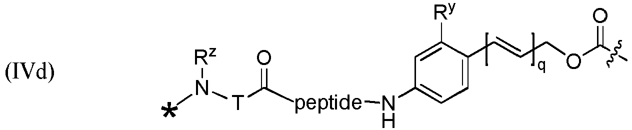 or a pharmaceutically acceptable salt thereof, wherein:
peptide represents a peptide (illustrated N→C, wherein peptide includes the amino and
carboxy "termini") cleavable by a lysosomal enzyme;
T represents a polymer comprising one or more ethylene glycol units or an alkylene chain, or combinations thereof;
R^{a} is selected from hydrogen, C₁₋₆ alkyl, SO₃H and CH₂SO₃H;
R^{y} is hydrogen or C₁₋₄ alkyl-(O)ᵣ-(C₁₋₄ alkylene)ₛ-G¹ or C₁₋₄ alkyl-(N)-[(C₁₋₄ alkylene)-G¹]₂;
R^{z} is C₁₋₄ alkyl-(O)ᵣ-(C₁₋₄ alkylene)ₛ-G²;
   G¹ is SO₃H, CO₂H, PEG 4-32, or sugar moiety;
   G² is SO₃H, CO₂H, or PEG 4-32 moiety;
   r is 0 or 1;
   s is 0 or 1;
   p is an integer ranging from 0 to 5;
   q is 0 or 1;
   x is 0 or 1;
   y is 0 or 1;
   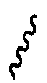 represents the point of attachment of the linker to the Bcl-xL inhibitor; and
   * represents the point of attachment to the remainder of the linker.

In certain embodiments, the linker comprises an enzymatically cleavable peptide moiety, for example, a linker comprising structural formula (IVa), (IVb), (IVc), or (IVd), or a pharmaceutically acceptable salt thereof.

In certain embodiments, the peptide is selected from a tripeptide or a dipeptide. In particular embodiments, the dipeptide is selected from: Val-Cit; Cit-Val; Ala-Ala; Ala-Cit; Cit-Ala; Asn-Cit; Cit-Asn; Cit-Cit; Val-Glu; Glu-Val; Ser-Cit; Cit-Ser; Lys-Cit; Cit-Lys; Asp-Cit; Cit-Asp; Ala-Val; Val-Ala; Phe-Lys; Lys-Phe; Val-Lys; Lys-Val; Ala-Lys; Lys-Ala; Phe-Cit; Cit-Phe; Leu-Cit; Cit-Leu; Ile-Cit; Cit-Ile; Phe-Arg; Arg-Phe; Cit-Trp; and Trp-Cit; or a pharmaceutically acceptable salt thereof.

Exemplary embodiments of linkers according to structural formula (IVa) that may be included in the ADCs described herein include the linkers illustrated below (as illustrated, the linkers include a group suitable for covalently linking the linker to an antibody):

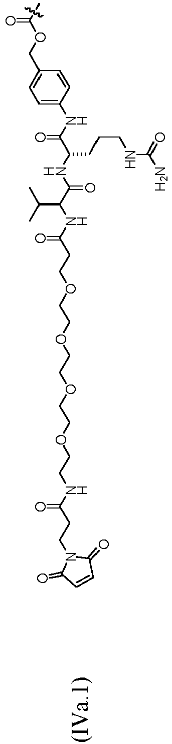

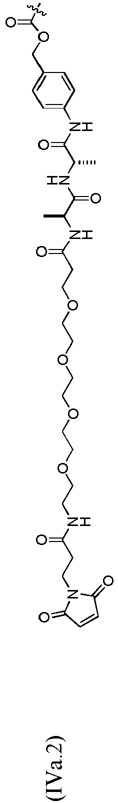

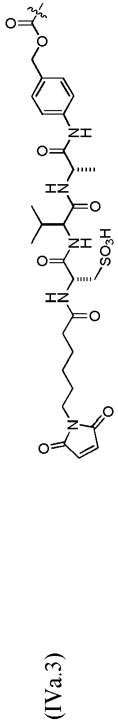

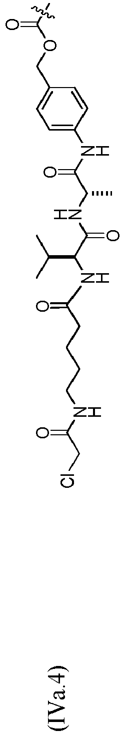

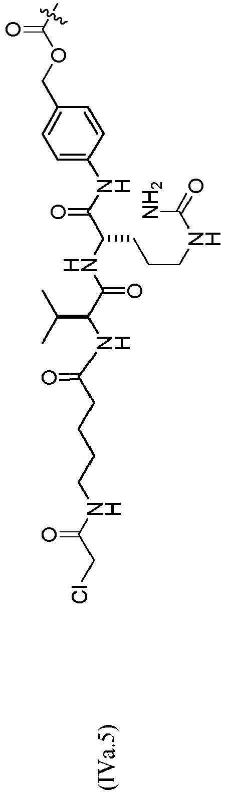

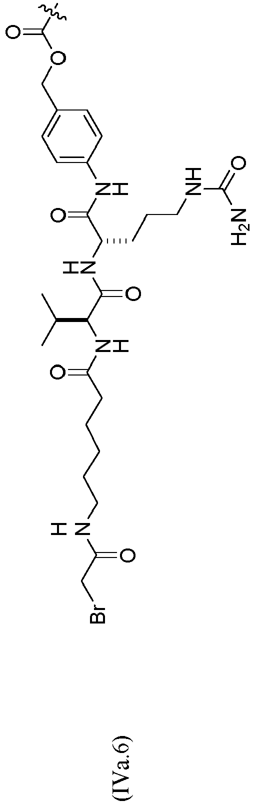

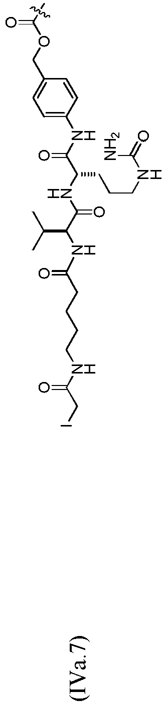

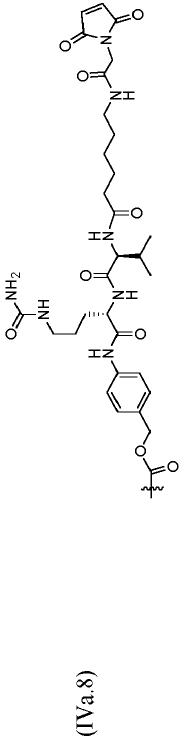

Exemplary embodiments of linkers according to structural formula (IVb), (IVc), or (IVd) that may be included in the ADCs described herein include the linkers illustrated below (as illustrated, the linkers include a group suitable for covalently linking the linker to an antibody):

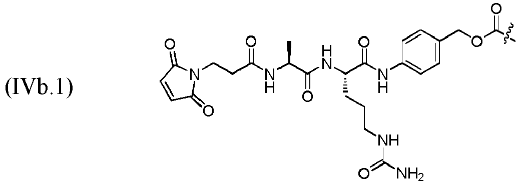

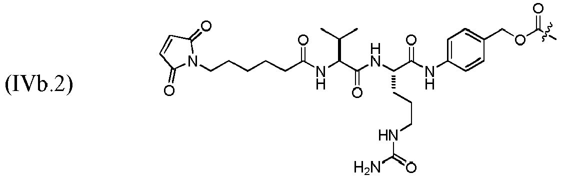

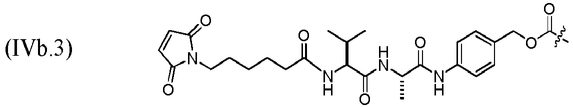

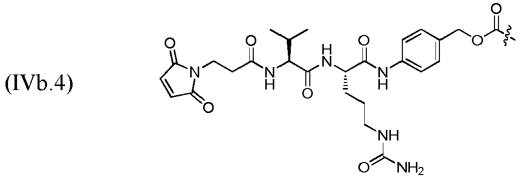

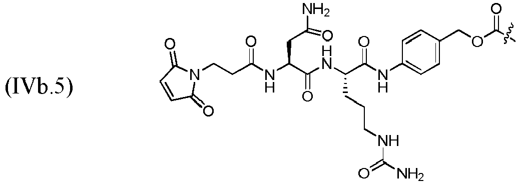

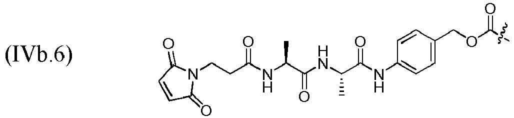

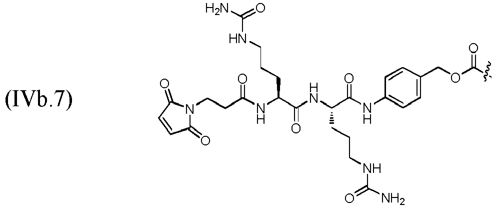

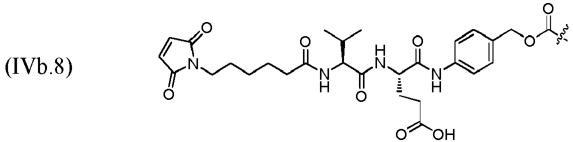

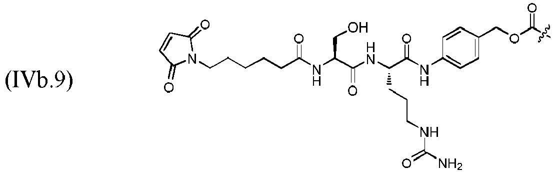

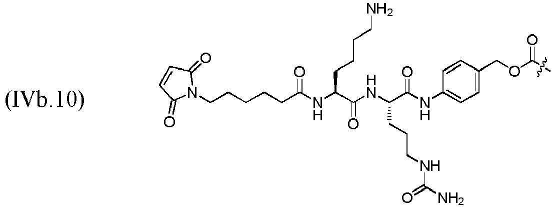

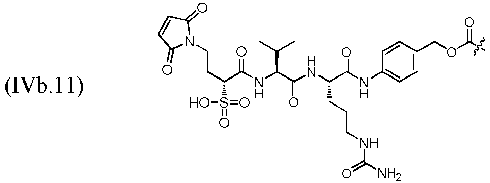

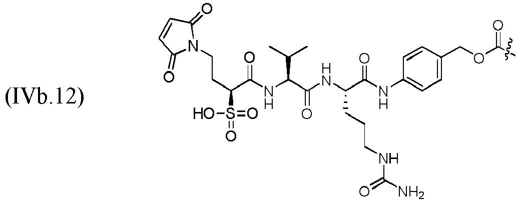

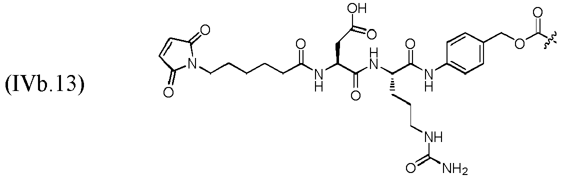

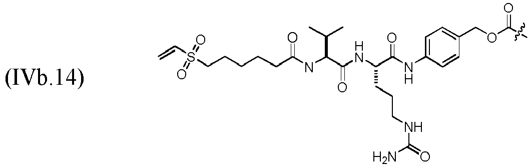

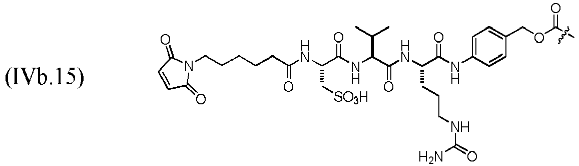

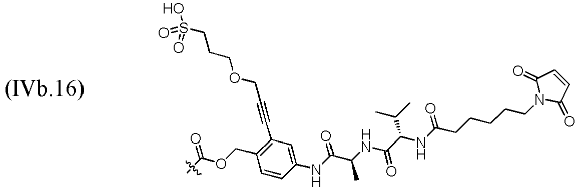

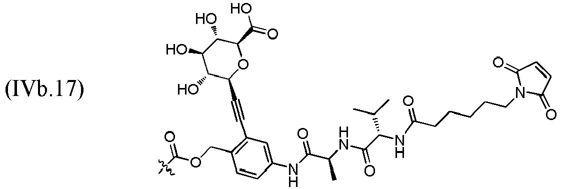

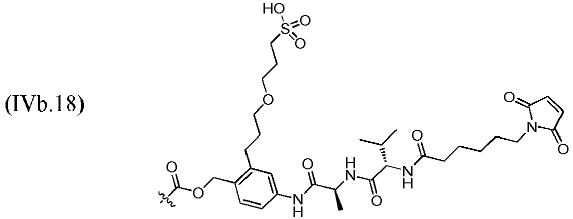

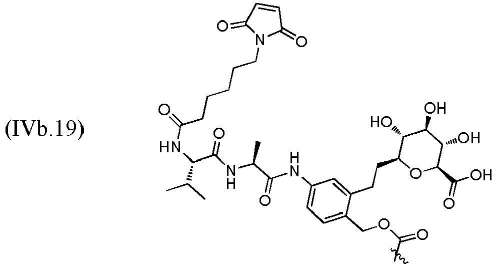

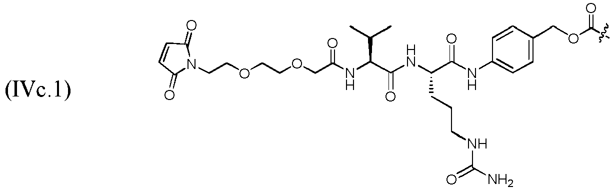

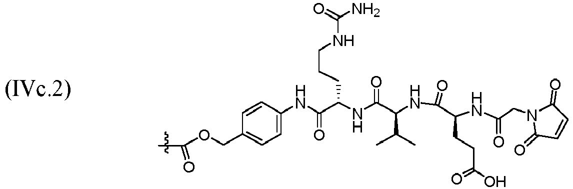

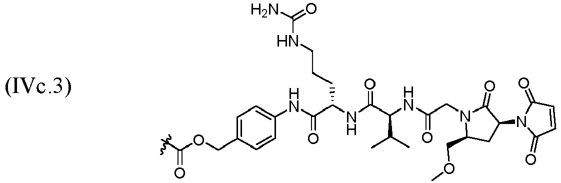

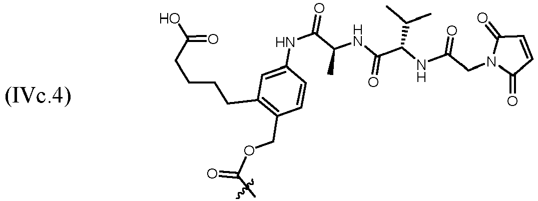

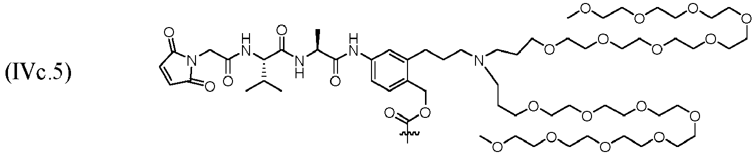

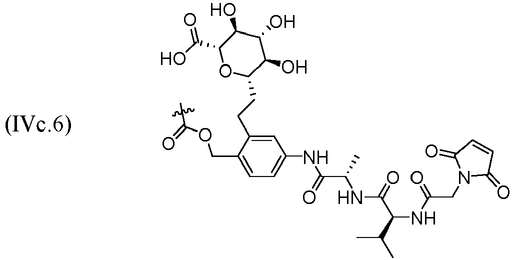

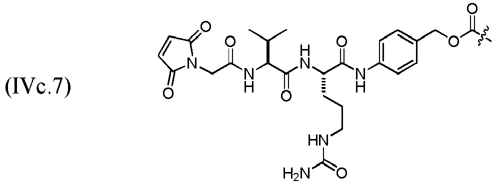

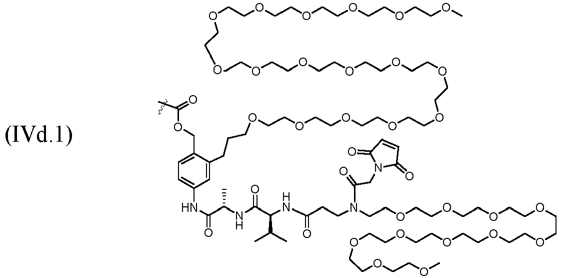

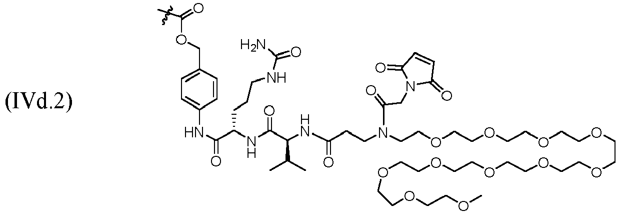

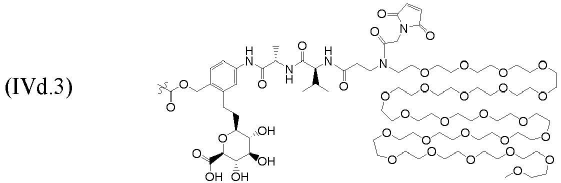

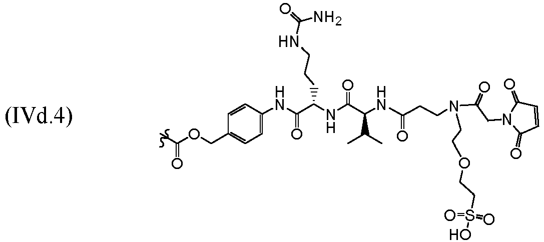

In certain embodiments, the linker comprises an enzymatically cleavable sugar moiety, for example, a linker comprising structural formula (Va), (Vb), (Vc), (Vd), or (Ve): 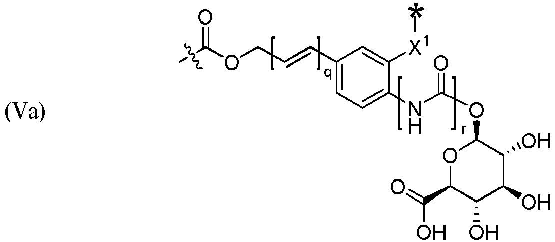 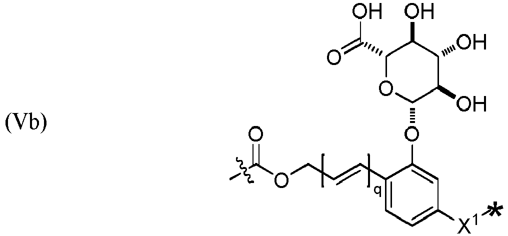 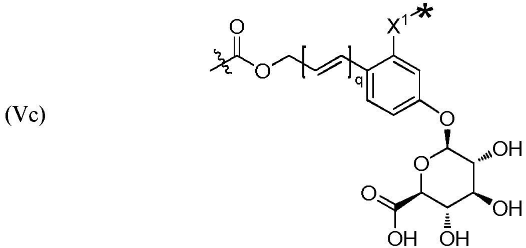 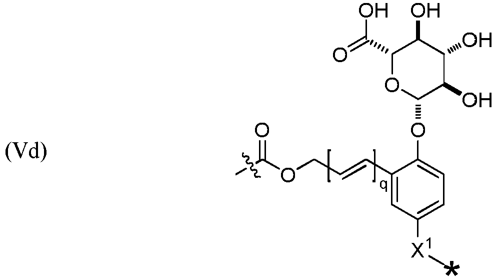 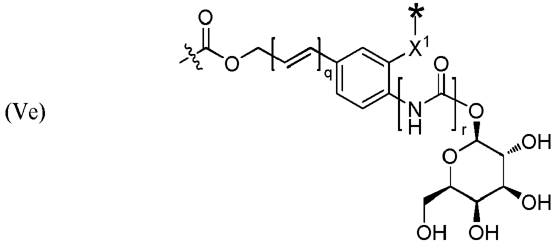 or a pharmaceutically acceptable salt thereof, wherein:
q is 0 or 1;
r is 0 or 1;
X¹ is CH₂, O or NH; 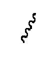
represents the point of attachment of the linker to the drug; and
^{∗} represents the point of attachment to the remainder of the linker.

Exemplary embodiments of linkers according to structural formula (Va) that may be included in the ADCs described herein include the linkers illustrated below (as illustrated, the linkers include a group suitable for covalently linking the linker to an antibody):

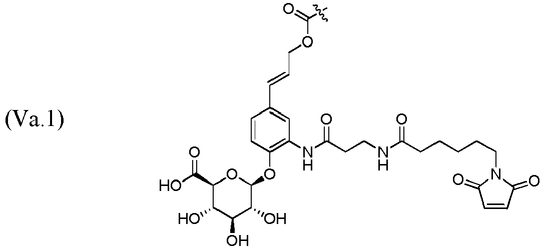

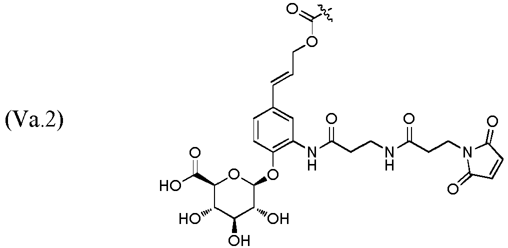

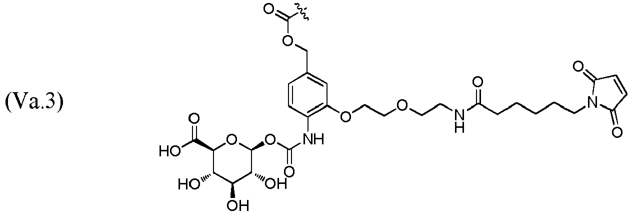

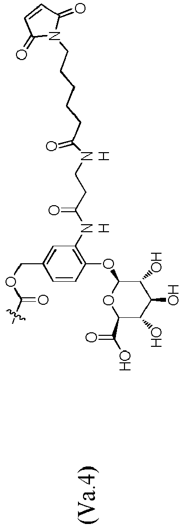

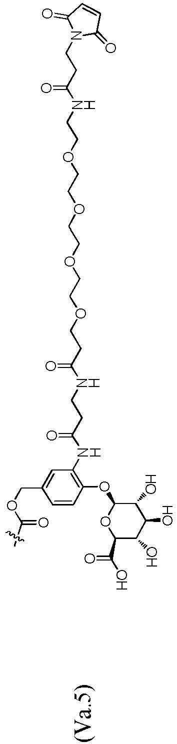

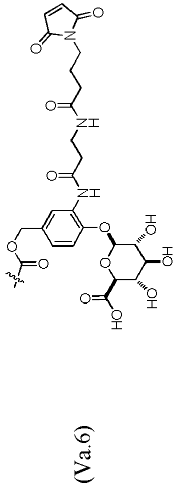

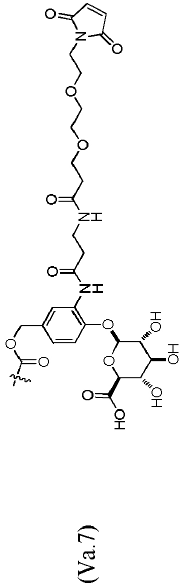

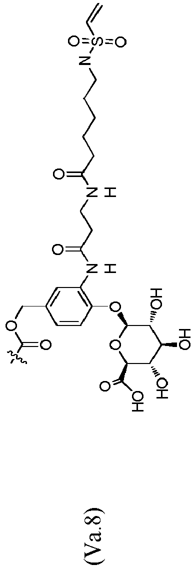

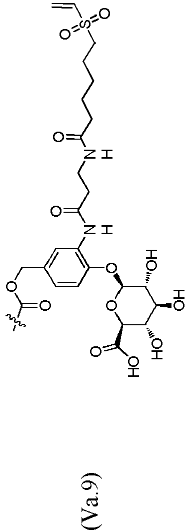

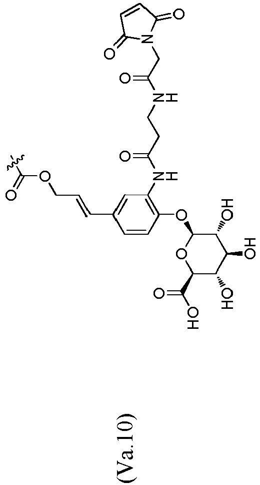

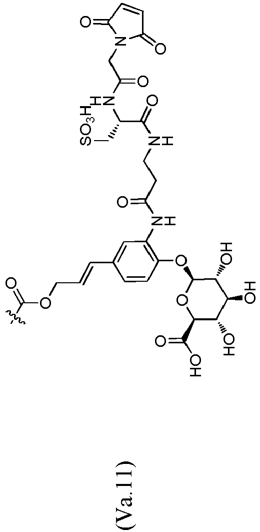

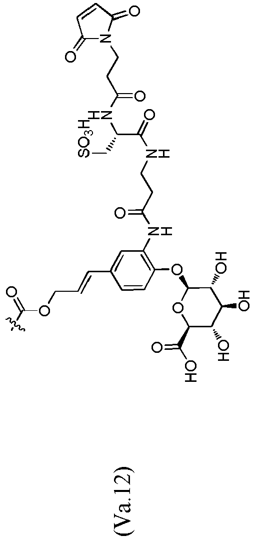

Exemplary embodiments of linkers according to structural formula (Vb) that may be included in the ADCs described herein include the linkers illustrated below (as illustrated, the linkers include a group suitable for covalently linking the linker to an antibody):

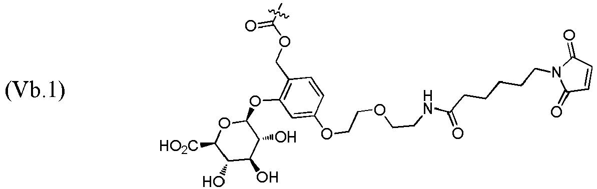

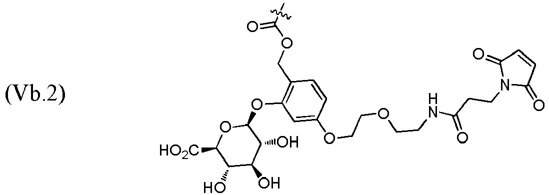

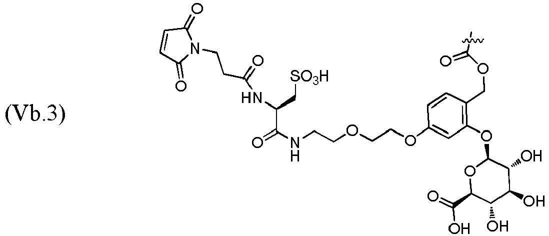

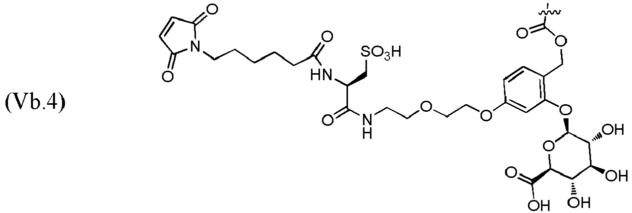

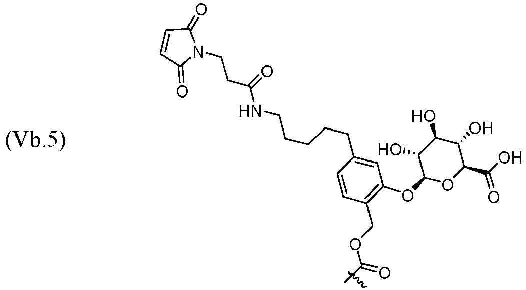

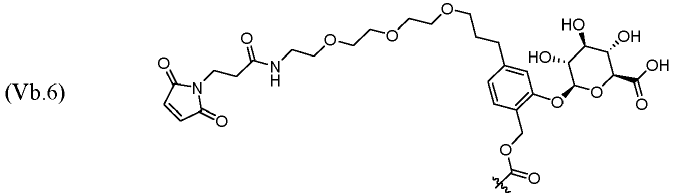

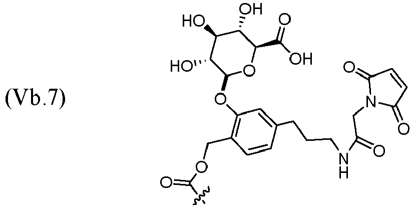

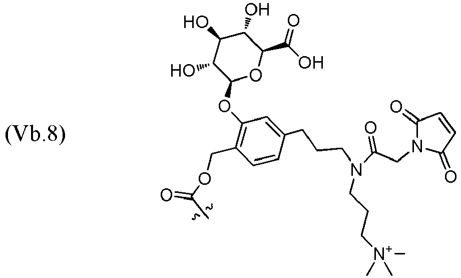

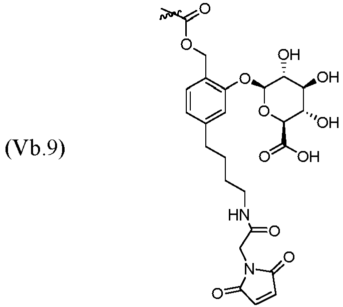

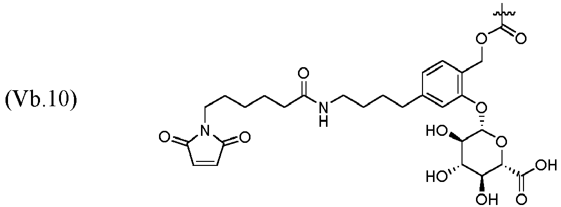

Exemplary embodiments of linkers according to structural formula (Vc) that may be included in the ADCs described herein include the linkers illustrated below (as illustrated, the linkers include a group suitable for covalently linking the linker to an antibody):

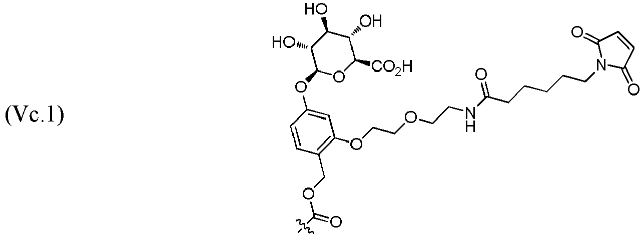

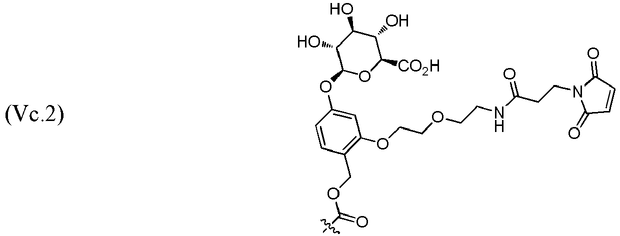

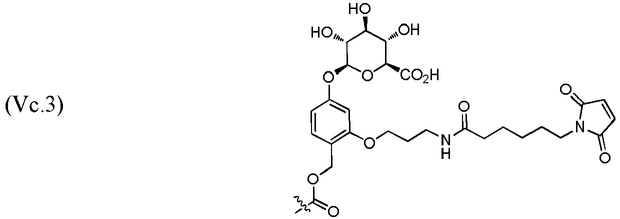

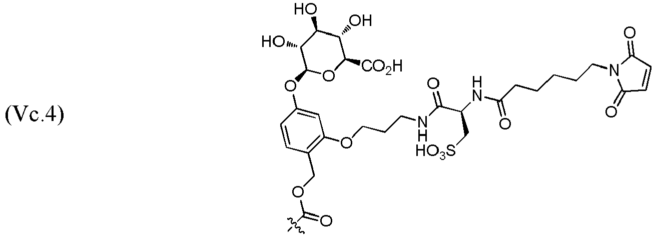

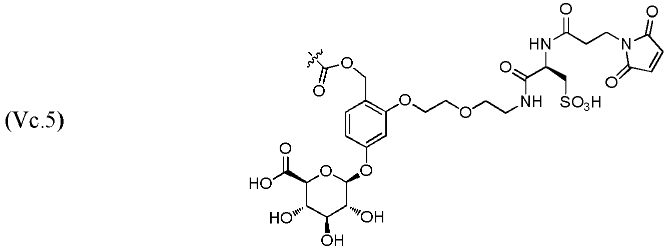

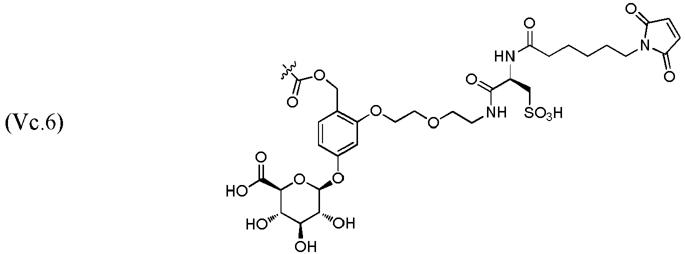

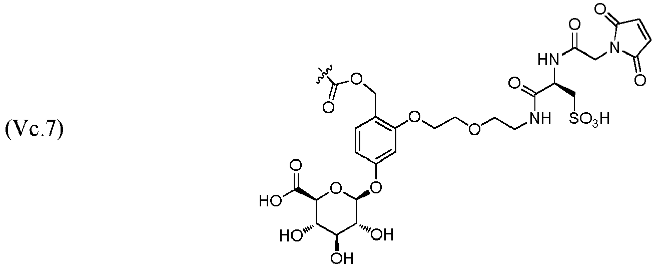

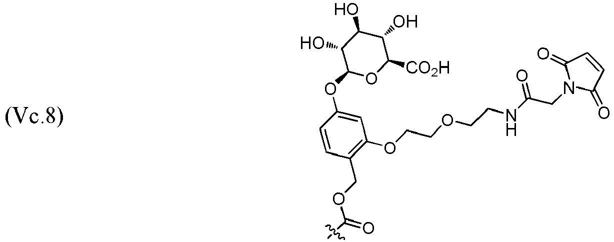

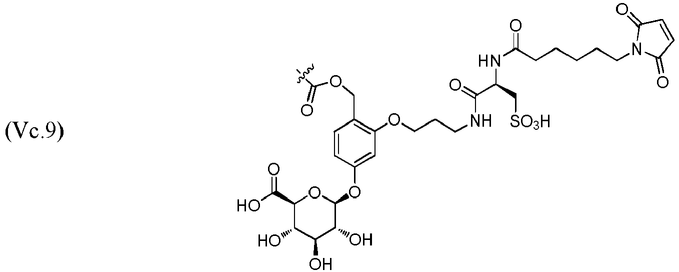

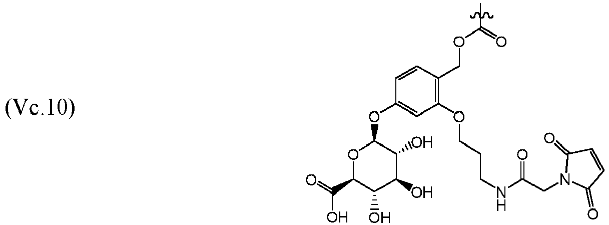

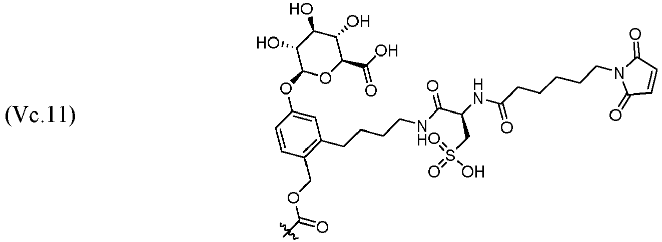

Exemplary embodiments of linkers according to structural formula (Vd) that may be included in the ADCs described herein include the linkers illustrated below (as illustrated, the linkers include a group suitable for covalently linking the linker to an antibody):

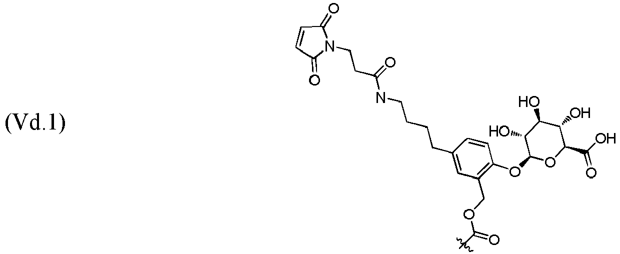

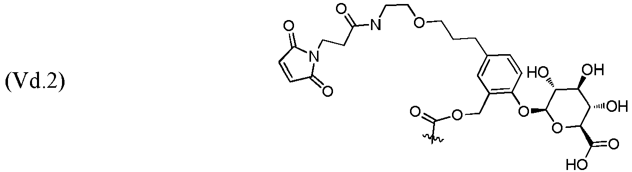

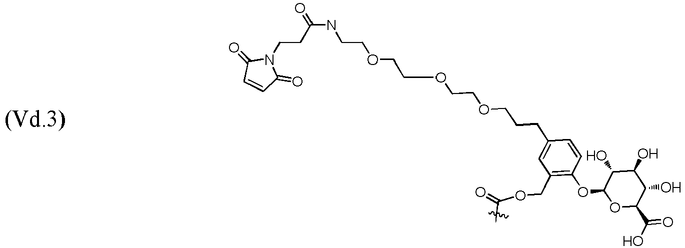

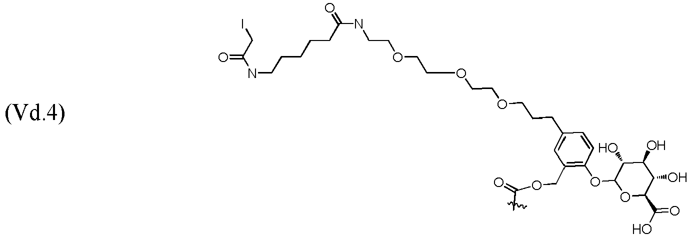

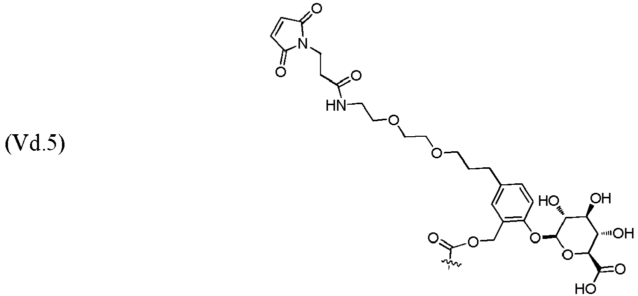

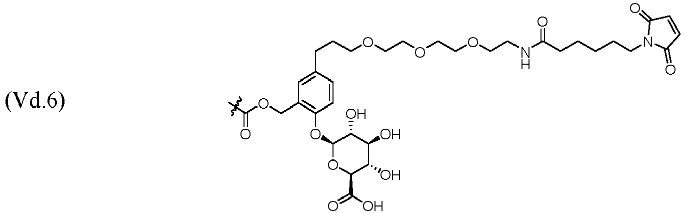

Exemplary embodiments of linkers according to structural formula (Ve) that may be included in the ADCs described herein include the linkers illustrated below (as illustrated, the linkers include a group suitable for covalently linking the linker to an antibody):

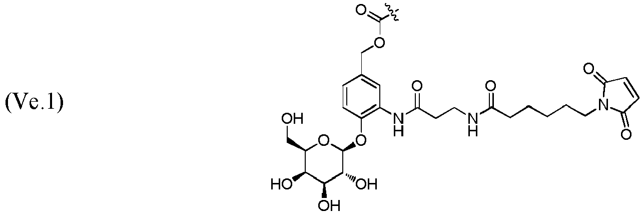

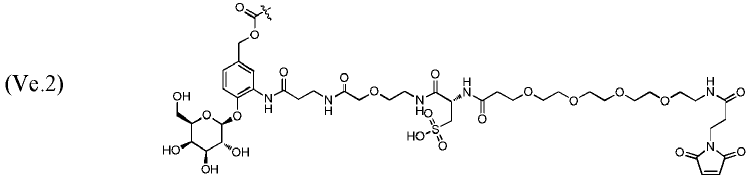

### 3.2.2 Non-Cleavable Linkers

Although cleavable linkers may provide certain advantages, the linkers comprising the ADC described herein need not be cleavable. For noncleavable linkers, the drug release does not depend on the differential properties between the plasma and some cytoplasmic compartments. The release of the drug is postulated to occur after internalization of the ADC via antigen-mediated endocytosis and delivery to lysosomal compartment, where the antibody is degraded to the level of amino acids through intracellular proteolytic degradation. This process releases a drug derivative, which is formed by the drug, the linker, and the amino acid residue to which the linker was covalently attached. The amino-acid drug metabolites from conjugates with noncleavable linkers are more hydrophilic and generally less membrane permeable, which leads to less bystander effects and less nonspecific toxicities compared to conjugates with a cleavable linker. In general, ADCs with noncleavable linkers have greater stability in circulation than ADCs with cleavable linkers. Non-cleavable linkers may be alkylene chains, or maybe polymeric in natures, such as, for example, based upon polyalkylene glycol polymers, amide polymers, or may include segments of alkylene chains, polyalkylene glycols and/or amide polymers. In certain embodiments, the linker comprises a polyethylene glycol segment having from 1 to 6 ethylene glycol units.

A variety of non-cleavable linkers used to link drugs to antibodies have been described. (*See,* Jeffrey et al., 2006, Bioconjug. Chem. 17;831-840; Jeffrey et al., 2007, Bioorg. Med. Chem. Lett. 17:2278-2280; and Jiang et al., 2005, J. Am . Chem. Soc. 127:11254-11255). All of these linkers may be included in the ADCs described herein.

In certain embodiments, the linker is non-cleavable *in vivo,* for example a linker according to structural formula (VIa), (VIb), (VIc) or (VId) (as illustrated, the linkers include a group suitable for covalently linking the linker to an antibody: 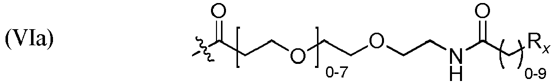 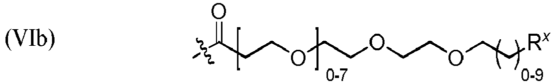 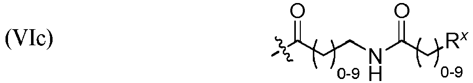 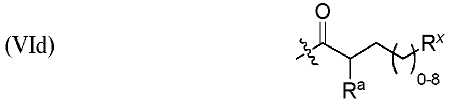 or a pharmaceutically acceptable salt thereof, , wherein:
R^{a} is selected from hydrogen, alkyl, sulfonate and methyl sulfonate;
R*^{x}* is a moiety including a functional group capable of covalently linking the linker to an antibody; and 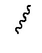
represents the point of attachment of the linker to the Bcl-xL inhibitor.

Exemplary embodiments of linkers according to structural formula (VIa)-(VId) that may be included in the ADCs described herein include the linkers illustrated below (as illustrated, the linkers include a group suitable for covalently linking the linker to an antibody, and " 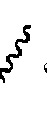 " represents the point of attachment to a Bcl-xL inhibitor):

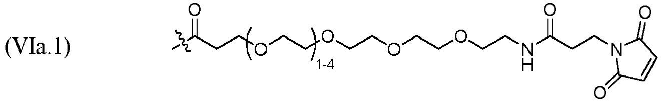

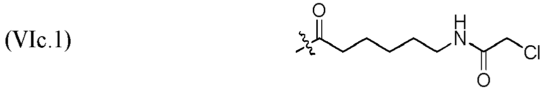

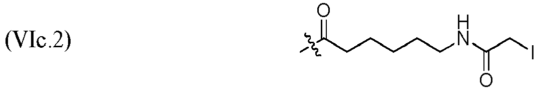

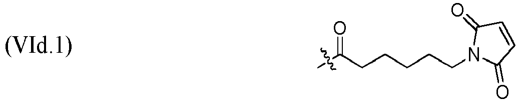

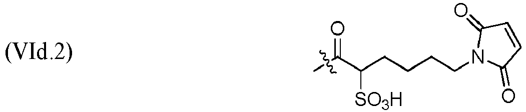

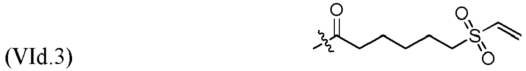

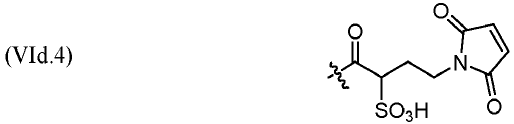

### 3.2.3 Groups Used to Attach Linkers to Anti-EGFR Antibodies

Attachment groups can be electrophilic in nature and include: maleimide groups, activated disulfides, active esters such as NHS esters and HOBt esters, haloformates, acid halides, alkyl and benzyl halides such as haloacetamides. As discussed below, there are also emerging technologies related to "self-stabilizing" maleimides and "bridging disulfides" that can be used in accordance with the disclosure.

Loss of the drug-linker from the ADC has been observed as a result of a maleimide exchange process with albumin, cysteine or glutathione (Alley et al., 2008, Bioconjugate Chem. 19: 759-769). This is particularly prevalent from highly solvent-accessible sites of conjugation while sites that are partially accessible and have a positively charged environment promote maleimide ring hydrolysis (Junutula et al., 2008, Nat. Biotechnol. 26: 925-932). A recognized solution is to hydrolyze the succinimide formed from conjugation as this is resistant to deconjugation from the antibody, thereby making the ADC stable in serum. It has been reported previously that the succinimide ring will undergo hydrolysis under alkaline conditions (Kalia et al., 2007, Bioorg. Med. Chem. Lett. 17: 6286-6289). One example of a "self-stabilizing" maleimide group that hydrolyzes spontaneously under antibody conjugation conditions to give an ADC species with improved stability is depicted in the schematic below. *See* U.S. Published Application No. 2013/0309256, International Application Publication No. WO 2013/173337, Tumey et al., 2014, Bioconjugate Chem. 25: 1871-1880, and Lyon et al., 2014, Nat. Biotechnol. 32: 1059-1062. Thus, the maleimide attachment group is reacted with a sulfhydryl of an antibody to give an intermediate succinimide ring. The hydrolyzed form of the attachment group is resistant to deconjugation in the presence of plasma proteins.

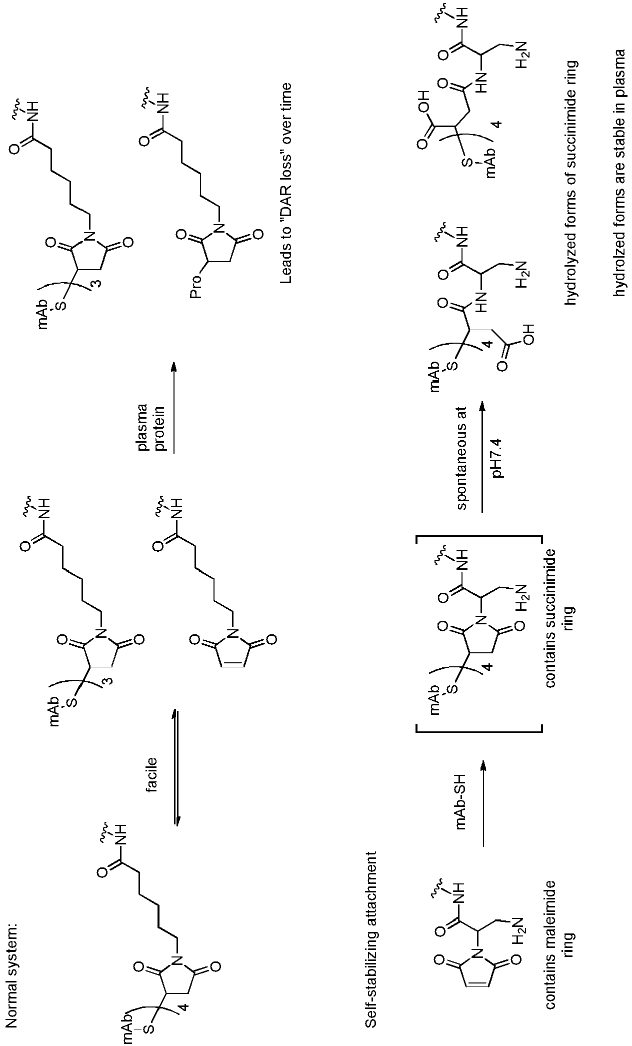

As shown above, the maleimide ring of a linker may react with an antibody Ab, forming a covalent attachment as either a succinimide (closed form) or succinamide (open form).

Polytherics has disclosed a method for bridging a pair of sulfhydryl groups derived from reduction of a native hinge disulfide bond. *See,* Badescu et al., 2014, Bioconjugate Chem. 25:1124-1136. The reaction is depicted in the schematic below. An advantage of this methodology is the ability to synthesize homogenous DAR4 ADCs by full reduction of IgGs (to give 4 pairs of sulfhydryls) followed by reaction with 4 equivalents of the alkylating agent. ADCs containing "bridged disulfides" are also claimed to have increased stability.

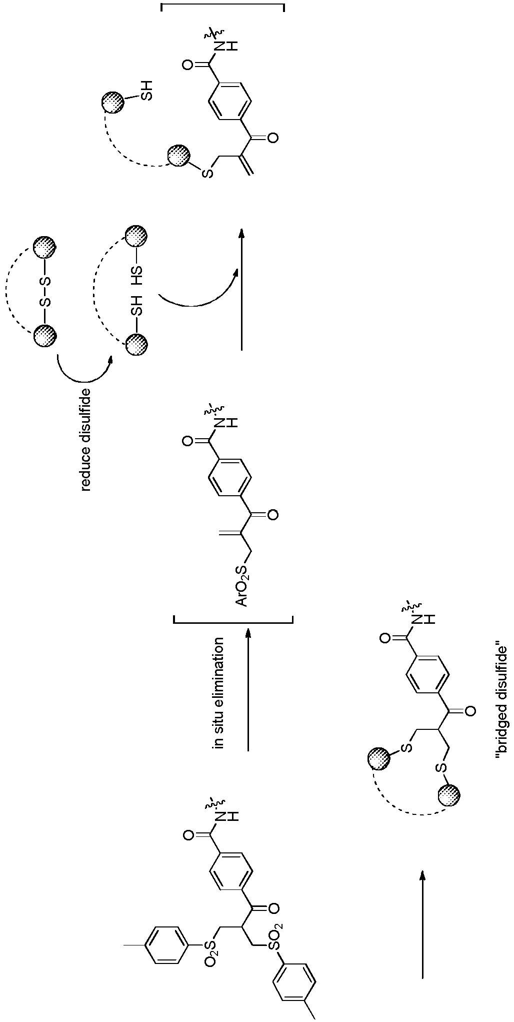

Similarly, as depicted below, a maleimide derivative that is capable of bridging a pair of sulfhydryl groups has been developed. *See* U.S. Published Application No. 2013/0224228.

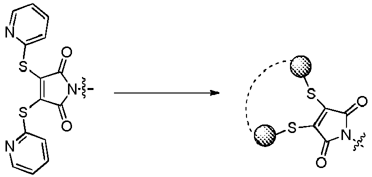

In certain embodiments the attachment moiety comprises the structural formulae (VIIa), (VIIb), or (VIIc): 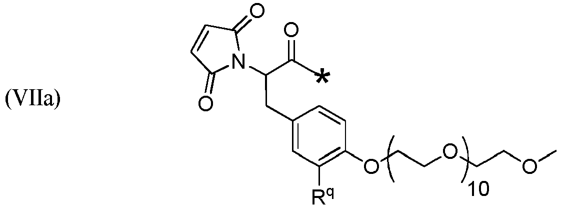 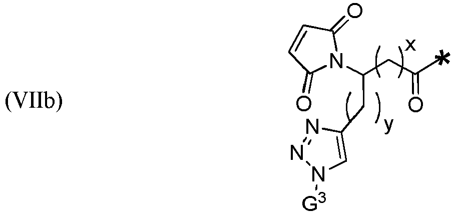 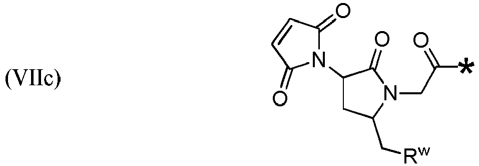 or a pharmaceutically acceptable salt thereof, wherein:
R^{q} is H or -O-(CH₂CH₂O)₁₁-CH₃;
x is 0 or 1;
y is 0 or 1;
G³ is -CH₂CH₂CH₂SO₃H or -CH₂CH₂O-(CH₂CH₂O)₁₁-CH₃;
R^{w} is -O-CH₂CH₂SO₃H or -NH(CO)-CH₂CH₂O-(CH₂CH₂O)₁₂-CH₃; and
^{∗} represents the point of attachment to the remainder of the linker.

In certain embodiments, the linker comprises a segment according to structural formulae (VIIIa), (VIIIb), or (VIIIc): 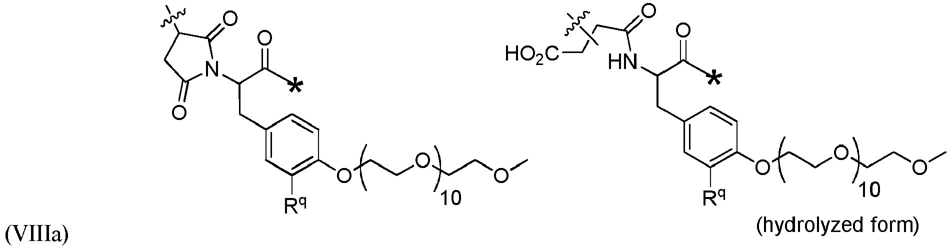 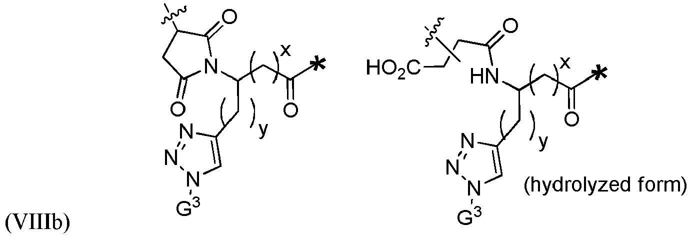 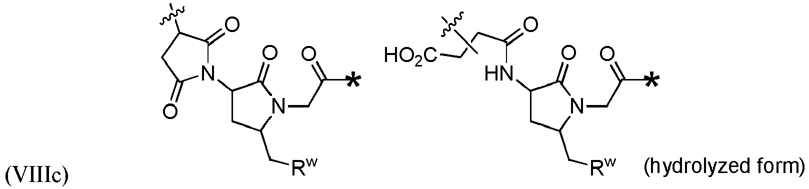 or a hydrolyzed derivative or a pharmaceutically acceptable salt thereof, wherein:
R^{q} is H or -O-(CH₂CH₂O)₁₁-CH₃;
x is 0 or 1;
y is 0 or 1;
G³ is -CH₂CH₂CH₂SO₃H or -CH₂CH₂O-(CH₂CH₂O)₁₁-CH₃;
R^{w} is -O-CH₂CH₂SO₃H or -NH(CO)-CH₂CH₂O-(CH₂CH₂O)₁₂-CH₃;
^{∗} represents the point of attachment to the remainder of the linker; and 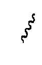
represents the point of attachment of the linker to the antibody.

Exemplary embodiments of linkers according to structural formula (VIIa) and (VIIb) that may be included in the ADCs described herein include the linkers illustrated below (as illustrated, the linkers include a group suitable for covalently linking the linker to an antibody):

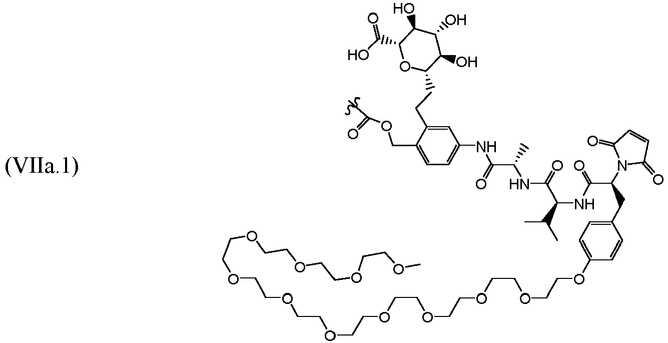

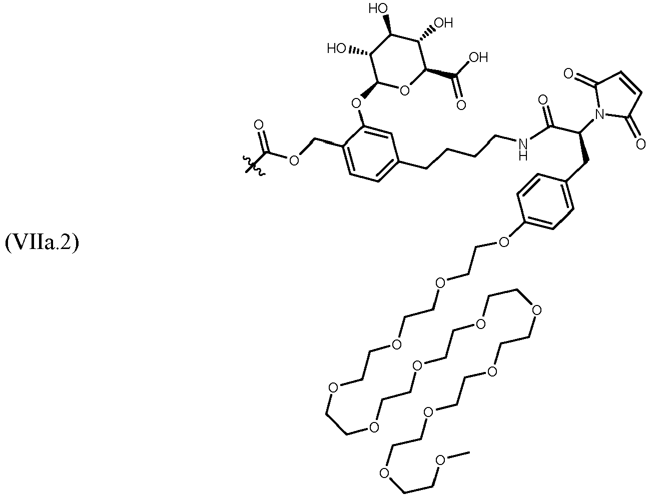

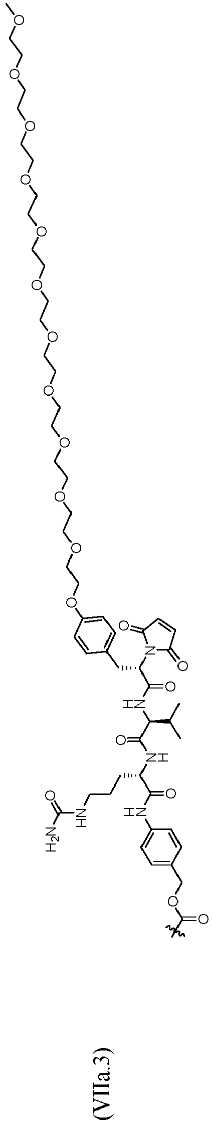

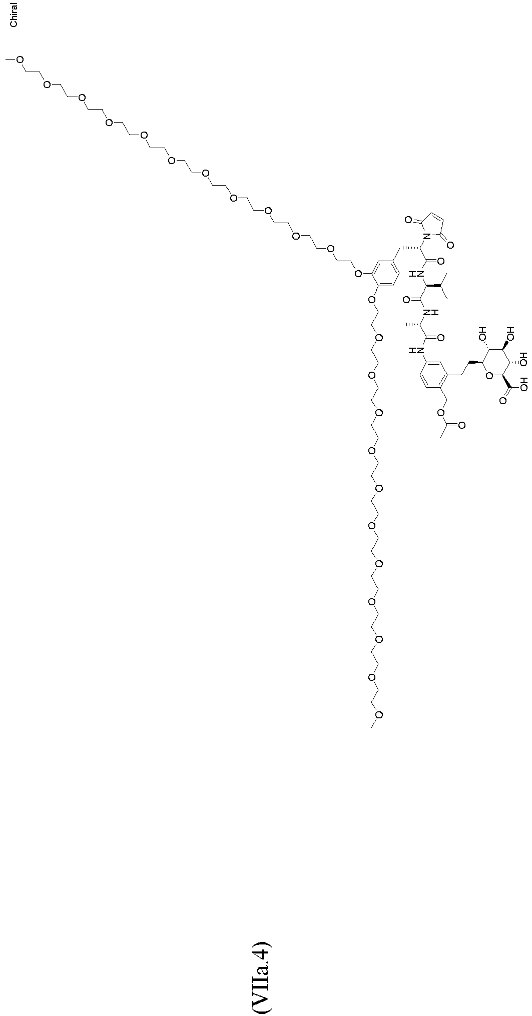

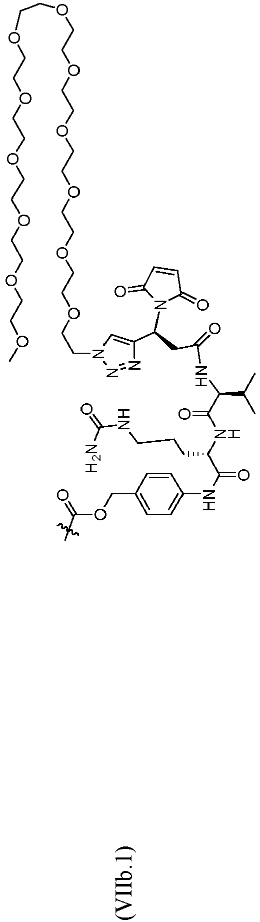

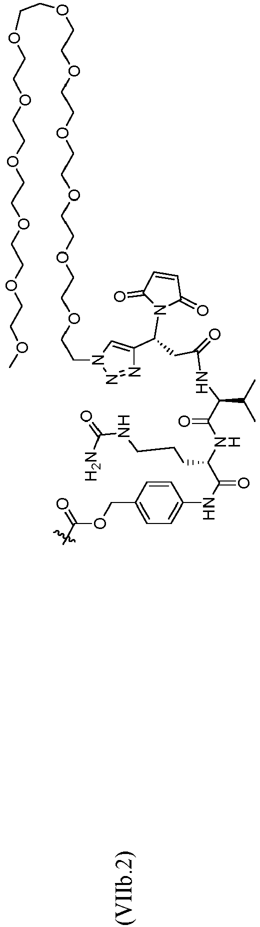

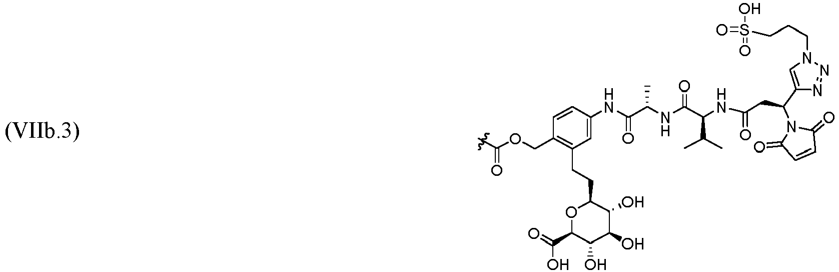

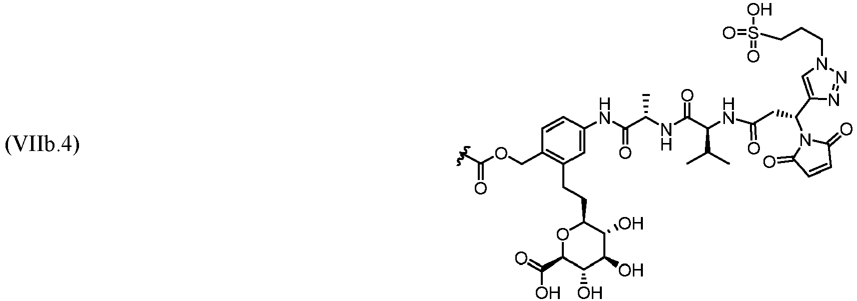

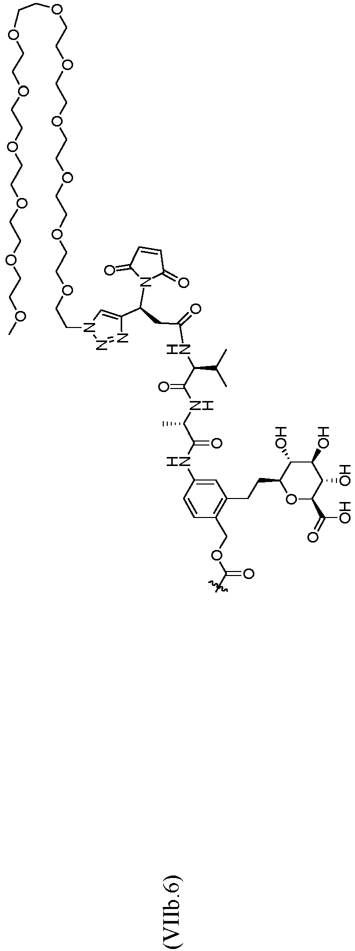

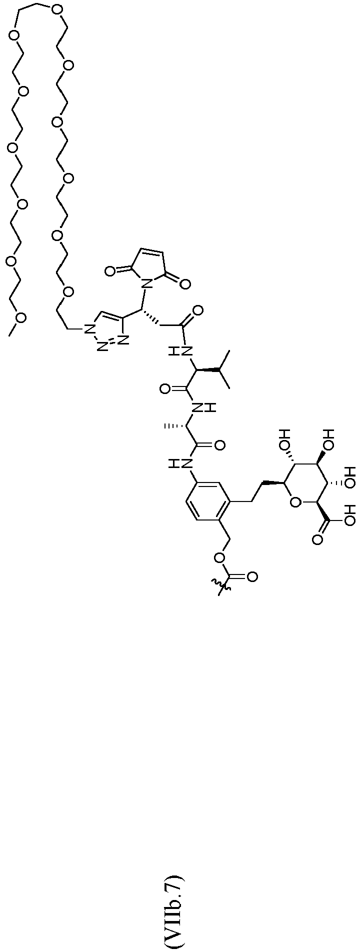

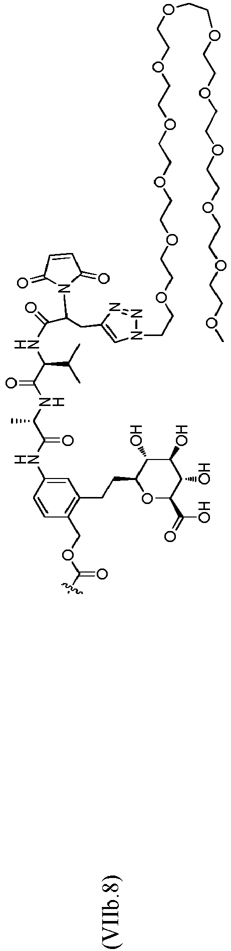

Exemplary embodiments of linkers according to structural formula (VIIc) that may be included in the ADCs described herein include the linkers illustrated below (as illustrated, the linkers include a group suitable for covalently linking the linker to an antibody):

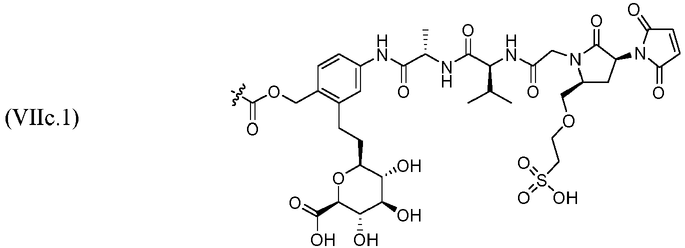

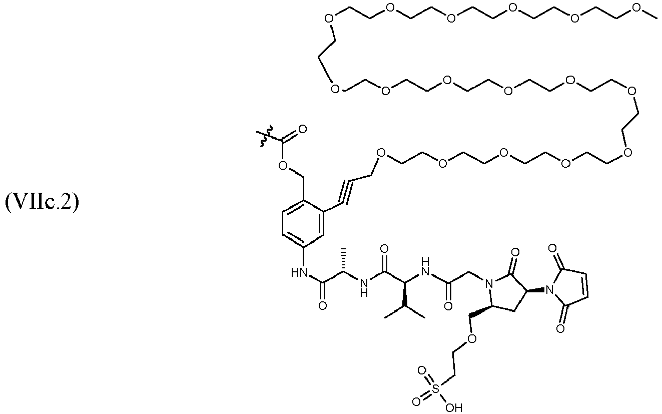

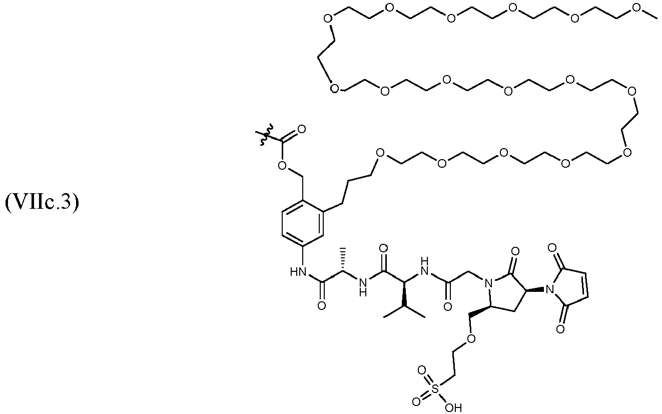

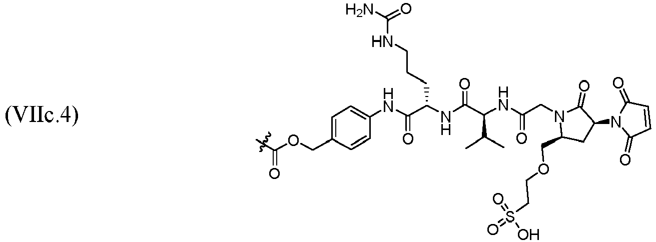

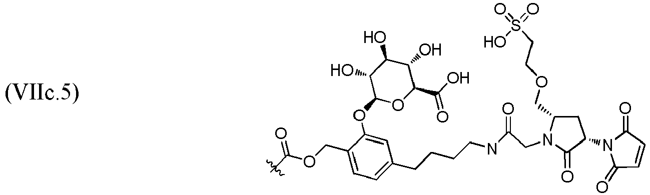

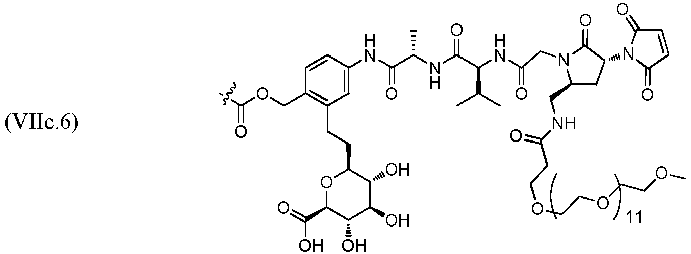

In certain embodiments, L is selected from the group consisting of IVa.1-IVa.8, IVb.1-IVb.19, IVc.1-IVc.7, IVd.1-IVd.4, Va.1-Va.12, Vb.1-Vb.10, Vc.1-Vc.11, Vd.1-Vd.6, Ve.1-Ve.2, VIa.1, VIc.1-V1c2, VId.1-VId.4, VIIa.1-VIIa.4, VIIb.1-VIIb.8, VIIc.1-VIIc.6 in either the closed or open form, and a pharmaceutically acceptable salt thereof.

In certain embodiments, L is selected from the group consisting of IVb.2, IVc.5, IVc.6, IVc.7, IVd.4, Vb.9, VIIa.1, VIIa.3, VIIc.1, VIIc.4, and VIIc.5, wherein the maleimide of each linker has reacted with the antibody Ab, forming a covalent attachment as either a succinimide (closed form) or succinamide (open form),and a pharmaceutically acceptable salt thereof.

In certain embodiments, L is selected from the group consisting of IVb.2, IVc.5, IVc.6, IVd.4, VIIa.1, VIIa.3, VIIc.1, VIIc.4, VIIc.5, wherein the maleimide of each linker has reacted with the antibody Ab, forming a covalent attachment as either a succinimide (closed form) or succinamide (open form), and a pharmaceutically acceptable salt thereof.

In certain embodiments, L is selected from the group consisting of IVb.2, VIIa.3, IVc.6, and VIIc. 1, wherein 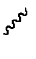 is the attachment point to drug D and @ is the attachment point to the LK, wherein when the linker is in the open form as shown below, @ can be either at the α-position or β-position of the carboxylic acid next to it: 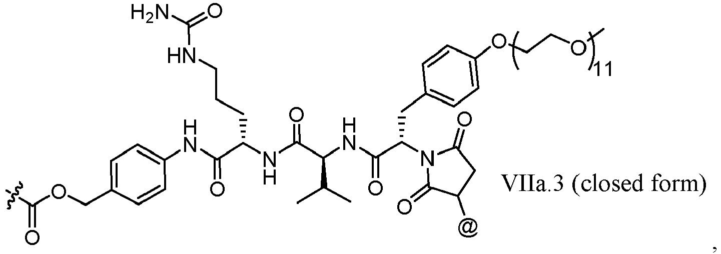 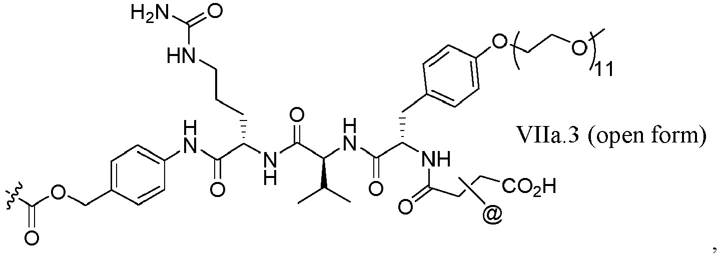 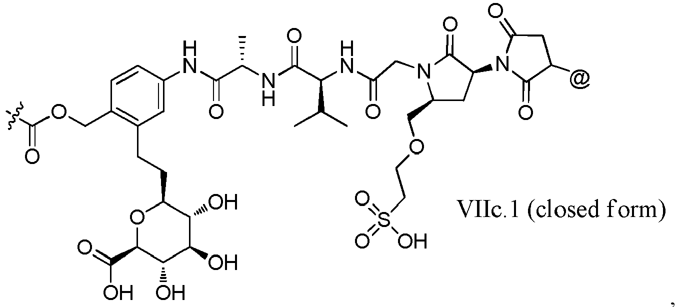 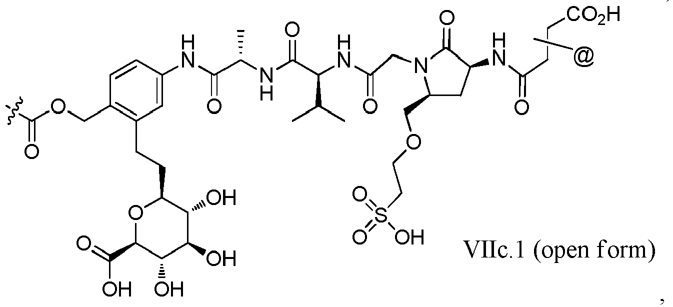 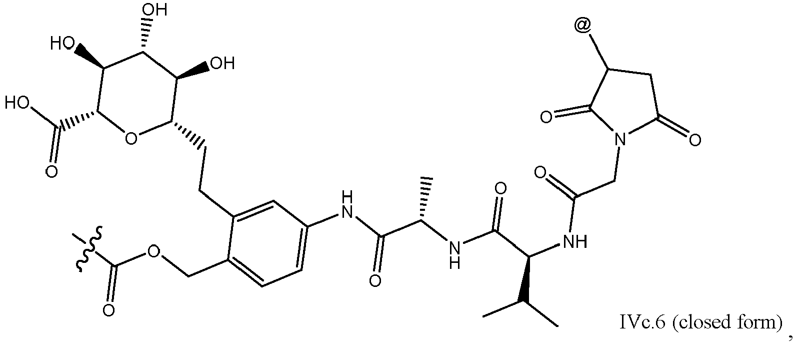 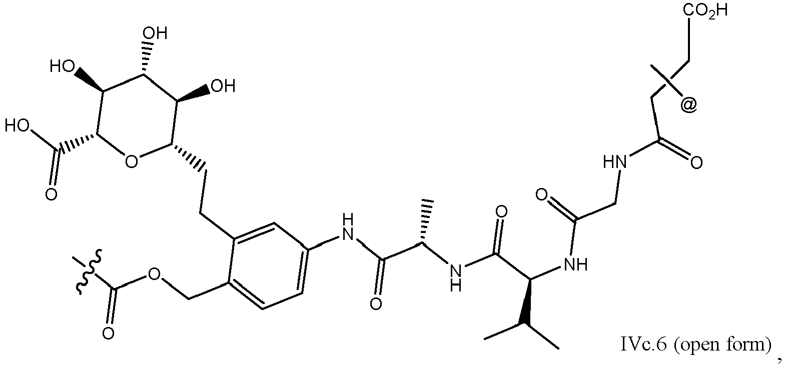 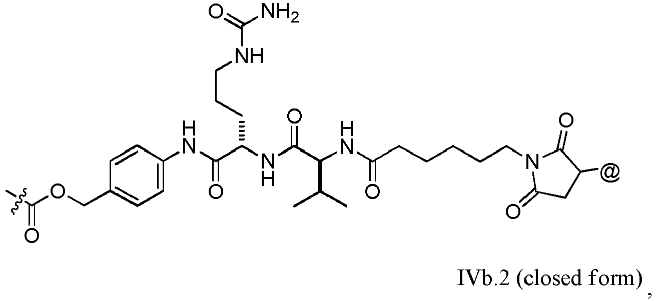 and

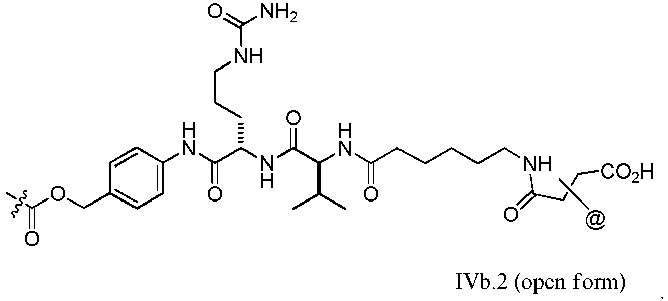

### 3.2.3 Linker Selection Considerations

As is known by skilled artisans, the linker selected for a particular ADC may be influenced by a variety of factors, including but not limited to, the site of attachment to the antibody (e.g., lys, cys or other amino acid residues), structural constraints of the drug pharmacophore and the lipophilicity of the drug. The specific linker selected for an ADC should seek to balance these different factors for the specific antibody/drug combination. For a review of the factors that are influenced by choice of linkers in ADCs, *see* Nolting, Chapter 5 "Linker Technology in Antibody-Drug Conjugates," In: Antibody-Drug Conjugates: Methods in Molecular Biology, vol. 1045, pp. 71-100, Laurent Ducry (Ed.), Springer Science & Business Medica, LLC, 2013.

For example, ADCs have been observed to effect killing of bystander antigen-negative cells present in the vicinity of the antigen-positive tumor cells. The mechanism of bystander cell killing by ADCs has indicated that metabolic products formed during intracellular processing of the ADCs may play a role. Neutral cytotoxic metabolites generated by metabolism of the ADCs in antigen-positive cells appear to play a role in bystander cell killing while charged metabolites may be prevented from diffusing across the membrane into the medium and therefore cannot affect bystander killing. In certain embodiments, the linker is selected to attenuate the bystander killing effect caused by cellular metabolites of the ADC. In certain embodiments, the linker is selected to increase the bystander killing effect.

The properties of the linker may also impact aggregation of the ADC under conditions of use and/or storage. Typically, ADCs reported in the literature contain no more than 3-4 drug molecules per antibody molecule *(see, e.g.,* Chari, 2008, Acc Chem Res 41:98-107). Attempts to obtain higher drug-to-antibody ratios ("DAR") often failed, particularly if both the drug and the linker were hydrophobic, due to aggregation of the ADC (*see* King et al., 2002, J Med Chem 45:4336-4343; Hollander et al., 2008, Bioconjugate Chem 19:358-361; Burke et al., 2009 Bioconjugate Chem 20:1242-1250). In many instances, DARs higher than 3-4 could be beneficial as a means of increasing potency. In instances where the Bcl-xL inhibitor is hydrophobic in nature, it may be desirable to select linkers that are relatively hydrophilic as a means of reducing ADC aggregation, especially in instances where DARS greater than 3-4 are desired. Thus, in certain embodiments, the linker incorporates chemical moieties that reduce aggregation of the ADCs during storage and/or use. A linker may incorporate polar or hydrophilic groups such as charged groups or groups that become charged under physiological pH to reduce the aggregation of the ADCs. For example, a linker may incorporate charged groups such as salts or groups that deprotonate, e.g., carboxylates, or protonate, e.g., amines, at physiological pH.

Exemplary polyvalent linkers that have been reported to yield DARs as high as 20 that may be used to link numerous Bcl-xL inhibitors to an antibody are described in U.S. Patent No 8,399,512; U.S. Published Application No. 2010/0152725; U.S. Patent No. 8,524,214; U.S. Patent No. 8,349,308; U.S. Published Application No. 2013/189218; U.S. Published Application No. 2014/017265; WO 2014/093379; WO 2014/093394; WO 2014/093640.

In particular embodiments, the aggregation of the ADCs during storage or use is less than about 40% as determined by size-exclusion chromatography (SEC). In particular embodiments, the aggregation of the ADCs during storage or use is less than 35%, such as less than about 30%, such as less than about 25%, such as less than about 20%, such as less than about 15%, such as less than about 10%, such as less than about 5%, such as less than about 4%, or even less, as determined by size-exclusion chromatography (SEC).

### 4. ADC Synthons

Antibody-Drug Conjugate synthons are synthetic intermediates used to form ADCs. The synthons are generally compounds according to structural formula (III):

(III) D-L-R^{x}

or a pharmaceutically acceptable salt thereof, wherein D is a Bcl-xL inhibitor as previously described, L is a linker as previously described, and R*^{x}* is a reactive group suitable for linking the synthon to an antibody.

In specific embodiments, the intermediate synthons are compounds according to structural formulae (IIIa), (IIIb), (IIIc) and (IIId), below, or a pharmaceutically acceptable salt thereof, where the various substituents Ar¹, Ar², Z¹, Z^{2a}, Z^{2b}, R', R¹, R², R⁴, R^{11a}, R^{11b}, R¹² and R¹³ are as previously defined for structural formulae (IIa), (IIb), (IIc) and (lid), respectively, L is a linker as previously described and R*^{x}* is a functional group as described above:

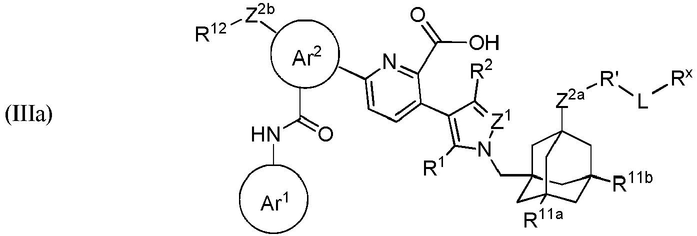

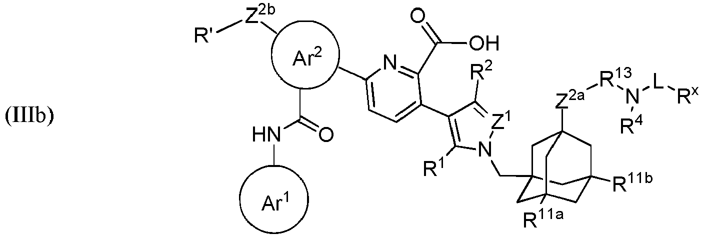

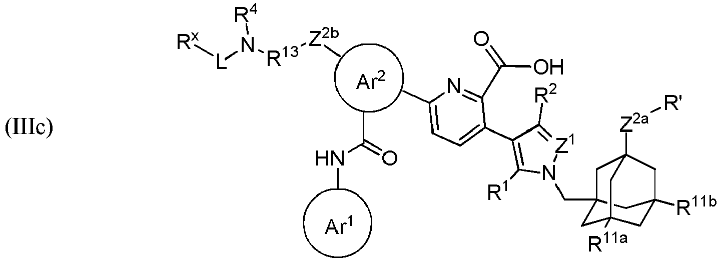

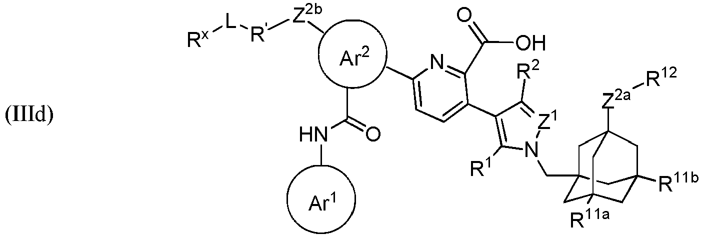

To synthesize an ADC, an intermediate synthon according to structural formula (III), or a salt thereof, is contacted with an antibody of interest under conditions in which functional group R*^{x}* reacts with a "complementary" functional group on the antibody, F^{x}, to form a covalent linkage.

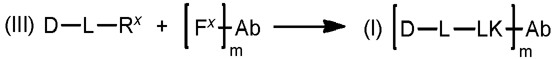

The identities of groups R*^{x}* and F*^{x}* will depend upon the chemistry used to link the synthon to the antibody. Generally, the chemistry used should not alter the integrity of the antibody, for example its ability to bind its target. Preferably, the binding properties of the conjugated antibody will closely resemble those of the unconjugated antibody. A variety of chemistries and techniques for conjugating molecules to biological molecules such as antibodies are known in the art and in particular to antibodies, are well-known. *See, e.g.,* Amon et al., "Monoclonal Antibodies For Immunotargeting Of Drugs In Cancer Therapy," in: Monoclonal Antibodies And Cancer Therapy, Reisfeld et al. Eds., Alan R. Liss, Inc., 1985; Hellstrom et al., "Antibodies For Drug Delivery," in: Controlled Drug Delivery, Robinson et al., Eds., Marcel Dekker, Inc., 2nd Ed. 1987; Thorpe, "Antibody Carriers Of Cytotoxic Agents In Cancer Therapy: A Review," in: Monoclonal Antibodies '84: Biological And Clinical Applications, Pinchera et al., Eds., 1985; "Analysis, Results, and Future Prospective of the Therapeutic Use of Radiolabeled Antibody In Cancer Therapy," in: Monoclonal Antibodies For Cancer Detection And Therapy, Baldwin et al., Eds., Academic Press, 1985; Thorpe et al., 1982, Immunol. Rev. 62:119-58; PCT publication WO 89/12624. Any of these chemistries may be used to link the synthons to an antibody.

Typically, the synthons are linked to the side chains of amino acid residues of the antibody, including, for example, the primary amino group of accessible lysine residues or the sulfhydryl group of accessible cysteine residues. Free sulfhydryl groups may be obtained by reducing interchain disulfide bonds. In certain embodiments, LK is a linkage formed with an amino group on the anti-hEGFR antibody Ab. In certain embodiments, LK is an amide, thioether, or thiourea. In certain embodiments, LK is an amide or thiourea. In certain embodiments, LK is a linkage formed with a sulfhydryl group on the anti-hEGFR antibody Ab. In certain embodiments, LK is a thioether. In certain embodiments, LK is an amide, thioether, or thiourea; and m is an integer ranging from 1 to 8.

A number of functional groups R*^{x}* and chemistries useful for linking synthons to accessible lysine residues are known, and include by way of example and not limitation NHS-esters and isothiocyanates.

A number of functional groups R*^{x}* and chemistries useful for linking synthons to accessible free sulfhydryl groups of cysteine residues are known, and include by way of example and not limitation haloacetyls and maleimides.

However, conjugation chemistries are not limited to available side chain groups. Side chains such as amines may be converted to other useful groups, such as hydroxyls, by linking an appropriate small molecule to the amine. This strategy can be used to increase the number of available linking sites on the antibody by conjugating multifunctional small molecules to side chains of accessible amino acid residues of the antibody. Functional groups R*^{x}* suitable for covalently linking the synthons to these "converted" functional groups are then included in the synthons.

The antibody may also be engineered to include amino acid residues for conjugation. An approach for engineering antibodies to include non-genetically encoded amino acid residues useful for conjugating drugs in the context of ADCs is described in Axup et al., 2003, Proc Natl Acad Sci 109:16101-16106 and Tian et al., 2014, Proc Natl Acad Sci 111:1776-1771 as are chemistries and functional groups useful for linking synthons to the non-encoded amino acids.

Exemplary synthons useful for making ADCs described herein include, but are not limited to, the following synthons listed below in Table 5.

**Table 5**

| **Example No.** | **Synthon Code** | **Synthon Structure** |
|---|---|---|
| 2.1 | CZ | 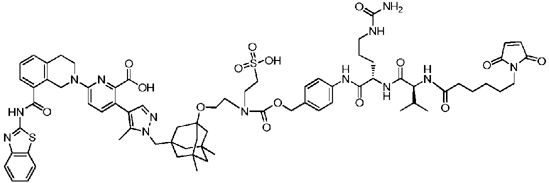 |
| 2.2 | DH | 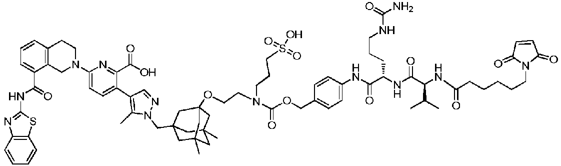 |
| 2.4 | EP | 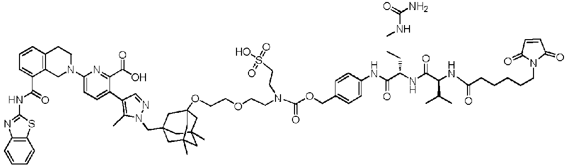 |
| 2.5 | EF | 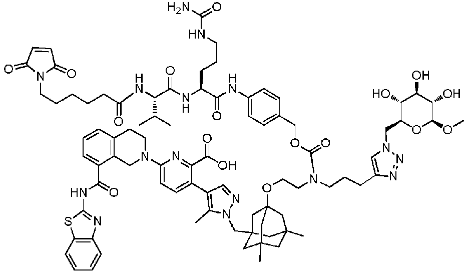 |
| 2.6 | EG | 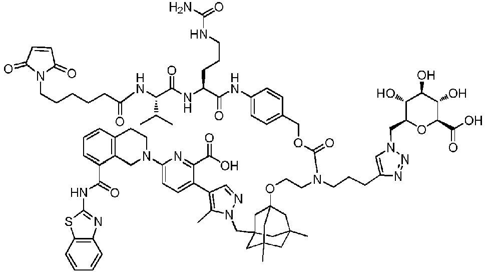 |
| 2.7 | EH | 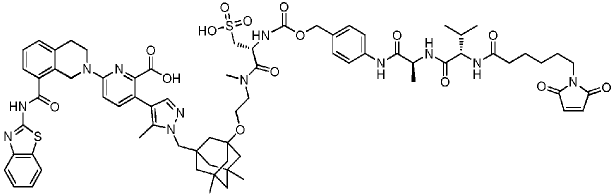 |
| 2.8 | ER | 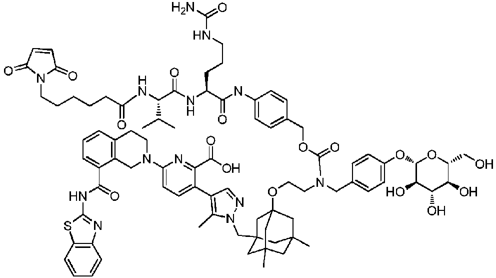 |
| 2.9 | ES | 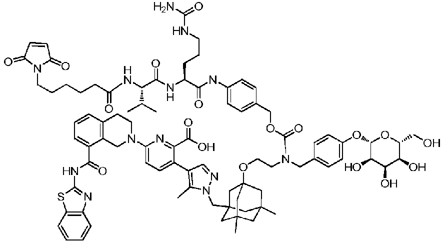 |
| 2.10 | EQ | 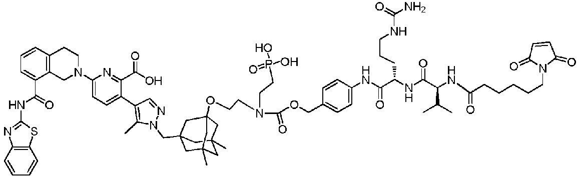 |
| 2.11 | EU | 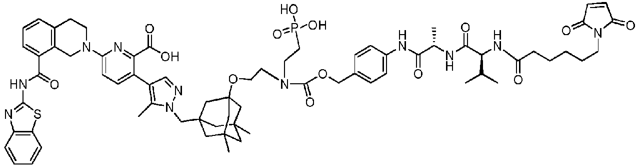 |
| 2.12 | EV | 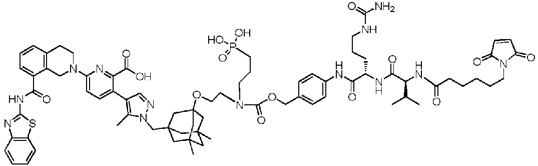 |
| 2.13 | EW | 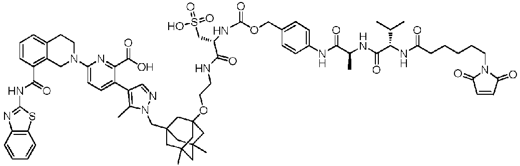 |
| 2.14 | EX | 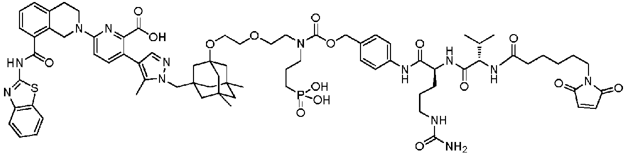 |
| 2.15 | EY | 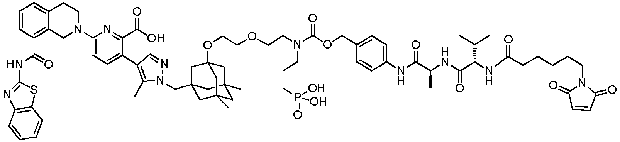 |
| 2.16 | EZ | 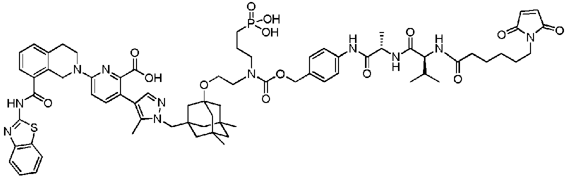 |
| 2.17 | FD | 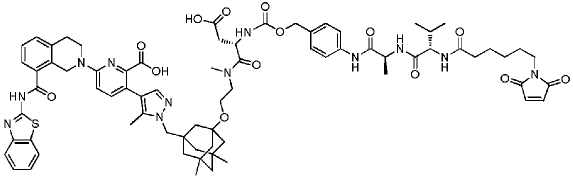 |
| 2.18 | FS | 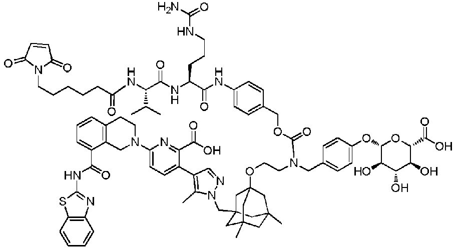 |
| 2.19 | FI | 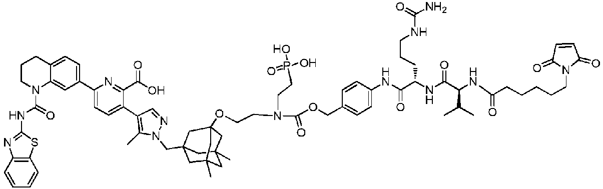 |
| 2.20 | FV | 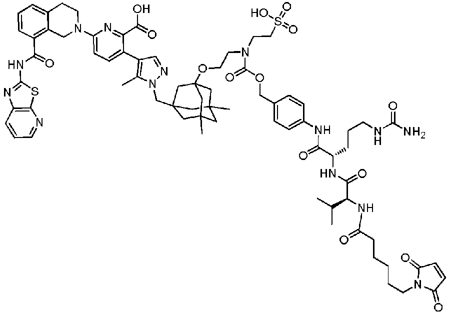 |
| 2.21 | GC | 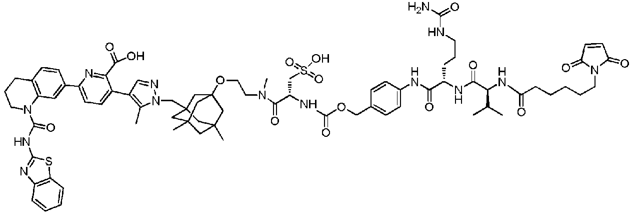 |
| 2.22 | GB | 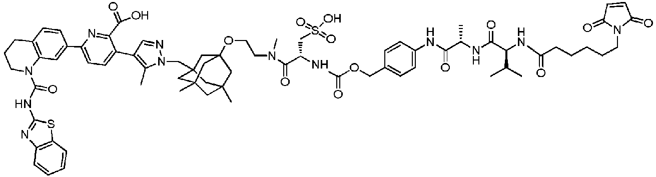 |
| 2.23 | FW | 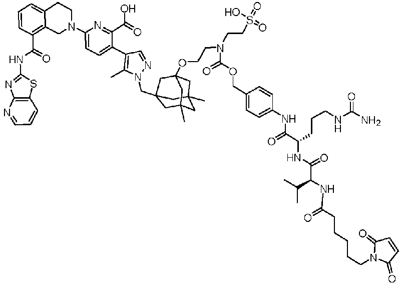 |
| 2.24 | GD | 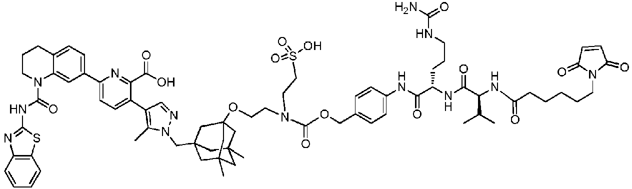 |
| 2.25 | GK | 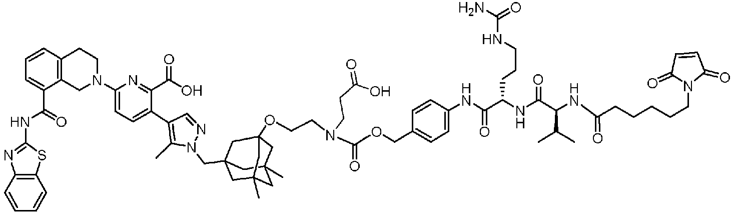 |
| 2.26 | GJ | 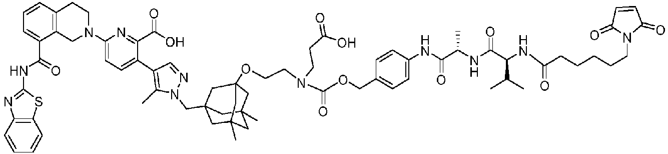 |
| 2.27 | GW | 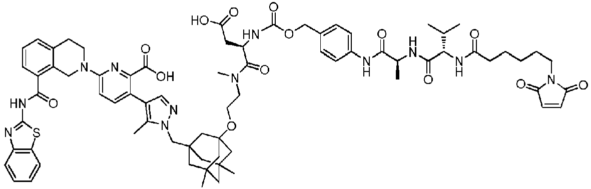 |
| 2.28 | HF | 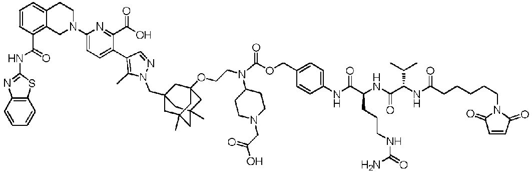 |
| 2.29 | HG | 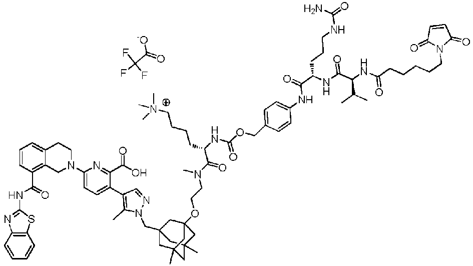 |
| 2.30 | HP | 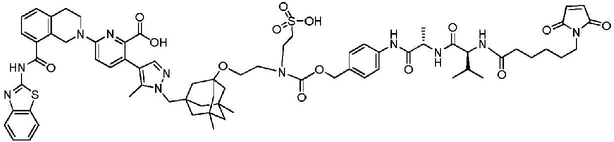 |
| 2.31 | HR | 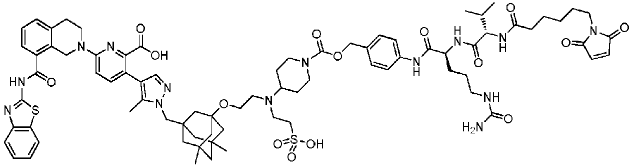 |
| 2.32 | HU | 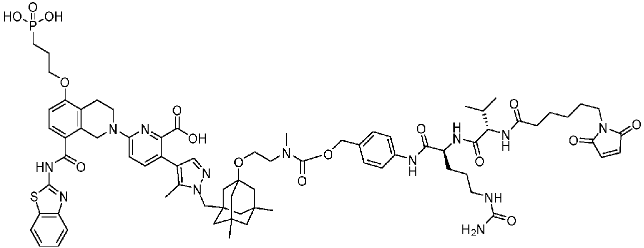 |
| 2.33 | HT | 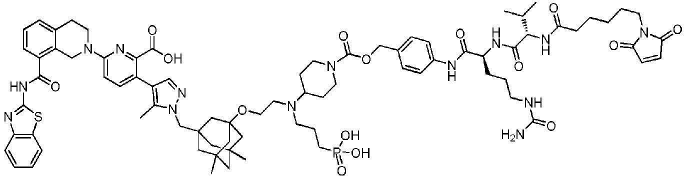 |
| 2.34 | HV | 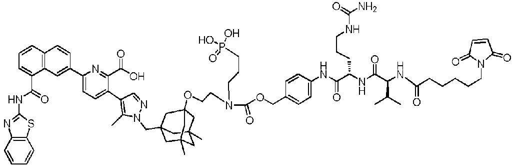 |
| 2.35 | HZ | 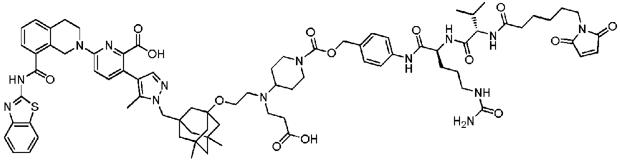 |
| 2.36 | IA | 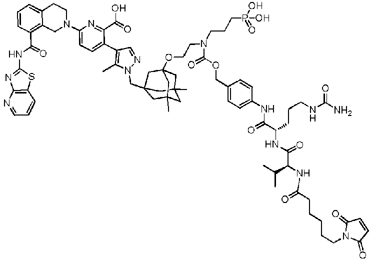 |
| 2.37 | IF | 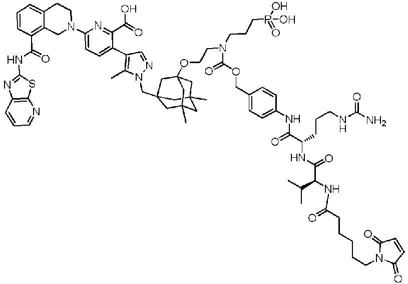 |
| 2.38 | IG | 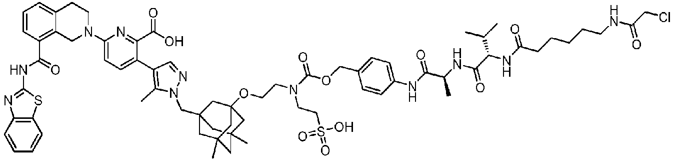 |
| 2.39 | IH | 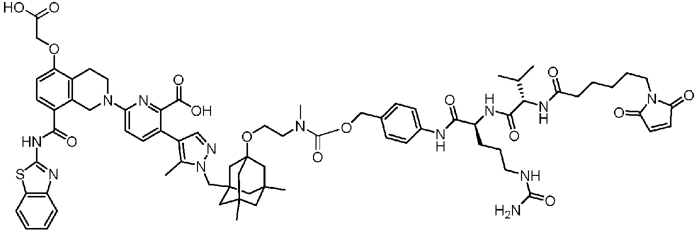 |
| 2.40 | IJ | 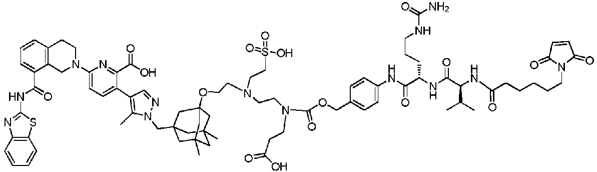 |
| 2.41 | IK | 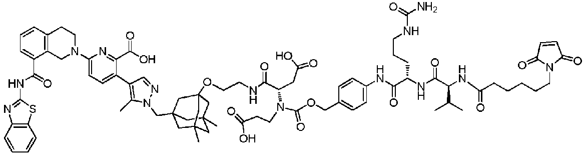 |
| 2.42 | IL | 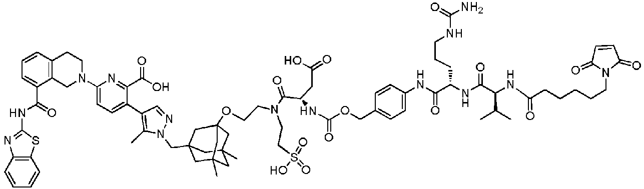 |
| 2.43 | IM | 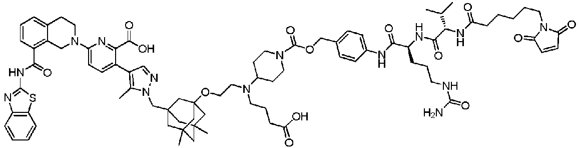 |
| 2.44 | IO | 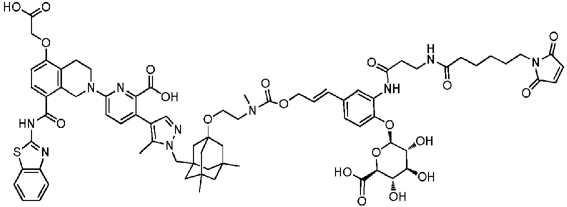 |
| 2.45 | IP | 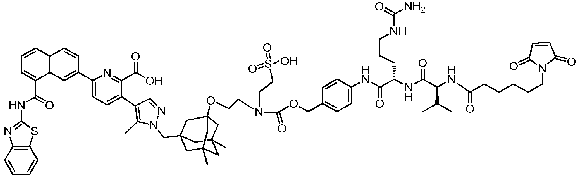 |
| 2.46 | IS | 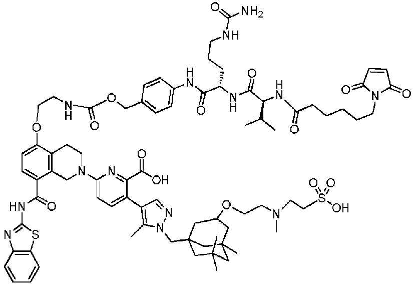 |
| 2.47 | IU | 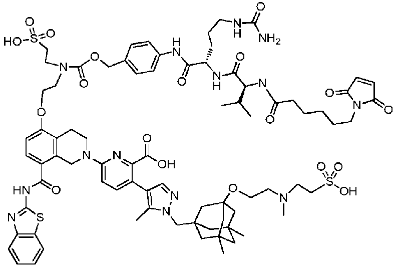 |
| 2.48 | IV | 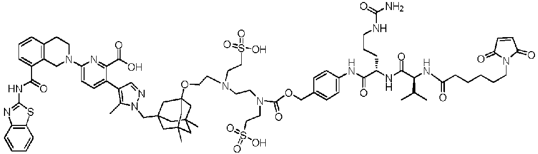 |
| 2.49 | IZ | 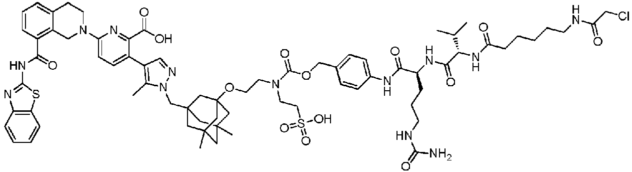 |
| 2.50 | JD | 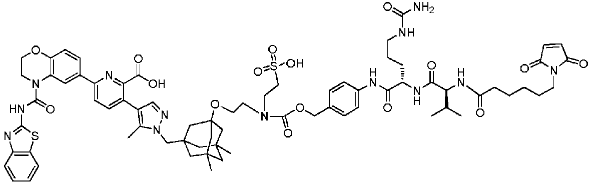 |
| 2.51 | JF | 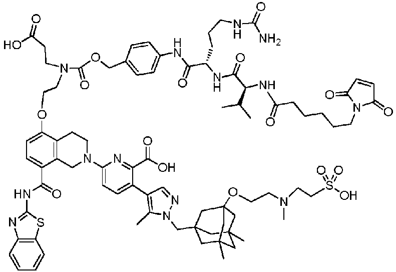 |
| 2.52 | JK | 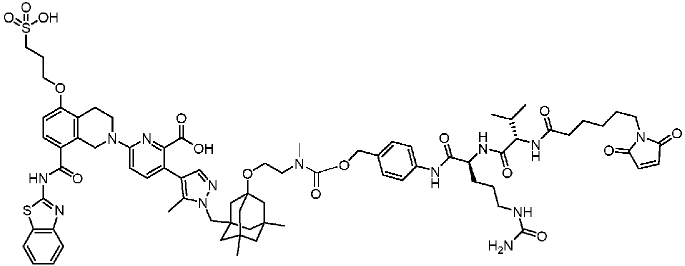 |
| 2.53 | JJ | 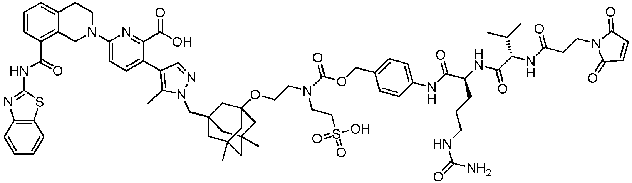 |
| 2.54 | JL | 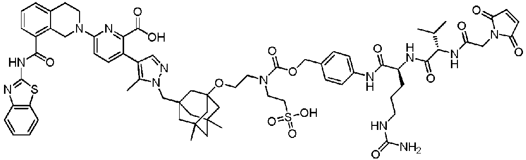 |
| 2.55 | FE | 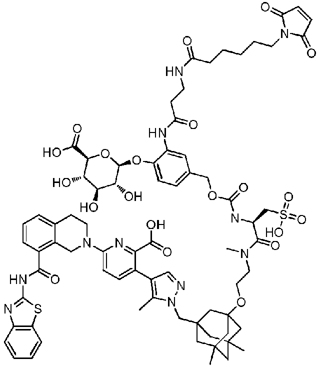 |
| 2.56 | GG | 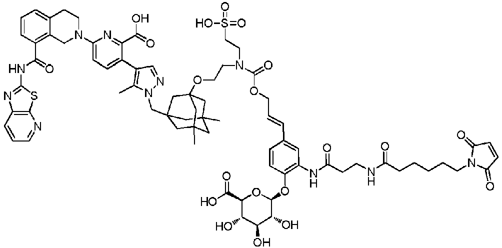 |
| 2.57 | GM | 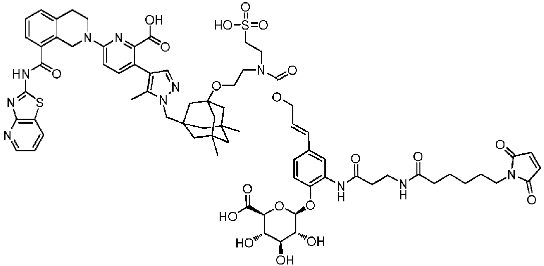 |
| 2.58 | HD | 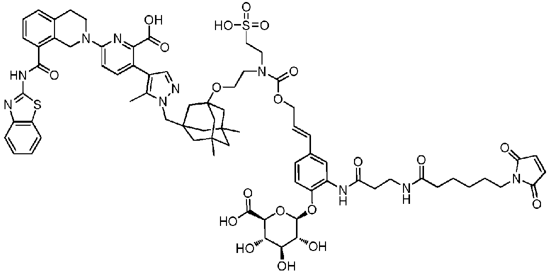 |
| 2.59 | HS | 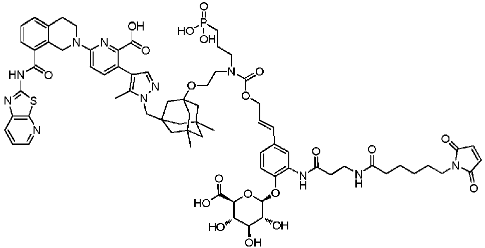 |
| 2.60 | HW | 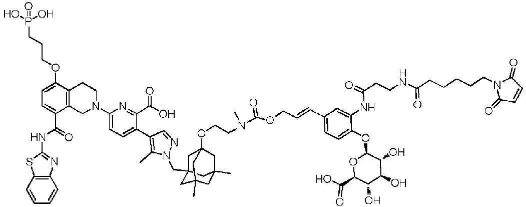 |
| 2.61 | HX | 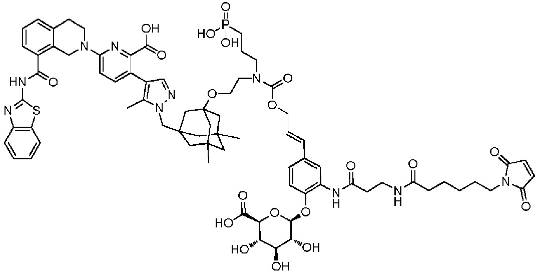 |
| 2.62 | HY | 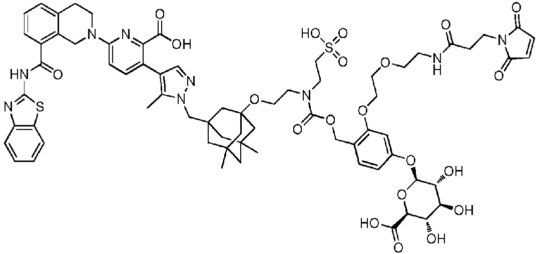 |
| 2.63 | IB | 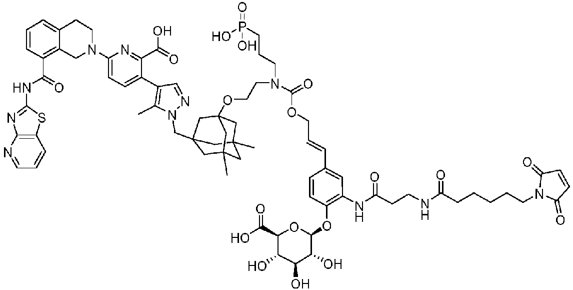 |
| 2.64 | IE | 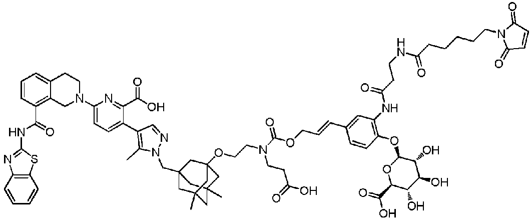 |
| 2.65 | II | 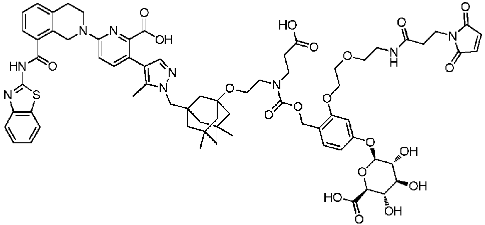 |
| 2.66 | KY | 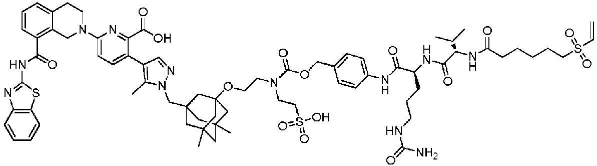 |
| 2.67 | IW | 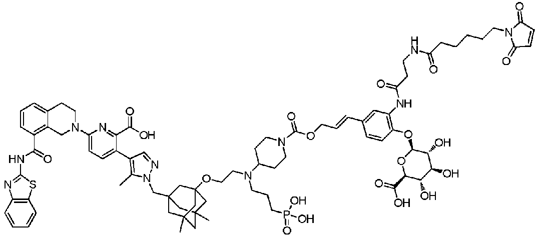 |
| 2.68 | IY | 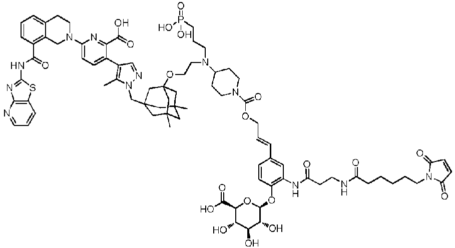 |
| 2.69 | JA | 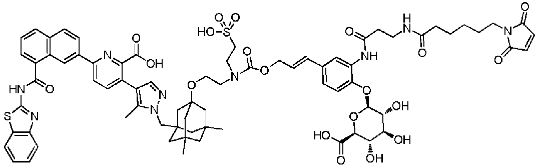 |
| 2.77 | FA | 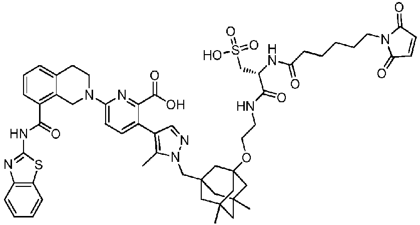 |
| 2.78 | FJ | 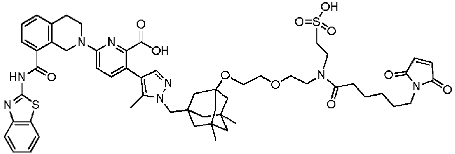 |
| 2.79 | FK | 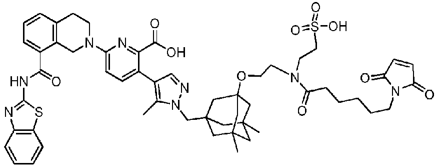 |
| 2.80 | FQ | 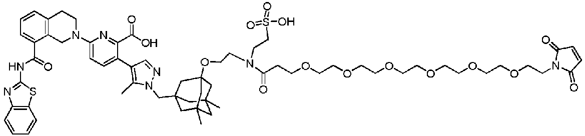 |
| 2.81 | FR | 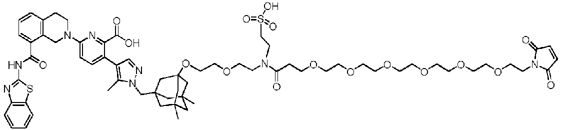 |
| 2.82 | JE | 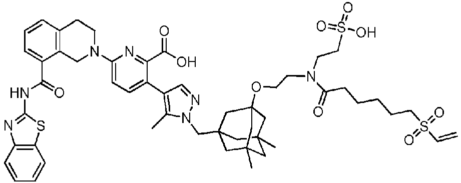 |
| 2.83 | JM | 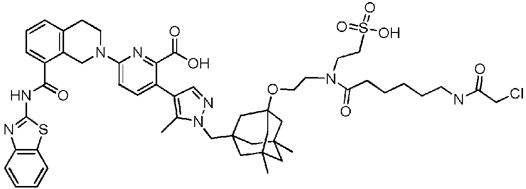 |
| 2.84 | LE | 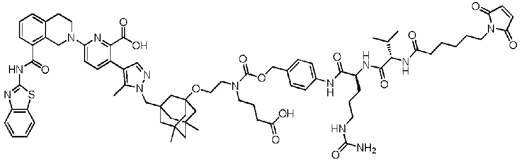 |
| 2.85 | LH | 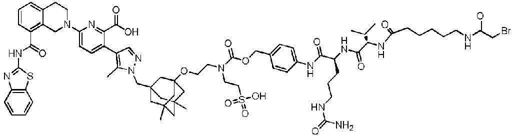 |
| 2.86 | LJ | 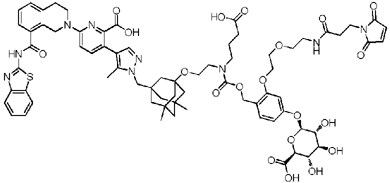 |
| 2.87 | MA | 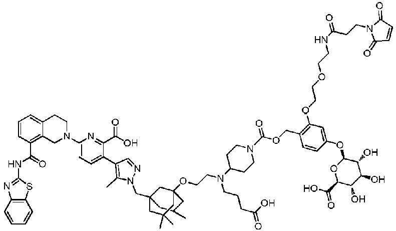 |
| 2.88 | MD | 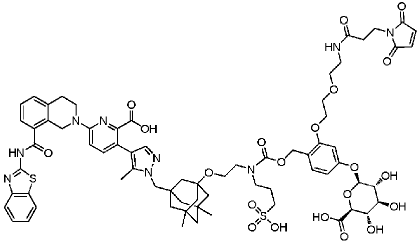 |
| 2.89 | MG | 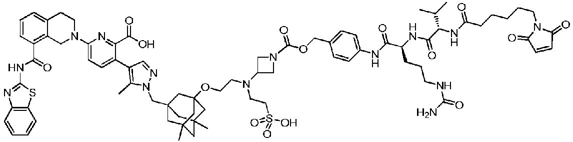 |
| 2.90 | MS | 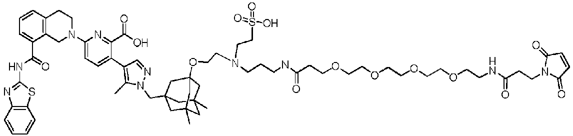 |
| 2.91 | MR | 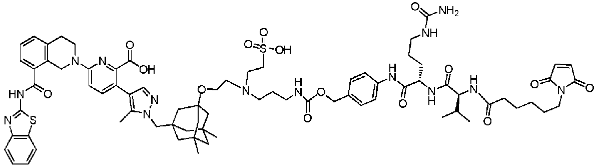 |
| 2.92 | MQ | 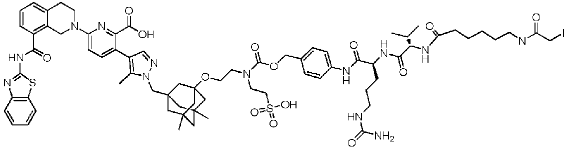 |
| 2.93 | MZ | 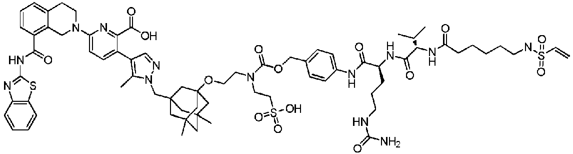 |
| 2.94 | NA | 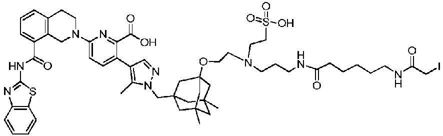 |
| 2.95 | NB | 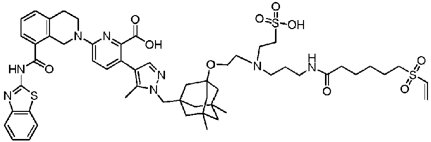 |
| 2.96 | NP | 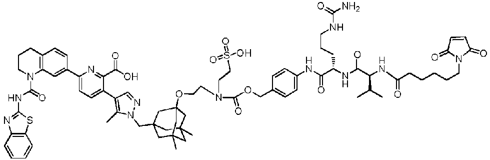 |
| 2.97 | NN | 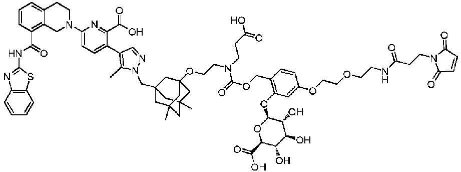 |
| 2.98 | NO | 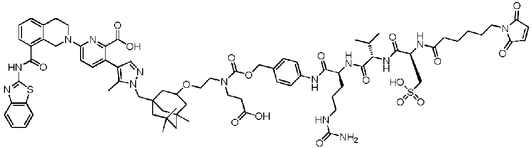 |
| 2.101 | OK | 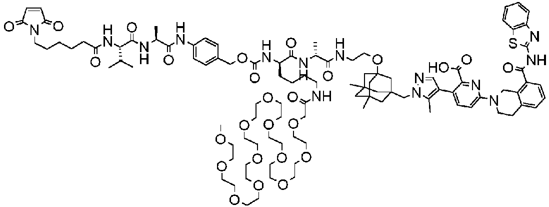 |
| 2.102 | OW | 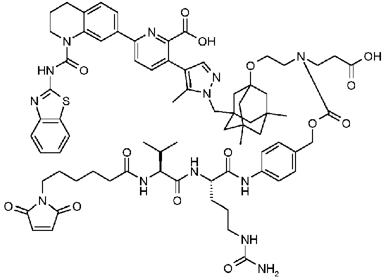 |
| 2.103 | PC | 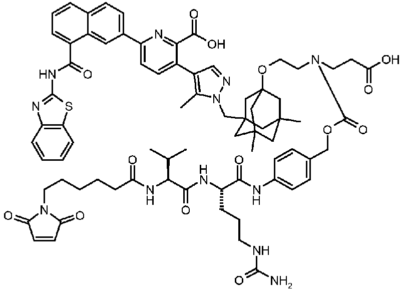 |
| 2.104 | PI | 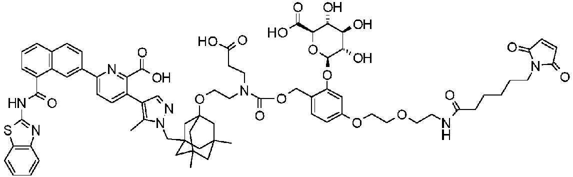 |
| 2.105 | PJ | 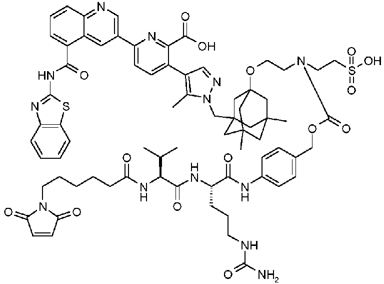 |
| 2.106 | PU | 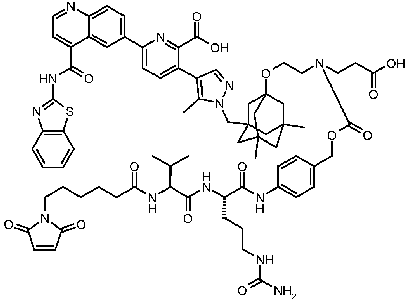 |
| 2.107 | PV | 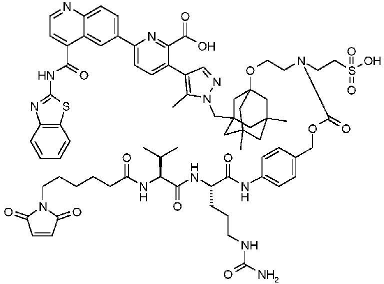 |
| 2.108 | PW | 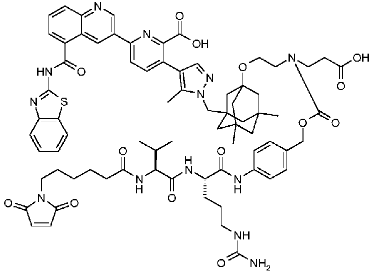 |
| 2.109 | QW | 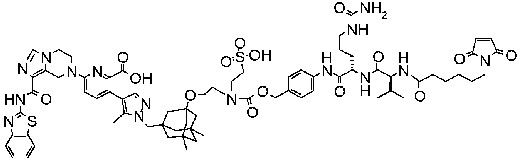 |
| 2.110 | RM | 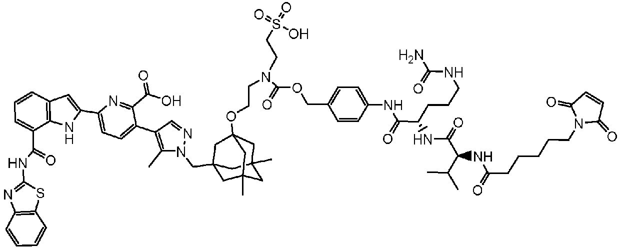 |
| 2.111 | RR | 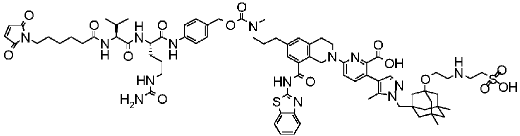 |
| 2.112 | SJ | 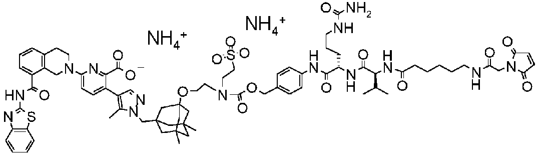 |
| 2.113 | SM | 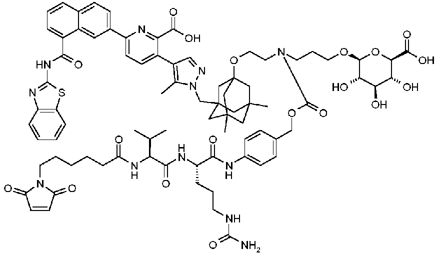 |
| 2.114 | SN | 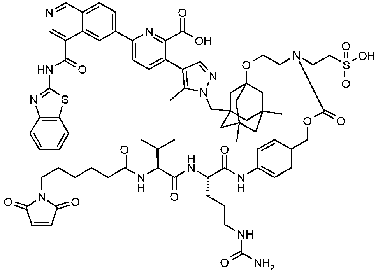 |
| 2.115 | SS | 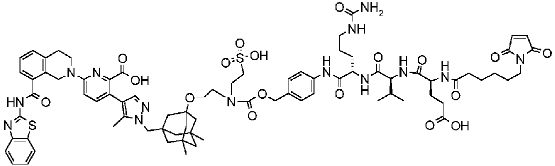 |
| 2.116 | TA | 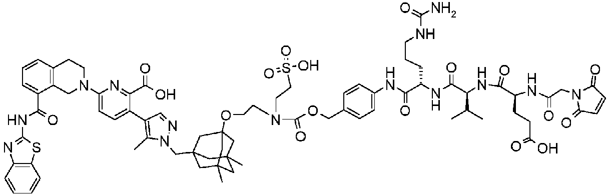 |
| 2.117 | TW | 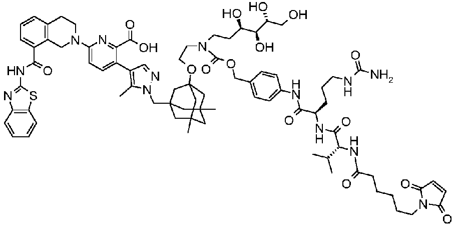 |
| 2.118 | ST | 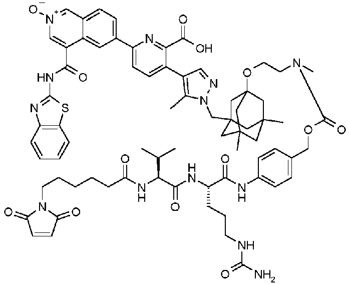 |
| 2.119 | ZL | 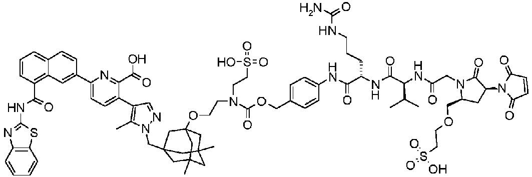 |
| 2.120 | SX | 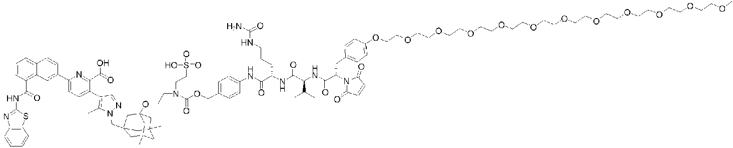 |
| 2.121 | SW | 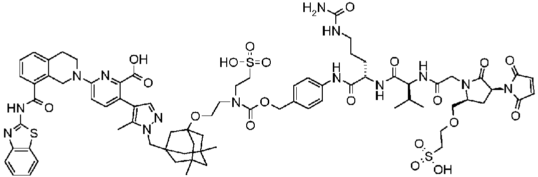 |
| 2.122 | TV | 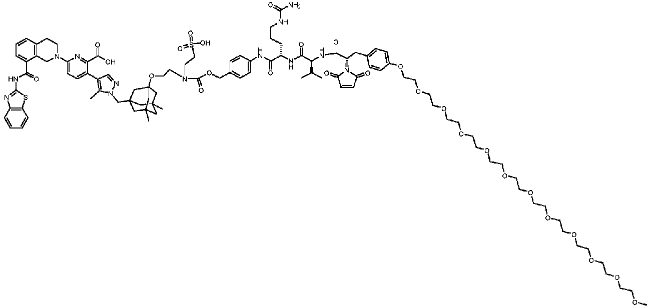 |
| 2.123 | SZ | 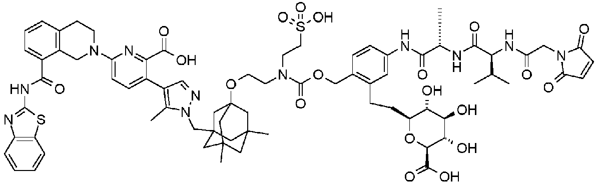 |
| 2.124 | ZM | 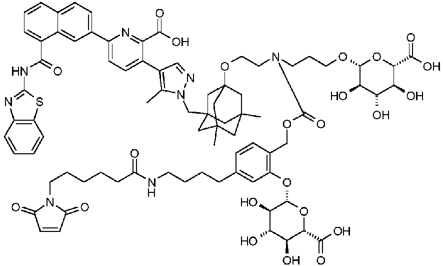 |
| 2.125 | SV | 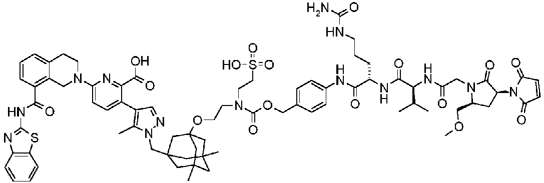 |
| 2.126 | SY | 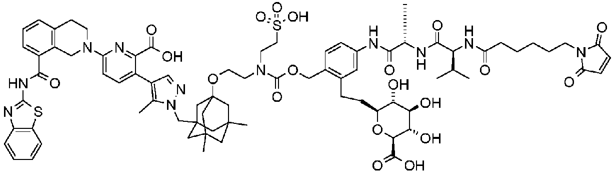 |
| 2.127 | TK | 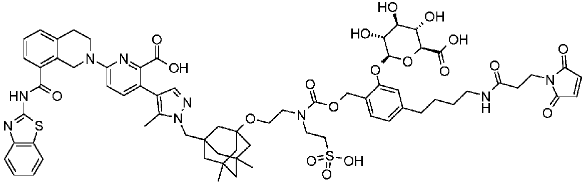 |
| 2.128 | TR | 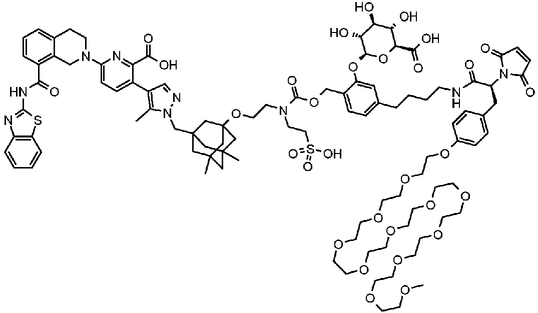 |
| 2.129 | TY | 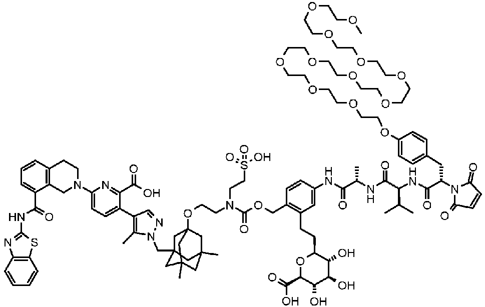 |
| 2.130 | TX | 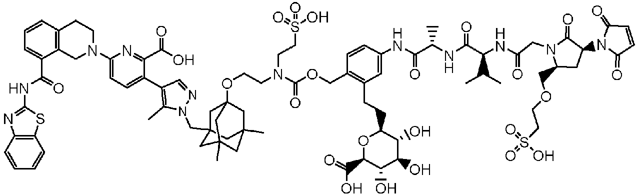 |
| 2.131 | TZ | 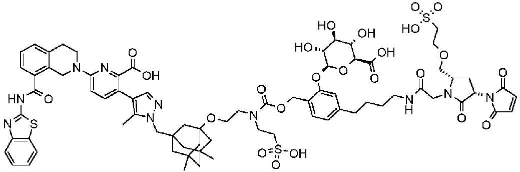 |
| 2.132 | UA | 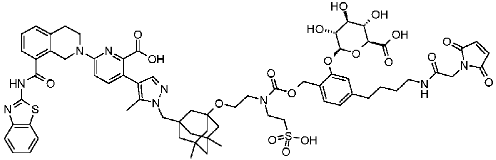 |
| 2.133 | UJ | 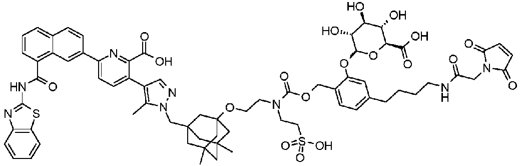 |
| 2.134 | UK | 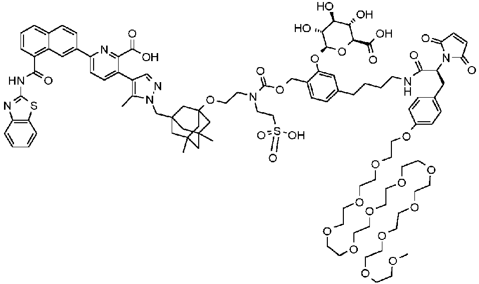 |
| 2.135 | UU | 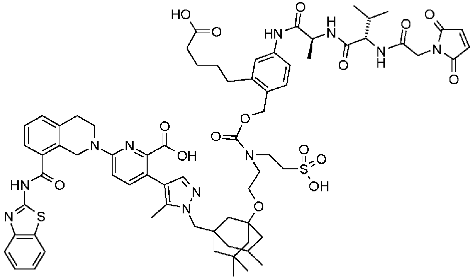 |
| 2.136 | UV | 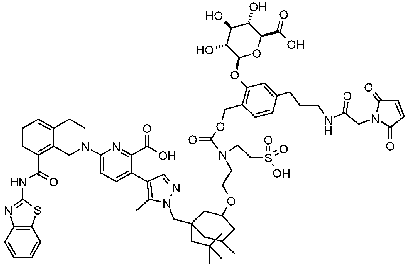 |
| 2.137 | UZ | 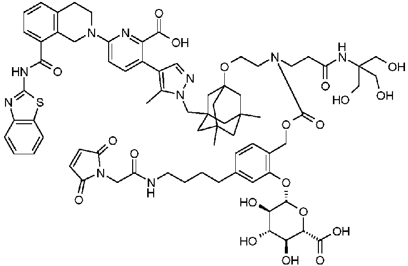 |
| 2.138 | VB | 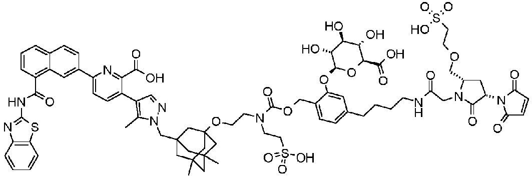 |
| 2.139 | VC | 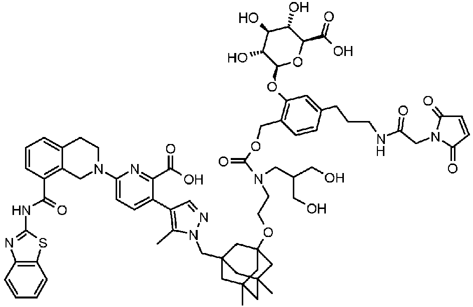 |
| 2.140 | VS | 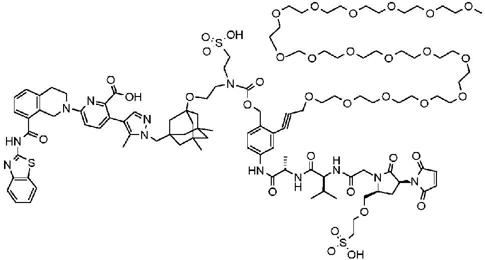 |
| 2.141 | VT | 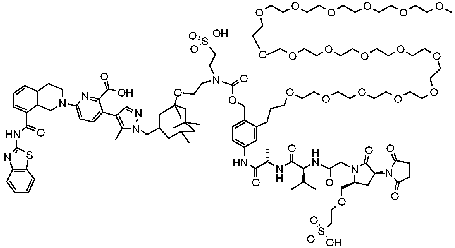 |
| 2.142 | VY | 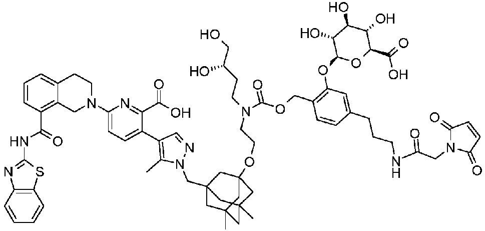 |
| 2.143 | WI | 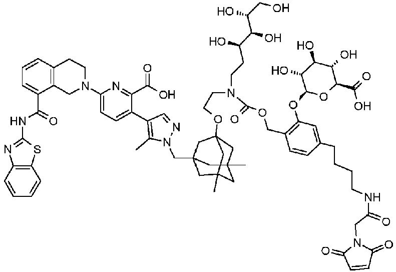 |
| 2.144 | WK | 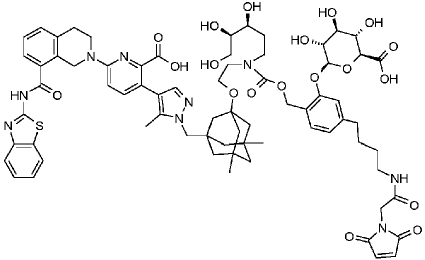 |
| 2.145 | WP | 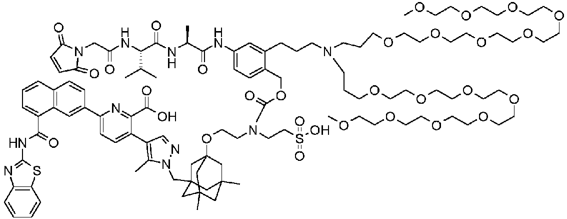 |
| 2.146 | XD | 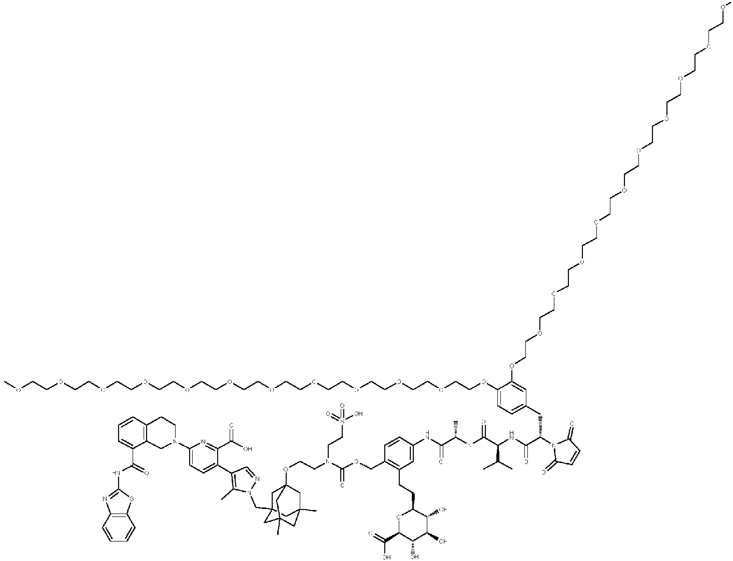 |
| 2.147 | XK | 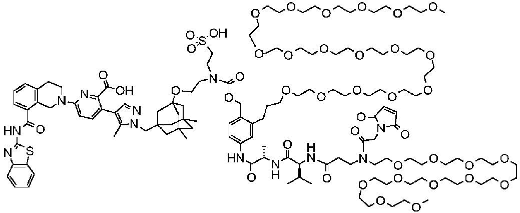 |
| 2.148 | XL | 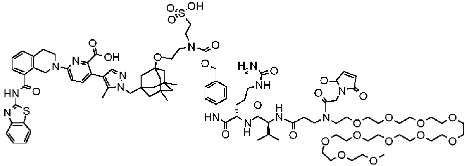 |
| 2.149 | YJ | 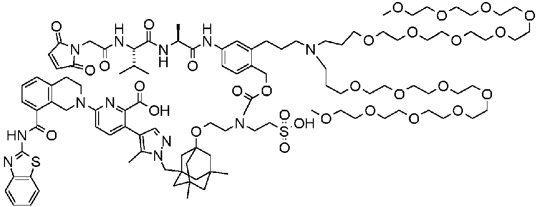 |
| 2.150 | YQ | 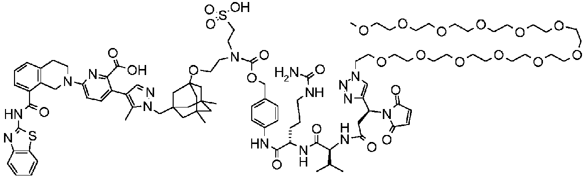 |
| 2.151 | YR | 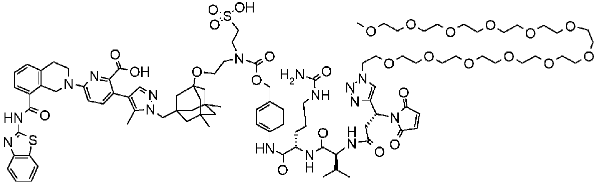 |
| 2.152 | YS | 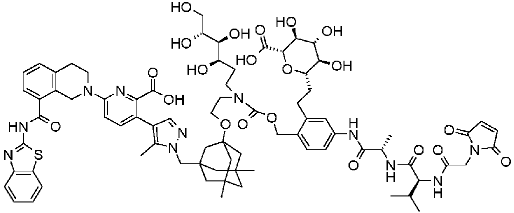 |
| 2.153 | YY | 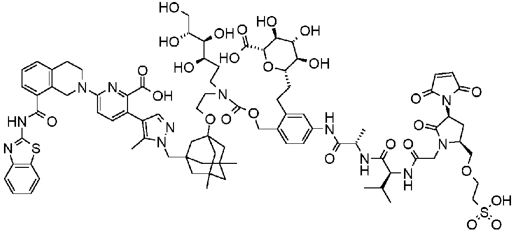 |
| 2.154 | YT | 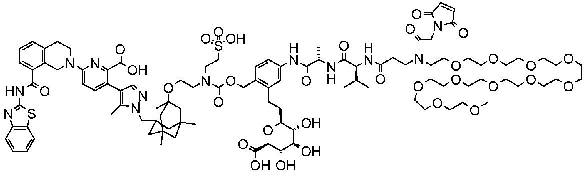 |
| 2.155 | YU | 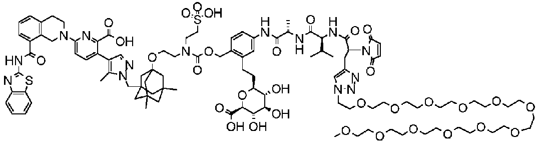 |
| 2.156 | YV | 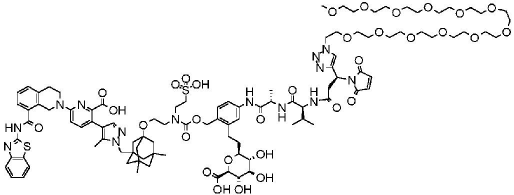 |
| 2.157 | YW | 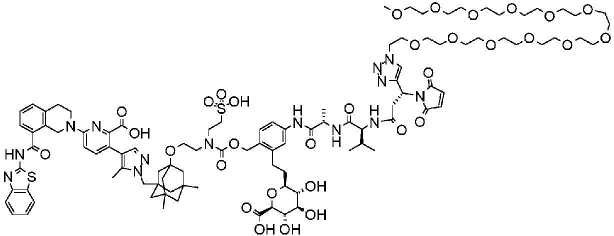 |
| 2.158 | ZB | 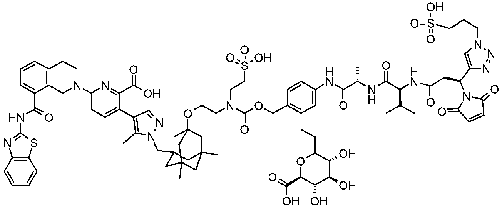 |
| 2.159 | ZC | 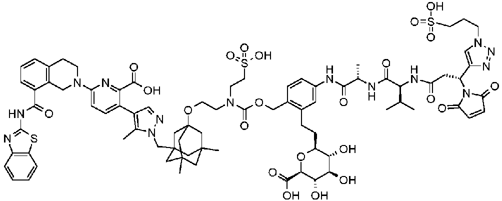 |
| 2.160 | ZJ | 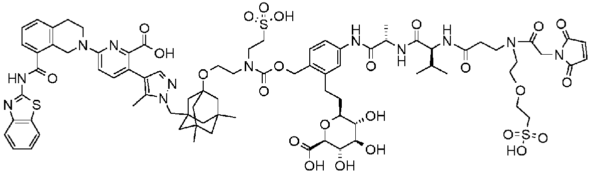 |
| 2.161 | ZE | 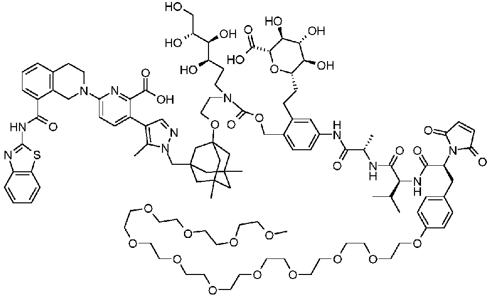 |
| 2.162 | ZS | 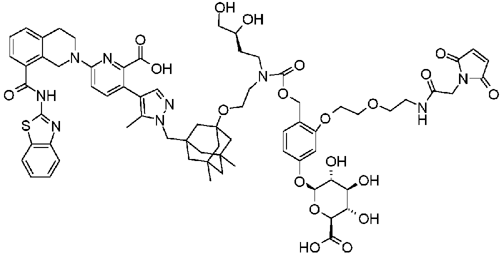 |
| 2.163 | ZW | 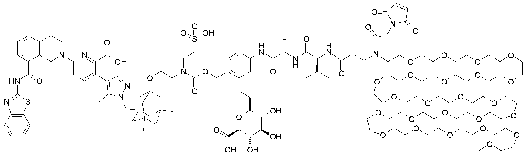 |
| 2.164 | ZX | 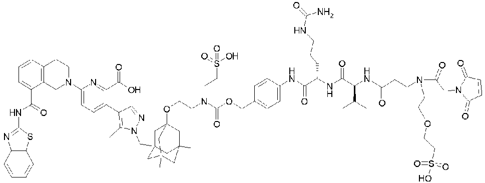 |
| 2.166 | AAA | 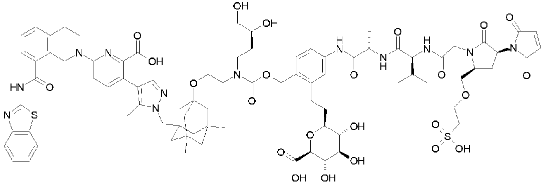 |
| 2.167 | AAD | 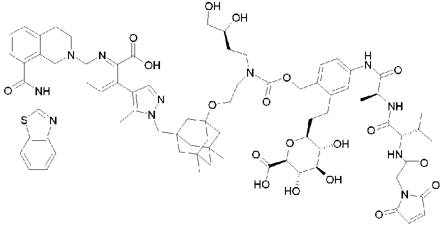 |
| 2.168 | AAE | 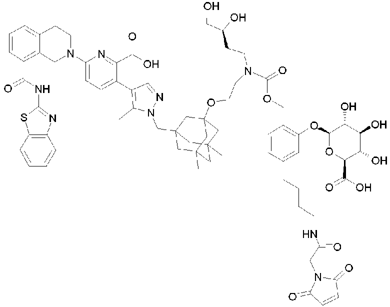 |
| 2.169 | ABG | 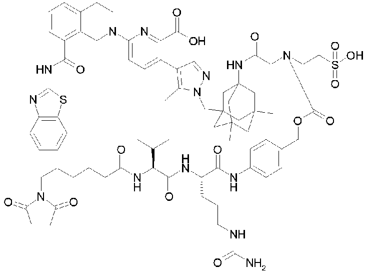 |
| 2.170 | ABL | 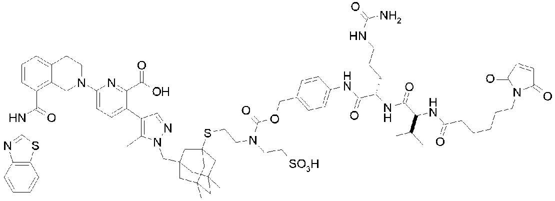 |
| 2.171 | ABN | 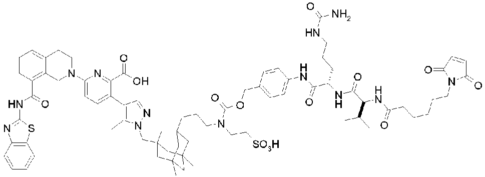 |
| 2.172 | AAF | 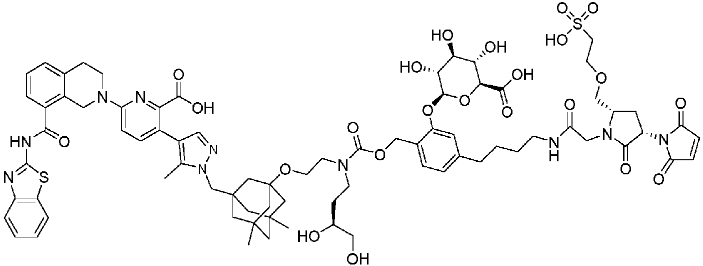 |
| 2.173 | ABO | 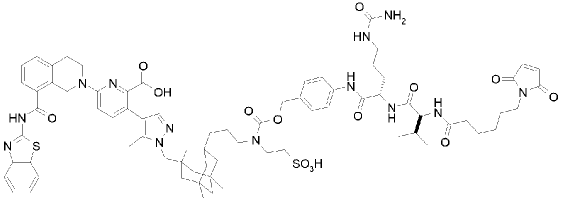 |
| 2.174 | ABM | 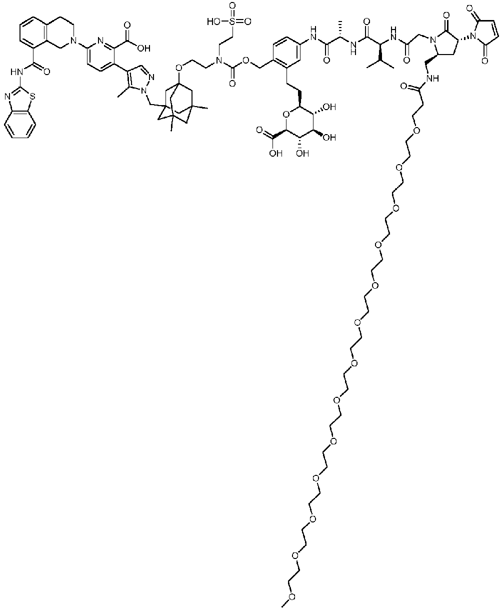 |
| 2.175 | ABU | 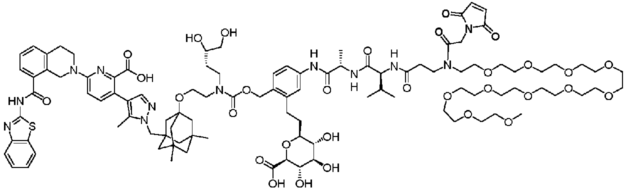 |
| 2.176 | ABV | 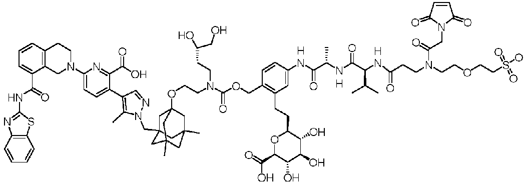 |
| 2.177 (control) | LB | 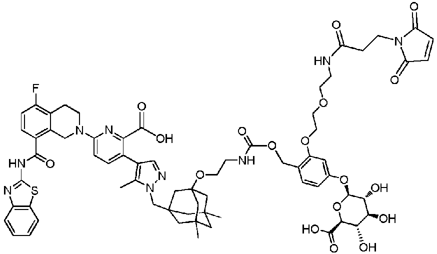 |
| 2.178 (control) | WD | 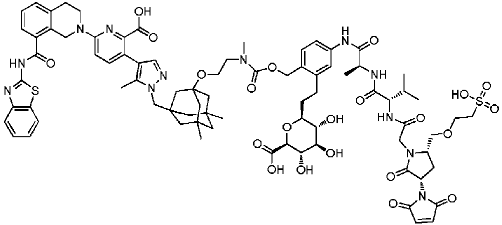 |
| 2.179 (control) | ZZ | 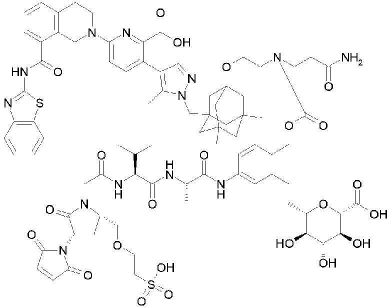 |
| 2.180 (control) | ZT | 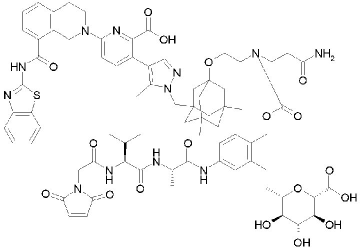 |
| 2.181 (control) | XW | 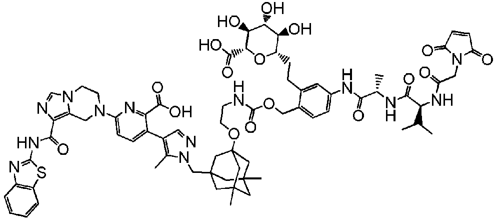 |
| 2.182 (control) | SE | 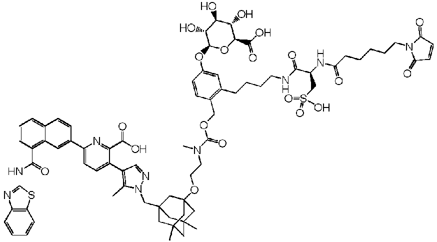 |
| 2.183 (control) | SR | 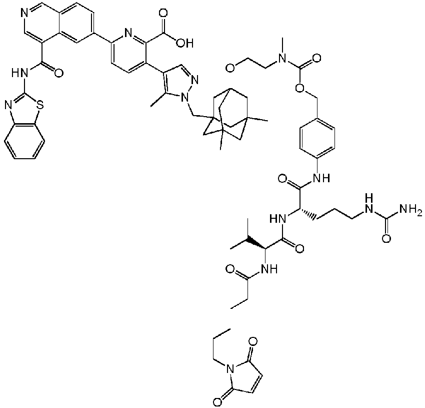 |
| 2.184 (control) | YG | 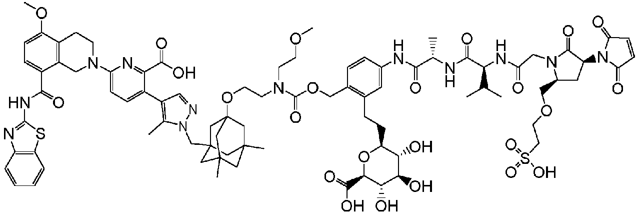 |
| 2.185 (control) | KZ | 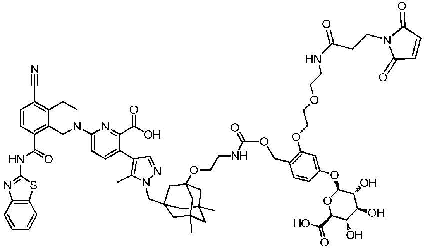 |

In certain embodiments, the synthon is selected from the group consisting of synthon examples 2.1, 2.2, 2.4, 2.5, 2.6, 2.7, 2.8, 2.9, 2.10, 2.11, 2.12, 2.13, 2.14, 2.15, 2.16, 2.17, 2.18, 2.19, 2.20, 2.21, 2.22, 2.23, 2.24, 2.25, 2.26, 2.27, 2.28, 2.29, 2.30, 2.31, 2.32, 2.33, 2.34, 2.35, 2.36, 2.37, 2.38, 2.39, 2.40, 2.41, 2.42, 2.43, 2.44, 2.45, 2.46, 2.47, 2.48, 2.49, 2.50, 2.51, 2.52, 2.53, 2.54, 2.55, 2.56, 2.57, 2.58, 2.59, 2.60, 2.61, 2.62, 2.63, 2.64, 2.65, 2.66, 2.67, 2.68, 2.69, 2.77, 2.78, 2.79, 2.80, 2.81, 2.82, 2.83, 2.84, 2.85, 2.86, 2.87, 2.88, 2.89, 2.90, 2.91, 2.92, 2.93, 2.94, 2.95, 2.96, 2.97, 2.98, 2.101, 2.102, 2.103, 2.104, 2.105, 2.106, 2.107, 2.108, 2.109, 2.110, 2.111, 2.112, 2.113, 2.114, 2.115, 2.116, 2.117, 2.118, 2.119, 2.120, 2.121, 2.122, 2.123, 2.124, 2.125, 2.126, 2.127, 2.128, 2.129, 2.130, 2.131, 2.132, 2.133, 2.134, 2.135, 2.136, 2.137, 2.138, 2.139, 2.140, 2.141, 2.142, 2.143, 2.144, 2.145, 2.146, 2.147, 2.148, 2.149, 2.150, 2.151, 2.152, 2.153, 2.154, 2.155, 2.156, 2.157, 2.158, 2.159, 2.160, 2.161, 2.162, 2.163, 2.164, 2.166, 2.167, 2.168, 2.169, 2.170, 2.171, 2.172, 2.173, 2.174, 2.175, and 2.176, or a pharmaceutically acceptable salt thereof. The compound names of these synthon are provided below:
N-[6-(2,5-dioxo-2,5-dihydro-1H-pyrrol-1-yl)hexanoyl]-L-valyl-N-{4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)methyl]phenyl}-N⁵-carbamoyl-L-ornithinamide;
N-[6-(2,5-dioxo-2,5-dihydro-1H-pyrrol-1-yl)hexanoyl]-L-valyl-N-{4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](3-sulfopropyl)carbamoyl}oxy)methyl]phenyl}-N⁵-carbamoyl-L-ornithinamide;
N-[6-(2,5-dioxo-2,5-dihydro-1H-pyrrol-l-yl)hexanoyl]-L-valyl-N-[4-({[{2-[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}dec-1-yl}oxy)ethoxy]ethyl}(2-sulfoethyl)carbamoyl]oxy}methyl)phenyl]-N⁵-carbamoyl-L-ornithinamide;
methyl 6-[4-(3-{[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](1[4-({N-[6-(2,5-dioxo-2,5-dihydro-1H-pyrrol-1-yl)hexanoyl]-L-valyl-N⁵-carbamoyl-L-ornithyl}amino)benzyl]oxy}carbonyl)amino}propyl)-1H-1,2,3-triazol-1-yl]-6-deoxy-beta-L-glucopyranoside;
N-[6-(2,5-dioxo-2,5-dihydro-1H-pyrrol-1-yl)hexanoyl]-L-valyl-N-(4-{[([2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl]{3-[1-(beta-D-glucopyranuronosyl)-1H-1,2,3-triazol-4-yl]propyl}carbamoyl) oxy]methyl}phenyl)-N⁵-carbamoyl-L-ornithinamide;
N-[6-(2,5-dioxo-2,5-dihydro-1H-pyrrol-1-yl)hexanoyl]-L-valyl-N-{4-[({[(2R)-1-{[2-({3-[(4-{6-[8-(1,3-benzothiazol-2-ylcarbamoyl)-3,4-dihydroisoqumolm-2(1H)-yl]-2-carboxypyridm-3-yl}-5-methyl-1H-pyrazol-1-yl)methyl]-5,7-dimethyltricyclo[3.3.1.1^{3,7}]dec-1-ylloxy)ethyl](methyl)aminol-1-oxo-3-sulfbpropan-2-yl]carbamoyl}oxy)methyl]phenyl}-L-alanmamide;
N-[6-(2,5-dioxo-2,5-dihydro-1H-pyrrol-1-yl)hexanoyl]-L-valyl-N-{4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-ylloxy)ethyl][4-(beta-D-glucopyranosyloxy)benzyl]carbainoyl}oxy)methyl]phenyl}-N⁵-carbarnoyl-L-omithinarnide;
N-[6-(2,5-dioxo-2,5-dihydro-1H-pyrrol-1-yl)hexanoyl]-L-valyl-N-{4-[({[4-(beta-D-allopyranosyloxy)benzyl][2-({3-[(4-{6-[8-(1,3-benzothiazol-2-ylcarbamoyl)-3,4-dihydroisoquinolin-2(1H)-yl]-2-carboxypyridin-3-yl} -5 -methyl- 1H-pyrazol-1-yl)methyl] -5,7-dimethyltricyclo[3.3.1.1^{3,7}]dec-1-yl}oxy)ethyl]carbamoyl}oxy)methyl]phenyl}-N⁵-carbamoyl-L-ornithinamide;
N-[6-(2,5-dioxo-2,5-dihydro-1H-pyrrol-1-yl)hexanoyl]-L-valyl-N-{4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{1,7}[dec-1-yl}oxy)ethyl](2-phosphonoethyl)carbarnoyl}oxy)methyl]phenyl}-N⁵-carbamoyl-L-omithinarnide;
N-[6-(2,5-dioxo-2,5-dihydro-1H-pyrrol-1-yl)hexanoyl]-L-valyl-N-{4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-phosphonoethyl)carbamoyl}oxy)methyl]phenyl}-L-alaninamide;
N-[6-(2,5-dioxo-2,5-dihydro-1H-pyrrol-1-yl)hexanoyl]-L-valyl-N-{4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](3-phosphonopropyl)carbamoyl}oxy)methyl]phenyl}-N⁵-carbamoyl-L-ornithinamide;
N-[6-(2,5-dioxo-2,5-dihydro-1H-pyrrol-1-yl)hexanoyl]-L-valyl-N-{4-[({[(2R)-1-{[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-ylloxy)ethyl]amino}-1-oxo-3-sulfopropan-2-yl]carbamoyl}oxy)methyl]phenyl}-L-alaninamide;
N-[6-(2,5-dioxo-2,5-dihydro-1H-pyrrol-1-yl)hexanoyl]-L-valyl-N-[4-({[{2-[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{1,7}]dec-1-yl}oxy)ethoxy]ethyl}(3-phosphonopropyl)carbamoyl]oxy}methyl)phenyl]-N⁵-carbamoyl-L-ornithinamide;
N-[6-(2,5-dioxo-2,5-dihydro-1H-pyrrol-1-yl)hexanoyl]-L-valyl-N-[4-({[{2-[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethoxy]ethyl}(3-phosphonopropyl)carbamoyl] oxy} methyl)phenyl] - L-alaninamide;
N-[6-(2,5-dioxo-2,5-dihydro-1H-pyrrol-1-yl)hexanoyl]-L-valyl-N-{4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](3-phosphonopropyl)carbamoyl} oxy)methyl]phenyl} -L-alaninamide;
6-[8-(1,3-benzothiazol-2-ylcarbamoyl)-3,4-dihydroisoquinolin-2(1H)-yl]-3-(1-{[3-(2-{[(2S)-3-carboxy-2-({[(4-{[(2S)-2-{[(2S)-2-{[6-(2,5-dioxo-2,5-dihydro-1H-pyrrol-1-yl)hexanoyl]amino}-3-methylbutanoyl]amino}propanoyl]amino}benzyl)oxy]carbonyl}amino)propanoyl](methyl)amino}eth oxy)-5,7-dimethyltricyclo[3.3.1.1^{3,7}]dec-1-yl]methyl}-5-methyl-1H-pyrazol-4-yl)pyridine-2-carboxylic acid;
N-[6-(2,5-dioxo-2,5-dihydro-1H-pyrrol-1-yl)hexanoyl]-L-valyl-N-{4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-ylloxy)ethyl][4-(beta-D-glucopyranuronosyloxy)benzyl]carbamoyl}oxy)methyl]phenyl}-N⁵-carbamoyl-L-ornithinamide;
N-[6-(2,5-dioxo-2,5-dihydro-1H-pyrrol-1-yl)hexanoyl]-L-valyl-N-{4-[({[2-({3-[(4-{6-[1-(1,3 -benzothiazol-2-ylcarbamoyl)-1,2,3,4-tetrahydroquinolin-7-yl] -2-carboxypyridin-3 -yl} -5 -methyl-1H-pyrazol-1-yl)methyl]-5,7-dimethyltricyclo[3.3.1.1^{3,7}]dec-1-yl}oxy)ethyl](2-phosphonoethyl)carbamoyl}oxy)methyl]phenyl}-N⁵-carbamoyl-L-ornithinamide;
N-[6-(2,5-dioxo-2,5-dihydro-1H-pyrrol-1-yl)hexanoyl]-L-valyl-N⁵-carbamoyl-N-{4-[({[2-({3-[(4-{2-carboxy-6-[8-([1,3]thiazolo[5,4-b]pyridin-2-ylcarbamoyl)-3,4-dihydroisoquinolin-2(1H)-yl]pyridin-3-yl}-5-methyl-1H-pyrazol-1-yl)methyl]-5,7-dimethyltricyclo[3.3.1.1^{3,7}]dec-1-yl} oxy)ethyl] (2-sulfoethyl)carbamoyl} oxy)methyl]phenyl} -L-omithinamide;
N-[6-(2,5-dioxo-2,5-dihydro-1H-pyrrol-1-yl)hexanoyl]-L-valyl-N-{4-[({[(2R)-1-{[2-({3-[(4-{6-[1-(1,3-benzothiazol-2-ylcarbamoyl)-1,2,3,4-tetrahydroquinolin-7-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](methyl)amino}-1-oxo-3-sulfopropan-2-yl]carbamoyl}oxy)methyl]phenyl}-N⁵-carbamoyl-L-ornithinamide;
N-[6-(2,5-dioxo-2,5-dihydro-1H-pyrrol-1-yl)hexanoyl]-L-valyl-N-{4-[({[(2R)-1-{[2-({3-[(4-{6-[1-(1,3-benzothiazol-2-ylcarbamoyl)-1,2,3,4-tetrahydroquinolin-7-yl]-2-carboxypyridin-3-yl}-5-methyl-1H-pyrazol-1-yl)methyl]-5,7-dimethyltricyclo[3.3.1.1^{1,7}]dec-1-ylloxy)ethyl](methyl)aminol-1-oxo-3-sulfopropan-2-yl]carbamoyl}oxy)methyl]phenyl}-L-alaninamide;
N-[6-(2,5-dioxo-2,5-dihydro-1H-pyrrol-1-yl)hexanoyl]-L-valyl-N⁵-carbamoyl-N-{4-[({[2-({3-[(4-{2-carboxy-6-[8-([1,3]thiazolo[4,5-b]pyridin-2-ylcarbamoyl)-3,4-dihydroisoquinolin-2(1H)-yl]pyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)methyl]phenyl}-L-omithinamide;
N-[6-(2,5-dioxo-2,5-dihydro-1H-pyrrol-1-yl)hexanoyl]-L-valyl-N-{4-[({[2-({3-[(4-{6-[1-(1,3-benzothiazol-2-ylcarbamoyl)-1,2,3,4-tetrahydroquinolin-7-yl]-2-carboxypyridin-3-yl}-5-methyl-1H-pyrazol-1-yl)methyl]-5,7-dimethyltricyclo[3.3.1.1^{3,7}]dec-1-ylloxy)ethyl](2-sulfoethyl)carbamoyl}oxy)methyl]phenyl}-N⁵-carbamoyl-L-ornithinamide;
N-[6-(2,5-dioxo-2,5-dihydro-1H-pyrrol-1-yl)hexanoyl]-L-valyl-N-{4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-carboxyethyl)carbamoyl}oxy)methyl]phenyl}-N⁵-carbamoyl-L-omithinamide;
N-[6-(2,5-dioxo-2,5-dihydro-1H-pyrrol-1-yl)hexanoyl]-L-valyl-N-{4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-carboxyethyl)carbamoyl}oxy)methyl]phenyl}-L-alaninamide;
6-[8-(1,3 -benzothiazol-2-ylcarbamoyl)-3,4-dihydroisoquinolin-2(1H)-yl]-3-(1- {[3 -(2- {[(2R)-3-carboxy-2-({[(4-{[(2S)-2-{[(2S)-2-{[6-(2,5-dioxo-2,5-dihydro-1H-pyrrol-1-yl)hexanoyl]amino}-3-methylbutanoyl]amino}propanoyl]amino}benzyl)oxy]carbonyl}amino)propanoyl](methyl)amino}eth oxy)-5,7-dimethyltricyclo[3.3.1.1^{3,7}]dec-1-yl]methyll-5-methyl-1H-pyrazol-4-yl)pyridine-2-carboxylic acid;
N-[6-(2,5-dioxo-2,5-dihydro-1H-pyrrol-1-yl)hexanoyl]-L-valyl-N-{4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl][1-(carboxymethyl)piperidin-4-yl]carbamoyl}oxy)methyl]phenyl}-N⁵-carbamoyl-L-omithinamide;
(S)-6-((2-((3-((4-(6-(8-(benzo[d]thiazol-2-ylcarbamoyl)-3,4-dihydroisoquinolm-2(1H)-yl)-2-carboxypyridin-3-yl)-5-methyl-1H-pyrazol-1-yl)methyl)-5,7-dimethyladamantan-1-yl)oxy)ethyl)(methyl)ammo)-5-((((4-((S)-2-((S)-2-(6-(2,5-dioxo-2,5-dihydro-1H-pyrrol-1-yl)hexanamido)-3-methylbutanamido)-5-ureidopentanamido)benzyl)oxy)carbonyl)amino)-N,N,N-trimethyl-6-oxohexan-1-aminium salt;
N-[6-(2,5-dioxo-2,5-dihydro-1H-pyrrol-1-yl)hexanoyl]-L-valyl-N-{4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-ylloxy)ethyl](2-sulfoethyl)carbamoyl}oxy)methyl]phenyl}-L-alaninamide;
N-[6-(2,5-dioxo-2,5-dihydro-1H-pyrrol-1-yl)hexanoyl]-L-valyl-N-[4-({[(4-{[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)amino}piperidin-1-yl)carbonyl]oxy}methyl)phenyl]-N⁵-carbamoyl-L-ornithinamide;
N-[6-(2,5-dioxo-2,5-dihydro-1H-pyrrol-1-yl)hexanoyl]-L-valyl-N-{4-[({[2-({3-[(4-{6-[8-(1,3-benzothiazol-2-ylcarbamoyl)-5-(3-phosphonopropoxy)-3,4-dihydroisoquinolm-2(1H)-yl]-2-carboxypyridin-3 -yl} -5 -methyl-1H-pyrazol-1-yl)methyl] -5,7-dimethyltricyclo[3.3.1.1^{3,7}]dec-1-yl}oxy)ethyl](methyl)carbamoyl}oxy)methyl]phenyl}-N⁵-carbamoyl-L-omithinamide;
N-[6-(2,5-dioxo-2,5-dihydro-1H-pyrrol-1-yl)hexanoyl]-L-valyl-N-[4-({[(4-{[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](3-phosphonopropyl)amino}piperidin-1-yl)carbonyl]oxy}methyl)phenyl]-N⁵-carbamoyl-L-ornithinamide;
N-[6-(2,5-dioxo-2,5-dihydro-1H-pyrrol-1-yl)hexanoyl]-L-valyl-N-{4-[({[2-({3-[(4-{6-[8-(1,3 -benzothiazol-2-ylcarbamoyl)naphthalen-2-yl] -2-carboxypyridin-3 -yl} -5 -methyl- 1H-pyrazol-1-yl)methyl]-5,7-dimethyltricyclo[3.3.1.1^{3,7}]dec-1-yl}oxy)ethyl](3-phosphonopropyl)carbamoyl}oxy)methyl]phenyl}-N⁵-carbamoyl-L-ornithinamide;
N-[6-(2,5-dioxo-2,5-dihydro-1H-pyrrol-1-yl)hexanoyl]-L-valyl-N-[4-({[(4-{[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-carboxyethyl)amino}piperidin-1-yl)carbonyl]oxy}methyl)phenyl]-N⁵-carbamoyl-L-ornithinamide;
N-[6-(2,5-dioxo-2,5-dihydro-1H-pyrrol-1-yl)hexanoyl]-L-valyl-N⁵-carbamoyl-N-{4-[({[2-({3-[(4-{2-carboxy-6-[8-([1,3]thiazolo[4,5-b]pyridin-2-ylcarbamoyl)-3,4-dihydroisoqumolm-2(1H)-yl]pyridin-3-yl} -5 -methyl-1H-pyrazol-1-yl)methyl] -5,7-dimethyltricyclo[3.3.1.1^{3,7}]dec-1-yl} oxy)ethyl] (3 -phosphonopropyl)carbamoyl} oxy)methyl]phenyl} -L-ornithinamide;
N-[6-(2,5-dioxo-2,5-dihydro-1H-pyrrol-1-yl)hexanoyl]-L-valyl-N⁵-carbamoyl-N-{4-[({[2-({3-[(4-{2-carboxy-6-[8-([1,3]thiazolo[5,4-b]pyridm-2-ylcarbamoyl)-3,4-dihydroisoqumolm-2(1H)-yl]pyridin-3-yl}-5-methyl-1H-pyrazol-l-yl)methyl]-5,7-dimethyltricyclo[3.3.1.1^{3,7}]dec-1-yl} oxy)ethyl] (3 -phosphonopropyl)carbamoyl} oxy)methyl]phenyl} -L-ornithinamide;
N-{6-[(chloroacetyl)amino]hexanoyl}-L-valyl-N-{4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltncyclo[3.3.1.1^{3,7}]dec-1-yl} oxy)ethyl] (2-sulfoethyl)carbamoyl}oxy)methyl]phenyl}-L-alaninamide;
N-[6-(2,5-dioxo-2,5-dihydro-1H-pyrrol-1-yl)hexanoyl]-L-valyl-N-{4-[({[2-({3-[(4-{6-[8-(1,3-benzothiazol-2-ylcarbamoyl)-5-(carboxymethoxy)-3,4-dihydroisoquinolm-2(1H)-yl]-2-carboxypyridin-3 -yl} -5 -methyl-1H-pyrazol-1-yl)methyl] -5,7-dimethyltricyclo[3.3.1.1^{3,7}]dec-1-yl} oxy)ethyl] (methyl)carbamoyl} oxy)methyl]phenyl} -N⁵-carbamoyl-L-ornithinamide;
N-[6-(2,5-dioxo-2,5-dihydro-1H-pyrrol-1-yl)hexanoyl]-L-valyl-N-[4-({[(2-{[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)amino}ethyl)(2-carboxyethyl)carbamoyl]oxy}methyl)phenyl]-N⁵-carbamoyl-L-ornithinamide;
6-[8-(1,3-benzothiazol-2-ylcarbamoyl)-3,4-dihydroisoquinolin-2(1H)-yl]-3-[1-({3-[2-({(2S)-2-[{[(4-{[(2S)-5-(carbamoylamino)-2-{[(2S)-2-{[6-(2,5-dioxo-2,5-dihydro-1H-pyrrol-1-yl)hexanoyl]amino}-3-methylbutanoyl]amino}pentanoyl]amino}benzyl)oxy]carbonyl}(2-carboxyethyl)amino]-3-carboxypropanoyl}amino)ethoxy]-5,7-dimethyltricyclo[3.3.1.1^{3,7}]dec-1-yl}methyl)-5-methyl-1H-pyrazol-4-yl]pyridine-2-carboxylic acid;
6-[8-(1,3-benzothiazol-2-ylcarbamoyl)-3,4-dihydroisoquinolin-2(1H)-yl]-3-(1-{[3-(2-{[(2S)-2-({[(4-{[(2S)-5-(carbamoylammo)-2-{[(2S)-2-{[6-(2,5-dioxo-2,5-dihydro-1H-pyrrol-1-yl)hexanoyl]amino}-3-methylbutanoyl]amino}pentanoyl]amino}benzyl)oxy]carbonyl}amino)-3-carboxypropanoyl] (2-sulfoethyl)amino }ethoxy)-5,7-dimethyltricyclo[3.3 .1. 1^{3,7}]dec-1-yl]methyl}-5-methyl-1H-pyrazol-4-yl)pyridine-2-carboxylic acid;
N-[6-(2,5-dioxo-2,5-dihydro-1H-pyrrol-1-yl)hexanoyl]-L-valyl-N-[4-({[(4-{[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-ylloxy)ethyl](3-carboxypropyl)amino}piperidin-1-yl)carbonyl]oxy}methyl)phenyl]-N⁵-carbamoyl-L-ornithinamide;
4-[(1E)-3-({[2-({3-[(4-{6-[8-(1,3-benzothiazol-2-ylcarbamoyl)-5-(carboxymethoxy)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-ylloxy)ethyl](methyl)carbarnoylloxy)prop-1-en-1-yl]-2-({N-[6-(2,5-dioxo-2,5-dihydro-1H-pyrrol-1-yl)hexanoyl]-beta-alanyl}amino)phenylbeta-D-glucopyranosiduronic acid;
N-[6-(2,5-dioxo-2,5-dihydro-1H-pyrrol-1-yl)hexanoyl]-L-valyl-N-{4-[({[2-({3-[(4-{6-[8-(1,3-benzothiazol-2-ylcarbamoyl)naphthalen-2-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)methyl]phenyl}-N⁵-carbamoyl-L-ornithinamide;
N-[6-(2,5-dioxo-2,5-dihydro-1H-pyrrol-1-yl)hexanoyl]-L-valyl-N-[4-({[(2-{[8-(1,3-benzothiazol-2-ylcarbamoyl)-2-(6-carboxy-5-{1-[(3,5-dimethyl-7-{2-[methyl(2-sulfoethyl)amino]ethoxyltricyclo[3.3.1.1^{3,7}]dec-1-yl)methyl]-5-methyl-1H-pyrazol-4-yl}pyridin-2-yl)-1,2,3,4-tetrahydroisoquinolin-5-yl]oxy}ethyl)carbamoyl]oxy}methyl)phenyl]-N⁵-carbamoyl-L-ornithinamide;
N-[6-(2,5-dioxo-2,5-dihydro-1H-pyrrol-1-yl)hexanoyl]-L-valyl-N-[4-({[(2-{[8-(1,3-benzothiazol-2-ylcarbamoyl)-2-(6-carboxy-5-{1-[(3,5-dimethyl-7-{2-[methyl(2-sulfoethyl)amino]ethoxyltricyclo[3.3.1.1^{3,7}]dec-1-yl)methyl]-5-methyl-1H-pyrazol-4-yl}pyridin-2-yl)-1,2,3,4-tetrahydroisoquinolin-5-yl]oxy}ethyl)(2-sulfoethyl)carbamoyl]oxy}methyl)phenyl]-N⁵-carbamoyl-L-ornithinamide;
N-[6-(2,5-dioxo-2,5-dihydro-1H-pyrrol-1-yl)hexanoyl]-L-valyl-N-[4-({[(2-{[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-ylloxy)ethyl](2-sulfoethyl)amino}ethyl)(2-sulfoethyl)carbamoyl]oxy}methyl)phenyl]-N⁵-carbamoyl-L-omithinamide;
N-{6-[(chloroacetyl)amino]hexanoyl}-L-valyl-N-{4-[({[2-({3-[(4-{6-[8-[1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)methyl]phenyl}-N⁵-carbamoyl-L-ornithinamide;
N-[6-(2,5-dioxo-2,5-dihydro-1H-pyrrol-1-yl)hexanoyl]-L-valyl-N-{4-[({[2-({3-[(4-{6-[4-(1,3-benzothiazol-2-ylcarbamoyl)-3,4-dihydro-2H-1,4-benzoxazin-6-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)methyl]phenyl}-N⁵-carbamoyl-L-ornithinamide;
N-[6-(2,5-dioxo-2,5-dihydro-1H-pyrrol-1-yl)hexanoyl]-L-valyl-N-[4-({[(2-{[8-(1,3-benzothiazol-2-ylcarbamoyl)-2-(6-carboxy-5-{1-[(3,5-dimethyl-7-{2-[methyl(2-sulfoethyl)amino]ethoxy}tricyclo[3.3.1.1^{1,7}]dec-1-yl)methyl]-5-methyl-1H-pyrazol-4-yl}pyridin-2-yl)-1,2,3,4-tetrahydroisoquinolin-5-yl]oxy}ethyl)(2-carboxyethyl)carbamoyl]oxy}methyl)phenyl]-N⁵-carbamoyl-L-ornithinamide;
N-[6-(2,5-dioxo-2,5-dihydro-1H-pyrrol-1-yl)hexanoyl]-L-valyl-N-{4-[({[2-({3-[(4-{6-[8-(1,3-benzothiazol-2-ylcarbamoyl)-5-(3-sulfopropoxy)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{1,7}]dec-1-yl}oxy)ethyl](methyl)carbamoyl}oxy)methyl]phenyl}-N⁵-carbamoyl-L-ornithinamide;
N-[3-(2,5-dioxo-2,5-dihydro-1H-pyrrol-1-yl)propanoyl]-L-valyl-N-{4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)methyl]phenyl}-N⁵-carbamoyl-L-ornithinamide;
N-[(2,5-dioxo-2,5-dihydro-1H-pyrrol-1-yl)acetyl]-L-valyl-N-{4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{1,7}]dec-1-yl}oxy)ethyl](2-sulfoethyl)carbamoyl}oxy)methyl]phenyl}-N⁵-carbamoyl-L-ornithinamide;
6-[8-(1,3-benzothiazol-2-ylcaibamoyl)-3,4-dihydroisoquinolin-2(1H)-yl]-3-(1-{[3-(2-{[(2S)-2-({[(4-{[(2S,3R,4S,5S,6S)-6-carboxy-3,4,5-trihydroxytetrahydro-2H-pyran-2-yl]oxy}-3-[(3-{[6-(2,5-dioxo-2,5-dihydro-1H-pyrrol-1-yl)hexanoyl]amino}propanoyl)amino]benzyl)oxy]carbonyl} amino)-3-sulfopropanoyl](methyl)amino}ethoxy)-5,7-dimethyltricyclo[3.3.1.1^{3,7}]dec-1-yl]methyl}-5-methyl-1H-pyrazol-4-yl)pyridine-2-carboxylic acid;
4-[(1E)-3-({[2-({3-[(4-{2-carboxy-6-[8-([1,3]thiazolo[5,4-b]pyridin-2-ylcarbamoyl)-3,4-dihydroisoquinolin-2(1H)-yl]pyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)prop-1-en-1-yl]-2-({N-[6-(2,5-dioxo-2,5-dlhydro-1H-pyrrol-1-yl)hexanoyl]-beta-alanyl}amino)phenyl beta-D-glucopyranosiduronic acid;
4-[(1E)-3-({[2-({3-[(4-{2-carboxy-6-[8-([1,3]thiazolo[4,5-b]pyridin-2-ylcarbamoyl)-3,4-dihydroisoquinolin-2(1H)-yl]pyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)prop-1-en-1-yl]-2-({N-[6-(2,5-dioxo-2,5-dlhydro-1H-pyrrol-1-yl)hexanoyl]-beta-alanyl}amino)phenyl beta-D-glucopyranosiduronic acid;
4-[(1E)-3-({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)prop-1-en-1-yl]-2-({N-[6-(2,5-dioxo-2,5-dihydro-1H-pyrrol-1-yl)hexanoyl]-beta-alanyl}amino)phenyl beta-D-glucopyranosiduronic acid;
4-[(1E)-3-({[2-({3-[(4-{2-carboxy-6-[8-([1,3]thiazolo[5,4-b]pyridin-2-ylcarbamoyl)-3,4-dihydroisoquinolin-2(1H)-yl]pyridin-3-yl}-5-methyl-1H-pyrazol-1-yl)methyl]-5,7-dimethyltricyclo[3.3.1.1^{3,7}]dec-1-yl}oxy)ethyl](3-phosphonopropyl)carbamoyl}oxy)prop-1-en-1-yl]-2-({N-[6-(2,5-dioxo-2,5-dihydro-1H-pyrrol-1-yl)hexanoyl]-beta-alanyl}amino)phenyl beta-D-glucopyranosiduronic acid;
4-[(1E)-3-({[2-({3-[(4-{6-[8-(1,3-benzothiazol-2-ylcarbamoyl)-5-(3-phosphonopropoxy)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](methyl)carbamoyl}oxy)prop-1-en-1-yl]-2-({N-[6-(2,5-dioxo-2,5-dihydro-1H-pyrrol-1-yl)hexanoyl]-beta-alanyl}amino)phenyl beta-D-glucopyranosiduronic acid;
4-[(1E)-3-({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](3-phosphonopropyl)carbamoyl}oxy)prop-1-en-1-yl]-2-({N-[6-(2,5-dioxo-2,5-dihydro-1H-pyrrol-1-yl)hexanoyl]-beta-alanyl}amino)phenyl beta-D-glucopyranosiduronic acid;
4-[({[2-({3-[(4-{6-[8-(1,3-benzothiazol-2-ylcarbamoyl)-3,4-dihydroisoquinolm-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)methyl]-3-[2-(2-{[3-(2,5-dioxo-2,5-dihydro-1H-pyrrol-1-yl)propanoyl]amino}ethoxy)ethoxy]phenylbeta-D-glucopyranosiduronic acid;
4-[(1E)-3-({[2-({3-[(4-{2-carboxy-6-[8-([1,3]thiazolo[4,5-b]pyridin-2-ylcarbamoyl)-3,4-dihydroisoquinolin-2(1H)-yl]pyridin-3-yl}-5-methyl-1H-pyrazol-1-yl)methyl]-5,7-dimethyltricyclo[3.3.1.1^{3,7}]dec-1-yl}oxy)ethyl](3-phosphonopropyl)carbamoyl}oxy)prop-1-en-1-yl]-2-({N-[6-(2,5-dioxo-2,5-dihydro-1H-pyrrol-1-yl)hexanoyl]-beta-alanyl}amino)phenyl beta-D-glucopyranosiduronic acid;
6-[8-(1,3-benzothiazol-2-ylcarbamoyl)-3,4-dihydroisoquinolm-2(1H)-yl]-3-{1-[(3-{2-[(2-carboxyethyl)({[(2E)-3-(4-{[(2S,3R,4S,5S,6S)-6-carboxy-3,4,5-trihydroxytetrahydro-2H-pyran-2-yl]oxy}-3-[(3-{[6-(2,5-dioxo-2,5-dihydro-1H-pyrrol-1-yl)hexanoyl]amino}propanoyl)amino]phenyl)prop-2-en-1-yl]oxy}carbonyl)amino]ethoxy}-5,7-dimethyltricyclo[3.3.1.1^{3,7}]dec-1-yl)methyl]-5-methyl-1H-pyrazol-4-yl}pyridine-2-carboxylic acid;
6-[8-(1,3-benzothiazol-2-ylcarbamoyl)-3,4-dihydroisoquinolm-2(1H)-yl]-3-{1-[(3-{2-[(2-carboxyethyl){[(4-{[(2S,3R,4S,5S,6S)-6-carboxy-3,4,5-trihydroxytetrahydro-2H-pyran-2-yl]oxy}-2-[2-(2-{[3-(2,5-dioxo-2,5-dihydro-1H-pyrrol-1-yl)propanoyl]amino}ethoxy)ethoxy]benzyl)oxy]carbonyl}amino]ethoxy}-5,7-dimethyltricyclo[3.3.1.1^{3,7}]dec-1-yl)methyl]-5-methyl-1H-pyrazol-4-yl}pyridine-2-carboxylic acid;
N-[6-(ethenylsulfonyl)hexanoyl]-L-valyl-N-{4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)methyl]phenyl}-N⁵-carbamoyl-L-ornithinamide;
4-[(1E)-3-{[(4-{[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](3-phosphonopropyl)amino}piperidin-1-yl)carbonyl]oxy}prop-1-en-1-yl]-2-({N-[6-(2,5-dioxo-2,5-dihydro-1H-pyrrol-1-yl)hexanoyl]-beta-alanyl}amino)phenyl beta-D-glucopyranosiduronic acid;
4-[(1E)-3-{[(4-{[2-({3-[(4-{2-carboxy-6-[8-([1,3]thiazolo[4,5-b]pyridin-2-ylcarbamoyl)-3,4-dihydroisoquinolin-2(1H)-yl]pyridin-3-yl}-5-methyl-1H-pyrazol-1-yl)methyl]-5,7-dimethyltricyclo[3.3.1.1^{3,7}]dec-1-yl}oxy)ethyl](3-phosphonopropyl)amino}piperidin-1-yl)carbonyl]oxy}prop-1-en-1-yl]-2-({N-[6-(2,5-dioxo-2,5-dihydro-1H-pyrrol-1-yl)hexanoyl]-beta-alanyl}amino)phenyl beta-D-glucopyranosiduronic acid;
4-[(1E)-3-({[2-({3-[(4-{6-[8-(1,3-benzothiazol-2-ylcarbamoyl)naphthalen-2-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)prop-1-en-1-yl]-2-({N-[6-(2,5-dioxo-2,5-dihydro-1H-pyrrol-1-yl)hexanoyl]-beta-alanyl}amino)phenyl beta-D-glucopyranosiduronic acid;
6-[8-(1,3-benzothiazol-2-ylcarbamoyl)-3,4-dihydroisoquinolin-2(1H)-yl]-3-[1-({3-[2-({N-[6-(2,5-dioxo-2,5-dihydro-1H-pyrrol-1-yl)hexanoyl]-3-sulfo-L-alanyl}amino)ethoxy]-5,7-dimethyltricyclo[3.3.1.1^{3,7}]dec-1-yl}methyl)-5-methyl-1H-pyrazol-4-yl]pyridine-2-carboxylic acid;
6-[8-(1,3-benzothiazol-2-ylcarbamoyl)-3,4-dihydroisoquinolin-2(1H)-yl]-3-[1-({3-[2-(2-{[6-(2,5-dioxo-2,5-dihydro-1H-pyrrol-1-yl)hexanoyl](2-sulfoethyl)amino}ethoxy)ethoxy]-5,7-dimethyltricyclo[3.3.1.1^{3,7}]dec-1-yl}methyl)-5-methyl-1H-pyrazol-4-yl]pyridine-2-carboxylic acid;
6-[8-(1,3-benzothiazol-2-ylcarbamoyl)-3,4-dihydroisoquinolin-2(1H)-yl]-3-(1-{[3-(2-{[6-(2,5-dioxo-2,5-dihydro-1H-pyrrol-1-yl)hexanoyl](2-sulfoethyl)amino}ethoxy)-5,7-dimethyltricyclo[3.3.1.1^{3,7}]dec-1-yl]methyl}-5-methyl-1H-pyrazol-4-yl)pyridine-2-carboxylic acid;
6-[8-(1,3-benzothiazol-2-ylcarbamoyl)-3,4-dihydroisoquinolin-2(1H)-yl]-3-{1-[(3-{[1-(2,5-dioxo-2,5-dihydro-1H-pyrrol-1-yl)-21-oxo-22-(2-sulfoethyl)-3,6,9,12,15,18-hexaoxa-22-azatetracosan-24-yl]oxy}-5,7-dimethyltricyclo[3.3.1.1^{1,7}]dec-1-yl)methyl]-5-methyl-1H-pyrazol-4-yl}pyridine-2-carboxylic acid;
6-[8-(1,3-benzothiazol-2-ylcarbamoyl)-3,4-dihydroisoquinolin-2(1H)-yl]-3-{1-[(3-{[1-(2,5-dioxo-2,5-dihydro-1H-pyrrol-1-yl)-21-oxo-22-(2-sulfoethyl)-3,6,9,12,15,18,25-heptaoxa-22-azaheptacosan-27-yl]oxy}-5,7-dimethyltricyclo[3.3.1.1^{3,7}]dec-1-yl)methyl]-5-methyl-1H-pyrazol-4-yl}pyridine-2-carboxylic acid;
6-[8-(1,3-benzothiazol-2-ylcarbamoyl)-3,4-dihydroisoquinolin-2(1H)-yl]-3-(-(1-{[3-(2-{[6-(ethenylsulfonyl)hexanoyl](2-sulfoethyl)amino}ethoxy)-5,7-dimethyltricyclo[3.3.1.1^{3,7}]dec-1-yl]methyl} -5 -methyl- 1H-pyrazol-4-yl)pyridine-2-carboxylic acid;
6-[8-(1,3-benzothiazol-2-ylcarbamoyl)-3,4-dihydroisoquinolin-2(1H)-yl]-3-{1-[(3-{2-[{6-[(chloroacetyl)amino]hexanoyl}(2-sulfoethyl)amino]ethoxy}-5,7-dimethyltricyclo[3.3.1.1^{3,7}]dec-1-yl)methyl]-5-methyl-1H-pyrazol-4-yl}pyridine-2-carboxylic acid;
N-[6-(2,5-dioxo-2,5-dihydro-1H-pyrrol-1-yl)hexanoyl]-L-valyl-N-{4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](3-carboxypropyl)carbamoyl}oxy)methyl]phenyl}-N⁵-carbamoyl-L-ornithinamide;
N-{6-[(bromoacetyl)amino]hexanoyl}-L-valyl-N-{4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltncyclo[3.3.1.1^{3,7}]dec-1-yl} oxy)ethyl] (2-sulfoethyl)carbamoyl}oxy)methyl]phenyl}-N⁵-carbamoyl-L-ornithinamide;
4-[({[2-({3-[(4-{6-[8-(1,3-benzothiazol-2-ylcarbamoyl)-3,4-dihydroisoquinolm-2(1H)-yl]-2-carboxypyridin-3 -yl} -5 -methyl-1H-pyrazol-1-yl)methyl] -5,7-dimethyltricyclo[3.3 .1.1^{3,7}]dec-1-yl}oxy)ethyl] (3 -carboxypropyl)carbamoyl}oxy)methyl] -3 -[2-(2- {[3 -(2,5 -dioxo-2,5 -dihydro- 1H-pyrrol-1-yl)propanoyl]amino}ethoxy)ethoxy]phenyl beta-D-glucopyranosiduronic acid;
4-({[(4-{[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](3-carboxypropyl)ammo}piperidm-1-yl)carbonyl]oxy}methyl)-3-[2-(2-{[3-(2,5-dioxo-2,5-dihydro-1H-pyrrol-1-yl)propanoyl]amino}ethoxy)ethoxy]phenyl beta-D-glucopyranosiduronic acid;
4-[({[2-({3-[(4-{6-[8-(1,3-benzothiazol-2-ylcarbamoyl)-3,4-dihydroisoquinolm-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](3-sulfopropyl)carbamoyl}oxy)methyl]-3 -[2-(2- {[3 -(2,5 -dioxo-2,5 -dihydro- 1H-pyrrol-1-yl)propanoyl]amino}ethoxy)ethoxy]phenyl beta-D-glucopyranosiduronic acid;
N-[6-(2,5-dioxo-2,5-dihydro-1H-pyrrol-1-yl)hexanoyl]-L-valyl-N-[4-({[(3-{[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)amino}azetidin-1-yl)carbonyl]oxy}methyl)phenyl]-N⁵-carbamoyl-L-ornithinamide;
6-[8-(1,3-benzothiazol-2-ylcarbamoyl)-3,4-dihydroisoquinolin-2(1H)-yl]-3-{1-[(3-{[26-(2,5-dioxo-2,5-dihydro-1H-pyrrol-1-yl)-8,24-dioxo-3-(2-sulfoethyl)-11,14,17,20-tetraoxa-3,7,23-triazahexacos-1-yl]oxy}-5,7-dimethyltricyclo[3.3.1.1^{3,7}]dec-1-yl)methyl]-5-methyl-1H-pyrazol-4-yl}pyridine-2-carboxylic acid;
N-[6-(2,5-dioxo-2,5-dihydro-1H-pyrrol-1-yl)hexanoyl]-L-valyl-N-[4-({[(3-{[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)amino}propyl)carbamoyl]oxy}methyl)phenyl] -N⁵-carbamoyl-L-ornithinamide;
N-{6-[(iodoacetyl)amino]hexanoyl}-L-valyl-N-{4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)methyl]phenyl}-N⁵-carbamoyl-L-ornithinamide;
N-{6-[(ethenylsulfonyl)amino]hexanoyl}-L-valyl-N-{4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)methyl]phenyl}-N⁵-carbamoyl-L-ornithinamide;
N-{6-[(ethenylsulfonyl)amino]hexanoyl}-L-valyl-N-{4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)methyl]phenyl}-N⁵-carbamoyl-L-ornithinamide;
6-[8-(1,3-benzothiazol-2-ylcarbamoyl)-3,4-dihydroisoquinolm-2(1H)-yl]-3-{1-[(3-{2-[(3-{[6-(ethenylsulfonyl)hexanoyl]amino}propyl)(2-sulfoethyl)amino]ethoxy}-5,7-dimethyltricyclo[3.3.1.1^{3,7}]dec-1-yl)methyl]-5-methyl-1H-pyrazol-4-yl}pyridine-2-carboxylic acid;
N-[3-(2,5-dioxo-2,5-dihydro-1H-pyrrol-1-yl)propanoyl]-L-valyl-N-{4-[({[2-({3-[(4-{6-[1-(1,3-benzothiazol-2-ylcarbamoyl)-1,2,3,4-tetrahydroquinolin-7-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)methyl]phenyl}-N⁵-carbamoyl-L-ornithinamide;
6-[8-(1,3-benzothiazol-2-ylcarbamoyl)-3,4-dihydroisoquinolin-2(1H)-yl]-3-{1-[(3-{2-[(2-carboxyethyl){[(2-{[(2S,3R,4S,5S,6S)-6-carboxy-3,4,5-trihydroxytetrahydro-2H-pyran-2-yl]oxy}-4-[2-(2-{[3-(2,5-dioxo-2,5-dihydro-1H-pyrrol-1-yl)propanoyl] amino } ethoxy)ethoxy]benzyl)oxy]carbonyl} amino]ethoxy } -5,7-dimethyltricyclo[3.3.1.1^{3,7}]dec-1-yl)methyl]-5-methyl-1H-pyrazol-4-yl}pyridine-2-carboxylic acid;
N-[6-(2,5-dioxo-2,5-dihydro-1H-pyrrol-1-yl)hexanoyl]-3-sulfo-L-alanyl-L-valyl-N-{4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-carboxyethyl)carbamoyl}oxy)methyl]phenyl}-N⁵-carbamoyl-L-omithinamide;
6-[8-(1,3-benzothiazol-2-ylcarbamoyl)-3,4-dihydroisoquinolin-2(1H)-yl]-3-{1-[(3-{[(43S,46S)-43-({[(4-{[(2S)-2-{[(2S)-2-{[6-(2,5-dioxo-2,5-dihydro-1H-pyrrol-1-yl)hexanoyl]amino}-3-methylbutanoyl]amino}propanoyl]amino}benzyl)oxy]carbonyl}amino)-46-methyl-37,44,47-trioxo-2,5,8,11,14,17,20,23,26,29,32,35-dodecaoxa-38,45,48-triazapentacontan-50-yl]oxy}-5,7-dimethyltricyclo[3.3.1.1^{3,7}]dec-1-yl)methyl]-5-methyl-1H-pyrazol-4-yl}pyridme-2-carboxylic acid;
N-[6-(2,5-dioxo-2,5-dihydro-1H-pyrrol-1-yl)hexanoyl]-L-valyl-N-{4-[({[2-({3-[(4-{6-[1-(1,3-benzothiazol-2-ylcarbamoyl)-1,2,3,4-tetrahydroquinolin-7-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-carboxyethyl)carbamoyl}oxy)methyl]phenyl}-N⁵-carbamoyl-L-omithinamide;
N-[6-(2,5-dioxo-2,5-dihydro-1H-pyrrol-1-yl)hexanoyl]-L-valyl-N-{4-[({[2-({3-[(4-{6-[8-(1,3 -benzothiazol-2-ylcarbamoyl)naphthalen-2-yl] -2-carboxypyridin-3 -yl} -5 -methyl- 1H-pyrazol-1-yl)methyl]-5,7-dimethyltncyclo[3.3.1.1^{3,7}]dec-1-yl} oxy)ethyl] (2-carboxyethyl)carbamoyl}oxy)methyl]phenyl}-N⁵-carbamoyl-L-omithinamide;
6-[8-(1,3-benzothiazol-2-ylcarbamoyl)naphthalen-2-yl]-3-{1-[(3-{2-[(2-carboxyethyl){[(2-{[(2R,3S,4R,5R,6R)-6-carboxy-3,4,5-trihydroxytetrahydro-2H-pyran-2-yl]oxy}-4-[2-(2-{[6-(2,5-dioxo-2,5-dihydro-1H-pyrrol-1-yl)hexanoyl] amino } ethoxy)ethoxy]benzyl)oxy]carbonyl}amino]ethoxy } -5,7-dimethyltricyclo[3.3.1.1^{3,7}]dec-1-yl)methyl]-5-methyl-1H-pyrazol-4-yl}pyridine-2-carboxylic acid;
N-[6-(2,5-dioxo-2,5-dihydro-1H-pyrrol-1-yl)hexanoyl]-L-valyl-N-{4-[({[2-({3-[(4-{6-[5-(1,3-benzothiazol-2-ylcarbamoyl)quinolin-3-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)methyl]phenyl}-N⁵-carbamoyl-L-ornithinamide;
N-[6-(2,5-dioxo-2,5-dihydro-1H-pyrrol-1-yl)hexanoyl]-L-valyl-N-{4-[({[2-({3-[(4-{6-[4-(1,3-benzothiazol-2-ylcarbamoyl)quinolin-6-yl]-2-carboxypyridin-3-yl}-5-methyl-1H-pyrazol-1-yl)methyl]-5,7-dimethyltncyclo[3.3.1.1^{3,7}]dec-1-yl} oxy)ethyl] (2-carboxyethyl)carbamoyl}oxy)methyl]phenyl}-N⁵-carbamoyl-L-omithinamide;
N-[6-(2,5-dioxo-2,5-dihydro-1H-pyrrol-1-yl)hexanoyl]-L-valyl-N-{4-[({[2-({3-[(4-{6-[4-(1,3-benzothiazol-2-ylcarbamoyl)quinolin-6-yl]-2-carboxypyridin-3-yl} -5 -methyl- 1H-pyrazol-1-yl)methyl]-5,7-dimethyltncyclo[3.3.1.1^{3,7}]dec-1-yl} oxy)ethyl] (2-sulfoethyl)carbamoyl}oxy)methyl]phenyl}-N⁵-carbamoyl-L-ornithinamide;
N-[6-(2,5-dioxo-2,5-dihydro-1H-pyrrol-1-yl)hexanoyl]-L-valyl-N-{4-[({[2-({3-[(4-{6-[5-(1,3-benzothiazol-2-ylcarbamoyl)quinolin-3-yl]-2-carboxypyridin-3-yl}-5-methyl-1H-pyrazol-1-yl)methyl]-5,7-dimethyltncyclo[3.3.1.1^{3,7}]dec-1-yl} oxy)ethyl] (2-carboxyethyl)carbamoyl}oxy)methyl]phenyl}-N⁵-carbamoyl-L-omithinamide;
N-[6-(2,5-dioxo-2,5-dihydro-1H-pyrrol-1-yl)hexanoyl]-L-valyl-N-{4-[({[2-({3-[(4-{6-[1-(1,3-benzothiazol-2-ylcarbamoyl)-5,6-dihydroimidazo[1,5-a]pyrazin-7(8H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)methyl]phenyl}-N⁵-carbamoyl-L-ornithinamide;
N-[6-(2,5-dioxo-2,5-dihydro-1H-pyrrol-1-yl)hexanoyl]-L-valyl-N-{4-[({[2-({3-[(4-{6-[7-(1,3 -benzothiazol-2-ylcarbamoyl)-1H-indol-2-yl]-2-carboxypyridin-3-yl} -5 -methyl- 1H-pyrazol-1-yl)methyl]-5,7-dimethyltncyclo[3.3.1.1^{3,7}]dec-1-yl} oxy)ethyl] (2-sulfoethyl)carbamoyl}oxy)methyl]phenyl}-N⁵-carbamoyl-L-ornithinamide;
N-[6-(2,5-dioxo-2,5-dihydro-1H-pyrrol-1-yl)hexanoyl]-L-valyl-N-[4-({[{3-[8-(1,3-benzothiazol-2-ylcarbamoyl)-2-(6-carboxy-5-{1-[(3,5-dimethyl-7-{2-[(2-sulfoethyl)amino]ethoxy}tricyclo[3.3.1.1^{3,7}]dec-1-yl)methyl]-5-methyl-1H-pyrazol-4-yl}pyridin-2-yl)-1,2,3,4-tetrahydroisoquinolin-6-yl]propyl}(methyl)carbamoyl]oxylmethyl)phenyl]-N⁵-carbamoyl-L-ornithinamide;
N-(6-{[(2,5-dioxo-2,5-dihydro-1H-pyrrol-1-yl)acetyl]amino}hexanoyl)-L-valyl-N-{4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)methyl]phenyl}-N⁵-carbamoyl-L-ornithinamide;
N-[6-(2,5-dioxo-2,5-dihydro-1H-pyrrol-1-yl)hexanoyl]-L-valyl-N-{4-[({[2-({3-[(4-{6-[8-(1,3 -benzothiazol-2-ylcarbamoyl)naphthalen-2-yl] -2-carboxypyridin-3-yl} -5 -methyl- 1H-pyrazol-1-yl)methyl] -5,7-dimethyltricyclo [3.3.1.1^{3,7}]dec-1-yl} oxy)ethyl] [3 -(beta-L-glucopyranuronosyloxy)propyl]carbamoyl}oxy)methyl]phenyl}-N⁵-carbamoyl-L-ornithinamide;
N-[6-(2,5-dioxo-2,5-dihydro-1H-pyrrol-1-yl)hexanoyl]-L-valyl-N-{4-[({[2-({3-[(4-{6-[4-(1,3-benzothiazol-2-ylcarbamoyl)isoquinolin-6-yl]-2-carboxypyridin-3-yl}-5-methyl-1H-pyrazol-1-yl)methyl]-5,7-dimethyltricyclo[3.3.1.1^{3,7}]dec-1-yl} oxy)ethyl] (2-sulfoethyl)carbamoyl}oxy)methyl]phenyl}-N⁵-carbamoyl-L-ornithinamide;
N-[6-(2,5-dioxo-2,5-dihydro-1H-pyrrol-1-yl)hexanoyl]-L-alpha-glutamyl-L-valyl-N-{4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)methyl]phenyl}-N⁵-carbamoyl-L-ornithinamide;
N-[(2,5-dioxo-2,5-dihydro-1H-pyrrol-1-yl)acetyl]-L-alpha-glutamyl-L-valyl-N-{4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)methyl]phenyl}-N⁵-carbamoyl-L-ornithinamide;
1-{[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl]({[4-({N-[6-(2,5-dioxo-2,5-dihydro-1H-pyrrol-1-yl)hexanoyl]-D-valyl-N⁵-carbamoyl-D-omithyl} amino)benzyl]oxy}carbonyl)amino}-1,2-dideoxy-D-arabino-hexitol;
N-[6-(2,5-dioxo-2,5-dihydro-1H-pyrrol-1-yl)hexanoyl]-L-valyl-N-{4-[({[2-({3-[(4-{6-[4-(1,3-benzothiazol-2-ylcarbamoyl)-2-oxidoisoquinolin-6-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](methyl)carbamoyl}oxy)methyl]phenyl}-N⁵-carbamoyl-L-ornithinamide;
N-({(3S,5S)-3-(2,5-dioxo-2,5-dihydro-1H-pyrrol-1-yl)-2-oxo-5-[(2-sulfoethoxy)methyl]pyrrolidin-1-yl}acetyl)-L-valyl-N-{4-[({[2-({3-[(4-{6-[8-(1,3-benzothiazol-2-ylcarbamoyl)naphthalen-2-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)methyl]phenyl}-N⁵-carbamoyl-L-ornithinamide;
N-{(2S)-2-(2,5-dioxo-2,5-dihydro-1H-pyrrol-1-yl)-3-[4-(2,5,8,11,14,17,20,23,26,29,32-undecaoxatetratriacontan-34-yloxy)phenyl]propanoyl}-L-valyl-N-{4-[({[2-({3-[(4-{6-[8-(1,3-benzothiazol-2-ylcarbamoyl)naphthalen-2-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)methyl]phenyl}-N⁵-carbamoyl-L-ornithinamide;
N-({(3S,5S)-3-(2,5-dioxo-2,5-dihydro-1H-pyrrol-1-yl)-2-oxo-5-[(2-sulfoethoxy)methyl]pyrrolidin-1-yl}acetyl)-L-valyl-N-{4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)methyl]phenyl}-N⁵-carbamoyl-L-ornithinamide;
N-{(2S)-2-(2,5-dioxo-2,5-dihydro-1H-pyrrol-1-yl)-3-[4-(2,5,8,11,14,17,20,23,26,29,32-undecaoxatetratriacontan-34-yloxy)phenyl]propanoyl}-L-valyl-N-{4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo [3.3.1.1^{3,7}]dec-1-yl} oxy)ethyl] (2-sulfoethyl)carbamoyl}oxy)methyl]phenyl}-N⁵-carbamoyl-L-ornithinamide;
(6S)-2,6-anhydro-6-(2-{2-[({[2-({3-[(4-{6-[8-(l,3-benzothiazol-2-ylcarbamoyl)-3,4-dihydroisoquinolin-2(1H)-yl]-2-carboxypyridin-3-yl} -5 -methyl- 1H-pyrazol-1-yl)methyl] -5,7-dimethyltricyclo[3.3.1.1^{3,7}]dec-1-yl}oxy)ethyl](2-sulfoethyl)carbamoyl}oxy)methyl]-5-({N-[(2,5-dioxo-2,5-dihydro-1H-pyrrol-1-yl)acetyl]-L-valyl-L-alanyl}ammo)phenyl}ethyl)-L-gulomc acid;
3-{[2-({3-[(4-{6-[8-(1,3-benzothiazol-2-ylcarbamoyl)naphthalen-2-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl]({[4-(4-{[6-(2,5-dioxo-2,5-dihydro-1H-pyrrol-1-yl)hexanoyl]amino}butyl)-2-(beta-D-glucopyranuronosyloxy)benzyl]oxy}carbonyl)amino}propyl beta-D-glucopyranosiduronic acid;
N-{ [(3 S,5S)-3-(2,5-dioxo-2,5-dihydro-1H-pyrrol-1-yl)-5-(methoxymethyl)-2-oxopyrrolidin-1-yl]acetyl}-L-valyl-N-{4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)methyl]phenyl}-N⁵-carbamoyl-L-ornithinamide;
(6S)-2,6-anhydro-6-(2-{2-[({[2-({3-[(4-{6-[8-(l,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)methyl]-5-({N-[6-(2,5-dioxo-2,5 -dihydro- 1H-pyrrol-1-yl)hexanoyl] -L-valyl-L-alanyl} amino)phenyl}ethyl)-L-gulonic acid;
2-[({[2-({3-[(4-{6-[8-(1,3-benzothiazol-2-ylcarbamoyl)-3,4-dihydroisoquinolm-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)methyl]-5-(4-{[3-(2,5-dioxo-2,5-dihydro-1H-pyrrol-1-yl)propanoyl]amino }butyl)phenyl beta-D-glucopyranosiduronic acid;
2-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)methyl]-5-[4-({(2S)-2-(2,5-dioxo-2,5-dihydro-1H-pyrrol-1-yl)-3-[4-(2,5,8,11,14,17,20,23,26,29,32-undecaoxatetratriacontan-34-yloxy)phenyl]propanoyl}amino)butyl]phenyl beta-D-glucopyranosiduronic acid;
(6S)-2,6-anhydro-6-(2-{2-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)methyl]-5-[(N-{(2S)-2-(2,5-dioxo-2,5-dihydro-1H-pyrrol-1-yl)-3-[4-(2,5,8,11,14,17,20,23,26,29,32-undecaoxatetratriacontan-34-yloxy)phenyl]propanoyl}-L-valyl-L-alanyl)amino]phenyl}ethyl)-L-gulonic acid;
6-(8-(benzo[d]thiazol-2-ylcarbamoyl)-3,4-dihydroisoquinolin-2(1H)-yl)-3-(1-((3-(2-((((2-(2-((2S,3R,4R,5S,6S)-6-carboxy-3,4,5-trihydroxytetrahydro-2H-pyran-2-yl)ethyl)-4-((S)-2-((S)-2-(2-((3S,5S)-3-(2,5-dioxo-2,5-dihydro-1H-pyrrol-1-yl)-2-oxo-5-((2-sulfoethoxy)methyl)pyrrolidin-1-yl)acetamido)-3-methylbutanamido)propanamido)benzyl)oxy)carbonyl)(2-sulfoethyl)amino)ethoxy)-5,7-dimethyladamantan-1-yl)methyl)-5-methyl-1H-pyrazol-4-yl)picolinic acid;
6-(8-(benzo[d]thiazol-2-ylcarbamoyl)-3,4-dihydroisoquinolin-2(1H)-yl)-3-(1-((3-(2-((((2-(((2S,3R,4S,5S,6S)-6-carboxy-3,4,5-trihydroxytetrahydro-2H-pyran-2-yl)oxy)-4-(4-(2-((3S,5S)-3-(2,5-dioxo-2,5-dihydro-1H-pyrrol-1-yl)-2-oxo-5-((2-sulfoethoxy)methyl)pyrrolidin-1-yl)acetamido)butyl)benzyl)oxy)carbonyl)(2-sulfoethyl)amino)ethoxy)-5,7-dimethyladamantan-1-yl)methyl)-5-methyl-1H-pyrazol-4-yl)picolinic acid;
2-[({[2-({3-[(4-{6-[8-(1,3-benzothiazol-2-ylcarbamoyl)-3,4-dihydroisoquinolm-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)methyl]-5-(4-{[(2,5-dioxo-2,5-dihydro-1H-pyrrol-1-yl)acetyl]amino}butyl)phenyl beta-D-glucopyranosiduronic acid;
2-[({[2-({3-[(4-{6-[8-(1,3-benzothiazol-2-ylcarbamoyl)naphthalen-2-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)methyl]-5-(4-{[(2,5-dioxo-2,5-dihydro-1H-pyrrol-1-yl)acetyl]amino}butyl)phenyl beta-D-glucopyranosiduronic acid;
2-[({[2-({3-[(4-{6-[8-(1,3-benzothiazol-2-ylcarbamoyl)naphthalen-2-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)methyl]-5-[4-({(2S)-2-(2,5-dioxo-2,5-dihydro-1H-pyrrol-1-yl)-3-[4-(2,5,8,11,14,17,20,23,26,29,32-undecaoxatetratriacontan-34-yloxy)phenyl]propanoyl}amino)butyl]phenyl beta-D-glucopyranosiduronic acid;
N-[(2,5-dioxo-2,5-dihydro-1H-pyrrol-1-yl)acetyl]-L-valyl-N-{4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)methyl]-3-(4-carboxybutyl)phenyl}-L-alaninamide;
2-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)methyl]-5 -(3 - { [(2,5-dioxo-2,5-dihydro-1H-pyrrol-1-yl)acetyl]amino}propyl)phenyl beta-D-glucopyranosiduronic acid;
6-[8-(1,3-benzothiazol-2-ylcarbamoyl)-3,4-dihydroisoquinolin-2(1H)-yl]-3-{1-[(3-{2-[({[2-{[(2S,3R,4S,5S,6S)-6-carboxy-3,4,5-trihydroxytetrahydro-2H-pyran-2-yl]oxy}-4-(4-{[(2,5-dioxo-2,5-dihydro-1H-pyrrol-1-yl)acetyl]amino}butyl)benzyl]oxy}carbonyl)(3 -{[1,3 -dihydroxy-2-(hydroxymethyl)propan-2-yl]amino}-3-oxopropyl)amino]ethoxy}-5,7-dimethyltricyclo[3.3.1.1^{3,7}]dec-1-yl)methyl]-5-methyl-1H-pyrazol-4-yl}pyridine-2-carboxylic acid;
6-(8-(benzo[d]thiazol-2-ylcarbamoyl)naphthalen-2-yl)-3-(1-((3-(2-((((2-(((2S,3R,4S,5S,6S)-6-carboxy-3,4,5-trihydroxytetrahydro-2H-pyran-2-yl)oxy)-4-(4-(2-((3S,5S)-3-(2,5-dioxo-2,5-dihydro-1H-pyrrol-1-yl)-2-oxo-5-((2-sulfoethoxy)methyl)pyrrolidin-1-yl)acetamido)butyl)benzyl)oxy)carbonyl)(2-sulfoethyl)amino)ethoxy)-5,7-dimethyladamantan-1-yl)methyl)-5-methyl-1H-pyrazol-4-yl)picolinic acid;
2-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl][3-hydroxy-2-(hydroxymethyl)propyl]carbamoyl}oxy)methyl]-5 -(3 - { [(2,5-dioxo-2,5-dihydro-1H-pyrrol-1-yl)acetyl]amino}propyl)phenyl beta-D-glucopyranosiduronic acid;
N-({(3S,5S)-3-(2,5-dioxo-2,5-dihydro-1H-pyrrol-1-yl)-2-oxo-5-[(2-sulfoethoxy)methyl]pyrrolidin-1-yl}acetyl)-L-valyl-N-{4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3 .3 .1.1^{3,7}]dec-1-yl} oxy)ethyl] (2-sulfoethyl)carbamoyl}oxy)methyl]-3-(2,5,8,11,14,17,20,23,26,29,32,35,38,41,44,47,50-heptadecaoxatripentacont-52-yn-53-yl)phenyl}-L-alaninamide;
N-({(3S,5S)-3-(2,5-dioxo-2,5-dihydro-1H-pyrrol-1-yl)-2-oxo-5-[(2-sulfoethoxy)methyl]pyrrolidin-1-yl}acetyl)-L-valyl-N-{4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)methyl]-3-(2,5,8,11,14,17,20,23,26,29,32,35,38,41,44,47,50-heptadecaoxatripentacontan-53-yl)phenyl}-L-alaninamide;
2-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl][(3S)-3,4-dihydroxybutyl]carbamoyl}oxy)methyl]-5-(3-{ [(2,5-dioxo-2,5-dihydro-1H-pyrrol-1-yl)acetyl]amino}propyl)phenyl beta-D-glucopyranosiduronic acid;
1{[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl]({[4-(4-{[(2,5-dioxo-2,5-dihydro-1H-pyrrol-1-yl)acetyl]amino}butyl)-2-(beta-D-glucopyranuronosyloxy)benzyl]oxylcarbonyl)aminol-1,2-dideoxy-D-arabino-hexitol;
1-{[2-({3-[(4-{6-[8-(1,3-benzothiazol-2-ylcarbamoyl)-3,4-dihydroisoqumolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl]({[4-(4-{[(2,5-dioxo-2,5-dihydro-1H-pyrrol-1-yl)acetyl]amino}butyl)-2-(beta-D-glucopyranuronosyloxy)benzyl]oxy}carbonyl)amino}-1,2-dideoxy-D-eiythro-pentitol;
N-[(2,5-dioxo-2,5-dihydro-1H-pyrrol-1-yl)acetyl]-L-valyl-N-{4-[({[2-({3-[(4-{6-[8-(1,3-benzothiazol-2-ylcarbamoyl)naphthalen-2-yl] -2-carboxypyridin-3-yl} -5 -methyl- 1H-pyrazol-1-yl)methyl]-5,7-dimethyltricyclo[3 .3 .1.1^{3,7}]dec-1-yl} oxy)ethyl] (2-sulfoethyl)carbamoyl}oxy)methyl]-3-[27-(2,5,8,11,14,17,20,23-octaoxahexacosan-26-yl)-2,5,8,11,14,17,20,23-octaoxa-27-azatriacontan-30-yl]phenyl}-L-alaninamide;
(6S)-2,6-anhydro-6-(2-{2-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)methyl]-5-({N-[(2S)-3-[3,4-bis(2,5,8,11,14,17,20,23,26,29,32-undecaoxatetratriacontan-34-yloxy)phenyl]-2-(2,5-dioxo-2,5-dihydro-1H-pyrrol-1-yl)propanoyl]-L-valyl-L-alanyl}amino)phenyl}ethyl)-L-gulonic acid;
N-[(2,5-dioxo-2,5-dihydro-1H-pyrrol-1-yl)acetyl]-N-(2,5,8,11,14,17,20,23,26,29,32-undecaoxatetratriacontan-34-yl)-beta-alanyl-L-valyl-N-{4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3 .3 .1.1^{3,7}]dec-1-yl} oxy)ethyl] (2-sulfoethyl)carbamoyl} oxy)methyl]-3-(2,5,8,11,14,17,20,23,26,29,32,35,38,41,44,47,50-heptadecaoxatripentacontan-53-yl)phenyl}-L-alaninamide;
N-[(2,5-dioxo-2,5-dihydro-1H-pyrrol-1-yl)acetyl]-N-(2,5,8,11,14,17,20,23,26,29,32-undecaoxatetratriacontan-34-yl)-beta-alanyl-L-valyl-N-{4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)methyl]phenyl}-N⁵-carbamoyl-L-ornithinamide;
N-[(2,5-dioxo-2,5-dihydro-1H-pyrrol-1-yl)acetyl]-L-valyl-N-{4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)methyl]-3-[27-(2,5,8,11,14,17,20,23-octaoxahexacosan-26-yl)-2,5,8,11,14,17,20,23-octaoxa-27-azatriacontan-30-yl]phenyl}-L-alaninamide;
N-{(3S)-3-(2,5-dioxo-2,5-dihydro-1H-pyrrol-1-yl)-3-[1-(2,5,8,11,14,17,20,23,26,29,32,35-dodecaoxaheptatriacontan-37-yl)-1H-1,2,3-triazol-4-yl]propanoyl}-L-valyl-N-{4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)methyl]phenyl}-N⁵-carbamoyl-L-ornithinamide;
N-{(3R)-3-(2,5-dioxo-2,5-dihydro-1H-pyrrol-1-yl)-3-[1-(2,5,8,11,14,17,20,23,26,29,32,35-dodecaoxaheptatriacontan-37-yl)-1H-1,2,3-triazol-4-yl]propanoyl}-L-valyl-N-{4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)methyl]phenyl}-N⁵-carbamoyl-L-ornithinamide;
6-[8-(1,3-benzothiazol-2-ylcarbamoyl)-3,4-dihydroisoquinolin-2(1H)-yl]-3-[1-({3-[2-({[(2-{2-[(2S,3R,4R,5S,6S)-6-carboxy-3,4,5-trihydroxytetrahydro-2H-pyran-2-yl]ethyl}-4-{[(2S)-2-{[(2S)-2-{[(2,5-dioxo-2,5-dihydro-1H-pyrrol-1-yl)acetyl]amino}-3-methylbutanoyl]amino}propanoyl]amino}benzyl)oxy]carbonyl}[(3R,4S,5R)-3,4,5,6-tetrahydroxyhexyl]amino)ethoxy]-5,7-dimethyltricyclo[3.3.1.1^{3,7}]dec-1-yl}methyl)-5-methyl-1H-pyrazol-4-yl]pyridine-2-carboxylic acid;
6-[8-(1,3-benzothiazol-2-ylcarbamoyl)-3,4-dihydroisoquinolin-2(1H)-yl]-3-[1-({3-[2-({[(2-{2-[(2S,3R,4R,5S,6S)-6-carboxy-3,4,5-trihydroxytetrahydro-2H-pyran-2-yl]ethyl}-4-{[(2S)-2-({(2S)-2-[({(3S,5S)-3-(2,5-dioxo-2,5-dihydro-1H-pyrrol-1-yl)-2-oxo-5-[(2-sulfoethoxy)methyl]pyrrolidin-1-yl}acetyl)amino]-3-methylbutanoyl}amino)propanoyl]amino}benzyl)oxy]carbonyl}[(3R,4S,5R)-3,4,5,6-tetrahydroxyhexyl]amino)ethoxy]-5,7-dimethyltricyclo[3.3.1.1^{3,7}]dec-1-yl}methyl)-5-methyl-1H-pyrazol-4-yl]pyridine-2-carboxylic acid;
(6S)-2,6-anhydro-6-(2-{2-[({[2-({3-[(4-{6-[8-(l,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)methyl]-5-({N-[(2,5-dioxo-2,5-dihydro-1H-pyrrol-1-yl)acetyl]-N-(2,5,8,11,14,17,20,23,26,29,32-undecaoxatetratriacontan-34-yl)-beta-alanyl-L-valyl-L-alanyl}amino)phenyl}ethyl)-L-gulonic acid;
(6S)-2,6-anhydro-6-(2-{2-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)methyl]-5-[(N-{2-(2,5-dioxo-2,5-dihydro-1H-pyrrol-1-yl)-3-[1-(2,5,8,11,14,17,20,23,26,29,32,35-dodecaoxaheptatriacontan-37-yl)-1H-1,2,3-triazol-4-yl]propanoyl}-L-valyl-L-alanyl)amino]phenyl}ethyl)-L-gulonic acid;
(6S)-2,6-anhydro-6-(2-{2-[({[2-({3-[(4-{6-[8-(l,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)methyl]-5-[(N-{(3S)-3-(2,5-dioxo-2,5-dihydro-1H-pyrrol-1-yl)-3-[1-(2,5,8,11,14,17,20,23,26,29,32,35-dodecaoxaheptatriacontan-37-yl)-1H-1,2,3-triazol-4-yl]propanoyl} -L-valyl-L-alanyl)amino]phenyl}ethyl)-L-gulonic acid;
(6S)-2,6-anhydro-6-(2-{2-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)methyl]-5-[(N-{(3R)-3-(2,5-dioxo-2,5-dihydro-1H-pyrrol-1-yl)-3-[1-(2,5,8,11,14,17,20,23,26,29,32,35-dodecaoxaheptatriacontan-37-yl)-1H-1,2,3-triazol-4-yl]propanoyl}-L-valyl-L-alanyl)amino]phenyl}ethyl)-L-gulonic acid;
(6S)-2,6-anhydro-6-(2-{2-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)methyl]-5-[(N-{(3S)-3-(2,5-dioxo-2,5-dihydro-1H-pyrrol-1-yl)-3-[1-(3-sulfopropyl)-1H-1,2,3-triazol-4-yl]propanoyl}-L-valyl-L-alanyl)amino]phenyl}ethyl)-L-gulonic acid;
(6S)-2,6-anhydro-6-(2-{2-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)methyl]-5-[(N-{(3R)-3-(2,5-dioxo-2,5-dihydro-1H-pyrrol-1-yl)-3-[1-(3-sulfopropyl)-1H-1,2,3-triazol-4-yl]propanoyl}-L-valyl-L-alanyl)amino]phenyl}ethyl)-L-gulonic acid;
(6S)-2,6-anhydro-6-(2-{2-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)methyl]-5-({N-[(2,5-dioxo-2,5-dihydro-1H-pyrrol-1-yl)acetyl]-N-[2-(2-sulfoethoxy)ethyl]-beta-alanyl-L-valyl-L-alanyl}amino)phenyl}ethyl)-L-gulonic acid;
6-{8-[(1,3-benzothiazol-2-yl)carbamoyl]-3,4-dihydroisoquinolm-2(1H)-yl}-3-[1-({3-[2-({[(2-{2-[(2S,3R,4R,5S,6S)-6-carboxy-3,4,5-trihydroxyoxan-2-yl]ethyl}-4-{[(2S)-2-{[(2S)-2-{[(2S)-2-(2,5-dioxo-2,5-dihydro-1H-pyrrol-1-yl)-3-{4-[(2,5,8,11,14,17,20,23,26,29,32-undecaoxatetratriacontan-34-yl)oxy]phenyl}propanoyl] amino }-3-methylbutanoyl]amino}propanoyl]amino}phenyl)methoxy]carbonyl}[(3R,4S,5R)-3,4,5,6-tetrahydroxyhexyl]amino)ethoxy]-5,7-dimethyltricyclo[3.3.1.1^{3,7}]decan-1-yl}methyl)-5-methyl-1H-pyrazol-4-yl]pyridine-2-carboxylic acid;
4-{[({2-[(3-{[4-(6-{8-[(1,3-benzothiazol-2-yl)carbamoyl]-3,4-dihydroisoquinolin-2(1H)-yl}-2-carboxypyridin-3-yl)-5-methyl-1H-pyrazol-1-yl]methyl}-5,7-dimethyltricyclo[3.3.1.1^{3,7}]decan-1-yl)oxy]ethyl}[(3S)-3,4-dihydroxybutyl]carbamoyl)oxy]methyl}-3-(2-{2-[2-(2,5-dioxo-2,5-dihydro-1H-pyrrol-1-yl)acetamido]ethoxy}ethoxy)phenyl beta-D-glucopyranosiduronic acid;
2,6-anhydro-8-[2-({[{2-[(3-{[4-(6-{8-[(1,3-benzothiazol-2-yl)carbamoyl]-3,4-dihydroisoquinolin-2(1H)-yl}-2-carboxypyridin-3-yl)-5-methyl-1H-pyrazol-1-yl]methyl}-5,7-dimethyltricyclo[3.3.1.1^{3,7}]decan-1-yl)oxy]ethyl}(2-sulfoethyl)carbamoyl]oxy}methyl)-5-{[(79S,82S)-74-[(2,5-dioxo-2,5-dihydro-1H-pyrrol-1-yl)acetyl]-82-methyl-77,80,83-trioxo-79-(propan-2-yl)-2,5,8,11,14,17,20,23,26,29,32,35,38,41,44,47,50,53,56,59,62,65,68,71-tetracosaoxa-74,78,81-triazatrioctacontan-83-yl]amino}phenyl]-7,8-dideoxy-L-glycero-L-gulo-octonic acid;
6-{8-[(1,3-benzothiazol-2-yl)carbamoyl]-3,4-dihydroisoquinolin-2(1H)-yl}-3-{1-[(3-{2-[{[(4-{[(2S,5S)-2-[3-(carbamoylamino)propyl]-10-[(2,5-dioxo-2,5-dihydro-1H-pyrrol-1-yl)acetyl]-4,7-dioxo-5-(propan-2-yl)-15-sulfo-13-oxa-3,6,10-triazapentadecanan-1-oyl]amino}phenyl)methoxy]carbonyl}(2-sulfoethyl)amino]ethoxy}-5,7-dimethyltricyclo[3.3.1.1^{3,7}]decan-1-yl)methyl]-5-methyl-1H-pyrazol-4-yl}pyridine-2-carboxylic acid;
6-(8-(benzo[d]thiazol-2-ylcarbamoyl)-3,4-dihydroisoquinolin-2(1H)-yl)-3-(1-((3-(2-((((2-(2-((2S,3R,4R,5S,6S)-6-carboxy-3,4,5-trihydroxytetrahydro-2H-pyran-2-yl)ethyl)-4-((S)-2-((S)-2-(2-((3S,5S)-3-(2,5-dioxo-2,5-dihydro-1H-pyrrol-1-yl)-2-oxo-5-((2-sulfoethoxy)methyl)pyrrolidin-1-yl)acetamido)-3-methylbutanamido)propanamido)benzyl)oxy)carbonyl)((S)-3,4-dihydroxybutyl)amino)ethoxy)-5,7-dimethyladamantan-1-yl)methyl)-5-methyl-1H-pyrazol-4-yl)picolinic acid;
2,6-anhydro-8-(2-{[({2-[(3-{[4-(6-{8-[(l,3-benzothiazol-2-yl)carbamoyl]-3,4-dihydroisoquinolin-2(1H)-yl}-2-carboxypyridin-3-yl)-5-methyl-1H-pyrazol-1-yl]methyl}-5,7-dimethyltricyclo[3.3.1.1^{3,7}]decan-1-yl)oxy]ethyl}[(3S)-3,4-dihydroxybutyl]carbamoyl)oxy]methyl}-5-{[(2S)-2-({(2S)-2-[2-(2,5-dioxo-2,5-dihydro-1H-pyrrol-1-yl)acetamido]-3-methylbutanoyl}amino)propanoyl]amino}phenyl)-7,8-dideoxy-L-glycero-L-gulo-octonic acid;
2-{[({2-[(3-{[4-(6-{8-[(1,3-benzothiazol-2-yl)carbamoyl]-3,4-dihydroisoquinolin-2(1H)-yl}-2-carboxypyridin-3-yl)-5-methyl-1H-pyrazol-1-yl]methyl}-5,7-dimethyltricyclo[3.3.1.1^{3,7}]decan-1-yl)oxy]ethyl}[(3S)-3,4-dihydroxybutyl]carbamoyl)oxy]methyl}-5-{4-[2-(2,5-dioxo-2,5-dihydro-1H-pyrrol-1-yl)acetamido]butyl}phenyl beta-D-glucopyranosiduronic acid;
6-{8-[(1,3-benzothiazol-2-yl)carbamoyl]-3,4-dihydroisoquinolin-2(1H)-yl}-3-{1-[(3-{2-[{[(4-{[(2S)-5-(carbamoylamino)-2-{[(2S)-2-{[6-(2,5-dioxo-2,5-dihydro-1H-pyrrol-1-yl)hexanoyl]amino}-3-methylbutanoyl]amino}pentanoyl]amino}phenyl)methoxy]carbonyl}(2-sulfoethyl)amino]acetamido}-5,7-dimethyltricyclo[3.3.1.1^{3,7}]decan-1-yl)methyl]-5-methyl-1H-pyrazol-4-yl}pyridine-2-carboxylic acid;
*N*-[6-(2,5-dioxo-2,5-dihydro-1*H*-pyrrol-1-yl)hexanoyl]-L-valyl-*N*-{4-[({[2-({3-[(4-{6-[8-(1,3-benzothiazol-2-ylcarbamoyl)-3,4-dihydroisoquinolin-2(1*H*)-yl]-2-carboxypyridin-3-yl}-5-methyl-1*H*-pyrazol-1-yl)methyl]-5,7-dimethyltricyclo[3.3.1.1^{3,7}]dec-1-yl}sulfanyl)ethyl](2-sulfoethyl)carbamoyl}oxy)methyl]phenyl}-*N*⁵-carbamoyl-L-ornithinamide;
*N-*[6-(2,5-dioxo-2,5-dihydro-1*H*-pyrrol-1-yl)hexanoyl]-L-valyl-*N*-[4-({[(3-{3-[(4-{6-[8-(1,3-benzothiazol-2-ylcarbamoyl)-3,4-dihydroisoquinolin-2(1*H*)-yl]-2-carboxypyridin-3-yl}-5-methyl-1*H-*pyrazol-1-yl)methyl]-5,7-dimethyltricyclo[3.3.1.1^{3,7}]dec-1-yl}propyl)(2-sulfoethyl)carbamoyl]oxy}methyl)phenyl]-*N*⁵-carbamoyl-L-ornithinamide;
2-[({[2-({3-[(4-{6-[8-(1,3-benzothiazol-2-ylcarbamoyl)-3,4-dihydroisoquinolin-2(1*H*)-yl]-2-carboxypyridin-3-yl}-5-methyl-1*H*-pyrazol-1-yl)methyl]-5,7-dimethyltricyclo[3.3.1.1^{3,7}]dec-1-yl}oxy)ethyl][(3*S*)-3,4-dihydroxybutyl]carbamoyl}oxy)methyl]-5-{4-[({(3*S*,5*S*)-3-(2,5-dioxo-2,5-dihydro-1*H*-pyrrol-1-yl)-2-oxo-5-[(2-sulfoethoxy)methyl]pyrrolidin-1-yl}acetyl)amino]butyl}phenyl beta-D-glucopyranosiduronic acid;
2,6-anhydro-8-[2-({[{2-[(3-{[4-(6-{8-[(1,3-benzothiazol-2-yl)carbamoyl]-3,4-dihydroisoquinolin-2(1*H*)-yl}-2-carboxypyridin-3-yl)-5-methyl-1*H*-pyrazol-1-yl]methyl}-5,7-dimethyltricyclo[3.3.1.1^{3,7}]decan-1-yl)oxy]ethyl}(2-sulfoethyl)carbamoyl]oxy }methyl)-5-{[*N-*({(3*R*,5*S*)-3-(2,5-dioxo-2,5-dihydro-1*H*-pyrrol-1-yl)-2-oxo-5-[(2-sulfoethoxy)methyl]pyrrolidin-1-yl}acetyl)-L-valyl-L-alanyl]amino}phenyl]-7,8-dideoxy-L-*glycero*-L-*gulo*-octonic acid;
2,6-anhydro-8-{2-({[{2-[(3-{[4-(6-{8-[(1,3-benzothiazol-2-yl)carbamoyl]-3,4-dihydroisoquinolin-2(1*H*)-yl}-2-carboxypyridin-3-yl)-5-methyl-1*H*-pyrazol-1-yl]methyl}-5,7-dimethyltricyclo[3.3.1.1^{3,7}]decan-1-yl)oxy]ethyl}(2-sulfoethyl)carbamoyl]oxy}methyl)-5-[(*N-*{[(3*R*,5*S*)-3-(2,5-dioxo-2,5-dihydro-1*H*-pyrrol-1-yl)-2-oxo-5-(41-oxo-2,5,8,11,14,17,20,23,26,29,32,35,38-tridecaoxa-42-azatritetracontan-43-yl)pyrrolidin-1-yl]acetyl}-L-valyl-L-alanyl)amino]phenyl}-7,8-dideoxy-L-*glycero*-L-*gulo*-octonic acid;
(6*S*)-2,6-anhydro-6-(2-{2-[({[2-({3-[(4-{6-[8-(1,3-benzothiazol-2-ylcarbamoyl)-3,4-dihydroisoquinolin-2(1*H*)-yl]-2-carboxypyridin-3-yl}-5-methyl-1*H*-pyrazol-1-yl)methyl]-5,7-dimethyltricyclo[3.3.1.1^{3,7}]dec-1-yl}oxy)ethyl][(3*S*)-3,4-dihydroxybutyl]carbamoyl}oxy)methyl]-5-({*N*[(2,5-dioxo-2,5-dihydro-1*H*-pyrrol-1-yl)acetyl]-*N*(2,5,8,11,14,17,20,23,26,29,32-undecaoxatetratriacontan-34-yl)-b-alanyl-L-valyl-L-alanyl}amino)phenyl}ethyl)-L-gulonic acid; and
(6*S*)-2,6-anhydro-6-(2-{2-[({[2-({3-[(4-{6-[8-(1,3-benzothiazol-2-ylcarbamoyl)-3,4-dihydroisoquinolin-2(1*H*)-yl]-2-carboxypyridin-3-yl}-5-methyl-1*H*-pyrazol-1-yl)methyl]-5,7-dimethyltricyclo[3.3.1.1^{3,7}]dec-1-yl}oxy)ethyl][(3*S*)-3,4-dihydroxybutyl]carbamoyl}oxy)methyl]-5-({*N*[(2,5-dioxo-2,5-dihydro-1*H*-pyrrol-1-yl)acetyl]-*N*[2-(2-sulfoethoxy)ethyl]-b-alanyl-L-valyl-L-alanyl}amino)phenyl}ethyl)-L-gulonic acid.

In certain embodiments, the ADC, or a pharmaceutically acceptable salt thereof,
D is the Bcl-xL inhibitor selected from the group consisting of the following compounds modified in that the hydrogen corresponding to the # position of structural formula (IIa), (IIb), (IIc), or (IId) is not present, forming a monoradical:
W2.01, W2.02, W2.03, W2.04, W2.05, W2.06, W2.07, W2.08, W2.09, W2.10, W2.11, W2.12, W2.13, W2.14, W2.15, W2.16, W2.17, W2.18, W2.19, W2.20, W2.21, W2.22, W2.23, W2.24, W2.25, W2.26, W2.27, W2.28, W2.29, W2.30, W2.31, W2.32, W2.33, W2.34, W2.35, W2.36, W2.37, W2.38, W2.39, W2.40, W2.41, W2.42, W2.43, W2.44, W2.45, W2.46, W2.47, W2.48, W2.49, W2.50, W2.51, W2.52, W2.53, W2.54, W2.55, W2.56, W2.57, W2.58, W2.59, W2.60, W2.61, W2.62, W2.63, W2.64, W2.65, W2.66, W2.67, W2.68, W2.69, W2.70, W2.71, W2.72, W2.73, W2.74, W2.75, W2.76, W2.77, W2.78, W2.79, W2.80, W2.81, W2.82, W2.83, W2.84, W2.85, W2.86, W2.87, W2.88, W2.89, W2.90, and W2.91, and a pharmaceutically acceptable salt thereof;
L is selected from the group consisting of linkers IVa.1-IVa.8, IVb.1-IVb.19, IVc.1-IVc.7, IVd.1-IVd.4, Va.1-Va.12, Vb.1-Vb.10, Vc.1-Vc.11, Vd.1-Vd.6, Ve.1-Ve.2, VIa.1, Vlc.1-Vlc.2, VId.1-VId.4, VIIa.1-VIIa.4, VIIb.1-VIIb.8, VIIc.1-VIIc.6, wherein each linker has reacted with the antibody, Ab, forming a covalent attachment;
LK is thioether; and
m is an integer ranging from 1 to 8.

In certain embodiments, the ADC, or a pharmaceutically acceptable salt thereof,
D is the Bcl-xL inhibitor selected from the group consisting of the following compounds modified in that the hydrogen corresponding to the # position of structural formula (IIa), (IIb), (IIc), or (IId) is not present, forming a monoradical:
6-[8-(1,3-benzothiazol-2-ylcarbamoyl)-3,4-dihydroisoquinolin-2(1H)-yl]-3-{1-[(3,5-dimethyl-7-{2-[(2-sulfoethyl)amino]ethoxy}tricyclo[3.3.1.1^{3,7}]dec-1-yl)methyl]-5-methyl-1H-pyrazol-4-yl}pyridine-2-carboxylic acid;
6-[8-(1,3-benzothiazol-2-ylcarbamoyl)-3,4-dihydroisoquinolin-2(1H)-yl]-3-{1-[(3-{2-[(2-carboxyethyl)amino]ethoxy}-5,7-dimethyltricyclo[3.3.1.1^{3,7}]dec-1-yl)methyl]-5-methyl-1H-pyrazol-4-yl}pyridine-2-carboxylic acid;
6-[8-(1,3-benzothiazol-2-ylcarbamoyl)naphthalen-2-yl]-3-{1-[(3,5-dimethyl-7-{2-[(2-sulfoethyl)amino]ethoxy}tricyclo[3.3.1.1^{3,7}]dec-1-yl)methyl]-5-methyl-1H-pyrazol-4-yl}pyridine-2-carboxylic acid;
1-{[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl} oxy)ethyl] amino }-1,2-dideoxy-D-arabino-hexitol;
6-[8-(1,3-benzothiazol-2-ylcarbamoyl)-3,4-dihydroisoquinolin-2(1H)-yl]-3-(1-{[3-(2-{[3-hydroxy-2-(hydroxymethyl)propyl]amino}ethoxy)-5,7-dimethyltricyclo[3.3.1.1^{3,7}]dec-1-yl]methyl}-5-methyl-1H-pyrazol-4-yl)pyridine-2-carboxylic acid;
6-[8-(1,3-benzothiazol-2-ylcarbamoyl)-3,4-dihydroisoquinolin-2(1*H*)-yl]-3-(1-{[3-(2-{[(3*S*)-3,4-dihydroxybutyl]amino}ethoxy)-5,7-dimethyltricyclo[3.3.1.1^{3,7}]dec-1-yl]methyl}-5-methyl-1*H-*pyrazol-4-yl)pyridine-2-carboxylic acid;
and pharmaceutically acceptable salts thereof;
L is selected from the group consisting of linkers IVb.2, IVc.5, IVc.6, IVc.7, IVd.4, Vb.9, Vc.11, VIIa.1, VIIa.3, VIIc.1, VIIc.4, and VIIc.5 in either closed or open forms and a pharmaceutically acceptable salt thereof;
LK is thioether; and
m is an integer ranging from 2 to 4.

To form an ADC, the maleimide ring of a synthon (for example, the synthons listed in Table 5) may react with an antibody Ab, forming a covalent attachment as either a succinimide (closed form) or succinamide (open form). Similarly, other functional groups, e.g. acetyl halide or vinyl sulfone may react with an antibody, Ab, forming a covalent attachment.

In certain embodiments, the ADC, or a pharmaceutically acceptable salt thereof, is selected from the group consisting of AbA-CZ, AbA-TX, AbA-TV, AbA-YY, AbA-AAA, AbA-AAD, AbB-CZ, AbB-TX, AbB-TV, AbB-YY, AbB-AAD, AbG-CZ, AbG-TX, AbG-TV, AbG-YY, AbG-AAA, AbG-AAD, AbK-CZ, AbK-TX, AbK-TV, AbK-YY, AbK-AAA, AbK-AAD, wherein CZ, TX, TV, YY, AAA, and AAD are synthons disclosed in Table 5, and where in the synthons are either in open or closed form.

In one embodiment, the ADC, or a pharmaceutically acceptable salt thereof, is 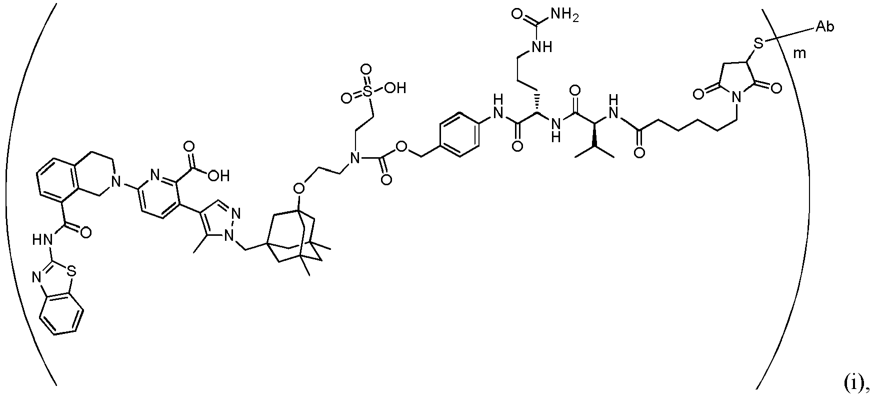 wherein m is 2, Ab is the hEGFR antibody, wherein the hEGFR antibody comprises a heavy chain CDR3 domain comprising the amino acid sequence set forth in SEQ ID NO: 12, a heavy chain CDR2 domain comprising the amino acid sequence set forth in SEQ ID NO: 11, and a heavy chain CDR1 domain comprising the amino acid sequence set forth in SEQ ID NO: 10; and a light chain CDR3 domain comprising the amino acid sequence set forth in SEQ ID NO: 8, a light chain CDR2 domain comprising the amino acid sequence set forth in SEQ ID NO: 7, and a light chain CDR1 domain comprising the amino acid sequence set forth in SEQ ID NO: 6; optionally wherein the hEGFR antibody comprises a heavy chain variable region comprising the amino acid sequence set forth in SEQ ID NO: 9, and a light chain variable region comprising the amino acid sequence set forth in SEQ ID NO: 5; optionally, wherein the hEGFR antibody comprises a heavy chain constant region comprising the amino acid sequence set forth in SEQ ID NO: 41 and/or a light chain constant region comprising the amino acid sequence set forth in SEQ ID NO: 43; optionally, wherein the hEGFR antibody comprises a heavy chain comprising the amino acid sequence set forth in SEQ ID NO: 15, and a light chain comprising the amino acid sequence set forth in SEQ ID NO: 13; optionally, wherein the hEGFR antibody comprises a heavy chain comprising the amino acid sequence set forth in SEQ ID NO: 102, and a light chain comprising the amino acid sequence set forth in SEQ ID NO: 13.

In one embodiment, the ADC, or a pharmaceutically acceptable salt thereof, is 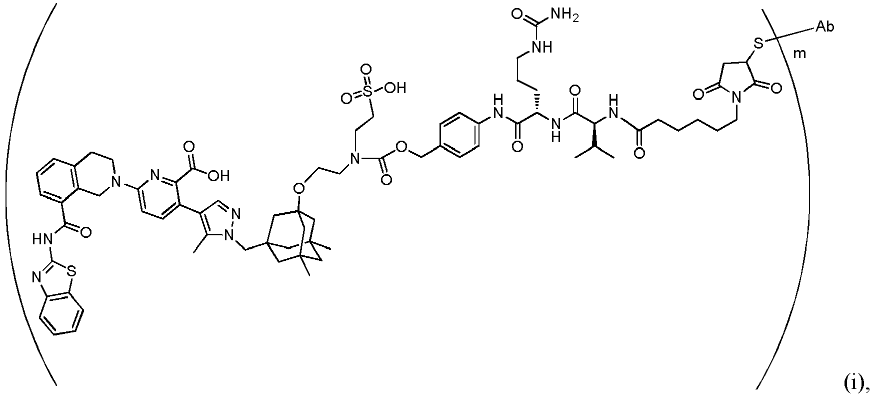 wherein m is 2, Ab is the hEGFR antibody, wherein the hEGFR antibody comprises a heavy chain CDR3 domain comprising the amino acid sequence set forth in SEQ ID NO: 18, a heavy chain CDR2 domain comprising the amino acid sequence set forth in SEQ ID NO: 17, and a heavy chain CDR1 domain comprising the amino acid sequence set forth in SEQ ID NO: 16; and a light chain CDR3 domain comprising the amino acid sequence set forth in SEQ ID NO: 25, a light chain CDR2 domain comprising the amino acid sequence set forth in SEQ ID NO: 24, and a light chain CDR1 domain comprising the amino acid sequence set forth in SEQ ID NO: 23; optionally, wherein the hEGFR antibody comprises a heavy chain variable region comprising the amino acid sequence set forth in SEQ ID NO: 72, and a light chain variable region comprising the amino acid sequence set forth in SEQ ID NO: 73; optionally, wherein the hEGFR antibody comprises a heavy chain constant region comprising the amino acid sequence set forth in SEQ ID NO: 41 and/or a light chain constant region comprising the amino acid sequence set forth in SEQ ID NO: 43; optionally, wherein the hEGFR antibody comprises a heavy chain comprising the amino acid sequence set forth in SEQ ID NO: 93, and a light chain comprising the amino acid sequence set forth in SEQ ID NO: 95; optionally, wherein the hEGFR antibody comprises a heavy chain comprising the amino acid sequence set forth in SEQ ID NO: 94, and a light chain comprising the amino acid sequence set forth in SEQ ID NO: 95.

In one embodiment, the ADC, or a pharmaceutically acceptable salt thereof, is 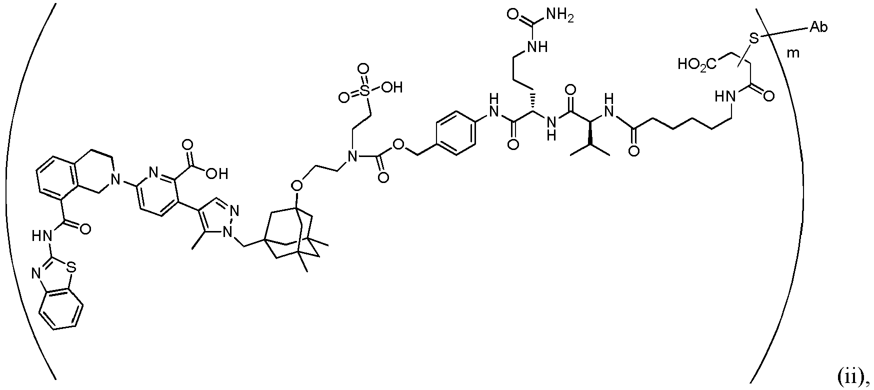 wherein m is 2, Ab is the hEGFR antibody, wherein the hEGFR antibody comprises a heavy chain CDR3 domain comprising the amino acid sequence set forth in SEQ ID NO: 12, a heavy chain CDR2 domain comprising the amino acid sequence set forth in SEQ ID NO: 11, and a heavy chain CDR1 domain comprising the amino acid sequence set forth in SEQ ID NO: 10; and a light chain CDR3 domain comprising the amino acid sequence set forth in SEQ ID NO: 8, a light chain CDR2 domain comprising the amino acid sequence set forth in SEQ ID NO: 7, and a light chain CDR1 domain comprising the amino acid sequence set forth in SEQ ID NO: 6; optionally wherein the hEGFR antibody comprises a heavy chain variable region comprising the amino acid sequence set forth in SEQ ID NO: 9, and a light chain variable region comprising the amino acid sequence set forth in SEQ ID NO: 5; optionally, wherein the hEGFR antibody comprises a heavy chain constant region comprising the amino acid sequence set forth in SEQ ID NO: 41 and/or a light chain constant region comprising the amino acid sequence set forth in SEQ ID NO: 43; optionally, wherein the hEGFR antibody comprises a heavy chain comprising the amino acid sequence set forth in SEQ ID NO: 15, and a light chain comprising the amino acid sequence set forth in SEQ ID NO: 13; optionally, wherein the hEGFR antibody comprises a heavy chain comprising the amino acid sequence set forth in SEQ ID NO: 102, and a light chain comprising the amino acid sequence set forth in SEQ ID NO: 13..

In one embodiment, the ADC, or a pharmaceutically acceptable salt thereof, is 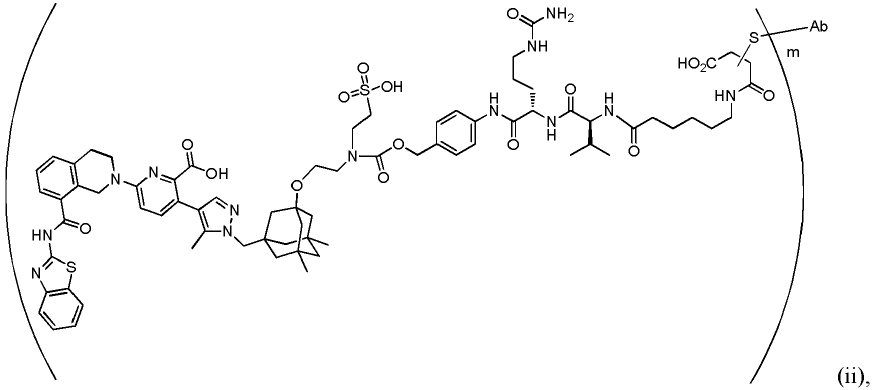 wherein m is 2, Ab is the hEGFR antibody, wherein the hEGFR antibody comprises a heavy chain CDR3 domain comprising the amino acid sequence set forth in SEQ ID NO: 18, a heavy chain CDR2 domain comprising the amino acid sequence set forth in SEQ ID NO: 17, and a heavy chain CDR1 domain comprising the amino acid sequence set forth in SEQ ID NO: 16; and a light chain CDR3 domain comprising the amino acid sequence set forth in SEQ ID NO: 25, a light chain CDR2 domain comprising the amino acid sequence set forth in SEQ ID NO: 24, and a light chain CDR1 domain comprising the amino acid sequence set forth in SEQ ID NO: 23; optionally, wherein the hEGFR antibody comprises a heavy chain variable region comprising the amino acid sequence set forth in SEQ ID NO: 72, and a light chain variable region comprising the amino acid sequence set forth in SEQ ID NO: 73; optionally, wherein the hEGFR antibody comprises a heavy chain constant region comprising the amino acid sequence set forth in SEQ ID NO: 41 and/or a light chain constant region comprising the amino acid sequence set forth in SEQ ID NO: 43; optionally, wherein the hEGFR antibody comprises a heavy chain comprising the amino acid sequence set forth in SEQ ID NO: 93, and a light chain comprising the amino acid sequence set forth in SEQ ID NO: 95; optionally, wherein the hEGFR antibody comprises a heavy chain comprising the amino acid sequence set forth in SEQ ID NO: 94, and a light chain comprising the amino acid sequence set forth in SEQ ID NO: 95.

In one embodiment, the ADC, or a pharmaceutically acceptable salt thereof, is 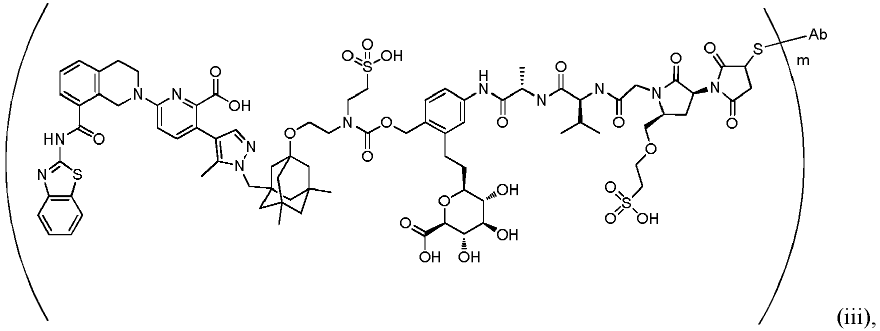 wherein m is 2, Ab is the hEGFR antibody, wherein the hEGFR antibody comprises a heavy chain CDR3 domain comprising the amino acid sequence set forth in SEQ ID NO: 12, a heavy chain CDR2 domain comprising the amino acid sequence set forth in SEQ ID NO: 11, and a heavy chain CDR1 domain comprising the amino acid sequence set forth in SEQ ID NO: 10; and a light chain CDR3 domain comprising the amino acid sequence set forth in SEQ ID NO: 8, a light chain CDR2 domain comprising the amino acid sequence set forth in SEQ ID NO: 7, and a light chain CDR1 domain comprising the amino acid sequence set forth in SEQ ID NO: 6; optionally wherein the hEGFR antibody comprises a heavy chain variable region comprising the amino acid sequence set forth in SEQ ID NO: 9, and a light chain variable region comprising the amino acid sequence set forth in SEQ ID NO: 5; optionally, wherein the hEGFR antibody comprises a heavy chain constant region comprising the amino acid sequence set forth in SEQ ID NO: 41 and/or a light chain constant region comprising the amino acid sequence set forth in SEQ ID NO: 43; optionally, wherein the hEGFR antibody comprises a heavy chain comprising the amino acid sequence set forth in SEQ ID NO: 15, and a light chain comprising the amino acid sequence set forth in SEQ ID NO: 13; optionally, wherein the hEGFR antibody comprises a heavy chain comprising the amino acid sequence set forth in SEQ ID NO: 102, and a light chain comprising the amino acid sequence set forth in SEQ ID NO: 13.

In one embodiment, the ADC, or a pharmaceutically acceptable salt thereof, is 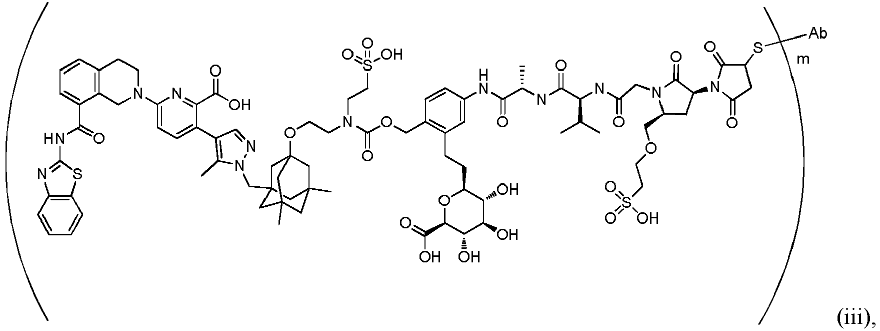 wherein m is 2, Ab is the hEGFR antibody, wherein the hEGFR antibody comprises a heavy chain CDR3 domain comprising the amino acid sequence set forth in SEQ ID NO: 18, a heavy chain CDR2 domain comprising the amino acid sequence set forth in SEQ ID NO: 17, and a heavy chain CDR1 domain comprising the amino acid sequence set forth in SEQ ID NO: 16; and a light chain CDR3 domain comprising the amino acid sequence set forth in SEQ ID NO: 25, a light chain CDR2 domain comprising the amino acid sequence set forth in SEQ ID NO: 24, and a light chain CDR1 domain comprising the amino acid sequence set forth in SEQ ID NO: 23; optionally, wherein the hEGFR antibody comprises a heavy chain variable region comprising the amino acid sequence set forth in SEQ ID NO: 72, and a light chain variable region comprising the amino acid sequence set forth in SEQ ID NO: 73; optionally, wherein the hEGFR antibody comprises a heavy chain constant region comprising the amino acid sequence set forth in SEQ ID NO: 41 and/or a light chain constant region comprising the amino acid sequence set forth in SEQ ID NO: 43; optionally, wherein the hEGFR antibody comprises a heavy chain comprising the amino acid sequence set forth in SEQ ID NO: 93, and a light chain comprising the amino acid sequence set forth in SEQ ID NO: 95; optionally, wherein the hEGFR antibody comprises a heavy chain comprising the amino acid sequence set forth in SEQ ID NO: 94, and a light chain comprising the amino acid sequence set forth in SEQ ID NO: 95.

In one embodiment, the ADC, or a pharmaceutically acceptable salt thereof, is 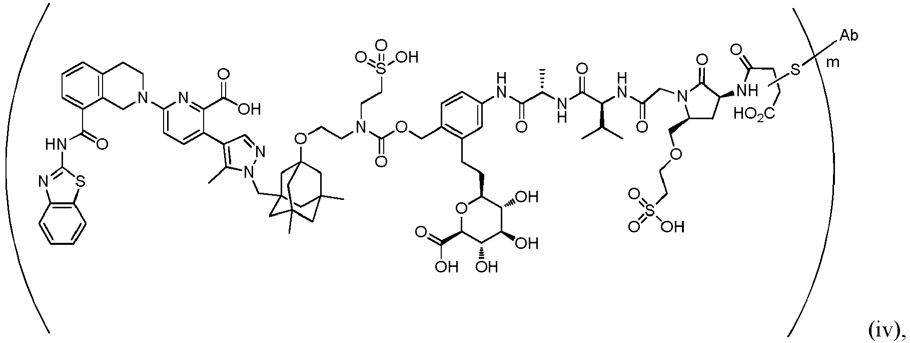 wherein m is 2, Ab is the hEGFR antibody, wherein the hEGFR antibody comprises a heavy chain CDR3 domain comprising the amino acid sequence set forth in SEQ ID NO: 12, a heavy chain CDR2 domain comprising the amino acid sequence set forth in SEQ ID NO: 11, and a heavy chain CDR1 domain comprising the amino acid sequence set forth in SEQ ID NO: 10; and a light chain CDR3 domain comprising the amino acid sequence set forth in SEQ ID NO: 8, a light chain CDR2 domain comprising the amino acid sequence set forth in SEQ ID NO: 7, and a light chain CDR1 domain comprising the amino acid sequence set forth in SEQ ID NO: 6; optionally wherein the hEGFR antibody comprises a heavy chain variable region comprising the amino acid sequence set forth in SEQ ID NO: 9, and a light chain variable region comprising the amino acid sequence set forth in SEQ ID NO: 5; optionally, wherein the hEGFR antibody comprises a heavy chain constant region comprising the amino acid sequence set forth in SEQ ID NO: 41 and/or a light chain constant region comprising the amino acid sequence set forth in SEQ ID NO: 43; optionally, wherein the hEGFR antibody comprises a heavy chain comprising the amino acid sequence set forth in SEQ ID NO: 15, and a light chain comprising the amino acid sequence set forth in SEQ ID NO: 13; optionally, wherein the hEGFR antibody comprises a heavy chain comprising the amino acid sequence set forth in SEQ ID NO: 102, and a light chain comprising the amino acid sequence set forth in SEQ ID NO: 13.

In one embodiment, the ADC, or a pharmaceutically acceptable salt thereof, is 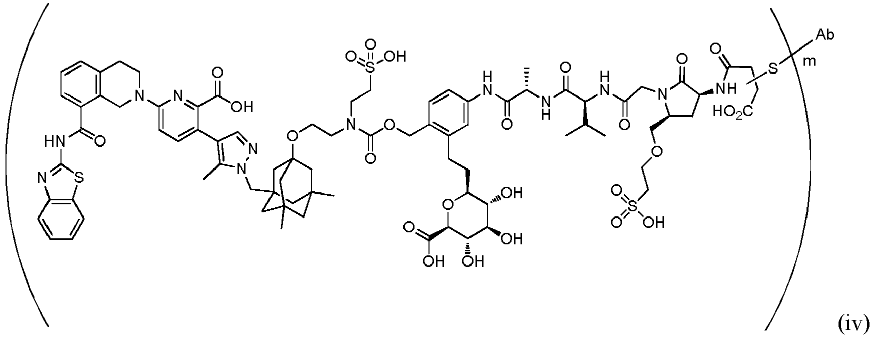 , wherein m is 2, Ab is the hEGFR antibody, wherein the hEGFR antibody comprises a heavy chain CDR3 domain comprising the amino acid sequence set forth in SEQ ID NO: 18, a heavy chain CDR2 domain comprising the amino acid sequence set forth in SEQ ID NO: 17, and a heavy chain CDR1 domain comprising the amino acid sequence set forth in SEQ ID NO: 16; and a light chain CDR3 domain comprising the amino acid sequence set forth in SEQ ID NO: 25, a light chain CDR2 domain comprising the amino acid sequence set forth in SEQ ID NO: 24, and a light chain CDR1 domain comprising the amino acid sequence set forth in SEQ ID NO: 23; optionally, wherein the hEGFR antibody comprises a heavy chain variable region comprising the amino acid sequence set forth in SEQ ID NO: 72, and a light chain variable region comprising the amino acid sequence set forth in SEQ ID NO: 73; optionally, wherein the hEGFR antibody comprises a heavy chain constant region comprising the amino acid sequence set forth in SEQ ID NO: 41 and/or a light chain constant region comprising the amino acid sequence set forth in SEQ ID NO: 43; optionally, wherein the hEGFR antibody comprises a heavy chain comprising the amino acid sequence set forth in SEQ ID NO: 93, and a light chain comprising the amino acid sequence set forth in SEQ ID NO: 95; optionally, wherein the hEGFR antibody comprises a heavy chain comprising the amino acid sequence set forth in SEQ ID NO: 94, and a light chain comprising the amino acid sequence set forth in SEQ ID NO: 95..

In one embodiment, the ADC, or a pharmaceutically acceptable salt thereof, is 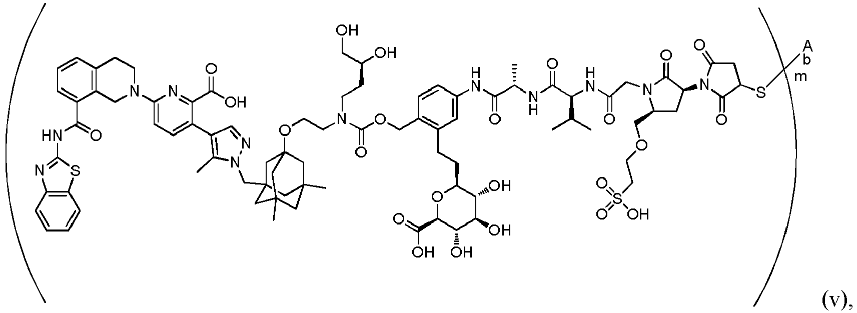 wherein m is 2, Ab is the hEGFR antibody, wherein the hEGFR antibody comprises a heavy chain CDR3 domain comprising the amino acid sequence set forth in SEQ ID NO: 12, a heavy chain CDR2 domain comprising the amino acid sequence set forth in SEQ ID NO: 11, and a heavy chain CDR1 domain comprising the amino acid sequence set forth in SEQ ID NO: 10; and a light chain CDR3 domain comprising the amino acid sequence set forth in SEQ ID NO: 8, a light chain CDR2 domain comprising the amino acid sequence set forth in SEQ ID NO: 7, and a light chain CDR1 domain comprising the amino acid sequence set forth in SEQ ID NO: 6; optionally wherein the hEGFR antibody comprises a heavy chain variable region comprising the amino acid sequence set forth in SEQ ID NO: 9, and a light chain variable region comprising the amino acid sequence set forth in SEQ ID NO: 5; optionally, wherein the hEGFR antibody comprises a heavy chain constant region comprising the amino acid sequence set forth in SEQ ID NO: 41 and/or a light chain constant region comprising the amino acid sequence set forth in SEQ ID NO: 43; optionally, wherein the hEGFR antibody comprises a heavy chain comprising the amino acid sequence set forth in SEQ ID NO: 15, and a light chain comprising the amino acid sequence set forth in SEQ ID NO: 13; optionally, wherein the hEGFR antibody comprises a heavy chain comprising the amino acid sequence set forth in SEQ ID NO: 102, and a light chain comprising the amino acid sequence set forth in SEQ ID NO: 13.

In one embodiment, the ADC, or a pharmaceutically acceptable salt thereof, is 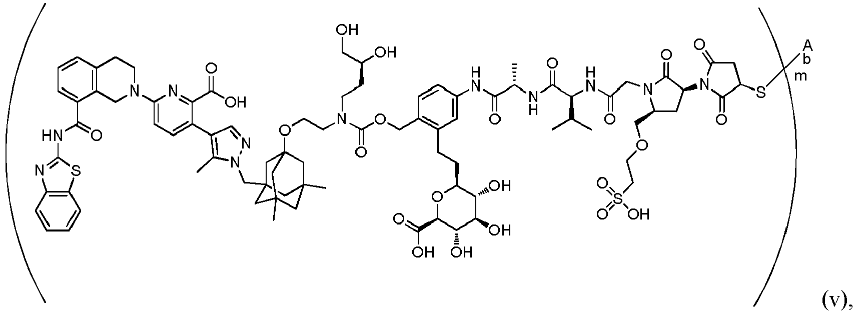 wherein m is 2, Ab is the hEGFR antibody, wherein the hEGFR antibody comprises a heavy chain CDR3 domain comprising the amino acid sequence set forth in SEQ ID NO: 18, a heavy chain CDR2 domain comprising the amino acid sequence set forth in SEQ ID NO: 17, and a heavy chain CDR1 domain comprising the amino acid sequence set forth in SEQ ID NO: 16; and a light chain CDR3 domain comprising the amino acid sequence set forth in SEQ ID NO: 25, a light chain CDR2 domain comprising the amino acid sequence set forth in SEQ ID NO: 24, and a light chain CDR1 domain comprising the amino acid sequence set forth in SEQ ID NO: 23; optionally, wherein the hEGFR antibody comprises a heavy chain variable region comprising the amino acid sequence set forth in SEQ ID NO: 72, and a light chain variable region comprising the amino acid sequence set forth in SEQ ID NO: 73; optionally, wherein the hEGFR antibody comprises a heavy chain constant region comprising the amino acid sequence set forth in SEQ ID NO: 41 and/or a light chain constant region comprising the amino acid sequence set forth in SEQ ID NO: 43; optionally, wherein the hEGFR antibody comprises a heavy chain comprising the amino acid sequence set forth in SEQ ID NO: 93, and a light chain comprising the amino acid sequence set forth in SEQ ID NO: 95; optionally, wherein the hEGFR antibody comprises a heavy chain comprising the amino acid sequence set forth in SEQ ID NO: 94, and a light chain comprising the amino acid sequence set forth in SEQ ID NO: 95.

In one embodiment, the ADC, or a pharmaceutically acceptable salt thereof, is 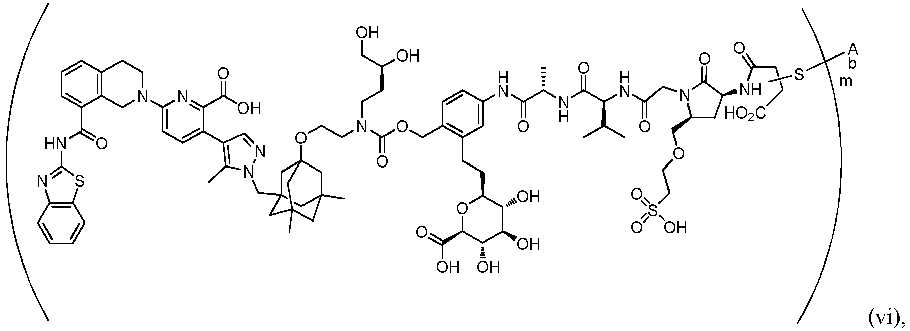 wherein m is 2, Ab is the hEGFR antibody, wherein the hEGFR antibody comprises a heavy chain CDR3 domain comprising the amino acid sequence set forth in SEQ ID NO: 12, a heavy chain CDR2 domain comprising the amino acid sequence set forth in SEQ ID NO: 11, and a heavy chain CDR1 domain comprising the amino acid sequence set forth in SEQ ID NO: 10; and a light chain CDR3 domain comprising the amino acid sequence set forth in SEQ ID NO: 8, a light chain CDR2 domain comprising the amino acid sequence set forth in SEQ ID NO: 7, and a light chain CDR1 domain comprising the amino acid sequence set forth in SEQ ID NO: 6; optionally wherein the hEGFR antibody comprises a heavy chain variable region comprising the amino acid sequence set forth in SEQ ID NO: 9, and a light chain variable region comprising the amino acid sequence set forth in SEQ ID NO: 5; optionally, wherein the hEGFR antibody comprises a heavy chain constant region comprising the amino acid sequence set forth in SEQ ID NO: 41 and/or a light chain constant region comprising the amino acid sequence set forth in SEQ ID NO: 43; optionally, wherein the hEGFR antibody comprises a heavy chain comprising the amino acid sequence set forth in SEQ ID NO: 15, and a light chain comprising the amino acid sequence set forth in SEQ ID NO: 13; optionally, wherein the hEGFR antibody comprises a heavy chain comprising the amino acid sequence set forth in SEQ ID NO: 102, and a light chain comprising the amino acid sequence set forth in SEQ ID NO: 13.

In one embodiment, the ADC, or a pharmaceutically acceptable salt thereof, is 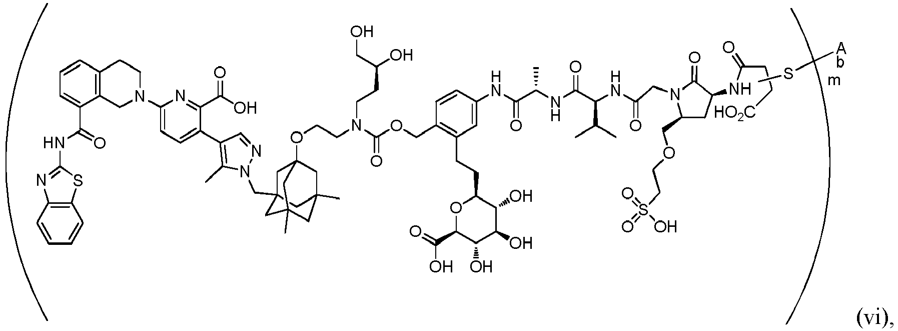 wherein m is 2, Ab is the hEGFR antibody, wherein the hEGFR antibody comprises a heavy chain CDR3 domain comprising the amino acid sequence set forth in SEQ ID NO: 18, a heavy chain CDR2 domain comprising the amino acid sequence set forth in SEQ ID NO: 17, and a heavy chain CDR1 domain comprising the amino acid sequence set forth in SEQ ID NO: 16; and a light chain CDR3 domain comprising the amino acid sequence set forth in SEQ ID NO: 25, a light chain CDR2 domain comprising the amino acid sequence set forth in SEQ ID NO: 24, and a light chain CDR1 domain comprising the amino acid sequence set forth in SEQ ID NO: 23; optionally, wherein the hEGFR antibody comprises a heavy chain variable region comprising the amino acid sequence set forth in SEQ ID NO: 72, and a light chain variable region comprising the amino acid sequence set forth in SEQ ID NO: 73; optionally, wherein the hEGFR antibody comprises a heavy chain constant region comprising the amino acid sequence set forth in SEQ ID NO: 41 and/or a light chain constant region comprising the amino acid sequence set forth in SEQ ID NO: 43; optionally, wherein the hEGFR antibody comprises a heavy chain comprising the amino acid sequence set forth in SEQ ID NO: 93, and a light chain comprising the amino acid sequence set forth in SEQ ID NO: 95; optionally, wherein the hEGFR antibody comprises a heavy chain comprising the amino acid sequence set forth in SEQ ID NO: 94, and a light chain comprising the amino acid sequence set forth in SEQ ID NO: 95.

Bcl-xL inhibitors, including warheads and synthons, and methods of making the same, are described in US 2016-0339117 (AbbVie Inc.).

### 5. Methods of Synthesis of ADCs

The Bcl-xL inhibitors and synthons described herein may be synthesized using standard, known techniques of organic chemistry. General schemes for synthesizing Bcl-xL inhibitors and synthons that may be used as-is or modified to synthesize the full scope of Bcl-xL inhibitors and synthons described herein are provided below. Specific methods for synthesizing exemplary Bcl-xL inhibitors and synthons that may be useful for guidance are provided in the Examples section. ADCs may likewise be prepared by standard methods, such as methods analogous to those described in Hamblett et al., 2004, "Effects of Drug Loading on the Antitumor Activity of a Monoclonal Antibody Drug Conjugate", Clin. Cancer Res. 10:7063-7070; Doronina et al., 2003, "Development of potent and highly efficacious monoclonal antibody auristatin conjugates for cancer therapy," Nat. Biotechnol. 21(7):778-784; and Francisco et al., 2003, Blood 102:1458-1465. For example, ADCs with four drugs per antibody may be prepared by partial reduction of the antibody with an excess of a reducing reagent such as DTT or TCEP at 37 °C for 30 min, then the buffer exchanged by elution through SEPHADEX^{®} G-25 resin with 1 mM DTPA in DPBS. The eluent is diluted with further DPBS, and the thiol concentration of the antibody may be measured using 5,5'-dithiobis(2-nitrobenzoic acid) [Ellman's reagent]. An excess, for example 5-fold, of a linker-drug synthon is added at 4 °C for 1 hr, and the conjugation reaction may be quenched by addition of a substantial excess, for example 20-fold, of cysteine. The resulting ADC mixture may be purified on SEPHADEX G-25 equilibrated in PBS to remove unreacted synthons, desalted if desired, and purified by size-exclusion chromatography. The resulting ADC may then be then sterile filtered, for example, through a 0.2 µm filter, and lyophilized if desired for storage. In certain embodiments, all of the interchain cysteine disulfide bonds are replaced by linker-drug conjugates. One embodiment pertains to a method of making an ADC, comprising contacting a synthon described herein with an antibody under conditions in which the synthon covalently links to the antibody.

Examples of the foregoing Bcl-xL inhibtors, linkers, and synthons thereof, as well as methods of making the same, can be found in US Patent Publication No. US 2016/0339117.

Specific methods for synthesizing exemplary ADCs that may be used to synthesize the full range of ADCs described herein are provided in the Examples section.

### 5.1. General Methods for Synthesizing Bcl-xL Inhibitors

In the schemes below, the various substituents Ar¹, Ar², Z¹, R⁴, R¹⁰, R^{11a} and R^{11b} are as defined in the Detailed Description section.

### 5.1.1. Synthesis of Compound (6)

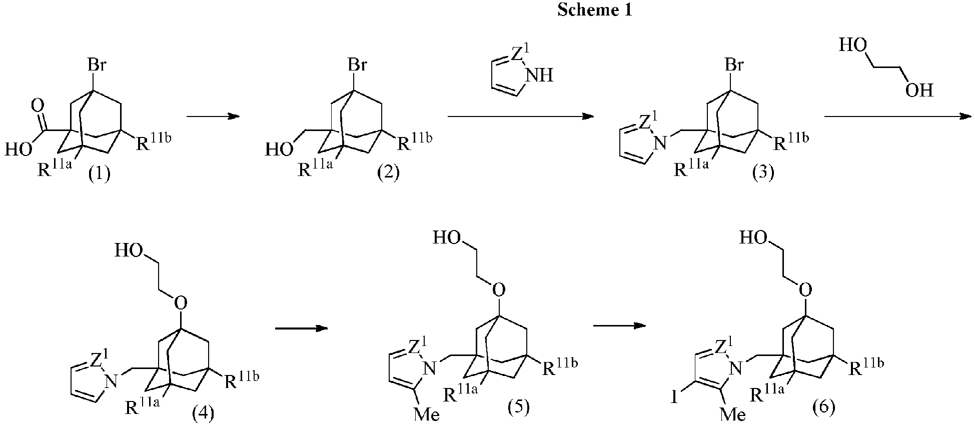

The synthesis of an intermediate (6) is described in Scheme 1. Compound (1) can be treated with BH₃·THF to provide compound (2). The reaction is typically performed at ambient temperature in a solvent, such as, but not limited to, tetrahydrofuran. Compound (3) can be prepared by treating compound (2) with 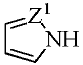 in the presence of cyanomethylenetributylphosphorane. The reaction is typically performed at an elevated temperature in a solvent such as, but not limited to, toluene. Compound (3) can be treated with ethane-1,2-diol in the presence of a base such as, but not limited to, triethylamine, to provide compound (4). The reaction is typically performed at an elevated temperature, and the reaction may be performed under microwave conditions. Compound (4) can be treated with a strong base, such as, but not limited to, n-butyllithium, followed by the addition of iodomethane, to provide compound (5). The addition and reaction is typically performed in a solvent such as, but not limited to, tetrahydrofuran, at a reduced temperature before warming up to ambient temperature for work up. Compound (5) can be treated with N-iodosuccinimide to provide compound (6). The reaction is typically performed at ambient temperature is a solvent such as, but not limited to, N,N-dimethylformamide.

### 5.1.2. Synthesis of Compound (12)

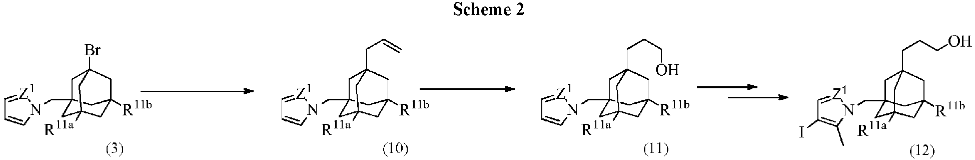

The synthesis of intermediate (12) is described in Scheme 2. Compound (3) can be treated with tri-n-butyl-allylstannane in the presence of ZnCl₂·Et₂O or N, N'-azoisobutyronitrile (AIBN) to provide compound (10) (Yamamoto et al., 1998, Heterocycles 47:765-780). The reaction is typically performed at -78 °C in a solvent, such as, but not limited to dichloromethane. Compound (10) can be treated under standard conditions known in the art for hydroboration/oxidation to provide compound (11). For example, treatment of compound (10) with a reagent such as BH₃·THF in a solvent such as, but not limited to, tetrahydrofuran followed by treatment of the intermediate alkylborane adduct with an oxidant such as, but not limited to, hydrogen peroxide in the presence of a base such as, but not limited to, sodium hydroxide would provide compound (11) (Brown et al., 1968, J. Am. Chem. Soc. 86:397). Typically the addition of BH₃·THF is performed at low temperature before warming to ambient temperature, which is followed by the addition of hydrogen peroxide and sodium hydroxide to generate the alcohol product. Compound (12) can be generated according to Scheme 1, as previously described for compound (6).

### 5.1.3. Synthesis of Compound (15)

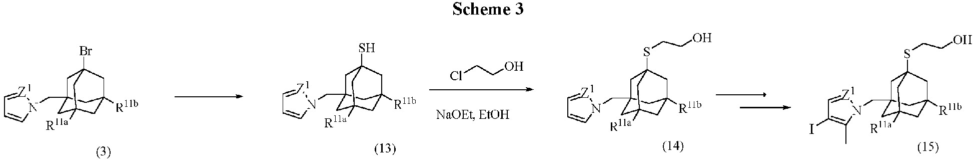

The synthesis of intermediate (15) is described in Scheme 3. Compound (3) can be reacted with thiourea in a solvent mixture of acetic acid and 48% aqueous HBr solution at 100 °C to yield an intermediate that can be subsequently treated with sodium hydroxide in a solvent mixture such as, but not limited to, 20% v/v ethanol in water to provide compound (13). Compound (13) can be reacted with 2-chloroethanol in the presence of a base such as, but not limited to, sodium ethoxide to provide compound (14). The reaction is typically performed at ambient or elevated temperatures in a solvent such as, but not limited to, ethanol. Compound (15) can be generated according to Scheme 1, as previously described for compound (6).

### 5.1.4. Synthesis of Compound (22)

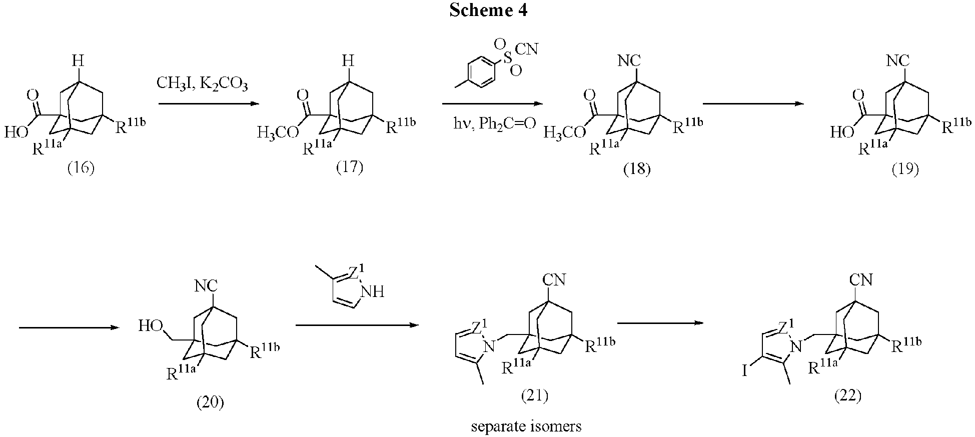

The synthesis of compound (22) is described in Scheme 4. Compound (16) can be reacted with iodomethane in the presence of a base such as, but not limited to, potassium carbonate to provide compound (17). The reaction is typically conducted at ambient or elevated temperature in a solvent such as, but not limited to, acetone or N,N-dimethylformamide. Compound (17) can be reacted under photochemical conditions with tosyl cyanide in the presence of benzophenone to provide compound (18) (*see* Kamijo et al., 2011, Org. Lett., 13:5928-5931). The reaction is typically run at ambient temperature in a solvent such as, but not limited to, acetonitrile or benzene using a Riko 100W medium pressure mercury lamp as the light source. Compound (18) can be reacted with lithium hydroxide in a solvent system such as, but not limited to, mixtures of water and tetrahydrofuran or water and methanol to provide compound (19). Compound (19) can be treated with BH₃·THF to provide compound (20). The reaction is typically performed at ambient temperature in a solvent, such as, but not limited to, tetrahydrofuran. Compound (21) can be prepared by treating compound (20) with 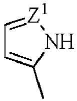 in the presence of cyanomethylenetributylphosphorane. The reaction is typically performed at an elevated temperature in a solvent such as, but not limited to, toluene. Compound (21) can be treated with N-iodosuccinimide to provide compound (22). The reaction is typically performed at ambient temperature is a solvent such as, but not limited to, N,N-dimethylformamide.

### 5.1.5. Synthesis of Compound (24)

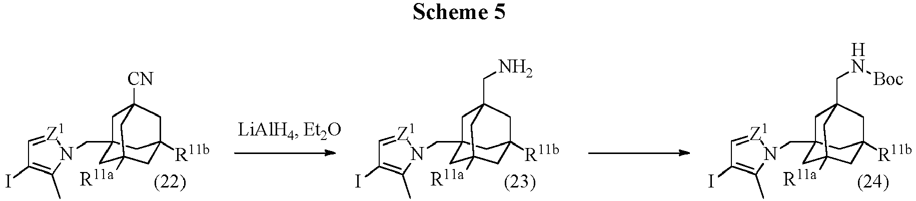

The synthesis of pyrazole compound (24), is described in Scheme 5. Compound (22) can be treated with a reducing agent such as, but not limited to, lithium aluminum hydride in a solvent such as, but not limited to, diethyl ether or tetrahydrofuran to provide compound (23). Typically the reaction is performed at 0 °C before warming to ambient or elevated temperature. Compound (23) can be reacted with di-tert-butyl dicarbonate under standard conditions described herein or in the literature to provide compound (24).

### 5.1.6. Synthesis of Compound (24a)

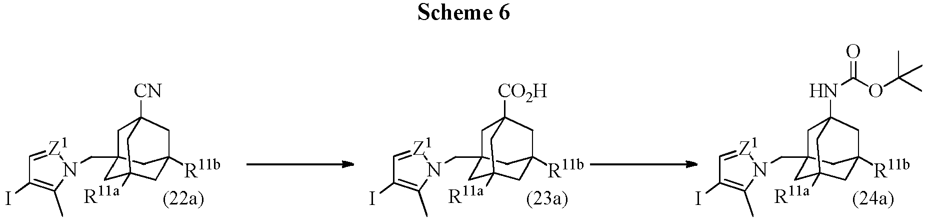

The synthesis of intermediate (24a) is described in Scheme 6. Compound (22a) can be hydrolyzed using conditions described in the literature to provide compound (23a). Typically the reaction is run in the presence of potassium hydroxide in a solvent such as, but not limited to, ethylene glycol at elevated temperatures (*see* Roberts et al., 1994, J. Org. Chem. 59:6464-6469; Yang et al, 2013, Org. Lett., 15:690-693). Compound (24a) can be made from compound (23a) by Curtius rearrangement using conditions described in the literature. For example, compound (23a) can be reacted with sodium azide in the presence of tetrabutylammonium bromide, zinc(II) triflate and di-tert-butyl dicarbonate to provide compound (24a) (*see* Lebel et al., Org. Lett., 2005, 7:4107-4110). Typically the reaction is run at elevated temperatures, preferably from 40-50°C, in a solvent such as, but not limited to, tetrahydrofuran.

### 5.1.7. Synthesis of Compound (29)

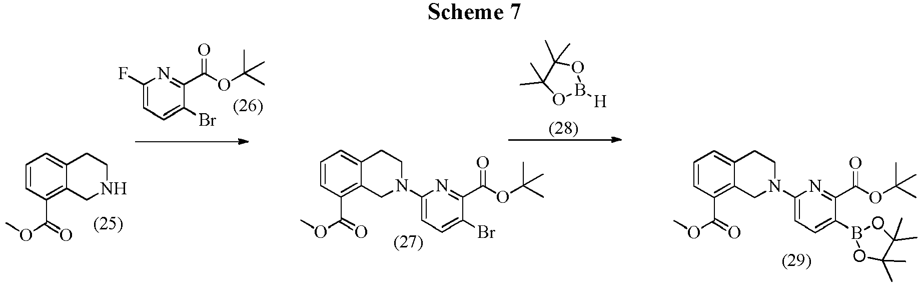

As shown in Scheme 7, compounds of formula (27) can be prepared by reacting compounds of formula (25) with *tert*-butyl 3-bromo-6-fluoropicolinate (26) in the presence of a base, such as, but not limited to, N,N-diisopropylethylamine, or triethylamine. The reaction is typically performed under an inert atmosphere at an elevated temperature in a solvent, such as, but not limited to, dimethyl sulfoxide. Compounds of formula (27) can be reacted with 4,4,5,5-tetramethyl-1,3,2-dioxaborolane (28), under borylation conditions described herein or in the literature to provide compounds of formula (29).

### 5.1.8. Synthesis of Compound (38)

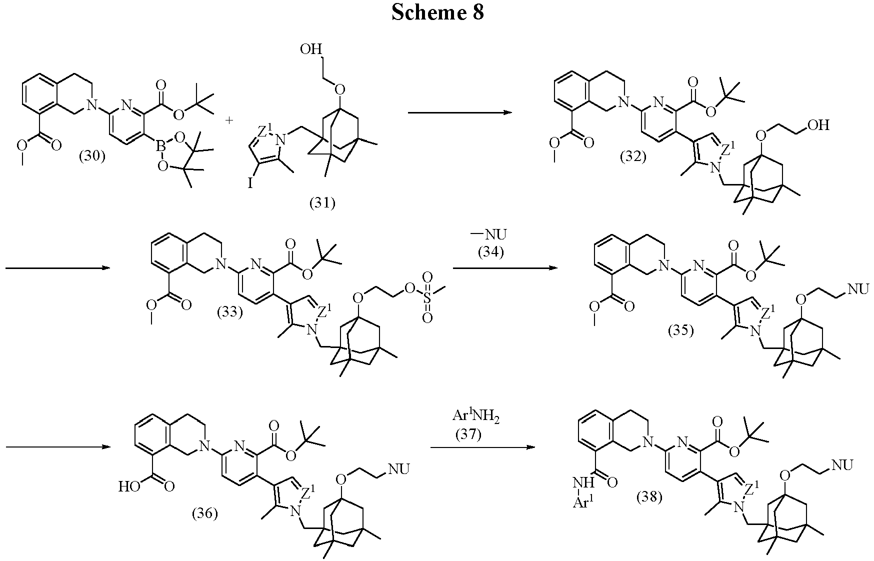

Scheme 8 describes a method to make intermediates which contain -Nu (nucleophile) tethered to an adamantane and picolinate protected as a t-butyl ester. Compound (30) can be reacted with compound (31) under Suzuki Coupling conditions described herein or in the literature to provide methyl compound (32). Compound (32) can be treated with a base such as but not limited to triethylamine, followed by methanesulfonyl chloride to provide compound (33). The addition is typically performed at low temperature before warming up to ambient temperature in a solvent, such as, but not limited to, dichloromethane. Compound (33) can be reacted with a nucleophile (Nu) of formula (34) to provide compound (35). Examples of nucleophiles include, but are not limited to, sodium azide, methylamine, ammonia and di-tert-butyl iminodicarbonate. Compound (17) can be reacted with lithium hydroxide to provide compound (36). The reaction is typically performed at ambient temperature in a solvent such as but not limited to tetrahydrofuran, methanol, water, or mixtures thereof. Compound (36) can be reacted with compound (37) under amidation conditions described herein or readily available in the literature to provide compounds of formula (38).

### 5.1.9. Synthesis of Compounds (42) and (43)

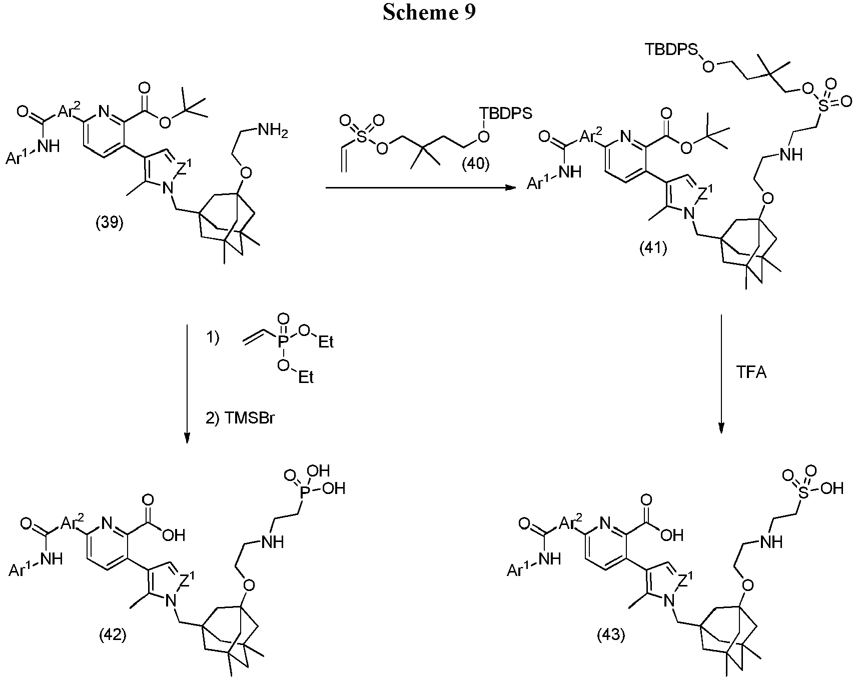

Scheme 9 shows representative methods used to make solubilized Bcl-xL inhibitors. Bcl-xL inhibitors can be synthesized using the general approach of modifying a primary amine with a solubilizing group and then attaching the resulting secondary amine to a linker as described in later schemes. For example, compound (41) can be prepared by reacting compound (39) with compound (40). The reaction is typically performed at ambient temperature in a solvent such as but not limited to N,N-dimethylformamide. Compound (41) can be reacted with trifluoroacetic acid to provide compound (43). The reaction is typically performed at ambient temperature in a solvent such as but not limited to dichloromethane. Another example shown in Scheme 9 is the reaction of compound (39) with diethyl vinylphosphonate, followed by reaction with bromotrimethylsilane and allyltrimethylsilane to provide compound (42). Other examples to introduce solubilizing groups on the Bcl-xL inhibitors described herein include, but are not limited to, reductive amination reactions, alkylations, and amidation reactions.

### 5.1.10. Synthesis of Compound (47)

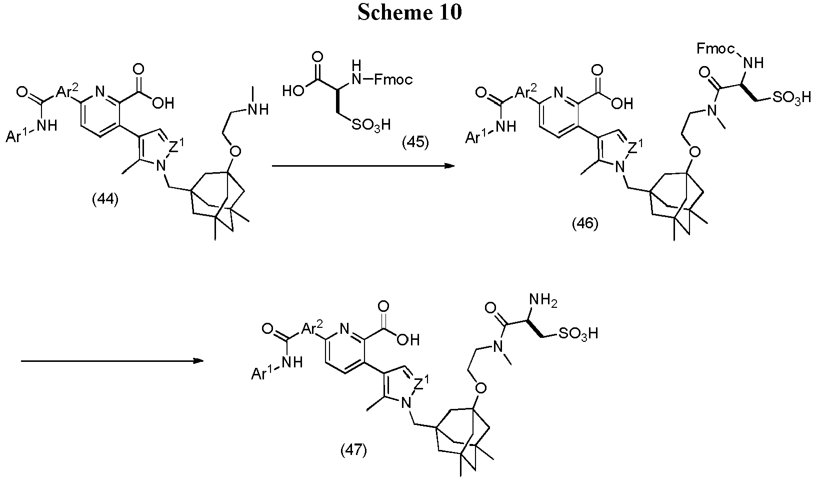

Scheme 10 shows introduction of a solubilizing group by amidation reaction. Bcl-xL inhibitors can be synthesized using the general approach of modifying a primary or secondary amine with a solubilizing group and then attaching the resulting amine to a linker as described in later schemes. For example, compound (45) can be treated sequentially with HATU and compound (44), to provide compound (46). Compound (46) can be treated with diethylamine in solvents such as, but not limited to, N,N-dimethylformamide to give compound (47).

### 5.1.11. Synthesis of Compound (51)

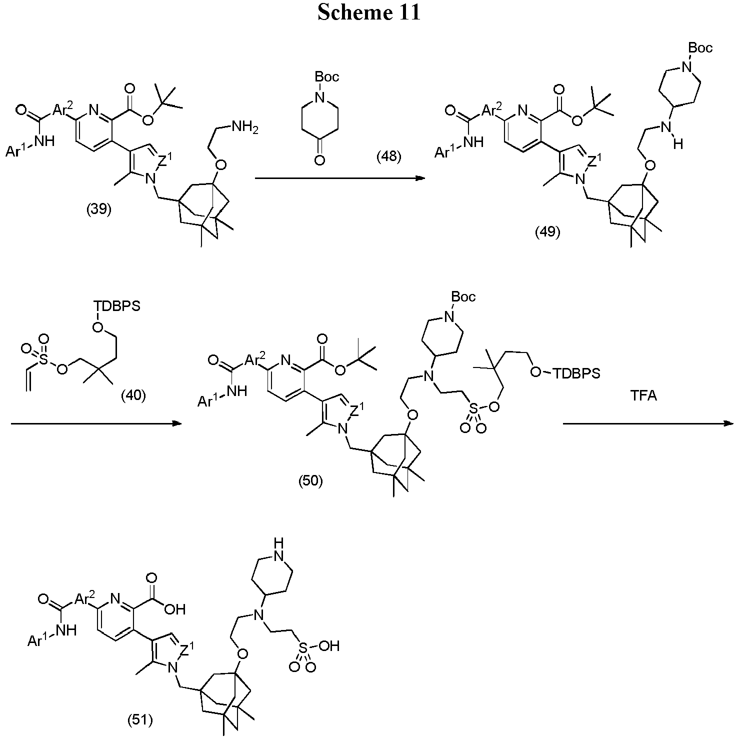

Scheme 11 shows representative methods to make solubilized Bcl-xL inhibitors. Bcl-xL inhibitors can be synthesized using the general approach of modifying a primary amine with a spacer to give a differentially protected diamine. The unprotected secondary amine can be modified with a solubilizing group. Deprotection of a protected amine them reveals a site for linker attachment, as described in later schemes. For example, compound (39) can be reductively alkylated with reagents such as, but not limited to tert-butyl 4-oxopiperidine-1-carboxylate (48), under conditions known in the art, to provide a secondary amine (49). Compound (50) can be prepared by reacting compound (49) with 4-((*tert*-butyldiphenylsilyl)oxy)-2,2-dimethylbutyl ethenesulfonate (40). The reaction is typically performed at ambient temperature in a solvent such as but not limited to N,N-dimethylformamide. Compound (40) can be reacted with trifluoroacetic acid to provide compound (51). The reaction is typically performed at ambient temperature in a solvent such as but not limited to dichloromethane.

### 5.1.12. Synthesis of Compound (61)

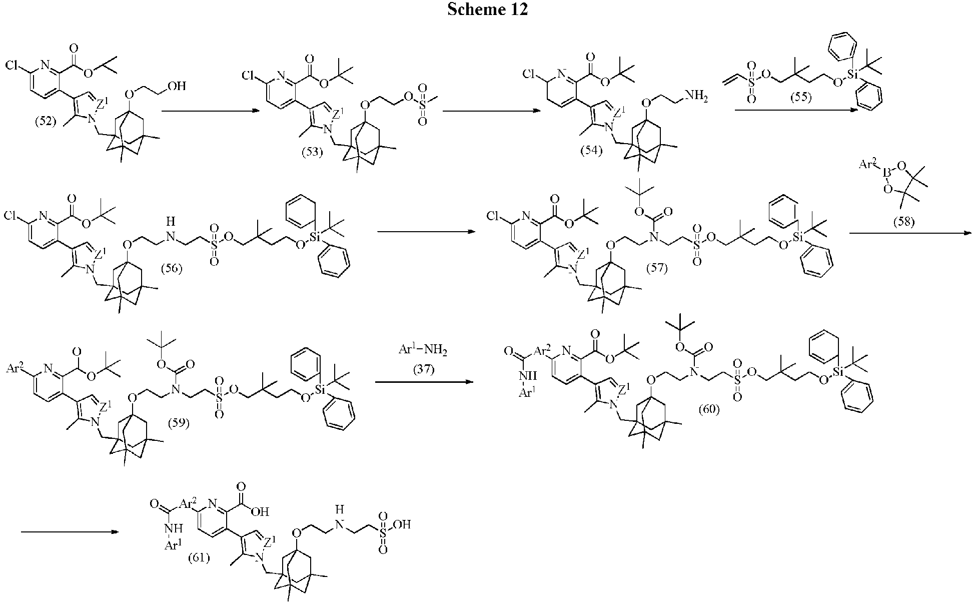

Scheme 12 describes a method to synthesize solubilized Bcl-xL inhibitors. Compound (52) can be reacted with methanesulfonyl chloride, in the presence of a base, such as, but not limited to, triethylamine, to provide compound (53). The reaction is typically performed at a low temperature in a solvent such as but not limited to dichloromethane. Compound (53) can be treated with ammonia in methanol to provide compound (54). The reaction is typically performed at an elevated temperature, and the reaction may be performed under microwave conditions. Compound (56) can be prepared by reacting compound (55) in the presence of a base such as but not limited to N,N-diisopropylethylamine. The reaction is typically performed at ambient temperature in a solvent such as but not limited to N,N-dimethylformamide. Compound (56) can be treated with di-t-butyldicarbonate and 4-(dimethylamino)pyridine to provide compound (57). The reaction is typically performed at ambient temperature in a solvent such as but not limited to tetrahydrofuran. Compound (59) can be prepared by reacting compound (57) with a boronate ester (or the equivalent boronic acid) of formula (58), under Suzuki Coupling conditions described herein or in the literature. Bis(2,5-dioxopyrrolidin-1-yl) carbonate can be reacted with compound (37), followed by reaction with compound (59), to provide compound (60). The reaction is typically performed at ambient temperature in a solvent such as, but not limited to, acetonitrile. Compound (61) can be prepared by treating compound (60) with trifluoroacetic acid. The reaction is typically performed at ambient temperature in a solvent such as but not limited to dichloromethane.

### 5.1.13. Synthesis of Compound (70)

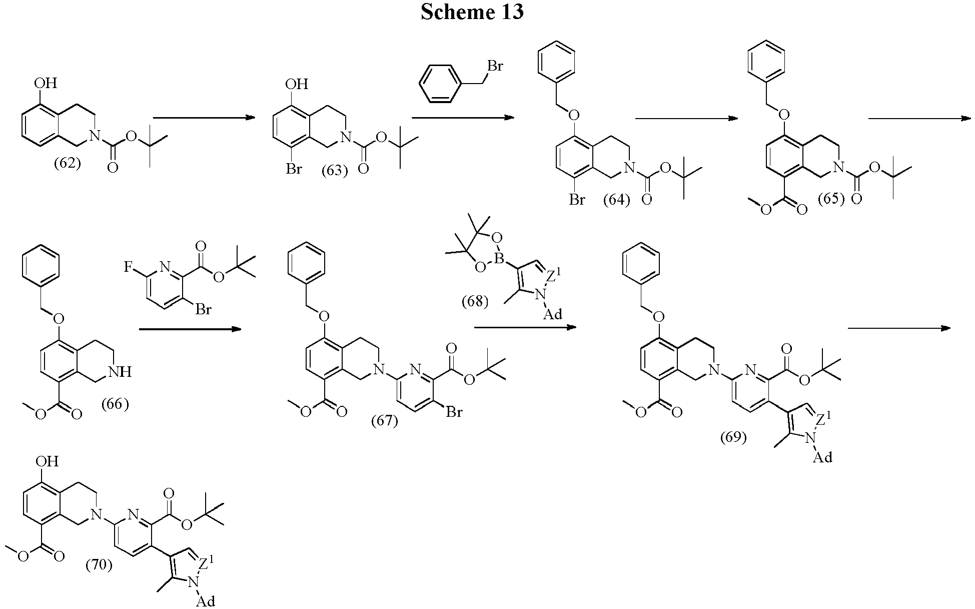

Scheme 13 describes the synthesis of 5-hydroxy tetrahydroisoquinoline intermediates. Compound (63) can be prepared by treating compound (62) with N-bromosuccinimide. The reaction is typically performed at ambient temperature is a solvent such as, but not limited to, N,N-dimethylformamide. Compound (63) can be reacted with benzyl bromide in the presence of a base, such as, but not limited to, potassium carbonate, to provide compound (64). The reaction is typically performed at an elevated temperature, in a solvent such as, but not limited to, acetone. Compound (64) can be treated with carbon monoxide and methanol in the presence of a base, such as, but not limited to, triethylamine, and a catalyst, such as, but not limited to, compound (65). The reaction is typically performed at an elevated temperature under an inert atmosphere. Compound (65) can be treated with an acid, such as, but not limited to, hydrochloric acid in dioxane, to provide compound (66). The reaction is typically performed at ambient temperature in a solvent, such as, but not limited to, tetrahydrofuran. Compound (67) can be prepared by reacting compound (66) with *tert*-butyl 3-bromo-6-fluoropicolinate in the presence of a base, such as, but not limited to, triethylamine. The reaction is typically performed under an inert atmosphere at an elevated temperature in a solvent, such as, but not limited to, dimethyl sulfoxide. Compound (67) can be reacted with a boronic acid of formula (68), wherein Ad is the methyladamantane moiety of the compounds of the disclosure (*e.g.*, the compounds of formulae (IIa)-(IId)), under Suzuki Coupling conditions described herein or in the literature to provide compound (69). Compound (70) can be prepared by reacting compound (69) with hydrogen in the presence of Pd(OH)₂. The reaction is typically performed at an elevated temperature in a solvent such as, but not limited to tetrahydrofuran.

### 5.1.14. Synthesis of Compound (75)

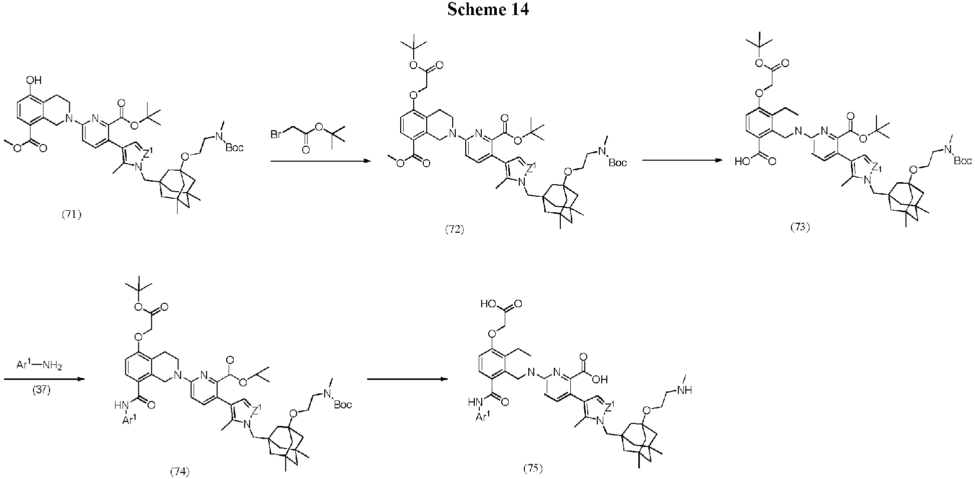

Scheme 14 shows representative methods used to make solubilized Bcl-xL inhibitors. Bcl-xL inhibitors can be synthesized using the general approach of modifying an Ar² substituent with a solubilizing group and then attaching an amine to a linker as described in later schemes. For example, compound (71) can be reacted with tert-butyl 2-bromoacetate in the presence of a base such as, but not limited to, potassium carbonate in a solvent such as, but not limited, to N,N-dimethylformamide. Compound (72) can be treated with aqueous lithium hydroxide in a solvent such as, but not limited to, methanol, tetrahydrofuran or mixtures thereof to provide compound (73). Compound (74) can be obtained by amidation of compound (73) with compound (37) under conditions previously described. Compound (74) can be treated with acids such as, but not limited to trifluoroacetic acid or HCl, to provide a Bcl-xL inhibitor of the formula (75). The reaction is typically performed at ambient temperature in solvents such as, but not limited to, dichloromethane or 1,4-dioxane.

### 5.1.2. General Methods for Synthesizing Synthons

In the schemes below, the various substituents Ar¹, Ar², Z¹, Y, G, R^{11a} and R^{11b} are as defined in the Detailed Description section.

### 5.2.1. Synthesis of Compound (89)

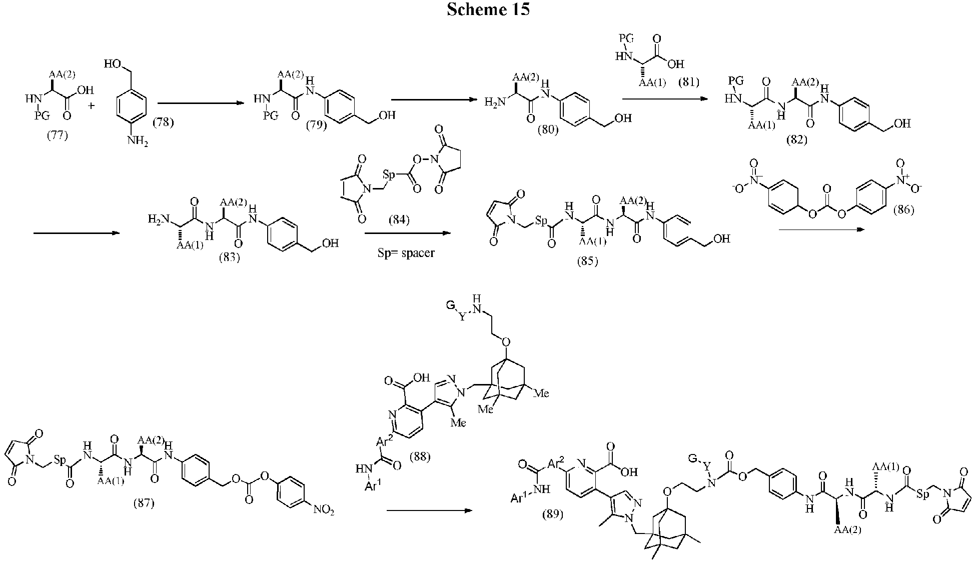

As shown in scheme 15, compounds of formula (77), wherein PG is an appropriate base labile protecting group and AA(2) is Cit, Ala, or Lys, can be reacted with 4-(aminophenyl)methanol (78), under amidation conditions described herein or readily available in the literature to provide compound (79). Compound (80) can be prepared by reacting compound (79) with a base such as, but not limited to, diethylamine. The reaction is typically performed at ambient temperature in a solvent such as but not limited to N,N-dimethylformamide. Compound (81), wherein PG is an appropriate base or acid labile protecting group and AA(1) is Val or Phe, can be reacted with compound (80), under amidation conditions described herein or readily available in the literature to provide compound (82). Compound (83) can be prepared by treating compound (82) with diethylamine or trifluoroacetic acid, as appropriate. The reaction is typically performed at ambient temperature in a solvent such as but not limited to dichloromethane. Compound (84), wherein Sp is a spacer, can be reacted with compound (83) to provide compound (85). The reaction is typically performed at ambient temperature in a solvent such as but not limited to N,N-dimethylformamide. Compound (85) can be reacted with bis(4-nitrophenyl) carbonate (86) in the presence of a base such as, but not limited to N,N-diisopropylethylamine, to provide compounds (87). The reaction is typically performed at ambient temperature in a solvent such as but not limited to N,N-dimethylformamide. Compounds (87) can be reacted with compound (88) in the presence of a base such as, but not limited to, N,N-diisopropylethylamine, to provide compound (89). The reaction is typically performed at ambient temperature in a solvent such as, but not limited to, N,N-dimethylformamide.

### 5.2.2. Synthesis of Compounds (94) and (96)

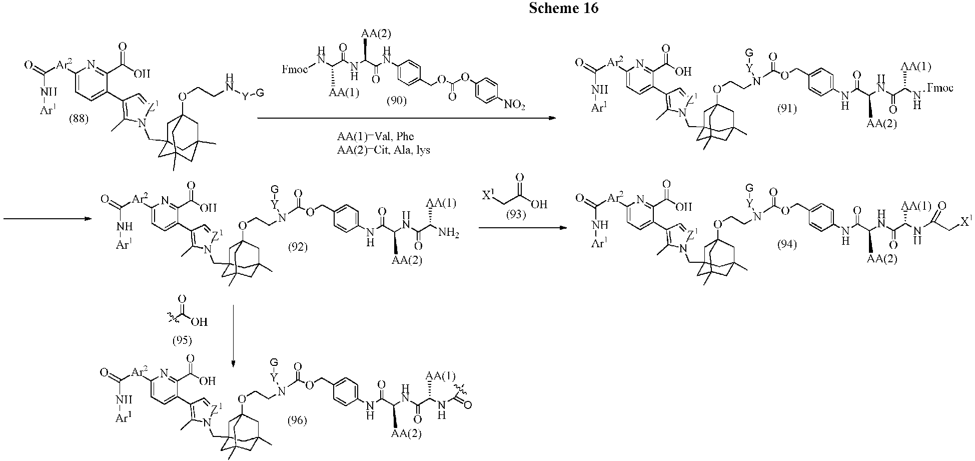

Scheme 16 describes the installment of alternative mAb-linker attachments to dipeptide Synthons. Compound (88) can be reacted with compound (90) in the presence of a base such as, but not limited to, N,N-diisopropylamine to provide compound (91). The reaction is typically performed at ambient temperature in a solvent such as but not limited to N,N-dimethylformamide. Compound (92) can be prepared by reacting compound (91) with diethylamine. The reaction is typically performed at ambient temperature in a solvent such as but not limited to N,N-dimethylformamide. Compound (93), wherein X¹ is Cl, Br, or I, can be reacted with compound (92), under amidation conditions described herein or readily available in the literature to provide compound (94). Compound (92) can be reacted with compounds of formula (95) under amidation conditions described herein or readily available in the literature to provide compound (96).

### 5.2.3. Synthesis of Compound (106)

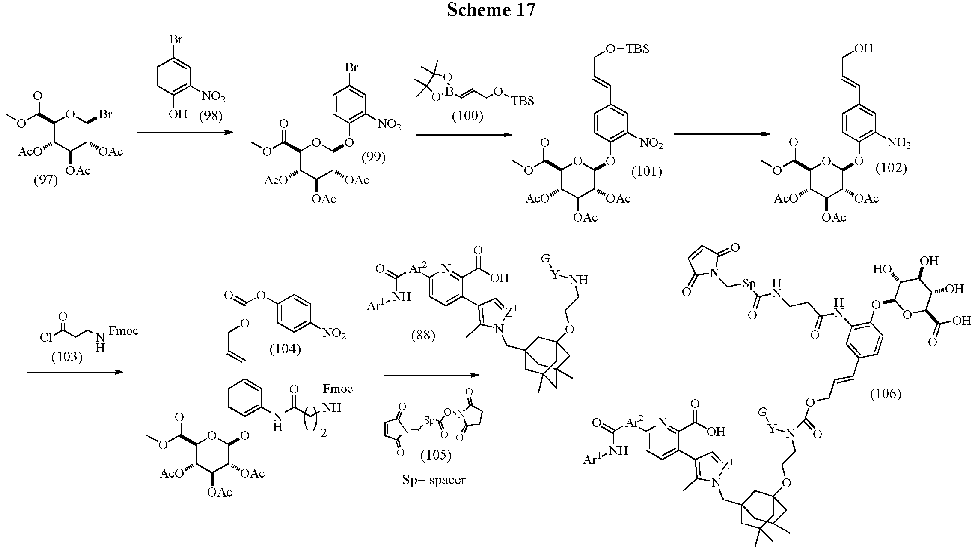

Scheme 17 describes the synthesis of vinyl glucuronide linker intermediates and synthons. (2R,3R,4S,5S,6S)-2-Bromo-6-(methoxycarbonyl)tetrahydro-2H-pyran-3,4,5-triyl triacetate (97) can be treated with silver oxide, followed by 4-bromo-2-nitrophenol (98) to provide (2S,3R,4S,5S,6S)-2-(4-bromo-2-nitrophenoxy)-6-(methoxycarbonyl)tetrahydro-2H-pyran-3,4,5-triyl triacetate (99). The reaction is typically performed at ambient temperature in a solvent, such as, but not limited to, acetonitrile. (2S,3R,4S,5S,6S)-2-(4-Bromo-2-nitrophenoxy)-6-(methoxycarbonyl)tetrahydro-2H-pyran-3,4,5-triyl triacetate (99) can be reacted with (E)-*tert*-butyldimethyl((3-(4,4,5,5-tetramethyl-1,3,2-dioxaborolan-2-yl)allyl)oxy)silane (100) in the presence of a base such as, but not limited to, sodium carbonate, and a catalyst such as but not limited to tris(dibenzylideneacetone)dipalladium (Pd₂(dba)₃), to provide (2S,3R,4S,5S,6S)-2-(4-((E)-3-((*tert*-butyldimethylsilyl)oxy)prop-1-en-1-yl)-2-nitrophenoxy)-6-(methoxycarbonyl)tetrahydro-2H-pyran-3,4,5-triyl triacetate (101). The reaction is typically performed at an elevated temperature in a solvent, such as, but not limited to, tetrahydrofuran. (2S,3R,4S,5S,6S)-2-(2-amino-4-((E)-3-hydroxyprop-1-en-1-yl)phenoxy)-6-(methoxycarbonyl)tetrahydro-2H-pyran-3,4,5-triyl triacetate (102) can be prepared by reacting (2S,3R,4S,5S,6S)-2-(4-((E)-3-((*tert*-butyldimethylsilyl)oxy)prop-1-en-1-yl)-2-nitrophenoxy)-6-(methoxycarbonyl)tetrahydro-2H-pyran-3,4,5-triyl triacetate (101) with zinc in the presence of an acid such as, but not limited to, hydrochloric acid. The addition is typically performed at low temperature before warming to ambient temperature in a solvent such as, but not limited to, tetrahydrofuran, water, or mixtures thereof. (2S,3R,4S,5S,6S)-2-(2-amino-4-((E)-3-hydroxyprop-1-en-1-yl)phenoxy)-6-(methoxycarbonyl)tetrahydro-2H-pyran-3,4,5-triyl triacetate (102) can be reacted with (9H-fluoren-9-yl)methyl (3-chloro-3-oxopropyl)carbamate (103), in the presence of a base such as, but not limited to, N,N-diisopropylethylamine, to provide (2S,3R,4S,5S,6S)-2-(2-(3-((((9H-fluoren-9-yl)methoxy)carbonyl)amino)propanamido)-4-((E)-3-hydroxyprop-1-en-1-yl)phenoxy)-6-(methoxycarbonyl)tetrahydro-2H-pyran-3,4,5-triyl triacetate (104). The addition is typically performed at low temperature before warming to ambient temperature in a solvent such as, but not limited to, dichloromethane. Compound (88) can be reacted with (2S,3R,4S,5S,6S)-2-(2-(3-((((9H-fluoren-9-yl)methoxy)carbonyl)amino)propanamido)-4-((E)-3-hydroxyprop-1-en-1-yl)phenoxy)-6-(methoxycarbonyl)tetrahydro-2H-pyran-3,4,5-triyl triacetate (104) in the presence of a base such as, but not limited to, N-ethyl-N-isopropylpropan-2-amine, followed by work up and reaction with compound (105) in the presence of a base such as, but not limited to, N,N-diisopropylethylamine to provide compound (106). The reactions are typically performed at ambient temperature in a solvent such as, but not limited to N,N- dimethylformamide.

### 5.2.4. Synthesis of Compound (115)

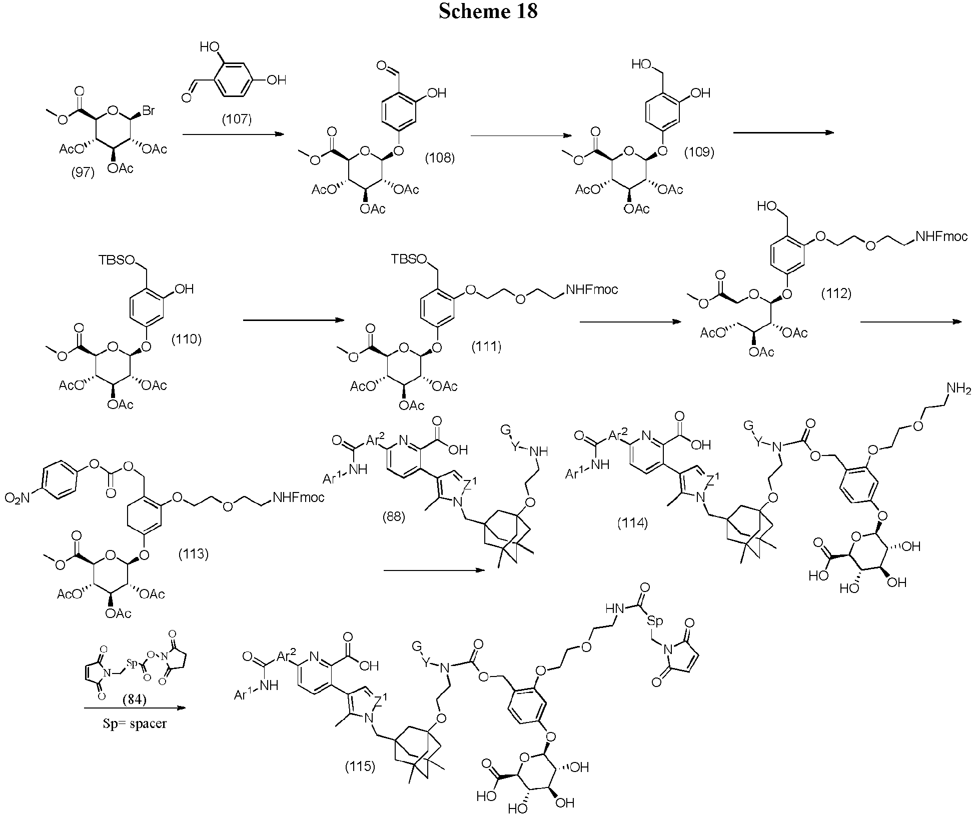

Scheme 18 describes the synthesis of a representative 2-ether glucuronide linker intermediate and synthon. (2S,3R,4S,5S,6S)-2-Bromo-6-(methoxycarbonyl)tetrahydro-2H-pyran-3,4,5-triyl triacetate (97) can be reacted with 2,4-dihydroxybenzaldehyde (107) in the presence of silver carbonate to provide (2S,3R,4S,5S,6S)-2-(4-formyl-3-hydroxyphenoxy)-6-(methoxycarbonyl)tetrahydro-2H-pyran-3,4,5-triyl triacetate (108). The reaction is typically performed at an elevated temperature in a solvent, such as, but not limited to, acetonitrile. (2S,3R,4S,5S,6S)-2-(4-Formyl-3-hydroxyphenoxy)-6-(methoxycarbonyl)tetrahydro-2H-pyran-3,4,5-triyl triacetate (108) can be treated with sodium borohydride to provide (2S,3R,4S,5S,6S)-2-(3-hydroxy-4-(hydroxymethyl)phenoxy)-6-(methoxycarbonyl)tetrahydro-2H-pyran-3,4,5-triyl triacetate (109). The addition is typically performed at low temperature before warming to ambient temperature in a solvent such as but not limited to tetrahydrofuran, methanol, or mixtures thereof. (2S,3R,4S,5S,6S)-2-(4-(((*tert*-butyldimethylsilyl)oxy)methyl)-3-hydroxyphenoxy)-6-(methoxycarbonyl)tetrahydro-2H-pyran-3,4,5-triyl triacetate (110) can be prepared by reacting (2S,3R,4S,5S,6S)-2-(3-hydroxy-4-(hydroxymethyl)phenoxy)-6-(methoxycarbonyl)tetrahydro-2H-pyran-3,4,5-triyl triacetate (109) with *tert*-butyldimethylsilyl chloride in the presence of imidazole. The reaction is typically performed at low temperature in a solvent, such as, but not limited to, dichloromethane. (2S,3R,4S,5S,6S)-2-(3-(2-(2-((((9H-Fluoren-9-yl)methoxy)carbonyl)amino)ethoxy)ethoxy)-4-(((*tert*-butyldimethylsilyl)oxy)methyl)phenoxy)-6-(methoxycarbonyl)tetrahydro-2H-pyran-3,4,5-triyl triacetate (111) can be prepared by reacting (2S,3R,4S,5S,6S)-2-(4-(((*tert*-butyldimethylsllyl)oxy)methyl)-3-hydroxyphenoxy)-6-(methoxycarbonyl)tetrahydro-2H-pyran-3,4,5-triyltriacetate (110) with (9H-fluoren-9-yl)methyl (2-(2-hydroxyethoxy)ethyl)carbamate in the presence of triphenylphosphine and a azodicarboxylate such as, but not limited to, di-*tert*-butyl diazene-1,2-dicarboxylate. The reaction is typically performed at ambient temperature in a solvent such as but not limited to toluene. (2S,3R,4S,5S,6S)-2-(3-(2-(2-((((9H-Fluoren-9-yl)methoxy)carbonyl)amino)ethoxy)ethoxy)-4-(((*tert-*butyldimethylsilyl)oxy)methyl)phenoxy)-6-(methoxycarbonyl)tetrahydro-2H-pyran-3,4,5-triyl triacetate (111) can be treated with acetic acid to provide (2S,3R,4S,5S,6S)-2-(3-(2-(2-((((9H-fluoren-9-yl)methoxy)carbonyl)amino)ethoxy)ethoxy)-4-(hydroxymethyl)phenoxy)-6-(methoxycarbonyl)tetrahydro-2H-pyran-3,4,5-triyl triacetate (112). The reaction is typically performed at ambient temperature in a solvent such as but not limited to water, tetrahydrofuran, or mixtures thereof. (2S,3R4S,5S,6S)-2-(3-(2-(2-((((9H-Fluoren-9-yl)methoxy)carbonyl)amino)ethoxy)ethoxy)-4-((((4-nitrophenoxy)carbonyl)oxy)methyl)phenoxy)-6-(methoxycarbonyl)tetrahydro-2H-pyran-3,4,5-triyl triacetate (113) can be prepared by reacting (2S,3R,4S,5S,6S)-2-(3-(2-(2-((((9H-fluoren-9-yl)methoxy)carbonyl)amino)ethoxy)ethoxy)-4-(hydroxymethyl)phenoxy)-6-(methoxycarbonyl)tetrahydro-2H-pyran-3,4,5-triyltriacetate (112) with bis(4-nitrophenyl) carbonate in the presence of a base such as but not limited to N-ethyl-N-isopropylpropan-2-amine. The reaction is typically performed at ambient temperature in a solvent such as but not limited to N,N-dimethylformamide. (2S,3R,4S,5S,6S)-2-(3-(2-(2-((((9H-Fluoren-9-yl)methoxy)carbonyl)amino)ethoxy)ethoxy)-4-((((4-nitrophenoxy)carbonyl)oxy)methyl)phenoxy)-6-(methoxycarbonyl)tetrahydro-2H-pyran-3,4,5-triyltriacetate (113) can be treated with compound (88) in the presence of a base such as but not limited to N-ethyl-N-isopropylpropan-2-amine, followed by treatment with lithium hydroxide to provide a compound (114). The reaction is typically performed at ambient temperature in a solvent such as but not limited to N,N-dimethylformamide, tetrahydrofuran, methanol, or mixtures thereof. Compound (115) can be prepared by reacting compound (114) with compound (84) in the presence of a base such as but not limited to N-ethyl-N-isopropylpropan-2-amine. The reaction is typically performed at ambient temperature in a solvent such as but not limited to N,N-dimethylformamide.

### 5.2.5. Synthesis of Compound (119)

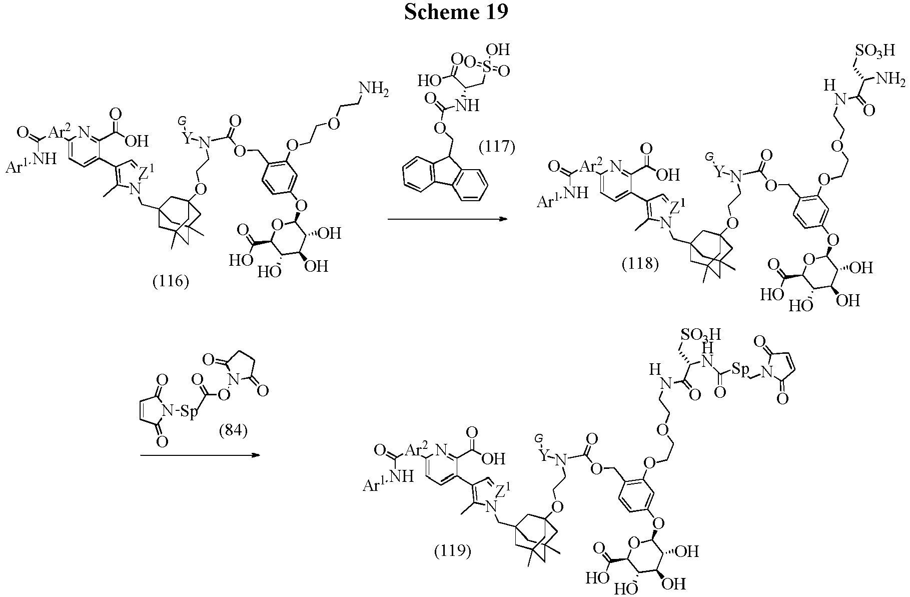

Scheme 19 describes the introduction of a second solubilizing group to a sugar linker. Compound (116) can be reacted with (R)-2-((((9H-fluoren-9-yl)methoxy)carbonyl)amino)-3-sulfopropanoic acid (117), under amidation conditions described herein or readily available in the literature, followed by treatment with a base such as but not limited to diethylamine, to provide compound (118). Compound (118) can be reacted with compound (84), wherein Sp is a spacer, under amidation conditions described herein or readily available in the literature, to provide compound (119).

### 5.2.6. Synthesis of Compound (129)

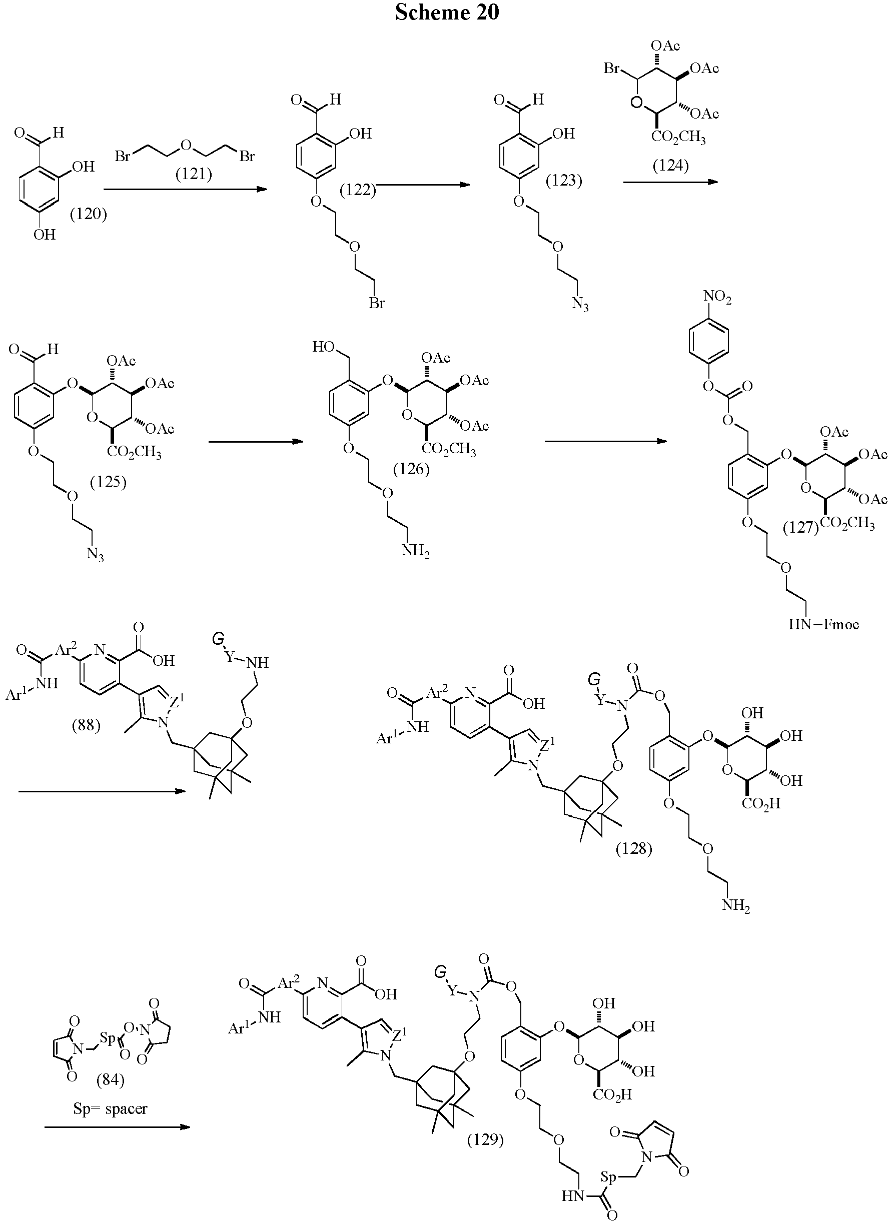

Scheme 20 describes the synthesis of 4-ether glucuronide linker intermediates and synthons. 4-(2-(2-Bromoethoxy)ethoxy)-2-hydroxybenzaldehyde (122) can be prepared by reacting 2,4-dihydroxybenzaldehyde (120) with 1-bromo-2-(2-bromoethoxy)ethane (121) in the presence of a base such as, but not limited to, potassium carbonate. The reaction is typically performed at an elevated temperature in a solvent such as but not limited to acetonitrile. 4-(2-(2-Bromoethoxy)ethoxy)-2-hydroxybenzaldehyde (122) can be treated with sodium azide to provide 4-(2-(2-azidoethoxy)ethoxy)-2-hydroxybenzaldehyde (123). The reaction is typically performed at ambient temperature in a solvent such as but not limited to N,N-dimethylformamide. (2S,3R,4S,5S,6S)-2-(5-(2-(2-Azidoethoxy)ethoxy)-2-formylphenoxy)-6-(methoxycarbonyl)tetrahydro-2H-pyran-3,4,5-triyl triacetate (125) can be prepared by reacting 4-(2-(2-azidoethoxy)ethoxy)-2-hydroxybenzaldehyde (123) with (3R,4S,5S,6S)-2-bromo-6-(methoxycarbonyl)tetrahydro-2H-pyran-3,4,5-triyl triacetate (124) in the presence of silver oxide. The reaction is typically performed at ambient temperature in a solvent such as, but not limited to, acetonitrile. Hydrogenation of (2S,3R,4S,5S,6S)-2-(5-(2-(2-azidoethoxy)ethoxy)-2-formylphenoxy)-6-(methoxycarbonyl)tetrahydro-2H-pyran-3,4,5-triyl triacetate (125) in the presence of Pd/C will provide (2S,3R,4S,5S,6S)-2-(5-(2-(2-aminoethoxy)ethoxy)-2-(hydroxymethyl)phenoxy)-6-(methoxycarbonyl)tetrahydro-2H-pyran-3,4,5-triyl triacetate (126). The reaction is typically performed at ambient temperature in a solvent such as, but not limited to, tetrahydrofuran. (2S,3R,4S,5S,6S)-2-(5-(2-(2-((((9H-Fluoren-9-yl)methoxy)carbonyl)amino)ethoxy)ethoxy)-2-(hydroxymethyl)phenoxy)-6-(methoxycarbonyl)tetrahydro-2H-pyran-3,4,5-triyl triacetate (127) can be prepared by treating (2S,3R,4S,5S,6S)-2-(5-(2-(2-aminoethoxy)ethoxy)-2-(hydroxymethyl)phenoxy)-6-(methoxycarbonyl)tetrahydro-2H-pyran-3,4,5-triyl triacetate (126) with (9H-fluoren-9-yl)methyl carbonochloridate in the presence of a base, such as, but not limited to, N-ethyl-N-isopropylpropan-2-amine. The reaction is typically performed at low temperature in a solvent such as, but not limited to, dichloromethane. Compound (88) can be reacted with (2S,3R,4S,5S,6S)-2-(5-(2-(2-((((9H-Fluoren-9-yl)methoxy)carbonyl)amino)ethoxy)ethoxy)-2-(hydroxymethyl)phenoxy)-6-(methoxycarbonyl)tetrahydro-2H-pyran-3,4,5-triyl triacetate (127) in the presence of a base, such as, but not limited to, N-ethyl-N-isopropylpropan-2-amine, followed by treatment with lithium hydroxide to provide compound (128). The reaction is typically performed at low temperature in a solvent such as, but not limited to, N,N-dimethylformamide. Compound (129) can be prepared by reacting compound (128) with compound (84) in the presence of a base such as, but not limited to, N-ethyl-N-isopropylpropan-2-amine. The reaction is typically performed at ambient temperature in a solvent such as but not limited to N,N-dimethylformamide.

### 5.2.7. Synthesis of Compound (139)

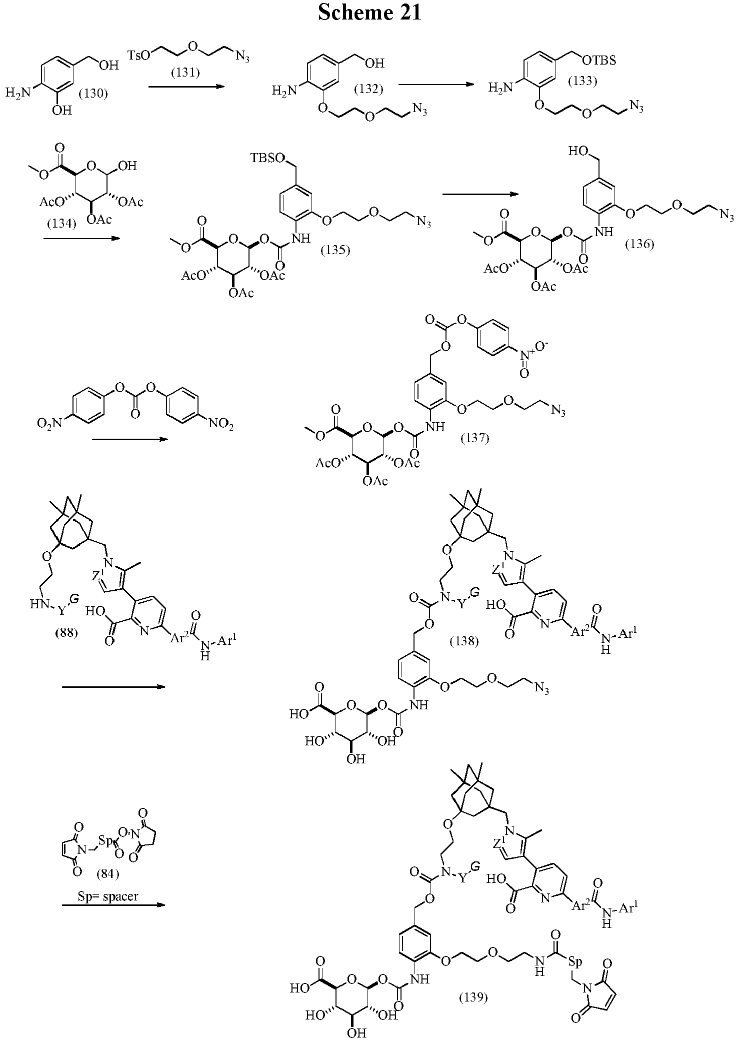

Scheme 21 describes the synthesis of carbamate glucuronide intermediates and synthons. 2-Amino-5-(hydroxymethyl)phenol (130) can be treated with sodium hydride and then reacted with 2-(2-azidoethoxy)ethyl 4-methylbenzenesulfonate (131) to provide (4-amino-3-(2-(2-azidoethoxy)ethoxy)phenyl)methanol (132). The reaction is typically performed at an elevated temperature in a solvent such as, but not limited to N,N-dimethylformamide. 2-(2-(2-Azidoethoxy)ethoxy)-4-(((tert-butyldimethylsilyl)oxy)methyl)aniline (133) can be prepared by reacting (4-amino-3-(2-(2-azidoethoxy)ethoxy)phenyl)methanol (132) with tert-butyldimethylchlorosilane in the presence of imidazole. The reaction is typically performed at ambient temperature in a solvent such as, but not limited to tetrahydrofuran. 2-(2-(2-Azidoethoxy)ethoxy)-4-(((tert-butyldimethylsilyl)oxy)methyl)aniline (133) can be treated with phosgene, in the presence of a base such as but not limited to triethylamine, followed by reaction with (3R,4S,5S,6S)-2-hydroxy-6-(methoxycarbonyl)tetrahydro-2H-pyran-3,4,5-triyl triacetate (134) in the presence of a base such as but not limited to triethylamine, to provide 2S,3R,4S,5S,6S)-2-(((2-(2-(2-azidoethoxy)ethoxy)-4-(((tert-butyldimethylsilyl)oxy)methyl)phenyl)carbamoyl)oxy)-6-(methoxycarbonyl)tetrahydro-2H-pyran-3,4,5-triyl triacetate (135). The reaction is typically performed in a solvent such as, but not limited to, toluene, and the additions are typically performed at low temperature, before warming up to ambient temperature after the phosgene addition and heating at an elevated temperature after the (3R,4S,5S,6S)-2-hydroxy-6-(methoxycarbonyl)tetrahydro-2H-pyran-3,4,5-triyl triacetate (134) addition. (2S,3R,4S,5S,6S)-2-(((2-(2-(2-Azidoethoxy)ethoxy)-4-(hydroxymethyl)phenyl)carbamoyl)oxy)-6-(methoxycarbonyl)tetrahydro-2H-pyran-3,4,5-triyl triacetate (136) can be prepared by reacting 2S,3R,4S,5S,6S)-2-(((2-(2-(2-azidoethoxy)ethoxy)-4-(((tert-butyldimethylsilyl)oxy)methyl)phenyl)carbamoyl)oxy)-6-(methoxycarbonyl)tetrahydro-2H-pyran-3,4,5-triyl triacetate (135) with p-toluenesulfonic acid monohydrate. The reaction is typically performed at ambient temperature in a solvent such as, but not limited to methanol. (2S,3R,4S,5S,6S)-2-(((2-(2-(2-Azidoethoxy)ethoxy)-4-(hydroxymethyl)phenyl)carbamoyl)oxy)-6-(methoxycarbonyl)tetrahydro-2H-pyran-3,4,5-triyl triacetate (136) can be reacted with bis(4-nitrophenyl)carbonate in the presence of a base such as, but not limited to, N,N-diisopropylethylamine, to provide (2S,3R,4S,5S,6S)-2-(((2-(2-(2-azidoethoxy)ethoxy)-4-((((4-nitrophenoxy)carbonyl)oxy)methyl)phenyl)carbamoyl)oxy)-6-(methoxycarbonyl)tetrahydro-2H-pyran-3,4,5-triyl triacetate (137). The reaction is typically performed at ambient temperature in a solvent such as, but not limited to, N,N-dimethylformamide. (2S,3R,4S,5S,6S)-2-(((2-(2-(2-Azidoethoxy)ethoxy)-4-((((4-nitrophenoxy)carbonyl)oxy)methyl)phenyl)carbamoyl)oxy)-6-(methoxycarbonyl)tetrahydro-2H-pyran-3,4,5-triyl triacetate (137) can be reacted with compound in the presence of a base such as, but not limited to, N,N-diisopropylethylamine, followed by treatment with aqueous lithium hydroxide, to provide compound (138). The first step is typically conducted at ambient temperature in a solvent such as, but not limited to N,N-dimethylformamide, and the second step is typically conducted at low temperature in a solvent such as but not limited to methanol. Compound (138) can be treated with tris(2-carboxyethyl))phosphine hydrochloride, followed by reaction with compound (84) in the presence of a base such as, but not limited to, N,N-diisopropylethylamine, to provide compound (139). The reaction with tris(2-carboxyethyl))phosphine hydrochloride is typically performed at ambient temperature in a solvent such as, but not limited to, tetrahydrofuran, water, or mixtures thereof, and the reaction with N-succinimidyl 6-maleimidohexanoate is typically performed at ambient temperature in a solvent such as, but not limited to, N,N-dimethylformamide.

### 5.2.8. Synthesis of Compound (149)

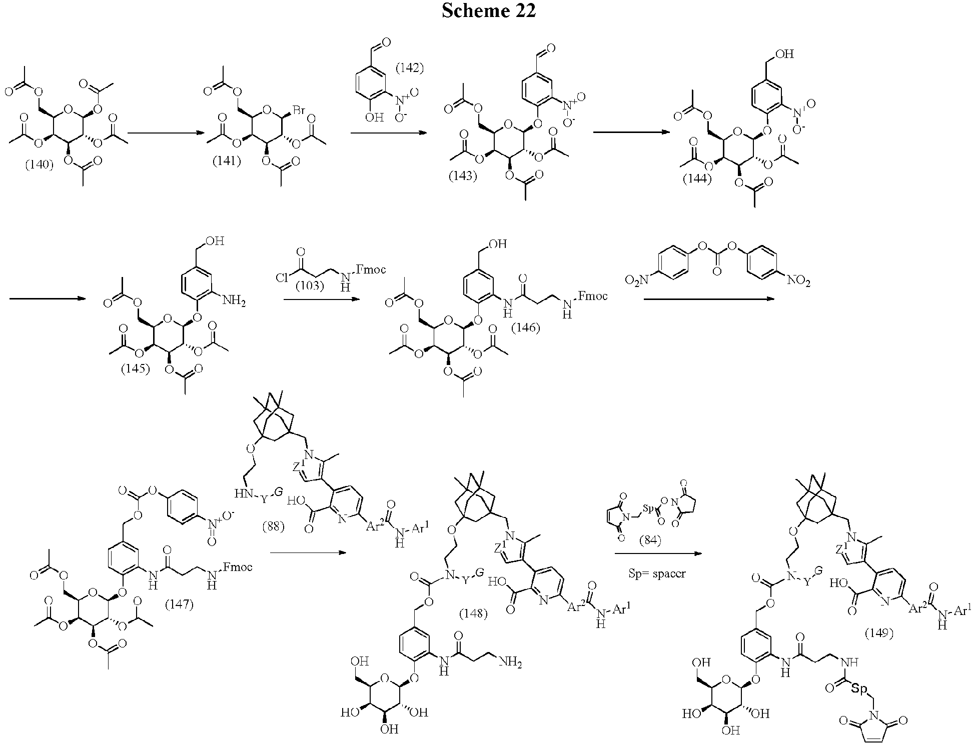

Scheme 22 describes the synthesis of galactoside linker intermediates and synthons. (2S,3R,4S,5S,6R)-6-(Acetoxymethyl)tetrahydro-2H-pyran-2,3,4,5-tetrayl tetraacetate (140) can be treated with HBr in acetic acid to provide (2R,3S,4S,5R,6S)-2-(acetoxymethyl)-6-bromotetrahydro-2H-pyran-3,4,5-triyl triacetate (141). The reaction is typically performed at ambient temperature under a nitrogen atmosphere. (2R,3S,4S,5R,6S)-2-(Acetoxymethyl)-6-(4-formyl-2-nitrophenoxy)tetrahydro-2H-pyran-3,4,5-triyl triacetate (143) can be prepared by treating (2R,3S,4S,5R,6S)-2-(acetoxymethyl)-6-bromotetrahydro-2H-pyran-3,4,5-triyl triacetate (141) with silver(I) oxide in the presence of 4-hydroxy-3-nitrobenzaldehyde (142). The reaction is typically performed at ambient temperature in a solvent such as, but not limited to, acetonitrile. (2R,3S,4S,5R,6S)-2-(Acetoxymethyl)-6-(4-formyl-2-nitrophenoxy)tetrahydro-2H-pyran-3,4,5-triyl triacetate (143) can be treated with sodium borohydride to provide (2R,3S,4S,5R,6S)-2-(acetoxymethyl)-6-(4-(hydroxymethyl)-2-nitrophenoxy)tetrahydro-2H-pyran-3,4,5-triyl triacetate (144). The reaction is typically performed at low temperature in a solvent such as but not limited to tetrahydrofuran, methanol, or mixtures thereof. (2R,3S,4S,5R,6S)-2-(Acetoxymethyl)-6-(2-amino-4-(hydroxymethyl)phenoxy)tetrahydro-2H-pyran-3,4,5-triyl triacetate (145) can be prepared by treating (2R,3S,4S,5R,6S)-2-(acetoxymethyl)-6-(4-(hydroxymethyl)-2-nitrophenoxy)tetrahydro-2H-pyran-3,4,5-triyl triacetate (144) with zinc in the presence of hydrochloric acid. The reaction is typically performed at low temperature, under a nitrogen atmosphere, in a solvent such as, but not limited to, tetrahydrofuran. (2S,3R,4S,5S,6R)-2-(2-(3-((((9H-Fluoren-9-yl)methoxy)carbonyl)amino)propanamido)-4-(hydroxymethyl)phenoxy)-6-(acetoxymethyl)tetrahydro-2H-pyran-3,4,5-triyl triacetate (146) can be prepared by reacting (2R,3S,4S,5R,6S)-2-(acetoxymethyl)-6-(2-amino-4-(hydroxymethyl)phenoxy)tetrahydro-2H-pyran-3,4,5-triyl triacetate (145) with (9H-fluoren-9-yl)methyl (3-chloro-3-oxopropyl)carbamate (103) in the presence of a base such as, but not limited to, N,N-diisopropylethylamine. The reaction is typically performed at low temperature, in a solvent such as, but not limited to, dichloromethane. (2S,3R,4S,5S,6R)-2-(2-(3-((((9H-Fluoren-9-yl)methoxy)carbonyl)amino)propanamido)-4-(hydroxymethyl)phenoxy)-6-(acetoxymethyl)tetrahydro-2H-pyran-3,4,5-triyl triacetate (146) can be reacted with bis(4-nitrophenyl)carbonate in the presence of a base such as, but not limited to, N,N-diisopropylethylamine, to provide (2S,3R,4S,5S,6R)-2-(2-(3-((((9H-fluoren-9-yl)methoxy)carbonyl)amino)propanamido)-4-((((4-nitrophenoxy)carbonyl)oxy)methyl)phenoxy)-6-(acetoxymethyl)tetrahydro-2H-pyran-3,4,5-triyl triacetate (147). The reaction is typically performed at low temperature, in a solvent such as, but not limited to, N,N-dimethylformamide. (2S,3R,4S,5S,6R)-2-(2-(3-((((9H-Fluoren-9-yl)methoxy)carbonyl)amino)propanamido)-4-((((4-nitrophenoxy)carbonyl)oxy)methyl)phenoxy)-6-(acetoxymethyl)tetrahydro-2H-pyran-3,4,5-triyl triacetate (147) can be reacted with compound (88) in the presence of a base such as, but not limited to N,N-diisopropylethylamine, followed by treatment with lithium hydroxide, to provide compound (148). The first step is typically performed at low temperature, in a solvent such as, but not limited to, N,N-dimethylformamide, and the second step is typically performed at ambient temperature, in a solvent such as, but not limited to, methanol. Compound (148) can be treated with compound (84), wherein Sp is a spacer, in the presence of a base, such as, but not limited to N,N-diisopropylethylamine, to provide compound (149). The reaction is typically performed at ambient temperature, in a solvent such as, but not limited to, N,N-dimethylformamide.

### 5.3 General Methods for Synthesizing Anti-EGFR ADCs

The present disclosure also discloses a process to prepare an anti-EGFR ADC according to structural formula (I): 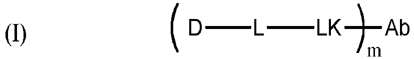 wherein D, L, LK, Ab and m are as defined in the Detailed Description section. The process comprises:
treating an antibody in an aqueous solution with an effective amount of a disulfide reducing agent at 30-40 °C for at least 15 minutes, and then cooling the antibody solution to 20-27 °C;
adding to the reduced antibody solution a solution of water/dimethyl sulfoxide comprising a synthon selected from the group of 2.1 to 2.176 (Table 5);
adjusting the pH of the solution to a pH of 7.5 to 8.5; and
allowing the reaction to run for 48 to 80 hours to form the ADC,
wherein the mass is shifted by 18 ± 2 amu for each hydrolysis of a succinimide to a succinamide as measured by electron spray mass spectrometry; and
wherein the ADC is optionally purified by hydrophobic interaction chromatography.

In certain embodiments, the antibody is the hEGFR antibody, wherein the hEGFR antibody comprises the heavy and light chain CDRs of AbA; AbB; AbG; and AbK.

The present disclosure is also directed to an anti-EGFR ADC prepared by the above-described process.

In certain embodiments, the anti-EGFR ADC disclosed in the present application is formed by contacting an antibody that binds an hEGFR cell surface receptor or tumor associated antigen expressed on a tumor cell with a drug-linker synthon under conditions in which the drug-linker synthon covalently links to the antibody through a maleimide moiety as shown in formulae (IIe) and (IIf), or through an acetyl halide as shown in(IIg), or through a vinyl sulfone as shown in (IIh). 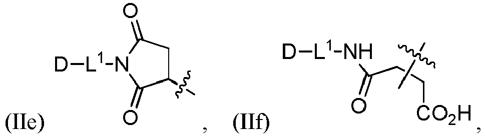 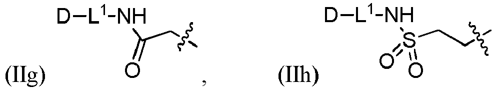 wherein D is the Bcl-xL inhibitor drug according to structural formula (IIa), (IIb), (IIc) or (IId) as described above and L¹ is the portion of the linker not formed from the maleimide, acetyl halide or vinyl sulfone upon attachment of the synthon to the antibody; and wherein the drug-linker synthon is selected from the group consisting of synthon examples 2.1 to 2.176 (Table 5), or a pharmaceutically acceptable salt thereof.

In certain embodiments, the contacting step is carried out under conditions such that the anti-EGFR ADC has a DAR of 2, 3 or 4.

### 6. Purification of Anti-EGFR ADCs

Purification of the ADCs may be achieved in such a way that ADCs having certain DARs are collected. For example, HIC resin may be used to separate high drug loaded ADCs from ADCs having optimal drug to antibody ratios (DARs), *e.g.* a DAR of 4 or less. In one embodiment, a hydrophobic resin is added to an ADC mixture such that undesired ADCs, *i.e.*, higher drug loaded ADCs, bind the resin and can be selectively removed from the mixture. In certain embodiments, separation of the ADCs may be achieved by contacting an ADC mixture (*e.g.*, a mixture comprising a drug loaded species of ADC of 4 or less and a drug loaded species of ADC of 6 or more) with a hydrophobic resin, wherein the amount of resin is sufficient to allow binding of the drug loaded species which is being removed from the ADC mixture. The resin and ADC mixture are mixed together, such that the ADC species being removed (*e.g.*, a drug loaded species of 6 or more) binds to the resin and can be separated from the other ADC species in the ADC mixture. The amount of resin used in the method is based on a weight ratio between the species to be removed and the resin, where the amount of resin used does not allow for significant binding of the drug loaded species that is desired. Thus, methods may be used to reduce the average DAR to less than 4. Further, the purification methods described herein may be used to isolate ADCs having any desired range of drug loaded species, *e.g.*, a drug loaded species of 4 or less, a drug loaded species of 3 or less, a drug loaded species of 2 or less, a drug loaded species of 1 or less.

Certain species of molecule(s) binds to a surface based on hydrophobic interactions between the species and a hydrophobic resin. In one embodiment, method of the disclosure refers to a purification process that relies upon the intermixing of a hydrophobic resin and a mixture of ADCs, wherein the amount of resin added to the mixture determines which species (*e.g.*, ADCs with a DAR of 6 or more) will bind. Following production and purification of an antibody from an expression system (e.g., a mammalian expression system), the antibody is reduced and coupled to a drug through a conjugation reaction. The resulting ADC mixture often contains ADCs having a range of DARs, *e.g.*, 1 to 8. In one embodiment, the ADC mixture comprises a drug loaded species of 4 or less and a drug loaded species of 6 or more. According to the methods of the disclosure, the ADC mixture may be purified using a process, such as, but not limited to, a batch process, such that ADCs having a drug loaded species of 4 or less are selected and separated from ADCs having a higher drug load (e.g., ADCs having a drug loaded species of 6 or more). Notably, the purification methods described herein may be used to isolate ADCs having any desired range of DAR, *e.g.*, a DAR of 4 or less, a DAR of 3 or less, a DAR of 2 or less.

Thus, in one embodiment, an ADC mixture comprising a drug loaded species of 4 or less and a drug loaded species of 6 or more may be contacted with a hydrophobic resin to form a resin mixture, wherein the amount of hydrophobic resin contacted with the ADC mixture is sufficient to allow binding of the drug loaded species of 6 or more to the resin but does not allow significant binding of the drug load species of 4 or less; and removing the hydrophobic resin from the ADC mixture, such that the composition comprising ADCs is obtained, wherein the composition comprises less than 15% of the drug loaded species of 6 or more, and wherein the ADC comprises an antibody conjugated to a Bcl-xL inhibitor. In a separate embodiment, the method of the disclosure comprises contacting an ADC mixture comprising a drug loaded species of 4 or less and a drug loaded species of 6 or more with a hydrophobic resin to form a resin mixture, wherein the amount of hydrophobic resin contacted with the ADC mixture is sufficient to allow binding of the drug loaded species of 6 or more to the resin but does not allow significant binding of the drug load species of 4 or less; and removing the hydrophobic resin from the ADC mixture, such that the composition comprising ADCs is obtained, wherein the composition comprises less than 15% of the drug loaded species of 6 or more, and wherein the ADC comprises an antibody conjugated to a Bcl-xL inhibitor, wherein the hydrophobic resin weight is 3 to 12 times the weight of the drug loaded species of 6 or more in the ADC mixture.

The ADC separation method described herein method may be performed using a batch purification method. The batch purification process generally includes adding the ADC mixture to the hydrophobic resin in a vessel, mixing, and subsequently separating the resin from the supernatant. For example, in the context of batch purification, a hydrophobic resin may be prepared in or equilibrated to the desired equilibration buffer. A slurry of the hydrophobic resin may thus be obtained. The ADC mixture may then be contacted with the slurry to adsorb the specific species of ADC(s) to be separated by the hydrophobic resin. The solution comprising the desired ADCs that do not bind to the hydrophobic resin material may then be separated from the slurry, e.g., by filtration or by allowing the slurry to settle and removing the supernatant. The resulting slurry can be subjected to one or more washing steps. In order to elute bound ADCs, the salt concentration can be decreased. In one embodiment, the process used in the disclosure includes no more than 50 g of hydrophobic resin.

Thus, a batch method may be used to contact an ADC mixture comprising a drug loaded species of 4 or less and a drug loaded species of 6 or more with a hydrophobic resin to form a resin mixture, wherein the amount of hydrophobic resin contacted with the ADC mixture is sufficient to allow binding of the drug loaded species of 6 or more to the resin but does not allow significant binding of the drug load species of 4 or less; and removing the hydrophobic resin from the ADC mixture, such that the composition comprising ADCs is obtained, wherein the composition comprises less than 15% of the drug loaded species of 6 or more, and wherein the ADC comprises an antibody conjugated to a Bcl-xL inhibitor. In a separate embodiment, a batch method is used to contact an ADC mixture comprising a drug loaded species of 4 or less and a drug loaded species of 6 or more with a hydrophobic resin to form a resin mixture, wherein the amount of hydrophobic resin contacted with the ADC mixture is sufficient to allow binding of the drug loaded species of 6 or more to the resin but does not allow significant binding of the drug load species of 4 or less; and removing the hydrophobic resin from the ADC mixture, such that the composition comprising ADCs is obtained, wherein the composition comprises less than 15% of the drug loaded species of 6 or more, and wherein the ADC comprises an antibody conjugated to a Bcl-xL inhibitor, wherein the hydrophobic resin weight is 3 to 12 times the weight of the drug loaded species of 6 or more in the ADC mixture.

Alternatively, in a separate embodiment, purification may be performed using a circulation process, whereby the resin is packed in a container and the ADC mixture is passed over the hydrophobic resin bed until the specific species of ADC(s) to be separated have been removed. The supernatant (containing the desired ADC species) is then pumped from the container and the resin bed may be subjected to washing steps.

A circulation process may be used to contact an ADC mixture comprising a drug loaded species of 4 or less and a drug loaded species of 6 or more with a hydrophobic resin to form a resin mixture, wherein the amount of hydrophobic resin contacted with the ADC mixture is sufficient to allow binding of the drug loaded species of 6 or more to the resin but does not allow significant binding of the drug load species of 4 or less; and removing the hydrophobic resin from the ADC mixture, such that the composition comprising ADCs is obtained, wherein the composition comprises less than 15% of the drug loaded species of 6 or more, and wherein the ADC comprises an antibody conjugated to a Bcl-xL inhibitor. In a separate embodiment, a circulation process is used to contact an ADC mixture comprising a drug loaded species of 4 or less and a drug loaded species of 6 or more with a hydrophobic resin to form a resin mixture, wherein the amount of hydrophobic resin contacted with the ADC mixture is sufficient to allow binding of the drug loaded species of 6 or more to the resin but does not allow significant binding of the drug load species of 4 or less; and removing the hydrophobic resin from the ADC mixture, such that the composition comprising ADCs is obtained, wherein the composition comprises less than 15% of the drug loaded species of 6 or more, and wherein the ADC comprises an antibody conjugated to a Bcl-xL inhibitor, wherein the hydrophobic resin weight is 3 to 12 times the weight of the drug loaded species of 6 or more in the ADC mixture.

Alternatively, a flow through process may be used to purify an ADC mixture to arrive at a composition comprising a majority of ADCs having a certain desired DAR. In a flow through process, resin is packed in a container, *e.g.*, a column, and the ADC mixture is passed over the packed resin such that the desired ADC species does not substantially bind to the resin and flows through the resin, and the undesired ADC species is bound to the resin. A flow through process may be performed in a single pass mode (where the ADC species of interest are obtained as a result of a single pass through the resin of the container) or in a multi-pass mode (where the ADC species of interest are obtained as a result of multiple passes through the resin of the container). The flow through process is performed such that the weight of resin selected binds to the undesired ADC population, and the desired ADCs (e.g., DAR 2-4) flow over the resin and are collected in the flow through after one or multiple passes.

A flow through process may be used to contact an ADC mixture comprising a drug loaded species of 4 or less and a drug loaded species of 6 or more with a hydrophobic resin, wherein the amount of hydrophobic resin contacted with the ADC mixture is sufficient to allow binding of the drug loaded species of 6 or more to the resin but does not allow significant binding of the drug load species of 4 or less, where the drug load species of 4 or less passes over the resin and is subsequently collected after one or multiple passes, such that the composition comprising the desired ADCs (e.g. DAR 2-4) is obtained, wherein the composition comprises less than 15% of the drug loaded species of 6 or more, and wherein the ADC comprises an antibody conjugated to a Bcl-xL inhibitor. In a separate embodiment, a flow through process is used to contact an ADC mixture comprising a drug loaded species of 4 or less and a drug loaded species of 6 or more with a hydrophobic resin by passing the ADC mixture over the resin, wherein the amount of hydrophobic resin contacted with the ADC mixture is sufficient to allow binding of the drug loaded species of 6 or more to the resin but does not allow significant binding of the drug load species of 4 or less, where the drug load species of 4 or less passes over the resin and is subsequently collected, such that the composition comprising ADCs is obtained, wherein the composition comprises less than 15% of the drug loaded species of 6 or more, and wherein the ADC comprises an antibody conjugated to a Bcl-xL inhibitor, wherein the amount of hydrophobic resin weight is 3 to 12 times the weight of the drug loaded species of 6 or more in the ADC mixture.

Following a flow through process, the resin may be washed with a one or more washes following in order to further recover ADCs having the desired DAR range (found in the wash filtrate). For example, a plurality of washes having decreasing conductivity may be used to further recover ADCs having the DAR of interest. The elution material obtained from the washing of the resin may be subsequently combined with the filtrate resulting from the flow through process for improved recovery of ADCs having the DAR of interest.

The aforementioned batch, circulation, and flow through process purification methods are based on the use of a hydrophobic resin to separate high vs. low drug loaded species of ADC. Hydrophobic resin comprises hydrophobic groups which interact with the hydrophobic properties of the ADCs. Hydrophobic groups on the ADC interact with hydrophobic groups within the hydrophobic resin. The more hydrophobic a protein is the stronger it will interact with the hydrophobic resin.

Hydrophobic resin normally comprises a base matrix (e.g., cross-linked agarose or synthetic copolymer material) to which hydrophobic ligands (e.g., alkyl or aryl groups) are coupled. Many hydrophobic resins are available commercially. Examples include, but are not limited to, Phenyl Sepharose^{™} 6 Fast Flow with low or high substitution (Pharmacia LKB Biotechnology, AB, Sweden); Phenyl Sepharose^{™} High Performance (Pharmacia LKB Biotechnology, AB, Sweden); Octyl Sepharose^{™} High Performance (Pharmacia LKB Biotechnology, AB, Sweden); Fractogel^{™} EMD Propyl or Fractogel^{™} EMD Phenyl columns (E. Merck, Germany); Macro-Prep^{™} Methyl or Macro-Prep^{™}. t-Butyl Supports (Bio-Rad, California); WP HI-Propyl (C₃)^{™} (J. T. Baker, New Jersey); and Toyopearl^{™} ether, hexyl, phenyl or butyl (TosoHaas, PA). In one embodiment, the hydrophobic resin is a butyl hydrophobic resin. In another embodiment, the hydrophobic resin is a phenyl hydrophobic resin. In another embodiment, the hydrophobic resin is a hexyl hydrophobic resin, an octyl hydrophobic resin, or a decyl hydrophobic resin. In one embodiment, the hydrophobic resin is a methacrylic polymer having n-butyl ligands (*e.g.* TOYOPEARL Butyl-600M).

Further methods for purifying ADC mixtures to obtain a composition having a desired DAR are described in U.S. Application No. 14/210,602 (U.S. Patent Appln. Publication No. US 2014/0286968).

In certain embodiments of the disclosure, ADCs described herein having a DAR2 are purified from ADCs having higher or lower DARs. Such purified DAR2 ADCs are referred to herein as "E2". Purification methods for achieving a composition having E2 anti-EGFR ADCs. In one embodiment, of the disclosure provides a composition comprising an ADC mixture, wherein at least 75% of the ADCs are anti-EGFR ADCs (like those described herein) having a DAR2. In another embodiment, the disclosure provides a composition comprising an ADC mixture, wherein at least 80% of the ADCs are anti-EGFR ADCs (like those described herein) having a DAR2. In another embodiment, the disclosure provides a composition comprising an ADC mixture, wherein at least 85% of the ADCs are anti-EGFR ADCs (like those described herein) having a DAR2. In another embodiment, the disclosure provides a composition comprising an ADC mixture, wherein at least 90% of the ADCs are anti-EGFR ADCs (like those described herein) having a DAR2.

### 7. Uses of Anti-EGFR ADCs

The Bcl-xL inhibitors included in the ADCs, as well as the synthons delivered by the ADCs, inhibit Bcl-xL activity and induce apoptosis in cells expressing Bcl-xL. Accordingly, the Bcl-xL inhibitors and/or ADCs may be used in methods to inhibit Bcl-xL activity and/or induce apoptosis in cells.

For Bcl-xL inhibitors, the method generally involves contacting a cell whose survival depends, at least in part, upon Bcl-xL expression with an amount of a Bcl-xL inhibitor sufficient to inhibit Bcl-xL activity and/or induce apoptosis. For ADCs, the method generally involves contacting a cell whose survival depends, at least in part upon Bcl-xL expression, and that expresses a cell-surface antigen, *i.e.,* EGFR, for the antibody of the ADC with an ADC under conditions in which the ADC binds the antigen.

In certain embodiments, the antibody of the ADC binds EGFR and facilitates internalization of the ADC into the cell, where the Bcl-xL inhibitory synthon is delivered. The method may be carried out *in vitro* in a cellular assay to inhibit Bcl-xL activity and/or inhibit apoptosis, or *in vivo* as a therapeutic approach towards treating diseases in which inhibition of apoptosis and/or induction of apoptosis would be desirable.

Dysregulated apoptosis has been implicated in a variety of diseases, including, for example, autoimmune disorders (*e.g*., systemic lupus erythematosus, rheumatoid arthritis, graft-versus-host disease, myasthenia gravis, or Sjogren's syndrome), chronic inflammatory conditions *(e.g.,* psoriasis, asthma or Crohn's disease), hyperproliferative disorders *(e.g.,* breast cancer, lung cancer), viral infections (*e.g.*, herpes, papilloma, or HIV), and other conditions, such as osteoarthritis and atherosclerosis. The Bcl-xL inhibitor or ADCs described herein may be used to treat or ameliorate any of these diseases. Such treatments generally involve administering to a subject suffering from the disease an amount of a Bcl-xL inhibitor or ADC described herein sufficient to provide therapeutic benefit. For ADCs, identity of the antibody of the ADC administered will depend upon the disease being treated - to the antibody should bind a cell-surface antigen expressed in the cell type where inhibition of Bcl-xL activity would be beneficial. The therapeutic benefit achieved will also depend upon the specific disease being treated. In certain instances, the Bcl-xL inhibitor or ADC may treat or ameliorate the disease itself, or symptoms of the disease, when administered as monotherapy. In other instances, the Bcl-xL inhibitor or ADC may be part of an overall treatment regimen including other agents that, together with the inhibitor or ADC, treat or ameliorate the disease being treated, or symptoms of the disease. Agents useful to treat or ameliorate specific diseases that may be administered adjunctive to, or with, the Bcl-xL inhibitors and/or ADCs described herein will be apparent to those of skill in the art.

Although absolute cure is always desirable in any therapeutic regimen, achieving a cure is not required to provide therapeutic benefit. Therapeutic benefit may include halting or slowing the progression of the disease, regressing the disease without curing, and/or ameliorating or slowing the progression of symptoms of the disease. Prolonged survival as compared to statistical averages and/or improved quality of life may also be considered therapeutic benefit. One particular class of diseases that involve dysregulated apoptosis and that are significant health burden world-wide are cancers. In a specific embodiment, the Bcl-xL inhibitors and/or ADCs described herein may be used to treat cancers. The cancer may be, for example, solid tumors or hematological tumors. Cancers that may be treated with the ADCs described herein include, but are not limited to include, but are not limited to bladder cancer, brain cancer, breast cancer, bone marrow cancer, cervical cancer, chronic lymphocytic leukemia, colorectal cancer, esophageal cancer, hepatocellular cancer, lymphoblastic leukemia, follicular lymphoma, lymphoid malignancies of T-cell or B-cell origin, melanoma, myelogenous leukemia, myeloma, oral cancer, ovarian cancer, non-small cell lung cancer, chronic lymphocytic leukemia, myeloma, prostate cancer, or spleen cancer. ADCs may be especially beneficial in the treatment of cancers because the antibody can be used to target the Bcl-xL inhibitory synthon specifically to tumor cells, thereby potentially avoiding or ameliorating undesirable side-effects and/or toxicities that may be associated with systemic administration of unconjugated inhibitors. One embodiment pertains to a use of the claimed ADC in a method of treating a disease involving dysregulated intrinsic apoptosis, comprising administering to a subject having a disease involving dysregulated apoptosis an amount of an ADC described herein effective to provide therapeutic benefit, wherein the antibody of the ADC binds a cell surface receptor on a cell whose intrinsic apoptosis is dysregulated. One embodiment pertains to a use of the claimed ADC in a method of treating cancer, comprising administering to a subject having cancer an ADC described and claimed herein that is capable of binding a cell surface receptor or a tumor associated antigen expressed on the surface of the cancer cells, in an amount effective to provide therapeutic benefit.

In the context of tumorigenic cancers, therapeutic benefit, in addition to including the effects discussed above, may also specifically include halting or slowing progression of tumor growth, regressing tumor growth, eradicating one or more tumors and/or increasing patient survival as compared to statistical averages for the type and stage of the cancer being treated. In one embodiment, the cancer being treated is a tumorigenic cancer.

The ADCs of the disclosure are capable of neutralizing human EGFR activity both *in vivo* and *in vitro.* Accordingly, such ADCs of the disclosure can be used to inhibit hEGFR activity, *e.g.,* in a cell culture containing hEGFR, in human subjects or in other mammalian subjects having EGFR with which an antibody of the disclosure cross-reacts. In one embodiment, the disclosure provides a method for inhibiting hEGFR activity comprising contacting hEGFR with an antibody or antibody portion of the disclosure such that hEGFR activity is inhibited. For example, in a cell culture containing, or suspected of containing hEGFR, an antibody or antibody portion of the disclosure can be added to the culture medium to inhibit hEGFR activity in the culture.

In another embodiment, the disclosure features a method for reducing hEGFR activity in a subject, advantageously from a subject suffering from a disease or disorder in which EGFR activity is detrimental. The disclosure provides methods for reducing EGFR activity in a subject suffering from such a disease or disorder, which method comprises administering to the subject an ADC of the disclosure such that EGFR activity in the subject is reduced. Preferably, the EGFR is human EGFR, and the subject is a human subject. Alternatively, the subject can be a mammal expressing an EGFR to which ADCs of the disclosure are capable of binding. Still further the subject can be a mammal into which EGFR has been introduced (*e.g.,* by administration of EGFR or by expression of an EGFR transgene). ADCs of the disclosure can be administered to a human subject for therapeutic purposes. Moreover, ADCs of the disclosure can be administered to a non-human mammal expressing an EGFR with which the antibody is capable of binding for veterinary purposes or as an animal model of human disease. Regarding the latter, such animal models may be useful for evaluating the therapeutic efficacy of antibodies of the disclosure (*e.g.,* testing of dosages and time courses of administration).

As used herein, the term "a disorder in which EGFR activity is detrimental" is intended to include diseases and other disorders in which the presence of EGFR in a subject suffering from the disorder has been shown to be or is suspected of being either responsible for the pathophysiology of the disorder or a factor that contributes to a worsening of the disorder. Accordingly, a disorder in which EGFR activity is detrimental is a disorder in which reduction of EGFR activity is expected to alleviate the symptoms and/or progression of the disorder. Such disorders may be evidenced, for example, by an increase in the concentration of EGFR in a biological fluid of a subject suffering from the disorder *(e.g.,* an increase in the concentration of EGFR in a tumor, serum, plasma, synovial fluid, *etc.* of the subject), which can be detected, for example, using an anti-EGFR antibody as described above. Non-limiting examples of disorders that can be treated with the ADCs of the disclosure, for example, an ADC comprising AbA, include those disorders discussed below. For example, suitable disorders include, but are not limited to, a variety of cancers including, but not limited to, breast cancer, lung cancer, a glioma, prostate cancer, pancreatic cancer, colon cancer, head and neck cancer, and kidney cancer. Other examples of cancer that may be treated using the compositions and methods disclosed herein include squamous cell carcinoma (*e.g.*, squamous lung cancer or squamous head and neck cancer), triple negative breast cancer, non-small cell lung cancer, colorectal cancer, and mesothelioma. In one embodiment, the ADCs disclosed herein are used to treat a solid tumor, *e.g.*, inhibit growth of or decrease size of a solid tumor, overexpressing EGFR or which is EGFR positive. In one embodiment, the disclosure is directed to the treatment of EGFR amplified squamous lung cancer. In one embodiment, the ADCs claimed herein are used to treat EGFR amplified squamous head and neck cancer. In another embodiment, the ADCs disclosed herein are used to treat triple negative breast cancer (TNBC). Diseases and disorders described herein may be treated by anti-EGFR ADCs of the disclosure, as well as pharmaceutical compositions comprising such anti-EGFR ADCs.

In certain embodiments, the cancer may be characterized as having EGFR overexpression.

In other embodiments, the cancer is characterized as having an activating EGFR mutation, e.g. a mutation(s) that activates the EGFR signaling pathway and/or mutation(s) that lead to overexpression of the EGFR protein. In specific exemplary embodiments, the activating EGFR mutation may be a mutation in the EGFR gene. In particular embodiments, the activating EGFR mutation is an exon 19 deletion mutation, a single-point substitution mutation L858R in exon 21, a T790M point mutation, and/or combinations thereof.

In certain embodiments, the ADCs disclosed herein are administered to a subject in need thereof in order to treat advanced solid tumor types likely to exhibit elevated levels of Epidermal Growth Factor Receptor (EGFR). Examples of such tumors include, but are not limited to, head and neck squamous cell carcinoma, non-small cell lung cancer, triple negative breast cancer, colorectal carcinoma, and glioblastoma multiforme.

In certain embodiments, the disclosure includes a method for inhibiting or decreasing solid tumor growth in a subject having a solid tumor, said method comprising administering an anti-EGFR ADC claimed herein, to the subject having the solid tumor, such that the solid tumor growth is inhibited or decreased. In certain embodiments, the solid tumor is a non-small cell lung carcinoma or a glioblastoma. In further embodiments, the solid tumor is an EGFRvIII positive tumor or an EGFR-expressing solid tumors. In further embodiments, the solid tumor is an EGFR amplified solid tumor or an EGFR overexpressing solid tumors. In certain embodiments the anti-EGFR ADCs claimed herein are administered to a subject having glioblastoma multiforme, alone or in combination with an additional agent, *e.g.*, radiation and/or temozolomide.

In certain embodiments, the disclosure includes a method for inhibiting or decreasing solid tumor growth in a subject having a solid tumor which was identified as an EGFR expressing or EGFR overexpressing tumor (or an EGFRvIII expressing tumor), said method comprising administering an anti-EGFR ADC claimed herein, to the subject having the solid tumor, such that the solid tumor growth is inhibited or decreased. Methods for identifying EGFR expressing tumors (e.g., EGFR overexpressing tumors) are known in the art, and include FDA-approved tests and validation assays. For example, the EGFR pharmDx^{™} assay (Dako North America, Inc.) is a qualitative immunohistochemical (IHC) kit system used to identify EGFR expression in normal and neoplastic tissues routinely-fixed for histological evaluation. EGFR pharmDx specifically detects the EGFR (HER1) protein in EGFR-expressing cells. In addition, PCR-based assays may also be used for identifying EGFR overexpressing tumors. For example, these assays may use primers that are specific for the variant EGFR gene (e.g., SEQ ID NO: 33) and/or cDNA and result in the amplification of the EGFR gene/cDNA, or a portion thereof. The amplified PCR products may be subsequently analyzed, for example, by gel electrophoresis using standard methods known in the art to determine the size of the PCR products. Such tests may be used to identify tumors that may be treated with the methods and compositions claimed herein.

Any of the methods for gene therapy available in the art can be used according to the disclosure. For general reviews of the methods of gene therapy, see Goldspiel et al., 1993, Clinical Pharmacy 12:488-505; Wu and Wu, 1991, Biotherapy 3:87-95; Tolstoshev, 1993, Ann. Rev. Pharmacol. Toxicol. 32:573-596; Mulligan, Science 260:926- 932 (1993); and Morgan and Anderson, 1993, Ann. Rev. Biochem. 62:191-217; May, 1993, TIBTECH 11(5):155-215. Methods commonly known in the art of recombinant DNA technology which can be used are described in Ausubel et al. (eds.), Current Protocols in Molecular Biology, John Wiley &Sons, NY (1993); and Kriegler, Gene Transfer and Expression, A Laboratory Manual, Stockton Press, NY (1990). Detailed description of various methods of gene therapy is provided in US20050042664 A1.

In another aspect, the claimed ADC can be used a method of treating (e.g., curing, suppressing, ameliorating, delaying or preventing the onset of, or preventing recurrence or relapse of) or preventing a EGFR-associated disorder, in a subject. The method includes: administering to the subject an EGFR binding agent (particularly an antagonist), *e.g*., an anti-EGFR antibody or fragment thereof as claimed herein, in an amount sufficient to treat or prevent the EGFR-associated disorder. The EGFR antagonist, *e.g.,* the anti-EGFR antibody or fragment thereof, can be administered to the subject, alone or in combination with other therapeutic modalities as described herein.

ADCs of the disclosure can be used alone or in combination to treat such diseases. It should be understood that the ADCs of the disclosure can be used alone or in combination with an additional agent, *e.g.,* a therapeutic agent, said additional agent being selected by the skilled artisan for its intended purpose. For example, the additional agent can be a therapeutic agent art-recognized as being useful to treat the disease or condition being treated by the ADC of the disclosure. The additional agent also can be an agent that imparts a beneficial attribute to the therapeutic composition, *e.g.,* an agent which affects the viscosity of the composition.

It should further be understood that the combinations which are to be included within this disclosure are those combinations useful for their intended purpose. The agents set forth below are illustrative for purposes and not intended to be limited. The combinations, which are part of this disclosure, can be the antibodies of the disclosure and at least one additional agent selected from the lists below. The combination can also include more than one additional agent, *e.g.*, two or three additional agents if the combination is such that the formed composition can perform its intended function.

The combination therapy can include anti-EGFR antagonists ADCs of the disclosure formulated with, and/or co-administered with, one or more additional therapeutic agents, *e.g.,* one or more cytokine and growth factor inhibitors, immunosuppressants, anti-inflammatory agents (*e.g.,* systemic anti-inflammatory agents), anti-fibrotic agents, metabolic inhibitors, enzyme inhibitors, and/or cytotoxic or cytostatic agents, mitotic inhibitors, antitumor antibiotics, immunomodulating agents, vectors for gene therapy, alkylating agents, antiangiogenic agents, antimetabolites, boron-containing agents, chemoprotective agents, hormones, antihormone agents, corticosteroids, photoactive therapeutic agents, oligonucleotides, radionuclide agents, topoisomerase inhibitors, kinase inhibitors, or radiosensitizers, as described in more herein.

In a particular embodiment, the anti-EGFR ADCs claimed herein, are used in combination with an anti-cancer agent or an antineoplastic agent. The terms "anti-cancer agent" and "antineoplastic agent" refer to drugs used to treat malignancies, such as cancerous growths. Drug therapy may be used alone, or in combination with other treatments such as surgery or radiation therapy. Several classes of drugs may be used in cancer treatment, depending on the nature of the organ involved. For example, breast cancers are commonly stimulated by estrogens, and may be treated with drugs which inactive the sex hormones. Similarly, prostate cancer may be treated with drugs that inactivate androgens, the male sex hormone. Anti-cancer agents that may be used in conjunction with the anti-EGFR ADCs of the disclosure include, among others, the following agents:

| **Anti-Cancer Agent** | **Comments** | **Examples** |
|---|---|---|
| Antibodies | Antibodies which bind IGF-1R (insulin-like growth factor type 1 receptor), which is expressed on the cell surface of most human cancers | A12 (fully humanized mAb) |
| (a) antibodies other than anti-EGFR antibodies; and | | 19D12 (fully humanized mAb) |
| | | Cp751-871 (fully humanized mAb) |
| | | H7C10 (humanized mAb) |
| (b) anti-EGFR antibodies which bind different epitopes | | alphaIR3 (mouse) |
| | | ScFV/FC (mouse/human chimera) |
| | | EM/164 (mouse) |
| | Antibodies which bind EGFR (epidermal growth factor receptor); Mutations affecting EGFR expression or activity could result in cancer | |
| | | Matuzumab (EMD72000) |
| | | Erbitux^{®} / Cetuximab (Imclone) |
| | | Vectibix^{®} / Panitumumab (Amgen) |
| | | mAb 806 |
| | | Nimotuxumab (TheraCIM) |
| | Antibodies which bind cMET (Mesenchymal epithelial transition factor); a member of the MET family of receptor tyrosine kinases) | AVEO (AV299) (AVEO) |
| | | AMG102 (Amgen) |
| | | 5D5 (OA-5d5) (Genentech) |
| | | H244G11 (Pierre Fabre) |
| | Anti-ErbB3 antibodies which bind different epitopes | |
| | | Ab #14 (MM 121-14) |
| | | Herceptin^{®} (Trastuzumab; Genentech) |
| | | 1B4C3; 2D1D12 (U3 Pharma AG) |
| Small Molecules | Insulin-like growth factor type 1 receptor which is expressed on the cell surface of many human cancers | NVP-AEW541-A |
| Targeting IGF1R | | BMS-536,924 (1H-benzoimidazol-2-yl)-1H-pyridin-2-one) |
| | | BMS-554,417 |
| | | Cycloligan |
| | | TAE226 |
| | | PQ401 |
| Small Molecules | EGFR (epidermal growth factor receptor); | Iressa^{®} / Gefitinib (AstraZeneca) |
| Targeting EGFR | | CI-1033 (PD 183805) (Pfizer) |
| | Overexpression or mutations affecting EGFR expression or activity could result in cancer | Lapatinib (GW-572016) (GlaxoSmithKline) |
| | | Tykerb^{®} / Lapatinib Ditosylate (Smith Kline Beecham) |
| | | Tarceva ^{®} / Erlotinib HCL (OSI-774) (OSI Pharma) |
| | | PKI-166 (Novartis) |
| | | PD-158780 |
| | | EKB-569 |
| | | Tyrphostin AG 1478 (4-(3-Chloroanillino)-6,7-dimethoxyquinazoline) |
| Small Molecules | cMET (Mesenchymal epithelial transition factor); | PHA665752 |
| Targeting cMET | | ARQ 197 |
| | a member of the MET family of receptor tyrosine kinases) | |
| Antimetabolites | | Flourouracil (5-FU) |
| | | Capecitabine / XELODA^{®} (HLR Roche) |
| | | 5-Trifluoromethyl-2'-deoxyuridine |
| | | Methotrexate sodium (Trexall) (Barr) |
| | | Raltitrexed/ Tomudex^{®} (AstraZeneca) |
| | | Pemetrexed / Alimta^{®} (Lilly) |
| | | Tegafur |
| | | Cytosine Arabinoside (Cytarabine, Ara-C) / Thioguanine^{®} (GlaxoSmithKline) |
| | | 5 -azacytidine |
| | | 6-mercaptopurine (Mercaptopurine, 6-MP) |
| | | Azathioprine / Azasan^{®} (AAIPHARMA LLC) |
| | | 6-thioguanine (6-TG) / Purinethol^{®} (TEVA) |
| | | Pentostatin / Nipent^{®} (Hospira Inc.) |
| | | Fludarabine phosphate / Fludara^{®} (Bayer Health Care) |
| | | Cladribine (2-CdA, 2-chlorodeoxyadenosine) / Leustatin^{®} (Ortho Biotech) |
| Alkylating agents | An alkylating antineoplastic agent is an alkylating agent that attaches an alkyl group to DNA. Since cancer cells generally proliferate unrestrictively more than do healthy cells they are more sensitive to DNA damage, and alkylating agents are used clinically to treat a variety of tumors. | Ribonucleotide Reductase Inhibitor (RNR) |
| | | Cyclophosphamide / Cytoxan (BMS) |
| | | Neosar (TEVA) |
| | | Ifosfamide / Mitoxana^{®} (ASTA Medica) |
| | | Thiotepa (Bedford, Abraxis, Teva) |
| | | BCNU→ 1,3-bis(2-chloroethyl)-1-nitosourea CCNU→ 1, -(2-chloroethyl)-3-cyclohexyl-1-nitrosourea (methyl CCNU) |
| | | Hexamethylmelamine (Altretamine, HMM) / Hexalen^{®} (MGI Pharma Inc.) |
| | | Busulfan / Myleran (GlaxoSmithKline) |
| | | Procarbazine HCL/ Matulane (Sigma Tau Pharmaceuticals, Inc.) |
| | | Dacarbazine (DTIC) |
| | | Chlorambucil / Leukara^{®} (SmithKline Beecham) |
| | | Melphalan / Alkeran^{®} (GlaxoSmithKline) |
| | | Cisplatin (Cisplatinum, CDDP) / Platinol (Bristol Myers) |
| | | Carboplatin / Paraplatin (BMS) |
| | | Oxaliplatin /Eloxitan^{®} (Sanofi-Aventis US) |
| Topoisomerase inhibitors | Topoisomerase inhibitors are chemotherapy agents designed to interfere with the action of topoisomerase enzymes (topoisomerase I and II), which are enzymes that control the changes in DNA structure by catalyzing the breaking and rejoining of the | Doxorubicin HCL / Doxil^{®} (Alza) |
| | | Daunorubicin citrate / Daunoxome^{®} (Gilead) |
| | | Mitoxantrone HCL / Novantrone (EMD Serono) |
| | | Actinomycin D |
| | | Etoposide / Vepesid^{®} (BMS)/ Etopophos^{®} |
| | | (Hospira, Bedford, Teva Parenteral, Etc.) |
| | | Topotecan HCL / Hycamtin^{®} |
| | | (GlaxoSmithKline) |
| | | Teniposide (VM-26) / Vumon^{®} (BMS) |
| | phosphodiester backbone of DNA strands during the normal cell cycle. | Irinotecan HCL(CPT-11) / Camptosar^{®} (Pharmacia & Upjohn) |
| Microtubule targeting agents | Microtubules are one of the components of the cytoskeleton. They have diameter of ~24 nm and length varying from several micrometers to possibly millimeters in axons of nerve cells. Microtubules serve as structural components within cells and are involved in many cellular processes including mitosis, cytokinesis, and vesicular transport. | Vincristine / Oncovin^{®} (Lilly) |
| | | Vinblastine sulfate / Velban^{®}(discontinued) (Lilly) |
| | | Vinorelbine tartrate / Navelbine^{®} (PierreFabre) |
| | | Vindesine sulphate / Eldisine^{®} (Lilly) |
| | | Paclitaxel / Taxol^{®} (BMS) |
| | | Docetaxel / Taxotere^{®} (Sanofi Aventis US) |
| | | Nanoparticle paclitaxel (ABI-007) / Abraxane^{®} (Abraxis BioScience, Inc.) |
| | | Ixabepilone / IXEMPRA^{™} (BMS) |
| Kinase inhibitors | Kinases are enzymes that catalyze the transfer of phosphate groups from high-energy, phosphate-donating molecules to specific substrates, and are utilized to transmit signals and regulate complex processes in cells. | Imatinib mesylate / Gleevec (Novartis) |
| | | Sunitinib malate / Sutent^{®} (Pfizer) |
| | | Sorafenib toslate / Nexavar^{®} (Bayer) |
| | | Nilotinib hydrochloride monohydrate / Tasigna^{®} (Novartis), Osimertinib, |
| | | Cobimetinib, Trametinib, Dabrafenib, Dinaciclib |
| Protein synthesis inhibitors | Induces cell apoptosis | L-asparaginase / Elspar^{®} (Merck & Co.) |
| Immunotherapeutic agents | Induces cancer patients to exhibit immune responsiveness | Alpha interferon |
| | | Angiogenesis Inhibitor / Avastin^{®} (Genentech) |
| | | IL-2→ Interleukin 2 (Aldesleukin) / Proleukin ^{®} (Chiron) |
| | | IL-12→ Interleukin 12 |
| | Antibody / small molecule immune checkpoint modulators | |
| | | Anti-CTLA-4 and PR-1 therapies |
| | | Yervoy^{®} (ipilimumab; Bristol-Myers Squibb) |
| | | Opdivo^{®} (nivolumab; Bristol-Myers Squibb) |
| | | Keytrada^{®} (pembrolizumab; Merck) |
| Hormones | Hormone therapies associated with menopause and aging seek to increase the amount of certain hormones in your body to compensate for age- or disease-related hormonal declines. Hormone therapy as a cancer treatment either reduces the level of specific hormones or alters the | Toremifene citrate / Fareston^{®} (GTX, Inc.) |
| | | Fulvestrant / Faslodex^{®} (AstraZeneca) |
| | | Raloxifene HCL / Evista^{®} (Lilly) |
| | | Anastrazole / Arimidex^{®} (AstraZeneca) |
| | | Letrozole / Femara^{®} (Novartis) |
| | | Fadrozole (CGS 16949A ) |
| | | Exemestane / Aromasin^{®} (Pharmacia & Upjohn) |
| | | Leuprolide acetate / Eligard^{®} (QTL USA) |
| | | Lupron^{®} (TAP Pharm) |
| | | Goserelin acetate / Zoladex^{®} (AstraZeneca) |
| | cancer's ability to use these hormones to grow and spread. | Triptorelin pamoate / Trelstar^{®} (Watson Labs) |
| | | Buserelin / Suprefact^{®} (Sanofi Aventis) |
| | | Nafarelin / Synarel^{®} (Pfizer) |
| | | Cetrorelix / Cetrotide^{®} (EMD Serono) |
| | | Bicalutamide / Casodex^{®} (AstraZeneca) |
| | | Nilutamide / Nilandron^{®} (Aventis Pharm.) |
| | | Megestrol acetate / Megace^{®} (BMS) |
| | | Somatostatin Analogs (Octreotide acetate / Sandostatin^{®} (Novartis) |
| Glucocorticoids | Anti-inflammatory drugs used to reduce swelling that causes cancer pain. | Prednisolone |
| | | Dexamethasone / Decadron^{®} (Wyeth) |
| Aromatose inhibitors | Includes imidazoles | Ketoconazole |
| mTOR inhibitors | the mTOR signaling pathway was originally discovered during studies of the immunosuppressive agent rapamycin. This highly conserved pathway regulates cell proliferation and metabolism in response to environmental factors, linking cell growth factor receptor signaling via phosphoinositide- 3-kinase(PI-3K) to cell growth, proliferation, and angiogenesis. | Sirolimus (Rapamycin) / Rapamune^{®} (Wyeth) |
| | | Temsirolimus (CCI-779) / Torisel^{®} (Wyeth) |
| | | Deforolimus (AP23573) / (Ariad Pharm.) |
| | | Everolimus (RAD00I) / Certican^{®} (Novartis) |

In addition to the above anti-cancer agents, the anti-EGFR ADCs claimed herein may be administered in combination with the agents described in section II. Further, the aforementioned anti-cancer agents may also be used in the ADCs of the disclosure.

In particular embodiments, the ADCs of the disclosure can be administered alone or with another anti-cancer agent which acts in conjunction with or synergistically with the antibody to treat the disease associated with EGFR activity. Such anti-cancer agents include, for example, agents well known in the art (e.g., cytotoxins, chemotherapeutic agents, small molecules and radiation). Examples of anti-cancer agents include, but are not limited to, Panorex (Glaxo-Welcome), Rituxan (IDEC/Genentech/Hoffman la Roche), Mylotarg (Wyeth), Campath (Millennium), Zevalin (IDEC and Schering AG), Bexxar (Corixa/GSK), Erbitux (Imclone/BMS), Avastin (Genentech) and Herceptin (Genentech/Hoffman la Roche). Other anti-cancer agents include, but are not limited to, those disclosed in U.S. Patent No. 7,598,028 and International Publication No. WO2008/100624. One or more anti-cancer agents may be administered either simultaneously or before or after administration of an antibody or antigen binding portion thereof of the disclosure.

In particular embodiments of the disclosure, the ADCs claimed herein can be used in a combination therapy with an inhibitor of NAMPT (see examples of inhibitors in US 2013/0303509; AbbVie, Inc.) to treat a subject in need thereof. NAMPT (also known as pre-B-cell-colony-enhancing factor (PBEF) and visfatin) is an enzyme that catalyzes the phosphoribosylation of nicotinamide and is the rate-limiting enzyme in one of two pathways that salvage NAD. In one embodiment of the disclosure, anti-EGFR antibodies and ADCs claimed herein are administered in combination with a NAMPT inhibitor for the treatment of cancer in a subject.

In particular embodiments of the disclosure, the ADCs claimed herein can be used in a combination therapy with SN-38, which is the active metabolite of the topoisomerase inhibitor irinotecan.

In other embodiments of the disclosure, the ADCs claimed herein can be used in a combination therapy with a PARP (poly ADP ribose polymerase) inhibitor, e.g.,veliparib, to treat cancer, including breast, ovarian and non-small cell lung cancers.

Further examples of additional therapeutic agents that can be co-administered and/or formulated with anti-EGFR ADCs claimed herein, include, but are not limited to, one or more of: inhaled steroids; beta-agonists, *e.g.*, short-acting or long- acting beta-agonists; antagonists of leukotrienes or leukotriene receptors; combination drugs such as ADVAIR; IgE inhibitors, *e.g.*, anti-IgE antibodies *(e.g.,* XOLAIR, omalizumab); phosphodiesterase inhibitors *(e.g.,* PDE4 inhibitors); xanthines; anticholinergic drugs; mast cell-stabilizing agents such as cromolyn; IL-4 inhibitors; IL-5 inhibitors; eotaxin/CCR3 inhibitors; antagonists of histamine or its receptors including H1, H2, H3, and H4, and antagonists of prostaglandin D or its receptors (DP1 and CRTH2). Such combinations can be used to treat, for example, asthma and other respiratory disorders. Other examples of additional therapeutic agents that can be co-administered and/or formulated with anti-EGFR ADCs claimed herein, include, but are not limited to, one or more of, temozolomide, ibrutinib, duvelisib, and idelalisib.

In certain embodiments, the ADC is administered in combination with an additional agent or an additional therapy, where the additional agent is selected from the group consisting of an anti-PD1 antibody (e.g. pembrolizumab), an anti-PD-Ll antibody (atezolizumab), an anti-CTLA-4 antibody (e.g. ipilimumab), a MEK inhibitor (e.g. trametinib), an ERK inhibitor, a BRAF inhibitor (e.g. dabrafenib), osimertinib, erlotinib, gefitinib, sorafenib, a CDK9 inhibitor (e.g. dinaciclib), a MCL-1 inhibitor, temozolomide, a Bcl-xL inhibitor, a Bcl-2 inhibitor (e.g. venetoclax), ibrutinib, a mTOR inhibitor (e.g. everolimus), a PI3K inhibitor (e.g. buparlisib), duvelisib, idelalisib, an AKT inhibitor, a HER2 inhibitor (e.g. lapatinib), a taxane (e.g. docetaxel, paclitaxel, nab-paclitaxel), an ADC comprising an auristatin, an ADC comprising a PBD (e.g. rovalpituzumab tesirine), an ADC comprising a maytansinoid (e.g. TDM1), a TRAIL agonist, a proteasome inhibitor (e.g. bortezomib), and a nicotinamide phosphoribosyltransferase (NAMPT) inhibitor, or in combination with auristatin ADCs or PBD ADCs.

Additional examples of therapeutic agents that can be co-administered and/or formulated with one or more anti-EGFR antibodies or fragments thereof include one or more of: TNF antagonists *(e.g.,* a soluble fragment of a TNF receptor, *e.g.,* p55 or p75 human TNF receptor or derivatives thereof, *e.g.,* 75 kD TNFR-IgG (75 kD TNF receptor-IgG fusion protein, ENBREL)); TNF enzyme antagonists, *e.g.*, TNF converting enzyme (TACE) inhibitors; muscarinic receptor antagonists; TGF-beta antagonists; interferon gamma; perfenidone; chemotherapeutic agents, *e.g.*, methotrexate, leflunomide, or a sirolimus (rapamycin) or an analog thereof, *e.g.*, CCI-779; COX2 and cPLA2 inhibitors; NSAIDs; immunomodulators; p38 inhibitors, TPL-2, MK-2 and NFkB inhibitors, among others.

Other preferred combinations are cytokine suppressive anti-inflammatory drug(s) (CSAIDs); antibodies to or antagonists of other human cytokines or growth factors, for example, IL-1, IL-2, IL-3, IL-4, IL-5, IL-6, IL-7, IL-8, IL-15, IL-16, IL-18, IL-21, IL-31, interferons, EMAP-II, GM-CSF, FGF, EGF, PDGF, and edothelin-1, as well as the receptors of these cytokines and growth factors. Antibodies of the disclosure, or antigen binding portions thereof, can be combined with antibodies to cell surface molecules such as CD2, CD3, CD4, CD8, CD25, CD28, CD30, CD40, CD45, CD69, CD80 (B7.1), CD86 (B7.2), CD90, CTLA, CTLA-4, PD-1, or their ligands including CD154 (gp39 or CD40L).

Preferred combinations of therapeutic agents may interfere at different points in the inflammatory cascade; preferred examples include TNF antagonists like chimeric, humanized or human TNF antibodies, adalimumab, (HUMIRA^{®}; D2E7; PCT Publication No. WO 97/29131 and U.S. Patent No. 6,090,382), CA2 (Remicade^{®}), CDP 571, and soluble p55 or p75 TNF receptors, derivatives, thereof, (p75TNFR1gG (Enbrel^{®}) or p55TNFR1gG (Lenercept), and also TNF converting enzyme (TACE) inhibitors; similarly IL-1 inhibitors (Interleukin-1-converting enzyme inhibitors, IL-1RA etc.) may be effective for the same reason. Other preferred combinations include Interleukin 4.

In certain embodiments, the ADC is administered in combination with a taxane to treat non small cell lung cancer.

The pharmaceutical compositions of the disclosure may include a "therapeutically effective amount" or a "prophylactically effective amount" of an antibody or antibody portion of the disclosure. A "therapeutically effective amount" refers to an amount effective, at dosages and for periods of time necessary, to achieve the desired therapeutic result. A therapeutically effective amount of the antibody or antibody portion may be determined by a person skilled in the art and may vary according to factors such as the disease state, age, sex, and weight of the individual, and the ability of the antibody or antibody portion to elicit a desired response in the individual. A therapeutically effective amount is also one in which any toxic or detrimental effects of the antibody, or antibody portion, are outweighed by the therapeutically beneficial effects. A "prophylactically effective amount" refers to an amount effective, at dosages and for periods of time necessary, to achieve the desired prophylactic result. Typically, since a prophylactic dose is used in subjects prior to or at an earlier stage of disease, the prophylactically effective amount will be less than the therapeutically effective amount.

The amount of ADC administered will depend upon a variety of factors, including but not limited to, the particular disease being treated, the mode of administration, the desired therapeutic benefit, the stage or severity of the disease, the age, weight and other characteristics of the patient, etc. Determination of effective dosages is within the capabilities of those skilled in the art.

Dosage regimens may be adjusted to provide the optimum desired response (e.g., a therapeutic or prophylactic response). For example, a single bolus may be administered, several divided doses may be administered over time or the dose may be proportionally reduced or increased as indicated by the exigencies of the therapeutic situation. It is especially advantageous to formulate parenteral compositions in dosage unit form for ease of administration and uniformity of dosage. Dosage unit form as used herein refers to physically discrete units suited as unitary dosages for the mammalian subjects to be treated; each unit containing a predetermined quantity of active compound calculated to produce the desired therapeutic effect in association with the required pharmaceutical carrier. The specification for the dosage unit forms of the disclosure are dictated by and directly dependent on (a) the unique characteristics of the active compound and the particular therapeutic or prophylactic effect to be achieved, and (b) the limitations inherent in the art of compounding such an active compound for the treatment of sensitivity in individuals.

An exemplary, non-limiting range for a therapeutically or prophylactically effective amount of an ADC, is 0.1-20 mg/kg, more preferably 1-10 mg/kg. In one embodiment, the dose of the ADCs claimed herein is 1 to 6 mg/kg, including the individual doses recited therein, *e.g.*, 1 mg/kg, 2 mg/kg, 3 mg/kg, 4 mg/kg, 5 mg/kg, and 6 mg/kg. In another embodiment, the dose of the ADCs claimed herein is 1 to 200 µg/kg, including the individual doses recited therein, *e.g.*, 1 µg/kg, 2 µg/kg, 3 µg/kg, 4 µg/kg, 5 µg/kg, 10 µg/kg, 20 µg/kg, 30 µg/kg, 40 µg/kg, 50 µg/kg, 60 µg/kg, 80 µg/kg, 100 µg/kg, 120 µg/kg, 140 µg/kg, 160 µg/kg, 180 µg/kg and 200 µg/kg, It is to be noted that dosage values may vary with the type and severity of the condition to be alleviated. It is to be further understood that for any particular subject, specific dosage regimens should be adjusted over time according to the individual need and the professional judgment of the person administering or supervising the administration of the compositions, and that dosage ranges set forth herein are exemplary only and are not intended to limit the scope or practice of the claimed composition.

In one embodiment, an anti-EGFR ADC claimed herein, *e.g.,* an ADC comprising AbA, is administered to a subject in need thereof, *e.g.,* a subject having cancer, as an ADC at a dose of 0.1 to 30 mg/kg. In another embodiment, the anti-EGFR ADC, *e.g.,* an ADC comprising AbA, is administered to a subject in need thereof, *e.g.,* a subject having cancer, as an ADC at a dose of 1 to 15 mg/kg. In another embodiment, the anti-EGFR ADC, *e.g.,* an ADC comprising AbA, is administered to a subject in need thereof, *e.g.,* a subject having cancer, as an ADC at a dose of 1 to 10 mg/kg. In another embodiment, the anti-EGFR ADC, *e.g.,* an ADC comprising AbA, is administered to a subject in need thereof, *e.g.,* a subject having cancer, as an ADC at a dose of 2 to 3 mg/kg. In another embodiment, the anti-EGFR ADC, *e.g.,* an ADC comprising AbA, is administered to a subject in need thereof, *e.g.,* a subject having cancer, as an ADC at a dose of 1 to 4 mg/kg.

In one embodiment, an anti-EGFR ADC claimed herein, *e.g.,* an ADC comprising AbA, is administered to a subject in need thereof, *e.g.,* a subject having cancer, as an ADC at a dose of 1 to 200 µg/kg. In another embodiment, the anti-EGFR ADC, *e.g.,* an ADC comprising AbA, is administered to a subject in need thereof, *e.g.,* a subject having cancer, as an ADC at a dose of 5 to 150 µg/kg. In another embodiment, the anti-EGFR ADC, *e.g.,* an ADC comprising AbA, is administered to a subject in need thereof, *e.g.,* a subject having cancer, as an ADC at a dose of 5 to 100 µg/kg. In another embodiment, the anti-EGFR ADC, *e.g.,* an ADC comprising AbA, is administered to a subject in need thereof, *e.g.,* a subject having cancer, as an ADC at a dose of 5 to 90 µg/kg, In another embodiment, the anti-EGFR ADC, *e.g.*, an ADC comprising AbA, is administered to a subject in need thereof, *e.g.,* a subject having cancer, as an ADC at a dose of 5 to 80 µg/kg. In another embodiment, the anti-EGFR ADC, *e.g.,* an ADC comprising AbA, is administered to a subject in need thereof, *e.g.,* a subject having cancer, as an ADC at a dose of 5 to 70 µg/kg. In another embodiment, the anti-EGFR ADC, *e.g.,* an ADC comprising AbA, is administered to a subject in need thereof, *e.g.,* a subject having cancer, as an ADC at a dose of 5 to 60 µg/kg. In another embodiment, the anti-EGFR ADC, *e.g.,* an ADC comprising AbA, is administered to a subject in need thereof, *e.g.,* a subject having cancer, as an ADC at a dose of 10 to 80 µg/kg.

In one embodiment, an anti-EGFR ADC claimed herein, is administered to a subject in need thereof, *e.g.,* a subject having cancer, at a dose of. 1 to 6 mg/kg. In another embodiment, an anti-EGFR ADC claimed herein, is administered to a subject in need thereof, *e.g.,* a subject having cancer, at a dose of .5 to 4 mg/kg. In another embodiment, an anti-EGFR ADC claimed herein, is administered to a subject in need thereof, *e.g.,* a subject having cancer, at a dose of 1.8 to 2.4 mg/kg. In another embodiment, an anti-EGFR ADC claimed herein, is administered to a subject in need thereof, *e.g.,* a subject having cancer, at a dose of 1 to 4 mg/kg. In another embodiment, an anti-EGFR ADC claimed herein, is administered to a subject in need thereof, *e.g.,* a subject having cancer, at a dose of about 1 mg/kg. In another embodiment, an anti-EGFR ADC claimed herein, is administered to a subject in need thereof, *e.g.,* a subject having cancer, at a dose of 3 to 6 mg/kg. In another embodiment, an anti-EGFR ADC claimed herein, is administered to a subject in need thereof, *e.g.,* a subject having cancer, at a dose of 3 mg/kg. In another embodiment, an anti-EGFR ADC claimed herein, is administered to a subject in need thereof, *e.g.,* a subject having cancer, at a dose of 2 to 3 mg/kg. In another embodiment, an anti-EGFR ADC claimed herein, is administered to a subject in need thereof, *e.g.,* a subject having cancer, at a dose of 6 mg/kg.

In another embodiment, an anti-EGFR ADC claimed herein is administered to a subject in need thereof, *e.g.,* a subject having cancer, at a dose of 1 to 200 µg/kg. In another embodiment, an anti-EGFR ADC claimed herein is administered to a subject in need thereof, *e.g.,* a subject having cancer, at a dose of 5 to 100 µg/kg. In another embodiment, an anti-EGFR ADC claimed herein is administered to a subject in need thereof, *e.g.,* a subject having cancer, at a dose of 5 to 90 µg/kg. In another embodiment, an anti-EGFR ADC claimed herein is administered to a subject in need thereof, *e.g.,* a subject having cancer, at a dose of 5 to 80 µg/kg. In another embodiment, an anti-EGFR ADC claimed herein is administered to a subject in need thereof, *e.g.,* a subject having cancer, at a dose of 5 to 70 µg/kg, In another embodiment, an anti-EGFR ADC claimed herein is administered to a subject in need thereof, *e.g.,* a subject having cancer, at a dose of 5 to 60 µg/kg .

In another aspect, this application provides a method for detecting the presence of EGFR in a sample *in vitro* (*e.g.,* a biological sample, such as serum, plasma, tissue, or biopsy). The subject method can be used to diagnose a disorder, *e.g.,* a cancer. The method includes: (i) contacting the sample or a control sample with the anti-EGFR ADC as claimed herein; and (ii) detecting formation of a complex between the anti-EGFR ADC and the sample or the control sample, wherein a statistically significant change in the formation of the complex in the sample relative to the control sample is indicative of the presence of EGFR in the sample.

Given their ability to bind to human EGFR, the ADCs of the disclosure can be used to detect human EGFR *(e.g.,* in a biological sample, such as serum or plasma), using a conventional immunoassay, such as an enzyme linked immunosorbent assays (ELISA), an radioimmunoassay (RIA) or tissue immunohistochemistry. In one aspect, the disclosure provides a method for detecting human EGFR in a biological sample comprising contacting a biological sample with an antibody, or antibody portion, of the disclosure and detecting either the antibody (or antibody portion) bound to human EGFR or unbound antibody (or antibody portion), to thereby detect human EGFR in the biological sample. The antibody is directly or indirectly labeled with a detectable substance to facilitate detection of the bound or unbound antibody. Suitable detectable substances include various enzymes, prosthetic groups, fluorescent materials, luminescent materials and radioactive materials. Examples of suitable enzymes include horseradish peroxidase, alkaline phosphatase, β-galactosidase, or acetylcholinesterase; examples of suitable prosthetic group complexes include streptavidin/biotin and avidin/biotin; examples of suitable fluorescent materials include umbelliferone, fluorescein, fluorescein isothiocyanate, rhodamine, dichlorotriazinylamine fluorescein, dansyl chloride or phycoerythrin; an example of a luminescent material includes luminol; and examples of suitable radioactive material include ³H, ¹⁴C, ³⁵S, ⁹⁰Y, ⁹⁹Tc, ¹¹¹In, ¹²⁵I, ¹³¹I, ¹⁷⁷Lu, ¹⁶⁶Ho, or ¹⁵³Sm.

Alternative to labeling the antibody, human EGFR can be assayed in biological fluids by a competition immunoassay utilizing rhEGFR standards labeled with a detectable substance and an unlabeled anti-human EGFR ADC. In this assay, the biological sample, the labeled rhEGFR standards and the anti-human EGFR antibody are combined and the amount of labeled rhEGFR standard bound to the unlabeled antibody is determined. The amount of human EGFR in the biological sample is inversely proportional to the amount of labeled rhEGFR standard bound to the anti-EGFR antibody. Similarly, human EGFR can also be assayed in biological fluids by a competition immunoassay utilizing rhEGFR standards labeled with a detectable substance and an unlabeled anti-human EGFR ADC.

### 8. Pharmaceutical Compositions

The Bcl-xL inhibitors and/or ADCs claimed herein may be in the form of compositions comprising the inhibitor or ADC and one or more carriers, excipients and/or diluents. The compositions may be formulated for specific uses, such as for veterinary uses or pharmaceutical uses in humans. The form of the composition *(e.g.,* dry powder, liquid formulation, *etc.)* and the excipients, diluents and/or carriers used will depend upon the intended uses of the inhibitors and/or ADCs and, for therapeutic uses, the mode of administration.

For therapeutic uses, the Bcl-xL inhibitor and/or ADC compositions may be supplied as part of a sterile, pharmaceutical composition that includes a pharmaceutically acceptable carrier. This composition can be in any suitable form (depending upon the desired method of administering it to a patient). The pharmaceutical composition can be administered to a patient by a variety of routes such as orally, transdermally, subcutaneously, intranasally, intravenously, intramuscularly, intrathecally, topically or locally. The most suitable route for administration in any given case will depend on the particular Bcl-xL inhibitor or ADC, the subject, and the nature and severity of the disease and the physical condition of the subject. Typically, the Bcl-xL inhibitors will be administered orally or parenterally, and ADC pharmaceutical composition will be administered intravenously or subcutaneously.

Pharmaceutical compositions can be conveniently presented in unit dosage forms containing a predetermined amount of Bcl-xL inhibitor or an ADC claimed herein per dose. The quantity of inhibitor or ADC included in a unit dose will depend on the disease being treated, as well as other factors as are well known in the art. For Bcl-xL inhibitors, such unit dosages may be in the form of tablets, capsules, lozenges, *etc.* containing an amount of Bcl-xL inhibitor suitable for a single administration. For ADCs, such unit dosages may be in the form of a lyophilized dry powder containing an amount of ADC suitable for a single administration, or in the form of a liquid. Dry powder unit dosage forms may be packaged in a kit with a syringe, a suitable quantity of diluent and/or other components useful for administration. Unit dosages in liquid form may be conveniently supplied in the form of a syringe pre-filled with a quantity of ADC suitable for a single administration.

The pharmaceutical compositions may also be supplied in bulk from containing quantities of ADC suitable for multiple administrations

Pharmaceutical compositions of ADCs may be prepared for storage as lyophilized formulations or aqueous solutions by mixing an ADC having the desired degree of purity with optional pharmaceutically-acceptable carriers, excipients or stabilizers typically employed in the art (all of which are referred to herein as "carriers"), *i.e.,* buffering agents, stabilizing agents, preservatives, isotonifiers, non-ionic detergents, antioxidants, and other miscellaneous additives. *See,* Remington's Pharmaceutical Sciences, 16th edition (Osol, ed. 1980). Such additives should be nontoxic to the recipients at the dosages and concentrations employed.

Buffering agents help to maintain the pH in the range which approximates physiological conditions. They may be present at concentrations ranging from about 2 mM to about 50 mM. Suitable buffering agents for use with the present disclosure include both organic and inorganic acids and salts thereof such as citrate buffers (e.g., monosodium citrate-disodium citrate mixture, citric acid-trisodium citrate mixture, citric acid-monosodium citrate mixture, *etc.),* succinate buffers (*e.g.,* succinic acid-monosodium succinate mixture, succinic acid-sodium hydroxide mixture, succinic acid-disodium succinate mixture, *etc.),* tartrate buffers (e.g., tartaric acid-sodium tartrate mixture, tartaric acid-potassium tartrate mixture, tartaric acid-sodium hydroxide mixture, *etc.),* fumarate buffers (*e.g.,* fumaric acid-monosodium fumarate mixture, fumaric acid-disodium fumarate mixture, monosodium fumarate-disodium fumarate mixture, *etc.),* gluconate buffers *(e.g.,* gluconic acid-sodium gluconate mixture, gluconic acid-sodium hydroxide mixture, gluconic acid-potassium gluconate mixture, *etc.),* oxalate buffer (e.g., oxalic acid-sodium oxalate mixture, oxalic acid-sodium hydroxide mixture, oxalic acid-potassium oxalate mixture, *etc.),* lactate buffers *(e.g.,* lactic acid-sodium lactate mixture, lactic acid-sodium hydroxide mixture, lactic acid-potassium lactate mixture, *etc.)* and acetate buffers (*e.g.,* acetic acid-sodium acetate mixture, acetic acid-sodium hydroxide mixture, *etc.).* Additionally, phosphate buffers, histidine buffers and trimethylamine salts such as Tris can be used.

Preservatives may be added to retard microbial growth, and can be added in amounts ranging from about 0.2%-1% (w/v). Suitable preservatives for use with the present disclosure include phenol, benzyl alcohol, meta-cresol, methyl paraben, propyl paraben, octadecyldimethylbenzyl ammonium chloride, benzalconium halides (e.g., chloride, bromide, and iodide), hexamethonium chloride, and alkyl parabens such as methyl or propyl paraben, catechol, resorcinol, cyclohexanol, and 3-pentanol. Isotonicifiers sometimes known as "stabilizers" can be added to ensure isotonicity of liquid compositions of the present disclosure and include polyhydric sugar alcohols, for example trihydric or higher sugar alcohols, such as glycerin, erythritol, arabitol, xylitol, sorbitol and mannitol. Stabilizers refer to a broad category of excipients which can range in function from a bulking agent to an additive which solubilizes the therapeutic agent or helps to prevent denaturation or adherence to the container wall. Typical stabilizers can be polyhydric sugar alcohols (enumerated above); amino acids such as arginine, lysine, glycine, glutamine, asparagine, histidine, alanine, ornithine, L-leucine, 2-phenylalanine, glutamic acid, threonine, *etc.,* organic sugars or sugar alcohols, such as lactose, trehalose, stachyose, mannitol, sorbitol, xylitol, ribitol, myoinisitol, galactitol, glycerol and the like, including cyclitols such as inositol; polyethylene glycol; amino acid polymers; sulfur containing reducing agents, such as urea, glutathione, thioctic acid, sodium thioglycolate, thioglycerol, α-monothioglycerol and sodium thio sulfate; low molecular weight polypeptides (e.g., peptides of 10 residues or fewer); proteins such as human serum albumin, bovine serum albumin, gelatin or immunoglobulins; hydrophylic polymers, such as polyvinylpyrrolidone monosaccharides, such as xylose, mannose, fructose, glucose; disaccharides such as lactose, maltose, sucrose and trisaccacharides such as raffinose; and polysaccharides such as dextran.

Non-ionic surfactants or detergents (also known as "wetting agents") may be added to help solubilize the glycoprotein as well as to protect the glycoprotein against agitation-induced aggregation, which also permits the formulation to be exposed to shear surface stressed without causing denaturation of the protein. Suitable non-ionic surfactants include polysorbates (20, 80, *etc.),* polyoxamers (184, 188 *etc.),* Pluronic polyols, polyoxyethylene sorbitan monoethers (TWEEN^{®}-20, TWEEN^{®}-80, *etc.).* Non-ionic surfactants may be present in a range of about 0.05 mg/ml to about 1.0 mg/ml, for example about 0.07 mg/ml to about 0.2 mg/ml.

Additional miscellaneous excipients include bulking agents (*e.g.,* starch), chelating agents *(e.g.,* EDTA), antioxidants *(e.g.,* ascorbic acid, methionine, vitamin E), and cosolvents..

### EXAMPLES

### Example 1. Synthesis of Exemplary Bcl-xL Inhibitors

This example provides synthetic methods for exemplary Bcl-xL inhibitory compounds W2.01-W2.91. Bcl-xL inhibitors (W2.01-W2.91) and synthons (Examples 2.1-2.176) were named using ACD/Name 2012 release (Build 56084, 05 April 2012, Advanced Chemistry Development Inc., Toronto, Ontario), ACD/Name 2014 release (Build 66687, 25 October 2013, Advanced Chemistry Development Inc., Toronto, Ontario), ChemDraw^{®} Ver. 9.0.7 (CambridgeSoft, Cambridge, MA), ChemDraw^{®} Ultra Ver. 12.0 (CambridgeSoft, Cambridge, MA), or ChemDraw^{®} Professional Ver. 15.0.0.106. Bcl-xL inhibitor and synthons were named with ACD/Name 2012 release (Build 56084, 05 April 2012, Advanced Chemistry Development Inc., Toronto, Ontario), ACD/Name 2014 release (Build 66687, 25 October 2013, Advanced Chemistry Development Inc., Toronto, Ontario), ChemDraw^{®} Ver. 9.0.7 (CambridgeSoft, Cambridge, MA), ChemDraw^{®} Ultra Ver. 12.0 (CambridgeSoft, Cambridge, MA), or ChemDraw^{®} Professional Ver. 15.0.0.106.

### 1.1 Synthesis of 6-[8-(1,3-benzothiazol-2-ylcarbamoyl)-3,4-dihydroisoquinolin-2(1H)-yl]-3- [1-({3-[2-({2-[2-(carboxymethoxy)ethoxy]ethyl}amino)ethoxy]-5,7-dimethyltricyclo[3.3.1.1^{3,7}]dec-1-yllmethyl)-5-methyl-1H-pyrazol-4-yl]pyridine-2-carboxylic acid (Compound W2.01)

### 1.1.1 3-bromo-5,7-dimethyladamantanecarboxylic acid

Into a 50 mL round-bottomed flask at 0 °C, was added bromine (16 mL). Iron powder (7 g) was added, and the reaction was stirred at 0 °C for 30 minutes. 3,5-Dimethyladamantane-1-carboxylic acid (12 g) was added. The mixture was warmed up to room temperature and stirred for 3 days. A mixture of ice and concentrated HCl was poured into the reaction mixture. The resulting suspension was treated twice with Na₂SO₃ (50 g in 200 mL water) and extracted three times with dichloromethane. The combined organics were washed with IN aqueous HCl, dried over sodium sulfate, filtered, and concentrated to give the title compound.

### 1.1.2 3-bromo-5,7-dimethyladamantanemethanol

To a solution of Example 1.1.1 (15.4 g) in tetrahydrofuran (200 mL) was added BH₃ (1M in tetrahydrofuran, 150 mL), and the mixture was stirred at room temperature overnight. The reaction mixture was then carefully quenched by adding methanol dropwise. The mixture was then concentrated under vacuum, and the residue was balanced between ethyl acetate (500 mL) and 2N aqueous HCl (100 mL). The aqueous layer was further extracted twice with ethyl acetate, and the combined organic extracts were washed with water and brine, dried over sodium sulfate, and filtered. Evaporation of the solvent gave the title compound.

### 1.1.3 1-((3-bromo-5,7-dimethyltricyclo[3.3.1.1^{3,7}]dec-1-yl)methyl)-1H-pyrazole

To a solution of Example 1.1.2 (8.0 g) in toluene (60 mL) was added 1H-pyrazole (1.55 g) and cyanomethylenetributylphosphorane (2.0 g), and the mixture was stirred at 90 °C overnight. The reaction mixture was concentrated, and the residue was purified by silica gel column chromatography (10:1 heptane:ethyl acetate) to give the title compound. MS (ESI) m/e 324.2 (M+H)⁺.

### 1.1.4 2-1[3,5-dimethyl-7-(1H-pyrazol-1-ylmethyl)tricyclo[3.3.1.1^{3,7}]dec-1-yl]oxy}ethanol

To a solution of Example 1.1.3 (4.0 g) in ethane-1,2-diol (12 mL) was added triethylamine (3 mL). The mixture was stirred at 150 °C under microwave conditions (Biotage Initiator) for 45 minutes. The mixture was poured into water (100 mL) and extracted three times with ethyl acetate. The combined organic extracts were washed with water and brine, dried over sodium sulfate, and filtered. Evaporation of the solvent gave a residue that was purified by silica gel chromatography, eluting with 20% ethyl acetate in heptane, followed by 5% methanol in dichloromethane, to give the title compound. MS (ESI) m/e 305.2 (M+H)⁺.

### 1.1.5 2-({3,5-dimethyl-7-[(5-methyl-1H-pyrazol-1-yl)methyl]tricyclo[3.3.1.1^{3,7}]dec-1-yl}oxy)ethanol

To a cooled (-78 °C) solution of Example 1.1.4 (6.05 g) in tetrahydrofuran (100 mL) was added n-BuLi (40 mL, 2.5M in hexane), and the mixture was stirred at-78 °C for 1.5 hours. Iodomethane (10 mL) was added through a syringe, and the mixture was stirred at-78 °C for 3 hours. The reaction mixture was then quenched with aqueous NH₄Cl and extracted twice with ethyl acetate, and the combined organic extracts were washed with water and brine. After drying over sodium sulfate, the solution was filtered and concentrated, and the residue was purified by silica gel column chromatography, eluting with 5% methanol in dichloromethane, to give the title compound. MS (ESI) m/e 319.5 (M+H)⁺.

### 1.1.6 1-({3,5-dimethyl-7-[2-(hydroxy)ethoxy]tricyclo[3.3.1.1^{3,7}]dec-1-yl}methyl)-4-iodo-5-methyl-1H-pyrazole

To a solution of Example 1.1.5 (3.5 g) in N,N-dimethylformamide (30 mL) was added N-iodosuccinimide (3.2 g), and the mixture was stirred at room temperature for 1.5 hours. The reaction mixture was diluted with ethyl acetate (600 mL) and washed with aqueous NaHSO₃, water and brine. The organic layer was dried over sodium sulfate, filtered and concentrated under reduced pressure. The residue was purified by silica gel chromatography, eluting with 20% ethyl acetate in dichloromethane, to give the title compound. MS (ESI) m/e 445.3 (M+H)⁺.

### 1.1.7 1-((3-(2-((tert-butyldimethylsilyl)oxy)ethoxy)-5,7-dimethyladamantan-1-yl)methyl)-4-iodo-5-methyl-1H-pyrazole

Tert-butyldimethylsilyl trifluoromethanesulfonate (5.34 mL) was added to a solution of Example 1.1.6 (8.6 g) and 2,6-lutidine (3.16 mL) in dichloromethane (125 mL) at-40 °C, and the reaction was allowed to warm to room temperature overnight. The mixture was concentrated, and the residue was purified by silica gel chromatography, eluting with 5-20% ethyl acetate in heptanes, to give the title compound. MS (ESI) m/e 523.4 (M+H)⁺.

### 1.1.8 1-((3-(2-((tert-butyldimethylsilyl)oxy)ethoxy)-5,7-dimethyladamantan-1-yl)methyl)-5-methyl-4-(4,4,5,5-tetramethyl-1,3,2-dioxaborolan-2-yl)-1H-pyrazole

n-Butyllithium (8.42 mL, 2.5M in hexanes) was added to Example 1.1.7 (9.8 g) in 120 mL tetrahydrofuran at-78 °C, and the reaction was stirred for 1 minute. Trimethyl borate (3.92 mL) was added, and the reaction stirred for 5 minutes. Pinacol (6.22 g) was added, and the reaction was allowed to warm to room temperature and was stirred 2 hours. The reaction was quenched with pH 7 buffer, and the mixture was poured into ether. The layers were separated, and the organic layer was concentrated under reduced pressure. The residue was purified by silica gel chromatography, eluting with 1-25% ethyl acetate in heptanes, to give the title compound.

### 1.1.9 6-fluoro-3-bromopicolinic acid

A slurry of 6-amino-3-bromopicolinic acid (25 g) in 400 mL 1:1 dichloromethane/chloroform was added to nitrosonium tetrafluoroborate (18.2 g) in dichloromethane (100 mL) at 5 °C over 1 hour. The resulting mixture was stirred for another 30 minutes, then warmed to 35 °C and stirred overnight. The reaction was cooled to room temperature, and then adjusted to pH 4 with aqueous NaH₂PO₄ solution. The resulting solution was extracted three times with dichloromethane, and the combined extracts were washed with brine, dried over sodium sulfate, filtered and concentrated to provide the title compound.

### 1.1.10 Tert-butyl 3-bromo-6-fluoropicolinate

Para-toluenesulfonyl chloride (27.6 g) was added to a solution of Example 1.1.9 (14.5 g) and pyridine (26.7 mL) in dichloromethane (100 mL) and tert-butanol (80 mL) at 0 °C. The reaction was stirred for 15 minutes, and then warmed to room temperature, and stirred overnight. The solution was concentrated and partitioned between ethyl acetate and aqueous Na₂CO₃ solution. The layers were separated, and the aqueous layer extracted with ethyl acetate. The organic layers were combined, rinsed with aqueous Na₂CO₃ solution and brine, dried over sodium sulfate, filtered, and concentrated to provide the title compound.

### 1.1.11 methyl 2-(5-bromo-6-(tert-butoxycarbonyl)pyridin-2-yl)-1,2,3,4-tetrahydroisoquinoline-8-carboxylate

To a solution of methyl 1,2,3,4-tetrahydroisoquinoline-8-carboxylate hydrochloride (12.37 g) and Example 1.1.10 (15 g) in dimethyl sulfoxide (100 mL) was added N,N-diisopropylethylamine (12 mL), and the mixture was stirred at 50 °C for 24 hours. The mixture was then diluted with ethyl acetate (500 mL) and washed with water and brine. The organic layer was dried over sodium sulfate, filtered and concentrated under reduced pressure. The residue was purified by silica gel chromatography, eluting with 20% ethyl acetate in hexane, to give the title compound. MS (ESI) m/e 448.4 (M+H)⁺.

### 1.1.12 methyl 2-(6-(tert-butoxycarbonyl)-5-(1-((3-(2-((tert-butyldimethylsilyl)oxy)ethoxy)-5,7-dimethyladamantan-1-yl)methyl)-5-methyl-1H-pyrazol-4-yl)pyridin-2-yl)-1,2,3,4-tetrahydroisoquinoline-8-carboxylate

A mixture of Example 1.1.11 (3.08 g), Example 1.1.8 (5 g), tris(dibenzylideneacetone)dipalladium(0) (126 mg), 1,3,5,7-tetramethyl-8-tetradecyl-2,4,6-trioxa-8-phosphaadamantane (170 mg), and K₃PO₄ (3.65 g) in 1,4-dioxane (25 mL) and water (25 mL) was heated to 90 °C for 2 hours. The mixture was cooled and poured into 1:1 diethyl ether:ethyl acetate. The layers were separated, and the organic was washed with saturated aqueous NaH₂PO₄ solution, water (2x), and brine. The organic layer was dried over sodium sulfate, filtered, and concentrated. The residue was purified by silica gel chromatography, eluting with 1-25% ethyl acetate in heptanes, to give the title compound. MS (ESI) m/e 799.6 (M+H)⁺.

### 1.1.13 2-(6-(tert-butoxycarbonyl)-5-(1-((3-(2-((tert-butyldimethylsilyl)oxy)ethoxy)-5,7-dimethyladamantan-1-yl)methyl)-5-methyl-1H-pyrazol-4-yl)pyridin-2-yl)-1,2,3,4-tetrahydroisoquinoline-8-carboxylic acid

Example 1.1.12 (5 g) and lithium hydroxide monohydrate (0.276 g) were stirred together in a solvent mixture of tetrahydrofuran (50 mL), methanol (5 mL) and water (15 mL) at 70 °C for 2 days. The reaction was cooled, acidified with 1M aqueous HCl solution, and extracted twice with ethyl acetate. The combined organic layers were washed with brine, dried over sodium sulfate, filtered, and concentrated. The residue was dissolved in dichloromethane (100 mL), cooled at-40 °C, and 2,6-lutidine (1.8 mL) and tert-butyldimethylsilyl trifluoromethanesulfonate (3.28 g) were added. The reaction was allowed to warm to room temperature and was stirred for 2 hours. The mixture was diluted with ether, and the layers were separated. The organic layer was concentrated. The residue was dissolved in tetrahydrofuran and treated with saturated aqueous K₂CO₃ solution for 1 hour. This mixture was acidified with concentrated HCl and extracted twice with ethyl acetate. The combined organic layers were dried over sodium sulfate, filtered, and concentrated under reduced pressure. The residue was purified by silica gel chromatography, eluting with 10-100% ethyl acetate in heptanes then 5% methanol in ethyl acetate, to give the title compound. MS (ESI) m/e 785.6 (M+H)⁺.

### 1.1.14 tert-butyl 6-(8-(benzo[d]thiazol-2-ylcarbamoyl)-3,4-dihydroisoquinolin-2(1H)-yl)-3-(1-((3-(2-hydroxyethoxy)-5,7-dimethyladamantan-1-yl)methyl)-5-methyl-1H-pyrazol-4-yl)picolinate

Example 1.1.13 (970 mg), N,N-diisopropylethylamine (208 mg), and 2-(3H-[1,2,3]triazolo[4,5-b]pyridin-3-yl)-1,1,3,3-tetramethylisouronium hexafluorophosphate (HATU) (970 mg) were stirred in 7 mL N,N-dimethylformamide at 0 °C for 10 minutes. Benzo[d]thiazol-2-amine (278 mg) was added, and the mixture was stirred for 24 hours at 50 °C. The mixture was cooled and diluted with ethyl acetate. The organic layer was washed with water and brine, dried over sodium sulfate, filtered, and concentrated. The residue was dissolved in tetrahydrofuran (50 mL), and tetrabutyl ammonium fluoride (10 mL, 1M in tetrahydrofuran) was added. The reaction was stirred for 1 hour, poured into ethyl acetate and washed with pH 7 buffer and brine. The organic layer was dried over sodium sulfate, filtered, and concentrated under reduced pressure. The residue was purified by silica gel chromatography, eluting with 10-100% ethyl acetate in heptanes, to give the title compound. MS (ESI) m/e 803.7 (M+H)⁺.

### 1.1.15 tert-butyl 6-(8-(benzo[d]thiazol-2-ylcarbamoyl)-3,4-dihydroisoquinolin-2(1H)-yl)-3-(1-((3,5-dimethyl-7-(2-oxoethoxy)adamantan-1-yl)methyl)-5-methyl-1H-pyrazol-4-yl)picolinate

To an ambient solution of Example 1.1.14 (100 mg) in dichloromethane (1.3 mL) was added Dess-Martin periodinane (58.1 mg) in a single portion. The reaction was stirred for 0.5 hours, and additional Dess-Martin periodinane (8 mg) was added. The reaction was stirred for 1 hour and quenched by the addition of ~10% aqueous NaOH solution and dichloromethane. The layers were separated, and the organic layer was washed with ~10% aqueous NaOH solution. The organic layer was dried with anhydrous sodium sulfate, filtered and concentrated under reduced pressure to a solid, which was used in the subsequent reaction without further purification. MS (ESI) m/e 801.3 (M+H)⁺.

### 1.1.16 2-(2-(2-((2-((3-((4-(6-(8-(benzo[d]thiazol-2-ylcarbamoyl)-3,4-dihydroisoquinolin-2(1H)-yl)-2-(tert-butoxycarbonyl)pyridin-3-yl)-5-methyl-1H-pyrazol-1-yl)methyl)-5,7-dimethyladamantan-1-yl)oxy)ethyl)amino)ethoxy)ethoxy)acetic acid

To an ambient solution of 2-(2-(2-aminoethoxy)ethoxy)acetic acid (22 mg) and Example 1.1.15 (100 mg) in methanol (1.3 mL) was added MP-CNBH₃ (65 mg, 2.49 mmol/g loading). The reaction was gently shaken overnight and filtered through a 0.4 micron filter. The crude material was purified by reverse phase HPLC using a Gilson system, eluting with 20-80% acetonitrile in water containing 0.1% v/v trifluoroacetic acid. The desired fractions were combined and freeze-dried to provide the title compound. MS (ESI) m/e 948.3 (M+H)⁺.

### 1.1.17 6-(8-(benzo[d]thiazol-2-ylcarbamoyl)-3,4-dihydroisoquinolin-2(1H)-yl)-3-(1-((3-(2-((2-(2-(carboxymethoxy)ethoxy)ethyl)amino)ethoxy)-5,7-dimethyladamantan-1-yl)methyl)-5-methyl-1H-pyrazol-4-yl)picolinic acid

To an ambient solution of Example 1.1.16 (15 mg) in dichloromethane (1 mL) was added trifluoroacetic acid (1 mL). The reaction was stirred for 16 hours and then concentrated under reduced pressure. The residue was purified by reverse phase HPLC using a Gilson system, eluting with 20-80% acetonitrile in water containing 0.1% v/v trifluoroacetic acid. The desired fractions were combined and freeze-dried to provide the title compound. ¹H NMR (400MHz, dimethyl sulfoxide-*d*₆) δ ppm 12.70 (bs, 2H), 8.29 (s, 1H), 8.03 (d, 1H), 7.79 (d, 1H), 7.62 (d, 1H), 7.53-7.42 (m, 3H), 7.40-7.32 (m, 2H), 7.29 (s, 1H), 6.96 (d, 1H), 4.96 (bs, 2H), 4.03 (s, 2H), 3.90 (t, 2H), 3.84 (s, 2H), 3.68 (t, 2H), 3.63-3.54 (m, 6H), 3.17-3.04 (m, 4H), 3.00 (t, 2H), 2.10 (s, 3H), 1.45-1.40 (m, 2H), 1.36-1.20 (m, 4H), 1.21-0.96 (m, 7H), 0.91-0.81 (m, 6H). MS (ESI) m/e 892.3 (M+H)⁺.

### 1.2 Synthesis of 6-[8-(1,3-benzothiazol-2-ylcarbamoyl)-3,4-dihydroisoquinolin-2(1H)-yl]-3-{1-[(3,5-dimethyl-7-{2-[(2-sulfoethyl)amino]ethoxyltricyclo[3.3.1.1^{3,7}]dec-1-yl)methyl]-5-methyl-1H-pyrazol-4-yl}pyridine-2-carboxylic acid (Compound W2.02)

### 1.2.1 methyl 2-(6-(tert-butoxycarbonyl)-5-(4,4,5,5-tetramethyl-1,3,2-dioxaborolan-2-yl)pyridin-2-yl)-1,2,3,4-tetrahydroisoquinoline-8-carboxylate

To a solution of Example 1.1.11 (2.25 g) and [1,1'-bis(diphenylphosphino)ferrocene]dichloropalladium(II) (205 mg) in acetonitrile (30 mL) was added triethylamine (3 mL) and pinacolborane (2 mL), and the mixture was stirred at reflux for 3 hours. The mixture was diluted with ethyl acetate (200 mL) and washed with water and brine. The organic layer was dried over sodium sulfate, filtered and concentrated under reduced pressure. Purification of the residue by silica gel chromatography, eluting with 20% ethyl acetate in hexane, provided the title compound.

### 1.2.2 methyl 2-(6-(tert-butoxycarbonyl)-5-(1-((3-(2-hydroxyethoxy)-5,7-dimethyladamantan-1-yl)methyl)-5-methyl-1H-pyrazol-4-yl)pyridin-2-yl)-1,2,3,4-tetrahydroisoquinoline-8-carboxylate

To a solution of Example 1.2.1 (2.25 g) in tetrahydrofuran (30 mL) and water (10 mL) was added Example 1.1.6 (2.0 g), 1,3,5,7-tetramethyl-6-phenyl-2,4,8-trioxa-6-phosphaadamantane (329 mg), tris(dibenzylideneacetone)dipalladium(0) (206 mg) and potassium phosphate tribasic (4.78 g). The mixture was refluxed overnight, cooled and diluted with ethyl acetate (500 mL). The resulting mixture was washed with water and brine, and the organic layer was dried over sodium sulfate, filtered and concentrated. The residue was purified by flash chromatography, eluting with 20% ethyl acetate in heptanes followed by 5% methanol in dichloromethane, to provide the title compound.

### 1.2.3 methyl 2-(6-(tert-butoxycarbonyl)-5-(1-((3,5-dimethyl-7-(2-((methylsulfonyl)oxy)ethoxy)adamantan-1-yl)methyl)-5-methyl-1H-pyrazol-4-yl)pyridin-2-yl)-1,2,3,4-tetrahydroisoquinoline-8-carboxylate

To a cold solution of Example 1.2.2 (3.32 g) in dichloromethane (100 mL) in an ice-bath was sequentially added triethylamine (3 mL) and methanesulfonyl chloride (1.1 g). The reaction mixture was stirred at room temperature for 1.5 hours and diluted with ethyl acetate, and washed with water and brine. The organic layer was dried over sodium sulfate, filtered, and concentrated to provide the title compound.

### 1.2.4 methyl 2-(5-(1-((3-(2-azidoethoxy)-5,7-dimethyladamantan-1-yl)methyl)-5-methyl-1H-pyrazol-4-yl)-6-(tert-butoxycarbonyl)pyridin-2-yl)-1,2,3,4-tetrahydroisoquinoline-8-carboxylate

To a solution of Example 1.2.3 (16.5 g) in N,N-dimethylformamide (120 mL) was added sodium azide (4.22 g). The mixture was heated at 80 °C for 3 hours, cooled, diluted with ethyl acetate and washed with water and brine. The organic layer was dried over sodium sulfate, filtered, and concentrated. The residue was purified by flash chromatography, eluting with 20% ethyl acetate in heptanes, to provide the title compound.

### 1.2.5 2-(5-(1-((3-(2-azidoethoxy)-5,7-dimethyladamantan-1-yl)methyl)-5-methyl-1H-pyrazol-4-yl)-6-(tert-butoxycarbonyl)pyridin-2-yl)-1,2,3,4-tetrahydroisoquinoline-8-carboxylic acid

To a solution of Example 1.2.4 (10 g) in a mixture of tetrahydrofuran (60 mL), methanol (30 mL) and water (30 mL) was added lithium hydroxide monohydrate (1.2g). The mixture was stirred at room temperature overnight and neutralized with 2% aqueous HCl. The resulting mixture was concentrated, and the residue was dissolved in ethyl acetate (800 mL), and washed with brine. The organic layer was dried over sodium sulfate, filtered, and concentrated to provide the title compound.

### 1.2.6 tert-butyl 3-(1-((3-(2-azidoethoxy)-5,7-dimethyladamantan-1-yl)methyl)-5-methyl-1H-pyrazol-4-yl)-6-(8-(benzo[d]thiazol-2-ylcarbamoyl)-3,4-dihydroisoquinolin-2(1H)-yl)picolinate

A mixture of Example 1.2.5 (10 g), benzo[d]thiazol-2-amine (3.24 g), fluoro-N,N,N',N'-tetramethylformamidinium hexafluorophosphate (5.69 g) and N,N-diisopropylethylamine (5.57 g) in N,N-dimethylformamide (20 mL) was heated at 60 °C for 3 hours, cooled and diluted with ethyl acetate. The resulting mixture was washed with water and brine. The organic layer was dried over sodium sulfate, filtered, and concentrated. The residue was purified by flash chromatography, eluting with 20% ethyl acetate in dichloromethane to give the title compound.

### 1.2.7 tert-butyl 3-(1-((3-(2-aminoethoxy)-5,7-dimethyladamantan-1-yl)methyl)-5-methyl-1H-pyrazol-4-yl)-6-(8-(benzo[d]thiazol-2-ylcarbamoyl)-3,4-dihydroisoquinolin-2(1H)-yl)picolinate

To a solution of Example 1.2.6 (2.0 g) in tetrahydrofuran (30 mL) was added Pd/C (10%, 200 mg). The mixture was stirred under a hydrogen atmosphere overnight. The insoluble material was filtered off and the filtrate was concentrated to provide the title compound.

### 1.2.8 tert-butyl 6-[8-(1,3-benzothiazol-2-ylcarbamoyl)-3,4-dihydroisoquinolin-2(1H)-yl]-3-[1-({3,5-dimethyl-7-[(2,2,7,7-tetramethyl-10,10-dioxido-3,3-diphenyl-4,9-dioxa-10λ⁶-thia-13-aza-3-silapentadecan-15-yl)oxy]tricyclo[3.3.1.1^{3,7}]dec-1-yl}methyl)-5-methyl-1H-pyrazol-4-yl]pyridine-2-carboxylate

To a solution of Example 1.2.7 (500 mg) in N,N-dimethylformamide (8 mL) was added 4-((tert-butyldiphenylsilyl)oxy)-2,2-dimethylbutyl ethenesulfonate (334 mg). The reaction was stirred at room temperature overnight and methylamine (0.3 mL) was added to quench the reaction. The resulting mixture was stirred for 20 minutes and purified by reverse-phase chromatography using an Analogix system (C18 column), eluting with 50-100% acetonitrile in water containing 0.1% v/v trifluoroacetic acid, to provide the title compound.

### 1.2.9 6-[8-(1,3-benzothiazol-2-ylcarbamoyl)-3,4-dihydroisoquinolin-2(1H)-yl]-3-{1-[(3,5-dimethyl-7-{2-[(2-sulfoethyl)amino]ethoxyltricyclo[3.3.1.1^{3,7}]dec-1-yl)methyl]-5-methyl-1H-pyrazol-4-yl}pyridine-2-carboxylic acid

Example 1.2.8 (200 mg) in dichloromethane (5 mL) was treated with trifluoroacetic acid (2.5 mL) overnight. The reaction mixture was concentrated and purified by reverse phase chromatography (C18 column), eluting with 20-60% acetonitrile in water containing 0.1% v/v trifluoroacetic acid, to provide the title compound. ¹H NMR (500 MHz, dimethyl sulfoxide-*d*₆) δ ppm 12.86 (s, 1H), 8.32 (s, 2H), 8.02 (d, 1H), 7.78 (d, 1H), 7.60 (d, 1H), 7.51 (d, 1H), 7.40-7.49 (m, 2H), 7.31-7.39 (m, 2H), 7.27 (s, 1H), 6.95 (d, 1H), 4.94 (s, 2H), 3.87 (t, 2H), 3.81 (s, 2H), 3.15-3.25 (m, 2H), 3.03-3.13 (m, 2H), 3.00 (t, 2H), 2.79 (t, 2H), 2.09 (s, 3H), 1.39 (s, 2H), 1.22-1.34 (m, 4H), 0.94-1.18 (m, 6H), 0.85 (s, 6H). MS (ESI) m/e 854.1 (M+H)⁺.

### 1.3 Synthesis of 2-{[(2-{[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyelo[3.3.1.1^{3,7}]dec-1-yl}oxy)ethyl]amino}ethyl)sulfonyl]amino}-2-deoxy-D-glucopyranose (Compound W2.03)

### 1.3.1 3-(1-((3-(2-aminoethoxy)-5,7-dimethyladamantan-1-yl)methyl)-5-methyl-1H-pyrazol-4-yl)-6-(8-(benzo[d]thiazol-2-ylcarbamoyl)-3,4-dihydroisoquinolin-2(1H)-yl)picolinic acid

Example 1.2.7 (200 mg) in dichloromethane (2.5 mL) was treated with trifluoroacetic acid (2.5 mL) overnight. The reaction mixture was concentrated, and the residue was purified by reverse phase chromatography (C18 column), eluting with 20-60% acetonitrile in water containing 0.1% v/v trifluoroacetic acid, to provide the title compound. MS (ESI) m/e 746.2 (M+H)⁺.

### 1.3.2 (3R,4R,5S,6R)-6-(acetoxymethyl)-3-(vinylsulfonamido)tetrahydro-2H-pyran-2,4,5-triyl triacetate

To a suspension of (3R,4R,5S,6R)-6-(acetoxymethyl)-3-aminotetrahydro-2H-pyran-2,4,5-triyl triacetate (7.7 g) in dichloromethane (100 mL) at 0 °C was added 2-chloroethanesulfonyl chloride (4.34 g). The mixture was stirred at 0 °C for 15 minutes, and triethylamine (12.1 mL) was added. The mixture was stirred at 0 °C for 1 hour, warmed to room temperature and stirred for 2 days. The mixture was diluted with dichloromethane and washed with water and brine. The organic layer was dried over sodium sulfate, filtered, and concentrated to provide the title compound.

### 1.3.3 N-((3R,4R,5S,6R)-2,4,5-trihydroxy-6-(hydroxym ethyl)tetrahydro-2H -pyran-3-yl)ethenesulfonamide

To a solution of Example 1.3.2 (6.74 g) in methanol (150 mL) was added triethylamine (10 mL). The mixture was stirred for 4 days and concentrated. The residue was dissolved in methanol and treated with Dowex HCR-5 until the solution was neutral. The mixture was filtered, and the filtrate was concentrated. The residue was purified by chromatography using a column of Sephadex LH-20 (100 g), eluting with methanol to provide the title compound.

### 1.3.4 2-{[(2-{[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl]amino}ethyl)sulfonyl]amino}-2-deoxy-D-glucopyranose

A mixture of Example 1.3.1 (23.5 mg), Example 1.3.3 (42.4 mg), and N,N-diisopropylethylamine (55 µL) in N,N-dimethylformamide (1 mL) and water (0.3 mL) was stirred for 5 days. The mixture was purified by reverse phase chromatography (C18 column), eluting with 20-60% acetonitrile in water containing 0.1% v/v trifluoroacetic acid, to provide the title compound. ¹H NMR (400 MHz, dimethyl sulfoxide-*d*₆) δ ppm 12.85 (s, 1H), 8.42 (s, 1H), 8.42 (s, 1H), 8.03 (d, 1H), 7.79 (d, 1H), 7.55-7.66 (m, 1H), 7.46-7.54 (m, 2H), 7.42-7.47 (m, 1H), 7.33-7.40 (m, 2H), 7.29 (s, 1H), 6.96 (d, 1H), 4.96 (s, 2H), 3.89 (t, 2H), 3.83 (s, 2H), 2.97-3.14 (m, 6H), 2.10 (s, 3H), 1.44 (s, 2H), 1.22-1.39 (m, 4H), 0.97-1.20 (m, 6H), 0.87 (s, 6H). MS (ESI) m/e 1015.3 (M+H)⁺.

### 1.4 This paragraph was intentionally left blank.

### 1.5 Synthesis of 6-[8-(1,3-benzothiazol-2-ylcarbamoyl)-3,4-dihydroisoquinolin-2(1H)-yl]-3-{1-[(3,5-dimethyl-7-{2-[(4-{[(3R,4R,5S,6R)-3,4,5-trihydroxy-6-(hydroxymethyl)tetrahydro-2H-pyran-2-yl]methyl}benzyl)amino]ethoxy}tricyclo[3.3.1.13,7]dec-1-yl)methyl]-5-methyl-1H-pyrazol-4-yl}pyridine-2-carboxylic acid (Compound W2.05)

### 1.5.1 [4-((3S,4R,5R,6R)-3,4,5-Tris-methoxymethoxy-6-methoxymethoxymethyl-tetrahydro-pyran-2-ylmethyl)-phenyl]-methanol

The title compound was prepared according to J. R. Walker et al., Bioorg. Med. Chem. 2006,14, 3038-3048. MS (ESI) m/e 478 (M+NH₄)⁺.

### 1.5.2 4-((3S,4R,5R,6R)-3,4,5-Tris-methoxymethoxy-6-methoxymethoxymethyl-tetrahydro-pyran-2-ylmethyl)-benzaldehyde

Example 1.5.1 (1.000 g) was dissolved in dichloromethane (25 mL), and Dess-Martin periodinane (1.013 g) was added. The solution was stirred 16 hours at room temperature. The solution was diluted with diethyl ether (25 mL) and 2 M aqueous sodium carbonate solution (25 mL) was added. The mixture was extracted with diethyl ether three times. The organic extracts were combined, washed with brine, and dried over anhydrous sodium sulfate. After filtration, the solution was concentrated under reduced pressure and purified by silica gel chromatography, eluting with 50-70% ethyl acetate in heptanes. The solvent was evaporated under reduced pressure to provide the title compound. MS (ESI) m/e 476 (M+NH₄)⁺.

### 1.5.3 Acetic acid (2R,3R,4R,5S)-3,4,5-triacetoxy-6-(4-formyl-benzyl)-tetrahydro-pyran-2-ylmethyl ester

Example 1.5.2 (660 mg) was dissolved in methanol (145 mL). 6 M Hydrochloric acid (8 mL) was added, and the solution was stirred at room temperature for two days. The solvents were removed under reduced pressure, azeotroping with ethyl acetate three times. The material was dried under vacuum for four days. The material was dissolved in N,N-dimethylformamide (50 mL). Acetic anhydride (12 mL), pyridine (6 mL), and N,N-dimethylpyridin-4-amine (10 mg) were added sequentially, and the solution was stirred at room temperature for 16 hours. The solution was diluted with water (150 mL) and extracted with ethyl acetate (50 mL) three times. The organics were combined, washed with water, washed with brine, and dried over anhydrous sodium sulfate. After filtration, the solution was concentrated under reduced pressure and purified by chromatography on silica gel, eluting with 40-50% ethyl acetate in heptanes. The solvent was evaporated under reduced pressure to provide the title compound.

### 1.5.4 (2R,3R,4R,5S)-2-(acetoxymethyl)-6-(4-(((2-((3-((4-(6-(8-(benzo[d]thiazol-2-ylcarbamoyl)-3,4-dihydroisoquinolin-2(1H)-yl)-2-(tert-butoxycarbonyl)pyridin-3-yl)-5-methyl-1H-pyrazol-1-yl)methyl)-5,7-dimethyladamantan-1-yl)oxy)ethyl)amino)methyl)benzyl)tetrahydro-2H-pyran-3,4,5-triyl triacetate

Example 1.5.7 (40 mg) and Example 1.5.3 (22.5 mg) were stirred in dichloromethane (1 mL) at room temperature for 10 minutes. Sodium triacetoxyborohydride (14 mg) was added, and the solution was stirred at room temperature for 16 hours. The material was purified by chromatography on silica gel, eluting with 10% methanol in dichloromethane. The solvent was evaporated under reduced pressure to provide the title compound. MS (ESI) m/e 1236 (M+H)⁺.

### 1.5.5 6-[8-(1,3-benzothiazol-2-ylcarbamoyl)-3,4-dihydroisoquinolin-2(1H)-yl]-3-{1-[(3,5-dimethyl-7-{2-[(4-{[(3R,4R,5S,6R)-3,4,5-trihydroxy-6-(hydroxymethyl)tetrahydro-2H-pyran-2-yl]methyl}benzyl)amino]ethoxy}tricyclo[3.3.1.1^{3,7}]dec-1-yl)methyl]-5-methyl-1H-pyrazol-4-yl}pyridine-2-carboxylic acid

Example 1.5.4 (68 mg) was dissolved in methanol (0.5 mL). Aqueous lithium hydroxide solution (2M, 1 mL) was added, and the solution was stirred at room temperature for 4.5 hours. Acetic acid (0.1 mL) was added, and the solvents were removed under vacuum. The material was then dissolved in trifluoroacetic acid (2 mL) and stirred at room temperature for 16 hours. The solution was concentrated under vacuum. The residue was purified by reverse phase HPLC using a Gilson PLC 2020 with a 150 x 30 mm C18 column, eluting with 20-70% acetonitrile in water containing 0.1% v/v trifluoroacetic acid. The desired fractions were combined and freeze-dried to provide the title compound. ¹H NMR (400MHz, dimethyl sulfoxide-*d*₆) δ ppm 12.86 (bs, 1H), 8.68 (bs, 2H), 8.04 (d, 1H), 7.80 (d, 1H), 7.62 (d, 1H), 7.51-7.43 (m, 3H), 7.39-7.24 (m, 6H), 6.96 (d, 1H), 5.23 (t, 1H), 4.96 (s, 2H), 4.56 (d, 1H), 4.42 (dd, 1H), 4.11 (m, 2H), 3.89 (t, 2H), 3.83 (s, 2H), 3.61-3.56 (m, 3H), 3.39 (dd, 1H), 3.22 (t, 1H), 3.15 (t, 1H), 3.09 (d, 1H), 3.01 (m, 6H), 2.89 (t, 1H), 2.60 (m, 1H), 2.10 (s, 3H), 1.43 (s, 2H), 1.30 (q, 4H), 1.14 (m, 4H), 1.03 (q, 2H), 0.86 (s, 6H). MS (ESI) m/e 1012 (M+H)⁺.

### 1.6 Synthesis of 6-[8-(1,3-benzothiazol-2-ylcarbamoyl)-3,4-dihydroisoquinolin-2(1H)-yl]-3-{1-[(3,5-dimethyl-7-{2-[(3-sulfopropyl)amino]ethoxy}tricyclo[3.3.1.1^{3,7}]dec-1-yl)methyl]-5-methyl-1H-pyrazol-4-yl}pyridine-2-carboxylic acid (Compound W2.06)

### 1.6.1 3-((2-((3-((4-(6-(8-(benzo[d]thiazol-2-ylcarbamoyl)-3,4-dihydroisoquinolin-2(1H)-yl)-2-(tert-butoxycarbonyl)pyridin-3-yl)-5-methyl-1H-pyrazol-1-yl)methyl)-5,7-dimethyladamantan-1-yl)oxy)ethyl)amino)propane-1-sulfonic acid

A mixture of Example 1.2.7 (100 mg), 1,2-oxathiolane 2,2-dioxide (13 mg) and N,N-diisopropylethylamine (19.07 µL) in N,N-dimethylformamide (2 mL) was heated to 50 °C overnight. The reaction was cooled and purified by reverse phase HPLC (C18 column), eluting with 20-60% acetonitrile in water containing 0.1% v/v trifluoroacetic acid, to provide the title compound. MS (ESI) m/e 924.1 (M+H)⁺.

### 1.6.2 6-[8-(1,3-benzothiazol-2-ylcarbamoyl)-3,4-dihydroisoquinolin-2(1H)-yl]-3-{1-[(3,5-dimethyl-7-{2-[(3-sulfopropyl)amino]ethoxyltricyclo[3.3.1.1^{3,7}]dec-1-yl)methyl]-5-methyl-1H-pyrazol-4-yl}pyridine-2-carboxylic acid

Example 1.6.1 (40 mg) in dichloromethane (2.5 mL) was treated with trifluoroacetic acid (2.5 mL) overnight. The reaction mixture was concentrated, and the residue was purified by reverse phase chromatography (C18 column), eluting with 20-60% acetonitrile in water containing 0.1% v/v trifluoroacetic acid, to provide the title compound. ¹H NMR (400 MHz, dimethyl sulfoxide-*d*₆) δ ppm 12.86 (s, 1H), 8.52 (s, 2H), 8.04 (d, 1H), 7.79 (d, 1H), 7.61 (d, 1H), 7.41-7.55 (m, 3H), 7.32-7.39 (m, 2H), 7.29 (s, 1H), 6.96 (d, 1H), 4.96 (s, 2H), 3.89 (t, 2H), 3.49-3.58 (m, 2H), 2.94-3.12 (m, 6H), 2.56-2.64 (m, 2H), 1.88-1.99 (m, 2H), 1.41 (s, 2H), 1.22-1.36 (m, 4H), 0.96-1.20 (m, 6H), 0.86 (s, 6H). MS (ESI) m/e 868.3 (M+H)⁺.

### 1.7 Synthesis of 6-[8-(1,3-benzothiazol-2-ylcarbamoyl)-3,4-dihydroisoquinolin-2(1H)-yl]-3-{1-[(3-{2-[(2,3-dihydroxypropyl)amino]ethoxyl-5,7-dimethyltricyclo[3.3.1.1^{3,7}]dec-1-yl)methyl]-5-methyl-1H-pyrazol-4-yl}pyridine-2-carboxylic acid (Compound W2.07)

To a solution of Example 1.2.7 (30 mg) in dichloromethane (3 mL) was added 2,3-dihydroxypropanal (3.6 mg), and NaCNBH₃ on resin (200 mg). The mixture was stirred overnight, filtered, and the solvent was evaporated. The residue was dissolved in dimethyl sulfoxide/methanol (1:1, 3 mL) and purified by reverse phase HPLC using a Gilson system, eluting with 10-85% acetonitrile in 0.1% trifluoroacetic acid in water, to give the title compound. ¹H NMR (400 MHz, dimethyl sulfoxide-*d*₆) δ ppm 12.85 (s, 1H), 8.27 (s, 2H), 8.03 (d, 1H), 7.79 (d, 1H), 7.61 (t, 1H), 7.33-7.54 (m, 6H), 7.29 (s, 1H), 6.96 (d, 1H), 4.96 (s, 3H), 3.72-3.89 (m, 8H), 3.25-3.64 (m, 6H), 2.99-3.10 (m, 4H), 2.11 (s, 3H), 1.00-1.52 (m, 8H), 0.86 (s, 6H). MS (ESI) m/e 820.3 (M+H)⁺.

### 1.8 Synthesis of 2-({[4-({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyelo[3.3.1.1^{3,7}]dec-1-yl}oxy)ethyl]amino}methyl)phenyl]sulfonyl}amino)-2-deoxy-beta-D-glucopyranose (Compound W2.08)

### 1.8.1 (2R,3S,4S,5R,6S)-6-(acetoxymethyl)-3-(4-formylphenylsulfonamido)tetrahydro-2H-pyran-2,4,5-triyl triacetate

4-Formylbenzene-1-sulfonyl chloride (100 mg) and (2S,3R,4R,5S,6R)-6-(acetoxymethyl)-3-aminotetrahydro-2H-pyran-2,4,5-triyl triacetate hydrochloride (563 mg) were added to 1,2-dichloroethane (4 mL). N,N-Diisopropylethylamine (0.51 mL) was added, and the solution was heated at 55 °C for three days. The solution was concentrated under reduced pressure and purified by flash column chromatography on silica gel, eluting with 70% ethyl acetate in heptanes. The solvent was evaporated under reduced pressure, and the material was dissolved in acetone (4 mL). Hydrochloric acid (1M, 4 mL) was added, and the solution was stirred at room temperature for 16 hours. The solution was then extracted with 70% ethyl acetate in heptanes (20 mL). The organic layer was washed with brine and dried over anhydrous sodium sulfate. After filtration, the solvent was evaporated under reduced pressure to provide the title compound. MS (ESI) m/e 514 (M+H)⁺.

### 1.8.2 (2R,3S,4S,5R,6S)-6-(acetoxymethyl)-3-(4-(((2-((3-((4-(6-(8-(benzo[d]thiazol-2-ylcarbamoyl)-3,4-dihydroisoquinolin-2(1H)-yl)-2-(tert-butoxycarbonyl)pyridin-3-yl)-5-methyl-1H-pyrazol-1-yl)methyl)-5,7-dimethyladamantan-1-yl)oxy)ethyl)amino)methyl)phenylsulfonamido)tetrahydro-2H-pyran-2,4,5-triyl triacetate

The title compound was prepared by substituting Example 1.8.1 for Example 1.5.3 in Example 1.5.4. MS (ESI) m/e 1301 (M+H)⁺.

### 1.8.3 2-({[4-({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl]amino}methyl)phenyl]sulfonyl}amino)-2-deoxy-beta-D-glucopyranose

The title compound was prepared by substituting Example 1.8.2 for Example 1.5.4 in Example 1.5.5. ¹HNMR (400MHz, dimethyl sulfoxide-*d*₆) δ ppm 12.86 (bs, 1H), 8.87 (bs, 2H), 8.04 (d, 1H), 7.91 (d, 2H), 7.79 (d, 1H), 7.70-7.55 (m, 3H), 7.52-7.42 (m, 3H), 7.39-7.33 (m, 2H), 7.29 (m, 1H), 6.96 (d, 1H), 4.96 (bs, 2H), 4.85 (dd, 1H), 4.62-4.52 (m, 2H), 4.32 (m, 2H), 3.89 (t, 2H), 3.83 (s, 2H), 3.70-3.35 (m, 10H), 3.02 (m, 4H), 2.91 (m, 1H), 2.10 (s, 3H), 1.44 (bs, 2H), 1.37-1.22 (m, 4H), 1.18-0.98 (m, 6H), 0.93-0.82 (m, 6H). MS (ESI) m/e 1075 (M+H)⁺.

### 1.9 Synthesis of 8-(1,3-benzothiazol-2-ylcarbamoyl)-2-{6-carboxy-5-[1-({3-[2-({2-[1-(beta-D-glucopyranuronosyl)-1H-1,2,3-triazol-4-yl]ethyl}amino)ethoxy]-5,7-dimethyltricyclo[3.3.1.1^{3,7}]dec-1-yl}methyl)-5-methyl-1H-pyrazol-4-yl]pyridin-2-yl}-1,2,3,4-tetrahydroisoquinoline (Compound W2.09)

### 1.9.1 (2R,3R,4S,5S,6S)-2-(4-(2-hydroxyethyl)-1H-1,2,3-triazol-1-yl)-6-(methoxycarbonyl)tetrahydro-2H-pyran-3,4,5-triyl triacetate

To a solution of (2R,3R,4S,5S,6S)-2-azido-6-(methoxycarbonyl)tetrahydro-2H-pyran-3,4,5-triyltriacetate (720 mg) in t-butanol (8 mL) and water (4 mL) was added but-3-yn-1-ol (140 mg), copper(II) sulfate pentahydrate (5.0 mg) and sodium ascorbate (40 mg). The mixture was stirred 20 minutes at 100 °C under microwave conditions (Biotage Initiator). The reaction mixture was diluted with ethyl acetate (300 mL), washed with water and brine, and dried over sodium sulfate. Filtration and evaporation of the solvent provided the title compound. MS (ESI) m/e 430.2 (M+H)⁺.

### 1.9.2 (2S,3S,4S,5R,6R)-2-(methoxycarbonyl)-6-(4-(2-oxoethyl)-1H-1,2,3-triazol-1-yl)tetrahydro-2H-pyran-3,4,5-triyl triacetate

To a solution of dimethyl sulfoxide (0.5 mL) in dichloromethane (10 mL) at-78 °C was added oxalyl chloride (0.2 mL). The mixture was stirred 20 minutes at-78 °C, and a solution of (2R,3R,4S,5 S,6S)-2-(4-(2-hydroxyethyl)-1H-1,2,3-triazol-1-yl)-6-(methoxycarbonyl)tetrahydro-2H-pyran-3,4,5-triyl triacetate (233 mg) in dichloromethane (10 mL) was added through a syringe. After 20 minutes, triethylamine (1 mL) was added to the mixture, and the mixture was stirred for 30 minutes while the temperature was allowed to rise to room temperature. The reaction mixture was diluted with ethyl acetate (300 mL), washed with water and brine, and dried over sodium sulfate. Filtration and evaporation of the solvent gave the crude product, which was used in the next reaction without further purification. MS (ESI) m/e 429.2 (M+H)⁺.

### 1.9.3 8-(1,3-benzothiazol-2-ylcarbamoyl)-2-{6-carboxy-5-[1-({3-[2-({2-[1-(beta-D-glucopyranuronosyl)-1H-1,2,3-triazol-4-yl]ethyl}amino)ethoxy]-5,7-dimethyltricyclo[3.3.1.1^{3,7}]dec-1-yl}methyl)-5-methyl-1H-pyrazol-4-yl]pyridin-2-yl}-1,2,3,4-tetrahydroisoquinoline

To a solution of Example 1.3.1 (150 mg) in dichloromethane (10 mL) was added Example 1.9.2 (86 mg) and NaBH₃CN on resin (2.49 mmol/g, 200 mg), and the mixture was stirred overnight. The reaction mixture was then filtered and concentrated. The residue was dissolved in tetrahydrofuran/methanol/H₂O (2:1:1, 12 mL) and lithium hydroxide monohydrate (50 mg) was added. The mixture was stirred overnight. The mixture was concentrated, and the residue was purified by reverse phase HPLC using a Gilson system, eluting with 10-85% acetonitrile in 0.1% trifluoroacetic acid in water, to provide the title compound. ¹H NMR (400 MHz, dimethyl sulfoxide-*d*₆) δ ppm 12.84 (s, 1H), 8.48 (s, 2H), 8.20 (s, 1H), 8.03 (d, 1H), 7.79 (d, 1H), 7.62 (d, 1H), 7.32-7.53 (m, 5H), 7.29 (s, 1H), 6.96 (d, 1H), 5.66 (d, 1H), 4.96 (s, 2H), 4.00 (d, 1H), 3.76-3.92 (m, 6H), 3.22-3.26 (m, 2H), 2.96-3.15 (m, 8H), 2.10 (s, 3H), 0.99-1.52 (m, 14H), 0.87 (s, 6H). MS (ESI) m/e 1028.3 (M+H)⁺.

### 1.10 Synthesis of 3-[1-({3-[2-(2-{[4-(beta-D-allopyranosyloxy)benzyl]amino}ethoxy)ethoxy]-5,7-dimethyltricyclo[3.3.1.1^{3,7}]dec-1-yl}methyl)-5-methyl-1H-pyrazol-4-yl]-6-[8-(1,3-benzothiazol-2-ylcarbamoyl)-3,4-dihydroisoquinolin-2(1H)-yl]pyridine-2-carboxylic acid (Compound W2.10)

### 1.10.1 2-(2-((3-((1H-pyrazol-1-yl)methyl)-5,7-dimethyladamantan-1-yl)oxy)ethoxy)ethanol

The title compound was prepared as in Example 1.1.4 by substituting ethane-1,2-diol with 2,2'-oxydiethanol. MS (ESI) m/e 349.2 (M+H)⁺.

### 1.10.2 2-(2-((3,5-dimethyl-7-((5-methyl-1H-pyrazol-1-yl)methyl)adamantan-1-yl)oxy)ethoxy)ethanol

The title compound was prepared as in Example 1.1.5 by substituting Example 1.1.4 with Example 1.10.1. MS (ESI) m/e 363.3 (M+H)⁺.

### 1.10.3 2-(2-((3-((4-iodo-5-methyl-1H-pyrazol-1-yl)methyl)-5,7-dimethyladamantan-1-yl)oxy)ethoxy)ethanol

The title compound was prepared as in Example 1.1.6 by substituting Example 1.1.5 with Example 1.10.2. MS (ESI) m/e 489.2 (M+H)⁺.

### 1.10.4 2-(2-((3-((4-iodo-5-methyl-1H-pyrazol-1-yl)methyl)-5,7-dimethyladamantan-1-yl)oxy)ethoxy)ethyl methanesulfonate

To a cooled solution of Example 1.10.3 (6.16 g) in dichloromethane (100 mL) was added triethylamine (4.21 g) followed by methanesulfonyl chloride (1.6 g), and the mixture was stirred at room temperature for 1.5 hours. The reaction mixture was then diluted with ethyl acetate (600 mL) and washed with water and brine. After drying over sodium sulfate, the solution was filtered and concentrated, and the residue was used in the next reaction without further purification. MS (ESI) m/e 567.2 (M+H)⁺.

### 1.10.5 2-(2-((3-((4-iodo-5-methyl-1H-pyrazol-1-yl)methyl)-5,7-dimethyladamantan-1-yl)oxy)ethoxy)ethanamine

A solution of Example 1.10.4 (2.5 g) in 7N ammonia in methanol (15 mL) was stirred at 100 °C for 20 minutes under microwave conditions (Biotage Initiator). The reaction mixture was concentrated under vacuum, and the residue was diluted with ethyl acetate (400 mL) and washed with aqueous NaHCO₃, water and brine. After drying over sodium sulfate, the solution was filtered and concentrated, and the residue was used in the next reaction without further purification. MS (ESI) m/e 488.2 (M+H)⁺.

### 1.10.6 tert-butyl (2-(2-((3-((4-iodo-5-methyl-1H-pyrazol-1-yt)methyl)-5,7-dimethyladamantan-1-yl)oxy)ethoxy)ethyl)carbamate

To a solution of Example 1.10.5 (2.2 g) in tetrahydrofuran (30 mL) was added *di-tert-*butyl dicarbonate (1.26 g) and 4-dimethylaminopyridine (100 mg). The mixture was stirred at room temperature for 1.5 hours and was diluted with ethyl acetate (300 mL). The solution was washed with saturated aqueous NaHCO₃, water (60 mL) and brine (60 mL). The organic layer was dried with sodium sulfate, filtered and concentrated. The residue was purified by silica gel chromatography, eluting with 20% ethyl acetate in dichloromethane, to give the title compound. MS (ESI) m/e 588.2 (M+H)⁺ .

### 1.10.7 methyl 2-(6-(tert-butoxycarbonyl)-5-(1-((3-(2-(2-((tert-butoxycarbonyl)amino)ethoxy)ethoxy)-5,7-dimethyladamantan-1-yl)methyl)-5-methyl-1H-pyrazol-4-yl)pyridin-2-yl)-1,2,3,4-tetrahydroisoquinoline-8-carboxylate

The title compound was prepared as in Example 1.2.2 by substituting Example 1.1.6 with Example 1.10.6. MS (ESI) m/e 828.5 (M+H)⁺.

### 1.10.8 2-(6-(tert-butoxycarbonyl)-5-(1-((3-(2-(2-((tert-butoxycarbonyl)amino)ethoxy)ethoxy)-5,7-dimethyladamantan-1-yl)methyl)-5-methyl-1H-pyrazol-4-yl)pyridin-2-yl)-1,2,3,4-tetrahydroisoquinoline-8-carboxylic acid

The title compound was prepared as in Example 1.2.5 by substituting Example 1.2.4 with Example 1.10.7. MS (ESI) m/e 814.5 (M+H)⁺.

### 1.10.10 tert-butyl 6-(8-(benzo[d]thiazol-2-ylcarbamoyl)-3,4-dihydroisoquinolin-2(1H)-yl)-3-(1-((3-(2-(2-((tert-butoxycarbonyl)amino)ethoxy)ethoxy)-5,7-dimethyladamantan-1-yl)methyl)-5-methyl-1H-pyrazol-4-yl)picolinate

The title compound was prepared as in Example 1.2.6 by substituting Example 1.2.5 with Example 1.10.8. MS (ESI) m/e 946.2 (M+H)⁺ .

### 1.10.11 3-(1-((3-(2-(2-aminoethoxy)ethoxy)-5,7-dimethyladamantan-1-yl)methyl)-5-methyl-1H-pyrazol-4-yl)-6-(8-(benzo[d]thiazol-2-ylcarbamoyl)-3,4-dihydroisoquinolin-2(1H)-yl)picolinic acid

The title compound was prepared as in Example 1.1.17 by substituting Example 1.1.16 with Example 1.10.9.

### 1.10.12 3-[1-({3-[2-(2-{[4-(beta-D-allopyranosyloxy)benzyl]amino}ethoxy)ethoxy]-5,7-dimethyltricyclo[3.3.1.1^{3,7}]dec-1-yl}methyl)-5-methyl-1H-pyrazol-4-yl]-6-[8-(1,3-benzothiazol-2-ylcarbamoyl)-3,4-dihydroisoquinolin-2(1H)-yl]pyridine-2-carboxylic acid

To a solution of Example 1.10.10 (88 mg) and triethylamine (0.04 mL) in dichloromethane (1.5 mL) was added 4-(((2S,3R,4R,5S,6R)-3,4,5-trihydroxy-6-(hydroxymethyl)tetrahydro-2H-pyran-2-yl)oxy)benzaldehyde (27.7 mg), methanol (1 mL), MP-CNBH₃ (2.49 mmol/g, 117 mg) and acetic acid (18 µL). The reaction mixture was stirred overnight. The reaction was filtered, and the filtrate was concentrated. The residue was purified by purified by reverse phase chromatography (C18 column), eluting with 20-60% acetonitrile in water containing 0.1% v/v trifluoroacetic acid, to provide the title compound. ¹H NMR (400 MHz, dimethyl sulfoxide-*d*₆) δ ppm 7.99 (d, 1H), 7.77 (d, 1H), 7.60 (d, 1H), 7.40-7.50 (m, 2H), 7.29-7.39 (m, 6H), 6.96 (d, 2H), 6.76 (d, 1H), 5.11 (d, 2H), 4.92 (s, 2H), 3.83-3.96 (m, 4H), 3.77 (s, 2H), 3.60-3.72 (m, 4H), 3.01 (d, 2H), 2.80 (t, 2H), 2.09 (s, 3H), 0.98-1.32 (m, 14H), 0.82 (s, 6H). MS (ESI) m/e 1058.3 (M+H)⁺.

### 1.11 Synthesisof6-[8-(1,3-benzothiazol-2-ylcarbamoyl)-3,4-dihydroisoquinolin-2(1H)-yl]-3-(1-{[3,5-dimethyl-7-(2-{2-[(2-sulfoethyl)amino]ethoxy]ethoxy}tricyclo[3.3.1.1^{3,7}]dec-1-yl]methyl}-5-methyl-1H-pyrazol-4-yl)pyridine-2-carboxylic acid(CompoundW2.11)

### 1.11.1 tert-butyl3-(1-((3-(2-(2-aminoethoxy)ethoxy)-5,7-dimethyladamantan-1-yl)methyl)-5-methyl-1H-pyrazol-4-yl)-6-(8-(benzo[d]thiazol-2-ylcarbamoyl)-3,4-dihydroisoquinolin-2(1H)-yl)picolinate

Example 1.10.9 (6.8 g) was dissolved in 50% trifluoroacetic acid in dichloromethane (10 mL) and stirred for 20 minutes, and the solvents were removed under vacuum. The residue was purified by reverse phase chromatography, eluting with 20-80% acetonitrile in water containing 0.1% trifluoroacetic acid, to provide the title compound. MS (ESI) m/e 790.2(M+H)⁺.

### 1.11.2 tert-butyl 6-(8-(benzo[d]thiazol-2-ylcarbamoyl)-3,4-dihydroisoquinolin-2(1H)-yl)-3-(1-((3,5-dimethyl-7-(2-(2-((2-(phenoxysulfonyl)ethyl)amino)ethoxy)ethoxy)adamantan-1-yl)methyl)-5-methyl-1H-pyrazol-4-yl)picolinate

To a solution of Example 1.11.1 (200 mg) and N,N-diisopropylethylamine (146 µL) in tetrahydrofuran (3 mL) at 0 °C was added phenyl ethenesulfonate (46 mg). The reaction mixture was stirred at 0 °C for 30 minutes, gradually warmed to room temperature, stirred overnight and concentrated to provide the title compound.

### 1.11.3 6-(8-(benzo[d]thiazol-2-ylcarbamoyl)-3,4-dihydroisoquinolin-2(1H)-yl)-3-(1-((3,5-dimethyl-7-(2-(2-((2-(phenoxysulfonyl)ethyl)amino)ethoxy)ethoxy)adamantan-1-yl)methyl)-5-methyl-1H-pyrazol-4-yl)picolinic acid

A solution of Example 1.11.2 (100 mg) in dichloromethane (5 mL) was treated with trifluoroacetic acid (2.5 mL) overnight and concentrated to provide the title compound. MS (APCI) m/e 974.9 (M+H)⁺.

### 1.11.4 6-[8-(1,3-benzothiazol-2-ylcarbamoyl)-3,4-dihydroisoquinolin-2(1H)-yl]-3-(1-{[3,5-dimethyl-7-(2-{2-[(2-sulfoethyl)amino]ethoxy}ethoxy)tricyclo[3.3.1.1^{3,7}]dec-1-yl]methyl}-5-methyl-1H-pyrazol-4-yl)pyridine-2-carboxylic acid

To a solution of Example 1.11.3 (195 mg) in tetrahydrofuran (3 mL) and methanol (2 mL) was slowly added 1M sodium hydroxide aqueous solution (2 mL). The mixture was stirred overnight, and NaOH pellets (0.5 g) were added. The resulting mixture was heated at 40 °C for 3 hours, cooled and concentrated. The concentrate was purified by reverse phase chromatography (C18 column), eluting with 10-70% acetonitrile in 10 mM aqueous NH₄OAc solution, to provide the title compound. ¹H NMR(400 MHz, dimethyl sulfoxide-*d*₆) δ ppm 8.04 (d, 1H), 7.79 (d, 1H), 7.61 (d, 1H), 7.41-7.51 (m, 3H), 7.32-7.39 (m, 2H), 7.29 (s, 1H), 6.88 (d, 1H), 4.93 (s, 2H), 3.89 (t, 2H), 3.81 (s, 2H), 3.60-3.66 (m, 4H), 3.13-3.19 (m, 2H), 3.05-3.10 (m, 2H), 3.01 (t, 2H), 2.79 (t, 2H), 2.11 (s, 3H), 1.34 (s, 2H), 1.26 (s, 4H), 0.96-1.22 (m, 6H), 0.85 (s, 6H). MS (ESI) m/e 898.2 (M+H)⁺.

### 1.12 Synthesis of 6-[8-(1,3-benzothiazol-2-ylcarbamoyl)-3,4-dihydroisoquinolin-2(1H)-yl]-3-{1-[(3,5-dimethyl-7-{2-[(2-phosphonoethyl)amino]ethoxy}tricyclo[3.3.1.1^{3,7}]dec-1-yl)methyl]-5-methyl-1H-pyrazol-4-yl}pyridine-2-carboxylic acid (Compound W2.12)

### 1.12.1 tert-butyl 6-(8-(benzo[d]thiazol-2-ylcarbamoyl)-3,4-dihydroisoquinolin-2(1H)-yl)-3-(1-((3-(2-((2-(diethoxyphosphoryl)ethyl)amino)ethoxy)-5,7-dimethyladamantan-1-yl)methyl)-5-methyl-1H-pyrazol-4-yl)picolinate

To a solution of Example 1.2.7 (307 mg) in tetrahydrofuran (5 mL) was added diethyl vinylphosphonate (176 mg) in water (2 mL). The reaction mixture was stirred at 70 °C for 3 days, and a few drops of acetic acid were added. The mixture was purified by reverse phase chromatography (C18 column), eluting with 10-70% acetonitrile in water containing 0.1% v/v trifluoroacetic acid, to provide the title compound. MS (APCI) m/e 966.8 (M+H)⁺.

### 1.12.2 6-[8-(1,3-benzothiazol-2-ylcarbamoyl)-3,4-dihydroisoquinolin-2(1H)-yl]-3-{1-[(3,5-dimethyl-7-{2-[(2-phosphonoethyl)amino]ethoxy}tricyclo[3.3.1.1^{3,7}]dec-1-yl)methyl]-5-methyl-1H-pyrazol-4-yl}pyridine-2-carboxylic acid

To a solution of Example 1.12.1 (170 mg) in dichloromethane (2.5 mL) was added bromotrimethylsilane (82 µL) and allyltrimethylsilane (50.4 µL). The reaction mixture was stirred overnight and water (0.02 mL) was added. The resulting mixture was stirred overnight and concentrated. The residue was purified by reverse phase chromatography (C18 column), eluting with 20-60% acetonitrile in water containing 0.1% trifluoroacetic acid, to provide the title compound. ¹H NMR (500 MHz, dimethyl sulfoxide-*d*₆) δ ppm 8.35 (s, 2H), 8.03 (d, 1H), 7.79 (d, 1H), 7.62 (d, 1H), 7.41-7.53 (m, 3H), 7.33-7.40 (m, 2H), 7.29 (s, 1H), 6.96 (d, 1H), 4.96 (s, 2H), 3.89 (t, 2H), 3.83 (s, 2H), 3.09 (s, 4H), 3.01 (t, 2H), 2.10 (s, 3H), 1.85-2.00 (m, 2H), 1.43 (s, 2H), 1.19-1.37 (m, 4H), 1.14 (s, 6H), 0.87 (s, 6H). MS (APCI) m/e 854.4 (M+H)⁺.

### 1.13 Synthesis of 6-[8-(1,3-benzothiazol-2-ylcarbamoyl)-3,4-dihydroisoquinolin-2(1H)-yl]-3-{1-[(3,5-dimethyl-7-{2-[methyl(3-sulfo-L-alanyl)amino]ethoxy}tricyclo[3.3.1.1^{3,7}]dec-1-yl)methyl]-5-methyl-1H-pyrazol-4-yl}pyridine-2-carboxylic acid (Compound W2.13)

### 1.13.1 2-({3-[(4-iodo-5-methyl-1H-pyrazol-1-yl)methyl]-5,7-dimethyltricyclo[3.3.1.1^{3,7}]dec-1-yl}oxy)ethyl methanesulfonate

To a cooled solution of Example 1.1.6 (6.16 g) in dichloromethane (100 mL) was added triethylamine (4.21 g) followed by methanesulfonyl chloride (1.6 g), and the mixture was stirred at room temperature for 1.5 hours. The reaction mixture was diluted with ethyl acetate (600 mL) and washed with water and brine. After drying over sodium sulfate, the solution was filtered and concentrated, and the residue was used in the next reaction without further purification. MS (ESI) m/e 523.4 (M+H)⁺ .

### 1.13.2 1-({3,5-dimethyl-7-[2-(methylamino)ethoxy]tricyclo[3.3.1.1^{3,7}]dec-1-yl}methyl)-4-iodo-5-methyl-1H-pyrazole

A solution of Example 1.13.1 (2.5 g) in 2M methylamine in methanol (15 mL) was stirred at 100 °C for 20 minutes under microwave conditions (Biotage Initiator). The reaction mixture was concentrated under vacuum, and the residue was diluted with ethyl acetate (400 mL) and washed with aqueous NaHCO₃, water and brine. After drying over sodium sulfate, the solution was filtered and concentrated, and the residue was used in the next reaction without further purification. MS (ESI) m/e 458.4 (M+H)⁺.

### 1.13.3 tert-butyl [2-({3-[(4-iodo-5-methy-1H-pyrazol-1-yl)methyl]-5,7-dimethyltricyclo[3.3.1.1^{3,7}]dec-1-yl}oxy)ethyl]methylcarbamate

To a solution of Example 1.13.2 (2.2 g) in tetrahydrofuran (30 mL) was added *di-tert-*butyl dicarbonate (1.26 g) and a catalytic amount of 4-dimethylaminopyridine. The mixture was stirred at room temperature for 1.5 hours and diluted with ethyl acetate (300 mL). The solution was washed with saturated aqueous NaHCO₃, water (60 mL) and brine (60 mL). The organic layer was dried with sodium sulfate, filtered and concentrated. The residue was purified by silica gel chromatography, eluting with 20% ethyl acetate in dichloromethane, to give the title compound. MS (ESI) m/e 558.5 (M+H)⁺.

### 1.13.4 methyl 2-(6-(tert-butoxycarbonyl)-5-(1-((3-(2-((tert-butoxycarbonyl)(methyl)amino)ethoxy)-5,7-dimethyltricyclo[3.3.1.1^{3,7}]dec-1-yl)methyl)-5-methyl-1H-pyrazol-4-yl)pyridin-2-yl)-1,2,3,4-tetrahydroisoquinoline-8-carboxylate

To a solution of Example 1.2.1 (4.94 g) in tetrahydrofuran (60 mL) and water (20 mL) was added Example 1.13.3 (5.57 g), 1,3,5,7-tetramethyl-8-tetradecyl-2,4,6-trioxa-8-phosphaadamantane (412 mg), tris(dibenzylideneacetone)dipalladium(0) (457 mg), and K₃PO₄ (11 g), and the mixture was stirred at reflux for 24 hours. The reaction mixture was cooled and diluted with ethyl acetate (500 mL), washed with water and brine. The organic layer was dried over sodium sulfate, filtered and concentrated under reduced pressure. Purification of the residue by silica gel chromatography, eluting with 20% ethyl acetate in heptane, provided the title compound. MS (ESI) m/e 799.1 (M+H)⁺.

### 1.13.5 2-(6-(tert-butoxycarbonyl)-5-(1-((3-(2-((tert-butoxycarbonyl)(methyl)amino)ethoxy)-5,7-dimethyltricyclo[3.3.1.1^{3,7}]dec-1-yl)methyl)-5-methyl-1H-pyrazol-4-yl)pyridin-2-yl)-1,2,3,4-tetrahydroisoquinoline-8-carboxylic acid

To a solution of Example 1.13.4 (10 g) in tetrahydrofuran (60 mL), methanol (30 mL) and water (30 mL) was added lithium hydroxide monohydrate (1.2 g), and the mixture was stirred at room temperature for 24 hours. The reaction mixture was neutralized with 2% aqueous HCl and concentrated under vacuum. The residue was diluted with ethyl acetate (800 mL) and washed with water and brine, and dried over sodium sulfate. Filtration and evaporation of the solvent provided the title compound. MS (ESI) m/e 785.1 (M+H)⁺.

### 1.13.6 tert-butyl 6-[8-(1,3-benzothiazol-2-ylcarbamoyl)-3,4-dihydroisoquinolin-2(1H)-yl]-3-{1-[(3-{2-[(tert-butoxycarbonyl)(methyl)amino]ethoxy}-5,7-dimethyltricyclo[3.3.1.1^{3,7}]dec-1-yl)methyl]-5-methyl-1H-pyrazol-4-yl}pyridine-2-carboxylate

To a solution of Example 1.13.5 (10 g) in N,N-dimethylformamide (20 mL) was added benzo[d]thiazol-2-amine (3.24 g), fluoro-N,N,N',N'-tetramethylformamidinium hexafluorophosphate (5.69 g) and N,N-diisopropylethylamine (5.57 g), and the mixture was stirred at 60 °C for 3 hours. The reaction mixture was diluted with ethyl acetate (800 mL) and washed with water and brine, and dried over sodium sulfate. Filtration and evaporation of the solvent and silica gel purification of the residue, eluting with 20% ethyl acetate in dichloromethane, provided the title compound. MS (ESI) m/e 915.5 (M+H)⁺.

### 1.13.7 6-[8-(1,3-benzothiazol-2-ylcarbamoyl)-3,4-dihydroisoquinolin-2(1H)-yl]-3-[1-({3,5-dimethyl-7-[2-(methylamino)ethoxy]tricyclo[3.3.1.1^{3,7}]dec-1-yl}methyl)-5-methyl-1H-pyrazol-4-yl]pyridine-2-carboxylic acid

To a solution of Example 1.13.6 (5 g) in dichloromethane (20 mL) was added trifluoroacetic acid (10 mL), and the mixture was stirred overnight. The solvent was evaporated under vacuum, and the residue was dissolved in dimethyl sulfoxide/methanol (1:1, 10 mL). The mixture was purified by reverse phase chromatography using an Analogix system and a C18 column (300 g), and eluting with 10-85% acetonitrile and 0.1% trifluoroacetic acid in water, to give the title compound.

### 1.13.8 6-[8-(1,3-benzothiazol-2-ylcarbamoyl)-3,4-dihydroisoquinolin-2(1H)-yl]-3-{1-[(3,5-dimethyl-7-{2-[methyl(3-sulfo-L-alanyl)amino]ethoxy}tricyclo[3.3.1.1^{3,7}]dec-1-yl)methyl]-5-methyl-1H-pyrazol-4-yl}pyridine-2-carboxylic acid

A solution of (R)-2-((((9H-fluoren-9-yl)methoxy)carbonyl)amino)-3-sulfopropanoic acid (0.020 g), N,N-diisopropylethylamine (0.045 mL) and O-(7-azabenzotriazol-1-yl)-N,N,N',N'-tetramethyluronium hexafluorophosphate (HATU, 0.020 g) were stirred together in N,N-dimethylformamide (0.75 mL) at room temperature. After stirring for 30 minutes, Example 1.13.7 (0.039 g) was added, and the reaction stirred for an additional 1 hour. Diethylamine (0.027 mL) was added to the reaction and stirring was continued for 3 hours. The reaction was diluted with water (0.75 mL) and N,N-dimethylformamide (1 mL), neutralized with trifluoroacetic acid (0.039 mL) and purified by reverse phase HPLC using a Gilson system, eluting with 20-80% acetonitrile in water containing 0.1% v/v trifluoroacetic acid. The desired fractions were combined and freeze-dried to provide the title compound. ¹H NMR (400 MHz, dimethyl sulfoxide-*d*₆) δ ppm 12.89 (s, 1H), 8.11-8.02 (m, 4H), 7.84 (d, 1H), 7.66 (d, 1H), 7.60-7.45 (m, 3H), 7.45-7.36 (m, 2H), 7.34 (d, 1H), 7.00 (dd, 1H), 5.00 (s, 2H), 4.57-4.40 (m, 1H), 3.93 (t, 2H), 3.90-3.84 (m, 2H), 3.58-3.43 (m, 2H), 3.41-3.21 (m, 2H), 3.18-3.02 (m, 3H), 2.95-2.85 (m, 2H), 2.76 (td, 2H), 2.14 (d, 3H), 1.51-0.85 (m, 18H). MS (ESI) m/e 911.2 (M+H)⁺.

### 1.14 Synthesis of 6-[8-(1,3-benzothiazol-2-ylcarbamoyl)-3,4-dihydroisoquinolin-2(1H)-yl]-3-{1-[(3,5-dimethyl-7-{2-[(3-phosphonopropyl)amino]ethoxy}tricyclo[3.3.1.1^{3,7}]dec-1-yl)methyl]-5-methyl-1H-pyrazol-4-yl}pyridine-2-carboxylic acid (Compound W2.14)

### 1.14.1 di-tert-butyl (3-hydroxypropyl)phosphonate

NaH (60% in mineral oil, 400 mg) was added to di-tert-butylphosphonate (1.93 g) in N,N-dimethylformamide (30 mL), and the reaction was stirred at room temperature for 30 minutes. (3-Bromopropoxy)(tert-butyl)dimethylsilane (2.1 g) was added, and the reaction was stirred overnight. The mixture was diluted with diethyl ether (300 mL), and the solution was washed three times with water, and brine, then dried over sodium sulfate, filtered, and concentrated. The residue was dissolved in 20 mL tetrahydrofuran, and tetrabutyl ammonium fluoride (TBAF, 1M in tetrahydrofuran, 9 mL) was added. The solution was stirred for 20 minutes, and then pH 7 buffer (50 mL) was added. The mixture was taken up in diethyl ether, and separated, and the organic layer was washed with brine, and then concentrated. The crude product was chromatographed on silica gel using 10-100% ethyl acetate in heptanes, followed by 5% methanol in ethyl acetate to provide the title compound.

### 1.14.2 di-tert-butyl (3-oxopropyl)phosphonate

Example 1.14.1 (200 mg) and Dess-Martin periodinane (370 mg) were stirred in dichloromethane (5 mL) for 2 hours. The mixture was taken up in ethyl acetate, and washed twice with 1M aqueous NaOH solution, and brine, and then concentrated. The crude product was chromatographed on silica gel, using 50-100% ethyl acetate in heptanes followed by 10% methanol in ethyl acetate, to provide the title compound.

### 1.14.3 tert-butyl 6-(8-(benzo[d]thiazol-2-ylcarbamoyl)-3,4-dihydroisoquinolin-2(1H)-yl)-3-(1-((3-(2-((3-(diethoxyphosphoryl)propyl)amino)ethoxy)-5,7-dimethyladamantan-1-yl)methyl)-5-methyl-1H-pyrazol-4-yl)picolinate

The title compound was prepared as described in Example 1.10.11, replacing Example 1.10.10 and 4-(((2S,3R,4R,5S,6R)-3,4,5-trihydroxy-6-(hydroxymethyl)tetrahydro-2H-pyran-2-yl)oxy)benzaldehyde with Example 1.2.7 and Example 1.14.2, respectively. MS (APCI) m/e 980.9 (M+H)⁺.

### 1.14.5 6-[8-(1,3-benzothiazol-2-ylcarbamoyl)-3,4-dihydroisoquinolin-2(1H)-yl]-3-{1-[(3,5-dimethyl-7-{2-[(3-phosphonopropyl)amino]ethoxy}tricyclo[3.3.1.1^{3,7}]dec-1-yl)methyl]-5-methyl-1H-pyrazol-4-yl}pyridine-2-carboxylic acid

The title compound was prepared as described in Example 1.12.2, replacing Example 1.12.1 with Example 1.14.3. ¹H NMR (400 MHz, dimethyl sulfoxide-*d*₆) δ ppm 8.37 (s, 2H), 8.03 (d, 1H), 7.79 (d, 1H), 7.62 (d, 1H), 7.42-7.53 (m, 3H), 7.33-7.40 (m, 2H), 7.29 (s, 1H), 6.96 (d, 1H), 4.96 (s, 2H), 3.86-3.93 (m, 2H), 3.52-3.59 (m, 2H), 2.93-3.06 (m, 6H), 2.10 (s, 3H), 1.71-1.89 (m, 2H), 1.53-1.65 (m, 2H), 1.43 (s, 2H), 1.23-1.37 (m, 4H), 0.96-1.19 (m, 6H), 0.87 (s, 6H). MS (APCI) m/e 868.3 (M+H)⁺.

### 1.15 Synthesis of 6-[8-(1,3-benzothiazol-2-ylcarbamoyl)-3,4-dihydroisoquinolin-2(1H)-yl]-3-{1-[(3,5-dimethyl-7-{2-[(3-sulfo-L-alanyl)amino]ethoxy}tricyclo[3.3.1.1^{3,7}]dec-1-yl)methyl]-5-methyl-1H-pyrazol-4-yl}pyridine-2-carboxylic acid(Compound W2.15)

A solution of (R)-2-((((9H-fluoren-9-yl)methoxy)carbonyl)amino)-3-sulfopropanoic acid (0.050 g) and O-(7-azabenzotriazol-1-yl)-N,N,N',N'-tetramethyluronium hexafluorophosphate (0.049 g) were dissolved in N,N-dimethylformamide (1 mL) and N,N-diisopropylethylamine (0.102 mL) was added. After stirring for 15 minutes, Example 1.3.1 (0.100 g) was added, and the reaction stirred for an additional 3 hours. Diethylamine (0.061 mL) was added to the reaction and stirring was continued overnight. The reaction was neutralized with 2,2,2-trifluoroacetic acid (0.090 mL) and diluted with N,N-dimethylformamide (1 mL) and water (1 mL). The mixture was purified by reverse phase HPLC using a Gilson system, eluting with 20-80% acetonitrile in water containing 0.1% v/v trifluoroacetic acid. The desired fractions were combined and freeze-dried to provide the title compound. ¹H NMR (500 MHz, dimethyl sulfoxide-*d*₆) δ ppm 12.86 (s, 1H), 8.63 (t, 1H), 8.15-8.01 (m, 4H), 7.79 (d, 1H), 7.62 (d, 1H), 7.56-7.41 (m, 3H), 7.40-7.33 (m, 2H), 7.30 (s, 1H), 6.96 (d, 1H), 4.96 (s, 2H), 4.08-3.97 (m, 1H), 3.89 (t, 2H), 3.82 (s, 2H), 3.42-3.31 (m, 2H), 3.28-3.17 (m, 1H), 3.16-3.06 (m, 1H), 3.01 (t, 2H), 2.97 (dd, 1H), 2.76 (dd, 1H), 2.10 (s, 3H), 1.39 (s, 2H), 1.32-1.20 (m, 4H), 1.19-1.07 (m, 4H), 1.07-0.95 (m, 2H), 0.85 (s, 6H). MS (ESI) m/e 897.2 (M+H)⁺.

### 1.16 Synthesis of 6-[8-(1,3-benzothiazol-2-ylcarbamoyl)-3,4-dihydroisoquinolin-2(1H)-yl]-3-(1-{[3,5-dimethyl-7-(2-{2-[(3-phosphonopropyl)amino]ethoxy}ethoxy)tricyclo[3.3.1.1^{3,7}]dec-1-yl]methyl}-5-methyl-1H-pyrazol-4-yl)pyridine-2-carboxylic acid (Compound W2.16)

### 1.16.1 tert-butyl 6-(8-(benzo[d]thiazol-2-ylcarbamoyl)-3,4-dihydroisoquinolin-2(1H)-yl)-3-(1-((3-(2-(2-((3-(di-tert-butoxyphosphoryl)propyl)amino)ethoxy)ethoxy)-5,7-dimethyladamantan-1-yl)methyl)-5-methyl-1H-pyrazol-4-yl)picolinate

Example 1.10.10 (338 mg) and Example 1.14.2 (120 mg) were dissolved in ethanol (20 mL), and the solution was concentrated. The residue was again taken up in ethanol (20 mL) and concentrated. The residue was then dissolved in dichloromethane (10 mL) and to this was added sodium triacetoxyborohydride (119 mg), and the reaction was stirred overnight. The crude mixture was chromatographed on silica gel, using 1% triethylamine in 95:5 ethyl acetate/methanol, to provide the title compound. MS (ESI) 1080.3 (M+H)⁺.

### 1.16.2 6-[8-(1,3-benzothiazol-2-ylcarbamoyl)-3,4-dihydroisoquinolin-2(1H)-yl]-3-(1-{[3,5-dimethyl-7-(2-{2-[(3-phosphonopropyl)amino]ethoxy}ethoxy)tricyclo[3.3.1.1^{3,7}]dec-1-yl]methyl}-5-methyl-1H-pyrazol-4-yl)pyridine-2-carboxylic acid

Example 1.16.1 (22 mg) was stirred in dichloromethane (3 mL) and trifluoroacetic acid (3 mL) for 2 days. The mixture was concentrated and chromatographed via reverse phase on a Biotage Isolera One system using a 40 g C18 column and eluting with 10-90% acetonitrile in 0.1% trifluoroacetic acid/water, to provide the title compound as a trifluoroacetic acid salt. ¹H NMR (400 MHz, dimethyl sulfoxide-*d*₆) δ ppm □8.62 (bs, 1H), 8.10 (d, 1H), 7.86 (d, 1H), 7.68 (d, 1H), 7.57 (d, 1H), 7.54 (dd, 1H), 7.50 (d, 1H), 7.42 (m, 2H), 7.35 (s, 1H), 7.02 (d, 1H), 5.02 (s, 2H), 3.94 (m, 2H), 3.97 (m, 2H), 3.68 (m, 2H), 3.55 (m, 2H), 3.15 (m, 1H), 3.09 (m, 4H), 2.55 (m, 4H), 2.15 (s, 3H), 1.86 (m, 1H), 1.66 (m, 2H), 1.45 (m, 2H), 1.31 (m, 4H), 1.19 (m, 4H), 1.08 (m, 2H), 0.90 (s, 6H). MS (ESI) 912.2 (M+H)⁺.

### 1.17 Synthesis of -3-{1-[(3-{2-[L-alpha-aspartyl(methyl)amino]ethoxy}-5,7 dimethyltricyclo[3.3.1.1^{3,7}]dec-1-yl)methyl]-5-methyl-1H-pyrazol-4-yl}-6-[8-(1,3-benzothiazol-2-ylcarbamoyl)-3,4-dihydroisoquinolin-2(1H)-yl]pyridine-2-carboxylic acid (Compound W2.17)

### 1.17.1 6-[8-(1,3-benzothiazol-2-ylcarbamoyl)-3,4-dihydroisoquinolin-2(1H)-yl]-3-{1-[(3-{2-[{(2S)-4-tert-butoxy-2-[(tert-butoxycarbonyl)amino]-4-oxobutanoyl}(methyl)amino]ethoxy}-5,7-dimethyltricyclo[3.3.1.1^{3,7}]dec-1-yl)methyl]-5-methyl-1H-pyrazol-4-yl}pyridine-2-carboxylic acid

A solution of Example 1.13.7 (0.060 g), (S)-4-tert-butyl 1-(2,5-dioxopyrrolidin-1-yl) 2-((tert-butoxycarbonyl)amino)succinate (0.034 g) and N,N-diisopropylethylamine were stirred together in dichloromethane (1 mL). After stirring overnight, the reaction was loaded onto silica gel and eluted using a gradient of 0.5-5% methanol/dichloromethane to give the title compound.

### 1.17.2 3-{1-[(3-{2-[L-alpha-aspartyl(methyl)amino]ethoxy}-5,7-dimethyltricyclo[3.3.1.1^{3,7}]dec-1-yl)methyl]-5-methyl-1H-pyrazol-4-yl}-6-[8-(1,3-benzothiazol-2-ylcarbamoyl)-3,4-dihydroisoquinolin-2(1H)-yl]pyridine-2-carboxylic acid

A solution of Example 1.17.1 (0.049 g) in dichloromethane (1 mL) was treated with trifluoroacetic acid (0.5 mL), and the reaction was stirred overnight. The reaction was concentrated, dissolved in N,N-dimethylformamide (2 mL) and water (0.5 mL) then purified by reverse phase HPLC using a Gilson system, eluting with 20-80% acetonitrile in water containing 0.1% v/v trifluoroacetic acid. The desired fractions were combined and freeze-dried to provide the title compound. ¹H NMR (400 MHz, dimethyl sulfoxide-*d*₆) δ ppm 12.85 (s, 1H), 8.15 (d, 3H), 8.03 (d, 1H), 7.79 (d, 1H), 7.62 (d, 1H), 7.55-7.41 (m, 3H), 7.36 (td, 2H), 7.29 (d, 1H), 6.95 (d, 1H), 4.96 (s, 2H), 4.55 (s, 1H), 3.92-3.86 (m, 2H), 3.60-3.47 (m, 2H), 3.47-3.37 (m, 2H), 3.32-3.21 (m, 1H), 3.09-2.97 (m, 4H), 2.92-2.72 (m, 3H), 2.67-2.53 (m, 1H), 2.10 (s, 3H), 1.46-0.94 (m, 12H), 0.85 (s, 6H). MS (ESI) m/e 875.2 (M+H)⁺.

### 1.18 Synthesis of 6-{4-[({2-[2-(2-aminoethoxy)ethoxy]ethyl}[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl]amino)methyl]benzyl}-2,6-anhydro-L-gulonic acid (Compound W2.18)

### 1.18.1 (2S,3S,4R,5S)-3,4,5-Triacetoxy-6-(4-bromomethyl-benzyl)-tetrahydro-pyran-2-carboxylic acid methyl ester

The title compound was prepared as described in J. R. Walker et al., Bioorg. Med. Chem. 2006, 14, 3038-3048. MS (ESI) m/e 518, 520 (M+NH₄)⁺.

### 1.18.2 (2S,3S,4R,5S)-3,4,5-Triacetoxy-6-(4-formyl-benzyl)-tetrahydropyran-2-carboxylic acid methyl ester

Example 1.18.1 (75 mg) and pyridine N-oxide (14 mg) were added to acetonitrile (0.75 mL). Silver (I) oxide (24 mg) was added to the solution, and the solution was stirred at room temperature for 16 hours. Anhydrous sodium sulfate (5 mg) was added, and the solution was stirred for five minutes. The solution was filtered and concentrated. The crude material was purified by flash column chromatography on silica gel, eluting with 50-70% ethyl acetate in heptanes. The solvent was evaporated under reduced pressure to provide the title compound.

### 1.18.3 (3R,4S,5R,6R)-2-(4-(((2-((3-((4-(6-(8-(benzo[d]thiazol-2-ylcarbamoyl)-3,4-dihydroisoquinolin-2(1H)-yl)-2-(tert-butoxycarbonyl)pyridin-3-yl)-5-methyl-1H-pyrazol-1-yl)methyl)-5,7-dimethyladamantan-1-yl)oxy)ethyl)amino)methyl)benzyl)-6-(methoxycarbonyl)tetrahydro-2H-pyran-3,4,5-triyl triacetate

The title compound was prepared by substituting Example 1.18.2 for Example 1.5.3 in Example 1.5.4. MS (ESI) m/e 1222 (M+H)⁺.

### 1.18.4 {2-[2-(2-Oxo-ethoxy)-ethoxy]-ethyl}-carbamic acid tert-butyl ester

The title compound was prepared by substituting {2-[2-(2-hydroxy-ethoxy)-ethoxy]-ethyl}-carbamic acid tert-butyl ester for Example 1.5.1 in Example 1.5.2.

### 1.18.5 (3R,4S,5R,6R)-2-(4-(2-(2-((3-((4-(6-(8-(benzo[d]thiazol-2-ylcarbamoyl)-3,4-dihydroisoquinolin-2(1H)-yl)-2-(tert-butoxycarbonyl)pyridin-3-yl)-5-methyl-1H-pyrazol-1-yl)methyl)-5,7-dimethyladamantan-1-yl)oxy)ethyl)-14,14-dimethyl-12-oxo-5,8,13-trioxa-2,11-diazapentadecyl)benzyl)-6-(methoxycarbonyl)tetrahydro-2H-pyran-3,4,5-triyl triacetate

The title compound was prepared by substituting Example 1.18.3 for Example 1.2.7 and Example 1.18.4 for Example 1.5.3 in Example 1.5.4. MS (ESI) m/e 1453 (M+H)⁺.

### 1.18.6 6-{4-[({2-[2-(2-aminoethoxy)ethoxy]ethyl}[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl]amino)methyl]benzyl}-2,6-anhydro-L-gulonic acid

The title compound was prepared by substituting Example 1.18.5 for Example 1.5.4 in Example 1.5.5. ¹H NMR (400MHz, dimethyl sulfoxide-*d*₆) δ ppm 9.38 (bs, 1H), 8.05 (dd, 1H), 7.90-7.68 (m, 6H), 7.62 (m, 2H), 7.53-7.27 (m, 8H), 6.94 (d, 1H), 4.96 (bs, 1H), 4.38 (bs, 4H), 3.91-3.57 (m, 11H), 3.37-3.11 (m, 14H), 2.98 (m, 6H), 2.61 (m, 1H), 2.10 (s, 3H), 1.44 (bs, 2H), 1.26 (m, 4H), 1.18-0.90 (m, 6H), 0.87 (bs, 6H). MS (ESI) m/e 1157 (M+H)⁺.

### 1.19 Synthesis of 4-({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyelo[3.3.1.1^{3,7}]dec-1-yl}oxy)ethyl]amino}methyl)phenyl hexopyranosiduronic acid (Compound W2.19)

### 1.19.1 (2R,3S,4R,5R,6R)-2-(4-formylphenoxy)-6-(methoxycarbonyl)tetrahydro-2H-pyran-3,4,5-triyl triacetate

To a solution of (2R,3R,4S,5S,6S)-2-bromo-6-(methoxycarbonyl)tetrahydro-2H-pyran-3,4,5-triyl triacetate (2.42 g) in acetonitrile (30 mL) was added silver(I) oxide (1.4 g) and 4-hydroxybenzaldehyde (620 mg). The reaction mixture was stirred for 4 hours and filtered. The filtrate was concentrated, and the residue was purified by silica gel chromatography, eluting with 5-50% ethyl acetate in heptanes, to provide the title compound. MS (ESI) m/e 439.2 (M+H)⁺.

### 1.19.2 4-({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl]amino}methyl)phenyl hexopyranosiduronic acid

To a solution of Example 1.2.7 (36 mg) in tetrahydrofuran (2 mL) and acetic acid (0.2 mL) was added Example 1.19.1 (21 mg) followed by MgSO₄ (60 mg). The mixture was stirred for 1 hour before the addition of NaBH₃CN on resin (153 mg). The mixture was then stirred for 3 hours. The mixture was filtered and lithium hydroxide monohydrate (20 mg) was added to the filtrate. The mixture was stirred for 2 hours and was acidified with trifluoroacetic acid and purified by reverse phase HPLC (Gilson system), eluting with 10-85% acetonitrile in 0.1% trifluoroacetic acid in water, to give the title compound. ¹H NMR (400 MHz, dimethyl sulfoxide-*d*₆) δ ppm 12.86 (s, 1H), 8.57-8.72 (m, 2H), 8.03 (d, 1H), 7.79 (d, 1H), 7.62 (d, 1H), 7.34-7.53 (m, 6H), 7.08 (t, 2H), 6.95 (d, 1H), 5.10 (d, , 1H), 4.96 (s, 2H), 4.06-4.15 (m, 4H), 3.83-3.97 (m, 6H), 3.26-3.42 (m, 8H), 2.93-3.10 (m, 6H), 2.10 (s, 3H), 1.43 (s, 2H), 1.24-1.38 (m, 6H), 0.97-1.16 (m, 4H), 0.86 (s, 6H). MS (ESI) m/e 1028.3 (M+H)⁺.

### 1.20 Synthesis of 6-[1-(1,3-benzothiazol-2-ylcarbamoyl)-1,2,3,4-tetrahydroquinolin-7-yl]-3-{1-[(3,5-dimethyl-7-{2-[(2-phosphonoethyl)amino]ethoxy}tricyclo[3.3.1.1^{3,7}]dec-1-yl)methyl]-5-methyl-1H-pyrazol-4-yl}pyridine-2-carboxylic acid (Compound W2.20)

### 1.20.1 2-((3,5-dimethyl-7-((5-methyl-4-(4,4,5,5-tetramethyl-1,3,2-dioxaborolan-2-yl)-1H-pyrazol-1-yl)methyl)adamantan-1-yl)oxy)ethanol

To a solution of Example 1.1.6 (9 g) and [1,1'-bis(diphenylphosphino)ferrocene]dichloropalladium(II) dichloromethane (827 mg) in acetonitrile (60 mL) was added triethylamine (10 mL) and pinacolborane (6 mL). The mixture was stirred at reflux overnight, cooled and used directly in the next step. MS (ESI) m/e 445.4 (M+H)⁺.

### 1.20.2 tert-butyl 6-chloro-3-(1-((3-(2-hydroxyethoxy)-5,7-dimethyladamantan-1-yl)methyl)-5-methyl-1H-pyrazol-4-yl)picolinate

To a solution of tert-butyl 3-bromo-6-chloropicolinate (5.92 g) in tetrahydrofuran (60 mL)and water (30 mL) was added the crude Example 1.20.1 (4.44 g), 1,3,5,7-tetramethyl-6-phenyl-2,4,8-trioxa-6-phosphaadamante (1.5 g), tris(dibenzylideneacetone)dipalladium(0) (927 mg) and K₃PO₄(22 g). The mixture was stirred at reflux overnight, cooled, diluted with ethyl acetate (800 mL) and washed with water and brine. The organic layer was dried over sodium sulfate, filtered, and concentrated. The residue was purified by flash chromatography, eluting with 20% ethyl acetate in heptane followed by 5% methanol in dichloromethane, to give the title compound. MS (ESI) m/e 531.1 (M+H)⁺.

### 1.20.3 tert-butyl 3-(1-((3-(2-((tert-butyldimethylsilyl)oxy)ethoxy)-5,7-dimethyladamantan-1-yl)methyl)-5-methyl-1H-pyrazol-4-yl)-6-chloropicolinate

To a solution of Example 1.20.2 (3.2 g) in N,N-dimethylformamide (20 mL) was added imidazole (0.62 g) and chloro t-buytldimethylsilane (1.37 g). The mixture was stirred overnight, diluted with ethyl acetate (300 mL), and washed with water and brine. The organic layer was dried over sodium sulfate, filtered, and concentrated. The residue was purified by flash chromatography, eluting with 20% ethyl acetate in heptanes, to provide the title compound. MS (ESI) m/e 645.4 (M+H)⁺.

### 1.20.4 tert-butyl 3-(1-((3-(2-((tert-butyldimethylsilyl)oxy)ethoxy)-5,7-dimethyladamantan-1-yl)methyl)-5-methyl-1H-pyrazol-4-yl)-6-(1,2,3,4-tetrahydroquinolin-7-yl)picolinate

To a solution of 7-(4,4,5,5-tetramethyl-1,3,2-dioxaborolan-2-yl)-1,2,3,4-tetrahydroquinoline (507 mg) in 1,4-dioxane (10 mL) and water (5 mL) was added Example 1.20.3 (1.25 g), bis(triphenylphosphine)palladium(II)dichloride (136 mg), and cesium fluoride (884 mg). The mixture was heated at 120 °C in a microwave synthesizer (Biotage, Initiator) for 20 minutes. The mixture was diluted with ethyl acetate (500 mL), and washed with water and brine. The organic layer was dried over sodium sulfate, filtered, concentrated and purified by flash chromatography, eluting with 20% ethyl acetate in heptanes and then with 5% methanol in dichloromethane, to provide the title compound. MS (ESI) m/e 741.5 (M+H)⁺.

### 1.20.5 tert-butyl 6-(1-(benzo[d]thiazol-2-ylcarbamoyl)-1,2,3,4-tetrahydroquinolin-7-yl)-3-(1-(3-(2-((tert-butyldimethylsilyl)oxy)ethoxy)-5,7-dimethyladamantan-1-yl)methyl)-5-methyl-1H-pyrazol-4-yl)picolinate

To a suspension of bis(2,5-dioxopyrrolidin-1-yl) carbonate (295 mg) in acetonitrile (10 mL) was added benzo[d]thiazol-2-amine (173 mg), and the mixture was stirred for 1 hour. A solution of Example 1.20.4 (710 mg) in acetonitrile (10 mL) was added, and the suspension was stirred overnight. The mixture was diluted with ethyl acetate (300 mL), washed with water and brine and dried over sodium sulfate. After filtration, the organic layer was concentrated and purified by silica gel chromatography, eluting with 20% ethyl acetate in heptanes, to provide the title compound. MS (ESI) m/e 917.2 (M+H)⁺.

### 1.20.6 tert-butyl 6-(1-(benzo[d]thiazol-2-ylcarbamoyl)-1,2,3,4-tetrahydroquinolin-7-yl)-3-(1-((3-(2-hydroxyethoxy)-5,7-dimethyladamantan-1-yl)methyl)-5-methyl-1H-pyrazol-4-yl)picolinate

To a solution of Example 1.20.5 (1.4 g) in tetrahydrofuran (10 mL) was added tetrabutyl ammonium fluoride (1.0 M in tetrahydrofuran, 6 mL). The mixture was stirred for 3 hours, diluted with ethyl acetate (300 mL) and washed with water and brine. The organic layer was dried over sodium sulfate, filtered, and concentrated to provide the title compound. MS (ESI) m/e 803.4 (M+H)⁺.

### 1.20.7 tert-butyl 6-(1-(benzo[d]thiazol-2-ylcarbamoyl)-1,2,3,4-tetrahydroquinolin-7-yl)-3-(1-((3,5-dimethyl-7-(2-((methylsulfonyl)oxy)ethoxy)adamantan-1-yl)methyl)-5-methyl-1H-pyrazol-4-yl)picolinate

To a cooled (0 °C) solution of Example 1.20.6 (1.2 g) in dichloromethane (20 mL) and triethylamine (2 mL) was added methanesulfonyl chloride (300 mg). The mixture was stirred for 4 hours, diluted with ethyl acetate (200 mL) and washed with water and brine. The organic layer was dried over sodium sulfate, filtered, and concentrated to provide the title compound. MS (ESI) m/e 881.3 (M+H)⁺.

### 1.20.8 tert-butyl 3-(1-((3-(2-azidoethoxy)-5,7-dimethyladamantan-1-yl)methyl)-5-methyl-1H-pyrazol-4-yl)-6-(1-(benzo[d]thiazol-2-ylcarbamoyl)-1,2,3,4-tetrahydroquinolin-7-yl)picolinate

To a solution of Example 1.20.7 (1.5 g) in N,N-dimethylformamide (20 mL)was added sodium azide (331 mg). The mixture was stirred for 48 hours, diluted with ethyl acetate (20.0 mL) and washed with water and brine. The organic layer was dried over sodium sulfate, filtered, concentrated and purified by silica gel chromatography, eluting with 20% ethyl acetate in dichloromethane, to provide the title compound. MS (ESI) m/e 828.4 (M+H)⁺.

### 1.20.9 tert-butyl 3-(1-((3-(2-aminoethoxy)-5,7-dimethyladamantan-1-yl)methyl)-5-methyl-1H-pyrazol-4-yl)-6-(1-(benzo[d]thiazol-2-ylcarbamoyl)-1,2,3,4-tetrahydroquinolin-7-yl)picolinate

To a solution of Example 1.20.8 (1.5 g) in tetrahydrofuran (30 mL) was added Pd/C (10%, 200 mg). The mixture was stirred under a hydrogen atmosphere overnight. The reaction was filtered, and the filtrate was concentrated to provide the title compound. MS (ESI) m/e 802.4 (M+H)⁺.

### 1.20.10 tert-butyl 6-(1-(benzo[d]thiazol-2-ylcarbamoyl)-1,2,3,4-tetrahydroquinolin-7-yl)-3-(1-((3-(2-((2-(diethoxyphosphoryl)ethyl)amino)ethoxy)-5,7-dimethyladamantan-1-yl)methyl)-5-methyl-1H-pyrazol-4-yl)picolinate

The title compound was prepared as described in Example 1.12.1, replacing Example 1.2.7 with Example 1.20.9.

### 1.20.11 6-[1-(1,3-benzothiazol-2-ylcarbamoyl)-1,2,3,4-tetrahydroquinolin-7-yl]-3-{1-[(3,5-dimethyl-7-{2-[(2-phosphonoethyl)amino]ethoxy}tricyclo[3.3.1.1^{3,7}]dec-1-yl)methyl]-5-methyl-1H-pyrazol-4-yl}pyridine-2-carboxylic acid

The title compound was prepared as described in Example 1.12.2, replacing Example 1.12.1 with Example 1.20.10. ¹H NMR (500 MHz, dimethyl sulfoxide-*d*₆) δ ppm 8.40 (s, 2H), 8.02 (d, 1H), 7.74-7.89 (m, 3H), 7.47 (s, 2H), 7.38 (t, 1H), 7.30 (d, 1H), 7.23 (t, 1H), 3.96 (s, 2H), 3.90 (s, 2H), 3.53-3.64 (m, 2H), 3.03-3.18 (m, 2H), 2.84 (t, 2H), 2.23 (s, 3H), 1.87-2.02 (m, 4H), 1.46 (s, 2H), 1.26-1.38 (m, 4H), 1.12-1.23 (m, 4H), 0.99-1.11 (m, 2H), 0.89 (s, 6H). MS (ESI) m/e 854.1 (M+H)⁺.

### 1.21 Synthesis of 6-[1-(1,3-benzothiazol-2-ylcarbamoyl)-1,2,3,4-tetrahydroquinolin-7-yl]-3-{1-[(3,5-dimethyl-7-{2-[methyl(3-sulfo-L-alanyl)amino]ethoxy}tricyclo[3.3.1.1^{3,7}]dec-1-yl)methyl]-5-methyl-1H-pyrazol-4-yl}pyridine-2-carboxylic acid (Compound W2.21)

### 1.21.1 tert-butyl (2-((3,5-dimethyl-7-((5-methyl-4-(4,4,5,5-tetramethyl-1,3,2-dioxaborolan-2-yl)-1H-pyrazol-1-yl)methyl)adamantan-1-yl)oxy)ethyl)(methyl)carbamate

To a solution of Example 1.13.3 (1.2 g) in 1,4-dioxane was added bis(benzonitrile)palladium(II) chloride (0.04 g), 4,4,5,5-tetramethyl-1,3,2-dioxaborolane (0.937 mL) and triethylamine (0.9 mL). The mixture was heated at reflux overnight, diluted with ethyl acetate and washed with water (60 mL) and brine (60 mL). The organic layer was dried over sodium sulfate, filtered and concentrated to provide the title compound.

### 1.21.2 tert-butyl 3-(1-((3-(2-((tert-butoxycarbonyl)(methyl)amino)ethoxy)-5,7-dimethyladamantan-1-yl)methyl)-5-methyl-1H-pyrazol-4-yl)-6-chloropicolinate

The title compound was prepared as described in Example 1.1.12, replacing Example 1.1.11 and Example 1.1.8 with tert-butyl 3-bromo-6-chloropicolinate and Example 1.21.1, respectively. MS (APCI) m/e 643.9 (M+H)⁺.

### 1.21.3 tert-butyl 3-(1-((3-(2-((tert-butoxycarbonyl)(methyl)amino)ethoxy)-5,7-dimethyladamantan-1-yl)methyl)-5-methyl-1H-pyrazol-4-yl)-6-(1,2,3,4-tetrahydroquinolin-7-yl)picolinate

A mixture of Example 1.21.2 (480 mg), 7-(4,4,5,5-tetramethyl-1,3,2-dioxaborolan-2-yl)-1,2,3,4-tetrahydroquinoline (387 mg), dichlorobis(triphenylphosphine)-palladium(II) (78 mg) and cesium fluoride (340 mg) in 1,4-dioxane (12 mL) and water (5 mL) was heated at 100 °C for 5 hours. The reaction was cooled and diluted with ethyl acetate. The resulting mixture was washed with water and brine, and the organic layer was dried over sodium sulfate, filtered, and concentrated. The residue was purified by flash chromatography, eluting with 50% ethyl acetate in heptanes, to provide the title compound. MS (APCI) m/e 740.4 (M+H)⁺.

### 1.21.4 tert-butyl 6-(1-(benzo[d]thiazol-2-ylcarbamoyl)-1,2,3,4-tetrahydroquinolin-7-yl)-3-(1-((3-(2-((tert-butoxycarbonyl)(methyl)amino)ethoxy)-5,7-dimethyladamantan-1-yl)methyl)-5-methyl-1H-pyrazol-4-yl)picolinate

To a solution of benzo[d]thiazol-2-amine (114 mg) in acetonitrile (5 mL) was added bis(2,5-dioxopyrrolidin-1-yl) carbonate (194 mg). The mixture was stirred for 1 hour, and Example 1.21.3 (432 mg) in acetonitrile (5 mL) was added. The mixture was stirred overnight, diluted with ethyl acetate, washed with water and brine. The organic layer was dried over sodium sulfate, filtered, and concentrated. The residue was purified by silica gel chromatography, eluting with 50% ethyl acetate in heptanes, to provide the title compound.

### 1.21.5 6-(1-(benzo[d]thiazol-2-ylcarbamoyl)-1,2,3,4-tetrahydroquinolin-7-yl)-3-(1-((3,5-dimethyl-7-(2-(methylamino)ethoxy)adamantan-1-yl)methyl)-5-methyl-1H-pyrazol-4-yl)picolinic acid

Example 1.2.4 (200 mg) in dichloromethane (5 mL) was treated with trifluoroacetic acid (2.5 mL) overnight. The mixture was concentrated to provide the title compound. ¹H NMR (400 MHz, dimethyl sulfoxide-*d*₆) δ ppm 8.40 (s, 1H), 8.30 (s, 2H), 8.02 (d, 1H), 7.85 (d, 1H), 7.74-7.83 (m, 2H), 7.42-7.53 (m, 2H), 7.38 (t, 1H), 7.30 (d, 1H), 7.23 (t, 1H), 3.93-4.05 (m, 2H), 3.52-3.62 (m, 2H), 2.97-3.10 (m, 2H), 2.84 (t, 2H), 2.56 (t, 2H), 2.23 (s, 3H), 1.88-2.00 (m, 2H), 1.45 (s, 2H), 1.25-1.39 (m, 4H), 1.12-1.22 (m, 4H), 1.00-1.09 (m, 2H), 0.89 (s, 6H). MS (ESI) m/e 760.1 (M+H)⁺.

### 1.21.6 6-(1-(benzo[d]thiazol-2-ylcarbamoyl)-1,2,3,4-tetrahydroquinolin-7-yl)-3-(1-((3-(2-((R)-2-((tert-butoxycarbonyl)amino)-N-methyl-3-sulfopropanamido)ethoxy)-5, 7-dimethyladamantan-1-yl)methyl)-5-methyl-1H-pyrazol-4-yl)picolinic acid

(R)-2-((tert-butoxycarbonyl)amino)-3-sulfopropanoic acid (70.9 mg) and O-(7-azabenzotriazol-1-yl)-N,N,N',N'-tetramethyluronium hexafluorophosphate (HATU, 65 mg) in N,N-dimethylformamide (1.5 ml) was cooled in ice-bath, and N,N-diisopropylethylamine (68.9 µL) was added. The mixture was stirred at 0 °C for 15 minutes and at room temperature for 8 hours. Example 1.21.5 (100 mg) in N,N-dimethylformamide (1 mL) and N,N-diisopropylethylamine (60 µL) were added. The resulting mixture was stirred overnight, concentrated and purified by reverse phase chromatography (C18 column), eluting with 20-60% acetonitrile in water containing 0.1% trifluoroacetic acid, to provide the title compound.

### 1.21.7 6-[1-(1,3-benzothiazol-2-ylcarbamoyl)-1,2,3,4-tetrahydroquinolin-7-yl]-3-{1-[(3,5-dimethyl-7-{2-[methyl(3-sulfo-L-alanyl)amino]ethoxyltricyclo[3.3.1.1^{3,7}]dec-1-yl)methyl]-5-methyl-1H-pyrazol-4-yl}pyridine-2-carboxylic acid

Example 1.21.6 (80 mg) in dichloromethane (3 mL) was treated with trifluoroacetic acid (1.5 mL) for 20 minutes. The reaction mixture was concentrated and purified by reverse phase chromatography (C18 column), eluting with 0-50% acetonitrile in 4 mM aqueous ammonium acetate solution, to provide the title compound. ¹H NMR (500 MHz, dimethyl sulfoxide-*d*₆) δ ppm 8.57 (s, 1H), 7.59-7.67 (m, 3H), 7.54 (d, 1H), 7.46-7.51 (m, 1H), 7.30 (d, 1H), 7.08-7.17 (m, 2H), 6.90 (t, 1H), 3.91-4.10 (m, 3H), 3.84 (s, 2H), 3.04 (s, 2H), 2.75-2.83 (m, 4H), 2.59-2.70 (m, 2H), 2.27-2.39 (m, 2H), 2.26 (s, 3H), 1.81-1.93 (m, 2H), 1.74 (s, 9H), 1.42 (s, 2H), 0.96-1.33 (m, 10H), 0.86 (s, 3H). MS (ESI) m/e 909.2 (M-H)⁻.

### 1.22 Synthesis of 3-{1-[(3,5-dimethyl-7-{2-[(2-sulfoethyl)amino]ethoxy}tricyclo[3.3.1.1^{3,7}]dec-1-yl)methyl]-5-methyl-1H-pyrazol-4-yl}-6-[8-([1,3]thiazolo[5,4-b|pyridin-2-ylcarbamoyl)-3,4-dihydroisoquinolin-2(1H)-yl]pyridine-2-carboxylic acid (Compound W2.22)

### 1.22.1 tert-butyl 3-(1-((3-(2-azidoethoxy)-5,7-dimethyladamantan-1-yl)methyl)-5-methyl-1H-pyrazol-4-yl)-6-(8-(thiazolo[5,4-b]pyridin-2-ylcarbamoyl)-3,4-dihydroisoquinolin-2(1H)-yl)picolinate

Example 1.2.5 (560 mg) and thiazolo[5,4-b]pyridin-2-amine (135 mg) were dissolved in dichloromethane (12 mL). N,N-Dimethylpyridin-4-amine (165 mg) and N-ethyl-N'-(3-dimethylaminopropyl)carbodiimide hydrochloride (260 mg) were added, and the reaction stirred at room temperature overnight. The reaction mixture was concentrated, and the crude residue was purified by silica gel chromatography, eluting with 65/35 dichloromethane/ethyl acetate, to provide the title compound. MS (ESI) m/e 829.1 (M+H)⁺.

### 1.22.2 tert-butyl 3-(1-((3-(2-aminoethoxy)-5,7-dimethyladamantan-1-yl)methyl)-5-methyl-1H-pyrazol-4-yl)-6-(8-(thiazolo[5,4-b]pyridin-2-ylcarbamoyl)-3,4-dihydroisoquinolin-2(1H)-yl)picolinate

The title compound was prepared by substituting Example 1.22.1 for Example 1.2.6 in Example 1.2.7. MS (ESI) m/e 803.2 (M+H)⁺.

### 1.22.3 tert-butyl 3-[1-(13,5-dimethyl-7-[(2,2,7,7-tetramethyl-10,10-dioxido-3,3-diphenyl-4,9-dioxa-10λ⁶-thia-13-aza-3-silapentadecan-15-yl)oxy]tricyclo [3.3.1.1^{3,7}]dec-1-yl}methyl)-5-methyl-1H-pyrazol-4-yl]-6-[8-([1,3]thiazolo[5,4-b]pyridin-2-ylcarbamoyl)-3,4-dihydroisoquinolin-2(1H)-yl]pyridine-2-carboxylate

To a solution of Example 1.22.2 (70 mg) and 4-((tert-butyldiphenylsilyl)oxy)-2,2-dimethylbutyl ethenesulfonate (48 mg) in dichloromethane (1 mL) was added N,N-diisopropylethylamine (0.06 mL), and the reaction stirred at room temperature overnight. The reaction was concentrated, and the crude residue was purified by silica gel chromatography, eluting with a gradient of 1-4% methanol in dichloromethane, to provide the title compound. MS (ESI) m/e 1249.2 (M+H)⁺.

### 1.22.4 2-((2-((3-((4-(2-(tert-butoxycarbonyl)-6-(8-(thiazolo[5,4-b]pyridin-2-ylcarbamoyl)-3,4-dihydroisoquinolin-2(1H)-yl)pyridin-3-yl)-5-methyl-1H-pyrazol-1-yl)methyl)-5,7-dimethyladamantan-1-yl)oxy)ethyl)amino)ethanesulfonic acid

To a solution of Example 1.22.3 (70 mg) in tetrahydrofuran (0.25 mL) was added tetrabutylammonium fluoride (60 µL, 1.0M solution in tetrahydrofuran), and the reaction was stirred at room temperature for two days. The reaction was concentrated, and the residue was purified by reverse phase chromatography (C18 column), eluting with 10-90% acetonitrile in water containing 0.1% trifluoroacetic acid, to provide the title compound as a trifluoroacetic acid salt. MS (ESI) m/e 911.1 (M+H)⁺.

### 1.22.5 3-{1-[(3,5-dimethyl-7-{2-[(2-sulfoethyl)amino]ethoxy}tricyclo[3.3.1.1^{3,7}]dec-1-yl)methyl]-5-methyl-1H-pyrazol-4-yl}-6-[8-([1,3]thiazolo[5,4-b]pyridin-2-ylcarbarnoyl)-3,4-dihydroisoquinolin-2(1H)-yl]pyridine-2-carboxylic acid

The title compound was prepared by substituting Example 1.22.4 for Example 1.2.8 in Example 1.2.9. ¹H NMR (400 MHz, dimethyl sulfoxide-*d*₆) δ ppm 13.00 (s, 1H), 8.52 (dd, 2H), 8.33 (br s, 2H), 8.16 (dd, 1H), 7.62 (m, 1H), 7.53 (m, 2H), 7.45 (d, 1H), 7.38 (m, 1H), 7.29 (s, 1H), 6.98 (d, 1H), 4.96 (s, 2H), 3.88 (m, 2H), 3.83 (s, 2H), 3.54 (m, 2H), 3.22 (m, 2H), 3.10 (m, 2H), 3.02 (t, 2H), 2.80 (t, 2H), 2.11 (s, 3H), 1.41 (s, 2H), 1.28 (m, 4H), 1.14 (m, 4H), 1.02 (m, 2H), 0.86 (s, 6H). MS (ESI) m/e 855.2 (M+H)⁺.

### 1.23 Synthesis of 3-{1-[(3,5-dimethyl-7-{2-[(2-sulfoethyl)amino]ethoxy}tricyclo[3.3.1.1^{3,7}]dec-1-yl)methyl]-5-methyl-1H-pyrazol-4-yl}-6-[8-([1,3]thiazolo[4,5-b]pyridin-2-ylcarbamoyl)-3,4-dihydroisoquinolin-2(1H)-yl]pyridine-2-carboxylic acid (Compound W2.23)

### 1.23.1 tert-butyl 3-(1-((3-(2-azidoethoxy)-5,7-dimethyladamantan-1-yl)methyl)-5-methyl-1H-pyrazol-4-yl)-6-(8-(thiazolo[4,5-b]pyridin-2-ylcarbamoyl)-3,4-dihydroisoquinolin-2(1H)-yl)picolinate

The title compound was prepared by substituting thiazolo[4,5-b]pyridin-2-amine for thiazolo[5,4-b]pyridin-2-amine in Example 1.22.1. MS (ESI) m/e 855.2 (M+H)⁺.

### 1.23.2 tert-butyl 3-(1-((3-(2-aminoethoxy)-5,7-dimethyladamantan-1-yl)methyl)-5-methyl-1H-pyrazol-4-yl)-6-(8-(thiazolo[4,5-b]pyridin-2-ylcarbamoyl)-3,4-dihydroisoquinolin-2(1H)-yl)picolinate

The title compound was prepared by substituting Example 1.23.1 for Example 1.2.6 in Example 1.2.7. MS (ESI) m/e 803.2 (M+H)⁺.

### 1.23.3 tert-butyl 3-[1-(13,5-dimethyl-7-[(2,2,7,7-tetramethyl-10,10-dioxido-3,3-diphenyl-4,9-dioxa-10λ⁶-thia-13-aza-3-silapentadecan-15-yl)oxy]tricyclo[3.3.1.1^{3,7}]dec-1-yl}methyl)-5-methyl-1H-pyrazol-4-yl]-6-[8-([1,3]thiazolo[4,5-b]pyridin-2-ylcarbamoyl)-3,4-dihydroisoquinonn-2(1H)-yl]pyridine-2-carboxylate

The title compound was prepared by substituting Example 1.23.2 for Example 1.22.2 in Example 1.22.3. MS (ESI) m/e 1249.2 (M+H)⁺.

### 1.23.4 3-{1-[(3,5-dimethyl-7-{2-[(2-sulfoethyl)amino]ethoxy}tricyclo[3.3.1.1^{3,7}]dec-1-yl)methyl]-5-methyl-1H-pyrazol-4-yl}-6-[8-([1,3]thiazolo[4,5-b]pyridin-2-ylcarbarnoyl)-3,4-dihydroisoquinolin-2(1H)-yl]pyridine-2-carboxylic acid

The title compound was prepared by substituting Example 1.23.3 for Example 1.2.8 in Example 1.2.9. ¹H NMR (400 MHz, dimethyl sulfoxide-*d*₆) δ ppm 13.20 (br s, 1H), 8.61 (dd, 1H), 8.56 (dd, 1H), 8.33 (br s, 2H), 7.56 (d, 1H) 7.52 (d, 1H), 7.46 (d, 1H), 7.39 (m, 2H), 7.29 (s, 1H), 6.98 (d, 1H), 4.98 (s, 2H), 3.88 (m, 2H), 3.83 (s, 2H), 3.54 (m, 2H), 3.22 (m, 2H), 3.10 (m, 2H), 3.02 (t, 2H), 2.80 (t, 2H), 2.10 (s, 3H), 1.41 (s, 2H), 1.30 (m, 4H), 1.12 (m, 4H), 1.02 (m, 2H), 0.86 (s, 6H). MS (ESI) m/e 855.1 (M+H)⁺.

### 1.24 Synthesis of 6-[1-(1,3-benzothiazol-2-ylcarbamoyl)-1,2,3,4-tetrahydroquinolin-7-yl]-3-{1-[(3,5-dimethyl-7-12-[(2-sulfoethyl)amino]ethoxy{tricyclo[3.3.1.1^{3,7}]dec-1-yl)methyl]-5-methyl-1H-pyrazol-4-yl}pyridine-2-carboxylic acid (Compound W2.24)

### 1.24.1 tert-butyl 6-[1-(1,3-benzothiazol-2-ylcarbamoyl)-1,2,3,4-tetrahydroquinolin-7-yl]-3-[1-({3,5-dimethyl-7-[(2,2,7,7-tetramethyl-10,10-dioxido-3,3-diphenyl-4,9-dioxa-10λ⁶-thia-13-aza-3-silapentadecan-15-yl)oxy]tricyclo[3.3.1.1^{3,7}]dec-1-yl}methyl)-5-methyl-1H-pyrazol-4-yl]pyridine-2-carboxylate

The title compound was prepared as described in Example 1.2.8, replacing Example 1.2.7 with Example 1.20.9.

### 1.24.2 6-[1-(1,3-benzothiazol-2-ylcarbamoyl)-1,2,3,4-tetrahydroquinolin-7-yl]-3-{1-[(3,5-dimethyl-7-{2-[(2-sulfoethyl)amino]ethoxy}tricyclo[3.3.1.1^{3,7}]dec-1-yl)methyl]-5-methyl-1H-pyrazol-4-yl}pyridine-2-carboxylic acid

The title compound was prepared as described in Example 1.2.9, replacing Example 1.2.8 with Example 1.24.1. ¹H NMR (500 MHz, dimethyl sulfoxide-*d*₆) δ ppm 8.26-8.46 (m, 3H), 8.02 (d, 1H), 7.89 (d, 1H), 7.82 (d, 1H), 7.75-7.79 (m, 1H), 7.47 (s, 2H), 7.37 (t, 1H), 7.30 (d, 1H), 7.22 (t, 1H), 3.96 (s, 2H), 3.90 (s, 2H), 3.54-3.61 (m, 2H), 3.18-3.29 (m, 2H), 3.07-3.15 (m, 2H), 2.78-2.92 (m, 4H), 2.23 (s, 3H), 1.87-2.02 (m, 2H), 1.44 (s, 2H), 1.32 (q, 4H), 1.12-1.25 (m, 4H), 1.00-1.11 (m, 2H), 0.88 (s, 6H). MS (ESI) m/e 854.0 (M+H)⁺.

### 1.25 Synthesis of 6-[8-(1,3-benzothiazol-2-ylcarbamoyl)-3,4-dihydroisoquinolin-2(1H)-yl]-3-{1-[(3-{2-[(2-carboxyethyl)amino]ethoxy}-5,7-dimethyltricyclo[3.3.1.1^{3,7}]dec-1-yl)methyl]-5-methyl-1H-pyrazol-4-yl}pyridine-2-carboxylic acid (W2.25)

### 1.25.1 tert-butyl 6-(8-(benzo[d]thiazol-2-ylcarbamoyl)-3,4-dihydroisoquinolin-2(1H)-yl)-3-(1-((3-(2-((3-(tert-butoxy)-3-oxopropyl)amino)ethoxy)-5, 7-dimethyladamantan-1-yl)methyl)-5-methyl-1H-pyrazol-4-yl)picolinate

The title compound was prepared as described in Example 1.12.1, replacing diethyl vinylphosphonate with tert-butyl acrylate. MS (APCI) m/e 930.6 (M+H)⁺.

### 1.25.2 6-[8-(1,3-benzothiazol-2-ylcarbamoyl)-3,4-dihydroisoquinolin-2(1H)-yl]-3-{1-[(3-{2-[(2-carboxyethyl)amino]ethoxy}-5,7-dimethyltricyclo[3.3.1.1^{3,7}]dec-1-yl)methyl]-5-methyl-1H-pyrazol-4-yl}pyridine-2-carboxylic acid

The title compound was prepared as described in Example 1.6.2, replacing Example 1.6.1 with Example 1.25.1. ¹H NMR (400 MHz, dimethyl sulfoxide-*d*₆) δ ppm 8.03 (d, 1H), 7.78 (d, 1H), 7.61 (d, 1H), 7.39-7.50 (m, 2H), 7.32-7.38 (m, 3H), 7.23 (s, 1H), 6.73 (d, 1H), 4.88 (s, 2H), 3.88 (t, 2H), 3.79 (s, 2H), 2.99 (t, 2H), 2.86-2.93 (m, 2H), 2.50-2.58 (m, 2H), 2.08 (s, 3H), 1.35 (d, 2H), 1.01-1.30 (m, 10H), 0.86 (s, 6H). MS (APCI) m/e 819.0 (M+H)⁺.

### 1.26 Synthesis of 6-[8-(1,3-benzothiazol-2-ylcarbamoyl)-3,4-dihydroisoquinolin-2(1H)-yl]-3-{1-[(3,5-dimethyl-7-{2-[(3-phosphonopropyl)(piperidin-4-yl)amino]ethoxy}tricyclo[3.3.1.1^{3,7}]dec-1-yl)methyl]-5-methyl-1H-pyrazol-4-yl}pyridine-2-carboxylic acid (Compound W2.26)

### 1.26.1 tert-butyl 6-(8-(benzo[d]thiazol-2-ylcarbamoyl)-3,4-dihydroisoquinolin-2(1H)-yl)-3-(1-(((1r,3r)-3-(2-((1-(tert-butoxycarbonyl)piperidin-4-yl)amino)ethoxy)-5, 7-dimethyladamantan-1-yl)methyl)-5-methyl-1H-pyrazol-4-yl)picolinate

A solution of Example 1.2.7 (0.020 g), *tert*-butyl 4-oxopiperidine-1-carboxylate (4.79 mg) and sodium triacetoxyborohydride (7 mg) was stirred in dichloromethane (0.5 mL) at room temperature. The reaction was stirred overnight and purified without workup by silica gel chromatography, eluting with 0 to 10% methanol in dichloromethane, to give the title compound. MS (ELSD) m/e 985.4 (M+H)⁺.

### 1.26.2 6-[8-(1,3-benzothiazol-2-ylcarbamoyl)-3,4-dihydroisoquinolin-2(1H)-yl]-3-{1-[(3,5-dimethyl-7-{2- [(3-phosphonopropyl)(piperidin-4-yl)amino]ethoxy}tricyclo[3.3.1.1^{3,7}]dec-1-yl)methyl]-5-methyl-1H-pyrazol-4-yl}pyridine-2-carboxylic acid

A solution of Example 1.26.1 (0.108 g), Example 1.14.2 (0.030 g) and sodium triacetoxyborohydride (0.035 g) in dichloromethane (1 mL) was stirred at room temperature for 1 hour. Trifluoroacetic acid (1 mL) was added to the reaction, and stirring was continued overnight. The reaction was concentrated, dissolved in N,N-dimethylformamide (2 mL) and water (0.5 mL) and purified by reverse phase HPLC using a Gilson system, eluting with 10-75% acetonitrile in water containing 0.1% v/v trifluoroacetic acid. The desired fractions were combined and freeze-dried to provide the title compound. ¹H NMR (400 MHz, dimethyl sulfoxide-*d*₆) δ ppm 8.83 (s, 1H), 8.50 (s, 1H), 8.04 (d, 2H), 7.80 (d, 2H), 7.63 (d, 2H), 7.56-7.42 (m, 5H), 7.37 (tt, 3H), 7.30 (s, 1H), 6.96 (d, 1H), 4.96 (s, 2H), 3.89 (t, 2H), 3.44 (d, 6H), 3.31-3.16 (m, 6H), 3.09-2.98 (m, 2H), 2.98-2.85 (m, 1H), 2.18 (d, 2H), 2.10 (s, 3H), 2.00-1.74 (m, 4H), 1.71-1.57 (m, 2H), 1.51-0.97 (m, 12H), 0.87 (s, 6H). MS (ESI) m/e 951.2 (M+H)⁺.

### 1.27 Synthesis of 3-{1-[(3-{2-[D-alpha-aspartyl(methyl)amino]ethoxy}-5,7-dimethyltricyclo[3.3.1.1^{3,7}]dec-1-yl)methyl]-5-methyl-1H-pyrazol-4-yl}-6-[8-(1,3-benzothiazol-2-ylcarbamoyl)-3,4-dihydroisoquinolin-2(1H)-yl]pyridine-2-carboxylic acid (Compound W2.27)

### 1.27.1 tert-butyl 6-(8-(benzo[d]thiazol-2-ylcarbamoyl)-3,4-dihydroisoquinolin-2(1H)-yl)-3-(1-((3,5-dimethyl-7-(2-(methylamino)ethoxy)adamantan-1-yl)methyl)-5-methyl-1H-pyrazol-4-yl)picolinate

The title compound was prepared as described in Example 1.11.1 by substituting Example 1.10.9 with Example 1.13.6.

### 1.27.2 3-{1-[(3-{2-[D-alpha-aspartyl(methyl)amino]ethoxy}-5,7-dimethyltricyclo[3.3.1.1^{3,7}]dec-1-yl)methyl]-5-methyl-1H-pyrazol-4-yl}-6-[8-(1,3-benzothiazol-2-ylcarbamoyl)-3,4-dihydroisoquinolin-2(1H)-yl]pyridine-2-carboxylic acid

A solution of Example 1.27.1 (0.074 g), 2-(3H-[1,2,3]triazolo[4,5-b]pyridin-3-yl)-1,1,3,3-tetramethylisouronium hexafluorophosphate(V) (0.038 g), N,N-diisopropylethylamine (0.048 mL) and (R)-4-(tert-butoxy)-2-((tert-butoxycarbonyl)amino)-4-oxobutanoic acid (0.029 g) in dichloromethane (1 mL) was stirred for 2 hours. Trifluoroacetic acid (0.5 mL) was added, and stirring was continued overnight. The reaction was concentrated, dissolved in N,N-dimethylformamide (1.5 mL) and water (0.5 mL), and purified by reverse phase HPLC using a Gilson system, eluting with 10-75% acetonitrile in water containing 0.1% v/v trifluoroacetic acid. The desired fractions were combined and freeze-dried to provide the title compound. ¹H NMR (500 MHz, dimethyl sulfoxide-*d*₆) δ ppm 12.88 (s, 1H), 8.16 (s, 3H), 8.04 (d, 1H), 7.80 (d, 1H), 7.62 (d, 1H), 7.55-7.42 (m, 3H), 7.41-7.33 (m, 2H), 7.33-7.27 (m, 1H), 6.96 (d, 1H), 4.96 (s, 2H), 4.63-4.49 (m, 1H), 3.89 (t, 2H), 3.82 (s, 2H), 3.61-3.37 (m, 4H), 3.10-2.97 (m, 4H), 2.89-2.73 (m, 2H), 2.67-2.52 (m, 1H), 2.10 (s, 3H), 1.45-0.95 (m, 12H), 0.85 (s, 6H). MS (ESI) m/e 875.3 (M+H)⁺.

### 1.28 Synthesis of 6-[8-(1,3-benzothiazol-2-ylcarbamoyl)-3,4-dihydroisoquinolin-2(1H)-yl]-3-(1-{[3-(2-{[1-(carboxymethyl)piperidin-4-yl]amino}ethoxy)-5,7-dimethyltricyclo[3.3.1.1^{3,7}]dec-1-yl]methyl}-5-methyl-1H-pyrazol-4-yl)pyridine-2-carboxylic acid (Compound W2.28)

A solution of Example 1.2.7 (0.055 g,), *tert*-butyl 2-(4-oxopiperidin-1-yl)acetate (0.014 g) and sodium triacetoxyborohydride (0.019 g) was stirred in dichloromethane (0.5 mL) at room temperature. After stirring for 2 hours, trifluoroacetic acid (0.5 mL) was added to the reaction, and stirring was continued overnight. The reaction was concentrated, dissolved in N,N-dimethylformamide (1.5 mL) and water (0.5 mL) and purified by reverse phase HPLC using a Gilson system, eluting with 10-80% acetonitrile in water containing 0.1% v/v trifluoroacetic acid. The desired fractions were combined and freeze-dried to provide the title compound. ¹H NMR (501 MHz, dimethyl sulfoxide-*d*₆) δ ppm 12.85 (s, 1H), 8.80 (s, 2H), 8.03 (d, 1H), 7.80 (d, 1H), 7.62 (d, 1H), 7.55-7.41 (m, 3H), 7.36 (q, 2H), 7.29 (s, 1H), 6.96 (d, 1H), 4.96 (s, 2H), 4.07 (s, 2H), 3.89 (t, 2H), 3.83 (s, 2H), 3.66-3.55 (m, 4H), 3.30 (s, 1H), 3.08 (s, 4H), 3.02 (t, 2H), 2.22 (d, 2H), 2.10 (s, 3H), 1.97-1.78 (m, 2H), 1.44 (s, 2H), 1.31 (q, 4H), 1.20-0.96 (m, 6H), 0.87 (s, 6H). MS (ESI) m/e 887.3 (M+H)⁺.

### 1.29 Synthesis of N-[(5S)-5-amino-6-{[2-({3-[(4-{6-[8-(1,3-benzothiazol-2-ylcarbarnoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](methyl)amino}-6-oxohexyl]-N,N-dimethylmethanaminium (Compound W2.29)

A solution of Fmoc-N-ε-(trimethyl)-L-lysine hydrochloride (0.032 g), 2-(3H-[1,2,3]triazolo[4,5-b]pyridin-3-yl)-1,1,3,3-tetramethylisouronium hexafluorophosphate(V) (0.028 g) and N,N-diisopropylethylamine (0.034 mL) in N,N-dimethylformamide (0.5 mL) was stirred for 5 minutes. The reaction was added to Example 1.13.7 (0.050 g), and stirring was continued at room temperature overnight. Diethylamine (0.069 mL) was added to the reaction, and stirring was continued for an additional 2 hours. The reaction was diluted with N,N-dimethylformamide (1 mL), water (0.5 mL), and trifluoroacetic acid (0.101 mL). The mixture was purified by reverse phase HPLC using a Gilson system, eluting with 10-90% acetonitrile in water containing 0.1% v/v trifluoroacetic acid. The desired fractions were combined and freeze-dried to provide the title compound. ¹H NMR (500 MHz, dimethyl sulfoxide-*d*₆) δ ppm 12.87 (s, 1H), 8.13 (s, 3H), 8.04 (d, 1H), 7.80 (d, 1H), 7.62 (d, 1H), 7.54-7.42 (m, 3H), 7.42-7.34 (m, 2H), 7.29 (s, 1H), 6.96 (d, 1H), 4.96 (s, 2H), 4.42-4.24 (m, 1H), 3.89 (t, 2H), 3.82 (s, 2H), 3.29-3.16 (m, 2H), 3.08-3.00 (m, 15H), 2.87 (s, 2H), 2.10 (s, 3H), 1.84-1.60 (m, 4H), 1.42-0.97 (m, 15H), 0.85 (s, 6H). MS (ESI) m/e 930.3 (M+H)⁺.

### 1.30 Synthesis of 6-[8-(1,3-benzothiazol-2-ylcarbamoyl)-3,4-dihydroisoquinolin-2(1H)-yl]-3-{1-[(3,5-dimethyl-7-{2-[piperidin-4-yl(2-sulfoethyl)amino]ethoxy}tricyclo[3.3.1.1^{3,7}]dee-1-yl)methyl]-5-methyl-1H-pyrazol-4-yl}pyridine-2-carboxylic acid (Compound W2.30)

### 1.30.1 tert-butyl 6-[8-(1,3-benzothiazol-2-ylcarbamoyl)-3,4-dihydroisoquinolin-2(1H)-yl]-3-(1-{[3-({13-[1-(tert-butoxycarbonyl)piperidin-4-yl]-2,2,7,7-tetramethyl-10,10-dioxido-3,3-diphenyl-4,9-dioxa-10λ⁶-thia-13-aza-3-silapentadecan-15-yl}oxy)-5,7-dimethyltricyclo[3.3.1.1^{3,7}]dec-1-yl]methyl}-5-methyl-1H-pyrazol-4-yl)pyridine-2-carboxylate

A solution of Example 1.2.8 (0.111 g), tert-butyl 4-oxopiperidine-1-carboxylate (0.021 g) and sodium triacetoxyborohydride (0.028 g) in dichloromethane (1 mL) was stirred at room temperature for 1 hour. Acetic acid (7.63 µL) was added, and stirring was continued overnight. Additional *tert*-butyl 4-oxopiperidine-1-carboxylate (0.021 g), sodium triacetoxyborohydride (0.028 g) and acetic acid (8 µL) were added to the reaction, and stirring was continued for an additional 4 hours. The reaction was loaded directly onto silica gel and eluted with a gradient of 0.5-4% methanol in dichloromethane to give the title compound.

### 1.30.2 6-[8-(1,3-benzothiazol-2-ylcarbamoyl)-3,4-dihydroisoquinolin-2(1H)-yl]-3-{1-[(3,5-dimethyl-7-{2-[piperidin-4-yl(2-sulfoethyl)amino]ethoxy}tricyclo[3.3.1.1^{3,7}]dec-1-yl)methyl]-5-methyl-1H-pyrazol-4-yl}pyridine-2-carboxylic acid

To a solution of Example 1.30.1 (0.078 g) in dichloromethane (1 mL) was added trifluoroacetic acid (0.5 mL), and the reaction was stirred at room temperature overnight. The reaction was concentrated and dissolved in N,N-dimethylformamide (1.5 mL) and water (0.5 mL). The mixture was purified by reverse phase HPLC using a Gilson system, eluting with 10-75% acetonitrile in water containing 0.1% v/v trifluoroacetic acid. The desired fractions were combined and freeze-dried to provide the title compound. ¹H NMR (400 MHz, dimethyl sulfoxide-*d*₆) δ ppm 12.89 (s, 1H), 9.31 (s, 1H), 8.75 (d, 1H), 8.36-8.19 (m, 1H), 8.08 (d, 1H), 7.84 (d, 1H), 7.66 (d, 1H), 7.58 (d, 1H), 7.55-7.45 (m, 2H), 7.40 (td, 2H), 7.34 (s, 1H), 6.99 (d, 1H), 5.00 (s, 2H), 3.93 (t, 2H), 3.87 (s, 2H), 3.49 (d, 6H), 3.39-3.31 (m, 2H), 3.01 (m, 6H), 2.15 (s, 6H), 1.94 (s, 2H), 1.58-0.99 (m, 12H), 0.91 (s, 6H). MS (ESI) m/e 937.3 (M+H)⁺.

### 1.31 Synthesis of 6-[8-(1,3-benzothiazol-2-ylcarbamoyl)-5-(3-phosphonopropoxy)-3,4-dihydroisoquinolin-2(1H)-yl]-3-[1-({3,5-dimethyl-7-[2-(methylamino)ethoxy]tricyclo[3.3.1.1^{3,7}]dec-1-yl}methyl)-5-methyl-1H-pyrazol-4-yl]pyridine-2-carboxylic acid (Compound W2.31)

### 1.31.1 tert-butyl 8-bromo-5-hydroxy-3,4-dihydroisoquinoline-2(1H)-carboxylate

To a solution of tert-butyl 5-hydroxy-3,4-dihydroisoquinoline-2(1H)-carboxylate (9 g) in N,N-dimethylformamide (150 mL) was added N-bromosuccinimide (6.43 g). The mixture was stirred overnight and quenched with water (200 mL). The mixture was diluted with ethyl acetate (500 mL), washed with water and brine, and dried over sodium sulfate. Evaporation of the solvent gave the title compound, which was used in the next reaction without further purification. MS(ESI) m/e 329.2 (M+H)⁺.

### 1.31.2 tert-butyl 5-(benzyloxy)-8-bromo-3,4-dihydroisoquinoline-2(1H)-carboxylate

To a solution of Example 1.31.1 (11.8 g) in acetone (200 mL) was added benzyl bromide (7.42 g) and K₂CO₃ (5 g), and the mixture was stirred at reflux overnight. The mixture was concentrated, and the residue was partitioned between ethyl acetate (600 mL) and water (200 mL). The organic layer was washed with water and brine, dried over sodium sulfate, filtered and concentrated. The residue was purified by silica gel chromatography, eluting with 10% ethyl acetate in heptane, to provide the title compound. MS (ESI) m/e 418.1 (M+H)⁺.

### 1.31.3 2-tert-butyl 8-methyl 5-(benzyloxy)-3,4-dihydroisoquinoline-2,8(1H)-dicarboxylate

Methanol (100 mL) and triethylamine (9.15 mL) were added to Example 1.31.2 (10.8 g) and [1,1'-bis(diphenylphosphino)ferrocene]dichloropalladium(II) (0.48 g) in a 500 mL stainless steel pressure reactor. The vessel was sparged with argon several times. The reactor was pressurized with carbon monoxide and stirred for 2 hours at 100 °C under 60 psi of carbon monoxide. After cooling, the crude reaction mixture was concentrated under vacuum. The residue was added to ethyl acetate (500 mL) and water (200 mL). The organic layer was further washed with water and brine, dried over sodium sulfate, filtered and concentrated. The residue was purified by silica gel chromatography, eluting with 10-20% ethyl acetate in heptane, to provide the title compound. MS (ESI) m/e 398.1 (M+H)⁺.

### 1.31.4 methyl 5-(benzyloxy)-1,2,3,4-tetrahydroisoquinoline-8-carboxylate hydrochloride

To a solution of Example 1.31.3 (3.78 g) in tetrahydrofuran (20 mL) was added 4N HCl in 1,4-dioxane (20 mL), and the mixture was stirred overnight. The mixture was concentrated under vacuum to give the title compound, which was used in the next reaction without further purification. MS(ESI) m/e 298.1 (M+H)⁺.

### 1.31.5 methyl 5-(benzyloxy)-2-(5-bromo-6-(tert-butoxycarbonyl)pyridin-2-yl)-1,2,3,4-tetrahydroisoquinoline-8-carboxylate

To a solution of Example 1.31.4 (3.03 g) in dimethyl sulfoxide (50 mL) was added Example 1.1.10 (2.52 g) and triethylamine (3.8 mL), and the mixture was stirred at 60 °C overnight under nitrogen. The reaction mixture was diluted with ethyl acetate (500 mL), washed with water and brine, dried over sodium sulfate, filtered and concentrated. The residue was purified by silica gel chromatography, eluting with 20% ethyl acetate in heptane, to give the title compound. MS (ESI) m/e 553.1 (M+H)⁺.

### 1.31.6 tert-butyl (2-((3,5-dimethyl-7-((5-methyl-4-(4,4,5,5-tetramethyl-1,3,2-dioxaborolan-2-yl)-1H-pyrazol-1-yl)methyl)adamantan-1-yl)oxy)ethyl)(methyl)carbamate

To a solution of Example 1.13.3 (2.6 g) and [1,1'-bis(diphenylphosphino)ferrocene]dichloropalladium(II) dichloromethane (190 mg) in acetonitrile (30 mL) was added triethylamine (2.0 mL) and pinacolborane (1.4 mL), and the mixture was stirred at reflux overnight. The mixture was used directly in the next reaction without work up. MS (ESI) m/e 558.4 (M+H)⁺.

### 1.31.7 methyl 5-(benzyloxy)-2-(6-(tert-butoxycarbonyl)-5-(1-((3-(2-((tert-butoxycarbonyl)(methyl)amino)ethoxy)-5, 7-dimethyladamantan-1-yl)methyl)-5-methyl-1H-pyrazol-4-yl)pyridin-2-yl)-1,2,3,4-tetrahydroisoquinoline-8-carboxylate

To a solution of Example 1.31.5 (2.58 g) in tetrahydrofuran (40 mL) and water (20 mL) was added Example 1.31.6 (2.66 g), 1,3,5,7-tetramethyl-6-phenyl-2,4,8-trioxa-6-phosphaadamante (341 mg), tris(dibenzylideneacetone)dipalladium(0) (214 mg), and K₃PO₄ (4.95 g), and the mixture was stirred at reflux for 4 hours. The mixture was diluted with ethyl acetate (500 mL), washed with water and brine, dried over sodium sulfate, filtered and concentrated. The residue was purified by silica gel chromatography, eluting with 20% ethyl acetate in dichloromethane, to provide the title compound. MS (ESI) m/e 904.5 (M+H)⁺.

### 1.31.8 methyl 2-(6-(tert-butoxycarbonyl)-5-(1-((3-(2-((tert-butoxycarbonyl)(methyl)amino)ethoxy)-5, 7-dimethyladamantan-1-yl)methyl)-5-methyl-1H-pyrazol-4-yl)pyridin-2-yl)-5-hydroxy-1,2,3,4-tetrahydroisoquinoline-8-carboxylate

Example 1.31.7 (3.0 g) in tetrahydrofuran (60 mL) was added to Pd(OH)₂ (0.6 g, Degussa #E101NE/W, 20% on carbon, 49% water content) in a 250 mL stainless steel pressure bottle. The mixture was shaken for 16 hours under 30 psi of hydrogen gas at 50 °C. The mixture was filtered through a nylon membrane, and the solvent was evaporated under vacuum to provide the title compound. MS (ESI) m/e 815.1(M+H)⁺.

### 1.31.9 methyl 2-(6-(tert-butoxycarbonyl)-5-(1-((3-(2-((tert-butoxycarbonyl)(methyl)amino)ethoxy)-5,7-dimethyladamantan-1-yl)methyl)-5-methyl-1H-pyrazol-4-yl)pyridin-2-yl)-5-(3-(di-tert-butoxyphosphoryl)propoxy)-1,2,3,4-tetrahydroisoquinoline-8-carboxylate

To a solution of Example 1.31.8 (163 mg) in tetrahydrofuran (10 mL) was added Example 1.14.1 (50.5 mg), triphenylphosphine (52.5 mg) and di-tert-butylazodicarboxylate (46.2 mg), and the mixture was stirred for 3 hours. The mixture was diluted with ethyl acetate (200 mL), washed with water and brine, dried over sodium sulfate, filtered and concentrated. The residue was purified by silica gel chromatography, eluting with 20% ethyl acetate in heptanes followed by 5% methanol in dichloromethane, to provide the title compound. MS (ESI) m/e 1049.2 (M+H)⁺.

### 1.31.10 2-(6-(tert-butoxycarbonyl)-5-(1-((3-(2-((tert-butoxycarbonyl)(methyl)amino)ethoxy)-5, 7-dimethyladamantan-1-yl)methyl)-5-methyl-1H-pyrazol-4-yl)pyridin-2-yl)-5-(3-(di-tert-butoxyphosphoryl)propoxy)-1,2,3,4-tetrahydroisoquinoline-8-carboxylic acid

To a solution of Example 1.31.9 (3 g) in tetrahydrofuran (20 mL), methanol (10 mL) and water (10 mL) was added lithium hydroxide monohydrate (30 mg), and the mixture was stirred at room temperature for 24 hours. The reaction mixture was neutralized with 2% aqueous HCl and concentrated under vacuum. The residue was diluted with ethyl acetate (800 mL), washed with water and brine, and dried over sodium sulfate. Filtration and evaporation of solvent provided the title compound. MS (ESI) m/e 1034.5 (M+H)⁺.

### 1.31.11 6-[8-(1,3-benzothiazol-2-ylcarbamoyl)-5-(3-phosphonopropoxy)-3,4-dihydroisoquinolin-2(1H)-yl]-3-[1-({3,5-dimethyl-7-[2-(methylamino)ethoxy]tricyclo[3.3.1.1^{3,7}]dec-1-yl}methyl)-5-methyl-1H-pyrazol-4-yl]pyridine-2-carboxylic acid

To a solution of Example 1.31.10 (207 mg) in N,N-dimethylformamide (4 mL) was added benzo[d]thiazol-2-amine(45.1mg, 0.3 mmol), fluoro-N,N,N',N'-tetramethylformamidinium hexafluorophosphate (79 mg) and N,N-diisopropylethylamine(150 mg), and the mixture was stirred at 60 °C for 3 hours. The reaction mixture was diluted with ethyl acetate (200 mL,) washed with water and brine, dried over sodium sulfate, filtered and concentrated. The residue was purified by silica gel chromatography, eluting with 20% ethyl acetate in heptane followed by 5% methanol in dichloromethane. After concentration, the material was dissolved in a mixture of dichloromethane and trifluoroacetic acid (1:1, 6 mL) and was allowed to sit at room temperature overnight. The solvent was evaporated, and the residue was dissolved in dimethyl sulfoxide/methanol (1:1, 9 mL). The mixture was purified by reverse phase HPLC using a Gilson system, eluting with 10-85% acetonitrile in water containing 0.1% v/v trifluoroacetic acid, to give the title compound. ¹H NMR (501 MHz, dimethyl sulfoxide-*d*₆) δ ppm 8.27 (s, 2H), 8.02 (d, 1H), 7.76 (dd, 2H), 7.43-7.56 (m, 2H), 7.32-7.37 (m, 1H), 7.29 (s, 1H), 7.00 (dd, 2H), 5.02 (s, 2H), 4.15 (t, 2H), 3.88-3.93 (m, 2H), 3.83 (s, 3H), 3.50-3.59 (m, 4H), 2.95-3.08 (m, 2H), 2.78-2.87 (m, 2H), 2.51-2.55 (m, 3H), 2.11 (s, 3H), 1.90-2.01 (m, 2H), 1.65-1.75 (m, 2H), 1.41 (s, 2H), 1.22-1.36 (m, 6H), 0.98-1.18 (m, 6H), 0.87 (s, 6H). MS (ESI) m/e 898.2 (M+H)⁺.

### 1.32 Synthesis of 6-[8-(1,3-benzothiazol-2-ylcarbamoyl)-3,4-dihydroisoquinolin-2(1H)-yl]-3-(1-{[3-(2-{[N-(2-carboxyethyl)-L-alpha-aspartyl]aminolethoxy)-5,7-dimethyltricyclo[3.3.1.1^{3,7}]dec-1-yl]methyl}-5-methyl-1H-pyrazol-4-yl)pyridine-2-carboxylic acid (Compound W2.32)

### 1.32.1 tert-butyl 6-(8-(benzo[d]thiazol-2-ylcarbamoyl)-3,4-dihydroisoquinolin-2(1H)-yl)-3-(1-((3-(2-((S)-4-(tert-butoxy)-2-((tert-butoxycarbonyl)amino)-4-oxobutanamido)ethoxy)-5,7-dimethyladamantan-1-yl)methyl)-5-methyl-1H-pyrazol-4-yl)picolinate

To a cold (0 °C) solution of (S)-4-(tert-butoxy)-2-((tert-butoxycarbonyl)amino)-4-oxobutanoic acid (136 mg) and O-(7-azabenzotriazol-1-yl)-N,N,N',N'-tetramethyluronium hexafluorophosphate (HATU, 179 mg) in N,N-dimethylformamide (3 mL) was added N,N-diisopropylethylamine (165 µL). The reaction mixture was stirred for 10 minutes, and Example 1.2.7 (252 mg) in N,N-dimethylformamide (1 mL) was added. The mixture was stirred at room temperature for 1.5 hours and was purified by reverse phase chromatography (C18 column), eluting with 50-100% acetonitrile in water containing 0.1% v/v trifluoroacetic acid, to provide the title compound.

### 1.32.2 3-(1-((3-(2-((S)-2-amino-3-carboxypropanamido)ethoxy)-5,7-dimethyladamantan-1-yl)methyl)-5-methyl-1H-pyrazol-4-yl)-6-(8-(benzo[d]thiazol-2-ylcarbamoyl)-3,4-dihydroisoquinolin-2(1H)-yl)picolinic acid

Example 1.32.1 (100 mg) in dichloromethane (3 mL) was treated with trifluoroacetic acid (2.5 mL) overnight. The reaction mixture was concentrated to provide the title compound.

### 1.32.3 6-(8-(benzo[d]thiazol-2-ylcarbamoyl)-3,4-dihydroisoquinolin-2(1H)-yl)-3-(1-((3-(2-((S)-2-((3-(tert-butoxy)-3-oxopropyl)amino)-3-carboxypropanamido)ethoxy)-5,7-dimethyladamantan-1-yl)methyl)-5-methyl-1H-pyrazol-4-yl)picolinic acid

To a mixture of Example 1.32.2 (102 mg) and N,N-diisopropylethylamine (0.21 mL) in N,N-dimethylformamide (1.5 mL) was added tert-butyl acrylate (80 mg) and water (1.5 mL). The mixture was heated at 50 °C for 24 hours and purified by reverse phase chromatography (C18 column), eluting with 20-60% acetonitrile in water containing 0.1% v/v trifluoroacetic acid, to provide the title compound. MS (APCI) m/e 989.1 (M+H)⁺.

### 1.32.4 6-[8-(1,3-benzothiazol-2-ylcarbamoyl)-3,4-dihydroisoquinolin-2(1H)-yl]-3-(1-{[3-(2-{[N-(2-carboxyethyl)-L-alpha-aspartyl]amino}ethoxy)-5,7-dimethyltricyclo[3.3.1.1^{3,7}]dec-1-yl]methyl}-5-methyl-1H-pyrazol-4-yl)pyridine-2-carboxylic acid

The title compound was prepared as described in Example 1.6.2, replacing Example 1.6.1 with Example 1.32.3. ¹H NMR (400 MHz, dimethyl sulfoxide-*d*₆) δ ppm 12.86 (s, 3H), 8.62-9.21 (m, 2H), 8.52 (t, 1H), 8.03 (d, 1H), 7.79 (d, 1H), 7.62 (d, 1H), 7.42-7.53 (m, 3H), 7.33-7.41 (m, 2H), 7.29 (s, 1H), 6.95 (d, 1H), 4.96 (s, 2H), 4.04-4.19 (m, 1H), 3.89 (t, 2H), 3.81 (s, 2H), 3.32-3.41 (m, 2H), 3.16-3.27 (m, 2H), 3.10 (t, 2H), 3.01 (t, 2H), 2.83 (d, 2H), 2.66 (t, 2H), 2.10 (s, 3H), 1.39 (s, 2H), 1.20-1.32 (m, 4H), 0.94-1.16 (m, 6H), 0.85 (s, 6H). MS (ESI) m/e 933.2 (M+H)⁺.

### 1.33 Synthesis of 3-{1-[(3-{2-[(2-aminoethyl)(2-sulfoethyl)amino]ethoxy}-5,7-dimethyltricyclo[3.3.1.1^{3,7}]dec-1-yl)methyl]-5-methyl-1H-pyrazol-4-yl1-6-[8-(1,3-benzothiazol-2-ylcarbamoyl)-3,4-dihydroisoquinolin-2(1H)-yl]pyridine-2-carboxylic acid (Compound W2.33)

### 1.33.1 6-(8-(benzo[d]thiazol-2-ylcarbamoyl)-3,4-dihydroisoquinolin-2(1H)-yl)-3-(1-((3-(2-((2-((tert-butoxycarbonyl)amino)ethyl)(2-sulfoethyl)amino)ethoxy)-5,7-dimethyladamantan-1-yl)methyl)-5-methyl-1H-pyrazol-4-yl)picolinic acid

To a solution of Example 1.2.9 (188 mg), tert-butyl (2-oxoethyl)carbamate (70.1 mg) and N,N-diisopropylethylamine (384 µL) was added sodium triacetoxyborohydride (140 mg), and the mixture was stirred overnight. NaCNBH₃ (13.83 mg) was added. The resulting mixture was stirred for 1 hour, and methanol (1 mL) was added. The mixture was stirred for 10 minutes, diluted with ethyl acetate, and washed with brine. The organic layer was dried over sodium sulfate, filtered and concentrated. The residue was purified by reverse phase chromatography (C18 column), eluting with 20-80% acetonitrile in water containing 0.1% v/v trifluoroacetic acid, to provide the title compound.

### 1.33.2 3-{1-[(3-{2-[(2-aminoethyl)(2-sulfoethyl)amino]ethoxy}-5,7-dimethyltricyclo[3.3.1.1^{3,7}]dec-1-yl)methyl]-5-methyl-1H-pyrazol-4-yl}-6-[8-(1,3-benzothiazol-2-ylcarbamoyl)-3,4-dihydroisoquinolin-2(1H)-yl]pyridine-2-carboxylic acid

The title compound was prepared as described in Example 1.6.2, replacing Example 1.6.1 with Example 1.33.1. ¹H NMR (400 MHz, dimethyl sulfoxide-*d*₆) δ ppm 12.85 (s, 1H), 8.03 (d, 1H), 7.87 (s, 2H), 7.79 (d, 1H), 7.62 (d, 1H), 7.41-7.56 (m, 3H), 7.33-7.40 (m, 2H), 7.29 (s, 1H), 6.96 (d, 1H), 4.96 (s, 2H), 3.89 (t, 2H), 3.50 (s, 2H), 3.29-3.40 (m, 4H), 3.19 (s, 2H), 3.01 (t, 2H), 2.94 (t, 2H), 2.11 (s, 3H), 1.43 (s, 2H), 1.25-1.37 (m, 4H), 0.98-1.19 (m, 6H), 0.87 (s, 6H). MS (ESI) m/e 897.2 (M+H)⁺.

### 1.34 Synthesis of 6-[5-(2-aminoethoxy)-8-(1,3-benzothiazol-2-ylcarbamoyl)-3,4-dihydroisoquinolin-2(1H)-yl]-3-[1-({3,5-dimethyl-7- [2-(methylamino)ethoxy]tricyclo [3.3.1.1^{3,7}]dec-1-yl}methyl)-5-methyl-1H-pyrazol-4-yl]pyridine-2-carboxylic acid (Compound W2.34)

### 1.34.1 methyl 5-(2-(((benzyloxy)carbonyl)amino)ethoxy)-2-(6-(tert-butoxycarbonyl)-5-(1-((3-(2-((tert-butoxycarbonyl)(methyl)amino)ethoxy)-5,7-dimethyladamantan-1-yl)methyl)-5-methyl-1H-pyrazol-4-yl)pyridin-2-yl)-1,2,3,4-tetrahydroisoquinoline-8-carboxylate

To a mixture of Example 1.31.8 (500 mg), benzyl (2-hydroxyethyl)carbamate (180 mg) and triphenyl phosphine (242 mg) in tetrahydrofuran (9 mL) was added (E)-di-tert-butyl diazene-1,2-dicarboxylate (212 mg). The mixture was stirred for 2 hours, diluted with ethyl acetate and washed with water and brine. The organic layer was dried over sodium sulfate, filtered, and concentrated. The residue was purified by silica gel chromatography, eluting with 50-100% ethyl acetate in heptanes, to provide the title compound. MS (APCI) m/e 991.1 (M+H)⁺.

### 1.34.2 5-(2-(((benzyloxy)carbonyl)amino)ethoxy)-2-(6-(tert-butoxycarbonyl)-5-(1-((3-(2-((tert-butoxycarbonyl)(methyl)amino)ethoxy)-5,7-dimethyladamantan-1-yl)methyl)-5-methyl-1H-pyrazol-4-yl)pyridin-2-yl)-1,2,3,4-tetrahydroisoquinoline-8-carboxylic acid

To a solution of Example 1.34.1 (480 mg) in tetrahydrofuran (10 mL) and methanol (5 mL) was added 1 M lithium hydroxide (1.94 mL). The mixture was heated at 50 °C overnight, cooled, acidified with 10% aqueous HCl to pH 3 and concentrated. The residue was purified by reverse phase chromatography (C18 column), eluting with 40-99% acetonitrile in water containing 0.1% v/v trifluoroacetic acid, to provide the title compound. MS (ESI) m/e 977.4 (M+H)⁺.

### 1.34.3 tert-butyl 6-(8-(benzo[d]thiazol-2-ylcarbamoyl)-5-(2-(((benzyloxy)carbonyl)amino)ethoxy)-3,4-dihydroisoquinolin-2(1H)-yl)-3-(1-((3-(2-((tert-butoxycarbonyl)(methyl)amino)ethoxy)-5,7-dimethyladamantan-1-yl)methyl)-5-methyl-1H-pyrazol-4-yl)picolinate

To a mixture of Example 1.34.2 (245 mg), benzo[d]thiazol-2-amine (151 mg) and fluoro-N,N,N',N'-tetramethylformamidinium hexafluorophosphate (TFFH) (132 mg) in N,N-dimethylformamide (3 mL) was added N,N-diisopropylethylamine (876 µL). The reaction mixture was heated at 65 °C for 24 hours, cooled, diluted with ethyl acetate and washed with water and brine. The organic layer was dried over sodium sulfate, filtered and concentrated. The residue was purified by silica gel chromatography, eluting with 0-80% ethyl acetate in heptanes, to provide the title compound. MS (APCI) m/e 1109.5 (M+H)⁺.

### 1.34.4 6-[5-(2-aminoethoxy)-8-(1,3-benzothiazol-2-ylcarbamoyl)-3,4-dihydroisoquinolin-2(1H)-yl]-3-[1-({3,5-dimethyl-7-[2-(methylamino)ethoxy]tricyclo[3.3.1.1^{3,7}]dec-1-yl}methyl)-5-methyl-1H-pyrazol-4-yl]pyridine-2-carboxylic acid

Example 1.34.3 (100 mg) in dichloromethane (0.5 mL) was treated with trifluoroacetic acid (10 mL) overnight. The reaction mixture was concentrated and purified by reverse phase chromatography (C18 column), eluting with 20-60% acetonitrile in water containing 0.1% v/v trifluoroacetic acid, to provide the title compound. ¹H NMR (400 MHz, dimethyl sulfoxide-*d*₆) δ ppm 12.75 (s, 2H), 8.27 (s, 2H), 7.89-8.09 (m, 4H), 7.77 (s, 2H), 7.44-7.53 (m, 2H), 7.35 (t, 1H), 7.29 (s, 1H), 7.02 (dd, 2H), 5.02 (s, 2H), 4.27 (t, 2H), 3.87-3.97 (m, 2H), 3.83 (s, 2H), 3.50-3.58 (m, 2H), 3.00 (s, 2H), 2.88-2.96 (m, 2H), 2.52-2.60 (m, 2H), 2.10 (s, 3H), 1.42 (s, 2H), 1.23-1.36 (m, 4H), 0.98-1.19 (m, 6H), 0.87 (s, 6H). MS (ESI) m/e 819.3 (M+H)⁺.

### 1.35 Synthesis of 6-[8-(1,3-benzothiazol-2-ylcarbamoyl)naphthalen-2-yl]-3-{1-[(3,5-dimethyl-7-{2-[(3-sulfopropyl)amino]ethoxy}tricyclo[3.3.1.1^{3,7}]dec-1-yl)methyl]-5-methyl-1H-pyrazol-4-yl}pyridine-2-carboxylic acid (Compound W2.35)

### 1.35.1 tert-butyl 6-chloro-3-(1-((3,5-dimethyl-7-(2-oxoethoxy)adamantan-1-yl)methyl)-5-methyl-1H-pyrazol-4-yl)picolinate

To a solution of oxalyl chloride (8 mL, 2.0 M in dichloromethane) in dichloromethane (20 mL) at-78 °C, was added dropwise dimethyl sulfoxide (1 mL) in dichloromethane (10 mL) over 20 minutes. The solution was stirred for 30 minutes under argon, and Example 1.20.2 (3.8 g) as a solution in dichloromethane (30 mL) was added over 10 minutes. The reaction mixture was stirred at-78 °C for an additional 60 minutes. Triethylamine (2 mL) was added at-78 °C, and the reaction mixture was stirred for 60 minutes. The cooling bath was removed, and the reaction allowed to warm to room temperature overnight. Water (60 mL) was added. The aqueous layer was acidified with 1% aqueous HCl solution and extracted with dichloromethane. The combined organic layers were washed with 1% aqueous HCl solution, aqueous NaHCO₃ solution, and brine. The organic layer was dried over sodium sulfate and concentrated to provide the title compound. MS (ESI) m/e 527.9 (M+H)⁺.

### 1.35.2 2,2,2-trifluoro-1-(p-tolyl)ethyl 3-iodopropane-1-sulfonate

The title compound was prepared according to a procedure reported in J. Org. Chem., 2013, 78, 711-716.

### 1.35.3 2,2,2-trifluoro-1-(p-tolyl)ethyl 3-aminopropane-1-sulfonate

A solution of Example 1.35.2 (2.0 g) in 7 N ammonia in methanol (20 mL) was heated to 80 °C under microwave conditions (Biotage Initiator) for 45 minutes. The mixture was concentrated, and the residue was dissolved in ethyl acetate (300 mL). The organic layer was washed with water and brine, dried over sodium sulfate, filtered, and concentrated to provide the title compound. MS (ESI) m/e 312.23 (M+H)⁺.

### 1.35.4 tert-butyl 6-chloro-3-(1-(((3,5-dimethyl-7-(2-((3-((2,2,2-trifluoro-1-(p-tolyl)ethoxy)sulfonyl)propyl)amino)ethoxy)adamantan-1-yl)methyl)-5-methyl-1H-pyrazol-4-yl)picolinate

To a solution of Example 1.35.3 (1.96 g) in dichloroethane (30 mL) was added Example 1.35.1 (3.33 g). The reaction mixture was stirred at room temperature for 1 hour, and a suspension of NaBH₄ (1.2 g) in methanol (8 mL) was added. The mixture was stirred at room temperature for 3 hours and diluted with ethyl acetate (300 mL). The organic layer was washed with 2N aqueous NaOH, water, and brine, dried over sodium sulfate, filtered and concentrated. The residue was dissolved in tetrahydrofuran (30 mL), and di-tert-butyl dicarbonate (2 g) was added followed by the addition of catalytic amount of 4-dimethylaminopyridine. The mixture was stirred at room temperature overnight. The mixture was diluted with ethyl acetate (300 mL) and washed with water and brine. The organic layer was dried over sodium sulfate, filtered, and concentrated to provide the title compound. MS (ESI) m/e 924,42 (M+H)⁺.

### 1.35.5 7-(6-(tert-butoxycarbonyl)-5-(1-((3-(2-((tert-butoxycarbonyl)(3-((2,2,2-trifluoro-1-(p-tolyl)ethoxy)sulfonyl)propyl)amino)ethoxy)-5,7-dimethyladamantan-1-yl)methyl)-5-methyl-1H-pyrazol-4-yl)pyridin-2-yl)-1-naphthoic acid

To a solution of methyl **7-(4,4,5,5-tetramethyl-1,3,2-dioxaborolan-2-yl)-1-naphthoate** (203 mg) in a mixture of 1,4-dioxane (10 mL) and water (5 mL) was added Example 1.35.4 (600 mg), bis(triphenylphosphine)palladium(II)dichloride (45.6 mg), and cesium fluoride (296 mg). The mixture was heated at 120 °C under microwave conditions (Biotage Initiator) for 30 minutes, diluted with ethyl acetate (200 mL), and washed with water and brine. The organic layer was dried over sodium sulfate, filtered, and concentrated. The residue was purified by silica gel chromatography, eluting with 20% ethyl acetate in heptane, to provide an ester intermediate. The residue was dissolved in a mixture of tetrahydrofuran (8 mL), methanol (4 mL) and water (4 mL), and was treated with lithium hydroxide monohydrate (200 mg) for 3 hours. The reaction was acidified with 1N aqueous HCl to pH 4 and was diluted with ethyl acetate (400 mL). The resulting mixture was washed with water and brine. The organic layer was dried over sodium sulfate, filtered, and concentrated to provide the title compound. MS (ESI) m/e 1060.24 (M+H)⁺.

### 1.35.6 6-[8-(1,3-benzothiazol-2-ylcarbamoyl)naphthalen-2-yl]-3-{1-[(3,5-dimethyl-7-{2-[(3-sulfopropyl)amino]ethoxy}tricyclo[3.3.1.1^{3,7}]dec-1-yl)methyl]-5-methyl-1H-pyrazol-4-yl}pyridine-2-carboxylic acid

To a solution of Example 1.35.5 (405 mg) in dichloromethane (10 mL) was added benzo[d]thiazol-2-amine (57.4 mg), 1-ethyl-3-[3-(dimethylamino)propyl]-carbodiimide hydrochloride (146 mg) and 4-(dimethylamino)pyridine (93 mg). The mixture was stirred at room temperature overnight, diluted with ethyl acetate (200 mL), and washed with water and brine. The organic layer was dried over sodium sulfate, filtered, and concentrated. The residue was dissolved in dichloromethane (3 mL) and treated with trifluoroacetic acid (3 mL) overnight. The reaction mixture was concentrated, and the residue was purified by reverse phase HPLC (Gilson system), eluting with a gradient of 10-85% acetonitrile in water containing 0.1% v/v trifluoroacetic acid, to provide the title compound. ¹H NMR (400 MHz, dimethyl sulfoxide-*d*₆) δ ppm 13.08 (s, 1H), 9.00 (s, 1H), 8.53 (s, 2H), 8.36 (dd, 1H), 8.26-8.13 (m, 3H), 8.06 (dd, 1H), 8.04-7.97 (m, 1H), 7.94 (d, 1H), 7.80 (d, 1H), 7.69 (dd, 1H), 7.51-7.43 (m, 2H), 7.40-7.31 (m, 1H), 7.19 (d, 0H), 3.88 (s, 2H), 3.54 (t, 2H), 3.16-2.91 (m, 4H), 2.68-2.55 (m, 2H), 2.29 (s, 0H), 2.22 (s, 3H), 1.93 (p, 2H), 1.43 (s, 2H), 1.38-1.23 (m, 4H), 1.10 (dq, 6H), 0.87 (s, 6H). MS (ESI) m/e 863.2 (M+H)⁺.

### 1.36 Synthesis of 6-[8-(1,3-benzothiazol-2-ylcarbamoyl)-3,4-dihydroisoquinolin-2(1H)-yl]-3-{1-[(3-{2-[(2-carboxyethyl)(piperidin-4-yl)amino]ethoxy}-5,7-dimethyltricyclo[3.3.1.1^{3,7}]dec-1-yl)methyl]-5-methyl-1H-pyrazol-4-yl}pyridine-2-carboxylic acid (Compound W2.36)

### 1.36.1 tert-butyl 6-(8-(benzo[d]thiazol-2-ylcarbamoyl)-3,4-dihydroisoquinolin-2(1H)-yl)-3-(1-(((1r,3r)-3-(2-((3-(tert-butoxy)-3-oxopropyl)(1-(tert-butoxycarbonyl)piperidin-4-yl)amino)ethoxy)-5,7-dimethyladamantan-1-yl)methyl)-5-methyl-1H-pyrazol-4-yl)picolinate

A solution of Example 1.25.1 (0.086 g), tert-butyl 4-oxopiperidine-1-carboxylate (0.037 g), sodium triacetoxyborohydride (0.039 g) and acetic acid (11 µL) in dichloromethane (1 mL) was stirred at room temperature. After stirring overnight, the reaction was loaded onto silica gel and eluted using a gradient of 0.5 to 5% methanol in dichloromethane to give the title compound. MS (ELSD) m/e 1113.5 (M+H)⁺.

### 1.36.2 6-[8-(1,3-benzothiazol-2-ylcarbamoyl)-3,4-dihydroisoquinolin-2(1H)-yl]-3-{1-[(3-{2-[(2-carboxyethyl)(piperidin-4-yl)amino]ethoxy}-5,7-dimethyltricyclo[3.3.1.1^{3,7}]dec-1-yl)methyl]-5-methyl-1H-pyrazol-4-yl}pyridine-2-carboxylic acid

A solution of Example 1.36.1 (0.050) in dichloromethane (0.5 mL) was treated with trifluoroacetic acid (0.5 mL), and the reaction was stirred overnight. The reaction was concentrated and dissolved in dimethyl sulfoxide and methanol (1:1). The mixture was purified by reverse phase HPLC using a Gilson system, eluting with 10-75% acetonitrile in water containing 0.1% v/v trifluoroacetic acid. The desired fractions were combined and freeze-dried to provide the title compound. ¹H NMR (400 MHz, dimethyl sulfoxide-*d*₆) δ ppm 12.84 (s, 1H), 9.38 (s, 1H), 8.78 (s, 1H), 8.42 (s, 1H), 8.03 (d, 1H), 7.80 (d, 1H), 7.63 (d, 1H), 7.55-7.42 (m, 3H), 7.41-7.33 (m, 2H), 7.30 (s, 1H), 6.96 (d, 1H), 4.96 (s, 2H), 3.89 (t, 2H), 3.83 (s, 2H), 3.73-3.54 (m, 3H), 3.53-3.34 (m, 4H), 3.34-3.25 (m, 2H), 3.02 (t, 2H), 2.99-2.85 (m, 2H), 2.78 (t, 2H), 2.23-2.04 (m, 5H), 1.92-1.76 (m, 2H), 1.43 (s, 2H), 1.39-1.23 (m, 4H), 1.23-0.96 (m, 6H), 0.87 (s, 6H). MS (ESI) m/e 901.3 (M+H)⁺.

### 1.37 Synthesis of 6-[8-(1,3-benzothiazol-2-ylcarbamoyl)-3,4-dihydroisoquinolin-2(1H)-yl]-3-{1-[(3,5-dimethyl-7-{2-[(3-sulfo-L-alanyl)(2-sulfoethyl)amino]ethoxy}tricyclo[3.3.1.1^{3,7}]dec-1-yl)methyl]-5-methyl-1H-pyrazol-4-yl}pyridine-2-carboxylic acid (Compound W2.37)

A solution of (R)-2-((((9H-fluoren-9-yl)methoxy)carbonyl)amino)-3-sulfopropanoic acid (0.011 g) and 2-(3H-[1,2,3]triazolo[4,5-b]pyridin-3-yl)-1,1,3,3-tetramethylisouronium hexafluorophosphate(V) (10.80 mg) in N,N-dimethylformamide (0.5 mL) was stirred for 5 minutes. This solution was added to Example 1.2.9 (0.025 g) and N,N-diisopropylethylamine (0.014 mL). After stirring for 2 hours, diethylamine (0.013 mL) was added to the reaction, and stirring was continued for an additional 1 hour. The reaction was diluted with N,N-dimethylformamide and water and quenched with trifluoroacetic acid. The mixture was purified by reverse phase HPLC using a Gilson system, eluting with 10-75% acetonitrile in water containing 0.1% v/v trifluoroacetic acid. The desired fractions were combined and freeze-dried to provide the title compound. ¹H NMR (400 MHz, dimethyl sulfoxide-*d*₆) δ ppm 12.84 (s, 1H), 8.03 (dd, 4H), 7.79 (d, 1H), 7.62 (d, 1H), 7.54 (dd, 1H), 7.51-7.41 (m, 2H), 7.36 (td, 2H), 7.33 (s, 1H), 6.98 (dd, 1H), 4.96 (s, 2H), 4.42 (dd, 2H), 3.89 (t, 2H), 3.83 (s, 2H), 3.73 (ddd, 2H), 3.57-3.38 (m, 2H), 3.31 (dt, 1H), 3.08 (dd, 1H), 3.02 (t, 2H), 2.87 (tt, 1H), 2.81-2.54 (m, 2H), 2.10 (d, 3H), 1.51-0.91 (m, 12H), 0.85 (s, 6H). MS (ESI) m/e 1005.2 (M+H)⁺.

### 1.38 Synthesis of 6-[8-(1,3-benzothiazol-2-ylcarbamoyl)-3,4-dihydroisoquinolin-2(1H)-yl]-3-{1-[(3-{2-[{2-[(2-carboxyethyl)amino]ethyl}(2-sulfoethyl)amino]ethoxy}-5,7-dimethyltricyclo[3.3.1.1^{3,7}]dec-1-yl)methyl]-5-methyl-1H-pyrazol-4-yl}pyridine-2-carboxylic acid (Compound W2.38)

### 1.38.1 6-(8-(benzo[d]thiazol-2-ylcarbamoyl)-3,4-dihydroisoquinolin-2(1H)-yl)-3-(1-((3-(2-((2-((3-(tert-butoxy)-3-oxopropyl)amino)ethyl)(2-sulfoethyl)amino)ethoxy)-5,7-dimethyladamantan-1-yl)methyl)-5-methyl-1H-pyrazol-4-yl)picolinic acid

The title compound was prepared as described in Example 1.32.3, replacing Example 1.32.2 with Example 1.33.2.

### 1.38.2 6-[8-(1,3-benzothiazol-2-ylcarbamoyl)-3,4-dihydroisoquinolin-2(1H)-yl]-3-{1-[(3-{2-[{2-[(2-carboxyethyl)amino]ethyl}(2-sulfoethyl)amino]ethoxy}-5,7-dimethyltricyclo[3.3.1.1^{3,7}]dec-1-yl)methyl]-5-methyl-1H-pyrazol-4-yl}pyridine-2-carboxylic acid

The title compound was prepared as described in Example 1.6.2, replacing Example 1.6.1 with Example 1.38.1. ¹H NMR (501 MHz, dimethyl sulfoxide-*d*₆) δ ppm 12.87 (s, 1H), 8.68 (s, 2H), 8.04 (d, 1H), 7.79 (d, 1H), 7.62 (d, 1H), 7.53 (d, 1H), 7.42-7.50 (m, 2H), 7.33-7.40 (m, 2H), 7.29 (s, 1H), 6.96 (d, 1H), 4.96 (s, 3H), 3.89 (t, 2H), 3.83 (s, 2H), 3.66 (t, 2H), 3.31-3.53 (m, 8H), 3.18 (t, 2H), 3.02 (t, 2H), 2.95 (t, 2H), 2.67 (t, 2H), 2.11 (s, 3H), 1.43 (s, 2H), 1.22-1.37 (m, 6H), 0.98-1.19 (m, 6H), 0.87 (s, 6H). MS (APCI) m/e 971.0 (M+H)⁺.

### 1.39 Synthesis of 3-{1-[(3,5-dimethyl-7-{2-[(3-phosphonopropyl)amino]ethoxy}tricyclo[3.3.1.1^{3,7}]dec-1-yl)methyl]-5-methyl-1H-pyrazol-4-yl}-6-[8-([1,3]thiazolo[4,5-b]pyridin-2-ylcarbarnoyl)-3,4-dihydroisoquinolin-2(1H)-yl]pyridine-2-carboxylic acid (Compound W2.39)

### 1.39.1 tert-butyl 3-(1-((3-(2-((3-(di-tert-butoxyphosphoryl)propyl)amino)ethoxy)-5, 7-dimethyladamantan-1-yl)methyl)-5-methyl-1H-pyrazol-4-yl)-6-(8-(thiazolo[4,5-b]pyridin-2-ylcarbamoyl)-3,4-dihydroisoquinolin-2(1H)-yl)picolinate

Example 1.23.2 (520 mg) and Example 1.14.2 (175 mg) were dissolved in dichloromethane (6 mL) and stirred at room temperature for two hours. A suspension of sodium borohydride (32 mg) in methanol (1 mL) was added, and the mixture was stirred for 30 minutes. The reaction was added to saturated aqueous NaHCO₃ solution and extracted with ethyl acetate. The organic layer was washed with brine and dried over sodium sulfate. After filtration and concentration, purification by silica gel chromatography, eluting with a gradient of 0.5-5.0% methanol in dichloromethane, gave the title compound. MS (ESI) m/e 1037.3 (M+H)⁺.

### 1.39.2 3-{1-[(3,5-dimethyl-7-{2-[(3-phosphonopropyl)amino]ethoxy}tricyclo[3.3.1.1^{3,7}]dec-1-yl)methyl]-5-methyl-1H-pyrazol-4-yl}-6-[8-([1,3]thiazolo[4,5-b]pyridin-2-ylcarbamoyl)-3,4-dihydroisoquinolin-2(1H)-yl]pyridine-2-carboxylic acid

The title compound was prepared by substituting Example 1.39.1 for Example 1.2.8 in Example 1.2.9. ¹H NMR (400 MHz, dimethyl sulfoxide-*d*₆) δ ppm 8.60 (dd, 1H), 8.52 (dd, 1H), 8.41 (br s, 2H), 7.65 (d, 1H) 7.48 (d, 1H), 7.46 (d, 1H), 7.38 (m, 2H), 7.29 (s, 1H), 6.97 (d, 1H), 4.97 (s, 2H), 3.89 (m, 2H), 3.83 (s, 2H), 3.56 (m, 2H), 3.02 (m, 6H), 2.11 (s, 3H), 1.81 (m, 2H), 1.61 (m, 2H), 2.11 (s, 3H), 1.43 (s, 2H), 1.30 (m, 4H), 1.14 (m, 4H), 1.04 (m, 2H), 0.87 (s, 6H). MS (ESI) m/e 869.2 (M+H)⁺.

### 1.40 Synthesis of 3-{1-[(3,5-dimethyl-7-{2-[(3-phosphonopropyl)amino]ethoxy}tricyclo[3.3.1.1^{3,7}]dec-1-yl)methyl]-5-methyl-1H-pyrazol-4-yl}-6-[8-([1,3]thiazolo[5,4-b]pyridin-2-ylcarbarnoyl)-3,4-dihydroisoquinolin-2(1H)-yl]pyridine-2-carboxylic acid (Compound W2.40)

### 1.40.1 tert-butyl 3-(1-((3-(2-((3-(di-tert-butoxyphosphoryl)propyl)amino)ethoxy)-5,7-dimethyladamantan-1-yl)methyl)-5-methyl-1H-pyrazol-4-yl)-6-(8-(thiazolo[5,4-b]pyridin-2-ylcarbamoyl)-3,4-dihydroisoquinolin-2(1H)-yl)picolinate

The title compound was prepared by substituting Example 1.22.2 for Example 1.23.2 in Example 1.39.1. MS (ESI) m/e 1037.3 (M+H)⁺.

### 1.40.2 3-{1-[(3,5-dimethyl-7-{2-[(3-phosphonopropyl)amino]ethoxy}tricyclo[3.3.1.1^{3,7}]dec-1-yl)methyl]-5-methyl-1H-pyrazol-4-yl}-6-[8-([1,3]thiazolo[5,4-b]pyridin-2-ylcarbamoyl)-3,4-dihydroisoquinolin-2(1H)-yl]pyridine-2-carboxylic acid

The title compound was prepared by substituting Example 1.40.1 for Example 1.2.8 in Example 1.2.9. ¹H NMR (500 MHz, dimethyl sulfoxide-*d*₆) δ ppm 8.52 (dd, 2H), 8.41 (br s, 2H), 8.17 (dd, 1H), 7.63 (m, 1H), 7.53 (m, 2H), 7.46 (d, 1H), 7.38 (t, 1H), 7.30 (s, 1H), 6.98 (d, 1H), 4.96 (s, 2H), 3.88 (m, 2H), 3.83 (s, 2H), 3.56 (t, 2H), 3.00 (m, 6H), 2.11 (s, 3H), 1.81 (m, 2H), 1.60 (m, 2H), 1.43 (s, 2H), 1.31 (m, 4H), 1.14 (m, 4H), 1.04 (m, 2H), 0.87 (s, 6H). MS (ESI) m/e 869.2 (M+H)⁺.

### 1.41 Synthesis of 6-[8-(1,3-benzothiazol-2-ylcarbamoyl)-5-(carboxymethoxy)-3,4-dihydroisoquinolin-2(4H)-yl]-3-[1-({3,5-dimethyl-7-[2-(methylamino)ethoxy]tricyclo[3.3.1.1^{3,7}]dec-1-yl}methyl)-5-methyl-1H-pyrazol-4-yl]pyridine-2-carboxylic acid (Compound W2.41)

### 1.41.1 methyl 5-(2-(tert-butoxy)-2-oxoethoxy)-2-(6-(tert-butoxycarbonyl)-5-(1-((3-(2-((tert-butoxycarbonyl)(methyl)amino)ethoxy)-5,7-dimethyladamantan-1-yl)methyl)-5-methyl-1H-pyrazol-4-yl)pyridin-2-yl)-1,2,3,4-tetrahydroisoquinoline-8-carboxylate

To a solution of Example 1.31.8 (163 mg) in N,N-dimethylformamide (10 mL) was added tert-butyl 2-bromoacetate (58.6 mg), and K₂CO₃ (83 mg), and the reaction was stirred overnight. The mixture was diluted with ethyl acetate (200 mL), washed with water and brine, and dried over sodium sulfate. Filtration and evaporation of the solvent gave a residue that was purified by silica gel chromatography, eluting with 20% ethyl acetate in heptane, to provide the title compound. MS (ESI) m/e 929.2 (M+H)⁺.

### 1.41.2 5-(2-(tert-butoxy)-2-oxoethoxy)-2-(6-(tert-butoxycarbonyl)-5-(1-((3-(2-((tert-butoxycarbonyl)(methyl)amino)ethoxy)-5,7-dimethyladamantan-1-yl)methyl)-5-methyl-1H-pyrazol-4-yl)pyridin-2-yl)-1,2,3,4-tetrahydroisoquinoline-8-carboxylic acid

To a solution of Example 1.41.1 (3 g) in tetrahydrofuran (20 mL), methanol (10 mL) and water (10 mL) was added lithium hydroxide monohydrate (300 mg). The mixture was stirred at room temperature for 24 hours. The reaction mixture was neutralized with 2% aqueous HCl solution and concentrated under vacuum. The residue was diluted with ethyl acetate (800 mL), washed with water and brine, and dried over sodium sulfate. Filtration and evaporation of the solvent provided the title compound. MS (ESI) m/e 914.5 (M+H)⁺.

### 1.41.3 6-[8-(1,3-benzothiazol-2-ylcarbamoyl)-5-(carboxymethoxy)-3,4-dihydroisoquinolin-2(1H)-yl]-3-[1-({3,5-dimethyl-7-[2-(methylamino)ethoxy]tricyclo[3.3.1.1^{3,7}]dec-1-yl}methyl)-5-methyl-1H-pyrazol-4-yl]pyridine-2-carboxylic acid

To a solution of Example 1.41.2 (183 mg) in N,N-dimethylformamide (4 mL) was added benzo[d]thiazol-2-amine (45.1 mg), fluoro-N,N,N',N'- tetramethylformamidinium hexafluorophosphate (79 mg) and N,N-diisopropylethylamine (0.203 mL). The mixture was stirred at 60 °C overnight. The mixture was diluted with ethyl acetate (300 mL), washed with water and brine, and dried over sodium sulfate. Filtration and evaporation of the solvent gave a residue that was dissolved in dichloromethane/trifluoroacetic acid (1:1, 10 mL) and stirred overnight. The mixture was concentrated, and the residue was purified by reverse phase HPLC using a Gilson system, eluting with 10-85% acetonitrile in in water containing 0.1% v/v trifluoroacetic acid, to provide the title compound. ¹H NMR (400 MHz, dimethyl sulfoxide-*d*₆) δ ppm 12.73 (s, 1H), 8.30 (s, 2H), 7.99-8.07 (m, 1H), 7.75-7.79 (m, 1H), 7.70 (d, 1H), 7.44-7.56 (m, 2H), 7.30-7.39 (m, 2H), 7.30 (s, 1H), 7.03 (t, 1H), 6.87-6.93 (m, 1H), 4.98-5.18 (m, 4H), 4.84 (s, 3H), 3.78-4.01 (m, 4H), 3.55 (t, 2H). 2.77-3.07 (m, 4H), 2.53-2.61 (m, 3H), 2.04-2.16 (m, 3H), 1.41 (s, 2H), 1.02-1.34 (m, 6H), 0.83-0.91 (m, 6H). MS (ESI) m/e 834.2 (M+H)⁺.

### 1.42 Synthesis of 6-[8-(1,3-benzothiazol-2-ylcarbamoyl)-3,4-dihydroisoquinolin-2(1H)-yl]-3-{1-[(3-{2-[(3-carboxypropyl)(piperidin-4-yl)amino]ethoxy}-5,7-dimethyltricyclo[3.3.1.1^{3,7}]dec-1-yl)methyl]-5-methyl-1H-pyrazol-4-yl}pyridine-2-carboxylic acid (Compound W2.42)

### 1.42.1 tert-butyl 6-(8-(benzo[d]thiazol-2-ylcarbamoyl)-3,4-dihydroisoquinolin-2(1H)-yl)-3-(1-(((1r,3r)-3-(2-((1-(tert-butoxycarbonyl)piperidin-4-yl)(4-methoxy-4-oxobutyl)amino)ethoxy)-5,7-dimethyladamantan-1-yl)methyl)-5-methyl-1H-pyrazol-4-yl)picolinate

A solution of Example 1.26.1 (0.169 g), methyl 4-oxobutanoate (0.024 g) and sodium triacetoxyborohydride (0.055 g) was stirred in dichloromethane (2 mL) at room temperature. After 2 hours, the reaction was diluted with dichloromethane (50 mL) and washed with saturated aqueous sodium bicarbonate (10 mL). The organic layer was separated, dried over magnesium sulfate, filtered and concentrated. Silica gel chromatography, eluting with a gradient of 0.5-5% methanol/dichloromethane containing ammonia, provided the title compound. MS (ELSD) m/e 1085.5 (M+H)⁺.

### 1.42.2 6-[8-(1,3-benzothiazol-2-ylcarbamoyl)-3,4-dihydroisoquinolin-2(1H)-yl]-3-{1-[(3-{2-[(3-carboxypropyl)(piperidin-4-yl)amino]ethoxy}-5,7-dimethyltricyclo[3.3.1.1^{3,7}]dec-1-yl)methyl]-5-methyl-1H-pyrazol-4-yl}pyridine-2-carboxylic acid

A solution of Example 1.42.1 (0.161 g) in dichloromethane (0.5 mL) was treated with trifluoroacetic acid (0.5 mL), and the reaction was stirred overnight. The reaction was concentrated, dissolved in methanol (0.6 mL) and treated with lithium hydroxide monohydrate (0.124 g) as a solution in water (0.5 mL). After stirring for 1.5 hours, the reaction was quenched with trifluoroacetic acid (0.229 mL) and diluted with N,N-dimethylformamide (0.5 mL). The mixture was purified by reverse phase HPLC using a Gilson system, eluting with 10-60% acetonitrile in water containing 0.1% v/v trifluoroacetic acid. The desired fractions were combined and freeze-dried to provide the title compound. ¹H NMR (400 MHz, dimethyl sulfoxide-*d*₆) δ ppm 12.84 (s, 1H), 9.40 (s, 1H), 8.89-8.79 (m, 1H), 8.57-8.41 (m, 1H), 8.03 (d, 1H), 7.80 (d, 1H), 7.62 (d, 1H), 7.55-7.41 (m, 3H), 7.41-7.32 (m, 2H), 7.30 (s, 1H), 6.96 (d, 1H), 4.96 (s, 2H), 3.89 (t, 2H), 3.83 (s, 2H), 3.44 (d, 2H), 3.26 (s, 2H), 3.22-3.11 (m, 2H), 3.09-2.85 (m, 6H), 2.34 (t, 2H), 2.19 (d, 2H), 2.10 (s, 3H), 1.95-1.71 (m, 5H), 1.44 (s, 2H), 1.39-1.27 (m, 4H), 1.22-0.96 (m, 6H), 0.87 (s, 6H). MS (ESI) m/e 915.3 (M+H)⁺.

### 1.43 Synthesis of 6-[8-(1,3-benzothiazol-2-ylcarbamoyl)naphthalen-2-yl]-3-{1-[(3,5-dimethyl-7-{2-[(2-sulfoethyl)amino]ethoxy}tricyclo[3.3.1.1^{3,7}]dec-1-yl)methyl]-5-methyl-1H-pyrazol-4-yl}pyridine-2-carboxylic acid (Compound W2.43)

### 1.43.1 tert-butyl 3-(1-((3-(2-hydroxyethoxy)-5,7-dimethyladamantan-1-yl)methyl)-5-methyl-1H-pyrazol-4-yl)-6-(8-(methoxycarbonyl)naphthalen-2-yl)picolinate

To a solution of methyl 7-(4,4,5,5-tetramethyl-1,3,2-dioxaborolan-2-yl)-1-naphthoate (2.47 g) in 1,4-dioxane (40 mL) and water (20 mL) was added Example 1.20.2 (4.2 g), bis(triphenylphosphine)palladium(II)dichloride (556 mg), and cesium fluoride (3.61 g), and the reaction was stirred at reflux overnight. The mixture was diluted with ethyl acetate (400 mL) and washed with water and brine, and dried over sodium sulfate. Filtration and evaporation of the solvent gave a residue that was purified by silica gel chromatography, eluting with 20% ethyl acetate in heptane followed by 5% methanol in dichloromethane, to provide the title compound. MS (ESI) m/e 680.7 (M+H)⁺.

### 1.43.2 tert-butyl 3-(1-((3,5-dimethyl-7-(2-((methylsulfonyl)oxy)ethoxy)adamantan-1-yl)methyl)-5-methyl-1H-pyrazol-4-yl)-6-(8-(methoxycarbonyl)naphthalen-2-yl)picolinate

To a cooled (0 °C) solution of Example 1.43.1 (725 mg) in dichloromethane (10 mL) and triethylamine (0.5 mL) was added methanesulfonyl chloride (0.249 mL), and the mixture was stirred for 4 hours. The reaction mixture was diluted with ethyl acetate (200 mL) and washed with water and brine, and dried over sodium sulfate. Filtration and evaporation of the solvent gave the title product, which was used in the next reaction without further purification. MS (ESI) m/e 759.9 (M+H)⁺.

### 1.43.3 tert-butyl 3-(1-(((3-(2-azidoethoxy)-5,7-dimethyladamantan-1-yl)methyl)-5-methyl-1H-pyrazol-4-yl)-6-(8-(methoxycarbonyl)naphthalen-2-yl)picolinate

To a solution of Example 1.43.2 (4.2 g) in N,N-dimethylformamide (30 mL) was added sodium azide (1.22 g), and the mixture was stirred for 96 hours. The reaction mixture was diluted with ethyl acetate (600 mL) , washed with water and brine, and dried over sodium sulfate. Filtration and evaporation of the solvent provided the title compound. MS (ESI) m/e 705.8 (M+H)⁺.

### 1.43.4 7-(5-(1-((3-(2-azidoethoxy)-5,7-dimethyladamantan-1-yl)methyl)-5-methyl-1H-pyrazol-4-yl)-6-(tert-butoxycarbonyl)pyridin-2-yl)-1-naphthoic acid

To a solution of Example 1.43.3 (3.5 g) in tetrahydrofuran/methanol/water (2:1:1, 30 mL) was added lithium hydroxide monohydrate (1.2 g), and the mixture was stirred overnight. The reaction mixture was acidified with IN aqueous HCl and was diluted with ethyl acetate (600 mL), washed with water and brine, and dried over sodium sulfate. Filtration and evaporation of the solvent provided the title compound. MS (ESI) m/e 691.8 (M+H)⁺.

### 1.43.5 tert-butyl 3-(1-((3-(2-azidoethoxy)-5,7-dimethyladamantan-1-yl)methyl)-5-methyl-1H-pyrazol-4-yl)-6-(8-(benzo[d]thiazol-2-ylcarbamoyl)naphthalen-2-yl)picolinate

To a solution of Example 1.43.4 (870 mg) in N,N-dimethylformamide (10 mL) was added benzo[d]thiazol-2-amine (284 mg), fluoro-N,N,N',N'- tetramethylformamidinium hexafluorophosphate (499 mg) and N,N-diisopropylethylamine (488 mg). The mixture was stirred at 60 °C for 3 hours. The reaction mixture was diluted with ethyl acetate (200 mL) and washed with water and brine, and dried over sodium sulfate. Filtration and evaporation of the solvent provided the title compound. MS (ESI) m/e 824.1 (M+H)⁺.

### 1.43.6 tert-butyl 3-(1-((3-(2-aminoethoxy)-5,7-dimethyladamantan-1-yl)methyl)-5-methyl-1H-pyrazol-4-yl)-6-(8-(benzo[d]thiazol-2-ylcarbamoyl)naphthalen-2-yl)picolinate

To a solution of Example 1.43.5 (890 mg) in tetrahydrofuran (30 mL) was added Pd/C (90 mg). The mixture was stirred under 1 atmosphere of hydrogen overnight. The reaction mixture was filtered, and the catalyst was washed with ethyl acetate. The solvent was evaporated to provide the title compound. MS (ESI) m/e 798.1 (M+H)⁺.

### 1.43.7 6-[8-(1,3-benzothiazol-2-ylcarbamoyl)naphthalen-2-yl]-3-11-[(3,5-dimethyl-7-{2-[(2-sulfoethyl)amino]ethoxyltricyclo[3.3.1 .1^{3,7}]dec-1-yl)methyl]-5-methyl-1H-pyrazol-4-yl}pyridine-2-carboxylic acid

To a solution of Example 1.43.6 (189 mg) in N,N-dimethylformamide (6 mL) was added 4-((tert-butyldiphenylsilyl)oxy)-2,2-dimethylbutyl ethenesulfonate (106 mg). The mixture was stirred for 4 days. The mixture was diluted with ethyl acetate (300 mL) and washed with water and brine and dried over sodium sulfate. After filtration and evaporation of the solvent, the residue was dissolved in trifluoroacetic acid (10 mL) and sat overnight. The trifluoroacetic acid was evaporated under vacuum, and the residue was dissolved in dimethyl sulfoxide/methanol (1:1, 6 mL). The mixture was purified by reverse phase HPLC (Gilson system), eluting with 10-85%acetonitrile in water containing 0.1% v/v trifluoroacetic acid, to give the title compound. ¹H NMR (400 MHz, dimethyl sulfoxide-*d*₆) δ ppm 13.09 (s, 1H), 9.02 (s, 1H), 8.31-8.43 (m, 3H), 8.16-8.26 (m, 3H), 7.93-8.08 (m, 3H), 7.82 (d, 1H), 7.66-7.75 (m, 1H), 7.46-7.55 (m, 2H), 7.37 (t, 1H), 3.90 (s, 3H), 3.17-3.28 (m, 2H), 3.07-3.16 (m, 2H), 2.82 (t, 2H), 2.24 (s, 3H), 1.44 (s, 2H), 0.99-1.37 (m, 12H), 0.87 (s, 6H). MS (ESI) m/e 849.1 (M+H)⁺.

### 1.44 Synthesis of 3-{1-[(3-{2-[L-alpha-aspartyl(2-sulfoethyl)amino]ethoxy}-5,7-dimethyltricyclo[3.3.1.1^{3,7}]dec-1-yl)methyl]-5-methyl-1H-pyrazol-4-yl}-6-[8-(1,3-benzothiazol-2-ylcarbamoyl)-3,4-dihydroisoquinolin-2(1H)-yl]pyridine-2-carboxylic acid (Compound W2.44)

### 1.44.1 6-(8-(benzo [d]thiazol-2-ylcarbamoyl)-3,4-dihydroisoquinolin-2(1H)-yl)-3-(1-((3-(2-((S)-4-(tert-butoxy)-2-((tert-butoxycarbonyl)amino)-4-oxo-N-(2-sulfoethyl)butanamido)ethoxy)-5,7-dimethyladamantan-1-yl)methyl)-5-methyl-1H-pyrazol-4-yl)picolinic acid

To a cold (0 °C) solution of (S)-4-(tert-butoxy)-2-((tert-butoxycarbonyl)amino)-4-oxobutanoic acid (40.7 mg) and O-(7-azabenzotriazol-1-yl)-N,N,N',N'-tetramethyluronium hexafluorophosphate (HATU, 40.1 mg,) in N,N-dimethylformamide (3 mL) was added N,N-diisopropylethylamine (98 µL). The reaction mixture was stirred at room temperature for 1 hour, and Example 1.2.9 (60 mg) in N,N-dimethylformamide (1 mL) was added. The mixture was stirred for 1.5 hours and was purified by reverse phase chromatography (C18 column), eluting with 20-90% acetonitrile in water containing 0.1% v/v trifluoroacetic acid, to provide the title compound. MS (ESI) m/e 1123.4 (M-H)⁻.

### 1.44.2 3-{1-[(3-{2-[L-alpha-aspartyl(2-sulfoethyl)amino]ethoxy}-5,7-dimethyltricyclo[3.3.1.1^{3,7}]dec-1-yl)methyl]-5-methyl-1H-pyrazol-4-yl}-6- [8-(1,3-benzothiazol-2-ylcarbamoyl)-3,4-dihydroisoquinolin-2(1H)-yl]pyridine-2-carboxylic acid

Example 1.44.1 (100 mg) in dichloromethane (5 mL) was treated with trifluoroacetic acid (1.5 mL) overnight. The reaction mixture was concentrated and purified by reverse phase chromatography (C18 column), eluting with 20-60% acetonitrile in water containing 0.1% v/v trifluoroacetic acid, to provide the title compound. ¹H NMR (500 MHz, dimethyl sulfoxide-*d*₆) δ ppm 12.85 (s, 2H), 8.11-8.22 (m, 3H), 8.04 (d, 1H), 7.79 (d, 1H), 7.62 (d, 1H), 7.41-7.54 (m, 3H), 7.32-7.39 (m, 2H), 7.29 (s, 1H), 6.95 (d, 1H), 4.95 (s, 2H), 4.80 (s, 1H), 3.89 (t, 2H), 3.81 (s, 2H), 3.55-3.71 (m, 2H), 3.01 (t, 4H), 2.74-2.86 (m, 1H), 2.57-2.73 (m, 2H), 2.09 (s, 3H), 0.91-1.46 (m, 13H), 0.84 (s, 6H). MS (ESI) m/e 969.2 (M+H)⁺.

### 1.45 Synthesis of 6-[8-(1,3-benzothiazol-2-ylcarbamoyl)-3,4-dihydroisoquinolin-2(1H)-yl]-3-{1-[(3-{2-[(1,3-dihydroxypropan-2-yl)amino]ethoxy}-5,7-dimethyltricyclo[3.3.1.1^{3,7}]dec-1-yl)methyl]-5-methyl-1H-pyrazol-4-yl}pyridine-2-carboxylic acid (Compound W2.45)

### 1.45.1 tert-butyl 6-(8-(benzo[d]thiazol-2-ylcarbamoyl)-3,4-dihydroisoquinolin-2(1H)-yl)-3-(1-((3,5-dimethyl-7-(2-(oxetan-3-ylamino)ethoxy)adamantan-1-yl)methyl)-5-methyl-1H-pyrazol-4-yl)picolinate

A solution of Example 1.2.7 (0.095 g), oxetan-3-one (10 mg) and sodium triacetoxyborohydride (0.038 g) was stirred in dichloromethane (1 mL) at room temperature. After stirring overnight, the reaction mixture was loaded directly onto silica gel and eluted using a gradient of 0.5- 5% methanol in dichloromethane containing ammonia to give the title compound. MS (ELSD) m/e 858.4 (M+H)⁺.

### 1.45.2 6-[8-(1,3-benzothiazol-2-ylcarbamoyl)-3,4-dihydroisoquinolin-2(1H)-yl]-3-{1-[(3-{2-[(1,3-dihydroxypropan-2-yl)amino]ethoxy}-5,7-dimethyltricyclo[3.3.1.1^{3,7}]dec-1-yl)methyl]-5-methyl-1H-pyrazol-4-yl}pyridine-2-carboxylic acid

Example 1.45.1 was dissolved in dichloromethane (0.5 mL) and was treated with trifluoroacetic acid (0.5 mL) and stirred overnight. The reaction was purified by reverse phase HPLC using a Gilson system, eluting with 10-60% acetonitrile in water containing 0.1% v/v trifluoroacetic acid. The desired fractions were combined and freeze-dried to provide the title compound. ¹H NMR (400 MHz, dimethyl sulfoxide-*d*₆) δ ppm 12.84 (s, 1H), 8.19 (s, 2H), 8.02 (d, 1H), 7.78 (d, 1H), 7.61 (d, 1H), 7.53-7.40 (m, 3H), 7.40-7.31 (m, 2H), 7.28 (s, 1H), 6.94 (d, 1H), 4.95 (s, 2H), 3.87 (t, 2H), 3.82 (s, 2H), 3.67-3.62 (m, 4H), 3.22-3.14 (m, 1H), 3.14-3.06 (m, 2H), 3.00 (t, 4H), 2.09 (s, 3H), 1.41 (s, 2H), 1.37-1.20 (m, 4H), 1.20-0.95 (m, 6H), 0.85 (s, 6H). MS (ESI) m/e 820.2 (M+H)⁺.

### 1.46 Synthesis of 6-[5-(2-aminoethoxy)-8-(1,3-benzothiazol-2-ylcarbamoyl)-3,4-dihydroisoquinolin-2(1H)-yl]-3-{1-[(3,5-dimethyl-7-{2-[methyl(2-sulfoethyl)amino]ethoxy}tricyclo[3.3.1.1^{3,7}]dec-1-yl)methyl]-5-methyl-1H-pyrazol-4-yl}pyridine-2-carboxylic acid (Compound W2.46)

### 1.46.1 6-[8-(1,3-benzothiazol-2-ylcarbamoyl)-5-(2-{[(benzyloxy)carbonyl] amino}ethoxy)-3,4-dihydroisoquinolin-2(1H)-yl]-3-[1-({3,5-dimethyl-7-[(2,2,7,7,13-pentamethyl-10,10-dioxido-3,3-diphenyl-4,9-dioxa-10λ⁶-thia-13-aza-3-silapentadecan-15-yl)oxy]tricyclo[3.3.1.1^{3,7}]dec-1-yl}methyl)-5-methyl-1H-pyrazol-4-yl]pyridine-2-carboxylic acid

The title compound was prepared as described in Example 1.2.8, replacing Example 1.2.7 with Example 1.35.

### 1.46.2 6-[5-(2-aminoethoxy)-8-(1,3-benzothiazol-2-ylcarbamoyl)-3,4-dihydroisoquinolin-2(1H)-yl]-3-{1-[(3,5-dimethyl-7-{2-[methyl(2-sulfoethyl)amino]ethoxy}tricyclo[3.3.1.1^{3,7}]dec-1-yl)methyl]-5-methyl-1H-pyrazol-4-yl}pyridine-2-carboxylic acid

The title compound was prepared as described in Example 1.34.4, replacing Example 1.34.3 with Example 1.46.1. ¹H NMR (500 MHz, dimethyl sulfoxide-*d*₆) δ ppm 12.74 (s, 2H), 8.96 (s, 1H), 8.03 (d, 1H), 7.94 (s, 3H), 7.72-7.81 (m, 2H), 7.53 (d, 1H), 7.47 (t, 1H), 7.35 (t, 1H), 7.28 (s, 1H), 7.02 (t, 2H), 5.03 (s, 2H), 4.26 (t, 2H), 3.92 (t, 2H), 3.83 (s, 2H), 3.23-3.38 (m, 4H), 3.13-3.25 (m, 1H), 2.82-3.00 (m, 4H), 2.78 (d, 3H), 2.11 (s, 3H), 1.23-1.50 (m, 6H), 0.95-1.21 (m, 6H), 0.86 (s, 6H). MS (ESI) m/e 927.2 (M+H)⁺.

### 1.47 Synthesis of 6-[8-(1,3-benzothiazol-2-ylcarbamoyl)-5-12-[(2-sulfoethyl)amino]ethoxy}-3,4-dihydroisoquinolin-2(1H)-yl]-3-{1-[(3,5-dimethyl-7-{2-[methyl(2-sulfoethyl)amino]ethoxy}tricyclo[3.3.1.1^{3,7}]dec-1-yl)methyl]-5-methyl-1H-pyrazol-4-yl}pyridine-2-carboxylic acid (Compound W2.47)

### 1.47.1 6-[8-(1,3-benzothiazol-2-ylcarbamoyl)-5-[(2,2,7,7-tetramethyl-10,10-dioxido-3,3-diphenyl-4,9-dioxa-10λ⁶-thia-13-aza-3-silapentadecan-15-yl)oxy]-3,4-dihydroisoquinolin-2(1H)-yl]-3-{1-[(3,5-dimethyl-7-{2-[methyl(2-sulfoethyl)amino]ethoxy}tricyclo[3.3.1.1^{3,7}]dec-1-yl)methyl]-5-methyl-1H-pyrazol-4-yl}pyridine-2-carboxylic acid

The title compound was prepared as described in Example 1.2.8, replacing Example 1.2.7 with Example 1.46.2.

### 1.47.2 6-[8-(1,3-benzothiazol-2-ylcarbamoyl)-5-{2-[(2-sulfoethyl)amino]ethoxy}-3,4-dihydroisoquinolin-2(1H)-yl]-3-{1-[(3,5-dimethyl-7-{2-[methyl(2-sulfoethyl)amino]ethoxy}tricyclo[3.3.1.1^{3,7}]dec-1-yl)methyl]-5-methyl-1H-pyrazol-4-yl}pyridine-2-carboxylic acid

Example 1.47.1 (100 mg) in dichloromethane (5 mL) was treated with trifluoroacetic acid (5 mL) overnight. The reaction mixture was concentrated and purified by reverse phase chromatography (C18 column), eluting with 20-60% acetonitrile in water containing 0.1% v/v trifluoroacetic acid, to provide the title compound. ¹H NMR (400 MHz, dimethyl sulfoxide-*d*₆) δ ppm m 12.74 (s, 1H), 8.96 (d, 1H), 8.64 (s, 2H), 8.02 (d, 1H), 7.76 (dd, 2H), 7.41-7.57 (m, 2H), 7.24-7.40 (m, 2H), 7.02 (t, 2H), 5.03 (s, 2H), 4.23-4.42 (m, 2H), 3.90 (t, 2H), 3.83 (s, 2H), 3.25-3.40 (m, 6H), 3.12-3.24 (m, 2H), 2.81-3.01 (m, 6H), 2.78 (d, 3H), 2.10 (s, 3H), 1.22-1.47 (m, 6H), 0.97-1.21 (m, 6H), 0.86 (s, 6H). MS (ESI) m/e 1035.3 (M+H)⁺.

### 1.48 Synthesis of 6-[8-(1,3-benzothiazol-2-ylcarbamoyl)-3,4-dihydroisoquinolin-2(1H)-yl]-3-{1-[(3,5-dimethyl-7-{2-[(2-sulfoethyl){2-[(2-sulfoethyl)amino]ethyl}amino]ethoxy}tricyclo[3.3.1.1^{3,7}]dec-1-yl)methyl]-5-methyl-1H-pyrazol-4-yl}pyridine-2-carboxylic acid (Compound W2.48)

### 1.48.1 6-[8-(1,3-benzothiazol-2-ylcarbamoyl)-3,4-dihydroisoquinolin-2(1H)-yl]-3-{1-[(3,5-dimethyl-7-{[2,2,7,7-tetramethyl-10,10-dioxido-3,3-diphenyl-16-(2-sulfoethyl)-4,9-dioxa-10λ⁶-thia-13,16-diaza-3-silaoctadecan-18-yl]oxy}tricyclo[3.3.1.1^{3,7}]dec-1-yl)methyl]-5-methyl-1H-pyrazol-4-yl}pyridine-2-carboxylic acid

The title compound was prepared as described in Example 1.2.8, replacing Example 1.2.7 with Example 1.33.2.

### 1.48.2 6-[8-(1,3-benzothiazol-2-ylcarbamoyl)-3,4-dihydroisoquinolin-2(1H)-yl]-3-{1-[(3,5-dimethyl-7-{2-[(2-sulfoethyl){2-[(2-sulfoethyl)amino]ethyl)amino]ethoxyltricyclo[3.3.1.1^{3,7}]dec-1-yl)methyl]-5-methyl-1H-pyrazol-4-yl}pyridine-2-carboxylic acid

The title compound was prepared as described in Example 1.47.2, replacing Example 1.47.1 with Example 1.48.1. ¹H NMR (400 MHz, dimethyl sulfoxide-*d*₆) δ ppm 12.87 (s, 3H), 8.55 (s, 4H), 8.04 (d, 2H), 7.79 (d, 2H), 7.62 (d, 1H), 7.40-7.56 (m, 3H), 7.32-7.40 (m, 2H), 7.29 (s, 1H), 6.96 (d, 2H), 4.96 (s, 3H), 3.89 (t, 2H), 3.83 (s, 2H), 3.47 (d, 2H), 3.36 (s, 2H), 3.18-3.30 (m, 2H), 3.01 (t, 2H), 2.94 (t, 2H), 2.82 (t, 2H), 2.11 (s, 3H), 1.26-1.49 (m, 6H), 0.96-1.20 (m, 6H), 0.87 (s, 6H). MS (ESI) m/e 1005.2 (M+H)⁺.

### 1.49 Synthesis of 6-[8-(1,3-benzothiazol-2-ylcarbamoyl)-5-12-[(2-carboxyethyl)amino]ethoxy}-3,4-dihydroisoquinolin-2(1H)-yl]-3-{1-[(3,5-dimethyl-7-{2-[methyl(2-sulfoethyl)amino]ethoxy}tricyclo[3.3.1.1^{3,7}]dec-1-yl)methyl]-5-methyl-1H-pyrazol-4-yl}pyridine-2-carboxylic acid (Compound W2.49)

### 1.49.1 6-(8-(benzo[d]thiazol-2-ylcarbamoyl)-5-(2-((3-(tert-butoxy)-3-oxopropyl)amino)ethoxy)-3,4-dihydroisoquinolin-2(1H)-yl)-3-(1-((3,5-dimethyl-7-(2-(methyl(2-sulfoethyl)amino)ethoxy)adamantan-1-yl)methyl)-5-methyl-1H-pyrazol-4-yl)picolinic acid

The title compound was prepared as described in Example 1.32.3, replacing Example 1.32.2 with Example 1.46.2.

### 1.49.2 6-[8-(1,3-benzothiazol-2-ylcarbamoyl)-5-{2-[(2-carboxyethyl)amino]ethoxy}-3,4-dihydroisoquinolin-2(1H)-yl]-3-{1-[(3,5-dimethyl-7-{2-[methyl(2-sulfoethyl)amino]ethoxy}tricyclo[3.3.1.1^{3,7}]dec-1-yl)methyl]-5-methyl-1H-pyrazol-4-yl}pyridine-2-carboxylic acid

The title compound was prepared as described in Example 1.6.2, replacing Example 1.6.1 with Example 1.49.1. ¹H NMR (400 MHz, dimethyl sulfoxide-*d*₆) δ ppm 12.75 (s, 1H), 8.96 (s, 1H), 8.59 (s, 2H), 8.03 (d, 1H), 7.72-7.82 (m, 2H), 7.54 (d, 1H), 7.43-7.51 (m, 2H), 7.35 (t, 1H), 7.28 (s, 1H), 7.02 (dd, 2H), 5.02 (s, 2H), 4.34 (s, 2H), 3.93 (s, 2H), 3.83 (s, 2H), 3.62 (s, 2H), 2.84-3.01 (m, 4H), 2.78 (d, 3H), 2.65-2.75 (m, 2H), 2.11 (s, 3H), 1.20-1.45 (m, 7H), 0.95-1.21 (m, 6H), 0.86 (s, 6H). MS (ESI) m/e 999.2 (M+H)⁺.

### 1.50 Synthesis of 3-11-[(3,5-dimethyl-7-12-[(3-phosphonopropyl)(piperidin-4-yl)amino]ethoxy}tricyclo[3.3.1.1^{3,7}]dec-1-yl)methyl]-5-methyl-1H-pyrazol-4-yl}-6-[8-([1,3]thiazolo[4,5-b]pyridin-2-ylcarbamoyl)-3,4-dihydroisoquinolin-2(1H)-yl]pyridine-2-carboxylic acid (Compound W2.50)

### 1.50.1 tert-butyl 3-(1-((3-(2-((1-(tert-butoxycarbonyl)piperidin-4-yl)amino)ethoxy)-5,7-dimethyladamantan-1-yl)methyl)-5-methyl-1H-pyrazol-4-yl)-6-(8-(thiazolo[4,5-b]pyridin-2-ylcarbamoyl)-3,4-dihydroisoquinolin-2(1H)-yl)picolinate

Example 1.23.2 (205 mg) was dissolved in dichloromethane (2.4 mL), and tert-butyl 4-oxopiperidine-1-carboxylate (51 mg) and sodium triacetoxyborohydride (75 mg) were added. The reaction was stirred at room temperature for two hours. More dichloromethane was added, and the reaction was poured into to saturated aqueous NaHCO₃ solution. The organic layer was washed with brine and dried over sodium sulfate. After filtration and concentration, the residue was purified by silica gel chromatography on a Grace Reveleris^{®} Amino cartridge, eluting with a gradient of 0.5 to 5.0% methanol in dichloromethane, to give the title compound. MS (ESI) m/e 986.3(M+H)⁺.

### 1.50.2 3-{1-[(3,5-dimethyl-7-{2-[(3-phosphonopropyl)(piperidin-4-yl)amino]ethoxy}tricyclo[3.3.1.1^{3,7}]dec-1-yl)methyl]-5-methyl-1H-pyrazol-4-yl}-6-[8-([1,3]thiazolo[4,5-b]pyridin-2-ylcarbamoyl)-3,4-dihydroisoquinolin-2(1H)-yl]pyridine-2-carboxylic acid

Example 1.50.1 (94 mg) was dissolved in dichloromethane (1 mL), then Example 1.14.2 (25 mg) and sodium triacetoxyborohydride (30 mg) were added. The reaction was stirred at room temperature for four hours. Trifluoroacetic acid (1.5 mL) was added, and the reaction stirred at room temperature overnight. The reaction mixture was concentrated and purified by reverse phase chromatography (C18 column), eluting with 10-90% acetonitrile in water containing 0.1% v/v trifluoroacetic acid, to provide the title compound as a trifluoroacetic acid salt. ¹H NMR (400 MHz, dimethyl sulfoxide-*d*₆) δ ppm 8.82 (br s, 1H) 8.60 (dd, 1H), 8.52 (dd, 1H), 8.50 (br s, 1H), 7.66 (d, 1H), 7.50 (d, 1H), 7.46 (d, 1H), 7.38 (m, 2H), 7.30 (s, 1H), 6.97 (d, 1H), 4.98 (s, 2H), 3.89 (t, 2H), 3.83 (s, 2H) 3.69 (m, 2H), 3.61 (m, 1H), 3.44 (m, 2H) 3.23 (m, 4H), 3.02 (t, 2H), 2.93 (m, 2H), 2.18 (m, 2H), 2.10 (s, 3H), 1.92 (m, 2H), 1.83 (m, 2H), 1.64 (m, 2H), 1.44 (s, 2H), 1.31 (m, 4H), 1.14 (m, 4H), 1.04 (m, 2H), 0.87 (s, 6H). MS (ESI) m/e 952.3 (M+H)⁺.

### 1.51 Synthesis of 6-[4-(1,3-benzothiazol-2-ylcarbamoyl)-3,4-dihydro-2H-1,4-benzoxazin-6-yl]-3-{1-[(3,5-dimethyl-7-{2-[(2-sulfoethyl)amino]ethoxy}tricyclo[3.3.1.1^{3,7}]dec-1-yl)methyl]-5-methyl-1H-pyrazol-4-yl}pyridine-2-carboxylic acid (Compound W2.51)

### 1.51.1 tert-butyl 3-(1-((3-(2-((tert-butyldimethylsilyl)oxy)ethoxy)-5,7-dimethyladamantan-1-yl)methyl)-5-methyl-1H-pyrazol-4-yl)-6-chloropicolinate

To a solution of Example 1.20.2 (3.2 g) in N,N-dimethylformamide (20 mL) was added imidazole (0.616 g) and chloro t-butyldimethylsilane (1.37 g). The mixture was stirred overnight. The reaction mixture was diluted with ethyl acetate (300 mL), washed with water and brine, and dried over sodium sulfate. Filtration and evaporation of the solvent gave the crude product that was purified by silica gel chromatography, eluting with 20% ethyl acetate in heptane, to provide the title compound. MS (ESI) m/e 645.4 (M+H)⁺.

### 1.51.2 tert-butyl 3-(1-((3-(2-((tert-butyldimethylsilyl)oxy)ethoxy)-5,7-dimethyladamantan-1-yl)methyl)-5-methyl-1H-pyrazol-4-yl)-6-(3,4-dihydro-2H-benzo[b][1,4]oxazin-6-yl)picolinate

To a solution of 6-(4,4,5,5-tetramethyl-1,3,2-dioxaborolan-2-yl)-3,4-dihydro-2H-benzo[b][1,4]oxazine (507 mg) in 1,4-dioxane (10 mL) and water (5 mL) was added Example 1.51.1 (1.25 g), bis(triphenylphosphine)palladium(II)dichloride (136 mg), and cesium fluoride (884 mg). The mixture was stirred at 120 °C under microwave conditions (Biotage, Initiator) for 20 minutes. The mixture was diluted with ethyl acetate (500 mL), washed with water and brine, and dried over sodium sulfate. Filtration and evaporation of the solvent gave a residue that was purified by silica gel chromatography, eluting with 20% ethyl acetate in heptane followed by 5% methanol in dichloromethane, to provide the title compound. MS (ESI) m/e 744.1 (M+H)⁺.

### 1.51.3 tert-butyl 6-(4-(benzo[d]thiazol-2-ylcarbamoyl)-3,4-dihydro-2H-benzo[b][1,4]oxazin-6-yl)-3-(1-((3-(2-((tert-butyldimethylsilyl)oxy)ethoxy)-5,7-dimethyladamantan-1-yl)methyl)-5-methyl-1H-pyrazol-4-yl)picolinate

To an ambient suspension of bis(2,5-dioxopyrrolidin-1-yl) carbonate (295 mg) in acetonitrile (10 mL) was added benzo[d]thiazol-2-amine (173 mg), and the mixture was stirred for 1 hour. A solution of Example 1.51.2 (710 mg) in acetonitrile (10 mL) was added, and the suspension was vigorously stirred overnight. The mixture was diluted with ethyl acetate (300 mL), washed with water and brine, and dried over sodium sulfate. Filtration and evaporation of the solvent gave a residue that was purified by silica gel chromatography, eluting with 20% ethyl acetate in heptane, to give the title compound. MS (ESI) m/e 920.2 (M+H)⁺.

### 1.51.4 tert-butyl 6-(4-(benzo[d]thiazol-2-ylcarbamoyl)-3,4-dihydro-2H-benzo[b][1,4]oxazin-6-yl)-3-(1-((3-(2-hydroxyethoxy)-5,7-dimethyladamantan-1-yl)methyl)-5-methyl-1H-pyrazol-4-yl)picolinate

To a solution of Example 1.51.3 (1.4 g) in tetrahydrofuran (10 mL) was added tetrabutyl ammonium fluoride (1.0M in tetrahydrofuran, 6 mL). The mixture was stirred for 3 hours. The mixture was diluted with ethyl acetate (300 mL), washed with water and brine, and dried over sodium sulfate. Filtration and evaporation of the solvent gave title product, which was used in the next reaction without further purification. MS (ESI) m/e 806.0 (M+H)⁺.

### 1.51.5 tert-butyl 6-(4-(benzo[d]thiazol-2-ylcarbamoyl)-3,4-dihydro-2H-benzo [b] [1,4]oxazin-6-yl)-3-(1-((3,5-dimethyl-7-(2-((methylsulfonyl)oxy)ethoxy)adamantan-1-yl)methyl)-5-methyl-1H-pyrazol-4-yl)picolinate

To a cooled (0 °C) solution of Example 1.51.4 (1.2 g) in dichloromethane (20 mL) and triethylamine (2 mL) was added methanesulfonyl chloride (300 mg). The mixture was stirred for 4 hours. The reaction mixture was diluted with ethyl acetate (200 mL), washed with water and brine, and dried over sodium sulfate. Filtration and evaporation of the solvent gave title product, which was used in the next reaction without further purification. MS (ESI) m/e 884.1 (M+H)⁺.

### 1.51.6 tert-butyl 3-(1-((3-(2-azidoethoxy)-5,7-dimethyladamantan-1-yl)methyl)-5-methyl-1H-pyrazol-4-yl)-6-(4-(benzo [d]thiazol-2-ylcarbamoyl)-3,4-dihydro-2H-benzo [b] [1,4] oxazin-6-yl)picolinate

To a solution of Example 1.51.5 (1.5 g) in N,N-dimethylformamide (20 mL) was added sodium azide (331mg). The mixture was stirred for 48 hours. The reaction mixture was diluted with ethyl acetate (200 mL), washed with water and brine, and dried over sodium sulfate. Filtration and evaporation of the solvent gave a residue that was purified by silica gel chromatography, eluting with 20% ethyl acetate in dichloromethane, to provide the title compound. MS (ESI) m/e 831.1 (M+H)⁺.

### 1.51.7 tert-butyl 3-(1-((3-(2-aminoethoxy)-5,7-dimethyladamantan-1-yl)methyl)-5-methyl-1H-pyrazol-4-yl)-6-(4-(benzo [d]thiazol-2-ylcarbamoyl)-3,4-dihydro-2H-benzo[b] [1,4]oxazin-6-yl)picolinate

To a solution of Example 1.51.6 (1.5 g) in tetrahydrofuran (30 mL) was added Pd/C (10%, 200 mg). The mixture was stirred under 1 atmosphere of hydrogen overnight. The reaction mixture was filtered, and the filtrate was concentrated under vacuum to give crude product. MS (ESI) m/e 805.1 (M+H)⁺.

### 1.51.8 6-[4-(1,3-benzothiazol-2-ylcarbamoyl)-3,4-dihydro-2H-1,4-benzoxazin-6-yl]-3-{1-[(3,5-dimethyl-7-{2- [(2-sulfoethyl)amino]ethoxyltricyclo[3.3.1.1^{3,7}]dec-1-yl)methyl]-5-methyl-1H-pyrazol-4-yl}pyridine-2-carboxylic acid

To a solution of Example 1.51.7 (164 mg) in N,N-dimethylformamide (10 mL) and N,N-diisopropylethylamine (0.5 mL) was added 4-((tert-butyldiphenylsilyl)oxy)-2,2-dimethylbutyl ethenesulfonate (91 mg). The mixture was stirred overnight. The reaction mixture was diluted with ethyl acetate (200 mL), washed with water and brine, and dried over sodium sulfate. Filtration and evaporation of the solvent gave a residue that was dissolved in tetrahydrofuran (2 mL). Tetrabutyl ammonium fluoride (1 mL, 1M in tetrahydrofuran) was added, and the mixture was stirred overnight. The mixture was concentrated under vacuum, and the residue was dissolved in dichloromethane/trifluoroacetic acid (1:1, 6 mL), which was allowed to sit overnight. After evaporation of the solvent, the residue was purified by reverse phase HPLC (Gilson system), eluting with 10-85% acetonitrile in water containing 0.1% v/v trifluoroacetic acid, to provide the title compound. ¹H NMR (400 MHz, dimethyl sulfoxide-*d*₆) δ ppm 8.74 (s, 1H), 8.35 (s, 2H), 7.94-8.00 (m, 1H), 7.86 (s, 1H), 7.71-7.82 (m, 2H), 7.46 (s, 1H), 7.34-7.44 (m, 2H), 7.24 (t, 1H), 7.02 (d, 1H), 4.28-4.39 (m, 2H), 4.10-4.19 (m, 2H), 3.90 (s, 3H), 3.55-3.61 (m, 4H), 3.21-3.30 (m, 3H), 3.07-3.16 (m, 3H), 2.23 (s, 3H), 1.44 (s, 2H), 0.98-1.37 (m, 9H), 0.89 (s, 6H). MS (ESI) m/e 856.1 (M+H)⁺.

### 1.52 Synthesis of 6-[8-(1,3-benzothiazol-2-ylcarbamoyl)-5-(3-sulfopropoxy)-3,4-dihydroisoquinolin-2(1H)-yl]-3-[1-({3,5-dimethyl-7-[2-(methylamino)ethoxy]tricyclo[3.3.1.1^{3,7}]dec-1-yl}methyl)-5-methyl-1H-pyrazol-4-yl]pyridine-2-carboxylic acid (Compound W2.52)

### 1.52.1 methyl 2-(6-(tert-butoxycarbonyl)-5-(1-((3-(2-((tert-butoxycarbonyl)(methyl)amino)ethoxy)-5,7-dimethyladamantan-1-yl)methyl)-5-methyl-1H-pyrazol-4-yl)pyridin-2-yl)-5-(3-((2,2,2-trifluoro-1-(p-tolyl)ethoxy)sulfonyl)propoxy)-1,2,3,4-tetrahydroisoquinoline-8-carboxylate

To a solution of Example 1.31.8 (460 mg) in N,N-dimethylformamide (10 mL) was added 2,2,2-trifluoro-1-(p-tolyl)ethyl 3-iodopropane-1-sulfonate (239 mg, prepared according to J. Org. Chem., 2013, 78, 711-716) and K₂CO₃ (234 mg), and the mixture was stirred overnight. The mixture was diluted with ethyl acetate (200 mL), washed with water and brine, and dried over sodium sulfate. Filtration and evaporation of the solvent gave a residue that was purified by silica gel chromatography, eluting with 20% ethyl acetate in heptane, to provide the title compound. MS (ESI) m/e 1018.5 (M+H)⁺.

### 1.52.2 2-(6-(tert-butoxycarbonyl)-5-(1-((3-(2-((tert-butoxycarbonyl)(methyl)amino)ethoxy)-5,7-dimethyladamantan-1-yl)methyl)-5-methyl-1H-pyrazol-4-yl)pyridin-2-yl)-5-(3-((2,2,2-trifluoro-1-(p-tolyl)ethoxy)sulfonyl)propoxy)-1,2,3,4-tetrahydroisoquinoline-8-carboxylic acid.

To a solution of Example 1.52.1 (176 mg) in tetrahydrofuran (4 mL), methanol (3 mL) and water (3 mL) was added lithium hydroxide monohydrate (60 mg), and the mixture was stirred overnight. The mixture was then diluted with ethyl acetate (200 mL), washed with IN aqueous HCl, water and brine, and dried over sodium sulfate. Filtration and evaporation of the solvent gave the title product, which was used in the next reaction without further purification. MS (ESI) m/e 1095.2 (M+H)⁺.

### 1.52.3 tert-butyl 6-(8-(benzo[d]thiazol-2-ylcarbamoyl)-5-(3-((2,2,2-trifluoro-1-(p-tolyl)ethoxy)sulfonyl)propoxy)-3,4-dihydroisoquinolin-2(1H)-yl)-3-(1-((-(2-((tert-butoxycarbonyl)(methyl)amino)ethoxy)-5,7-dimethyladamantan-1-yl)methyl)-5-methyl-1H-pyrazol-4-yl)picolinate

To a solution of Example 1.52.2 (117 mg) in dichloromethane (6 mL) was added benzo[d]thiazol-2-amine (19.27 mg), 1-ethyl-3-[3-(dimethylamino)propyl]-carbodiimide hydrochloride (37 mg) and 4-(dimethylamino)pyridine (23.5 mg), and the mixture was stirred overnight. The reaction mixture was diluted with ethyl acetate (200 mL), washed with water and brine, and dried over sodium sulfate. Filtration and evaporation of the solvent gave the title product. MS (ESI) m/e 1226.1 (M+H)⁺.

### 1.52.4 6-[8-(1,3-benzothiazol-2-ylcarbamoyl)-5-(3-sulfopropoxy)-3,4-dihydroisoquinolin-2(1H)-yl]-3-[1-({3,5-dimethyl-7-[2-(methylamino)ethoxy]tricyclo[3.3.1.1^{3,7}]dec-1-yl}methyl)-5-methyl-1H-pyrazol-4-yl]pyridine-2-carboxylic acid

Example 1.52.3 (130 mg) was dissolved in dichloromethane/trifluoroacetic acid (1:1, 6 mL) and stirred overnight. After evaporation of the solvent, the residue was dissolved in N,N-dimethylformamide/water (1:1, 12 mL) and purified by reverse phase HPLC (Gilson), eluting with 10 to 85% acetonitrile in water containing 0.1% v/v trifluoroacetic acid, to give the title compound. ¹H NMR (400 MHz, dimethyl sulfoxide-*d*₆) δ ppm 12.68 (s, 1H), 8.13-8.32 (m, 2H), 8.01 (d, 1H), 7.75 (dd, 2H), 7.42-7.56 (m, 2H), 7.29 (s, 1H), 7.28-7.34 (m, 1H), 7.00 (dd, 2H), 5.03 (s, 2H), 4.19 (t, 2H), 3.83 (s, 3H), 3.50-3.57 (m, 4H), 2.95-3.05 (m, 2H), 2.81 (t, 2H), 2.52-2.65 (m, 4H), 1.39 (s, 2H), 0.96-1.32 (m, 12H), 0.87 (s, 6H). MS (ESI) m/e 898.3 (M+H)⁺.

### 1.53 Synthesis of 3-{1-[(3,5-dimethyl-7-{2-[(2-sulfoethyl)amino]ethoxy}tricyclo[3.3.1.1^{3,7}]dec-1-yl)methyl]-5-methyl-1H-pyrazol-4-yl}-6-[1-([1,3]thiazolo[4,5-b]pyridin-2-ylcarbamoyl)-1,2,3,4-tetrahydroquinolin-7-yl]pyridine-2-carboxylic acid (Compound W2.53)

### 1.53.1 tert-butyl 6-chloro-3-(1-((3-(2-hydroxyethoxy)-5,7-dimethyladamantan-1-yl)methyl)-5-methyl-1H-pyrazol-4-yl)picolinate

The title compound was prepared as described in Example 1.51.4, replacing Example 1.51.3 with Example 1.51.1.

### 1.53.2 tert-butyl 6-chloro-3-(1-((3,5-dimethyl-7-(2-((methylsulfonyl)oxy)ethoxy)adamantan-1-yl)methyl)-5-methyl-1H-pyrazol-4-yl)picolinate

To a cooled (0 °C) solution of Example 1.53.1 (1.89 g) in dichloromethane (30 mL) and triethylamine (3 mL) was added methanesulfonyl chloride (1.03 g), and the mixture was stirred for 4 hours. The reaction mixture was diluted with ethyl acetate (200 mL), washed with water and brine, and dried over sodium sulfate. Filtration and evaporation of the solvent gave the title product, which was used in the next reaction without further purification.

### 1.53.4 tert-butyl 3-(1-((3-(2-aminoethoxy)-5,7-dimethyladamantan-1-yl)methyl)-5-methyl-1H-pyrazol-4-yl)-6-chloropicolinate

Example 1.53.2 (2.2 g) was dissolved in 7N ammonia in methanol (40 mL), and the mixture was stirred at 80 °C under microwave conditions (Biotage Initiator) for 2 hours. The mixture was concentrated under vacuum and, and the residue was dissolved in ethyl acetate, washed with water and brine, and dried over sodium sulfate. Filtration and evaporation of the solvent provided the title compound.

### 1.53.5 tert-butyl 6-chloro-3-[1-({3,5-dimethyl-7-[(2,2,7,7-tetramethyl-10,10-dioxido-3,3-diphenyl-4,9-dioxa-10λ⁶-thia-13-aza-3-silapentadecan-15-yl)oxy]tricyclo[3.3.1.1^{3,7}]dec-1-yl}methyl)-5-methyl-1H-pyrazol-4-yl] pyridine-2-carboxylate

To a solution of Example 1.53.3 (1.59 g) in N,N-dimethylformamide (30 mL) was added 4-((tert-butyldiphenylsilyl)oxy)-2,2-dimethylbutyl ethenesulfonate (1.6 g) and N,N-diisopropylethylamine (1 mL), and the mixture was stirred for 4 days. The reaction mixture was dissolved in ethyl acetate (400 mL), washed with **water** and brine, and dried over sodium sulfate. Filtration and evaporation of the solvent gave the title product, which was used in the next reaction without further purification. MS (ESI) m/e 976.8 (M+H)⁺.

### 1.53.6 tert-butyl 3-11-[(3-1[13-(tert-butoxycarbonyl)-2,2,7,7-tetramethyl-10,10-dioxido-3,3-diphenyl-4,9-dioxa-10λ⁶-thia-13-aza-3-silapentadecan-15-yl]oxyl-5,7-dimethyltricyclo[3.3.1.1^{3,7}]dec-1-yl)methyl]-5-methyl-1H-pyrazol-4-yl}-6-chloropyridine-2-carboxylate

To a solution of Example 1.53.4 (2.93 g) in tetrahydrofuran (50 mL) was added di-t-butyldicarbonate (0.786 g) and 4-(dimethylamino)pyridine (100 mg), and the mixture was stirred overnight. The mixture was concentrated under vacuum, and the residue was dissolved in ethyl acetate (300 mL), washed with IN aqueous HCl solution, water and brine, and dried over sodium sulfate. Filtration and evaporation of the solvent gave a residue that was purified by silica gel chromatography, eluting with 20% ethyl acetate in heptane, to provide the title compound. MS (ESI) m/e 1076.9 (M+H)⁺.

### 1.53.7 tert-butyl 3-{1-[(3-{[13-(tert-butoxycarbonyl)-2,2,7,7-tetramethyl-10,10-dioxido-3,3-diphenyl-4,9-dioxa-10λ⁶-thia-13-aza-3-silapentadecan-15-yl]oxyl-5,7-dimethyltricyclo[3.3.1.1^{3,7}]dec-1-yl)methyl]-5-methyl-1H-pyrazol-4-yl}-6-(1,2,3,4-tetrahydroquinolin-7-yl)pyridine-2-carboxylate

To a solution of 7-(4,4,5,5-tetramethyl-1,3,2-dioxaborolan-2-yl)-1,2,3,4-tetrahydroquinoline (65 mg) in 1,4-dioxane (10 mL) and water (5 mL) was added Example 1.53.5 (220 mg), bis(triphenylphosphine)palladium(II)dichloride (7 mg), and cesium fluoride (45.6 mg). The mixture was stirred at 120 °C for 30 minutes under microwave conditions (Biotage Initiator). The mixture was diluted with ethyl acetate (200 mL), washed with water and brine, and dried over sodium sulfate. Filtration and evaporation of the solvent gave a residue that was purified by silica gel chromatography, eluting with 20% ethyl acetate in heptane, to give the title compound. MS (ESI) m/e 1173.9 (M+H)⁺.

### 1.53.8 3-{1-[(3,5-dimethyl-7-{2-[(2-sulfoethyl)amino]ethoxy}tricyclo[3.3.1.1^{3,7}]dec-1-yl)methyl]-5-methyl-1H-pyrazol-4-yl}-6-[1-([1,3]thiazolo[4,5-b] pyridin-2-ylcarbamoyl)-1,2,3,4-tetrahydroquinolin-7-yl]pyridine-2-carboxylic acid

To an ambient suspension of bis(2,5-dioxopyrrolidin-1-yl) carbonate (48.2 mg) in acetonitrile (10 mL) was added thiazolo[4,5-b]pyridin-2-amine (34 mg), and the mixture was stirred for 1 hour. A solution of Example 1.53.6 (220 mg) in acetonitrile (5 mL) was added, and the suspension was vigorously stirred overnight. The mixture was diluted with ethyl acetate (200 mL), washed with water and brine, and dried over sodium sulfate. Filtration and evaporation of the solvent gave a residue, which was dissolved in trifluoroacetic acid (10 mL) and stirred overnight. After evaporation of the solvent, the residue was purified by reverse phase HPLC (Gilson system), eluting with 10-85% acetonitrile in water containing 0.1% v/v trifluoroacetic acid, to provide the title compound. ¹H NMR (500 MHz, dimethyl sulfoxide-*d*₆) δ ppm 8.42-8.48 (m, 1H), 8.31-8.40 (m, 4H), 8.03 (d, 1H), 7.89 (d, 1H), 7.80 (d, 1H), 7.47 (s, 1H), 7.26-7.37 (m, 2H), 3.93-4.02 (m, 3H), 3.90 (s, 3H), 3.52-3.60 (m, 3H), 3.17-3.26 (m, 2H), 3.05-3.14 (m, 2H), 2.76-2.89 (m, 5H), 2.23 (s, 3H), 1.90-2.01 (m, 2H), 1.44 (s, 2H), 1.27-1.37 (m, 4H), 0.99-1.22 (m, 5H), 0.88 (s, 6H). MS (ESI) m/e 855.1 (M+H)⁺.

### 1.54 Synthesis of 3-{1-[(3,5-dimethyl-7-{2-[(2-sulfoethyl)amino]ethoxy}tricyclo[3.3.1.1^{3,7}]dec-1-yl)methyl]-5-methyl-1H-pyrazol-4-yl}-6-[8-([1,3]thiazolo[4,5-b]pyridin-2-ylcarbamoyl)naphthalen-2-yl]pyridine-2-carboxylic acid (Compound W2.54)

### 1.54.1 tert-butyl 3-11-[(3-1[13-(tert-butoxycarbonyl)-2,2,7,7-tetramethyl-10,10-dioxido-3,3-diphenyl-4,9-dioxa-10λ⁶-thia-13-aza-3-silapentadecan-15-yl]oxy}-5,7-dimethyltricyclo[3.3.1.1^{3,7}]dec-1-yl)methyl]-5-methyl-1H-pyrazol-4-yl}-6-[8-(methoxycarbonyl)naphthalen-2-yl]pyridine-2-carboxylate

The title compound was prepared by substituting methyl 7-(4,4,5,5-tetramethyl-1,3,2-dioxaborolan-2-yl)-1-naphthoate for 7-(4,4,5,5-tetramethyl-1,3,2-dioxaborolan-2-yl)-1,2,3,4-tetrahydroquinoline in Example 1.53.6. MS (ESI) m/e 1226.6 (M+H)⁺.

### 1.54.2 7-[6-(tert-butoxycarbonyl)-5-{1-[(3-{[13-(tert-butoxycarbonyl)-2,2,7,7-tetramethyl-10,10-dioxido-3,3-diphenyl-4,9-dioxa-10λ⁶-thia-13-aza-3-silapentadecan-15-yl]oxy}-5,7-dimethyltricyclo[3.3.1.1^{3,7}]dec-1-yl)methyl]-5-methyl-1H-pyrazol-4-yl}pyridin-2-yl]naphthalene-1-carboxylic acid

To a solution of Example 1.54.1 (79 mg) in tetrahydrofuran (4 mL), methanol (3 mL) and water (3 mL) was added lithium hydroxide monohydrate(60 mg), and the mixture was stirred overnight. The reaction was diluted with ethyl acetate (200 mL), washed with IN aqueous HCl, water and brine, and dried over sodium sulfate. Filtration and evaporation of the solvent gave the title product, which was used in the next step without further purification. MS (ESI) m/e 1211.6 (M+H)⁺.

### 1.54.3 3-{1-[(3,5-dimethyl-7-{2-[(2-sulfoethyl)amino]ethoxy}tricyclo[3.3.1.1^{3,7}]dec-1-yl)methyl]-5-methyl-1H-pyrazol-4-yl}-6-[8-([1,3]thiazolo[4,5-b]pyridin-2-ylcarbamoyl)naphthalen-2-yl]pyridine-2-carboxylic acid

To a solution of Example 1.54.2 (60 mg) in dichloromethane (4 mL) was added thiazolo[4,5-b]pyridin-2-amine (7.56 mg), 1-ethyl-3-[3-(dimethylamino)propyl]-carbodiimide hydrochloride **(**19 mg) and 4-(dimethylamino)pyridine (12.2 mg), and the mixture was stirred overnight. The reaction mixture was diluted with ethyl acetate (200 mL), washed with water and brine, and dried over sodium sulfate. Filtration and evaporation of the solvent gave the title product, which was dissolved in dichloromethane/trifluoroacetic acid (1:1, 6 mL) and stirred overnight. After evaporation of solvent, the residue was dissolved in N,N-dimethylformamide/water (1:1, 12 mL) and purified by reverse phase HPLC (Gilson system), eluting with 10-85% acetonitrile in water containing 0.1% trifluoroacetic acid, to give the title compound. ¹H NMR (400 MHz, dimethyl sulfoxide-*d*₆) δ ppm 13.42 (s, 1H), 9.05 (s, 1H), 8.51-8.69 (m, 2H), 8.31-8.41 (m, 2H), 8.18-8.26 (m, 4H), 8.06 (d, 1H), 7.97 (d, 1H), 7.68-7.79 (m, 1H), 7.49 (s, 1H), 7.40 (dd, 1H), 3.90 (s, 3H), 3.18-3.29 (m, 3H), 3.07-3.15 (m, 2H), 2.82 (t, 3H), 2.24 (s, 3H), 1.44 (s, 2H), 0.97-1.37 (m, 10H), 0.88 (s, 6H). MS (ESI) m/e 850.1 (M+H)⁺.

### 1.55 Synthesis of (1ξ)-1-({2-[5-(1-{[3-(2-aminoethoxy)-5,7-dimethyltricyclo[3.3.1.1^{3,7}]dec-1-yl]methyl}-5-methyl-1H-pyrazol-4-yl)-6-carboxypyridin-2-yl]-8-(1,3-benzothiazol-2-ylcarbamoyl)-1,2,3,4-tetrahydroisoquinolin-5-yl}methyl)-1,5-anhydro-D-glucitol (Compound W2.55)

### 1.55.1 (2R,3R,4S,5R)-3,4,5-tris(methoxymethoxy)-2-((methoxymethoxy)methyl)-6-methylenetetrahydro-2H-pyran

The title compound was prepared according to J. R. Walker et al., Bioorg. Med. Chem. 2006,14, 3038-3048. MS (ESI) m/e 370 (M+NH₄)⁺.

### 1.55.2 4-Bromo-3-cyanomethyl-benzoic acid methyl ester

To a solution of trimethylsilanecarbonitrile (3.59 mL) in tetrahydrofuran (6 mL) was added 1M tetrabutylammonium fluoride (26.8 mL, 1 M in tetrahydrofuran) dropwise over 30 minutes. The solution was stirred at room temperature for 30 minutes. Methyl 4-bromo-3-(bromomethyl)benzoate (7.50 g) was dissolved in acetonitrile (30 mL) and was added to the first solution dropwise over 30 minutes. The solution was heated to 80 °C for 30 minutes and cooled. The solution was concentrated under reduced pressure, and the residue was purified by silica gel chromatography, eluting with 20-30% ethyl acetate in heptanes, to provide the title compound.

### 1.55.3 3-(2-Aminoethyl)-4-bromobenzoic acid methyl ester

Example 1.55.2 (5.69 g) was dissolved in tetrahydrofuran (135 mL), and 1 M borane (in tetrahydrofuran, 24.6 mL) was added. The solution was stirred at room temperature for 16 hours and was slowly quenched with methanol and 1 M aqueous hydrochloric acid. 4 M Aqueous hydrochloric acid (150 mL) was added, and the solution was stirred at room temperature for 16 hours. The mixture was concentrated under reduced pressure, and the pH was adjusted to between 11 and 12 using solid potassium carbonate. The solution was then extracted with dichloromethane (3 × 100 mL). The organic extracts were combined and dried over anhydrous sodium sulfate. The solution was filtered and concentrated under reduced pressure, and the residue was purified by silica gel chromatography, eluting with 10- 20% methanol in dichloromethane, to provide the title compound. MS (ESI) m/e 258, 260 (M+H)⁺.

### 1.55.4 4-Bromo-3-[2-(2,2,2-trifluoroacetylamino)-ethyl]-benzoic acid methyl ester

Example 1.55.2 (3.21 g) was dissolved in dichloromethane (60 mL). The solution was cooled to 0 °C, and triethylamine (2.1 mL) was added. Trifluoroacetic anhydride (2.6 mL) was added dropwise. The solution was stirred at 0 °C for ten minutes, and the cooling bath was removed. After 1 hour, water (50 mL) was added, and the solution was diluted with ethyl acetate (100 mL). 1 M Aqueous hydrochloric acid was added (50 mL), and the organic layer was separated, washed with 1 M aqueous hydrochloric acid, and washed with brine. The solution was dried with anhydrous sodium sulfate, filtered and concentrated under reduced pressure to provide the title compound. MS (ESI) m/e 371, 373 (M+H)⁺.

### 1.55.5 5-Bromo-2-(2,2,2-trifluoroacetyl)-1,2,3,4-tetrahydroisoquinoline-8-carboxylic acid methyl ester

Example 1.55.4 (4.40 g) and paraformaldehyde (1.865 g) were placed in a flask and concentrated sulfuric acid (32 mL) was added. The solution was stirred at room temperature for one hour. Cold water (120 mL) was added, and the solution was extracted with ethyl acetate (3x 100 mL). The extracts were combined, washed with saturated aqueous sodium bicarbonate (100 mL) and water (100 mL), and dried over anhydrous sodium sulfate. The mixture was filtered and concentrated under reduced pressure. The residue was purified by silica gel chromatography, eluting with 20-30% ethyl acetate in heptanes, to provide the title compound. MS (ESI) m/e 366, 368 (M+H)⁺.

### 1.55.6 Methyl 2-(2,2,2-trifluoroacetyl)-5-(((3S,4R,5R,6R)-3,4,5-tris(methoxymethoxy)-6-((methoxymethoxy)methyl)tetrahydro-2H-pyran-2-yl)methyl)-1,2,3,4-tetrahydroisoquinoline-8-carboxylate

Example 1.55.1 (242 mg) was dissolved in tetrahydrofuran (7 mL) and 9-borabicyclo[3.3.1]nonane (3.0 mL) was added dropwise. The solution was refluxed for 4.5 hours and allowed to cool to room temperature. Potassium phosphate (3M, 0.6 mL) was added, and the solution was stirred for 10 minutes. The solution was then degassed and flushed with nitrogen three times. Separately, Example 1.55.5 (239 mg) and dichloro[1,1'-bis(diphenylphosphino)ferrocene]palladium (II) dichloromethane adduct (39 mg) were dissolved in N,N-dimethylformamide (7 mL), and the solution was degassed and flushed with nitrogen three times. The N,N-dimethylformamide solution was added dropwise to the tetrahydrofuran solution, and the mixture was stirred for 18 hours. HCl solution (0.1 M aqueous, 25 mL) was added, and the solution was extracted with ethyl acetate (30 mL) three times. The organic extracts were combined, washed with brine, dried over anhydrous sodium sulfate, filtered and concentrated. The residue was purified by silica gel chromatography, eluting with 30-50% ethyl acetate in heptanes, to yield the title compound. MS (ESI) m/e 710 (M+NH₄)⁺.

### 1.55.7 Methyl 5-(((3S,4R,5R,6R)-3,4,5-tris(methoxymethoxy)-6-((methoxymethoxy)methyl)tetrahydro-2H-pyran-2-yl)methyl)-1,2,3,4-tetrahydroisoquinoline-8-carboxylate

Example 1.55.6 (247 mg) was dissolved in methanol (1 mL), tetrahydrofuran (1 mL), and water (0.5 mL). Potassium carbonate (59 mg) was added, and the solution was stirred at room temperature for 16 hours. The solution was diluted with ethyl acetate (10 mL) and washed with saturated aqueous sodium bicarbonate (1 mL). The organic layer was dried over anhydrous sodium sulfate, filtered and concentrated under reduced pressure to yield the title compound. MS (ESI) m/e 600 (M+H)⁺.

### 1.55.8 Methyl 2-(5-bromo-6-(tert-butoxycarbonyl)pyridin-2-yl)-5-(((3S,4R,5R,6R)-3,4,5-tris(methoxymethoxy)-6-((methoxymethoxy)methyl)tetrahydro-2H-pyran-2-yl)methyl)-1,2,3,4-tetrahydroisoquinoline-8-carboxylate

The title compound was prepared by substituting Example 1.55.7 for methyl 1,2,3,4-tetrahydroisoquinoline-8-carboylate in Example 1.1.11. MS (ESI) m/e 799, 801 (M-tert-butyl)⁺.

### 1.55.9 Methyl 2-(6-(tert-butoxycarbonyl)-5-(4,4,5,5-tetramethyl-1,3,2-dioxaborolan-2-yl)pyridin-2-yl)-5-(((3S,4R,5R,6R)-3,4,5-tris(methoxymethoxy)-6-((methoxymethoxy)methyl)tetrahydro-2H-pyran-2-yl)methyl)-1,2,3,4-tetrahydroisoquinoline-8-carboxylate

The title compound was prepared by substituting Example 1.55.8 for Example 1.1.11 in Example 1.2.1. MS (ESI) m/e 903 (M+H)⁺, 933 (M+MeOH-H)⁻.

### 1.55.10 2-((3-((4-iodo-5-methyl-1H-pyrazol-1-yl)methyl)-5,7-dimethyladamantan-1-yl)oxy)ethanamine

The title compound was prepared by substituting Example 1.13.1 for Example 1.10.4 in Example 1.10.5. MS (ESI) m/e 444 (M+H)⁺.

### 1.55.11 tert-butyl (2-((3-((4-iodo-5-methyl-1H-pyrazol-1-yl)methyl)-5,7-dimethyladamantan-1-yl)oxy)ethyl)carbaniate

The title compound was prepared by substituting Example 1.55.10 for Example 1.10.5 in Example 1.10.6. MS (ESI) m/e 544 (M+H)⁺, 488 (M-tert-butyl)⁺, 542 (M-H)⁻.

### 1.55.12 Methyl 2-(6-(tert-butoxycarbonyl)-5-(1-((3-(2-((tert-butoxycarbonyl)amino)ethoxy)-5,7-dimethyladamantan-1-yl)methyl)-5-methyl-1H-pyrazol-4-yl)pyridin-2-yl)-5-(((3R,4S,SS,6S)-3,4,5-tris(methoxymethoxy)-6-((methoxymethoxy)methyl)tetrahydro-2H-pyran-2-yl)methyl)-1,2,3,4-tetrahydroisoquinoline-8-carboxylate

The title compound was prepared by substituting Example 1.55.9 for Example 1.2.1 and Example 1.55.11 for Example 1.13.3 in Example 1.13.4. MS (ESI) m/e 1192 (M+H)⁺.

### 1.55.13 2-(6-(tert-butoxycarbonyl)-5-(1-((3-(2-((tert-butoxycarbonyl)amino)ethoxy)-5,7-dimethyladamantan-1-yl)methyl)-5-methyl-1H-pyrazol-4-yl)pyridin-2-yl)-5-(((3R,4S,SS,6S)-3,4,5-tris(methoxymethoxy)-6-((methoxymethoxy)methyl)tetrahydro-2H-pyran-2-yl)methyl)-1,2,3,4-tetrahydroisoquinoline-8-carboxylic acid

The title compound was prepared by substituting Example 1.55.12 for Example 1.2.4 in Example 1.2.5. MS (ESI) m/e 1178 (M+H)⁺, 1176 (M-H)⁻.

### 1.55.14 Tert-butyl 6-(8-(benzo[d]thiazol-2-ylcarbamoyl)-5-(((3R,4S,5S,6S)-3,4,5-tris(methoxymethoxy)-6-((methoxymethoxy)methyl)tetrahydro-2H-pyran-2-yl)methyl)-3,4-dihydroisoquinolin-2(1H)-yl)-3-(1-((3-(2-((tert-butoxycarbonyl)amino)ethoxy)-5,7-dimethyladamantan-1-yl)methyl)-5-methyl-1H-pyrazol-4-yl)picolinate

The title compound was prepared by substituting Example 1.55.13 for Example 1.52.2 in Example 1.52.3. MS (ESI) m/e 1310 (M+H)⁺, 1308 (M-H)⁻.

### 1.55.15 (1ξ)-1-({2-[5-(1-{[3-(2-aminoethoxy)-5,7-dimethyltricyclo[3.3.1.1^{3,7}]dec-1-yl]methyl}-5-methyl-1H-pyrazol-4-yl)-6-carboxypyridin-2-yl]-8-(1,3-benzothiazol-2-ylcarbamoyl)-1,2,3,4-tetrahydroisoquinolin-5-yl}methyl)-1,5-anhydro-D-glucitol

The title compound was prepared by substituting Example 1.55.14 for Example 1.52.3 and 4M aqueous hydrochloric acid for trifluoroacetic acid in Example 1.52.4. ¹H NMR (400 MHz, dimethyl sulfoxide-*d*₆) δ ppm 7.96 (d, 1H), 7.73 (d, 1H), 7.58 (bs, 3H), 7.46 (d, 1H), 7.43-7.39 (m, 2H), 7.30 (d, 1H), 7.27-7.25 (m, 2H), 6.88 (d, 1H), 4.90 (q, 2H), 3.76 (m, 4H), 3.51 (m, 1H), 3.21 (d, 2H), 3.18 (d, 1H), 3.12 (m, 2H), 3.02 (m, 4H), 2.93 (m, 4H), 2.83 (m, 2H), 2.59 (m ,2H), 2.03 (s, 3H), 1.44 (s, 1H), 1.34 (s, 2H), 1.23 (q, 4H), 1.07 (m, 4H), 0.97 (q, 2 H), 0.80 (s, 6H). MS (ESI) m/e 922 (M+H)⁺, 920 (M-H)⁻.

### 1.56 Synthesis of 6-[8-(1,3-benzothiazol-2-ylcarbamoyl)-3,4-dihydroisoquinolin-2(1H)-yl]-3-{1-[(3-{2-[(3-carboxypropyl)amino]ethoxy}-5,7-dimethyltricyclo[3.3.1.1^{3,7}]dec-1-yl)methyl]-5-methyl-1H-pyrazol-4-yl}pyridine-2-carboxylic acid (Compound W2.56)

### 1.56.1 tert-butyl 6-(8-(benzo[d]thiazol-2-ylcarbamoyl)-3,4-dihydroisoquinolin-2(1H)-yl)-3-(1-((3-(2-((4-(tert-butoxy)-4-oxobutyl)amino)ethoxy)-5,7-dimethyladamantan-1-yl)methyl)-5-methyl-1H-pyrazol-4-yl)picolinate

To a solution of Example 1.2.7 (0.103 g) and tert-butyl 4-bromobutanoate (0.032 g) in dichloromethane (0.5 mL) was added N,N-diisopropylethylamine (0.034 mL) at 50 °C in a sealed amber vial overnight. The reaction was concentrated, dissolved in dimethyl sulfoxide/methanol (1:1, 2 mL) and purified by reverse phase HPLC using a Gilson system, eluting with 5-75% acetonitrile in water containing 0.1% v/v trifluoroacetic acid. The desired fractions were combined and freeze-dried to provide the title compound. MS (ESI) m/e 944.6 (M+1).

### 1.56.1 6-[8-(1,3-benzothiazol-2-ylcarbamoyl)-3,4-dihydroisoquinolin-2(1H)-yl]-3-{1-[(3-{2-[(3-carboxypropyl)amino]ethoxy}-5,7-dimethyltricyclo[3.3.1.1^{3,7}]dec-1-yl)methyl]-5-methyl-1H-pyrazol-4-yl}pyridine-2-carboxylic acid

A solution of Example 1.56.1 (0.049 g) was dissolved in dichloromethane (1 mL) and treated with trifluoroacetic acid (0.5 mL) and the mixture was stirred overnight. The reaction was concentrated, dissolved in a (1:1) N,N-dimethylformamide /water mixture (2 mL), and purified by reverse phase HPLC using a Gilson system, eluting with 5-75% acetonitrile in water containing 0.1% v/v trifluoroacetic acid. The desired fractions were combined and freeze-dried to provide the title compound. ¹H NMR (400 MHz, dimethyl sulfoxide-*d*₆) δ ppm 13.09- 12.32 (m, 2H), 8.31 (s, 2H), 8.03 (d, 1H), 7.79 (d, 1H), 7.62 (d, 1H), 7.54- 7.40 (m, 3H), 7.40- 7.32 (m, 2H), 7.29 (s, 1H), 6.96 (d, 1H), 4.96 (s, 2H), 3.89 (t, 2H), 3.83 (s, 2H), 3.55 (d, 2H), 3.02 (q, 4H), 2.92 (q, 2H), 2.33 (t, 2H), 2.10 (s, 3H), 1.80 (p, 2H), 1.43 (s, 2H), 1.30 (q, 4H), 1.21- 0.95 (m, 6H), 0.87 (s, 6H). MS (ESI) m/e 832.3 (M+H)⁺.

### 1.57 Synthesis of 6-[8-(1,3-benzothiazol-2-ylcarbamoyl)naphthalen-2-yl]-3-11-[(3,5-dimethyl-7-{2-[(3-phosphonopropyl)amino]ethoxyltricyclo[3.3.1.1^{3,7}]dec-1-yl)methyl]-5-methyl-1H-pyrazol-4-yl}pyridine-2-carboxylic acid (Compound W2.57)

### 1.57.1 tert-butyl 3-(1-((3-(2-hydroxyethoxy)-5,7-dimethyladamantan-1-yl)methyl)-5-methyl-1H-pyrazol-4-yl)-6-(8-(methoxycarbonyl)naphthalen-2-yl)picolinate

To a solution of methyl 7-(4,4,5,5-tetramethyl-1,3,2-dioxaborolan-2-yl)-1-naphthoate (2.47 g) in 1,4-dioxane (40 mL) and water (20 mL) was added Example 1.20.2 (4.2 g), bis(triphenylphosphine)palladium(II)dichloride (556 mg), and cesium fluoride (3.61 g). The mixture was refluxed overnight, diluted with ethyl acetate (400 mL) and washed with water and brine. The organic layer was dried over sodium sulfate, filtered, and concentrated. The residue was purified by silica gel chromatography, eluting with 20% ethyl acetate in dichloromethane and then with 5% methanol in dichloromethane, to provide the title compound. MS (ESI) m/e 680.84 (M+H)⁺.

### 1.57.2 tert-butyl 3-(1-((3,5-dimethyl-7-(2-((methylsulfonyl)oxy)ethoxy)adamantan-1-yl)methyl)-5-methyl-1H-pyrazol-4-yl)-6-(8-(methoxycarbonyl)naphthalen-2-yl)picolinate

To a cooled (0 °C) solution of Example 1.57.1 (725 mg) in dichloromethane (10 mL) and triethylamine (0.5 mL) was added methanesulfonyl chloride (0.249 mL). The mixture was stirred at room temperature for 4 hours, diluted with ethyl acetate, and washed with water and brine. The organic layer was dried over sodium sulfate, filtered, and concentrated to provide the title compound. MS (ESI) m/e 758.93 (M+H)⁺.

### 1.57.3 tert-butyl 3-(1-((3-(2-azidoethoxy)-5,7-dimethyladamantan-1-yl)methyl)-5-methyl-1H-pyrazol-4-yl)-6-(8-(methoxycarbonyl)naphthalen-2-yl)picolinate

To a solution of Example 1.57.2 (4.2 g) in N,N-dimethylformamide (30 mL) was added sodium azide (1.22 g). The mixture was stirred at room temperature for 96 hours, diluted with ethyl acetate (600 mL) and washed with water and brine. The organic layer was dried over sodium sulfate, filtered, and concentrated to provide the title compound. MS (ESI) m/e 704.86 (M+H)⁺.

### 1.57.4 7-(5-(1-((3-(2-azidoethoxy)-5,7-dimethyladamantan-1-yl)methyl)-5-methyl-1H-pyrazol-4-yl)-6-(tert-butoxycarbonyl)pyridin-2-yl)-1-naphthoic acid

To a solution of Example 1.57.3 (3.5 g) in tetrahydrofuran/methanol/H₂O (2:1:1, 30 mL) was added lithium hydroxide monohydrate (1.2 g), and the mixture was stirred at room temperature overnight. The reaction mixture was acidified with IN aqueous HCl solution, diluted with ethyl acetate (600 mL) and washed with water and brine. The organic layer was dried over sodium sulfate, filtered, and concentrated to provide the title compound. MS (ESI) m/e 691.82 (M+H)⁺.

### 1.57.5 tert-butyl 3-(1-((3-(2-azidoethoxy)-5,7-dimethyladamantan-1-yl)methyl)-5-methyl-1H-pyrazol-4-yl)-6-(8-(benzo[d]thiazol-2-ylcarbamoyl)naphthalen-2-yl)picolinate

To a solution of Example 1.57.4 (870 mg) in N,N-dimethylformamide (10 mL) was added benzo[d]thiazol-2-amine (284 mg), fluoro-N,N,N'N'-tetramethylformamidium hexafluorophosphate (499 mg) and N,N-diisopropylethylamine (488 mg). The mixture was stirred at 60 °C for 3 hours, diluted with ethyl acetate (200 mL), and washed with water and brine. The organic layer was dried over sodium sulfate, filtered, and concentrated to provide the title compound. MS (ESI) m/e 824.02 (M+H)⁺.

### 1.57.6 tert-butyl 3-(1-((3-(2-aminoethoxy)-5,7-dimethyladamantan-1-yl)methyl)-5-methyl-1H-pyrazol-4-yl)-6-(8-(benzo[d]thiazol-2-ylcarbamoyl)naphthalen-2-yl)picolinate

To a solution of Example 1.57.5 (890 mg) in tetrahydrofuran (30 mL) was added Pd/C (90 mg, 5%). The mixture was stirred under a hydrogen atmosphere at room temperature overnight, and filtered. The filtrate was concentrated to provide the title compound. MS (ESI) m/e 798.2 (M+H)⁺.

### 1.57.7 6-[8-(1,3-benzothiazol-2-ylcarbamoyl)naphthalen-2-yl]-3-{1-[(3,5-dimethyl-7-{2-[(3-phosphonopropyl)amino]ethoxyltricyclo[3.3.1.1^{3,7}]dec-1-yl)methyl]-5-methyl-1H-pyrazol-4-yl}pyridine-2-carboxylic acid

To a solution of Example 1.57.6 (137 mg) in dichloromethane (6 mL) was added Example 1.14.2 (43 mg). The mixture was stirred at room temperature for 1.5 hours, and a solution of NaBH₄ (26 mg) in methanol (2 mL) was added. The mixture was stirred at room temperature for 2 hours, diluted with ethyl acetate (200 mL) and washed with 2N aqueous NaOH solution, water and brine. The organic layer was dried over sodium sulfate, filtered, and concentrated. The residue was dissolved in dichloromethane (5 mL) and treated with trifluoroacetic acid (5 mL) overnight. The reaction mixture was concentrated. The residue was purified by reverse phase HPLC (Gilson system), eluting with a gradient of 10-85% acetonitrile in water containing 0.1% v/v trifluoroacetic acid solution, to provide the title compound. ¹H NMR (500 MHz, dimethyl sulfoxide-*d*₆) δ 9.03 (s, 1H), 8.48-8.35 (m, 3H), 8.29-8.16 (m, 3H), 8.08 (dd, 1H), 8.03 (dd, 1H), 7.94 (d, 1H), 7.82 (d, 1H), 7.71 (dd, 1H), 7.53-7.47 (m, 2H), 7.38 (td, 1H), 4.81-0.53 (m, 89H). MS (ESI) m/e 863.2 (M+H)⁺.

### 1.58 Synthesis of 6-[8-(1,3-benzothiazol-2-ylcarbamoyl)-3,4-dihydroisoquinolin-2(1H)-yl]-3-(1-{[3-(2-{[4-(beta-D-glucopyranosyloxy)benzyl]amino}ethoxy)-5,7-dimethyltricyclo[3.3.1.1^{3,7}]dec-1-yl]methyl}-5-methyl-1H-pyrazol-4-yl)pyridine-2-carboxylic acid (Compound W2.58)

To a solution of Example 1.3.1 (44.5 mg) in tetrahydrofuran (2 mL) and acetic acid (0.2 mL) was added 4-(((2S,3R,4R,5S,6R)-3,4,5-trihydroxy-6-(hydroxymethyl)tetrahydro-2H-pyran-2-yl)oxy)benzaldehyde (17 mg) and MgSO₄ (300 mg). The mixture was stirred at room temperature for 1 hour before the addition of sodium cyanoborohydride on resin (300 mg). The mixture was stirred at room temperature overnight and filtered. The filtrate was concentrated, and the residue was purified by reverse phase HPLC (Gilson system), eluting with a gradient of 10-85% acetonitrile in water containing 0.1% v/v trifluoroacetic acid solution, to provide the title compound. MS (ESI) m/e 1015.20 (M+H)⁺.

### 1.59 Synthesis of 3-(1-1[3-(2-1[4-(beta-D-allopyranosyloxy)benzyl]amino}ethoxy)-5,7-dimethyltricyclo[3.3.1.1^{3,7}]dec-1-yl]methyl}-5-methyl-1H-pyrazol-4-yl)-6-[8-(1,3-benzothiazol-2-ylcarbamoyl)-3,4-dihydroisoquinolin-2(1H)-yl]pyridine-2-carboxylic acid (Compound W2.59)

To a solution of Example 1.3.1 (44.5 mg) in tetrahydrofuran (2 mL) and acetic acid (0.2 mL) was added 4-(((2S,3R,4S,SS,6R)-3,4,5-trihydroxy-6-(hydroxymethyl)tetrahydro-2H-pyran-2-yl)oxy)benzaldehyde (17 mg) and MgSO₄ (300 mg), and the mixture was stirred at room temperature for 1 hour before the addition of sodium cyanoborohydride on resin (300 mg). The mixture was stirred at room temperature overnight and filtered. The filtrate was concentrated, and the residue was purified by reverse phase HPLC (Gilson system), eluting with a gradient of 10-85% acetonitrile in water containing 0.1% v/v trifluoroacetic acid, to provide the title compound. MS (ESI) m/e 1015.20 (M+H)⁺.

### 1.60 Synthesis of 3-{1-[(3-{2-[azetidin-3-yl(2-sulfoethyl)amino]ethoxy}-5,7-dimethyltricyclo[3.3.1.1^{3,7}]dec-1-yl)methyl]-5-methyl-1H-pyrazol-4-yl}-6-[8-(1,3-benzothiazol-2-ylcarbamoyl)-3,4-dihydroisoquinolin-2(1H)-yl]pyridine-2-carboxylic acid (Compound W2.60)

### 1.60.1 tert-butyl 6-(8-(benzo[d]thiazol-2-ylcarbamoyl)-3,4-dihydroisoquinolin-2(1H)-yl)-3-(1-((3-(2-((1-(tert-butoxycarbonyl)azetidin-3-yl)(2-((4-(tert-butyldiphenylsilyl)hydroxy-2,2-dimethylbutoxy)sulfonyl)ethyl)amino)ethoxy)-5,7-dimethyladamantan-1-yl)methyl)-5-methyl-1H-pyrazol-4-yl)picolinate

A solution of Example 1.2.8 (0.075 g), tert-butyl 3-oxoazetidine-1-carboxylate (0.021 g) and sodium triacetoxyborohydride (0.025 g) in dichloromethane (0.5 mL) was stirred at room temperature overnight. The reaction was loaded onto silica gel and eluted with 0-10% methanol in dichloromethane to give the title compound. MS (ESI) m/e 1403.9 (M+1).

### 1.60.2 3-{1-[(3-{2-[azetidin-3-yl(2-sulfoethyl)amino]ethoxy}-5,7-dimethyltricyclo[3.3.1.1^{3,7}]dec-1-yl)methyl]-5-methyl-1H-pyrazol-4-yl}-6-[8-(1,3-benzothiazol-2-ylcarbamoyl)-3,4-dihydroisoquinolin-2(1H)-yl]pyridine-2-carboxylic acid

A solution of Example 1.60.1 (0.029 g) in dichloromethane (1 mL) was treated with trifluoroacetic acid (1 mL) and stirred overnight. The reaction was concentrated, dissolved in 1:1 dimethyl sulfoxide/methanol (2 mL), and the mixture was purified by reverse phase HPLC using a Gilson system, eluting with 10-80% acetonitrile in water containing 0.1% v/v trifluoroacetic acid. The desired fractions were combined and freeze-dried to provide the title compound. ¹H NMR (400 MHz, dimethyl sulfoxide-*d*₆) δ ppm 12.86 (s, 1H), 8.81 (s, 2H), 8.04 (d, 1H), 7.79 (d, 1H), 7.62 (d, 1H), 7.52 (d, 1H), 7.50- 7.46 (m, 1H), 7.44 (d, 1H), 7.40- 7.33 (m, 2H), 7.30 (s, 1H), 6.96 (d, 1H), 4.96 (s, 2H), 4.37 (q, 1H), 4.27 (s, 2H), 4.11 (s, 2H), 3.89 (t, 2H), 3.83 (s, 2H), 3.58- 3.54 (m, 2H), 3.32 (t, 2H), 3.24 (s, 2H), 3.01 (t, 2H), 2.85 (t, 2H), 2.10 (s, 3H), 1.48- 0.97 (m, 12H), 0.87 (s, 6H). MS (ESI) m/e 909.2 (M+H)⁺.

### 1.61 Synthesis of 3-{1-[(3-{2-[(3-aminopropyl)(2-sulfoethyl)amino]ethoxy}-5,7-dimethyltricyclo[3.3.1.1^{3,7}]dec-1-yl)methyl]-5-methyl-1H-pyrazol-4-yl}-6-[8-(1,3-benzothiazol-2-ylcarbamoyl)-3,4-dihydroisoquinolin-2(1H)-yl]pyridine-2-carboxylic acid (Compound W2.61)

### 1.61.1 6-(8-(benzo[d]thiazol-2-ylcarbamoyl)-3,4-dihydroisoquinolin-2(1H)-yl)-3-(1-((3-(2-((3-((tert-butoxycarbonyl)amino)propyl)(2-sulfoethyl)amino)ethoxy)-5,7-dimethyladamantan-1-yl)methyl)-5-methyl-1H-pyrazol-4-yl)picolinic acid

The title compound was prepared using the procedure for Example 1.33.1, replacing tert-butyl (2-oxoethyl)carbamate with tert-butyl (3-oxopropyl)carbamate. MS (ESI) m/e 1011.5 (M+H).

### 1.61.2 3-{1-[(3-{2-[(3-aminopropyl)(2-sulfoethyl)amino]ethoxy}-5,7-dimethyltricyclo[3.3.1.1^{3,7}]dec-1-yl)methyl]-5-methyl-1H-pyrazol-4-yl}-6-[8-(1,3-benzothiazol-2-ylcarbamoyl)-3,4-dihydroisoquinolin-2(1H)-yl]pyridine-2-carboxylic acid

The title compound was prepared as described in Example 1.6.2, replacing Example 1.6.1 with Example 1.61.1. ¹H NMR (400 MHz, dimethyl sulfoxide-*d*₆) δ ppm 12.87 (s, 1H), 9.10 (s, 1H), 8.04 (d, 1H), 7.88-7.67 (m, 4H), 7.62 (d, 1H), 7.57-7.40 (m, 3H), 7.36 (td, 2H), 6.96 (d, 1H), 4.96 (s, 2H), 4.05-3.78 (m, 4H), 3.41-3.08 (m, 3H), 2.94 (tt, 6H), 2.11 (s, 3H), 1.92 (t, 2H), 1.53-0.95 (m, 11H), 0.87 (s, 6H). MS (ESI) m/e 911.3 (M+H).

### 1.62 Synthesis of 6-[1-(1,3-benzothiazol-2-ylcarbamoyl)-1,2,3,4-tetrahydroquinolin-7-yl]-3-{1-[(3-{2-[(2-carboxyethyl)amino]ethoxy}-5,7-dimethyltricyclo[3.3.1.1^{3,7}]dec-1-yl)methyl]-5-methyl-1H-pyrazol-4-yl}pyridine-2-carboxylic acid (Compound W2.62)

### 1.62.1 tert-butyl 3-(1-((3-(2-((3-(tert-butoxy)-3-oxopropyl)amino)ethoxy)-5,7-dimethyladamantan-1-yl)methyl)-5-methyl-1H-pyrazol-4-yl)-6-chloropicolinate

To an ambient solution of Example 1.53.3 (521 mg) in ethanol (10 mL) was added triethylamine (3 mL) followed by tert-butyl acrylate (2 mL). The mixture was stirred at room temperature for 3 hours and then concentrated to dryness. The residue was dissolved in ethyl acetate (200 mL), and the solution was washed with water and brine. The organic layer was dried over sodium sulfate, filtered and concentrated under reduced pressure to give the title compound, which was used in the next reaction without further purification. MS (ESI) m/e 657.21 (M+H)⁺.

### 1.62.2 tert-butyl 3-(1-((3-(2-((3-(tert-butoxy)-3-oxopropyl)(tert-butoxycarbonyl)amino)ethoxy)-5,7-dimethyladamantan-1-yl)methyl)-5-methyl-1H-pyrazol-4-yl)-6-chloropicolinate

To a solution of Example 1.62.1 (780 mg) in tetrahydrofuran (10 mL) was added *di-tert-*butyl dicarbonate (259 mg) followed by a catalytic amount of 4-dimethylaminopyridine. The reaction was stirred at room temperature for 3 hours and then concentrated to dryness. The residue was dissolved in ethyl acetate (200 mL), and the solution was washed with saturated aqueous NaHCO₃ solution, water and brine. The organic layer was dried over sodium sulfate, filtered and concentrated under reduced pressure. The residue was purified by chromatography on silica gel, eluting with 20% ethyl acetate in heptane, to give the title compound. MS (ESI) m/e 757.13 (M+H)⁺.

### 1.62.3 tert-butyl 3-(1-((3-(2-((3-(tert-butoxy)-3-oxopropyl)(tert-butoxycarbonyl)amino)ethoxy)-5,7-dimethyladamantan-1-yl)methyl)-5-methyl-1H-pyrazol-4-yl)-6-(1,2,3,4-tetrahydroquinolin-7-yl)picolinate

To a solution of 7-(4,4,5,5-tetramethyl-1,3,2-dioxaborolan-2-yl)-1,2,3,4-tetrahydroquinoline (234 mg) in 1,4-dioxane (10 mL) and water (5 mL) was added Example 1.62.2 (685 mg), bis(triphenylphosphine)palladium(II)dichloride (63.2 mg), and cesium fluoride (410 mg). The mixture was heated to 120 °C for 30 minutes by microwave irradiation (Biotage Initiator). The reaction was quenched by the addition of ethyl acetate and water. The layers were separated, and the organic layer was washed with brine, dried over sodium sulfate, filtered and concentrated under reduced pressure. The residue was purified by chromatography on silica gel, eluting with 20% ethyl acetate in heptane, to give the title compound. MS (ESI) m/e 854.82 (M+H)⁺.

### 1.62.4 tert-butyl 6-(1-(benzo[d]thiazol-2-ylcarbamoyl)-1,2,3,4-tetrahydroquinolin-7-yl)-3-(1-((3-(2-((3-(tert-butoxy)-3-oxopropyl)(tert-butoxycarbonyl)amino)ethoxy)-5,7-dimethyladamantan-1-yl)methyl)-5-methyl-1H-pyrazol-4-yl)picolinate

To an ambient suspension of bis(2,5-dioxopyrrolidin-1-yl) carbonate (150 mg) in acetonitrile (10 mL) was added benzo[d]thiazol-2-amine (88 mg), and the mixture was stirred for 1 hour. A solution of Example 1.62.3 (500 mg) in acetonitrile (2 mL) was added, and the suspension was vigorously stirred overnight. The reaction was quenched by the addition of ethyl acetate and water. The layers were separated, and the organic layer was washed with brine, dried over sodium sulfate, filtered and concentrated under reduced pressure. The residue was purified by chromatography on silica gel, eluting with 20% ethyl acetate in dichloromethane, to give the title compound. MS (ESI) m/e 1030.5 (M+H)⁺.

### 1.62.5 6-[1-(1,3-benzothiazol-2-ylcarbamoyl)-1,2,3,4-tetrahydroquinolin-7-yl]-3-{1-[(3-{2-[(2-carboxyethyl)amino]ethoxy}-5,7-dimethyltricyclo[3.3.1.1^{3,7}]dec-1-yl)methyl]-5-methyl-1H-pyrazol-4-yl}pyridine-2-carboxylic acid

To an ambient solution of Example 1.62.4 (110 mg) in dichloromethane (0.53 mL) was added trifluoroacetic acid (0.53 mL). The reaction was stirred overnight and was concentrated to a viscous oil. The residue was dissolved in dimethyl sulfoxide/methanol (1:1, 2 mL) and purified by reverse phase HPLC (Gilson system), eluting with 10-55% acetonitrile in 0.1% trifluoroacetic acid in water, to give the title compound. ¹H NMR (400 MHz, dimethyl sulfoxide-*d*₆) δ ppm 13.10 (s, 3H), 8.37 (s, 1H), 8.26 (s, 2H), 7.98 (d, 1H), 7.86-7.71 (m, 3H), 7.44 (s, 1H), 7.39-7.31 (m, 1H), 7.26 (d, 1H), 7.19 (t, 1H), 3.92 (d, 2H), 3.87 (s, 2H), 3.55 (t, 2H), 3.17-3.00 (m, 4H), 2.80 (t, 2H), 2.62 (t, 2H), 2.19 (s, 3H), 1.95-1.88 (m, 2H), 1.43 (s, 2H), 1.33-1.25 (m, 4H), 1.18-1.11 (m, 4H), 1.09-0.97 (m, 2H), 0.85 (s, 6H). MS (ESI) m/e 818.0 (M+H)⁺.

### 1.63 Synthesis of 6-[8-(1,3-benzothiazol-2-ylcarbamoyl)-3,4-dihydroisoquinolin-2(1H)-yl]-3-{1-[(3-{2-[(N6,N6-dimethyl-L-lysyl)(methyl)amino]ethoxyl-5,7-dimethyltricyclo[3.3.1.1^{3,7}]dec-1-yl)methyl]-5-methyl-1H-pyrazol-4-yl}pyridine-2-carboxylicacid (W2.63)

A solution of (S)-2-((((9H-fluoren-9-yl)methoxy)carbonyl)amino)-6-(dimethylamino)hexanoic acid (0.029 g) and 1-[bis(dimethylamino)methylene]-1H-12)-triazolo[4,5-h]pyridinium 3-oxid hexafluorophosphate (0.028 g) was stirred together in N,N-dimethylformamide (0.5 mL) with N,N-diisopropylamine (0.035 mL). After stirring for 5 minutes, the solution was added to Example 1.13.7 (0.051 g) and stirring was continued at room temperature overnight. To the reaction was added diethylamine (0.070 mL), and the reaction was stirred for 2 hours. The reaction was diluted with N,N-dimethylformamide (1 mL), water (0.5 ml), and 2,2,2-trifluoroacetic acid (0.103 ml) then purified via reverse-phase HPLC using a gradient of 10% to 90% acetonitrile/water. The product containing fractions were collected and lyophilized to give the title compound. ¹H NMR (500 MHz, dimethyl sulfoxide-*d*₆) δ 9.59 (s, 1H), 8.41 (s, 1H), 8.12 (t, 3H), 8.01 (d, 1H), 7.85 (dd, 1H), 7.81 (d, 1H), 7.77 (dd, 1H), 7.47 (s, 1H), 7.38 (t, 1H), 7.30 (d, 1H), 7.22 (t, 1H), 3.97 (t, 2H), 3.89 (s, 2H), 3.49 (dt, 4H), 3.06 (s, 2H), 2.99 (q, 2H), 2.88 (s, 2H), 2.84 (t, 2H), 2.75 (d, 6H), 2.22 (s, 3H), 2.00 - 1.90 (m, 2H), 1.84 - 1.52 (m, 4H), 1.48 - 0.95 (m, 14H), 0.87 (d, 6H). MS (ESI) m/e 916.2 (M+H)⁺.

### 1.64 Synthesis of 3-{1-[(3-{2-[(3-aminopropyl)(methyl)amino]ethoxy}-5,7-dimethyltricyclo[3.3.1.1^{3,7}]dec-1-yl)methyl]-5-methyl-1H-pyrazol-4-yl}-6-[1-(1,3-benzothiazol-2-ylcarbamoyl)-1,2,3,4-tetrahydroquinolin-7-yl]pyridine-2-carboxylic acid (W2.64)

### 1.64.1 6-(1-(benzo[d]thiazol-2-ylcarbamoyl)-1,2,3,4-tetrahydroquinolin-7-yl)-3-(1-((3-(2-((3-((tert-butoxycarbonyl)amino)propyl)(methyl)amino)ethoxy)-5,7-dimethyladamantan-1-yl)methyl)-5-methyl-1H-pyrazol-4-yl)picolinic acid

A solution of Example 1.21.5 (100 mg), N,N-diisopropylethylamine (68.9 µL) and tert-butyl (3-oxopropyl)carbamate (68.4 mg) in dichloromethane (3 mL) was stirred at ambient temperature for 2 hours, and NaCNBH₄ (8.27 mg) was added. The reaction was stirred at ambient temperature overnight. Methanol (1 mL) and water (0.2 mL) were added. The resulting mixture was stirred for 10 minutes and concentrated. The residue was dissolved in dimethyl sulfoxide and purified by reverse-phase HPLC on a Gilson system (C18 column), eluting with 30-80% acetonitrile in 0.1% trifluoroacetic acid water solution, to provide the title compound as a trifluoroacetic acid salt. MS (ESI) m/e 459.4 (M+2H)²⁺.

### 1.64.2 3-{1-[(3-{2-[(3-aminopropyl)(methyl)amino]ethoxy}-5,7-dimethyltricyclo[3.3.1.1^{3,7}]dec-1-yl)methyl]-5-methyl-1H-pyrazol-4-yl}-6-[1-(1,3-benzothiazol-2-ylcarbamoyl)-1,2,3,4-tetrahydroquinolin-7-yl]pyridine-2-carboxylic acid

Example 1.64.1 (100 mg) in dichloromethane (4 mL) at 0 °C was treated with trifluoroacetic acid (1 mL) for 1 hour, and the mixture was concentrated. The residue was purified by reverse phase HPLC (C18 column), eluting with a gradient of 10-60% acetonitrile in 0.1% trifluoroacetic acid water solution, to provide the title compound as a trifluoroacetic acid salt. ¹H NMR (400 MHz, dimethyl sulfoxide-*d*₆) δ 9.38 (s, 1H), 8.37 (s, 1H), 7.98 (d, 1H), 7.90 - 7.69 (m, 6H), 7.44 (s, 2H), 7.35 (td, 1H), 7.27 (d, 1H), 7.22 - 7.16 (m, 1H), 3.94 (d, 2H), 3.87 (s, 2H), 3.64 (t, 2H), 3.28 - 2.98 (m, 4H), 2.87 - 2.70 (m, 8H), 2.19 (s, 3H), 1.90 (dp, 4H), 1.43 (s, 2H), 1.36 - 1.22 (m, 4H), 1.15 (s, 4H), 1.08 - 0.95 (m, 2H), 0.86 (s, 6H). MS (ESI) m/e 817.6 (M+H)⁺.

### 1.65 Synthesis of 3-{1-[(3-{2-[azetidin-3-yl(methyl)amino]ethoxy}-5,7-dimethyltricyclo[3.3.1.1^{3,7}]dec-1-yl)methyl]-5-methyl-1H-pyrazol-4-yl}-6-[1-(1,3-benzothiazol-2-ylcarbamoyl)-1,2,3,4-tetrahydroquinolin-7-yl]pyridine-2-carboxylic acid (W2.65)

### 1.65.1 6-(1-(benzo[d]thiazol-2-ylcarbamoyl)-1,2,3,4-tetrahydroquinolin-7-yl)-3-(1-((3-(2-((1-(tert-butoxycarbonyl)azetidin-3-yl)(methyl)amino)ethoxy)-5,7-dimethyladamantan-1-yl)methyl)-5-methyl-1H-pyrazol-4-yl)picolinic acid

The title compound was prepared using the procedure described in Example 1.64.1, substituting tert-butyl (3-oxopropyl)carbamate with tert-butyl 3-oxoazetidine-1-carboxylate. MS (ESI) m/e 915.3 (M+H)⁺.

### 1.65.2 3-{1-[(3-{2-[azetidin-3-yl(methyl)amino]ethoxy}-5,7-dimethyltricyclo[3.3.1.1^{3,7}]dec-1-yl)methyl]-5-methyl-1H-pyrazol-4-yl}-6-[1-(1,3-benzothiazol-2-ylcarbamoyl)-1,2,3,4-tetrahydroquinolin-7-yl]pyridine-2-carboxylic acid

The title compound was prepared using the procedure in Example 1.64.2, substituting Example 1.64.1 with Example 1.65.1. ¹H NMR (400 MHz, dimethyl sulfoxide-*d*₆) δ 9.01 (s, 2H), 8.37 (s, 1H), 7.98 (d, 1H), 7.86 - 7.70 (m, 3H), 7.44 (s, 2H), 7.34 (td, 1H), 7.27 (d, 1H), 7.23 - 7.15 (m, 1H), 4.22 (s, 4H), 4.07 (s, 2H), 3.93 (t, 2H), 3.58 (t, 2H), 3.11 (s, 2H), 2.80 (t, 2H), 2.68 (s, 3H), 2.19 (s, 3H), 1.92 (p, 2H), 1.42 (s, 2H), 1.30 (s, 4H), 1.15 (s, 4H), 1.09 - 0.96 (m, 2H), 0.85 (s, 6H). MS (ESI) m/e 815.5 (M+H)⁺.

### 1.66 Synthesis of N6-(37-oxo-2,5,8,11,14,17,20,23,26,29,32,35-dodecaoxaheptatriacontan-37-yl)-L-lysyl-N- [2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl]-L-alaninamide (W2.66)

### 1.66.1 (S)-6-((((9H-fluoren-9-yl)methoxy)carbonyl)amino)-2-((tert-butoxycarbonyl)amino)hexanoic acid

To a solution of (S)-6-amino-2-((tert-butoxycarbonyl)amino)hexanoic acid (8.5 g) in a mixture of 5% aqueous NaHCO₃ solution (300 mL) and dioxane (40 mL), chilled in an ice bath, was added dropwise a solution of (9H-fluoren-9-yl)methyl pyrrolidin-1-yl carbonate (11.7 g) in dioxane (40 mL). The reaction mixture was allowed to warm to room temperature and was stirred for 24 hours. Three additional vials were set up as described above. After the reaction was completed, all four reaction mixtures were combined, and the organic solvent was removed under vacuum. The aqueous residue was acidified to pH 3 with aqueous hydrochloric acid solution (1N) and then extracted with ethyl acetate (3 × 500 mL). The combined organic layers were washed with brine, dried over magnesium sulfate, filtered, and concentrated under vacuum to give a crude compound which was recrystallized from methyl tert-butyl ether to afford the title compound. ¹H NMR (400MHz, chloroform-*d*) δ 11.05 (br. s., 1H), 7.76 (d, 2H), 7.59 (d, 2H), 7.45 - 7.27 (m, 4H), 6.52 - 6.17 (m, 1H), 5.16 - 4.87 (m, 1H), 4.54 - 4.17 (m, 4H), 3.26 - 2.98 (m, 2H), 1.76 - 1.64 (m, 1H), 1.62 - 1.31 (m, 14H).

### 1.66.2 tert-butyl 17-hydroxy-3,6,9,12,15-pentaoxaheptadecan-1-oate

To a solution of 3,6,9,12-tetraoxatetradecane-1,14-diol (40 g) in toluene (800 mL) was added portion-wise potassium tert-butoxide (20.7 g). The mixture was stirred at room temperature for 30 minutes. Tert-butyl 2-bromoacetate (36 g) was added dropwise to the mixture. The reaction was stirred at room temperature for 16 hours. Two additional vials were set up as described above. After the reactions were completed, all three reaction mixtures were combined. Water (500 mL) was added to the combined mixture, and the mixture was concentrated to 1 L. The mixture was extracted with dichloromethane and was washed with aqueous IN potassium tert-butoxide solution (1 L). The organic layer was dried over Na₂SO₄, filtered and concentrated to obtain crude product, which was purified by silica gel column chromatography, eluting with dichloromethane:methanol 50:1, to obtain the title compound. ¹H NMR (400MHz, chloroform-*d*) δ 4.01 (s, 2H), 3.75 - 3.58 (m, 21H), 1.46 (s,9H).

### 1.66.3 tert-butyl 17-(tosyloxy)-3,6,9,12,15-pentaoxaheptadecan-1-oate

To a solution of Example 1.66.2 (30 g) in dichloromethane (500 mL) was added dropwise a solution of 4-methylbenzene-1-sulfonyl chloride (19.5 g) and triethylamine (10.3 g) in dichloromethane (500 mL) at 0 °C under a nitrogen atmosphere. The mixture was stirred at room temperature for 18 hours and was poured into water (100 mL). The solution was extracted with dichloromethane (3 × 150 mL), and the organic layer was washed with hydrochloric acid (6N, 15 mL) then NaHCO₃ (5% aqueous solution, 15 mL) followed by water (20 mL). The organic layer was dried over Na₂SO₄, filtered and concentrated to obtain a residue, which was purified by silica gel column chromatography, eluting with petroleum ether:ethyl acetate 10:1 to dichloromethane:methanol 5:1, to obtain the title compound. ¹H NMR (400MHz, chloroform-*d*) δ 7.79 (d, 2H), 7.34 (d, 2H), 4.18 - 4.13 (m, 2H), 4.01 (s, 2H), 3.72 - 3.56 (m, 18H), 2.44 (s, 3H), 1.47 (s, 9H).

### 1.66.4 2,5,8,11,14,17,20,23,26,29,32,35-dodecaoxaheptatriacontan-37-oic acid

To a solution of 2,5,8,11,14,17-hexaoxanonadecan-19-ol (32.8 g) in tetrahydrofuran (300 mL) was added sodium hydride (1.6 g) at 0 °C. The mixture was stirred at room temperature for 4 hours. A solution of Example 1.66.3 (16 g) in tetrahydrofuran (300 mL) was added dropwise at room temperature to the reaction mixture. The resulting reaction mixture was stirred at room temperature for 16 hours and then water (20 mL) was added. The mixture was stirred at room temperature for another 3 hours to complete the tert-butyl ester hydrolysis. The final reaction mixture was concentrated under vacuum to remove the organic solvent. The aqueous residue was extracted with dichloromethane (2 × 150 mL). The aqueous layer was acidified to pH 3 and then extracted with ethyl acetate (2 × 150 mL). The aqueous layer was concentrated to obtain crude product, which was purified by silica gel column chromatography, eluting with a gradient of petroleum ether:ethyl acetate 1:1 to dichloromethane:methanol 5:1, to obtain the title compound. ¹H NMR (400MHz, chloroform-d) δ 4.19 (s, 2H), 3.80 - 3.75 (m, 2H), 3.73 - 3.62 (m, 40H), 3.57 (dd, 2H), 3.40 (s, 3H).

### 1.66.5 (43S,46S)-43-((tert-butoxycarbonyl)amino)-46-methyl-37,44-dioxo-2,5,8,11,14,17,20,23,26,29,32,35-dodecaoxa-38,45-diazaheptatetracontan-47-oic acid

Example 1.66.5 was synthesized using standard Fmoc solid phase peptide synthesis procedures and a 2-chlorotrytil resin. 2-Chlorotrytil resin (12 g, 100 mmol), (S)-2-((((9H-fluoren-9-yl)methoxy)carbonyl)amino)propanoic acid (10 g, 32.1 mmol) and N,N-diisopropylethylamine (44.9 mL, 257 mmol) in anhydrous, sieve-dried dichloromethane (100 mL) was shaken at 14 °C for 24 hours. The mixture was filtered and the cake was washed with dichloromethane (3 × 500 mL), dimethylformamide (2 × 250 mL) and methanol (2 × 250 mL) (for 5 minutes for each step). To the above resin was added 20% piperidine/dimethylformamide (100 mL) to remove the Fmoc group. The mixture was bubbled with nitrogen for 15 minutes and then filtered. The resin was washed with 20% piperidine/dimethylformamide (100 mL) another five times (5 minutes each step), and washed with dimethylformamide (5 × 100 mL) to give the deprotected, L-Ala loaded resin.

To a solution of Example 1.66.1 (9.0 g) in N,N-dimethylformamide (50 mL) was added hydroxybenzotriazole (3.5 g), 2-(6-chloro-1H-benzotriazole-1-yl)-1,1,3,3-ethylaminium hexafluorophosphate (9.3 g) and N,N-diisopropylethylamine (8.4 mL). The mixture was stirred at 20 °C for 30 minutes. The above mixture was added to the D-Ala loaded resin and mixed by bubbling with nitrogen at room temperature for 90 minutes. The mixture was filtered and the resin was washed with dimethylformamide (5 minutes each step). To the above resin was added approximately 20% piperidine/ N,N-dimethylformamide (100 mL) to remove the Fmoc group. The mixture was bubbled with nitrogen for 15 minutes and filtered. The resin was washed with 20% piperidine/dimethylformamide (100 mL) for another five times (5 minutes for each step), and finally washed with dimethylformamide (5 × 100 mL).

To a solution of Example 1.66.4 (11.0 g) in N,N-dimethylformamide (50 mL) was added hydroxybenzotriazole (3.5 g), 2-(6-chloro-1H-benzotriazole-1-yl)-1,1,3,3-tetramethylaminium hexafluorophosphate (9.3g) and N,N-diisopropylethylamine (8.4 mL), and the mixture was added to the resin and mixed by bubbling with nitrogen at room temperature for 3 hours. The mixture was filtered and the residue was washed with dimethylformamide (5 × 100 mL), dichloromethane (8 × 100 mL) (5 minutes for each step).

To the final resin was added 1% trifluoroacetic acid/dichloromethane (100 mL) and nitrogen was bubbled through for 5 minutes. The mixture was filtrated and the filtrate was collected. The cleavage operation was repeated for four times. The combined filtrate was brought to pH 7 by NaHCO₃ and washed with water. The organic layer was dried over Na₂SO₄, filtered and concentrated to obtain the title compound. ¹H NMR (400MHz, methanol-*d₄*) δ 4.44 - 4.33 (m, 1H), 4.08 - 4.00 (m, 1H), 3.98 (s, 2H), 3.77 - 3.57 (m, 42H), 3.57 - 3.51 (m, 2H), 3.36 (s, 3H), 3.25 (t, 2H), 1.77 (br. s., 1H), 1.70 - 1.51 (m, 4H), 1.44 (s, 9H), 1.42 - 1.39 (m, 3H).

### 1.66.6 tert-butyl 6-(8-(benzo[d]thiazol-2-ylcarbamoyl)-3,4-dihydroisoquinolin-2(1H)-yl)-3-(1-((3-(((43S,46S)-43-((tert-butoxycarbonyl)amino)-46-methyl-37,44,47-trioxo-2,5,8,11,14,17,20,23,26,29,32,35-dodecaoxa-38,45,48-triazapentacontan-50-yl)oxy)-5,7-dimethyladamantan-1-yl)methyl)-5-methyl-1H-pyrazol-4-yl)picolinate

Example 1.66.5 (123 mg, 0.141 mmol), was mixed with 1-[bis(dimethylamino)methylene]-1H-1,2,3-triazolo[4,5-b]pyridinium 3-oxid hexafluorophosphate (58.9 mg) and N,N-diisopropylethylamine (0.049 mL) in N-methyl-2-pyrrolidone (1 mL) for 10 minutes and then added to a solution of Example 1.2.7 (142 mg) and N,N-diisopropylethylamine (0.049 mL) in N-methyl-2-pyrrolidone (1.5 mL). The reaction mixture was stirred at room temperature for two hours. The crude reaction mixture was purified by reverse phase HPLC using a Gilson system and a C18 25 x 100 mm column, eluting with 5-85% acetonitrile in water containing 0.1% v/v trifluoroacetic acid. The product fractions were lyophilized to give the title compound. MS (LC/MS) m/e 1695.5 (M+H)⁺.

### 1.66.7 3-(1-((3-(((43S,46S)-43-amino-46-methyl-37,44,47-trioxo-2,5,8,11,14,17,20,23,26,29,32,35-dodecaoxa-38,45,48-triazapentacontan-50-yl)oxy)-5,7-dimethyladamantan-1-yl)methyl)-5-methyl-1H-pyrazol-4-yl)-6-(8-(benzo[d]thiazol-2-ylcarbamoyl)-3,4-dihydroisoquinolin-2(1H)-yl)picolinic acid

Example 1.66.6 (82 mg) was treated with 1 mL of trifluoroacetic acid at room temperature for 30 minutes. The solvent was evaporated under a gentle stream of nitrogen, and the residue was purified by reverse phase HPLC using a Gilson system and a C18 25 x 100 mm column, eluting with 5-85% acetonitrile in water containing 0.1% v/v trifluoroacetic acid. The product fractions were lyophilized to give the title compound as the trifluoroacetic acid salt. ¹H NMR (400 MHz, dimethyl sulfoxide-*d₆*) δ ppm 12.86 (s, 1H), 8.04 (dd, 4H), 7.64 (dt, 2H), 7.55 - 7.41 (m, 3H), 7.36 (q, 2H), 6.95 (d, 1H), 4.96 (s, 2H), 4.40 - 4.27 (m, 1H), 3.93 - 3.72 (m, 7H), 3.59 - 3.47 (m, 42H), 3.33 - 3.27 (m, 3H), 3.23 (s, 5H), 3.05 (dt, 5H), 2.10 (s, 3H), 1.72 - 1.64 (m, 2H), 1.48 - 1.36 (m, 4H), 1.35 - 1.16 (m, 10H), 1.16 - 0.94 (m, 6H), 0.84 (d, 6H). MS (ESI) m/e 751.8 (2M+H)²⁺.

### 1.67 Synthesis of methyl 6-[4-(3-{[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl]amino}propyl)-1H-1,2,3-triazol-1-yl]-6-deoxy-beta-L-glucopyranoside (W2.67)

### 1.67.1 6-(8-(benzo[d]thiazol-2-ylcarbamoyl)-3,4-dihydroisoquinolin-2(1H)-yl)-3-(1-((3,5-dimethyl-7-(2-(pent-4-yn-1-ylamino)ethoxy)adamantan-1-yl)methyl)-5-methyl-1H-pyrazol-4-yl)picolinic acid

To a solution oftert-butyl 3-(1-((3-(2-aminoethoxy)-5,7-dimethyladamantan-1-yl)methyl)-5-methyl-1H-pyrazol-4-yl)-6-(8-(benzo[d]thiazol-2-ylcarbamoyl)-3,4-dihydroisoquinolin-2(1H)-yl)picolinate (85 mg) in tetrahydrofuran (2 mL) was added pent-4-ynal (8.7 mg), acetic acid (20 mg, 0.318) and anhydrous sodium sulfate (300 mg). The mixture was stirred at room temperature for 1 hour. Sodium triacetoxyborohydride (45 mg) was added to the reaction mixture. The mixture was stirred at room temperature overnight. The reaction mixture was diluted with ethyl acetate (200 mL), washed with water and brine, and dried over anhydrous sodium sulfate. Filtration and evaporation of the solvent gave crude product, which was dissolved in dichloromethane (5 mL) and trifluoroacetic acid (3 mL). The mixture was stirred at room temperature overnight. After evaporation of the solvent, the residue was dissolved in dimethyl sulfoxide/methanol (1:1, 3 mL) and purified by reverse-phase HPLC on a Gilson system (C18 column), eluting with 20-80% acetonitrile in water containing 0.1% trifluoroacetic acid, to give the title compound. MS (APCI) m/e 812.2 (M+H)⁺.

### 1.67.2 methyl 6-[4-(3-{[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl]amino}propyl)-1H-1,2,3-triazol-1-yl]-6-deoxy-beta-L-glucopyranoside

To a solution of (2R,3R,4S,5S,6S)-2-azido-6-(methoxycarbonyl)tetrahydro-2H-pyran-3,4,5-triyl triacetate (8.63 mg) in t-BuOH (2 mL) and water (1 mL) was added Example 1.67.1 (20 mg), copper (II) sulfate pentahydrate (2.0 mg) and sodium ascorbate (5 mg). The mixture was heated for 20 minutes at 100 °c under microwave conditions (Biotage Initiator). LiOH H₂O (50 mg) was added to the mixture, which was stirred at room temperature overnight. The mixture was neutralized with trifluoroacetic acid and purified by reverse-phase HPLC on a Gilson system (C18 column), eluting with 20-80% acetonitrile in water containing 0.1% trifluoroacetic acid, to give the title compound. MS (APCI) m/e 1032.2 (M+H)⁺.

### 1.68 Synthesis of 6-[8-(1,3-benzothiazol-2-ylcarbamoyl)naphthalen-2-yl]-3-{1-[(3-{2-[(2-carboxyethyl)amino]ethoxy}-5,7-dimethyltricyclo[3.3.1.1^{3,7}]dec-1-yl)methyl]-5-methyl-1H-pyrazol-4-yl}pyridine-2-carboxylic acid

### 1.68.1 2-((3,5-dimethyl-7-((5-methyl-4-(4,4,5,5-tetramethyl-1,3,2-dioxaborolan-2-yl)-1H-pyrazol-1-yl)methyl)adamantan-1-yl)oxy)ethanol (W2.68)

To a solution of 2-((3-((4-iodo-5-methyl-1H-pyrazol-1-yl)methyl)-5,7-dimethyladamantan-1-yl)oxy)ethanol (8.9 g) and PdCl₂(dppf)-CH2Cl₂ adduct (([1,1'-bis(diphenylphosphino)ferrocene]dichloropalladium(II) (1:1), 818 mg) in acetonitrile (120 mL) was added trimethylamine (10 mL) and 4,4,5,5-tetramethyl-1,3,2-dioxaborolane (12.8 mL). The mixture was stirred at reflux overnight. The mixture was cooled to room temperature and used in the next reaction without further work up. MS (ESI) m/e 467.3 (M+Na)⁺.

### 1.68.2 tert-butyl 6-chloro-3-(1-((3-(2-hydroxyethoxy)-5,7-dimethyladamantan-1-yl)methyl)-5-methyl-1H-pyrazol-4-yl)picolinate

To a solution of tert-butyl 3-bromo-6-chloropicolinate (6.52 g) in tetrahydrofuran (100 mL) and water (20 mL) was added Example 1.68.1 (9.90 g), (1S,3R,5R,7S)-1,3,5,7-tetramethyl-8-tetradecyl-2,4,6-trioxa-8-phosphaadamantane (0.732 g), tris(dibenzylideneacetone)dipalladium(0) (Pd₂(dba)₃, 1.02 g), and K₃PO₄ (23.64 g). The mixture was stirred at reflux overnight. The mixture was concentrated under reduced pressure, the residue was dissolved in ethyl acetate (500 mL), washed with water and brine, and dried over anhydrous sodium sulfate. Filtration and evaporation of the solvent gave crude product, which was purified by silica gel chromatography eluting with 20 to 40% ethyl acetate in dichloromethane to give the title compound. MS (ESI) m/e 530.3 (M+H)⁺.

### 1.68.3 tert-butyl 6-chloro-3-(1-((3,5-dimethyl-7-(2-((methylsulfonyl)oxy)ethoxy)adamantan-1-yl)methyl)-5-methyl-1H-pyrazol-4-yl)picolinate

To a cooled (0 °c) solution of Example 1.68.2 (3.88 g) in dichloromethane (30 mL) and triethylamine (6 mL) was added methanesulfonyl chloride (2.52 g). The mixture was stirred at room temperature for 4 hours. The reaction mixture was diluted with ethyl acetate (400 mL), washed with water and brine, and dried over anhydrous sodium sulfate. Filtration and evaporation of the solvent gave the crude product (4.6 g), which was used in the next reaction without further purification. MS (ESI) m/e 608.1 (M+H)⁺.

### 1.68.4 tert-butyl 3-{1-[(3-{2-[bis(tert-butoxycarbonyl)amino]ethoxy}-5,7-dimethyltricyclo[3.3.1.1^{3,7}]dec-1-yl)methyl]-5-methyl-1H-pyrazol-4-yl}-6-chloropyridine-2-carboxylate

To a solution of Example 1.68.3 (151 mg) in N,N-dimethylformamide (3 mL) was added di-tert-butyl iminodicarboxylate (54 mg). The mixture was stirred at room temperature overnight. The reaction mixture was diluted with ethyl acetate (200 mL), washed with water and brine, and dried over anhydrous sodium sulfate. Filtration and evaporation of the solvent gave the title compound, which was used in the next step without further purification. MS (ESI) m/e 729.4 (M+H)⁺.

### 1.68.5 7-(6-(tert-butoxycarbonyl)-5-(1-((3-(2-((tert-butoxycarbonyl)amino)ethoxy)-5,7-dimethyladamantan-1-yl)methyl)-5-methyl-1H-pyrazol-4-yl)pyridin-2-yl)-1-naphthoic acid

To a solution of methyl 7-(4,4,5,5-tetramethyl-1,3,2-dioxaborolan-2-yl)-1-naphthoate (257 mg) in 1,4-dioxane (10 mL) and water (5 mL) was added Example 1.68.4 (600 mg), bis(triphenylphosphine)palladium(II) dichloride (57.8 mg), and CsF (375 mg). The mixture was stirred at 120 °C for 30 minutes under microwave conditions (Biotage Initiator). The mixture was diluted with ethyl acetate (200 mL), washed with water and brine, and dried over anhydrous sodium sulfate. Filtration and evaporation of the solvent gave crude product, which was purified by silica gel chromatography, eluting with 20% ethyl acetate in heptane to give a di-ester intermediate. The residue was dissolved in tetrahydrofuran (10 mL), methanol (5 mL) and water (5 mL) and LiOH H₂O (500 mg) was added, and the mixture was stirred at room temperature overnight. The mixture was acidified with 2N aqueous HC1, dissolved in 400 mL of ethyl acetate, washed with water and brine, and dried over anhydrous sodium sulfate. Filtration and evaporation of the solvent gave the title compound. MS (APCI) m/e 765.3 (M+H)⁺.

### 1.68.6 3-(1-((3-(2-aminoethoxy)-5,7-dimethyladamantan-1-yl)methyl)-5-methyl-1H-pyrazol-4-yl)-6-(8-(benzo[d]thiazol-2-ylcarbamoyl)naphthalen-2-yl)picolinic acid

To a solution of Example 1.68.5 (500 mg) in dichloromethane (10 mL) was added benzo[d]thiazol-2-amine (98 mg), 1-ethyl-3-(3-dimethylaminopropyl)carbodiimide (251 mg) and 4-dimethylaminopyridine (160 mg). The mixture was stirred at room temperature overnight. The reaction mixture was diluted with ethyl acetate (400 mL), washed with water and brine, and dried over anhydrous sodium sulfate. Filtration and evaporation of the solvent gave a residue that was dissolved in dichloromethane and trifluoroacetic acid (10 mL, 1:1). After stirring overnight, the solution was concentrated under reduced pressure. The residue was dissolved in N,N-dimethylformamide (12 mL) and purified by reverse-phase HPLC (using a Gilson system and a C18 column, eluting with 20-80% acetonitrile in water containing 0.1% trifluoroacetic acid) to give the title compound. MS (ESI) m/e 741.2 (M+H)⁺.

### 1.68.7 6-[8-(1,3-benzothiazol-2-ylcarbamoyl)naphthalen-2-yl]-3-{1-[(3-{2-[(2-carboxyethyl)amino]ethoxyl-5,7-dimethyltricyclo[3.3.1.1^{3,7}]dec-1-yl)methyl]-5-methyl-1H-pyrazol-4-yl}pyridine-2-carboxylic acid

To a solution of Example 1.68.6 (35 mg) in N,N-dimethylformamide (4 mL) was added tert-butyl acrylate (120 mg) and H₂O (138 mg). The mixture was stirred at room temperature overnight. The reaction mixture was diluted with ethyl acetate (400 mL), washed with water and brine, and dried over anhydrous sodium sulfate. Filtration and evaporation of the solvent gave a residue that was dissolved in dichloromethane and trifluoroacetic acid (10 mL, 1:1). After 16 hours, the mixture was concentrated under reduced pressure. The residue was dissolved in N,N-dimethylformamide (2 mL) and purified by reverse-phase HPLC on a Gilson system (C18 column), eluting with 20-80% acetonitrile in water containing 0.1% trifluoroacetic acid, to give the title compound. ¹H NMR (400 MHz, dimethyl sulfoxide-*d*₆) δ ppm 13.08 (s, 1H), 8.99 (d, 1H), 8.43 - 8.24 (m, 4H), 8.24 - 8.11 (m, 3H), 8.04 (d, 1H), 7.99 (d, 1H), 7.90 (d, 1H), 7.78 (d, 1H), 7.74 - 7.62 (m, 1H), 7.53 - 7.43 (m, 2H), 7.35 (q, 1H), 3.87 (s, 2H), 3.08 (dp, 4H), 2.62 (t, 2H), 2.20 (s, 3H), 1.43 (s, 2H), 1.29 (q, 4H), 1.14 (s, 4H), 1.03 (q, 2H), 0.85 (s, 6H).

### 1.69 Synthesis of 6-[5-(1,3-benzothiazol-2-ylcarbamoyl)quinolin-3-yl]-3-{1-[(3,5-dimethyl-7-{2-[(2-sulfoethyl)amino]ethoxy}tricyclo[3.3.1.1^{3,7}]dec-1-yl)methyl]-5-methyl-1H-pyrazol-4-yl}pyridine-2-carboxylic acid 1.69.1 methyl 3-bromoquinoline-5-carboxylate (W2.69)

To a solution of 3-bromoquinoline-5-carboxylic acid (2 g) in methanol (30 mL) was added concentrated H₂SO₄ (5 mL). The solution was stirred at reflux overnight. The mixture was concentrated under reduced pressure. The residue was dissolved in ethyl acetate (300 mL) and washed with aqueous Na₂CO₃ solution, water and brine. After drying over anhydrous sodium sulfate, filtration and evaporation of the solvent gave the title compound. MS (ESI) m/e 266 (M+H)⁺.

### 1.69.2 methyl 3-(4,4,5,5-tetramethyl-1,3,2-dioxaborolan-2-yl)quinoline-5-carboxylate

To a solution of Example 1.69.1 (356 mg) in N,N-dimethylformamide (5 mL) was added PdCl₂(dppf)-CH₂Cl₂ adduct ([1,1'-bis(diphenylphosphino)ferrocene]dichloropaladium(II) (1:1), 55 mg) potassium acetate (197 mg) and bis(pinacolato)diboron (510 mg). The mixture was stirred at 60 °C overnight. The mixture was cooled to room temperature and used in the next reaction without further work up. MS (ESI) m/e 339.2 (M+Na)⁺.

### 1.69.3 methyl 3-[5-{1-[(3-{2-[bis(tert-butoxycarbonyl)amino]ethoxy}-5,7-dimethyltricyclo[3.3.1.1^{3,7}]dec-1-yl)methyl]-5-methyl-1H-pyrazol-4-yl}-6-(tert-butoxycarbonyl)pyridin-2-yl]quinoline-5-carboxylate

To a solution of Example 1.69.2 (626 mg) in 1,4-dioxane (10 mL) and water (5 mL) was added Example 1.68.4 (1.46 g), bis(triphenylphosphine)palladium(II) dichloride (140 mg), and CsF (911 mg). The mixture was stirred at 120 °C for 30 minutes under microwave conditions (Biotage Initiator). The mixture was diluted with ethyl acetate (200 mL), washed with water and brine, dried over anhydrous sodium sulfate, filtered and concentrated. The residue was purified by silica gel chromatography, eluting with 20% ethyl acetate in heptane (1 L) to give the title compound. MS (ESI) m/e 880.3 (M+H)⁺.

### 1.69.4 3-(6-(tert-butoxycarbonyl)-5-(1-((3-(2-((tert-butoxycarbonyl)amino)ethoxy)-5,7-dimethyladamantan-1-yl)methyl)-5-methyl-1H-pyrazol-4-yl)pyridin-2-yl)quinoline-5-carboxylic acid

To a solution of Example 1.69.3 (1.34 g) in tetrahydrofuran (10 mL), methanol (5 mL) and water (5 mL) was added LiOH H₂O (120 mg), and the mixture was stirred at room temperature overnight. The mixture was acidified with 2N aqueous HC1, diluted with ethyl acetate (400 mL), washed with water and brine, and dried over anhydrous sodium sulfate. Filtration and evaporation of the solvent gave the title compound. MS (APCI) m/e 766.3 (M+H)⁺.

### 1.69.5 3-(1-((3-(2-aminoethoxy)-5,7-dimethyladamantan-1-yl)methyl)-5-methyl-1H-pyrazol-4-yl)-6-(5-(benzo[d]thiazol-2-ylcarbamoyl)quinolin-3-yl)picolinic acid

To a solution of Example 1.69.4 (200 mg) in dichloromethane (10 mL) was added benzo[d]thiazol-2-amine (39.2 mg), 1-ethyl-3-(3-dimethylaminopropyl)carbodiimide (50 mg) and 4-dimethylaminopyridine (32 mg). The mixture was stirred at room temperature overnight. The reaction mixture was diluted with ethyl acetate (200 mL), washed with water and brine, dried over anhydrous sodium sulfate, filtered, and concentrated. The residue was dissolved in dichloromethane and trifluoroacetic acid (10 mL, 1:1), and the reaction was stirred overnight. The mixture was concentrated, and the residue was dissolved in N,N-dimethylformamide (12 mL) and purified by reverse-phase HPLC on a Gilson system (C18 column), eluting with 20-80% acetonitrile in water containing 0.1% trifluoroacetic acid, to give the title compound. MS (ESI) m/e 742.1 (M+H)⁺.

### 1.69.6 6-[5-(1,3-benzothiazol-2-ylcarbamoyl)quinolin-3-yl]-3-{1-[(3,5-dimethyl-7-{2-[(2-sulfoethyl)amino]ethoxy}tricyclo[3.3.1.1^{3,7}]dec-1-yl)methyl]-5-methyl-1H-pyrazol-4-yl}pyridine-2-carboxylic acid

To a solution of Example 1.69.5 (36 mg) in N,N-dimethylformamide (2 mL) was added 4-((tert-butyldiphenylsilyl)oxy)-2,2-dimethylbutyl ethenesulfonate (22 mg) and H₂O (0.3 mL)). The mixture was stirred at room temperature for 3 hours. The reaction mixture was diluted with dichloromethane and trifluoroacetic acid (10 mL, 1:1) and stirred overnight. The mixture was concentrated, and the residue was dissolved in N,N-dimethylformamide (4 mL) and purified by reverse-phase HPLC on a Gilson system (C18 column), eluting with 20-80% acetonitrile in water containing 0.1% trifluoroacetic acid, to give the title compound. ¹H NMR (400 MHz, dimethyl sulfoxide-*d*₆) δ ppm 13.19 (s, 2H), 9.70 (d, 1H), 9.40 (s, 1H), 8.31 (d, 2H), 8.16 (d, 1H), 8.06 (d, 1H), 8.01 (d, 1H), 7.98 - 7.88 (m, 1H), 7.80 (d, 1H), 7.52 - 7.43 (m, 2H), 7.37 (q, 1H), 3.89 (s, 2H), 3.22 (p, 2H), 3.10 (q, 2H), 2.80 (t, 2H), 2.23 (s, 3H), 1.43 (s, 2H), 1.30 (q, 4H), 1.23 - 1.10 (m, 4H), 1.04 (q, 2H), 0.87 (s, 6H). MS (ESI) m/e 850.2 (M+H)⁺.

### 1.70 Synthesis of 6-[4-(1,3-benzothiazol-2-ylcarbamoyl)quinolin-6-yl]-3-{1-[(3,5-dimethyl-7-{2-[(2-sulfoethyl)amino]ethoxy}tricyclo[3.3.1.1^{3,7}]dec-1-yl)methyl]-5-methyl-1H-pyrazol-4-yl}pyridine-2-carboxylic acid (W2.70)

### 1.70.1 ethyl 6-(4,4,5,5-tetramethyl-1,3,2-dioxaborolan-2-yl)quinoline-4-carboxylate

To a solution of ethyl 6-bromoquinoline-4-carboxylate (140 mg) in N,N-dimethylformamide (2 mL) was added PdCl₂(dppf)-CH₂Cl₂ adduct (([1,1'-bis(diphenylphosphino)ferrocene]dichloropalladium(II) (1:1), 20.42 mg), potassium acetate (147 mg) and bis(pinacolato)diboron (190 mg). The mixture was stirred at 60 °C overnight. The mixture was cooled to room temperature and used in the next reaction without further work up. MS (ESI) m/e 328.1(M+H)⁺.

### 1.70.2 ethyl 6-[5-{1-[(3-{2-[bis(tert-butoxycarbonyl)amino]ethoxy}-5,7-dimethyltricyclo[3.3.1.1^{3,7}] dec-1-yl)methyl]-5-methyl-1H-pyrazol-4-yl}-6-(tert-butoxycarbonyl)pyridin-2-yl]quinoline-4-carboxylate

To a solution of Example 1.70.1 (164 mg) in 1,4-dioxane (10 mL) and water (5 mL) was added Example 1.68.4 (365 mg), bis(triphenylphosphine)palladium(II) dichloride (35 mg), and CsF (228 mg). The mixture was stirred at 120 °C for 30 minutes under microwave conditions (Biotage Initiator). The mixture was diluted with ethyl acetate (200 mL), washed with water and brine, dried over anhydrous sodium sulfate, filtered and concentrated. The residue was purified by silica gel chromatography, eluting with 20% ethyl acetate in heptane (1 L) to give the title compound. MS (ESI) m/e 894.3 (M+H)⁺.

### 1.70.3 6-(6-(tert-butoxycarbonyl)-5-(1-((3-(2-((tert-butoxycarbonyl)amino)ethoxy)-5,7-dimethyladamantan-1-yl)methyl)-5-methyl-1H-pyrazol-4-yl)pyridin-2-yl)quinoline-4-carboxylic acid

To a solution of Example 1.70.2 (3.1 g) in tetrahydrofuran (20 mL), methanol (10 mL) and water (10 mL) was added LiOH H₂O(240 mg). The mixture was stirred at room temperature overnight. The mixture was acidified with 2N aqueous HCl and diluted with ethyl acetate (400 mL). The organic layer was washed with water and brine and dried over anhydrous sodium sulfate. Filtration and evaporation of the solvent gave the title compound. MS (ESI) m/e 766.3 (M+H)⁺.

### 1.70.4 3-(1-((3-(2-aminoethoxy)-5,7-dimethyladamantan-1-yl)methyl)-5-methyl-1H-pyrazol-4-yl)-6-(4-(benzo[d]thiazol-2-ylcarbamoyl)quinolin-6-yl)picolinic acid

To a solution of Example 1.70.3 (4.2 g) in dichloromethane (30 mL) was added benzo[d]thiazol-2-amine (728 mg), 1-ethyl-3-(3-dimethylaminopropyl)carbodiimide (1.40 g) and 4-dimethylaminopyridine (890 mg), and the mixture was stirred at room temperature overnight. The reaction mixture was diluted with ethyl acetate (500 mL), washed with water and brine, and dried over anhydrous sodium sulfate. Filtration and evaporation of the solvent gave a residue that was dissolved in dichloromethane and trifluoroacetic acid (10 mL, 1:1) and stirred overnight. The mixture was concentrated, and the residue was dissolved in N,N-dimethylformamide (4 mL) and purified by reverse-phase HPLC on a Gilson system (C18 column), eluting with 20-80% acetonitrile in water containing 0.1% trifluoroacetic acid, to give the title compound. MS (ESI) m/e 742.2 (M+H)⁺.

### 1.70.5 6-[4-(1,3-benzothiazol-2-ylcarbamoyl)quinolin-6-yl]-3-{1-[(3,5-dimethyt-7-{2-[(2-sutfoethyt)amino]ethoxy}tricyclo[3.3.1.1^{3,7}]dec-1-yl)methyl]-5-methyl-1H-pyrazol-4-yl}pyridine-2-carboxylic acid

To a solution of Example 1.70.4 (111 mg) in N,N-dimethylformamide (4 mL) was added 4-((tert-butyldiphenylsilyl)oxy)-2,2-dimethylbutylethenesulfonate (67 mg), N,N-diisopropylethylamine (0.2 mL) and H₂O (0.3 mL). The mixture was stirred at room temperature for 3 hours. The reaction mixture was diluted with dichloromethane and trifluoroacetic acid (10 mL, 1:1) and stirred overnight. The mixture was concentrated, and the residue was dissolved in N,N-dimethylformamide (4 mL) and purified by reverse-phase HPLC on a Gilson system (C18 column), eluting with 20-80% acetonitrile in water containing 0.1% trifluoroacetic acid, to give the title compound. ¹HNMR(400 MHz, dimethyl sulfoxide-*d*₆) δ ppm 13.31 (s, 1H), 9.10 (d, 1H), 8.91 (s, 1H), 8.58 (dd, 1H), 8.47 - 8.16 (m, 4H), 8.06 (dd, 1H), 7.99 - 7.89 (m, 2H), 7.79 (d, 1H), 7.53 - 7.43 (m, 2H), 7.42 - 7.31 (m, 1H), 3.87 (s, 2H), 3.53 (d, 1H), 3.20 (p, 2H), 3.07 (p, 2H), 2.78 (t, 2H), 2.20 (s, 3H), 1.40 (s, 2H), 1.28 (q, 4H), 1.21 - 1.07 (m, 4H), 1.02 (q, 2H), 0.84 (s, 6H). MS (ESI) m/e 850.1 (M+H)⁺.

### 1.71 Synthesis of 6-[5-(1,3-benzothiazol-2-ylcarbamoyl)quinolin-3-yl]-3-{1-[(3-{2-[(2-carboxyethyl)amino]ethoxy}-5,7-dimethyltricyclo[3.3.1.1^{3,7}]dec-1-yl)methyl]-5-methyl-1H-pyrazol-4-yl}pyridine-2-carboxylic acid (W2.71)

To a solution of Example 1.69.5 (140 mg) in N,N-dimethylformamide (10 mL) was added tert-butyl acrylate (242 mg), and H₂O (0.3 mL), and the mixture was stirred at room temperature over the weekend. The reaction mixture was diluted with dichloromethane and trifluoroacetic acid (10 mL, 1:1) and stirred overnight. The mixture was concentrated, and the residue was dissolved in N,N-dimethylformamide (4 mL) and purified by reverse-phase HPLC on a Gilson system (C18 column), eluting with 20-80% acetonitrile in water containing 0.1% trifluoroacetic acid, to give the title compound. ¹H NMR (400 MHz, dimethyl sulfoxide-*d*₆) δ ppm 13.17 (s, 2H), 9.69 (d, 1H), 9.37 (d, 1H), 8.30 (dd, 3H), 8.15 (dd, 1H), 8.04 (dd, 1H), 7.99 - 7.88 (m, 2H), 7.79 (d, 1H), 7.53 - 7.40 (m, 2H), 7.34 (td, 1H), 3.88 (s, 2H), 3.55 (t, 2H), 3.08 (dt, 4H), 2.62 (t, 2H), 2.21 (s, 3H), 1.43 (s, 2H), 1.29 (q, 4H), 1.14 (s, 4H), 1.03 (q, 2H), 0.85 (s, 6H). MS (ESI) m/e 814.2 (M+H)⁺.

### 1.72 Synthesis of 6-[1-(1,3-benzothiazol-2-ylcarbamoyl)-5,6-dihydroimidazo[1,5-a]pyrazin-7(8H)-yl]-3-{1-[(3,5-dimethyl-7-{2-[(2-sulfoethyl)amino]ethoxy}tricyclo[3.3.1.1^{3,7}]dec-1-yl)methyl]-5-methyl-1H-pyrazol-4-yl}pyridine-2-carboxylic acid (W2.72)

### 1.72.1 ethyl 7-(5-bromo-6-(tert-butoxycarbonyl)pyridin-2-yl)-5,6,7,8-tetrahydroimidazo[1,5-a]pyrazine-1-carboxylate

The title compound was prepared by substituting ethyl 5,6,7,8-tetrahydroimidazo[1,5-a]pyrazine-1-carboxylate hydrochloride for 1,2,3,4-tetrahydroisoquinoline-8-carboxylate hydrochloride in Example 1.1.11. MS (ESI) m/e 451, 453 (M+H)⁺, 395, 397 (M-tert-butyl)⁺.

### 1.72.2 ethyl 7-(6-(tert-butoxycarbonyl)-5-(4,4,5,5-tetramethyl-1,3,2-dioxaborolan-2-yl)pyridin-2-yl)-5,6,7,8-tetrahydroimidazo[1,5-a]pyrazine-1-carboxylate

The title compound was prepared by substituting Example 1.72.1 for Example 1.1.11 in Example 1.2.1. MS (ESI) m/e 499 (M+H)⁺, 443 (M- tert-butyl)+, 529 (M+CH₃OH-H)⁻.

### 1.72.3 ethyl 7-(6-(tert-butoxycarbonyl)-5-(1-((3-(2-((tert-butoxycarbonyl)amino)ethoxy)-5,7-dimethyladamantan-1-yl)methyl)-5-methyl-1H-pyrazol-4-yl)pyridin-2-yl)-5,6,7,8-tetrahydroimidazo[1,5-a]pyrazine-1-carboxylate

The title compound was prepared by substituting Example 1.72.2 for Example 1.2.1 and Example 1.55.11 for Example 1.13.3 in Example 1.13.4. MS (ESI) m/e 760 (M+H)⁺, 758 (M-H)⁻.

### 1.72.4 7-(6-(tert-butoxycarbonyl)-5-(1-((3-(2-((tert-butoxycarbonyl)amino)ethoxy)-5,7-dimethyladamantan-1-yl)methyl)-5-methyl-1H-pyrazol-4-yl)pyridin-2-yl)-5,6,7,8-tetrahydroimidazo[1,5-a]pyrazine-1-carboxylic acid

The title compound was prepared by substituting Example 1.72.3 for Example 1.1.12 in Example 1.1.13. MS (ESI) m/e 760 (M+H)⁺, 758 (M-H)⁻.

### 1.72.5 tert-butyl 6-(1-(benzo[d]thiazol-2-ylcarbamoyl)-5,6-dihydroimidazo[1,5-a]pyrazin-7(8H)-yl)-3-(1-((3-(2-((tert-butoxycarbonyl)amino)ethoxy)-5,7-dimethyladamantan-1-yl)methyl)-5-methyl-1H-pyrazol-4-yl)picolinate

The title compound was prepared by substituting Example 1.72.4 for Example 1.52.2 in Example 1.52.3. MS (ESI) m/e 892 (M+H)⁺, 890 (M-H)⁻.

### 1.72.6 3-(1-{[3-(2-aminoethoxy)-5,7-dimethyltricyclo[3.3.1.1^{3,7}]dec-1-yl]methyl}-5-methyl-1H-pyrazol-4-yl)-6-[1-(1,3-benzothiazol-2-ylcarbamoyl)-5,6-dihydroimidazo[1,5-a]pyrazin-7(8H)-yl]pyridine-2-carboxylic acid

The title compound was prepared by substituting Example 1.72.5 for Example 1.1.16 in Example 1.1.17. MS (ESI) m/e 736 (M+H)⁺, 734 (M-H)⁻.

### 1.72.7 6-(1-(benzo[d]thiazol-2-ylcarbamoyl)-5,6-dihydroimidazo[1,5-a]pyrazin-7(8H)-yl)-3-(1-((3-(2-((2-(((4-((tert-butyldiphenylsilyl)oxy)-2-methylbutan-2-yl)oxy)sulfonyl)ethyl)amino)ethoxy)-5,7-dimethyladamantan-1-yl)methyl)-5-methyl-1H-pyrazol-4-yl)picolinic acid

The title compound was prepared by substituting Example 1.72.6 for Example 1.2.7 in Example 1.2.8.

### 1.72.8 6-[1-(1,3-benzothiazol-2-ylcarbamoyl)-5,6-dihydroimidazo[1,5-a]pyrazin-7(8H)-yl]-3-{1-[(3,5-dimethyl-7-{2-[(2-sulfoethyl)amino]ethoxy}tricyclo[3.3.1.1^{3,7}]dec-1-yl)methyl]-5-methyl-1H-pyrazol-4-yl}pyridine-2-carboxylic acid

The title compound was prepared by substituting Example 1.72.7 for Example 1.2.8 in Example 1.2.9. ¹H NMR (400 MHz, dimethyl sulfoxide-*d*₆) δ ppm 8.36 (bs, 2H), 8.03 (bs, 1H), 7.99 (d, 1H), 7.76 (d, 1H), 7.64 (d, 1H), 7.46 (t, 1H), 7.34 (s, 1H), 7.33 (t, 1H), 7.17 (d, 1H), 5.12 (s, 2H), 4.28 (t, 2H), 4.11 (t, 2H), 3.86 (s, 2H), 3.56 (t, 2H), 3.24 (m, 2H), 3.11 (m, 2H), 2.82 (t, 2H), 2.15 (s, 3H), 1.42 (s, 2H), 1.32 (q, 4H), 1.17 (q, 4, H), 1.03 (m, 2H), 0.88 (s, 6H). MS (ESI) m/e 844 (M+H)⁺, 842 (M-H)⁻.

### 1.73 Synthesis of 8-(1,3-benzothiazol-2-ylcarbamoyl)-2-{6-carboxy-5-[1-({3-[2-({3-[1-(beta-D-glucopyranuronosyl)-1H-1,2,3-triazol-4-yl]propyllamino)ethoxy]-5,7-dimethyltricyclo[3.3.1.1^{3,7}]dec-1-yl}methyl)-5-methyl-1H-pyrazol-4-yl]pyridin-2-yl}-1,2,3,4-tetrahydroisoquinoline (W2.73)

To a solution of (2R,3R,4S,5S,6S)-2-azido-6-(methoxycarbonyl)tetrahydro-2H-pyran-3,4,5-triyltriacetate (8.63 mg) in t-CH₃OH (2 mL) and water (1 mL) was added Example 1.67.1 (20 mg), copper(II) sulfate pentahydrate (2.0 mg) and sodium ascorbate (5 mg). The mixture was stirred for 20 minutes at 100 °C under microwave conditions (Biotage Initiator). LiOH H₂O (50 mg) was added to the mixture, and stirring was continued overnight. The mixture was neutralized with trifluoroacetic acid and purified by reverse-phase HPLC on a Gilson system (C18 column), eluting with 20-80% acetonitrile in water containing 0.1% trifluoroacetic acid, to give the title compound. MS (APCI) m/e 987.3 (M+H)⁺.

### 1.74 Synthesis of 6-[7-(1,3-benzothiazol-2-ylcarbamoyl)-1H-indol-2-yl]-3-{1-[(3,5-dimethyl-7-{2-[(2-sulfoethyl)amino]ethoxy}tricyclo[3.3.1.1^{3,7}]dec-1-yl)methyl]-5-methyl-1H-pyrazol-4-yl}pyridine-2-carboxylic acid (W2.74)

### 1.74.1 methyl 2-[5-{1-[(3-{2-[bis(tert-butoxycarbonyl)amino]ethoxy}-5,7-dimethyltricyclo[3.3.1.1^{3,7}]dec-1-yl)methyl]-5-methyl-1H-pyrazol-4-yl}-6-(tert-butoxycarbonyl)pyridin-2-yl]-1H-indole-7-carboxylate

Example 1.74.1 was prepared by substituting methyl 2-(4,4,5,5-tetratnethyl-1,3,2-dioxaborolan-2-yl)-1H-indole-7-carboxylate for Example 1.2.1 and substituting Example 1.68.4 for Example 1.1.6 in Example 1.1.12. MS (ESI) m/e 866.3 (M-H)⁻.

### 1.74.2 2-(6-(tert-butoxycarbonyl)-5-(1-((3-(2-((tert-butoxycarbonyl)amino)ethoxy)-5,7-dimethyladamantan-1-yl)methyl)-5-methyl-1H-pyrazol-4-yl)pyridin-2-yl)-1H-indole-7-carboxylic acid

Example 1.74.2 was prepared by substituting Example 1.74.1 for Example 1.1.12 in Example 1.1.13. MS (ESI) m/e 754.4 (M+H)⁺.

### 1.74.3 tert-butyl 6-(7-(benzo[d]thiazol-2-ylcarbamoyl)-1H-indol-2-yl)-3-(1-((3-(2-((tert-butoxycarbonyl)amino)ethoxy)-5,7-dimethyladamantan-1-yl)methyl)-5-methyl-1H-pyrazol-4-yl)picolinate

Example 1.74.3 was prepared by substituting Example 1.74.2 for Example 1.1.13 in Example 1.1.14. MS (ESI) m/e 886.5 (M+H)⁺.

### 1.74.4 3-(1-((3-(2-aminoethoxy)-5,7-dimethyladamantan-1-yl)methyl)-5-methyl-1H-pyrazol-4-yl)-6-(7-(benzo[d]thiazol-2-ylcarbamoyl)-1H-indol-2-yl)picolinic acid

Example 1.74.4 was prepared by substituting Example 1.74.3 for Example 1.1.16 in Example 1.1.17. MS (ESI) m/e 730.2 (M+H)⁺.

### 1.74.5 6-[7-(1,3-benzothiazol-2-ylcarbamoyl)-1H-indol-2-yl]-3-[1-({3,5-dimethyl-7-[(2,2,7,7-tetramethyl-10,10-dioxido-3,3-diphenyl-4,9-dioxa-1016-thia-13-aza-3-silapentadecan-15-yl)oxy]tricyclo[3.3.1.1^{3,7}]dec-1-yl}methyl)-5-methyl-1H-pyrazol-4-yl]pyridine-2-carboxylic acid

Example 1.74.5 was prepared by substituting Example 1.74.4 for Example 1.2.7 in Example 1.2.8. MS (ESI) m/e 1176.7 (M+H)⁺.

### 1.74.6 6-[7-(1,3-benzothiazol-2-ylcarbamoyl)-1H-indol-2-yl]-3-{1-[(3,5-dimethyl-7-{2-[(2-sulfoethyl)amino]ethoxy}tricyclo[3.3.1.1^{3,7}]dec-1-yl)methyl]-5-methyl-1H-pyrazol-4-yl}pyridine-2-carboxylic acid

Example 1.74.6 was prepared by substituting Example 1.74.5 for Example 1.2.8 in Example 1.2.9. ¹H NMR (400 MHz, dimethyl sulfoxide-*d₆*) δ ppm 11.32 (d, 1H), 8.23 (dd, 1H), 8.18 (d, 1H), 7.93 - 7.82 (m, 3H), 7.71 (d, 1H), 7.62 (s, 3H), 7.57 - 7.51 (m, 1H), 7.47 (s, 1H), 7.40 (d, 1H), 7.35 (t, 1H), 7.22 (t, 1H), 4.86 (t, 2H), 3.85 (s, 2H), 3.47 (t, 2H), 3.08 (t, 2H), 2.88 (p, 2H), 2.21 (s, 3H), 1.37 (s, 2H), 1.32 - 1.20 (m, 4H), 1.14 (q, 4H), 1.07 - 0.94 (m, 2H), 0.84 (s, 6H). MS (ESI) m/e 838.2 (M+H)⁺.

### 1.75 Synthesis of 6-[8-(1,3-benzothiazol-2-ylcarbamoyl)-6-[3-(methylamino)propyl]-3,4-dihydroisoquinolin-2(1H)-yl]-3-{1-[(3,5-dimethyl-7-{2-[(2-sulfoethyl)amino]ethoxy}tricyclo[3.3.1.1^{3,7}]dec-1-yl)methyl]-5-methyl-1H-pyrazol-4-yl}pyridine-2-carboxylic acid (W2.75)

### 1.75.1 methyl 3-bromo-5-(bromomethyl)benzoate

Azobisisobutyronitrile (1.79 g) was added to methyl 3-bromo-5-methylbenzoate (50 g) and N-bromosuccinimide (44.7 g) in 350 mL acetonitrile, and the mixture was refluxed overnight. An additional 11 g of N-bromosuccinimide and 0.5 g of azobisisobutyronitrile was added, and the refluxing was continued for 3 hours. The mixture was concentrated, taken up in 500 mL diethyl ether, and stirred for 30 minutes. The mixture was filtered, and the resulting solution was concentrated. The crude product was chromatographed on silica gel using 10% ethyl acetate in heptanes to give the title compound.

### 1.75.2 methyl 3-bromo-5-(cyanomethyl)benzoate

Tetrabutylammonium cyanide (50 g) was added to Example 1.75.1 (67.1 g) in 300 mL acetonitrile, and the mixture was heated to 70°C overnight. The mixture was cooled, poured into diethyl ether, and rinsed with water and brine. The mixture was then concentrated and chromatographed on silica gel using 2-20% ethyl acetate in heptanes to give the title compound.

### 1.75.3 methyl 3-(2-aminoethyl)-5-bromobenzoate

Borane-THF complex (126 mL, 1M solution) was added to a solution of Example 1.75.2 (16 g) in 200 mL tetrahydrofuran, and the mixture was stirred overnight. The reaction was carefully quenched with methanol (50 mL), and then concentrated to 50 mL volume. The mixture was taken up in 120 mL methanol / 120 mL 4M HCl / 120 mL dioxane, and stirred overnight. The organics were removed under reduced pressure, and the residue was extracted twice with diethyl ether. The extracts were discarded. The organic layer was basified with solid K₂CO₃, and then extracted with ethyl acetate, and dichloromethane (2x). The extracts were combined, dried over Na₂SO₄, filtered and concentrated to give the title compound.

### 1.75.4 methyl 3-bromo-5-(2-(2,2,2-trifluoroacetamido)ethyl)benzoate

Trifluoroacetic anhydride (9.52 mL) was added dropwise to a mixture of Example 1.75.3 (14.5 g) and trimethylamine (11.74 mL) in 200 mL dichloromethane at 0 °C. Upon addition the mixture was allowed to warm to room temperature and was stirred for three days. The mixture was poured into diethyl ether, and washed with NaHCO₃ solution and brine. The mixture was concentrated and chromatographed on silica gel using 5-30% ethyl acetate in heptanes to give the title compound.

### 1.75.5 methyl 6-bromo-2-(2,2,2-trifluoroacetyl)-1,2,3,4-tetrahydroisoquinoline-8-carboxylate

Sulfuric acid was added to Example 1.75.4 (10 g) until it went into solution (40 mL), at which time paraformaldehyde (4.24 g) was added and the mixture was stirred for 2 hours. The solution was then poured onto 400 mL ice, and stirred 10 minutes. The mixture was extracted with ethyl acetate (3x), and the combined extracts were washed with NaHCO₃ solution and brine, and then concentrated The crude product was chromatographed on silica gel using 2-15% ethyl acetate in heptanes to give the title compound.

### 1.75.6 methyl 6-(3-((tert-butoxycarbonyl)(methyl)amino)prop-1-yn-1-yl)-2-(2,2,2-trifluoroacetyl)-1,2,3,4-tetrahydroisoquinoline-8-carboxylate

A solution of Example 1.75.5 (5.1 g), tert-butyl methyl(prop-2-yn-1-yl)carbamate (2.71 g), bis(tirphenylphosphine)palladium(II) dichloride (PdCl₂(PPh₃)₂, 0.49 g), CuI (0.106 g), and triethylamine (5.82 mL) was stirred in 50 mL dioxane at 50 °C overnight. The mixture was concentrated and chromatographed on silica gel using 10-50% ethyl acetate in heptanes to give the title compound.

### 1.75.7 methyl 6-(3-((tert-butoxycarbonyl)(methyl)amino)propyl)-2-(2,2,2-trifluoroacetyl)-1,2,3,4-tetrahydroisoquinoline-8-carboxylate

Example 1.75.6 (4.2 g), tetrahydrofuran (20 mL) and methanol (20.00 mL) were added to wet 20% Pd(OH)₂/C (3 g) in a 250 mL pressure bottle and shaken under a pressure of 50 psi and 50 °C for 12 hours. The solution was filtered and concentrated to give the title compound.

### 1.75.8 methyl 2-(5-bromo-6-(tert-butoxycarbonyl)pyridin-2-yl)-6-(3-((tert-butoxycarbonyl)(methyl)amino)propyl)-1,2,3,4-tetrahydroisoquinoline-8-carboxylate

Example 1.75.7 (4.22 g), and potassium carbonate (1.53 g) were stirred in 60 mL tetrahydrofuran, 25 mL methanol, and 10 mL water overnight. The mixture was concentrated and 60 mL N,N-dimethylformamide was added. To this was then added Example 1.1.9 (3.05 g) and triethylamine (5 mL), and the reaction was stirred at 60 °C overnight. The mixture was cooled to room temperature, poured into ethyl acetate (600 mL), washed with water (3x) and brine, dried over Na₂SO₄, filtered, and concentrated. The residue was chromatographed on silica gel using 5-50% ethyl acetate in heptanes to give the title compound. MS (ESI) m/e 618.2 (M+H)⁺.

### 1.75.9 methyl 6-(3-((tert-butoxycarbonyl)(methyl)amino)propyl)-2-(6-(tert-butoxycarbonyl)-5-(4,4,5,5-tetramethyl-1,3,2-dioxaborolan-2-yl)pyridin-2-yl)-1,2,3,4-tetrahydroisoquinoline-8-carboxylate

To a solution of Example 1.75.8 (3.7 g), triethylamine (2.50 mL) and PdCl₂(dppf) (([1,1'-bis(diphenylphosphino)ferrocene]dichloropalladium(II) (1:1), 0.29 g) in 25 mL acetonitrile was added 4,4,5,5-tetramethyl-1,3,2-dioxaborolane (1.74 mL), and the reaction mixture was heated to 75 °C for 5 hours, then stirred at 60 °C overnight. The mixture was concentrated and chromatographed on silica gel using 5-50% ethyl acetate in heptanes to give the title compound. MS (ESI) m/e 666.4 (M+H)⁺.

### 1.75.10 4-((tert-butyldiphenylsilyl)oxy)-2,2-dimethylbutyl 2-((2-((3-((4-iodo-5-methyl-1H-pyrazol-1-yl)methyl)-5,7-dimethyladamantan-1-yl)oxy)ethyl)amino)ethanesulfonate

Example 1.55.10 (2.39 g), 4-((tert-butyldiphenylsilyl)oxy)-2,2-dimethylbutyl ethenesulfonate (2.41 g), and triethylamine (1.51 mL) were stirred in 30 mL N,N-dimethylformamide at 45 °C for 3 hours. The mixture was cooled and poured into diethyl ether (400 mL), and the diethyl ether solution was washed with water (3x) and brine, and concentrated. The crude product was chromatographed on silica gel using 2-50% ethyl acetate in heptanes, with 1% added triethylamine to give the title compound. MS (ESI) m/e 890.6 (M+H)⁺.

### 1.75.11 6-(6-(3-((tert-butoxycarbonyl)(methyl)amino)propyl)-8-(methoxycarbonyl)-3,4-dihydroisoquinolin-2(1H)-yl)-3-(1-((3-(2-((2-((4-((tert-butyldiphenylsilyl)oxy)-2,2-dimethylbutoxy)sulfonyl)ethyl)amino)ethoxy)-5,7-dimethyladamantan-1-yl)methyl)-5-methyl-1H-pyrazol-4-yl)picolinic acid

Example 1.75.9 (1.777 g), Example 1.75.10 (1.98 g), tris(dibenzylideneacetone)dipalladium(0) (0.102 g), 1,3,5,7-tetramethyl-8-tetradecyl-2,4,6-trioxa-8-phosphaadamantane (0.918 g), and potassium phosphate (1.889 g) were added to 25 mL dioxane / 10 mL water, and the solution was evacuated/filled with nitrogen several times. The reaction was clear, and was stirred at 70 °C overnight. The mixture was cooled and poured into ethyl acetate (200 mL), and washed with water and brine. The mixture was concentrated and chromatographed on silica gel using 5-50% ethyl acetate in heptanes, followed by 10% methanol in ethyl acetate with 1% triethylamine to give the title compound. MS (ESI) m/e 1301.4 (M+H)⁺.

### 1.75.12 6-(3-((tert-butoxycarbonyl)(methyl)amino)propyl)-2-(5-(1-((3-(2-((2-((4-((tert-butyldiphenylsilyl)oxy)-2,2-dimethylbutoxy)sulfonyl)ethyl)amino)ethoxy)-5,7-dimethyladamantan-1-yl)methyl)-5-methyl-1H-pyrazol-4-yl)-6-carboxypyridin-2-yl)-1,2,3,4-tetrahydroisoquinoline-8-carboxylic acid

Example 1.75.11 (1.5 g) and LiOH-H₂O (0.096 g) were stirred in 15 mL tetrahydrofuran and 3 mL water at 45 °C for 10 days. The mixture was poured into 200 mL ethyl acetate / 20 mL NaH₂PO₄ solution, and concentrated HCl solution was added until the pH reached 3. The layers were separated, and the aqueous layer was extracted twice with ethyl acetate. The combined organic layers were washed with brine and concentrated. The residue was chromatographed on silica gel using 0-5% methanol in ethyl acetate to give the title compound. MS (ESI) m/e 1287.3 (M+H)⁺.

### 1.75.13 6-(8-(benzo[d]thiazol-2-ylcarbamoyl)-6-(3-((tert-butoxycarbonyl)(methyl)amino)propyl)-3,4-dihydroisoquinolin-2(1H)-yl)-3-(1-((3-(2-((2-((4-((tert-butyldiphenylsilyl)oxy)-2,2-dimethylbutoxy)sulfonyl)ethyl)amino)ethoxy)-5,7-dimethyladamantan-1-yl)methyl)-5-methyl-1H-pyrazol-4-yl)picolinic acid

The title compound was prepared as described in Example 1.2.6, substituting Example 1.2.5 with Example 1.75.12. MS (ESI) m/e 1419.5 (M+H)⁺.

### 1.75.14 6-[8-(1,3-benzothiazol-2-ylcarbamoyl)-6-[3-(methylamino)propyl]-3,4-dihydroisoquinolin-2(1H)-yl]-3-{1-[(3,5-dimethyl-7-{2-[(2-sulfoethyl)amino]ethoxy}tricyclo[3.3.1.1^{3,7}]dec-1-yl)methyl]-5-methyl-1H-pyrazol-4-yl}pyridine-2-carboxylic acid

The title compound was prepared as described in Example 1.2.9, substituting Example 1.2.8 with Example 1.75.13. ¹H NMR (400 MHz, dimethyl sulfoxide-*d₆*) δ ppm 12.90 (bs, 1H), 8.33 (m, 2H), 8.02 (d, 1H), 7.78 (d, 1H), 7.66 (m, 1H), 7.47 (m, 3H), 7.35 (m, 3H), 7.25 (s, 2H), 6.95 (d, 1H), 4.95 (s, 2H), 4.28 (t, 2H), 4.11 (t, 2H), 3.95 (m, 2H), 3.20 (m, 2H), 3.08 (m, 2H), 2.96 (m, 2H), 2.89 (m, 2H), 2.78 (m, 2H), 2.65 (m, 2H), 2.55 (t, 2H), 2.12 (s, 3H), 1.95 (m, 2H), 1.39 (s, 2H), 1.25 (m, 6H), 1.12 (m, 6H), 0.93 (s, 3H), 0.85 (s, 6H). MS (ESI) m/e 926.8 (M+H)⁺.

### 1.76 Synthesis of 5-{[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl]amino}-5-deoxy-D-arabinitol (W2.76)

### 1.76.1 tert-butyl 6-(8-(benzo[d]thiazol-2-ylcarbamoyl)-3,4-dihydroisoquinolin-2(1H)-yl)-3-(1-((3,5-dimethyl-7-(2-((((4R,4'R,5R)-2,2,2',2'-tetramethyl-[4,4'-bi(1,3-dioxolan)]-5-yl)methyl)amino)ethoxy)adamantan-1-yl)methyl)-5-methyl-1H-pyrazol-4-yl)picolinate

Example 1.2.7 (75 mg) and (4R,4'R,5S)-2,2,2',2'-tetramethyl-[4,4'-bi(1,3-dioxolane)]-5-carbaldehyde (22 mg) were dissolved in dichloromethane (1 mL). Sodium triacetoxyborohydride (40 mg) was added, and the solution was stirred for 16 hours at room temperature. The solution was concentrated under reduced pressure, and the material was purified by flash column chromatography on silica gel, eluting with 5-10% methanol in dichloromethane. The solvent was evaporated under reduced pressure to provide the title compound. MS (ESI) m/e 1016 (M+H)⁺, 1014 (M-H)⁻.

### 1.76.2 5-{[2-({3-[(4-{6-[8-(1,3-benzothiazol-2-ylcarbamoyl)-3,4-dihydroisoquinolin-2(1H)-yl]-2-carboxypyridin-3-yl]-5-methyl-1H-pyrazol-1-yl)methyl]-5,7-dimethyltricyclo[3.3.1.1^{3,7}]dec-1-yl}oxy)ethyl]amino}-5-deoxy-D-arabinitol

Example 1.76.1 (45 mg) was dissolved in trifluoroacetic acid (1 mL) and water (0.2 mL). The solution was mixed at room temperature for five days. The solvents were removed under reduced pressure, and the material was taken up in methanol (2 mL). The material was purified by reverse-phase HPLC using 25-75% acetonitrile in water (w/0.1% TFA) over 30 minutes on a Grace Reveleris equipped with a Luna column: C18(2), 100 A, 250 x 30 mm. Product fractions were pooled, frozen, and lyophilized to yield the title compound as the bis trifluoroacetic acid salt. ¹H NMR (400 MHz, dimethyl sulfoxide-*d₆*) δ ppm 12.85 (bs, 2H), 8.31 (m, 1H), 8.16 (m, 1H), 8.04 (d, 1H), 7.80 (d, 1H), 7.62 (d, 1H), 7.51-7.43 (m, 3H), 7.37 (q, 2H), 7.29 (s, 1H), 6.69 (d, 1H), 4.96 (s, 2H), 4.04 (t, 2H), 3.89 (m, 2H), 3.59 (m, 3H), 3.49 (m, 4H), 3.42 (dd, 2H), 3.22 (dd, 2H), 3.06 (m, 2H), 3.02 (m, 4H), 2.10 (s, 3H), 1.43 (s, 2H), 1.30 (q, 4H), 1.14 (t, 4H), 1.04 (q, 2H), 0.87 (s, 6H). MS (ESI) m/e 880 (M+H)⁺, 878 (M-H)⁻.

### 1.77 Synthesis of 1-{[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl]amino}-1,2-dideoxy-D-arabino-hexitol (W2.77)

### 1.77.1 tert-butyl 6-(8-(benzo[d]thiazol-2-ylcarbamoyl)-3,4-dihydroisoquinolin-2(1H)-yl)-3-(1-((3,5-dimethyl-7-(2-(((3R,4S,5R)-3,4,5,6-tetrahydroxyhexyl)amino)ethoxy)adamantan-1-yl)methyl)-5-methyl-1H-pyrazol-4-yl)picolinate

(4R,5S,6R)-6-(Hydroxymethyl)tetrahydro-2H-pyran-2,4,5-triol (15 mg) was dissolved in dimethyl sulfoxide (0.5 mL). Example 1.2.7 (88 mg) was added, followed by sodium cyanoborohydride (27 mg). Acetic acid (82 mg) was added dropwise, and the solution was heated at 60 °C for 16 hours. The reaction was cooled, diluted with 1 mL of methanol, and purified by reverse-phase HPLC using 20-75% acetonitrile in water (w/0.1% TFA) over 60 minutes on a Grace Reveleris equipped with a Luna column: C18(2), 100 A, 150 x 30 mm. Product fractions were pooled, frozen, and lyophilized to yield the title compound as the bis trifluoroacetic acid salt. MS (ESI) m/e 950 (M+H)⁺, 948 (M-H)⁻.

### 1.77.2 1-{[2-({3-[(4-{6-[8-(1,3-benzothiazol-2-ylcarbamoyl)-3,4-dihydroisoquinolin-2(1H)-yl]-2-carboxypyridin-3-yl]-5-methyl-1H-pyrazol-1-yl)methyl]-5,7-dimethyltricyclo[3.3.1.1^{3,7}]dec-1-yl}oxy)ethyl]amino}-1,2-dideoxy-D-arabino-hexitol

Example 1.77.1 (39 mg) was dissolved in dichloromethane (0.5 mL). Trifluoroacetic acid (740 mg) was added, and the solution was stirred at room temperature for 16 hours. The solvents were removed under reduced pressure. The residue was dissolved in N,N-dimethylformamide (0.5 mL) and 1 M aqueous sodium hydroxide (0.5 mL) was added. The solution was stirred at room temperature for one hour. Trifluoroacetic acid (0.25 mL) was added, and the material was purified by reverse-phase HPLC using 20-75% acetonitrile in water (w/0.1% TFA) over 60 minutes on a Grace Reveleris equipped with a Luna column: C18(2), 100 A, 150 x 30 mm. Product fractions were pooled, frozen, and lyophilized to yield the title compound as the bis trifluoroacetic acid salt. ¹H NMR (400 MHz, dimethyl sulfoxide-*d₆*) δ ppm 12.86 (s, 1H), 12.74 (bs, 1H), 8.28 (bs, 1H), 8.20 (bs, 1H), 8.04 (d, 1H), 7.80 (d, 1H), 7.62 (d, 1H), 7.51-7.43 (m, 3H), 7.37 (q, 2H), 7.29 (s, 1H), 6.96 (d, 1H), 4.96 (s, 2H), 4.53 (bs, 3H), 3.89 (t, 2H), 3.83 (s, 2H), 3.77 (d, 1H), 3.60 (dd, 2H), 3.56 (t, 2H), 3.48 (m, 2H), 3.15 (d, 1H), 3.02 (m, 6H), 2.10 (s, 3H), 1.84 (m, 1H), 1.69 (m, 1H), 1.43 (s, 2H), 1.31 (q, 4H), 1.14 (t, 4H), 1.05 (q, 2H), 0.87 (s, 6H). MS (ESI) m/e 894 (M+H)⁺, 892 (M-H)⁻.

### 1.78 Synthesis of 6-[4-(1,3-benzothiazol-2-ylcarbamoyl)isoquinolin-6-yl]-3-{1-[(3,5-dimethyl-7-{2-[(2-sulfoethyl)amino]ethoxy}tricyclo[3.3.1.1^{3,7}]dec-1-yl)methyl]-5-methyl-1H-pyrazol-4-yl}pyridine-2-carboxylic acid (W2.78)

### 1.78.1 methyl 6-(4,4,5,5-tetramethyl-1,3,2-dioxaborolan-2-yl)isoquinoline-4-carboxylate

To a solution of methyl 6-bromoisoquinoline-4-carboxylate (1.33 g) in N,N-dimethylformamide (30 mL) was added PdCl₂(dppf)-CH₂Cl₂ adduct (([1,1'-bis(diphenylphosphino)ferrocene]dichloropalladium(II) (1:1), 204 mg), potassium acetate (1.48 g) and bis(pinacolato)diboron (1.92 g). The mixture was stirred at 60 °c overnight. The mixture was cooled to room temperature and used in the next reaction without further work up. MS (APCI) m/e 313.3 (M+H)⁺.

### 1.78.2 methyl 6-[5-{1-[(3-{2-[bis(tert-butoxycarbonyl)amino]ethoxy}-5,7-dimethyltricyclo[3.3.1.1^{3,7}]dec-1-yl)methyl]-5-methyl-1H-pyrazol-4-yl}-6-(tert-butoxycarbonyl)pyridin-2-yl]isoquinoline-4-carboxylate

To a solution of the Example 1.68.4 (1.2 g) in 1,4-dioxane (20 mL) and water (10 mL) was added Example 1.78.1 (517 mg), bis(triphenylphosphine)palladium(II) dichloride (58 mg), and CsF (752 mg). The mixture was stirred at reflux overnight. LC/MS showed the expected product as a major peak. The mixture was diluted with ethyl acetate (200 mL), washed with water and brine, dried over anhydrous sodium sulfate, filtered and concentrated. The residue was purified by silica gel chromatography, eluting with 20% ethyl acetate in dichloromethane to give the title compound. MS (ESI) m/e 880.8 (M+H)⁺.

### 1.78.3 6-(6-(tert-butoxycarbonyl)-5-(1-((3-(2-((tert-butoxycarbonyl)amino)ethoxy)-5,7-dimethyladamantan-1-yl)methyl)-5-methyl-1H-pyrazol-4-yl)pyridin-2-yl)isoquinoline-4-carboxylic acid

To a solution of Example 1.78.2 (3.1 g) in tetrahydrofuran (20 mL), methanol (10 mL) and water(10 mL) was added LiOH H₂O (240 mg). The mixture was stirred at room temperature overnight. The mixture was acidified with aqueous 2N HCl and diluted with ethyl acetate (400 mL). The organic layer was washed with water and brine and dried over anhydrous sodium sulfate. Filtration and evaporation of the solvent gave the title compound. MS (ESI) m/e 766.4 (M+H)⁺.

### 1.78.4 3-(1-((3-(2-aminoethoxy)-5,7-dimethyladamantan-1-yl)methyl)-5-methyl-1H-pyrazol-4-yl)-6-(4-(benzo[d]thiazol-2-ylcarbamoyl)isoquinolin-6-yl)picolinic acid

To a solution of Example 1.78.3 (1.2 g) in dichloromethane (20 mL) was added benzo[d]thiazol-2-amine (0.236 g), 1-ethyl-3-(3-dimethylaminopropyl)carbodiimide (451 mg), and 4-dimethylaminopyridine (288 mg), and the mixture was stirred at room temperature overnight. The reaction mixture was diluted with ethyl acetate (500 mL), washed with water and brine, and dried over anhydrous sodium sulfate. Filtration and evaporation of the solvent gave a residue that was dissolved in dichloromethane and trifluoroacetic acid (10 mL, 1:1) and stirred overnight. The mixture was concentrated, and the residue was dissolved in N,N-dimethylformamide (4 mL) and purified by reverse-phase HPLC on a Gilson system (C18 column), eluting with 20-80% acetonitrile in water containing 0.1% trifluoroacetic acid, to give the title compound. MS (ESI) m/e 742.1 (M+H)⁺.

### 1.78.5 6-[4-(1,3-benzothiazol-2-ylcarbamoyl)isoquinolin-6-yl]-3-{1-[(3,5-dimethyl-7-{2-[(2-sulfoethyl)amino]ethoxy}tricyclo[3.3.1.1^{3,7}]dec-1-yl)methyl]-5-methyl-1H-pyrazol-4-yl}pyridine-2-carboxylic acid

To a solution of Example 1.78.4 (55 mg) in N,N-dimethylformamide (6 mL) was added 4-((tert-butyldiphenylsilyl)oxy)-2,2-dimethylbutyl ethenesulfonate (34 mg), *N,N*-diisopropylethylamine (0.6 mL) and H₂O (0.6 mL). The mixture was stirred at room temperature overnight. The reaction mixture was diluted with dichloromethane and trifluoroacetic acid (10 mL, 1:1) and stirred overnight. The mixture was concentrated, and the residue was dissolved in N,N-dimethylformamide (4 mL) and purified by reverse-phase HPLC on a Gilson system (C18 column), eluting with 20-80% acetonitrile in water containing 0.1% trifluoroacetic acid, to give the title compound. ¹H NMR (400 MHz, dimethyl sulfoxide-*d*₆) δ ppm 13.25 (s, 2H), 9.58 (s, 1H), 9.06 (s, 1H), 9.00 (s, 1H), 8.52 (dd, 1H), 8.42 (d, 1H), 8.35 (d, 2H), 8.26 (d, 1H), 8.11 - 8.03 (m, 1H), 8.01 (d, 1H), 7.80 (d, 1H), 7.52 - 7.44 (m, 2H), 7.41 - 7.28 (m, 1H), 3.89 (s, 2H), 3.55 (t, 2H), 3.22 (t, 2H), 3.09 (s, 2H), 2.80 (t, 2H), 2.23 (s, 3H), 1.43 (s, 2H), 1.30 (q, 4H), 1.23 - 1.11 (m, 4H), 1.04 (q, 2H), 0.86 (s, 6H). MS (ESI+) m/e 850.1 (M+H)⁺.

### 1.79 Synthesis of 6-[8-(1,3-benzothiazol-2-ylcarbamoyl)-3,4-dihydroisoquinolin-2(1H)-yl]-3-(1-{[3-(2-{[3-hydroxy-2-(hydroxymethyl)propyl]amino}ethoxy)-5,7-dimethyltricyclo[3.3.1.1^{3,7}]dec-1-yl]methyl}-5-methyl-1H-pyrazol-4-yl)pyridine-2-carboxylic acid (W2.79)

### 1.79.1 2,2-dimethyl-1,3-dioxane-5-carbaldehyde

To a stirred suspension of pyridinium chlorochromate (1.1 g) and diatomaceous earth (10 g) in dichloromethane (10 mL) was added (2,2-dimethyl-1,3-dioxan-5-yl)methanol (0.5 g) as a solution in dichloromethane (3 mL) dropwise. The mixture was stirred at room temperature for 2 hours. The suspension was filtered through diatomaceous earth and washed with ethyl acetate. The crude product was filtered through silica gel and concentrated to give the title compound. ¹H NMR (501 MHz, chloroform-*d*) δ 9.89 (s, 1H), 4.28 - 4.17 (m, 4H), 2.42 - 2.32 (m, 1H), 1.49 (s, 3H), 1.39 (s, 3H). MS (ESI) m/e 305.9 (2M+NH4)⁺.

### 1.79.2 tert-butyl 6-(8-(benzo[d]thiazol-2-ylcarbamoyl)-3,4-dihydroisoquinolin-2(1H)-yl)-3-(1-((3-(2-(((2,2-dimethyl-1,3-dioxan-5-yl)methyl)amino)ethoxy)-5,7-dimethyladamantan-1-yl)methyl)-5-methyl-1H-pyrazol-4-yl)picolinate

To a solution of Example 1.2.7 (100 mg) and Example 1.79.1 (20 mg) in dichloromethane (1 mL) was added sodium triacetoxyborohydride (40 mg), and the mixture was stirred at room temperature for 2 hours. The reaction was diluted with dichloromethane and washed with saturated sodium bicarbonate solution. The aqueous layer was back extracted with dichloromethane. The combined organic layers were dried over sodium sulfate, filtered and concentrated. Purification of the residue by silica gel chromatography, eluting with 20%-100% ethyl acetate/ethanol (3:1) in heptane, provided the title compound. MS (ESI) m/e 930.3 (M+H)⁺.

### 1.79.3 6-[8-(1,3-benzothiazol-2-ylcarbamoyl)-3,4-dihydroisoquinolin-2(1H)-yl]-3-(1-{[3-(2-{[3-hydroxy-2-(hydroxymethyl)propyl]amino}ethoxy)-5,7-dimethyltricyclo[3.3.1.1^{3,7}]dec-1-yl]methyl}-5-methyl-1H-pyrazol-4-yl)pyridine-2-carboxylic acid

Example 1.79.3 was prepared by substituting Example 1.79.2 for Example 1.2.8 in Example 1.2.9. ¹H NMR (400 MHz, dimethyl sulfoxide-*d₆*) δ ppm 12.82 (s, 1H), 8.13 (s, 2H), 8.00 (dd, 1H), 7.76 (d, 1H), 7.59 (d, 1H), 7.49 - 7.38 (m, 3H), 7.37 - 7.29 (m, 2H), 7.25 (s, 1H), 6.92 (d, 1H), 4.92 (s, 4H), 3.85 (t, 2H), 3.79 (s, 2H), 3.53 (t, 2H), 3.47 (dd, 2H), 3.00 (dt, 7H), 2.07 (s, 3H), 1.93 (p, 1H), 1.38 (s, 2H), 1.32 - 1.19 (m, 4H), 1.16 - 0.91 (m, 6H), 0.83 (s, 7H). MS (ESI) m/e 834.3 (M+H)⁺.

### 1.80 Synthesis of 1-{[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl]amino}-1,2-dideoxy-D-erythro-pentitol (W2.80)

The title compound was prepared by substituting (4S,5R)-tetrahydro-2H-pyran-2,4,5-triol for (4R,5S,6R)-6-(hydroxymethyl)tetrahydro-2H-pyran-2,4,5-triol and Example 1.3.1 for Example 1.2.7 in Example 1.77.1. ¹H NMR (400 MHz, dimethyl sulfoxide-*d₆*) □ ppm 12.85 (bs, 1H), 12.72 (bs, 1H), 8.21 (bs, 2H), 8.04 (d, 1H), 7.79 (d, 1H), 7.62 (d, 1H), 7.52-7.42 (m, 3H), 7.37 (q, 2H), 7.29 (s, 1H), 6.95 (d, 1H), 4.96 (s, 2H), 3.89 (t, 2H), 3.83 (s, 2H), 3.65 (m, 2H), 3.56 (m, 2H), 3.38 (m, 2H), 3.32 (m, 2H), 3.24 (m, 2H), 3.03 (m, 5H), 2.10 (s, 3H), 1.89 (m, 1H), 1.67 (m, 1H), 1.44 (s, 2H), 1.31 (q, 4H), 1.14 (t, 4H), 1.05 (q, 2H), 0.86 (s, 6H). MS (ESI) m/e 864 (M+H)⁺, 862 (M-H)⁻.

### 1.81 Synthesis of 6-[8-(1,3-benzothiazol-2-ylcarbamoyl)-3,4-dihydroisoquinolin-2(1H)-yl]-3-(1-{[3,5-dimethyl-7-(2-{[(2S,3S)-2,3,4-trihydroxybutyl]amino}ethoxy)tricyclo[3.3.1.1^{3,7}]dec-1-yl]methyl}-5-methyl-1H-pyrazol-4-yl)pyridine-2-carboxylic acid (W2.81)

### 1.81.1 carbonic acid tert-butyl ester (4S,5S)-5-hydroxymethyl-2,2-dimethyl-[1,3]dioxolan-4-ylmethyl ester

((4S,5S)-2,2-Dimethyl-1,3-dioxolane-4,5-diyl)dimethanol (1000 mg) was dissolved in N,N-dimethylformamide (50 mL). Sodium hydride (60% in mineral oil, 259 mg) was added. The solution was mixed at room temperature for 15 minutes. Di-tert-butyl dicarbonate (1413 mg) was added slowly. The solution was mixed for 30 minutes, and the reaction was quenched with saturated aqueous ammonium chloride solution. The solution was diluted with water (150 mL) and extracted twice using 70% ethyl acetate in heptanes. The organic portions were combined and extracted with water (100 mL), extracted with brine (50 mL), and dried on anhydrous sodium sulfate. The solution was concentrated under reduced pressure, and the material was purified by flash column chromatography on silica gel, eluting with 30% ethyl acetate in heptanes. The solvent was evaporated under reduced pressure to provide the title compound. MS (ESI) m/e 284 (M+Na)⁺.

### 1.81.2 carbonic acid tert-butyl ester (4S,5R)-5-formyl-2,2-dimethyl-[1,3]dioxolan-4-ylmethyl ester

Example 1.81.1 (528 mg) was dissolved in dichloromethane (20 mL). Dess-Martin periodinane (896 mg) was added, and the solution was stirred at room temperature for four hours. The solution was concentrated under reduced pressure, and the material was purified by flash column chromatography on silica gel, eluting with 20%-50% ethyl acetate in heptanes. The solvent was evaporated under reduced pressure to provide the title compound.

### 1.81.3 tert-butyl 6-(8-(benzo[d]thiazol-2-ylcarbamoyl)-3,4-dihydroisoquinolin-2(1H)-yl)-3-(1-(((1S,3s,5R,7S)-3-(2-((((4S,5S)-5-(((tert-butoxycarbonyl)oxy)methyl)-2,2-dimethyl-l,3-dioxolan-4-yl)methyl)amino)ethoxy)-5,7-dimethyladamantan-1-yl)methyl)-5-methyl-1H-pyrazol-4-yl)picolinate

The title compound was prepared by substituting Example 1.81.2 for (4R,4'R,5S)-2,2,2',2'-tetramethyl-[4,4'-bi(1,3-dioxolane)]-5-carbaldehyde in Example 1.76.1.

### 1.81.4 6-[8-(1,3-benzothiazol-2-ylcarbamoyl)-3,4-dihydroisoquinolin-2(1H)-yl]-3-(1-{[3,5-dimethyl-7-(2-{[(2S,3S)-2,3,4-trihydroxybutyl]amino}ethoxy)tricyclo[3.3.1.1^{3,7}]dec-1-yl]methyl}-5-methyl-1H-pyrazol-4-yl)pyridine-2-carboxylic acid

The title compound was prepared by substituting Example 1.81.3 for Example 1.76.1 in Example 1.76.2. ¹H NMR (400 MHz, dimethyl sulfoxide-*d₆*) □ ppm 12.86 (bs, 2H), 8.28 (bs, 1H), 8.18 (bs, 1H), 8.04 (d, 1H), 7.80 (d, 1H), 7.63 (d, 1H), 7.51-7.43 (m, 3H), 7.36 (q, 2H), 7.29 (s, 1H), 6.96 (d, 1H), 4.96 (s, 2H), 3.89 (t, 2H), 3.83 (m, 3H), 3.46 (m, 4H), 3.40 (m, 4H), 3.08-2.96 (m, 6H), 2.10 (s, 3H), 1.43 (s, 2H), 1.30 (q, 4H), 1.14 (t, 4H), 1.04 (q, 2H), 0.87 (s, 6H). MS (ESI) m/e 850 (M+H)⁺, 848 (M-H)⁻.

### 1.82 Synthesis of 6-[8-(1,3-benzothiazol-2-ylcarbamoyl)-3,4-dihydroisoquinolin-2(1H)-yl]-3-(1-{[3-(2-{[(2S,3S,4R,5R,6R)-2,3,4,5,6,7-hexahydroxyheptyl]amino}ethoxy)-5,7-dimethyltricyclo[3.3.1.1^{3,7}]dec-1-yl]methyl}-5-methyl-1H-pyrazol-4-yl)pyridine-2-carboxylic acid (W2.82)

The title compound was prepared by substituting (2R,3R,4S,5R,6R)-2,3,4,5,6,7-hexahydroxyheptanal for (4R,5S,6R)-6-(hydroxymethyl)tetrahydro-2H-pyran-2,4,5-triol and Example 1.3.1 for Example 1.2.7 in Example 1.77.1. ¹H NMR (400 MHz, dimethyl sulfoxide-*d₆*) □ ppm 12.86 (bs, 1H), 8.34-8.08 (m, 2H), 8.05 (d, 1H), 7.79 (d, 1H), 7.54-7.43 (m, 3H), 7.37 (m, 2H), 7.30 (s, 1H), 6.95 (d, 1H), 4.96 (s, 2H), 3.93 (m, 2H), 3.90 (m, 4H), 3.83 (s, 2H), 3.47 (m, 4H), 3.41 (m, 4H), 3.18-3.08 (m, 7H), 3.03 (t, 2H), 2.12 (s, 3H), 1.46 (s, 2H), 1.28 (q, 4H), 1.15 (t, 4H), 1.05 (q, 2H), 0.89 (s, 6H). MS (ESI) m/e 940 (M+H)⁺.

### 1.83 Synthesis of 6-[8-(1,3-benzothiazol-2-ylcarbamoyl)-3,4-dihydroisoquinolin-2(1H)-yl]-3-{1-[(3-{2-[({3-[(1,3-dihydroxypropan-2-yl)amino]propyl}sulfonyl)amino]ethoxy}-5,7-dimethyltricyclo[3.3.1.1^{3,7}]dec-1-yl)methyl]-5-methyl-1H-pyrazol-4-yl}pyridine-2-carboxylic acid (W2.83)

### 1.83.1 tert-butyl 6-(8-(benzo[d]thiazol-2-ylcarbamoyl)-3,4-dihydroisoquinolin-2(1H)-yl)-3-(1-((3-(2-(3-((1,3-dihydroxypropan-2-yl)amino)propylsulfonamido)ethoxy)-5,7-dimethyladamantan-1-yl)methyl)-5-methyl-1H-pyrazol-4-yl)picolinate

To a cooled (ice bath) solution of Example 1.2.7 (31 mg) and N,N-diisopropylethylamine (60 µL) in dichloromethane (1 mL) was added 3-chloropropane-1-sulfonyl chloride (5 µL). The mixture was stirred at room temperature for 2 hours. The reaction was concentrated, dissolved in N,N-dimethylformamide (1 mL), transferred to a 2 mL microwave tube and 2-aminopropane-1,3-diol (70 mg) was added. The mixture was heated at 130 °C under microwave conditions (Biotage Initiator) for 90 minutes. The reaction mixture was concentrated, and the residue was purified by reverse-phase HPLC using a Gilson system, eluting with 20-100% acetonitrile in water containing 0.1% v/v trifluoroacetic acid. The desired fractions were combined and freeze-dried to provide the title compound. MS (ESI) m/e 997.2 (M+H)⁺.

### 1.83.2 6-[8-(1,3-benzothiazol-2-ylcarbamoyl)-3,4-dihydroisoquinolin-2(1H)-yl]-3-{1-[(3-{2-[({3-[(1,3-dihydroxypropan-2-yl)amino]propyl}sulfonyl)amino]ethoxy}-5,7-dimethyltricyclo[3.3.1.1^{3,7}]dec-1-yl)methyl]-5-methyl-1H-pyrazol-4-yl}pyridine-2-carboxylic acid

Example 1.83.2 was prepared by substituting Example 1.83.1 for Example 1.2.8 in Example 1.2.9. ¹H NMR (400 MHz, dimethyl sulfoxide-*d₆*) δ ppm 12.84 (s, 1H), 8.40 (s, 2H), 8.05-7.98 (m, 1H), 7.77 (d, 1H), 7.60 (d, 1H), 7.51 - 7.39 (m, 3H), 7.38 - 7.30 (m, 2H), 7.27 (s, 1H), 7.13 (t, 1H), 6.93 (d, 1H), 4.94 (s, 2H), 3.61 (qd, 4H), 3.36 (t, 2H), 3.16 - 2.93 (m, 10H), 2.08 (s, 3H), 2.00 (p, 2H), 1.38 (s, 2H), 1.25 (q, 4H), 1.15 - 0.92 (m, 6H), 0.84 (s, 6H). MS (ESI) m/e 941.2 (M+H)⁺.

### 1.84 Synthesis of 6-[8-(1,3-benzothiazol-2-ylcarbamoyl)-3,4-dihydroisoquinolin-2(1H)-yl]-3-{1-[(3-{2-[(3-{[1,3-dihydroxy-2-(hydroxymethyl)propan-2-yl]amino}-3-oxopropyl)amino]ethoxy}-5,7-dimethyltricyclo[3.3.1.1^{3,7}]dec-1-yl)methyl]-5-methyl-1H-pyrazol-4-yl}pyridine-2-carboxylic acid (W2.84)

To a solution oftert-butyl 3-(1-((3-(2-aminoethoxy)-5,7-dimethyladamantan-1-yl)methyl)-5-methyl-1H-pyrazol-4-yl)-6-(8-(benzo[d]thiazol-2-ylcarbamoyl)-3,4-dihydroisoquinolin-2(1H)-yl)picolinate (55 mg) in N,N-dimethylformamide (6 mL) was added N-(1,3-dihydroxy-2-(hydroxymethyl)propan-2-yl)acrylamide (73.4 mg), *N,N*-diisopropylethylamine (0.2 mL) and H₂O (0.2 mL). The mixture was stirred at room temperature 4 days. LC/MS showed the expected product as a major peak. The reaction mixture was diluted with ethyl acetate (500 mL), washed with water and brine, and dried over anhydrous sodium sulfate. Filtration and evaporation of the solvent gave a residue that was dissolved in dichloromethane and trifluoroacetic acid (10 mL, 1:1) and stirred overnight. The mixture was concentrated, and the residue was dissolved in N,N-dimethylformamide (8 mL) and purified by reverse-phase HPLC on a Gilson system (C18 column), eluting with 20-80% acetonitrile in water containing 0.1% trifluoroacetic acid, to give the title compound. ¹H NMR (400 MHz, dimethylsulfonxide-*d*₆) δ ppm 12.84 (s, 1H), 8.45 (s, 2H), 8.01 (d, 4H), 7.78 (d, 1H), 7.60 (d, 1H), 7.53 - 7.39 (m, 3H), 7.39 - 7.30 (m, 2H), 7.27 (s, 1H), 6.94 (d, 1H), 4.94 (s, 2H), 4.14 (s, 2H), 3.87 (t, 2H), 3.81 (s, 2H), 3.52 (d, 4H), 3.19 (s, 3H), 3.13 - 2.97 (m, 5H), 2.75 (t, 2H), 2.08 (s, 3H), 1.42 (s, 2H), 1.29 (q, 4H), 1.12 (s, 4H), 1.09 - 0.99 (m, 2H), 0.85 (s, 7H). MS (ESI) m/e 921.2 (M+H)⁺.

### 1.85 Synthesis of 6-[8-(1,3-benzothiazol-2-ylcarbamoyl)-3,4-dihydroisoquinolin-2(1H)-yl]-3-(1-{[3-(2-{[(3S)-3,4-dihydroxybutyl]amino}ethoxy)-5,7-dimethyltricyclo[3.3.1.1^{3,7}]dec-1-yl]methyl}-5-methyl-1H-pyrazol-4-yl)pyridine-2-carboxylic acid (W2.85)

To a solution of Example 1.2.7 (213 mg) in dichloromethane (2 mL) was added (S)-2-(2,2-dimethyl-1,3-dioxolan-4-yl)acetaldehyde (42 mg). After stirring at room temperature for 30 minutes, sodium triacetoxyborohydride (144 mg) was added. The reaction mixture was stirred at room temperature overnight. Trifluoroacetic acid (2 mL) was added and stirring was continued overnight. The reaction mixture was concentrated, and the residue was purified by reverse-phase HPLC using a Gilson system, eluting with 5-85% acetonitrile in water containing 0.1% v/v trifluoroacetic acid. The desired fractions were combined and freeze-dried to provide the title compound. ¹H NMR (400 MHz, dimethyl sulfoxide-*d₆*) δ ppm 12.86 (s, 1H), 8.22 (d, 2H), 8.05 - 8.01 (m, 1H), 7.79 (d, 1H), 7.61 (d, 1H), 7.53 - 7.41 (m, 3H), 7.36 (td, 2H), 7.28 (s, 1H), 6.95 (d, 1H), 4.95 (s, 2H), 3.88 (t, 2H), 3.82 (s, 2H), 3.26 - 2.94 (m, 7H), 2.10 (s, 3H), 1.84- 1.75 (m, 1H), 1.52-1.63 (m, 1H), 1.45 - 1.23 (m, 6H), 1.19 - 0.96 (m, 7H), 0.86 (s, 6H). MS (ESI) m/e 834.3 (M+H)⁺.

### 1.86 Synthesis of 4-({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl]amino}methyl)phenyl beta-D-glucopyranosiduronic acid (W2.86)

To a solution of 3-(1-((3-(2-aminoethoxy)-5,7-dimethyladamantan-1-yl)methyl)-5-methyl-1H-pyrazol-4-yl)-6-(8-(benzo[d]thiazol-2-ylcarbamoyl)-3,4-dihydroisoquinolin-2(1H)-yl)picolinic acid (36 mg) in tetrahydrofuran (2 mL) and acetic acid (0.2 mL) was added (2S,3R,4S,5S,6S)-2-(4-formylphenoxy)-6-(methoxycarbonyl)tetrahydro-2H-pyran-3,4,5-triyl triacetate (21 mg) followed by MgSO₄ (60 mg). The mixture was stirred at room temperature for 1 hour before the addition of MP-cyanoborohydride (Biotage, 153 mg, 2.49 mmol/g). The mixture was then stirred at room temperature for 3 hours. The mixture was filtered, and LiOH H₂O (20 mg) was added to the filtrate. The mixture was stirred at room temperature for 2 hours and then acidified with trifluoroacetic acid. The solution was purified by reverse-phase HPLC on a Gilson system (C18 column), eluting with 20-80% acetonitrile in water containing 0.1% trifluoroacetic acid, to give the title compound. MS (ESI) m/e 1028.3 (M+H)⁺.

### 1.87 Synthesis of 3-{[2-({3-[(4-{6-[8-(1,3-benzothiazol-2-ylcarbamoyl)naphthalen-2-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl]amino}propyl beta-D-glucopyranosiduronic acid (W2.87)

### 1.87.1 (2R,3R,5S,6S)-2-(3-hydroxypropoxy)-6-(methoxycarbonyl)tetrahydro-2H-pyran-3,4,5-triyl triacetate

To a stirred solution of (2R,3R,5S,6S)-2-bromo-6-(methoxycarbonyl)tetrahydro-2H-pyran-3,4,5-triyl triacetate (3.98 g) in toluene (60 mL) was added propane-1,3-diol (15.22 g). The mixture was stirred at 75 °C, and Ag₂CO₃ (5.52 g) was added in three portions over a period of 3 hours. The mixture was stirred at room temperature overnight, after which the suspension was filtered. The filtrate was concentrated, and the residue was purified by silica gel chromatography eluting with 50% ethyl acetate in heptane to give the title compound. MS (ESI) m/e 409.9 (M+NH₄)⁺.

### 1.87.2 (2S,3S,5R,6R)-2-(methoxycarbonyl)-6-(3-oxopropoxy)tetrahydro-2H-pyran-3,4,5-triyl triacetate

To a solution of dimethyl sulfoxide (0.5 mL) in dichloromethane (10 mL) at -78 °C was added oxalyl chloride (0.2 mL). The mixture was stirred 20 minutes at -78 °C, and a solution of Example 1.87.1 (393 mg ) in dichloromethane (10 mL) was added through a syringe. After 20 minutes, triethylamine (1 mL) was added. The mixture was stirred for 30 minutes, and the temperature was allowed to rise to room temperature. The reaction mixture was diluted with ethyl acetate (300 mL), washed with water and brine, and dried over anhydrous sodium sulfate. Filtration and evaporation of the solvent gave the title compound, which was used without further purification. MS (DCI) m/e 408.1 (M+NH₄)⁺.

### 1.87.3 3-{[2-({3-[(4-{6-[8-(1,3-benzothiazol-2-ylcarbamoyl)naphthalen-2-yl]-2-carboxypyridin-3-y }-5-methyl-1H-pyrazol-1-yl)methyl]-5,7-dimethyltricyclo[3.3.1.1^{3,7}]dec-1-yl}oxy)ethyl]amino}propyl beta-D-glucopyranosiduronic acid

To a solution of Example 1.68.6 (171 mg) in dichloromethane (10 mL) was added Example 1.87.2 (90 mg), and NaBH(OAc)₃ (147 mg). The mixture was stirred at room temperature overnight. The reaction mixture was diluted with ethyl acetate (200 mL), washed with 2% aqueous HCl solution, water, and brine, dried over anhydrous sodium sulfate, filtered and concentrated. The residue was dissolved in tetrahydrofuran (6 mL), methanol (3 mL) and water (3 mL) and LiOH H₂O (100 mg) was added. The mixture was stirred at room temperature for 2 hours, acidified with trifluoroacetic acid and concentrated under reduced pressure. The residue was dissolved in dimethyl sulfoxide/methanol (1:1 , 12 mL) and purified by reverse-phase HPLC on a Gilson system (C18 column), eluting with 20-80% acetonitrile in water containing 0.1% trifluoroacetic acid) to give the title compound. ¹H NMR (400 MHz, dimethylsulfonxide-*d*₆) δ ppm 13.07 (s, 2H), 8.99 (s, 1H), 8.34 (dd, 1H), 8.29 - 8.11 (m, 5H), 8.06 - 8.02 (m, 1H), 7.99 (d, 1H), 7.90 (d, 1H), 7.78 (d, 1H), 7.68 (dd, 1H), 7.55 - 7.40 (m, 2H), 7.34 (td, 1H), 4.23 (d, 1H), 3.87 (s, 2H), 3.76 (dt, 1H), 3.60 (d, 1H), 3.53 (dt, 3H), 3.29 (t, 1H), 3.15 (t, 1H), 3.06 - 2.91 (m, 6H), 2.20 (s, 3H), 1.83 (p, 2H), 1.44 (s, 2H), 1.30 (q, 4H), 1.14 (s, 4H), 1.03 (q, 2H), 0.85 (s, 7H). MS (ESI) m/e 975.2(M+H)⁺.

### 1.88 Synthesis of 6-[4-(1,3-benzothiazol-2-ylcarbamoyl)-2-oxidoisoquinolin-6-yl]-3-[1-({3,5-dimethyl-7-[2-(methylamino)ethoxy]tricyclo[3.3.1.1^{3,7}]dec-1-yl}methyl)-5-methyl-1H-pyrazol-4-yl]pyridine-2-carboxylic acid (W2.88)

### 1.88.1 methyl 6-(6-(tert-butoxycarbonyl)-5-(1-((3-(2-((tert-butoxycarbonyl)(methyl)amino)ethoxy)-5,7-dimethyladamantan-1-yl)methyl)-5-methyl-1H-pyrazol-4-yl)pyridin-2-yl)isoquinoline-4-carboxylate

To a solution of Example 1.78.1 (0.73 g) in 1,4-dioxane (20 mL) and water (10 mL) was added tert-butyl 3-(1-((3-(2-((tert-butoxycarbonyl)(methyl)amino)ethoxy)-5,7-dimethyladamantan-1-yl)methyl)-5-methyl-1H-pyrazol-4-yl)-6-chloropicolinate (1.5 g), bis(triphenylphosphine)palladium(II) dichloride (82 mg), and CsF (1.06 g), and the reaction was stirred at reflux overnight. The mixture was diluted with ethyl acetate (200 mL), washed with water and brine, dried over anhydrous sodium sulfate, filtered, and concentrated. The residue was purified by silica gel chromatography, eluting with 20% ethyl acetate in heptane (1 L) to give the title compound. MS (ESI) m/e 794.8 (M+H)⁺.

### 1.88.2 6-(6-(tert-butoxycarbonyl)-5-(1-((3-(2-((tert-butoxycarbonyl)(methyl)amino)ethoxy)-5,7-dimethyladamantan-1-yl)methyl)-5-methyl-1H-pyrazol-4-yl)pyridin-2-yl)isoquinoline-4-carboxylic acid

To a solution of Example 1.88.1 (300 mg) in tetrahydrofuran (6 mL), methanol (3 mL) and water (3 mL) was added LiOH H₂O (100 mg). The mixture was stirred at room temperature for 2 hours. The mixture was acidified with aqueous 2N HCl solution, diluted with ethyl acetate (300 mL), washed with water and brine, dried over anhydrous sodium sulfate, filtered and concentrated to give the title compound, which was used without further purification. MS (ESI) m/e 781.2 (M+H)⁺.

### 1.88.3 tert-butyl 6-(4-(benzo[d]thiazol-2-ylcarbamoyl)isoquinolin-6-yl)-3-(1-((3-(2-((tert-butoxycarbonyl)(methyl)amino)ethoxy)-5,7-dimethyladamantan-1-yl)methyl)-5-methyl-1H-pyrazol-4-yl)picolinate

To a solution of Example 1.88.2 (350 mg) in dichloromethane (10 mL) was added benzo[d]thiazol-2-amine (67.5 mg), 1-ethyl-3-(3-dimethylaminopropyl)carbodiimide (129 mg), and 4-dimethylaminopyridine (82 mg). The mixture was stirred at room temperature overnight. The mixture was diluted with ethyl acetate (300 mL), washed with water and brine, and dried over anhydrous sodium sulfate. Filtration and evaporation of the solvent gave a residue, which was purified by silica gel chromatography, eluting with 5% methanol in dichloromethane, to give the title compound. MS (APCI) m/e 912.3 (M+H)⁺.

### 1.88.4 4-(benzo[d]thiazol-2-ylcarbamoyl)-6-(6-carboxy-5-(1-((3,5-dimethyl-7-(2-(methylamino)ethoxy)adamantan-1-yl)methyl)-5-methyl-1H-pyrazol-4-yl)pyridin-2-yl)isoquinoline 2-oxide

To a solution of Example 1.88.3 (100 mg) in dichloromethane (6 mL) was added *m-*chloroperoxybenzoic acid (19 mg). The mixture was stirred at room temperature for 4 hours. The mixture was diluted with ethyl acetate (200 mL), washed with saturated aqueous NaHCO₃ solution, water, and brine, and dried over anhydrous sodium sulfate. Filtration and evaporation of the solvent gave a residue that was dissolved in dichloromethane/trifluoroacetic acid (10 mL, 1:1) and stirred at room temperature overnight. The solvents were evaporated, and the residue was purified by reverse-phase HPLC on a Gilson system (C18 column), eluting with 20-80% acetonitrile in water containing 0.1% trifluoroacetic acid, to give the title compound. ¹H NMR (501 MHz, dimethyl sulfoxide-*d*₆) δ ppm 13.32 (s, 2H), 9.21 (d, 1H), 8.71 (d, 1H), 8.49 (dd, 1H), 8.36 - 8.19 (m, 4H), 8.12 (dd, 1H), 8.07 (d, 1H), 7.96 (dd, 1H), 7.82 (d, 1H), 7.56 - 7.46 (m, 3H), 7.42 - 7.35 (m, 1H), 3.90 (d, 3H), 3.56 (td, 3H), 3.02 (p, 3H), 2.55 (t, 4H), 2.29 - 2.19 (m, 4H), 1.45 (d, 3H), 1.37 - 1.26 (m, 5H), 1.16 (d, 6H), 1.10 - 1.01 (m, 3H), 0.88 (d, 8H). MS (ESI) m/e 772.1 (M+H)⁺.

### 1.89 Synthesis of 6-{8-[(1,3-benzothiazol-2-yl)carbamoyl]-3,4-dihydroisoquinolin-2(1H)-yl}-3-{1-[(3,5-dimethyl-7-{2-[(2-sulfoethyl)amino]acetamido}tricyclo[3.3.1.1^{3,7}]decan-1-yl)methyl]-5-methyl-1H-pyrazol-4-yl}pyridine-2-carboxylic acid (W2.89)

### 1.89.1 1-((3-bromo-5,7-dimethyladamantan-1-yl)methyl)-5-methyl-1H-pyrazole

To a cooled (-30 °C) solution of Example 1.1.3 (500 mg) in tetrahydrofuran (30 mL) was added n-butyllithium (9.67 mL), and the mixture was stirred at -30 °C for 2 hours. Methyl iodide (1.934 mL) was added dropwise at -30 °C. After completion of the addition, the mixture was stirred at -30 °C for additional 2 hours. 1N aqueous HCl in ice water was added slowly, such that the temperature was maintained below 0 °C, until the pH reached 6. The mixture was stirred at room temperature for 10 minutes, and diluted with ice-water (10 mL) and ethyl acetate (20 mL). The layers were separated, and the aqueous layer was extracted twice with ethyl acetate. The combined organic phases were washed with brine, dried over MgSO₄, filtered and concentrated. The residue was purified by flash silica gel chromatography, eluting with 15/1 to 10/1petroleumeum/ethyl acetate, to give the title compound. MS (LC-MS) m/e 337, 339 (M+H)⁺.

### 1.89.2 1-(3,5-dimethyl-7-((5-methyl-1H-pyrazol-1-yl)methyl)adamantan-1-yl)urea

Example 1.89.1 (2.7 g) and urea (4.81 g) was mixed and stirred at 140 °C for 16 hours. The mixture was cooled to room temperature and suspended in methanol (200 mL x 2). The insoluble material was removed by filtration. The filtrate was concentrated to give the title compound. MS (LC-MS) m/e 317.3 (M+H) ⁺.

### 1.89.3 3,5-dimethyl-7-((5-methyl-1H-pyrazol-1-yl)methyl)adamantan-1-amine

To a solution of Example 1.40.2 (2.53 g) in 20% ethanol in water (20 mL) was added sodium hydroxide (12.79 g). The mixture was stirred at 120 °C for 16 hours and at 140 °C for another 16 hours. 6N Aqueous HCl was added until pH 6. The mixture was concentrated, and the residue was suspended in methanol (200 mL). The insoluble material was filtered off. The filtrate was concentrated to give the title compound as an HCl salt. MS (LC-MS) m/e 273.9 (M+H)⁺.

### 1.89.4 tert-butyl (2-((3,5-dimethyl-7-((5-methy1H-pyrazol-1-yl)methyl)adamantan-1-yl)amino)-2-oxoethyl)carbamate

To a solution of Example 1.89.3 (2.16g) in N,N-dimethylformamide (100 mL) was added triethylamine (3.30 mL), 2-((tert-butoxycarbonyl)amino)acetic acid (1.799 g) and 1-[bis(dimethylamino)methylene]-1H-1,2,3-triazolo[4,5-b]pyridinium 3-oxid hexafluorophosphate (3.90 g). The mixture was stirred at room temperature for 2 hours. Water (40 mL) was added, and the mixture was extracted with ethyl acetate (70 mL x 2). The combined organic phases were washed with brine, dried over sodium sulfate, filtered and concentrated. The residue was purified by silica gel chromatography, eluting with 3/1 to 2/1 petroleum/ethyl acetate, to give the title compound. MS (LC-MS) m/e 430.8 (M+H)⁺.

### 1.89.5 tert-butyl (2-((3-((4-iodo-5-methyl-1H-pyrazol-1-yl)methyl)-5,7-dimethyladamantan-1-yl)amino)-2-oxoethyl)carbamate

To an ambient solution of Example 1.89.4 (1.7 g) in N,N-dimethylformamide (20 mL) was added NIS (N-iodosuccinimide, 1.066 g) in portions, and the mixture was stirred at room temperature for 16 hours. Ice-water (10 mL) and saturated aqueous Na₂S₂O₃ solution (10 mL) were added. The mixture was extracted with ethyl acetate (30 mL x 2). The combined organic phases were washed with brine, dried over sodium sulfate, filtered and concentrated. The residue was purified by silica gel chromatography, eluting with 3/1 to 2/1 petroleum/ethyl acetate, to give the title compound. MS (LC-MS) m/e 556.6 (M+H)⁺.

### 1.89.6 methyl 2-(5-bromo-6-(tert-butoxycarbonyl)pyridin-2-yl)-1,2,3,4-tetrahydroisoquinoline-8-carboxylate

To a solution of methyl 1,2,3,4-tetrahydroisoquinoline-8-carboxylate hydrochloride (12.37 g) and Example 1.1.10 (15 g) in dimethyl sulfoxide (100 mL) was added N,N-diisopropylethylamine (12 mL), and the mixture was stirred at 50 °C for 24 hours. The mixture was then diluted with ethyl acetate (500 mL) and washed with water and brine. The organic layer was dried over sodium sulfate, filtered and concentrated under reduced pressure. The residue was purified by silica gel chromatography, eluting with 20% ethyl acetate in hexane, to give the title compound. MS (ESI) m/e 448.4 (M+H)⁺.

### 1.89.7 methyl 2-(6-(tert-butoxycarbonyl)-5-(4,4,5,5-tetramethyl-1,3,2-dioxaborolan-2-yl)pyridin-2-yl)-1,2,3,4-tetrahydroisoquinoline-8-carboxylate

To a solution of Example 1.89.6 (2.25 g) and [1,1'-bis(diphenylphosphino)ferrocene]dichloropalladium(II) (205 mg) in acetonitrile (30 mL) was added triethylamine (3 mL) and pinacolborane (2 mL), and the mixture was stirred at reflux for 3 hours. The mixture was diluted with ethyl acetate (200 mL) and washed with water and brine. The organic layer was dried over sodium sulfate, filtered and concentrated under reduced pressure. Purification of the residue by flash chromatography, eluting with 20% ethyl acetate in hexane, provided the title compound.

### 1.89.8 methyl 2-(6-(tert-butoxycarbonyl)-5-(1-((3-(2-((tert-butoxycarbonyl)amino)acetamido)-5,7-dimethyladamantan-1-yl)methyl)-5-methyl-1H-pyrazol-4-yl)pyridin-2-yl)-1,2,3,4-tetrahydroisoquinoline-8-carboxylate

The title compound was prepared using the procedure in Example 1.2.2, substituting Example 1.1.6 with Example 1.89.5. MS (ESI) m/e 797.4 (M+H)⁺.

### 1.89.9 2-(6-(tert-butoxycarbonyl)-5-(1-((3-(2-((tert-butoxycarbonyl)amino)acetamido)-5,7-dimethyladamantan-1-yl)methyl)-5-methyl-1H-pyrazol-4-yl)pyridin-2-yl)-1,2,3,4-tetrahydroisoquinoline-8-carboxylic acid

The title compound was prepared using the procedure in Example 1.2.5, substituting Example 1.2.4 with Example 1.89.8. MS (ESI) m/e 783.4 (M+H)⁺.

### 1.89.10 tert-butyl 6-(8-(benzo[d]thiazol-2-ylcarbamoyl)-3,4-dihydroisoquinolin-2(1H)-yl)-3-(1-((3-(2-((tert-butoxycarbonyl)amino)acetamido)-5,7-dimethyladamantan-1-yl)methyl)-5-methyl-1H-pyrazol-4-yl)picolinate

The title compound was prepared using the procedure in Example 1.2.6, substituting Example 1.2.5 with Example 1.89.9. MS (ESI) m/e 915.3 (M+H)⁺.

### 1.89.11 3-(1-{[3-(2-aminoacetamido)-5,7-dimethyltricyclo[3.3.1.1^{3,7}]decan-1-yl]methyl}-5-methyl-1H-pyrazol-4-yl)-6-{8-[(1,3-benzothiazol-2-yl)carbamoyl]-3,4-dihydroisoquinolin-2(1H)-yl}pyridine-2-carboxylic acid

The title compound was prepared using the procedure in Example 1.2.9, substituting Example 1.2.8 with Example 1.89.10. ¹H NMR (400 MHz, dimethyl sulfoxide-*d*₆) δ 12.82 (s, 1H), 8.00 (dd, 1H), 7.90 - 7.79 (m, 4H), 7.76 (d, 1H), 7.59 (dd, 1H), 7.49 - 7.38 (m, 3H), 7.37 - 7.29 (m, 2H), 7.25 (s, 1H), 6.92 (d, 1H), 4.92 (s, 2H), 3.85 (t, 2H), 3.77 (s, 2H), 3.40 (q, 2H), 2.98 (t, 2H), 2.07 (s, 3H), 1.63 (s, 2H), 1.57 - 1.38 (m, 4H), 1.15 - 0.93 (m, 6H), 0.80 (s, 6H). MS (ESI) m/e 759.2 (M+H)⁺.

### 1.89.12 6-{8-[(1,3-benzothiazol-2-yl)carbamoyl]-3,4-dihydroisoquinolin-2(1H)-yl}-3-{1-[(3,5-dimethyl-7-{2-[(2-sulfoethyl)amino]acetamido}tricyclo[3.3.1.1^{3,7}]decan-1-yl)methyl]-5-methyl-1H-pyrazol-4-yl}pyridine-2-carboxylic acid

To a solution of Example 1.89.11 (102 mg) in N,N-dimethylformamide (6 mL) was added 4-((tert-butyldiphenylsilyl)oxy)-2,2-dimethylbutyl ethenesulfonate (60 mg),and the mixture was stirred at room temperature over a weekend. The mixture was diluted with ethyl acetate (300 mL), washed with water and brine, and dried over anhydrous sodium sulfate. Filtration and evaporation of the solvent gave a residue that was dissolved in dichloromethane/trifluoroacetic acid (10 mL, 1:1) and stirred at room temperature overnight. The solvents were evaporated, and the residue was purified by reverse-phase HPLC on a Gilson system (C18 column), eluting with 20-80% acetonitrile in water containing 0.1% trifluoroacetic acid, to give the title compound. ¹H NMR (501 MHz, dimethyl sulfoxide-*d*₆) δ 12.83 (s, 1H), 8.57 (s, 2H), 8.02 (d, 1H), 7.95 (s, 1H), 7.77 (d, 1H), 7.60 (d, 1H), 7.52 - 7.37 (m, 3H), 7.39 - 7.29 (m, 2H), 7.26 (s, 1H), 6.94 (d, 1H), 4.94 (s, 2H), 3.87 (t, 2H), 3.79 (s, 2H), 3.16 (q, 2H), 2.99 (t, 2H), 2.77 (t, 2H), 2.08 (s, 3H), 1.64 (s, 2H), 1.55 (d, 2H), 1.45 (d, 2H), 1.21 - 0.95 (m, 6H), 0.82 (s, 6H). MS (ESI) m/e 867.2 (M+H)⁺.

### 1.90 Synthesis of 6-[8-(1,3-benzothiazol-2-ylcarbamoyl)-3,4-dihydroisoquinolin-2(1H)-yl]-3-(1-{[3,5-dimethyl-7-({2-[(2-sulfoethyl)amino]ethyl}sulfanyl)tricyclo[3.3.1.1^{3,7}]dec-1-yl]methyl}-5-methyl-1H-pyrazol-4-yl)pyridine-2-carboxylic acid (W2.90)

### 1.90.1 3-((1H-pyrazol-1-yl)methyl)-5,7-dimethyladamantane-1-thiol

A mixture of Example 1.1.3 (2.8g) and thiourea (15.82 g) in 33% (w/w) HBr in acetic acid (50 mL) was stirred at 110 °C for 16 hours and was concentrated under reduced pressure to give a residue. The residue was dissolved in 20% ethanol in water (v/v: 200 mL), and sodium hydroxide (19.06 g) was added. The resulting solution was stirred at room temperature for 16 hours and was concentrated. The residue was dissolved in water (60 mL), and acidified with 6 N aqueous HCl to pH 5 - pH 6. The mixture was extracted with ethyl acetate (200 mL x 2). The combined organic layers were washed with brine, dried over MgSO₄, filtered and concentrated to give the title compound. MS (ESI) m/e 319.1 (M+H)⁺.

### 1.90.2 2-((-3-((1H-pyrazol-1-yl)methyl)-5,7-dimethyladamantan-1-yl)thio)ethanol

To a solution of Example 1.90.1 (3.3g) in ethanol (120 mL) was added sodium ethoxide (2.437 g). The mixture was stirred for 10 minutes, and 2-chloroethanol (1.80 mL) was added dropwise. The mixture was stirred at room temperature for 6 hours and was neutralized with 1 N aqueous HCl to pH 7. The mixture was concentrated, and the residue was extracted with ethyl acetate (200 mL x 2). The combined organic layers were washed with brine, dried over MgSO₄, filtered and concentrated. The residue was purified by column chromatography on silica gel, eluting with petroleum ether /ethyl acetate from 6/1 to 2/1, to give the title compound. MS (ESI) m/e 321.2 (M+H)⁺.

### 1.90.3 2-((-3,5-dimethyl-7-((5-methyl-1H-pyrazol-1-yl)methyl)adamantan-1-yl)thio)ethanol

To a solution of Example 1.90.2 (2.3 g) in tetrahydrofuran (60 mL) was added n-butyllithium (14.35 mL, 2M in hexane) at -20 °C dropwise under nitrogen. The mixture was stirred at this temperature for 2 hours. Methyl iodide (4.49 mL) was added to the resulting mixture at -20 °C, and the mixture was stirred at -20 °C for 2 hours. The reaction was quenched by the dropwise addition of saturated aqueous NH₄Cl solution at -20 °C. The resulting mixture was stirred for 10 minutes and acidified with 1 N aqueous HCl to pH 5. The mixture was extracted with ethyl acetate twice. The combined organic layers were washed with brine, dried over MgSO₄, filtered and concentrated to give the title compound. MS (ESI) m/e 335.3 (M+H)⁺.

### 1.90.4 2-((-3-((4-iodo-5-methyl-1H-pyrazol-1-yl)methyl)-5,7-dimethyladamantan-1-yl)thio)ethanol

To a solution of Example 1.90.3 (3.65 g) in N,N-dimethylformamide (90 mL) was added N-iodosuccinimide (3.68 g). The mixture was stirred at room temperature for 16 hours. The reaction was quenched by the addition of ice-water (8 mL) and saturated aqueous NaS₂O₃solution (8 mL). The mixture was stirred for an additional 10 minutes and was extracted with ethyl acetate (30 mL x 2). The combined organic layers were washed with brine, dried over MgSO₄, filtered and concentrated under reduced pressure. The residue was purified by silica gel chromatography, eluting with petroleum ether/ethyl acetate (6/1 to 3/1), to give the title compound. MS (ESI) m/e 461.2 (M+H)⁺.

### 1.90.5 di-tert-butyl [2-({3-[(4-iodo-5-methyl-1H-pyrazol-1-yl)methyl]-5,7-dimethyltricyclo[3.3.1.1^{3,7}]decan-1-yl}sulfanyl)ethyl]-2-imidodicarbonate

To a cold solution (0 °C bath) of Example 1.90.4 (3 g) in dichloromethane (100 mL) was added triethylamine (1.181 mL) and mesyl chloride (0.559 mL). The mixture was stirred at room temperature for 4 hours, and the reaction was quenched by the addition of ice-water (30 mL). The mixture was stirred for an additional 10 minutes and was extracted with dichloromethane (50 mL x 2). The combined organic layers were washed with brine, dried over MgSO₄, filtered and concentrated under reduced pressure. The residue was dissolved in acetonitrile (100 mL) and NH(Boc)₂ (1.695 g) and Cs₂CO₃ (4.24 g) were added. The mixture was stirred at 85 °C for 16 hours, and the reaction was quenched by the addition of water (20 mL). The mixture was stirred for 10 minutes and was extracted with ethyl acetate (40 mL x 2). The combined organic layers were washed with brine, dried over MgSO₄, filtered and concentrated. The residue was purified by silica gel chromatography, eluting with petroleum ether/ethyl acetate from 10/1 to 6/1, to give the title compound. MS (ESI) m/e 660.1 (M+H)⁺.

### 1.90.6 methyl 2-[5-(1-{[3-({2-[bis(tert-butoxycarbonyl)amino]ethyl}sulfanyl)-5,7-dimethyltricyclo[3.3.1.1^{3,7}]decan-1-yl]methyl}-5-methyl-1H-pyrazol-4-yl)-6-(tert-butoxycarbonyl)pyridin-2-yl]-1,2,3,4-tetrahydroisoquinoline-8-carboxylate

The title compound was prepared using the procedure in Example 1.2.2, replacing Example 1.1.6 with Example 1.90.5. MS (ESI) m/e 900.2 (M+H)⁺.

### 190.7A 2-(6-(tert-butoxycarbonyl)-5-(1-((3-((2-((tert-butoxycarbonyl)amino)ethyl)thio)-5,7-dimethyladamantan-1-yl)methyl)-5-methyl-1H-pyrazol-4-yl)pyridin-2-yl)-1,2,3,4-tetrahydroisoquinoline-8-carboxylic acid

The title compound was prepared as described in Example 1.2.5, replacing Example 1.2.4 with Example 1.90.6. MS (ESI) m/e 786.2 (M+H)⁺.

### 190.7B tert-butyl 6-(8-(benzo[d]thiazol-2-ylcarbamoyl)-3,4-dihydroisoquinolin-2(1H)-yl)-3-(1-((3-((2-((tert-butoxycarbonyl)amino)ethyl)thio)-5,7-dimethyladamantan-1-yl)methyl)-5-methyl-1H-pyrazol-4-yl)picolinate

The title compound was prepared as described in Example 1.2.6, replacing Example 1.2.5 with Example 1.90.7A. MS (ESI) m/e 918.8 (M+H)⁺.

### 1.90.8 tert-butyl 3-(1-((3-((2-aminoethyl)thio)-5,7-dimethyladamantan-1-yl)methyl)-5-methyl-1H-pyrazol-4-yl)-6-(8-(benzo[d]thiazol-2-ylcarbamoyl)-3,4-dihydroisoquinolin-2(1H)-yl)picolinate

To a solution of Example 1.90.7B (510 mg) in dichloromethane (5 mL) was added trifluoroacetic acid (5 mL,) and the reaction was stirred at room temperature for 30 minutes. The reaction was quenched by the addition of saturated aqueous sodium bicarbonate solution and extracted with dichloromethane thrice. The combined organics were dried with anhydrous sodium sulfate, filtered and concentrated under reduced pressure. The residue was purified by reverse-phase HPLC on a Gilson system (C18 column), eluting with 20-80% acetonitrile in water containing 0.1% trifluoroacetic acid, to give the title product. MS (ESI) m/e 818.1 (M+H)⁺.

### 1.90.9 3-(1-((3-((2-aminoethyl)thio)-5,7-dimethyladamantan-1-yl)methyl)-5-methyl-1H-pyrazol-4-yl)-6-(8-(benzo[d]thiazol-2-ylcarbamoyl)-3,4-dihydroisoquinolin-2(1H)-yl)picolinic acid

Example 1.90.9 was isolated during the preparation of Example 1.90.8. MS (ESI) 762.2 (M+H)⁺.

### 1.90.10 tert-butyl 6-(8-(benzo[d]thiazol-2-ylcarbamoyl)-3,4-dihydroisoquinolin-2(1H)-yl)-3-(1-((3-((2-((2-((4-((tert-butyldiphenylsilyl)oxy)-2,2-dimethylbutoxy)sulfonyl)ethyl)amino)ethyl)thio)-5,7-dimethyladamantan-1-yl)methyl)-5-methyl-1H-pyrazol-4-yl)picolinate

Example 1.90.8 (235 mg) and 4-((tert-butyldiphenylsilyl)oxy)-2,2-dimethylbutyl ethenesulfonate (150 mg) were dissolved in dichloromethane (1 mL), N,N-diisopropylethylamine (140 µL) was added, and the mixture was stirred at room temperature for six days. The reaction was directly purified by silica gel chromatography, eluting with a gradient of 0.5-3.0% methanol in dichloromethane, to give the title compound.

### 1.90.11 6-(8-(benzo[d]thiazol-2-ylcarbamoyl)-3,4-dihydroisoquinolin-2(1H)-yl)-3-(1-((3,5-dimethyl-7-((2-((2-sulfoethyl)amino)ethyl)thio)adamantan-1-yl)methyl)-5-methyl-1H-pyrazol-4-yl)picolinic acid

The title compound was prepared by substituting Example 1.90.10 for Example 1.2.8 in Example 1.2.9. ¹H NMR (500 MHz, dimethyl sulfoxide-*d*₆) δ ppm 8.39 (br s, 2H), 8.03 (d, 1H), 7.79 (d, 1H), 7.62 (d, 1H), 7.51 (d, 1H),7.47 (ddd, 1H), 7.43 (d, 1H), 7.37 (d, 1H), 7.35 (ddd, 1H), 7.30 (s, 1H), 6.96 (d, 1H), 4.96 (s, 2H), 3.89 (t, 2H), 3.81 (s, 2H), 3.22 (m, 2H), 3.06 (br m, 2H), 3.01 (t, 2H), 2.79 (t, 2H), 2.74 (m, 2H), 2.10 (s, 3H), 1.51 (s, 2H), 1.37 (m, 4H), 1.15 (m, 4H), 1.05 (m, 2H), 0.83 (s, 6H). MS (ESI) m/e 870.1 (M+H)⁺.

### 1.91 Synthesis of 6-{8-[(1,3-benzothiazol-2-yl)carbamoyl]-3,4-dihydroisoquinolin-2(1H)-yl}-3-{1-[(3,5-dimethyl-7-{3-[(2-sulfoethyl)amino]propyl}tricyclo[3.3.1.1^{3,7}]decan-1-yl)methyl]-5-methyl-1H-pyrazol-4-yl}pyridine-2-carboxylic acid (W2.91)

### 1.91.1 1-((3-allyl-5,7-dimethyladamantan-1-yl)methyl)-1H-pyrazole

To a solution of Example 1.1.3 (0.825 g, 2.55 mmol) in toluene (5 mL) was added N, N'-azoisobutyronitrile (AIBN, 0.419 g, 2.55 mmol) and allyltributylstannane (2.039 ml, 6.38 mmol). The mixture was purged with N₂ stream for 15 minutes, heated at 80°C for 8 hours and concentrated. The residue was purified by flash chromatography, eluting with 5% ethyl acetate in petroleum ether to provide the title compound. MS (ESI) m/e 285.2 (M+H)⁺.

### 1.91.2 1-((3-allyl-5,7-dimethyladamantan-1-yl)methyl)-5-methyl-1H-pyrazole

To a solution of Example 1.91.1 (200 mg, 0.703 mmol) in tetrahydrofuran (5 ml) at -78°C under N₂ was added n-butyllithium (2.81 mL, 7.03 mmol). The mixture was stirred for 2 hours while the temperature increased to -20°C and then it was stirred at -20°C for 1 hour. Iodomethane (0.659 ml, 10.55 mmol) was added and the resulting mixture was stirred for 0.5 hours at -20°C. The reaction was quenched with saturated NH₄Cl and extracted with ethyl acetate twice. The combined organic layer was washed with brine and concentrated to give the title compound. MS (ESI) m/e 299.2 (M+H)⁺.

### 1.91.3 3-(3,5-dimethyl-7-((5-methyl-1H-pyrazol-1-yl)methyl)adamantan-1-yl)propan-1-ol

Under nitrogen atmosphere, a solution of Example 1.91.2 (2.175 g, 7.29 mmol) in anhydrous tetrahydrofuran (42.5 mL) was cooled to 0°C. BH₃·THF (15.30 mL, 15.30 mmol) was added dropwise. The reaction mixture was stirred at room temperature for 2 hours and cooled to 0°C. To the reaction mixture was added 10 N aqueous NaOH (5.03 mL, 50.3 mmol) dropwise, followed by 30 percent H₂O₂ (16.52 mL, 146 mmol) water solution. The resulting mixture was warmed to room temperature and stirred for 90 minutes. The reaction was quenched with 10 percent hydrochloric acid (35 mL). The organic layer was separated and the aqueous layer was extracted with ethyl acetate (2 x 60 mL). The combined organic layers were washed with brine (3 x 60 mL) and cooled in an ice bath. A saturated aqueous solution of sodium sulfite (15 mL) was carefully added and the mixture was stirred for a few minutes. The organic layer was dried over sodium sulfate, filtered, and concentrated in vacuo. The residue was purified by flash chromatography, eluting with petroleum ether/ethyl acetate (3:1 to 1:1) to provide the title compound. MS (ESI) m/e 317.3 (M+H)⁺.

### 1.91.4 3-(3-((4-iodo-5-methyl-1H-pyrazol-1-yl)methyl)-5,7-dimethyladamantan-1-yl)propan-1-ol

A mixture of Example 1.91.3 (1.19 g, 3.76 mmol) and 1-iodopyrrolidine-2, 5-dione (1.015 g, 4.51 mmol) in N,N-dimethylformamide (7.5 mL) was stirred for 16 hours at room temperature. The reaction was quenched with saturated Na₂SO₃. The mixture was diluted with ethyl acetate and washed with saturated Na₂SO₃, saturated Na₂CO₃, water and brine. The organic layer was dried over anhydrous Na₂SO₄, filtered, and concentrated. The residue was purified by flash chromatography, eluting with petroleum ether/ ethyl acetate (3:1 to1:1) to provide the title compound. MS (ESI) m/e 443.1 (M+H)⁺.

### 1.91.5 3-(3-((4-iodo-5-methyl-1H-pyrazol-1-yl)methyl)-5,7-dimethyladamantan-1-yl)propyl methanesulfonate

To a solution of Example 1.91.4 (1.55 g, 3.50 mmol) in CH₂Cl₂ (20 mL) at 0°C were added (CH₃CH₂)₃N (0.693 mL, 4.98 mmol) and mesyl chloride (0.374 mL, 4.80 mmol) slowly. The mixture was stirred for 3.5 hours at 20°C and diluted with CH₂Cl₂, washed with saturated NH₄Cl, NaHCO₃ and brine. The organic layer was dried over Na₂SO₄, filtered, and concentrated to provide the title compound. MS (ESI) m/e 521.1 (M+H)⁺.

### 1.91.6 di-tert-butyl (3-{3-[(4-iodo-5-methyl-1H-pyrazol-1-yl)methyl]-5,7-dimethyltricyclo[3.3.1.1^{3,7}]decan-1-yl}propyl)-2-imidodicarbonate

To a solution of Example 1.91.5 (1.92 g, 3.69 mmol) in CH₃CN (40 ml) at 20°C was added di-tert-butyl iminodicarbonate (0.962 g, 4.43 mmol) and Cs₂CO₃ (2.404 g, 7.38 mmol). The mixture was stirred for 16 hours at 80°C and was diluted with ethyl acetate, and was washed with water and brine. The organic layer was dried over Na₂SO₄, filtered, and concentrated. The residue was purified by flash chromatography, eluting with petroleum ether/ ethyl acetate (10:1) to provide the title compound. MS (ESI) m/e 642.3 (M+H)⁺.

### 1.91.7 methyl 2-[5-{1-[(3-{3-[bis(tert-butoxycarbonyl)amino]propyl}-5,7-dimethyltricyclo[3.3.1.1^{3,7}]decan-1-yl)methyl]-5-methyl-1H-pyrazol-4-yl}-6-(tert-butoxycarbonyl)pyridin-2-yl]-1,2,3,4-tetrahydroisoquinoline-8-carboxylate

The title compound was prepared using the procedure in Example 1.2.2, replacing Example 1.1.6 with Example 1.91.6. MS (ESI) m/e 882.2 (M+H)⁺.

### 1.91.8 2- [6-(tert-butoxycarbonyl)-5-{1-[(3-{3-[(tert-butoxycarbonyl)amino]propyl}-5,7-dimethyltricyclo[3.3.1.1^{3,7}]decan-1-yl)methyl]-5-methyl-1H-pyrazol-4-yl}pyridin-2-yl]-1,2,3,4-tetrahydroisoquinoline-8-carboxylic acid

The title compound was prepared using the procedure in Example 1.2.5, replacing Example 1.2.4 with Example 1.91.7. MS (ESI) m/e 768.4 (M+H)⁺.

### 1.91.9 tert-butyl 6-(8-(benzo[d]thiazol-2-ylcarbamoyl)-3,4-dihydroisoquinolin-2(1H)-yl)-3-(1-((3-(3-((tert-butoxycarbonyl)amino)propyl)-5,7-dimethyladamantan-1-yl)methyl)-5-methyl-1H-pyrazol-4-yl)picolinate

The title compound was prepared using the procedure in Example 1.2.6, replacing Example 1.2.5 with Example 1.91.8. MS (ESI) m/e 901.1 (M+H)⁺.

### 1.91.10 tert-butyl 3-(1-((3-(3-aminopropyl)-5,7-dimethyladamantan-1-yl)methyl)-5-methyl-1H-pyrazol-4-yl)-6-(8-(benzo[d]thiazol-2-ylcarbamoyl)-3,4-dihydroisoquinolin-2(1H)-yl)picolinate

To a solution of Example 1.91.9 (500 mg) in dichloromethane (5 mL) was added trifluoroacetic acid (5 mL) and the reaction was stirred at room temperature for 30 minutes. The reaction was quenched by the addition of saturated aqueous sodium bicarbonate solution and extracted with dichloromethane thrice. The combined organics were dried with anhydrous sodium sulfate, filtered and concentrated under reduced pressure. The residue was purified by reverse-phase HPLC on a Gilson system (C18 column), eluting with 20-80% acetonitrile in water containing 0.1% trifluoroacetic acid, to give the title product.

### 1.91.11 3-(1-((3-(3-aminopropyl)-5,7-dimethyladamantan-1-yl)methyl)-5-methyl-1H-pyrazol-4-yl)-6-(8-(benzo[d]thiazol-2-ylcarbamoyl)-3,4-dihydroisoquinolin-2(1H)-yl)picolinic acid

To a solution of Example 1.91.9 (350 mg) in dichloromethane (5 mL) was added trifluoroacetic acid (5 mL). The mixture was stirred overnight. The mixture was concentrated and the residue was purified by reverse phase HPLC using a Gilson system, eluting with 20-80% acetonitrile in water containing 0.1% v/v trifluoroacetic acid, to provide the title compound. ¹H NMR (500 MHz, DMSO-*d*₆) δ ppm 12.86 (s, 1H), 8.03 (d, 1H), 7.79 (d, 1H), 7.62 (d, 4H), 7.47 (dt, 3H), 7.36 (q, 2H), 7.27 (s, 1H), 6.95 (d, 1H), 4.95 (s, 2H), 3.77 (s, 2H), 3.01 (t, 2H), 2.72 (q, 2H), 2.09 (s, 3H), 1.45 (t, 2H), 1.18 - 1.05 (m, 9H), 1.00 (d, 6H), 0.80 (s, 6H). MS (ESI) m/e 744.2 ( M+H)⁺.

### 1.91.12 tert-butyl 6-(8-(benzo[d]thiazol-2-ylcarbamoyl)-3,4-dihydroisoquinolin-2(1H)-yl)-3-(1-((3-(3-((2-((4-((tert-butyldiphenylsilyl)oxy)-2,2-dimethylbutoxy)sulfonyl)ethyl)amino)propyl)-5,7-dimethyladamantan-1-yl)methyl)-5-methyl-1H-pyrazol-4-yl)picolinate

The title compound was prepared using the procedure in Example 1.2.8, replacing Example 1.2.7 with Example 1.91.10.

### 1.91.13 6-{8-[(1,3-benzothiazol-2-yl)carbamoyl]-3,4-dihydroisoquinolin-2(1H)-yl}-3-{1-[(3,5-dimethyl-7-{3-[(2-sulfoethyl)amino]propyl}tricyclo[3.3.1.1^{3,7}]decan-1-yl)methyl]-5-methyl-1H-pyrazol-4-yl}pyridine-2-carboxylic acid

The title compound was prepared using the procedure in Example 1.2.9, replacing Example 1.2.8 with Example 1.91.12. ¹H NMR (501 MHz, DMSO-*d*₆) δ 12.85 (s, 1H), 8.02 (dd, 1H), 7.77 (d, 1H), 7.60 (d, 1H), 7.54 - 7.39 (m, 3H), 7.38 - 7.31 (m, 2H), 7.26 (s, 1H), 6.94 (d, 1H), 4.94 (s, 2H), 3.87 (t, 2H), 3.15 (p, 2H), 3.00 (t, 2H), 2.86 (dq, 2H), 2.76 (t, 2H), 2.08 (s, 3H), 1.47 (td, 2H), 1.08 (d, 9H), 0.99 (d, 7H), 0.79 (s, 7H). MS (ESI) m/e 852.2 (M+H)⁺.

### Example 2. Synthesis of Exemplary Synthons

This example provides synthetic methods for exemplary synthons useful to make ADCs.

### 2.1 Synthesis of N-[6-(2,5-dioxo-2,5-dihydro-1H-pyrrol-1-yl)hexanoyl]-L-valyl-N-{4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)methyl]phenyl}-N⁵-carbamoyl-L-ornithinamide (Synthon CZ)

Example 1.2.9 (100 mg) and 4-((S)-2-((S)-2-(6-(2,5-dioxo-2,5-dihydro-1H-pyrrol-1-yl)hexanamido)-3-methylbutanamido)-5-ureidopentanamido)benzyl (4-nitrophenyl) carbonate (purchased from Synchem, 114 mg) in N,N-dimethylformamide (7 mL) was cooled in an water-ice bath, and N,N-diisopropylethylamine (0.15 mL) was added. The mixture was stirred at 0 °C for 30 minutes and then at room temperature overnight. The reaction was purified by a reverse phase HPLC using a Gilson system, eluting with 20-60% acetonitrile in water containing 0.1% v/v trifluoroacetic acid, to provide the title compound. ¹H NMR (400 MHz, dimethyl sulfoxide-*d*₆) δ ppm 12.85 (s, 1H), 9.99 (s, 1H), 8.04 (t, 2H), 7.75-7.82 (m, 2H), 7.40-7.63 (m, 6H), 7.32-7.39 (m, 2H), 7.24-7.29 (m, 3H), 6.99 (s, 2H), 6.95 (d, 1H), 6.01 (s, 1H), 4.83-5.08 (m, 4H), 4.29-4.48 (m, 1H), 4.19 (t, 1H), 3.84-3.94 (m, 2H), 3.80 (d, 2H), 3.14-3.29 (m, 2H), 2.87-3.06 (m, 4H), 2.57-2.69 (m, 2H), 2.03-2.24 (m, 5H), 1.89-2.02 (m, 1H), 1.53-1.78 (m, 2H), 1.26-1.53 (m, 8H), 0.89-1.27 (m, 12H), 0.75-0.88 (m, 12H). MS (ESI) m/e 1452.2 (M+H)⁺.

### 2.2 Synthesis of N-[6-(2,5-dioxo-2,5-dihydro-1H-pyrrol-1-yl)hexanoyl]-L-valyl-N-{4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](3-sulfopropyl)carbamoyl}oxy)methyl]phenyl}-N⁵-carbamoyl-L-ornithinamide (Synthon DH)

The title compound was prepared as described in Example 2.1, replacing Example 1.2.9 with Example 1.6.2. ¹H NMR (400 MHz, dimethyl sulfoxide-*d*₆) δ ppm 12.83 (s, 1H), 9.98 (s, 1H), 8.04 (t, 2H), 7.75-7.81 (m, 2H), 7.54-7.64 (m, 3H), 7.40-7.54 (m, 3H), 7.32-7.39 (m, 2H), 7.24-7.31 (m, 3H), 6.93-7.01 (m, 3H), 4.86-5.03 (m, 4H), 4.32-4.48 (m, 2H), 4.13-4.26 (m, 2H), 3.31-3.45 (m, 4H), 3.24 (d, 4H), 2.88-3.07 (m, 4H), 2.30-2.39 (m, 2H), 2.04-2.24 (m, 5H), 1.86-2.03 (m, 1H), 0.89-1.82 (m, 27H), 0.74-0.88 (m, 13H). MS (ESI) m/e 1466.3 (M+H)⁺.

### 2.3This paragraph was intentionally left blank.

### 2.4 Synthesis of N-[6-(2,5-dioxo-2,5-dihydro-1H-pyrrol-1-yl)hexanoyl]-L-valyl-N-[4-({[{2-[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethoxy]ethyl}(2-sulfoethyl)carbamoyl]oxy}methyl)phenyl]-N⁵-carbamoyl-L-ornithinamide (Synthon EP)

The title compound was prepared as described in Example 2.1, replacing Example 1.2.9 with Example 1.11.4. ¹H NMR (500 MHz, dimethyl sulfoxide-*d*₆) δ ppm 12.85 (s, 1H), 10.00 (s, 1H), 8.01-8.10 (m, 2H), 7.79 (dd, 2H), 7.55-7.65 (m, 3H), 7.41-7.53 (m, 3H), 7.32-7.38 (m, 2H), 7.25-7.30 (m, 3H), 6.97-7.02 (m, 2H), 6.96 (d, 1H), 6.03 (s, 1H), 4.90-5.03 (m, 4H), 4.31-4.46 (m, 1H), 4.20 (s, 1H), 3.88 (t, 2H), 3.82 (s, 2H), 2.97-3.06 (m, 2H), 2.88-2.98 (m, 1H), 2.58-2.68 (m, 2H), 2.05-2.22 (m, 5H), 1.92-2.02 (m, 1H), 0.89-1.75 (m, 23H), 0.77-0.87 (m, 12H). MS (ESI) m/e 1496.3 (M+H)⁺.

### 2.5 Synthesis of methyl 6-[4-(3-{[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl]({[4-({N-[6-(2,5-dioxo-2,5-dihydro-1H-pyrrol-1-yl)hexanoyl]-L-valyl-N⁵-carbamoyl-L-ornithyl}amino)benzyl]oxy}carbonyl)amino}propyl)-1H-1,2,3-triazol-1-yl]-6-deoxy-beta-L-glucopyranoside (Synthon EF)

### 2.5.1 pent-4-ynal

To a solution of oxalyl chloride (9.12 mL) dissolved in dichloromethane (200 mL) at-78 °C was added dimethyl sulfoxide (14.8 mL) dissolved in dichloromethane (40 mL) over 20 minutes. After the solution was stirred for an additional 30 minutes, 4-pentynol (8.0 g) dissolved in dichloromethane (80 mL) was added over 10 minutes. The reaction mixture was stirred at-78 °C for an additional 60 minutes. Triethylamine (66.2 mL) was added at-78 °C, and the reaction mixture was stirred for 60 minutes and then allowed to warm to 10 °C over an additional hour. Water (200 mL) was added, and the two layers were separated. The aqueous layer was acidified with 1% aqueous HCl and then back-extracted with dichloromethane (3x 100 mL). The combined organic layers were washed with 1% aqueous HCl, and aqueous NaHCO₃. The aqueous extracts were back-extracted with dichloromethane (2x 100 mL), and the combined organic extracts were washed with brine and dried over sodium sulfate. After filtration, the solvent was removed by rotary evaporation (30 °C water bath) to provide the title compound.

### 2.5.2 6-(8-(benzo[d]thiazol-2-ylcarbamoyl)-3,4-dihydroisoquinolin-2(1H)-yl)-3-(1-((3,5-dimethyl-7-(2-(pent-4-yn-1-ylamino)ethoxy)adamantan-1-yl)methyl)-5-methyl-1H-pyrazol-4-yl)picolinic acid

To a solution of Example 1.2.7 (85 mg) in tetrahydrofuran (2 mL) was added pent-4-yanl (8.7 mg), acetic acid (20 mg) and sodium sulfate (300 mg). The mixture was stirred for 1 hour, and sodium triacetoxyborohydride (45 mg) was added to the reaction mixture. The mixture was stirred overnight, then diluted with ethyl acetate (200 mL), washed with water and brine, and dried over sodium sulfate. Filtration and evaporation of the solvent gave a residue, which was dissolved in dimethyl sulfoxide/methanol (1:1, 3 mL). The mixture was purified by reverse phase HPLC on a Gilson system, eluting with 10-85% acetonitrile in 0.1% trifluoroacetic acid in water, to give the title compound. MS (ESI) m/e 812.1 (M+H)⁺.

### 2.5.3 6-(8-(benzo[d]thiazol-2-ylcarbamoyl)-3,4-dihydroisoquinolin-2(1H)-yl)-3-(1-((3,5-dimethyl-7-(2-((3-(1-(((2S,3R,4R,5S,6S)-3,4,5-trihydroxy-6-methoxytetrahydro-2H-pyran-2-yl)methyl)-1H-1,2,3-triazol-4-yl)propyl)amino)ethoxy)adamantan-1-yl)methyl)-5-methyl-1H-pyrazol-4-yl)picolinic acid

To a solution of (2S,3S,4R,5S,6S)-2-(azidomethyl)-6-methoxytetrahydro-2H-pyran-3,4,5-triyl triacetate (8.63 mg) in t-butanol (2 mL) and water (1 mL) was added Example 2.5.2 (20 mg), copper(II) sulfate pentahydrate (2.0 mg) and sodium ascorbate (5 mg). The mixture was stirred 20 minutes at 100 °C under microwave conditions (Biotage Initiator). Lithium hydroxide monohydrate (50 mg) was added to the mixture, and it was stirred overnight. The mixture was neutralized with trifluoroacetic acid and purified by reverse phase HPLC (Gilson system), eluting with 10-85% acetonitrile in 0.1% trifluoroacetic acid in water, to provide the title compound. MS (ESI) m/e 1032.2 (M+H)⁺.

### 2.5.4 methyl 6-[4-(3-{[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl]({[4-({N-[6-(2,5-dioxo-2,5-dihydro-1H-pyrrol-1-yl)hexanoyl]-L-valyl-N⁵-carbamoyl-L-ornithyl}amino)benzyl]oxy}carbonyl)amino} propyl)-1H-1,2,3-triazol-1-yl]-6-deoxy-beta-L-glucopyranoside

To a solution of 4-((S)-2-((S)-2-(6-(2,5-dioxo-2,5-dihydro-1H-pyrrol-1-yl)hexanamido)-3-methylbutanamido)-5-ureidopentanamido)benzyl 4-nitrophenyl carbonate (7.16 mg) and Example 2.5.3 (10 mg) in N,N-dimethylformamide (2 mL) was added N,N-diisopropylethylamine (0.1 mL). The mixture was stirred overnight, then acidified with trifluoroacetic acid and purified by reverse phase HPLC (Gilson system), eluting with 10-85% acetonitrile in 0.1% trifluoroacetic acid in water, to provide the title compound. ¹H NMR (400 MHz, dimethyl sulfoxide-*d*₆) δ ppm 9.65 (s, 1H), 7.97 (d, 1H), 7.76 (d, 1H), 7.64-7.72 (m, 2H), 7.53-7.63 (m, 3H), 7.38-7.51 (m, 4H), 7.30-7.37 (m, 2H), 7.22-7.27 (m, 3H), 6.84-6.98 (m, 3H), 4.97 (d, 4H), 4.65 (dd, 1H), 4.50 (d, 1H), 4.36-4.46 (m, 1H), 4.25-4.32 (m, 1H), 4.10-4.20 (m, 1H), 3.85-3.95 (m, 2H), 3.79 (s, 2H) , 3.66-3.73 (m, 2H), 2.99-3.03 (m, 7H), 2.57 (t, 3H), 2.12-2.22 (m, 3H), 2.08 (s, 3H), 1.99-2.05 (m, 2H), 1.70-1.88 (m, 4H) , 1.39-1.67 (m, 8H), 1.35 (s, 3H), 0.92-1.28 (m, 14H), 0.80-0.88 (m, 16H). MS (ESI) m/e 1629.5 (M+H)⁺.

### 2.6 Synthesis of N-[6-(2,5-dioxo-2,5-dihydro-1H-pyrrol-1-yl)hexanoyl]-L-valyl-N-(4-{[([2-({3-[(4-{6-[8-(1,3-benzothiazol-2-ylcarbamyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl]oxy)ethyl]{3-[1-(beta-D-glucopyranuronosyl)-1H-1,2,3-triazol-4-yl]propyl}carbamoyl) oxy]methyl}phenyl)-N⁵-carbamoyl-L-ornithinamide (Synthon EG)

### 2.6.1 6-(8-(benzo[d]thiazol-2-ylcarbamoyl)-3,4-dihydroisoquinolin-2(1H)-yl)-3-(1-((3-(2-((3-(1-((2R,3R,4S,5S,6S)-6-carboxy-3,4,5-trihydroxytetrahydro-2H-pyran-2-yl)-1H-1,2,3-triazol-4-yl)propyl)amino)ethoxy)-5,7-dimethyladamantan-1-yl)methyl)-5-methyl-1H-pyrazol-4-yl)picolinic acid

To a solution of (2R,3R,4S,5S,6S)-2-azido-6-(methoxycarbonyl)tetrahydro-2H-pyran-3,4,5-triyl triacetate (8.63 mg) in t-butanol (2 mL) and water (1 mL) was added Example 2.5.2 (20 mg), copper(II) sulfate pentahydrate ( 2.0 mg) and sodium ascorbate (5 mg). The mixture was stirred 20 minutes at 100 °C under microwave conditions (Biotage Initiator). Lithium hydroxide monohydrate (50 mg) was added to the mixture, and it was stirred overnight. The mixture was neutralized with trifluoroacetic acid and purified by reverse phase HPLC (Gilson system) eluting with 10-85% acetonitrile in 0.1% trifluoroacetic acid in water, to provide the title compound. MS (ESI) m/e 1032.1 (M+H)⁺.

### 2.6.2 N-[6-(2,5-dioxo-2,5-dihydro-1H-pyrrol-1-yl)hexanoyl]-L-valyl-N-(4-{[([2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl]{3-[1-(beta-D-glucopyranuronosyl)-1H-1,2,3-triazol-4-yl]propyl}carbamoyl)oxy]methyl}phenyl)-N⁵-carbamoyl-L-ornithinamide

The title compound was prepared by substituting Example 2.6.1 for Example 2.5.3 in Example 2.5.4. ¹H NMR (400 MHz, dimethyl sulfoxide-*d*₆) δ ppm 9.64 (s, 1H), 7.98 (d, 1H), 7.90 (s, 1H), 7.76 (d, 1H), 7.68 (s, 1H), 7.52-7.62 (m, 3H), 7.20-7.50 (m, 9H), 6.84-6.98 (m, 3H), 5.56 (d, 1H), 4.98 (d, 4H), 4.36-4.49 (m, 2H), 4.11-4.23 (m, 2H), 3.96 (d, 2H), 3.74-3.91 (m, 7H), 3.51-3.58 (m, 5H), 3.35-3.49 (m, 10H), 2.97-3.02 (m, 6H), 2.57-2.66 (m, 3H), 2.12-2.24 (m, 2H) , 2.08 (s, 3H), 1.69-2.01 (m, 3H), 1.35-1.65 (m, 9H), 0.93-1.28 (m, 10H), 0.81-0.89 (m, 10H). MS (ESI) m/e 1629.4 (M+H)⁺.

### 2.7 Synthesis of N-[6-(2,5-dioxo-2,5-dihydro-1H-pyrrol-1-yl)hexanoyl]-L-valyl-N-{4-[({[(2R)-1-{[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](methyl)amino}-1-oxo-3-sulfopropan-2-yl]carbamoyl}oxy)methyl]phenyl}-L-alaninamide (Synthon EH)

To a solution of Example 1.13.8 (0.018 g) and 4-((S)-2-((S)-2-(6-(2,5-dioxo-2,5-dihydro-1H-pyrrol-1-yl)hexanamido)-3-methylbutanamido)propanamido)benzyl (4-nitrophenyl) carbonate (0.015 g, 0.023 mmol) in N,N-dimethylformamide (0.75 mL) was added N,N-diisopropylethylamine (0.015 mL). After stirring overnight, the reaction was diluted with N,N-dimethylformamide (0.75 mL) and water (0.5 mL). The mixture was purified by reverse phase HPLC using a Gilson system, eluting with 10-70% acetonitrile in water containing 0.1% v/v trifluoroacetic acid. The desired fractions were combined and freeze-dried to provide the title compound. ¹H NMR (500 MHz, dimethyl sulfoxide-*d*₆) δ ppm 12.86 (s, 1H), 9.93 (s, 1H), 8.14 (d, 1H), 8.04 (d, 1H), 7.84-7.76 (m, 2H), 7.61 (d, 1H), 7.57 (d, 2H), 7.53 (dd, 1H), 7.47 (t, 1H), 7.43 (d, 1H), 7.39-7.30 (m, 4H), 7.26 (d, 2H), 6.99 (s, 2H), 6.97 (dd, 1H), 4.96 (s, 2H), 4.90 (t, 2H), 4.75-4.65 (m, 1H), 4.46-4.33 (m, 2H), 4.17 (dd, 2H), 3.66-3.47 (m, 4H), 3.36 (t, 4H), 3.12 (s, 2H), 3.01 (t, 2H), 2.85-2.60 (m, 4H), 2.25-2.05 (m, 5H), 2.05-1.90 (m, 1H), 1.58-0.76 (m, 32H). MS (ESI) m/e 1423.2 (M+H)⁺.

### 2.8 Synthesis of N-[6-(2,5-dioxo-2,5-dihydro-1H-pyrrol-1-yl)hexanoyl]-L-valyl-N-{4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl][4-(beta-D-glucopyranosyloxy)benzyl]carbamoyl}oxy)methyl]phenyl}-N⁵-carbamoyl-L-ornithinamide(Synthon ER)

### 2.8.1 6-(8-(benzo[d]thiazol-2-ylcarbamoyl)-3,4-dihydroisoquinolin-2(1H)-yl)-3-(1-((3,5-dimethyl-7-(2-((4-(((2S,3R,4S,5S,6R)-3,4,5-trihydroxy-6-(hydroxymethyl)tetrahydro-2H-pyran-2-yl)oxy)benzyl)amino)ethoxy)adamantan-1-yl)methyl)-5-methyl-1H-pyrazol-4-yl)picolinic acid

To a solution of Example 1.2.7 (44.5 mg) in tetrahydrofuran (2 mL) and acetic acid (0.2 mL) was added 4-(((2S,3R,4S,5S,6R)-3,4,5-trihydroxy-6-(hydroxymethyl)tetrahydro-2H-pyran-2-yl)oxy)benzaldehyde (17 mg) and MgSO₄ (300 mg). The mixture was stirred for 1 hour before the addition of sodium cyanoborohydride on resin (300 mg). The mixture was stirred overnight. The mixture was filtered, and the solvent was evaporated. The residue was dissolved in dimethyl sulfoxide/methanol (1:1, 4 mL) and purified by reverse phase HPLC (Gilson system), eluting with 10-85% acetonitrile in 0.1% trifluoroacetic acid in water, to give the title compound. MS (ESI) m/e 1015.2 (M+H)⁺.

### 2.8.2 N-[6-(2,5-dioxo-2,5-dihydro-1H-pyrrol-1-yl)hexanoyl]-L-valyl-N-{4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl][4-(beta-D-glucopyranosyloxy)benzyl]carbamoyl}oxy)methyl]phenyl}-N⁵-carbamoyl-L-ornithinamide

The title compound was prepared by substituting Example 2.8.1 for Example 2.5.3 in Example 2.5.4. ¹H NMR (400 MHz, dimethyl sulfoxide-*d*₆) δ ppm 12.87 (s, 1H), 10.00 (s, 1H), 7.96-8.14 (m, 2H) , 7.79 (d, 2H), 7.55-7.68 (m, 3H), 7.09-7.52 (m, 11H), 6.91-7.01 (m, 5H), 5.09 (d, 1H), 4.95 (dd, 4H) , 4.35-4.47 (m, 4H), 4.14-4.23 (m, 3H), 3.86-3.94 (m, 6H), 3.31-3.46 (m, 8H), 3.16-3.25 (m, 3H), 2.90-3.04 (m, 4H), 2.59 (s, 1H), 1.88-2.24 (m, 6H), 0.88-1.75 (m, 24H), 0.76-0.90 (m, 12H). MS (ESI) m/e 1613.7 (M+H)⁺.

### 2.9 Synthesis of N-[6-(2,5-dioxo-2,5-dihydro-1H-pyrrol-1-yl)hexanoyl]-L-valyl-N-{4-[({[4-(beta-D-allopyranosyloxy)benzyl][2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl]carbamoyl}oxy)methyl]phenyl}-N⁵-carbamoyl-L-ornithinamide (Synthon ES)

### 2.9.1 6-(8-(benzo [d]thiazol-2-ylcarbamoyl)-3,4-dihydroisoquinolin-2(1H)-yl)-3-(1-((3,5-dimethyl-7-(2-((4-(((2S,3R,4R,5S,6R)-3,4,5-trihydroxy-6-(hydroxymethyl)tetrahydro-2H-pyran-2-yl)oxy)benzyl)amino)ethoxy)adamantan-1-yl)methyl)-5-methyl-1H-pyrazol-4-yl)picolinic acid

To a solution of Example 1.2.7 (44.5 mg) in tetrahydrofuran (2 mL) and acetic acid (0.2 mL) was added 4-(((2S,3R,4R,5S,6R)-3,4,5-trihydroxy-6-(hydroxymethyl)tetrahydro-2H-pyran-2-yl)oxy)benzaldehyde (17 mg) and MgSO₄ (300 mg). The mixture was stirred for 1 hour before the addition of sodium cyanoborohydride on resin (300 mg). The mixture was stirred overnight. The mixture was filtered, and the solvent was evaporated. The residue was dissolved in dimethyl sulfoxide/methanol (1:1, 4 mL) and purified by reverse phase HPLC (Gilson system), eluting with 10-85% acetonitrile in 0.1% trifluoroacetic acid in water, to give the title compound. MS (ESI) m/e 1015.2 (M+H)⁺.

### 2.9.2 N-[6-(2,5-dioxo-2,5-dihydro-1H-pyrrol-1-yl)hexanoyl]-L-valyl-N-{4-[({[4-(beta-D-allopyranosyloxy)benzyl][2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl]carbamoyl}oxy)methyl]phenyl}-N⁵-carbamoyl-L-ornithinamide

The title compound was prepared by substituting Example 2.9.1 for Example 2.5.3 in Example 2.5.4. ¹H NMR (500 MHz, dimethyl sulfoxide-*d*₆) δ ppm 12.86 (s, 1H), 10.00 (s, 1H), 7.96-8.11 (m, 2H), 7.79 (d, 2H), 7.53-7.65 (m, 3H), 7.08-7.52 (m, 10H), 6.91-7.00 (m, 5H), 5.09 (d, 1H), 4.99 (d, 4H), 4.35-4.48 (m, 3H), 4.13-4.23 (m, 2H), 3.82-3.96 (m, 8H), 3.32-3.50 (m, 10H), 3.12-3.25 (m, 3H), 2.90-3.06 (m, 5H), 1.89-2.19 (m, 6H), 0.88-1.75 (m, 22H), 0.76-0.88 (m, 11H). MS (ESI) m/e 1612.5 (M+H)⁺.

### 2.10 Synthesis of N-[6-(2,5-dioxo-2,5-dihydro-1H-pyrrol-1-yl)hexanoyl]-L-valyl-N-{4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-phosphonoethyl)carbamoyl}oxy)methyl]phenyl}-N⁵-carbamoyl-L-ornithinamide (Synthon EQ)

The title compound was prepared as described in Example 2.1, replacing Example 1.2.9 with Example 1.12.2. ¹H NMR (500 MHz, dimethyl sulfoxide-*d*₆) δ ppm 9.99 (s, 1H), 8.01-8.09 (m, 2H), 7.76-7.81 (m, 2H), 7.56-7.64 (m, 3H), 7.41-7.53 (m, 3H), 7.36 (q, 2H), 7.25-7.30 (m, 3H), 6.99 (s, 2H), 6.94 (d, 1H), 5.98 (s, 1H), 4.89-5.07 (m, 4H), 4.38 (s, 1H), 4.19 (t, 1H), 3.88 (t, 2H), 3.80 (d, 2H), 2.89-3.08 (m, 5H), 2.04-2.24 (m, 5H), 1.89-2.02 (m, 1H), 1.76-1.87 (m, 2H), 0.89-1.72 (m, 23H), 0.78-0.88 (m, 12H). MS (ESI) m/e 1452.2 (M+H)⁺.

### 2.11 Synthesis of N-[6-(2,5-dioxo-2,5-dihydro-1H-pyrrol-1-yl)hexanoyl]-L-valyl-N-{4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-phosphonoethyl)carbamoyl}oxy)methyl]phenyl}-L-alaninamide (Synthon EU)

The title compound was prepared as described in Example 2.1, replacing Example 1.2.9 and 4-((S)-2-((S)-2-(6-(2,5-dioxo-2,5-dihydro-1H-pyrrol-1-yl)hexanamido)-3-methylbutanamido)-5-ureidopentanamido)benzyl (4-nitrophenyl) carbonate with Example 1.12.2 and 4-((S)-2-((S)-2-(6-(2,5-dioxo-2,5-dihydro-1H-pyrrol-1-yl)hexanamido)-3-methylbutanamido)propanamido)benzyl (4-nitrophenyl) carbonate, respectively. ¹H NMR (400 MHz, dimethyl sulfoxide-*d*₆) δ ppm 9.93 (s, 1H), 8.12 (d, 1H), 8.03 (d, 1H), 7.72-7.83 (m, 2H), 7.54-7.65 (m, 3H), 7.41-7.54 (m, 3H), 7.31-7.40 (m, 2H), 7.24-7.30 (m, 3H), 6.99 (s, 2H), 6.94 (d, 1H), 4.87-5.11 (m, 3H), 4.11-4.45 (m, 1H), 3.88 (t, 2H), 3.79 (d, 2H), 2.97-3.05 (m, 2H), 2.63-2.70 (m, 1H), 2.29-2.37 (m, 1H), 2.03-2.20 (m, 5H), 1.73-2.00 (m, 5H), 1.39-1.55 (m, 4H), 0.88-1.38 (m, 19H), 0.72-0.89 (m, 12H). MS (ESI) m/e 1364.5 (M-H)⁻.

### 2.12 Synthesis of N-[6-(2,5-dioxo-2,5-dihydro-1H-pyrrol-1-yl)hexanoyl]-L-valyl-N-{4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](3-phosphonopropyl)carbamoyl}oxy)methyl]phenyl}-N⁵-carbamoyl-L-ornithinamide (Synthon EV)

The title compound was prepared as described in Example 2.1, replacing Example 1.2.9 with Example 1.14.4. ¹H NMR (400 MHz, dimethyl sulfoxide-*d*₆) δ ppm 9.98 (s, 1H), 8.04 (t, 2H), 7.78 (t, 2H), 7.61 (t, 3H), 7.39-7.54 (m, 3H), 7.32-7.39 (m, 2H), 7.25-7.30 (m, 3H), 6.99 (s, 2H), 6.95 (d, 1H), 6.01 (s, 1H), 4.97 (d, 4H), 4.29-4.47 (m, 2H), 4.14-4.23 (m, 2H), 3.85-3.93 (m, 2H), 3.32-3.42 (m, 2H), 3.24 (s, 2H), 2.88-3.09 (m, 3H), 1.87-2.23 (m, 6H), 0.91-1.74 (m, 27H), 0.72-0.89 (m, 12H). MS (ESI) m/e 1466.3 (M+H)⁺.

### 2.13 Synthesis of N-[6-(2,5-dioxo-2,5-dihydro-1H-pyrrol-1-yl)hexanoyl]-L-valyl-N-{4-[({[(2R)-1-{[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl]amino}-1-oxo-3-sulfopropan-2-yl]carbamoyl}oxy)methyl]phenyl}-L-alaninamide (Synthon EW)

To a solution of Example 1.15 (0.020 g) and 4-((S)-2-((S)-2-(6-(2,5-dioxo-2,5-dihydro-1H-pyrrol-1-yl)hexanamido)-3-methylbutanamido)propanamido)benzyl (4-nitrophenyl) carbonate (0.017 g) in N,N-dimethylformamide (0.5 mL) was added N,N-diisopropylethylamine (0.017 mL). The reaction was stirred overnight and was diluted with N,N-dimethylformamide (1 mL), water (0.5 mL). The mixture was purified by reverse phase HPLC using a Gilson system, eluting with 10-70% acetonitrile in water containing 0.1% v/v trifluoroacetic acid. The desired fractions were combined and freeze-dried to provide the title compound. ¹H NMR (400 MHz, dimethyl sulfoxide-*d*₆) δ ppm 12.85 (s, 1H), 9.93 (s, 1H), 8.12 (d, 1H), 8.04 (d, 1H), 7.86-7.76 (m, 3H), 7.63-7.41 (m, 7H), 7.39-7.32 (m, 2H), 7.30 (s, 1H), 7.30-7.21 (m, 2H), 6.99 (s, 2H), 6.97 (d, 1H), 4.96 (s, 2H), 4.93 (s, 2H), 4.49-4.33 (m, 2H), 4.18 (dd, 2H), 4.15-4.08 (m, 2H), 3.90-3.86 (m, 2H), 3.36 (t, 2H), 3.34-3.27 (m, 1H), 3.18-3.04 (m, 2H), 3.04-2.96 (m, 2H), 2.89-2.61 (m, 2H), 2.27-2.05 (m, 5H), 2.03-1.87 (m, 1H), 1.59-1.42 (m, 4H), 1.42-0.91 (m, 18H), 0.91-0.76 (m, 11H). MS (-ESI) m/e 1407.5 (M-H)⁻.

### 2.14 Synthesis of N-[6-(2,5-dioxo-2,5-dihydro-1H-pyrrol-1-yl)hexanoyl]-L-valyl-N-[4-({[{2-[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethoxy]ethyl}(3-phosphonopropyl)carbamoyl]oxy}methyl)phenyl]-N⁵-carbamoyl-L-ornithinamide (Synthon EX)

A mixture of Example 1.16.2 (59 mg), 4-((S)-2-((S)-2-(6-(2,5-dioxo-2,5-dihydro-1H-pyrrol-1-yl)hexanamido)-3 -methylbutanamido)-5 -ureidopentanamido)benzyl (4-nitrophenyl) carbonate (48 mg), and N,N-diisopropylethylamine (0.056 mL) in 2 mL N,N-dimethylformamide was stirred for 24 hours. The mixture was purified via reverse phase chromatography on a Biotage Isolera One system using a 40 g C18 column, eluting with 10-90% acetonitrile in 0.1% trifluoroacetic acid/water. The desired fractions were concentrated and the product was lyophilized from water and 1,4-dioxane to give the title compound as a trifluoroacetic acid salt. ¹H NMR (400MHz, dimethyl sulfoxide-*d*₆) δ ppm 9.97 (bs, 1H), 8.04 (m, 2H), 7.79 (d, 2H), 7.59 (m, 3H), 7.46 (m, 3H), 7.36 (m, 2H), 7.27 (m, 2H), 6.99 (s, 2H), 6.94 (d, 1H), 4.97 (m, 4H), 4.40 (m, 2H), 4.17 (dd, 2H), 3.50-4.10 (m, 6H), 3.45 (m, 2H), 3.40 (m, 2H), 3.26 (m, 2H), 3.01 (m, 2H), 2.95 (s, 2H), 2.79 (s, 2H), 2.15 (m, 2H), 2.09 (s, 2H), 1.68 (m, 2H), 1.60 (m, 1-2H), 1.35-1.50 (m, 6H), 1.25 (m, 4H), 1.17 (m, 2H), 1.10 (m, 2H), 0.97 (m, 1-2H), 0.84 (m, 12H). MS (ESI) m/e 1510.4 (M+H)⁺.

### 2.15 Synthesis of N-[6-(2,5-dioxo-2,5-dihydro-1H-pyrrol-1-yl)hexanoyl]-L-valyl-N-[4-({[{2-[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethoxy]ethyl}(3-phosphonopropyl)carbamoyl]oxy}methyl) phenyl]-L-alaninamide (Synthon EY)

A mixture of Example 1.16.2 (59 mg), 4-((S)-2-((S)-2-(6-(2,5-dioxo-2,5-dihydro-1H-pyrrol-1-yl)hexanamido)-3-methylbutanamido)propanamido)benzyl (4-nitrophenyl) carbonate (42 mg), and N,N-diisopropylethylamine (0.042 mg) in 2 mL N,N-dimethylformamide was stirred for 24 hours. The mixture was purified via reverse phase chromatography on a Biotage Isolera One system using a 40 g C18 column, eluting with 10-90% acetonitrile in 0.1% trifluoroacetic acid/water. Fractions were concentrated and the product was lyophilized from water and 1,4-dioxane to give the title compound as a trifluoroacetic acid salt. MS (ESI) m/e 1422.6 (M-H)⁺.

### 2.16 Synthesis of N-[6-(2,5-dioxo-2,5-dihydro-1H-pyrrol-1-yl)hexanoyl]-L-valyl-N-{4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](3-phosphonopropyl)carbamoyl}oxy)methyl]phenyl}-L-alaninamide (Synthon EZ)

A mixture of Example 1.14.4 (50 mg), 4-((S)-2-((S)-2-(6-(2,5-dioxo-2,5-dihydro-1H-pyrrol-1-yl)hexanamido)-3-methylbutanamido)propanamido)benzyl (4-nitrophenyl) carbonate (38 mg), and N,N-diisopropylethylamine (0.050 mL) in 2 mL N,N-dimethylformamide was stirred for 24 hours. The mixture was purified via reverse phase chromatography on a Biotage Isolera One system using a 40 g C18 column, eluting with 10-90% acetonitrile in 0.1% trifluoroacetic acid/water. The desired fractions were concentrated and the product was lyophilized from water and 1,4-dioxane to give the title compound as a trifluoroacetic acid salt. ¹H NMR (400MHz, dimethyl sulfoxide-*d*₆) δ ppm 9.94 (bs, 1H), 8.12 (d, 1H), 8.04 (d, 1H), 7.80 (d, 2H), 7.61 (m, 3H), 7.47 (m, 3H), 7.36 (m, 2H), 7.29 (m, 2H), 6.99 (s, 2H), 6.95 (d, 1H), 4.97 (m, 4H), 4.40 (m, 2H), 4.16 (dd, 2H), 3.50-4.10 (m, 6H), 3.68 (m, 2H), 3.55 (m, 2H), 3.25 (m, 4H), 3.02 (m, 2H), 2.94 (s, 2H), 2.79 (s, 2H), 2.15 (m, 1H), 2.08 (s, 2H), 1.65 (m, 2H), 1.40-1.50 (m, 6H), 1.20-1.30 (m, 6H), 1.08-1.19 (m, 4H), 0.97 (m, 1-2H), 0.76-0.89 (m, 12H). MS (ESI) m/e 1380.3 (M+H)⁺.

### 2.17 Synthesis of 6-[8-(1,3-benzothiazol-2-ylcarbamoyl)-3,4-dihydroisoquinolin-2(1H)-yl]-3-(1-{[3-(2-{[(2S)-3-carboxy-2-({[(4-{[(2S)-2-{[(2S)-2-{[6-(2,5-dioxo-2,5-dihydro-1H-pyrrol-1-yl)hexanoyl]amino}-3-methylbutanoyl]amino}propanoyl]amino}benzyl)oxy]carbonyl}amino)pr opanoyl](methyl)amino}ethoxy)-5,7-dimethyltricyclo[3.3.1.1^{3,7}]dec-1-yl]methyl}-5-methyl-1H-pyrazol-4-yl)pyridine-2-carboxylic acid (Synthon FD)

To a solution of Example 1.17 (0.040 g) and 4-((S)-2-((S)-2-(6-(2,5-dioxo-2,5-dihydro-1H-pyrrol-1-yl)hexanamido)-3-methylbutanamido)propanamido)benzyl (4-nitrophenyl) carbonate (0.034 g) in N,N-dimethylformamide (1 mL) was added N,N-diisopropylethylamine (0.035 mL). The reaction was stirred overnight and diluted with N,N-dimethylformamide (1 mL) and water (0.5 mL). The mixture was purified by reverse phase HPLC using a Gilson system, eluting with 10-70% acetonitrile in water containing 0.1% v/v trifluoroacetic acid. The desired fractions were combined and freeze-dried to provide the title compound. ¹H NMR (400 MHz, dimethyl sulfoxide-*d*₆) δ ppm 12.84 (s, 1H), 9.92 (s, 1H), 8.13 (d, 1H), 8.03 (d, 1H), 7.79 (d, 2H), 7.62 (d, 1H), 7.57 (d, 2H), 7.54-7.41 (m, 3H), 7.40-7.32 (m, 2H), 7.31-7.23 (m, 4H), 6.99 (s, 2H), 6.95 (dd, 1H), 5.01-4.89 (m, 4H), 4.78 (dq, 1H), 4.45-4.30 (m, 1H), 4.23-4.11 (m, 1H), 3.88 (t, 2H), 3.80 (s, 2H), 3.42-3.26 (m, 6H), 3.06 (s, 1H), 3.01 (t, 2H), 2.80 (s, 2H), 2.76-2.62 (m, 1H), 2.46-2.36 (m, 1H), 2.25-2.05 (m, 5H), 2.05-1.92 (m, 1H), 1.58-1.42 (m, 4H), 1.42-0.91 (m, 20H), 0.91-0.78 (m, 9H). MS (ESI) m/e 1387.4 (M+H)⁺.

### 2.18 Synthesis of N-[6-(2,5-dioxo-2,5-dihydro-1H-pyrrol-1-yl)hexanoyl]-L-valyl-N-{4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl][4-(beta-D-glucopyranuronosyloxy)benzyl]carbamoyl}oxy)methyl]phenyl}-N⁵-carbamoyl-L-ornithinamide (Synthon FS)

The title compound was prepared by substituting Example 1.19.2 for Example 2.5.3 in Example 2.5.4. ¹H NMR (500 MHz, dimethyl sulfoxide-*d*₆) δ ppm 12.86 (s, 1H), 10.00 (s, 1H), 7.97-8.14 (m, 2H), 7.79 (d, 2H), 7.07-7.65 (m, 13H), 6.87-7.01 (m, 4H), 5.92-6.08 (m, 1H), 4.87-5.07 (m, 4H), 4.33-4.48 (m, 3H), 4.13-4.26 (m, 1H), 3.74-3.94 (m, 6H), 3.14-3.34 (m, 8H), 2.84-3.05 (m, 6H), 1.87-2.25 (m, 6H), 0.89-1.73 (m, 21H), 0.76-0.87 (m, 12H). MS (ESI) m/e 1626.4 (M+H)⁺.

### 2.19 Synthesis of N-[6-(2,5-dioxo-2,5-dihydro-1H-pyrrol-1-yl)hexanoyl]-L-valyl-N-{4-[({[2-({3-[(4-{6-[1-(1,3-benzothiazol-2-ylcarbamoyl)-1,2,3,4-tetrahydroquinolin-7-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-phosphonoethyl)carbamoyl}oxy)methyl]phenyl}-N⁵-carbamoyl-L-ornithinamide (Synthon FI)

The title compound was prepared as described in Example 2.1, replacing Example 1.2.9 with Example 1.20.11. ¹H NMR (500 MHz, dimethyl sulfoxide-*d*₆) δ ppm 10.00 (s, 1H), 8.40 (s, 1H), 8.07 (d, 1H), 8.00 (d, 1H), 7.84-7.90 (m, 1H), 7.79 (dd, 3H), 7.55-7.66 (m, 2H), 7.46 (s, 2H), 7.37 (t, 1H), 7.29 (t, 3H), 7.18-7.25 (m, 1H), 6.99 (s, 2H), 5.99 (s, 1H), 5.00 (d, 1H), 4.38 (s, 1H), 4.13-4.24 (m, 1H), 3.96 (s, 2H), 3.87 (d, 2H), 2.88-3.08 (m, 4H), 2.84 (q, 2H), 2.04-2.26 (m, 5H), 1.89-2.01 (m, 3H), 1.75-1.88 (m, 2H), 1.63-1.74 (m, 1H), 0.91-1.63 (m, 21H), 0.76-0.89 (m, 12H). MS (ESI) m/e 1450.5 (M-H)⁻.

### 2.20 Synthesis of N-[6-(2,5-dioxo-2,5-dihydro-1H-pyrrol-1-yl)hexanoyl]-L-valyl-N⁵-carbamoyl-N-{4-[({[2-({3-[(4-{2-carboxy-6-[8-([1,3]thiazolo[5,4-b]pyridin-2-ylcarbamoyl)-3,4-dihydroisoquinolin-2(1H)-yl]pyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)methyl]phenyl}-L-ornithinamide (Synthon FV)

The title compound was prepared by substituting Example 1.22.5 for Example 1.2.9 in Example 2.1. ¹H NMR (500 MHz, dimethyl sulfoxide-*d*₆) δ ppm 13.00 (v br s, 1H), 10.00 (s, 1H), 8.52 (dd, 1H), 8.16 (dd, 1H), 8.06 (d, 1H), 7.78 (d, 1H), 7.62 (d, 1H), 7.59 (br m, 2H), 7.53 (m, 2H), 7.45 (d, 1H), 7.37 (t, 1H), 7.30 (s, 1H) 7.27 (d, 2H), 6.99 (s, 2H), 6.97 (d, 1H), 4.98 (m, 4H), 4.39 (m, 1H), 4.19 (br m, 1H), 3.88 (t, 2H), 3.80 (br d, 2H), 3.44, 3.36 (br m, m, total 6H), 3.24 (m, 2H), 2.94-3.01 (m, 4H), 2.63 (br m, 2H), 2.14 (m, 2H), 2.10 (s, 3H), 1.97 (br m, 1H), 1.68 (br m, 1H), 1.58 (br m, 1H), 1.34-1.47 (m, 8H), 1.08-1.23 (m 10H), 0.95 (br m, 2H), 0.85-0.80 (m, 12H). MS (ESI) m/e 1451.4 (M-H)⁻.

### 2.21 Synthesis of N-[6-(2,5-dioxo-2,5-dihydro-1H-pyrrol-1-yl)hexanoyl]-L-valyl-N-{4-[({[(2R)-1-{[2-({3-[(4-{6-[1-(1,3-benzothiazol-2-ylcarbamoyl)-1,2,3,4-tetrahydroquinolin-7-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](methyl)amino}-1-oxo-3-sulfopropan-2-yl]carbamoyl}oxy)methyl]phenyl}-N⁵-carbamoyl-L-ornithinamide (Synthon GC)

The title compound was prepared as described in Example 2.1, replacing Example 1.2.9 with Example 1.21.7. ¹H NMR (500 MHz, dimethyl sulfoxide-*d*₆) δ ppm 9.98 (s, 1H), 8.40 (s, 1H), 8.07 (d, 1H), 8.01 (dd, 1H), 7.89 (t, 1H), 7.74-7.84 (m, 3H), 7.58 (d, 2H), 7.47 (s, 2H), 7.37 (t, 1H), 7.19-7.33 (m, 5H), 7.00 (s, 2H), 4.91 (q, 2H), 4.64-4.76 (m, 2H), 4.33-4.43 (m, 2H), 4.15-4.24 (m, 2H), 3.92-4.03 (m, 2H), 3.88 (s, 2H), 3.32-3.50 (m, 6H), 3.10-3.22 (m, 2H), 2.89-3.07 (m, 2H), 2.70-2.89 (m, 4H), 2.60-2.70 (m, 1H), 2.05-2.28 (m, 5H), 1.90-2.03 (m, 3H), 1.64-1.77 (m, 1H), 1.53-1.65 (m, 1H), 0.92-1.53 (m, 21H), 0.77-0.92 (m, 12H). MS (ESI) m/e 1507.3 (M-H)⁻.

### 2.22 Synthesis of N-[6-(2,5-dioxo-2,5-dihydro-1H-pyrrol-1-yl)hexanoyl]-L-valyl-N-{4-[({[(2R)-1-{[2-({3-[(4-{6-[1-(1,3-benzothiazol-2-ylcarbamoyl)-1,2,3,4-tetrahydroquinolin-7-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](methyl)amino}-1-oxo-3-sulfopropan-2-yl]carbamoyl}oxy)methyl]phenyl}-L-alaninamide (Synthon GB)

The title compound was prepared as described in Example 2.1, replacing Example 1.2.9 and 4-((S)-2-((S)-2-(6-(2,5-dioxo-2,5-dihydro-1H-pyrrol-1-yl)hexanamido)-3-methylbutanamido)-5-ureidopentanamido)benzyl (4-nitrophenyl) carbonate with Example 1.21.7 and 4-((S)-2-((S)-2-(6-(2,5-dioxo-2,5-dihydro-1H-pyrrol-1-yl)hexanamido)-3-methylbutanamido)propanamido)benzyl (4-nitrophenyl) carbonate, respectively. ¹H NMR (500 MHz, dimethyl sulfoxide-*d*₆) δ ppm 9.93 (s, 1H), 8.39 (s, 1H), 8.13 (d, 1H), 8.01 (dd, 1H), 7.88 (t, 1H), 7.74-7.84 (m, 3H), 7.57 (d, 2H), 7.46 (s, 2H), 7.37 (t, 1H), 7.17-7.33 (m, 5H), 6.99 (s, 2H), 4.91 (d, 2H), 4.65-4.76 (m, 1H), 4.30-4.51 (m, 1H), 4.13-4.21 (m, 1H), 3.92-4.00 (m, 2H), 3.88 (s, 2H), 3.29-3.46 (m, 4H), 2.93-3.21 (m, 3H), 2.68-2.88 (m, 4H), 2.58-2.68 (m, 1H), 2.04-2.26 (m, 5H), 1.89-2.02 (m, 3H), 1.37-1.54 (m, 6H), 0.92-1.34 (m, 15H), 0.75-0.91 (m, 12H). MS (ESI) m/e (M+H)⁺.

### 2.23 Synthesis of N-[6-(2,5-dioxo-2,5-dihydro-1H-pyrrol-1-yl)hexanoyl]-L-valyl-N⁵-carbamoyl-N-{4-[({[2-({3-[(4-{2-carboxy-6-[8-([1,3]thiazolo[4,5-b]pyridin-2-ylcarbamoyl)-3,4-dihydroisoquinolin-2(1H)-yl]pyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)methyl]phenyl}-L-ornithinamide (Synthon FW)

The title compound was prepared by substituting Example 1.23.4 for Example 1.2.9 in Example 2.1. ¹H NMR (500 MHz, dimethyl sulfoxide-*d*₆) δ ppm 13.38 (v br s, 1H), 10.00 (s, 1H), 8.66 (m, 2H), 8.06 (d, 1H), 7.78 (d, 1H), 7.65 (d, 1H), 7.59 (br m, 2H), 7.53 (m, 1H), 7.47 (m 2H), 7.37 (t, 1H), 7.30 (s, 1H) 7.27 (d, 2H), 6.99 (s, 2H), 6.97 (d, 1H), 4.98 (m, 4H), 4.39 (m, 1H), 4.19 (br m, 1H), 3.88 (t, 2H), 3.80 (br d, 2H), 3.40 (br m, 6H), 3.24 (m, 2H), 2.98 (m, 4H), 2.63 (m, 2H), 2.16 (m, 2H), 2.10 (s, 3H), 1.97 (br m, 1H), 1.68 (br m, 1H), 1.58 (br m, 1H), 1.34-1.47(m, 8H), 1.08-1.23 (m, 10H), 0.95 (br m, 2H), 0.85-0.80 (m, 12H). MS (ESI) m/e 1451.5 (M-H)⁻.

### 2.24 Synthesis of N-[6-(2,5-dioxo-2,5-dihydro-1H-pyrrol-1-yl)hexanoyl]-L-valyl-N-{4-[({[2-({3-[(4-{6-[1-(1,3-benzothiazol-2-ylcarbamoyl)-1,2,3,4-tetrahydroquinolin-7-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)methyl]phenyl}-N⁵-carbamoyl-L-ornithinamide (Synthon GD)

The title compound was prepared as described in Example 2.1, replacing Example 1.2.9 with Example 1.24.2. ¹H NMR (500 MHz, dimethyl sulfoxide-*d*₆) δ ppm 10.00 (s, 1H), 8.38 (s, 1H), 8.07 (d, 1H), 8.00 (d, 1H), 7.85-7.92 (m, 1H), 7.73-7.85 (m, 3H), 7.55-7.65 (m, 2H), 7.46 (s, 2H), 7.37 (t, 1H), 7.28 (t, 3H), 7.22 (t, 1H), 6.99 (s, 2H), 6.00 (s, 1H), 4.99 (d, 1H), 4.28-4.50 (m, 1H), 4.19 (s, 1H), 3.77-4.03 (m, 4H), 3.31-3.41 (m, 2H), 3.20-3.29 (m, 2H), 2.87-3.08 (m, 3H), 2.83 (t, 2H), 2.63 (d, 2H), 2.05-2.25 (m, 5H), 1.88-2.01 (m, 3H), 1.69 (t, 1H), 1.53-1.63 (m, 1H), 1.31-1.53 (m, 8H), 1.04-1.29 (m, 11H), 0.89-1.02 (m, 2H), 0.77-0.88 (m, 12H). MS (ESI) m/e 1450.4 (M-H)⁻.

### 2.25 Synthesis of N-[6-(2,5-dioxo-2,5-dihydro-1H-pyrrol-1-yl)hexanoyl]-L-valyl-N-{4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-carboxyethyl)carbamoyl}oxy)methyl]phenyl}-N⁵-carbamoyl-L-ornithinamide (Synthon GK)

The title compound was prepared as described in Example 2.1, replacing Example 1.2.9 with Example 1.25.2. ¹H NMR (400 MHz, dimethyl sulfoxide-*d*₆) δ ppm 12.85 (s, 1H), 9.98 (s, 1H), 8.04 (t, 2H), 7.75-7.82 (m, 2H), 7.60 (t, 3H), 7.41-7.53 (m, 3H), 7.32-7.39 (m, 2H), 7.24-7.29 (m, 3H), 6.99 (s, 2H), 6.94 (d, 3H), 5.97 (s, 1H), 4.88-5.04 (m, 4H), 4.38 (d, 1H), 4.12-4.24 (m, 1H), 3.88 (t, 2H), 3.75-3.84 (m, 2H), 3.32-3.40 (m, 2H), 3.28 (d, 2H), 2.90-3.05 (m, 4H), 2.42-2.49 (m, 2H), 2.05-2.22 (m, 5H), 1.87-2.01 (m, 1H), 0.90-1.76 (m, 22H), 0.74-0.88 (m, 12H). MS (ESI) m/e 1414.5 (M-H)⁻.

### 2.26 Synthesis of N-[6-(2,5-dioxo-2,5-dihydro-1H-pyrrol-1-yl)hexanoyl]-L-valyl-N-{4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-carboxyethyl)carbamoyl}oxy)methyl]phenyl}-L-alaninamide (Synthon GJ)

The title compound was prepared as described in Example 2.1, replacing Example 1.2.9 and 4-((S)-2-((S)-2-(6-(2,5-dioxo-2,5-dihydro-1H-pyrrol-1-yl)hexanamido)-3-methylbutanamido)-5-ureidopentanamido)benzyl (4-nitrophenyl) carbonate with Example 1.25.2 and 4-((S)-2-((S)-2-(6-(2,5-dioxo-2,5-dihydro-1H-pyrrol-1-yl)hexanamido)-3-methylbutanamido)propanamido)benzyl (4-nitrophenyl) carbonate, respectively. ¹H NMR (400 MHz, dimethyl sulfoxide-*d*₆) δ ppm 12.78 (s, 1H), 9.93 (s, 1H), 8.12 (d, 1H), 8.03 (d, 1H), 7.75-7.83 (m, 2H), 7.54-7.65 (m, 3H), 7.41-7.52 (m, 3H), 7.32-7.40 (m, 2H), 7.24-7.29 (m, 3H), 6.98 (s, 2H), 6.94 (d, 1H), 4.90-5.04 (m, 4H), 4.32-4.45 (m, 2H), 4.12-4.21 (m, 2H), 3.88 (t, 2H), 3.79 (d, 2H), 3.31-3.46 (m, 4H), 3.23-3.31 (m, 2H), 3.01 (t, 2H), 2.46 (t, 2H), 2.04-2.22 (m, 5H), 1.87-2.02 (m, 1H), 1.40-1.60 (m, 4H), 0.91-1.37 (m, 17H), 0.76-0.88 (m, 12H). MS (ESI) m/e 1328.4 (M-H)⁻.

### 2.27 Synthesis of 6-[8-(1,3-benzothiazol-2-ylcarbamoyl)-3,4-dihydroisoquinolin-2(1H)-yl]-3-(1-{[3-(2-{[(2R)-3-carboxy-2-({[(4-{[(2S)-2-{[(2S)-2-{[6-(2,5-dioxo-2,5-dihydro-1H-pyrrol-1-yl)hexanoyl]amino}-3-methylbutanoyl]amino}propanoyl]amino}benzyl)oxy]carbonyl}amino)pr opanoyl](methyl)amino}ethoxy)-5,7-dimethyltricyclo[3.3.1.1^{3,7}]dec-1-yl]methyl}-5-methyl-1H-pyrazol-4-yl)pyridine-2-carboxylic acid (Synthon GW)

A solution of Example 1.27 (0.043 g) in N,N-dimethylformamide (0.5 mL) was added 4-((S)-2-((S)-2-(6-(2,5-dioxo-2,5-dihydro-1H-pyrrol-1-yl)hexanamido)-3-methylbutanamido)propanamido)benzyl (4-nitrophenyl) carbonate (0.042 g) followed by N,N-diisopropylethylamine (0.038 mL), and the reaction was stirred at room temperature. After stirring for 16 hours, the reaction was diluted with water (0.5 mL) and N,N-dimethylformamide (1 mL). The mixture was purified by reverse phase HPLC using a Gilson system, eluting with 10-70% acetonitrile in water containing 0.1% v/v trifluoroacetic acid. The desired fractions were combined and freeze-dried to provide the title compound. ¹H NMR (400 MHz, dimethyl sulfoxide-*d*₆) δ ppm 13.05 (s, 1H), 10.15 (s, 1H), 8.36 (d, 1H), 8.26 (d, 1H), 8.02 (d, 2H), 7.95-7.77 (m, 4H), 7.77-7.63 (m, 3H), 7.63-7.54 (m, 2H), 7.54-7.46 (m, 3H), 7.22 (s, 2H), 7.18 (dd, 1H), 5.17 (d, 4H), 5.01 (dq, 1H), 4.61 (p, 1H), 4.39 (t, 1H), 4.11 (t, 2H), 4.03 (s, 2H), 3.64-3.49 (m, 2H), 3.29 (s, 1H), 3.24 (t, 2H), 3.03 (s, 2H), 2.92 (dt, 1H), 2.73-2.61 (m, 4H), 2.35 (d, 4H), 2.18 (dt, 1H), 1.71 (h, 4H), 1.65-1.13 (m, 18H), 1.13-1.01 (m, 13H). MS (ESI) m/e 1387.3 (M+H)⁺.

### 2.28 Synthesis of N-[6-(2,5-dioxo-2,5-dihydro-1H-pyrrol-1-yl)hexanoyl]-L-valyl-N-{4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl][1-(carboxymethyl)piperidin-4-yl]carbamoyl}oxy)methyl]phenyl}-N⁵-carbamoyl-L-ornithinamide (Synthon HF)

A solution of Example 1.28 (0.0449 g), 4-((S)-2-((S)-2-(6-(2,5-dioxo-2,5-dihydro-1H-pyrrol-1-yl)hexanamido)-3-methylbutanamido)-5-ureidopentanamido)benzyl (4-nitrophenyl) carbonate (0.049 g) and N,N-diisopropylethylamine (0.044 mL) were stirred together in N,N-dimethylformamide (0.5 mL) at room temperature. The reaction mixture was stirred overnight and diluted with N,N-dimethylformamide (1 mL) and water (0.5 mL). The mixture was purified by reverse phase HPLC using a Gilson system, eluting with 10-90% acetonitrile in water containing 0.1% v/v trifluoroacetic acid. The desired fractions were combined and freeze-dried to provide the title compound. ¹H NMR (400 MHz, dimethyl sulfoxide-*d*₆) δ ppm 12.85 (s, 1H), 9.99 (s, 1H), 8.04 (t, 2H), 7.78 (t, 2H), 7.65-7.58 (m, 3H), 7.54-7.41 (m, 3H), 7.38 (d, 1H), 7.34 (d, 1H), 7.32-7.24 (m, 3H), 6.99 (s, 2H), 6.95 (d, 1H), 5.97 (s, 1H), 5.01 (s, 2H), 4.96 (s, 2H), 4.38 (q, 1H), 4.23-4.14 (m, 1H), 4.05 (s, 2H), 3.88 (t, 2H), 3.80 (s, 2H), 3.36 (t, 2H), 3.26-2.86 (m, 8H), 2.27-2.02 (m, 6H), 2.02-1.86 (m, 2H), 1.86-1.75 (m, 2H), 1.75-1.54 (m, 2H), 1.54-0.90 (m, 24H), 0.89-0.72 (m, 14H). MS (ESI) m/e 1485.2 (M+H)⁺.

### 2.29 Synthesis of (S)-6-((2-((3-((4-(6-(8-(benzo[d]thiazol-2-ylcarbamoyl)-3,4-dihydroisoquinolin-2(1H)-yl)-2-carboxypyridin-3-yl)-5-methyl-1H-pyrazol-1-yl)methyl)-5,7-dimethyladamantan-1-yl)oxy)ethyl)(methyl)amino)-5-((((4-((S)-2-((S)-2-(6-(2,5-dioxo-2,5-dihydro-1H-pyrrol-1-yl)hexanamido)-3-methylbutanamido)-5-ureidopentanamido)benzyl)oxy)carbonyl)amino)-N,N,N-trimethyl-6-oxohexan-1-aminium salt (Synthon HG)

A solution of Example 1.29 (8 mg), 4-((S)-2-((S)-2-(6-(2,5-dioxo-2,5-dihydro-1H-pyrrol-1-yl)hexanamido)-3-methylbutanamido)-5-ureidopentanamido)benzyl (4-nitrophenyl) carbonate (8.24 mg) and N,N-diisopropylethylamine (7.50 µl, 0.043 mmol) in N,N-dimethylformamide (0.250 mL) was stirred at room temperature. After 3 hours, the reaction was diluted with N,N-dimethylformamide (1.25 mL) and water (0.5 mL). The mixture was purified by reverse phase HPLC using a Gilson system, eluting with 10-90% acetonitrile in water containing 0.1% v/v trifluoroacetic acid. The desired fractions were combined and freeze-dried to provide the title compound. ¹H NMR (400 MHz, dimethyl sulfoxide-*d*₆) δ ppm 12.85 (s, 1H), 9.96 (s, 1H), 8.04 (t, 2H), 7.83-7.76 (m, 2H), 7.66-7.56 (m, 3H), 7.53-7.42 (m, 4H), 7.41-7.32 (m, 2H), 7.31-7.23 (m, 3H), 6.99 (s, 2H), 6.95 (d, 1H), 5.99 (s, 1H), 5.04-4.87 (m, 4H), 4.44-4.33 (m, 2H), 4.24-4.12 (m, 2H), 3.88 (t, 2H), 3.81 (s, 2H), 3.50-3.13 (m, 9H), 3.11-2.92 (m, 14H), 2.80 (s, 1H), 2.25-2.04 (m, 5H), 2.03-1.89 (m, 1H), 1.75-0.91 (m, 28H), 0.91-0.77 (m, 12H). MS (ESI) m/e 1528.5 (M+H)⁺.

### 2.30 Synthesis of N-[6-(2,5-dioxo-2,5-dihydro-1H-pyrrol-1-yl)hexanoyl]-L-valyl-N-{4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)methyl]phenyl}-L-alaninamide (Synthon HP)

The title compound was prepared as described in Example 2.1, replacing 4-((S)-2-((S)-2-(6-(2,5-dioxo-2,5-dihydro-1H-pyrrol-1-yl)hexanamido)-3-methylbutanamido)-5-ureidopentanamido)benzyl (4-nitrophenyl) carbonate with 4-((S)-2-((S)-2-(6-(2,5-dioxo-2,5-dihydro-1H-pyrrol-1-yl)hexanamido)-3-methylbutanamido)propanamido)benzyl (4-nitrophenyl) carbonate. ¹H NMR (400 MHz, dimethyl sulfoxide-*d*₆) δ ppm 12.83 (s, 1H), 9.94 (s, 1H), 8.12 (d, 1H), 8.04 (d, 1H), 7.79 (d, 2H), 7.40-7.63 (m, 6H), 7.32-7.39 (m, 2H), 7.24-7.30 (m, 3H), 6.99 (s, 2H), 6.95 (d, 1H), 4.90-5.03 (m, 4H), 4.31-4.47 (m, 1H), 4.09-4.24 (m, 1H), 3.84-3.93 (m, 2H), 3.81 (s, 2H), 3.30-3.39 (m, 2H), 3.20-3.28 (m, 2H), 3.01 (t, 2H), 2.57-2.65 (m, 2H), 2.05-2.22 (m, 5H), 1.87-2.02 (m, 2H), 1.41-1.58 (m, 4H), 1.22 (d, 18H), 0.74-0.89 (m, 12H). MS (ESI) m/e 1364.5 (M-H)⁻.

### 2.31 Synthesis of N-[6-(2,5-dioxo-2,5-dihydro-1H-pyrrol-1-yl)hexanoyl]-L-valyl-N-[4-({[(4-{[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)aminolpiperidin-1-yl)carbonyl]oxy}methyl)phenyl]-N⁵-carbamoyl-L-ornithinamide (Synthon HR)

A solution of Example 1.30.2 (0.038 g), 4-((S)-2-((S)-2-(6-(2,5-dioxo-2,5-dihydro-1H-pyrrol-1-yl)hexanamido)-3-methylbutanamido)-5-ureidopentanamido)benzyl (4-nitrophenyl) carbonate (0.035 g) and N,N-diisopropylethylamine (0.032 mL) in N,N-dimethylformamide (0.5 mL) was stirred at room temperature. After stirring for 3 hours, the reaction was diluted with N,N-dimethylformamide (1.25 mL) and water (0.5 mL). The mixture was purified by reverse phase HPLC using a Gilson system, eluting with 10-90% acetonitrile in water containing 0.1% v/v trifluoroacetic acid. The desired fractions were combined and freeze-dried to provide the title compound. ¹H NMR (400 MHz, dimethyl sulfoxide-*d*₆) δ ppm 9.98 (s, 1H), 9.02 (s, 1H), 8.10-8.00 (m, 2H), 7.79 (d, 2H), 7.64-7.56 (m, 3H), 7.53 (d, 1H), 7.47 (t, 1H), 7.43 (d, 1H), 7.39-7.32 (m, 2H), 7.29 (d, 3H), 6.99 (s, 2H), 6.95 (d, 1H), 6.00 (s, 1H), 4.99 (s, 2H), 4.96 (s, 2H), 4.48-4.32 (m, 2H), 4.27-4.15 (m, 2H), 4.11 (d, 2H), 3.88 (t, 2H), 3.82 (s, 2H), 3.40-3.33 (m, 4H), 3.24-3.11 (m, 2H), 3.11-2.72 (m, 8H), 2.26-2.04 (m, 4H), 2.04-1.80 (m, 3H), 1.80-0.92 (m, 26H), 0.92-0.77 (m, 12H). MS (ESI) m/e 1535.4 (M+H)⁺.

### 2.32 Synthesis of N-[6-(2,5-dioxo-2,5-dihydro-1H-pyrrol-1-yl)hexanoyl]-L-valyl-N-{4-[({[2-({3-[(4-{6-[8-(1,3-benzothiazol-2-ylcarbamoyl)-5-(3-phosphonopropoxy)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}] dec-1-yl}oxy)ethyl](methyl)carbamoyl}oxy)methyl]phenyl}-N⁵-carbamoyl-L-ornithinamide (Synthon HU)

The title compound was prepared by substituting Example 1.31.11 for Example 2.5.3 in Example 2.5.4. ¹H NMR (400 MHz, dimethyl sulfoxide-*d*₆) δ ppm 9.98 (s, 1H) , 8.03 (dd, 2H), 7.70-7.84 (m, 3H), 7.59 (d, 2H), 7.48 (dd, 2H), 7.23-7.37 (m, 4H), 6.93-7.02 (m, 4H), 4.99 (d, 4H), 4.12-4.21 (m, 8H), 3.88-3.96 (m, 4H), 3.75-3.84 (m, 4H), 3.23-3.49 (m, 7H), 2.73-3.07 (m, 8H), 1.89-2.21 (m, 9H), 0.91-1.77 (m, 25H), 0.77-0.91 (m, 12H). MS (ESI) m/e 1496.3 (M+H)⁺.

### 2.33 Synthesis of N-[6-(2,5-dioxo-2,5-dihydro-1H-pyrrol-1-yl)hexanoyl]-L-valyl-N-[4-({[(4-{[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](3-phosphonopropyl)amino}piperidin-1-yl)carbonyl]oxy}methyl)phenyl]-N⁵-carbamoyl-L-ornithinamide (Synthon HT)

A solution of Example 1.26.2 (0.040 g), 4-((S)-2-((S)-2-(6-(2,5-dioxo-2,5-dihydro-1H-pyrrol-1-yl)hexanamido)-3-methylbutanamido)-5-ureidopentanamido)benzyl (4-nitrophenyl) carbonate (0.030 g) and N,N-diisopropylethylamine (0.020 mL) in N,N-dimethylformamide (0.5 mL) was stirred at room temperature. After stirring for 3 hours, the reaction was diluted with N,N-dimethylformamide (1.25 mL) and water (0.5 mL). The mixture was purified by reverse phase HPLC using a Gilson system, eluting with 10-90% acetonitrile in water containing 0.1% v/v trifluoroacetic acid. The desired fractions were combined and freeze-dried to provide the title compound. ¹H NMR (500 MHz, dimethyl sulfoxide-*d*₆) δ ppm 9.98 (s, 1H), 9.26 (s, 1H), 8.06 (d, 1H), 8.05-8.01 (m, 1H), 7.79 (d, 2H), 7.62 (d, 1H), 7.61-7.57 (m, 2H), 7.52-7.42 (m, 3H), 7.38 (d, 1H), 7.35 (d, 1H), 7.32-7.26 (m, 3H), 6.99 (s, 2H), 6.95 (d, 1H), 6.01 (s, 1H), 4.99 (s, 2H), 4.96 (s, 3H), 4.44-4.33 (m, 2H), 4.18 (dd, 2H), 3.88 (t, 2H), 3.83 (s, 2H), 3.71-3.61 (m, 2H), 3.53 (t, 2H), 3.36 (t, 2H), 3.07-2.66 (m, 8H), 2.28-2.06 (m, 6H), 2.05-1.92 (m, 2H), 1.92-1.80 (m, 2H), 1.78-0.95 (m, 32H), 0.92-0.77 (m, 14H). MS (ESI) m/e 1549.5 (M+H)⁺.

### 2.34 Synthesis of N-[6-(2,5-dioxo-2,5-dihydro-1H-pyrrol-1-yl)hexanoyl]-L-valyl-N-{4-[({[2-({3-[(4-{6-[8-(1,3-benzothiazol-2-ylcarbamoyl)naphthalen-2-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](3-phosphonopropyl)carbamoyl}oxy)methyl]phenyl}-N⁵-carbamoyl-L-ornithinamide (Synthon HV)

The title compound was prepared by substituting Example 1.14.4 for Example 2.5.3 in Example 2.5.4. ¹H NMR (400 MHz, dimethyl sulfoxide-*d*₆) δ ppm 9.98 (s, 1H), 9.02 (s, 1H), 8.32-8.45 (m, 1H), 8.12-8.27 (m, 3H), 7.98-8.09 (m, 3H), 7.93 (d, 1H), 7.66-7.83 (m, 4H), 7.54-7.64 (m, 2H), 7.46-7.50 (m, 2H), 7.24-7.40 (m, 3H), 6.99 (s, 2H), 5.93-6.09 (m, 1H), 4.99 (s, 3H), 4.33-4.49 (m, 3H), 4.15-4.20 (m, 3H), 3.19-3.50 (m, 10H), 2.86-3.07 (m, 3H), 1.87-2.27 (m, 7H), 0.91-1.77 (m, 26H), 0.76-0.89 (m, 10H). MS (ESI) m/e 1461.1 (M+H)⁺ .

### 2.35 Synthesis of N-[6-(2,5-dioxo-2,5-dihydro-1H-pyrrol-1-yl)hexanoyl]-L-valyl-N-[4-({[(4-{[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-carboxyethyl)amino}piperidin-1-yl)carbonyl]oxy}methyl)phenyl]-N⁵-carbamoyl-L-ornithinamide (Synthon HZ)

A solution of Example 1.36.2 (0.031 g), 4-((S)-2-((S)-2-(6-(2,5-dioxo-2,5-dihydro-1H-pyrrol-1-yl)hexanamido)-3-methylbutanamido)-5-ureidopentanamido)benzyl (4-nitrophenyl) carbonate (0.025 g) and N,N-diisopropylethylamine (0.016 mL) in N,N-dimethylformamide (0.5 mL) was stirred at room temperature. After stirring for 3 hours, the reaction was diluted with N,N-dimethylformamide (1.25 mL) and water (0.5 mL). The mixture was purified by reverse phase HPLC using a Gilson system, eluting with 10-90% acetonitrile in water containing 0.1% v/v trifluoroacetic acid. The desired fractions were combined and freeze-dried to provide the title compound. ¹H NMR (400 MHz, dimethyl sulfoxide-*d*₆) δ ppm 12.84 (s, 1H), 9.98 (s, 1H), 8.82 (s, 1H), 8.05 (dd, 2H), 7.79 (d, 2H), 7.70-7.53 (m, 2H), 7.53-7.24 (m, 6H), 6.99 (s, 2H), 6.95 (d, 1H), 6.00 (s, 1H), 4.99 (s, 2H), 4.96 (s, 2H), 4.37 (q, 2H), 4.25-4.15 (m, 2H), 3.88 (t, 2H), 3.83 (s, 2H), 3.69-3.61 (m, 2H), 3.44-3.30 (m, 4H), 3.08-2.90 (m, 4H), 2.90-2.72 (m, 4H), 2.27-2.04 (m, 5H), 2.04-1.89 (m, 2H), 1.77-0.94 (m, 28H), 0.91-0.78 (m, 14H). MS (ESI) m/e 1499.5 (M+H)⁺.

### 2.36 Synthesis of N-[6-(2,5-dioxo-2,5-dihydro-1H-pyrrol-1-yl)hexanoyl]-L-valyl-N⁵-carbamoyl-N-{4-[({[2-({3-[(4-{2-carboxy-6-[8-([1,3]thiazolo[4,5-b]pyridin-2-ylcarbamoyl)-3,4-dihydroisoquinolin-2(1H)-yl]pyridin-3-yl}-5-methyl-1H-pyrazol-1-yl)methyl]-5,7-dimethyltricyclo[3.3.1.1^{3,7}]dec-1-yl}oxy)ethyl](3-phosphonopropyl)carbamoyl}oxy)methyl]phenyl}-L-ornithinamide (Synthon IA)

The title compound was prepared by substituting Example 1.39.2 for Example 1.2.9 in Example 2.1. ¹H NMR (400 MHz, dimethyl sulfoxide-*d*₆) δ ppm 9.98 (s, 1H), 8.60 (dd, 1H), 8.52 (dd, 1H), 8.06 (d, 1H), 7.78 (d, 1H), 7.65 (d, 1H), 7.59 (br m, 2H), 7.50 (m, 1H), 7.45 (d, 1H), 7.38 (m, 2H), 7.28 (s, 1H), 7.27 (d, 2H), 6.99 (s, 2H), 6.97 (d, 1H), 5.98 (br s, 1H), 4.98 (s, 4H), 4.39 (m, 1H), 4.19 (br m, 1H), 3.88 (t, 2H), 3.80 (br d, 2H), 3.36 (br m, 3H), 3.24 br (m, 4H), 2.98 (m, 4H), 2.16 (m, 2H), 2.12 (s, 3H), 1.95 (br m, 1H), 1.67 (br m, 3H), 1.34-1.47 (m, 9H), 1.08-1.23 (m, 11H), 0.95 (br m, 2H), 0.85-0.80 (m, 12H). MS (ESI) m/e 1465.5 (M-H)⁻.

### 2.37 Synthesis of N-[6-(2,5-dioxo-2,5-dihydro-1H-pyrrol-1-yl)hexanoyl]-L-valyl-N⁵-carbamoyl-N-{4-[({[2-({3-[(4-{2-carboxy-6-[8-([1,3]thiazolo[5,4-b]pyridin-2-ylcarbamoyl)-3,4-dihydroisoquinolin-2(1H)-yl]pyridin-3-yl}-5-methyl-1H-pyrazol-1-yl)methyl]-5,7-dimethyltricyclo[3.3.1.1^{3,7}]dec-1-yl}oxy)ethyl](3-phosphonopropyl)carbamoyl}oxy)methyl]phenyl}-L-ornithinamide (Synthon IF)

The title compound was prepared by substituting Example 1.40.2 for Example 1.2.9 in Example 2.1. ¹H NMR (400 MHz, dimethyl sulfoxide-*d*₆) δ ppm 9.98 (s, 1H), 8.52 (dd, 1H), 8.16 (dd, 1H), 8.05 (br d, 1H), 7.78 (br d, 1H), 7.62 (m, 1H), 7.58 (br m, 2H), 7.52 (m, 2H), 7.44 (d, 1H), 7.38 (t, 1H), 7.29 (s, 1H) 7.27 (d, 2H), 6.99 (s, 2H), 6.97 (d, 1H), 4.98 (s, 2H), 4.96 (s, 2H), 4.39 (m, 1H), 4.19 (br m, 1H), 3.88 (t, 2H), 3.80 (br d, 2H), 3.36 (br m, 3H), 3.24 br (m, 4H), 2.98 (m, 4H), 2.16 (m, 2H), 2.12 (s, 3H), 1.95 (br m, 1H), 1.67 (br m, 3H), 1.47-1.34 (m, 9H), 1.08-1.23 (m, 11H), 0.95 (br m, 2H), 0.85-0.80 (m, 12H). MS (ESI) m/e 1451.5 (M-H)⁻.

### 2.38 Synthesis of N-{6-[(chloroacetyl)amino]hexanoyl}-L-valyl-N-{4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)methyl]phenyl}-L-alaninamide (Synthon IG)

### 2.38.1 3-(1-((3-(2-((((4-((S)-2-((S)-2-amino-3-methylbutanamido)propanamido)benzyl)oxy)carbonyl)(2-sulfoethyl)amino)ethoxy)-5,7-dimethyladamantan-1-yl)methyl)-5-methyl-1H-pyrazol-4-yl)-6-(8-(benzo[d]thiazol-2-ylcarbamoyl)-3,4-dihydroisoquinolin-2(1H)-yl)picolinic acid

A solution of Example 1.2.9 (0.050 g), (9H-fluoren-9-yl)methyl ((S)-3-methyl-1-(((S)-1-((4-((((4-nitrophenoxy)carbonyl)oxy)methyl)phenyl)amino)-1-oxopropan-2-yl)amino)-1-oxobutan-2-yl)carbamate (0.039 g) and N,N-diisopropylethylamine (0.027 mL) in N,N-dimethylformamide (1 mL) was stirred at room temperature. After stirring overnight, diethylamine (0.027 mL) was added to the reaction, and stirring was continued for 2 hours. The reaction was quenched with trifluoroacetic acid, and the mixture was purified by reverse phase HPLC using a Gilson system, eluting with 5-75% acetonitrile in water containing 0.1% v/v trifluoroacetic acid. The desired fractions were combined and freeze-dried to provide the title compound. MS (ESI) m/e 1499.5 (M+H)⁺.

### 2.38.2 N-{6-[(chloroacetyl)amino]hexanoyl}-L-valyl-N-{4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)methyl]phenyl}-L-alaninamide

To a solution of 6-(2-chloroacetamido)hexanoic acid (6 mg) and 2-(3H-[1,2,3]triazolo[4,5-b]pyridin-3-yl)-1,1,3,3-tetramethylisouronium hexafluorophosphate(V) (0.011 g) in N,N-dimethylformamide (1 mL) was added N,N-diisopropylethylamine (0.015 mL), and the reaction stirred for 5 minutes. This solution was added to Example 2.38.1 (0.022 g) and was stirred for 1 hour. The reaction was diluted with N,N-dimethylformamide (1 mL) and water (0.5 mL). The mixture was purified by reverse phase HPLC using a Gilson system, eluting with 10-90% acetonitrile in water containing 0.1% v/v trifluoroacetic acid. The desired fractions were combined and freeze-dried to provide the title compound. ¹H NMR (400 MHz, dimethyl sulfoxide-*d*₆) δ ppm 12.83 (s, 1H), 9.93 (s, 1H), 8.20-8.10 (m, 2H), 8.04 (d, 1H), 7.83-7.76 (m, 2H), 7.64-7.55 (m, 3H), 7.55-7.50 (m, 1H), 7.50-7.41 (m, 2H), 7.40-7.32 (m, 2H), 7.32-7.24 (m, 3H), 6.96 (d, 1H), 5.07-4.92 (m, 3H), 4.39 (p, 1H), 4.18 (dd, 2H), 4.01 (s, 2H), 3.92-3.76 (m, 6H), 3.54-3.32 (m, 4H), 3.25 (t, 2H), 3.13-2.93 (m, 4H), 2.72-2.58 (m, 2H), 2.29-2.12 (m, 2H), 2.09 (s, 3H), 2.05-1.92 (m, 1H), 1.58-0.89 (m, 18H), 0.89-0.77 (m, 12H). MS (ESI) m/e 1362.2 (M+H)⁺.

### 2.39 Synthesis of N-[6-(2,5-dioxo-2,5-dihydro-1H-pyrrol-1-yl)hexanoyl]-L-valyl-N-{4-[({[2-({3-[(4-{6-[8-(1,3-benzothiazol-2-ylcarbamoyl)-5-(carboxymethoxy)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](methyl)carbamoyl}oxy)methyl]phenyl}-N⁵-carbamoyl-L-ornithinamide (Synthon IJ)

The title compound was prepared by substituting Example 1.41.3 for Example 2.5.3 in Example 2.5.4. ¹H NMR (400 MHz, dimethyl sulfoxide-*d*₆) δ ppm 10.03 (s, 1H), 9.96 (s, 1H), 8.26-8.34 (m, 1H), 7.95-8.11 (m, 2H), 7.73-7.82 (m, 2H), 7.22-7.70 (m, 11H) , 6.95-7.05 (m, 3H), 6.89 (d, 1H), 5.23 (s, 1H), 4.98 (d, 3H), 4.83 (s, 1H), 4.33-4.43 (m, 1H), 4.11-4.23 (m, 1H), 3.74-3.95 (m, 3H), 3.22-3.39 (m, 10H), 2.78-3.06 (m, 12H), 1.91-2.22 (m, 8H) , 0.93-1.68 (m, 20H), 0.77-0.88 (m, 10H). MS (ESI) m/e 1432.2 (M+H)⁺.

### 2.40 Synthesis of N-[6-(2,5-dioxo-2,5-dihydro-1H-pyrrol-1-yl)hexanoyl]-L-valyl-N-[4-({[(2-{[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)amino}ethyl)(2-carboxyethyl)carbamoyl]oxy}methyl)phenyl]-N⁵-carbamoyl-L-ornithinamide (Synthon IJ)

The title compound was prepared as described in Example 2.1, replacing Example 1.2.9 with Example 1.38.2. ¹H NMR (400 MHz, dimethyl sulfoxide-*d*₆) δ ppm 12.86 (s, 1H), 9.99 (s, 1H), 9.10 (s, 1H), 8.04 (t, 2H), 7.73-7.85 (m, 2H), 7.61 (t, 3H), 7.41-7.55 (m, 3H), 7.26-7.39 (m, 5H), 6.99 (s, 2H), 6.95 (d, 1H), 6.00 (s, 1H), 4.99 (d, 4H), 4.34-4.45 (m, 2H), 4.19 (dd, 2H), 3.88 (t, 2H), 3.82 (s, 2H), 3.36 (t, 4H), 2.85-3.09 (m, 5H), 2.06-2.22 (m, 4H), 1.89-2.02 (m, 1H), 0.94-1.77 (m, 20H), 0.77-0.90 (m, 11H). MS (ESI) m/e 1567.4 (M+H)⁺.

### 2.41 Synthesis of 6-[8-(1,3-benzothiazol-2-ylcarbamoyl)-3,4-dihydroisoquinolin-2(1H)-yl]-3-[1-({3-[2-({(2S)-2-[{[(4-{[(2S)-5-(carbamoylamino)-2-{[(2S)-2-{[6-(2,5-dioxo-2,5-dihydro-1H-pyrrol-1-yl)hexanoyl]amino}-3-methylbutanoyl]amino}pentanoyl]amino}benzyl)oxy]carbonyl}(2-carboxyethyl)amino]-3-carboxypropanoyl}amino)ethoxy]-5,7-dimethyltricyclo[3.3.1.1^{3,7}]dec-1-yl}methyl)-5-methyl-1H-pyrazol-4-yl]pyridine-2-carboxylic acid (Synthon IK)

The title compound was prepared as described in Example 2.1, replacing Example 1.2.9 with Example 1.32.4. MS (ESI) m/e 1592.4 (M-H)⁻.

### 2.42 Synthesis of 6-[8-(1,3-benzothiazol-2-ylcarbamoyl)-3,4-dihydroisoquinolin-2(1H)-yl]-3-(1-{[3-(2-{[(2S)-2-({[(4-{[(2S)-5-(carbamoylamino)-2-{[(2S)-2-{[6-(2,5-dioxo-2,5-dihydro-1H-pyrrol-1-yl)hexanoyl]amino}-3-methylbutanoyl]amino}pentanoyl]amino}benzyl)oxy]carbonyl}amino)-3-carboxypropanoyl](2-sulfoethyl)amino}ethoxy)-5,7-dimethyltricyclo[3.3.1.1^{3,7}]dec-1-yl]methyl}-5-methyl-1H-pyrazol-4-yl)pyridine-2-carboxylic acid (Synthon IL)

The title compound was prepared as described in Example 2.1, replacing Example 1.2.9 with Example 1.44.2. ¹H NMR (400 MHz, dimethyl sulfoxide-*d*₆) δ ppm 12.82 (s, 1H), 9.96 (s, 1H), 8.03 (t, 2H), 7.77 (d, 2H), 7.39-7.62 (m, 7H), 7.30-7.39 (m, 2H), 7.22-7.29 (m, 3H), 6.98 (s, 2H), 6.92-6.96 (m, 1H), 5.97 (s, 1H), 4.83-5.05 (m, 3H), 3.83-3.92 (m, 1H), 3.79 (s, 1H), 3.00 (s, 2H), 2.03-2.22 (m, 8H), 1.94 (s, 2H), 1.34 (d, 30H), 0.69-0.90 (m, 13H). MS (ESI) m/e 1565.5 (M-H)⁻.

### 2.43 Synthesis of N-[6-(2,5-dioxo-2,5-dihydro-1H-pyrrol-1-yl)hexanoyl]-L-valyl-N-[4-({[(4-{[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](3-carboxypropyl)aminolpiperidin-1-yl)carbonyl]oxy}methyl)phenyl]-N⁵-carbamoyl-L-ornithinamide (Synthon IM)

A solution of Example 1.42.2 (0.045 g), 4-((S)-2-((S)-2-(6-(2,5-dioxo-2,5-dihydro-1H-pyrrol-1-yl)hexanamido)-3 -methylbutanamido)-5 -ureidopentanamido)benzyl (4-nitrophenyl) carbonate (0.035 g) and N,N-diisopropylethylamine (0.038 mL) in N,N-dimethylformamide (0.5 mL) was stirred at room temperature. After stirring for 3 hours, the reaction was diluted with N,N-dimethylformamide (1.25 mL) and water (0.5 mL). The mixture was purified by reverse phase HPLC using a Gilson system, eluting with 10-90% acetonitrile in water containing 0.1% v/v trifluoroacetic acid. The desired fractions were combined and freeze-dried to provide the title compound. ¹H NMR (400 MHz, dimethyl sulfoxide-*d*₆) δ ppm 12.76 (s, 1H), 9.91 (s, 1H), 8.79 (s, 1H), 7.98 (dd, 2H), 7.72 (d, 2H), 7.68-7.47 (m, 3H), 7.47-7.00 (m, 7H), 6.96-6.83 (m, 3H), 5.93 (s, 1H), 4.91 (d, 3H), 4.30 (q, 1H), 4.17-3.97 (m, 4H), 3.96-3.53 (m, 4H), 3.34-2.65 (m, 12H), 2.25 (t, 2H), 2.16-1.67 (m, 12H), 1.67-0.88 (m, 26H), 0.84-0.70 (m, 12H). MS (ESI) m/e 1513.6 (M+H)⁺.

### 2.44 Synthesis of 4-[(1E)-3-({[2-({3-[(4-{6-[8-(1,3-benzothiazol-2-ylcarbamoyl)-5-(carboxymethoxy)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](methyl)carbamoyl}oxy)prop-1-en-1-yl]-2-({N-[6-(2,5-dioxo-2,5-dihydro-1H-pyrrol-1-yl)hexanoyl]-beta-alanyl}amino)phenyl beta-D-glucopyranosiduronic acid (Synthon IO)

### 2.44.1 (E)-tert-butyldimethyl((3-(4,4,5,5-tetramethyl-1,3,2-dioxaborolan-2-yl)allyl)oxy)silane

To a flask charged with tert-butyldimethyl(prop-2-yn-1-yloxy)silane (5 g) and dichloromethane (14.7 mL) under nitrogen atmosphere was added dropwise 4,4,5,5-tetramethyl-1,3,2-dioxaborolane (3.94 g). The mixture was stirred at room temperature for one minute then transferred via cannula to a nitrogen-sparged flask containing Cp₂ZrClH (chloridobis(η5-cyclopentadienyl)hydridozirconium, Schwartz's Reagent) (379 mg). The resulting reaction mixture was stirred at room temperature for 16 hours. The mixture was carefully quenched with water (15 mL), and then extracted with diethyl ether (3x 30 mL). The combined organic phases were washed with water (15 mL), dried over MgSO₄, filtered, and purified by silica gel chromatography, eluting with a gradient from 0-8% ethyl acetate/heptanes to give the title compound. MS (ESI) m/z 316.0 (M+NH₄)⁺.

### 2.44.2 (2S,3R,4S,5S,6S)-2-(4-bromo-2-nitrophenoxy)-6-(methoxycarbonyl)tetrahydro-2H-pyran-3,4,5-triyl triacetate

(2R,3R,4S,5S,6S)-2-Bromo-6-(methoxycarbonyl)tetrahydro-2H-pyran-3,4,5-triyl triacetate (5 g) was dissolved in acetonitrile (100 mL). Ag₂O (2.92 g) was added to the solution, and the reaction was stirred for 5 minutes at room temperature. 4-Bromo-2-nitrophenol (2.74 g) was added, and the reaction mixture was stirred at room temperature for 4 hours. The silver salt residue was filtered through diatomaceous earth, and the filtrate was concentrated under reduced pressure. The residue was purified by silica gel chromatography, eluting with a gradient of 10-70% ethyl acetate in heptanes, to give the title compound. MS (ESI+) m/z 550.9 (M+NH₄)⁺.

### 2.44.3 (2S,3R,4S,5S,6S)-2-(4-((E)-3-((tert-butyldimethylsilyl)oxy)prop-1-en-1-yl)-2-nitrophenoxy)-6-(methoxycarbonyl)tetrahydro-2H-pyran-3,4,5-triyl triacetate

Example 2.44.2 (1 g), sodium carbonate (0.595 g), tris(dibenzylideneacetone)dipalladium (Pd₂(dba)₃) (0.086 g), and 1,3,5,7-tetramethyl-6-phenyl-2,4,8-trioxa-6-phosphaadamantane (0.055 g) were combined in a 3-neck 50-mL round bottom flask equipped with a reflux condenser and the system was degassed with nitrogen. Separately, a solution of Example 2.44.1 (0.726 g) in tetrahydrofuran (15 mL) was degassed with nitrogen for 30 minutes. The latter solution was transferred via cannula into the flask containing the solid reagents, followed by addition of degassed water (3 mL) via syringe. The reaction was heated to 60 °C for two hours. The reaction mixture was partitioned between ethyl acetate (3x 30 mL) and water (30 mL). The combined organic phases were dried (Na₂SO₄), filtered, and concentrated. The residue was purified by silica gel chromatography, eluting with a gradient from 0-35% ethyl acetate in heptanes, to provide the title compound. MS (ESI+) m/z 643.1 (M+NH₄)⁺.

### 2.44.4 (2S,3R,4S,5S,6S)-2-(2-amino-4-((E)-3-hydroxyprop-1-en-1-yl)phenoxy)-6-(methoxycarbonyl)tetrahydro-2H-pyran-3,4,5-triyl triacetate

A 500-mL three-neck, nitrogen-flushed flask equipped with a pressure-equalizing addition funnel was charged with zinc dust (8.77 g). A degassed solution of Example 2.44.3 (8.39 g) in tetrahydrofuran (67 mL) was added via cannula. The resulting suspension was chilled in an ice bath, and 6N HCl (22.3 mL) was added dropwise via the addition funnel at such a rate that the internal temperature of the reaction did not exceed 35 °C. After the addition was complete, the reaction was stirred for two hours at room temperature, and filtered through a pad of diatomaceous earth, rinsing with water and ethyl acetate. The filtrate was treated with saturated aqueous NaHCO₃ solution until the water layer was no longer acidic, and the mixture was filtered to remove the resulting solids. The filtrate was transferred to a separatory funnel, and the layers were separated. The aqueous layer was extracted with ethyl acetate (3x 75 mL), and the combined organic layers were washed with water (100 mL), dried over Na₂SO₄, filtered, and concentrated. The residue was triturated with diethyl ether and the solid collected by filtration to provide the title compound. MS (ESI+) m/z 482.0 (M+H)⁺.

### 2.44.5 (9H-fluoren-9-yl)methyl (3-chloro-3-oxopropyl)carbamate

To a solution of 3-((((9H-fluoren-9-yl)methoxy)carbonyl)amino)propanoic acid (5.0 g) in dichloromethane (53.5 mL) was added sulfurous dichloride (0.703 mL). The mixture was stirred at 60 °C for one hour. The mixture was cooled and concentrated to give the title compound, which was used in the next step without further purification.

### 2.44.6 (2S,3R,4S,5S,6S)-2-(2-(3-((((9H-fluoren-9-yl)methoxy)carbonyl)amino)propanamido)-4-((E)-3-hydroxyprop-1-en-1-yl)phenoxy)-6-(methoxycarbonyl)tetrahydro-2H-pyran-3,4,5-triyl triacetate

Example 2.44.4 (6.78 g) was dissolved in dichloromethane (50 mL), and the solution was chilled to 0 °C in an ice bath. N,N-Diisopropylethylamine (3.64 g) was added, followed by dropwise addition of a solution of Example 2.44.5 (4.88 g) in dichloromethane (50 mL). The reaction was stirred for 16 hours allowing the ice bath to come to room temperature. Saturated aqueous NaHCO₃ solution (100 mL) was added, and the layers were separated. The aqueous layer was further extracted with dichloromethane (2 x 50 mL). The extracts were dried over Na₂SO₄, filtered, concentrated and purified by silica gel chromatography, eluting with a gradient of 5-95% ethyl acetate/heptane, to give an inseparable mixture of starting aniline and desired product. The mixture was partitioned between 1N aqueous HCl (40 mL) and a 1:1 mixture of diethyl ether and ethyl acetate (40 mL), and then the aqueous phase was further extracted with ethyl acetate (2x 25 mL). The organic phases were combined, washed with water (2x 25 mL), dried over Na₂SO₄, filtered, and concentrated to give the title compound. MS (ESI+) m/z 774.9 (M+H)⁺.

### 2.44.7 (2S,3R,4S,5S,6S)-2-(2-(3-((((9H-fluoren-9-yl)methoxy)carbonyl)amino)propanamido)-4-((E)-3-(((4-nitrophenoxy)carbonyl)oxy)prop-1-en-1-yl)phenoxy)-6(methoxycarbonyl)tetrahydro-2H-pyran-3,4,5-triyl triacetate

Example 2.44.6 (3.57 g) was dissolved in dichloromethane (45 mL) and bis(4-nitrophenyl)carbonate (2.80 g) was added, followed by dropwise addition of N,N-diisopropylethylamine (0.896 g). The reaction mixture was stirred at room temperature for two hours. Silica gel (20 g) was added to the reaction solution, and the mixture was concentrated to dryness under reduced pressure, keeping the bath temperature at or below 25 °C. The silica residue was loaded atop a column, and the product was purified by silica gel chromatography, eluting with a gradient from 0-100% ethyl acetate-heptane, providing partially purified product which was contaminated with nitrophenol. The material was triturated with methyl tert-butyl ether (250 mL), and the resulting slurry was allowed to sit for 1 hour. The product was collected by filtration. Three successive crops were collected in a similar fashion to give the title compound. MS (ESI+) m/z 939.8 (M+H)⁺.

### 2.44.8 3-(1-((3-(2-(((((E)-3-(3-(3-aminopropanamido)-4-(((2S,3R,4S,5S,6S)-6-carboxy-3,4,5-trihydroxytetrahydro-2H-pyran-2-yl)oxy)phenyl)allyl)oxy)carbonyl)(methyl)amino)ethoxy)-5,7-dimethyladamantan-1-yl)methyl)-5-methyl-1H-pyrazol-4-yl)-6-(8-(benzo[d]thiazol-2-ylcarbamoyl)-5-(carboxymethoxy)-3,4-dihydroisoquinolin-2(1H)-yl)picolinic acid

To a cold (0 °C) solution of Example 2.44.7 (19.7 mg) and Example 1.41.3 (18.5 mg) in N,N-dimethylformamide (2 mL) was added N,N-diisopropylethylamine (0.054 mL). The reaction was slowly warmed to room temperature and stirred overnight. To the reaction mixture was added water (2 mL) and lithium hydroxide monohydrate (50 mg), and the mixture was stirred overnight. The mixture was acidified with trifluoroacetic acid and filtered. The mixture was purified by reverse phase HPLC (Gilson system), eluting with 10-85% acetonitrile in 0.1% trifluoroacetic acid in water, to provide the title compound. MS (ESI) m/e 1273.2 (M+H)⁺.

### 2.44.9 4-[(1E)-3-({[2-({3-[(4-{6-[8-(1,3-benzothiazol-2-ylcarbamoyl)-5-(carboxymethoxy)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](methyl)carbamoyl}oxy)prop-1-en-1-yl]-2-({N-[6-(2,5-dioxo-2,5-dihydro-1H-pyrrol-1-yl)hexanoyl]-beta-alanyl}amino)phenyl beta-D-glucopyranosiduronic acid

To a solution of Example 2.44.8 (10 mg) and 2,5-dioxopyrrolidin-1-yl 6-(2,5-dioxo-2,5-dihydro-1H-pyrrol-1-yl)hexanoate (2.3 mg) in N,N-dimethylformamide (2 mL) was added N,N-diisopropylethylamine (0.054 mL). The reaction was stirred overnight. The reaction mixture was diluted with methanol (2 mL) and acidified with trifluoroacetic acid. The mixture was purified by reverse phase HPLC (Gilson system), eluting with 10-85% acetonitrile in 0.1% trifluoroacetic acid in water, to give the title compound. ¹H NMR (400 MHz, dimethyl sulfoxide-*d*₆) δ ppm 12.70 (s, 1H) , 9.03 (s, 1H), 8.25 (s, 1H), 8.01 (d, 1H), 7.87 (t, 1H), 7.77 (d, 1H), 7.69 (d, 1H), 7.41-7.55 (m, 2H), 7.23-7.38 (m, 2H), 6.79-7.16 (m, 7H) , 6.56 (d, 1H), 6.09-6.25 (m, 1H), 4.96-5.07 (m, 3H), 4.84 (s, 3H), 4.64 (d, 3H), 3.87-3.97 (m, 5H), 3.24-3.47 (m, 12H), 2.77-2.95 (m, 6H), 1.94-2.08 (m, 6H), 0.92-1.56 (m, 20H), 0.74-0.86 (m, 6H). MS (ESI) m/e 1487.3 (M+Na)⁺.

### 2.45 Synthesis of N-[6-(2,5-dioxo-2,5-dihydro-1H-pyrrol-1-yl)hexanoyl]-L-valyl-N-{4-[({[2-({3-[(4-{6-[8-(1,3-benzothiazol-2-ylcarbamoyl)naphthalen-2-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)methyl]phenyl}-N⁵-carbamoyl-L-ornithinamide (Synthon IP)

The title compound was prepared by substituting Example 1.43.7 for Example 2.5.3 in Example 2.5.4. ¹H NMR (400 MHz, dimethyl sulfoxide-*d*₆) δ ppm 13.09 (s, 1H), 9.99 (s, 1H), 9.02 (s, 1H), 8.30-8.40 (m, 3H), 7.93-8.25 (m, 6H), 7.23-7.86 (m, 10H), 6.92-7.05 (m, 2H), 4.99 (d, 2H), 4.36-4.44 (m, 2H), 4.14-4.23 (m, 2H), 2.87-3.35 (m, 12H) , 2.81 (t, 2H), 2.59-2.70 (m, 2H), 1.84-2.28 (m, 8H), 0.97-1.77 (m, 20H), 0.77-0.88 (m,10H). MS (ESI) m/e 1448.3 (M+Na)⁺.

### 2.46 Synthesis of N-[6-(2,5-dioxo-2,5-dihydro-1H-pyrrol-1-yl)hexanoyl]-L-valyl-N-[4-({[(2-{[8-(1,3-benzothiazol-2-ylcarbamoyl)-2-(6-carboxy-5-{1-[(3,5-dimethyl-7-{2-[methyl(2-sulfoethyl)amino]ethoxy}tricyclo[3.3.1.1^{3,7}]dec-1-yl)methyl]-5-methyl-1H-pyrazol-4-yl}pyridin-2-yl)-1,2,3,4-tetrahydroisoquinolin-5-yl]oxy}ethyl)carbamoyl]oxy}methyl)phenyl]-N⁵-carbamoyl-L-ornithinamide (Synthon IS)

The title compound was prepared as described in Example 2.1, replacing Example 1.2.9 with Example 1.46.2. ¹H NMR (400 MHz, dimethyl sulfoxide-*d*₆) δ ppm 12.69 (s, 1H), 9.97 (s, 1H), 8.97 (s, 1H), 8.04 (dd, 2H), 7.78 (d, 2H), 7.71 (d, 1H), 7.59 (d, 2H), 7.44-7.54 (m, 3H), 7.26-7.37 (m, 4H), 6.96-7.03 (m, 4H), 5.97 (s, 1H), 4.99 (d, 4H), 4.31-4.45 (m, 1H), 4.18 (dd, 1H), 4.09 (s, 2H), 3.85-3.93 (m, 2H), 3.83 (s, 2H), 3.39-3.47 (m, 2H), 3.24-3.39 (m, 4H), 3.12-3.24 (m, 2H), 2.75-3.07 (m, 9H), 2.06-2.23 (m, 5H), 1.90-2.01 (m, 1H), 1.54-1.75 (m, 2H), 1.24-1.52 (m, 12H), 0.91-1.24 (m, 8H), 0.77-0.88 (m, 12H). MS (ESI) m/e 1525.4 (M+H)⁺.

### 2.47 Synthesis of N-[6-(2,5-dioxo-2,5-dihydro-1H-pyrrol-1-yl)hexanoyl]-L-valyl-N-[4-({[(2-{[8-(1,3-benzothiazol-2-ylcarbamoyl)-2-(6-carboxy-5-{1-[(3,5-dimethyl-7-{2-[methyl(2-sulfoethyl)amino]ethoxy}tricyclo[3.3.1.1^{3,7}]dec-1-yl)methyl]-5-methyl-1H-pyrazol-4-yl}pyridin-2-yl)-1,2,3,4-tetrahydroisoquinolin-5-yl]oxy}ethyl)(2-sulfoethyl)carbamoyl]oxy}methyl)phenyl]-N⁵-carbamoyl-L-ornithinamide (Synthon IU)

The title compound was prepared as described in Example 2.1, replacing Example 1.2.9 with Example 1.47.2. ¹H NMR (400 MHz, dimethyl sulfoxide-*d*₆) δ ppm 12.70 (s, 1H), 9.99 (s, 1H), 8.97 (s, 1H), 8.04 (dd, 2H), 7.78 (d, 2H), 7.71 (d, 1H), 7.59 (d, 2H), 7.43-7.55 (m, 2H), 7.28-7.37 (m, 4H), 6.94-7.07 (m, 4H), 6.05 (s, 1H), 4.93-5.11 (m, 4H), 4.31-4.46 (m, 2H), 4.12-4.26 (m, 4H), 3.80-3.95 (m, 4H), 3.40-3.50 (m, 2H), 3.24-3.40 (m, 6H), 3.13-3.24 (m, 2H), 2.74-3.08 (m, 9H), 2.63-2.73 (m, 2H), 2.05-2.23 (m, 5H), 1.96 (s, 1H), 1.52-1.77 (m, 2H), 1.23-1.53 (m, 12H), 0.97-1.22 (m, 8H), 0.77-0.89 (m, 12H). MS (ESI) m/e 1631.5 (M-H)⁻.

### 2.48 Synthesis of N-[6-(2,5-dioxo-2,5-dihydro-1H-pyrrol-1-yl)hexanoyl]-L-valyl-N-[4-({[(2-{[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)amino}ethyl)(2-sulfoethyl)carbamoyl]oxy}methyl)phenyl]-N⁵-carbamoyl-L-ornithinamide (Synthon IV)

The title compound was prepared as described in Example 2.1, replacing Example 1.2.9 with Example 1.48.2. ¹H NMR (400 MHz, dimethyl sulfoxide-*d*₆) δ ppm 12.82 (s, 1H), 10.00 (s, 1H), 9.29-9.57 (m, 1H), 8.05 (t, 2H), 7.79 (d, 2H), 7.51-7.63 (m, 4H), 7.40-7.50 (m, 2H), 7.27-7.39 (m, 5H), 6.93-7.02 (m, 3H), 4.99 (d, 3H), 4.30-4.47 (m, 1H), 4.19 (t, 1H), 3.79-3.92 (m, 3H), 3.60-3.74 (m, 2H), 3.01 (s, 9H), 2.70 (d, 4H), 2.05-2.23 (m, 6H), 1.96 (d, 2H), 1.53-1.78 (m, 3H), 1.22-1.54 (m, 13H), 0.89-1.22 (m, 9H), 0.75-0.89 (m, 13H). MS (ESI) m/e 1603.3 (M+H)⁺.

### 2.49 Synthesis of N-{6-[(chloroacetyl)amino]hexanoyl}-L-valyl-N-{4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)methyl]phenyl}-N⁵-carbamoyl-L-ornithinamide (Synthon IZ)

### 2.49.1 3-(1-(((1r,3r)-3-(2-((((4-((S)-2-((S)-2-amino-3-methylbutanamido)-5-ureidopentanamido)benzyl)oxy)carbonyl)(2-sulfoethyl)amino)ethoxy)-5,7-dimethyladamantan-1-yl)methyl)-5-methyl-1H-pyrazol-4-yl)-6-(8-(benzo[d]thiazol-2-ylcarbamoyl)-3,4-dihydroisoquinolin-2(1H)-yl)picolinic acid

A solution of Example 1.2.9 (0.045 g) (9H-fluoren-9-yl)methyl ((S)-3-methyl-1-(((S)-1-((4-((((4-nitrophenoxy)carbonyl)oxy)methyl)phenyl)amino)-1-oxo-5-ureidopentan-2-yl)amino)-1-oxobutan-2-yl)carbamate (0.043 g) and N,N-diisopropylethylamine (0.041 mL) were stirred together in N,N-dimethylformamide (1 mL) at room temperature. After stirring overnight, diethylamine (0.024 mL) was added to the reaction, and stirring was continued for 2 hours. The reaction was quenched with trifluoroacetic acid then purified by reverse phase HPLC using a Gilson system, eluting with 10-75% acetonitrile in water containing 0.1% v/v trifluoroacetic acid. The desired fractions were combined and freeze-dried to provide the title compound.

### 2.49.2 N-{6-[(chloroacetyl)amino]hexanoyl}-L-valyl-N-{4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)methyl]phenyl}-N⁵-carbamoyl-L-ornithinamide

A solution of 6-(2-chloroacetamido)hexanoic acid (6.43 mg) and 2-(3H-[1,2,3]triazolo[4,5-b]pyridin-3-yl)-1,1,3,3-tetramethylisouronium hexafluorophosphate(V) (0.012 g) in N,N-dimethylformamide (0.5 mL) was added N,N-diisopropylethylamine (0.019 mL), and the reaction stirred for 5 minutes. This solution was added to Example 2.49.1 (0.026 g) and was stirred for 1 hour. The reaction was diluted with N,N-dimethylformamide (1 mL) and water (0.5 mL). The mixture was purified by reverse phase HPLC using a Gilson system, eluting with 10-60% acetonitrile in water containing 0.1% v/v trifluoroacetic acid. The desired fractions were combined and freeze-dried to provide the title compound. ¹H NMR (500 MHz, dimethyl sulfoxide-*d*₆) δ ppm 12.85 (s, 1H), 9.99 (s, 1H), 8.18 (q, 1H), 8.08 (d, 1H), 8.04 (d, 1H), 7.84-7.76 (m, 2H), 7.64-7.56 (m, 3H), 7.56-7.50 (m, 1H), 7.47 (t, 1H), 7.43 (d, 1H), 7.37 (d, 1H), 7.35 (d, 1H), 7.29 (s, 1H), 7.27 (d, 2H), 6.95 (d, 1H), 6.05 (s, 1H), 5.05-4.91 (m, 4H), 4.48-4.33 (m, 1H), 4.26-4.14 (m, 1H), 4.02 (s, 2H), 3.88 (t, 2H), 3.81 (d, 2H), 3.25 (t, 2H), 3.14-2.98 (m, 6H), 2.98-2.87 (m, 2H), 2.74-2.59 (m, 2H), 2.27-2.05 (m, 6H), 2.04-1.92 (m, 1H), 1.78-1.65 (m, 1H), 1.65-1.53 (m, 1H), 1.53-0.90 (m, 22H), 0.90-0.73 (m, 12H). MS (ESI) m/e 1448.2 (M+H)⁺.

### 2.50 Synthesis of N-[6-(2,5-dioxo-2,5-dihydro-1H-pyrrol-1-yl)hexanoyl]-L-valyl-N-{4-[({[2-({3-[(4-{6-[4-(1,3-benzothiazol-2-ylcarbamoyl)-3,4-dihydro-2H-1,4-benzoxazin-6-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)methyl]phenyl}-N⁵-carbamoyl-L-ornithinamide (Synthon JD)

The title compound was prepared by substituting Example 1.51.8 for Example 2.5.3 in Example 2.5.4. ¹H NMR (400 MHz, dimethyl sulfoxide-*d*₆) δ ppm 9.56 (s, 1H), 8.51-8.59 (m, 1H), 7.89 (d, 1H), 7.82 (d, 1H), 7.69-7.77 (m, 2H), 7.34-7.62 (m, 7H) , 7.16-7.34 (m, 4H), 6.95 (dd, 1H), 5.95-6.05 (m, 1H), 4.95 (s, 2H) , 4.06-4.44 (m, 6H), 3.85 (s, 3H), 3.39-3.59 (m, 7H), 2.61-2.74 (m, 3H), 2.19 (s, 3H), 1.88-2.16 (m, 3H), 0.96-1.75 (m, 22H), 0.71-0.89 (m, 13H). MS (ESI) m/e 1454.2 (M+Na)⁺.

### 2.51 Synthesis of N-[6-(2,5-dioxo-2,5-dihydro-1H-pyrrol-1-yl)hexanoyl]-L-valyl-N-[4-({[(2-{[8-(1,3-benzothiazol-2-ylcarbamoyl)-2-(6-carboxy-5-{1-[(3,5-dimethyl-7-{2-[methyl(2-sulfoethyl)amino]ethoxy}tricyclo[3.3.1.1^{3,7}]dec-1-yl)methyl]-5-methyl-1H-pyrazol-4-yl}pyridin-2-yl)-1,2,3,4-tetrahydroisoquinolin-5-yl]oxy}ethyl)(2-carboxyethyl)carbamoyl]oxy}methyl)phenyl]-N⁵-carbamoyl-L-ornithinamide (Synthon JF)

The title compound was prepared as described in Example 2.1, replacing Example 1.2.9 with Example 1.49.2. ¹H NMR (500 MHz, dimethyl sulfoxide-*d*₆) δ ppm 12.71 (s, 1H), 10.00 (s, 1H), 8.97 (s, 1H), 8.08 (d, 1H), 8.02 (d, 1H), 7.78 (d, 2H), 7.72 (d, 1H), 7.60 (d, 2H), 7.52 (d, 1H), 7.44-7.50 (m, 1H), 7.27-7.39 (m, 4H), 6.96-7.06 (m, 3H), 5.98 (s, 1H), 5.01 (d, 4H), 4.31-4.46 (m, 1H), 4.18 (s, 3H), 3.79-3.95 (m, 4H), 3.67-3.76 (m, 2H), 3.12-3.39 (m, 6H), 2.73-3.07 (m, 8H), 2.04-2.24 (m, 4H), 1.87-2.02 (m, 1H), 1.22-1.75 (m, 12H), 0.96-1.20 (m, 7H), 0.76-0.90 (m, 10H). MS (ESI) m/e 1597.4 (M+H)⁺.

### 2.52 Synthesis of N-[6-(2,5-dioxo-2,5-dihydro-1H-pyrrol-1-yl)hexanoyl]-L-valyl-N-{4-[({[2-({3-[(4-{6-[8-(1,3-benzothiazol-2-ylcarbamoyl)-5-(3-sulfopropoxy)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](methyl)carbamoyl}oxy)methyl]phenyl}-N⁵-carbamoyl-L-ornithinamide (Synthon JK)

The title compound was prepared by substituting Example 1.52.4 for Example 2.5.3 in Example 2.5.4. ¹H NMR (400 MHz, dimethyl sulfoxide-*d*₆) δ ppm 9.97 (s, 1H), 7.96-8.11 (m, 2H), 7.67-7.82 (m, 3H), 7.59 (d, 2H), 7.42-7.52 (m, 2H), 7.23-7.36 (m, 4H), 6.91-7.08 (m, 4H), 4.99 (d, 4H), 4.33-4.47 (m, 1H), 4.14-4.23 (m, 4H), 3.86-3.95 (m, 6H), 3.21-3.45 (m, 15H), 2.75-3.07 (m, 9H), 2.56-2.69 (m, 2H), 1.93-2.20 (m, 8H), 0.88-1.72 (m, 20H), 0.74-0.89 (m, 11H). MS (ESI) m/e 1496.3 (M+Na)⁺.

### 2.53 Synthesis of N-[3-(2,5-dioxo-2,5-dihydro-1H-pyrrol-1-yl)propanoyl]-L-valyl-N-{4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)methyl]phenyl}-N⁵-carbamoyl-L-ornithinamide (Synthon JJ)

A solution of Example 2.49.1 (0.030 g), 2,5-dioxopyrrolidin-1-yl 3-(2,5-dioxo-2,5-dihydro-1H-pyrrol-1-yl)propanoate (6.34 mg) and N,N-diisopropylethylamine (0.012 mL) in N,N-dimethylformamide (0.5 mL) was stirred at room temperature. After 1 hour the reaction was quenched with a 3:1 mixture of N,N-dimethylformamide:water (1.5 mL). The mixture was purified by reverse phase HPLC using a Gilson system, eluting with 10-85% acetonitrile in water containing 0.1% v/v trifluoroacetic acid. The desired fractions were combined and freeze-dried to provide the title compound. ¹H NMR (500 MHz, dimethyl sulfoxide-*d*₆) δ ppm 12.85 (s, 1H), 9.99 (s, 1H), 8.18 (q, 1H), 8.12-8.00 (m, 2H), 7.86-7.75 (m, 2H), 7.65-7.55 (m, 3H), 7.53 (dd, 1H), 7.47 (t, 1H), 7.43 (d, 1H), 7.36 (q, 2H), 7.33-7.23 (m, 3H), 6.95 (d, 1H), 6.05 (s, 1H), 5.03-4.92 (m, 4H), 4.39 (q, 1H), 4.24-4.14 (m, 1H), 4.02 (s, 2H), 3.88 (t, 2H), 3.81 (d, 2H), 3.39-3.16 (m, 2H), 3.14-2.86 (m, 10H), 2.68-2.60 (m, 2H), 2.25-2.04 (m, 6H), 2.03-1.90 (m, 1H), 1.78-1.65 (m, 1H), 1.64-1.54 (m, 1H), 1.54-0.90 (m, 20H), 0.89-0.75 (m, 12H). MS (ESI) m/e 1410.1 (M+H)⁺.

### 2.54 Synthesis of N-[(2,5-dioxo-2,5-dihydro-1H-pyrrol-1-yl)acetyl]-L-valyl-N-{4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)methyl]phenyl}-N⁵-carbamoyl-L-ornithinamide (Synthon JL)

A solution of Example 2.49.1 (0.039 g), 2,5-dioxopyrrolidin-1-yl 2-(2,5-dioxo-2,5-dihydro-1H-pyrrol-1-yl)acetate (7.81 mg) and N,N-diisopropylethylamine (0.016 mL) in N,N-dimethylformamide (0.5 mL) was stirred at room temperature. After 1 hour, the reaction was quenched with a 3:1 mixture of N,N-dimethylformamide:water (1.5 mL). The mixture was purified by reverse phase HPLC using a Gilson system, eluting with 10-85% acetonitrile in water containing 0.1% v/v trifluoroacetic acid. The desired fractions were combined and freeze-dried to provide the title compound. ¹H NMR (400 MHz, dimethyl sulfoxide-*d*₆) δ ppm 12.85 (s, 1H), 10.00 (d, 1H), 8.24 (d, 2H), 8.04 (d, 1H), 7.79 (d, 1H), 7.59 (q, 3H), 7.53 (dd, 1H), 7.47 (t, 1H), 7.43 (d, 1H), 7.36 (td, 2H), 7.30 (s, 1H), 7.27 (d, 2H), 7.07 (s, 2H), 6.96 (d, 1H), 5.04-4.85 (m, 4H), 4.39 (q, 2H), 4.26 (dd, 2H), 4.13 (s, 2H), 3.86-3.17 (m, 8H), 3.07-2.81 (m, 4H), 2.63 (t, 2H), 2.09 (s, 3H), 2.03-1.79 (m, 1H), 1.75-1.51 (m, 2H), 1.51-1.03 (m, 12H), 1.01-0.76 (m, 16H). MS (ESI) m/e 1394.4 (M-H)⁻.

### 2.55 Synthesis of 6-[8-(1,3-benzothiazol-2-ylcarbamoyl)-3,4-dihydroisoquinolin-2(1H)-yl]-3-(1-{[3-(2-{[(2S)-2-({[(4-{[(2S,3R,4S,5S,6S)-6-carboxy-3,4,5-trihydroxytetrahydro-2H-pyran-2-yl]oxy}-3-[(3-{[6-(2,5-dioxo-2,5-dihydro-1H-pyrrol-1-yl)hexanoyl]amino}propanoyl)amino]benzyl)oxy]carbonyl}amino)-3-sulfopropanoyl](methyl)amino}ethoxy)-5,7-dimethyltricyclo[3.3.1.1^{3,7}]dec-1-yl]methyl}-5-methyl-1H-pyrazol-4-yl)pyridine-2-carboxylic acid (Synthon FE) 2.55.1 (2S,3R,4S,5S,6S)-2-(4-formyl-2-nitrophenoxy)-6-(methoxycarbonyl)tetrahydro-2H-pyran-3,4,5-triyl triacetate

To a solution of (2R,3R,4S,5S,6S)-2-bromo-6-(methoxycarbonyl)tetrahydro-2H- pyran-3,4,5-triyl triacetate (4 g) in acetonitrile (100 mL)) was added silver(I) oxide (10.04 g) and 4-hydroxy-3-nitrobenzaldehyde (1.683 g). The reaction mixture was stirred for 4 hours at room temperature and filtered. The filtrate was concentrated, and the residue was purified by silica gel chromatography, eluting with 5-50% ethyl acetate in heptanes, to provide the title compound. MS (ESI) m/e (M+18)⁺.

### 2.55.2 (2S,3R,4S,5S,6S)-2-(4-(hydroxymethyl)-2-nitrophenoxy)-6-(methoxycarbonyl)tetrahydro-2H-pyran-3,4,5-triyl triacetate

To a solution of Example 2.55.1 (6 g) in a mixture of chloroform (75 mL) and isopropanol (18.75 mL) was added 0.87 g of silica gel. The resulting mixture was cooled to 0 °C, NaBH₄ (0.470 g) was added, and the resulting suspension was stirred at 0 °C for 45 minutes. The reaction mixture was diluted with dichloromethane (100 mL) and filtered through diatomaceous earth. The filtrate was washed with water and brine and concentrated to give the crude product, which was used without further purification. MS (ESI) m/e (M+NH₄)⁺:

### 2.55.3 (2S,3R,4S,5S,6S)-2-(2-amino-4-(hydroxymethyl)phenoxy)-6-(methoxycarbonyl)tetrahydro-2H-pyran-3,4,5-triyl triacetate

A stirred solution of Example 2.55.2 (7 g) in ethyl acetate (81 mL) was hydrogenated at 20 °C under 1 atmosphere H₂, using 10% Pd/C (1.535 g) as a catalyst for 12 hours. The reaction mixture was filtered through diatomaceous earth, and the solvent was evaporated under reduced pressure. The residue was purified by silica gel chromatography, eluting with 95/5 dichloromethane/methanol, to give the title compound.

### 2.55.4 3-((((9H-fluoren-9-yl)methoxy)carbonyl)amino)propanoic acid

3-Aminopropanoic acid (4.99 g) was dissolved in 10% aqueous Na₂CO₃ solution (120 mL) in a 500 mL flask and cooled with an ice bath. To the resulting solution, (9H-fluoren-9-yl)methyl carbonochloridate (14.5 g) in 1,4-dioxane (100 mL) was gradually added. The reaction mixture was stirred at room temperature for 4 hours, and water (800 mL) was then added. The aqueous phase layer was separated from the reaction mixture and washed with diethyl ether (3 x 750 mL). The aqueous layer was acidified with 2N HCl aqueous solution to a pH value of 2 and extracted with ethyl acetate (3 x 750 mL). The organic layers were combined and concentrated to obtain crude product. The crude product was recrystallized in a mixed solvent of ethyl acetate: hexane 1:2 (300 mL) to give the title compound.

### 2.55.5 (9H-fluoren-9-yl)methyl (3-chloro-3-oxopropyl)carbamate

To a solution of Example 2.55.4 in dichloromethane (160 mL) was added sulfurous dichloride (50 mL). The mixture was stirred at 60 °C for 1 hour. The mixture was cooled and concentrated to give the title compound.

### 2.55.6 (2S,3R,4S,5S,6S)-2-(2-(3-((((9H-fluoren-9-yl)methoxy)carbonyl)amino)propanamido)-4-(hydroxymethyl)phenoxy)-6-(methoxycarbonyl)tetrahydro-2H-pyran-3,4,5-triyl triacetate

To a solution of Example 2.55.3 (6 g) in dichloromethane (480 mL) was added N,N-diisopropylethylamine (4.60 mL). Example 2.55.5 (5.34 g) was added, and the mixture was stirred at room temperature for 30 minutes. The mixture was poured into saturated aqueous sodium bicarbonate and was extracted with ethyl acetate. The combined extracts were washed with water and brine and were dried over sodium sulfate. Filtration and concentration gave a residue that was purified via radial chromatography, using 0-100% ethyl acetate in petroleum ether as mobile phase, to give the title compound.

### 2.55.7 (2S,3R,4S,5S,6S)-2-(2-(3-((((9H-fluoren-9-yl)methoxy)carbonyl)amino)propanamido)-4-((((4-nitrophenoxy)carbonyl)oxy)methyl)phenoxy)-6-(methoxycarbonyl)tetrahydro-2H-pyran-3,4,5-triyl triacetate

To a mixture of Example 2.55.6 (5.1 g) in N,N-dimethylformamide (200 mL) was added bis(4-nitrophenyl) carbonate (4.14 g) and N,N-diisopropylethylamine (1.784 mL). The mixture was stirred for 16 hours at room temperature and concentrated under reduced pressure. The crude material was dissolved in dichloromethane and aspirated directly onto a 1 mm radial Chromatotron plate and eluted with 50-100% ethyl acetate in hexanes to give the title compound. MS (ESI) m/e (M+H)⁺.

### 2.55.8 3-(1-((3-(2-((R)-2-((((3-(3-aminopropanamido)-4-(((2S,3R,4S,5S,6S)-6-carboxy-3,4,5-trihydroxytetrahydro-2H-pyran-2-yl)oxy)benzyl)oxy)carbonyl)amino)-N-methyl-3-sulfopropanamido)ethoxy)-5,7-dimethyladamantan-1-yl)methyl)-5-methyl-1H-pyrazol-4-yl)-6-(8-(benzo[d]thiazol-2-ylcarbamoyl)-3,4-dihydroisoquinolin-2(1H)-yl)picolinic acid

A solution of Example 1.13.7 (0.055 g) and Example 2.55.7 (0.055 g) were stirred together in N,N-dimethylformamide (1.5 mL) and N,N-diisopropylethylamine (0.053 mL) was added. After stirring for 3 hours, the reaction was diluted with ethyl acetate (75 mL) and washed with water (20 mL) and brine (25 mL), dried over magnesium sulfate, filtered, and concentrated. The residue was dissolved in methanol (1 mL) and treated with lithium hydroxide hydrate (0.025 g) in water (0.6 mL). After stirring for 2 hours, the reaction was quenched with trifluoroacetic acid (0.047 ml) and diluted with N,N-dimethylformamide (1 mL). The mixture was purified by reverse phase HPLC using a Gilson system, eluting with 10-80% acetonitrile in water containing 0.1% v/v trifluoroacetic acid. The desired fractions were combined and freeze-dried to provide the title compound as a trifluoroacetic acid salt.

### 2.55.9 6-[8-(1,3-benzothiazol-2-ylcarbamoyl)-3,4-dihydroisoquinolin-2(1H)-yl]-3-(1-{[3-(2-{[(2S)-2-({[(4-{[(2S,3R,4S,5S,6S)-6-carboxy-3,4,5-trihydroxytetrahydro-2H-pyran-2-yl]oxy}-3-[(3-{[6-(2,5-dioxo-2,5-dihydro-1H-pyrrol-1-yl)hexanoyl]amino}propanoyl)amino]benzyl)oxy]carbonyl}ami no)-3-sulfopropanoyl](methyl)amino}ethoxy)-5,7-dimethyltricyclo[3.3.1.1^{3,7}]dec-1-yl]methyl}-5-methyl-1H-pyrazol-4-yl)pyridine-2-carboxylic acid

A solution of Example 2.55.8 (0.013 g) and 2,5-dioxopyrrolidin-1-yl 6-(2,5-dioxo-2,5-dihydro-1H-pyrrol-1-yl)hexanoate (3.07 mg) were stirred in N,N-dimethylformamide (1 mL) and N,N-diisopropylethylamine (7.90 µL) was added. The reaction was stirred for 1 hour and diluted with N,N-dimethylformamide and water. The mixture was purified by reverse phase HPLC using a Gilson system, eluting with 10-75% acetonitrile in water containing 0.1% v/v trifluoroacetic acid. The desired fractions were combined and freeze-dried to provide the title compound. ¹H NMR (400 MHz, dimethyl sulfoxide-*d*₆) δ ppm 12.84 (s, 1H), 9.07 (s, 1H), 8.15 (s, 1H), 8.04 (d, 1H), 7.89 (t, 1H), 7.79 (d, 1H), 7.61 (d, 1H), 7.56-7.50 (m, 1H), 7.47 (t, 1H), 7.43 (d, 1H), 7.39-7.32 (m, 2H), 7.31 (s, 1H), 7.28 (d, 1H), 7.06 (d, 1H), 7.04-6.92 (m, 4H), 5.00-4.79 (m, 5H), 4.73-4.64 (m, 1H), 3.94-3.78 (m, 4H), 3.57-2.84 (m, 12H), 2.84-2.56 (m, 6H), 2.14-1.73 (m, 5H), 1.57-0.89 (m, 22H), 0.84 (s, 6H). MS (ESI) m/e 1516.2 (M-H)⁻.

### 2.56 Synthesis of 4-[(1E)-3-({[2-({3-[(4-{2-carboxy-6-[8-([1,3]thiazolo[5,4-b]pyridin-2-ylcarbamoyl)-3,4-dihydroisoquinolin-2(1H)-yl]pyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)prop-1-en-1-yl]-2-({N-[6-(2,5-dioxo-2,5-dihydro-1H-pyrrol-1-yl)hexanoyl]-beta-alanyl}amino)phenyl beta-D-glucopyranosiduronic acid (Synthon GG)

### 2.56.1 3-(1-((3-(2-(((((E)-3-(3-(3-aminopropanamido)-4-(((2S,3R,4S,5S,6S)-6-carboxy-3,4,5-trihydroxytetrahydro-2H-pyran-2-yl)oxy)phenyl)allyl)oxy)carbonyl)(2-sulfoethyl)amino)ethoxy)-5,7-dimethyladamantan-1-yl)methyl)-5-methyl-1H-pyrazol-4-yl)-6-(8-(thiazolo[5,4-b]pyridin-2-ylcarbamoyl)-3,4-dihydroisoquinolin-2(1H)-yl)picolinic acid

Example 1.22.5 (48 mg) was dissolved in dimethylformamide (0.5 mL), and Example 2.44.7 (55 mg) and N,N-diisopropylethylamine (90 µL) were added. The reaction mixture was stirred at room temperature overnight. The reaction was concentrated, and the residue was dissolved in methanol (1 mL) and 1.94*N* aqueous LiOH (0.27 mL) was added. The mixture was stirred at room temperature for one hour. Purification of the mixture by reverse phase chromatography (C18 column), eluting with 10-90% acetonitrile in water containing 0.1% v/v trifluoroacetic acid, provided the title compound as a trifluoroacetic acid salt. MS (ESI-) m/e 1291.4 (M-H)⁻.

### 2.56.2 4-[(1E)-3-({[2-({3-[(4-{2-carboxy-6-[8-([1,3]thiazolo[5,4-b]pyridin-2-ylcarbamoyl)-3,4-dihydroisoquinolin-2(1H)-yl]pyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)prop-1-en-1-yl]-2-({N-[6-(2,5-dioxo-2,5-dihydro-1H-pyrrol-1-yl)hexanoyl]-beta-alanyl}amino)phenyl beta-D-glucopyranosiduronic acid

The title compound was prepared by substituting Example 1.56.1 for Example 1.2.9 in Example 2.1. ¹H NMR (400 MHz, dimethyl sulfoxide-*d*₆) δ ppm 13.00 (v br s, 1H), 9.03 (s, 1H), 8.53 (dd, 1H), 8.24 (s, 1H), 8.16 (dd, 1H), 7.90 (br s, 1H), 7.61 (d, 1H), 7.54 (d, 1H) 7.52 (d, 1H), 7.44 (d, 1H), 7.37 (t, 1H), 7.30 (s, 1H), 7.11 (br d, 1H), 7.03 (d, 1H), 6.98 (s, 2H), 6.97 (d, 1H), 6.58 (m, 1H), 6.15 (m,1H), 4.96 (s, 2H), 4.88 (br m, 1 H), 4.64 (br m, 2H), 3.88 (m, 3H), 3.79 (br m, 2H), 3.27-3.48 (m, 14H), 3.01 (m, 2H), 2.67 (br m, 2H), 2.54 (m, 2H), 2.09 (s, 3H), 2.03 (t, 2H), 1.45 (m, 6H), 1.37 (br m, 2H), 1.28-0.90 (m, 10H), 0.77-0.82 (m, 6H). MS (ESI) m/e 1484.4 (M-H)⁻.

### 2.57 Synthesis of 4-[(1E)-3-({[2-({3-[(4-{2-carboxy-6-[8-([1,3]thiazolo[4,5-b]pyridin-2-ylcarbamoyl)-3,4-dihydroisoquinolin-2(1H)-yl]pyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)prop-1-en-1-yl]-2-({N-[6-(2,5-dioxo-2,5-dihydro-1H-pyrrol-1-yl)hexanoyl]-beta-alanyl}amino)phenyl beta-D-glucopyranosiduronic acid (Synthon GM) 2.57.13-(1-((3-(2-(((((E)-3-(3-(3-aminopropanamido)-4-(((2S,3R,4S,5S,6S)-6-carboxy-3,4,5-trihydroxytetrahydro-2H-pyran-2-yl)oxy)phenyl)allyl)oxy)carbonyl)(2-sulfoethyl)amino)ethoxy)-5,7-dimethyladamantan-1-yl)methyl)-5-methyl-1H-pyrazol-4-yl)-6-(8-(thiazolo[4,5-b]pyridin-2-ylcarbamoyl)-3,4-dihydroisoquinolin-2(1H)-yl)picolinic acid

The title compound was prepared by substituting Example 1.23.4 for Example 1.22.5 in Example 2.56.1. MS (ESI) m/e 1291.4 (M-H)⁻.

### 2.57.24-[(1E)-3-({[2-({3-[(4-{2-carboxy-6-[8-([1,3]thiazolo[4,5-b]pyridin-2-ylcarbamoyl)-3,4-dihydroisoquinolin-2(1H)-yl]pyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)prop-1-en-1-yl]-2-({N-[6-(2,5-dioxo-2,5-dihydro-1H-pyrrol-1-yl)hexanoyl]-beta-alanyl}amino)phenyl beta-D-glucopyranosiduronic acid

The title compound was prepared by substituting Example 1.57.1 for Example 1.2.9 in Example 2.1. ¹H NMR (500 MHz, dimethyl sulfoxide-*d*₆) δ ppm 9.03 (s, 1H), 8.72 (d, 1H), 8.66 (d, 1H), 8.25 (s, 1H), 7.89 (br m, 1H), 7.65 (d, 1H), 7.52 (br m, 2H), 7.46 (d, 1H), 7.39 (t, 1H), 7.30 (s, 1H), 7.11 (br d, 1H), 7.03 (d, 1H), 6.98 (s, 2H), 6.97 (d, 1H), 6.58 (m, 1H), 6.15 (m,1H), 4.96 (s, 2H), 4.88 (br m, 1 H), 4.64 (br m, 2H), 3.88 (m, 3H), 3.79 (br m, 2H), 3.27-3.48 (m, 14H), 3.01 (m, 2H), 2.67 (br m, 2H), 2.54 (m, 2H), 2.09 (s, 3H), 2.03 (t, 2H), 1.45 (m, 6H), 1.37 (br m, 2H), 1.28-0.90 (m, 10H), 0.77-0.82 (m, 6H). MS (ESI) m/e 1484.4 (M-H)⁻.

### 2.58 Synthesis of 4-[(1E)-3-({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)prop-1-en-1-yl]-2-({N-[6-(2,5-dioxo-2,5-dihydro-1H-pyrrol-1-yl)hexanoyl]-beta-alanyl}amino)phenyl beta-D-glucopyranosiduronic acid (Synthon HD) 2.58.13-(1-((3-(2-(((((E)-3-(3-(3-aminopropanamido)-4-(((2S,3R,4S,5S,6S)-6-carboxy-3,4,5-trihydroxytetrahydro-2H-pyran-2-yl)oxy)phenyl)allyl)oxy)carbonyl)(2-sulfoethyl)amino)ethoxy)-5,7-dimethyladamantan-1-yl)methyl)-5-methyl-1H-pyrazol-4-yl)-6-(8-(benzo[d]thiazol-2-ylcarbamoyl)-3,4-dihydroisoquinolin-2(1H)-yl)picolinic acid

The title compound was prepared by substituting Example 1.2.9 for Example 1.22.5 in Example 2.56.1. MS (ESI-) m/e 1290.2 (M-H)⁻.

### 2.58.2 4-[(1E)-3-({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)prop-1-en-1-yl]-2-({N-[6-(2,5-dioxo-2,5-dihydro-1H-pyrrol-1-yl)hexanoyl]-beta-alanyl}amino)phenyl beta-D-glucopyranosiduronic acid

The title compound was prepared by substituting Example 1.58.1 for Example 1.56.1 in Example 2.56.2. ¹H NMR (400 MHz, dimethyl sulfoxide-*d*₆) δ ppm 9.03 (s, 1H), 8.25 (s, 1H), 8.03 (d, 1H), 7.89 (br m, 1H), 7.79 (d, 1H), 7.61 (d, 1H), 7.53 (br m, 1H), 7.46 (m, 2H), 7.37 (m, 2H), 7.32 (s, 1H), 7.11 (br d, 1H), 7.03 (d, 1H), 6.98 (s, 2H), 6.97 (d, 1H), 6.58 (m, 1H), 6.15 (m,1H), 4.96 (s, 2H), 4.88 (br m, 1 H), 4.64 (br m, 2H), 3.88 (m, 3H), 3.79 (br m, 2H), 3.27-3.48 (m, 14H), 3.01 (m, 2H), 2.67 (br m, 2H), 2.54 (m, 2H), 2.09 (s, 3H), 2.03 (t, 2H), 1.45 (m, 6H), 1.37 (br m, 2H), 1.28-0.90 (m, 10H), 0.77-0.82 (m, 6H). MS (ESI-) m/e 1483.3 (M-H)⁻.

### 2.59 Synthesis of 4-[(1E)-3-({[2-({3-[(4-{2-carboxy-6-[8-([1,3]thiazolo[5,4-b]pyridin-2-ylcarbamoyl)-3,4-dihydroisoquinolin-2(1H)-yl]pyridin-3-yl}-5-methyl-1H-pyrazol-1-yl)methyl]-5,7-dimethyltricyclo[3.3.1.1^{3,7}]dec-1-yl}oxy)ethyl](3-phosphonopropyl)carbamoyl}oxy)prop-1-en-1-yl]-2-({N-[6-(2,5-dioxo-2,5-dihydro-1H-pyrrol-1-yl)hexanoyl]-beta-alanyl}amino)phenyl beta-D-glucopyranosiduronic acid (Synthon HS)

### 2.59.1 3-(1-((3-(2-(((((E)-3-(3-(3-aminopropanamido)-4-(((2S,3R,4S,5S,6S)-6-carboxy-3,4,5-trihydroxytetrahydro-2H-pyran-2-yl)oxy)phenyl)allyl)oxy)carbonyl)(3-phosphonopropyl)amino)ethoxy)-5,7-dimethyladamantan-1-yl)methyl)-5-methyl-1H-pyrazol-4-yl)-6-(8-(thiazolo[5,4-b]pyridin-2-ylcarbamoyl)-3,4-dihydroisoquinolin-2(1H)-yl)picolinic acid

The title compound was prepared by substituting Example 1.40.2 for Example 1.22.5 in Example 2.56.1. MS (ESI-) m/e 1305.4 (M-H)⁻.

### 2.59.2 4-[(1E)-3-({[2-({3-[(4-{2-carboxy-6-[8-([1,3]thiazolo[5,4-b]pyridin-2-ylcarbamoyl)-3,4-dihydroisoquinolin-2(1H)-yl]pyridin-3-yl}-5-methyl-1H-pyrazol-1-yl)methyl]-5,7-dimethyltricyclo[3.3.1.1^{3,7}] dec-1-yl}oxy)ethyl] (3-phosphonopropyl)carbamoyl}oxy)prop-1-en-1-yl]-2-({N-[6-(2,5-dioxo-2,5-dihydro-1H-pyrrol-1-yl)hexanoyl]-beta-alanyl}amino)phenyl beta-D-glucopyranosiduronic acid

The title compound was prepared by substituting Example 1.59.1 for Example 1.56.1 in Example 2.56.2. ¹H NMR (400 MHz, dimethyl sulfoxide-*d*₆) δ ppm 9.03 (s, 1H), 8.53 (dd, 1H), 8.24 (s, 1H), 8.16 (dd, 1H), 7.90 (br s, 1H), 7.61 (d, 1H), 7.54 (d, 1H) 7.52 (d, 1H), 7.44 (d, 1H), 7.37 (t, 1H), 7.28 (s, 1H), 7.11 (br d, 1H), 7.03 (d, 1H), 6.98 (s, 2H), 6.97 (d, 1H), 6.56 (m, 1H), 6.16 (m,1H), 4.96 (s, 2H), 4.86 (br m, 1 H), 4.64 (br d, 2H), 3.88 (m, 3H), 3.79 (br m, 2H), 3.27-3.44 (m, 14H), 3.01 (m, 2H), 2.54 (m, 2H), 2.08 (s, 3H), 2.03 (t, 2H), 1.46 (m, 6H), 1.37 (br m, 2H), 1.28-0.90 (m, 10H), 0.77-0.82 (m, 6H). MS (ESI) m/e 1498.4 (M-H)⁻.

### 2.60 Synthesis of 4-[(1E)-3-({[2-({3-[(4-{6-[8-(1,3-benzothiazol-2-ylcarbarmoyl)-5-(3-phosphonopropoxy)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}] dec-1-yl}oxy)ethyl](methyl)carbamoyl}oxy)prop-1-en-1-yl]-2-({N-[6-(2,5-dioxo-2,5-dihydro-1H-pyrrol-1-yl)hexanoyl]-beta-alanyl}amino)phenyl beta-D-glucopyranosiduronic acid (Synthon HW) 2.60.13-(1-(((3-(2-(((((E)-3-(3-(3-aminopropanamido)-4-(((2S,3R,4S,5S,6S)-6-carboxy-3,4,5-trihydroxytetrahydro-2H-pyran-2-yl)oxy)phenyl)allyl)oxy)carbonyl)(methyl)amino)ethoxy)-5,7-dimethyladamantan-1-yl)methyl)-5-methyl-1H-pyrazol-4-yl)-6-(8-(benzo[d]thiazol-2-ylcarbam oyl)-5-(3-phosphon opropoxy)-3,4-dihydroisoquinolin-2(1H)-yl)picolinic acid

The title compound was prepared by substituting Example 1.31.11 for Example 1.22.5 in Example 2.56.1. MS (ESI) m/e 1336.2 (M+Na)⁺.

### 2.60.2 4- [(1E)-3-({[2-({3-[(4-{6-[8-(1,3-benzothiazol-2-ylcarbamoyl)-5-(3-phosphonopropoxy)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}] dec-1-yl}oxy)ethyl](methyl)carbamoyl}oxy)prop-1-en-1-yl]-2-({N-[6-(2,5-dioxo-2,5-dihydro-1H-pyrrol-1-yl)hexanoyl]-beta-alanyl}amino)phenyl beta-D-glucopyranosiduronic acid

The title compound was prepared by substituting Example 1.60.1 for Example 1.56.1 in Example 2.56.2. ¹H NMR (400 MHz, dimethyl sulfoxide-*d*₆) δ ppm 9.03 (s, 1H) 8.25 (s, 1H), 8.01 (d, 1H), 7.83-7.91 (m, 1H), 7.75 (dd, 2H), 7.42-7.58 (m, 2H), 7.34 (t, 1H), 7.28 (s, 1H), 6.93-7.15 (m, 6H), 6.56 (d, 1H), 6.09-6.24 (m, 1H), 5.01 (s, 3H), 4.80-4.92 (m, 2H), 4.57-4.69 (m, 3H), 4.12-4.21 (m, 6H), 3.86-3.94 (m, 7H), 3.28-3.47 (m, 12H), 2.77-2.96 (m, 6H), 2.52-2.58 (m, 2H), 2.09 (s, 3H), 1.90-2.05 (m, 4H) , 1.65-1.78 (m, 2H), 0.90-1.53 (m, 16H), 0.80 (m, 6H). MS (ESI) m/e 1529.5 (M+H)⁺.

### 2.61 Synthesis of 4-[(1E)-3-({[2-({3-[(4-{6-[8-(1,3-benzothiazol-2-ylcarbarnoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](3-phosphonopropyl)carbamoyl}oxy)prop-1-en-1-yl]-2-({N-[6-(2,5-dioxo-2,5-dihydro-1H-pyrrol-1-yl)hexanoyl]-beta-alanyl}amino)phenyl beta-D-glucopyranosiduronic acid (Synthon HX)

### 2.61.1 3-(l-((3-(2-(((((E)-3-(3-(3-aminopropanamido)-4-(((2S,3R,4S,5S,6S)-6-carboxy-3,4,5-trihydroxytetrahydro-2H-pyran-2-yl)oxy)phenyl)allyl)oxy)carbonyl)(3-phosphonopropyl)amino)ethoxy)-5,7-dimethyladamantan-1-yl)methyl)-5-methyl-1H-pyrazol-4-yl)-6-(8-(benzo[d]thiazol-2-ylcarbarnoyl)-3,4-dihydroisoquinolin-2(1H)-yl)picolinic acid

The title compound was prepared by substituting Example 1.14.4 for Example 1.22.5 in Example 2.56.1. MS (ESI) m/e 1304.3 (M-H)⁻.

### 2.61.2 4-[(1E)-3-({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](3-phosphonopropyl)carbamoyl}oxy)prop-1-en-1-yl]-2-({N- [6-(2,5-dioxo-2,5-dihydro-1H-pyrrol-1-yl)hexanoyl]-beta-alanyl}amino)phenyl beta-D-glucopyranosiduronic acid

The title compound was prepared by substituting Example 1.61.1 for Example 1.56.1 in Example 2.56.2. ¹H NMR (400 MHz, dimethyl sulfoxide-*d*₆) δ ppm 9.03 (s, 1H), 8.25 (br s, 1H), 8.03 (d, 1H), 7.89 (br m, 1H), 7.79 (d, 1H), 7.61 (d, 1H), 7.53 (br m, 1H), 7.46 (m, 2H), 7.37 (m, 2H), 7.28 (s, 1H), 7.11 (br d, 1H), 7.03 (d, 1H), 6.98 (s, 2H), 6.97 (d, 1H), 6.56 (m, 1H), 6.17 (m,1H), 4.96 (s, 2H), 4.86 (br m, 1H), 4.64 (br d, 2H), 3.88 (m, 3H), 3.79 (br m, 2H), 3.27-3.44 (m, 14H), 3.01 (m, 2H), 2.54 (m, 2H), 2.08 (s, 3H), 2.03 (t, 2H), 1.46 (m, 6H), 1.37 (br m, 2H), 1.28-0.90 (m, 10H), 0.77-0.82 (m, 6H). MS (ESI-) m/e 1497.4 (M-H)⁻.

### 2.62 Synthesis of 4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)methyl]-3-[2-(2-{[3-(2,5-dioxo-2,5-dihydro-1H-pyrrol-1-yl)propanoyl]amino}ethoxy)ethoxy]phenyl beta-D-glucopyranosiduronic acid (Synthon HY)

### 2.62.1 (2S,3R,4S,5S,6S)-2-(4-formyl-3-hydroxyphenoxy)-6-(methoxycarbonyl)tetrahydro-2H-pyran-3,4,5-triyl triacetate

2,4-Dihydroxybenzaldehyde (15 g) and (2S,3R,4S,5S,6S)-2-bromo-6-(methoxycarbonyl)tetrahydro-2H-pyran-3,4,5-triyl triacetate (10 g) were dissolved in acetonitrile followed by the addition of silver carbonate (10 g) and the reaction was heated to 49°C. After stirring for 4 hours, the reaction was cooled, filtered and concentrated. The crude title compound was suspended in dichloromethane and was filtered through diatomaceous earth and concentrated. The residue was purified by silica gel chromatography eluting with 1-100% ethyl acetate/heptane to provide the title compound.

### 2.62.2 (2S,3R,4S,5S,6S)-2-(3-hydroxy-4-(hydroxymethyl)phenoxy)-6-(methoxycarbonyl)tetrahydro-2H-pyran-3,4,5-triyl triacetate

A solution of Example 2.62.1 (16.12 g) in tetrahydrofuran (200 mL) and methanol (200 mL) was cooled to 0°C and sodium borohydride (1.476 g) was added portionwise. The reaction was stirred for 20 minutes and was quenched with a 1:1 mixture of water:aqueous saturated sodium bicarbonate solution (400 mL). The resulting solids were filtered off and rinsed with ethyl acetate. The phases were separated and the aqueous layer was extracted four times with ethyl acetate. The combined organic layers were dried over magnesium sulfate, filtered, and concentrated. The crude title compound was purified via silica gel chromatography eluting with 1-100% ethyl acetate/heptanes to provide the title compound. MS (ESI) m/e 473.9 (M+NH₄)⁺.

### 2.62.3 (2S,3R,4S,5S,6S)-2-(4-(((tert-butyldimethylsilyl)oxy)methyl)-3-hydroxyphenoxy)-6-(methoxycarbonyl)tetrahydro-2H-pyran-3,4,5-triyl triacetate

Example 2.62.2 (7.66 g) and *tert*-butyldimethylsilyl chloride (2.78 g) in dichloromethane (168 mL) at-5°C was added imidazole (2.63 g) and the reaction was stirred overnight allowing the internal temperature of the reaction to warm to 12°C. The reaction mixture was poured into saturated aqueous ammonium chloride and extracted four times with dichloromethane. The combined organics were washed with brine, dried over magnesium sulfate, filtered and concentrated. The crude title compound was purified via silica gel chromatography eluting with 1-50% ethyl acetate/heptanes to provide the title compound. MS (ESI) m/e 593.0 (M+Na)⁺.

### 2.62.4 (2S,3R,4S,5S,6S)-2-(3-(2-(2-((((9H-fluoren-9-yl)methoxy)carbonyl)amino)ethoxy)ethoxy)-4-(((tert-butyldimethylsilyl)oxy)methyl)phenoxy)-6-(methoxycarbonyl)tetrahydro-2H-pyran-3,4,5-triyl triacetate

To Example 2.62.3 (5.03 g) and triphenylphosphine (4.62 g) in toluene (88 mL) was added di-*tert*-butyl-azodicarboxylate (4.06 g) and the reaction was stirred for 30 minutes. (9H-Fluoren-9-yl)methyl (2-(2-hydroxyethoxy)ethyl)carbamate was added and the reaction was stirred for an addition 1.5 hours. The reaction was loaded directly onto silica gel and was eluted with 1-50% ethyl acetate/heptanes to provide the title compound.

### 2.62.5 (2S,3R,4S,5S,6S)-2-(3-(2-(2-((((9H-fluoren-9-yl)methoxy)carbonyl)amino)ethoxy)ethoxy)-4-(hydroxymethyl)phenoxy)-6-(meth oxycarbonyl)tetrahydro-2H-pyran-3,4,5-triyl triacetate

Example 2.62.4 (4.29 g) was stirred in a 3:1:1 solution of acetic acid:water:tetrahydrofuran (100 mL) overnight. The reaction was poured into saturated aqueous sodium bicarbonate and extracted with ethyl acetate. The organic layer was dried over magnesium sulfate, filtered and concentrated. The crude title compound was purified via silica gel chromatography, eluting with 1-50% ethyl acetate/heptanes to provide the title compound.

### 2.62.6 (2S,3R,4S,5S,6S)-2-(3-(2-(2-((((9H-fluoren-9-yl)methoxy)carbonyl)amino)ethoxy)ethoxy)-4-((((4-nitrophenoxy)carbonyl)oxy)methyl)phenoxy)-6-(methoxycarbonyl)tetrahydro-2H-pyran-3,4,5-triyl triacetate

To a solution of Example 2.62.5 (0.595 g) and bis(4-nitrophenyl) carbonate (0.492 g) in N,N-dimethylformamide (4 mL) was added N-ethyl-N-isopropylpropan-2-amine (0.212 mL). After 1.5 hours, the reaction was concentrated under high vacuum. The reaction was loaded directly onto silica gel and eluted using 1-50% ethyl acetate/heptanes to provide the title compound. MS (ESI) m/e 922.9 (M+Na)⁺.

### 2.62.7 3-(1-((3-(2-((((2-(2-(2-aminoethoxy)ethoxy)-4-(((2S,3R,4S,5S,6S)-6-carboxy-3,4,5-trihydroxytetrahydro-2H-pyran-2-yl)oxy)benzyl)oxy)carbonyl)(2-sulfoethyl)amino)ethoxy)-5,7-dimethyladamantan-1-yl)methyl)-5-methyl-1H-pyrazol-4-yl)-6-(8-(benzo[d]thiazol-2-ylcarbamoyl)-3,4-dihydroisoquinolin-2(1H)-yl)picolinicacid

To a solution of Example 1.2.9 (0.073 g) and Example 2.62.6 (0.077 g) in N,N-dimethylformamide (0.5 mL) was added N,N-diisopropylethylamine (0.066 mL), and the reaction was stirred overnight. The reaction was concentrated, and the residue was dissolved in tetrahydrofuran (0.5 mL) and methanol (0.5 mL) and treated with lithium hydroxide monohydrate (0.047 g) as a solution in water (0.5 mL). After 1 hour, the reaction was diluted with N,N-dimethylformamide and water and was quenched by the addition of trifluoroacetic acid (0.116 mL). The mixture was purified by reverse phase HPLC using a Gilson system, eluting with 10-75% acetonitrile in water containing 0.1% v/v trifluoroacetic acid. The desired fractions were combined and freeze-dried to provide the title compound.

### 2.62.8 4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)methyl]-3-[2-(2-{[3-(2,5-dioxo-2,5-dihydro-1H-pyrrol-1-yl)propanoyl]amino}ethoxy)ethoxy]phenyl beta-D-glucopyranosiduronic acid

A solution of Example 2.62.7 (0.053 g), 2,5-dioxopyrrolidin-1-yl 3-(2,5-dioxo-2,5-dihydro-1H-pyrrol-1-yl)propanoate (0.012 g) and N,N-diisopropylethylamine (0.033 mL) in N,N-dimethylformamide (0.75 mL) was stirred at room temperature. After stirring for 1 hour, the reaction was diluted with N,N-dimethylformamide and water. The mixture was purified by reverse phase HPLC using a Gilson system, eluting with 10-75% acetonitrile in water containing 0.1% v/v trifluoroacetic acid. The desired fractions were combined and freeze-dried to provide the title compound. ¹H NMR (400 MHz, dimethyl sulfoxide-*d*₆) δ ppm 12.85 (s, 1H), 8.04 (d, 2H), 7.79 (d, 1H), 7.61 (d, 1H), 7.54 (d, 1H), 7.51-7.40 (m, 2H), 7.40-7.31 (m, 3H), 7.20 (d, 1H), 7.00-6.94 (m, 3H), 6.73-6.57 (m, 2H), 5.06 (t, 1H), 5.01-4.91 (m, 4H), 3.96-3.85 (m, 2H), 3.85-3.78 (m, 2H), 3.78-3.69 (m, 2H), 3.59 (t, 2H), 3.53-3.34 (m, 6H), 3.34-3.21 (m, 4H), 3.17 (q, 2H), 3.02 (t, 2H), 2.66 (t, 2H), 2.33 (t, 2H), 2.10 (s, 3H), 1.44-0.90 (m, 16H), 0.83 (d, 6H). MS (-ESI) m/e 1432.4 (M-H)⁻.

### 2.63 Synthesis of 4-[(1E)-3-({[2-({3-[(4-{2-carboxy-6-[8-([1,3]thiazolo[4,5-b]pyridin-2-ylcarbamoyl)-3,4-dihydroisoquinolin-2(1H)-yl]pyridin-3-yl}-5-methyl-1H-pyrazol-1-yl)methyl]-5,7-dimethyltricyclo[3.3.1.1^{3,7}]dec-1-yl}oxy)ethyl](3-phosphonopropyl)carbamoyl}oxy)prop-1-en-1-yl]-2-({N-[6-(2,5-dioxo-2,5-dihydro-1H-pyrrol-1-yl)hexanoyl]-beta-alanyl}amino)phenyl beta-D-glucopyranosiduronic acid (Synthon IB)

### 2.63.1 3-(1-((3-(2-(((((E)-3-(3-(3-aminopropanamido)-4-(((2S,3R,4S,5S,6S)-6-carboxy-3,4,5-trihydroxytetrahydro-2H-pyran-2-yl)oxy)phenyl)allyl)oxy)carbonyl)(3-phosphonopropyl)amino)ethoxy)-5,7-dimethyladamantan-1-yl)methyl)-5-methyl-1H-pyrazol-4-yl)-6-(8-(thiazolo[4,5-b]pyridin-2-ylcarbamoyl)-3,4-dihydroisoquinolin-2(1H)-yl)picolinic acid

The title compound was prepared by substituting Example 1.39.2 for Example 1.22.5 in Example 2.56.1.

### 2.63.2 4-[(1E)-3-({[2-({3-[(4-{2-carboxy-6-[8-([1,3]thiazolo[4,5-b]pyridin-2-ylcarbamoyl)-3,4-dihydroisoquinolin-2(1H)-yl]pyridin-3-yl}-5-methyl-1H-pyrazol-1-yl)methyl]-5,7-dimethyltricyclo[3.3.1.1^{3,7}] dec-1-yl}oxy)ethyl](3-phosphonopropyl)carbamoyl}oxy)prop-1-en-1-yl]-2-({N-[6-(2,5-dioxo-2,5-dihydro-1H-pyrrol-1-yl)hexanoyl]-beta-alanyl}amino)phenyl beta-D-glucopyranosiduronic acid

The title compound was prepared by substituting Example 2.63.1 for Example 1.56.1 in Example 2.56.2. ¹H NMR (400 MHz, dimethyl sulfoxide-*d*₆) δ ppm 9.03 (s, 1H), 8.61 (d, 1H), 8.55 (d, 1H), 8.25 (br s, 1H), 7.89 (br m, 1H), 7.65 (d, 1H), 7.50 (br d, 1H), 7.46 (d, 1H), 7.39 (m, 2H), 7.28 (s, 1H), 7.11 (br d, 1H), 7.03 (d, 1H), 6.98 (s, 2H), 6.97 (d, 1H), 6.56 (m, 1H), 6.17 (m,1H), 4.97 (s, 2H), 4.86 (br m, 1 H), 4.64 (br d, 2H), 3.88 (m, 3H), 3.79 (br m, 2H), 3.27-3.44 (m, 14H), 3.01 (m, 2H), 2.54 (m, 2H), 2.08 (s, 3H), 2.03 (t, 2H), 1.46 (m, 6H), 1.37 (br m, 2H), 1.28-0.90 (m, 10H), 0.77-0.82 (m, 6H). MS (ESI) m/e 1498.3 (M-H)⁻.

### 2.64 Synthesis of 6-[8-(1,3-benzothiazol-2-ylcarbamoyl)-3,4-dihydroisoquinolin-2(1H)-yl]-3-{1-[(3-{2-[(2-carboxyethyl)({[(2E)-3-(4-{[(2S,3R,4S,5S,6S)-6-carboxy-3,4,5-trihydroxytetrahydro-2H-pyran-2-yl]oxy}-3-[(3-{[6-(2,5-dioxo-2,5-dihydro-1H-pyrrol-1-yl)hexanoyl]amino}propanoyl)amino]phenyl)prop-2-en-1-yl]oxy}carbonyl)amino]ethoxy}-5,7-dimethyltricyclo[3.3.1.1^{3,7}]dec-1-yl)methyl]-5-methyl-1H-pyrazol-4-yl}pyridine-2-carboxylic acid (Synthon IE)

### 2.64.1 3-(1-((3-(2-(((((E)-3-(3-(3-aminopropanamido)-4-(((2S,3R,4S,5S,6S)-6-carboxy-3,4,5-trihydroxytetrahydro-2H-pyran-2-yl)oxy)phenyl)allyl)oxy)carbonyl)(2-carboxyethyl)amino)ethoxy)-5,7-dimethyladamantan-1-yl)methyl)-5-methyl-1H-pyrazol-4-yl)-6-(8-(benzo[d]thiazol-2-ylcarbarmoyl)-3,4-dihydroisoquinolin-2(1H)-yl)picolinic acid, trifluoroacetic acid salt

To a solution of Example 1.25.2 (0.050 g) and Example 2.44.7 (0.061 g) in N,N-dimethylformamide (1 mL) was added N,N-diisopropylethylamine (0.047 mL), and the reaction was stirred at room temperature overnight. The reaction was concentrated, and the residue was dissolved in methanol (0.5 mL) and tetrahydrofuran (0.5 mL) and treated with a solution of lithium hydroxide hydrate (0.034 g) in water (0.5 mL). The reaction was stirred at room temperature for 1 hour. The reaction was quenched with trifluoroacetic acid (0.083 mL) and diluted with N,N-dimethylformamide (1 mL). The mixture was purified by reverse phase HPLC using a Gilson system, eluting with 10-75% acetonitrile in water containing 0.1% v/v trifluoroacetic acid. The desired fractions were combined and freeze-dried to provide the title compound

### 2.64.2 6-[8-(1,3-benzothiazol-2-ylcarbamoyl)-3,4-dihydroisoquinolin-2(1H)-yl]-3-{1-[(3-{2-[(2-carboxyethyl)({[(2E)-3-(4-{[(2S,3R,4S,5S,6S)-6-carboxy-3,4,5-trihydroxytetrahydro-2H-pyran-2-yl]oxy}-3-[(3-{[6-(2,5-dioxo-2,5-dihydro-1H-pyrrol-1-yl)hexanoyl]amino}propanoyl)amino]phenyl)prop-2-en-1-yl] oxy}carbonyl)amino]ethoxy}-5,7-dimethyltricyclo[3.3.1.1^{3,7}] dec-1-yl)methyl]-5-methyl-1H-pyrazol-4-yl}pyridine-2-carboxylic acid

To a solution of Example 2.64.1 (0.042 g) and 2,5-dioxopyrrolidin-1-yl 6-(2,5-dioxo-2,5-dihydro-1H-pyrrol-1-yl)hexanoate (10 mg) in N,N-dimethylformamide (0.5 mL) was added N,N-diisopropylethylamine (0.027 mL), and the reaction was stirred at room temperature for 2 hours. The reaction was diluted with N,N-dimethylformamide (1 mL) and water (0.5 mL). The mixture was purified by reverse phase HPLC using a Gilson system, eluting with 10-75% acetonitrile in water containing 0.1% v/v trifluoroacetic acid. The desired fractions were combined and freeze-dried to provide the title compound. ¹H NMR (400 MHz, dimethyl sulfoxide-*d*₆) δ ppm 12.85 (s, 1H), 9.04 (s, 1H), 8.25 (s, 1H), 8.03 (d, 1H), 7.87 (t, 1H), 7.79 (d, 1H), 7.61 (d, 1H), 7.54-7.40 (m, 3H), 7.40-7.31 (m, 2H), 7.28 (s, 1H), 7.10 (d, 1H), 7.04 (d, 1H), 6.98 (s, 2H), 6.95 (d, 1H), 6.57 (d, 1H), 6.24-6.11 (m, 1H), 4.96 (s, 2H), 4.86 (t, 1H), 4.65 (d, 2H), 3.95-3.84 (m, 2H), 3.84-3.75 (m, 4H), 3.44-3.24 (m, 10H), 3.01 (t, 2H), 2.62-2.52 (m, 4H), 2.09 (s, 3H), 2.03 (t, 2H), 1.46 (h, 4H), 1.40-1.31 (m, 2H), 1.30-0.88 (m, 14H), 0.87-0.75 (m, 6H). MS (ESI) m/e 1447.5 (M-H)⁻.

### 2.65 Synthesis of 6-[8-(1,3-benzothiazol-2-ylcarbamoyl)-3,4-dihydroisoquinolin-2(1H)-yl]-3-{1-[(3-{2-[(2-carboxyethyl){[(4-{[(2S,3R,4S,5S,6S)-6-carboxy-3,4,5-trihydroxytetrahydro-2H-pyran-2-yl] oxy}-2-[2-(2-{[3-(2,5-dioxo-2,5-dihydro-1H-pyrrol-1-yl)propanoyl]amino}ethoxy)ethoxy]benzyl)oxy]carbonyl}amino]ethoxy}-5,7-dimethyltricyclo[3.3.1.1^{3,7}]dec-1-yl)methyl]-5-methyl-1H-pyrazol-4-yl}pyridine-2-carboxylic acid (Synthon II)

### 2.65.1 3-(1-((3-(2-((((2-(2-(2-aminoethoxy)ethoxy)-4-(((2S,3R,4S,5S,6S)-6-carboxy-3,4,5-trihydroxytetrahydro-2H-pyran-2-yl)oxy)benzyl)oxy)carbonyl)(2-carboxyethyl)amino)ethoxy)-5,7-dimethyladamantan-1-yl)methyl)-5-methyl-1H-pyrazol-4-yl)-6-(8-(benzo[d]thiazol-2-ylcarbarnoyl)-3,4-dihydroisoquinolin-2(1H)-yl)picolinic acid

A solution of Example 1.25.2 (0.055 g,), Example 2.62.6 (0.060 g) and N,N-diisopropylethylamine (0.052 mL) in N,N-dimethylformamide (0.4 mL) as stirred overnight. The reaction was concentrated, and the residue was dissolved in tetrahydrofuran (0.5 mL), methanol (0.5 mL) then treated with lithium hydroxide hydrate (0.037 g) as a solution in water (0.5 mL). After stirring for 1 hour, the reaction was quenched with trifluoroacetic acid (0.091 mL) and diluted with N,N-dimethylformamide (1 mL). The mixture was purified by reverse phase HPLC using a Gilson system, eluting with 10-75% acetonitrile in water containing 0.1% v/v trifluoroacetic acid. The desired fractions were combined and freeze-dried to provide the title compound as the trifluoroacetic acid salt.

### 2.65.2 6-[8-(1,3-benzothiazol-2-ylcarbamoyl)-3,4-dihydroisoquinolin-2(1H)-yl]-3-{1-[(3-{2-[(2-carboxyethyl){[(4-{[(2S,3R,4S,5S,6S)-6-carboxy-3,4,5-trihydroxytetrahydro-2H-pyran-2-yl] oxy}-2-[2-(2-{[3-(2,5-dioxo-2,5-dihydro-1H-pyrrol-1-yl)propanoyl]amino}ethoxy)ethoxy]benzyl)oxy]carbonyl}amino ]ethoxy}-5,7-dimethyltricyclo[3.3.1.1^{3,7}]dec-1-yl)methyl]-5-methyl-1H-pyrazol-4-yl}pyridine-2-carboxylic acid

A solution of the trifluoroacetic acid salt of Example 2.65.1 (0.043), 2,5-dioxopyrrolidin-1-yl 3-(2,5-dioxo-2,5-dihydro-1H-pyrrol-1-yl)propanoate (10 mg) and N,N-diisopropylethylamine (0.028 mL) were stirred together in N,N-dimethylformamide (1 mL) at room temperature. After stirring for 1 hour, the reaction was diluted with N,N-dimethylformamide (0.5 mL) and water (0.5 mL). The mixture was purified by reverse phase HPLC using a Gilson system, eluting with 5-75% acetonitrile in water containing 0.1% v/v trifluoroacetic acid. The desired fractions were combined and freeze-dried to provide the title compound. ¹H NMR (400 MHz, dimethyl sulfoxide-*d*₆) δ ppm 12.84 (s, 1H), 8.03 (d, 1H), 8.00 (t, 1H), 7.79 (d, 1H), 7.62 (d, 1H), 7.54-7.41 (m, 3H), 7.36 (td, 2H), 7.29 (s, 1H), 7.19 (d, 1H), 6.97 (s, 2H), 6.95 (d, 1H), 6.67 (d, 1H), 6.60 (dd, 1H), 5.14-5.03 (m, 1H), 4.96 (d, 4H), 4.08 (tt, 4H), 3.89 (q, 4H), 3.84-3.77 (m, 2H), 3.71 (t, 2H), 3.59 (t, 2H), 3.52-3.35 (m, 6H), 3.28 (dq, 4H), 3.17 (q, 2H), 3.01 (t, 2H), 2.46 (d, 1H), 2.33 (t, 2H), 2.09 (s, 3H), 1.45-0.90 (m, 12H), 0.82 (d, 6H). MS (ESI) m/e 1396.4 (M-H)⁻.

### 2.66 Synthesis of N-[6-(ethenylsulfonyl)hexanoyl]-L-valyl-N-{4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}] dec-1-yl}oxy)ethyl](2-sulfoethyl)carbamoyl}oxy)methyl]phenyl}-N⁵⁻carbamoyl-L-ornithinamide (Synthon KY)

### 2.66.1 3-(1-((3-(2-((((4-((S)-2-((S)-2-amino-3-methylbutanamido)-5-ureidopentanamido)benzyl)oxy)carbonyl)(2-sulfoethyl)amino)ethoxy)-5,7-dimethyladamantan-1-yl)methyl)-5-methyl-1H-pyrazol-4-yl)-6-(8-(benzo[d]thiazol-2-ylcarbarnoyl)-3,4-dihydroisoquinolin-2(1H)-yl)picolinic acid

To a mixture of Example 1.2.9 (57 mg) and (9H-fluoren-9-yl)methyl ((S)-3-methyl-1-(((S)-1-((4-((((4-nitrophenoxy)carbonyl)oxy)methyl)phenyl)amino)-1-oxo-5-ureidopentan-2-yl)amino)-1-oxobutan-2-yl)carbamate (54 mg) in N,N-dimethylformamide (2 mL) was added N,N-diisopropylethylamine (103 µL). The mixture was stirred overnight, and diethylamine (61.5 µL) was added. The resulting mixture was stirred for 4 hours and purified by reverse phase HPLC using a Gilson system and C18 column, eluting with 10-70% acetonitrile in water containing 0.1% v/v trifluoroacetic acid, to provide the title compound. MS (ESI) m/e 1257.4 (M-H).

### 2.66.2 N-[6-(ethenylsulfonyl)hexanoyl]-L-valyl-N-{4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)methyl]phenyl}-N⁵⁻carbamoyl-L-ornithinamide

The title compound was prepared using the procedure in Example 2.83, replacing Example 1.2.9 and 2,5-dioxopyrrolidin-1-yl 6-(2-chloroacetamido)hexanoate with Example 2.66.1 and Example 2.82.5, respectively. ¹H NMR (400 MHz, dimethyl sulfoxide-*d*₆) δ ppm 12.88 (s, 0H), 9.99 (s, 1H), 8.05 (t, 2H), 7.80 (t, 2H), 7.60 (q, 3H), 7.36 (td, 2H), 7.28 (d, 3H), 7.01-6.89 (m, 2H), 6.29-6.15 (m, 2H), 6.02 (s, 1H), 4.97 (d, 4H), 4.40 (td, 1H), 4.20 (t, 1H), 4.00-3.77 (m, 4H), 3.55-3.33 (m, 4H), 3.25 (d, 2H), 3.14-2.88 (m, 6H), 2.62 (t, 2H), 2.09 (s, 4H), 1.82-0.90 (m, 10H), 0.84 (dd, 13H). MS (ESI) m/e 1447.2 (M+H).

### 2.67 Synthesis of 4-[(1E)-3-{[(4-{[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](3-phosphonopropyl)amino}piperidin-1-yl)carbonyl]oxy}prop-1-en-1-yl]-2-({N-[6-(2,5-dioxo-2,5-dihydro-1H-pyrrol-1-yl)hexanoyl]-beta-alanyl}amino)phenyl beta-D-glucopyranosiduronic acid (Synthon IW)

### 2.67.1 3-(1-((3-(2-((1-((((E)-3-(3-(3-aminopropanamido)-4-(((2S,3R,4S,5S,6S)-6-carboxy-3,4,5-trihydroxytetrahydro-2H-pyran-2-yl)oxy)phenyl)allyl)oxy)carbonyl)piperidin-4-yl)(3-phosphonopropyl)amino)ethoxy)-5,7-dimethyladamantan-1-yl)methyl)-5-methyl-1H-pyrazol-4-yl)-6-(8-(benzo[d]thiazol-2-ylcarbamoyl)-3,4-dihydroisoquinolin-2(1H)-yl)picolinic acid

To a solution of Example 1.26.2 (0.045 g) and Example 2.44.7 (0.053 g) in N,N-dimethylformamide (1 mL) was added N,N-diisopropylethylamine(0.041 mL), and the reaction was stirred at room temperature overnight. The reaction was concentrated, and the residue was dissolved in methanol (0.5 mL) and tetrahydrofuran (0.5 mL) and treated with a solution of lithium hydroxide monohydrate (0.030 g) in water (0.5 mL) at room temperature. After stirring for 1 hour, the reaction was quenched with trifluoroacetic acid (0.073 mL) and diluted with N,N-dimethylformamide (1 mL). The mixture was purified by reverse phase HPLC using a Gilson system, eluting with 10-60% acetonitrile in water containing 0.1% v/v trifluoroacetic acid. The desired fractions were combined and freeze-dried to provide the title compound.

### 2.67.2 4-[(1E)-3-{[(4-{[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](3-phosphonopropyl)amino}piperidin-1-yl)carbonyl]oxy}prop-1-en-1-yl]-2-({N-[6-(2,5-dioxo-2,5-dihydro-1H-pyrrol-1-yl)hexanoyl]-beta-alanyl}amino)phenyl beta-D-glucopyranosiduronic acid

To a solution of Example 2.67.1 (0.040 g) and 2,5-dioxopyrrolidin-1-yl 6-(2,5-dioxo-2,5-dihydro-1H-pyrrol-1-yl)hexanoate (9.84 mg) in N,N-dimethylformamide (1 mL) was added N,N-diisopropylethylamine (0.023 mL), and the reaction was stirred at room temperature for 2 hours. The reaction was diluted with N,N-dimethylformamide (1 mL) and water (1 mL). The mixture was purified by reverse phase HPLC using a Gilson system, eluting with 10-60% acetonitrile in water containing 0.1% v/v trifluoroacetic acid. The desired fractions were combined and freeze-dried to provide the title compound. ¹H NMR (400 MHz, dimethyl sulfoxide-*d*₆) δ ppm 9.28 (s, 1H), 9.04 (s, 1H), 8.25 (s, 1H), 8.03 (d, 1H), 7.87 (t, 1H), 7.79 (d, 1H), 7.62 (dd, 1H), 7.55-7.40 (m, 3H), 7.36 (td, 2H), 7.29 (s, 1H), 7.11 (dd, 1H), 7.05 (d, 1H), 6.98 (s, 2H), 6.95 (d, 1H), 6.59 (d, 1H), 6.20 (t, 1H), 6.16 (t, 0H), 4.96 (s, 2H), 4.88 (d, 1H), 4.66 (d, 2H), 4.14 (d, 2H), 3.96-3.86 (m, 2H), 3.83 (s, 2H), 3.54 (t, 7H), 3.48-3.28 (m, 12H), 3.01 (t, 2H), 2.84 (s, 2H), 2.55 (t, 2H), 2.10 (s, 3H), 2.07-1.95 (m, 4H), 1.88 (s, 2H), 1.73-1.54 (m, 4H), 1.54-1.38 (m, 6H), 1.39-1.26 (m, 4H), 1.26-0.93 (m, 8H), 0.86 (s, 6H). MS (ESI) m/e 1582.4 (M+H)⁺.

### 2.68 Synthesis of 4-[(1E)-3-{[(4-{[2-({3-[(4-{2-carboxy-6-[8-([1,3]thiazolo[4,5-b]pyridin-2-ylcarbamoyl)-3,4-dihydroisoquinolin-2(1H)-yl]pyridin-3-yl}-5-methyl-1H-pyrazol-1-yl)methyl]-5,7-dimethyltricyclo[3.3.1.1^{3,7}]dec-1-yl}oxy)ethyl](3-phosphonopropyl)amino}piperidin-1-yl)carbonyl]oxy}prop-1-en-1-yl]-2-({N-[6-(2,5-dioxo-2,5-dihydro-1H-pyrrol-1-yl)hexanoyl]-beta-alanyl}amino)phenyl beta-D-glucopyranosiduronic acid (Synthon IY)

### 2.68.1 3-(1-((3-(2-((1-((((E)-3-(3-(3-aminopropanamido)-4-(((2S,3R,4S,5S,6S)-6-carboxy-3,4,5-trihydroxytetrahydro-2H-pyran-2-yl)oxy)phenyl)allyl)oxy)carbonyl)piperidin-4-yl)(3-phosphonopropyl)amino)ethoxy)-5,7-dimethyladamantan-1-yl)methyl)-5-methyl-1H-pyrazol-4-yl)-6-(8-(thiazolo[4,5-b]pyridin-2-ylcarbamoyl)-3,4-dihydroisoquinolin-2(1H)-yl)picolinic acid

The title compound was prepared by substituting Example 1.50.2 for Example 1.44.7 in Example 2.56.1. MS (ESI) m/e 1388.5 (M-H)⁻.

### 2.68.2 4-[(1E)-3-{[(4-{[2-({3-[(4-{2-carboxy-6-[8-([1,3]thiazolo[4,5-b]pyridin-2-ylcarbamoyl)-3,4-dihydroisoquinolin-2(1H)-yl]pyridin-3-yl}-5-methyl-1H-pyrazol-1-yl)methyl]-5,7-dimethyltricyclo[3.3.1.1^{3,7}]dec-1-yl}oxy)ethyl](3-phosphonopropyl)amino}piperidin-1-yl)carbonyl]oxy}prop-1-en-1-yl]-2-({N-[6-(2,5-dioxo-2,5-dihydro-1H-pyrrol-1-yl)hexanoyl]-beta-alanyl}amino)phenyl beta-D-glucopyranosiduronic acid

The title compound was prepared by substituting Example 1.68.1 for Example 1.56.1 in Example 2.56.2. ¹H NMR (400 MHz, dimethyl sulfoxide-*d*₆) δ ppm 9.03 (s, 1H), 8.61 (d, 1H), 8.50 (d, 1H), 8.25 (br s, 1H), 7.89 (t, 1H), 7.65 (d, 1H), 7.49 (d, 1H), 7.46 (d, 1H), 7.36 (m, 2H), 7.29 (s, 1H), 7.11 (br d, 1H), 7.03 (d, 1H), 6.98 (s, 2H), 6.97 (d, 1H), 6.58 (m, 1H), 6.17 (m,1H), 4.97 (s, 2H), 4.88 (d, 1 H), 4.65 (br d, 2H), 3.88 (m, 3H), 3.79 (br m, 2H), 3.66 (br m, 2H), 3.27-3.44, (m, 14H), 3.01 (m, 2H), 2.85 (br m, 2H), 2.54 (m, 2H), 2.10 (s, 3H), 2.03 (t, 2H), 1.98 (br m, 2H), 1.89 (m, 1H), 1.62 (m, 4H), 1.46 (m, 6H), 1.31 (m, 4H), 1.15 (m, 6H), 1.04 (m, 2H), 0.86 (s, 6H). MS (ESI) m/e 1581.4 (M-H)⁻.

### 2.69 Synthesis of 4-[(1E)-3-({[2-({3-[(4-{6-[8-(1,3-benzothiazol-2-ylcarbamoyl)naphthalen-2-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)prop-1-en-1-yl]-2-({N-[6-(2,5-dioxo-2,5-dihydro-1H-pyrrol-1-yl)hexanoyl]-beta-alanyl}amino)phenyl beta-D-glucopyranosiduronic acid (Synthon JA) 2.69.13-(1-((3-(2-(((((E)-3-(3-(3-aminopropanamido)-4-(((2S,3R,4S,5S,6S)-6-carboxy-3,4,5-trihydroxytetrahydro-2H-pyran-2-yl)oxy)phenyl)allyl)oxy)carbonyl)(2-sulfoethyl)amino)ethoxy)-5,7-dimethyladamantan-1-yl)methyl)-5-methyl-1H-pyrazol-4-yl)-6-(8-(benzo[d]thiazol-2-ylcarbamoyl)naphthalen-2-yl)picolinic acid

The title compound was prepared by substituting Example 1.43.7 for Example 2.44.7 in Example 2.56.1. MS (ESI) m/e 1309.1 (M+Na)⁺.

### 2.69.2 4-[(1E)-3-({[2-({3-[(4-{6-[8-(1,3-benzothiazol-2-ylcarbamoyl)naphthalen-2-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)prop-1-en-1-yl]-2-({N-[6-(2,5-dioxo-2,5-dihydro-1H-pyrrol-1-yl)hexanoyl]-beta-alanyl}amino)phenyl beta-D-glucopyranosiduronic acid

The title compound was prepared by substituting Example 2.69.1 for Example 2.56.1 in Example 2.56.2. ¹H NMR (400 MHz, dimethyl sulfoxide-*d*₆) δ ppm 13.09 (s, 1H), 9.02 (s, 2H), 8.35 (d, 1H), 8.13-8.29 (m, 4H), 7.86-8.09 (m, 5H), 7.81 (d, 1H), 7.66-7.75 (m, 1H), 7.44-7.55 (m, 1H), 7.37 (t, 1H), 7.09-7.18 (m, 1H), 7.03 (d, 1H), 6.98 (s, 1H), 6.48-6.62 (m, 1H), 6.07-6.22 (m, 1H), 4.81-4.92 (m, 1H), 4.58-4.74 (m, 2H), 3.80-3.93 (m, 3H), 3.27-3.37 (m, 5H), 2.53-2.68 (m, 4H), 2.15-2.23 (m, 3H), 2.03 (t, 2H), 1.36-1.53 (m, 6H) , 0.97-1.33 (m, 24H), 0.81 (d, 6H). MS (ESI) m/e 1478.3(M-H)⁻.

### 2.70 This paragraph was intentionally left blank.

### 2.71 This paragraph was intentionally left blank.

### 2.72 This paragraph was intentionally left blank.

### 2.73 This paragraph was intentionally left blank.

### 2.74 This paragraph was intentionally left blank.

### 2.75 This paragraph was intentionally left blank.

### 2.76 This paragraph was intentionally left blank.

### 2.77 Synthesis of 6-[8-(1,3-benzothiazol-2-ylcarbamoyl)-3,4-dihydroisoquinolin-2(1H)-yl]-3-[1-({3-[2-({N-[6-(2,5-dioxo-2,5-dihydro-1H-pyrrol-1-yl)hexanoyl]-3-sulfo-L-alanyl}amino)ethoxy]-5,7-dimethyltricyclo[3.3.1.1^{3,7}]dec-1-yl}methyl)-5-methyl-1H-pyrazol-4-yl]pyridine-2-carboxylic acid (Synthon FA)

To a solution of Example 1.15 (0.023 g) and 2,5-dioxopyrrolidin-1-yl 6-(2,5-dioxo-2,5-dihydro-1H-pyrrol-1-yl)hexanoate (9.12 mg) in N,N-dimethylformamide (0.5 mL) was added N,N-diisopropylethylamine (0.012 mL), and the reaction was stirred overnight. The reaction was diluted with N,N-dimethylformamide (1 mL) and water (0.5 mL). The mixture was purified by reverse phase HPLC using a Gilson system, eluting with 10-85% acetonitrile in water containing 0.1% v/v trifluoroacetic acid. The desired fractions were combined and freeze-dried to provide the title compound. ¹H NMR (400 MHz, dimethyl sulfoxide-*d*₆) δ ppm 12.84 (s, 1H), 8.04 (d, 1H), 7.90 (d, 1H), 7.79 (d, 1H), 7.65-7.57 (m, 2H), 7.54 (d, 1H), 7.51-7.41 (m, 2H), 7.40-7.31 (m, 3H), 7.01-6.96 (m, 3H), 4.96 (s, 2H), 4.34-4.28 (m, 3H), 3.89 (t, 2H), 3.83 (s, 2H), 3.37 (t, 2H), 3.29 (t, 2H), 3.16-2.95 (m, 4H), 2.80 (dd, 1H), 2.70 (dd, 1H), 2.11 (s, 3H), 2.06 (t, 2H), 1.47 (tt, 4H), 1.40-0.92 (m, 12H), 0.84 (s, 6H). MS (ESI) m/e 1090.3 (M+H)⁺.

### 2.78 Synthesis of 6-[8-(1,3-benzothiazol-2-ylcarbamoyl)-3,4-dihydroisoquinolin-2(1H)-yl]-3-[1-({3-[2-(2-{[6-(2,5-dioxo-2,5-dihydro-1H-pyrrol-1-yl)hexanoyl](2-sulfoethyl)amino}ethoxy)ethoxy]-5,7-dimethyltricyclo[3.3.1.1^{3,7}]dec-1-yl}methyl)-5-methyl-1H-pyrazol-4-yl]pyridine-2-carboxylic acid (Synthon FJ)

The title compound was prepared as described in Example 2.1, replacing Example 1.2.9 and 4-((S)-2-((S)-2-(6-(2,5-dioxo-2,5-dihydro-1H-pyrrol-1-yl)hexanamido)-3-methylbutanamido)-5-ureidopentanamido)benzyl (4-nitrophenyl) carbonate with Example 1.11.4 and perfluorophenyl 6-(2,5-dioxo-2,5-dihydro-1H-pyrrol-1-yl)hexanoate, respectively. ¹H NMR (400 MHz, dimethyl sulfoxide-*d*₆) δ ppm 12.84 (s, 1H), 8.04 (d, 1H), 7.79 (d, 1H), 7.61 (d, 1H), 7.52 (dd, 1H), 7.42-7.49 (m, 2H), 7.33-7.39 (m, 2H), 7.30 (s, 1H), 6.98 (s, 2H), 6.96 (d, 1H), 4.95 (s, 2H), 3.89 (t, 2H), 3.82 (s, 2H), 3.46-3.56 (m, 4H), 3.31-3.46 (m, 10H), 3.01 (t, 2H), 2.61-2.68 (m, 1H), 2.55-2.60 (m, 1H), 2.21-2.32 (m, 2H), 2.10 (s, 3H), 1.40-1.51 (m, 4H), 1.37 (d, 2H), 0.91-1.30 (m, 12H), 0.83 (s, 6H). MS (ESI) m/e 1091.2 (M+H)⁺.

### 2.79 Synthesis of 6-[8-(1,3-benzothiazol-2-ylcarbamoyl)-3,4-dihydroisoquinolin-2(1H)-yl]-3-(1-{[3-(2-{[6-(2,5-dioxo-2,5-dihydro-1H-pyrrol-1-yl)hexanoyl](2-sulfoethyl)amino}ethoxy)-5,7-dimethyltricyclo[3.3.1.1^{3,7}]dec-1-yl]methyl}-5-methyl-1H-pyrazol-4-yl)pyridine-2-carboxylic acid (Synthon FK)

The title compound was prepared as described in Example 2.1, replacing 4-((S)-2-((S)-2-(6-(2,5-dioxo-2,5-dihydro-1H-pyrrol-1-yl)hexanamido)-3-methylbutanamido)-5-ureidopentanamido)benzyl (4-nitrophenyl) carbonate with perfluorophenyl 6-(2,5-dioxo-2,5-dihydro-1H-pyrrol-1-yl)hexanoate. 1H NMR (400 MHz, dimethyl sulfoxide-*d*₆) δ ppm 12.85 (s, 1H), 8.04 (d, 1H), 7.79 (d, 1H), 7.61 (d, 1H), 7.52 (dd, 1H), 7.41-7.49 (m, 2H), 7.32-7.39 (m, 2H), 7.28 (s, 1H), 6.93-6.98 (m, 3H), 4.95 (s, 2H), 3.89 (t, 2H), 3.81 (s, 2H), 3.32-3.38 (m, 2H), 3.21-3.27 (m, 2H), 3.01 (t, 2H), 2.61-2.67 (m, 2H), 2.53-2.58 (m, 2H), 2.33-2.39 (m, 1H), 2.20-2.29 (m, 2H), 2.09 (s, 3H), 1.40-1.51 (m, 4H), 1.34 (s, 2H), 0.93-1.27 (m, 13H), 0.83 (s, 6H). MS (ESI) m/e 1047.2 (M+H)⁺.

### 2.80 Synthesis of 6-[8-(1,3-benzothiazol-2-ylcarbamoyl)-3,4-dihydroisoquinolin-2(1H)-yl]-3-{1-[(3-{[1-(2,5-dioxo-2,5-dihydro-1H-pyrrol-1-yl)-21-oxo-22-(2-sulfoethyl)-3,6,9,12,15,18-hexaoxa-22-azatetracosan-24-yl] oxy}-5,7-dimethyltricyclo [3.3.1.1^{3,7}]dec-1-yl)methyl]-5-methyl-1H-pyrazol-4-yl}pyridine-2-carboxylic acid (Synthon FQ)

The title compound was prepared as described in Example 2.1, replacing 4-((S)-2-((S)-2-(6-(2,5-dioxo-2,5-dihydro-1H-pyrrol-1-yl)hexanamido)-3-methylbutanamido)-5-ureidopentanamido)benzyl (4-nitrophenyl) carbonate with perfluorophenyl 1-(2,5-dioxo-2,5-dihydro-1H-pyrrol-1-yl)-3,9,12,15,18-pentaoxahenicosan-21-oate. ¹H NMR (400 MHz, dimethyl sulfoxide-*d*₆) δ ppm 12.84 (s, 1H), 8.04 (d, 1H), 7.79 (d, 1H), 7.61 (d, 1H), 7.42-7.54 (m, 3H), 7.33-7.38 (m, 2H), 7.28 (s, 1H), 6.95 (dd, 1H), 4.95 (s, 2H), 3.89 (t, 2H), 3.81 (s, 2H), 3.07-3.53 (m, 24H), 3.01 (t, 2H), 2.61-2.69 (m, 1H), 2.54-2.60 (m, 1H), 2.09 (s, 3H), 1.96 (d, 2H), 0.92-1.39 (m, 13H), 0.84 (s, 6H). MS (ESI) m/e 1269.4 (M+H)⁺.

### 2.81 Synthesis of 6-[8-(1,3-benzothiazol-2-ylcarbamoyl)-3,4-dihydroisoquinolin-2(1H)-yl]-3-1-[(3-{[1-(2,5-dioxo-2,5-dihydro-1H-pyrrol-1-yl)-21-oxo-22-(2-sulfoethyl)-3,6,9,12,15,18,25-heptaoxa-22-azaheptacosan-27-yl]oxy}-5,7-dimethyltricyclo[3.3.1.1^{3,7}]dec-1-yl)methyl]-5-methyl-1H-pyrazol-4-yl}pyridine-2-carboxylic acid (Synthon FR)

The title compound was prepared as described in Example 2.1, replacing Example 1.2.9 and 4-((S)-2-((S)-2-(6-(2,5-dioxo-2,5-dihydro-1H-pyrrol-1-yl)hexanamido)-3-methylbutanamido)-5-ureidopentanamido)benzyl (4-nitrophenyl) carbonate with Example 1.11.4 and perfluorophenyl 1-(2,5-dioxo-2,5-dihydro-1H-pyrrol-1-yl)-3,6,9,12,15,18-hexaoxahenicosan-21-oate, respectively. ¹H NMR (500 MHz, dimethyl sulfoxide-*d*₆) δ ppm 12.84 (s, 1H), 8.04 (d, 1H), 7.79 (d, 1H), 7.61 (d, 1H), 7.52 (d, 1H), 7.41-7.50 (m, 2H), 7.33-7.39 (m, 2H), 7.31 (s, 1H), 7.01 (d, 2H), 6.97 (d, 1H), 4.96 (s, 2H), 3.89 (t, 2H), 3.83 (s, 2H), 3.31-3.60 (m, 30H), 3.01 (t, 2H), 2.64-2.71 (m, 1H), 2.53-2.61 (m, 3H), 2.10 (s, 3H), 1.38 (s, 2H), 1.20-1.31 (m, 4H), 1.12-1.18 (m, 2H), 0.91-1.12 (m, 4H), 0.84 (s, 6H).

### 2.82 Synthesis of 6-[8-(1,3-benzothiazol-2-ylcarbamoyl)-3,4-dihydroisoquinolin-2(1H)-yl]-3-(1-{[3-(2-{[6-(ethenylsulfonyl)hexanoyl](2-sulfoethyl)amino}ethoxy)-5,7-dimethyltricyclo[3.3.1.1^{3,7}]dec-1-yl]methyl}-5-methyl-1H-pyrazol-4-yl)pyridine-2-carboxylic acid (Synthon JE)

### 2.82.1 ethyl 6-((2-hydroxyethyl)thio)hexanoate

A mixture of ethyl 6-bromohexanoate (3 g), 2-mercaptoethanol (0.947 mL) and K₂CO₃ (12 g) in ethanol (100 mL) was stirred overnight and filtered. The filtrate was concentrated. The residue was dissolved in dichloromethane (100 mL) and washed with water and brine. The organic layer was dried over sodium sulfate, filtered, and concentrated to provide the title compound.

### 2.82.2 6-((2-hydroxyethyl)thio)hexanoic acid

A mixture of Example 2.82.1 (12 g) and 3 M aqueous NaOH solution (30 mL) in ethanol (30 mL) was stirred overnight. The organics were removed under reduced pressure. The residual aqueous phase was washed with ethyl acetate, acidified with HCl to pH 5 and extracted with dichloromethane. The extracts were combined, dried over sodium sulfate, filtered and concentrated to provide the title compound.

### 2.82.3 6-((2-hydroxyethyl)sulfonyl)hexanoic acid

To a stirred solution of Example 2.82.2 (4 g) in a mixture of water (40 mL) and 1,4-dioxane (160 mL) was added Oxone^{®} (38.4 g), and the mixture was stirred overnight. The mixture was filtered, and the filtrate was concentrated. The residual aqueous layer was extracted with dichloromethane. The extracts were combined and dried over sodium sulfate, filtered, and concentrated to provide the title compound.

### 2.82.4 6-(vinylsulfonyl)hexanoic acid

To a cold (0 °C) solution of Example 2.82.3 (1 g) in dichloromethane (10 mL) was added triethylamine (2.8 mL), followed by the addition of methanesulfonyl chloride (1.1 mL) under argon. The mixture was stirred overnight and washed with water and brine. The organic layer was dried over sodium sulfate, filtered, and concentrated to provide the title compound.

### 2.82.5 2,5-dioxopyrrolidin-1-yl 6-(vinylsulfonyl)hexanoate

To a stirred solution of Example 2.82.4 (0.88 g) in dichloromethane (10 ml) was added 1-hydroxypyrrolidine-2,5-dione (0.54 g) and N,N'-methanediylidenedicyclohexanamine (0.92 g). The mixture was stirred overnight and filtered. The filtrate was concentrated and purified by flash chromatography, eluting with 10-25% ethyl acetate in petroleum, to provide the title compound. MS (ESI) m/e 304.1 (M+1).

### 2.82.6 6-[8-(1,3-benzothiazol-2-ylcarbamoyl)-3,4-dihydroisoquinolin-2(1H)-yl]-3-(1-{[3-(2-{[6-(ethenylsulfonyl)hexanoyl](2-sulfoethyl)amino}ethoxy)-5,7-dimethyltricyclo[3.3.1.1^{3,7}]dec-1-yl]methyl}-5-methyl-1H-pyrazol-4-yl)pyridine-2-carboxylic acid

The title compound was prepared as described in Example 2.83, replacing 2,5-dioxopyrrolidin-1-yl 6-(2-chloroacetamido)hexanoate with Example 2.82.5. ¹H NMR (400 MHz, dimethyl sulfoxide-*d*₆) δ ppm 12.86 (s, 1H), 8.04 (d, 1H), 7.79 (d, 1H), 7.61 (d, 1H), 7.53 (dd, 1H), 7.42-7.49 (m, 2H), 7.33-7.40 (m, 2H), 7.28 (s, 1H), 6.88-7.00 (m, 2H), 6.17-6.25 (m, 2H), 4.95 (s, 2H), 3.89 (t, 2H), 3.81 (s, 2H), 3.38 (dd, 2H), 3.25 (t, 2H), 3.04-3.12 (m, 2H), 3.01 (t, 2H), 2.62-2.69 (m, 1H), 2.56 (dd, 1H), 2.27 (q, 2H), 2.09 (s, 3H), 1.53-1.62 (m, 2H), 1.43-1.51 (m, 2H), 1.28-1.38 (m, 4H), 1.20-1.27 (m, 4H), 0.92-1.19 (m, 6H), 0.84 (s, 6H). MS (ESI) m/e 1042.2 (M+H)⁺.

### 2.83 Synthesis of 6-[8-(1,3-benzothiazol-2-ylcarbamoyl)-3,4-dihydroisoquinolin-2(1H)-yl]-3-1-[(3-{2-[{6-[(chloroacetyl)amino]hexanoyl}(2-sulfoethyl)amino]ethoxy}-5,7-dimethyltricyclo[3.3.1.1^{3,7}]dec-1-yl)methyl]-5-methyl-1H-pyrazol-4-yl}pyridine-2-carboxylic acid (Synthon JM)

To a mixture of Example 1.2.9 (12.5 mg) and 2,5-dioxopyrrolidin-1-yl 6-(2-chloroacetamido)hexanoate (6.7 mg) in N,N-dimethylformamide (1.5 mL) was added N,N-diisopropylethylamine (26 µL). The mixture was stirred for 10 days and purified by reverse phase HPLC using a Gilson system and C18 column, eluting with 20-60% acetonitrile in water containing 0.1% v/v trifluoroacetic acid, to provide the title compound. ¹H NMR (500 MHz, dimethyl sulfoxide-d₆) δ ppm 12.83 (s, 1H), 8.15-8.21 (m, 1H), 8.04 (d, 1H), 7.79 (d, 1H), 7.61 (d, 1H), 7.52 (dd, 1H), 7.41-7.49 (m, 2H), 7.32-7.39 (m, 2H), 7.28 (s, 1H), 6.96 (dd, 1H), 4.95 (s, 2H), 4.01 (d, 2H), 3.89 (t, 2H), 3.81 (s, 2H), 3.39 (d, 2H), 3.25 (t, 2H), 2.98-3.10 (m, 5H), 2.62-2.70 (m, 1H), 2.56-2.61 (m, 1H), 2.23-2.30 (m, 2H), 2.09 (s, 3H), 1.33-1.52 (m, 5H), 1.19-1.30 (m, 6H), 0.91-1.18 (m, 6H), 0.84 (s, 6H). MS (ESI) m/e 1043.2 (M+H)⁺.

### 2.84 Synthesis of N-[6-(2,5-dioxo-2,5-dihydro-1H-pyrrol-1-yl)hexanoyl]-L-valyl-N-{4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](3-carboxypropyl)carbamoyl}oxy)methyl]phenyl}-N⁵-carbamoyl-L-ornithinamide (Synthon LE)

A mixture of Example 1.56 (0.020 g), 4-((S)-2-((S)-2-(6-(2,5-dioxo-2,5-dihydro-1H-pyrrol-1-yl)hexanamido)-3 -methylbutanamido)-5 -ureidopentanamido)benzyl (4-nitrophenyl) carbonate (0.022 g) and N,N-diisopropylethylamine (0.018 mL) were stirred together in N,N-dimethylformamide (0.4 mL) at room temperature. After stirring for 5 hours, the reaction was diluted with a 1:1 mixture of N,N-dimethylformamide and water (2 mL). The mixture was purified by reverse phase HPLC using a Gilson system, eluting with 10-85% acetonitrile in water containing 0.1% v/v trifluoroacetic acid. The desired fractions were combined and freeze-dried to provide the title compound. ¹H NMR (400 MHz, dimethyl sulfoxide-d₆) δ ppm 12.82 (s, 1H), 9.97 (s, 1H), 8.10-7.98 (m, 2H), 7.84-7.72 (m, 2H), 7.67-7.54 (m, 3H), 7.54-7.41 (m, 3H), 7.40-7.32 (m, 2H), 7.30-7.23 (m, 3H), 6.99 (s, 2H), 6.94 (d, 1H), 5.99 (s, 1H), 4.98 (s, 2H), 4.95 (s, 2H), 4.45-4.35 (m, 2H), 4.19 (dd, 2H), 3.88 (t, 2H), 3.82-3.76 (m, 2H), 3.47-3.31 (m, 4H), 3.28-3.19 (m, 4H), 3.07-2.89 (m, 4H), 2.21-2.11 (m, 4H), 2.09 (s, 2H), 2.02-1.89 (m, 1H), 1.77-1.63 (m, 2H), 1.62-1.27 (m, 10H), 1.27-0.90 (m, 13H), 0.88-0.78 (m, 12H); MS (ESI) m/e 1430.3 (M+1)⁺.

### 2.85 Synthesis of N-{6-[(bromoacetyl)amino]hexanoyl}-L-valyl-N-{4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)methyl]phenyl}-N⁵-carbamoyl-L-ornithinamide (Synthon LH)

### 2.85.1 1H-benzo[d][1,2,3]triazol-1-yl 6-(2-bromoacetamido)hexanoate

To a solution of 6-(2-bromoacetamido)hexanoic acid (105 mg) and benzotriazol-1-yloxy)tripyrrolidinophosphonium hexafluorophosphate (PyBOP, 325 mg) in N,N-dimethylformamide (3 mL) was added triethylamine (87 µL). The mixture was stirred for 1 hour and purified by a Gilson HPLC system (C18 column), eluting with 20-60% acetonitrile in 0.1% TFA water to provide the title compound. MS (ESI) m/e 368.7 (M+H).

### 2.85.2 N-{6-[(bromoacetyl)amino]hexanoyl}-L-valyl-N-{4-[({[2-({3-[(4-{6-[8-(1,3-benzothiazol-2-ylcarbarnoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)methyl]phenyl}-N⁵-carbamoyl-L-ornithinamide

To a mixture of Example 2.66.1 (6.6 mg) and Example 2.85.2 (3.6 mg) in N,N-dimethylformamide (0.3 mL) was added N,N-diisopropylethylamine (2.52 µL). The mixture was stirred for 5 minutes, diluted with dimethyl sulfoxide and purified by reverse phase HPLC using a Gilson system and C18 column, eluting with 20-60% acetonitrile in water containing 0.1% v/v trifluoroacetic acid, to provide the title compound. ¹H NMR (500 MHz, dimethyl sulfoxide-*d*₆) δ 9.99 (s, 1H), 8.24 (s, 1H), 8.08 (d, 1H), 8.04 (d, 1H), 7.80 (dd, 2H), 7.60 (q, 3H), 7.56-7.50 (m, 1H), 7.50-7.41 (m, 2H), 7.36 (q, 2H), 7.32-7.25 (m, 3H), 6.96 (d, 1H), 4.98 (d, 4H), 4.39 (q, 1H), 4.20 (dd, 1H), 3.92-3.68 (m, 6H), 3.42 (dd, 1H), 3.25 (t, 2H), 3.09-2.87 (m, 6H), 2.64 (s, 2H), 2.25-1.87 (m, 5H), 1.79-0.89 (m, 17H), 0.88-0.67 (m, 12H). MS (ESI) m/e 1492.5 (M-H).

### 2.86 Synthesis of 4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](3-carboxypropyl)carbamoyl}oxy)methyl]-3-[2-(2-{[3-(2,5-dioxo-2,5-dihydro-1H-pyrrol-1-yl)propanoyl]amino}ethoxy)ethoxy]phenyl beta-D-glucopyranosiduronic acid (Synthon LJ)

### 2.86.1 3-(1-((3-(2-((((2-(2-(2-aminoethoxy)ethoxy)-4-(((2S,3R,4S,5S,6S)-6-carboxy-3,4,5-trihydroxytetrahydro-2H-pyran-2-yl)oxy)benzyl)oxy)carbonyl)(3-carboxypropyl)amino)ethoxy)-5,7-dimethyladamantan-1-yl)methyl)-5-methyl-1H-pyrazol-4-yl)-6-(8-(benzo[d]thiazol-2-ylcarbarnoyl)-3,4-dihydroisoquinolin-2(1H)-yl)picolinic acid

To a solution of Example 1.56 (0.024 g) and Example 2.62.6 (0.030 g) in N,N-dimethylformamide (0.4 mL) was added N,N-diisopropylethylamine (0.025 mL), and the reaction was stirred overnight. The reaction was concentrated, and the residue dissolved in tetrahydrofuran (0.5 mL) and methanol (0.5 mL) and treated with lithium hydroxide hydrate (0.018 g) as a solution in water (0.5 mL). After stirring for 1 hour, the reaction was diluted with N,N-dimethylformamide (1 mL) and purified by reverse phase HPLC using a Gilson system, eluting with 10-75% acetonitrile in water containing 0.1% v/v trifluoroacetic acid. The desired fractions were combined and freeze-dried to provide the title compound. MS (ESI) m/e 1262.7 (M+H)⁺.

### 2.86.2 4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](3-carboxypropyl)carbamoyl}oxy)methyl]-3-[2-(2-{[3-(2,5-dioxo-2,5-dihydro-1H-pyrrol-1-yl)propanoyl]amino}ethoxy)ethoxy]phenyl beta-D-glucopyranosiduronic acid

To a solution of Example 2.86.1 (0.0173 g) and 2,5-dioxopyrrolidin-1-yl 3-(2,5-dioxo-2,5-dihydro-1H-pyrrol-1-yl)propanoate (4.38 mg) in N,N-dimethylformamide (0.8 mL) was added 2,5-dioxopyrrolidin-1-yl 3-(2,5-dioxo-2,5-dihydro-1H-pyrrol-1-yl)propanoate (4.38 mg), and the reaction was stirred for 2 hours. The reaction was diluted with a 1:1 mixture of N,N-dimethylformamide:water (1 mL), and the mixture was purified by reverse phase HPLC using a Gilson system, eluting with 10-80% acetonitrile in water containing 0.1% v/v trifluoroacetic acid. The desired fractions were combined and freeze-dried to provide the title compound. ¹H NMR (400 MHz, dimethyl sulfoxide-d₆) δ ppm 12.77 (s, 1H), 8.03 (d, 1H), 7.99 (t, 1H), 7.77 (d,1H), 7.62 (d, 1H), 7.55-7.41 (m, 3H), 7.40-7.32 (m, 2H), 8.28 (s, 1H), 7.23-7.17 (m, 1H), 6.97 (s, 2H), 6.94 (d, 1H), 6.66 (s, 1H), 6.60 (dd, 1H), 5.07 (m, 1H), 5.00-4.91 (m, 4H), 4.17-4.02 (m, 2H), 3.96-3.85 (m, 2H), 3.85-3.76 (m, 2H), 3.71 (t, 2H), 3.64-3.56 (m, 4H), 3.34-3.12 (m, 10H), 3.01 (, 2H), 2.33 (t, 2H), 2.24-2.12 (m, 2H), 2.09 (s, 3H), 1.70 (p, 2H), 1.45-0.88 (m, 12H), 0.88-0.77 (m, 6H); MS (ESI) m/e 1434.2 (M+Na)⁺.

### 2.87 Synthesis of 4-({[(4-{[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](3-carboxypropyl)amino}piperidin-1-yl)carbonyl] oxy}methyl)-3- [2-(2-{ [3-(2,5-dioxo-2,5-dihydro-1H-pyrrol-1-yl)propanoyl]amino}ethoxy)ethoxy]phenyl beta-D-glucopyranosiduronic acid (Synthon MA)

### 2.87.1 3-(1-((3-(2-((1-(((2-(2-(2-aminoethoxy)ethoxy)-4-(((2S,3R,4S,5S,6S)-6-carboxy-3,4,5-trihydroxytetrahydro-2H-pyran-2-yl)oxy)benzyl)oxy)carbonyl)piperidin-4-yl)(3-carboxypropyl)amino)ethoxy)-5,7-dimethyladamantan-1-yl)methyl)-5-methyl-1H-pyrazol-4-yl)-6-(8-(benzo[d]thiazol-2-ylcarbarnoyl)-3,4-dihydroisoquinolin-2(1H)-yl)picolinic acid

A solution of Example 1.42 (0.050 g) and Example 2.62.6 (0.050 g) in N,N-dimethylformamide (0.5 mL) was treated with N,N-diisopropylethylamine (0.042 mL), and the reaction was stirred at room temperature for 2 hours. The reaction was concentrated, and the residue was dissolved in methanol (0.5 mL) and tetrahydrofuran (0.5 mL) and treated with lithium hydroxide hydrate (0.031 g) as a solution in water (0.5 mL). The reaction was stirred for 1.5 hours and diluted with N,N-dimethylformamide (1 mL). The mixture was purified by reverse phase HPLC using a Gilson system, eluting with 10-80% acetonitrile in water containing 0.1 % v/v trifluoroacetic acid. The desired fractions were combined and freeze-dried to provide the title compound. MS (ESI) m/e 1345.7 (M+H)⁺.

### 2.87.2 4-({[(4-{[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](3-carboxypropyl)amino}piperidin-1-yl)carbonyl]oxy}methyl)-3-[2-(2-{[3-(2,5-dioxo-2,5-dihydro-1H-pyrrol-1-yl)propanoyl]amino}ethoxy)ethoxy]phenyl beta-D-glucopyranosiduronic acid

A solution of Example 2.87.1 (0.047 g) and 2,5-dioxopyrrolidin-1-yl 3-(2,5-dioxo-2,5-dihydro-1H-pyrrol-1-yl)propanoate (0.011 g) in N,N-dimethylformamide (0.5 mL) was treated with N,N-diisopropylethylamine (0.031 mL), and the reaction was stirred at room temperature for 2 hours. The reaction was diluted with a 1:1 mixture of N,N-dimethylformamide:water(2 mL). The mixture was purified by reverse phase HPLC using a Gilson system, eluting with 10-85% acetonitrile in water containing 0.1% v/v trifluoroacetic acid. The desired fractions were combined and freeze-dried to provide the title compound. ¹H NMR (400 MHz, dimethyl sulfoxide-d₆) δ ppm 12.87 (s, 1H), 8.96 (s, 1H), 8.15-8.07 (m, 2H), 7.88 (d, J= 8.1 Hz, 1H), 7.71 (d, J= 7.5 Hz, 1H), 7.62-7.50 (m, 3H), 7.50-7.45 (m, 1H), 7.45-7.42 (m, 1H), 7.37 (s, 1H), 7.33-7.27 (m, 1H), 7.07 (s, 2H), 7.07-7.02 (m, 1H), 6.80-6.74 (m, 1H), 6.72-6.66 (m, 1H), 5.23-5.14 (m, 1H), 5.13-5.00 (m, 4H), 4.27-4.12 (m, 4H), 4.06-3.95 (m, 4H), 3.92 (s, 2H), 3.83-3.78 (m, 2H), 3.57-3.32 (m, 10H), 3.32-3.14 (m, 4H), 3.14-3.06 (m, 2H), 2.90 (s, 2H), 2.49-2.37 (m, 4H), 2.19 (s, 3H), 2.12-2.01 (m, 2H), 2.02-1.88 (m, 2H), 1.74-1.57 (m, 2H), 1.52 (s, 2H), 1.45-1.30 (m, 4H), 1.30-1.05 (m, 6H), 0.95 (s, 6H); MS (ESI) m/e 1495.4 (M+H)⁺.

### 2.88 Synthesis of 4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](3-sulfopropyl)carbamoyl}oxy)methyl]-3-[2-(2-{[3-(2,5-dioxo-2,5-dihydro-1H-pyrrol-1-yl)propanoyl]amino}ethoxy)ethoxy]phenyl beta-D-glucopyranosiduronic acid (Synthon MD)

### 2.88.1 3-(1-((3-(2-((((2-(2-(2-aminoethoxy)ethoxy)-4-(((2S,3R,4S,5S,6S)-6-carboxy-3,4,5-trihydroxytetrahydro-2H-pyran-2-yl)oxy)benzyl)oxy)carbonyl)(3-sulfopropyl)amino)ethoxy)-5,7-dimethyladamantan-1-yl)methyl)-5-methyl-1H-pyrazol-4-yl)-6-(8-(benzo[d]thiazol-2-ylcarbamoyl)-3,4-dihydroisoquinolin-2(1H)-yl)picolinic acid

A solution of Example 1.6 (0.039 g) and Example 2.62.6 (0.041 g) in N,N-dimethylformamide (0.5 mL) was treated with N,N-diisopropylethylamine (0.035 mL), and the reaction was stirred at room temperature for 2 hours. The reaction was concentrated, and the residue was dissolved in methanol (0.5 mL) and tetrahydrofuran (0.5 mL) and treated with lithium hydroxide hydrate (0.025 g) as a solution in water (0.5 mL). The reaction was stirred for 1.5 hours and diluted with N,N-dimethylformamide (1 mL). The mixture was purified by reverse phase HPLC using a Gilson system, eluting with 10-80% acetonitrile in water containing 0.1% v/v trifluoroacetic acid. The desired fractions were combined and freeze-dried to provide the title compound. MS (ESI) m/e 1297.8 (M+H)⁺.

### 2.88.2 4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](3-sulfopropyl)carbamoyl}oxy)methyl]-3- [2-(2- {[3-(2,5-dioxo-2,5-dihydro-1H-pyrrol-1-yl)propanoyl]amino}ethoxy)ethoxy]phenyl beta-D-glucopyranosiduronic acid

To a solution of Example 2.88.1 (0.024 g) and 2,5-dioxopyrrolidin-1-yl 3-(2,5-dioxo-2,5-dihydro-1H-pyrrol-1-yl)propanoate (6.40 mg) in N,N-dimethylformamide (0.5 mL) was added N,N-diisopropylethylamine (0.016 mL), and the reaction was stirred at room temperature for 1 hour. The reaction was diluted with a 1:1 mixture of N,N-dimethylformamide:water (2 mL). The mixture was purified by reverse phase HPLC using a Gilson system, eluting with 10-80% acetonitrile in water containing 0.1% v/v trifluoroacetic acid. The desired fractions were combined and freeze-dried to provide the title compound. ¹H NMR (500 MHz, dimethyl sulfoxide-*d*₆) δ ppm 12.87 (s, 1H), 8.09-8.02 (m, 2H), 7.79 (d, 1H), 7.61 (d, 1H), 7.52 (dd, 1H), 7.50-7.42 (m, 2H), 7.40-7.33 (m, 2H), 7.31 (s, 1H), 7.20 (t, 1H), 6.98 (s, 3H), 6.66 (s, 1H), 6.60 (dd, 1H), 5.06 (t, 1H), 4.96 (s, 4H), 4.10 (dq, 4H), 3.81 (d, 4H), 3.71 (t, 2H), 3.59 (t, 2H), 3.51-3.35 (m, 4H), 3.26 (td, 6H), 3.17 (q, 2H), 3.01 (t, 2H), 2.35 (dt, 4H), 2.10 (d, 3H), 1.75 (d, 2H), 1.44-0.88 (m, 12H), 0.82 (d, 6H); MS (ESI) m/e 1446.4 (M-H)⁻.

### 2.89 Synthesis of N-[6-(2,5-dioxo-2,5-dihydro-1H-pyrrol-1-yl)hexanoyl]-L-valyl-N-[4-({[(3-{[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)amino}azetidin-1-yl)carbonyl]oxy}methyl)phenyl]-N⁵-carbamoyl-L-ornithinamide (Synthon MG)

A solution of Example 1.60 (0.026 g), 4-((S)-2-((S)-2-(6-(2,5-dioxo-2,5-dihydro-1H-pyrrol-1-yl)hexanamido)-3 -methylbutanamido)-5 -ureidopentanamido)benzyl (4-nitrophenyl) carbonate (0.024 g) and N,N-diisopropylethylamine (0.022 mL) were stirred together in N,N-dimethylformamide (0.8 mL) at room temperature for 3 hours. The reaction was diluted with a 1:1 mixture of N,N-dimethylformamide:water (2 mL). The mixture was purified by reverse phase HPLC using a Gilson system, eluting with 10-80% acetonitrile in water containing 0.1% v/v trifluoroacetic acid. The desired fractions were combined and freeze-dried to provide the title compound. ¹H NMR (400 MHz, dimethyl sulfoxide-*d*₆) δ ppm 12.85 (s, 1H), 9.99 (s, 1H), 8.06 (d, 1H), 8.03 (d, 1H), 7.79 (dd, 2H), 7.60 (dd, 3H), 7.55-7.41 (m, 3H), 7.36 (td, 2H), 7.29 (t, 3H), 6.99 (s, 2H), 6.95 (d, 1H), 5.99 (s, 1H), 5.04-4.92 (m, 4H), 4.37 (q, 1H), 4.34-4.24 (m, 1H), 4.24-4.10 (m, 4H), 3.88 (t, 2H), 3.82 (s, 2H), 3.40-3.29 (m, 4H), 3.01 (t, 2H), 2.99-2.91 (m, 1H), 2.87 (t, 2H), 2.25-2.06 (m, 5H), 1.95 (dt, 1H), 1.68 (s, 1H), 1.60 (s, 1H), 1.54-1.24 (m, 12H), 1.24-0.94 (m, 9H), 0.90-0.78 (m, 12H); MS (ESI) m/e 1507.4 (M+H)⁺.

### 2.90 Synthesis of 6-[8-(1,3-benzothiazol-2-ylcarbamoyl)-3,4-dihydroisoquinolin-2(1H)-yl]-3-{1-[(3-{[26-(2,5-dioxo-2,5-dihydro-1H-pyrrol-1-yl)-8,24-dioxo-3-(2-sulfoethyl)-11,14,17,20-tetraoxa-3,7,23-triazahexacos-1-yl] oxy}-5,7-dimethyltricyclo[3.3.1.1^{3,7}]dec-1-yl)methyl]-5-methyl-1H-pyrazol-4-yl}pyridine-2-carboxylic acid (Synthon MS)

To a mixture of Example 1.61.2 (15 mg) and 2,5-dioxopyrrolidin-1-yl 1-(2,5-dioxo-2,5-dihydro-1H-pyrrol-1-yl)-3-oxo-7,10,13,16-tetraoxa-4-azanonadecan-19-oate (16.91 mg) in N,N-dimethylformamide (0.8 mL) was added N,N-diisopropylethylamine (28.8 µL) at 0 °C. The mixture was stirred for 3 hours and purified by reverse phase HPLC, using a Gilson system and C18 column, eluting with 20-60% acetonitrile in water containing 0.1% trifluoroacetic acid, to provide the title compound. ¹H NMR (400 MHz, dimethyl sulfoxide-*d*₆) δ ppm 12.87 (s, 1H), 8.98 (s, 1H), 8.08-7.92 (m, 3H), 7.79 (d, 1H), 7.62 (d, 1H), 7.57-7.41 (m, 3H), 7.36 (td, 2H), 7.29 (s, 1H), 7.04-6.92 (m, 3H), 4.96 (s, 2H), 3.89 (t, 2H), 3.83 (s, 2H), 3.48 (d, 4H), 3.44-3.17 (m, 3H), 3.18-2.83 (m, 10H), 2.38-2.24 (m, 4H), 2.11 (s, 3H), 1.78 (m, 2H), 1.50-0.94 (m, 12H), 0.86 (s, 6H). MS (ESI) m/e 1309.3 (M-H).

### 2.91 Synthesis of N-[6-(2,5-dioxo-2,5-dihydro-1H-pyrrol-1-yl)hexanoyl]-L-valyl-N-[4-({[(3-{[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)amino}propyl)carbamoyl]oxy}methyl)phenyl]-N⁵-carbamoyl-L-ornithinamide (Synthon MR)

To a mixture of Example 1.61.2 (12.8 mg) and 4-((S)-2-((S)-2-(6-(2,5-dioxo-2,5-dihydro-1H-pyrrol-1-yl)hexanamido)-3-methylbutanamido)-5-ureidopentanamido)benzyl (4-nitrophenyl) carbonate (10.4 mg) in N,N-dimethylformamide (0.5 mL) at 0 °C was added N,N-diisopropylethylamine (24.54 µL). The mixture was stirred for 3 hours and purified by reverse phase HPLC using a Gilson system and a C18 column, eluting with 20-60% acetonitrile in water containing 0.1% trifluoroacetic acid, to provide the title compound. ¹H NMR (400 MHz, dimethyl sulfoxide-*d*₆) δ ppm 12.85 (s, 1H), 9.97 (s, 1H), 8.97 (s, 1H), 8.04 (t, 2H), 7.79 (dd, 2H), 7.65-7.40 (m, 7H), 7.36 (td, 3H), 7.28 (d, 3H), 6.99 (s, 2H), 6.95 (d, 1H), 5.98 (s, 1H), 4.95 (d, 4H), 4.49-4.30 (m, 1H), 4.24-4.11 (m, 1H), 3.88 (t, 2H), 3.82 (s, 2H), 3.36 (t, 3H), 3.18-2.84 (m, 9H), 2.25-1.88 (m, 5H), 1.85-0.90 (m, 14H), 0.91-0.75 (m, 13H). MS (ESI) m/e (M+H).

### 2.92 Synthesis of N-{6-[(iodoacetyl)amino]hexanoyl}-L-valyl-N-{4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)methyl]phenyl}-N⁵-carbamoyl-L-ornithinamide (Synthon MQ)

To a mixture of Example 1.2.9 (8.2 mg) and 2,5-dioxopyrrolidin-1-yl 6-(2-iodoacetamido)hexanoate (4.7 mg) in N,N-dimethylformamide (0.3 mL) in an ice-bath was added N,N-diisopropylethylamine (3 µL). The mixture was stirred at 0 °C for 1.5 hours. The reaction was diluted with dimethyl sulfoxide, and the mixture purified by reverse phase HPLC using a Gilson system and a C18 column, eluting with 20-60% acetonitrile in water containing 0.1% trifluoroacetic acid, to provide the title compound. ¹H NMR (400 MHz, dimethyl sulfoxide-*d*₆) δ ppm 12.87 (s, 1H), 10.00 (s, 1H), 8.21 (d, 1H), 8.06 (dd, 2H), 7.81 (dd, 2H), 7.60 (t, 3H), 7.48 (ddd, 3H), 7.36 (td, 2H), 7.28 (d, 3H), 6.95 (d, 1H), 4.97 (d, 4H), 4.39 (q, 1H), 4.19 (t, 1H), 3.88 (t, 2H), 3.80 (d, 2H), 3.25 (d, 2H), 2.97 (dq, 6H), 2.63 (s, 2H), 2.25-1.88 (m, 5H), 1.78-0.70 (m, 29H). MS (ESI) m/e 1538.4 (M-H).

### 2.93 Synthesis of N-{6-[(ethenylsulfonyl)amino]hexanoyl}-L-valyl-N-{4-[({[2-({3- [(4- {6- [8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)methyl]phenyl}-N⁵-carbamoyl-L-ornithinamide (Synthon MZ)

### 2.93.1 methyl 6-(vinylsulfonamido)hexanoate

To a solution of 6-methoxy-6-oxohexan-1-aminium chloride (0.3 g) and triethylamine (1.15 mL) in dichloromethane at 0 °C was added ethenesulfonyl chloride (0.209 g) dropwise. The reaction mixture was warmed to room temperature and stirred for 1 hour. The mixture was diluted with dichloromethane and washed with brine. The organic layer was dried over sodium sulfate, filtered, and concentrated to provide the title compound. MS (ESI) m/e 471.0 (2M+H)⁺.

### 2.93.2 6-(vinylsulfonamido)hexanoic acid

A solution of Example 2.93.1 (80 mg) and lithium hydroxide monohydrate (81 mg) in a mixture of tetrahydrofuran (1 mL) and water (1 mL) was stirred for 2 hours, then diluted with water (20 mL), and washed with diethyl ether (10 mL). The aqueous layer was acidified to pH 4 with IN aqueous HCl and extracted with dichloromethane (3 x 10 mL). The organic layer was washed with brine (5 mL), dried over sodium sulfate, filtered and concentrated to provide the title compound.

### 2.93.3 2,5-dioxopyrrolidin-1-yl 6-(vinylsulfonamido)hexanoate

A mixture of Example 2.93.2 (25 mg), 1-ethyl-3-[3-(dimethylamino)propyl]-carbodiimide hydrochloride (43.3 mg) and 1-hydroxypyrrolidine-2,5-dione (15.6 mg) in dichloromethane (8 mL) was stirred overnight, washed with saturated aqueous ammonium chloride solution and brine, and concentrated to provide the title compound.

### 2.93.4 N-{6-[(ethenylsulfonyl)amino]hexanoyl}-L-valyl-N-{4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.13,7]dec-1-yl}oxy)ethyl](2-sulfoethyl)carbamoyl}oxy)methyl]phenyl}-N5-carbamoyl-L-ornithinamide

The title compound was prepared as described in Example 2.83, replacing Example 1.2.9 and 2,5-dioxopyrrolidin-1-yl 6-(2-chloroacetamido)hexanoate with Example 2.66.1 and Example 2.93.3, respectively. ¹H NMR (400 MHz, dimethyl sulfoxide-*d*₆) δ ppm 12.85 (s, 1H), 9.98 (s, 1H), 8.05 (dd, 2H), 7.79 (d, 2H), 7.60 (t, 3H), 7.55-7.40 (m, 3H), 7.36 (td, 2H), 7.27 (d, 3H), 7.19 (t, 1H), 6.95 (d, 1H), 6.66 (dd, 1H), 6.09-5.90 (m, 2H), 4.97 (d, 4H), 4.39 (q, 1H), 4.20 (t, 1H), 3.88 (t, 2H), 3.80 (d, 2H), 3.25 (d, 2H), 2.97 (dt, 4H), 2.78 (q, 2H), 2.64 (q, 2H), 2.22-1.86 (m, 6H), 1.77-0.89 (m, 16H), 0.89-0.72 (m, 12H). MS (ESI) m/e 1460.6 (M-H).

### 2.94 Synthesis of 6-[8-(1,3-benzothiazol-2-ylcarbamoyl)-3,4-dihydroisoquinolin-2(1H)-yl]-3-(1-{[3-(2-{[3-({6-[(iodoacetyl)amino]hexanoyl}amino)propyl](2-sulfoethyl)amino}ethoxy)-5,7-dimethyltricyclo[3.3.1.1^{3,7}]dec-1-yl]methyl}-5-methyl-1H-pyrazol-4-yl)pyridine-2-carboxylic acid (Synthon NA)

The title compound was prepared using the procedure in Example 2.83, replacing Example 1.2.9 and 2,5-dioxopyrrolidin-1-yl 6-(2-chloroacetamido)hexanoate with Example 2.61.2 and 2,5-dioxopyrrolidin-1-yl 6-(2-iodoacetamido)hexanoate, respectively. ¹H NMR (400 MHz, dimethyl sulfoxide-*d*₆) δ ppm 12.87 (s, 1H), 8.98 (s, 1H), 8.20 (t, 1H), 8.04 (d, 1H), 7.91 (t, 1H), 7.79 (d, 1H), 7.62 (d, 1H), 7.53 (d, 1H), 7.50-7.41 (m, 2H), 7.36 (td, 2H), 7.29 (s, 1H), 6.96 (d, 1H), 4.96 (s, 2H), 3.89 (t, 2H), 3.83 (s, 2H), 3.06 (dt, 8H), 2.89 (t, 2H), 2.17-1.99 (m, 5H), 1.76 (s, 2H), 1.56-0.93 (m, 14H), 0.86 (s, 6H). MS (ESI) m/e 1190.3 (M-H).

### 2.95 Synthesis of 6-[8-(1,3-benzothiazol-2-ylcarbamoyl)-3,4-dihydroisoquinolin-2(1H)-yl]-3-{1-[(3-{2-[(3-{[6-(ethenylsulfonyl)hexanoyl]amino}propyl)(2-sulfoethyl)amino]ethoxy}-5,7-dimethyltricyclo[3.3.1.1^{3,7}]dec-1-yl)methyl]-5-methyl-1H-pyrazol-4-yl}pyridine-2-carboxylic acid (Synthon NB)

The title compound was prepared using the procedure in Example 2.83, replacing Example 1.2.9 and 2,5-dioxopyrrolidin-1-yl 6-(2-chloroacetamido)hexanoate with Example 1.61.2 and Example 2.82.5, respectively. ¹H NMR (400 MHz, dimethyl sulfoxide-*d*₆) δ ppm 12.87 (s, 1H), 8.98 (s, 1H), 8.04 (d, 1H), 7.92 (t, 1H), 7.79 (d, 1H), 7.62 (d, 1H), 7.53 (d, 1H), 7.51-7.41 (m, 2H), 7.36 (td, 2H), 7.29 (s, 1H), 7.01-6.90 (m, 2H), 6.29-6.16 (m, 2H), 4.96 (s, 2H), 3.89 (t, 2H), 3.83 (s, 2H), 3.45-3.19 (m, 2H), 3.19-2.95 (m, 8H), 2.89 (t, 2H), 2.16-1.98 (m, 5H), 1.84-1.66 (m, 2H), 1.64-1.21 (m, 13H), 1.08 (dq, 6H), 0.86 (s, 6H). MS (ESI) m/e 1199.3 (M+H).

### 2.96 Synthesis of N-[3-(2,5-dioxo-2,5-dihydro-1H-pyrrol-1-yl)propanoyl]-L-valyl-N-{4-[({[2-({3-[(4-{6-[1-(1,3-benzothiazol-2-ylcarbamoyl)-1,2,3,4-tetrahydroquinolin-7-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)methyl]phenyl}-N⁵-carbamoyl-L-ornithinamide (Synthon NP)

### 2.96.1 (S)-(9H-fluoren-9-yl)methyl (1-((4-(hydroxymethyl)phenyl)amino)-1-oxo-5-ureidopentan-2-yl)carbamate

(S)-2-((((9H-Fluoren-9-yl)methoxy)carbonyl)amino)-5-ureidopentanoic acid (40 g) was dissolved in dichloromethane (1.3L). (4-Aminophenyl)methanol (13.01 g), 2-(3H-[1,2,3]triazolo[4,5-b]pyridin-3-yl)-1,1,3,3-tetramethylisouronium hexafluorophosphate(V) (42.1 g) and N,N-diisopropylethylamine (0.035 L) were added to the solution, and the resulting mixture was stirred at room temperature for 16 hours. The product was collected by filtration and rinsed with dichloromethane. The combined solids were dried under vacuum to yield the title compound, which was used in the next step without further purification. MS (ESI) m/e 503.3 (M+H)⁺.

### 2.96.2 (S)-2-amino-N-(4-(hydroxymethyl)phenyl)-5-ureidopentanamide

Example 2.96.1 (44 g) was dissolved in N,N-dimethylformamide (300 mL). The solution was treated with diethylamine (37.2 mL) and stirred for one hour at room temperature. The reaction mixture was filtered, and the solvent was concentrated under reduced pressure. The crude product was purified by basic alumina chromatography eluting with a gradient of 0-30% methanol in ethyl acetate to give the title compound. MS (ESI) m/e 281.2 (M+H)⁺.

### 2.96.3 tert-butyl ((S)-1-(((S)-1-((4-(hydroxymethyl)phenyl)amino)-1-oxo-5-ureidopentan-2-yl)amino)-3-methyl-1-oxobutan-2-yl)carbamate

(S)-2-(Tert-butoxycarbonylamino)-3-methylbutanoic acid (9.69 g) was dissolved in N,N-dimethylformamide (200 mL). To the solution was added 2-(3H-[1,2,3]triazolo[4,5-b]pyridin-3-yl)-1,1,3,3-tetramethylisouronium hexafluorophosphate(V) (18.65 g), and the reaction was stirred for one hour at room temperature. Example 2.96.2 (12.5 g) and N,N-diisopropylethylamine (15.58 mL) were added and the reaction mixture was stirred for 16 hours at room temperature. The solvent was concentrated under reduced pressure and the residue was purified by silica gel chromatography, eluting with 10% methanol in dichloromethane, to give the title compound. MS (ESI) m/e 480.2 (M+H)⁺.

### 2.96.4 (S)-2-((S)-2-amino-3-methylbutanamido)-N-(4-(hydroxymethyl)phenyl)-5-ureidopentanamide

Example 2.96.3 (31.8 g) was dissolved in dichloromethane (650 mL) and trifluoroacetic acid (4.85 mL) was added to the solution. The reaction mixture was stirred for three hours at room temperature. The solvent was concentrated under reduced pressure to yield a mixture of the crude title compound and 4-((S)-2-((S)-2-amino-3-methylbutanamido)-5-ureidopentanamido)benzyl 2,2,2-trifluoroacetate. The crude material was dissolved in a 1:1 dioxane/water solution (300 mL) and to the solution was added sodium hydroxide (5.55 g). The mixture was stirred for three hours at room temperature. The solvent was concentrated under vacuum, and the crude product was purified by reverse phase HPLC using a CombiFlash system, eluting with a gradient of 5-60% acetonitrile in water containing 0.05% v/v ammonium hydroxide, to give the title compound. MS (ESI) m/e 380.2 (M+H)⁺.

### 2.96.5 (S)-2-((S)-2-(3-(2,5-dioxo-2,5-dihydro-1H-pyrrol-1-yl)propanamido)-3-methylbutanamido)-N-(4-(hydroxymethyl)phenyl)-5-ureidopentanamide

To a solution of Example 2.96.4 (38 mg) in N,N-dimethylformamide (1 mL)was added 2,5-dioxopyrrolidin-1-yl 3-(2,5-dioxo-2,5-dihydro-1H-pyrrol-1-yl)propanoate (26.7 mg). The reaction mixture was stirred at room temperature overnight and purified by reverse phase HPLC using a Gilson system, eluting with a gradient of 10-85% acetonitrile in water containing 0.1% v/v trifluoroacetic acid, to give the title compound. MS (ESI) m/e 531.06 (M+H)⁺.

### 2.96.6 4-((S)-2-((S)-2-(3-(2,5-dioxo-2,5-dihydro-1H-pyrrol-1-yl)propanamido)-3-methylbutanamido)-5-ureidopentanamido)benzyl (4-nitrophenyl) carbonate

To a solution of Example 2.96.5 (53.1 mg) in N,N-dimethylformamide (3 mL) was added bis(4-nitrophenyl) carbonate (60.8 mg). The reaction mixture was stirred at room temperature overnight and purified by reverse phase HPLC using a Gilson system, eluting with a gradient of 10-85% acetonitrile in water containing 0.1% v/v trifluoroacetic acid, to give the title compound. MS (ESI) m/e 696.2 (M+H)⁺.

### 2.96.7 N-[3-(2,5-dioxo-2,5-dihydro-1H-pyrrol-1-yl)propanoyl]-L-valyl-N-{4- [({[2-({3-[(4-{6-[1-(1,3-benzothiazol-2-ylcarbamoyl)-1,2,3,4-tetrahydroquinolin-7-yl]-2-carboxypyridin-3-yl}-5-methyl-1H-pyrazol-1-yl)methyl]-5,7-dimethyltricyclo[3.3.1.13,7]dec-1-yl}oxy)ethyl](2-sulfoethyl)carbamoyl}oxy)methyl]phenyl}-N5-carbamoyl-L-ornithinamide

The title compound was prepared as described in Example 2.1, replacing Example 1.2.9 and 4-((S)-2-((S)-2-(6-(2,5-dioxo-2,5-dihydro-1H-pyrrol-1-yl)hexanamido)-3-methylbutanamido)-5-ureidopentanamido)benzyl (4-nitrophenyl) carbonate with Example 1.24.2 and Example 2.96.6, respectively. ¹H NMR (400 MHz, dimethyl sulfoxide-*d*₆) δ ppm 9.91 (s, 1H), 9.80 (s, 2H), 8.33 (s, 2H), 7.96 (s, 2H), 7.81 (d, 4H), 7.61 (s, 2H), 7.43 (d, 10H), 7.34-7.02 (m, 14H), 5.92 (s, 8H), 4.94-4.70 (m, 6H), 4.18 (d, 11H), 3.85 (s, 8H), 3.05-2.66 (m, 8H), 2.30-2.13 (m, 14H), 2.03-1.49 (m, 2H), 0.92-0.63 (m, 40H). MS (ESI) m/e 1408.3 (M-H)⁺.

### 2.97 Synthesis of 6-[8-(1,3-benzothiazol-2-ylcarbamoyl)-3,4-dihydroisoquinolin-2(1H)-yl]-3-{1-[(3-{2-[(2-carboxyethyl){[(2-{[(2S,3R,4S,5S,6S)-6-carboxy-3,4,5-trihydroxytetrahydro-2H-pyran-2-yl] oxy}-4-[2-(2-{[3-(2,5-dioxo-2,5-dihydro-1H-pyrrol-1-yl)propanoyl]amino}ethoxy)ethoxy]benzyl)oxy]carbonyl}amino]ethoxy}-5,7-dimethyltricyclo[3.3.1.1^{3,7}]dec-1-yl)methyl]-5-methyl-1H-pyrazol-4-yl}pyridine-2-carboxylic acid (Synthon NN)

### 2.97.1 4-(2-(2-bromoethoxy)ethoxy)-2-hydroxybenzaldehyde

A solution of 2,4-dihydroxybenzaldehyde (1.0 g), 1-bromo-2-(2-bromoethoxy)ethane (3.4 g) and potassium carbonate (1.0 g) in acetonitrile (30 mL) was heated to 75 °C for 2 days. The reaction was cooled, diluted with ethyl acetate (100 mL), washed with water (50 mL) and brine (50 mL), dried over magnesium sulfate, filtered and concentrated. Purification of the residue by silica gel chromatography, eluting with a gradient of 5-30% ethyl acetate in heptane, provided the title compound. MS (ELSD) m/e 290.4 (M+H)⁺.

### 2.97.2 4-(2-(2-azidoethoxy)ethoxy)-2-hydroxybenzaldehyde

To a solution of Example 2.97.1 (1.26 g) in N,N-dimethylformamide (10 mL) was added sodium azide (0.43 g), and the reaction was stirred at room temperature overnight. The reaction was diluted with diethyl ether (100 mL), washed with water (50 mL) and brine (50 mL), dried over magnesium sulfate, filtered, and concentrated. Purification of the residue by silica gel chromatography, eluting with a gradient of 5-30% ethyl acetate in heptane, gave the title compound. MS (ELSD) m/e 251.4 (M+H)⁺.

### 2.97.3 (2S,3R,4S,5S,6S)-2-(5-(2-(2-azidoethoxy)ethoxy)-2-formylphenoxy)-6-(methoxycarbonyl)tetrahydro-2H-pyran-3,4,5-triyl triacetate

A solution of Example 2.97.2 (0.84 g), (3R,4S,5S,6S)-2-bromo-6-(methoxycarbonyl)tetrahydro-2H-pyran-3,4,5-triyl triacetate (1.99 g) and silver (I) oxide (1.16 g) were stirred together in acetonitrile (15 mL). After stirring overnight, the reaction was diluted with dichloromethane (20 mL). Diatomaceous earth was added, and the reaction filtered and concentrated. Purification of the residue by silica gel chromatography, eluting with a gradient of 5-75% ethyl acetate in heptane, gave the title compound.

### 2.97.4 (2S,3R,4S,5S,6S)-2-(5-(2-(2-azidoethoxy)ethoxy)-2-(hydroxymethyl)phenoxy)-6-(methoxycarbonyl)tetrahydro-2H-pyran-3,4,5-triyl triacetate

A solution of Example 2.97.3 (0.695 g) in methanol (5 mL) and tetrahydrofuran (2 mL) was cooled to 0 °C. Sodium borohydride (0.023 g) was added, and the reaction was warmed to room temperature. After stirring for a total of 1 hour, the reaction was poured into a mixture of ethyl acetate (75 mL) and water (25 mL), and saturated aqueous sodium bicarbonate (10 mL) was added. The organic layer was separated, washed with brine (50 mL), dried over magnesium sulfate, filtered, and concentrated. Purification of the residue by silica gel chromatography, eluting with a gradient of 5-85% ethyl acetate in heptane, gave the title compound. MS (ELSD) m/e 551.8 (M-H₂O)⁻.

### 2.97.5 (2S,3R,4S,5S,6S)-2-(5-(2-(2-aminoethoxy)ethoxy)-2-(hydroxymethyl)phenoxy)-6-(methoxycarbonyl)tetrahydro-2H-pyran-3,4,5-triyl triacetate

To Example 2.97.4 (0.465 g) in tetrahydrofuran (20 mL) was added 5% Pd/C (0.1 g) in a 50 mL pressure bottle, and the mixture was shaken for 16 hours under 30 psi hydrogen. The reaction was filtered and concentrated to give the title compound, which was used without further purification. MS (ELSD) m/e 544.1 (M+H)⁺.

### 2.97.6 (2S,3R,4S,5S,6S)-2-(5-(2-(2-((((9H-fluoren-9-yl)methoxy)carbonyl)amino)ethoxy)ethoxy)-2-(hydroxymethyl)phenoxy)-6-(methoxycarbonyl)tetrahydro-2H-pyran-3,4,5-triyl triacetate

A solution of Example 2.97.5 (0.443 g) in dichloromethane (8 mL) was cooled to 0 °C, then N,N-diisopropylethylamine (0.214 mL) and (9H-fluoren-9-yl)methyl carbonochloridate (0.190 g) were added. After 1 hour, the reaction was concentrated. Purification of the residue by silica gel chromatography, eluting with a gradient of 5-95% ethyl acetate in heptane, gave the title compound. MS (ELSD) m/e 748.15 (M-OH)⁻.

### 2.97.7 (2S,3R,4S,5S,6S)-2-(5-(2-(2-((((9H-fluoren-9-yl)methoxy)carbonyl)amino)ethoxy)ethoxy)-2-((((4-nitrophenoxy)carbonyl)oxy)methyl)phenoxy)-6-(methoxycarbonyl)tetrahydro-2H-pyran-3,4,5-triyl triacetate

To a solution of Example 2.97.6 (0.444 g) in N,N-dimethylformamide (5 mL) was added N,N-diisopropylethylamine (0.152 mL) and bis(4-nitrophenyl) carbonate (0.353 g), and the reaction was stirred at room temperature. After 5 hours, the reaction was concentrated. Purification of the residue by silica gel chromatography, eluting with a gradient of 5-90% ethyl acetate in heptane, gave the title compound.

### 2.97.8 3-(1-((3-(2-((((4-(2-(2-aminoethoxy)ethoxy)-2-(((2S,3R,4S,5S,6S)-6-carboxy-3,4,5-trihydroxytetrahydro-2H-pyran-2-yl)oxy)benzyl)oxy)carbonyl)(2-carboxyethyl)amino)ethoxy)-5,7-dimethyladamantan-1-yl)methyl)-5-methyl-1H-pyrazol-4-yl)-6-(8-(benzo[d]thiazol-2-ylcarbamoyl)-3,4-dihydroisoquinolin-2(1H)-yl)picolinic acid, trifluoroacetic acid salt

To a solution of Example 1.25 (0.070 g) and Example 2.97.7 (0.070 g) in N,N-dimethylformamide (0.4 mL) was added N,N-diisopropylethylamine (0.066 mL). After stirring overnight, the reaction was concentrated. The residue was dissolved in tetrahydrofuran (0.75 mL) and methanol (0.75 mL), and lithium hydroxide monohydrate (0.047 g) was added as a solution in water (0.75 mL). After 3 hours, the reaction was diluted with N,N-dimethylformamide (1 mL) and quenched with trifluoroacetic acid (0.116 mL). The mixture was purified by reverse phase HPLC using a Gilson system, eluting with 10-75% acetonitrile in water containing 0.1% v/v trifluoroacetic acid. The desired fractions were combined and freeze-dried to provide the title compound.

### 2.97.9 6-(8-(benzo[d]thiazol-2-ylcarbamoyl)-3,4-dihydroisoquinolin-2(1H)-yl)-3-(1-((3-(2-((((2-(((2S,3R,4S,5S,6S)-6-carboxy-3,4,5-trihydroxytetrahydro-2H-pyran-2-yl)oxy)-4-(2-(2-(3-(2,5-dioxo-2,5-dihydro-1H-pyrrol-1-yl)propanamido)ethoxy)ethoxy)benzyl)oxy)carbonyl)(2-carboxyethyl)amino)ethoxy)-5,7-dimethyladamantan-1-yl)methyl)-5-methyl-1H-pyrazol-4-yl)picolinic acid

A solution of Example 2.97.8 (0.027 g), 2,5-dioxopyrrolidin-1-yl 3-(2,5-dioxo-2,5-dihydro-1H-pyrrol-1-yl)propanoate (7.92 mg) and N,N-diisopropylethylamine (0.017 mL) were stirred together in N,N-dimethylformamide (0.4 mL) for 1 hour. The reaction was quenched with a 1:1 mixture of water and N,N-dimethylformamide (2 mL). The mixture was purified by reverse phase HPLC using a Gilson system, eluting with 10-75% acetonitrile in water containing 0.1% v/v trifluoroacetic acid. The desired fractions were combined and freeze-dried to provide the title compound. ¹H NMR (400 MHz, dimethyl sulfoxide-d₆) δ ppm 12.81 (s, 1H), 8.03 (d, 2H), 7.79 (d, 1H), 7.62 (d, 1H), 7.54-7.40 (m, 3H), 7.36 (td, 2H), 7.28 (s, 1H), 7.18 (d, 1H), 6.98 (s, 2H), 6.95 (d, 1H), 6.69 (d, 1H), 6.60 (d, 1H), 5.03 (d, 3H), 4.96 (s, 2H), 4.05 (s, 2H), 3.93 (d, 2H), 3.88 (t, 2H), 3.80 (d, 2H), 3.75-3.67 (m, 2H), 3.59 (t, 6H), 3.29 (q, 6H), 3.17 (q, 2H), 3.01 (t, 2H), 2.47 (d, 2H), 2.33 (t, 2H), 2.09 (s, 3H), 1.44-0.88 (m, 12H), 0.82 (d, 6H); MS (ESI) m/e 1396.5 (M-H)⁻.

### 2.98 Synthesis of N-[6-(2,5-dioxo-2,5-dihydro-1H-pyrrol-1-yl)hexanoyl]-3-sulfo-L-alanyl-L-valyl-N-{4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-carboxyethyl)carbamoyl}oxy)methyl]phenyl}-N⁵-carbamoyl-L-ornithinamide (Synthon NO)

### 2.98.1 3-(1-((3-(2-((((4-((S)-2-((S)-2-amino-3-methylbutanamido)-5-ureidopentanamido)benzyl)oxy)carbonyl)(2-carboxyethyl)amino)ethoxy)-5,7-dimethyladamantan-1-yl)methyl)-5-methyl-1H-pyrazol-4-yl)-6-(8-(benzo[d]thiazol-2-ylcarbamoyl)-3,4-dihydroisoquinolin-2(1H)-yl)picolinic acid

A solution of Example 1.25.2 (0.059 g), (9H-fluoren-9-yl)methyl ((S)-3-methyl-1-(((S)-1-((4-((((4-nitrophenoxy)carbonyl)oxy)methyl)phenyl)amino)-1-oxo-5-ureidopentan-2-yl)amino)-1-oxobutan-2-yl)carbamate (0.053 g) and N,N-diisopropylethylamine (0.055 mL) in N,N-dimethylformamide (0.5 mL) was stirred at room temperature overnight. Diethylamine (0.066 mL) was added to the reaction, and stirring was continued for 30 minutes. The reaction was diluted with a 1:1 mixture of N,N-dimethylformamide and water (2 mL) and quenched by the addition of trifluoroacetic acid (0.073 mL). The mixture was purified by reverse phase HPLC using a Gilson system, eluting with 10-75% acetonitrile in water containing 0.1% v/v trifluoroacetic acid. The desired fractions were combined and freeze-dried to provide the title compound. MS (ESI) m/e 1223.8 (M+H)⁺.

### 2.98.2 3-(1-((3-(2-((((4-((S)-2-((S)-2-((R)-2-amino-3-sulfopropanamido)-3-methylbutanamido)-5-ureidopentanamido)benzyl)oxy)carbonyl)(2-carboxyethyl)amino)ethoxy)-5,7-dimethyladamantan-1-yl)methyl)-5-methyl-1H-pyrazol-4-yl)-6-(8-(benzo[d]thiazol-2-ylcarbamoyl)-3,4-dihydroisoquinolin-2(1H)-yl)picolinic acid, trifluoroacetic acid salt

A solution of (R)-2-((((9H-fluoren-9-yl)methoxy)carbonyl)amino)-3-sulfopropanoic acid (0.021 g), O-(7-azabenzotriazol-1-yl)-N,N,N',N'-tetramethyluronium hexafluorophosphate (0.020 g) and N,N-diisopropylethylamine (0.031 mL) in N,N-dimethylformamide (0.4 mL) was stirred for 3 minutes. The solution was added to Example 2.98.1 (0.043 g) as a solution in N,N-dimethylformamide (0.4 mL). After stirring for 30 minutes, a solution of lithium hydroxide monohydrate (0.022 g) in water (0.5 mL) was added, and the reaction was stirred for 1 hour. The reaction was diluted with a 1:1 mixture of N,N-dimethylformamide and water (2 mL) and quenched by the addition of trifluoroacetic acid (0.054 mL). The mixture was purified by reverse phase HPLC using a Gilson system, eluting with 10-75% acetonitrile in water containing 0.1% v/v trifluoroacetic acid. The desired fractions were combined and freeze-dried to provide the title compound. MS (ESI) m/e 1376.5 (M+1).

### 2.98.3 6-(8-(benzo[d]thiazol-2-ylcarbamoyl)-3,4-dihydroisoquinolin-2(1H)-yl)-3-(1-((3-(2-((2-carboxyethyl)(((4-((S)-2-((S)-2-((R)-2-(6-(2,5-dioxo-2,5-dihydro-1H-pyrrol-1-yl)hexanamido)-3-sulfopropanamido)-3-methylbutanamido)-5-ureidopentanamido)benzyl)oxy)carbonyl)amino)ethoxy)-5,7-dimethyladamantan-1-yl)methyl)-5-methyl-1H-pyrazol-4-yl)picolinic acid

A solution of Example 2.98.2 (0.025 g), 2,5-dioxopyrrolidin-1-yl 6-(2,5-dioxo-2,5-dihydro-1H-pyrrol-1-yl)hexanoate (7.77 mg) and N,N-diisopropylethylamine (0.015 mL) in N,N-dimethylformamide (0.4 mL) was stirred for 1 hour. The reaction was diluted with a 1:1 mixture of water and N,N-dimethylformamide (2 mL). The mixture was purified by reverse phase HPLC using a Gilson system, eluting with 10-75% acetonitrile in water containing 0.1% v/v trifluoroacetic acid. The desired fractions were combined and freeze-dried to provide the title compound. ¹H NMR (400 MHz, dimethyl sulfoxide-d₆) δ ppm 12.85 (s, 1H), 9.46 (s, 1H), 8.20 (d, 1H), 8.07 (d, 1H), 8.03 (d, 1H), 8.00 (d, 1H), 7.79 (d, 1H), 7.69 (d, 2H), 7.61 (d, 1H), 7.51 (d, 1H), 7.49-7.45 (m, 1H), 7.43 (d, 1H), 7.36 (td, 2H), 7.29 (s, 1H), 7.25 (d, 2H), 6.97 (s, 2H), 6.95 (d, 1H), 4.98 (s, 2H), 4.96 (s, 2H), 4.73 (s, 2H), 4.16 (s, 2H), 4.03 (dd, 2H), 3.88 (t, 2H), 3.81 (s, 2H), 3.51-3.32 (m, 6H), 3.28 (t, 2H), 3.09 (dd, 1H), 3.06-2.94 (m, 4H), 2.89 (dd, 1H), 2.46 (d, 2H), 2.16 (dd, 1H), 2.09 (d, 4H), 1.74 (s, 2H), 1.62-1.29 (m, 8H), 1.29-0.92 (m, 12H), 0.92-0.78 (m, 12H). MS (ESI) m/e 1566.6 (M-H)⁻.

### 2.99 Synthesis of Control Synthon 4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](methyl)carbamoyl}oxy)methyl]-2-({N-[6-(2,5-dioxo-2,5-dihydro-1H-pyrrol-1-yl)hexanoyl]-beta-alanyl}amino)phenyl beta-D-glucopyranosiduronic acid (Synthon H)

### 2.99.1 (2S,3R,4S,5S,6S)-2-(4-formyl-2-nitrophenoxy)-6-(methoxycarbonyl)tetrahydro-2H-pyran-3,4,5-triyl triacetate

To a solution of (2R,3R,4S,5S,6S)-2-bromo-6-(methoxycarbonyl)tetrahydro-2H- pyran-3,4,5-triyl triacetate (4 g) in acetonitrile (100 mL)) was added silver(I) oxide (10.04 g) and 4-hydroxy-3-nitrobenzaldehyde (1.683 g). The reaction mixture was stirred for 4 hours at room temperature and filtered. The filtrate was concentrated, and the residue was purified by silica gel chromatography, eluting with 5-50% ethyl acetate in heptanes, to provide the title compound. MS (ESI) m/e (M+18)⁺.

### 2.99.2 (2S,3R,4S,5S,6S)-2-(4-(hydroxymethyl)-2-nitrophenoxy)-6-(methoxycarbonyl)tetrahydro-2H-pyran-3,4,5-triyl triacetate

To a solution of Example 2.99.1 (6 g) in a mixture of chloroform (75 mL) and isopropanol (18.75 mL) was added 0.87 g of silica gel. The resulting mixture was cooled to 0 °C, NaBH₄ (0.470 g) was added, and the resulting suspension was stirred at 0 °C for 45 minutes. The reaction mixture was diluted with dichloromethane (100 mL) and filtered through diatomaceous earth. The filtrate was washed with water and brine and concentrated to give the crude product, which was used without further purification. MS (ESI) m/e (M+NH₄)⁺:

### 2.99.3 (2S,3R,4S,5S,6S)-2-(2-amino-4-(hydroxymethyl)phenoxy)-6-(methoxycarbonyl)tetrahydro-2H-pyran-3,4,5-triyl triacetate

A stirred solution of Example 2.99.2 (7 g) in ethyl acetate (81 mL) was hydrogenated at 20 °C under 1 atmosphere H₂, using 10% Pd/C (1.535 g) as a catalyst for 12 hours. The reaction mixture was filtered through diatomaceous earth, and the solvent was evaporated under reduced pressure. The residue was purified by silica gel chromatography, eluting with 95/5 dichloromethane/methanol, to give the title compound.

### 2.99.4 3-((((9H-fluoren-9-yl)methoxy)carbonyl)amino)propanoic acid

3-Aminopropanoic acid (4.99 g) was dissolved in 10% aqueous Na₂CO₃ solution (120 mL) in a 500 mL flask and cooled with an ice bath. To the resulting solution, (9H-fluoren-9-yl)methyl carbonochloridate (14.5 g) in 1,4-dioxane (100 mL) was gradually added. The reaction mixture was stirred at room temperature for 4 hours, and water (800 mL) was then added. The aqueous phase layer was separated from the reaction mixture and washed with diethyl ether (3 x 750 mL). The aqueous layer was acidified with 2N HCl aqueous solution to a pH value of 2 and extracted with ethyl acetate (3 x 750 mL). The organic layers were combined and concentrated to obtain crude product. The crude product was recrystallized in a mixed solvent of ethyl acetate: hexane 1:2 (300 mL) to give the title compound.

### 2.99.5 (9H-fluoren-9-yl)methyl (3-chloro-3-oxopropyl)carbamate

To a solution of Example 2.99.4 in dichloromethane (160 mL) was added sulfurous dichloride (50 mL). The mixture was stirred at 60 °C for 1 hour. The mixture was cooled and concentrated to give the title compound.

### 2.99.6 (2S,3R,4S,5S,6S)-2-(2-(3-((((9H-fluoren-9-yl)methoxy)carbonyl)amino)propanamido)-4-(hydroxymethyl)phenoxy)-6-(methoxycarbonyl)tetrahydro-2H-pyran-3,4,5-triyl triacetate

To a solution of Example 2.99.3 (6 g) in dichloromethane (480 mL) was added N,N-diisopropylethylamine (4.60 mL). Example 2.99.5 (5.34 g) was added, and the mixture was stirred at room temperature for 30 minutes. The mixture was poured into saturated aqueous sodium bicarbonate and was extracted with ethyl acetate. The combined extracts were washed with water and brine and were dried over sodium sulfate. Filtration and concentration gave a residue that was purified via radial chromatography, using 0-100% ethyl acetate in petroleum ether as mobile phase, to give the title compound.

### 2.99.7 (2S,3R,4S,5S,6S)-2-(2-(3-((((9H-fluoren-9-yl)methoxy)carbonyl)amino)propanamido)-4-((((4-nitrophenoxy)carbonyl)oxy)methyl)phenoxy)-6-(methoxycarbonyl)tetrahydro-2H-pyran-3,4,5-triyl triacetate

To a mixture of Example 2.99.6 (5.1 g) in N,N-dimethylformamide (200 mL) was added bis(4-nitrophenyl) carbonate (4.14 g) and N,N-diisopropylethylamine (1.784 mL). The mixture was stirred for 16 hours at room temperature and concentrated under reduced pressure. The crude material was dissolved in dichloromethane and aspirated directly onto a 1 mm radial Chromatotron plate and eluted with 50-100% ethyl acetate in hexanes to give the title compound. MS (ESI) m/e (M+H)⁺.

### 2.99.8 3-(1-((3-(2-((((3-(3-aminopropanamido)-4-(((2S,3R,4S,5S,6S)-6-carboxy-3,4,5-trihydroxytetrahydro-2H-pyran-2-yl)oxy)benzyl)oxy)carbonyl)(methyl)amino)ethoxy)-5,7-dimethyladamantan-1-yl)methyl)-5-methyl-1H-pyrazol-4-yl)-6-(8-(benzo[d]thiazol-2-ylcarbamoyl)-3,4-dihydroisoquinolin-2(1H)-yl)picolinic acid

To a solution of Example 1.13.7 (325 mg) and Example 2.99.7 (382 mg) in N,N-dimethylformamide (9 mL) at 0 °C was added N,N-diisopropylamine (49.1 mg). The reaction mixture was stirred at 0 °C for 5 hours, and acetic acid (22.8 mg) was added. The resulting mixture was diluted with ethyl acetate and washed with water and brine. The organic layer was dried over Na₂SO₄, filtered and concentrated. The residue was dissolved in a mixture of tetrahydrofuran (10 mL) and methanol (5 mL). To this solution at 0 °C was added 1 M aqueous lithium hydroxide solution (3.8 mL). The resulting mixture was stirred at 0 °C for 1 hour, acidified with acetic acid and concentrated. The concentrate was lyophilized to provide a powder. The powder was dissolved in N,N-dimethylformamide (10 mL), cooled in an ice-bath, and piperidine (1 mL) at 0 °C was added. The mixture was stirred at 0 °C for 15 minutes and 1.5 mL of acetic acid was added. The solution was purified by reverse-phase HPLC using a Gilson system, eluting with 30-80% acetonitrile in water containing 0.1% v/v trifluoroacetic acid, to provide the title compound. MS (ESI) m/e 1172.2 (M+H)⁺.

### 2.99.9 4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.13,7]dec-1-yl}oxy)ethyl](methyl)carbamoyl}oxy)methyl]-2-({N-[6-(2,5-dioxo-2,5-dihydro-1H-pyrrol-1-yl)hexanoyl]-beta-alanyl}amino)phenyl beta-D-glucopyranosiduronic acid

To Example 2.99.8 (200 mg) in N,N-dimethylformamide (5 mL) at 0 °C was added 2,5-dioxopyrrolidin-1-yl 6-(2,5-dioxo-2,5-dihydro-1H-pyrrol-1-yl)hexanoate (105 mg) and N,N-diisopropylethylamine (0.12 mL). The mixture was stirred at 0 °C for 15 minutes, warmed to room temperature and purified by reverse-phase HPLC on a Gilson system using a 100g C18 column, eluting with 30-80% acetonitrile in water containing 0.1% v/v trifluoroacetic acid, to provide the title compound. ¹H NMR (500 MHz, dimethyl sulfoxide-d₆) δ ppm 12.85 (s, 2H) 9.07 (s, 1H) 8.18 (s, 1H) 8.03 (d, 1H) 7.87 (t, 1H) 7.79 (d, 1H) 7.61 (d, 1H) 7.41-7.53 (m, 3H) 7.36 (q, 2H) 7.28 (s, 1H) 7.03-7.09 (m, 1H) 6.96-7.03 (m, 3H) 6.94 (d, 1H) 4.95 (s, 4H) 4.82 (t, 1H) 3.88 (t, 3H) 3.80 (d, 2H) 3.01 (t, 2H) 2.86 (d, 3H) 2.54 (t, 2H) 2.08 (s, 3H) 2.03 (t, 2H) 1.40-1.53 (m, 4H) 1.34 (d, 2H) 0.90-1.28 (m, 12H) 0.82 (d, 6H). MS (ESI) m/e 1365.3 (M+H)⁺.

### 2.100 Synthesis of Control Synthon 4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](methyl)carbamoyl}oxy)methyl]-2-({N-[19-(2,5-dioxo-2,5-dihydro-1H-pyrrol-1-yl)-17-oxo-4,7,10,13-tetraoxa-16-azanonadecan-1-oyl]-beta-alanyl}amino)phenyl beta-D-glucopyranosiduronic acid (Synthon I)

The title compound was prepared using the procedure in Example 2.99.9, replacing 2,5-dioxopyrrolidin-1-yl 6-(2,5-dioxo-2,5-dihydro-1H-pyrrol-1-yl)hexanoate with 2,5-dioxopyrrolidin-1-yl 1-(2,5-dioxo-2,5-dihydro-1H-pyrrol-1-yl)-3-oxo-7,10,13,16-tetraoxa-4-azanonadecan-19-oate. ¹H NMR (500 MHz, dimethyl sulfoxide-d₆) δ ppm 8.95 (s, 1H) 8.16 (s, 1H) 7.99 (d, 1H) 7.57-7.81 (m, 4H) 7.38-7.50 (m, 3H) 7.34 (q, 2H) 7.27 (s, 1H) 7.10 (d, 1H) 7.00 (d, 1H) 6.88-6.95 (m, 2H) 4.97 (d, 4H) 4.76 (d, 2H) 3.89 (t, 2H) 3.84 (d, 2H) 3.80 (s, 2H) 3.57-3.63 (m, 4H) 3.44-3.50 (m, 4H) 3.32-3.43 (m, 6H) 3.29 (t, 2H) 3.16 (q, 2H) 3.02 (t, 2H) 2.87 (s, 3H) 2.52-2.60 (m, 2H) 2.29-2.39 (m, 3H) 2.09 (s, 3H) 1.37 (s, 2H) 1.20-1.29 (m, 4H) 1.06-1.18 (m, 4H) 0.92-1.05 (m, 2H) 0.83 (s, 6H). MS (ESI) m/e 1568.6 (M-H)⁻.

### 2.101 Synthesis of 6-[8-(1,3-benzothiazol-2-ylcarbamoyl)-3,4-dihydroisoquinolin-2(1H)-yl]-3-{1-[(3-{[(43S,46S)-43-({[(4-{[(2S)-2-{[(2S)-2-{[6-(2,5-dioxo-2,5-dihydro-1H-pyrrol-1-yl)hexanoyl]amino}-3-methylbutanoyl]amino}propanoyl]amino}benzyl)oxy] carbonyl}amino)-46-methyl-37,44,47-trioxo-2,5,8,11,14,17,20,23,26,29,32,35-dodecaoxa-38,45,48-triazapentacontan-50-yl]oxyl-5,7-dimethyltricyclo[3.3.1.1^{3,7}]dec-1-yl)methyl]-5-methyl-1H-pyrazol-4-yl}pyridine-2-carboxylic acid (Synthon OK)

The title compound was prepared as described in Example 2.7, replacing Example 1.13.8 with Example 1.66.7. ¹H NMR (400 MHz, dimethyl sulfoxide-*d*₆) δ ppm 12.85 (s, 1H), 8.21 - 7.97 (m, 4H), 7.79 (d, 4H), 7.71 - 7.32 (m, 15H), 7.28 (t, 4H), 7.02 - 6.91 (m, 3H), 4.95 (d, 5H), 4.33 - 4.12 (m, 3H), 3.98 - 3.76 (m, 11H), 3.41 - 321 (m, 22H), 3.21 - 2.90 (m, 12H), 2.24 - 2.05 (m, 7H), 1.81 - 0.90 (m, 46H), 0.90 - 0.78 (m, 17H). MS (ESI) m/e 2014.0 (M+H)⁺, 1007.5 (M+2H)²⁺, 672.0 (M+3H)³⁺.

### 2.102 Synthesis of N-[6-(2,5-dioxo-2,5-dihydro-1H-pyrrol-1-yl)hexanoyl]-L-valyl-N-{4-[({[2-({3-[(4-{6-[1-(1,3-benzothiazol-2-ylcarbamoyl)-1,2,3,4-tetrahydroquinolin-7-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-carboxyethyl)carbamoyl}oxy)methyl]phenyl}-N⁵-carbamoyl-L-ornithinamide (Synthon OW)

The title compound was prepared as described in Example 2.1, replacing Example 1.2.9 with Example 1.62.5 ¹H NMR (400 MHz, dimethyl sulfoxide-*d*₆) δ ppm 9.95 (s, 1H), 8.36 (s, 1H), 8.02 (d, 1H), 7.96 (d, 1H), 7.88 - 7.68 (m, 4H), 7.57 (d, 2H), 7.42 (s, 2H), 7.34 (t, 1H), 7.25 (dd, 3H), 7.19 (t, 1H), 6.95 (s, 2H), 5.96 (s, 1H), 4.96 (s, 2H), 4.35 (q, 1H), 4.15 (dd, 1H), 3.93 (t, 2H), 3.83 (d, 2H), 3.32 (t, 2H), 3.27 (d, 1H), 2.93 (dtd, 1H), 2.80 (t, 2H), 2.47 (p, 19H), 2.24 - 2.02 (m, 5H), 1.91 (p, 3H), 1.74 - 1.25 (m, 8H), 1.27 - 0.89 (m, 10H), 0.79 (dd, 13H). MS (ESI) m/e 1414.4 (M+H)⁺.

### 2.103 Synthesis of N-[6-(2,5-dioxo-2,5-dihydro-1H-pyrrol-1-yl)hexanoyl]-L-valyl-N-{4-[({[2-({3-[(4-{6-[8-(1,3-benzothiazol-2-ylcarbamoyl)naphthalen-2-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-carboxyethyl)carbamoyl}oxy)methyl]phenyl}-N⁵-carbamoyl-L-ornithinamide (Synthon PC)

The title compound was prepared as described in Example 2.1, replacing Example 1.2.9 with Example 1.68.7. ¹H NMR (400 MHz, dimethyl sulfoxide-*d*₆) δ ppm 13.07 (s, 1H), 9.95 (s, 1H), 8.99 (s, 1H), 8.33 (dd, 1H), 8.25 - 8.09 (m, 3H), 8.12 - 7.95 (m, 3H), 7.90 (d, 1H), 7.76 (dd, 2H), 7.73 - 7.63 (m, 1H), 7.56 (s, 3H), 7.41 - 7.29 (m, 1H), 6.95 (s, 2H), 5.97 (s, 1H), 4.96 (s, 2H), 4.35 (d, 2H), 4.15 (dd, 1H), 3.50 - 3.22 (m, 10H), 2.92 (dtd, 3H), 2.29 - 2.00 (m, 6H), 1.92 (q, 1H), 1.75 - 0.88 (m, 24H), 0.79 (dd, 15H). MS (ESI) m/e 1409.5 (M+H)⁺.

### 2.104 Synthesis of 6-[8-(1,3-benzothiazol-2-ylcarbamoyl)naphthalen-2-yl]-3-{1-[(3- {2- [(2-carboxyethyl) { [(2-{[(2R,3S,4R,5R,6R)-6-carboxy-3,4,5-trihydroxytetrahydro-2H-pyran-2-yl] oxy}-4-[2-(2- {[6-(2,5-dioxo-2,5-dihydro-1H-pyrrol-1-yl)hexanoyl]amino}ethoxy)ethoxy]benzyl)oxy] carbonyl}amino] ethoxy}-5,7-dimethyltricyclo[3.3.1.1^{3,7}]dec-1-yl)methyl]-5-methyl-1H-pyrazol-4-yl}pyridine-2-carboxylic acid (Synthon PI)

### 2.104.1 3-(1-((3-(2-((((4-(2-(2-aminoethoxy)ethoxy)-2-(((2R,3S,4R,5R,6R)-6-carboxy-3,4,5-trihydroxytetrahydro-2H-pyran-2-yl)oxy)benzyl)oxy)carbonyl)(2-carboxyethyl)amino)ethoxy)-5,7-dimethyladamantan-1-yl)methyl)-5-methyl-1H-pyrazol-4-yl)-6-(8-(benzo[d]thiazol-2-ylcarbamoyl)naphthalen-2-yl)picolinic acid

To a cold (0 °C) mixture of Example 2.97.7 (26.9 mg) and Example 1.68.7 (23.5 mg) in N,N-dimethylformamide (2 mL) was added N-ethyl-N-isopropylpropan-2-amine (0.043 mL). The reaction was slowly warmed to room temperature and stirred overnight. LC/MS showed the expected product as the major peak. To the reaction mixture was added water (1 mL) and LiOH H₂O (20 mg). The mixture was stirred at room temperature for 3 hours. The mixture was diluted with N,N-dimethylformamide (2 mL), filtered and purified by reverse-phase HPLC on a Gilson system (C18 column), eluting with 20-80% acetonitrile in water containing 0.1% trifluoroacetic acid, to give the title compound. MS (ESI) m/e 1242.2 (M-H)⁻.

### 2.104.2 6-[8-(1,3-benzothiazol-2-ylcarbamoyl)naphthalen-2-yl]-3-{1-[(3- {2- [(2-carboxyethyl) {[(2- {[(2R,3S,4R,5R,6R)-6-carboxy-3,4,5-trihydroxytetrahydro-2H-pyran-2-yl] oxy}-4-[2-(2- {[6-(2,5-dioxo-2,5-dihydro-1H-pyrrol-1-yl)hexanoyl]amino}ethoxy)ethoxy]benzyl)oxy]carbonyl}amino] ethoxy}-5,7-dimethyltricyclo[3.3.1.13,7]dec-1-yl)methyl]-5-methyl-1H-pyrazol-4-yl}pyridine-2-carboxylic acid

The title compound was prepared as described in Example 2.97.9 by replacing Example 2.97.8 with Example 2.104.1 and replacing 2,5-dioxopyrrolidin-1-yl 3-(2,5-dioxo-2,5-dihydro-1H-pyrrol-1-yl)propanoate with 2,5-dioxopyrrolidin-1-yl 6-(2,5-dioxo-2,5-dihydro-1H-pyrrol-1-yl)hexanoate. ¹H NMR (400 MHz, dimethyl sulfoxide-*d*₆) δ ppm 13.06 (s, 2H), 8.99 (s, 1H), 8.34 (dd, 1H), 8.25 - 8.10 (m, 3H), 8.04 (d, 1H), 7.98 (d, 1H), 7.90 (d, 1H), 7.78 (d, 2H), 7.72 - 7.63 (m, 1H), 7.50 - 7.42 (m, 2H), 7.34 (t, 1H), 7.16 (d, 1H), 6.94 (s, 2H), 6.65 (d, 1H), 6.56 (dd, 1H), 4.02 (t, 2H), 3.90 (d, 1H), 3.83 (s, 2H), 3.66 (t, 3H), 3.28 (q, 4H), 3.15 (q, 2H), 2.19 (s, 3H), 1.99 (t, 2H), 1.51 - 1.30 (m, 6H), 1.28 - 0.88 (m, 11H), 0.81 (d, 6H). MS (ESI) m/e 1433.4 (M+H)⁺.

### 2.105 Synthesis of N-[6-(2,5-dioxo-2,5-dihydro-1H-pyrrol-1-yl)hexanoyl]-L-valyl-N-{4-[({[2-({3-[(4-{6-[5-(1,3-benzothiazol-2-ylcarbamoyl)quinolin-3-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)methyl]phenyl}-N⁵-carbamoyl-L-ornithinamide (Synthon PJ)

The title compound was prepared as described in Example 2.1, replacing Example 1.2.9 with Example 1.69.6. ¹H NMR (500 MHz, dimethyl sulfoxide-*d*₆) δ ppm 13.23 (s, 1H), 9.99 (s, 1H), 9.73 (d, 1H), 9.45 (s, 1H), 8.33 (t, 2H), 8.18 (d, 1H), 8.07 (dd, 2H), 8.02 (dd, 1H), 7.97 (dd, 1H), 7.80 (t, 2H), 7.65 - 7.55 (m, 2H), 7.53 - 7.44 (m, 2H), 7.37 (t, 1H), 7.27 (d, 2H), 6.98 (s, 2H), 4.98 (d, 2H), 4.38 (d, 1H), 4.18 (d, 1H), 3.56 - 3.31 (m, 3H), 3.26 (d, 2H), 3.08 - 2.89 (m, 2H), 2.64 (t, 2H), 2.23 (d, 3H), 2.12 (dp, 2H), 1.95 (s, 1H), 1.68 (s, 1H), 1.62 - 1.29 (m, 7H), 1.29 - 0.90 (m, 9H), 0.90 - 0.74 (m, 12H). MS (ESI) m/e 1446.3 (M-H)⁻.

### 2.106 Synthesis of N-[6-(2,5-dioxo-2,5-dihydro-1H-pyrrol-1-yl)hexanoyl]-L-valyl-N-{4-[({[2-({3-[(4-{6-[4-(1,3-benzothiazol-2-ylcarbamoyl)quinolin-6-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-carboxyethyl)carbamoyl}oxy)methyl]phenyl}-N⁵-carbamoyl-L-ornithinamide (Synthon PU)

The title compound was prepared as described in Example 2.1, replacing Example 1.2.9 with Example 1.70. ¹H NMR (400 MHz, dimethyl sulfoxide-*d*₆) δ ppm 9.97 (s, 1H), 9.12 (d, 1H), 8.93 (s, 1H), 8.60 (dd, 1H), 8.24 (dd, 2H), 8.05 (dd, 2H), 7.99 - 7.87 (m, 2H), 7.78 (dd, 2H), 7.67 - 7.51 (m, 3H), 7.43 - 7.31 (m, 1H), 7.26 (d, 2H), 6.97 (s, 2H), 5.98 (s, 1H), 4.97 (s, 2H), 4.37 (d, 2H), 4.17 (dd, 1H), 3.49 - 3.22 (m, 11H), 2.95 (ddd, 3H), 2.20 (s, 4H), 2.19 - 1.86 (m, 3H), 1.74 - 0.89 (m, 22H), 0.81 (dd, 15H). MS (ESI) m/e 1410.4 (M-H)⁻.

### 2.107 Synthesis of N-[6-(2,5-dioxo-2,5-dihydro-1H-pyrrol-1-yl)hexanoyl]-L-valyl-N-{4-[({[2-({3-[(4-{6-[4-(1,3-benzothiazol-2-ylcarbamoyl)quinolin-6-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)methyl]phenyl}-N⁵-carbamoyl-L-ornithinamide (Synthon PV)

The title compound was prepared as described in Example 2.1, replacing Example 1.2.9 with Example 1.70.5. ¹H NMR (400 MHz, dimethyl sulfoxide-*d*₆) δ ppm 9.96 (s, 1H), 9.11 (d, 1H), 8.92 (s, 1H), 8.60 (dd, 1H), 8.23 (dd, 2H), 8.12 - 7.97 (m, 2H), 7.98 - 7.92 (m, 2H), 7.77 (dd, 2H), 7.56 (t, 2H), 7.51 - 7.42 (m, 2H), 7.42 - 7.31 (m, 1H), 7.24 (d, 2H), 6.95 (s, 2H), 4.95 (d, 2H), 4.36 (q, 1H), 3.90 - 3.80 (m, 3H), 3.52 - 3.27 (m, 3H), 3.23 (t, 2H), 3.06 - 2.83 (m, 2H), 2.67 - 2.58 (m, 2H), 2.19 (s, 3H), 2.09 (dp, 2H), 1.93 (d, 1H), 1.72 - 1.25 (m, 7H), 1.27 - 0.88 (m, 10H), 0.88 - 0.70 (m, 13H). MS (ESI) m/e 1446.3 (M-H)⁻.

### 2.108 Synthesis of N-[6-(2,5-dioxo-2,5-dihydro-1H-pyrrol-1-yl)hexanoyl]-L-valyl-N-{4-[({[2-({3-[(4-{6-[5-(1,3-benzothiazol-2-ylcarbamoyl)quinolin-3-yl]-2-carboxypyridin-3-yl}-5-methyl-1H-pyrazol-1-yl)methyl]-5,7-dimethyltricyclo[3.3.1.1^{3,7}]dec-2-yl}oxy)ethyl](2-carboxyethyl)carbamoyl}oxy)methyl]phenyl}-N⁵-carbamoyl-L-ornithinamide (Synthon PW)

The title compound was prepared as described in Example 2.1, replacing Example 1.2.9 with Example 1.71. ¹H NMR (400 MHz, dimethyl sulfoxide-*d*₆) δ ppm 9.97 (s, 1H), 9.70 (d, 1H), 9.40 (d, 1H), 8.31 (dd, 2H), 8.16 (d, 1H), 8.05 (t, 2H), 8.01 - 7.91 (m, 2H), 7.78 (dd, 2H), 7.59 (d, 3H), 7.52 - 7.44 (m, 2H), 7.36 (t, 1H), 7.26 (d, 2H), 6.96 (s, 2H), 5.99 (s, 1H), 4.97 (s, 2H), 4.37 (d, 2H), 4.16 (dd, 1H), 3.53 - 3.20 (m, 9H), 2.94 (dtd, 2H), 2.21 (s, 3H), 2.17 - 1.85 (m, 3H), 1.71 - 0.89 (m, 22H), 0.81 (dd, 14H). MS (ESI) m/e 1410.4 (M-H)⁻.

### 2.109 Synthesis of N-[6-(2,5-dioxo-2,5-dihydro-1H-pyrrol-1-yl)hexanoyl]-L-valyl-N-{4-[({[2-({3-[(4-{6-[1-(1,3-benzothiazol-2-ylcarbamoyl)-5,6-dihydroimidazo[1,5-a]pyrazin-7(8H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)methyl]phenyl}-N⁵-carbamoyl-L-ornithinamide (Synthon QW)

The title compound was prepared by substituting Example 1.72.8 for Example 1.2.9 in Example 2.1. ¹H NMR (400 MHz, dimethyl sulfoxide d₆) δ ppm 11.07 (bs, 1H), 10.00 (bs, 1H), 8.27 (bs, 1H), 8.12 (m, 2H), 8.07 (d, 1H), 7.99 (d, 1H), 7.84-7.74 (m, 2H), 7.65 (d, 1H), 7.59 (m, 2H), 7.54-7.44 (m, 1H), 7.42-7.31 (m, 2H), 7.28 (m, 2H), 7.21 (q, 1H), 7.00 (m, 1H) 6.94-6.92 (m, 2H), 6.04 (bs, 1H), 5.14 (s, 2H), 4.99 (m, 3H), 4.39 (m, 2H), 4.30 (bs, 2H), 4.20 (m, 2H), 4.12 (bs, 2H), 3.70-3.64 (m, 2H), 3.50 (m, 2H), 3.44-3.35 (m, 2H), 3.27 (m, 2H), 3.02 (m, 2H), 2.95 (m, 2H), 2.68 (t, 2H), 2.14 (m, 4H), 1.96 (m, 1H), 1.69 (m, 1H), 1.58 (m, 1H), 1.47 (m, 4H), 1.36 (m, 2H), 1.30-1.02 (m, 8H), 0.98 (m, 2H), 0.85-0.80 (m, 16 H).

### 2.110 Synthesis of N-[6-(2,5-dioxo-2,5-dihydro-1H-pyrrol-1-yl)hexanoyl]-L-valyl-N-{4-[({[2-({3-[(4-{6-[7-(1,3-benzothiazol-2-ylcarbamoyl)-1H-indol-2-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)methyl]phenyl}-N⁵-carbamoyl-L-ornithinamide (Synthon RM)

Example 2.110 was prepared by substituting Example 1.74.6 for Example 1.2.9 in Example 2.1. ¹H NMR (400 MHz, dimethyl sulfoxide-d₆) δ ppm 11.30 (s, 1H), 9.93 (s, 1H), 8.26 (d, 1H), 8.17 (d, 1H), 8.02 (d, 1H), 7.92 - 7.84 (m, 3H), 7.76 (d, 1H), 7.69 (d, 1H), 7.54 (d, 3H), 7.47 (s, 1H), 7.35 (dd, 2H), 7.22 (t, 3H), 7.08 (t, 1H), 6.93 (s, 2H), 4.90 (s, 2H), 4.84 (t, 2H), 4.33 (q, 1H), 4.16 - 4.09 (m, 1H), 3.32 (t, 4H), 2.99 (m, 6H), 2.21 (s, 3H), 2.09 (m, 2H), 1.91 (m, 1H), 1.71 - 0.71 (m, 25H). MS (ESI) m/e 1434.4 (M-H)⁻.

### 2.111 Synthesis of N-[6-(2,5-dioxo-2,5-dihydro-1H-pyrrol-1-yl)hexanoyl]-L-valyl-N-[4-({[{3-[8-(1,3-benzothiazol-2-ylcarbamoyl)-2-(6-carboxy-5-{1-[(3,5-dimethyl-7-12-[(2-sulfoethyl)amino]ethoxyltricyclo[3.3.1.1^{3,7}]dec-1-yl)methyl]-5-methyl-1H-pyrazol-4-yl}pyridin-2-yl)-1,2,3,4-tetrahydroisoquinolin-6-yl]propyl}(methyl)carbamoyl]oxy}methyl)phenyl]-N⁵-carbamoyl-L-ornithinamide (Synthon RR)

The title compound was prepared as described in Example 2.1, replacing Example 1.2.9 with Example 1.75.14. ¹H NMR (400 MHz, dimethyl sulfoxide-*d*₆) δ ppm 12.60 (bs, 1H), 9.98 (s, 1H), 8.33 (m, 2H), 8.02 (d, 2H), 7.75 (d, 2H), 7.55 (d, 2H), 7.49 (m, 3H), 7.29 (m, 1H), 7.25 (s, 4H), 6.99 (d, 2H), 6.95 (d, 1H), 5.90 (m, 1H), 5.42 (m, 2H), 4.95 (s, 2H), 4.90 (m, 2H), 4.35 (t, 1H), 4.18 (t, 1H), 3.85 (m, 2H), 3.80 (s, 3H), 3.55 (s, 3H), 3.52 (m, 2H), 3.35 (m, 4H), 3.22 (m, 4H), 3.08 (m, 2H), 2.99 (m, 2H), 2.92 (m, 2H), 2.85 (m, 2H), 2.79 (t, 2H), 2.52 (m, 1H), 2.15 (m, 1H), 2.09 (s, 3H), 1.94 (m, 1H), 1.88 (m, 1H), 1.68 (m, 1H), 1.54 (m, 1H), 1.42 (m, 4H), 1.38 (m, 4H), 1.27 (m, 4H), 1.13 (m, 4H), 1.02 (m, 2H), 0.85 (s, 6H), 0.78 (m, 6H). MS (ESI) m/e 1523.3 (M+H)⁺, 1521.6 (M-H)⁻.

### 2.112 Synthesis of N-(6-{[(2,5-dioxo-2,5-dihydro-1H-pyrrol-1-yl)acetyl]amino}hexanoyl)-L-valyl-N-{4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)methyl]phenyl}-N⁵-carbamoyl-L-ornithinamide (Synthon SJ)

### 2.112.1 6-(8-(benzo[d]thiazol-2-ylcarbamoyl)-3,4-dihydroisoquinolin-2(1H)-yl)-3-(1-((3-(2-((((4-((S)-2-((S)-2-((tert-butoxycarbonyl)amino)-3-methylbutanamido)-5-ureidopentanamido)benzyl)oxy)carbonyl)(2-sulfoethyl)amino)ethoxy)-5,7-dimethyladamantan-1-yl)methyl)-5-methyl-1H-pyrazol-4-yl)picolinic acid

Example 1.2.9, trifluoroacetic acid salt (390 mg), tert-butyl ((S)-3-methyl-1-(((S)-1-((4-((((4-nitrophenoxy)carbonyl)oxy)methyl)phenyl)amino)-1-oxo-5-ureidopentan-2-yl)amino)-1-oxobutan-2-yl)carbamate (286 mg) and 1-hydroxybenzotriazole hydrate (185 mg) in N,N-dimethylformamide (5 mL) was cooled in an ice-bath and N,N-diisopropylethylamine (0.35 mL) was added. The mixture was stirred at 0 °C for 30 minutes and at room temperature overnight. The reaction mixture was diluted with dimethyl sulfoxide to 10 mL and purified by reverse-phase HPLC on a Gilson system (C18 column), eluting with 20-80% acetonitrile in water containing 0.1% trifluoroacetic acid, to give the title compound. MS (ESI) m/e 680.1 (M+2H)²⁺.

### 2.112.2 3-(1-((3-(2-((((4-((S)-2-((S)-2-amino-3-methylbutanamido)-5-ureidopentanamido)benzyl)oxy)carbonyl)(2-sulfoethyl)amino)ethoxy)-5,7-dimethyladamantan-1-yl)methyl)-5-methyl-1H-pyrazol-4-yl)-6-(8-(benzo[d]thiazol-2-ylcarbamoyl)-3,4-dihydroisoquinolin-2(1H)-yl)picolinic acid

Example 2.112.1 (300 mg) in 10 mL of dichloromethane at 0 °C was treated with trifluoroacetic acid (4 mL) for 30 minutes and the mixture was concentrated. The residue was dissolved in a mixture of acetonitrile and water and lyophilized to provide the desired product as a TFA salt. MS (ESI) m/e 1257.4 (M-H)⁻.

### 2.112.3 6-(8-(benzo [d]thiazol-2-ylcarbamoyl)-3,4-dihydroisoquinolin-2(1H)-yl)-3-(1-((3-(2-((((4-((13S,16S)-13-isopropyl-2,2-dimethyl-4,11,14-trioxo-16-(3-ureidopropyl)-3-oxa-5,12,15-triazaheptadecanamido)benzyl)oxy)carbonyl)(2-sulfoethyl)amino)ethoxy)-5,7-dimethyladamantan-1-yl)methyl)-5-methyl-1H-pyrazol-4-yl)picolinic acid

Example 2.112.2 (trifluoroacetic acid salt, 385 mg,) and 1-hydroxybenzotriazole hydrate (140 mg) in N,N-dimethylformamide (3 mL) was cooled in an ice-water bath. N,N-Diisopropylethylamine (226 µL) was added dropwise, followed by the addition of 2,5-dioxopyrrolidin-1-yl 6-((tert-butoxycarbonyl)amino)hexanoate (127 mg), and the mixture was stirred overnight. The mixture was purified by reverse-phase HPLC on a Gilson system (C18 column), eluting with 20-75% acetonitrile in water containing 0.1% trifluoroacetic acid, to give the title compound. MS (ESI) m/e 1470.2 (M-H)⁻.

### 2.112.4 3-(1-((3-(2-((((4-((S)-2-((S)-2-(6-aminohexanamido)-3-methylbutanamido)-5-ureidopentanamido)benzyl)oxy)carbonyl)(2-sulfoethyl)amino)ethoxy)-5,7-dimethyladamantan-1-yl)methyl)-5-methyl-1H-pyrazol-4-yl)-6-(8-(benzo[d]thiazol-2-ylcarbamoyl)-3,4-dihydroisoquinolin-2(1H)-yl)picolinic acid

The title compound was prepared using the procedure in Example 2.112.2, replacing Example 2.112.1 with Example 2.112.3. MS (ESI) m/e 1370.5 (M-H)⁻.

### 2.112.5 N-(6-{[(2,5-dioxo-2,5-dihydro-1H-pyrrol-1-yl)acetyl]amino}hexanoyl)-L-valyl-N-{4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.13,7]dec-1-yl}oxy)ethyl](2-sulfoethyl)carbamoyl}oxy)methyl]phenyl}-N5-carbamoyl-L-ornithinamide

Example 2.112.4 (25 mg) and 2,5-dioxopyrrolidin-1-yl 2-(2,5-dioxo-2,5-dihydro-1H-pyrrol-1-yl)acetate (9.19 mg) in N,N-dimethylformamide (0.3 mL) was treated with N,N-diisopropylethylamine (25.4 µL) for 30 minutes at 0 °C. The reaction mixture was purified by reverse-phase HPLC on a Gilson system (C18 column), eluting with 35-65% acetonitrile in 4 mM ammonium acetate water mixture, to provide the title compound as an ammonium salt. ¹H NMR (400 MHz, dimethyl sulfoxide-*d*₆) δ 12.81 (s, 1H), 9.94 (s, 1H), 8.01 (dd, 2H), 7.75 (d, 2H), 7.56 (s, 3H), 7.51 - 7.45 (m, 1H), 7.45 - 7.37 (m, 2H), 7.36 - 7.28 (m, 2H), 7.24 (t, 3H), 7.17 (s, 2H), 7.05 (s, 3H), 7.04 (s, 2H), 6.92 (s, 3H), 5.93 (s, 1H), 5.36 (s, 2H), 5.05 - 4.85 (m, 4H), 4.36 (q, 1H), 4.16 (dd, 1H), 3.95 (s, 2H), 3.85 (t, 2H), 3.76 (d, 2H), 3.22 (d, 1H), 3.05 - 2.81 (m, 6H), 2.68 - 2.53 (m, 2H), 2.09 (d, 4H), 1.76 - 0.86 (m, 14H), 0.86 - 0.71 (m, 12H). MS (ESI) m/e 1507.5 (M-H)⁻.

### 2.113 Synthesis of N-[6-(2,5-dioxo-2,5-dihydro-1H-pyrrol-1-yl)hexanoyl]-L-valyl-N-{4-[({[2-({3-[(4-{6-[8-(1,3-benzothiazol-2-ylcarbamoyl)naphthalen-2-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl][3-(beta-L-glucopyranuronosyloxy)propyl]carbamoyl}oxy)methyl]phenyl}-N⁵-carbamoyl-L-ornithinamide (Synthon SM)

The title compound was prepared as described in Example 2.1, replacing Example 1.2.9 with Example 1.87.3. ¹H NMR (501 MHz, dimethyl sulfoxide-*d*₆) δ ppm 13.08 (s, 1H), 9.96 (s, 1H), 9.00 (s, 1H), 8.35 (dd, 1H), 8.24 - 8.13 (m, 3H), 8.09 - 8.02 (m, 2H), 8.00 (d, 1H), 7.91 (d, 1H), 7.77 (dd, 2H), 7.71 - 7.64 (m, 1H), 7.58 (t, 2H), 7.49 - 7.44 (m, 2H), 7.39 - 7.32 (m, 1H), 7.26 (d, 2H), 6.96 (s, 2H), 5.97 (s, 1H), 4.96 (s, 2H), 4.37 (d, 1H), 4.22 - 4.12 (m, 2H), 3.84 (s, 1H), 3.37 - 3.20 (m, 6H), 3.15 (t, 1H), 3.04 - 2.81 (m, 2H), 2.20 (s, 3H), 2.11 (dp, 2H), 1.99 - 1.88 (m, 1H), 1.71 (q, 2H), 1.62 - 1.26 (m, 8H), 1.29 - 0.88 (m, 11H), 0.80 (dd, 14H). MS (ESI) m/e 1571.4 (M-H)⁻.

### 2.114 Synthesis of N-[6-(2,5-dioxo-2,5-dihydro-1H-pyrrol-1-yl)hexanoyl]-L-valyl-N-{4-[({[2-({3-[(4-{6-[4-(1,3-benzothiazol-2-ylcarbamoyl)isoquinolin-6-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)methyl]phenyl}-N⁵-carbamoyl-L-ornithinamide (Synthon SN)

The title compound was prepared as described in Example 2.1, replacing Example 1.2.9 with Example 1.78.5. ¹H NMR (400 MHz, dimethyl sulfoxide-*d*₆) δ ppm 9.95 (s, 1H), 9.61 (s, 1H), 9.08 (s, 1H), 9.00 (s, 1H), 8.54 (dd, 1H), 8.43 (d, 1H), 8.24 (d, 1H), 8.08 - 7.95 (m, 3H), 7.77 (dd, 2H), 7.63 - 7.51 (m, 2H), 7.50 - 7.42 (m, 2H), 7.40 - 7.31 (m, 1H), 7.24 (d, 2H), 6.95 (s, 2H), 6.00 (s, 1H), 4.95 (d, 2H), 4.36 (q, 1H), 4.15 (t, 1H), 3.27 (dt, 4H), 3.10 - 2.79 (m, 2H), 2.68 - 2.56 (m, 2H), 2.20 (s, 3H), 1.98 - 1.84 (m, 1H), 1.72 - 0.87 (m, 19H), 0.79 (dd, 13H). MS (ESI) m/e 1446.4 (M-H)⁻.

### 2.115 Synthesis of N-[6-(2,5-dioxo-2,5-dihydro-1H-pyrrol-1-yl)hexanoyl]-L-alpha-glutamyl-L-valyl-N-{4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)methyl]phenyl}-N⁵-carbamoyl-L-ornithinamide (Synthon SS)

### 2.115.1 6-(8-(benzo[d]thiazol-2-ylcarbamoyl)-3,4-dihydroisoquinolin-2(1H)-yl)-3-(1-((3-(2-((((4-((6S,9S,12S)-6-(3-(tert-butoxy)-3-oxopropyl)-9-isopropyl-2,2-dimethyl-4,7,10-trioxo-12-(3-ureidopropyl)-3-oxa-5,8,11-triazatridecanamido)benzyl)oxy)carbonyl)(2-sulfoethyl)amino)ethoxy)-5,7-dimethyladamantan-1-yl)methyl)-5-methyl-1H-pyrazol-4-yl)picolinic acid

To a mixture of Example 2.112.2 (85 mg), 1-hydroxybenzotriazole hydrate (41.3 mg), and (S)-5-tert-butyl 1-(2,5-dioxopyrrolidin-1-yl) 2-((tert-butoxycarbonyl)amino)pentanedioate (54.0 mg) in N,N-dimethylformamide (3 mL) at 0 °C was added N,N-diisopropylethylamine (118 µL) dropwise, and the mixture was stirred at 0 °C for 1 hour. The mixture was purified by reverse-phase HPLC on a Gilson system (C18 column), eluting with 35-100% acetonitrile in water containing 0.1% trifluoroacetic acid, to give the title compound. MS (ESI) m/e 773.4 (M+2H)²⁺.

### 2.115.2 3-(1-((3-(2-((((4-((S)-2-((S)-2-((S)-2-amino-4-carboxybutanamido)-3-methylbutanamido)-5-ureidopentanamido)benzyl)oxy)carbonyl)(2-sulfoethyl)amino)ethoxy)-5,7-dimethyladamantan-1-yl)methyl)-5-methyl-1H-pyrazol-4-yl)-6-(8-(benzo[d]thiazol-2-ylcarbamoyl)-3,4-dihydroisoquinolin-2(1H)-yl)picolinic acid

Example 2.115.1 (100 mg) in dichloromethane (11 mL) at 0 °C was treated with trifluoroacetic acid (4 mL). The mixture was stirred at 0 °C for 3.5 hours and concentrated. The residue was purified by reverse phase HPLC, eluting with 5-60% acetonitrile in 0.1% trifluoroacetic acid water mixture to provide the title compound.

### 2.115.3 N-[6-(2,5-dioxo-2,5-dihydro-1H-pyrrol-1-yl)hexanoyl]-L-alpha-glutamyl-L-valyl-N-{4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.13,7]dec-1-yl}oxy)ethyl](2-sulfoethyl)carbamoyl}oxy)methyl]phenyl}-N5-carbamoyl-L-ornithinamide

To a mixture of 1-hydroxybenzotriazole hydrate (2.87 mg), 2,5-dioxopyrrolidin-1-yl 6-(2,5-dioxo-2,5-dihydro-1H-pyrrol-1-yl)hexanoate (5.77 mg) and Example 2.115.2 (13 mg) at 0 °C was added N,N-diisopropylethylamine (13.08 µL), and the mixture was stirred at 0 °C for 1 hour. The reaction was purified by reverse-phase HPLC on a Gilson system (C18 column), eluting with 20-75% acetonitrile in water containing 0.1% trifluoroacetic acid, to give the title compound. ¹H NMR (501 MHz, dimethyl sulfoxide-*d*₆) δ 12.83 (s, 1H), 9.99 (s, 1H), 8.13 (d, 1H), 8.02 (dd, 1H), 7.97 (d, 1H), 7.80 - 7.74 (m, 1H), 7.64 (t, 1H), 7.61 - 7.48 (m, 4H), 7.47 - 7.38 (m, 2H), 7.38 - 7.30 (m, 2H), 7.29 - 7.23 (m, 3H), 6.96 (s, 2H), 6.93 (d, 1H), 5.99 (s, 1H), 5.06 - 4.88 (m, 5H), 4.37 (q, 1H), 4.28 (q, 1H), 4.18 (dd, 1H), 3.86 (t, 2H), 3.78 (d, 2H), 3.34 (t, 3H), 3.23 (d, 2H), 2.99 (t, 3H), 2.97 - 2.85 (m, 1H), 2.62 (dt, 1H), 2.26 - 2.15 (m, 2H), 2.16 - 2.00 (m, 5H), 2.01 - 1.79 (m, 1H), 1.75 - 1.50 (m, 3H), 1.50 - 0.87 (m, 17H), 0.81 (dd, 14H). MS (ESI) m/e 1579.6 (M-H)⁻.

### 2.116 Synthesis of N-[(2,5-dioxo-2,5-dihydro-1H-pyrrol-1-yl)acetyl]-L-alpha-glutamyl-L-valyl-N-{4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)methyl]phenyl}-N⁵-carbamoyl-L-ornithinamide (Synthon TA)

The title compound was prepared using the procedure in Example 2.115.3, replacing 2,5-dioxopyrrolidin-1-yl 6-(2,5-dioxo-2,5-dihydro-1H-pyrrol-1-yl)hexanoate with 2,5-dioxopyrrolidin-1-yl 2-(2,5-dioxo-2,5-dihydro-1H-pyrrol-1-yl)acetate. ¹H NMR (400 MHz, dimethyl sulfoxide-*d*₆) δ 10.02 (s, 1H), 8.38 (d, 1H), 8.14 (d, 1H), 8.03 (d, 1H), 7.82 (dd, 2H), 7.60 (t, 3H), 7.55 - 7.40 (m, 3H), 7.35 (td, 2H), 7.31 - 7.24 (m, 3H), 7.07 (s, 2H), 6.95 (d, 1H), 4.97 (d, 4H), 4.37 (ddd, 2H), 4.23 - 4.05 (m, 3H), 3.88 (t, 6H), 3.80 (d, 2H), 3.25 (d, 2H), 3.09 - 2.88 (m, 4H), 2.64 (s, 2H), 2.22 (dd, 2H), 2.09 (s, 3H), 2.02 - 1.49 (m, 5H), 1.47 - 0.89 (m, 12H), 0.83 (dd, 12H). MS (ESI) m/e 1523.5 (M-H)⁻.

### 2.117 Synthesis of 1-{[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl]({[4-({N-[6-(2,5-dioxo-2,5-dihydro-1H-pyrrol-1-yl)hexanoyl]-D-valyl-N⁵carbamoyl-D-ornithyl}amino)benzyl]oxy}carbonyl)amino}-1,2-dideoxy-D-arabino-hexitol (Synthon TW)

The title compound was prepared by substituting Example 1.77.2 for Example 1.2.9 in Example 2.1. ¹HNMR(400 MHz, dimethyl sulfoxide-d₆) δ ppm 12.85 (bs, 1H), 9.98 (s, 1H), 8.06 (d, 1H), 8.03 (d, 1H), 7.78 (t, 2H), 7.60 (m, 3H), 7.52-7.42 (m, 4H), 7.36 (q, 2H), 7.28 (s, 1H), 7.27 (d, 2H), 6.99 (s, 1H), 6.95 (d, 1H), 5.97 (bs, 1H), 5.00 (m, 2H), 4.95 (s, 2H), 4.39 (m, 1H), 4.19 (m, 2H), 3.88 (t, 2H), 3.79 (m, 4H), 3.58 (m, 4H), 3.46-3.33 (m, 10H), 3.26 (m, 4H), 3.01 (m, 2H), 2.94 (m, 1H), 2.14 (m, 2H), 2.09 (s, 3H), 1.96 (m, 1H), 1.69 (m, 2H), 1.59 (m, 1H), 1.47 (m, 4H), 1.35 (m, 4H), 1.28-1.03 (m, 10H), 0.95 (m, 2H), 0.82 (m, 12H). MS (ESI) m/e 1493 (M+H)⁺, 1491 (M-H)⁻.

### 2.118 Synthesis of N-[6-(2,5-dioxo-2,5-dihydro-1H-pyrrol-1-yl)hexanoyl]-L-valyl-N-{4- [({[2-({3-[(4-{6-[4-(1,3-benzothiazol-2-ylcarbamoyl)-2-oxidoisoquinolin-6-yl]-2-carboxypyridin-3-yl }-5-methyl-1H-pyrazol-1-yl)methyl]-5,7-dimethyltricyclo[3.3.1.1^{3,7}]dec-1-yl}oxy)ethyl](methyl)carbamoyl}oxy)methyl]phenyl}-N⁵⁻carbamoyl-L-ornithinamide (Synthon ST)

The title compound was prepared as described in Example 2.1, replacing Example 1.2.9 with Example 1.88.4. ¹H NMR (400 MHz, dimethyl sulfoxide-d₆) δ ppm 13.29 (s, 2H), 9.95 (s, 1H), 9.18 (s, 1H), 8.67 (s, 1H), 8.57 - 8.36 (m, 1H), 8.29 - 7.87 (m, 4H), 7.77 (dd, 2H), 7.56 (d, 2H), 7.53 - 7.41 (m, 2H), 7.24 (d, 2H), 6.95 (s, 2H), 5.95 (s, 1H), 4.94 (s, 2H), 4.35 (q, 1H), 4.15 (dd, 1H), 3.84 (s, 3H), 3.28 (dt, 4H), 3.06 - 2.77 (m, 3H), 2.19 (d, 3H), 2.17 - 1.80 (m, 3H), 1.74 - 0.88 (m, 22H), 0.79 (dd, 13H). MS (ESI) m/e 1368.4 (M-H)⁻.

### 2.119 Synthesis of N-({(3S,5S)-3-(2,5-dioxo-2,5-dihydro-1H-pyrrol-1-yl)-2-oxo-5-[(2-sulfoethoxy)methyl]pyrrolidin-1-yll}acetyl)-L-valyl-N-{4-[({[2-({3-[(4-{6-[8-(1,3-benzothiazol-2-ylcarbamoyl)naphthalen-2-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)methyl]phenyl}-N⁵-carbamoyl-L-ornithinamide (Synthon ZL)

### 2.119.1 (3R,7aS)-3-phenyltetrahydropyrrolo[1,2-c]oxazol-5(3H)-one

A mixture of (S)-5-(hydroxymethyl)pyrrolidin-2-one (25g), benzaldehyde (25.5g) and para-toluenesulfonic acid monohydrate (0.50 g) in toluene (300 mL) was heated to reflux using a Dean-Stark trap under a drying tube for 16 hours. The reaction was cooled to room temperature, and the solvent was decanted from the insoluble materials. The organic layer was washed with saturated aqueous sodium bicarbonate mixture (2x) and brine (1x). The organic layer was dried over sodium sulfate, filtered and concentrated under reduced pressure. The residue was purified by flash chromatography on silica gel, eluting with 35/65 heptane/ethyl acetate, to give the title compound. MS (DCI) m/e 204.0 (M+H)⁺.

### 2.119.2 (3R,6R,7aS)-6-bromo-3-phenyltetrahydropyrrolo[1,2-c]oxazol-5(3H)-one

To a cold (-77 °C) mixture of Example 2.119.1 (44.6 g) in tetrahydrofuran (670 mL) was added lithium bis(trimethylsilyl)amide (1.0M in hexanes, 250 mL) dropwise over 40 minutes, keeping Tᵣₓₙ < -73 °C. The reaction was stirred at -77 °C for 2 hours, and bromine (12.5 mL) was added dropwise over 20 minutes, keeping Tᵣₓₙ < -64 °C. The reaction was stirred at -77 °C for 75 minutes and was quenched by the addition of 150 mL cold 10% aqueous sodium thiosulfate mixture to the -77 °C reaction. The reaction was warmed to room temperature and partitioned between half-saturated aqueous ammonium chloride mixture and ethyl acetate. The layers were separated, and the organic layer was washed with water and brine, dried over sodium sulfate, filtered and concentrated under reduced pressure. The residue was purified by silica gel chromatography, eluting with a gradient of 80/20, 75/25, and 70/30 heptane/ethyl acetate to give the title compound. MS (DCI) m/e 299.0 and 301.0 (M+NH₃+H)⁺.

### 2.119.3 (3R,6S,7aS)-6-bromo-3-phenyltetrahydropyrrolo[1,2-c]oxazol-5(3H)-one

The title compound was isolated as a by-product from Example 2.119.2. MS (DCI) m/e 299.0 and 301.0 (M+NH₃+H)⁺.

### 2.119.4 (3R,6S,7aS)-6-azido-3-phenyltetrahydropyrrolo[1,2-c]oxazol-5(3H)-one

To a mixture of Example 2.119.2 (19.3 g) in N,N-dimethylformamide (100 mL) was added sodium azide (13.5 g). The reaction was heated to 60 °C for 2.5 hours. The reaction was cooled to room temperature and quenched by the addition of water (500 mL) and ethyl acetate (200 mL). The layers were separated, and the organic layer was washed brine. The combined aqueous layers were back-extracted with ethyl acetate (50 mL). The combined organic layers were dried with sodium sulfate, filtered and concentrated under reduced pressure. The residue was purified by silica gel chromatography, eluting with 78/22 heptane/ethyl acetate, to give the title compound. MS (DCI) m/e 262.0 (M+NH₃+H)⁺.

### 2.119.5 (3R,6S,7aS)-6-amino-3-phenyltetrahydropyrrolo[1,2-c]oxazol-5(3H)-one

To a mixture of Example 2.119.4 (13.5 g) in tetrahydrofuran (500 mL) and water (50 mL) was added polymer-supported triphenylphosphine (55 g). The reaction was mechanically stirred overnight at room temperature. The reaction was filtered through diatomaceous earth, eluting with ethyl acetate and toluene. The mixture was concentrated under reduced pressure, dissolved in dichloromethane (100 mL), dried with sodium sulfate, then filtered and concentrated to give the title compound, which was used in the subsequent step without further purification. MS (DCI) m/e 219.0 (M+H)⁺.

### 2.119.6 (3R,6S,7aS)-6-(dibenzylamino)-3-phenyltetrahydropyrrolo[1,2-c] oxazol-5(3H)-one

To a mixture of Example 2.119.5 (11.3 g) in N,N-dimethylformamide (100 mL) was added potassium carbonate (7.0 g), potassium iodide (4.2 g), and benzyl bromide (14.5 mL). The reaction was stirred at room temperature overnight and quenched by the addition of water and ethyl acetate. The layers were separated, and the organic layer was washed brine. The combined aqueous layers were back-extracted with ethyl acetate. The combined organic layers were dried with sodium sulfate, filtered and concentrated under reduced pressure. The residue was purified by silica gel chromatography, eluting with a gradient of 10 to 15% ethyl acetate in heptane to give a solid that was triturated with heptane to give the title compound. MS (DCI) m/e 399.1 (M+H)⁺.

### 2.119.7 (3S,5S)-3-(dibenzylamino)-5-(hydroxymethyl)pyrrolidin-2-one

To a mixture of Example 2.119.6 (13 g) in tetrahydrofuran (130 mL) was added *para*toluene sulfonic acid monohydrate (12.4 g) and water (50 mL), and the reaction was heated to 65 °C for 6 days. The reaction was cooled to room temperature and quenched by the addition of saturated aqueous sodium bicarbonate and ethyl acetate. The layers were separated, and the organic layer was washed with brine. The combined aqueous layers were back-extracted with ethyl acetate. The combined organic layers were dried with sodium sulfate, filtered and concentrated under reduced pressure. The waxy solids were triturated with heptane (150 mL) to give the title compound. MS (DCI) m/e 311.1 (M+H)⁺.

### 2.119.8 (3S,5S)-5-(((tert-butyldimethylsilyl)oxy)methyl)-3-(dibenzylamino)pyrrolidin-2-one

To a mixture of Example 2.119.7 (9.3 g) and 1H-imidazole (2.2 g) in N,N-dimethylformamide was added tert-butylchlorodimethylsilane (11.2 mL, 50 weight % in toluene), and the reaction mixture was stirred overnight. The reaction mixture was quenched by the addition of water and ethyl ether. The layers were separated, and the organic layer was washed with brine. The combined aqueous layers were back-extracted with diethyl ether. The combined organic layers were dried with sodium sulfate, filtered and concentrated under reduced pressure. The residue was purified by silica gel chromatography, eluting with 35% ethyl acetate in heptane, to give the title compound. MS (DCI) m/e 425.1 (M+H)⁺.

### 2.119.9 tert-butyl 2-((3S,5S)-5-(((tert-butyldimethylsilyl)oxy)methyl)-3-(dibenzylamino)-2-oxopyrrolidin-1-yl)acetate

To a cold (0 °C) mixture of Example 2.119.8 (4.5 g) in tetrahydrofuran (45 mL) was added 95% sodium hydride (320 mg) in two portions. The cold mixture was stirred for 40 minutes, and tert-butyl 2-bromoacetate (3.2 mL) was added. The reaction was warmed to room temperature and stirred overnight. The reaction was quenched by the addition of water and ethyl acetate. The layers were separated, and the organic layer was washed with brine. The combined aqueous layers were back-extracted with ethyl acetate. The combined organic layers were dried with sodium sulfate, filtered and concentrated under reduced pressure. The residue was purified by silica gel chromatography, eluting with a gradient of 5-12% ethyl acetate in heptane, to give the title compound. MS (DCI) m/e 539.2 (M+H)⁺.

### 2.119.10 tert-butyl 2-((3S,5S)-3-(dibenzylamino)-5-(hydroxymethyl)-2-oxopyrrolidin-1-yl)acetate

To a mixture of Example 2.119.9 (5.3 g) in tetrahydrofuran (25 mL) was added tetrabutylammonium fluoride (11 mL, 1.0M in 95/5 tetrahydrofuran /water). The reaction was stirred at room temperature for one hour and then quenched by the addition of saturated aqueous ammonium chloride mixture, water and ethyl acetate. The layers were separated, and the organic layer was washed with brine. The combined aqueous layers were back-extracted with ethyl acetate. The combined organic layers were dried with sodium sulfate, filtered and concentrated under reduced pressure. The residue was purified by silica gel chromatography, eluting with 35% ethyl acetate in heptane, to give the title compound. MS (DCI) m/e 425.1 (M+H)⁺.

### 2.119.11 tert-butyl 2-((3S,5S)-5-((2-((4-((tert-butyldimethylsilyl)oxy)-2,2-dimethylbutoxy)sulfonyl)ethoxy)methyl)-3-(dibenzylamino)-2-oxopyrrolidin-1-yl)acetate

To a mixture of Example 2.119.10 (4.7 g) in dimethyl sulfoxide (14 mL) was added a mixture of 4-((tert-butyldiphenylsilyl)oxy)-2,2-dimethylbutyl ethenesulfonate (14.5 g) in dimethyl sulfoxide (14 mL). Potassium carbonate (2.6 g) and water (28 µL) were added, and the reaction was heated at 60 °C under nitrogen for one day. The reaction was cooled to room temperature, and then quenched by the addition of brine mixture, water and diethyl ether. The layers were separated, and the organic layer was washed with brine. The combined aqueous layers were back-extracted with diethyl ether. The combined organic layers were dried with sodium sulfate, filtered and concentrated under reduced pressure. The residue was purified by silica gel chromatography, eluting with a gradient of 15-25% ethyl acetate in heptane, to give the title compound. MS (ESI+) m/e 871.2 (M+H)⁺.

### 2.119.12 tert-butyl 2-((3S,5S)-3-amino-5-((2-((4-((tertbutyldimethylsilyl)oxy)-2,2-dimethylbutoxy)sulfonyl)ethoxy)methyl)-2-oxopyrrolidin-1-yl)acetate

Example 2.119.11 (873 mg) was dissolved in ethyl acetate (5 mL) and methanol (15 mL), and palladium hydroxide on carbon, 20% by wt (180 mg) was added. The reaction mixture was stirred under a hydrogen atmosphere (30 psi) at room temperature for 30 hours, then at 50 °C for one hour. The reaction was cooled to room temperature, filtered, and concentrated to give the desired product. MS (ESI+) m/e 691.0 (M+H)⁺.

### 2.119.13 4-(((3S,5S)-1-(2-(tert-butoxy)-2-oxoethyl)-5-((2-((4-((tert-butyldimethylsilyl)oxy)-2,2-dimethylbutoxy)sulfonyl)ethoxy)methyl)-2-oxopyrrolidin-3-yl)amino)-4-oxobut-2-enoic acid

Maleic anhydride (100 mg) was dissolved in dichloromethane (0.90 mL), and a mixture of Example 2.119.12 (650 mg) in dichloromethane (0.90 mL) was added dropwise, and then heated at 40 °C for 2 hours. The reaction mixture was directly purified by silica gel chromatography, eluting with a gradient of 1.0-2.5% methanol in dichloromethane containing 0.2% acetic acid. After concentrating the product-bearing fractions, toluene (10 mL) was added, and the mixture was concentrated again to give the title compound. MS (ESI-) m/e 787.3 (M-H)⁻.

### 2.119.14 tert-butyl 2-((3S,5S)-5-((2-((4-((tert-butyldimethylsilyl)oxy)-2,2-dimethylbutoxy)sulfonyl)ethoxy)methyl)-3-(2,5-dioxo-2,5-dihydro-1H-pyrrol-1-yl)-2-oxopyrrolidin-1-yl)acetate

Example 2.119.13 (560 mg) was slurried in toluene (7 mL), and triethylamine (220 µL) and sodium sulfate (525 mg) were added. The reaction was heated at reflux under a nitrogen atmosphere for 6 hours, and the reaction mixture was stirred at room temperature overnight. The reaction was filtered, and the solids were rinsed with ethyl acetate. The eluent was concentrated under reduced pressure, and the residue was purified by silica gel chromatography, eluting with 45/55 heptane/ethyl acetate to give the title compound.

### 2.119.15 2-((3S,5S)-3-(2,5-dioxo-2,5-dihydro-1H-pyrrol-1-yl)-2-oxo-5-((2-sulfoethoxy)methyl)pyrrolidin-1-yl)acetic acid

Example 2.119.14 (1.2 g) was dissolved in trifluoroacetic acid (15 mL) and heated to 65-70 °C under nitrogen overnight. The trifluoroacetic acid was removed under reduced pressure. The residue was dissolved in acetonitrile (2.5 mL) and purified by preparative reverse-phase liquid chromatography on a Luna C18(2) AXIA column (250 x 50 mm, 10µ particle size) using a gradient of 5-75% acetonitrile containing 0.1% trifluoroacetic acid in water over 30 minutes, to give the title compound. MS (ESI-) m/e 375.2 (M-H)⁻.

### 2.119.16 3-(1-((3-(2-((((4-((S)-2-((S)-2-amino-3-methylbutanamido)-5-ureidopentanamido)benzyl)oxy)carbonyl)(2-sulfoethyl)amino)ethoxy)-5,7-dimethyladamantan-1-yl)methyl)-5-methyl-1H-pyrazol-4-yl)-6-(8-(benzo[d]thiazol-2-ylcarbamoyl)naphthalen-2-yl)picolinic acid

The title compound was prepared by substituting Example 1.43.7 for Example 1.2.9 in Example 2.49.1. MS (ESI-) m/e 1252.4 (M-H)⁻.

### 2.119.17 N-({(3S,5S)-3-(2,5-dioxo-2,5-dihydro-1H-pyrrol-1-yl)-2-oxo-5-[(2-sulfoethoxy)methyl]pyrrolidin-1-yl}acetyl)-L-valyl-N-{4-[({[2-({3-[(4-{6-[8-(1,3-benzothiazol-2-ylcarbamoyl)naphthalen-2-yl]-2-carboxypyridin-3-yl}-5-methyl-1H-pyrazol-1-yl)methyl]-5,7-dimethyltricyclo [3.3.1.13,7] dec-1-yl}oxy)ethyl](2-sulfoethyl)carbamoyl}oxy)methyl]phenyl}-N5-carbamoyl-L-ornithinamide

Example 2.119.15 (7 mg) was dissolved in N,N-dimethylformamide (0.15 mL), and 0-(7-azabenzotriazol-l-yl)-N,N,N',N'-tetramethyluronium hexafluorophosphate (9 mg) and N,N-diisopropylethylamine (7 µL) were added. The mixture was stirred for 3 minutes at room temperature and added to a mixture of Example 2.119.16 (28 mg) and N,N-diisopropylethylamine (15 µL) in N,N-dimethylformamide (0.15 mL). After 1 hour, the reaction was diluted with N,N-dimethylformamide/water 1/1 (1.0 mL) and purified by reverse-phase chromatography (C18 column), eluting with 5-75% acetonitrile in 0.1% TFA water, to provide the title compound. ¹H NMR (500 MHz, dimethyl sulfoxide-d₆) δ ppm 9.95 (s, 1H), 9.02 (s, 1H), 8.37 (d, 1H), 8.22 (m, 2H), 8.18 (m, 2H), 8.08 (m, 2H), 8.03 (m, 1H), 7.96 (br d, 1H), 7.81 (d, 1H), 7.70 (t, 1H), 7.61 (br m, 3H), 7.48 (m, 2H), 7.37 (t, 1H), 7.27 (br m, 2H), 7.08 (s, 2H), 4.99 (br d, 3H), 4.68 (t, 1H), 4.39 (m, 1H), 4.20 (m, 2H), 4.04 (m, 1H), 3.87 (br d, 2H), 3.74 (br m, 1H) 3.65 (br t, 2H), 3.48 (br m, 4H), 3.43 (br m, 2H), 3.26 (br m, 2H), 3.00 (br m, 2H), 2.80 (m, 1H), 2.76 (m, 1H), 2.66 (br m, 2H), 2.36 (br m, 1H), 2.22 (s, 3H), 2.00 (m, 1H), 1.87 (m, 1H), 1.69 (br m, 1H), 1.62 (br m, 1H), 1.40 (br m, 4H), 1.31-1.02 (m, 10 H), 0.96 (m, 2H), 0.85 (m, 12H). MS (ESI-) m/e 1610.3 (M-H)⁻.

### 2.120 Synthesis of N-{(2S)-2-(2,5-dioxo-2,5-dihydro-1H-pyrrol-1-yl)-3-[4-(2,5,8,11,14,17,20,23,26,29,32-undecaoxatetratriacontan -34-yloxy)phenyl]propanoyl}-L-valyl-N-{4-[({[2-({3-[(4-{6-[8-(1,3-benzothiazol-2-ylcarbamoyl)naphthalen-2-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)methyl]phenyl}-N⁵-carbamoyl-L-ornithinamide (Synthon SX)

### 2.120.1 (S)-methyl 3-(4-(2,5,8,11,14,17,20,23,26,29,32-undecaoxatetratriacontan-34-yloxy)phenyl)-2-((tert-butoxycarbonyl)amino)propanoate

To a mixture of 2,5,8,11,14,17,20,23,26,29,32-undecaoxatetratriacontan-34-yl 4-methylbenzenesulfonate (82.48 g) and potassium carbonate (84.97 g) in acetonitrile (1.5 L) was added (S)-methyl 2-((tert-butoxycarbonyl)amino)-3-(4-hydroxyphenyl)propanoate (72.63 g), and the reaction mixture was stirred at 30 °C for 12 hours. After LC/MS indicated the starting material was consumed and the major product was the desired product, the reaction was filtered, and the filtrate was concentrated to afford the crude product which was purified by prep-HPLC to provide the title compound. MS (ESI): m/e 811 (M+H₂O)⁺.

### 2.120.2 3-(4-(2,5,8,11,14,17,20,23,26,29,32-undecaoxatetratriacontan-34-yloxy)phenyl)-2-((tert-butoxycarbonyl)amino)propanoic acid

To a mixture of Example 2.120.1 (90.00 g) in tetrahydrofuran (1.5 L) and water (500 mL) was added lithium hydroxide monohydrate (14.27 g). The reaction mixture was stirred at 30 °C for 12 hours, and LC/MS indicated the starting material was consumed and the major product was the desired product. The reaction mixture was adjusted using aqueous HCl to pH=6, and the mixture was concentrated to provide the crude title compound. MS (ESI): m/e 778.3 (M-H)⁻..

### 2.120.3 3-(4-(2,5,8,11,14,17,20,23,26,29,32-undecaoxatetratriacontan-34-yloxy)phenyl)-2-aminopropanoic acid

To a mixture of Example 2.120.2 (88.41 g) in dichloromethane (1.5 L) was added trifluoroacetic acid (100 mL) at 25°C under N₂, and the reaction mixture was stirred at 40 °C for 12 hours. LC/MS indicated the starting material was consumed, and the major product was the desired product. The mixture was concentrated to afford the crude product which was purified by prep-HPLC provide the title compound as a trifluoroacetic acid salt. ¹H NMR (400 MHz, CDCl₃) δ ppm 7.20 (d, J=8.6 Hz, 2H), 6.93 (d, J=8.2 Hz, 2H), 4.22 (dd, J=5.5, 7.4 Hz, 1H), 4.14-4.06 (m, 2H), 3.84 - 3.79 (m, 2H), 3.68-3.50 (m, 40H), 3.33 (s,3H), 3.21 (d, J=5.5 Hz, 1H), 3.12-3.05 (m,1H). MS (ESI) m/e 680.1 (M+H)+.

### 2.120.4 4-((2-(4-(2,5,8,11,14,17,20,23,26,29,32-undecaoxatetratriacontan-34-yloxy)phenyl)-1-carboxyethyl)amino)-4-oxobut-2-enoic acid

To a mixture of Example 2.120.3 (80.00 g) in dioxane (1 L) was added furan-2, 5-dione (35 g), and the reaction mixture was stirred at 120 °C for 4 hours. LC/MS indicated the starting material was consumed, and the major product was the desired product. The mixture was concentrated to afford crude title compound which was used without purification in the next step. MS (ESI) m/e 795.4 (**M+H)⁺**.

### 2.120.5 (S)-3-(4-(2,5,8,11,14,17,20,23,26,29,32-undecaoxatetratriacontan-34-yloxy)phenyl)-2-(2,5-dioxo-2,5-dihydro-1H-pyrrol-1-yl)propanoic acid

To a mixture of Example 2.120.4 (96 g, crude) in toluene (1.5 L) and was added triethylamine (35.13 g), and the reaction mixture was stirred at 120 °C for 4 hours. LC/MS indicated the starting material was consumed, and the major product was the desired product. The reaction was filtered to isolate the organic phase, and the organics were concentrated to afford the crude product which was purified by prep-HPLC (Instrument: Shimadzu LC-20AP preparative HPLC, Column: Phenomenex^{®} Luna^{®} (2) C18 250^{∗}50 mm i.d. 10µm, Mobile phase: A for H₂O (0.09% trifluoroacetic acid) and B for CH₃CN, Gradient: B from 15% to 43 % in 20 minutes, Flow rate: 80 ml/minute, Wavelength: 220 & 254 nm, Injection amount: 1 gram per injection), followed by SFC-HPLC to provide the title compound. ¹H NMR (400 MHz, CDC1₃) δ ppm 6.98 (d, 2H), 6.74 (d, 2H), 6.56 (s, 2H), 4.85 (dd, 1H), 4.03 (t, 2H), 3.84 - 3.76 (m, 2H), 3.71 - 3.66 (m, 2H), 3.65 - 3.58 (m, 39H), 3.55 - 3.50 (m, 2H), 3.41 - 3.30 (m, 4H). MS (ESI) m/e 760.3 (**M+H)⁺**.

### 2.120.6 N-{(2S)-2-(2,5-dioxo-2,5-dihydro-1H-pyrrol-1-yl)-3-[4-(2,5,8,11,14,17,20,23,26,29,32-undecaoxatetratriacontan-34-yloxy)phenyl]propanoyl}-L-valyl-N-{4-[({[2-({3-[(4-{6-[8-(1,3-benzothiazol-2-ylcarbamoyl)naphthalen-2-yl]-2-carboxypyridin-3-yl}-5-methyl-1H-pyrazol-1-yl)methyl]-5,7-dimethyltricyclo[3.3.1.13,7]dec-1-yt}oxy)ethyl](2-sulfoethyl)carbamoyl}oxy)methyl]phenyl}-N5-carbamoyl-L-ornithinamide

The title compound was prepared by substituting Example 2.120.5 for Example 2.119.15 in Example 2.119.17. ¹H NMR (400 MHz, dimethyl sulfoxide-d₆) δ ppm 10.03 (s, 1H), 9.02 (s, 1H), 8.37 (d, 1H), 8.22 (m, 3H), 8.16 (d, 1H), 8.12 (br m, 1H), 8.07 (d, 1H), 8.01 (d, 1H), 7.96 (br d, 1H), 7.81 (d, 1H), 7.70 (t, 1H), 7.59 (br m, 2H), 7.48 (m, 2H), 7.37 (t, 1H), 7.28 (d, 2H), 7.02 (d, 2H), 6.89 (s, 2H), 6.77 (d, 2H), 4.98 (br d, 2H), 4.79 (dd, 1H), 4.39 (br m, 1H), 4.23 (br m, 2H), 3.99 (br m, 2H), 3.88 (br m, 2H), 3.69 (br m, 4H), 3.55 (m, 4H), 3.50 (s, 32H), 3.42 (m, 4H), 3.27 (m, 4H), 3.23 (s, 3H), 3.20 (m, 1H), 3.03 (br m, 1H), 2.98 (m, 1H), 2.65 (br t, 2H), 2.22 (s, 3H), 1.97 (br m, 1H), 1.69 (br m, 1H), 1.61 (br m, 1H), 1.39 (m, 4H), 1.31-0.91 (m, 12H), 0.85 (m, 9H), 0.77 (d, 3H). MS (ESI) m/e 1993.7 (M-H)⁻.

### 2.121 Synthesis of N-({(3S,5S)-3-(2,5-dioxo-2,5-dihydro-1H-pyrrol-1-yl)-2-oxo-5-[(2-sulfoethoxy)methyl]pyrrolidin-1-yl}acetyl)-L-valyl-N-{4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1³'⁷]dec-1-yl}oxy)ethyl](2-sulfoethyl)carbamoyl}oxy)methyl]phenyl}-N⁵-carbamoyl-L-ornithinamide (Synthon SW)

The title compound was prepared by substituting Example 2.49.1 for Example 2.119.16 in Example 2.119.17. ¹HNMR (400 MHz, dimethyl sulfoxide-d₆) δ ppm 9.96 (s, 1H), 8.17 (br d, 1H), 8.03 (d, 2H), 7.79 (d, 1H), 7.61 (m, 3H), 7.55 (d, 1H), 7.45 (m, 2H), 7.37 (m, 3H), 7.27 (d, 2H), 7.08 (s, 2H), 6.98 (d, 1H), 4.97 (m, 4H), 4.68 (t, 1H), 4.37 (br m, 1H), 4.22 (br s, 1H), 4.17 (d, 1H), 4.03 (d, 1H), 3.89 (br t, 2H), 3.83 (br d, 2H), 3.74 (br m, 1H), 3.65 (t, 2H), 3.49 (m, 3H), 3.40 (br m, 4H), 3.25 (br m, 2H), 3.02 (br m, 4H), 2.80 (m, 2H), 2.67 (br m, 2H), 2.37 (br m, 1H), 2.10 (s, 3H), 1.99 (m, 1H), 1.86 (m, 1H), 1.69 (br m, 1H), 1.61 (br m, 1H), 1.52-0.91 (m, 16H), 0.85 (m, 12H). MS (ESI) m/e 1615.4 (M-H)⁻.

### 2.122 Synthesis of N-{(2S)-2-(2,5-dioxo-2,5-dihydro-1H-pyrrol-1-yl)-3-[4-(2,5,8,11,14,17,20,23,26,29,32-undecaoxatetratriacontan-34-yloxy)phenyl]propanoyl}-L-valyl-N-{4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)methyl]phenyl}-N⁵-carbamoyl-L-ornithinamide (Synthon TV)

To a mixture of Example 2.120.5 (19.61 mg), and O-(7-azabenzotriazol-1-yl)-N,N,N',N'-tetramethyluronium hexafluorophosphate (9.81 mg) in N,N-dimethylformamide (0.8 mL) was added N,N-diisopropylethylamine (27.7 µL). The mixture was stirred for 5 minutes and added to a cold mixture of Example 2.112.2 in N,N-dimethylformamide (0.5 mL) at 0 °C. The reaction mixture was stirred at 0 °C for 40 minutes, and purified by reverse-phase HPLC on a Gilson system (C18 column), eluting with 20-80% acetonitrile in water containing 0.1% trifluoroacetic acid, to give the title compound. ¹H NMR (400 MHz, dimethyl sulfoxide-d₆) δ 9.99 (s, 1H), 8.19 (d, 1H), 8.14 - 8.04 (m, 1H), 8.00 (dd, 1H), 7.75 (d, 1H), 7.62 - 7.52 (m, 3H), 7.49 (d, 1H), 7.46 - 7.37 (m, 2H), 7.36 - 7.29 (m, 2H), 7.28 - 7.21 (m, 3H), 6.99 (d, 2H), 6.92 (d, 1H), 6.85 (s, 2H), 6.79 - 6.71 (m, 2H), 4.94 (d, 3H), 4.76 (dd, 1H), 4.35 (d, 1H), 4.20 (t, 1H), 3.96 (dd, 2H), 3.85 (t, 2H), 3.77 (d, 2H), 3.66 (dd, 2H), 3.52 (dd, 2H), 3.50 - 3.47 (m, 2H), 3.39 (dd, 2H), 3.20 (s, 4H), 2.97 (t, 3H), 2.60 (t, 2H), 2.13 - 2.01 (m, 3H), 1.93 (s, 1H), 1.61 (d, 2H), 1.49 - 0.88 (m, 10H), 0.87 - 0.59 (m, 12H). MS (ESI) m/e 1998.7 (M-H)⁻.

### 2.123 Synthesis of (6S)-2,6-anhydro-6-(2-{2-[({[2-({3-[(4-{6-[8-(1,3-benzothiazol-2-ylcarbarmoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)methyl]-5-({N-[(2,5-dioxo-2,5-dihydro-1H-pyrrol-1-yl)acetyl]-L-valyl-L-alanyl}amino)phenyl}ethyl)-L-gulonic acid (Synthon SZ)

### 2.123.1 (3R,4S,5R,6R)-3,4,5-tris(benzyloxy)-6-(benzyloxymethyl)-tetrahydropyran-2-one

To a mixture of (3R,4S,5R,6R)-3,4,5-tris(benzyloxy)-6-((benzyloxy)methyl)tetrahydro-2H-pyran-2-ol (75 g) in dimethyl sulfoxide (400 mL) at 0 °C was added acetic anhydride (225 mL). The mixture was stirred for 16 hours at room temperature before it was cooled to 0 °C. A large volume of water was added, and stirring was stopped so that the reaction mixture was allowed to settle for 3 hours (the crude lactone migrated to the bottom of the flask). The supernatant was removed, and the crude mixture was diluted with ethyl acetate and was washed 3 times with water, neutralized with saturated aqueous mixture of NaHCO₃, and washed again twice with water. The organic layer was then dried over magnesium sulfate, filtered and concentrated to give the title compound. MS (ESI) m/e 561 (M+Na)⁺.

### 2.123.2 (3R,4S,5R,6R)-3,4,5-tris(benzyloxy)-6-(benzyloxymethyl)-2-ethynyl- tetrahydro-2H-pyran-2-ol

To a mixture of ethynyltrimethylsilane (18.23 g) in tetrahydrofuran (400 mL) under nitrogen and chilled in a dry ice/acetone bath (internal temp -65 °C) was added 2.5M BuLi in hexane (55.7 mL) dropwise, keeping the temperature below -60 °C. The mixture was stirred in a cold bath for 40 minutes, followed by an ice-water bath (internal temp rose to 0.4°C) for 40 minutes, and finally cooled to -75°C again. A mixture of Example 2.123.1 (50 g) in tetrahydrofuran (50 mL) was added dropwise, keeping the internal temperature below -70 °C. The mixture was stirred in a dry ice/acetone bath for additional 3 hours. The reaction was quenched with saturated aqueous NaHCO₃ mixture (250 mL). The mixture was allowed to warm to room temperature, extracted with ethyl acetate (3x 300 mL), dried over MgSO₄, filtered, and concentrated in *vacuo* to give the title compound. MS (ESI) m/e 659 (M+Na)⁺.

### 2.123.3 trimethyl(((3S,4R,5R,6R)-3,4,5-tris(benzyloxy)-6-(benzyloxymethyl)-tetrahydro-2H-pyran-2-yl)ethynyl)silane

To a mixed mixture of Example 2.123.2 (60 g) in acetonitrile (450 mL) and dichloromethane (150 mL) at -15 °C in an ice-salt bath was added triethylsilane (81 mL) dropwise, followed by addition of boron trifluoride diethyl ether complex (40.6 mL) at such a rate that the internal temperature did not exceed -10 °C. The mixture was then stirred at -15 °C to -10 °C for 2 hours. The reaction was quenched with saturated aqueous NaHCO₃ mixture (275 mL) and stirred for 1 hour at room temperature. The mixture was then extracted with ethyl acetate (3 x 550 mL). The extracts were dried over MgSO₄, filtered, and concentrated. The residue was purified by flash chromatography eluting with a gradient of 0% to 7% ethyl acetate/petroleum ether to give the title compound. MS (ESI) m/e 643 (M+Na)⁺.

### 2.123.4 (2R,3R,4R,5S)-3,4,5-tris(benzyloxy)-2-(benzyloxymethyl)-6-ethynyl-tetrahydro-2H-pyran

To a mixed mixture of Example 2.123.3 (80 g) in dichloromethane (200 mL) and methanol (1000 mL) was added IN aqueous NaOH mixture (258 mL). The mixture was stirred at room temperature for 2 hours. The solvent was removed. The residue was then partitioned between water and dichloromethane. The extracts were washed with brine, dried over Na₂SO₄, filtered, and concentrated to give the title compound. MS (ESI) m/e 571 (M+Na)⁺.

### 2.123.5 (2R,3R,4R,5S)-2-(acetoxymethyl)-6-ethynyl-tetrahydro-2H-pyran-3,4,5-triyl triacetate

To a mixture of Example 2.123.4 (66 g) in acetic anhydride (500 mL) cooled by an ice/water bath was added boron trifluoride diethyl ether complex (152 mL) dropwise. The mixture was stirred at room temperature for 16 hours, cooled with an ice/water bath and neutralized with saturated aqueous NaHCO₃ mixture. The mixture was extracted with ethyl acetate (3x500 mL), dried over Na₂SO₄, filtered, and concentrated in *vacuo.* The residue was purified by flash chromatography eluting with a gradient of 0% to 30% ethyl acetate/petroleum ether to give the title compound. MS (ESI) m/e 357 (M+H)⁺.

### 2.123.6 (3R,4R,5S,6R)-2-ethynyl-6-(hydroxymethyl)-tetrahydro-2H-pyran-3,4,5-triol

To a mixture of Example 2.123.5 (25 g) in methanol (440 mL) was added sodium methanolate (2.1 g). The mixture was stirred at room temperature for 2 hours, then neutralized with 4M HCl in dioxane. The solvent was removed, and the residue was adsorbed onto silica gel and loaded onto a silica gel column. The column was eluted with a gradient of 0 to 100% ethyl acetate/petroleum ether then 0% to 12% methanol/ethyl acetate to give the title compound. MS (ESI) m/e 211 (M+Na)⁺.

### 2.123.7 (2S,3S,4R,5R)-6-ethynyl-3,4,5-trihydroxy-tetrahydro-2H-pyran-2-carboxylic acid

A three-necked round bottom flask was charged with Example 2.123.6 (6.00 g), KBr (0.30 g), tetrabutylammonium bromide (0.41 g) and 60 mL of saturated aqueous NaHCO₃ mixture. TEMPO ((2,2,6,6-tetramethylpiperidin-1-yl)oxyl, 0.15 g) in 60 mL dichloromethane was added. The mixture was stirred vigorously and cooled in an ice-salt bath to -2 °C internal temperature. A mixture of brine (12 mL), aqueous NaHCO₃ mixture (24 mL) and NaOCl (154 mL) was added dropwise such that the internal temperature was maintained below 2 °C. The pH of the reaction mixture was maintained in the 8.2-8.4 range with the addition of solid Na₂CO₃. After a total of 6 hours, the reaction mixture was cooled to 3 °C internal temperature and ethanol (~20 mL) was added dropwise. The mixture was stirred for ~ 30 minutes. The mixture was transferred to a separatory funnel, and the dichloromethane layer was discarded. The pH of the aqueous layer was adjusted to 2-3 using 1 M aqueous HCl. The aqueous layer was then concentrated to dryness to afford a solid. Methanol (100 mL was) added to the dry solid, and the slurry was stirred for ~30 minutes. The mixture was filtered over a pad of diatomaceous earth, and the residue in the funnel was washed with ~100 mL of methanol. The filtrate was concentrated under reduced pressure to obtain the title compound.

### 2.123.8 (2S,3S,4R,5R)-methyl 6-ethynyl-3,4,5-trihydroxytetrahydro-2H-pyran-2-carboxylate

A 500 mL three-necked round bottom flask was charged with a suspension of Example 2.123.7 (6.45 g) in methanol (96 mL) and was cooled in an ice-salt-bath with internal temperature of - 1 °C. Neat thionyl chloride (2.79 mL) was carefully added. The internal temperature kept rising throughout the addition but did not exceed 10 °C. The reaction was allowed to slowly warm up to 15-20 °C over 2.5 hours. After 2.5 hours, the reaction was concentrated to give the title compound.

### 2.123.9 (3S,4R,5S,6S)-2-ethynyl-6-(methoxycarbonyl)tetrahydro-2H-pyran-3,4,5-triyl triacetate

To Example 2.123.8 (6.9 g) as a mixture in N,N-dimethylformamide (75 mL) was added 4-(dimethylamino)pyridine (0.17 g) and acetic anhydride (36.1 mL). The suspension was cooled in an ice-bath and pyridine (18.04 mL) was added via syringe over 15 minutes. The reaction was allowed to warm to room temperature overnight. Additional acetic anhydride (12 mL) and pyridine (6 mL) were added and stirring was continued for an additional 6 hours. The reaction was cooled in an ice-bath and 250 mL of saturated aqueous NaHCO₃ mixture was added and stirred for 1 hour. Water (100 mL) was added, and the mixture was extracted with ethyl acetate. The organic extract was washed twice with saturated CuSO₄ mixture, dried, filtered, and concentrated. The residue was purified by flash chromatography, eluting with 50% ethyl acetate/petroleum ether to give the title compound. ¹H NMR (500 MHz, methanol-*d₄*) δ ppm 5.29 (t, 1H), 5.08 (td, 2H), 4.48 (dd, 1H), 4.23 (d, 1H), 3.71 (s, 3H), 3.04 (d, 1H), 2.03 (s, 3H), 1.99 (s, 3H), 1.98 (s, 4H).

### 2.123.10 2-iodo-4-nitrobenzoic acid

A 3L fully jacketed flask equipped with a mechanical stirrer, temperature probe and an addition funnel under a nitrogen atmosphere, was charged with 2-amino-4-nitrobenzoic acid (69.1 g, Combi-Blocks) and sulfuric acid, 1.5 M aqueous (696 mL). The resulting suspension was cooled to 0 °C internal temperature, and a mixture of sodium nitrite (28.8 g) in water (250 mL) was added dropwise over 43 minutes with the temperature kept below 1 °C. The reaction was stirred at ca. 0 °C for 1 hour. A mixture of potassium iodide (107 g) in water (250 mL) was added dropwise over 44 minutes with the internal temperature kept below 1 °C. (Initially addition was exothermic and there was gas evolution). The reaction was stirred 1 hour at 0 °C. The temperature was raised to 20 °C and then stirred at ambient temperature overnight. The reaction mixture became a suspension. The reaction mixture was filtered, and the collected solid was washed with water. The wet solid (~ 108 g) was stirred in 10 % sodium sulfite (350 ml, with ~ 200 mL water used to wash in the solid) for 30 minutes. The suspension was acidified with concentrated hydrochloric acid (35 mL), and the solid was collected by filtration and washed with water. The solid was slurried in water (1L) and refiltered, and the solid was left to dry in the funnel overnight. The solid was then dried in a vacuum oven for 2 hours at 60 °C. The resulting solid was triturated with dichloromethane (500 mL), and the suspension was filtered and washed with additional dichloromethane. The solid was air-dried to give the title compound

### 2.123.11 (2-iodo-4-nitrophenyl)methanol

A flame-dried 3 L 3-necked flask was charged with Example 2.123.10 (51.9 g) and tetrahydrofuran (700 mL). The mixture was cooled in an ice bath to 0.5 °C, and boranetetrahydrofuran complex (443 mL, 1M in THF) was added dropwise (gas evolution) over 50 minutes, reaching a final internal temperature of 1.3 °C. The reaction mixture was stirred for 15 minutes, and the ice bath was removed. The reaction was left to come to ambient temperature over 30 minutes. A heating mantle was installed, and the reaction was heated to an internal temperature of 65.5 °C for 3 hours, and then allowed to cool to room temperature while stirring overnight. The reaction mixture was cooled in an ice bath to 0 °C and quenched by dropwise addition of methanol (400 mL). After a brief incubation period, the temperature rose quickly to 2.5 °C with gas evolution. After the first 100 mL are added over ~ 30 minutes, the addition was no longer exothermic, and the gas evolution ceased. The ice bath was removed, and the mixture was stirred at ambient temperature under nitrogen overnight. The mixture was concentrated to a solid, dissolved in dichloromethane/methanol and adsorbed on to silica gel (~ 150 g). The residue was loaded on a plug of silica gel (3000 mL) and eluted with dichloromethane to give the title compound.

### 2.123.12 (4-amino-2-iodophenyl)methanol

A 5 L flask equipped with a mechanical stirrer, heating mantle controlled by a JKEM temperature probe and a condenser was charged with Example 2.123.11 (98.83 g) and ethanol (2 L). The reaction was stirred rapidly, and iron (99 g) was added, followed by a mixture of ammonium chloride (20.84 g) in water (500 mL). The reaction was heated over the course of 20 minutes to an internal temperature of 80.3 °C, where it began to reflux vigorously. The mantle was dropped until the reflux calmed. Thereafter, the mixture was heated to 80 °C for 1.5 hour. The reaction was filtered hot through a membrane filter, and the iron residue was washed with hot 50% ethyl acetate/methanol (800 mL). The eluent was passed through a diatomaceous earth pad, and the filtrate was concentrated. The residue was partitioned between 50% brine (1500 mL) and ethyl acetate (1500 mL). The layers were separated, and the aqueous layer was extracted with ethyl acetate (400 mL x 3). The combined organic layers were dried over sodium sulfate, filtered and concentrated to give the title compound, which was used without further purification.

### 2.123.13 4-(((tert-butyldimethylsilyl)oxy)methyl)-3-iodoaniline

A 5 L flask with a mechanical stirrer was charged with Example 2.123.12 (88 g) and dichloromethane (2 L). The suspension was cooled in an ice bath to an internal temperature of 2.5 °C, and tert-butylchlorodimethylsilane (53.3 g) was added portion-wise over 8 minutes. After 10 minutes, 1H-imidazole (33.7 g) was added portionwise to the cold reaction. The reaction was stirred 90 minutes while the internal temperature rose to 15 °C. The reaction mixture was diluted with water (3 L) and dichloromethane (1 L). The layers were separated, and the organic layer was dried over sodium sulfate, filtered, and concentrated to an oil. The residue was purified by silica gel chromatography (1600 g silica gel), eluting a gradient of 0 - 25% ethyl acetate in heptane, to give the title compound as an oil.

### 2.123.14 (S)-2-((S)-2-((((9H-fluoren-9-yl)methoxy)carbonyl)amino)-3-methylbutanamido)propanoic acid

To a mixture of (S)-2-((((9H-fluoren-9-yl)methoxy)carbonyl)amino)-3-methylbutanoic acid (6.5 g) in dimethoxyethane (40 mL) was added (S)-2-aminopropanoic acid (1.393 g) and sodium bicarbonate (1.314 g) in water (40 mL). Tetrahydrofuran (20 mL) was added to aid solubility. The resulting mixture was stirred at room temperature for 16 hours. Aqueous citric acid (15%, 75 mL) was added, and the mixture was extracted with 10% 2-propanol in ethyl acetate (2 x 100 mL). A precipitate formed in the organic layer. The combined organic layers were washed with water (2 x 150 mL). The organic layer was concentrated under reduced pressure and then triturated with diethyl ether (80 mL). After brief sonication, the title compound was collected by filtration. MS (ESI) m/e 411 (M+H)⁺.

### 2.123.15 (9H-fluoren-9-yl)methyl ((S)-1-(((S)-1-((4-(((tert-butyldimethylsilyl)oxy)methyl)-3-iodophenyl)amino)-1-oxopropan-2-yl)amino)-3-methyl-1-oxobutan-2-yl)carbamate

A mixture of Example 2.123.13 (5.44 g) and Example 2.123.14 (6.15 g) in a mixture of dichloromethane (70 mL) and methanol (35.0 mL) was added ethyl 2-ethoxyquinoline-l(2H)-carboxylate (4.08 g), and the reaction was stirred overnight. The reaction mixture was concentrated and loaded onto silica gel, eluting with a gradient of 10% to 95% heptane in ethyl acetate followed by 5% methanol in dichloromethane. The product-containing fractions were concentrated, dissolved in 0.2% methanol in dichloromethane (50 mL), loaded onto silica gel and eluted with a gradient of 0.2% to 2% methanol in dichloromethane. The product containing fractions were collected to give the title compound. MS (ESI) m/e 756.0 (M+H)⁺.

### 2.123.16 (2S,3S,4R,5S,6S)-2-((5-((S)-2-((S)-2-((((9H-fluoren-9-yl)methoxy)carbonyl)amino)-3-methylbutanamido)propanamido)-2-(((tertbutyldimethylsilyl)oxy)methyl)phenyl)ethynyl)-6-(methoxycarbonyl)tetrahydro-2H-pyran-3,4,5-triyl triacetate

A mixture of Example 2.123.9 (4.500 g), Example 2.123.15 (6.62 g), copper(I) iodide (0.083 g) and bis(triphenylphosphine)palladium(II) dichloride (0.308 g) were combined in vial and degassed. N,N-dimethylformamide (45 mL) and N-ethyl-N-isopropylpropan-2-amine (4.55 mL) were added, and the reaction vessel was flushed with nitrogen and stirred at room temperature overnight. The reaction was partitioned between water (100 mL) and ethyl acetate (250 mL). The layers were separated, and the organic layer was dried over magnesium sulfate, filtered, and concentrated. The residue was purified by silica gel chromatography, eluting with a gradient of 5% to 95% ethyl acetate in heptane. The product containing fractions were collected, concentrated and purified by silica gel chromatography, eluting with a gradient of 0.25% to 2.5% methanol in dichloromethane to give the title compound. MS (ESI) m/e 970.4 (M+H)⁺.

### 2.123.17 (2S,3S,4R,5S,6S)-2-(5-((S)-2-((S)-2-((((9H-fluoren-9-yl)methoxy)carbonyl)amin o)-3-methylbutanamido)propanamido)-2-(((tertbutyldimethylsilyl)oxy)methyl)phenethyl)-6-(methoxycarbonyl)tetrahydro-2H-pyran-3,4,5-triyl triacetate

Example 2.123.16 (4.7 g) and tetrahydrofuran (95 mL) were added to 5% Pt/C (2.42 g, wet) in a 50 mL pressure bottle and shaken for 90 minutes at room temperature under 50 psi of hydrogen. The reaction was filtered and concentrated to give the title compound. MS (ESI) m/e 974.6 (M+H)⁺.

### 2.123.18 (2S,3S,4R,5S,6S)-2-(5-((S)-2-((S)-2-((((9H-fluoren-9-yl)methoxy)carbonyl)amin o)-3-methylbutanamido)propanamido)-2-(hydroxymethyl)phenethyl)-6-(methoxycarbonyl)tetrahydro-2H-pyran-3,4,5-triyl triacetate

A mixture of Example 2.123.17 (5.4 g) in tetrahydrofuran (7 mL), water (7 mL) and glacial acetic acid (21 mL) was stirred overnight at room temperature. The reaction was diluted with ethyl acetate (200 mL) and washed with water (100 mL), saturated aqueous NaHCO₃ mixture (100 mL), brine (100 mL), dried over magnesium sulfate, filtered, and concentrated. The residue was purified by silica gel chromatography, eluting with a gradient of 0.5% to 5% methanol in dichloromethane, to give the title compound. MS (ESI) m/e 860.4 (M+H)⁺.

### 2.123.19 (2S,3S,4R,5S,6S)-2-(5-((S)-2-((S)-2-((((9H-fluoren-9-yl)methoxy)carbonyl)amin o)-3-methylbutanamido)propanamido)-2-((((4-nitrophenoxy)carbonyl)oxy)methyl)phenethyl)-6-(methoxycarbonyl)tetrahydro-2H-pyran-3,4,5-triyl triacetate

To a mixture of Example 2.123.18 (4.00 g) and bis(4-nitrophenyl) carbonate (2.83 g) in acetonitrile (80 mL) was added N-ethyl-N-isopropylpropan-2-amine (1.22 mL) at room temperature. After stirring overnight, the reaction was concentrated, dissolved in dichloromethane (250 mL) and washed with saturated aqueous NaHCO₃ mixture (4 x 150 mL). The organic layer was dried over magnesium sulfate, filtered, and concentrated. The resulting foam was purified by silica gel chromatography, eluting with a gradient of 5% to 75% ethyl acetate in hexanes to give the title compound. MS (ESI) m/e 1025.5 (M+H)⁺.

### 2.123.20 3-(1-((3-(2-((((4-((R)-2-((R)-2-amino-3-methylbutanamido)propanamido)-2-(2-((2S,3R,4R,5S,6S)-6-carboxy-3,4,5-trihydroxytetrahydro-2H-pyran-2-yl)ethyl)benzyl)oxy)carbonyl)(2-sulfoethyl)amino)ethoxy)-5,7-dimethyladamantan-1-yl)methyl)-5-methyl-1H-pyrazol-4-yl)-6-(8-(benzo[d]thiazol-2-ylcarbamoyl)-3,4-dihydroisoquinolin-2(1H)-yl)picolinic acid

To a cold (0 °C) mixture of Example 2.123.19 (70 mg) and Example 1.2.9 (58.1 mg) in N,N-dimethylformamide (4 mL) was added N-ethyl-N-isopropylpropan-2-amine (0.026 mL). The reaction was slowly warmed to room temperature and stirred overnight. To the reaction mixture was added water (1 mL) and LiOH H₂O (20 mg). The mixture was stirred at room temperature for 3 hours. The mixture was acidified with trifluoroacetic acid, filtered and purified by reverse-phase HPLC on a Gilson system (C18 column), eluting with 20-80% acetonitrile in water containing 0.1% trifluoroacetic acid, to give the title compound. MS (ESI) m/e 1564.4 (M-H)⁻.

### 2.123.21 (6S)-2,6-anhydro-6-(2-{2-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.13,7]dec-1-yl}oxy)ethyl](2-sulfoethyl)carbamoyl}oxy)methyl]-5-({N-[(2,5-dioxo-2,5-dihydro-1H-pyrrol-1-yl)acetyl]-L-valyl-L-alanyl}amino)phenyl}ethyl)-L-gulonic acid

The title compound was prepared as described in Example 2.54, replacing Example 2.49.1 with Example 2.123.20. ¹H NMR (500 MHz, dimethyl sulfoxide-d₆) δ ppm 12.86 (s, 1H), 9.92 (d, 1H), 8.35 - 8.19 (m, 2H), 8.04 (d, 1H), 7.80 (d, 1H), 7.61 (d, 1H), 7.57 - 7.32 (m, 8H), 7.28 (s, 1H), 7.22 (d, 1H), 7.08 (s, 2H), 6.95 (d, 1H), 5.12 - 4.91 (m, 5H), 4.39 (t, 1H), 4.32 - 4.19 (m, 1H), 4.12 (s, 2H), 3.89 (t, 2H), 3.80 (d, 2H), 3.14 (t, 1H), 3.06 - 2.87 (m, 4H), 2.69 - 2.58 (m, 4H), 2.37 (p, 1H), 2.09 (d, 4H), 2.04 - 1.91 (m, 4H), 1.54 (d, 1H), 1.40 - 0.99 (m, 20H), 0.99 - 0.74 (m, 16H). MS (ESI) m/e 1513.5 (M-H)⁻.

### 2.124 Synthesis of 3-{[2-({3-[(4-{6-[8-(1,3-benzothiazol-2-ylcarbamoyl)naphthalen-2-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl]({[4-(4-{[6-(2,5-dioxo-2,5-dihydro-1H-pyrrol-1-yl)hexanoyl] amino}butyl)-2-(beta-D-glucopyranuronosyloxy)benzyl]oxy}carbonyl)amino}propyl beta-D-glucopyranosiduronic acid (Synthon ZM)

### 2.124.1A (9H-fluoren-9-yl)methyl but-3-yn-1-ylcarbamate

A mixture of but-3-yn-1-amine hydrochloride (9 g) and N,N-diisopropylethylamine (44.7 mL) was stirred in dichloromethane (70 mL) and cooled to 0 °C. A mixture of (9H-fluoren-9-yl)methyl carbonochloridate (22.06 g) in dichloromethane (35 mL) was added, and the reaction stirred for 2 hours. The reaction was concentrated, and the residue purified by silica gel chromatography, eluting with petroleum ether in ethyl acetate (10%-25%) to give the title compound. MS (ESI) m/e 314 (M+Na)⁺.

### 2.124.1B (3R,4S,5S,6S)-2-(2-formyl-5-iodophenoxy)-6-(methoxycarbonyl)tetrahydro-2H-pyran-3,4,5-triyl triacetate

To a stirred solution of 2-hydroxy-4-iodobenzaldehyde (0.95 g) in acetonitrile (10 ml) was added (3R,4S,5S,6S)-2-bromo-6-(methoxycarbonyl)tetrahydro-2H-pyran-3,4,5-triyl triacetate (2.5 g) and silver oxide (2 g). The mixture was covered with aluminum foil and was stirred at room temperature overnight. After filtration through diatomaceous earth, the filtrate was washed with ethyl acetate, the solution was concentrated. The reaction mixture was purified by flash chromatography using an ISCO CombiFlash system, SF40-80g column, eluted with 15-30% ethyl acetate/heptane (flow rate : 60ml/min), to provide the title compound. MS (ESI) m/e 586.9 (M+Na)⁺.

### 2.124.2 (2S,3S,4S,5R,6S)-methyl 6-(5-(4-(((9H-fluoren-9-yl)methoxy)carbonylamino)but-1-ynyl)-2-formylphenoxy)-3,4,5-triacetoxy-tetrahydro-2H-pyran-2-carboxylate

Example 2.124.1B (2.7 g), Example 2.124.1A (2.091 g), bis(triphenylphosphine)palladium(II) chloride (0.336 g) and copper(I) iodide (0.091 g) were weighed into a vial and flushed with a stream of nitrogen. Triethylamine (2.001 mL) and tetrahydrofuran (45 mL) were added, and the reaction stirred at room temperature. After stirring for 16 hours, the reaction was diluted with ethyl acetate (200 mL) and washed with water (100 mL) and brine (100 mL). The organic layer was dried over magnesium sulfate, filtered, and concentrated. The residue was purified by silica gel chromatography, eluting with petroleum ether in ethyl acetate (10%-50%), to give the title compound. MS (ESI) m/e 750 (M+Na)⁺.

### 2.124.3 (2S,3S,4S,5R,6S)-methyl 6-(5-(4-(((9H-fluoren-9-yl)methoxy)carbonylamino)butyl)-2-formylphenoxy)-3,4,5-triacetoxy-tetrahydro-2H-pyran-2-carboxylate

Example 2.124.2 (1.5 g) and tetrahydrofuran (45 mL) were added to 10% Pd-C (0.483 g) in a 100 mL pressure bottle and stirred for 16 hours under 1 atm H₂ at room temperature. The reaction was filtered and concentrated to give the title compound. MS (ESI) m/e 754 (M+Na)⁺.

### 2.124.4 (2S,3S,4S,5R,6S)-methyl 6-(5-(4-(((9H-fluoren-9-yl)methoxy)carbonylamino)butyl)-2-(hydroxymethyl)phenoxy)-3,4,5-triacetoxy-tetrahydro-2H-pyran-2-carboxylate

A mixture of Example 2.124.3 (2.0 g) in tetrahydrofuran (7.00 mL) and methanol (7 mL) was cooled to 0 °C and NaBH4 (0.052 g) was added in one portion. After 30 minutes, the reaction was diluted with ethyl acetate (150 mL) and water (100 mL). The organic layer was separated, washed with brine (100 mL), dried over magnesium sulfate, filtered, and concentrated. The residue was purified by silica gel chromatography, eluting with petroleum ether in ethyl acetate (10%-40%), to give the title compound. MS (ESI) m/e 756 (M+Na)⁺.

### 2.124.5 (2S,3S,4S,5R,6S)-methyl 6-(5-(4-(((9H-fluoren-9-yl)methoxy)carbonylamino)butyl)-2-(((4-nitrophenoxy)carbonyloxy)methyl)phenoxy)-3,4,5-triacetoxy-tetrahydro-2H-pyran-2-carboxylate

To a mixture of Example 2.124.4 (3.0 g) and bis(4-nitrophenyl) carbonate (2.488 g) in dry acetonitrile (70 mL) at 0 °C was added N,N-diisopropylethylamine (1.07 mL). After stirring at room temperature for 16 hours, the reaction was concentrated to give the residue, which was purified by silica gel chromatography, eluting with petroleum ether in ethyl acetate (10%-50%), to give the title compound. MS (ESI) m/e 921 (M+Na)⁺.

### 2.124.6 3-(1-((3-(2-((((4-(4-aminobutyl)-2-(((2R,3S,4R,5R,6R)-6-carboxy-3,4,5-trihydroxytetrahydro-2H-pyran-2-yl)oxy)benzyl)oxy)carbonyl)(3-(((2S,3S,4R,5R,6R)-6-carboxy-3,4,5-trihydroxytetrahydro-2H-pyran-2-yl)oxy)propyl)amino)ethoxy)-5,7-dimethyladamantan-1-yl)methyl)-5-methyl-1H-pyrazol-4-yl)-6-(8-(benzo[d]thiazol-2-ylcarbamoyl)naphthalen-2-yl)picolinic acid

To a cold (0 °C) mixture of Example 2.124.5 (44 mg) and Example 1.87.3 (47.4 mg) in N,N-dimethylformamide (4 mL) was added N-ethyl-N-isopropylpropan-2-amine (0.026 mL). The reaction was slowly warmed to room temperature and stirred overnight. To the reaction mixture was added water (1 mL) and LiOH H₂O(20 mg). The mixture was stirred at room temperature for 3 hours. The mixture was acidified with trifluoroacetic acid, filtered and purified by reverse-phase HPLC on a Gilson system (C18 column), eluting with 20-80% acetonitrile in water containing 0.1% trifluoroacetic acid, to give the title compound. MS (ESI) m/e 1564.4 (M-H)⁻.

### 2.124.7 3-{[2-({3-[(4-{6-[8-(1,3-benzothiazol-2-ylcarbamoyl)naphthalen-2-yl]-2-carboxypyridin-3-yl}-5-methyl-1H-pyrazol-1-yl)methyl]-5,7-dimethyltricyclo [3.3.1.13,7]dec-1-yl}oxy)ethyl]({ [4-(4-{ [6-(2,5-dioxo-2,5-dihydro-1H-pyrrol-1-yl)hexanoyl]amino}butyl)-2-(beta-D-glucopyranuronosyloxy)benzyl]oxy}carbonyl)amino}propyl beta-D-glucopyranosiduronic acid

The title compound was prepared as described in Example 2.5.4, replacing Example 2.5.3 with Example 2.124.6. ¹H NMR (400 MHz, dimethyl sulfoxide-d₆) δ ppm 13.06 (s, 2H), 8.99 (s, 1H), 8.34 (dd, 1H), 8.25 - 8.09 (m, 3H), 8.08 - 8.02 (m, 1H), 7.98 (d, 1H), 7.89 (d, 1H), 7.78 (d, 1H), 7.66 (q, 2H), 7.50 - 7.41 (m, 2H), 7.37 - 7.31 (m, 1H), 7.14 (t, 1H), 6.94 (s, 2H), 6.90 (s, 1H), 6.82 (d, 1H), 5.14 - 5.02 (m, 2H), 4.97 (d, 1H), 4.19 (d, 1H), 3.85 (dd, 3H), 3.37 - 3.23 (m, 9H), 3.14 (t, 1H), 3.04 - 2.92 (m, 4H), 2.19 (s, 3H), 1.96 (t, 2H), 1.73 (s, 2H), 1.55 - 0.87 (m, 21H), 0.81 (d, 6H). MS (ESI) m/e 1564.4 (M-H)⁻.

### 2.125 Synthesis of N-{[(3S,5S)-3-(2,5-dioxo-2,5-dihydro-1H-pyrrol-1-yl)-5-(methoxymethyl)-2-oxopyrrolidin-1-yl]acetyl}-L-vatyl-N-{4-[({[2-({3-[(4-{6-[8-(1,3-benzothiazol-2-ylcarbarmoyl)-3,4-dihydroisoquinolin-2(1H)-yl]-2-carboxypyridin-3-yl}-5-methyl-1H-pyrazol-1-yl)methyl]-5,7-dimethyltricyclo [3.3.1.1^{3,7}]dec-1-yl}oxy)ethyl](2-sulfoethyl)carbamoyl}oxy)methyl]phenyl}-N⁵-carbamoyl-L-ornithinamide (Synthon SV)

### 2.125.1 tert-butyl 2-((3S,5S)-3-(dibenzylamino)-5-(methoxymethyl)-2-oxopyrrolidin-1-yl)acetate

To a mixture of Example 2.119.10 (1.4 g) in N,N-dimethylformamide (5 mL) was added iodomethane (0.8 mL). The reaction was cooled to 0 °C, and 95% sodium hydride (80 mg) was added. After five minutes the cooling bath was removed, and the reaction stirred at room temperature for 2.5 hours. The reaction was quenched by the addition of water (20 mL) and ethyl acetate (40 mL). The layers were separated, and the organic layer was washed with brine. The combined aqueous layers were back-extracted with ethyl acetate (10 mL). The combined organic layers were dried with sodium sulfate, filtered and concentrated under reduced pressure. The residue was purified by silica gel chromatography, eluting with 80/20 heptane/ethyl acetate, to give the title compound. MS (DCI) m/e 439.2 (M+H)⁺.

### 2.125.2 tert-butyl 2-((3S,5S)-3-amino-5-(methoxymethyl)-2-oxopyrrolidin-1-yl)acetate

To a mixture of Example 2.125.1 (726 mg) in 2,2,2-trifluoroethanol (10 mL) was added palladium hydroxide on carbon (20% by wt, 150 mg). The reaction was stirred under a hydrogen atmosphere (50 psi) at room temperature for two hours. The reaction was filtered and concentrated to give the title compound. MS (DCI) m/e 259.0 (M+H)⁺.

### 2.125.3 4-(((3S,5S)-1-(2-(tert-butoxy)-2-oxoethyl)-5-(methoxymethyl)-2-oxopyrrolidin-3-yl)amino)-4-oxobut-2-enoic acid

The title compound was prepared by substituting Example 2.125.2 for Example 2.119.12 in Example 2.119.13. MS (DCI) m/e 374.0 (M+NH₃+H)⁺.

### 2.125.4 tert-butyl 2-((3S,5S)-3-(2,5-dioxo-2,5-dihydro-1H-pyrrol-1-yl)-5-(methoxymethyl)-2-oxopyrrolidin-1-yl)acetate

The title compound was prepared by substituting Example 2.125.3 for Example 2.119.13 in Example 2.119.14. MS (DCI) m/e 356.0 (M+NH₃+H)⁺.

### 2.125.5 2-((3S,5S)-3-(2,5-dioxo-2,5-dihydro-1H-pyrrol-1-yl)-5-(methoxymethyl)-2-oxopyrrolidin-1-yl)acetic acid

To a mixture of Example 2.125.4 (120 mg) in dichloromethane (8 mL) was added trifluoroacetic acid (4 mL). The reaction was stirred at room temperature for 90 minutes and then concentrated under reduced pressure. The residue was dissolved in acetonitrile (4 mL) and purified by preparative reverse-phase HPLC with a Luna C18(2) AXIA column, 250 x 50 mm, l0µ particle size, using a gradient of 5-75% acetonitrile in 0.1% trifluoroacetic acid in water over 30 minutes, to give the title compound. MS (DCI) m/e 300.0 (M+NH₃+H)⁺.

### 2.125.6 N-{[(3S,5S)-3-(2,5-dioxo-2,5-dihydro-1H-pyrrol-1-yl)-5-(methoxymethyl)-2-oxopyrrolidin-1-yl]acetyl}-L-valyl-N-{4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.13,7]dec-1-yl}oxy)ethyl](2-sulfoethyl)carbamoyl}oxy)methyl]phenyl}-N5-carbamoyl-L-ornithinamide

The title compound was prepared by substituting Example 2.125.5 for Example 2.119.15 and Example 2.49.1 for Example 2.119.16 in Example 2.119.17. ¹H NMR (400 MHz, dimethyl sulfoxide-d₆) δ ppm 9.98 (s, 1H), 8.19 (br d, 1H), 8.03 (d, 1H), 7.96 (d, 1H), 7.79 (d, 1H), 7.61 (m, 3H), 7.55 (d, 1H), 7.45 (m, 2H), 7.37 (m, 2H), 7.32 (s, 1H), 7.27 (d, 2H), 7.08 (s, 2H), 6.96 (d, 1H), 5.00 (m, 2H), 4.96 (s, 2H), 4.69 (t, 1H), 4.39 (br m, 1H), 4.28 (m, 1H), 4.20 (d, 1H), 3.88 (t, 3H), 3.81 (br m, 3H), 3.46 (m, 3H), 3.40 (m, 2H), 3.26 (brm, 2H), 3.25 (s, 3H), 3.01 (m, 3H), 2.96 (m,1H), 2.65 (t, 2H), 2.36 (br m, 1H), 2.10 (s, 3H), 2.00 (m, 1H), 1.94 (m, 1H), 1.69 (br m, 1H), 1.59 (br m, 1H), 1.49-0.92 (m, 16H), 0.88 (d, 3H), 0.83 (m, 9H). MS (ESI) m/e 1521.5 (M-H)⁻.

### 2.126 Synthesis of (6S)-2,6-anhydro-6-(2-{2-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-ylloxy)ethyl](2-sulfoethyl)carbamoyl}oxy)methyl]-5-({N-[6-(2,5-dioxo-2,5-dihydro-1H-pyrrol-1-yl)hexanoyl]-L-valyl-L-alanyl}amino)phenyl}ethyl)-L-gulonic acid (Synthon SY)

The title compound was prepared as described in Example 2.123.21, replacing 2,5-dioxopyrrolidin-1-yl 2-(2,5 -dioxo-2,5 -dihydro- 1H-pyrrol-1-yl)acetate with 2,5-dioxopyrrolidin-1-yl 6-(2,5-dioxo-2,5-dihydro-1H-pyrrol-1-yl)hexanoate. ¹H NMR (501 MHz, dimethyl sulfoxide-*d*₆) δ ppm 12.83 (s, 1H), 9.87 (s, 1H), 8.09 (d, 1H), 8.05 - 7.95 (m, 1H), 7.77 (d, 2H), 7.59 (d, 1H), 7.55-7.31 (m, 7H), 7.28 (s, 1H), 7.20 (d, 1H), 6.97 (s, 2H), 6.94 (d, 1H), 5.08 - 4.84 (m, 5H), 4.36 (p, 1H), 3.78 (d, 2H), 3.54 (t, 1H), 3.48 - 3.28 (m, 9H), 3.21 (s, 2H), 3.12 (t, 2H), 3.02 - 2.84 (m, 4H), 2.81-2.54 (m, 6H), 2.19 - 1.84 (m, 9H), 1.63 - 1.39 (m, 6H), 1.35 (s, 1H), 1.29 - 0.86 (m, 18H), 0.80 (td, 15H). MS (ESI) m/e 1568.4 (M-H)⁻.

### 2.127 Synthesis of 2-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo [3.3.1.1^{3,7}] dec-1-yl}oxy)ethyl](2-sulfoethyl)carbam oyl}oxy)methyl]-5-(4-{[3-(2,5-dioxo-2,5-dihydro-1H-pyrrol-1-yl)propanoyl]amino}butyl)phenyl beta-D-glucopyranosiduronic acid (Synthon TK)

### 2.127.1 3-(1-((3-(2-((((4-(4-aminobutyl)-2-(((2S,3R,4S,5S,6S)-6-carboxy-3,4,5-trihydroxytetrahydro-2H-pyran-2-yl)oxy)benzyl)oxy)carbonyl)(2-sulfoethyl)amino)ethoxy)-5,7-dimethyladamantan-1-yl)methyl)-5-methyl-1H-pyrazol-4-yl)-6-(8-(benzo[d]thiazol-2-ylcarbamoyl)-3,4-dihydroisoquinolin-2(1H)-yl)picolinic acid

To a mixture of Example 1.2.9 (0.030 g), Example 2.124.5 (0.031 g) and 1H-benzo[d][1,2,3]triazol-1-ol hydrate (5 mg) in N,N-dimethylformamide (0.5 mL) was added N-ethyl-N-isopropylpropan-2-amine (0.017 mL), and the reaction mixture was stirred for 3 hours. The reaction mixture was concentrated, dissolved in tetrahydrofuran (0.4 mL) and methanol (0.4 mL) and treated with lithium hydroxide hydrate (0.020 g) as a mixture in water (0.5 mL). After 1 hour, the reaction was quenched with 2,2,2-trifluoroacetic acid (0.072 mL), diluted with N,N-dimethylformamide:water (1:1) (1 mL) and purified by preparatory reverse-phase HPLC using a Gilson PLC 2020 system, eluting with a gradient of 5% to 75% acetonitrile/water. Product-containing fractions were combined and lyophilized to give to title compound. MS (ESI) m/e 1251.7 (M+H)⁺.

### 2.127.2 2-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo [3.3.1.13,7] dec-1-yl}oxy)ethyl](2-sulfoethyl)carbamoyl}oxy)methyl]-5-(4-{[3-(2,5-dioxo-2,5-dihydro-1H-pyrrol-1-yl)propanoyl]amino}butyl)phenyl beta-D-glucopyranosiduronic acid

To a mixture of Example 2.127.1 (0.027 g) and 2,5-dioxopyrrolidin-1-yl 3-(2,5-dioxo-2,5-dihydro-1H-pyrrol-1-yl)propanoate (6.32 mg) in N,N-dimethylformamide (0.4 mL) was added N-ethyl-N-isopropylpropan-2-amine (0.017 mL), and the reaction was stirred for 1 hour at room temperature. The reaction was quenched with a mixture of 2,2,2-trifluoroacetic acid (0.038 mL), water (1.5 mL) and N,N-dimethylformamide (0.5 mL) and purified by preparatory reverse-phase HPLC on a Gilson 2020 system, using a gradient of 5% to 75% acetonitrile/water. The product-containing fractions were lyophilized to give the title compound. ¹H NMR (501 MHz, dimethyl sulfoxide-d₆) δ 12.84 (s, 1H), 8.03 (dd, 1H), 7.91 - 7.85 (m, 1H), 7.78 (d, 1H), 7.61 (dd, 1H), 7.52 (dd, 1H), 7.50 - 7.40 (m, 2H), 7.39 - 7.31 (m, 2H), 7.31 (s, 1H), 7.17 (dd, 1H), 6.99 - 6.90 (m, 4H), 6.83 (d, 1H), 5.15 - 5.04 (m, 2H), 5.05 - 4.96 (m, 1H), 4.95 (s, 2H), 3.91 - 3.83 (m, 4H), 3.81 (d, 3H), 3.58 (t, 2H), 3.42 (td, 3H), 3.33 - 3.24 (m, 5H), 3.00 (q, 4H), 2.68 (dt, 2H), 2.29 (t, 2H), 2.09 (d, 3H), 1.49 (d, 3H), 1.34 (td, 5H), 1.21 (dd, 5H), 1.15 - 1.07 (m, 2H), 1.07 (s, 4H), 0.95 (q, 1H), 0.82 (d, 6H). MS (ESI) m/e 1402.1 (M+H)⁺.

### 2.128 Synthesis of 2-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)methyl]-5-[4-({(2S)-2-(2,5-dioxo-2,5-dihydro-1H-pyrrol-1-yl)-3-[4-(2,5,8,11,14,17,20,23,26,29,32-un decaoxatetratriacontan-34-yloxy)phenyl]propanoyl}amino)butyl]phenyl beta-D-glucopyranosiduronic acid (Synthon TR)

A mixture of Example 2.120.5 (0.035 g), O-(7-azabenzotriazol-1-yl)-N,N,N',N'-tetramethyluronium hexafluorophosphate (0.015 g) and N-ethyl-N-isopropylpropan-2-amine (0.015 mL) was stirred in N,N-dimethylformamide (0.4 mL) for 5 minutes. The mixture was added to a mixture of Example 2.127.1 (0.030 g) and N-ethyl-N-isopropylpropan-2-amine (0.015 mL) in N,N-dimethylformamide (0.4 mL) and stirred at room temperature for 3 hours. The reaction was diluted with a mixture of water (1.5 mL), N,N-dimethylformamide (0.5 mL) and 2,2,2-trifluoroacetic acid (0.034 mL) and purified by preparatory reverse-phase HPLC on a Gilson 2020 system, using a gradient of 5% to 85% acetonitrile/water. The product-containing fractions were lyophilized to give the title compound. ¹H NMR (400 MHz, dimethyl sulfoxide-d₆) δ 12.83 (s, 1H), 8.04 - 7.93 (m, 2H), 7.76 (d, 1H), 7.58 (dd, 1H), 7.53 - 7.36 (m, 3H), 7.37 - 7.25 (m, 3H), 7.15 (d, 1H), 6.97 - 6.88 (m, 4H), 6.87 (d, 2H), 6.85 - 6.77 (m, 1H), 6.76 - 6.69 (m, 2H), 5.13 - 4.96 (m, 3H), 4.92 (s, 2H), 3.95 (dd, 2H), 3.84 (d, 2H), 3.78 (s, 8H), 3.69 - 3.60 (m, 2H), 3.47 (d, 38H), 3.48 - 3.35 (m, 6H), 3.20 (s, 8H), 3.10 (dd, 2H), 2.98 (t, 2H), 2.69 - 2.60 (m, 2H), 2.50 (d, 1H), 2.06 (s, 3H), 1.49 (t, 2H), 1.35 (s, 4H), 1.21 (d, 4H), 1.05 (s, 6H), 0.79 (d, 6H). MS (ESI) m/e 1991.6 (M-H)⁻.

### 2.129 Synthesis of (6S)-2,6-anhydro-6-(2-{2-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)methyl]-5-[(N-{(2S)-2-(2,5-dioxo-2,5-dihydro-1H-pyrrol-1-yl)-3-[4-(2,5,8,11,14,17,20,23,26,29,32-undecaoxatetratriacontan-34-yloxy)phenyl]propanoyl}-L-valyl-L-alanyl)amino]phenyl}ethyl)-L-gulonic acid (Synthon TY)

A mixture of Example 2.120.5 (0.033 g), O-(7-azabenzotriazol-1-yl)-N,N,N',N'-tetramethyluronium hexafluorophosphate (0.014 g) and N-ethyl-N-isopropylpropan-2-amine (0.015 mL) was stirred in N,N-dimethylformamide (0.4 mL) for 5 minutes. This mixture was added to a mixture of Example 2.123.20 (0.032 g) and N-ethyl-N-isopropylpropan-2-amine (0.015 mL) in N,N-dimethylformamide (0.4 mL) and stirred at room temperature for 3 hours. The reaction was diluted with a mixture of water (1.5 mL), N,N-dimethylformamide (0.5 mL) and 2,2,2-trifluoroacetic acid (0.033 mL) and purified by preparatory reverse-phase HPLC on a Gilson 2020 system, using a gradient of 5% to 85% acetonitrile/water. The product-containing fractions were lyophilized to give the title compound. ¹H NMR (501 MHz, dimethyl sulfoxide-*d*₆) δ 9.90 (d, 1H), 8.25 (d, 1H), 8.12 (m, 1), 8.01 (m, 1H), 1.78 (m, 1H), 7.59 (d, 1H), 7.53 - 7.40 (m, 4H), 7.43 - 7.30 (m, 4H), 7.27 (s, 1H), 7.18 (d, 2H), 7.06 (s, 1H), 7.00 (d, 2H), 6.97 - 6.91 (m, 2H), 6.87 (s, 2H), 6.76 (d, 2H), 5.02 - 4.92 (m, 4H), 4.77 (dd, 1H), 4.20 (t, 1H), 3.98 (dd, 2H), 3.86 (t, 2H), 3.78 (d, 2H), 3.70 - 3.65 (m, 2H), 3.54 (s, 2H), 3.55 - 3.45 (m, 38H), 3.45 - 3.37 (m, 2H), 3.35 - 3.25 (m, 2H), 3.21 (s, 4H), 3.17 - 3.06 (m, 2H), 2.99 (t, 2H), 2.73 (s, 2H), 2.61 (s, 4H), 2.07 (d, 4H), 2.01 (s, 2H), 1.94 (s, 2H), 1.54 (s, 2H), 1.27 (d, 4H), 1.22 (s, 2H), 1.11 (s, 6H), 1.08 - 0.99 (m, 2H), 0.90 - 0.79 (m, 6H), 0.76 (d, 6H). MS (ESI) m/e 705.6 (M-3H)³⁻.

### 2.130 Synthesis of 6-(8-(benzo[d]thiazol-2-ylcarbamoyl)-3,4-dihydroisoquinolin-2(1H)-yl)-3-(1-((3-(2-((((2-(2-((2S,3R,4R,5S,6S)-6-carboxy-3,4,5-trihydroxytetrahydro-2H-pyran-2-yl)ethyl)-4-((S)-2-((S)-2-(2-((3S,5S)-3-(2,5-dioxo-2,5-dihydro-1H-pyrrol-1-yl)-2-oxo-5-((2-sulfoethoxy)methyl)pyrrolidin-1-yl)acetamido)-3-methylbutanamido)propanamido)benzyl)oxy)carbonyl)(2-sulfoethyl)amino)ethoxy)-5,7-dimethyladamantan-1-yl)methyl)-5-methyl-1H-pyrazol-4-yl)picolinic acid (Synthon TX)

The title compound was prepared by substituting Example 2.123.20 for Example 2.119.16 in Example 2.119.17. ¹H NMR (400 MHz, dimethyl sulfoxide-*d₆*) δ ppm 9.85 (s, 1H), 8.17 (br d, 1H), 8.01 (d, 2H), 7.77 (d, 1H), 7.59 (d, 1H), 7.53 (d, 1H), 7.43 (m, 4H), 7.34 (m, 3H), 7.19 (d, 1H), 7.06 (s, 2H), 6.96 (d, 1H), 4.99 (m, 2H), 4.95 (s, 2H), 4.63 (t, 1H), 4.36 (t, 1H), 4.19 (br m, 1H), 4.16 (d, 1H), 3.98 (d, 1H), 3.87 (br t, 2H), 3.81 (br d, 2H), 3.73 (brm, 1H), 3.63 (t, 2H), 3.53 (m, 2H), 3.44 (m, 4H), 3.31 (t, 2H), 3.21 (br m, 2H), 3.17 (m, 2H), 3.00 (m, 2H), 2.92 (br m, 1H), 2.75 (m, 3H), 2.65 (br m, 3H), 2.35 (br m, 1H), 2.07 (s, 3H), 1.98 (br m, 2H), 1.85 (m, 1H), 1.55 (br m, 1H), 1.34 (br m, 1H), 1.26 (br m, 6H), 1.09 (br m, 7H), 0.93 (br m, 1H), 0.87, 0.83, 0.79 (all d, total 12H). MS (ESI) m/e 1733.4 (M-H)⁻.

### 2.131 Synthesis of 6-(8-(benzo[d]thiazol-2-ylcarbamoyl)-3,4-dihydroisoquinolin-2(1H)-yl)-3-(1-((3-(2-((((2-(((2S,3R,4S,5S,6S)-6-carboxy-3,4,5-trihydroxytetrahydro-2H-pyran-2-yl)oxy)-4-(4-(2-((3S,5S)-3-(2,5-dioxo-2,5-dihydro-1H-pyrrol-1-yl)-2-oxo-5-((2-sulfoethoxy)methyl)pyrrolidin-1-yl)acetamido)butyl)benzyl)oxy)carbonyl)(2-sulfoethyl)amino)ethoxy)-5,7-dimethyladamantan-1-yl)methyl)-5-methyl-1H-pyrazol-4-yl)picolinic acid (Synthon TZ)

The title compound was prepared by substituting Example 2.127.1 for Example 2.119.16 in Example 2.119.17. ¹H NMR (500 MHz, dimethyl sulfoxide-d₆) δ ppm 8.02 (d, 1H), 7.82 (br t, 1H), 7.77 (d, 1H), 7.60 (d, 1H), 7.53 (br d, 1H), 7.45 (ddd, 1H), 7.42 (d, 1H), 7.36 (d, 1H), 7.35 (s, 1H), 7.33 (m, 1H), 7.15 (d, 1H), 7.05 (s, 2H), 6.97 (d, 1H), 6.94 (s, 1H), 6.83 (d, 1H), 5.07 (br m, 2H), 5.00 (d, 1H), 4.95 (s, 2H), 4.69 (t, 1H), 4.04 (d, 2H), 3.87 (m, 3H), 3.82 (m, 3H), 3.73 (br m, 1H), 3.61 (m, 2H), 3.47 (br m, 3H), 3.40 (m, 4H), 3.29 (m, 4H), 3.06 (br m, 2H), 3.00 (t, 2H), 2.73 (br m, 2H) 2.69 (br m, 2H), 2.52 (br t, 2H), 2.35 (br m, 1H), 2.08 (s, 3H), 1.81 (m, 1H), 1.53 (br m, 2H), 1.40 (m, 2H), 1.35 (br m, 2H), 1.29-0.88 (br m, 10H), 0.82, 0.80 (both s, total 6H). MS (ESI-) m/e 1607.5 (M-H)⁻.

### 2.132 Synthesis of 2-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1³'⁷] dec-1-yl}oxy)ethyl](2-sulfoethyl)carbamoyl}oxy)methyl]-5-(4-{[(2,5-dioxo-2,5-dihydro-1H-pyrrol-1-yl)acetyl]amino}butyl)phenyl beta-D-glucopyranosiduronic acid (Synthon UA)

To a mixture of Example 2.127.1 (0.032 g) in N,N-dimethylformamide (0.4 mL) was added N-ethyl-N-isopropylpropan-2-amine (0.025 mL), and the mixture cooled to 0 °C. 2,5-Dioxopyrrolidin-1-yl 2-(2,5-dioxo-2,5-dihydro-1H-pyrrol-1-yl)acetate (8.86 mg) was added in one portion and stirred at 0 °C for 45 minutes. The reaction was diluted with a mixture of water (1.5 mL), N,N-dimethylformamide (0.5 mL) and 2,2,2-trifluoroacetic acid (0.036 mL) and was purified by preparatory reverse-phase HPLC on a Gilson 2020 system, using a gradient of 5% to 75% acetonitrile/water. The product-containing fractions were lyophilized to give the title compound. ¹H NMR (501 MHz, dimethyl sulfoxide-d₆) δ 12.86 (s, 1H), 8.06 (s, 1H), 8.02 (dd, 1H), 7.77 (d, 1H), 7.60 (dd, 1H), 7.51 (dd, 1H), 7.49 - 7.39 (m, 2H), 7.38 - 7.28 (m, 3H), 7.17 (dd, 1H), 7.06 (d, 2H), 6.98 - 6.89 (m, 2H), 6.83 (d, 1H), 5.13 - 5.03 (m, 2H), 5.04 - 4.96 (m, 1H), 4.94 (s, 2H), 3.97 (s, 2H), 3.90 - 3.77 (m, 6H), 3.50 (s, 1H), 3.50 - 3.41 (m, 2H), 3.41 (dt, 3H), 3.28 (dt, 4H), 3.06 - 2.96 (m, 4H), 2.66 (dt, 2H), 2.51 (s, 2H), 2.08 (d, 3H), 1.52 (s, 2H), 1.42 - 1.32 (m, 4H), 1.23 (d, 4H), 1.11 (q, 2H), 1.06 (s, 4H), 0.81 (d, 6H). MS (ESI) m/e 1388.0 (M+H)⁺.

### 2.133 Synthesis of 2-[({[2-({3-[(4-{6-[8-(1,3-benzothiazol-2-ylcarbamoyl)naphthalen-2-yl]-2-carboxypyridin-3-yl}-5-methyl-1H-pyrazol-I-yl)methyl]-5,7-dimethyltricyclo[3.3.1.1^{3,7}]dec-1-yl}oxy)ethyl](2-sulfoethyl)carbamoyl}oxy)methyl]-5-(4-{[(2,5-dioxo-2,5-dihydro-1H-pyrrol-1-yl)acetyl]amino}butyl)phenyl beta-D-glucopyranosiduronic acid (Synthon UZ)

### 2.133.1 3-(1-((3-(2-((((4-(4-aminobutyl)-2-(((2S,3R,4S,5S,6S)-6-carboxy-3,4,5-trihydroxytetrahydro-2H-pyran-2-yl)oxy)benzyl)oxy)carbonyl)(2-sulfoethyl)amino)ethoxy)-5,7-dimethyladamantan-1-yl)methyl)-5-methyl-1H-pyrazol-4-yl)-6-(8-(benzo [d]thiazol-2-ylcarbamoyl)naphthalen-2-yl)picolinic acid

To a mixture of Example 2.124.5 (0.060 g), Example 1.43.7 (0.056 g) and 1H-benzo[d][1,2,3]triazol-1-ol (8 mg) in dimethyl sulfoxide (0.5 mL) was added N-ethyl-N-isopropylpropan-2-amine (0.056 mL), and the reaction was stirred at room temperature for 3 hours. The reaction was treated with a mixture of lithium hydroxide hydrate (0.026 g) in water (1 mL) and stirred for 30 minutes. Methanol (0.5 mL) was added to the reaction and stirring was continued for 30 minutes. Diethylamine (0.033 mL) was added to the reaction and stirring was continued overnight. The reaction was quenched with 2,2,2-trifluoroacetic acid (0.120 mL) and purified by preparatory reverse-phase HPLC on a Gilson 2020 system, using a gradient of 5% to 75% acetonitrile/water. The product-containing fractions were lyophilized to give the title compound. MS (ESI) m/e 1247.7 (M+H)⁺.

### 2.133.2 2-[({[2-({3-[(4-{6-[8-(1,3-benzothiazol-2-ylcarbamoyl)naphthalen-2-yl]-2-carboxypyridin-3-yl}-5-methyl-1H-pyrazol-1-yl)methyl]-5,7-dimethyltricyclo [3.3.1.13,7] dec-1-yl}oxy)ethyl](2-sulfoethyl)carbamoyl}oxy)methyl]-5-(4-{[(2,5-dioxo-2,5-dihydro-1H-pyrrol-1-yl)acetyl]amino}butyl)phenyl beta-D-glucopyranosiduronic acid

To a mixture of Example 2.133.1 (0.030 g) in N,N-dimethylformamide (0.400 mL) was added N-ethyl-N-isopropylpropan-2-amine (0.023 mL) and the mixture was cooled to 0 °C. 2,5-Dioxopyrrolidin-1-yl 2-(2,5-dioxo-2,5-dihydro-1H-pyrrol-1-yl)acetate (8.34 mg) was added in one portion and he mixture was stirred at 0 °C for 30 minutes. The reaction was diluted with a mixture of water (1.5 mL), N,N-dimethylformamide (0.5 mL) and 2,2,2-trifluoroacetic acid (0.034 mL) and was purified by preparatory reverse-phase HPLC on a Gilson 2020 system, using a gradient of 5% to 75% acetonitrile/water. The product-containing fractions were lyophilized to give the title compound. ¹H NMR (400 MHz, dimethyl sulfoxide-*d*₆) δ 13.08 (s, 1H), 9.01 (s, 1H), 8.39 - 8.31 (m, 1H), 8.25 - 8.11 (m, 3H), 8.06 (d, 2H), 7.99 (d, 1H), 7.94 (d, 1H), 7.79 (d, 1H), 7.68 (t, 1H), 7.51 - 7.42 (m, 1H), 7.46 (s, 1H), 7.35 (t, 1H), 7.22 - 7.13 (m, 1H), 7.06 (d, 2H), 6.93 (d, 1H), 6.83 (d, 1H), 5.15 - 5.00 (m, 2H), 4.99 (d, 1H), 3.97 (s, 2H), 3.86 (d, 3H), 3.42 (d, 4H), 3.29 (d, 5H), 3.03 (p, 2H), 2.72 - 2.62 (m, 2H), 2.51 (d, 3H), 2.21 (s, 3H), 1.51 (q, 2H), 1.37 (q, 4H), 1.24 (d, 4H), 1.10 (s, 5H), 0.83 (d, 6H), 0.61 (s, 2H). MS (ESI) m/e 1383.0 (M+H)⁺.

### 2.134 Synthesis of 2-[({[2-({3-[(4-{6-[8-(1,3-benzothiazol-2-ylcarbamoyl)naphthalen-2-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)methyl]-5-[4-({(2S)-2-(2,5-dioxo-2,5-dihydro-1H-pyrrol-1-yl)-3-[4-(2,5,8,11,14,17,20,23,26,29,32-undecaoxatetratriacontan-34-yloxy)phenyl]propanoyl}amino)butyl]phenyl beta-D-glucopyranosiduronic acid (Synthon UK)

A mixture of Example 2.120.5 (0.028 g), O-(7-azabenzotriazol-1-yl)-N,N,N',N'-tetramethyluronium hexafluorophosphate (0.013 g) and N-ethyl-N-isopropylpropan-2-amine (0.015 mL) were stirred in N,N-dimethylformamide (0.4 mL) for 5 minutes. The mixture was added to a mixture of Example 2.133.1 (0.030 g) and N-ethyl-N-isopropylpropan-2-amine (0.015 mL) in N,N-dimethylformamide (0.4 mL) and was stirred at room temperature for 1 hour. The reaction was diluted with a mixture of water (1.5 mL), N,N-dimethylformamide (0.5 mL) and 2,2,2-trifluoroacetic acid (0.042 mL) and was purified by preparatory reverse-phase HPLC on a Gilson 2020 system, using a gradient of 5% to 75% acetonitrile/water. The product-containing fractions were lyophilized to give the title compound. ¹H NMR (400 MHz, dimethyl sulfoxide-*d*₆) δ 9.01 (s, 1H), 8.35 (dd, 1H), 8.27 - 8.13 (m, 3H), 8.06 (d, 1H), 8.00 (d, 1H), 7.94 (d, 1H), 7.79 (d, 1H), 7.73 - 7.64 (m, 1H), 7.53 - 7.43 (m, 2H), 7.42 - 7.32 (m, 1H), 7.17 (d, 1H), 7.06 (s, 1H), 7.04 - 6.91 (m, 3H), 6.89 (d, 2H), 6.83 (d, 1H), 6.74 (d, 1H), 5.16 - 4.93 (m, 4H), 4.63 (dd, 2H), 3.96 (t, 2H), 3.86 (d, 4H), 3.66 (s, 4H), 3.55 - 3.46 (m, 36H), 3.45 - 3.35 (m, 8H), 3.35 - 3.24 (m, 6H), 3.21 (s, 2H), 3.11 (s, 2H), 2.99 (d, 2H), 2.83 - 2.59 (m, 3H), 2.52 (d, 2H), 2.21 (s, 3H), 1.57 - 0.86 (m, 14H), 0.83 (d, 4H). MS (ESI) m/e 1986.6 (M-H)⁻.

### 2.135 Synthesis of N-[(2,5-dioxo-2,5-dihydro-1H-pyrrol-1-yl)acetyl]-L-valyl-N-{4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)methyl]-3-(4-carboxybutyl)phenyl}-L-alaninamide (Synthon UU)

### 2.135.1 methyl 4-((tert-butoxycarbonyl)amino)-2-iodobenzoate

3-Iodo-4-(methoxycarbonyl)benzoic acid (9 g) was dissolved in tert-butanol (100 mL), and diphenyl phosphorazidate (7.6 mL) and triethylamine (4.9 mL) were added. The mixture was heated to 83 °C (internal temperature) overnight. The mixture was concentrated to dryness and purified by flash chromatography, eluting with a gradient of 0% to 20% ethyl acetate in heptane to give the title compound. MS (ESI) m/e 377.9 (M+H)⁺.

### 2.135.2 methyl 4-amino-2-iodobenzoate

Example 2.135.1 (3 g) was stirred in dichloromethane (30 mL) and trifluoroacetic acid (10 mL) at room temperature for 1.5 hours. The reaction was concentrated to dryness and partitioned between water (adjusted to pH 1 with hydrochloric acid) and diethyl ether. The layers were separated, and the aqueous layer was washed with aqueous sodium bicarbonate mixture, dried over sodium sulfate, filtered and concentrated to dryness. The resulting solid was triturated with toluene to give the title compound. MS (ESI) m/e 278.0 (M+H)⁺.

### 2.135.3 methyl 4-((S)-2-((S)-2-((((9H-fluoren-9-yl)methoxy)carbonyl)amino)-3-methylbutanamido)propanamido)-2-iodobenzoate

A flask was charged with Example 2.135.2 (337 mg) and Example 2.123.14 (500 mg). Ethyl acetate (18 mL) was added followed by pyridine (0.296 mL). The resulting suspension was chilled in an ice bath, and 2,4,6-tripropyl-1,3,5,2,4,6-trioxatriphosphinane 2,4,6-trioxide (50% mixture in ethyl acetate, 1.4 mL) was added dropwise. Stirring was continued at 0 °C for 45 minutes, and the reaction was placed in a -20°C freezer overnight. The reaction was allowed to warm to room temperature and was quenched with water. The layers were separated, and the aqueous layer was extracted twice more with ethyl acetate. The combined extracts were dried with anhydrous sodium sulfate, filtered and concentrated. The residue was dissolved in dichloromethane and diluted with diethyl ether to precipitate the title compound, which was collected by filtration. MS (ESI) m/e 669.7 (M+H)⁺.

### 2.135.4 (9H-fluoren-9-yl)methyl ((S)-1-(((S)-1-((4-(hydroxymethyl)-3-iodophenyl)amino)-1-oxopropan-2-yl)amino)-3-methyl-1-oxobutan-2-yl)carbamate

Example 2.54.3 (1 g) was dissolved in tetrahydrofuran (15 mL), and the mixture was chilled to -15 °C in an ice-acetone bath. Lithium aluminum hydride (IN in tetrahydrofuran, 3 mL) was then added dropwise, keeping the temperature below -10 °C. The reaction was stirred for 1 hour and carefully quenched with 10% citric acid (25 mL). The layers were separated, and the aqueous layer was extracted thrice with ethyl acetate. The combined organic layers were washed with water and brine, dried over anhydrous sodium sulfate, filtered and concentrated. The residue was adsorbed onto silica gel and purified by flash chromatography, eluting with a gradient of 5% to 6% methanol in dichloromethane, to give the title compound. MS (ESI) m/e 664.1 (M+H)⁺.

### 2.135.5 methyl 5-(5-((S)-2-((S)-2-((((9H-fluoren-9-yl)methoxy)carbonyl)amino)-3-methylbutanamido)propanamido)-2-(hydroxymethyl)phenyl)pent-4-ynoate

To a stirred mixture of methyl pent-4-ynoate (50 mg), Example 2.135.4 (180 mg) and N,N-diisopropylethylamine (0.15 mL) in N,N-dimethylformamide (2 mL) was added bis(triphenylphosphine)palladium(II) dichloride (20 mg) and copper iodide (5 mg). The mixture was purged with nitrogen three times and stirred at room temperature overnight. The reaction was diluted with ethyl acetate and washed with water and brine. The aqueous layers were back extracted with ethyl acetate. The combined organic layers were dried over sodium sulfate, filtered and concentrated. The residue was purified by reverse-phase HPLC on a Gilson system, eluting with 20-90% acetonitrile in water containing 0.1% v/v trifluoroacetic acid. The desired fractions were combined and freeze-dried to provide the title compound. MS (ESI) m/e 608.0 (M-H₂O)⁺.

### 2.135.6 methyl 5-(5-((S)-2-((S)-2-((((9H-fluoren-9-yl)methoxy)carbonyl)amino)-3-methylbutanamido)propanamido)-2-(hydroxymethyl)phenyl)pentanoate

A mixture of Example 2.135.5 (0.084 g) and 10% Pd/C (0.02 g) in tetrahydrofuran (5 mL) was stirred at 20 °C under an atmosphere of 50 psi H₂ for 1 hour. The reaction mixture was filtered through diatomaceous earth, and the solvent was evaporated under reduced pressure to provide the title compound. MS (ESI) m/e 612.0 (M-H₂O)⁺.

### 2.135.7 methyl 5-(5-((S)-2-((S)-2-((((9H-fluoren-9-yl)methoxy)carbonyl)amino)-3-methylbutanamido)propanamido)-2-((((4-nitrophenoxy)carbonyl)oxy)methyl)phenyl)pentanoate

Example 2.135.7 was prepared by substituting Example 2.135.7 for Example 2.55.6 in Example 2.55.7. MS (ESI) m/e 795.4 (M+H)⁺.

### 2.135.8 3-(1-((3-(2-((((4-((S)-2-((S)-2-amino-3-methylbutanamido)propanamido)-2-(4-carboxybutyl)benzyl)oxy)carbonyl)(2-sulfoethyl)amino)ethoxy)-5,7-dimethyladamantan-1-yl)methyl)-5-methyl-1H-pyrazol-4-yl)-6-(8-(benzo[d]thiazol-2-ylcarbamoyl)-3,4-dihydroisoquinolin-2(1H)-yl)picolinic acid

Example 2.135.8 was prepared by substituting 2.135.7 for (9H-fluoren-9-yl)methyl ((S)-3-methyl-1-(((S)-1-((4-((((4-nitrophenoxy)carbonyl)oxy)methyl)phenyl)amino)-1-oxo-5-ureidopentan-2-yl)amino)-1-oxobutan-2-yl)carbamate in Example 2.49.1. MS (ESI) m/e 1271.4 (M-H)⁻.

### 2.135.9 N-[(2,5-dioxo-2,5-dihydro-1H-pyrrol-1-yl)acetyl]-L-valyl-N-{4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.13,7]dec-1-yl}oxy)ethyl](2-sulfoethyl)carbamoyl}oxy)methyl]-3-(4-carboxybutyl)phenyl}-L-alaninamide

Example 2.135.9 was prepared by substituting 2.135.8 for Example 2.49.1 in Example 2.54. ¹H NMR (400 MHz, dimethyl sulfoxide-d₆) δ ppm 9.88 (d, 1H), 8.3 - 8.2 (m, 2H), 8.01 (dd, 1H), 7.77 (d, 1H), 7.59 (dd, 1H), 7.52 (dd, 1H), 7.47 - 7.29 (m, 8H), 7.23 - 7.18 (m, 1H), 7.05 (s, 2H), 6.95 (d, 1H), 5.00 (d, 2H), 4.94 (s, 2H), 4.37 (p, 1H), 3.51 - 3.28 (m, 5H), 3.26 - 3.14 (m, 2H), 2.99 (t, 2H), 2.65 (t, 2H), 2.57 (s, 2H), 2.26 - 2.17 (m, 3H), 2.07 (d, 3H), 1.94 (dd, 1H), 1.61 - 0.69 (m, 35H). MS (ESI) m/e 1408.5 (M-H)⁺.

### 2.136 Synthesis of 2-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)methyl]-5-(3-{[(2,5-dioxo-2,5-dihydro-1H-pyrrol-1-yl)acetyl]amino}propyl)phenyl beta-D-glucopyranosiduronic acid (Synthon UV)

### 2.136.1 (3R,4S,5S,6S)-2-(5-(3-((((9H-fluoren-9-yl)methoxy)carbonyl)amino)prop-1-yn-1-yl)-2-formylphenoxy)-6-(methoxycarbonyl)tetrahydro-2H-pyran-3,4,5-triyl triacetate

Example 2.136.1 was prepared by substituting (9H-fluoren-9-yl)methyl prop-2-yn-1-ylcarbamate for 2.124.1A in Example 2.124.2. MS (ESI) m/e 714.1 (M+H)⁺.

### 2.136.2 (2S,3R,4S,5S,6S)-2-(5-(3-((((9H-fluoren-9-yl)methoxy)carbonyl)amino)propyl)-2-formylphenoxy)-6-(methoxycarbonyl)tetrahydro-2H-pyran-3,4,5-triyl triacetate

Example 2.136.2 was prepared by substituting 2.136.1 for 2.124.2 in Example 2.124.3. MS (ESI) m/e 718.5 (M+H)⁺.

### 2.136.3 (2S,3R,4S,5S,6S)-2-(5-(3-((((9H-fluoren-9-yl)methoxy)carbonyl)amino)propyl)-2-(hydroxymethyl)phenoxy)-6-(methoxycarbonyl)tetrahydro-2H-pyran-3,4,5-triyl triacetate

Example 2.136.3 was prepared by substituting 2.136.2 for 2.124.3 in Example 2.124.4. MS (ESI) m/e 742.2 (M+Na)⁺.

### 2.136.4 (2S,3R,4S,5S,6S)-2-(5-(3-((((9H-fluoren-9-yl)methoxy)carbonyl)amino)propyl)-2-((((4-nitrophenoxy)carbonyl)oxy)methyl)phenoxy)-6-(methoxycarbonyl)tetrahydro-2H-pyran-3,4,5-triyl triacetate

Example 2.136.4 was prepared by substituting 2.136.3 for 2.124.4 in Example 2.124.5. MS (ESI) m/e 885.2 (M+Na)⁺.

### 2.136.5 3-(1-((3-(2-((((4-(3-aminopropyl)-2-(((3R,4S,5S,6S)-6-carboxy-3,4,5-trihydroxytetrahydro-2H-pyran-2-yl)oxy)benzyl)oxy)carbonyl)(2-sulfoethyl)amino)ethoxy)-5,7-dimethyladamantan-1-yl)methyl)-5-methyl-1H-pyrazol-4-yl)-6-(8-(benzo[d]thiazol-2-ylcarbamoyl)-3,4-dihydroisoquinolin-2(1H)-yl)picolinic acid

Example 2.136.5 was prepared by substituting Example 2.136.4 for (9H-fluoren-9-yl)methyl ((S)-3-methyl-1-(((S)-1-((4-((((4-nitrophenoxy)carbonyl)oxy)methyl)phenyl)amino)-1-oxo-5-ureidopentan-2-yl)amino)-1-oxobutan-2-yl)carbamate in Example 2.49.1. MS (ESI) m/e 1237.7 (M+H)⁺.

### 2.136.6 2-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.13,7]dec-1-yl}oxy)ethyl](2-sulfoethyl)carbamoyl}oxy)methyl]-5-(3-{[(2,5-dioxo-2,5-dihydro-1H-pyrrol-1-yl)acetyl]amino}propyl)phenyl beta-D-glucopyranosiduronic acid

Example 2.136.6 was prepared by substituting Example 2.136.5 for Example 2.49.1 in Example 2.54. ¹H NMR (400 MHz, dimethyl sulfoxide-*d₆*) δ ppm 8.14 (d, 1H), 8.01 (d, 1H), 7.59 (d, 1H), 7.53 - 7.39 (m, 4H), 7.38 - 7.28 (m, 3H), 7.22 - 7.15 (m, 2H), 7.13 - 6.91 (m, 5H), 6.84 (d, 1H), 5.17 - 4.91 (m, 5H), 3.35-3.2 (m, 4H), 3.10-2.90 (m, 4H), 2.75-2.65 (m, 2H), 2.08 (s, 3H), 1.65 (s, 2H), 1.39 - 0.71 (m, 21H). MS (ESI) m/e 1372.3 (M-H)⁻.

### 2.137 Synthesis of 6-[8-(1,3-benzothiazol-2-ylcarbamoyl)-3,4-dihydroisoquinolin-2(1H)-yl]-3-{1-[(3-{2-[({[2-{[(2S,3R,4S,5S,6S)-6-carboxy-3,4,5-trihydroxytetrahydro-2H-pyran-2-yl]oxy}-4-(4-{[(2,5-dioxo-2,5-dihydro-1H-pyrrol-1-yl)acetyl]amino}butyl)benzyl]oxy}carbonyl)(3-{[1,3-dihydroxy-2-(hydroxymethyl)propan-2-yl]amino}-3-oxopropyl)amino]ethoxy}-5,7-dimethyltricyclo[3.3.1.1^{3,7}]dec-1-yl)methyl]-5-methyl-1H-pyrazol-4-yl}pyridine-2-carboxylic acid (Synthon UZ)

### 2.137.1 3-(1-((3-(2-((((4-(4-aminobutyl)-2-(((2R,3S,4R,5R,6R)-6-carboxy-3,4,5-trihydroxytetrahydro-2H-pyran-2-yl)oxy)benzyl)oxy)carbonyl)(3-((1,3-dihydroxy-2-(hydroxymethyl)propan-2-yl)amino)-3-oxopropyl)amino)ethoxy)-5,7-dimethyladamantan-1-yl)methyl)-5-methyl-1H-pyrazol-4-yl)-6-(8-(benzo[d]thiazol-2-ylcarbamoyl)-3,4-dihydroisoquinolin-2(1H)-yl)picolinic acid

The title compound was prepared as described in Example 2.124.6, replacing Example 1.87.3 with Example 1.84. MS (ESI) m/e 1319.4 (M-H)⁻.

### 2.137.2 6-[8-(1,3-benzothiazol-2-ylcarbamoyl)-3,4-dihydroisoquinolin-2(1H)-yl]-3-{1-[(3-{2-[({[2-{[(2S,3R,4S,5S,6S)-6-carboxy-3,4,5-trihydroxytetrahydro-2H-pyran-2-yl]oxy}-4-(4-{[(2,5-dioxo-2,5-dihydro-1H-pyrrol-1-yl)acetyl]amino}butyl)benzyl]oxy}carbonyl)(3-{[1,3-dihydroxy-2-(hydroxymethyl)propan-2-yl]amino}-3-oxopropyl)amino]ethoxy}-5,7-dimethyltricyclo[3.3.1.13,7]dec-1-yl)methyl]-5-methyl-1H-pyrazol-4-yl}pyridine-2-carboxylic acid

The title compound was prepared as described in Example 2.54, replacing Example 2.49.1 with Example 2.137.1. ¹H NMR (501 MHz, dimethyl sulfoxide-*d*₆) δ ppm 12.83 (s, 2H), 8.12 (s, 0H), 8.06 (s, 1H), 8.03 - 7.99 (m, 1H), 7.77 (d, 1H), 7.72 (s, 0H), 7.60 (d, 1H), 7.52 - 7.39 (m, 3H), 7.34 (td, 2H), 7.26 (s, 1H), 7.21 - 7.11 (m, 2H), 7.05 (s, 2H), 6.93 (d, 2H), 6.83 (d, 1H), 5.09 (d, 2H), 5.00 (d, 1H), 4.94 (s, 2H), 4.12 (t, 1H), 3.97 (s, 2H), 3.87 (q, 4H), 3.79 (d, 2H), 3.29 (q, 2H), 3.12 - 2.93 (m, 5H), 2.47 - 2.23 (m, 1H), 2.07 (d, 3H), 1.50 (d, 3H), 1.36 (d, 5H), 1.31 - 0.85 (m, 9H), 0.81 (d, 7H). MS (ESI) m/e 1568.4 (M-H)⁻.

### 2.138 Synthesis of 6-(8-(benzo[d]thiazol-2-ylcarbamoyl)naphthalen-2-yl)-3-(1-((3-(2-((((2-(((2S,3R,4S,5S,6S)-6-carboxy-3,4,5-trihydroxytetrahydro-2H-pyran-2-yl)oxy)-4-(4-(2-((3S,5S)-3-(2,5-dioxo-2,5-dihydro-1H-pyrrol-1-yl)-2-oxo-5-((2-sulfoethoxy)methyl)pyrrolidin-1-yl)acetamido)butyl)benzyl)oxy)carbonyl)(2-sulfoethyl)amino)ethoxy)-5,7-dimethyladamantan-1-yl)methyl)-5-methyl-1H-pyrazol-4-yl)picolinic acid (Synthon VB)

The title compound was prepared by substituting Example 2.133.1 for Example 2.119.16 in Example 2.119.17. ¹H NMR (400 MHz, dimethyl sulfoxide-*d₆*) δ ppm 8.99 (s, 1H), 8.34 (dd, 1H), 8.19 (d, 1H), 8.17 (d, 1H), 8.13 (d, 1H), 8.04 (d, 1H), 7.97 (d, 1H), 7.93 (d, 1H), 7.80 (br t, 1H), 7.77 (d, 1H), 7.67 (dd, 1H), 7.45 (s, 1H), 7.45 (dd, 1H), 7.34 (dd, 1H), 7.14 (d, 1H), 7.03 (s, 2H), 6.93 (s, 1H), 6.82 (br d, 1H), 5.06 (br m, 2H), 4.98 (d, 1H), 4.67 (t, 1H), 4.02 (d, 2H), 3.85 (m, 3H), 3.71 (br m, 1H), 3.59 (t, 2H), 3.45 (br m, 3H), 3.41 (m, 4H), 3.27 (m, 4H), 3.03 (m, 2H), 2.70 (m, 2H) 2.65 (br m, 2H), 2.50 (br t, 2H), 2.31 (br m, 1H), 2.19 (s, 3H), 1.80 (m, 1H), 1.52 (br m, 2H), 1.38 (m, 2H), 1.35 (br m, 2H), 1.29-0.88 (br m, 10H), 0.82 (s, 3H), 0.80 (s, 3H). MS (ESI) m/e 1602.4 (M-H)⁻.

### 2.139 Synthesis of 2-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl][3-hydroxy-2-(hydroxymethyl)propyl]carbamoyl}oxy)methyl]-5-(3-{[(2,5-dioxo-2,5-dihydro-1H-pyrrol-1-yl)acetyl]amino}propyl)phenyl beta-D-glucopyranosiduronic acid (Synthon VC)

### 2.139.1 3-(1-((3-(2-((((4-(3-aminopropyl)-2-(((2S,3R,4S,5S,6S)-6-carboxy-3,4,5-trihydroxytetrahydro-2H-pyran-2-yl)oxy)benzyl)oxy)carbonyl)(3-hydroxy-2-(hydroxymethyl)propyl)amino)ethoxy)-5,7-dimethyladamantan-1-yl)methyl)-5-methyl-1H-pyrazol-4-yl)-6-(8-(benzo[d]thiazol-2-ylcarbamoyl)-3,4-dihydroisoquinolin-2(1H)-yl)picolinic acid

Example 2.139.1 was prepared by substituting Example 2.136.4 for (9H-fluoren-9-yl)methyl ((S)-3-methyl-1-(((S)-1-((4-((((4-nitrophenoxy)carbonyl)oxy)methyl)phenyl)amino)-1-oxo-5-ureidopentan-2-yl)amino)-1-oxobutan-2-yl)carbamate and substituting Example 1.79.3 for Example 1.2.9 in Example 2.49.1. MS (ESI) m/e 1217.7 (M+H)⁺.

### 2.139.2 2-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.13,7]dec-1-yl}oxy)ethyl] [3-hydroxy-2-(hydroxymethyl)propyl]carbamoyl}oxy)methyl]-5-(3-{[(2,5-dioxo-2,5-dihydro-1H-pyrrol-1-yl)acetyl]amino}propyl)phenyl beta-D-glucopyranosiduronic acid

Example 2.139.1 was prepared by substituting Example 2.139.1 for Example 2.49.1 in Example 2.54. ¹H NMR (400 MHz, dimethyl sulfoxide-*d₆*) δ ppm 12.84 (s, 2H), 8.11 (t, 1H), 8.00 (dd, 1H), 7.76 (d, 1H), 7.62 - 7.56 (m, 1H), 7.50 - 7.37 (m, 3H), 7.37 - 7.29 (m, 2H), 7.25 (s, 1H), 7.16 (d, 1H), 7.04 (s, 2H), 6.96 - 6.88 (m, 2H), 6.82 (d, 1H), 5.06 (s, 2H), 4.98 (d, 1H), 4.92 (s, 2H), 3.97 (s, 2H), 3.44 - 3.18 (m, 11H), 3.07 - 2.90 (m, 4H), 2.05 (s, 3H), 1.80 (s, 1H), 1.64 (p, 2H), 1.38 - 0.67 (m, 19H). (m, 21H). MS (ESI) m/e 1352.5 (M-H)⁻.

### 2.140 Synthesis of N-({(3S,5S)-3-(2,5-dioxo-2,5-dihydro-1H-pyrrol-1-yl)-2-oxo-5-[(2-sulfoethoxy)methyl]pyrrolidin-1-yl}acetyl)-L-valyl-N-{4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)methyl] -3-(2,5,8,11,14,17,20,23,26,29,32,35,38,41,44,47,50-heptadecaoxatripentacont-52-yn-53-yl)phenyl}-L-alaninamide(Synthon VS)

### 2.140.1 2-iodo-4-nitrobenzoic acid

2-Amino-4-nitrobenzoic acid (50 g) was added to a mixture of concentrated H₂SO₄ (75 mL) and water (750 mL) at 0 °C, and the mixture was stirred for 1 hour. To the mixture was added a mixture of sodium nitrite (24.62 g) in water (300 mL) dropwise at 0 °C. The resulting mixture was stirred at 0 °C for 3 hours. A mixture of sodium iodide (65.8 g) in water (300 mL) was added to above mixture slowly. After the completion of the addition, the resulting mixture was stirred at 0 °C for 2 hours, then at room temperature for 16 hours and at 60 °C for 2 hours. The resulting mixture was cooled to room temperature and diluted with ice-water (300 mL). The solid was collected by filtration, washed by water (100 mL x 5), and dried in air for 16 hours to give the title compound. MS (LC-MS) m/e 291.9 (M-H)⁻.

### 2.140.2 methyl 2-iodo-4-nitrobenzoate

A mixture of Example 2.140.1 (130 g) in a mixture of methanol (1000 mL) and sulfuric acid (23.65 mL) was stirred at 85 °C for 16 hours and concentrated to dryness. The residue was triturated with methanol (100 mL) and the suspension was stirred for 10 minutes. The solid was collected by filtration, washed with water (200 mL x 3) and methanol (20 mL), and air-dried for 16 hours to give the title compound. MS (LC-MS) m/e 308.0 (M+H)⁺.

### 2.140.3 methyl 4-amino-2-iodobenzoate

To a mixture of ammonium chloride (122 g) and iron (38.2 g) in ethanol (1000 mL) and water (100 mL) was added Example 2.140.2 (70 g,) at room temperature. The mixture was stirred at 80 °C for 4 hours and filtered to remove insoluble material. The filtrate was concentrated under reduced pressure. The residue was dissolved in ethyl acetate (1000 mL) and washed with water (500 mL). The aqueous phase was extracted with ethyl acetate (1000 mL x 2). The combined organic phase was washed with brine, dried over MgSO₄, filtered and concentrated to give the title compound. MS (LC-MS) m/e 278.0 (M+H)⁺.

### 2.140.4 (4-amino-2-iodophenyl)methanol

To a mixture of Example 2.140.3 (40 g) in tetrahydrofuran (800 mL) was added 1M diisobutylaluminum hydride (505 mL) dropwise at -50 °C. The mixture was stirred at -50 °C for 3 hours and cooled to -20 °C. Ice-water (180 mL) was added dropwise (keeping temperature below 0 °C) to the mixture. After the addition of ice-water, the mixture was stirred for 10 minutes and filtered. The filtrate was concentrated, and the residue was dissolved in ethyl acetate (800 mL) and water (200 mL). The aqueous phase was extracted with ethyl acetate (300 mL x 2). The combined organic phases were washed with brine, dried over MgSO₄, filtered and concentrated to give the title compound. MS (LC-MS) m/e 250.0 (M+H)⁺.

### 2.140.5 4-(((tert-butyldimethylsilyl)oxy)methyl)-3-iodoaniline

To a mixture of Example 2.140.4 (40 g) and imidazole (21.87 g) in dichloromethane (600 mL) and tetrahydrofuran (150 mL) was added tert-butyldimethylchlorosilane (29.0 g). The mixture was stirred at room temperature for 16 hours and filtered to remove the solid. To the filtrate was added ice-water (50 mL). The mixture was stirred for 10 minutes and water (100 mL) was added. The mixture was extracted with dichloromethane (500 mL x 2). The combined organic phases were washed with brine, dried over MgSO₄, filtered and concentrated. The residue was purified by silica gel chromatography, eluting with 15/1 to 10/1 petroleum ether/ethyl acetate, to give the title compound. MS (LC-MS) m/e 364.0 (M+H)⁺.

### 2.140.6 (S)-tert-butyl (1-((4-(((tert-butyldimethylsilyl)oxy)methyl)-3-iodophenyl)amino)-1-oxopropan-2-yl)carbamate

To a mixed mixture of (S)-2-((tert-butoxycarbonyl)amino)propanoic acid (15.62 g) and Example 2.140.5 (30 g) in dichloromethane (600 mL) at 0 °C was added POCl₃ (15.39 mL) dropwise. The mixture was stirred at 0 °C for 2 hours. Ice-water (60 mL) was carefully added to the mixture dropwise (keeping temperature below 5 °C). The mixture was stirred for 30 minutes and concentrated to remove dichloromethane. The residue was suspended in ethyl acetate (500 mL) and water (100 mL). The suspension was filtered. The organic phase was washed by water (200 mL x 2) and brine, dried over MgSO₄, filtered and concentrated to give the title compound. MS (LC-MS) m/e 533.0 (M-H) ⁺.

### 2.140.7 (S)-tert-butyl (1-((4-(hydroxymethyl)-3-iodophenyl)amino)-1-oxopropan-2-yl)carbamate

To a mixture of Example 2.140.6 (60 g) in tetrahydrofuran (600 mL) was added tetrabutyl ammonium fluoride (28.2 g) in tetrahydrofuran (120 mL) at 0 °C. The mixture was stirred at room temperature for 16 hours and filtered. To the filtrate was added water (100 mL). The mixture was stirred for 10 minutes and then concentrated. The residue was diluted with ethyl acetate (800 mL) and water (300 mL). The aqueous phase was extracted with ethyl acetate (200 mL x 3). The combined organic phases were washed with brine, dried over MgSO₄, filtered and concentrated. The residue was purified by silica gel chromatography, eluting with 3/1 to 1/1 petroleum ether/ethyl acetate, to give the title compound. MS (LC-MS) m/e 443.0 (M+Na)⁺.

### 2.140.8 (S)-2-amino-N-(4-(hydroxymethyl)-3-iodophenyl)propanamide

A mixture of Example 2.140.7 (20 g) in a mixture of dichloromethane (80 mL) and trifluoroacetic acid (40 mL) was stirred at room temperature for 2 hours and concentrated. The residue was dissolved in dichloromethane (80 mL) and triethylamine (16.95 mL) was added to adjust the pH to 8. The title compound was obtained as free base in dichloromethane, which was used in next step without further purification. MS (LC-MS) m/e 321.1 (M+H)⁺.

### 2.140.9 tert-butyl ((S)-1-(((S)-1-((4-(hydroxymethyl)-3-iodophenyl)amino)-1-oxopropan-2-yl)amino)-3-methyl-1-oxobutan-2-yl)carbam ate

A mixture of (S)-2-((tert-butoxycarbonyl)amino)-3-methylbutanoic acid (6.79 g), triethylamine (9.58 mL) and 1-hydroxybenzotriazole hydrate (5.26 g) in dichloromethane (250 mL) was stirred for 20 minutes. The resulting mixture was added to a mixture of Example 2.140.8 (10 g) and 1-ethyl-3-[3-(dimethylamino)propyl]-carbodiimide hydrochloride (6.59 g) in dichloromethane (100 mL) at 0 °C, dropwise. After the completion of addition, the mixture was stirred at 0 °C for 2 hours. Ice-water (200 mL) was added, and the resulting mixture was stirred for 20 minutes. The organic phase was washed with saturated aqueous sodium bicarbonate mixture (100 mL x 2), water (100 mL x 2) and brine (100 mL), dried over MgSO₄, filtered and concentrated. The residue was purified by silica gel chromatography, eluting with 3/1 to 1/1 petroleum ether/ethyl acetate, to give the title compound. LC-MS m/e 542.1 (M+Na)⁺.

### 2.140.10 tert-butyl ((S)-1-(((S)-1-((4-(hydroxymethyl)-3-(2,5,8,11,14,17,20,23,26,29,32,35,38,41,44,47,50-heptadecaoxatripentacont-52-yn-53-yl)phenyl)amino)-1-oxopropan-2-yl)amino)-3-methyl-1-oxobutan-2-yl)carbamate

To a mixture of Example 2.140.9 (50 mg), 2,5,8,11,14,17,20,23,26,29,32,35,38,41,44,47,50-heptadecaoxatripentacont-52-yne (149 mg), bis(triphenylphosphine)palladium(II) dichloride (27.0 mg) and N,N-diisopropylethylamine (0.05 mL) in N,N-dimethylformamide (1 mL) was added copper(I) iodide (3.67 mg). The reaction was purged with a stream of nitrogen gas for 10 minutes and stirred overnight. The reaction was diluted with dimethyl sulfoxide purified by reverse-phase HPLC on a Gilson system (C18 column), eluting with 20-70% acetonitrile in water containing 0.1% trifluoroacetic acid, to give the title compound. MS (LC-MS) m/e 1164.2 (M-H)⁻.

### 2.140.11 tert-butyl ((S)-3-methyl-1-(((S)-1-((4-((((4-nitrophenoxy)carbonyl)oxy)methyl)-3-(2,5,8,11,14,17,20,23,26,29,32,35,38,41,44,47,50-heptadecaoxatripentacont-52-yn-53-yl)phenyl)amino)-1-oxopropan-2-yl)amino)-1-oxobutan-2-yl)carbamate

To a mixture of Example 2.140.10 (80 mg) and bis(4-nitrophenyl) carbonate (31.3 mg) in N,N-dimethylformamide (0.2 mL) was added N,N-diisopropylethylamine (0.06 mL). The mixture was stirred 3 hours and was purified by reverse-phase HPLC on a Gilson system (C18 column), eluting with 35-75% acetonitrile in water containing 0.1% trifluoroacetic acid, to give the title compound.

### 2.140.12 6-(8-(benzo[d]thiazol-2-ylcarbamoyl)-3,4-dihydroisoquinolin-2(1H)-yl)-3-(1-((3-(2-((((4-((S)-2-((S)-2-((tert-butoxycarbonyl)amino)-3-methylbutanamido)propanamido)-2-(2,5,8,11,14,17,20,23,26,29,32,35,38,41,44,47,50-heptadecaoxatripentacont-52-yn-53-yl)benzyl)oxy)carbonyl)(2-sulfoethyl)amino)ethoxy)-5,7-dimethyladamantan-1-yl)methyl)-5-methyl-1H-pyrazol-4-yl)picolinic acid

To a mixture of Example 1.2.9 (95 mg), Example 2.140.11 (148 mg) and 1-hydroxybenzotriazole hydrate (68.1 mg) in N,N-dimethylformamide (2.5 mL) was added N,N-diisopropylethylamine (97 µL). The mixture was stirred for 3.5 hours and purified by reverse-phase HPLC on a Gilson system (C18 column), eluting with 35-80% acetonitrile in water containing 0.1% trifluoroacetic acid, to give the title compound.

### 2.140.13 3-(1-((3-(2-((((4-((S)-2-((S)-2-amino-3-methylbutanamido)propanamido)-2-(2,5,8,11,14,17,20,23,26,29,32,35,38,41,44,47,50-heptadecaoxatripentacont-52-yn-53-yl)benzyl)oxy)carbonyl)(2-sulfoethyl)amino)ethoxy)-5,7-dimethyladamantan-1-yl)methyl)-5-methyl-1H-pyrazol-4-yl)-6-(8-(benzo[d]thiazol-2-ylcarbamoyl)-3,4-dihydroisoquinolin-2(1H)-yl)picolinic acid

A cold (0 °C) mixture of Example 2.140.12 (135 mg) in dichloromethane (4 mL) was treated with trifluoroacetic acid (1 mL) for 5 hours. The mixture was concentrated and purified by reverse-phase HPLC on a Gilson system (C18 column), eluting with 20-60% acetonitrile in water containing 0.1% trifluoroacetic acid, to give the title compound. MS (ESI) m/e 973.4 (M+2H)²⁺.

### 2.140.14 N-({(3S,5S)-3-(2,5-dioxo-2,5-dihydro-1H-pyrrol-1-yl)-2-oxo-5-[(2-sulfoethoxy)methyl]pyrrolidin-1-yl}acetyl)-L-valyl-N-14-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.13,7]dec-1-yl}oxy)ethyl](2-sulfoethyl)carbamoyl}oxy)methyl]-3-(2,5,8,11,14,17,20,23,26,29,32,35,38,41,44,47,50-heptadecaoxatripentacont-52-yn-53-yl)phenyl}-L-alaninamide

A mixture of Example 2.119.15 (20.88 mg) and O-(7-azabenzotriazol-1-yl)-N,N,N',N'-tetramethyluronium hexafluorophosphate (21.1 mg) in N,N-dimethylformamide (0.4 mL) was treated with N,N-diisopropylethylamine (16.2 µL) for 7 minutes, and a mixture of Example 2.140.13 (60 mg) and N,N-diisopropylethylamine (32.3 µL) in N,N-dimethylformamide (0.6 mL) was slowly added. The reaction mixture was stirred for 10 minutes and was purified by reverse-phase HPLC on a Gilson system (C18 column), eluting with 20-70% acetonitrile in water containing 0.1% trifluoroacetic acid, to give the title compound. 1H NMR (500 MHz, dimethyl sulfoxide-d6) δ 10.01 (d, 1H), 8.22 (d, 1H), 8.02 (t, 2H), 7.90 - 7.75 (m, 2H), 7.66 - 7.50 (m, 3H), 7.50 - 7.39 (m, 3H), 7.35 (q, 3H), 7.05 (s, 2H), 7.00 (d, 1H), 5.08 (d, 2H), 4.97 (s, 2H), 4.65 (t, 1H), 4.47 - 4.31 (m, 4H), 4.23 - 4.14 (m, 2H), 3.90 - 3.69 (m, 5H), 3.68 - 3.58 (m, 4H), 3.57 - 3.53 (m, 2H), 3.52 - 3.43 (m, 57H), 3.42 - 3.33 (m, 4H), 3.22 (s, 5H), 3.01 (t, 2H), 2.49 (p, 3H), 2.09 (d, 3H), 2.04 - 1.77 (m, 1H), 1.40 - 1.17 (m, 6H), 1.06 (dd, 6H), 0.97 - 0.63 (m, 11H). MS (ESI) m/e 1153.3 (M+2H)²⁺.

### 2.141 Synthesis of N-(1(3S,5S)-3-(2,5-dioxo-2,5-dihydro-1H-pyrrol-1-yl)-2-oxo-5-[(2-sulfoethoxy)methyl]pyrrolidin-1-yl}acetyl)-L-valyl-N-{4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)methyl]-3-(2,5,8,11,14,17,20,23,26,29,32,35,38,41,44,47,50-heptadecaoxatripentacontan-53-yl)phenyl}-L-alaninamide (Synthon VT)

### 2.141.1 tert-butyl ((S)-1-(((S)-1-((3-(2,5,8,11,14,17,20,23,26,29,32,35,38,41,44,47,50-heptadecaoxadopentacontan-52-yl)-4-(hydroxymethyl)phenyl)amino)-1-oxopropan-2-yl)amino)-3-methyl-1-oxobutan-2-yl)carbamate

A mixture of Example 2.140.10 (304 mg) and 10% Pd/C (90 mg, dry) in tetrahydrofuran (20 mL) was shaken in a pressure bottle for 2 hours under 50 psi of hydrogen gas. The insoluble material was filtered off, and the filtrate was concentrated to provide the title compound. MS (ESI) m/e 1168.3 (M-H)⁻.

### 2.141.2 tert-butyl ((S)-1-(((S)-1-((3-(2,5,8,11,14,17,20,23,26,29,32,35,38,41,44,47,50-heptadecaoxadopentacontan-52-yl)-4-((((4-nitrophenoxy)carbonyl)oxy)methyl)phenyl)amino)-1-oxopropan-2-yl)amino)-3-methyl-1-oxobutan-2-yl)carbamate

The title compound was prepared using the procedure in Example 2.140.11, replacing Example 2.140.10 with Example 2.141.1.

### 2.141.3 6-(8-(benzo[d]thiazol-2-ylcarbamoyl)-3,4-dihydroisoquinolin-2(1H)-yl)-3-(1-((3-(2-((((4-((S)-2-((S)-2-((tert-butoxycarbonyl)amino)-3-methylbutanamido)propanamido)-2-(2,5,8,11,14,17,20,23,26,29,32,35,38,41,44,47,50-heptadecaoxatripentacontan-53-yl)benzyl)oxy)carbonyl)(2-sulfoethyl)amino)ethoxy)-5,7-dimethyladamantan-1-yl)methyl)-5-methyl-1H-pyrazol-4-yl)picolinic acid

The title compound was prepared using the procedure in Example 2.140.12, replacing Example 2.140.11 with Example 2.141.2.

### 2.141.4 3-(1-((3-(2-((((4-((S)-2-((S)-2-amino-3-methylbutanamido)propanamido)-2-(2,5,8,11,14,17,20,23,26,29,32,35,38,41,44,47,50-heptadecaoxatripentacontan-53-yl)benzyl)oxy)carbonyl)(2-sulfoethyl)amino)ethoxy)-5,7-dimethyladamantan-1-yl)methyl)-5-methyl-1H-pyrazol-4-yl)-6-(8-(benzo[d]thiazol-2-ylcarbamoyl)-3,4-dihydroisoquinolin-2(1H)-yl)picolinic acid

The title compound was prepared using the procedure in Example 2.140.13, replacing Example 2.140.12 with Example 2.141.3. MS (ESI) m/e 1948.8 (M-H)⁻.

### 2.141.5 N-({(3S,5S)-3-(2,5-dioxo-2,5-dihydro-1H-pyrrol-1-yl)-2-oxo-5-[(2-sulfoethoxy)methyl]pyrrolidin-1-yl}acetyl)-L-valyl-N-{4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.13,7]dec-1-yl}oxy)ethyl](2-sulfoethyl)carbamoyl}oxy)methyl]-3-(2,5,8,11,14,17,20,23,26,29,32,35,38,41,44,47,50-heptadecaoxatripentacontan-53-yl)phenyl}-L-alaninamide

The title compound was prepared using the procedure in Example 2.140.14, replacing Example 2.140.13 with Example 2.141.4. ¹H NMR (501 MHz, dimethyl sulfoxide-*d*₆) δ 12.87 (s, 1H), 9.84 (s, 1H), 8.18 (d, 1H), 8.03 (dd, 2H), 7.78 (d, 1H), 7.61 (d, 1H), 7.52 (d, 1H), 7.45 (ddd, 4H), 7.40 - 7.32 (m, 2H), 7.30 (s, 1H), 7.22 (d, 1H), 7.07 (s, 2H), 6.96 (d, 1H), 5.01 (d, 2H), 4.95 (s, 2H), 4.64 (t, 1H), 4.38 (t, 1H), 4.24 - 4.12 (m, 2H), 4.00 (d, 1H), 3.88 (t, 2H), 3.78 (t, 3H), 3.64 (ddt, 2H), 3.49 (dd, 62H), 3.43 - 3.37 (m, 6H), 3.23 (s, 3H), 3.01 (t, 2H), 2.84 - 2.68 (m, 1.5H), 2.63 (dd, 4H), 2.36 (d, 0.5H), 2.08 (d, 3H), 1.74 (t, 2H), 1.25 (dt, 6H), 1.17 - 1.00 (m, 6H), 0.99 - 0.72 (m, 11H). MS (ESI) m/e 1153.0 (M-2H)²⁻.

### 2.142 Synthesis of 2-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl][(3S)-3,4-dihydroxybutyl]carbamoyl}oxy)methyl]-5-(3-{[(2,5-dioxo-2,5-dihydro-1H-pyrrol-1-yl)acetyl]amino}propyl)phenyl beta-D-glucopyranosiduronic acid (Synthon VY)

### 2.142.1 3-(1-((3-(2-((((4-(3-aminopropyl)-2-(((2S,3R,4S,5S,6S)-6-carboxy-3,4,5-trihydroxytetrahydro-2H-pyran-2-yl)oxy)benzyl)oxy)carbonyl)((S)-3,4-dihydroxybutyl)amino)ethoxy)-5,7-dimethyladamantan-1-yl)methyl)-5-methyl-1H-pyrazol-4-yl)-6-(8-(benzo[d]thiazol-2-ylcarbamoyl)-3,4-dihydroisoquinolin-2(1H)-yl)picolinic acid

Example 2.142.1 was prepared by substituting Example 2.136.4 for (9H-fluoren-9-yl)methyl ((S)-3-methyl-1-(((S)-1-((4-((((4-nitrophenoxy)carbonyl)oxy)methyl)phenyl)amino)-1-oxo-5-ureidopentan-2-yl)amino)-1-oxobutan-2-yl)carbamate and substituting Example 1.85 for Example 1.2.9 in Example 2.49.1. MS (ESI) m/e 1217.3 (M+H)⁺.

### 2.142.2 2-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.13,7]dec-1-yl}oxy)ethyl][(3S)-3,4-dihydroxybutyl]carbamoyl}oxy)methyl]-5-(3-{[(2,5-dioxo-2,5-dihydro-1H-pyrrol-1-yl)acetyl]amino}propyl)phenyl beta-D-glucopyranosiduronic acid

Example 2.142.2 was prepared by substituting Example 2.142.1 for Example 2.49.1 in Example 2.54. 1H NMR (400 MHz, dimethyl sulfoxide-d₆) δ ppm 8.14 (d, 1H), 8.03 (dt, 1H), 7.81 - 7.76 (m, 1H), 7.61 (dd, 1H), 7.53 - 7.41 (m, 3H), 7.38 - 7.32 (m, 2H), 7.28 (s, 1H), 7.18 (d, 1H), 7.06 (d, 2H), 6.97 - 6.92 (m, 2H), 6.85 (dd, 1H), 5.10 (q, 2H), 5.01 (d, 1H), 4.96 (s, 2H), 3.48 - 3.18 (m, 12H), 3.06 (q, 2H), 3.00 (t, 2H), 2.08 (s, 3H), 1.77 - 0.66 (m, 16H). MS (ESI) m/e 1352.5 (M-H)⁻.

### 2.143 Synthesis of 1-{[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl]({[4-(4-{[(2,5-dioxo-2,5-dihydro-1H-pyrrol-1-yl)acetyl]amino}butyl)-2-(beta-D-glucopyranuronosyloxy)benzyl]oxy}carbonyl)amino}-1,2-dideoxy-D-arabino-hexitol (Synthon WI)

### 2.143.1 3-(1-((3-(2-((((4-(4-aminobutyl)-2-(((2S,3R,4S,5S,6S)-6-carboxy-3,4,5-trihydroxytetrahydro-2H-pyran-2-yl)oxy)benzyl)oxy)carbonyl)((3R,4S,5R)-3,4,5,6-tetrahydroxyhexyl)amino)ethoxy)-5,7-dimethyladamantan-1-yl)methyl)-5-methyl-1H-pyrazol-4-yl)-6-(8-(benzo[d]thiazol-2-ylcarbamoyl)-3,4-dihydroisoquinolin-2(1H)-yl)picolinic acid

The title compound was prepared by substituting Example 1.77.2 for Example 1.25 and Example 2.124.5 for Example 2.97.7 in Example 2.97.8. MS (ESI) m/e 1291 (M+H)⁺, 1289 (M-H)⁻.

### 2.143.2 1-{[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.13,7]dec-1-yl}oxy)ethyl]({[4-(4-{[(2,5-dioxo-2,5-dihydro-1H-pyrrol-1-yl)acetyl]amino}butyl)-2-(beta-D-glucopyranuronosyloxy)benzyl]oxy}carbonyl) amino}-1,2-dideoxy-D-arabino-hexitol

The title compound was prepared by substituting Example 2.143.1 for Example 2.49.1 in Example 2.54. ¹H NMR (400 MHz, dimethyl sulfoxide-*d₆*) δ ppm 8.04 (d, 1H), 7.81 (d, 1H), 7.61 (d, 1H), 7.54-7.43 (m, 3H), 7.41-7.35 (m, 2H), 7.29 (s, 1H), 7.18 (m, 1H), 7.03 (s, 2H), 6.97 (d, 1H), 6.93 (s, 1H), 6.86 (d, 1H), 5.18-5.05 (m, 3H), 5.03 (d, 1H), 4.97 (s, 2H), 4.01 (s, 2H), 3.91 (d, 1H), 3.87 (t, 2H), 3.83 (m, 2H), 3.72 (s, 2H), 3.67 (m, 2H), 3.59 (dd, 2H), 3.50-3.27 (m, 16H), 3.14 (d, 2H), 3.04 (m, 4H), 2.09 (s, 3H), 1.68 (m, 2H), 1.52 (m, 2H), 1.44-1.31 (m, 4H), 1.26-1.14 (m, 4H), 1.10 (m, 4H), 0.98 (q, 2H), 0.85 (m, 6H). MS (ESI) m/e 1428 (M+H)⁺, 1426 (M-H)⁻.

### 2.144 Synthesis of 1-{[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl]({[4-(4-{[(2,5-dioxo-2,5-dihydro-1H-pyrrol-1-yl)acetyl]amino}butyl)-2-(beta-D-glucopyranuronosyloxy)benzyl]oxy} carbonyl)aminol-1,2-dideoxy-D-erythro-pentitol (Synthon WK)

### 2.144.1 3-(1-((3-(2-((((4-(4-aminobutyl)-2-(((2S,3R,4S,5S,6S)-6-carboxy-3,4,5-trihydroxytetrahydro-2H-pyran-2-yl)oxy)benzyl)oxy)carbonyl)((3S,4R)-3,4,5-trihydroxypentyl)amino)ethoxy)-5,7-dimethyladamantan-1-yl)methyl)-5-methyl-1H-pyrazol-4-yl)-6-(8-(benzo[d]thiazol-2-ylcarbamoyl)-3,4-dihydroisoquinolin-2(1H)-yl)picolinic acid

The title compound was prepared by substituting Example 1.80 for Example 1.25 and Example 2.124.5 for Example 2.97.7 in Example 2.97.8. MS (ESI) m/e 1261 (M+H)⁺, 1259 (M-H)⁻.

### 2.144.2 1-{[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.13,7]dec-1-yl}oxy)ethyl]({[4-(4-{[(2,5-dioxo-2,5-dihydro-1H-pyrrol-1-yl)acetyl] amino}butyl)-2-(beta-D-glucopyranuronosyloxy)benzyl]oxy} carbonyl)amino}-1,2-dideoxy-D-erythro-pentitol

The title compound was prepared by substituting Example 2.144.1 for Example 2.49.1 in Example 2.54. ¹H NMR (400 MHz, dimethyl sulfoxide-*d₆*) δ ppm 8.08 (t, 1H), 8.03 (d, 1H), 7.79 (d, 1H), 7.62 (d, 1H), 7.53-7.42 (m, 3H), 7.38-7.33 (m, 2H), 7.20 (s, 1H), 7.17 (m, 1H), 7.07 (s, 2H), 6.97-6.93 (m, 2H), 6.85 (d, 1H), 5.17-5.05 (m, 3H), 5.02 (d, 1H), 4.96 (s, 2H), 3.98 (s, 2H), 3.88 (m, 4H), 3.80 (m, 4H), 3.67 (m, 2H), 3.42 (m, 4H), 3.36-3.23 (m, 13H), 3.08-2.99 (m, 5H), 2.09 (s, 3H), 1.86 (m, 1H), 1.53 (m, 2H), 1.38 (m, 4H), 1.25 (m, 4H), 1.11 (m, 4H), 0.96 (m, 2H), 0.83 (m, 6H). MS (ESI) m/e 1398 (M+H)⁺, 1396 (M-H)⁻.

### 2.145 Synthesis of N-[(2,5-dioxo-2,5-dihydro-1H-pyrrol-1-yl)acetyl]-L-valyl-N-{4-[({[2-({3-[(4-{6-[8-(1,3-benzothiazol-2-ylcarbamoyl)naphthalen-2-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)methyl]-3-[27-(2,5,8,11,14,17,20,23-octaoxahexacosan-26-yl)-2,5,8,11,14,17,20,23-octaoxa-27-azatriacontan-30-yl]phenyl}-L-alaninamide (Synthon WP)

### 2.145.1 tert-butyl ((S)-1-(((S)-1-((3-(3-(((benzyloxy)carbonyl)amino)prop-1-yn-1-yl)-4-(hydroxymethyl)phenyl)amino)-1-oxopropan-2-yl)amino)-3-methyl-1-oxobutan-2-yl)carbamate

To a mixture of tert-butyl ((S)-1-(((S)-1-((4-(hydroxymethyl)-3-iodophenyl)amino)-1-oxopropan-2-yl)amino)-3-methyl-1-oxobutan-2-yl)carbamate (0.5 g) in N,N-dimethylformamide (6 mL) was added benzyl prop-2-yn-1-ylcarbamate (0.182 g), CuI (9.2 mg), bis(triphenylphosphine)palladium(II) dichloride (35 mg) and N,N-diisopropylethylamine (1.0 mL). The mixture was stirred at room temperature overnight. The mixture was concentrated under vacuum. The residue was dissolved in ethyl acetate (300 mL), washed with water, brine, dried over anhydrous sodium sulfate, filtered and concentrated. Evaporation of the solvent, and purification of the residue by silica gel chromatography, eluting with 30% ethyl acetate in dichloromethane, gave the title compound. MS (APCI) m/e 581.2 (M-H)⁻.

### 2.145.2 tert-butyl ((S)-1-(((S)-1-((3-(3-aminopropyl)-4-(hydroxymethyl)phenyl)amino)-1-oxopropan-2-yl)amino)-3-methyl-1-oxobutan-2-yl)carbamate

To a mixture of Example 2.145.1 (1.7g) in ethanol (30 mL) was added 5% Pd/C (0.3 g) and cyclohexene (large excess). The reaction was stirred at 100 °C for 45 minutes. The reaction was filtered and concentrated under reduced pressure. The residue was dissolved in N,N-dimethylformamide and purified by reverse-phase HPLC on a Gilson system (C18 column), eluting with 20-80% acetonitrile in water containing 0.1% trifluoroacetic acid, to give the title compound. MS (ESI) m/e 451.1(M-H)⁻.

### 2.145.3 tert-butyl ((S)-1-(((S)-1-((3-(27-(2,5,8,11,14,17,20,23-octaoxahexacosan-26-yl)-2,5,8,11,14,17,20,23-octaoxa-27-azatriacontan-30-yl)-4-(hydroxymethyl)phenyl)amino)-1-oxopropan-2-yl)amino)-3-methyl-1-oxobutan-2-yl)carbamate

To a mixture of Example 2.145.2 (45 mg) in dichloromethane (4 mL) was added 2,5,8,11,14,17,20,23-octaoxahexacosan-26-al (79 mg) followed by NaH(OAc)₃ (63.5 mg). The mixture was stirred at room temperature for 3 hours and then concentrated under reduced pressure. The residue was dissolved in N,N-dimethylformamide and purified by reverse-phase HPLC on a Gilson system (C18 column), eluting with 20-80% acetonitrile in water containing 0.1% trifluoroacetic acid, to give the title compound. MS (ESI) m/e 1212.1 (M-H)⁻.

### 2.145.4 tert-butyl ((S)-1-(((S)-1-((3-(27-(2,5,8,11,14,17,20,23-octaoxahexacosan-26-yl)-2,5,8,11,14,17,20,23-octaoxa-27-azatriacontan-30-yl)-4-((((4-nitrophenoxy)carbonyl)oxy) methyl)phenyl)amino)-1-oxopropan-2-yl)amino)-3-methyl-1-oxobutan-2-yl)carbam ate

To a mixture of Example 2.145.3 (80 mg) in N,N-dimethylformamide (2 mL) was added bis(4-nitrophenyl) carbonate (26 mg) followed by N,N-diisopropylamine (0.012 mL). The mixture was stirred at room temperature overnight and purified directly by reverse phase HPLC on a Gilson system (C18 column), eluting with 20-80% acetonitrile in water containing 0.1% trifluoroacetic acid, to give the title compound. MS (ESI) m/e 1376.97 (M-H)⁻.

### 2.145.5 3-(1-((3-(2-((((2-(27-(2,5,8,11,14,17,20,23-octaoxahexacosan-26-yl)-2,5,8,11,14,17,20,23-octaoxa-27-azatriacontan-30-yl)-4-((S)-2-((S)-2-amino-3-methylbutanamido)propanamido) benzyl)oxy)carbonyl)(2-sulfoethyl)amino)ethoxy)-5,7-dimethyladamantan-1-yl)methyl)-5-methyl-1H-pyrazol-4-yl)-6-(8-(benzo [d]thiazol-2-ylcarbamoyl)naphthalen-2-yl)picolinic acid

To a mixture of Example 2.145.4 (30 mg) in N,N-dimethylformamide (4 mL) was added Example 1.43 (18.68 mg) followed by 1-hydroxybenzotriazole hydrate (3.4 mg) and N,N-diisopropylamine (3.84 uL). The mixture was stirred at room temperature overnight. Trifluoroacetic acid (0.55 mL) was added to the mixture and stirred at room temperature for 3 hours. The mixture was purified by reverse-phase HPLC on a Gilson system (C18 column), eluting with 20-80% acetonitrile in water containing 0.1% trifluoroacetic acid, to give the title compound. MS (ESI) m/e 1986.6 (M-H)⁻.

### 2.145.6 N-[(2,5-dioxo-2,5-dihydro-1H-pyrrol-1-yl)acetyl]-L-valyl-N-{4-[({[2-({3-[(4-{6-[8-(1,3-benzothiazol-2-ylcarbamoyl)naphthalen-2-yl]-2-carboxypyridin-3-yl}-5-methyl-1H-pyrazol-1-yl)methyl]-5,7-dimethyltricyclo[3.3.1.13,7]dec-1-yl}oxy)ethyl](2-sulfoethyl)carbamoyl}oxy)methyl]-3-[27-(2,5,8,11,14,17,20,23-octaoxahexacosan-26-yl)-2,5,8,11,14,17,20,23-octaoxa-27-azatriacontan-30-yl]phenyl}-L-alaninamide

The title compound was prepared as described in Example 2.123.21, replacing Example 2.123.20 with Example 2.145.5. ¹H NMR (400 MHz, dimethyl sulfoxide-*d₆*) δ ppm 13.10 (s, 1H), 9.92 (s, 1H), 9.43 (s, 1H), 9.02 (s, 1H), 8.37 (dd, 1H), 8.30 - 8.14 (m, 5H), 8.07 (d, 1H), 8.02 (d, 1H), 7.96 (d, 1H), 7.81 (d, 1H), 7.74 - 7.68 (m, 1H), 7.57 (s, 1H), 7.52 - 7.45 (m, 2H), 7.42 - 7.34 (m, 2H), 7.28 (d, 1H), 7.08 (s, 2H), 5.05 (d, 2H), 4.39 (t, 1H), 4.21 (dd, 1H), 4.12 (s, 2H), 3.88 (s, 2H), 3.49 (d, 55H), 3.34 (s, 200H), 3.23 (s, 5H), 3.13 (d, 4H), 2.79 - 2.65 (m, 5H), 2.23 (s, 3H), 1.94 (d, 8H), 1.47 - 0.94 (m, 15H), 0.92 - 0.76 (m, 12H).

### 2.146 Synthesis of (6S)-2,6-anhydro-6-(2-{2-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)methyl]-5-({N-[(2S)-3-[3,4-bis(2,5,8,11,14,17,20,23,26,29,32-undecaoxatetratriacontan-34-yloxy)phenyl]-2-(2,5-dioxo-2,5-dihydro-1H-pyrrol-1-yl)propanoyl]-L-valyl-L-alanyl}amino)phenyl}ethyl)-L-gulonic acid (Synthon XD)

### 2.146.1 (S)-2-(((benzyloxy)carbonyl)amino)-3-(3,4-dihydroxyphenyl)propanoic acid

To a mixture of (S)-2-amino-3-(3,4-dihydroxyphenyl)propanoic acid (1.00 kg) and NaHCO₃ (1.28 kg) in dioxane (5.00 L) and water (5.00 L) was added benzyl carbonochloridate (1.04 k) dropwise. The reaction mixture was stirred at 25 °C for 12 hours. The reaction mixture was adjusted to pH = 3.0 ~ 4.0 by addition of 6 N aqueous HCl and extracted with ethyl acetate (25 L). The organic layer was dried over Na₂SO₄, filtered, and concentrated in vacuo to afford the title compound. ¹H NMR (400MHz, dimethyl sulfoxide-*d₆*) δ ppm 8.73 (s, 1H), 7.54-7.26 (m, 8H), 6.64-6.45 (m, 3H), 4.98 (s, 2H), 4.49 (s, 1H), 2.87 (d, *J* = 9.60 Hz, 1H), 2.68-2.62 (m, 1H).

### 2.146.2 (S)-benzyl 2-(((benzyloxy)carbonyl)amino)-3-(3,4-dihydroxyphenyl)propanoate

To a mixture of Example 2.146.1 (800.00 g) and Cs₂CO₃ (1.18 kg) was added bromomethylbenzene (259.67 g) at 20°C. The reaction mixture was stirred for 1 hour, and TLC showed the reaction was complete. The residue was diluted with H₂O (5 L) and extracted with ethyl acetate (three times 5 L). The combined organic layers were washed with brine (5 L), dried over Na₂SO₄ (150 g), filtered, and concentrated under reduce pressure. The residue was purified by column chromatography (SiO₂, petroleum ether/ethyl acetate = 100:1 to 1:1) twice to provide the title compound. ¹H NMR (400 MHz, CDCl₃) δ ppm 2.77 - 3.02 (m, 2 H), 4.47 (br. s., 1 H), 4.61 (d, *J*=7.94 Hz, 1 H), 5.01 - 5.17 (m, 4 H), 5.35 - 5.47 (m, 1 H), 6.32 (br. s., 1 H), 6.38 (d, *J*=7.94 Hz, 1 H), 6.51 (s, 1 H), 6.65 (d, *J*=7.94 Hz, 1 H), 7.17 - 7.42 (m, 9 H).

### 2.146.3 (S)-benzyl 2-(((benzyloxy)carbonyl)amino)-3-(3,4-bis(2,5,8,11,14,17,20,23,26,29,32-undecaoxatetratriacontan-34-yloxy)phenyl)propanoate

To a mixture of K₂CO₃ (27.04 g) and KI (5.95 g) in N,N-dimethylformamide (150 mL) was added Example 2.146.2 (8.12 g) and 2,5,8,11,14,17,20,23,26,29,32-undecaoxatetratriacontan-34-yl 4-methylbenzenesulfonate (27.00 g) in dimethylformamide (150 mL). The mixture was stirred at 75 °C for 12 hours under N₂. Two additional vials were set up as described above. All three reaction mixtures were combined for purification. The mixture was poured into NH₄Cl aqueous mixture (9 L), and extracted with ethyl acetate (five times with 900 mL). The combined organic layers were washed with brine (1500 mL), dried over Na₂SO₄ (150 g), filtered, and concentrated under reduce pressure to afford the crude residue. The residue was purified by column chromatography (SiO₂, dichloromethane/methanol=100/1 to 20:1) to provide the title compound. ¹H NMR (400 MHz, CDCl₃) δ ppm 2.95 - 3.08 (m, 2 H), 3.38 (s, 6 H), 3.57 - 3.68 (m, 80 H), 3.78 (t, *J*=4.85 Hz, 2 H), 3.83 (t, *J*=5.29 Hz, 2 H), 4.01 (t, *J*=5.07 Hz, 2 H), 4.10 (t, *J*=5.07 Hz, 2 H), 4.58 - 4.70 (m, 1 H), 5.09 (s, 2 H), 5.14 (d, *J*=3.53 Hz, 2 H), 6.55 (d, *J*=8.38 Hz, 1 H), 6.62 (d, *J*=1.76 Hz, 1 H), 6.74 (d, *J*=7.94 Hz, 1 H), 7.27 - 7.49 (m, 10 H).

### 2.146.4 (S)-2-amino-3-(3,4-bis(2,5,8,11,14,17,20,23,26,29,32-undecaoxatetratriacontan-34-yloxy)phenyl)propanoic acid

To a mixture of Example 2.146.3 (16.50 g) in methanol (200 mL) was added Pd/C (9.00 g), and the mixture was stirred at 50 °C under H₂ (50 psi) for 16 hours. An additional reaction was set up as described above. LC/MS showed the reaction was complete, and both reaction mixtures were combined for purification. The mixture was filtered and concentrated. The crude title compound was used in the next step without further purification.

### 2.146.5 (S)-3-(3,4-bis(2,5,8,11,14,17,20,23,26,29,32-undecaoxatetratriacontan-34-yloxy)phenyl)-2-(2,5-dioxo-2,5-dihydro-1H-pyrrol-1-yl)propanoic acid

To a mixture of Example 2.146.4 (5.94 g) in H₂O (60.00 mL) was added Na₂CO₃ (790.67 mg) and methyl 2,5-dioxopyrrole-1-carboxylate (1.19 g). The mixture was stirred at 25 °C for 3 hours. Four additional reactions were set up as described above. All five reaction mixtures were combined for purification. Aqueous 4M HCl was added to adjust the pH to 2. The combined mixture was purified by preparatory reverse-phase HPLC (trifluoroacetic acid conditions) to provide the title compound. ¹H NMR (400 MHz, CDCl₃) δ ppm 3.35 - 3.40 (m, 6 H), 3.51 - 3.58 (m, 4 H), 3.58 - 3.75 (m, 78 H), 3.81 (q, *J*=4.70 Hz, 4 H), 4.11 (dt, *J*=10.14, 5.07 Hz, 4 H), 4.91 (dd, *J*=11.47, 5.29 Hz, 1 H), 6.53 - 6.69 (m, 3 H), 6.71 - 6.89 (m, 2 H). MS (ESI) m/e6 38.0 (M+H)⁺.

### 2.146.6 (6S)-2,6-anhydro-6-(2-{2-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.13,7]dec-1-yl}oxy)ethyl](2-sulfoethyl)carbamoyl}oxy)methyl]-5-({N-[(2S)-3-[3,4-bis(2,5,8,11,14,17,20,23,26,29,32-undecaoxatetratriacontan-34-yloxy)phenyl]-2-(2,5-dioxo-2,5-dihydro-1H-pyrrol-1-yl)propanoyl]-L-valyl-L-alanyl}amino)phenyl}ethyl)-L-gulonic acid

A mixture of Example 2.146.5 (0.020 mL), O-(7-azabenzotriazol-1-yl)-N,N,N',N'-tetramethyluronium hexafluorophosphate (0.014 g) and N-ethyl-N-isopropylpropan-2-amine (0.020 mL) was stirred in N,N-dimethylformamide (0.4 mL) for 5 minutes. The mixture was added to a mixture of Example 2.123.20 (0.042 g) and N-ethyl-N-isopropylpropan-2-amine (0.020 mL) in N,N-dimethylformamide (0.4 mL) and it was stirred at room temperature for 3 hours. The reaction was diluted with a mixture of water (1.5 mL), N,N-dimethylformamide (0.5 mL) and 2,2,2-trifluoroacetic acid (0.054 mL) and purified by preparatory reverse-phase HPLC on a Gilson 2020 system, using a gradient of 5% to 85% acetonitrile/water. The product-containing fractions were lyophilized to give the title compound. ¹H NMR (501 MHz, dimethyl sulfoxide-*d₆*) δ 12.86 (s, 4H), 9.92 (s, 2H), 8.26 (d, 1H), 8.10 (s, 1H), 8.02 (dd, 1H), 7.77 (d, 1H), 7.64 (s, 1H), 7.54 - 7.49 (m, 1H), 7.49 - 7.39 (m, 2H), 7.39 - 7.31 (m, 2H), 7.28 (s, 1H), 7.20 (d, 1H), 6.94 (d, 1H), 6.87 (s, 2H), 6.77 (d, 1H), 6.60 - 6.53 (m, 1H), 5.05 - 4.91 (m, 5H), 4.80 (dd, 2H), 4.37 (t, 2H), 4.21 (t, 2H), 3.97 (dt, 3H), 3.86 (t, 3H), 3.78 (d, 3H), 3.68 (dt, 4H), 3.65 - 3.28 (m, 102H), 3.20 - 3.08 (m, 2H), 2.99 (t, 2H), 2.92 (d, 2H), 2.68 (dd, 2H), 2.07 (d, 4H), 1.54 (s, 2H), 1.37 - 0.71 (m, 16H). MS (ESI) m/e 2631.2 (M-H)⁻.

### 2.147 Synthesis of N-[(2,5-dioxo-2,5-dihydro-1H-pyrrol-1-yl)acetyl]-N-(2,5,8,11,14,17,20,23,26,29,32-undecaoxatetratriacontan-34-yl)-beta-alanyl-L-valyl-N-{4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)methyl]-3-(2,5,8,11,14,17,20,23,26,29,32,35,38,41,44,47,50-heptadecaoxatripentacontan-53-yl)phenyl}-L-alaninamide (Synthon XK)

### 2.147.1 benzyl 2,5,8,11,14,17,20,23,26,29,32-undecaoxa-35-azaoctatriacontan-38-oate

To a mixture of 2,5,8,11,14,17,20,23,26,29,32-undecaoxatetratriacontan-34-amine (1 g) in N,N-dimethylformamide (4 mL) and water (3 mL) was added benzyl acrylate (0.377 g), dropwise. The reaction mixture was stirred overnight purified by reverse-phase HPLC on a Gilson system (C18 column), eluting with 20-70% acetonitrile in water containing 0.1% trifluoroacetic acid, to give the title compound. MS (ESI) m/e 678.4 (M+H)⁺.

### 2.147.2 2,5,8,11,14,17,20,23,26,29,32-undecaoxa-35-azaoctatriacontan-38-oic acid

Example 2.147.1 (220 mg) and 10% Pd/C (44 mg, dry) in tetrahydrofuran (10 mL) was shaken in a pressure bottle for 1 hour under 50 psi of hydrogen gas. The reaction was filtered, and the filtrate was concentrated. The residue was dried under high vacuum to provide the title compound. MS (ESI) m/e 588.3 (M+H)⁺.

### 2.147.3 2,5-dioxopyrrolidin-1-yl 35-(2-(2,5-dioxo-2,5-dihydro-1H-pyrrol-1-yl)acetyl)-2,5,8,11,14,17,20,23,26,29,32-undecaoxa-35-azaoctatriacontan-38-oate

A cold (0 °C) mixture of 2,5-dioxopyrrolidin-1-yl 2-(2,5-dioxo-2,5-dihydro-1H-pyrrol-1-yl)acetate (566 mg), 1-hydroxybenzotriazole hydrate (229 mg), 1-hydroxypyrrolidine-2,5-dione (86 mg) and Example 2.147.2 (440 mg) in N,N-dimethylformamide (3mL) was treated with N,N-diisopropylethylamine (785 µL) for 25 minutes. The reaction was diluted with dimethyl sulfoxide and purified by reverse-phase HPLC on a Gilson system (C18 column), eluting with 5-55% acetonitrile in water containing 0.1% trifluoroacetic acid, to give the title compound. MS (ESI) m/e 822.3 (M+H)⁺.

### 2.147.4 N-[(2,5-dioxo-2,5-dihydro-1H-pyrrol-1-yl)acetyl]-N-(2,5,8,11,14,17,20,23,26,29,32-undecaoxatetratriacontan-34-yl)-beta-alanyl-L-valyl-N-{4-[({[2-({3-[(4-{6-[8-(1,3-benzothiazol-2-ylcarbarnoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.13,7]dec-1-yl}oxy)ethyl](2-sulfoethyl)carbamoyl}oxy)methyl]-3-(2,5,8,11,14,17,20,23,26,29,32,35,38,41,44,47,50-heptadecaoxatripentacontan-53-yl)phenyl}-L-alaninamide

To a cold (0 °C) mixture of Example 2.141.4 (28 mg), Example 2.147.3 (27.1 mg) and 1-hydroxybenzotriazole hydrate (6.6 mg) in N,N-dimethylformamide (0.8 mL) was added N,N-diisopropylethylamine-2 (20.1 µL). The mixture was stirred for 10 minutes and was purified by reverse-phase HPLC on a Gilson system (C18 column), eluting with 30-70% acetonitrile in water containing 0.1% trifluoroacetic acid, to give the title compound. ¹H NMR (400 MHz, dimethyl sulfoxide-*d₆*) δ 12.81 (s, 1H), 9.84 (s, 1H), 8.21 - 7.86 (m, 2H), 7.75 (d, 1H), 7.57 (d, 1H), 7.52 - 7.28 (m, 7H), 7.27 - 7.15 (m, 2H), 7.04 (d, 2H), 6.91 (d, 1H), 4.94 (d, 4H), 4.36 (dt, 3H), 4.19 (dt, 1H), 3.84 (t, 2H), 3.75 (d, 2H), 3.63 (d, 1H), 3.46 (dd, 104H), 3.36 (s, 2H), 3.19 (s, 5H), 2.97 (t, 2H), 2.57 (t, 5H), 2.42 - 2.26 (m, 1H), 2.03 (s, 7H), 2.00 - 1.83 (m, 1H), 1.70 (t, 2H), 1.38 - 0.96 (m, 13H), 0.96 - 0.69 (m, 13H). MS (ESI) m/e 1327.7 (M-2H)²⁻.

### 2.148 Synthesis of N-[(2,5-dioxo-2,5-dihydro-1H-pyrrol-1-yl)acetyl]-N-(2,5,8,11,14,17,20,23,26,29,32-undecaoxatetratriacontan-34-yl)-beta-alanyl-L-valyl-N-{4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)methyl]phenyl}-N⁵-carbamoyl-L-ornithinamide (Synthon XL)

The title compound was prepared using the procedure in Example 2.147.4, replacing Example 2.141.4 with Example 2.112.2. ¹H NMR (400 MHz, dimethyl sulfoxide-*d*₆) δ 12.83 (s, 1H), 9.96 (d, 1H), 8.18 - 7.85 (m, 3H), 7.75 (d, 1H), 7.64 - 7.37 (m, 7H), 7.32 (td, 2H), 7.28 - 7.20 (m, 3H), 7.04 (s, 2H), 6.92 (d, 1H), 5.17 - 4.79 (m, 4H), 4.59 - 4.31 (m, 3H), 4.21 (dt, 1H), 3.84 (t, 2H), 3.77 (d, 2H), 3.52 (s, 4H), 3.39 (d, 2H), 3.19 (s, 5H), 2.94 (dt, 4H), 2.60 (t, 3H), 2.43 - 2.27 (m, 1H), 2.05 (s, 4H), 1.60 (d, 2H), 1.44 - 0.57 (m, 22H). MS (ESI) m/e 1964.8 (M-H)⁻.

### 2.149 Synthesis of N-[(2,5-dioxo-2,5-dihydro-1H-pyrrol-1-yl)acetyl]-L-valyl-N-{4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)methyl]-3-[27-(2,5,8,11,14,17,20,23-octaoxahexacosan-26-yl)-2,5,8,11,14,17,20,23-octaoxa-27-azatriacontan-30-yl]phenyl}-L-alaninamide (Synthon YJ)

### 2.149.1 3-(1-((3-(2-((((2-(27-(2,5,8,11,14,17,20,23-octaoxahexacosan-26-yl)-2,5,8,11,14,17,20,23-octaoxa-27-azatriacontan-30-yl)-4-((S)-2-((S)-2-amino-3-methylbutanamido)propanamido)benzyl)oxy)carbonyl)(2-sulfoethyl)amino)ethoxy)-5,7-dimethyladamantan-1-yl)methyl)-5-methyl-1H-pyrazol-4-yl)-6-(8-(benzo[d]thiazol-2-ylcarbamoyl)-3,4-dihydroisoquinolin-2(1H)-yl)picolinic acid

The title compound was prepared as described in Example 2.145.5, replacing Example 1.43 with Example 1.2.9. MS (ESI) m/e 1991.4 (M-H)⁻.

### 2.149.2 N-[(2,5-dioxo-2,5-dihydro-1H-pyrrol-1-yl)acetyl]-L-valyl-N-{4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.13,7]dec-1-ylloxy)ethyl](2-sulfoethyl)carbamoyl}oxy)methyl]-3-[27-(2,5,8,11,14,17,20,23-octaoxahexacosan-26-yl)-2,5,8,11,14,17,20,23-octaoxa-27-azatriacontan-30-yl]phenyl}-L-alaninamide

The title compound was prepared as described in Example 2.145, replacing Example 2.145.5 with Example 2.149.1. ¹H NMR (400 MHz, dimethyl sulfoxide-*d*₆) δ ppm 12.83 (s, 1H), 9.90 (s, 1H), 9.41 (s, 1H), 8.24 (d, 2H), 8.01 (d, 1H), 7.77 (d, 1H), 7.67 - 7.29 (m, 8H), 7.26 (s, 2H), 7.06 (s, 2H), 6.93 (d, 1H), 5.03 (d, 2H), 4.93 (s, 2H), 4.37 (t, 1H), 4.19 (dd, 1H), 4.11 (s, 2H), 3.86 (t, 2H), 3.79 (s, 2H), 3.70 - 3.26 (m, 226H), 3.21 (s, 6H), 3.11 (s, 5H), 2.99 (t, 2H), 2.66 (d, 4H), 2.08 (s, 3H), 1.89 (s, 8H), 1.44 - 0.90 (m, 14H), 0.89 - 0.68 (m, 11H).

### 2.150 Synthesis of N-{(3S)-3-(2,5-dioxo-2,5-dihydro-1H-pyrrol-1-yl)-3-[1-(2,5,8,11,14,17,20,23,26,29,32,35-dodecaoxaheptatriacontan-37-yl)-1H-1,2,3-triazol-4-yl]propanoyl}-L-valyl-N-{4-[({[2-({3-[(4-{6-[8-(1,3-benzothiazol-2-ylcarbarnoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-ylloxy)ethyl](2-sulfoethyl)carbamoyl}oxy)methyl]phenyl}-N⁵-carbamoyl-L-ornithinamide (Synthon YQ)

### 2.150.1 3-(2,5-dioxo-2,5-dihydro-1H-pyrrol-1-yl)pent-4-ynoic acid

To a mixture of 3-aminopent-4-ynoic acid trifluoroacetic acid salt (1.9 g) in tetrahydrofuran (30 mL) was added methyl 2,5-dioxo-2,5-dihydro-1H-pyrrole-1-carboxylate (1.946 g), followed by the rapid addition of N,N-diisopropylethylamine (8.04 mL). The resulting mixture was stirred at 60 °C for 16 hours. The mixture was concentrated to dryness. The residue was purified by reverse-phase HPLC on a Gilson system (C18 column), eluting with 20-80% acetonitrile in water containing 0.1% trifluoroacetic acid, to give the title compound. MS (LC-MS) m/e 194 (M+H). ¹H-NMR (dimethyl sulfoxide-*d₆*, 400 MHz) δ 2.92-3.07 (m, 2H), 3.38 (d, 1H), 5.07-5.12 (m, 1H), 7.08 (s, 2H), 12.27 (bs, 0.6H).

### 2.150.2 3-(1-(2,5,8,11,14,17,20,23,26,29,32,35-dodecaoxaheptatriacontan-37-yl)-1H-1,2,3-triazol-4-yl)-3-(2,5-dioxo-2,5-dihydro-1H-pyrrol-1-yl)propanoic acid

To Example 2.150.1 (700 mg) in a mixture of *t*-butanol/H₂O, (2:1, 15 mL) was added 37-azido-2,5,8,11,14,17,20,23,26,29,32,35-dodecaoxaheptatriacontane (2123 mg). Sodium (R)-2-((S)-1,2-dihydroxyethyl)-4-hydroxy-5-oxo-2,5-dihydrofuran-3-olate (71.8 mg) and copper(II) sulfate (28.9 mg) were sequentially added to the mixture. The resulting mixture was stirred at room temperature for 16 hours and concentrated. The residue was purified by reverse-phase HPLC on a Gilson system (C18 column), eluting with 20-80% acetonitrile in water containing 0.1% trifluoroacetic acid, to give the title compound. ¹H NMR (400 MHz, dimethyl sulfoxide-*d*₆) δ 3.24 (s, 3H), 3.15-3.28 (m, 2H), 3.41-3.52 (m, 44H), 3.79 (t, 2H), 4.48 (t, 2H), 5.56-5.60 (m, 1H), 7.05 (s, 2H), 8.03 (s, 1H). MS (LC-MS) m/e 779 (M+H)⁺.

### 2.150.3 N-{(3S)-3-(2,5-dioxo-2,5-dihydro-1H-pyrrol-1-yl)-3-[1-(2,5,8,11,14,17,20,23,26,29,32,35-dodecaoxaheptatriacontan-37-yl)-1H-1,2,3-triazol-4-yl]propanoyl}-L-valyl-N-{4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.13,7]dec-1-yl}oxy)ethyl](2-sulfoethyl)carbamoyl}oxy)methyl]phenyl}-N5-carbamoyl-L-ornithinamide

To a mixture of O-(7-azabenzotriazol-1-yl)-N,N,N',N'-tetramethyluronium hexafluorophosphate (8.45 mg), and Example 2.150.2 (20 mg) in N,N-dimethylformamide (0.3 mL) at 0 °C was slowly added N,N-diisopropylethylamine (22.19 µL)., and the reaction mixture was stirred for 1 minute. A cold (0 °C) mixture of Example 2.112.2 (20 mg) and N,N-diisopropylethylamine (22 µL) in N,N-dimethylformamide (0.4 mL) was added. The resulting mixture was stirred for 10 minutes and was purified by reverse-phase HPLC on a Gilson system (C18 column), eluting with 20-80% acetonitrile in water containing 0.1% trifluoroacetic acid, to give the title compound. (The absolute configuration of the 3-position was arbitrarily assigned.) ¹H NMR (501 MHz, dimethyl sulfoxide-*d*₆) δ 9.95 (s, 1H), 8.07 (d, 3H), 8.04 - 7.96 (m, 2H), 7.77 (d, 1H), 7.64 - 7.53 (m, 3H), 7.50 (s, 1H), 7.48 - 7.39 (m, 2H), 7.34 (q, 2H), 7.30 - 7.23 (m, 3H), 6.98 (s, 2H), 6.93 (d, 1H), 5.61 (t, 1H), 4.96 (d, 4H), 4.54 - 4.27 (m, 3H), 4.14 (t, 1H), 3.86 (t, 2H), 3.77 (q, 4H), 3.43 (d, 71H), 3.21 (s, 6H), 3.00 (d, 5H), 2.61 (s, 2H), 2.07 (d, 3H), 1.92 (s, 1H), 1.60 (d, 2H), 1.47 - 0.86 (m, 10H), 0.85 - 0.67 (m, 12H). MS (ESI) m/e 1010.6 (M-2H)²⁻.

### 2.151 Synthesis of N-{(3R)-3-(2,5-dioxo-2,5-dihydro-1H-pyrrol-1-yl)-3-[1-(2,5,8,11,14,17,20,23,26,29,32,35-dodecaoxaheptatriacontan-37-yl)-1H-1,2,3-triazol-4-yl]propanoyl}-L-valyl-N-{4-[({[2-({3-[(4-{6-[8-(1,3-benzothiazol-2-ylcarbarnoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)methyl]phenyl}-N⁵-carbamoyl-L-ornithinamide (Synthon YR)

Example 2.151 was isolated during the preparation of 2.150.3. (The absolute configuration of the 3-position was arbitrarily assigned.) ¹H NMR (501 MHz, dimethyl sulfoxide-*d₆*) δ 9.91 (s, 1H), 8.11 (dd, 2H), 8.04 - 7.99 (m, 1H), 7.96 (s, 1H), 7.77 (d, 1H), 7.58 (t, 3H), 7.54 - 7.39 (m, 2H), 7.39 - 7.31 (m, 2H), 7.31 - 7.24 (m, 3H), 7.00 (s, 2H), 6.94 (d, 1H), 5.61 (dd, 1H), 5.08 - 4.79 (m, 4H), 4.40 (dt, 3H), 4.16 (s, 1H), 3.86 (t, 2H), 3.82 - 3.73 (m, 4H), 3.51 - 3.30 (m, 46H), 3.21 (s, 7H), 3.05 - 2.87 (m, 3H), 2.62 (t, 2H), 2.07 (d, 3H), 1.95 (s, 2H), 1.69 (s, 1H), 1.51 - 0.86 (m, 10H), 0.88 - 0.70 (m, 13H). MS (ESI) m/e 1010.6 (M-2H)²⁻.

### 2.152 Synthesis of 6-[8-(1,3-benzothiazol-2-ylcarbamoyl)-3,4-dihydroisoquinolin-2(1H)-yl]-3-[1-({3-[2-({[(2-{2-[(2S,3R,4R,5S,6S)-6-carboxy-3,4,5-trihydroxytetrahydro-2H-pyran-2-yl]ethyl}-4-{[(2S)-2-{[(2S)-2-{[(2,5-dioxo-2,5-dihydro-1H-pyrrol-1-yl)acetyl]amino}-3-methylbutanoyl]amino}propanoyl]amino}benzyl)oxy]carbonyl}[(3R,4S,5 R)-3,4,5,6-tetrahydroxyhexyl]amino)ethoxy]-5,7-dimethyltricyclo[3.3.1.1^{3,7}]dec-1-yl}methyl)-5-methyl-1H-pyrazol-4-yl]pyridine-2-carboxylic acid (Synthon YS)

### 2.152.1 3-(1-((3-(2-((((4-((S)-2-((S)-2-amino-3-methylbutanamido)propanamido)-2-(2-((2S,3R,4R,5S,6S)-6-carboxy-3,4,5-trihydroxytetrahydro-2H -pyran-2-yl)ethyl)benzyl)oxy)carbonyl)((3R,4S,5R)-3,4,5,6-tetrahydroxyhexyl)amino)ethoxy)-5,7-dimethyladamantan-1-yl)methyl)-5-methyl-1H-pyrazol-4-yl)-6-(8-(benzo[d]thiazol-2-ylcarbarnoyl)-3,4-dihydroisoquinolin-2(1H)-yl)picolinic acid

The title compound was prepared by substituting Example 1.77.2 for Example 1.25 and Example 2.123.19 for Example 2.97.7 in Example 2.97.8. MS (ESI) m/e 1417 (M+H)⁺, 1415 (M-H)⁺.

### 2.152.2 6-[8-(1,3-benzothiazol-2-ylcarbamoyl)-3,4-dihydroisoquinolin-2(1H)-yl]-3-[1-({3-[2-({[(2-{2-[(2S,3R,4R,5S,6S)-6-carboxy-3,4,5-trihydroxytetrahydro-2H-pyran-2-yl]ethyl}-4-{[(2S)-2-{[(2S)-2-{[(2,5-dioxo-2,5-dihydro-1H-pyrrol-1-yl)acetyl]amino}-3-methylbutanoyl]amino}propanoyl]amino}benzyl)oxy]carbonyl} [(3R,4S,5R)-3,4,5,6-tetrahydroxyhexyl]amino)ethoxy]-5,7-dimethyltricyclo[3.3.1.13,7]dec-1-yl}methyl)-5-methyl-1H-pyrazol-4-yl]pyridine-2-carboxylic acid

The title compound was prepared by substituting Example 2.152.1 for Example 2.49.1 in Example 2.54. ¹H NMR (400 MHz, dimethyl sulfoxide-*d₆*) δ ppm 9.85 (m, 1H), 8.18 (t, 2H), 7.96 (d, 1H), 7.73 (d, 1H), 7.55 (d, 1H), 7.46-7.25 (m, 8H), 7.21 (s, 1H), 7.15 (d, 1H), 7.00 (s, 1H), 6.99 (d, 1H), 6.88 (d, 1H), 4.95 (bs, 2H), 4.88 (s, 2H), 4.32 (m, 1H), 4.15 (t, 1H), 4.05 (s, 2H), 3.82 (t, 2H), 3.72 (m, 4H), 3.58-3.29 (m, 6H), 3.19 (m, 4H), 3.11-3.00 (m, 6H), 2.97 (t, 2H), 2.91 (t, 2H), 2.72 (m, 2H), 2.55 (m, 2H), 2.04 (s, 3H), 2.02-1.85 (m, 3H), 1.54 (m, 4H), 1.44 (s, 1H), 1.33 (bs, 1H), 1.22 (m, 6H), 1.04 (m, 6H), 0.86 (m, 2H), 0.77 (m, 12H). MS (ESI) m/e 1554 (M+H)⁺, 1552 (M-H)⁻.

### 2.153 Synthesis of 6-[8-(1,3-benzothiazol-2-ylcarbamoyl)-3,4-dihydroisoquinolin-2(1H)-yl]-3-[1-({3-[2-({[(2-{2-[(2S,3R,4R,5S,6S)-6-carboxy-3,4,5-trihydroxytetrahydro-2H-pyran-2-yt]ethyt}-4-{[(2S)-2-({(2S)-2-[({(3S,5S)-3-(2,5-dioxo-2,5-dihydro-1H-pyrrol-1-yl)-2-oxo-5-[(2-sulfoethoxy)methyl]pyrrolidin-1-yl}acetyl)amino]-3-methylbutanoyl}amino)propanoyl]amino}benzyl)oxy]carbonyl}[(3R,4S,5 R)-3,4,5,6-tetrahydroxyhexyl]amino)ethoxy]-5,7-dimethyltricyclo[3.3.1.1^{3,7}]dec-1-yl}methyl)-5-methyl-1H-pyrazol-4-yl]pyridine-2-carboxylic acid (Synthon YY)

Example 2.119.15 (11 mg) was dissolved in N,N-dimethylformamide (0.1 mL). 2-(3H-[1,2,3]triazolo[4,5-b]pyridin-3-yl)-1,1,3,3-tetramethylisouronium hexafluorophosphate(V) (11 mg) and N,N-diisopropylethylamine (7.4 mg) were added. The mixture was stirred at room temperature for five minutes. The mixture was then added to another mixture of Example 2.152.1 (34 mg) and N,N-diisopropylethylamine (16.3 mg) in N,N-dimethylformamide (0.2 mL). The reaction was stirred for 60 minutes at room temperature and quenched with trifluoroacetic acid (36 mg). The mixture was diluted with water (0.75 mL) and dimethyl sulfoxide (0.75 mL) and purified by reverse-phase HPLC using 10-75% acetonitrile in water (w/0.1% TFA) over 30 minutes on a Grace Reveleris equipped with a Luna column: C18(2), 100 A, 150 x 30 mm. Product fractions were pooled, frozen, and lyophilized to yield the title compound as the trifluoroacetic acid salt. ¹H NMR (400 MHz, dimethyl sulfoxide-*d₆*) δ ppm 9.85 (m, 1H), 8.18 (d, 1H), 8.05 (d, 1H), 8.04 (d, 1H), 7.79 (d, 1H), 7.53-7.39 (m, 8H), 7.36 (q, 2H), 7.29 (s, 1H), 7.22 (d, 1H), 7.07 (s, 1H), 6.96 (d, 1H),5.18 (bs, 2H), 4.96 (s, 2H), 4.65 (t, 1H), 4.37 (t, 1H), 4.19 (t, 1H), 4.16 (s, 1H), 4.01 (d, 2H), 3.89 (t, 2H), 3.78 (m, 4H), 3.73 (m, 2H), 3.49-3.44 (m, 4H), 3.40-3.20 (m, 8H), 3.24 (m, 4H), 3.17-3.07 (m, 4H), 3.02 (t, 2H), 2.95 (t, 2H), 2.76 (m, 4H), 2.62 (m, 1H), 2.37 (m, 1H), 2.09 (s, 3H), 1.99 (m, 2H), 1.86 (q, 1H), 1.62 (m, 4H), 1.38 (bs, 2H), 1.28 (m, 6H), 1.18-1.02 (m, 6H), 0.96 (m, 2H), 0.91-0.79 (m, 12H). MS (ESI) m/e 1773 (M-H)⁻.

### 2.154 Synthesis of (6S)-2,6-anhydro-6-(2-{2-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)methyl]-5-({N-[(2,5-dioxo-2,5-dihydro-1H-pyrrol-1-yl)acetyl]-N-(2,5,8,11,14,17,20,23,26,29,32-undecaoxatetratriacontan-34-yl)-beta-alanyl-L-valyl-L-alanyl}amino)phenyl}ethyl)-L-gulonic acid (Synthon YT)

### 2.154.1 3-(1-((3-(2-((((4-((S)-2-((S)-2-amino-3-methylbutanamido)propanamido)-2-(2-((2S,3R,4R,5S,6S)-6-carboxy-3,4,5-trihydroxytetrahydro-2H -pyran-2-yl)ethyl)benzyl)oxy)carbonyl)(2-sulfoethyl)amino)ethoxy)-5,7-dimethyladamantan-1-yl)methyl)-5-methyl-1H-pyrazol-4-yl)-6-(8-(benzo [d]thiazol-2-ylcarbamoyl)-3,4-dihydroisoquinolin-2(1H)-yl)picolinic acid

A mixture of Example 1.2.9 (200 mg), Example 2.123.19 (288 mg), and 1-hydroxybenzotriazole hydrate (50.2 mg) in N,N-dimethylformamide (2 mL) was cooled in an ice-bath, and N,N-diisopropylethylamine (143 µL) was added. The reaction mixture was stirred at room temperature for 2.5 hours and concentrated. Tetrahydrofuran (0.5 mL) and methanol (0.5 mL) were added into the residue. The resulting mixture was cooled in ice-bath and lithium hydroxide hydrate (147 mg) in water (2.5 mL) was slowly added. The mixture was stirred at room temperature for 1.5 hours, and cooled in ice bath. Trifluoroacetic acid (361 µL) was added dropwise until the pH reached 6. The mixture was purified by reverse-phase HPLC on a Gilson system (C18 column), eluting with 35-45% acetonitrile in water containing 0.1% trifluoroacetic acid, to give the title compound. MS (ESI) m/e 1375.5 (M-H)⁻.

### 2.154.2 (6S)-2,6-anhydro-6-(2-{2-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.13,7]dec-1-yl}oxy)ethyl](2-sulfoethyl)carbamoyl}oxy)methyl]-5-({N-[(2,5-dioxo-2,5-dihydro-1H-pyrrol-1-yl)acetyl]-N-(2,5,8,11,14,17,20,23,26,29,32-undecaoxatetratriacontan-34-yl)-beta-alanyl-L-valyl-L-alanyl}amino)phenyl}ethyl)-L-gulonic acid

To a mixture of 1-hydroxybenzotriazole hydrate (5.22 mg), Example 2.154.1 (23.5 mg) and Example 2.147.3 (24 mg) in N,N-dimethylformamide (1 mL) at 0 °C was slowly added N,N-diisopropylethylamine (23.84 µL). The reaction mixture was stirred at room temperature for 15 minutes and purified by reverse-phase HPLC on a Gilson system (C18 column), eluting with 35-50% acetonitrile in water containing 0.1% trifluoroacetic acid, to give the title compound. ¹H NMR (501 MHz, dimethyl sulfoxide-*d₆*) δ 12.83 (s, 1H), 9.88 (s, 1H), 8.23 - 8.04 (m, 2H), 8.02 (dd, 1H), 7.92 (s, 1H), 7.77 (d, 1H), 7.59 (d, 1H), 7.55 - 7.30 (m, 7H), 7.27 (s, 1H), 7.20 (d, 1H), 7.07 (d, 2H), 6.93 (d, 1H), 5.07 - 4.88 (m, 4H), 4.47 - 4.32 (m, 3H), 4.22 (dt, 1H), 3.97 - 3.73 (m, 4H), 3.62 - 3.45 (m, 35H), 3.31 (t, 3H), 3.21 (s, 3H), 3.06 (d, 2H), 2.83 - 2.54 (m, 5H), 2.47 - 2.29 (m, 1H), 2.13 - 1.84 (m, 5H), 1.52 (d, 1H), 1.43 - 0.69 (m, 26H). MS (ESI) m/e 1043.0 (M-2H)²⁻.

### 2.155 Synthesis of (6S)-2,6-anhydro-6-(2-{2-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)methyl]-5-[(N-12-(2,5-dioxo-2,5-dihydro-1H-pyrrol-1-yl)-3-[1-(2,5,8,11,14,17,20,23,26,29,32,35-dodecaoxaheptatriacontan-37-yl)-1H-1,2,3-triazol-4-yl]propanoyl}-L-valyl-L-alanyl)amino]phenyl}ethyl)-L-gulonic acid (Synthon YU)

### 2.155.1 3-(1-(2,5,8,11,14,17,20,23,26,29,32,35-dodecaoxaheptatriacontan-37-yl)-1H-1,2,3-triazol-4-yl)-2-(2,5-dioxo-2,5-dihydro-1H-pyrrol-1-yl)propanoic acid

The title compound was prepared using the procedure in Example 2.150.2, replacing Example 2.150.1 with 2-(2,5-dioxo-2,5-dihydro-1H-pyrrol-1-yl)pent-4-ynoic acid.

### 2.155.2 (6S)-2,6-anhydro-6-(2-{2-[({[2-({3-[(4-{6-[8-(1,3-benzothiazol-2-ylcarbarnoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.13,7]dec-1-ylloxy)ethyl](2-sulfoethyl)carbamoyl}oxy)methyl]-5-[(N-{2-(2,5-dioxo-2,5-dihydro-1H-pyrrol-1-yl)-3-[1-(2,5,8,11,14,17,20,23,26,29,32,35-dodecaoxaheptatriacontan-37-yl)-1H-1,2,3-triazol-4-yl]propanoyl}-L-valyl-L-alanyl)amino]phenyl}ethyl)-L-gulonic acid

The title compound was prepared using the procedure in Example 2.150.3, replacing Example 2.150.2 and Example 2.112.2 with Example 2.155.1 and Example 2.154.1, respectively. ¹H NMR (400 MHz, dimethyl sulfoxide-*d*₆) δ 12.83 (s, 1H), 9.87 (d, 1H), 8.25 - 8.06 (m, 2H), 8.00 (d, 1H), 7.75 (d, 1H), 7.71 (s, 1H), 7.57 (d, 1H), 7.54 - 7.28 (m, 6H), 7.25 (s, 1H), 7.18 (d, 1H), 6.98 - 6.85 (m, 3H), 5.09 - 4.89 (m, 4H), 4.76 (ddd, 1H), 4.36 (ddd, 3H), 4.17 (q, 1H), 3.84 (t, 2H), 3.76 (d, 2H), 3.72 - 3.66 (m, 2H), 3.49 - 3.44 (m, 37H), 3.20 (s, 5H), 3.01 - 2.82 (m, 3H), 2.13 - 1.81 (m, 5H), 1.52 (s, 1H), 1.39 - 0.50 (m, 23H). MS (ESI) m/e 1069.7 (M+2H)²⁺.

### 2.156 Synthesis of (6S)-2,6-anhydro-6-(2-{2-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)methyl]-5-[(N-{(3S)-3-(2,5-dioxo-2,5-dihydro-1H-pyrrol-1-yl)-3-[1-(2,5,8,11,14,17,20,23,26,29,32,35-dodecaoxaheptatriacontan-37-yl)-1H-1,2,3-triazol-4-yl]propanoyl}-L-valyl-L-alanyl)amino]phenyl}ethyl)-L-gulonic acid (Synthon YV)

Example 2.156 was isolated as a pure diastereomer during the preparation of Example 2.155.2. (The assignment of absolute configuration at the 3-position is arbitrary.) ¹H NMR (400 MHz, dimethyl sulfoxide-*d*₆) δ 12.82 (s, 1H), 9.85 (s, 1H), 8.08 (d, 2H), 8.03 - 7.95 (m, 2H), 7.75 (d, 1H), 7.57 (d, 1H), 7.51 - 7.29 (m, 6H), 7.24 (s, 1H), 7.18 (d, 1H), 6.95 (s, 2H), 6.91 (d, 1H), 5.59 (dd, 1H), 5.06 - 4.86 (m, 4H), 4.43 (dt, 2H), 4.32 (t, 1H), 4.11 (t, 1H), 3.84 (t, 2H), 3.75 (t, 3H), 3.55 - 3.41 (m, 43H), 3.41 - 3.36 (m, 2H), 3.19 (s, 5H), 3.10 (t, 1H), 3.03 - 2.86 (m, 3H), 2.59 (s, 3H), 2.13 - 1.82 (m, 6H), 1.52 (s, 1H), 1.37 - 0.65 (m, 26H). MS (ESI) m/e 1067.8 (M-2H)²⁻.

### 2.157 Synthesis of (6S)-2,6-anhydro-6-(2-{2-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-ylloxy)ethyl](2-sulfoethyl)carbamoyl}oxy)methyl]-5-[(N-{(3R)-3-(2,5-dioxo-2,5-dihydro-1H-pyrrol-1-yl)-3-[1-(2,5,8,11,14,17,20,23,26,29,32,35-dodecaoxaheptatriacontan-37-yl)-1H-1,2,3-triazol-4-yl]propanoyl}-L-valyl-L-alanyl)amino]phenyl}ethyl)-L-gulonic acid (Synthon YW)

Example 2.157was isolated as a pure diastereomer during the preparation of Example 2.155.2. (The assignment of absolute configuration at the 3-position is arbitrary.) ¹H NMR (400 MHz, dimethyl sulfoxide-*d*₆) δ 12.81 (s, 1H), 9.81 (s, 1H), 8.10 (d, 2H), 8.00 (d, 1H), 7.94 (s, 1H), 7.75 (d, 1H), 7.57 (d, 1H), 7.51 - 7.28 (m, 6H), 7.24 (s, 1H), 7.18 (d, 1H), 6.98 (s, 2H), 6.91 (d, 1H), 5.59 (t, 1H), 5.06 - 4.87 (m, 4H), 4.46 - 4.26 (m, 2H), 4.12 (d, 1H), 3.84 (t, 2H), 3.75 (d, 3H), 3.46 (d, 27H), 3.40 - 3.36 (m, 2H), 3.19 (s, 5H), 3.01 - 2.85 (m, 3H), 2.60 (s, 3H), 1.99 (d, 4H), 1.52 (s, 1H), 1.35 - 0.65 (m, 23H). MS (ESI) m/e 1067.8 (M-2H)²⁻.

### 2.158 Synthesis of (6S)-2,6-anhydro-6-(2-{2-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)methyl]-5-[(N-{(3S)-3-(2,5-dioxo-2,5-dihydro-1H-pyrrol-1-yl)-3-[1-(3-sulfopropyl)-1H-1,2,3-triazol-4-yl]propanoyl}-L-valyl-L-alanyl)amino]phenyl}ethyl)-L-gulonic acid (Synthon ZB)

### 2.158.1 sodium 3-azidopropane-1-sulfonate

To a mixture of sodium azide (3.25 g) in water (25 mL) was added 1, 2-oxathiolane 2,2-dioxide (6.1 g) in acetone (25mL). The resulting mixture was stirred at room temperature for 24 hours and concentrated to dryness. The solid was suspended in diethyl ether (100 mL) and stirred at reflux for 1 hour. The suspension was cooled to room temperature, and the solid was collected by filtration, washed with acetone and diethyl ether, and dried under vacuum to afford the title compound. MS (LC-MS) m/e 164 (M-H)⁻.

### 2.158.2 isopropyl 3-azidopropane-1-sulfonate

A mixture of Example 2.158.1 (6.8 g) in concentrated HCl (90 mL) was stirred at room temperature for 1 hour. The mixture was concentrated to dryness. The residue was dissolved in dichloromethane (350 mL), and triisopropoxymethane (42.0 mL) was added in one portion to the mixture. The resulting mixture was stirred at 50 °C for 2 hours and concentrated to dryness. The crude residue was purified by silica gel chromatography, eluting with 10/1 petroleum ether/ethyl acetate, to give the title compound. ¹H-NMR (CDCl₃, 400 MHz): 1.42 (s, 3H), 1.44 (s, 3H), 2.08-2.15 (m, 2H), 3.17 (t, 2H), 3.51 (t, 2H), 4.95-5.01 (m, 1H).

### 2.158.3 3-(2,5-dioxo-2,5-dihydro-1H-pyrrol-1-yl)-3-(1-(3-sulfopropyl)-1H-1,2,3-triazol-4-yl)propanoic acid

To a mixture of Example 2.150.1 (450 mg) in *t*-butanol/H₂O (2:1, 9 mL) was added Example 2.158.2 (483 mg) followed by copper(II) sulfate (18.59 mg) and sodium (R)-2-((S)-1,2-dihydroxyethyl)-4-hydroxy-5-oxo-2,5-dihydrofuran-3-olate (46.2 mg). The resulting mixture was stirred at room temperature for 16 hours, and the mixture was concentrated to dryness. The residue was purified by reverse-phase HPLC on a Gilson system (C18 column), eluting with 20-80% acetonitrile in water containing 0.1% trifluoroacetic acid, to give the title compound. ¹H-NMR (dimethyl sulfoxide-*d₆*, 400 MHz): 2.06-2.10 (m, 2H), 2.45-2.48 (m, 2H), 3.21-3.23 (m, 2H), 4.40-4.44 (m, 2H), 5.55-5.59 (m, 1H), 7.05 (s, 2H), 8.10 (s, 1H). MS (LCMS) m/e 359 (M+H)⁺.

### 2.158.4 (6S)-2,6-anhydro-6-(2-{2-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.13,7]dec-1-yl}oxy)ethyl](2-sulfoethyl)carbamoyl}oxy)methyl]-5-[(N-{(3S)-3-(2,5-dioxo-2,5-dihydro-1H-pyrrol-1-yl)-3-[1-(3-sulfopropyl)-1H-1,2,3-triazol-4-yl]propanoyl}-L-valyl-L-alanyl)amino]phenyl}ethyl)-L-gulonic acid

The title compound was prepared using the procedure in Example 2.150.3, replacing Example 2.150.2 and Example 2.112.2 with Example 2.158.3 and Example 2.154.1, respectively. The compound was isolated as a pure diastereomer. (The absolute configuration of the 3-position was arbitrarily assigned.) ¹H NMR (400 MHz, dimethyl sulfoxide-*d*₆) δ 10.14 - 9.66 (m, 1H), 8.07 (d, 2H), 8.04 - 7.96 (m, 2H), 7.75 (d, 1H), 7.57 (d, 1H), 7.52 - 7.29 (m, 7H), 7.26 (s, 1H), 7.18 (d, 1H), 6.92 (d, 3H), 5.58 (t, 1H), 5.09 - 4.84 (m, 4H), 4.35 (dt, 3H), 4.15 - 4.02 (m, 1H), 3.89 - 3.65 (m, 4H), 3.28 (d, 1H), 3.21 (dd, 2H), 3.14-3.02 (m, 2H), 3.01 - 2.86 (m, 4H), 2.62 (d, 3H), 2.37 (t, 2H), 2.29 (s, 0H), 2.02 (dt, 5H), 1.52 (s, 1H), 1.40 - 0.59 (m, 24H). MS (ESI) m/e 1715.3 (M-H)⁻.

### 2.159 Synthesis of (6S)-2,6-anhydro-6-(2-{2-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)methyl]-5-[(N-{(3R)-3-(2,5-dioxo-2,5-dihydro-1H-pyrrol-1-yl)-3-[1-(3-sulfopropyl)-1H-1,2,3-triazol-4-yl]propanoyl}-L-valyl-L-alanyl)amino]phenyl}ethyl)-L-gulonic acid (Synthon ZC)

Example 2.159 was isolated as a pure diastereomer during the preparation of Example 2.158. (The absolute configuration of the 3-position was arbitrarily assigned.) ¹H NMR (400 MHz, dimethyl sulfoxide-*d*₆) δ 9.97 (d, 1H), 8.21 (d, 1H), 8.13 (d, 1H), 8.04 - 7.96 (m, 2H), 7.75 (d, 1H), 7.57 (d, 1H), 7.55 - 7.37 (m, 4H), 7.36 - 7.25 (m, 3H), 7.17 (d, 1H), 6.98 (s, 2H), 6.93 (d, 1H), 5.58 (t, 1H), 4.94 (d, 4H), 4.50 - 4.26 (m, 3H), 4.10 (s, 1H), 3.98 - 3.73 (m, 3H), 3.51 (d, 1H), 3.42 (s, 3H), 3.34-3.01 (m, 6H), 3.01 - 2.83 (m, 4H), 2.63 (d, 4H), 2.42 (d, 1H), 2.18 - 1.80 (m, 8H), 1.53 (s, 1H), 1.39 - 0.68 (m, 27H). MS (ESI) m/e 1715.4 (M-H)⁻.

### 2.160 Synthesis of (6S)-2,6-anhydro-6-(2-{2-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.1^{3,7}]dec-1-yl}oxy)ethyl](2-sulfoethyl)carbamoyl}oxy)methyl]-5-({N-[(2,5-dioxo-2,5-dihydro-1H-pyrrol-1-yl)acetyl]-N-[2-(2-sulfoethoxy)ethyl]-beta-alanyl-L-valyl-L-alanyl}amino)phenyl}ethyl)-L-gulonic acid (Synthon ZJ)

### 2.160.1 4-((tert-butyldiphenylsilyl)oxy)-2,2-dimethylbutyl 2-(2-((tert-butoxycarbonyl)amino)ethoxy)ethanesulfonate

To a mixture of tert-butyl (2-hydroxyethyl)carbamate (433 mg) in dimethyl sulfoxide (0.9 mL) at 20 °C were added 4-((tert-butyldiphenylsilyl)oxy)-2,2-dimethylbutyl ethenesulfonate (500 mg) and K₂CO₃ (210 mg). The mixture was warmed to 60 °C and stirred for 16 hours in a capped bottle. The mixture was diluted with ethyl acetate, washed with water and brine. The organic layer was dried over anhydrous sodium sulfate, filtered, and concentrated. The residue was purified by silica gel flash chromatography, eluting with petrol ether/ ethyl acetate (10:1~2:1), to give the title compound. MS (LC-MS) m/e 630.3 (M+Na)⁺.

### 2.160.2 4-((tert-butyldiphenylsilyl)oxy)-2,2-dimethylbutyl 2-(2-aminoethoxy)ethanesulfonate

To a mixture of Example 2.160.1 (1.5 g) in anhydrous dichloromethane (100 mL) at 20 °C was added zinc(II) bromide (0.445 g). The mixture was stirred at room temperature for 16 hours. Additional zinc(II) bromide (278 mg) was added to above mixture, and the reaction was stirred for additional 16 hours. The reaction was quenched with 1 M aqueous Na₂CO₃ mixture (5 mL), and the aqueous layer was extracted with ethyl acetate three times. The combined organic layers were dried over sodium sulfate, filtered, and concentrated. The residue was purified by silica gel column chromatography, eluting with dichloromethane/methanol (10:1), to give the title compound. MS (LC-MS) m/e 508.2 (M+H)⁺.

### 2.160.3 tert-butyl 3-((2-(2-((4-((tert-butyldiphenylsilyl)oxy)-2,2-dimethylbutoxy)sulfonyl)ethoxy)ethyl)amino)propanoate

To a mixture of Example 2.160.2 (0.365 g) in *N*, *N*-dimethylformamide (5.5 mL) and water (0.55 mL) were added tert-butyl acrylate (0.105 mL) and triethylamine (10.02 µL). The mixture was stirred at 60 °C for 30 hours. The mixture was concentrated. The residue was mixed with 1 M aqueous Na₂CO₃ mixture (5 mL). The aqueous layer was extracted with ethyl acetate three times. The combined organic layers were dried over sodium sulfate, filtered and concentrated. The residue was purified by silica gel column chromatography, eluting with dichloromethane/ ethyl acetate(3:1) and dichloromethane /methanol (10:1), to give the title compound. MS (LC-MS) m/e 636.3 (M+H)⁺.

### 2.160.4 tert-butyl 3-(N-(2-(2-((4-((tert-butyldiphenylsilyl)oxy)-2,2-dimethylbutoxy)sulfonyl)ethoxy)ethyl)-2-(2,5-dioxo-2,5-dihydro-1H-pyrrol-1-yl)acetamido)propanoate

To a mixture of Example 2.160.3 (557.5 mg), 2-(2,5-dioxo-2,5-dihydro-1H-pyrrol-1-yl)acetic acid (272 mg) and O-(7-azabenzotriazol-1-yl)-N,N,N',N'-tetramethyluronium hexafluorophosphate (667 mg) in *N*, *N*-dimethylformamide (1.75 mL) at 0 °C was added N,N-diisopropylethylamine (0.459 mL). The resulting mixture was stirred at 0 °C for 1 hour. The reaction mixture was mixed with saturated aqueous NH₄Cl mixture, extracted with ethyl acetate and washed with brine. The organic layer was dried over sodium sulfate, filtered and concentrated. The residue was purified by silica gel column chromatography, eluting with petroleum ether/ ethyl acetate (2/1), to provide the title compound. MS (LC-MS) m/e 795.3 (M+Na)⁺.

### 2.160.5 3-(2-(2,5-dioxo-2,5-dihydro-1H-pyrrol-1-yl)-N-(2-(2-sulfoethoxy)ethyl)acetamido)propanoic acid

To a mixture of Example 2.160.4 (230 mg) in dichloromethane (4 mL) was added trifluoroacetic acid (3 mL). The mixture was stirred at 20 °C for 16 hours and was concentrated. The residue was purified by reverse-phase HPLC on a Gilson system (C18 column), eluting with 20-80% acetonitrile in water containing 0.1% trifluoroacetic acid, to give the title compound. MS (LC-MS) m/e 379.0 (M+Na)⁺.

### 2.160.6 2-(2-(2-(2,5-dioxo-2,5-dihydro-1H-pyrrol-1-yl)-N-(3-((2,5-dioxopyrrolidin-1-yl)oxy)-3-oxopropyl)acetamido)ethoxy)ethane-1-sulfonic acid

A mixture of 1-hydroxypyrrolidine-2,5-dione (16.43 mg), Example 2.160.5 (30 mg), 1-ethyl-3-[3-(dimethylamino)propyl]-carbodiimide hydrochloride (45.6 mg) in N,N-dimethylformamide were stirred overnight. The reaction mixture was purified by reverse-phase HPLC on a Gilson system (C18 column), eluting with 2-30% acetonitrile in water containing 0.1% trifluoroacetic acid, to give the title compound. MS (ESI) m/e 475.9(M+H)⁺.

### 2.160.7 (6S)-2,6-anhydro-6-(2-{2-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.13,7]dec-1-yl}oxy)ethyl](2-sulfoethyl)carbamoyl}oxy)methyl]-5-({N-[(2,5-dioxo-2,5-dihydro-1H-pyrrol-1-yl)acetyl]-N-[2-(2-sulfoethoxy)ethyl]-beta-alanyl-L-valyl-L-alanyl}amino)phenyl}ethyl)-L-gulonic acid

To a mixture of 1-hydroxybenzotriazole hydrate (4.45 mg), Example 2.160.6 (8.97 mg) and Example 2.154.1 (20 mg) in N,N-dimethylformamide (0.8 mL) at 0 °C was added N,N-diisopropylethylamine (20 µL dropwise). The reaction mixture was stirred at room temperature for 1 hour and purified by reverse-phase HPLC on a Gilson system (C18 column), eluting with 30-55% acetonitrile in water containing 0.1% trifluoroacetic acid, to give the title compound. ¹H NMR (500 MHz, dimethyl sulfoxide-*d*₆) δ 12.87 (s, 1H), 9.88 (d, 1H), 8.28 - 8.10 (m, 1H), 8.03 (d, 1H), 7.95 (d, 1H), 7.78 (d, 1H), 7.60 (d, 1H), 7.56 - 7.31 (m, 7H), 7.28 (s, 1H), 7.21 (d, 1H), 7.06 (d, 2H), 6.95 (d, 1H), 5.06 - 4.90 (m, 4H), 4.38 (q, 3H), 4.28 - 4.11 (m, 1H), 3.87 (t, 2H), 3.79 (d, 2H), 3.71 - 3.49 (m, 5H), 3.21 (d, 2H), 3.12 (q, 2H), 2.97 (dt, 3H), 2.84 - 2.57 (m, 6H), 2.38 (dd, 1H), 2.13 - 1.86 (m, 5H), 1.55 (s, 1H), 1.39 - 0.64 (m, 25H). MS (ESI) m/e 867.6 (M-2H)²⁻.

### 2.161 Synthesis of 6-{8-[(1,3-benzothiazol-2-yl)carbamoyl]-3,4-dihydroisoquinolin-2(1H)-yl}-3-[1-({3-[2-({[(2-{2-[(2S,3R,4R,5S,6S)-6-carboxy-3,4,5-trihydroxyoxan-2-yl]ethyl}-4-{[(2S)-2-{[(2S)-2-{[(2S)-2-(2,5-dioxo-2,5-dihydro-1H-pyrrol-1-yl)-3-14-[(2,5,8,11,14,17,20,23,26,29,32-undecaoxatetratriacontan-34-yl)oxy]phenyl}propanoyl]amino}-3-methylbutanoyl]amino}propanoyl]amino}phenyl)methoxy]carbonyl} [(3R ,4S,5R)-3,4,5,6-tetrahydroxyhexyl]amino)ethoxy]-5,7-dimethyltricyclo[3.3.1.1^{3,7}]decan-1-yl}methyl)-5-methyl-1H-pyrazol-4-yl]pyridine-2-carboxylic acid (Synthon ZE)

The title compound was prepared by substituting Example 2.120.5 for Example 2.119.15 in Example 2.153. ¹H NMR (400 MHz, dimethyl sulfoxide-*d₆*) δ ppm 12.84 (bs, 2H), 9.92 (m, 1H), 8.26 (d, 1H), 8.13 (d, 1H), 8.03 (d, 1H), 7.79 (d, 1H), 7.61 (d, 1H), 7.52-7.41 (m, 4H), 7.36 (m, 3H), 7.27 (s, 1H), 7.21 (d, 1H), 7.02 (d, 2H), 6.95 (d, 1H), 6.89 (s, 2H), 6.78 (d, 2H), 5.02 (bs, 4H), 4.96 (s, 2H), 4.59 (dd, 1H), 4.38 (m, 2H), 4.21 (t, 1H), 3.99 (t, 2H), 3.88 (t, 2H), 3.79 (m, 2H), 3.69 (t, 2H), 3.64 (m, 1H), 3.57 (m, 4H), 3.53 (m, 4H), 3.50 (s, 40H), 3.42 (m, 2H), 3.38 (m, 1H), 3.30 (m, 2H), 3.23 (s, 6H), 3.20-3.08 (m, 6H), 3.01 (t, 2H), 2.94 (t, 1H), 2.76 (m, 1H), 2.61 (m, 1H), 2.08 (s, 3H), 2.06-1.92 (m, 2H), 1.67-1.52 (m, 3H), 1.38 (m, 1H), 1.32-1.22 (m, 6H), 1.18-1.01 (m, 6H), 0.92 (m, 2H), 0.84 (m, 6H), 0.78 (m, 6H). MS (ESI) m/e 1078 (M-2H)⁻.

### 2.162 Synthesis of 4-{[({2-[(3-{[4-(6-{8-[(1,3-benzothiazol-2-yl)carbamoyl]-3,4-dihydroisoquinolin-2(1H)-yl}-2-carboxypyridin-3-yl)-5-methyl-1H-pyrazol-1-yl]methyl}-5,7-dimethyltricyclo[3.3.1.1^{3,7}]decan-1-yl)oxy]ethyl}[(3S)-3,4-dihydroxybutyl]carbamoyl)oxy]methyl}-3-(2-{2-[2-(2,5-dioxo-2,5-dihydro-1H-pyrrol-1-yl)acetamido]ethoxy}ethoxy)phenyl beta-D-glucopyranosiduronic acid (Synthon ZS)

### 2.162.1 3-(1-((3-(2-((((2-(2-(2-aminoethoxy)ethoxy)-4-(((2S,3R,4S,5S,6S)-6-carboxy-3,4,5-trihydroxytetrahydro-2H-pyran-2-yl)oxy)benzyl)oxy)carbonyl)((S)-3,4-dihydroxybutyl)amino)ethoxy)-5,7-dimethyladamantan-1-yl)methyl)-5-methyl-1H-pyrazol-4-yl)-6-(8-(benzo[d]thiazol-2-ylcarbamoyl)-3,4-dihydroisoquinolin-2(1H)-yl)picolinic acid

Example 2.162.1 was prepared by substituting Example 2.62.6 for (9H-fluoren-9-yl)methyl ((S)-3-methyl-1-(((S)-1-((4-((((4-nitrophenoxy)carbonyl)oxy)methyl)phenyl)amino)-1-oxo-5-ureidopentan-2-yl)amino)-1-oxobutan-2-yl)carbamate and substituting Example 1.85 for Example 1.2.9 in Example 2.49.1. MS (ESI) m/e 1261.4 (M-H)⁻.

### 2.162.2 4-{[({2-[(3-{[4-(6-{8-[(1,3-benzothiazol-2-yl)carbamoyl]-3,4-dihydroisoquinolin-2(1H)-yl}-2-carboxypyridin-3-yl)-5-methyl-1H-pyrazol-1-yl]methyl}-5,7-dimethyltricyclo[3.3.1.13,7]decan-1-yl)oxy]ethyl}[(3S)-3,4-dihydroxybutyl]carbamoyl)oxy]methyl}-3-(2-{2-[2-(2,5-dioxo-2,5-dihydro-1H-pyrrol-1-yl)acetamido]ethoxy}ethoxy)phenyl beta-D-glucopyranosiduronic acid

Example 2.162.2 was prepared by substituting Example 2.162.1 for Example 2.49.1 in Example 2.54. ¹H NMR (400 MHz, dimethyl sulfoxide-*d₆*) δ 8.18 (t, 1H), 8.00 (dd, 1H), 7.76 (d, 1H), 7.58 (dd, 1H), 7.50 - 7.29 (m, 6H), 7.26 (s, 1H), 7.17 (d, 1H), 7.03 (s, 2H), 6.92 (d, 1H), 6.64 (d, 1H), 6.57 (dd, 1H), 4.94 (d, 4H), 4.08 (hept, 2H), 4.00 (s, 2H), 3.92 - 3.68 (m, 8H), 3.51 - 3.13 (m, 12H), 2.98 (t, 2H), 2.06 (s, 3H), 1.65 (s, 1H), 1.43 - 0.66 (m, 18H). MS (ESI) m/e 1398.5 (M-H)⁻.

### 2.163 Synthesis of 2,6-anhydro-8-[2-({[{2-[(3-{[4-(6-{8-[(1,3-benzothiazol-2-yl)carbamoyl]-3,4-dihydroisoquinolin-2(1H)-yl}-2-carboxypyridin-3-yl)-5-methyl-1H-pyrazol-1-yl]methyl}-5,7-dimethyltricyclo[3.3.1.1^{3,7}]decan-l-yl)oxy]ethyl}(2-sulfoethyl)carbamoyl]oxy}methyl)-5-{[(79S,82S)-74-[(2,5-dioxo-2,5-dihydro-1H-pyrrol-1-yl)acetyl]-82-methyl-77,80,83-trioxo-79-(propan-2-yl)-2,5,8,11,14,17,20,23,26,29,32,35,38,41,44,47,50,53,56,59,62,65,68,71-tetracosaoxa-74,78,81-triazatrioctacontan-83-yl]amino}phenyl]-7,8-dideoxy-L-glycero-L-gulo-octonic acid (Synthon ZW)

### 2.163.1 benzyl 2,5,8,11,14,17,20,23,26,29,32,35,38,41,44,47,50,53, 56,59,62, 65,68,71-tetracosaoxa-74-azaheptaheptacontan-77-oate

The title compound was prepared using the procedure in Example 2.147.1, replacing 2,5,8,11,14,17,20,23,26,29,32-undecaoxatetratriacontan-34-amine with 2,5,8,11,14,17,20,23,26,29,32,35,38,41,44,47,50,53,56,59,62,65,68,71-tetracosaoxatriheptacontan-73-amine. MS (ESI) m/e 625.9 (M+2H)²⁺.

### 2.163.2 2,5,8,11,14,17,20,23,26,29,32,35,38,41,44,47,50,53,56,59, 62,65,68,71-tetracosaoxa-74-azaheptaheptacontan-77-oic acid

The title compound was prepared using the procedure in Example 2.147.2, replacing Example 2.147.1 with Example 2.163.1. MS (ESI) m/e 1160.7 (M+H)⁺.

### 2.163.3 2,5-dioxopyrrolidin-1-yl 74-(2-(2,5-dioxo-2,5-dihydro-1H-pyrrol-1-yl)acetyl)-2,5,8,11,14,17,20,23,26,29,32,35,38,41,44, 47,50,53, 56,59,62,65,68,71-tetracosaoxa-74-azaheptaheptacontan-77-oate

The title compound was prepared using the procedure in Example 2.147.3, replacing Example 2.147.2 with Example 2.163.2. MS (ESI) m/e 698.1(M+2H)²⁺.

### 2.163.4 2,6-anhydro-8-[2-({[{2-[(3-{[4-(6-{8-[(1,3-benzothiazol-2-yl)carbamoyl]-3,4-dihydroisoquinolin-2(1H)-yl}-2-carboxypyridin-3-yl)-5-methyl-1H-pyrazol-1-yl]methyl)-5,7-dimethyltricyclo[3.3.1.13,7]decan-1-yl)oxy]ethyl}(2-sulfoethyl)carbamoyl]oxy}methyl)-5-{[(79S,82S)-74-[(2,5-dioxo-2,5-dihydro-1H-pyrrol-1-yl)acetyl]-82-methyl-77,80,83-trioxo-79-(propan-2-yl)-2,5,8,11,14,17,20,23,26,29,32,35,38, 41,44,47,50,53,56, 59,62,65,68,71-tetracosaoxa-74,78,81-triazatrioctacontan-83-yl]amino}phenyl]-7,8-dideoxy-L-glycero-L-gulo-octonic acid

The title compound was prepared using the procedure in Example 2.147.4, replacing Example 2.147.3 and Example 2.141.4 with Example 2.163.3 and Example 2.154.1, respectively. ¹H NMR (400 MHz, dimethyl sulfoxide-*d*₆) δ 9.86 (s, 1H), 8.23 - 7.87 (m, 3H), 7.76 (d, 1H), 7.58 (dd, 1H), 7.53 - 7.25 (m, 7H), 7.19 (d, 1H), 7.05 (d, 2H), 6.92 (d, 1H), 5.07 - 4.85 (m, 4H), 4.49 - 4.30 (m, 3H), 4.20 (dt, 1H), 3.52 (d, 8H), 3.46 - 3.26 (m, 7H), 3.20 (s, 4H), 3.15 - 2.82 (m, 4H), 2.61 (s, 3H), 2.38 (dq, 1H), 2.11 - 1.82 (m, 5H), 1.53 (s, 1H), 1.39 - 0.66 (m, 24H). MS (ESI) m/e 1326.9 (M-2H)²⁻.

### 2.164 Synthesis of 6-{8-[(1,3-benzothiazol-2-yl)carbamoyl]-3,4-dihydroisoquinolin-2(1H)-yl}-3-{1-[(3-{2-[{[(4-{[(2S,SS)-2-[3-(carbamoylamino)propyl]-10-[(2,5-dioxo-2,5-dihydro-1H-pyrrol-1-yl)acetyl]-4,7-dioxo-5-(propan-2-yl)-15-sulfo-13-oxa-3,6,10-triazapentadecanan-1-oyl]amino}phenyl)methoxy]carbonyl}(2-sulfoethyl)amino]ethoxy}-5,7-dimethyltricyclo[3.3.1.13,7]decan-1-yl)methyl]-5-methyl-1H-pyrazol-4-yl}pyridine-2-carboxylic acid (Synthon ZX)

A mixture of 1-hydroxypyrrolidine-2,5-dione (2.74 mg), 1-ethyl-3-[3-(dimethylamino)propyl]-carbodiimide hydrochloride (4.26 mg) and Example 2.160.5 (9.01 mg) in N,N-dimethylformamide (0.3 mL) were stirred at room temperature overnight. The mixture was cooled in ice bath. 1-Hydroxybenzotriazole hydrate (3.65 mg) and a mixture of Example 2.112.2 (20 mg) and N,N-diisopropylethylamine (22.19 µL) were added. The resulting mixture was stirred at 0 °C for 10 minutes and purified by reverse phase HPLC, eluting with 30%-55% acetonitrile in 0.1% trifluoroacetic acid water, to provide the title compound. ¹H NMR (400 MHz, dimethyl sulfoxide-*d*₆) δ 9.95 (d, 1H), 8.18 - 7.89 (m, 3H), 7.76 (d, 1H), 7.57 (d, 3H), 7.52 - 7.21 (m, 8H), 7.04 (d, 2H), 6.92 (d, 1H), 4.94 (d, 4H), 4.37 (d, 2H), 4.19 (d, 1H), 3.85 (t, 2H), 3.77 (d, 2H), 3.22 (d, 2H), 2.96 (dt, 4H), 2.73 (dt, 2H), 2.66 - 2.55 (m, 2H), 2.36 (s, 1H), 2.06 (s, 3H), 1.91 (s, 1H), 1.61 (d, 3H), 1.47 - 0.86 (m, 11H), 0.80 (ddd, 12H). MS (ESI) m/e 1617.5 (M-H)⁻.

### 2.165 This paragraph was intentionally left blank.

### 2.166 Synthesis of 6-(8-(benzo[d]thiazol-2-ylcarbamoyl)-3,4-dihydroisoquinolin-2(1H)-yl)-3-(1-((3-(2-((((2-(2-((2S,3R,4R,5S,6S)-6-carboxy-3,4,5-trihydroxytetrahydro-2H-pyran-2-yl)ethyl)-4-((S)-2-((S)-2-(2-((3S,5S)-3-(2,5-dioxo-2,5-dihydro-1H-pyrrol-1-yl)-2-oxo-5-((2-sulfoethoxy)methyl)pyrrolidin-1-yl)acetamido)-3-methylbutanamido)propanamido)benzyl)oxy)carbonyl)((S)-3,4-dihydroxybutyl)amino)ethoxy)-5,7-dimethyladamantan-1-yl)methyl)-5-methyl-1H-pyrazol-4-yl)picolinic acid (Synthon AAA)

The title compound was prepared by substituting Example 2.167.1 for Example 2.119.16 in Example 2.119.17. ¹H NMR (500 MHz, dimethyl sulfoxide-*d₆*) δ ppm 9.86 (br d, 1H), 8.17 (br d, 1H), 8.04 (m, 2H), 7.78 (d, 1H), 7.61 (d, 1H), 7.51 (br d, 1H), 7.49-7.39 (m, 4H), 7.36 (m, 2H), 7.29 (s, 1H), 7.21 (d, 1H), 7.07 (s, 2H), 6.95 (d, 1H), 5.00 (s, 2H), 4.96 (s, 2H), 4.64 (t, 1H), 4.36 (m, 1H), 4.19 (m, 1H), 4.16 (d, 1H), 4.01 (d, 1H), 3.88 (br t, 2H), 3.82 (br m, 3H), 3.75 (br m, 1H), 3.64 (t, 2H), 3.54 (d, 2H), 3.47 (m, 4H), 3.43 (br m, 4H), 3.23 (br m, 5H), 3.13 (t, 1H), 3.10 (br m, 1H), 3.01 (br m, 2H), 2.93 (t, 1H), 2.83-2.68 (m, 3H), 2.37 (m, 1H), 2.08 (s, 3H), 1.99 (br m, 2H), 1.85 (m, 1H), 1.55 (br m, 1H), 1.37 (br m, 1H), 1.28 (br m, 6H), 1.10 (br m, 7H), 0.93 (br m, 1H), 0.88-0.69 (m, 12H). MS (ESI) m/e 1713.6 (M-H)⁻.

### Alternative Synthesis of 6-(8-(benzo[d]thiazol-2-ylcarbamoyl)-3,4-dihydroisoquinolin-2(1H)-yl)-3-(1-((3-(2-((((2-(2-((2S,3R,4R,5S,6S)-6-carboxy-3,4,5-trihydroxytetrahydro-2H-pyran-2-yl)ethyl)-4-((S)-2-((S)-2-(2-((3S,5S)-3-(2,5-dioxo-2,5-dihydro-1H-pyrrol-1-yl)-2-oxo-5-((2-sulfoethoxy)methyl)pyrrolidin-1-yl)acetamido)-3-methylbutanamido)propanamido)benzyl)oxy)carbonyl)((S)-3,4-dihydroxybutyl)amino)ethoxy)-5,7-dimethyladamantan-1-yl)methyl)-5-methyl-1H-pyrazol-4-yl)picolinic acid (Synthon AAA)

### 2.166.1 3-(1-((3-(2-((((4-((S)-2-((S)-2-amino-3-methylbutanamido)propanamido)-2-(2-((2S,3R,4R,5S,6S)-6-carboxy-3,4,5-trihydroxytetrahydro-2H-pyran-2-yl)ethyl)benzyl)oxy)carbonyl)((S)-3,4-dihydroxybutyl)amino)ethoxy)-5,7-dimethyladamantan-1-yl)methyl)-5-methyl-1H-pyrazol-4-yl)-6-(8-(benzo[d]thiazol-2-ylcarbamoyl)-3,4-dihydroisoquinolin-2(1H)-yl)picolinic acid

To a stirred solution of Example 1.85 (0.065 g), 1-hydroxybenzotriazole (0.013 g) and N,N-diisopropylethylamine (0.06 mL) in N,N-dimethylformamide (0.5 mL) was added Example 2.123.19 (0.085 g), and the mixture was stirred at room temperature for 2 hours. The reaction was concentrated under reduced pressure. The residue was dissolved in a solvent mixture of methanol (0.5 mL) and tetrahydrofuran (0.5 mL), and lithium hydroxide monohydrate (30 mg) was added. The reaction was stirred for 1 hour at ambient temperature, after which the reaction was concentrated under reduced pressure. The residue was dissolved in methanol/water (1:1, 1mL) containing 0.1 mL trifluoroacetic acid. The sample was purified by reverse-phase HPLC (Phenomenex^{®} Luna^{®} C18 250 x 50 mm column, 100 mL/min), eluting with 20-100% acetonitrile in water containing 0.01% trifluoroacetic acid over 40 minutes. The fractions containing product were lyophilized to give the title compound. MS (ESI) m/z 1357.5 (M+H)⁺.

### 2.166.2 6-(8-(benzo[d]thiazol-2-ylcarbamoyl)-3,4-dihydroisoquinolin-2(1H)-yl)-3-(1-((3-(2-((((2-(2-((2S,3R,4R,5S,6S)-6-carboxy-3,4,5-trihydroxytetrahydro-2H-pyran-2-yl)ethyl)-4-((S)-2-((S)-2-(2-((3S,5S)-3-(2,5-dioxo-2,5-dihydro-1H-pyrrol-1-yl)-2-oxo-5-((2-sulfoethoxy)methyl)pyrrolidin-1-yl)acetamido)-3-methylbutanamido)propanamido)benzyl)oxy)carbonyl)((S)-3,4-dihydroxybutyl)amino)ethoxy)-5,7-dimethyladamantan-1-yl)methyl)-5-methyl-1H-pyrazol-4-yl)picolinic acid (Synthon AAA)

To a solution of Example 2.119.15 (16 mg) in N,N-dimethylformamide (200 µL) was added 1-[bis(dimethylamino)methylene]-1H-1,2,3-triazolo[4,5-b]pyridinium 3-oxid hexafluorophosphate (16 mg, HATU) and N,N-diisopropylethylamine (17 µL). The reaction was stirred for 5 minutes, and a solution of Example 2.166.1 (48 mg) and N,N-diisopropylethylamine (20 µL) in N,N-dimethylformamide (200 µL) was added. The reaction was stirred for one hour and diluted with a mixture ofN,N-dimethylformamide/water (1/1, 1.5 mL). The sample was purified by reverse-phase HPLC (Phenomenex^{®} Luna^{®} C18 250 x 50 mm column, 100 mL/min), eluting with 20-70% acetonitrile in water containing 0.01% trifluoroacetic acid over 40 minutes. The fractions containing the product were lyophilized to give the title compound. ¹H NMR (500 MHz, dimethyl sulfoxide-*d*₆) δ ppm 9.86 (br d, 1H), 8.17 (br d, 1H), 8.04 (m, 2H), 7.78 (d, 1H), 7.61 (d, 1H), 7.51 (br d, 1H), 7.49-7.39 (m, 4H), 7.36 (m, 2H), 7.29 (s, 1H), 7.21 (d, 1H), 7.07 (s, 2H), 6.95 (d, 1H), 5.00 (s, 2H), 4.96 (s, 2H), 4.64 (t, 1H), 4.36 (m, 1H), 4.19 (m, 1H), 4.16 (d, 1H), 4.01 (d, 1H), 3.88 (br t, 2H), 3.82 (br m, 3H), 3.75 (br m, 1H), 3.64 (t, 2H), 3.54 (d, 2H), 3.47 (m, 4H), 3.43 (br m, 4H), 3.23 (br m, 5H), 3.13

(t, 1H), 3.10 (br m, 1H), 3.01 (br m, 2H), 2.93 (t, 1H), 2.83-2.68 (m, 3H), 2.37 (m, 1H), 2.08 (s, 3H), 1.99 (br m, 2H), 1.85 (m, 1H), 1.55 (br m, 1H), 1.37 (br m, 1H), 1.28 (br m, 6H), 1.10 (br m, 7H), 0.93 (br m, 1H), 0.88 - 0.69 (m, 12H). MS (ESI) m/z 1713.6 (M-H)⁻.

### 2.167 Synthesis of 2,6-anhydro-8-(2-{[({2-[(3-{[4-(6-{8-[(1,3-benzothiazol-2-yl)carbamoyl]-3,4-dihydroisoquinolin-2(1H)-yl}-2-carboxypyridin-3-yl)-5-methyl-1H-pyrazol-1-yl]methyl}-5,7-dimethyltricyclo[3.3.1.13,7]decan-1-yl)oxy]ethyl}[(3S)-3,4-dihydroxybutyl]carbamoyl)oxy]methyl}-5-{[(2S)-2-({(2S)-2-[2-(2,5-dioxo-2,5-dihydro-1H-pyrrol-1-yl)acetamido]-3-methylbutanoyl}amino)propanoyl]amino}phenyl)-7,8-dideoxy-L-glycero-L-gulo-octonic acid (Synthon AAD)

### 2.167.1 3-(1-((3-(2-((((4-((S)-2-((S)-2-amino-3-methylbutanamido)propanamido)-2-(2-((2S,3R,4R,5S,6S)-6-carboxy-3,4,5-trihydroxytetrahydro-2H -pyran-2-yl)ethyl)benzyl)oxy)carbonyl)((S)-3,4-dihydroxybutyl)amino)ethoxy)-5,7-dimethyladamantan-1-yl)methyl)-5-methyl-1H-pyrazol-4-yl)-6-(8-(benzo[d]thiazol-2-ylcarbamoyl)-3,4-dihydroisoquinolin-2(1H)-yl)picolinic acid

Example 2.167.1 was prepared by substituting Example 2.123.19 for (9H-fluoren-9-yl)methyl ((S)-3-methyl-1-(((S)-1-((4-((((4-nitrophenoxy)carbonyl)oxy)methyl)phenyl)amino)-1-oxo-5-ureidopentan-2-yl)amino)-1-oxobutan-2-yl)carbamate and substituting Example 1.85 for Example 1.2.9 in Example 2.49.1. MS (ESI) m/e 1355.5 (M-H)⁻.

### 2.167.2 2,6-anhydro-8-(2-{[({2-[(3-{[4-(6-{8-[(1,3-benzothiazol-2-yl)carbamoyl]-3,4-dihydroisoquinolin-2(1H)-yl}-2-carboxypyridin-3-yl)-5-methyl-1H-pyrazol-1-yl]methyl)-5,7-dimethyltricyclo[3.3.1.13,7]decan-l-yl)oxy]ethyl}[(3S)-3,4-dihydroxybutyl]carbamoyl)oxy]methyl}-5-{[(2S)-2-({(2S)-2-[2-(2,5-dioxo-2,5-dihydro-1H-pyrrol-1-yl)acetamido]-3-methylbutanoyl}amino)propanoyl]amino}phenyl)-7,8-dideoxy-L-glycero-L-gulo-octonic acid

Example 2.167.2 was prepared by substituting Example 2.167.1 for Example 2.49.1 in Example 2.54. ¹H NMR (501 MHz, dimethyl sulfoxide-*d₆*) δ 9.90 (d, 1H), 8.25 (m, 2H), 8.01 (d, 1H), 7.77 (d, 1H), 7.59 (d, 1H), 7.51 - 7.40 (m, 4H), 7.40 - 7.31 (m, 3H), 7.26 (s, 1H), 7.20 (d, 1H), 7.05 (s, 2H), 6.93 (d, 1H), 4.96 (d, 4H), 4.36 (t, 1H), 4.22 - 4.06 (m, 3H), 3.85 (t, 2H), 3.26 - 3.17 (m, 4H), 3.14-2.88 (m, 5H), 2.78 - 2.55 (m, 2H), 2.10 - 1.88 (m, 5H), 1.69 - 1.49 (m, 2H), 1.39 - 0.73 (m, 28H). MS (ESI) m/e 1492.5 (M-H)⁻.

### 2.168 Synthesis of 2-{[({2-[(3-{[4-(6-{8-[(1,3-benzothiazol-2-yl)carbamoyl]-3,4-dihydroisoquinolin-2(1H)-yl}-2-carboxypyridin-3-yl)-5-methyl-1H-pyrazol-1-yl]methyl}-5,7-dimethyltricyclo[3.3.1.13,7]decan-1-yl)oxy]ethyl}[(3S)-3,4-dihydroxybutyl]carbamoyl)oxy]methyl}-5-{4-[2-(2,5-dioxo-2,5-dihydro-1H-pyrrol-1-yl)acetamido]butyl}phenyl beta-D-glucopyranosiduronic acid (Synthon AAE)

### 2.168.1 3-(1-((3-(2-((((4-(4-aminobutyl)-2-(((2S,3R,4S,5S,6S)-6-carboxy-3,4,5-trihydroxytetrahydro-2H-pyran-2-yl)oxy)benzyl)oxy)carbonyl)((S)-3,4-dihydroxybutyl)amino)ethoxy)-5,7-dimethyladamantan-1-yl)methyl)-5-methyl-1H-pyrazol-4-yl)-6-(8-(benzo[d]thiazol-2-ylcarbamoyl)-3,4-dihydroisoquinolin-2(1H)-yl)picolinic acid

Example 2.168.1 was prepared by substituting Example 2.124.5 for (9H-fluoren-9-yl)methyl ((S)-3-methyl-1-(((S)-1-((4-((((4-nitrophenoxy)carbonyl)oxy)methyl)phenyl)amino)-1-oxo-5-ureidopentan-2-yl)amino)-1-oxobutan-2-yl)carbamate and substituting Example 1.85 for Example 1.2.9 in Example 2.49.1. MS (ESI) m/e 1229.5 (M-H)⁻.

### 2.168.2 2-{[({2-[(3-{[4-(6-{8-[(1,3-benzothiazol-2-yl)carbamoyl]-3,4-dihydroisoquinolin-2(1H)-yl}-2-carboxypyridin-3-yl)-5-methyl-1H-pyrazol-1-yl]methyl}-5,7-dimethyltricyclo[3.3.1.13,7]decan-1-yl)oxy]ethyl}[(3S)-3,4-dihydroxybutyl]carbamoyl)oxy]methyl}-5-{4-[2-(2,5-dioxo-2,5-dihydro-1H-pyrrol-1-yl)acetamido]butyl}phenyl beta-D-glucopyranosiduronic acid

Example 2.168.2 was prepared by substituting Example 2.168.1 for Example 2.49.1 in Example 2.54. ¹H NMR (501 MHz, dimethyl sulfoxide-*d₆*) δ 8.07 (s, 1H), 8.01 (dt, 1H), 7.77 (dt, 1H), 7.63 - 7.57 (m, 1H), 7.51 - 7.39 (m, 3H), 7.38 - 7.31 (m, 2H), 7.26 (s, 1H), 7.16 (d, 1H), 7.05 (s, 2H), 6.93 (d, 2H), 6.84 - 6.80 (m, 1H), 5.14 - 4.98 (m, 3H), 4.94 (s, 2H), 3.79 (d, 2H), 3.48 - 3.19 (m, 10H), 3.08 - 2.96 (m, 4H), 2.52 (s, 4H), 2.07 (s, 2H), 1.77 - 0.72 (m, 14H). MS (ESI) m/e 1366.5 (M-H)⁻.

### 2.169 Synthesis of 6-{8-[(1,3-benzothiazol-2-yl)carbamoyl]-3,4-dihydroisoquinolin-2(1H)-yl}-3-{1-[(3-{2-[{[(4-{[(2S)-5-(carbamoylamino)-2-{[(2S)-2-{[6-(2,5-dioxo-2,5-dihydro-1H-pyrrol-1-yl)hexanoyl]amino}-3-methylbutanoyl]amino}pentanoyl]amino} phenyl)methoxy]carbonyl}(2-sulfoethyl)amino]acetamido}-5,7-dimethyltricyclo[3.3.1.1^{3,7}]decan-1-yl)methyl]-5-methyl-1H-pyrazol-4-yl}pyridine-2-carboxylic acid (Synthon ABG)

The title compound was prepared as described in Example 2.54, replacing Example 2.49.1 with Example 1.89.12. ¹H NMR (501 MHz, dimethyl sulfoxide-*d*₆) δ ppm 9.95 (d, 1H), 8.10 - 7.96 (m, 1H), 7.75 (t, 2H), 7.57 (dd, 3H), 7.51 - 7.18 (m, 8H), 6.95 (d, 3H), 6.92 (s, 0H), 5.03 - 4.86 (m, 4H), 4.36 (d, 1H), 3.85 (t, 2H), 3.78 - 3.67 (m, 4H), 3.42 (s, 2H), 3.33 (t, 2H), 3.04 - 2.86 (m, 4H), 2.63 (d, 2H), 2.13 (dd, 1H), 2.07 (s, 3H), 1.98 - 1.87 (m, 0H), 1.71 - 1.23 (m, 10H), 1.24 - 0.85 (m, 6H), 0.78 (t, 11H). MS (ESI) m/e 1463.5 (M-H)⁻.

### 2.170 Synthesis of N-[6-(2,5-dioxo-2,5-dihydro-1H-pyrrol-1-yl)hexanoyl]-L-valyl-N-{4-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.13,7]dec-1-yl}sulfanyl)ethyl](2-sulfoethyl)carbamoyl}oxy)methyl]phenyl}-N5-carbamoyl-L-ornithinamide (Synthon ABL)

The title compound was prepared by substituting Example 1.90.11 for Example 1.2.9 in Example 2.1. ¹H NMR (500 MHz, dimethyl sulfoxide-*d₆*) δ ppm 10.0 (s, 1H), 8.08 (br s, 1H), 8.03 (d, 1H), 7.81 (br s, 1H) 7.78 (d, 1H), 7.60 (m, 3H) 7.52 (t, 1H), 7.47 (t, 1H), 7.43 (d, 1H), 7.37 (d, 1H), 7.34 (d, 1H) 7.32 (s, 1H), 7.28 (d, 2H), 6.99 (s, 1H), 6.96 (d, 2H), 5.00 (s, 2H), 4.96 (s, 2H), 4.39 (m, 1H), 4.18 (m, 2H), 3.88 (m, 2H), 3.82 (s, 1H), 3.77 (s, 1H), 3.46 (br m, 2H), 3.58 (t, 2H), 3.29 (v br m, 2H), 3.01 (br m, 3H), 2.95 (br m, 1H), 2.47 (m, 2H), 2.61 (brm, 2H) 2.16 (m, 1H), 2.10 (m, 4H), 1.96 (br m, 1H), 1.69 (v br m, 1H), 1.59 (v br m, 1H), 1.53-1.40 (m, 7H), 1.39-1.22 (m, 5H), 1.17 (m, 3H), 1.13-0.88 (m, 6H), 0.87-0.77 (m, 9H), 0.75 (s, 3H). MS (ESI) m/e 1466.5 (M-H)⁻.

### 2.171 Synthesis of N-[6-(2,5-dioxo-2,5-dihydro-1H-pyrrol-1-yl)hexanoyl]-L-valyl-N-[4-({[(3-{3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.13,7]dec-1-yl}propyl)(2-sulfoethyl)carbamoyl]oxy}methyl)phenyl]-N5-carbamoyl-L-ornithinamide (Synthon ABN)

The title compound was prepared as described in Example 2.1, replacing Example 1.2.9 with Example 1.91.13. ¹H NMR (501 MHz, DMSO-*d*₆) δ ppm 12.83 (s, 1H), 9.96 (s, 1H), 8.03 (t, 2H), 7.77 (d, 2H), 7.64 - 7.52 (m, 3H), 7.45 (ddd, 3H), 7.34 (td, 2H), 7.29 - 7.21 (m, 3H), 7.03 - 6.91 (m, 3H), 4.95 (d, 4H), 4.37 (q, 1H), 4.17 (s, 1H), 3.86 (t, 2H), 3.45 - 3.29 (m, 4H), 3.10 (t, 2H), 2.95 (dt, 4H), 2.61 (q, 2H), 2.15 (td, 2H), 2.07 (s, 3H), 2.00 - 1.89 (m, 1H), 1.74 - 1.24 (m, 10H), 1.25 - 0.87 (m, 13H), 0.88 - 0.70 (m, 12H). MS (ESI) m/e 1450.2 (M+H)⁺.

### 2.172 Synthesis of 2-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.13,7]dec-1-yl}oxy)ethyl][(3S)-3,4-dihydroxybutyl]carbamoyl}oxy)methyl]-5-{4-[(1(3S,5S)-3-(2,5-dioxo-2,5-dihydro-1H-pyrrol-1-yl)-2-oxo-5-[(2-sulfoethoxy)methyl]pyrrolidin-1-yl}acetyl)amino]butyl}phenyl beta-D-glucopyranosiduronic acid (Synthon AAF)

The title compound was prepared as described in Example 2.119.17, replacing Example 2.168.1 for Example 2.119.16. ¹H NMR (400 MHz, dimethyl sulfoxide-*d₆*) δ ppm 8.03 (d, 1H), 7.84 (br t, 1H), 7.78 (d, 1H), 7.61 (d, 1H), 7.50 (br d, 1H), 7.45 (dd, 1H), 7.43 (d, 1H), 7.36 (m, 2H), 7.29 (s, 1H), 7.17 (br m, 1H), 7.06 (s, 2H), 6.95 (m, 2H), 6.85 (d, 1H), 5.08 (s, 2H), 5.02 (d, 1H), 4.96 (s, 2H), 4.70 (t, 1H), 4.06 (d, 2H), 3.88 (m, 4H), 3.81 (m, 2H), 3.73 (br m, 1H), 3.62 (m, 2H), 3.47 (br m, 4H), 3.40 (m, 4H), 3.35 (m, 2H), 3.29 (m, 4H), 3.07 (m, 2H), 3.00 (t, 2H), 2.73 (m, 2H), 2.54 (m, 2H), 2.36 (br m, 1H), 2.09 (s, 3H), 1.83 (m, 1H), 1.71 (br m, 1H), 1.55 (br m, 2H), 1.40 (br m, 5H), 1.24 (br m, 4H), 1.10 (br m, 5H), 0.94 (br m, 1H), 0.83, 0.81 (both s, total 6H). MS (ESI) m/e 1587.5 (M-H)⁻.

### 2.173 Synthesis of 2,6-anhydro-8-[2-({[{2-[(3-{[4-(6-{8-[(1,3-benzothiazol-2-yl)carbamoyl]-3,4-dihydroisoquinolin-2(1H)-yl}-2-carboxypyridin-3-yl)-5-methyl-1H-pyrazol-1-yl]methyl}-5,7-dimethyltricyclo[3.3.1.13,7]decan-1-yl)oxy]ethyl}(2-sulfoethyl)carbamoyl]oxy}methyl)-5-{[N-({(3R,5S)-3-(2,5-dioxo-2,5-dihydro-1H-pyrrol-1-yl)-2-oxo-5-[(2-sulfoethoxy)methyl]pyrrolidin-1-yl}acetyl)-L-valyl-L-alanyl]amino}phenyl]-7,8-dideoxy-L-glycero-L-gulo-octonic acid (Synthon ABO)

### 2.173.1 (3R,6R,7aS)-6-azido-3-phenyltetrahydropyrrolo[1,2-c]oxazol-5(3H)-one

The title compound was prepared by substituting Example 2.119.3 for Example 2.119.2 in Example 2.119.4. MS (DCI) m/e 262.0 (M+NH₄)⁺.

### 2.173.2 (3R,6R,7aS)-6-amino-3-phenyltetrahydropyrrolo[1,2-c]oxazol-5(3H)-one

The title compound was prepared by substituting Example 2.173.1 for Example 2.119.4 in Example 2.119.5. MS (DCI) m/e 219.0 (M+H)⁺.

### 2173.3 (3R,6R,7aS)-6-(dibenzylamino)-3-phenyltetrahydropyrrolo[1,2-c]oxazol-5(3H)-one

The title compound was prepared by substituting Example 2.173.2 for Example 2.119.5 in Example 2.119.6. MS (DCI) m/e 399.1 (M+H)⁺.

### 2.173.4 (3R,5S)-3-(dibenzylamino)-5-(hydroxymethyl)pyrrolidin-2-one

The title compound was prepared by substituting Example 2.173.3 for Example 2.119.6 in Example 2.119.7, with the exception that the reaction was heated to 65 °C for one day rather than 6 days. MS (DCI) m/e 311.1 (M+H)⁺.

### 2.173.5 (3R,5S)-5-(((tert-butyldimethylsilyl)oxy)methyl)-3-(dibenzylamino)pyrrolidin-2-one

The title compound was prepared by substituting Example 2.173.4 for Example 2.119.7 in Example 2.119.8. The title compound was carried on to the next step without purification. MS (DCI) m/e 425.2 (M+H)⁺.

### 2.173.6 tert-butyl 2-((3R,5S)-5-(((tert-butyldimethylsilyl)oxy)methyl)-3-(dibenzylamino)-2-oxopyrrolidin-1-yl)acetate

The title compound was prepared by substituting Example 2.173.5 for Example 2.119.8 in Example 2.119.9. The title compound was carried on to the next step without purification. MS (DCI) m/e 539.3 (M+H)⁺.

### 2.173.7 tert-butyl 2-((3R,5S)-3-(dibenzylamino)-5-(hydroxymethyl)-2-oxopyrrolidin-1-yl)acetate

The title compound was prepared by substituting Example 2.173.6 for Example 2.119.9 in Example 2.119.10. MS (DCI) m/e 425.2 (M+H)⁺.

### 2.173.8 tert-butyl 2-((3R,5S)-5-((2-((4-((tert-butyldiphenylsilyl)oxy)-2,2-dimethylbutoxy)sulfonyl)ethoxy)methyl)-3-(dibenzylamino)-2-oxopyrrolidin-1-yl)acetate

The title compound was prepared by substituting Example 2.173.7 for Example 2.119.10 in Example 2.119.11.

### 2.173.9 tert-butyl (S)-2-(2-((2-((4-((tert-butyldiphenylsilyl)oxy)-2,2-dimethylbutoxy)sulfonyl)ethoxy)methyl)-5-oxopyrrolidin-1-yl)acetate

The title compound was prepared by substituting Example 2.173.8 for Example 2.119.11 in Example 2.119.12. MS (ESI) m/e 691.1 (M+H)⁺.

### 2.173.10 4-(((3R,5S)-1-(2-(tert-butoxy)-2-oxoethyl)-5-((2-((4-((tert-butyldiphenylsilyl)oxy)-2,2-dimethylbutoxy)sulfonyl)ethoxy)methyl)-2-oxopyrrolidin-3-yl)amino)-4-oxobut-2-enoic acid

The title compound was prepared by substituting Example 2.173.9 for Example 2.119.12 in Example 2.119.13. MS (ESI) m/e 789.0 (M+H)⁺.

### 2.173.11 tert-butyl 2-((3R,5S)-5-((2-((4-((tert-butyldiphenylsilyl)oxy)-2,2-dimethylbutoxy)sulfonyl)ethoxy)methyl)-3-(2,5-dioxo-2,5-dihydro-1H-pyrrol-1-yl)-2-oxopyrrolidin-1-yl)acetate

The title compound was prepared by substituting Example 2173.10 for Example 2.119.13 in Example 2.119.14.

### 2.173.12 2-((3R,5S)-3-(2,5-dioxo-2,5-dihydro-1H-pyrrol-1-yl)-2-oxo-5-((2-sulfoethoxy)methyl)pyrrolidin-1-yl)acetic acid

The title compound was prepared by substituting Example 2.173.11 for Example 2.119.14 in Example 2.119.15. MS (ESI) m/e 377.0 (M+H)⁺.

### 2.173.13 2,6-anhydro-8-[2-({[{2-[(3-{[4-(6-{8-[(1,3-benzothiazol-2-yl)carbamoyl]-3,4-dihydroisoquinolin-2(1H)-yl}-2-carboxypyridin-3-yl)-5-methyl-1H-pyrazol-1-yl]methyl}-5,7-dimethyltricyclo[3.3.1.13,7]decan-1-yl)oxy]ethyl}(2-sulfoethyl)carbamoyl]oxy}methyl)-5-{[N-({(3R,5S)-3-(2,5-dioxo-2,5-dihydro-1H-pyrrol-1-yl)-2-oxo-5-[(2-sulfoethoxy)methyl]pyrrolidin-1-yl}acetyl)-L-valyl-L-alanyl]amino}phenyl]-7,8-dideoxy-L-glycero-L-gulo-octonic acid

The title compound was prepared by substituting Example 2.123.20 for Example 2.119.16 and Example 2.173.12 for Example 2.119.15 in Example 2.119.17. ¹H NMR (400 MHz, dimethyl sulfoxide-*d₆*) δ ppm 9.94 (d, 1H), 8.28 (br d, 1H), 8.01 (d, 2H), 7.77 (d, 1H), 7.59 (d, 1H), 7.53 (d, 1H), 7.43 (m, 4H), 7.34 (m, 3H), 7.19 (d, 1H), 7.06 (s, 2H), 6.96 (d, 1H), 4.99 (m, 2H), 4.95 (s, 2H), 4.78 (t, 1H), 4.36 (t, 1H), 4.19 (br m, 1H), 4.16 (d, 1H), 3.98 (d, 1H), 3.87 (br t, 2H), 3.81 (br d, 2H), 3.73 (brm, 1H), 3.63 (t, 2H), 3.53 (m, 2H), 3.44 (m, 4H), 3.31 (t, 2H), 3.21 (br m, 2H), 3.17 (m, 2H), 3.00 (m, 2H), 2.92 (br m, 1H), 2.75 (m, 3H), 2.65 (br m, 3H), 2.35 (br m, 1H), 2.16 (m, 1H), 2.07 (s, 3H), 1.98 (br m, 2H), 1.55 (br m, 1H), 1.34 (br m, 1H), 1.26 (br m, 6H), 1.09 (br m, 7H), 0.93 (br m, 1H), 0.87, 0.83, 0.79 (all d, total 12H). MS (ESI) m/e 1733.3 (M-H)⁻.

### 2.174 Synthesis of 2,6-anhydro-8-{2-({[{2-[(3-{[4-(6-{8-[(l,3-benzothiazol-2-yl)carbamoyl]-3,4-dihydroisoquinolin-2(1H)-yl}-2-carboxypyridin-3-yl)-5-methyl-1H-pyrazol-1-yl]methyl}-5,7-dimethyltricyclo[3.3.1.13,7]decan-1-yl)oxy]ethyl}(2-sulfoethyl)carbamoyl]oxy}methyl)-5-[(N-{[(3R,5S)-3-(2,5-dioxo-2,5-dihydro-1H-pyrrol-1-yl)-2-oxo-5-(41-oxo-2,5,8,11,14,17,20,23,26,29,32,35,38-tridecaoxa-42-azatritetracontan-43-yl)pyrrolidin-1-yl]acetyl}-L-valyl-L-alanyl)amino]phenyl}-7,8-dideoxy-L-glycero-L-gulo-octonic acid (Synthon ABM)

### 2.174.1 tert-butyl [(3R,5S)-5-{[bis(tert-butoxycarbonyl)amino] methyl}-3-(dibenzylamino)-2-oxopyrrolidin-1-yl]acetate

To a cold (0 °C) solution of Example 2.173.7 (1.6 g) in dichloromethane (15 mL) was added triethylamine (0.70 mL) and methanesulfonyl chloride (0.39 mL) dropwise. The ice-bath was removed, and the reaction was stirred at room temperature for two hours. The reaction was quenched by the addition of saturated aqueous sodium bicarbonate solution. The layers were separated, and the organic layer was washed with brine. The combined aqueous layers were back-extracted with dichloromethane. The combined organic layers were dried with anhydrous sodium sulfate, filtered and concentrated under reduced pressure to give the intermediate mesylate (1.9 g). The residue was dissolved in acetonitrile (15 mL), and di-tert-butyl-iminodicarboxylate (1.0 g) and cesium carbonate (2.4 g) were added. The reaction was heated to reflux under nitrogen for one day. The reaction was cooled and quenched by the addition of water and diethyl ether. The layers were separated, and the organic was washed with brine. The combined aqueous layers were back-extracted with diethyl ether. The combined organic layers were dried with anhydrous sodium sulfate, filtered and concentrated under reduced pressure. The residue was purified by silica gel chromatography, eluting with 20% ethyl acetate in heptanes, to give the title compound. MS (DCI) m/e 624.3 (M+H)⁺.

### 2.174.2 tert-butyl [(3R,5S)-3-amino-5-{[bis(tert-butoxycarbonyl) amino]methyl}-2-oxopyrrolidin-1-yl]acetate

To a solution of Example 2.174.1 (1.0 g) in ethyl acetate (6 mL) and methanol (18 mL) was added palladium hydroxide on carbon (100 mg, 20% by weight). The reaction was stirred at room temperature under a hydrogen balloon for one day. The reaction was filtered through diatomaceous earth, eluting with ethyl acetate. The filtrate was concentrated under reduced pressure, dissolved in dichloromethane (10 mL) and filtered through a syringe-tip Teflon 40 micron filter. The filtrate was concentrated under reduced pressure to give the title compound. MS (DCI) m/e 444.1 (M+H)⁺.

### 2.174.3 4-{[(3R,5S)-5-{[bis(tert-butoxycarbonyl)amino]methyl}-1-(2-tert-butoxy-2-oxoethyl)-2-oxopyrrolidin-3-yl]amino}-4-oxobut-2-enoic acid

The title compound was prepared by substituting Example 2.174.2 for Example 2.119.12 in Example 2.119.13. MS (ESI) m/e 540.2 (M-H)⁻.

### 2.174.4 tert-butyl [(3R,5S)-5-{[bis(tert-butoxycarbonyl)amino] methyl}-3-(2,5-dioxo-2,5-dihydro-1H-pyrrol-1-yl)-2-oxopyrrolidin-1-yl]acetate

The title compound was prepared by substituting Example 2.174.3 for Example 2.119.13 in Example 2.119.14. MS (DCI) m/e 541.1 (M+NH₄)⁺.

### 2.174.5 2-((3R,5S)-5-(aminomethyl)-3-(2,5-dioxo-2,5-dihydro-1H-pyrrol-1-yl)-2-oxopyrrolidin-1-yl)acetic acid

To a solution of Example 2.174.4 (284 mg) in dichloromethane (10 mL) was added trifluoroacetic acid (5 mL). The reaction was stirred at room temperature for two hours and was concentrated under reduced pressure. The residue was dissolved in water/acetonitrile 7/3 (5 mL), frozen and lyophilized to provide the title compound, which was used in the subsequent step without further purification. MS (ESI) m/e 266.1 (M-H)⁻.

### 2.174.6 2-((3R,5S)-3-(2,5-dioxo-2,5-dihydro-1H-pyrrol-1-yl)-2-oxo-5-(41-oxo-2,5,8,11,14,17,20,23,26,29,32,35,38-tridecaoxa-42-azatritetracontan-43-yl)pyrrolidin-1-yl)acetic acid

To a solution of 2,5,8,11,14,17,20,23,26,29,32,35,38-tridecaoxahentetracontan-41-oic acid (160 mg) in N,N-dimethylformamide (1.0 mL) was added O-(7-azabenzotriazol-1-yl)-N,N,N',N'-tetramethyluronium hexafluorophosphate (85 mg) and N,N-diisopropylethylamine (130 µL). The reaction mixture was stirred for three minutes at room temperature, and a solution of Example 2.174.5 (70 mg) and N,N-diisopropylethylamine (130 µL) in N,N-dimethylformamide (1.0 mL) was added. The reaction was stirred at room temperature for one hour and diluted with N,N-dimethylformamide/water 1/1 (3.5 mL). The solution was purified by reverse phase HPLC on a Gilson system (C18 column), eluting with 20-70% acetonitrile in 0.1% TFA water, to provide the title compound. MS (ESI) m/e 880.4 (M-H)⁻.

### 2.174.7 2,6-anhydro-8-{2-({[{2-[(3-{[4-(6-{8-[(1,3-benzothiazol-2-yl)carbamoyl]-3,4-dihydroisoquinolin-2(1H)-yl}-2-carboxypyridin-3-yl)-5-methyl-1H-pyrazol-1-yl]methyl}-5,7-dimethyltricyclo[3.3.1.1^{3,7}]decan-1-yl)oxy]ethyl)(2-sulfoethyl)carbamoyl]oxy}methyl)-5-[(N-{[(3R,5S)-3-(2,5-dioxo-2,5-dihydro-1H-pyrrol-1-yl)-2-oxo-5-(41-oxo-2,5,8,11,14,17,20,23,26,29,32,35,38-tridecaoxa-42-azatritetracontan-43-yl)pyrrolidin-1-yl]acetyl}-L-valyl-L-alanyl)amino]phenyl}-7,8-dideoxy-L-glycero-L-gulo-octonic acid

The title compound was prepared by substituting Example 2.174.6 for Example 2.119.15 and Example 2.123.20 for Example 2.119.16 in Example 2.119.17 ¹H NMR (500 MHz, dimethyl sulfoxide-*d₆*) δ ppm 9.93 (br d, 1H), 8.28 (d, 1H), 8.03 (d, 1H), 8.02 (br s, 1H), 7.91 (br d, 1H), 7.79 (d, 1H), 7.61 (d, 1H), 7.51 (br d, 1H), 7.49-7.42 (m, 3H), 7.40 (br d, 1H), 7.36 (m, 2H), 7.28 (s, 1H), 7.22 (d, 1H), 7.06 (s, 2H), 6.95 (d, 1H), 5.00 (br d, 2H), 4.95 (s, 2H), 4.70 (t, 1H), 4.39 (m, 1H), 4.28 (m, 1H), 4.00 (dd, 2H), 3.88 (br m, 2H), 3.85 (br m, 1H), 3.80 (br m, 2H), 3.62 (t, 2H), 3.50 (s, 44H), 3.48 (d, 4H), 3.43 (br m, 2H), 3.34 (br m, 2H), 3.23 (s, 3H), 3.21 (v br m, 2H), 3.14 (t, 2H), 3.10 (v br m, 1H), 3.00 (t, 2H), 2.94 (br m, 1H), 2.76 (v br m, 1H), 2.64 (v br m, 3H), 2.34 (br t, 2H), 2.32 (m, 1H), 2.17 (m, 1H), 2.09 (br d, 3H), 2.00 (br m, 1H), 1.56 (br m, 1H), 1.39-1.19 (br m, 8H), 1.19-0.92 (br m, 8H), 0.88 (br d, 3H), 0.87 (br m, 1H), 0.82 (br d, 6H), 0.79 (br s, 3H). MS (ESI) m/e 1119.2 [(M-2H)/2]⁻.

### 2.175 Synthesis of (6S)-2,6-anhydro-6-(2-{2-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo[3.3.1.13,7]dec-1-yl}oxy)ethyl][(3S)-3,4-dihydroxybutyl]carbamoyl}oxy)methyl]-5-({N-[(2,5-dioxo-2,5-dihydro-1H-pyrrol-1-yl)acetyl]-N-(2,5,8,11,14,17,20,23,26,29,32-undecaoxatetratriacontan-34-yl)-b-alanyl-L-valyl-L-alanyl}amino)phenyl}ethyl)-L-gulonic acid (Synthon ABU)

The title compound was prepared using the procedure in Example 2.147.4, replacing Example 2.141.4 with Example 2.167.1. MS (ESI) m/e 1033.4 (M+2H)²⁺.

### 2.176 Synthesis of (6S)-2,6-anhydro-6-(2-{2-[({[2-({3-[(4-{6-[8-(1,3-benzothiazol-2-ylcarbamoyl)-3,4-dihydroisoquinolin-2(1H)-yl]-2-carboxypyridin-3-yl}-5-methyl-1H-pyrazol-1-yl)methyl]-5,7-dimethyltricyclo [3.3.1.13,7]dec-1-yl}oxy)ethyl][(3S)-3,4-dihydroxybutyl]carbamoyl}oxy)methyl]-5-({N-[(2,5-dioxo-2,5-dihydro-1H-pyrrol-1-yl)acetyl]-N-[2-(2-sulfoethoxy)ethyl]-b-alanyl-L-valyl-L-alanyl}amino)phenyl}ethyl)-L-gulonic acid (Synthon ABV)

The title compound was prepared using the procedure in Example 2.160.7, replacing Example 2.154.1 with Example 2.167.1. MS (ESI) m/e 859.4 (M+2H)²⁺.

### Example 3. Synthesis of Exemplary Bcl-xL Inhibitory ADCs

Exemplary ADCs were synthesized using one of nine exemplary methods, described below. Table 6 correlates which method was used to synthesize each exemplary ADC.

**Method A**. A solution of Bond-Breaker^{™} tris(2-carboxyethyl)phosphine (TCEP) solution (10 mM, 0.017 mL) was added to a solution of antibody (10 mg/mL, 1 mL) preheated to 37 °C. The reaction mixture was kept at 37 °C for 1 hour. The solution of reduced antibody was added to a solution of synthon (3.3 mM, 0.160 mL in dimethyl sulfoxide (DMSO)) and gently mixed for 30 minutes. The reaction solution was loaded onto a desalting column (PD10, washed with DPBS 3x before use), followed by Dulbecco's phosphate-buffered saline (DPBS) (1.6 mL) and eluted with additional DPBS (3 mL). The purified ADC solution was filtered through a 0.2 micron, low protein-binding 13 mm syringe-filter and stored at 4 °C.

**Method B**. A solution of Bond-Breaker^{™} tris(2-carboxyethyl)phosphine (TCEP) solution (10 mM, 0.017 mL) was added to the solution of antibody (10 mg/mL, 1 mL) preheated to 37 °C. The reaction mixture was kept at 37 °C for 1 hour. The solution of reduced antibody was adjusted to pH=8 by adding boric buffer (0.05 mL, 0.5 M, pH8), added to a solution of synthon (3.3 mM, 0.160 mL in DMSO) and gently mixed for 4 hours. The reaction solution was loaded onto a desalting column (PD10, washed with DPBS 3x before use), followed by DPBS (1.6 mL) and eluted with additional DPBS (3 mL). The purified ADC solution was filtered through a 0.2 micron, low protein-binding 13 mm syringe-filter and stored at 4 °C.

**Method C**. Conjugations were performed using a PerkinElmer Janus (part AJL8M01) robotic liquid handling system equipped with an 1235/96 tip ModuLar Dispense Technology (MDT), disposable head (part 70243540) containing a gripper arm (part 7400358), and an 8-tip Varispan pipetting arm (part 7002357) on an expanded deck. The PerkinElmer Janus system was controlled using the WinPREP version 4.8.3.315 Software.

A Pall Filter plate 5052 was prewet with 100 µL 1x DPBS using the MDT. Vacuum was applied to the filter plate for 10 seconds and was followed by a 5 second vent to remove DPBS from filter plate. A 50% slurry of Protein A resin (GE MabSelect Sure) in DPBS was poured into an 8 well reservoir equipped with a magnetic ball, and the resin was mixed by passing a traveling magnet underneath the reservoir plate. The 8 tip Varispan arm, equipped with 1mL conductive tips, was used to aspirate the resin (250 µL) and transfer to a 96-well filter plate. A vacuum was applied for 2 cycles to remove most of the buffer. Using the MDT, 150 µL of 1xPBS was aspirated and dispensed to the 96-well filter plate holding the resin. A vacuum was applied, removing the buffer from the resin. The rinse/vacuum cycle was repeated 3 times. A 2 mL, 96-well collection plate was mounted on the Janus deck, and the MDT transferred 450 µL of 5x DPBS to the collection plate for later use. Reduced antibody (2 mg) as a solution in (200 µL) DPBS was prepared as described above for Conditions A and preloaded into a 96 well plate. The solutions of reduced antibody were transferred to the filter plate wells containing the resin, and the mixture was mixed with the MDT by repeated aspiration/dispensation of a 100 µL volume within the well for 45 seconds per cycle. The aspiration/dispensation cycle was repeated for a total of 5 times over the course of 5 minutes. A vacuum was applied to the filter plate for 2 cycles, thereby removing excess antibody. The MDT tips were rinsed with water for 5 cycles (200 µL, 1 mL total volume). The MDT aspirated and dispensed 150 µL of DPBS to the filter plate wells containing resin-bound antibody, and a vacuum was applied for two cycles. The wash and vacuum sequence was repeated two more times. After the last vacuum cycle, 100 µL of 1x DPBS was dispensed to the wells containing the resin-bound antibody. The MDT then collected 30 µL each of 3.3 mM dimethyl sulfoxide solutions of synthons plated in a 96-well format and dispensed it to the filter plate containing resin-bound antibody in DPBS. The wells containing the conjugation mixture were mixed with the MDT by repeated aspiration/dispensation of a 100 µL volume within the well for 45 seconds per cycle. The aspiration/dispensation sequence was repeated for a total of 5 times over the course of 5 minutes. A vacuum was applied for 2 cycles to remove excess synthon to waste. The MDT tips were rinsed with water for 5 cycles (200 µL, 1 mL total volume). The MDT aspirated and dispensed DPBS (150 µL) to the conjugation mixture, and a vacuum was applied for two cycles. The wash and vacuum sequence was repeated two more times. The MDT gripper then moved the filter plate and collar to a holding station. The MDT placed the 2 mL collection plate containing 450 µL of 10x DPBS inside the vacuum manifold. The MDT reassembled the vacuum manifold by placement of the filter plate and collar. The MDT tips were rinsed with water for 5 cycles (200 µL, 1 mL total volume). The MDT aspirated and dispensed 100 µL of IgG Elution Buffer 3.75 (Pierce) to the conjugation mixture. After one minute, a vacuum was applied for 2 cycles, and the eluent was captured in the receiving plate containing 450 µL of 5x DPBS. The aspiration/dispensation sequence was repeated 3 additional times to deliver ADC samples with concentrations in the range of 1.5-2.5 mg/mL at pH 7.4 in DPBS.

**Method D.** Conjugations were performed using a PerkinElmer Janus (part AJL8M01) robotic liquid handling system equipped with an 1235/96 tip ModuLar Dispense Technology (MDT), disposable head (part 70243540) containing a gripper arm (part 7400358), and an 8-tip Varispan pipetting arm (part 7002357) on an expanded deck. The PerkinElmer Janus system was controlled using the WinPREP version 4.8.3.315 Software.

A Pall Filter plate 5052 was prewet with 100 µL 1x DPBS using the MDT. Vacuum was applied to the filter plate for 10 seconds and was followed by a 5 second vent to remove DPBS from filter plate. A 50% slurry of Protein A resin (GE MabSelect Sure) in DPBS was poured into an 8-well reservoir equipped with a magnetic ball, and the resin was mixed by passing a traveling magnet underneath the reservoir plate. The 8 tip Varispan arm, equipped with 1mL conductive tips, was used to aspirate the resin (250 µL) and transfer to a 96-well filter plate. A vacuum was applied to the filter plate for 2 cycles to remove most of the buffer. The MDT aspirated and dispensed 150 µL of DPBS to the filter plate wells containing the resin. The wash and vacuum sequence was repeated two more times. A 2 mL, 96-well collection plate was mounted on the Janus deck, and the MDT transferred 450 µL of 5x DPBS to the collection plate for later use. Reduced antibody (2 mg) as a solution in (200 µL) DPBS was prepared as described above for Method A and dispensed into the 96-well plate. The MDT then collected 30 µL each of 3.3 mM dimethyl sulfoxide solutions of synthons plated in a 96-well format and dispensed it to the plate loaded with reduced antibody in DPBS. The mixture was mixed with the MDT by twice repeated aspiration/dispensation of a 100 µL volume within the well. After five minutes, the conjugation reaction mixture (230 µL) was transferred to the 96-well filter plate containing the resin. The wells containing the conjugation mixture and resin were mixed with the MDT by repeated aspiration/dispensation of a 100 µL volume within the well for 45 seconds per cycle. The aspiration/dispensation sequence was repeated for a total of 5 times over the course of 5 minutes. A vacuum was applied for 2 cycles to remove excess synthon and protein to waste. The MDT tips were rinsed with water for 5 cycles (200 µL, 1 mL total volume). The MDT aspirated and dispensed DPBS (150 µL) to the conjugation mixture, and a vacuum was applied for two cycles. The wash and vacuum sequence was repeated two more times. The MDT gripper then moved the filter plate and collar to a holding station. The MDT placed the 2 mL collection plate containing 450 L of 10x DPBS inside the vacuum manifold. The MDT reassembled the vacuum manifold by placement of the filter plate and collar. The MDT tips were rinsed with water for 5 cycles (200 µL, 1 mL total volume). The MDT aspirated and dispensed 100 µL of IgG Elution Buffer 3.75 (P) to the conjugation mixture. After one minute, a vacuum was applied for 2 cycles, and the eluent was captured in the receiving plate containing 450 µL of 5x DPBS. The aspiration/dispensation sequence was repeated 3 additional times to deliver ADC samples with concentrations in the range of 1.5-2.5 mg/mL at pH 7.4 in DPBS.

**Method E**. A solution of Bond-Breaker^{™} tris(2-carboxyethyl)phosphine (TCEP) solution (10 mM, 0.017 mL) was added to the solution of antibody (10 mg/mL, 1 mL) at room temperature. The reaction mixture was heated to 37 °C for 75 minutes. The solution of reduced antibody cooled to room temperature and was added to a solution of synthon (10 mM, 0.040 mL in DMSO) followed by addition of boric buffer (0.1 mL, 1M, pH 8). The reaction solution was let to stand for 3 days at room temperature, loaded onto a desalting column (PD10, washed with DPBS 3x5mL before use), followed by DPBS (1.6 mL) and eluted with additional DPBS (3 mL). The purified ADC solution was filtered through a 0.2 micron, low protein-binding 13 mm syringe-filter and stored at 4 C.

**Method F**. Conjugations were performed using a Tecan Freedom Evo robotic liquid handling system. The solution of antibody (10 mg/mL) was preheated to 37 °C and aliquoted to a heated 96 deep-well plate in amounts of 3 mg per well (0.3 mL) and kept at 37 °C. A solution of Bond-Breaker^{™} tris(2-carboxyethyl)phosphine (TCEP) solution (1 mM, 0.051 mL/well) was added to antibodies, and the reaction mixture was kept at 37 °C for 75 minutes. The solution of reduced antibody was transferred to an unheated 96 deep-well plate. Corresponding solutions of synthons (5 mM, 0.024 mL in DMSO) were added to the wells with reduced antibodies and treated for 15 minutes. The reaction solutions were loaded onto a platform (8 × 12) of desalting columns (NAP5, washed with DPBS 4x before use), followed by DPBS (0.3 mL) and eluted with additional DPBS (0.8 mL). The purified ADC solutions were further aliquoted for analytics and stored at 4 C.

**Method G**. Conjugations were performed using a Tecan Freedom Evo robotic liquid handling system. The solution of antibody (10 mg/mL) was preheated to 37 °C and aliquoted onto a heated 96 deep-well plate in amounts of 3 mg per well (0.3 mL) and kept at 37 °C. A solution of Bond-Breaker^{™} tris(2-carboxyethyl)phosphine (TCEP) solution (1 mM, 0.051 mL/well) was added to antibodies, and the reaction mixture was kept at 37 °C for 75 minutes. The solutions of reduced antibody were transferred to an unheated 96 deep-well plate. Corresponding solutions of synthons (5 mM, 0.024 mL/well in DMSO) were added to the wells with reduced antibodies followed by addition of boric buffer (pH=8, 0.03 mL/well) and treated for 3 days. The reaction solutions were loaded onto a platform (8 × 12) of desalting columns (NAP5, washed with DPBS 4x before use), followed by DPBS (0.3 mL) and eluted with additional DPBS (0.8 mL). The purified ADC solutions were further aliquoted for analytics and stored at 4 °C.

**Method H**. A solution of Bond-Breaker^{™} tris(2-carboxyethyl)phosphine (TCEP) solution (10 mM, 0.17 mL) was added to the solution of antibody (10 mg/mL, 10 mL) at room temperature. The reaction mixture was heated to 37 °C for 75 minutes. The solution of synthon (10 mM, 0.40 mL in DMSO) was added to a solution of reduced antibody cooled to room temperature. The reaction solution was let to stand for 30 minutes at room temperature. The solution of ADC was treated with saturated ammonium sulfate solution (~2 - 2.5 mL) until a slightly cloudy solution formed. This solution was loaded onto butyl sepharose column (5 mL of butyl sepharose) equilibrated with 30% phase B in phase A (phase A: 1.5 M ammonium sulfate, 25 mM phosphate; phase B: 25 mM phosphate, 25% isopropanol v/v). Individual fractions with DAR2 (also referred to as "E2") and DAR4 (also referred to as "E4") eluted upon applying gradient A/B up to 75% phase B. Each ADC solution was concentrated and buffer switched using centrifuge concentrators or TFF for larger scales. The purified ADC solutions were filtered through a 0.2 micron, low protein-binding 13 mm syringe-filter and stored at 4 C.

**Method I**. A solution of Bond-Breaker^{™} tris(2-carboxyethyl)phosphine (TCEP) solution (10 mM, 0.17 mL) was added to the solution of antibody (10 mg/mL, 10 mL) at room temperature. The reaction mixture was heated to 37 °C for 75 minutes. The solution of synthon (10 mM, 0.40 mL in DMSO) was added to a solution of reduced antibody cooled to room temperature. The reaction solution was let to stand for 30 minutes at room temperature. The solution of ADC was treated with saturated ammonium sulfate solution (~2 - 2.5 mL) until a slightly cloudy solution formed. This solution was loaded onto a butyl sepharose column (5 mL of butyl sepharose) equilibrated with 30% phase B in Phase A (phase A: 1.5 M ammonium sulfate, 25 mM phosphate; phase B: 25 mM phosphate, 25% isopropanol v/v). Individual fractions with DAR2 (also referred to as "E2") and DAR 4 (also referred to as "E4") eluted upon applying a gradient A/B up to 75% phase B. Each ADC solution was concentrated and buffer switched using centrifuge concentrators or TFF for larger scales. The ADC solutions were treated with boric buffer (0.1 mL, 1M, pH8). The reaction solution was let stand for 3 days at room temperature, then loaded onto a desalting column (PD10, washed with DPBS 3x5mL before use), followed by DPBS (1.6 mL) and eluted with additional DPBS (3 mL). The purified ADC solution was filtered through a 0.2 micron, low protein-binding 13 mm syringe-filter and stored at 4 C.

Table 6 below indicates which exemplary ADCs were synthesized via which exemplary method. The AbB, AbG, AbK, AbA1 are affinity matured variants of Abl described in Table 2. Monoclonal antibody to CMV glycoprotein H (MSL109) is an isotype matched non-targeting control.

**Table 6: Synthetic Methods Used to Synthesize Exemplary ADCs**

| **Ex. No.** | **ADC** | **Method** |
|---|---|---|
| 3.1 | AbA-CZ | G |
| 3.2 | AbA-TX | G |
| 3.3 | AbA-TV | G |
| 3.4 | AbA-YY | G |
| 3.5 | AbA-AAA | G |
| 3.6 | AbA-AAD | G |
| 3.7 | AbB-CZ | G |
| 3.8 | AbB-TX | G |
| 3.9 | AbB-TV | G |
| 3.10 | AbB-YY | G |
| 3.11 | AbB-AAD | G |
| 3.12 | AbG-CZ | G |
| 3.13 | AbG-TX | G |
| 3.14 | AbG-TV | G |
| 3.15 | AbG-YY | G |
| 3.16 | AbG-AAA | G |
| 3.17 | AbG-AAD | G |
| 3.18 | AbK-CZ | G |
| 3.19 | AbK-TX | G |
| 3.20 | AbK-TV | G |
| 3.21 | AbK-YY | G |
| 3.22 | AbK-AAA | G |
| 3.23 | AbK-AAD | G |
| 3.24 | MSL109-CZ | G |
| 3.25 | MSL109-TX | G |
| 3.26 | MSL109-TV | G |
| 3.27 | MSL109-YY | G |
| 3.28 | MSL109-AAA | G |
| 3.29 | MSL109-AAD | G |
| 3.30 | AbA-WD | E |
| 3.31 | AbA-LB | A |
| 3.32 | AbB-WD | E |
| 3.33 | AbB-LB | A |
| 3.34 | AbG-WD | E |
| 3.35 | AbG-LB | A |
| 3.36 | AbK-WD | E |
| 3.37 | AbK-LB | A |
| 3.38 | MSL109-WD | E |
| 3.39 | MSL109-LB | A |
| 3.40 | AbA-ZT | G |
| 3.41 | AbA-ZZ | G |
| 3.42 | AbA-XW | G |
| 3.43 | AbA-SE | A |
| 3.44 | AbA-SR | A |
| 3.45 | AbA-YG | E |
| 3.46 | AbA-KZ | A |
| 3.47 | AbB-ZT | G |
| 3.48 | AbB-ZZ | G |
| 3.49 | AbB-XW | G |
| 3.50 | AbB-SE | A |
| 3.51 | AbB-SR | A |
| 3.52 | AbB-YG | E |
| 3.53 | AbB-KZ | A |
| 3.54 | AbG-ZT | G |
| 3.55 | AbG-ZZ | G |
| 3.56 | AbG-XW | G |
| 3.57 | AbG-SE | A |
| 3.58 | AbG-SR | A |
| 3.59 | AbG-YG | E |
| 3.60 | AbG-KZ | A |
| 3.61 | AbK-ZT | G |
| 3.62 | AbK-ZZ | G |
| 3.63 | AbK-XW | G |
| 3.64 | AbK-SE | A |
| 3.65 | AbK-SR | A |
| 3.66 | AbK-YG | E |
| 3.67 | AbK-KZ | A |
| 3.68 | MSL109-ZT | G |
| 3.69 | MSL109-ZZ | G |
| 3.70 | MSL109-XW | G |
| 3.71 | MSL109-SE | A |
| 3.72 | MSL109-SR | A |
| 3.73 | MSL109-YG | E |
| 3.74 | MSL109-KZ | A |

### Example 4. Drug to Antibody Ratio (DAR) and Aggregation of Exemplary ADCs

The DAR and percentage aggregation of exemplary ADCs synthesized as described in Example 3, above, were determined by LC-MS and size exclusion chromatography (SEC), respectively.

### 4.1 LC-MS General Methodology

LC-MS analysis was performed using an Agilent 1100 HPLC system interfaced to an Agilent LC/MSD TOF 6220 ESI mass spectrometer. The ADC was reduced with 5 mM (final concentration) BOND BREAKER TCEP solution (Thermo Scientific, Rockford, IL), loaded onto a Protein Microtrap (Michrom Bioresorces, Auburn, CA) desalting cartridge, and eluted with a gradient of 10% B to 75% B in 0.2 minutes at ambient temperature. Mobile phase A was H₂0 with 0.1% formic acid (FA), mobile phase B was acetonitrile with 0.1% FA, and the flow rate was 0.2 ml/min. Electrospray-ionization time-of-flight mass spectra of the co-eluting light and heavy chains were acquired using Agilent MassHunter(TM) acquisition software. The extracted intensity vs. m/z spectrum was deconvoluted using the Maximum Entropy feature of MassHunter software to determine the mass of each reduced antibody fragment. DAR was calculated from the deconvoluted spectrum by summing intensities of the naked and modified peaks for the light chain and heavy chain, normalized by multiplying intensity by the number of drugs attached. The summed, normalized intensities were divided by the sum of the intensities, and the summing results for two light chains and two heavy chains produced a final average DAR value for the full ADC.

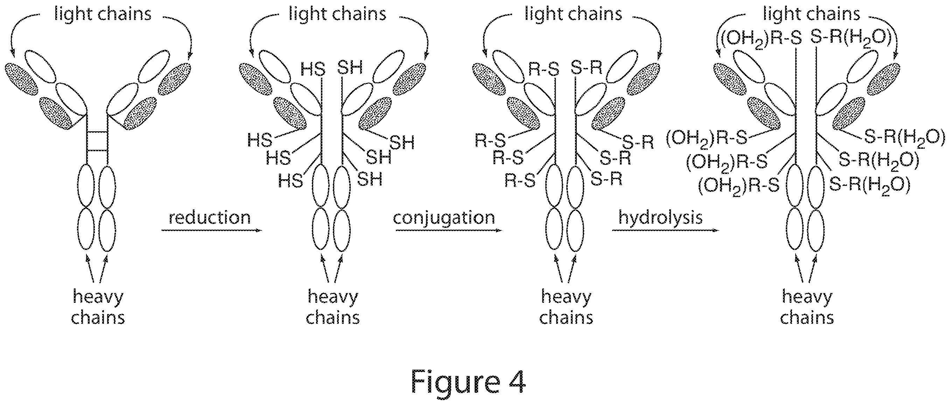

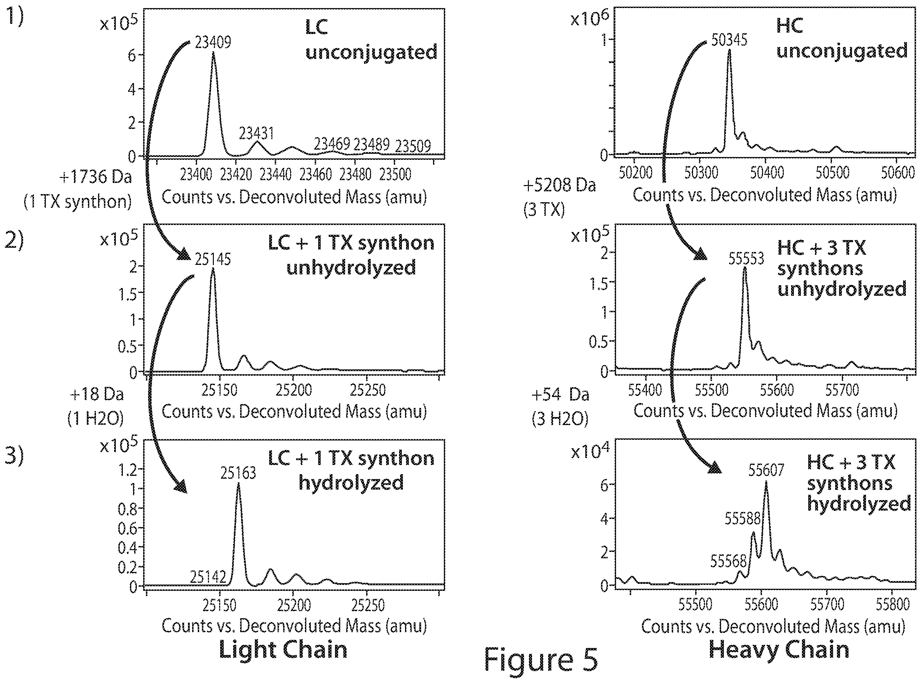

Thiosuccinimide hydrolysis of a bioconjugate can be monitored by electrospray mass spectrometry, since the addition of water to the conjugate results in an increase of 18 Daltons to the observable molecular weight of the conjugate. When a conjugate is prepared by fully reducing the interchain disulfides of a human IgG1 antibody and conjugating the maleimide derivative to each of the resulting cysteines, each light chain of the antibody will contain a single maleimide modification and each heavy chain will contain three maleimide modifications, as described in Figure 4. Upon complete hydrolysis of the resulting thiosuccinimides, the mass of the light chain will therefore increase by 18 Daltons, while the mass of each heavy chain will increase by 54 Daltons. This is illustrated in Figure 5, with the conjugation and subsequent hydrolysis of an exemplary maleimide drug-linker (synthon TX, molecular weight 1736 Da) to the fully reduced AbA antibody. The presence of the single N-linked glycosylation site on heavy chain results in the heterogeneity of mass observed in the unconjugated antibody.

Figure 5 shows MS characterization of light chain and heavy chain of an exemplary antibody Aba 1) prior to conjugation, 2) after conjugation to a maleimide derivative to give a thiosuccinimide intermediate and 3) post pH8-mediated hydrolysis of the thiosuccinimide ring.

### 4.2 Size Exclusion Chromatography General Methodology

Size exclusion chromatography (SEC) was performed using a Shodex KW802.5 column in 0.2M potassium phosphate pH 6.2 with 0.25 mM potassium chloride and 15% isopropyl alcohol at a flow rate of 0.75 ml/min. The peak area absorbance at 280 nm was determined for each of the high molecular weight and monomeric eluents by integration of the area under the curve. The % aggregate fraction of the conjugate sample was determined by dividing the peak area absorbance at 280 nM for the high molecular weight eluent by the sum of the peak area absorbances at 280 nM of the high molecular weight and monomeric eluents multiplied by 100%.

### 4.3. Results

The average DAR values were determined using the above LC-MS method. The % aggregate fraction for the ADCs was also determined using the SEC method described in Example 4.2. The DAR and % aggregation are both reported below in Table 7.

**Table 7: ADC Analytical Characterization**

| **Appln Ex. No.** | **ADC Code** | **DAR (by MS)** | **%Agg (by SEC)** |
|---|---|---|---|
| 3.1 | AbA-CZ | 3.2 | 4.7 |
| 3.2 | AbA-TX | 2.8 | 0.7 |
| 3.3 | AbA-TV | 3.7 | 2.4 |
| 3.4 | AbA-YY | 2.2 | 18.8 |
| 3.5 | AbA-AAA | 2 | 19 |
| 3.6 | AbA-AAD | 3.3 | 3.6 |
| 3.7 | AbB-CZ | 3.5 | 0 |
| 3.8 | AbB-TX | 2.2 | 0 |
| 3.9 | AbB -TV | 2.3 | 0.7 |
| 3.10 | AbB -YY | 2.2 | 0 |
| 3.11 | AbB -AAD | 2.7 | 0 |
| 3.12 | AbG-CZ | 3.4 | 4 |
| 3.13 | AbG-TX | 3.3 | 1.6 |
| 3.14 | AbG-TV | 3.7 | 1.4 |
| 3.15 | AbG-YY | 2.2 | 16.5 |
| 3.16 | AbG-AAA | 1.9 | 17.5 |
| 3.17 | AbG-AAD | 3.4 | 2.5 |
| 3.18 | AbK-CZ | 3.4 | 3.3 |
| 3.19 | AbK-TX | 2.2 | 1.6 |
| 3.20 | AbK-TV | 2.4 | 2.6 |
| 3.21 | AbK-YY | 1.7 | 20 |
| 3.22 | AbK-AAA | 1.6 | 20.4 |
| 3.23 | AbK-AAD | 2.8 | 3.6 |
| 3.24 | MSL109-CZ | 3.4 | 4.1 |
| 3.25 | MSL109-TX | 3.5 | 0.7 |
| 3.26 | MSL109-TV | 4.2 | 0.7 |
| 3.27 | MSL109-YY | 2.3 | 17.5 |
| 3.28 | MSL109-AAA | 2.2 | 17.7 |
| 3.29 | MSL109-AAD | 3.6 | 2.9 |
| 3.30 | AbA-WD | 1.8 | 0 |
| 3.31 | AbA-LB | 2.4 | 14.5 |
| 3.32 | AbB-WD | 1.6 | 0 |
| 3.33 | AbB-LB | 1.8 | 0 |
| 3.34 | AbG-WD | 3.4 | 3.2 |
| 3.35 | AbG-LB | 2.5 | 15.3 |
| 3.36 | AbK-WD | 1.7 | 4.9 |
| 3.37 | AbK-LB | 1.8 | 13.6 |
| 3.38 | MSL109-WD | 2.9 | 0 |
| 3.39 | MSL109-LB | 1.8 | 0 |
| 3.40 | AbA-ZT | 2 | 17.1 |
| 3.41 | AbA-ZZ | 1.3 | 19.2 |
| 3.42 | AbA-XW | 3.7 | 6.6 |
| 3.43 | AbA-SE | 2.8 | 0 |
| 3.44 | AbA-SR | 2.3 | 37.1 |
| 3.45 | AbA-YG | 1.9 | 0 |
| 3.46 | AbA-KZ | 2 | 4.4 |
| 3.47 | AbB -ZT | 1.4 | 0 |
| 3.48 | AbB -ZZ | 1.1 | 0 |
| 3.49 | AbB -XW | 3.2 | 0 |
| 3.50 | AbB -SE | 2.2 | 0 |
| 3.51 | AbB -SR | 2.1 | 0 |
| 3.52 | AbB -YG | 1.1 | 0 |
| 3.53 | AbB -KZ | 1.9 | 0 |
| 3.54 | AbG-ZT | 1.6 | 12.4 |
| 3.55 | AbG-ZZ | 1.4 | 16.8 |
| 3.56 | AbG-XW | 3.7 | 5.9 |
| 3.57 | AbG-SE | 3.8 | 2.1 |
| 3.58 | AbG-SR | 2.8 | 36.7 |
| 3.59 | AbG-YG | 3.7 | 2.4 |
| 3.60 | AbG-KZ | 2.7 | 11.6 |
| 3.61 | AbK-ZT | 1.3 | 13.4 |
| 3.62 | AbK-ZZ | 1.9 | 4.5 |
| 3.63 | AbK-XW | 2.8 | 6.2 |
| 3.64 | AbK-SE | 2.7 | 2.5 |
| 3.65 | AbK-SR | 2.3 | 30.1 |
| 3.66 | AbK-YG | 0.9 | 0 |
| 3.67 | AbK-KZ | 2.3 | 10.2 |
| 3.68 | MSL109-ZT | 2.3 | 7.5 |
| 3.69 | MSL109-ZZ | 1.4 | 15 |
| 3.70 | MSL109-XW | 3.3 | 3.7 |
| 3.71 | MSL109-SE | 3.6 | 33.4 |
| 3.72 | MSL109-SR | 1.8 | 2.3 |
| 3.73 | MSL109-YG | 3.1 | 13.2 |
| 3.74 | MSL109-KZ | 2.5 | 18 |

### Example 5. EGFR-Targeted ADCs Inhibit the Growth of Cancer Cells In Vitro

The cytotoxicity of the anti-EGFR antibodies AbB, AbG, AbK, and AbL as Bcl-xL ADCs against the EGFR positive non-small cell lung cancer cells (NCI-H1650) was compared to non-targeting MSL109 isotype matched exemplary ADCs. To further evaluate the in vitro efficacy of these exemplary EGFR-targeted Bcl-xL-ADCs, human EGFR was over-expressed in *mcl-1*^{*-*/*-*} mouse embryonic fibroblasts (MEFs). Mcl-1 refers to the gene myeloid cell leukemia 1. *Mcl-1*^{*-*/*-*} MEFs are dependent upon Bcl-xL for survival (Lessene et al., 2013, Nature Chemical Biology 9:390-397).

### 5.1 Methods

Retroviral supernatants were produced through transfection of the GP2-293 packaging cell line (Clontech) with the retroviral construct pLVC-IRES-Hygro (Clontech) containing huEGFR sequence or the empty vector utilizing FuGENE 6 transfection reagent (Roche Molecular Biochemicals, Mannheim, Germany). After 48 hours of culture, virus-containing supernatant was harvested and applied to *mcl-1*^{*-*/*-*} MEFs in 75 cm² culture flasks (0.5×10⁶ per flask) for a further 48 hours in the presence of polybrene (8 µg/ml; Sigma). *Mcl-1*^{*-*/*-*} MEFs were washed and selected after 3 days with 250 µg/ml hygromycin B (Invitrogen) in the full complement of media. The expression of huEGFR was confirmed by flow cytometry and compared to the parental cell line or those transfected with the empty vector.

*Mcl-1*^{*-*/*-*} MEFs expressing huEGFR or the pLVX empty vector (Vct Ctrl) were treated with AB033-targeted Bcl-xL-ADCs, AB033 alone or MSL109-targeted Bcl-xL-ADCs for 96 hours in DMEM containing 10% FBS. For the assay, the cells were plated at 250 cells per well in 384-well tissue culture plates (Corning, Corning, NY) in a total volume of 25 µL of assay media (DMEM and 10% HI FBS). The plated cells were treated with a 4-fold serial dilution of the Antibody Drug Conjugates of interest from either 1 µM or 0.5 µM to either 1 50 pM or 25 pM, respectively 1 µM to 1 pM dispensed by an Echo 550 Acoustic Liquid Handler (Labcyte). Each concentration was tested in at least three replicates for the *Mcl-1*^{*-*/*-*} MEF huEGFR cell line and for the *Mcl-1*^{*-*/*-*} MEF vector cell line. The fraction of viable cells following 96 hours of Antibody Drug Conjugate treatment at 37 °C and 5% CO₂ was determined using the CellTiter-Glo Luminescent Cell Viability Assay according to the manufacturer's recommendations (Promega Corp., Madison, WI). The plates were read in a Perkin Elmer Envision using a Luminescence protocol with 0.1 sec integration time. The replicate values for each dilution point were averaged and the EC₅₀ values for the Antibody Drug Conjugates were generated by fitting the data with GraphPad Prism 5 (GraphPad Software, Inc.) to a sigmoidal curve model using linear regression, Y=((Bottom-Top)/(1+((x/K)ⁿ)))+Top, where Y is the measured response, x is the compound concentration, n is the Hill Slope and K is the EC₅₀ and Bottom and Top are the lower and higher asymptotes respectively. Visual inspection of curves was used to verify curve fit results. *Mcl-1*^{*-*/*-*} MEFs were obtained from David C. S. Huang of the Walter and Eliza Hall Institute of Medical Research.

NCI-H1650 cells stably overexpressing eGFP were maintained in RPMI media (Invitrogen cat#22400) containing 10% Fetal Bovine Serum (Invitrogen cat# 10082). The cells were removed from plates with Trypsin and plated at 300 cells/well in 25 µL of the same media in Corning 384 well spheroid plates (cat# 3830). The plates were centrifuged at 500 × g for 5 minutes and placed in an Essen INCUCYTE Zoom Live Cell Analysis System in a 37°C with 5% CO₂ and 95% humidity. The cells were allowed to form spheroids for 3 days before dosing with an equal volume of the antibody drug conjugates at 2X the indicated concentration. The spheroids were incubated for an additional 6 days while monitoring growth and GFP Fluorescence in the Incucyte Zoom prior to addition of 40 µL of Promega CELLTITER-GLO 3D (cat# G968B) and subsequent luminescent reading. *IC₅₀*s were determined from both the final GFP fluorescence monitored by the Incucyte Zoom (referred to as "H1650 GFP Flourescence EC₅₀ (µg/mL)" in Table 8) and the chemiluminescent readings from the CellTiter-Glo reagent (referred to as "H1650 CTG-3D EC₅₀ (µg/mL)" in Table 8).

### 5.2 Results

Cell viability assay results (EC₅₀ in nanomolar or µg/mL) for representative ADCs are provided below in Table 8.

**Table 8: In Vitro Cell Viability Efficacy of Exemplary EGFR-Targeted ADCs**

| **ADC Code.** | **DAR** | **huEGFR⁺ *mcl-1*^{*-*/*-*} MEF ECso (µM)** | ***mcl-1*^{*-*/*-*} MEF Vector Control ECso (µM)** | **H1650 GFP Flourescence EC₅₀ (µg/mL)** | **H1650 CTG-3D EC₅₀ (µg/mL)** |
|---|---|---|---|---|---|
| AbA-CZ | 3.2 | 0.0003 | >0.5 | 1.24 | 0.88 |
| AbA-TX | 2.8 | 0.016 | >0.5 | 13.88 | >40 |
| AbA-TV | 3.7 | 0.003 | 0.364 | 0.69 | 0.34 |
| AbA-YY | 2.2 | 0.46 | >0.5 | 7.85 | >40 |
| AbA-AAA | 2 | 0.22 | 0.306 | 0.98 | 0.65 |
| AbA-AAD | 3.3 | 0.065 | >0.5 | 0.36 | 0.79 |
| AbB-CZ | 3.5 | 0.0059 | 0.104 | 0.88 | 1.27 |
| AbB-TX | 2.2 | 0.011 | 0.491 | 3.72 | 2.39 |
| AbB-TV | 2.3 | 0.0024 | 0.31 | 0.74 | 0.86 |
| AbB-YY | 2.2 | 0.051 | 0.4 | 7.73 | 8.6 |
| AbB-AAD | 2.7 | 0.0046 | >10 | 0.59 | 0.16 |
| AbG-CZ | 3.4 | 0.0034 | 0.194 | 0.25 | 0.07 |
| AbG-TX | 3.3 | 0.0053 | 0.368 | 0.51 | 0.15 |
| AbG-TV | 3.7 | 0.0026 | 0.196 | 0.04 | 0.03 |
| AbG-YY | 2.2 | >0.5 | >0.5 | 1.1 | 0.4 |
| AbG-AAA | 1.9 | 0.22 | >0.5 | 0.17 | 0.12 |
| AbG-AAD | 3.4 | 0.108 | >0.5 | 0.03 | 0.02 |
| AbK-CZ | 3.4 | 0.0001 | >0.5 | 0.21 | 0.08 |
| AbK-TX | 2.2 | 0.00079 | >1.0 | 1.47 | 1.07 |
| AbK-TV | 2.4 | 0.00015 | 0.455 | 0.16 | 0.05 |
| AbK-YY | 1.7 | 0.047 | >1.0 | 1.09 | 0.49 |
| AbK-AAA | 1.6 | 0.0034 | >1.0 | 0.16 | 0.11 |
| AbK-AAD | 2.8 | 0.0004 | >0.5 | 0.06 | 0.01 |
| MSL109-CZ | 3.4 | 0.087 | 0.154 | >40 | >40 |
| MSL109-TX | 3.5 | 0.191 | >0.5 | 24 | 22 |
| MSL109-TV | 4.2 | 0.143 | 0.411 | >40 | >40 |
| MSL109-YY | 2.3 | >0.5 | >0.5 | >40 | >40 |
| MSL109-AAA | 2.2 | >0.5 | >0.5 | >40 | >40 |
| MSL109-AAD | 3.6 | >0.5 | >0.5 | >40 | >40 |
| AbA-WD | 1.8 | 0.304 | NT | 7.62 | 23.59 |
| AbA-LB | 2.4 | 0.027 | NT | 2.48 | 4.05 |
| AbB-WD | 1.6 | 0.023 | NT | 1.01 | 2.69 |
| AbB-LB | 1.8 | 0.031 | NT | 2.38 | 1.07 |
| AbG-WD | 3.4 | 0.0058 | NT | 0.36 | 2.03 |
| AbG-LB | 2.5 | 0.024 | NT | 1.62 | 1.68 |
| AbK-WD | 1.7 | 0.035 | NT | 2.57 | 4.36 |
| AbK-LB | 1.8 | 0.017 | NT | 1.33 | 1.23 |
| MSL109-WD | 2.9 | 0.22 | NT | 1.791 | 7.668 |
| MSL109-LB | 1.8 | 0.025 | NT | 0.5744 | 0.6426 |
| AbA-ZT | 2 | 0.104 | >0.5 | 1.13 | 0.35 |
| AbA-ZZ | 1.3 | 0.186 | >0.5 | 1.18 | 1.86 |
| AbA-XW | 3.7 | 0.032 | 0.229 | >40 | >40 |
| AbA-SE | 2.8 | 0.105 | NT | 4.23 | 4.00 |
| AbA-SR | 2.3 | 0.0059 | NT | 2.22 | 2.12 |
| AbA-YG | 1.9 | 0.0064 | NT | 2.84 | 3.80 |
| AbA-KZ | 2 | 0.152 | NT | 29.31 | >40 |
| AbB-ZT | 1.4 | 0.0089 | 0.404 | 0.64 | 0.26 |
| AbB-ZZ | 1.1 | 0.0074 | 0.311 | 0.61 | 0.52 |
| AbB-XW | 3.2 | 0.00065 | >0.5 | 12.14 | 6.92 |
| AbB-SE | 2.2 | 0.039 | NT | 2.18 | 0.59 |
| AbB-SR | 2.1 | 0.007 | NT | 2.10 | 0.62 |
| AbB-YG | 1.1 | 0.0033 | NT | 1.55 | 3.28 |
| AbB-KZ | 1.9 | 0.055 | NT | 37.73 | 16.47 |
| AbG-ZT | 1.6 | 0.033 | >0.5 | 0.08 | 0.1 |
| AbG-ZZ | 1.4 | 0.068 | >0.5 | 0.44 | 0.47 |
| AbG-XW | 3.7 | 0.019 | 0.246 | >40 | >40 |
| AbG-SE | 3.8 | 0.024 | NT | 0.99 | 0.96 |
| AbG-SR | 2.8 | 0.007 | NT | 1.38 | 1.31 |
| AbG-YG | 3.7 | 0.001 | NT | 0.63 | 1.24 |
| AbG-KZ | 2.7 | 0.096 | NT | 38.19 | 38.34 |
| AbK-ZT | 1.3 | 0.0002 | >0.5 | 0.12 | 0.05 |
| AbK-ZZ | 1.9 | 0.045 | >0.5 | 0.16 | 0.03 |
| AbK-XW | 2.8 | 0.0006 | >0.5 | 8.53 | >40 |
| AbK-SE | 2.7 | 0.161 | NT | 1.68 | 2.67 |
| AbK-SR | 2.3 | 0.0089 | NT | 1.36 | 1.74 |
| AbK-YG | 0.9 | 0.037 | NT | 10.40 | 12.32 |
| AbK-KZ | 2.3 | 0.224 | NT | >40 | >40 |
| MSL109-ZT | 2.3 | >0.5 | >0.5 | >40 | >40 |
| MSL109-ZZ | 1.4 | >0.5 | >0.5 | >40 | >40 |
| MSL109-XW | 3.3 | 0.297 | 0.494 | >40 | >40 |
| MSL109-SE | 3.6 | >0.5 | NT | 3.692 | 6.079 |
| MSL109-SR | 1.8 | 0.142 | NT | 2.046 | 9.138 |
| MSL109-YG | 3.1 | 0.057 | NT | 3.895 | 5.852 |
| MSL109-KZ | 2.5 | 0.255 | NT | >40 | >40 |

| | | | | | |
|---|---|---|---|---|---|
| NT = not tested | | | | | |

As described above in Table 8, anti-EGFR ADCs comprising an anti-EGFR antibody and a Bcl-xL inhibitor, were effective at reducing cell viability of the human EGFR expressing *mcl1*^{*-*/*-*}fibroblasts with a range of potencies. The anti-EGFR Bcl-xL ADCs also inhibited the growth of NSCLC spheroids (H1650 GFP) as measured by remaining cell fluorescence and reduced viability. In contrast the non-targeting (MSL109) control Bcl-xL ADCs displayed reduced potency as Bcl-xL ADCs.

### Example 6. In Vivo Efficacy of anti-EGFR-BCL-xL ADCs

The *in vivo* anti-tumor activity of the anti-EGFR antibodies AbB, AbG, AbK, AbA as Bcl-xL inhibiting ADCs was evaluated using a murine xenograft non-small cell lung cancer (NSCLC) model. Specifically, EGFR positive NSCLC NCI-H1650 cells (ATCC deposit no. CRL-5883) were grown as a flank xenograft in mice. The activity of ADCs was compared to non-targeting IgG isotype matched antibody (AB095) (a human IgGl antibody recognizing tetanus toxoid; *See* Larrick et al., 1992, Immunological Reviews 69-85) was used as a negative control. The results are presented in Tables 9, 10 and 11 below.

### 6.1 Evaluation of Efficacy in Xenograft Models Methods

The cell line NCI-H1650 was obtained from the American Type Culture Collection (ATCC Deposit No. CRL-5883, Manassas, VA). The cells were cultured as monolayers in RPMI-1640 that was supplemented with 10% Fetal Bovine Serum (FBS, Hyclone, Logan, UT). To generate xenografts, 5×10⁶ viable cells were inoculated subcutaneously into the right flank of immune deficient female SCID/bg mice (Charles River Laboratories, Wilmington, MA) respectively. The injection volume was 0.2 ml and composed of a 1:1 mixture of S MEM and Matrigel (BD, Franklin Lakes, NJ). Tumors were size matched at approximately 200 mm³.

The control antibody and ADCs were formulated in 0.9% sodium chloride for injection and injected intraperitoneally. Injection volume did not exceed 200 µl. Therapy began within 24 hours after size matching of the tumors. Mice weighed approximately 22 g at the onset of therapy. Anti-EGFR ADCs and AB095 were administered intraperitoneally (IP) for a single dose (QDx1) or weekly for a total of six doses (Q7Dx6).

Tumor volume was estimated two to three times weekly. Measurements of the length (L) and width (W) of the tumor were taken via electronic caliper and the volume was calculated according to the following equation: V = L × W²/2. Eight mice were housed per cage. Food and water were available ad libitum. Mice were acclimated to the animal facilities for a period of at least one week prior to commencement of experiments. Animals were tested in the light phase of a 12-hour light: 12-hour dark schedule (lights on at 06:00 hours). Mice were euthanized when tumor volume reached 3,000 mm³ or skin ulcerations occurred.

To refer to efficacy of therapeutic agents, parameters of amplitude (TGIₘₐₓ), durability (TGD) of therapeutic response were used. TGIₘₐₓ is the maximum tumor growth inhibition during the experiment. Tumor growth inhibition was calculated by 100^{∗}(1-Tᵥ/Cᵥ) where Tᵥ and Cᵥ are the mean tumor volumes of the treated and control groups, respectively. TGD or tumor growth delay is the extended time of a treated tumor needed to reach a volume of 1 cm³ relative to the control group (AB095). TGD is calculated by 100^{∗}(Tₜ/Cₜ-1) where Tₜ and Cₜ are the median time periods to reach 1 cm³ of the treated and control groups, respectively.

Certain anti-Bcl-xL inhibiting synthons were conjugated to EGFR targeting antibodies AbA, AbB, AbG and AbK according to the synthetic methods noted in Tables 9, 10 and 11 (and described in the above Examples).

**Table 9. In vivo efficacy of anti-EGFR-BCL-xL ADCs in NCI-H1650 model of NSCLC**

| Drug | Synthetic Method | DAR | Dose (mg/kg/day) | Regimen/Route | N | TGIₘₐₓ (%) | TGD (%) |
|---|---|---|---|---|---|---|---|
| AB095^{∗∗} | - | - | 10 | QDxl / IP | 8 | 0 | 0 |
| AbA-CZ | A | 3.7 | 3 | QDxl / IP | 8 | 81 | 100 |
| AbA-CZ | A | 3.7 | 10 | QDxl / IP | 8 | 99 | 144 |
| ^{∗∗} IgG1 mAb | | | | | | | |

**Table 10 . In vivo efficacy of anti-EGFR-BCL-xL ADCs in NCI-H1650 model of NSCLC**

| Drug | Conjugation Method | DAR | Dose (mg/kg/day) | Regimen/Route | N | TGIₘₐₓ (%) |
|---|---|---|---|---|---|---|
| AB095^{∗∗} | - | - | 10 | QDxl / IP | 8 | 0 |
| AbG-TX | E | 3.4 | 10 | QDxl / IP | 8 | 88 |
| AbG-AAA | E | 3.6 | 10 | QDxl / IP | 8 | 76 |
| AbG-XW | E | 4.2 | 10 | QDxl / IP | 8 | 76 |
| AbK-CZ | A | 3.5 | 10 | QDxl / IP | 8 | 77 |
| AbK-AAA | E | 3.1 | 10 | QDxl / IP | 8 | 84 |
| AbB-CZ | A | 3.5 | 10 | QDxl / IP | 8 | 82 |
| ^{∗∗} IgG1 mAb | | | | | | |

The results provided in Tables 9 and 10 indicate that the anti-EGFR antibodies AbB, AbG, AbK, AbA as Bcl-xL inhibitor ADCs were similarly effective at tumor growth inhibition of the H1650 xenograft non-small cell lung cancer (NSCLC) model.

The *in vivo* anti-tumor activity of anti-EGFR antibodies AbA and AbG were compared as DAR2 (E2) and DAR4 (E4) BCL-xL inhibitor conjugates against the EGFR positive non-small cell lung cancer model NCI-H1650 grown as a flank xenograft in mice. The activity of these ADCs was compared to non-targeting IgG isotype matched antibody (AB095) as control. The results are presented in Table 11. The results shown in Table 11 indicate that the anti-EGFR antibodies AbA and AbG as Bcl-xL ADCs were effective as either purified DAR2 or DAR4 conjugates against the H1650 xenograft model, with TGI and TGD proportional to the total amount of Bcl-xL warhead dosed. Moreover, a comparison of the efficacy of the conjugates listed in Table 11 revealed that the growth inhibition was proportional to the amount of Bcl-xL administered.

**Table 11. In vivo efficacy of anti-EGFR-BCL-xL ADCs in NCI-H1650 model of NSCLC**

| Drug | Conjugation Method | DAR | Dose (mg/kg/day) | Regimen/Route | N | TGIₘₐₓ (%) | TGD (%) |
|---|---|---|---|---|---|---|---|
| AB095^{∗∗} | - | - | 8 | Q7Dx6 / IP | 8 | 0 | 0 |
| AbA-CZ E2 | H | 2 | 2 | Q7Dx6 / IP | 8 | 67 | 93 |
| AbA-CZ E2 | H | 2 | 4 | Q7Dx6 / IP | 8 | 67 | 93 |
| AbA-CZ E2 | H | 2 | 8 | Q7Dx6 / IP | 8 | 83 | 193 |
| AbA-CZ E4 | H | 4 | 1 | Q7Dx6 / IP | 8 | 62 | 75 |
| AbA-CZ E4 | H | 4 | 2 | Q7Dx6 / IP | 8 | 73 | 100 |
| AbA-CZ E4 | H | 4 | 4 | Q7Dx6 / IP | 8 | 77 | 114 |
| AbG-CZ E2 | H | 2 | 2 | Q7Dx6 / IP | 8 | 64 | 93 |
| AbG-CZ E2 | H | 2 | 4 | Q7Dx6 / IP | 8 | 80 | 143 |
| AbG-CZ E2 | H | 2 | 8 | Q7Dx6 / IP | 8 | 75 | 143 |
| AbG-CZ E4 | H | 4 | 1 | Q7Dx6 / IP | 8 | 61 | 64 |
| AbG-CZ E4 | H | 4 | 2 | Q7Dx6 / IP | 8 | 80 | 114 |
| AbG-CZ E4 | H | 4 | 4 | Q7Dx6 / IP | 8 | 74 | 114 |
| ^{∗∗} IgG1 mAb | | | | | | | |

As a control, the *in vivo* anti-tumor activity of an ADC comprising the non-targeting antibody MSL109 (MSL109 is a monoclonal antibody to CMV glycoprotein H) conjugated to Bcl-xL inhibitors was evaluated against the EGFR positive non-small cell lung cancer model NCI-H1650 grown as a flank xenograft in mice. The activity of these ADCs was compared to non-targeting IgG isotype matched antibody (AB095) as control showing very modest tumor growth inhibition and low or no tumor growth delay. The results are presented in Table 12, and show only modest tumor growth inhibition and low or no tumor growth delay caused by of Bcl-xL ADCs that use a non-targeting antibody as a carrier. This low anti-tumor activity is contrasted with much greater TGI and TGD observed with the EGFR-targeting Bcl-xL ADCs (Tables 9 and 10), and reflected the antigen dependent delivery of these ADCs in EGFR expressing models.

**Table 12. In vivo efficacy of non-targeting (MSL109) BCL-xL inhibiting ADCs in NCI-H1650 model ofNSCLC**

| | | **Growth Inhibition** | |
|---|---|---|---|
| **Treatment** | **Dose^{[a]}/route/reg imen** | **TGIₘₐₓ (%)** | **TGD (%)** |
| MSL109^{†}-H | 3/IP/Q4Dx6 | 18^{∗} | 0 |
| MSL109^{†}-H | 10/IP/Q4Dx6 | 43^{∗} | 20^{∗} |
| MSL109^{†}-H | 30/IP/Q4Dx6 | 8 | 0 |
| MSL109^{†}-CZ | 3/IP/Q4Dx6 | 29^{∗} | 0 |
| MSL109^{†}-CZ | 3/IP/Q7Dx6 | 18^{∗} | 0 |
| MSL109^{†}-CZ | 10/IP/Q4Dx6 | 32^{∗} | 16 |
| MSL109^{†}-CZ | 30/IP/Q4Dx6 | 32^{∗} | 12 |
| † Non-targeting antibody | | | |
| ^{[a]} dose is given in mg/kg/day | | | |
| ^{∗} = p < 0.05 as compared to control treatment (AB095) Q4Dx6 indicates one dose every 4 days for a total of 6 doses | | | |

**SEQUENCE TABLE**

| **SEQ ID NO:** | **Description** |
|---|---|
| 1 | Ab 1 VH amino acid sequence |
| 2 | Ab1, AbC, AbD, and AbE VH CDR1 amino acid sequence |
| 3 | Ab1, AbC, AbD, AbE, AbF, AbJ, and AbN VH CDR2 amino acid sequence |
| 4 | Ab1, AbC, AbD, and AbE VH CDR3 amino acid sequence |
| 5 | Ab 1 and AbA VL amino acid sequence |
| 6 | Ab1, AbA, AbB, AbC, and AbF VL CDR1 amino acid sequence |
| 7 | Ab1, AbA, AbB, and AbC, and AbF VL CDR2 amino acid sequence |
| 8 | Ab1, AbA, AbB, and AbF VL CDR3 amino acid sequence |
| 9 | AbA VH amino acid sequence |
| 10 | AbA, AbF, and AbK VH CDR1 amino acid sequence |
| 11 | AbA, AbH, AbK, AbL, AbM, AbO, and AbQ VH CDR2 amino acid sequence |
| 12 | AbA, AbF, AbM, AbN, and AbO VH CDR3 amino acid sequence |
| 13 | Ab 1 and AbA light chain amino acid sequence |
| 14 | Ab 1 heavy chain amino acid sequence |
| 15 | AbA heavy chain amino acid sequence |
| 16 | AbB and AbG VH CDR1 amino acid sequence |
| 17 | AbB and AbG VH CDR2 amino acid sequence |
| 18 | AbG, AbH, AbJ, and AbL VH CDR3 amino acid sequence |
| 19 | AbB and AbK VH CDR3 amino acid sequence |
| 20 | AbM and AbN VH CDR1 amino acid sequence |
| 21 | AbP VH CDR1 amino acid sequence |
| 22 | AbP and AbQ VH CDR3 amino acid sequence |
| 23 | AbG, AbH, and AbJ VL CDR1 amino acid sequence |
| 24 | AbG, AbH, and AbJ VL CDR2 amino acid sequence |
| 25 | AbG, AbH, and AbJ VL CDR3 amino acid sequence |
| 26 | AbK, AbL, AbM, AbN, and AbO VL CDR1 amino acid sequence |
| 27 | AbE, AbK, AbL, AbM, AbN, and AbO VL CDR2 amino acid sequence |
| 28 | AbK, AbL, AbM, AbN, and AbO VL CDR3amino acid sequence |
| 29 | AbP and AbQ VL CDR1 amino acid sequence |
| 30 | AbP and AbQ VL CDR2 amino acid sequence |
| 31 | AbD, AbP, and AbQ VL CDR3 amino acid sequence |
| 32 | Human EGFR amino acid sequence (with signal sequence) |
| 33 | Human Epidermal Growth Factor Receptor variant III (hEGFRvIII) amino acid |
| 34 | Human EGFR extracellular domain (ECD) amino acid sequence |
| 35 | VH CDR1 consensus sequence of AbA, AbG, AbK, AbM, and AbP |
| 36 | VH CDR2 consensus sequence of AbA, AbG, AbK, AbM, and AbP |
| 37 | VH CDR3 consensus sequence of AbA, AbG, AbK, AbM, and AbP |
| 38 | VL CDR1 consensus sequence of AbA, AbG, AbK, AbM, and AbP |
| 39 | VL CDR2 consensus sequence of AbA, AbG, AbK, AbM, and AbP |
| 40 | VL CDR3 consensus sequence of AbA, AbG, AbK, AbM, and AbP |
| 41 | Ig gamma-1 constant region |
| 42 | Ig gamma-1 constant region mutant |
| 43 | Ig kappa constant region |
| 44 | Ig lambda constant region |
| 45 | Epitope of EGFR |
| 46 | ECD of EGFRvIII amino acid sequence |
| 47 | EGFR 1-525 amino acid sequence |
| 48 | Heavy chain amino acid sequence Ab2 |
| 49 | Light chain amino acid sequence Ab2 |
| 50 | VH amino acid sequence AbE |
| 51 | VL amino acid sequence AbE |
| 52 | VH amino acid sequence AbF |
| 53 | VL amino acid sequence AbF |
| 54 | VH amino acid sequence AbH |
| 55 | VL amino acid sequence AbH |
| 56 | VH amino acid sequence AbJ |
| 57 | VL amino acid sequence AbJ |
| 58 | VH amino acid sequence AbL |
| 59 | VL amino acid sequence AbL |
| 60 | VH amino acid sequence AbN |
| 61 | VL amino acid sequence AbN |
| 62 | VH amino acid sequence AbO |
| 63 | VL amino acid sequence AbO |
| 64 | VH amino acid sequence AbB |
| 65 | VL amino acid sequence AbB |
| 66 | VH amino acid sequence AbC |
| 67 | VL amino acid sequence AbC |
| 68 | VH amino acid sequence AbD |
| 69 | VL amino acid sequence AbD |
| 70 | VH amino acid sequence AbQ |
| 71 | VL amino acid sequence AbQ |
| 72 | VH amino acid sequence AbG |
| 73 | VL amino acid sequence AbG |
| 74 | VH amino acid sequence AbK |
| 75 | VL amino acid sequence AbK |
| 76 | VH amino acid sequence AbM |
| 77 | VL amino acid sequence AbM |
| 78 | VH amino acid sequence AbP |
| 79 | VL amino acid sequence AbP |
| 80 | AbH, AbJ, AbL, and AbO VH CDR1 amino acid sequence |
| 81 | AbQ VH CDR1 amino acid sequence |
| 82 | AbD and AbE VL CDR1 amino acid sequence |
| 83 | AbD VL CDR2 amino acid sequence |
| 84 | AbC VL CDR3 amino acid sequence |
| 85 | AbE VL CDR3 amino acid sequence |
| 86 | AbA heavy chain nucleic acid sequence |
| 87 | AbA light chain nucleic acid sequence |
| 88 | Heavy chain amino acid leader sequence |
| 89 | Light chain amino acid leader sequence |
| 90 | AbB heavy chain amino acid sequence |
| 91 | AbB heavy chain amino acid sequence, LALA mutation |
| 92 | AbB light chain amino acid sequence |
| 93 | AbG heavy chain amino acid sequence |
| 94 | AbG heavy chain amino acid sequence, LALA mutation |
| 95 | AbG light chain amino acid sequence |
| 96 | AbK heavy chain amino acid sequence |
| 97 | AbK heavy chain amino acid sequence, LALA mutation |
| 98 | AbK light chain amino acid sequence |

## Claims

1. An anti-human Epidermal Growth Factor Receptor (hEGFR) antibody drug conjugate comprising the following structure: 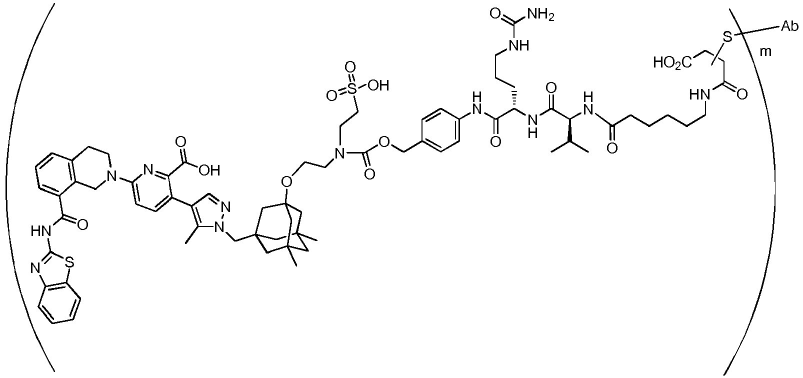 wherein Ab is an IgG1 anti-hEGFR antibody comprising
a heavy chain variable region comprising a heavy chain CDR3 domain comprising the amino acid sequence set forth in SEQ ID NO: 12, a heavy chain CDR2 domain comprising the amino acid sequence set forth in SEQ ID NO: 11, and a heavy chain CDR1 domain comprising the amino acid sequence set forth in SEQ ID NO: 10; and
a light chain variable region comprising a light chain CDR3 domain comprising the amino acid sequence set forth in SEQ ID NO: 8, a light chain CDR2 domain comprising the amino acid sequence set forth in SEQ ID NO: 7, and a light chain CDR1 domain comprising the amino acid sequence set forth in SEQ ID NO: 6; and
wherein m is 2.

2. The antibody drug conjugate according to claim 1, wherein the anti-hEGFR antibody comprises a heavy chain variable region comprising the amino acid sequence set forth in SEQ ID NO: 9 and a light chain variable region comprising the amino acid sequence set forth in SEQ ID NO: 5.

3. The antibody drug conjugate according to claim 1 or 2, wherein the anti-hEGFR antibody comprises a heavy chain comprising the amino acid sequence set forth in SEQ ID NO: 15, and a light chain comprising the amino acid sequence set forth in SEQ ID NO: 13.

## Patentansprüche

1. Antikörper-Wirkstoff-Konjugat gegen einen anti-humanen epidermalen Wachstumsfaktorrezeptor (human Epidermal Growth Factor Receptor - hEGFR), das die folgende Struktur umfasst: 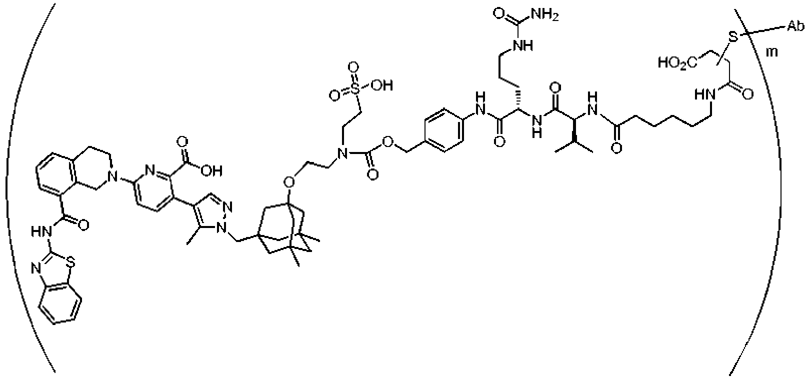 wobei Ab ein IgG1-Anti-hEGFR-Antikörper ist, der Folgendes umfasst:
eine variable Region der schweren Kette, die eine CDR3-Domäne der schweren Kette umfasst, die die in SEQ ID NO: 12 dargelegte Aminosäuresequenz umfasst, eine CDR2-Domäne der schweren Kette, die die in SEQ ID NO: 11 dargelegte Aminosäuresequenz umfasst, und eine CDR1-Domäne der schweren Kette, die die in SEQ ID NO: 10 dargelegte Aminosäuresequenz umfasst; und
eine variable Region der leichten Kette, die eine CDR3-Domäne der leichten Kette, die die in SEQ ID NO: 8 dargelegte Aminosäuresequenz umfasst, eine CDR2-Domäne der leichten Kette, die die in SEQ ID NO: 7 dargelegte Aminosäuresequenz umfasst, und eine CDR1-Domäne der leichten Kette, die die in SEQ ID NO: 6 dargelegte Aminosäuresequenz umfasst; und
wobei m 2 ist.

2. Antikörper-Wirkstoff-Konjugat nach Anspruch 1, wobei der Anti-hEGFR-Antikörper eine variable Region der schweren Kette, die die in SEQ ID NO: 9 dargelegte Aminosäuresequenz umfasst, und eine variable Region der leichten Kette umfasst, die die in SEQ ID NO: 5 dargelegte Aminosäuresequenz umfasst.

3. Antikörper-Wirkstoff-Konjugat nach Anspruch 1 oder 2, wobei der Anti-hEGFR-Antikörper eine schwere Kette, die die in SEQ ID NO: 15 dargelegte Aminosäuresequenz umfasst, und eine leichte Kette umfasst, die die in SEQ ID NO: 13 dargelegte Aminosäuresequenz umfasst.

## Revendications

1. Conjugué anticorps-médicament d'anti-récepteur de facteur de croissance épidermique humain (hEGFR) comprenant la structure suivante : 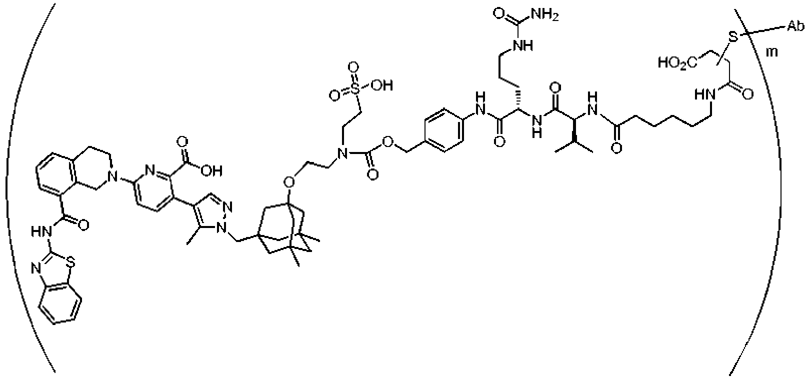 Ab étant un anticorps IgGI anti-hEGFR comprenant
une région variable de chaîne lourde comprenant un domaine CDR3 de chaîne lourde comprenant la séquence d'acides aminés présentée dans SEQ ID N° : 12, un domaine CDR2 de chaîne lourde comprenant la séquence d'acides aminés présentée dans SEQ ID N° : 11, et un domaine CDR1 de chaîne lourde comprenant la séquence d'acides aminés présentée dans SEQ ID N° : 10 ; et
une région variable de chaîne légère comprenant un domaine CDR3 de chaîne légère comprenant la séquence d'acides aminés présentée dans SEQ ID N° : 8, un domaine CDR2 de chaîne légère comprenant la séquence d'acides aminés présentée dans SEQ ID N° : 7, et un domaine CDR1 de chaîne légère comprenant la séquence d'acides aminés présentée dans SEQ ID N° : 6 ; et
m valant 2.

2. Conjugué anticorps-médicament selon la revendication 1, dans lequel l'anticorps anti-hEGFR comprend une région variable de chaîne lourde comprenant la séquence d'acides aminés présentée dans SEQ ID NO° : 9 et une région variable de chaîne légère comprenant la séquence d'acides aminés présentée dans SEQ ID N° : 5.

3. Conjugué anticorps-médicament selon la revendication 1 ou 2, dans lequel l'anticorps anti-hEGFR comprend une chaîne lourde comprenant la séquence d'acides aminés présentée dans SEQ ID N° : 15, et une chaîne légère comprenant la séquence d'acides aminés présentée dans SEQ ID N° : 13.


### SEQUENCE LISTING

<110> ABBVIE INC. BRUNCKO, MILAN
<120> ANTI-EGFR ANTIBODY DRUG CONJUGATES
<130> 117813-13420
<140>
   <141>
<150> 62/347,416 <151> 2016-06-08
<160> 102
<170> PatentIn version 3.5
<210> 1
   <211> 116
   <212> PRT
   <213> Artificial Sequence
<220>
   <221> source
   <223> /note="Description of Artificial Sequence: Synthetic polypeptide"
<400> 1
   Gln Val Gln Leu Gln Glu Ser Gly Pro Gly Leu Val Lys Pro Ser Gln
   1               5                   10                  15
   Thr Leu Ser Leu Thr Cys Thr Val Ser Gly Tyr Ser Ile Ser Ser Asp
               20                  25                  30
   Phe Ala Trp Asn Trp Ile Arg Gln Pro Pro Gly Lys Gly Leu Glu Trp
           35                  40                  45
   Met Gly Tyr Ile Ser Tyr Ser Gly Asn Thr Arg Tyr Gln Pro Ser Leu
       50                  55                  60
   Lys Ser Arg Ile Thr Ile Ser Arg Asp Thr Ser Lys Asn Gln Phe Phe
   65                  70                  75                  80
   Leu Lys Leu Asn Ser Val Thr Ala Ala Asp Thr Ala Thr Tyr Tyr Cys
                   85                  90                  95
   Val Thr Ala Gly Arg Gly Phe Pro Tyr Trp Gly Gln Gly Thr Leu Val
               100                 105                 110
   Thr Val Ser Ser
           115
<210> 2
   <211> 11
   <212> PRT
   <213> Artificial Sequence
<220>
   <221> source
   <223> /note="Description of Artificial Sequence: Synthetic peptide"
<400> 2
   Gly Tyr Ser Ile Ser Ser Asp Phe Ala Trp Asn
   1 5 10
<210> 3
   <211> 16
   <212> PRT
   <213> Artificial Sequence
<220>
   <221> source
   <223> /note="Description of Artificial Sequence: Synthetic peptide"
<400> 3
   Tyr Ile Ser Tyr Ser Gly Asn Thr Arg Tyr Gln Pro Ser Leu Lys Ser
   1 5 10 15
<210> 4
   <211> 7
   <212> PRT
   <213> Artificial Sequence
<220>
   <221> source
   <223> /note="Description of Artificial Sequence: Synthetic peptide"
<400> 4
   Ala Gly Arg Gly Phe Pro Tyr
   1 5
<210> 5
   <211> 107
   <212> PRT
   <213> Artificial Sequence
<220>
   <221> source
   <223> /note="Description of Artificial Sequence: Synthetic polypeptide"
<400> 5
   Asp Ile Gln Met Thr Gln Ser Pro Ser Ser Met Ser Val Ser Val Gly
   1 5 10 15
   Asp Arg Val Thr Ile Thr Cys His Ser Ser Gln Asp Ile Asn Ser Asn
   20 25 30
   Ile Gly Trp Leu Gln Gln Lys Pro Gly Lys Ser Phe Lys Gly Leu Ile
   35 40 45
   Tyr His Gly Thr Asn Leu Asp Asp Gly Val Pro Ser Arg Phe Ser Gly
   50 55 60
   Ser Gly Ser Gly Thr Asp Tyr Thr Leu Thr Ile Ser Ser Leu Gln Pro
   65 70 75 80
   Glu Asp Phe Ala Thr Tyr Tyr Cys Val Gln Tyr Ala Gln Phe Pro Trp
   85 90 95
   Thr Phe Gly Gly Gly Thr Lys Leu Glu Ile Lys
   100 105
<210> 6
   <211> 11
   <212> PRT
   <213> Artificial Sequence
<220>
   <221> source
   <223> /note="Description of Artificial Sequence: Synthetic peptide"
<400> 6
   His Ser Ser Gln Asp Ile Asn Ser Asn Ile Gly
   1 5 10
<210> 7
   <211> 7
   <212> PRT
   <213> Artificial Sequence
<220>
   <221> source
   <223> /note="Description of Artificial Sequence: Synthetic peptide"
<400> 7
   His Gly Thr Asn Leu Asp Asp
   1 5
<210> 8
   <211> 9
   <212> PRT
   <213> Artificial Sequence
<220>
   <221> source
   <223> /note="Description of Artificial Sequence: Synthetic peptide"
<400> 8
   Val Gln Tyr Ala Gln Phe Pro Trp Thr
   1 5
<210> 9
   <211> 116
   <212> PRT
   <213> Artificial Sequence
<220>
   <221> source
   <223> /note="Description of Artificial Sequence: Synthetic polypeptide"
<400> 9
   Glu Val Gln Leu Gln Glu Ser Gly Pro Gly Leu Val Lys Pro Ser Gln
   1 5 10 15
   Thr Leu Ser Leu Thr Cys Thr Val Ser Gly Tyr Ser Ile Ser Arg Asp
   20 25 30
   Phe Ala Trp Asn Trp Ile Arg Gln Pro Pro Gly Lys Gly Leu Glu Trp
   35 40 45
   Met Gly Tyr Ile Ser Tyr Asn Gly Asn Thr Arg Tyr Gln Pro Ser Leu
   50 55 60
   Lys Ser Arg Ile Thr Ile Ser Arg Asp Thr Ser Lys Asn Gln Phe Phe
   65 70 75 80
   Leu Lys Leu Asn Ser Val Thr Ala Ala Asp Thr Ala Thr Tyr Tyr Cys
   85 90 95
   Val Thr Ala Ser Arg Gly Phe Pro Tyr Trp Gly Gln Gly Thr Leu Val
   100 105 110
   Thr Val Ser Ser
   115
<210> 10
   <211> 11
   <212> PRT
   <213> Artificial Sequence
<220>
   <221> source
   <223> /note="Description of Artificial Sequence: Synthetic peptide"
<400> 10
   Gly Tyr Ser Ile Ser Arg Asp Phe Ala Trp Asn
   1 5 10
<210> 11
   <211> 16
   <212> **PRT**
   <213> Artificial Sequence
<220>
   <221> source
   <223> /note="Description of Artificial Sequence: Synthetic peptide"
<400> 11
   Tyr Ile Ser Tyr Asn Gly Asn Thr Arg Tyr Gln Pro Ser Leu Lys Ser
   1               5                   10                  15
<210> 12
   <211> 7
   <212> PRT
   <213> Artificial Sequence
<220>
   <221> source
   <223> /note="Description of Artificial Sequence: Synthetic peptide"
<400> 12
   Ala Ser Arg Gly Phe Pro Tyr
   1               5
<210> 13
   <211> 214
   <212> PRT
   <213> Artificial Sequence
<220>
   <221> source
   <223> /note="Description of Artificial Sequence: Synthetic polypeptide"
<400> 13
   Asp Ile Gln Met Thr Gln Ser Pro Ser Ser Met Ser Val Ser Val Gly
   1               5                   10                  15
   Asp Arg Val Thr Ile Thr Cys His Ser Ser Gln Asp Ile Asn Ser Asn
               20                  25                  30
   Ile Gly Trp Leu Gln Gln Lys Pro Gly Lys Ser Phe Lys Gly Leu Ile
           35                  40                  45
   Tyr His Gly Thr Asn Leu Asp Asp Gly Val Pro Ser Arg Phe Ser Gly
       50                  55                  60
   Ser Gly Ser Gly Thr Asp Tyr Thr Leu Thr Ile Ser Ser Leu Gln Pro
   65                  70                  75                  80
   Glu Asp Phe Ala Thr Tyr Tyr Cys Val Gln Tyr Ala Gln Phe Pro Trp
                   85                  90                  95
   Thr Phe Gly Gly Gly Thr Lys Leu Glu Ile Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys
       210
<210> 14
   <211> 445
   <212> PRT
   <213> Artificial Sequence
<220>
   <221> source
   <223> /note="Description of Artificial Sequence: Synthetic polypeptide"
<400> 14
   Gln Val Gln Leu Gln Glu Ser Gly Pro Gly Leu Val Lys Pro Ser Gln
   1               5                   10                  15
   Thr Leu Ser Leu Thr Cys Thr Val Ser Gly Tyr Ser Ile Ser Ser Asp
               20                  25                  30
   Phe Ala Trp Asn Trp Ile Arg Gln Pro Pro Gly Lys Gly Leu Glu Trp
           35                  40                  45
   Met Gly Tyr Ile Ser Tyr Ser Gly Asn Thr Arg Tyr Gln Pro Ser Leu
       50                  55                  60
   Lys Ser Arg Ile Thr Ile Ser Arg Asp Thr Ser Lys Asn Gln Phe Phe
   65                  70                  75                  80
   Leu Lys Leu Asn Ser Val Thr Ala Ala Asp Thr Ala Thr Tyr Tyr Cys
                   85                  90                  95
   Val Thr Ala Gly Arg Gly Phe Pro Tyr Trp Gly Gln Gly Thr Leu Val
               100                 105                 110
   Thr Val Ser Ser Ala Ser Thr Lys Gly Pro Ser Val Phe Pro Leu Ala
           115                 120                 125
   Pro Ser Ser Lys Ser Thr Ser Gly Gly Thr Ala Ala Leu Gly Cys Leu
       130                 135                 140
   Val Lys Asp Tyr Phe Pro Glu Pro Val Thr Val Ser Trp Asn Ser Gly
   145                 150                 155                 160
   Ala Leu Thr Ser Gly Val His Thr Phe Pro Ala Val Leu Gln Ser Ser
                   165                 170                 175
   Gly Leu Tyr Ser Leu Ser Ser Val Val Thr Val Pro Ser Ser Ser Leu
               180                 185                 190
   Gly Thr Gln Thr Tyr Ile Cys Asn Val Asn His Lys Pro Ser Asn Thr
           195                 200                 205
   Lys Val Asp Lys Lys Val Glu Pro Lys Ser Cys Asp Lys Thr His Thr
       210                 215                 220
   Cys Pro Pro Cys Pro Ala Pro Glu Leu Leu Gly Gly Pro Ser Val Phe
   225                 230                 235                 240
   Leu Phe Pro Pro Lys Pro Lys Asp Thr Leu Met Ile Ser Arg Thr Pro
                   245                 250                 255
   Glu Val Thr Cys Val Val Val Asp Val Ser His Glu Asp Pro Glu Val
               260                 265                 270
   Lys Phe Asn Trp Tyr Val Asp Gly Val Glu Val His Asn Ala Lys Thr
           275                 280                 285
   Lys Pro Arg Glu Glu Gln Tyr Asn Ser Thr Tyr Arg Val Val Ser Val
       290                 295                 300
   Leu Thr Val Leu His Gln Asp Trp Leu Asn Gly Lys Glu Tyr Lys Cys
   305                 310                 315                 320
   Lys Val Ser Asn Lys Ala Leu Pro Ala Pro Ile Glu Lys Thr Ile Ser
                   325                 330                 335
   Lys Ala Lys Gly Gln Pro Arg Glu Pro Gln Val Tyr Thr Leu Pro Pro
   340 345 350
   Ser Arg Asp Glu Leu Thr Lys Asn Gln Val Ser Leu Thr Cys Leu Val
   355 360 365
   Lys Gly Phe Tyr Pro Ser Asp Ile Ala Val Glu Trp Glu Ser Asn Gly
   370 375 380
   Gln Pro Glu Asn Asn Tyr Lys Thr Thr Pro Pro Val Leu Asp Ser Asp
   385 390 395 400
   Gly Ser Phe Phe Leu Tyr Ser Lys Leu Thr Val Asp Lys Ser Arg Trp
   405 410 415
   Gln Gln Gly Asn Val Phe Ser Cys Ser Val Met His Glu Ala Leu His
   420 425 430
   Asn His Tyr Thr Gln Lys Ser Leu Ser Leu Ser Pro Gly
   435 440 445
<210> 15
   <211> 445
   <212> PRT
   <213> Artificial Sequence
<220>
   <221> source
   <223> /note="Description of Artificial Sequence: Synthetic polypeptide"
<400> 15
   Glu Val Gln Leu Gln Glu Ser Gly Pro Gly Leu Val Lys Pro Ser Gln
   1 5 10 15
   Thr Leu Ser Leu Thr Cys Thr Val Ser Gly Tyr Ser Ile Ser Arg Asp
   20 25 30
   Phe Ala Trp Asn Trp Ile Arg Gln Pro Pro Gly Lys Gly Leu Glu Trp
   35 40 45
   Met Gly Tyr Ile Ser Tyr Asn Gly Asn Thr Arg Tyr Gln Pro Ser Leu
   50 55 60
   Lys Ser Arg Ile Thr Ile Ser Arg Asp Thr Ser Lys Asn Gln Phe Phe
   65 70 75 80
   Leu Lys Leu Asn Ser Val Thr Ala Ala Asp Thr Ala Thr Tyr Tyr Cys
   85 90 95
   Val Thr Ala Ser Arg Gly Phe Pro Tyr Trp Gly Gln Gly Thr Leu Val
               100                 105                 110
   Thr Val Ser Ser Ala Ser Thr Lys Gly Pro Ser Val Phe Pro Leu Ala
           115                 120                 125
   Pro Ser Ser Lys Ser Thr Ser Gly Gly Thr Ala Ala Leu Gly Cys Leu
       130                 135                 140
   Val Lys Asp Tyr Phe Pro Glu Pro Val Thr Val Ser Trp Asn Ser Gly
   145                 150                 155                 160
   Ala Leu Thr Ser Gly Val His Thr Phe Pro Ala Val Leu Gln Ser Ser
                   165                 170                 175
   Gly Leu Tyr Ser Leu Ser Ser Val Val Thr Val Pro Ser Ser Ser Leu
               180                 185                 190
   Gly Thr Gln Thr Tyr Ile Cys Asn Val Asn His Lys Pro Ser Asn Thr
           195                 200                 205
   Lys Val Asp Lys Lys Val Glu Pro Lys Ser Cys Asp Lys Thr His Thr
       210                 215                 220
   Cys Pro Pro Cys Pro Ala Pro Glu Leu Leu Gly Gly Pro Ser Val Phe
   225                 230                 235                 240
   Leu Phe Pro Pro Lys Pro Lys Asp Thr Leu Met Ile Ser Arg Thr Pro
                   245                 250                 255
   Glu Val Thr Cys Val Val Val Asp Val Ser His Glu Asp Pro Glu Val
               260                 265                 270
   Lys Phe Asn Trp Tyr Val Asp Gly Val Glu Val His Asn Ala Lys Thr
           275                 280                 285
   Lys Pro Arg Glu Glu Gln Tyr Asn Ser Thr Tyr Arg Val Val Ser Val
       290                 295                 300
   Leu Thr Val Leu His Gln Asp Trp Leu Asn Gly Lys Glu Tyr Lys Cys
   305                 310                 315                 320
   Lys Val Ser Asn Lys Ala Leu Pro Ala Pro Ile Glu Lys Thr Ile Ser
                   325                 330                 335
   Lys Ala Lys Gly Gln Pro Arg Glu Pro Gln Val Tyr Thr Leu Pro Pro
               340                 345                 350
   Ser Arg Glu Glu Met Thr Lys Asn Gln Val Ser Leu Thr Cys Leu Val
           355                 360                 365
   Lys Gly Phe Tyr Pro Ser Asp Ile Ala Val Glu Trp Glu Ser Asn Gly
       370                 375                 380
   Gln Pro Glu Asn Asn Tyr Lys Thr Thr Pro Pro Val Leu Asp Ser Asp
   385                 390                 395                 400
   Gly Ser Phe Phe Leu Tyr Ser Lys Leu Thr Val Asp Lys Ser Arg Trp
                   405                 410                 415
   Gln Gln Gly Asn Val Phe Ser Cys Ser Val Met His Glu Ala Leu His
               420                 425                 430
   Asn His Tyr Thr Gln Lys Ser Leu Ser Leu Ser Pro Gly
           435                 440                 445
<210> 16
   <211> 11
   <212> PRT
   <213> Artificial Sequence
<220>
   <221> source
   <223> /note="Description of Artificial Sequence: Synthetic peptide"
<400> 16
   Gly Tyr Ser Ile Ser Asn Asp Phe Ala Trp Asn
   1               5                   10
<210> 17
   <211> 16
   <212> PRT
   <213> Artificial Sequence
<220>
   <221> source
   <223> /note="Description of Artificial Sequence: Synthetic peptide"
<400> 17
   Tyr Ile Ser Tyr Lys Gly Asn Thr Arg Tyr Gln Pro Ser Leu Lys Ser
   1               5                   10                  15
<210> 18
   <211> 7
   <212> PRT
   <213> Artificial Sequence
<220>
   <221> source
   <223> /note="Description of Artificial Sequence: Synthetic peptide"
<400> 18
   Ala Ser Arg Gly Leu Pro Tyr
   1 5
<210> 19
   <211> 7
   <212> PRT
   <213> Artificial Sequence
<220>
   <221> source
   <223> /note="Description of Artificial Sequence: Synthetic peptide"
<400> 19
   Ala Ser Arg Gly Phe Pro Trp
   1 5
<210> 20
   <211> 11
   <212> PRT
   <213> Artificial Sequence
<220>
   <221> source
   <223> /note="Description of Artificial Sequence: Synthetic peptide"
<400> 20
   Gly Tyr Ser Ile Gly Arg Asp Phe Ala Trp Asn
   1 5 10
<210> 21
   <211> 11
   <212> PRT
   <213> Artificial Sequence
<220>
   <221> source
   <223> /note="Description of Artificial Sequence: Synthetic peptide"
<400> 21
   Gly Tyr Ser Ile His Ser Asp Phe Ala Trp Asn
   1 5 10
<210> 22
   <211> 7
   <212> PRT
   <213> Artificial Sequence
<220>
   <221> source
   <223> /note="Description of Artificial Sequence: Synthetic peptide"
<400> 22
   Ala Ser Trp Gly Leu Pro Trp
   1 5
<210> 23
   <211> 11
   <212> PRT
   <213> Artificial Sequence
<220>
   <221> source
   <223> /note="Description of Artificial Sequence: Synthetic peptide"
<400> 23
   His Ser Ser Gln Asp Ile Thr Tyr Asn Ile Gly
   1 5 10
<210> 24
   <211> 7
   <212> PRT
   <213> Artificial Sequence
<220>
   <221> source
   <223> /note="Description of Artificial Sequence: Synthetic peptide"
<400> 24
   His Gly Ala Asn Leu Asp Asp
   1 5
<210> 25
   <211> 9
   <212> PRT
   <213> Artificial Sequence
<220>
   <221> source
   <223> /note="Description of Artificial Sequence: Synthetic peptide"
<400> 25
   Val Gln Tyr Asp Glu Phe Pro Trp Thr
   1 5
<210> 26
   <211> 11
   <212> PRT
   <213> Artificial Sequence
<220>
   <221> source
   <223> /note="Description of Artificial Sequence: Synthetic peptide"
<400> 26
   His Ser Ser Gln Asp Ile Thr Tyr Asn Val Gly
   1 5 10
<210> 27
   <211> 7
   <212> PRT
   <213> Artificial Sequence
<220>
   <221> source
   <223> /note="Description of Artificial Sequence: Synthetic peptide"
<400> 27
   His Gly Ser Asn Leu Asp His
   1               5
<210> 28
   <211> 9
   <212> PRT
   <213> Artificial Sequence
<220>
   <221> source
   <223> /note="Description of Artificial Sequence: Synthetic peptide"
<400> 28
   Val Gln Tyr Asp Asp Phe Pro Trp Thr
   1               5
<210> 29
   <211> 11
   <212> PRT
   <213> Artificial Sequence
<220>
   <221> source
   <223> /note="Description of Artificial Sequence: Synthetic peptide"
<400> 29
   His Ser Ser Gln Asp Ile Asn Met Asn Val Gly
   1               5                   10
<210> 30
   <211> 7
   <212> PRT
   <213> Artificial Sequence
<220>
   <221> source
   <223> /note="Description of Artificial Sequence: Synthetic peptide"
<400> 30
   His Gly Ala Ile Leu Asp Asp
   1               5
<210> 31
   <211> 9
   <212> PRT
   <213> Artificial Sequence
<220>
   <221> source
   <223> /note="Description of Artificial Sequence: Synthetic peptide"
<400> 31
   Val Gln Tyr Ala Glu Phe Pro Trp Thr
   1               5
<210> 32
   <211> 1210
   <212> PRT
   <213> Homo sapiens
<400> 32
<210> 33
   <211> 943
   <212> **PRT**
   <213> Homo sapiens
<400> 33
   Met Arg Pro Ser Gly Thr Ala Gly Ala Ala Leu Leu Ala Leu Leu Ala
   1               5                   10                  15
   Ala Leu Cys Pro Ala Ser Arg Ala Leu Glu Glu Lys Lys Gly Asn Tyr
               20                  25                  30
   Val Val Thr Asp His Gly Ser Cys Val Arg Ala Cys Gly Ala Asp Ser
           35                  40                  45
   Tyr Glu Met Glu Glu Asp Gly Val Arg Lys Cys Lys Lys Cys Glu Gly
       50                  55                  60
   Pro Cys Arg Lys Val Cys Asn Gly Ile Gly Ile Gly Glu Phe Lys Asp
   65                  70                  75                  80
   Ser Leu Ser Ile Asn Ala Thr Asn Ile Lys His Phe Lys Asn Cys Thr
                   85                  90                  95
   Ser Ile Ser Gly Asp Leu His Ile Leu Pro Val Ala Phe Arg Gly Asp
               100                 105                 110
   Ser Phe Thr His Thr Pro Pro Leu Asp Pro Gln Glu Leu Asp Ile Leu
           115                 120                 125
   Lys Thr Val Lys Glu Ile Thr Gly Phe Leu Leu Ile Gln Ala Trp Pro
       130                 135                 140
   Glu Asn Arg Thr Asp Leu His Ala Phe Glu Asn Leu Glu Ile Ile Arg
   145                 150                 155                 160
   Gly Arg Thr Lys Gln His Gly Gln Phe Ser Leu Ala Val Val Ser Leu
                   165                 170                 175
   Asn Ile Thr Ser Leu Gly Leu Arg Ser Leu Lys Glu Ile Ser Asp Gly
               180                 185                 190
   Asp Val Ile Ile Ser Gly Asn Lys Asn Leu Cys Tyr Ala Asn Thr Ile
           195                 200                 205
   Asn Trp Lys Lys Leu Phe Gly Thr Ser Gly Gln Lys Thr Lys Ile Ile
       210                 215                 220
   Ser Asn Arg Gly Glu Asn Ser Cys Lys Ala Thr Gly Gln Val Cys His
   225                 230                 235                 240
   Ala Leu Cys Ser Pro Glu Gly Cys Trp Gly Pro Glu Pro Arg Asp Cys
                   245                 250                 255
   Val Ser Cys Arg Asn Val Ser Arg Gly Arg Glu Cys Val Asp Lys Cys
               260                 265                 270
   Asn Leu Leu Glu Gly Glu Pro Arg Glu Phe Val Glu Asn Ser Glu Cys
           275                 280                 285
   Ile Gln Cys His Pro Glu Cys Leu Pro Gln Ala Met Asn Ile Thr Cys
       290                 295                 300
   Thr Gly Arg Gly Pro Asp Asn Cys Ile Gln Cys Ala His Tyr Ile Asp
   305                 310                 315                 320
   Gly Pro His Cys Val Lys Thr Cys Pro Ala Gly Val Met Gly Glu Asn
                   325                 330                 335
   Asn Thr Leu Val Trp Lys Tyr Ala Asp Ala Gly His Val Cys His Leu
               340                 345                 350
   Cys His Pro Asn Cys Thr Tyr Gly Cys Thr Gly Pro Gly Leu Glu Gly
           355                 360                 365
   Cys Pro Thr Asn Gly Pro Lys Ile Pro Ser Ile Ala Thr Gly Met Val
       370                 375                 380
   Gly Ala Leu Leu Leu Leu Leu Val Val Ala Leu Gly Ile Gly Leu Phe
   385                 390                 395                 400
   Met Arg Arg Arg His Ile Val Arg Lys Arg Thr Leu Arg Arg Leu Leu
                   405                 410                 415
   Gln Glu Arg Glu Leu Val Glu Pro Leu Thr Pro Ser Gly Glu Ala Pro
               420                 425                 430
   Asn Gln Ala Leu Leu Arg Ile Leu Lys Glu Thr Glu Phe Lys Lys Ile
   435 440 445
   Lys Val Leu Gly Ser Gly Ala Phe Gly Thr Val Tyr Lys Gly Leu Trp
   450 455 460
   Ile Pro Glu Gly Glu Lys Val Lys Ile Pro Val Ala Ile Lys Glu Leu
   465 470 475 480
   Arg Glu Ala Thr Ser Pro Lys Ala Asn Lys Glu Ile Leu Asp Glu Ala
   485 490 495
   Tyr Val Met Ala Ser Val Asp Asn Pro His Val Cys Arg Leu Leu Gly
   500 505 510
   Ile Cys Leu Thr Ser Thr Val Gln Leu Ile Thr Gln Leu Met Pro Phe
   515 520 525
   Gly Cys Leu Leu Asp Tyr Val Arg Glu His Lys Asp Asn Ile Gly Ser
   530 535 540
   Gln Tyr Leu Leu Asn Trp Cys Val Gln Ile Ala Lys Gly Met Asn Tyr
   545 550 555 560
   Leu Glu Asp Arg Arg Leu Val His Arg Asp Leu Ala Ala Arg Asn Val
   565 570 575
   Leu Val Lys Thr Pro Gln His Val Lys Ile Thr Asp Phe Gly Leu Ala
   580 585 590
   Lys Leu Leu Gly Ala Glu Glu Lys Glu Tyr His Ala Glu Gly Gly Lys
   595 600 605
   Val Pro Ile Lys Trp Met Ala Leu Glu Ser Ile Leu His Arg Ile Tyr
   610 615 620
   Thr His Gln Ser Asp Val Trp Ser Tyr Gly Val Thr Val Trp Glu Leu
   625 630 635 640
   Met Thr Phe Gly Ser Lys Pro Tyr Asp Gly Ile Pro Ala Ser Glu Ile
   645 650 655
   Ser Ser Ile Leu Glu Lys Gly Glu Arg Leu Pro Gln Pro Pro Ile Cys
   660 665 670
   Thr Ile Asp Val Tyr Met Ile Met Val Lys Cys Trp Met Ile Asp Ala
   675 680 685
   Asp Ser Arg Pro Lys Phe Arg Glu Leu Ile Ile Glu Phe Ser Lys Met
   690 695 700
   Ala Arg Asp Pro Gln Arg Tyr Leu Val Ile Gln Gly Asp Glu Arg Met
   705 710 715 720
   His Leu Pro Ser Pro Thr Asp Ser Asn Phe Tyr Arg Ala Leu Met Asp
   725 730 735
   Glu Glu Asp Met Asp Asp Val Val Asp Ala Asp Glu Tyr Leu Ile Pro
   740 745 750
   Gln Gln Gly Phe Phe Ser Ser Pro Ser Thr Ser Arg Thr Pro Leu Leu
   755 760 765
   Ser Ser Leu Ser Ala Thr Ser Asn Asn Ser Thr Val Ala Cys Ile Asp
   770 775 780
   Arg Asn Gly Leu Gln Ser Cys Pro Ile Lys Glu Asp Ser Phe Leu Gln
   785 790 795 800
   Arg Tyr Ser Ser Asp Pro Thr Gly Ala Leu Thr Glu Asp Ser Ile Asp
   805 810 815
   Asp Thr Phe Leu Pro Val Pro Glu Tyr Ile Asn Gln Ser Val Pro Lys
   820 825 830
   Arg Pro Ala Gly Ser Val Gln Asn Pro Val Tyr His Asn Gln Pro Leu
   835 840 845
   Asn Pro Ala Pro Ser Arg Asp Pro His Tyr Gln Asp Pro His Ser Thr
   850 855 860
   Ala Val Gly Asn Pro Glu Tyr Leu Asn Thr Val Gln Pro Thr Cys Val
   865 870 875 880
   Asn Ser Thr Phe Asp Ser Pro Ala His Trp Ala Gln Lys Gly Ser His
   885 890 895
   Gln Ile Ser Leu Asp Asn Pro Asp Tyr Gln Gln Asp Phe Phe Pro Lys
   900 905 910
   Glu Ala Lys Pro Asn Gly Ile Phe Lys Gly Ser Thr Ala Glu Asn Ala
   915 920 925
   Glu Tyr Leu Arg Val Ala Pro Gln Ser Ser Glu Phe Ile Gly Ala
   930 935 940
<210> 34
   <211> 645
   <212> **PRT**
   <213> Homo sapiens
<400> 34
   Met Arg Pro Ser Gly Thr Ala Gly Ala Ala Leu Leu Ala Leu Leu Ala
   1               5                   10                  15
   Ala Leu Cys Pro Ala Ser Arg Ala Leu Glu Glu Lys Lys Val Cys Gln
               20                  25                  30
   Gly Thr Ser Asn Lys Leu Thr Gln Leu Gly Thr Phe Glu Asp His Phe
           35                  40                  45
   Leu Ser Leu Gln Arg Met Phe Asn Asn Cys Glu Val Val Leu Gly Asn
       50                  55                  60
   Leu Glu Ile Thr Tyr Val Gln Arg Asn Tyr Asp Leu Ser Phe Leu Lys
   65                  70                  75                  80
   Thr Ile Gln Glu Val Ala Gly Tyr Val Leu Ile Ala Leu Asn Thr Val
                   85                  90                  95
   Glu Arg Ile Pro Leu Glu Asn Leu Gln Ile Ile Arg Gly Asn Met Tyr
               100                 105                 110
   Tyr Glu Asn Ser Tyr Ala Leu Ala Val Leu Ser Asn Tyr Asp Ala Asn
           115                 120                 125
   Lys Thr Gly Leu Lys Glu Leu Pro Met Arg Asn Leu Gln Glu Ile Leu
       130                 135                 140
   His Gly Ala Val Arg Phe Ser Asn Asn Pro Ala Leu Cys Asn Val Glu
   145                 150                 155                 160
   Ser Ile Gln Trp Arg Asp Ile Val Ser Ser Asp Phe Leu Ser Asn Met
                   165                 170                 175
   Ser Met Asp Phe Gln Asn His Leu Gly Ser Cys Gln Lys Cys Asp Pro
               180                 185                 190
   Ser Cys Pro Asn Gly Ser Cys Trp Gly Ala Gly Glu Glu Asn Cys Gln
           195                 200                 205
   Lys Leu Thr Lys Ile Ile Cys Ala Gln Gln Cys Ser Gly Arg Cys Arg
       210                 215                 220
   Gly Lys Ser Pro Ser Asp Cys Cys His Asn Gln Cys Ala Ala Gly Cys
   225                 230                 235                 240
   Thr Gly Pro Arg Glu Ser Asp Cys Leu Val Cys Arg Lys Phe Arg Asp
                   245                 250                 255
   Glu Ala Thr Cys Lys Asp Thr Cys Pro Pro Leu Met Leu Tyr Asn Pro
               260                 265                 270
   Thr Thr Tyr Gln Met Asp Val Asn Pro Glu Gly Lys Tyr Ser Phe Gly
           275                 280                 285
   Ala Thr Cys Val Lys Lys Cys Pro Arg Asn Tyr Val Val Thr Asp His
       290                 295                 300
   Gly Ser Cys Val Arg Ala Cys Gly Ala Asp Ser Tyr Glu Met Glu Glu
   305                 310                 315                 320
   Asp Gly Val Arg Lys Cys Lys Lys Cys Glu Gly Pro Cys Arg Lys Val
                   325                 330                 335
   Cys Asn Gly Ile Gly Ile Gly Glu Phe Lys Asp Ser Leu Ser Ile Asn
               340                 345                 350
   Ala Thr Asn Ile Lys His Phe Lys Asn Cys Thr Ser Ile Ser Gly Asp
           355                 360                 365
   Leu His Ile Leu Pro Val Ala Phe Arg Gly Asp Ser Phe Thr His Thr
       370                 375                 380
   Pro Pro Leu Asp Pro Gln Glu Leu Asp Ile Leu Lys Thr Val Lys Glu
   385                 390                 395                 400
   Ile Thr Gly Phe Leu Leu Ile Gln Ala Trp Pro Glu Asn Arg Thr Asp
                   405                 410                 415
   Leu His Ala Phe Glu Asn Leu Glu Ile Ile Arg Gly Arg Thr Lys Gln
               420                 425                 430
   His Gly Gln Phe Ser Leu Ala Val Val Ser Leu Asn Ile Thr Ser Leu
           435                 440                 445
   Gly Leu Arg Ser Leu Lys Glu Ile Ser Asp Gly Asp Val Ile Ile Ser
       450                 455                 460
   Gly Asn Lys Asn Leu Cys Tyr Ala Asn Thr Ile Asn Trp Lys Lys Leu
   465                 470                 475                 480
   Phe Gly Thr Ser Gly Gln Lys Thr Lys Ile Ile Ser Asn Arg Gly Glu
   485 490 495
   Asn Ser Cys Lys Ala Thr Gly Gln Val Cys His Ala Leu Cys Ser Pro
   500 505 510
   Glu Gly Cys Trp Gly Pro Glu Pro Arg Asp Cys Val Ser Cys Arg Asn
   515 520 525
   Val Ser Arg Gly Arg Glu Cys Val Asp Lys Cys Asn Leu Leu Glu Gly
   530 535 540
   Glu Pro Arg Glu Phe Val Glu Asn Ser Glu Cys Ile Gln Cys His Pro
   545 550 555 560
   Glu Cys Leu Pro Gln Ala Met Asn Ile Thr Cys Thr Gly Arg Gly Pro
   565 570 575
   Asp Asn Cys Ile Gln Cys Ala His Tyr Ile Asp Gly Pro His Cys Val
   580 585 590
   Lys Thr Cys Pro Ala Gly Val Met Gly Glu Asn Asn Thr Leu Val Trp
   595 600 605
   Lys Tyr Ala Asp Ala Gly His Val Cys His Leu Cys His Pro Asn Cys
   610 615 620
   Thr Tyr Gly Cys Thr Gly Pro Gly Leu Glu Gly Cys Pro Thr Asn Gly
   625 630 635 640
   Pro Lys Ile Pro Ser
   645
<210> 35
   <211> 11
   <212> PRT
   <213> Artificial Sequence
<220>
   <221> source
   <223> /note="Description of Artificial Sequence: Synthetic peptide"
<220>
   <221> VARIANT
   <222> (5) .. (5)
   <223> /replace="Gly" or "His"
<220>
   <221> VARIANT
   <222> (6) .. (6)
   <223> /replace="Arg" or "Asn"
<220>
   <221> MISC_FEATURE
   <222> (1)..(11)
   <223> /note="Variant residues given in the sequence have no preference with respect to those in the annotations for variant positions"
<400> 35
   Gly Tyr Ser Ile Ser Ser Asp Phe Ala Trp Asn
   1               5                   10
<210> 36
   <211> 16
   <212> PRT
   <213> Artificial Sequence
<220>
   <221> source
   <223> /note="Description of Artificial Sequence: Synthetic peptide"
<220>
   <221> VARIANT
   <222> (5)..(5)
   <223> /replace="Asn" or "Lys"
<220>
   <221> MISC_FEATURE
   <222> (1) .. (16)
   <223> /note="Variant residues given in the sequence have no preference with respect to those in the annotations for variant positions"
<400> 36
   Tyr Ile Ser Tyr Ser Gly Asn Thr Arg Tyr Gln Pro Ser Leu Lys Ser
   1               5                   10                  15
<210> 37
   <211> 7
   <212> PRT
   <213> Artificial Sequence
<220>
   <221> source
   <223> /note="Description of Artificial Sequence: Synthetic peptide"
<220>
   <221> VARIANT
   <222> (3)..(3)
   <223> /replace="Trp"
<220>
   <221> VARIANT
   <222> (5)..(5)
   <223> /replace="Leu"
<220>
   <221> VARIANT
   <222> (7)..(7)
   <223> /replace="Trp"
<220>
   <221> MISC_FEATURE
   <222> (1)..(7)
   <223> /note="Variant residues given in the sequence have no preference with respect to those in the annotations for variant positions"
<400> 37
   Ala Ser Arg Gly Phe Pro Tyr
   1               5
<210> 38
   <211> 11
   <212> PRT
   <213> Artificial Sequence
<220>
   <221> source
   <223> /note="Description of Artificial Sequence: Synthetic peptide"
<220>
   <221> VARIANT
   <222> (7)..(7)
   <223> /replace="Thr"
<220>
   <221> VARIANT
   <222> (8)..(8)
   <223> /replace="Met" or "Ser"
<220>
   <221> VARIANT
   <222> (10)..(10)
   <223> /replace="Val"
<220>
   <221> MISC_FEATURE
   <222> (1)..(11)
   <223> /note="Variant residues given in the sequence have no preference with respect to those in the annotations for variant positions"
<400> 38
   His Ser Ser Gln Asp Ile Asn Tyr Asn Ile Gly
   1               5                   10
<210> 39
   <211> 7
   <212> PRT
   <213> Artificial Sequence
<220>
   <221> source
   <223> /note="Description of Artificial Sequence: Synthetic peptide"
<220>
   <221> VARIANT
   <222> (3)..(3)
   <223> /replace="Ala" or "Ser"
<220>
   <221> VARIANT
   <222> (4) .. (4)
   <223> /replace="Ile"
<220>
   <221> VARIANT
   <222> (7)..(7)
   <223> /replace="His"
<220>
   <221> MISC_FEATURE
   <222> (1)..(7)
   <223> /note="Variant residues given in the sequence have no preference with respect to those in the annotations for variant positions"
<400> 39
   His Gly Thr Asn Leu Asp Asp
   1               5
<210> 40
   <211> 9
   <212> PRT
   <213> Artificial Sequence
<220>
   <221> source
   <223> /note="Description of Artificial Sequence: Synthetic peptide"
<220>
   <221> VARIANT
   <222> (4)..(4)
   <223> /replace="Asp"
<220>
   <221> VARIANT
   <222> (5) .. (5)
   <223> /replace="Glu" or "Asp"
<220>
   <221> MISC_FEATURE
   <222> (1) .. (9)
   <223> /note="Variant residues given in the sequence have no preference with respect to those in the annotations for variant positions"
<400> 40
   Val Gln Tyr Ala Gln Phe Pro Trp Thr
   1               5
<210> 41
   <211> 330
   <212> **PRT**
   <213> Homo sapiens
<400> **41**
   Ala Ser Thr Lys Gly Pro Ser Val Phe Pro Leu Ala Pro Ser Ser Lys
   1               5                   10                  15
   Ser Thr Ser Gly Gly Thr Ala Ala Leu Gly Cys Leu Val Lys Asp Tyr
               20                  25                  30
   Phe Pro Glu Pro Val Thr Val Ser Trp Asn Ser Gly Ala Leu Thr Ser
           35                  40                  45
   Gly Val His Thr Phe Pro Ala Val Leu Gln Ser Ser Gly Leu Tyr Ser
       50                  55                  60
   Leu Ser Ser Val Val Thr Val Pro Ser Ser Ser Leu Gly Thr Gln Thr
   65                  70                  75                  80
   Tyr Ile Cys Asn Val Asn His Lys Pro Ser Asn Thr Lys Val Asp Lys
                   85                  90                  95
   Lys Val Glu Pro Lys Ser Cys Asp Lys Thr His Thr Cys Pro Pro Cys
               100                 105                 110
   Pro Ala Pro Glu Leu Leu Gly Gly Pro Ser Val Phe Leu Phe Pro Pro
           115                 120                 125
   Lys Pro Lys Asp Thr Leu Met Ile Ser Arg Thr Pro Glu Val Thr Cys
       130                 135                 140
   Val Val Val Asp Val Ser His Glu Asp Pro Glu Val Lys Phe Asn Trp
   145                 150                 155                 160
   Tyr Val Asp Gly Val Glu Val His Asn Ala Lys Thr Lys Pro Arg Glu
                   165                 170                 175
   Glu Gln Tyr Asn Ser Thr Tyr Arg Val Val Ser Val Leu Thr Val Leu
               180                 185                 190
   His Gln Asp Trp Leu Asn Gly Lys Glu Tyr Lys Cys Lys Val Ser Asn
           195                 200                 205
   Lys Ala Leu Pro Ala Pro Ile Glu Lys Thr Ile Ser Lys Ala Lys Gly
       210                 215                 220
   Gln Pro Arg Glu Pro Gln Val Tyr Thr Leu Pro Pro Ser Arg Glu Glu
   225                 230                 235                 240
   Met Thr Lys Asn Gln Val Ser Leu Thr Cys Leu Val Lys Gly Phe Tyr
                   245                 250                 255
   Pro Ser Asp Ile Ala Val Glu Trp Glu Ser Asn Gly Gln Pro Glu Asn
               260                 265                 270
   Asn Tyr Lys Thr Thr Pro Pro Val Leu Asp Ser Asp Gly Ser Phe Phe
           275                 280                 285
   Leu Tyr Ser Lys Leu Thr Val Asp Lys Ser Arg Trp Gln Gln Gly Asn
       290                 295                 300
   Val Phe Ser Cys Ser Val Met His Glu Ala Leu His Asn His Tyr Thr
   305                 310                 315                 320
   Gln Lys Ser Leu Ser Leu Ser Pro Gly Lys
                   325                 330
<210> 42
   <211> 330
   <212> **PRT**
   <213> Homo sapiens
<400> **42**
   Ala Ser Thr Lys Gly Pro Ser Val Phe Pro Leu Ala Pro Ser Ser Lys
   1               5                   10                  15
   Ser Thr Ser Gly Gly Thr Ala Ala Leu Gly Cys Leu Val Lys Asp Tyr
               20                  25                  30
   Phe Pro Glu Pro Val Thr Val Ser Trp Asn Ser Gly Ala Leu Thr Ser
           35                  40                  45
   Gly Val His Thr Phe Pro Ala Val Leu Gln Ser Ser Gly Leu Tyr Ser
       50                  55                  60
   Leu Ser Ser Val Val Thr Val Pro Ser Ser Ser Leu Gly Thr Gln Thr
   65                  70                  75                  80
   Tyr Ile Cys Asn Val Asn His Lys Pro Ser Asn Thr Lys Val Asp Lys
                   85                  90                  95
   Lys Val Glu Pro Lys Ser Cys Asp Lys Thr His Thr Cys Pro Pro Cys
               100                 105                 110
   Pro Ala Pro Glu Ala Ala Gly Gly Pro Ser Val Phe Leu Phe Pro Pro
   115 120 125
   Lys Pro Lys Asp Thr Leu Met Ile Ser Arg Thr Pro Glu Val Thr Cys
   130 135 140
   Val Val Val Asp Val Ser His Glu Asp Pro Glu Val Lys Phe Asn Trp
   145 150 155 160
   Tyr Val Asp Gly Val Glu Val His Asn Ala Lys Thr Lys Pro Arg Glu
   165 170 175
   Glu Gln Tyr Asn Ser Thr Tyr Arg Val Val Ser Val Leu Thr Val Leu
   180 185 190
   His Gln Asp Trp Leu Asn Gly Lys Glu Tyr Lys Cys Lys Val Ser Asn
   195 200 205
   Lys Ala Leu Pro Ala Pro Ile Glu Lys Thr Ile Ser Lys Ala Lys Gly
   210 215 220
   Gln Pro Arg Glu Pro Gln Val Tyr Thr Leu Pro Pro Ser Arg Glu Glu
   225 230 235 240
   Met Thr Lys Asn Gln Val Ser Leu Thr Cys Leu Val Lys Gly Phe Tyr
   245 250 255
   Pro Ser Asp Ile Ala Val Glu Trp Glu Ser Asn Gly Gln Pro Glu Asn
   260 265 270
   Asn Tyr Lys Thr Thr Pro Pro Val Leu Asp Ser Asp Gly Ser Phe Phe
   275 280 285
   Leu Tyr Ser Lys Leu Thr Val Asp Lys Ser Arg Trp Gln Gln Gly Asn
   290 295 300
   Val Phe Ser Cys Ser Val Met His Glu Ala Leu His Asn His Tyr Thr
   305 310 315 320
   Gln Lys Ser Leu Ser Leu Ser Pro Gly Lys
   325 330
<210> 43
   <211> 107
   <212> **PRT**
   <213> Homo sapiens
<400> 43
<210> **44**
   <211> 105
   <212> **PRT**
   <213> Homo sapiens
<400> **44**
   Gln Pro Lys Ala Ala Pro Ser Val Thr Leu Phe Pro Pro Ser Ser Glu
   1               5                   10                  15
   Glu Leu Gln Ala Asn Lys Ala Thr Leu Val Cys Leu Ile Ser Asp Phe
               20                  25                  30
   Tyr Pro Gly Ala Val Thr Val Ala Trp Lys Ala Asp Ser Ser Pro Val
           35                  40                  45
   Lys Ala Gly Val Glu Thr Thr Thr Pro Ser Lys Gln Ser Asn Asn Lys
       50                  55                  60
   Tyr Ala Ala Ser Ser Tyr Leu Ser Leu Thr Pro Glu Gln Trp Lys Ser
   65                  70                  75                  80
   His Arg Ser Tyr Ser Cys Gln Val Thr His Glu Gly Ser Thr Val Glu
                   85                  90                  95
   Lys Thr Val Ala Pro Thr Glu Cys Ser
               100                 105
<210> 45
   <211> 16
   <212> **PRT**
   <213> Homo sapiens
<400> **45**
   Cys Gly Ala Asp Ser Tyr Glu Met Glu Glu Asp Gly Val Arg Lys Cys
   1               5                   10                  15
<210> 46
   <211> 350
   <212> PRT
   <213> Homo sapiens
<400> 46
   Leu Glu Glu Lys Lys Gly Asn Tyr Val Val Thr Asp His Gly Ser Cys
   1               5                   10                  15
   Val Arg Ala Cys Gly Ala Asp Ser Tyr Glu Met Glu Glu Asp Gly Val
               20                  25                  30
   Arg Lys Cys Lys Lys Cys Glu Gly Pro Cys Arg Lys Val Cys Asn Gly
           35                  40                  45
   Ile Gly Ile Gly Glu Phe Lys Asp Ser Leu Ser Ile Asn Ala Thr Asn
       50                  55                  60
   Ile Lys His Phe Lys Asn Cys Thr Ser Ile Ser Gly Asp Leu His Ile
   65                  70                  75                  80
   Leu Pro Val Ala Phe Arg Gly Asp Ser Phe Thr His Thr Pro Pro Leu
                   85                  90                  95
   Asp Pro Gln Glu Leu Asp Ile Leu Lys Thr Val Lys Glu Ile Thr Gly
               100                 105                 110
   Phe Leu Leu Ile Gln Ala Trp Pro Glu Asn Arg Thr Asp Leu His Ala
           115                 120                 125
   Phe Glu Asn Leu Glu Ile Ile Arg Gly Arg Thr Lys Gln His Gly Gln
       130                 135                 140
   Phe Ser Leu Ala Val Val Ser Leu Asn Ile Thr Ser Leu Gly Leu Arg
   145                 150                 155                 160
   Ser Leu Lys Glu Ile Ser Asp Gly Asp Val Ile Ile Ser Gly Asn Lys
                   165                 170                 175
   Asn Leu Cys Tyr Ala Asn Thr Ile Asn Trp Lys Lys Leu Phe Gly Thr
               180                 185                 190
   Ser Gly Gln Lys Thr Lys Ile Ile Ser Asn Arg Gly Glu Asn Ser Cys
           195                 200                 205
   Lys Ala Thr Gly Gln Val Cys His Ala Leu Cys Ser Pro Glu Gly Cys
       210                 215                 220
   Trp Gly Pro Glu Pro Arg Asp Cys Val Ser Cys Arg Asn Val Ser Arg
   225                 230                 235                 240
   Gly Arg Glu Cys Val Asp Lys Cys Asn Leu Leu Glu Gly Glu Pro Arg
                   245                 250                 255
   Glu Phe Val Glu Asn Ser Glu Cys Ile Gln Cys His Pro Glu Cys Leu
               260                 265                 270
   Pro Gln Ala Met Asn Ile Thr Cys Thr Gly Arg Gly Pro Asp Asn Cys
           275                 280                 285
   Ile Gln Cys Ala His Tyr Ile Asp Gly Pro His Cys Val Lys Thr Cys
       290                 295                 300
   Pro Ala Gly Val Met Gly Glu Asn Asn Thr Leu Val Trp Lys Tyr Ala
   305                 310                 315                 320
   Asp Ala Gly His Val Cys His Leu Cys His Pro Asn Cys Thr Tyr Gly
                   325                 330                 335
   Cys Thr Gly Pro Gly Leu Glu Gly Cys Pro Thr Asn Gly Pro
               340                 345                 350
<210> 47
   <211> 525
   <212> **PRT**
   <213> Homo sapiens
<400> **47**
   Met Arg Pro Ser Gly Thr Ala Gly Ala Ala Leu Leu Ala Leu Leu Ala
   1               5                   10                  15
   Ala Leu Cys Pro Ala Ser Arg Ala Leu Glu Glu Lys Lys Val Cys Gln
               20                  25                  30
   Gly Thr Ser Asn Lys Leu Thr Gln Leu Gly Thr Phe Glu Asp His Phe
           35                  40                  45
   Leu Ser Leu Gln Arg Met Phe Asn Asn Cys Glu Val Val Leu Gly Asn
       50                  55                  60
   Leu Glu Ile Thr Tyr Val Gln Arg Asn Tyr Asp Leu Ser Phe Leu Lys
   65                  70                  75                      80
   Thr Ile Gln Glu Val Ala Gly Tyr Val Leu Ile Ala Leu Asn Thr Val
                   85                  90                  95
   Glu Arg Ile Pro Leu Glu Asn Leu Gln Ile Ile Arg Gly Asn Met Tyr
               100                 105                 110
   Tyr Glu Asn Ser Tyr Ala Leu Ala Val Leu Ser Asn Tyr Asp Ala Asn
           115                 120                 125
   Lys Thr Gly Leu Lys Glu Leu Pro Met Arg Asn Leu Gln Glu Ile Leu
       130                 135                 140
   His Gly Ala Val Arg Phe Ser Asn Asn Pro Ala Leu Cys Asn Val Glu
   145                 150                 155                 160
   Ser Ile Gln Trp Arg Asp Ile Val Ser Ser Asp Phe Leu Ser Asn Met
                   165                 170                 175
   Ser Met Asp Phe Gln Asn His Leu Gly Ser Cys Gln Lys Cys Asp Pro
               180                 185                 190
   Ser Cys Pro Asn Gly Ser Cys Trp Gly Ala Gly Glu Glu Asn Cys Gln
           195                 200                 205
   Lys Leu Thr Lys Ile Ile Cys Ala Gln Gln Cys Ser Gly Arg Cys Arg
       210                 215                 220
   Gly Lys Ser Pro Ser Asp Cys Cys His Asn Gln Cys Ala Ala Gly Cys
   225                 230                 235                 240
   Thr Gly Pro Arg Glu Ser Asp Cys Leu Val Cys Arg Lys Phe Arg Asp
                   245                 250                 255
   Glu Ala Thr Cys Lys Asp Thr Cys Pro Pro Leu Met Leu Tyr Asn Pro
               260                 265                 270
   Thr Thr Tyr Gln Met Asp Val Asn Pro Glu Gly Lys Tyr Ser Phe Gly
           275                 280                 285
   Ala Thr Cys Val Lys Lys Cys Pro Arg Asn Tyr Val Val Thr Asp His
       290                 295                 300
   Gly Ser Cys Val Arg Ala Cys Gly Ala Asp Ser Tyr Glu Met Glu Glu
   305                 310                 315                 320
   Asp Gly Val Arg Lys Cys Lys Lys Cys Glu Gly Pro Cys Arg Lys Val
                   325                 330                 335
   Cys Asn Gly Ile Gly Ile Gly Glu Phe Lys Asp Ser Leu Ser Ile Asn
               340                 345                 350
   Ala Thr Asn Ile Lys His Phe Lys Asn Cys Thr Ser Ile Ser Gly Asp
           355                 360                 365
   Leu His Ile Leu Pro Val Ala Phe Arg Gly Asp Ser Phe Thr His Thr
       370                 375                 380
   Pro Pro Leu Asp Pro Gln Glu Leu Asp Ile Leu Lys Thr Val Lys Glu
   385                 390                 395                 400
   Ile Thr Gly Phe Leu Leu Ile Gln Ala Trp Pro Glu Asn Arg Thr Asp
                   405                 410                 415
   Leu His Ala Phe Glu Asn Leu Glu Ile Ile Arg Gly Arg Thr Lys Gln
               420                 425                 430
   His Gly Gln Phe Ser Leu Ala Val Val Ser Leu Asn Ile Thr Ser Leu
           435                 440                 445
   Gly Leu Arg Ser Leu Lys Glu Ile Ser Asp Gly Asp Val Ile Ile Ser
       450                 455                 460
   Gly Asn Lys Asn Leu Cys Tyr Ala Asn Thr Ile Asn Trp Lys Lys Leu
   465                 470                 475                 480
   Phe Gly Thr Ser Gly Gln Lys Thr Lys Ile Ile Ser Asn Arg Gly Glu
                   485                 490                 495
   Asn Ser Cys Lys Ala Thr Gly Gln Val Cys His Ala Leu Cys Ser Pro
               500                 505                 510
   Glu Gly Cys Trp Gly Pro Glu Pro Arg Asp Cys Val Ser
           515                 520                 525
<210> 48
   <211> 452
   <212> PRT
   <213> Artificial Sequence
<220>
   <221> source
   <223> /note="Description of Artificial Sequence: Synthetic polypeptide"
<400> 48
   Gln Val Gln Leu Lys Gln Ser Gly Pro Gly Leu Val Gln Pro Ser Gln
   1 5 10 15
   Ser Leu Ser Ile Thr Cys Thr Val Ser Gly Phe Ser Leu Thr Asn Tyr
   20 25 30
   Gly Val His Trp Val Arg Gln Ser Pro Gly Lys Gly Leu Glu Trp Leu
   35 40 45
   Gly Val Ile Trp Ser Gly Gly Asn Thr Asp Tyr Asn Thr Pro Phe Thr
   50 55 60
   Ser Arg Leu Ser Ile Asn Lys Asp Asn Ser Lys Ser Gln Val Phe Phe
   65 70 75 80
   Lys Met Asn Ser Leu Gln Ser Asn Asp Thr Ala Ile Tyr Tyr Cys Ala
   85 90 95
   Arg Ala Leu Thr Tyr Tyr Asp Tyr Glu Phe Ala Tyr Trp Gly Gln Gly
   100 105 110
   Thr Leu Val Thr Val Ser Ala Ala Ser Thr Lys Gly Pro Ser Val Phe
   115 120 125
   Pro Leu Ala Pro Ser Ser Lys Ser Thr Ser Gly Gly Thr Ala Ala Leu
   130 135 140
   Gly Cys Leu Val Lys Asp Tyr Phe Pro Glu Pro Val Thr Val Ser Trp
   145 150 155 160
   Asn Ser Gly Ala Leu Thr Ser Gly Val His Thr Phe Pro Ala Val Leu
   165 170 175
   Gln Ser Ser Gly Leu Tyr Ser Leu Ser Ser Val Val Thr Val Pro Ser
   180 185 190
   Ser Ser Leu Gly Thr Gln Thr Tyr Ile Cys Asn Val Asn His Lys Pro
   195 200 205
   Ser Asn Thr Lys Val Asp Lys Arg Val Glu Pro Lys Ser Pro Lys Ser
   210 215 220
   Cys Asp Lys Thr His Thr Cys Pro Pro Cys Pro Ala Pro Glu Leu Leu
   225 230 235 240
   Gly Gly Pro Ser Val Phe Leu Phe Pro Pro Lys Pro Lys Asp Thr Leu
   245 250 255
   Met Ile Ser Arg Thr Pro Glu Val Thr Cys Val Val Val Asp Val Ser
               260                 265                 270
   His Glu Asp Pro Glu Val Lys Phe Asn Trp Tyr Val Asp Gly Val Glu
           275                 280                 285
   Val His Asn Ala Lys Thr Lys Pro Arg Glu Glu Gln Tyr Asn Ser Thr
       290                 295                 300
   Tyr Arg Val Val Ser Val Leu Thr Val Leu His Gln Asp Trp Leu Asn
   305                 310                 315                 320
   Gly Lys Glu Tyr Lys Cys Lys Val Ser Asn Lys Ala Leu Pro Ala Pro
                   325                 330                 335
   Ile Glu Lys Thr Ile Ser Lys Ala Lys Gly Gln Pro Arg Glu Pro Gln
               340                 345                 350
   Val Tyr Thr Leu Pro Pro Ser Arg Asp Glu Leu Thr Lys Asn Gln Val
           355                 360                 365
   Ser Leu Thr Cys Leu Val Lys Gly Phe Tyr Pro Ser Asp Ile Ala Val
       370                 375                 380
   Glu Trp Glu Ser Asn Gly Gln Pro Glu Asn Asn Tyr Lys Thr Thr Pro
   385                 390                 395                 400
   Pro Val Leu Asp Ser Asp Gly Ser Phe Phe Leu Tyr Ser Lys Leu Thr
                   405                 410                 415
   Val Asp Lys Ser Arg Trp Gln Gln Gly Asn Val Phe Ser Cys Ser Val
               420                 425                 430
   Met His Glu Ala Leu His Asn His Tyr Thr Gln Lys Ser Leu Ser Leu
           435                 440                 445
   Ser Pro Gly Lys
       450
<210> 49
   <211> 213
   <212> PRT
   <213> Artificial Sequence
<220>
   <221> source
   <223> /note="Description of Artificial Sequence: Synthetic polypeptide"
<400> 49
   Asp Ile Leu Leu Thr Gln Ser Pro Val Ile Leu Ser Val Ser Pro Gly
   1               5                   10                  15
   Glu Arg Val Ser Phe Ser Cys Arg Ala Ser Gln Ser Ile Gly Thr Asn
               20                  25                  30
   Ile His Trp Tyr Gln Gln Arg Thr Asn Gly Ser Pro Arg Leu Leu Ile
           35                  40                  45
   Lys Tyr Ala Ser Glu Ser Ile Ser Gly Ile Pro Ser Arg Phe Ser Gly
       50                  55                  60
   Ser Gly Ser Gly Thr Asp Phe Thr Leu Ser Ile Asn Ser Val Glu Ser
   65                  70                  75                  80
   Glu Asp Ile Ala Asp Tyr Tyr Cys Gln Gln Asn Asn Asn Trp Pro Thr
                   85                  90                  95
   Thr Phe Gly Ala Gly Thr Lys Leu Glu Leu Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Ala
       210
<210> 50
   <211> 116
   <212> PRT
   <213> Artificial Sequence
<220>
   <221> source
   <223> /note="Description of Artificial Sequence: Synthetic polypeptide"
<400> 50
   Glu Val Gln Leu Gln Glu Ser Gly Pro Gly Leu Val Lys Pro Ser Gln
   1               5                   10                  15
   Thr Leu Ser Leu Thr Cys Thr Val Ser Gly Tyr Ser Ile Ser Ser Asp
               20                  25                  30
   Phe Ala Trp Asn Trp Ile Arg Gln Pro Pro Gly Lys Gly Leu Glu Trp
           35                  40                  45
   Met Gly Tyr Ile Ser Tyr Ser Gly Asn Thr Arg Tyr Gln Pro Ser Leu
       50                  55                  60
   Lys Ser Arg Ile Thr Ile Ser Arg Asp Thr Ser Lys Asn Gln Phe Phe
   65                  70                  75                  80
   Leu Lys Leu Asn Ser Val Thr Ala Ala Asp Thr Ala Thr Tyr Tyr Cys
                   85                  90                  95
   Val Thr Ala Gly Arg Gly Phe Pro Tyr Trp Gly Gln Gly Thr Leu Val
               100                 105                 110
   Thr Val Ser Ser
           115
<210> 51
   <211> 107
   <212> PRT
   <213> Artificial Sequence
<220>
   <221> source
   <223> /note="Description of Artificial Sequence: Synthetic polypeptide"
<400> 51
   Asp Ile Gln Met Thr Gln Ser Pro Ser Ser Met Ser Val Ser Val Gly
   1               5                   10                  15
   Asp Arg Val Thr Ile Thr Cys His Ser Ser Gln Asp Ile Asn Ser Asn
               20                  25                  30
   Leu Gly Trp Leu Gln Gln Lys Pro Gly Lys Ser Phe Lys Gly Leu Ile
           35                  40                  45
   Tyr His Gly Ser Asn Leu Asp His Gly Val Pro Ser Arg Phe Ser Gly
       50                  55                  60
   Ser Gly Ser Gly Thr Asp Tyr Thr Leu Thr Ile Ser Ser Leu Gln Pro
   65                  70                  75                  80
   Glu Asp Phe Ala Thr Tyr Tyr Cys Val Gln Tyr Asp Gln Phe Pro Trp
                   85                  90                  95
   Thr Phe Gly Gly Gly Thr Lys Leu Glu Ile Lys
               100                 105
<210> 52
   <211> 116
   <212> PRT
   <213> Artificial Sequence
<220>
   <221> source
   <223> /note="Description of Artificial Sequence: Synthetic polypeptide"
<400> 52
   Glu Val Gln Leu Gln Glu Ser Gly Pro Gly Leu Val Lys Pro Ser Gln
   1               5                   10                  15
   Thr Leu Ser Leu Thr Cys Thr Val Ser Gly Tyr Ser Ile Ser Arg Asp
               20                  25                  30
   Phe Ala Trp Asn Trp Ile Arg Gln Pro Pro Gly Lys Gly Leu Glu Trp
           35                  40                  45
   Met Gly Tyr Ile Ser Tyr Ser Gly Asn Thr Arg Tyr Gln Pro Ser Leu
       50                  55                  60
   Lys Ser Arg Ile Thr Ile Ser Arg Asp Thr Ser Lys Asn Gln Phe Phe
   65                  70                  75                  80
   Leu Lys Leu Asn Ser Val Thr Ala Ala Asp Thr Ala Thr Tyr Tyr Cys
                   85                  90                  95
   Val Thr Ala Ser Arg Gly Phe Pro Tyr Trp Gly Gln Gly Thr Leu Val
               100                 105                 110
   Thr Val Ser Ser
           115
<210> 53
   <211> 107
   <212> PRT
   <213> Artificial Sequence
<220>
   <221> source
   <223> /note="Description of Artificial Sequence: Synthetic polypeptide"
<400> 53
   Asp Ile Gln Met Thr Gln Ser Pro Ser Ser Met Ser Val Ser Val Gly
   1 5 10 15
   Asp Arg Val Thr Ile Thr Cys His Ser Ser Gln Asp Ile Asn Ser Asn
   20 25 30
   Ile Gly Trp Leu Gln Gln Lys Pro Gly Lys Ser Phe Lys Gly Leu Ile
   35 40 45
   Tyr His Gly Thr Asn Leu Asp Asp Gly Val Pro Ser Arg Phe Ser Gly
   50 55 60
   Ser Gly Ser Gly Thr Asp Tyr Thr Leu Thr Ile Ser Ser Leu Gln Pro
   65 70 75 80
   Glu Asp Phe Ala Thr Tyr Tyr Cys Val Gln Tyr Ala Gln Phe Pro Trp
   85 90 95
   Thr Phe Gly Gly Gly Thr Lys Leu Glu Ile Lys
   100 105
<210> 54
   <211> 116
   <212> PRT
   <213> Artificial Sequence
<220>
   <221> source
   <223> /note="Description of Artificial Sequence: Synthetic polypeptide"
<400> 54
   Glu Val Gln Leu Gln Glu Ser Gly Pro Gly Leu Val Lys Pro Ser Gln
   1 5 10 15
   Thr Leu Ser Leu Thr Cys Thr Val Ser Gly Tyr Ser Ile Gly Lys Asp
   20 25 30
   Phe Ala Trp Asn Trp Ile Arg Gln Pro Pro Gly Lys Gly Leu Glu Trp
   35 40 45
   Met Gly Tyr Ile Ser Tyr Asn Gly Asn Thr Arg Tyr Gln Pro Ser Leu
   50 55 60
   Lys Ser Arg Ile Thr Ile Ser Arg Asp Thr Ser Lys Asn Gln Phe Phe
   65 70 75 80
   Leu Lys Leu Asn Ser Val Thr Ala Ala Asp Thr Ala Thr Tyr Tyr Cys
   85 90 95
   Val Thr Ala Ser Arg Gly Leu Pro Tyr Trp Gly Gln Gly Thr Leu Val
   100 105 110
   Thr Val Ser Ser
   115
<210> 55
   <211> 107
   <212> PRT
   <213> Artificial Sequence
<220>
   <221> source
   <223> /note="Description of Artificial Sequence: Synthetic polypeptide"
<400> 55
   Asp Ile Gln Met Thr Gln Ser Pro Ser Ser Met Ser Val Ser Val Gly
   1 5 10 15
   Asp Arg Val Thr Ile Thr Cys His Ser Ser Gln Asp Ile Thr Tyr Asn
   20 25 30
   Ile Gly Trp Leu Gln Gln Lys Pro Gly Lys Ser Phe Lys Gly Leu Ile
   35 40 45
   Tyr His Gly Ala Asn Leu Asp Asp Gly Val Pro Ser Arg Phe Ser Gly
   50 55 60
   Ser Gly Ser Gly Thr Asp Tyr Thr Leu Thr Ile Ser Ser Leu Gln Pro
   65 70 75 80
   Glu Asp Phe Ala Thr Tyr Tyr Cys Val Gln Tyr Asp Glu Phe Pro Trp
   85 90 95
   Thr Phe Gly Gly Gly Thr Lys Leu Glu Ile Lys
   100 105
<210> 56
   <211> 116
   <212> PRT
   <213> Artificial Sequence
<220>
   <221> source
   <223> /note="Description of Artificial Sequence: Synthetic polypeptide"
<400> 56
   Glu Val Gln Leu Gln Glu Ser Gly Pro Gly Leu Val Lys Pro Ser Gln
   1               5                   10                  15
   Thr Leu Ser Leu Thr Cys Thr Val Ser Gly Tyr Ser Ile Gly Lys Asp
               20                  25                  30
   Phe Ala Trp Asn Trp Ile Arg Gln Pro Pro Gly Lys Gly Leu Glu Trp
           35                  40                  45
   Met Gly Tyr Ile Ser Tyr Ser Gly Asn Thr Arg Tyr Gln Pro Ser Leu
       50                  55                  60
   Lys Ser Arg Ile Thr Ile Ser Arg Asp Thr Ser Lys Asn Gln Phe Phe
   65                  70                  75                  80
   Leu Lys Leu Asn Ser Val Thr Ala Ala Asp Thr Ala Thr Tyr Tyr Cys
                   85                  90                  95
   Val Thr Ala Ser Arg Gly Leu Pro Tyr Trp Gly Gln Gly Thr Leu Val
               100                 105                 110
   Thr Val Ser Ser
           115
<210> 57
   <211> 107
   <212> PRT
   <213> Artificial Sequence
<220>
   <221> source
   <223> /note="Description of Artificial Sequence: Synthetic polypeptide"
<400> 57
   Asp Ile Gln Met Thr Gln Ser Pro Ser Ser Met Ser Val Ser Val Gly
   1               5                   10                  15
   Asp Arg Val Thr Ile Thr Cys His Ser Ser Gln Asp Ile Thr Tyr Asn
               20                  25                  30
   Ile Gly Trp Leu Gln Gln Lys Pro Gly Lys Ser Phe Lys Gly Leu Ile
           35                  40                  45
   Tyr His Gly Ala Asn Leu Asp Asp Gly Val Pro Ser Arg Phe Ser Gly
       50                  55                  60
   Ser Gly Ser Gly Thr Asp Tyr Thr Leu Thr Ile Ser Ser Leu Gln Pro
   65                  70                  75                  80
   Glu Asp Phe Ala Thr Tyr Tyr Cys Val Gln Tyr Asp Glu Phe Pro Trp
                   85                  90                  95
   Thr Phe Gly Gly Gly Thr Lys Leu Glu Ile Lys
               100                 105
<210> 58
   <211> 116
   <212> PRT
   <213> Artificial Sequence
<220>
   <221> source
   <223> /note="Description of Artificial Sequence: Synthetic polypeptide"
<400> 58
   Glu Val Gln Leu Gln Glu Ser Gly Pro Gly Leu Val Lys Pro Ser Gln
   1               5                   10                  15
   Thr Leu Ser Leu Thr Cys Thr Val Ser Gly Tyr Ser Ile Gly Lys Asp
               20                  25                  30
   Phe Ala Trp Asn Trp Ile Arg Gln Pro Pro Gly Lys Gly Leu Glu Trp
           35                  40                  45
   Met Gly Tyr Ile Ser Tyr Asn Gly Asn Thr Arg Tyr Gln Pro Ser Leu
       50                  55                  60
   Lys Ser Arg Ile Thr Ile Ser Arg Asp Thr Ser Lys Asn Gln Phe Phe
   65                  70                  75                  80
   Leu Lys Leu Asn Ser Val Thr Ala Ala Asp Thr Ala Thr Tyr Tyr Cys
                   85                  90                  95
   Val Thr Ala Ser Arg Gly Leu Pro Tyr Trp Gly Gln Gly Thr Leu Val
               100                 105                 110
   Thr Val Ser Ser
           115
<210> 59
   <211> 107
   <212> PRT
   <213> Artificial Sequence
<220>
   <221> source
   <223> /note="Description of Artificial Sequence: Synthetic polypeptide"
<400> 59
   Asp Ile Gln Met Thr Gln Ser Pro Ser Ser Met Ser Val Ser Val Gly
   1               5                   10                  15
   Asp Arg Val Thr Ile Thr Cys His Ser Ser Gln Asp Ile Thr Tyr Asn
   20 25 30
   Val Gly Trp Leu Gln Gln Lys Pro Gly Lys Ser Phe Lys Gly Leu Ile
   35 40 45
   Tyr His Gly Ser Asn Leu Asp His Gly Val Pro Ser Arg Phe Ser Gly
   50 55 60
   Ser Gly Ser Gly Thr Asp Tyr Thr Leu Thr Ile Ser Ser Leu Gln Pro
   65 70 75 80
   Glu Asp Phe Ala Thr Tyr Tyr Cys Val Gln Tyr Asp Asp Phe Pro Trp
   85 90 95
   Thr Phe Gly Gly Gly Thr Lys Leu Glu Ile Lys
   100 105
<210> 60
   <211> 116
   <212> PRT
   <213> Artificial Sequence
<220>
   <221> source
   <223> /note="Description of Artificial Sequence: Synthetic polypeptide"
<400> 60
   Glu Val Gln Leu Gln Glu Ser Gly Pro Gly Leu Val Lys Pro Ser Gln
   1 5 10 15
   Thr Leu Ser Leu Thr Cys Thr Val Ser Gly Tyr Ser Ile Gly Arg Asp
   20 25 30
   Phe Ala Trp Asn Trp Ile Arg Gln Pro Pro Gly Lys Gly Leu Glu Trp
   35 40 45
   Met Gly Tyr Ile Ser Tyr Ser Gly Asn Thr Arg Tyr Gln Pro Ser Leu
   50 55 60
   Lys Ser Arg Ile Thr Ile Ser Arg Asp Thr Ser Lys Asn Gln Phe Phe
   65 70 75 80
   Leu Lys Leu Asn Ser Val Thr Ala Ala Asp Thr Ala Thr Tyr Tyr Cys
   85 90 95
   Val Thr Ala Ser Arg Gly Phe Pro Tyr Trp Gly Gln Gly Thr Leu Val
   100 105 110
   Thr Val Ser Ser
   115
<210> 61
   <211> 107
   <212> PRT
   <213> Artificial Sequence
<220>
   <221> source
   <223> /note="Description of Artificial Sequence: Synthetic polypeptide"
<400> 61
   Asp Ile Gln Met Thr Gln Ser Pro Ser Ser Met Ser Val Ser Val Gly
   1 5 10 15
   Asp Arg Val Thr Ile Thr Cys His Ser Ser Gln Asp Ile Thr Tyr Asn
   20 25 30
   Val Gly Trp Leu Gln Gln Lys Pro Gly Lys Ser Phe Lys Gly Leu Ile
   35 40 45
   Tyr His Gly Ser Asn Leu Asp His Gly Val Pro Ser Arg Phe Ser Gly
   50 55 60
   Ser Gly Ser Gly Thr Asp Tyr Thr Leu Thr Ile Ser Ser Leu Gln Pro
   65 70 75 80
   Glu Asp Phe Ala Thr Tyr Tyr Cys Val Gln Tyr Asp Asp Phe Pro Trp
   85 90 95
   Thr Phe Gly Gly Gly Thr Lys Leu Glu Ile Lys
   100 105
<210> 62
   <211> 116
   <212> PRT
   <213> Artificial Sequence
<220>
   <221> source
   <223> /note="Description of Artificial Sequence: Synthetic polypeptide"
<400> 62
   Glu Val Gln Leu Gln Glu Ser Gly Pro Gly Leu Val Lys Pro Ser Gln
   1 5 10 15
   Thr Leu Ser Leu Thr Cys Thr Val Ser Gly Tyr Ser Ile Gly Lys Asp
   20 25 30
   Phe Ala Trp Asn Trp Ile Arg Gln Pro Pro Gly Lys Gly Leu Glu Trp
   35 40 45
   Met Gly Tyr Ile Ser Tyr Asn Gly Asn Thr Arg Tyr Gln Pro Ser Leu
   50 55 60
   Lys Ser Arg Ile Thr Ile Ser Arg Asp Thr Ser Lys Asn Gln Phe Phe
   65 70 75 80
   Leu Lys Leu Asn Ser Val Thr Ala Ala Asp Thr Ala Thr Tyr Tyr Cys
   85 90 95
   Val Thr Ala Ser Arg Gly Phe Pro Tyr Trp Gly Gln Gly Thr Leu Val
   100 105 110
   Thr Val Ser Ser
   115
<210> 63
   <211> 107
   <212> PRT
   <213> Artificial Sequence
<220>
   <221> source
   <223> /note="Description of Artificial Sequence: Synthetic polypeptide"
<400> 63
   Asp Ile Gln Met Thr Gln Ser Pro Ser Ser Met Ser Val Ser Val Gly
   1 5 10 15
   Asp Arg Val Thr Ile Thr Cys His Ser Ser Gln Asp Ile Thr Tyr Asn
   20 25 30
   Val Gly Trp Leu Gln Gln Lys Pro Gly Lys Ser Phe Lys Gly Leu Ile
   35 40 45
   Tyr His Gly Ser Asn Leu Asp His Gly Val Pro Ser Arg Phe Ser Gly
   50 55 60
   Ser Gly Ser Gly Thr Asp Tyr Thr Leu Thr Ile Ser Ser Leu Gln Pro
   65 70 75 80
   Glu Asp Phe Ala Thr Tyr Tyr Cys Val Gln Tyr Asp Asp Phe Pro Trp
   85 90 95
   Thr Phe Gly Gly Gly Thr Lys Leu Glu Ile Lys
   100 105
<210> 64
   <211> 116
   <212> PRT
   <213> Artificial Sequence
<220>
   <221> source
   <223> /note="Description of Artificial Sequence: Synthetic polypeptide"
<400> 64
   Glu Val Gln Leu Gln Glu Ser Gly Pro Gly Leu Val Lys Pro Ser Gln
   1               5                   10                  15
   Thr Leu Ser Leu Thr Cys Thr Val Ser Gly Tyr Ser Ile Ser Asn Asp
               20                  25                  30
   Phe Ala Trp Asn Trp Ile Arg Gln Pro Pro Gly Lys Gly Leu Glu Trp
           35                  40                  45
   Met Gly Tyr Ile Ser Tyr Lys Gly Asn Thr Arg Tyr Gln Pro Ser Leu
       50                  55                  60
   Lys Ser Arg Ile Thr Ile Ser Arg Asp Thr Ser Lys Asn Gln Phe Phe
   65                  70                  75                  80
   Leu Lys Leu Asn Ser Val Thr Ala Ala Asp Thr Ala Thr Tyr Tyr Cys
                   85                  90                  95
   Val Thr Ala Ser Arg Gly Phe Pro Trp Trp Gly Gln Gly Thr Leu Val
               100                 105                 110
   Thr Val Ser Ser
           115
<210> 65
   <211> 107
   <212> PRT
   <213> Artificial Sequence
<220>
   <221> source
   <223> /note="Description of Artificial Sequence: Synthetic polypeptide"
<400> 65
   Asp Ile Gln Met Thr Gln Ser Pro Ser Ser Met Ser Val Ser Val Gly
   1               5                   10                  15
   Asp Arg Val Thr Ile Thr Cys His Ser Ser Gln Asp Ile Asn Ser Asn
               20                  25                  30
   Ile Gly Trp Leu Gln Gln Lys Pro Gly Lys Ser Phe Lys Gly Leu Ile
           35                  40                  45
   Tyr His Gly Thr Asn Leu Asp Asp Gly Val Pro Ser Arg Phe Ser Gly
       50                  55                  60
   Ser Gly Ser Gly Thr Asp Tyr Thr Leu Thr Ile Ser Ser Leu Gln Pro
   65                  70                  75                  80
   Glu Asp Phe Ala Thr Tyr Tyr Cys Val Gln Tyr Ala Gln Phe Pro Trp
                   85                  90                  95
   Thr Phe Gly Gly Gly Thr Lys Leu Glu Ile Lys
               100                 105
<210> 66
   <211> 116
   <212> PRT
   <213> Artificial Sequence
<220>
   <221> source
   <223> /note="Description of Artificial Sequence: Synthetic polypeptide"
<400> 66
   Glu Val Gln Leu Gln Glu Ser Gly Pro Gly Leu Val Lys Pro Ser Gln
   1               5                   10                  15
   Thr Leu Ser Leu Thr Cys Thr Val Ser Gly Tyr Ser Ile Ser Ser Asp
               20                  25                  30
   Phe Ala Trp Asn Trp Ile Arg Gln Pro Pro Gly Lys Gly Leu Glu Trp
           35                  40                  45
   Met Gly Tyr Ile Ser Tyr Ser Gly Asn Thr Arg Tyr Gln Pro Ser Leu
       50                  55                  60
   Lys Ser Arg Ile Thr Ile Ser Arg Asp Thr Ser Lys Asn Gln Phe Phe
   65                  70                  75                  80
   Leu Lys Leu Asn Ser Val Thr Ala Ala Asp Thr Ala Thr Tyr Tyr Cys
                   85                  90                  95
   Val Thr Ala Gly Arg Gly Phe Pro Tyr Trp Gly Gln Gly Thr Leu Val
               100                 105                 110
   Thr Val Ser Ser
           115
<210> 67
   <211> 107
   <212> PRT
   <213> Artificial Sequence
<220>
   <221> source
   <223> /note="Description of Artificial Sequence: Synthetic polypeptide"
<400> 67
   Asp Ile Gln Met Thr Gln Ser Pro Ser Ser Met Ser Val Ser Val Gly
   1               5                   10                  15
   Asp Arg Val Thr Ile Thr Cys His Ser Ser Gln Asp Ile Asn Ser Asn
               20                  25                  30
   Ile Gly Trp Leu Gln Gln Lys Pro Gly Lys Ser Phe Lys Gly Leu Ile
           35                  40                  45
   Tyr His Gly Thr Asn Leu Asp Asp Gly Val Pro Ser Arg Phe Ser Gly
       50                  55                  60
   Ser Gly Ser Gly Thr Asp Tyr Thr Leu Thr Ile Ser Ser Leu Gln Pro
   65                  70                  75                  80
   Glu Asp Phe Ala Thr Tyr Tyr Cys Val Gln Tyr Glu Gln Phe Pro Trp
                   85                  90                  95
   Thr Phe Gly Gly Gly Thr Lys Leu Glu Ile Lys
               100                 105
<210> 68
   <211> 116
   <212> PRT
   <213> Artificial Sequence
<220>
   <221> source
   <223> /note="Description of Artificial Sequence: Synthetic polypeptide"
<400> 68
   Glu Val Gln Leu Gln Glu Ser Gly Pro Gly Leu Val Lys Pro Ser Gln
   1               5                   10                  15
   Thr Leu Ser Leu Thr Cys Thr Val Ser Gly Tyr Ser Ile Ser Ser Asp
               20                  25                  30
   Phe Ala Trp Asn Trp Ile Arg Gln Pro Pro Gly Lys Gly Leu Glu Trp
           35                  40                  45
   Met Gly Tyr Ile Ser Tyr Ser Gly Asn Thr Arg Tyr Gln Pro Ser Leu
       50                  55                  60
   Lys Ser Arg Ile Thr Ile Ser Arg Asp Thr Ser Lys Asn Gln Phe Phe
   65 70 75 80
   Leu Lys Leu Asn Ser Val Thr Ala Ala Asp Thr Ala Thr Tyr Tyr Cys
   85 90 95
   Val Thr Ala Gly Arg Gly Phe Pro Tyr Trp Gly Gln Gly Thr Leu Val
   100 105 110
   Thr Val Ser Ser
   115
<210> 69
   <211> 107
   <212> PRT
   <213> Artificial Sequence
<220>
   <221> source
   <223> /note="Description of Artificial Sequence: Synthetic polypeptide"
<400> 69
   Asp Ile Gln Met Thr Gln Ser Pro Ser Ser Met Ser Val Ser Val Gly
   1 5 10 15
   Asp Arg Val Thr Ile Thr Cys His Ser Ser Gln Asp Ile Asn Ser Asn
   20 25 30
   Leu Gly Trp Leu Gln Gln Lys Pro Gly Lys Ser Phe Lys Gly Leu Ile
   35 40 45
   Tyr His Gly Ala Asn Leu His Asp Gly Val Pro Ser Arg Phe Ser Gly
   50 55 60
   Ser Gly Ser Gly Thr Asp Tyr Thr Leu Thr Ile Ser Ser Leu Gln Pro
   65 70 75 80
   Glu Asp Phe Ala Thr Tyr Tyr Cys Val Gln Tyr Ala Glu Phe Pro Trp
   85 90 95
   Thr Phe Gly Gly Gly Thr Lys Leu Glu Ile Lys
   100 105
<210> 70
   <211> 116
   <212> PRT
   <213> Artificial Sequence
<220>
   <221> source
   <223> /note="Description of Artificial Sequence: Synthetic polypeptide"
<400> 70
   Glu Val Gln Leu Gln Glu Ser Gly Pro Gly Leu Val Lys Pro Ser Gln
   1               5                   10                  15
   Thr Leu Ser Leu Thr Cys Thr Val Ser Gly Tyr Ser Ile Ser His Asp
               20                  25                  30
   Phe Ala Trp Asn Trp Ile Arg Gln Pro Pro Gly Lys Gly Leu Glu Trp
           35                  40                  45
   Met Gly Tyr Ile Ser Tyr Asn Gly Asn Thr Arg Tyr Gln Pro Ser Leu
       50                  55                  60
   Lys Ser Arg Ile Thr Ile Ser Arg Asp Thr Ser Lys Asn Gln Phe Phe
   65                  70                  75                  80
   Leu Lys Leu Asn Ser Val Thr Ala Ala Asp Thr Ala Thr Tyr Tyr Cys
                   85                  90                  95
   Val Thr Ala Ser Trp Gly Leu Pro Trp Trp Gly Gln Gly Thr Leu Val
               100                 105                 110
   Thr Val Ser Ser
           115
<210> 71
   <211> 107
   <212> PRT
   <213> Artificial Sequence
<220>
   <221> source
   <223> /note="Description of Artificial Sequence: Synthetic polypeptide"
<400> 71
   Asp Ile Gln Met Thr Gln Ser Pro Ser Ser Met Ser Val Ser Val Gly
   1               5                   10                  15
   Asp Arg Val Thr Ile Thr Cys His Ser Ser Gln Asp Ile Asn Met Asn
               20                  25                  30
   Val Gly Trp Leu Gln Gln Lys Pro Gly Lys Ser Phe Lys Gly Leu Ile
           35                  40                  45
   Tyr His Gly Ala Ile Leu Asp Asp Gly Val Pro Ser Arg Phe Ser Gly
       50                  55                  60
   Ser Gly Ser Gly Thr Asp Tyr Thr Leu Thr Ile Ser Ser Leu Gln Pro
   65                  70                  75                  80
   Glu Asp Phe Ala Thr Tyr Tyr Cys Val Gln Tyr Ala Glu Phe Pro Trp
                   85                  90                  95
   Thr Phe Gly Gly Gly Thr Lys Leu Glu Ile Lys
               100                 105
<210> 72
   <211> 116
   <212> PRT
   <213> Artificial Sequence
<220>
   <221> source
   <223> /note="Description of Artificial Sequence: Synthetic polypeptide"
<400> 72
   Glu Val Gln Leu Gln Glu Ser Gly Pro Gly Leu Val Lys Pro Ser Gln
   1               5                   10                  15
   Thr Leu Ser Leu Thr Cys Thr Val Ser Gly Tyr Ser Ile Ser Asn Asp
               20                  25                  30
   Phe Ala Trp Asn Trp Ile Arg Gln Leu Pro Gly Lys Gly Leu Glu Trp
           35                  40                  45
   Met Gly Tyr Ile Ser Tyr Lys Gly Asn Thr Arg Tyr Gln Pro Ser Leu
       50                  55                  60
   Lys Ser Arg Ile Thr Ile Ser Arg Asp Thr Ser Lys Asn Gln Phe Phe
   65                  70                  75                  80
   Leu Lys Leu Asn Ser Val Thr Ala Ala Asp Thr Ala Thr Tyr Tyr Cys
                   85                  90                  95
   Val Thr Ala Ser Arg Gly Leu Pro Tyr Trp Gly Gln Gly Thr Leu Val
               100                 105                 110
   Thr Val Ser Ser
           115
<210> 73
   <211> 107
   <212> PRT
   <213> Artificial Sequence
<220>
   <221> source
   <223> /note="Description of Artificial Sequence: Synthetic polypeptide"
<400> 73
   Asp Ile Gln Met Thr Gln Ser Pro Ser Ser Met Ser Val Ser Val Gly
   1               5                   10                  15
   Asp Arg Val Thr Ile Thr Cys His Ser Ser Gln Asp Ile Thr Tyr Asn
               20                  25                  30
   Ile Gly Trp Leu Gln Gln Lys Pro Gly Lys Ser Phe Lys Gly Leu Ile
           35                  40                  45
   Tyr His Gly Ala Asn Leu Asp Asp Gly Val Pro Ser Arg Phe Ser Gly
       50                  55                  60
   Ser Gly Ser Gly Thr Asp Tyr Thr Leu Thr Ile Ser Ser Leu Gln Pro
   65                  70                  75                  80
   Glu Asp Phe Ala Thr Tyr Tyr Cys Val Gln Tyr Asp Glu Phe Pro Trp
                   85                  90                  95
   Thr Phe Gly Gly Gly Thr Lys Leu Glu Ile Lys
               100                 105
<210> 74
   <211> 116
   <212> PRT
   <213> Artificial Sequence
<220>
   <221> source
   <223> /note="Description of Artificial Sequence: Synthetic polypeptide"
<400> 74
   Glu Val Gln Leu Gln Glu Ser Gly Pro Gly Leu Val Lys Pro Ser Gln
   1               5                   10                  15
   Thr Leu Ser Leu Thr Cys Thr Val Ser Gly Tyr Ser Ile Ser Arg Asp
               20                  25                  30
   Phe Ala Trp Asn Trp Ile Arg Gln Pro Pro Gly Lys Gly Leu Glu Trp
           35                  40                  45
   Met Gly Tyr Ile Ser Tyr Asn Gly Asn Thr Arg Tyr Gln Pro Ser Leu
       50                  55                  60
   Lys Ser Arg Ile Thr Ile Ser Arg Asp Thr Ser Lys Asn Gln Phe Phe
   65                  70                  75                  80
   Leu Lys Leu Asn Ser Val Thr Ala Ala Asp Thr Ala Thr Tyr Tyr Cys
   85 90 95
   Val Thr Ala Ser Arg Gly Phe Pro Trp Trp Gly Gln Gly Thr Leu Val
   100 105 110
   Thr Val Ser Ser
   115
<210> 75
   <211> 107
   <212> PRT
   <213> Artificial Sequence
<220>
   <221> source
   <223> /note="Description of Artificial Sequence: Synthetic polypeptide"
<400> 75
   Asp Ile Gln Met Thr Gln Ser Pro Ser Ser Met Ser Val Ser Val Gly
   1 5 10 15
   Asp Arg Val Thr Ile Thr Cys His Ser Ser Gln Asp Ile Thr Tyr Asn
   20 25 30
   Val Gly Trp Leu Gln Gln Lys Pro Gly Lys Ser Phe Lys Gly Leu Ile
   35 40 45
   Tyr His Gly Ser Asn Leu Asp His Gly Val Pro Ser Arg Phe Ser Gly
   50 55 60
   Ser Gly Ser Gly Thr Asp Tyr Thr Leu Thr Ile Ser Ser Leu Gln Pro
   65 70 75 80
   Glu Asp Phe Ala Thr Tyr Tyr Cys Val Gln Tyr Asp Asp Phe Pro Trp
   85 90 95
   Thr Phe Gly Gly Gly Thr Lys Leu Glu Ile Lys
   100 105
<210> 76
   <211> 116
   <212> PRT
   <213> Artificial Sequence
<220>
   <221> source
   <223> /note="Description of Artificial Sequence: Synthetic polypeptide"
<400> 76
   Glu Val Gln Leu Gln Glu Ser Gly Pro Gly Leu Val Lys Pro Ser Gln
   1 5 10 15
   Thr Leu Ser Leu Thr Cys Thr Val Ser Gly Tyr Ser Ile Gly Arg Asp
   20 25 30
   Phe Ala Trp Asn Trp Ile Arg Gln Pro Pro Gly Lys Gly Leu Glu Trp
   35 40 45
   Met Gly Tyr Ile Ser Tyr Asn Gly Asn Thr Arg Tyr Gln Pro Ser Leu
   50 55 60
   Lys Ser Arg Ile Thr Ile Ser Arg Asp Thr Ser Lys Asn Gln Phe Phe
   65 70 75 80
   Leu Lys Leu Asn Ser Val Thr Ala Ala Asp Thr Ala Thr Tyr Tyr Cys
   85 90 95
   Val Thr Ala Ser Arg Gly Phe Pro Tyr Trp Gly Gln Gly Thr Leu Val
   100 105 110
   Thr Val Ser Ser
   115
<210> 77
   <211> 107
   <212> PRT
   <213> Artificial Sequence
<220>
   <221> source
   <223> /note="Description of Artificial Sequence: Synthetic polypeptide"
<400> 77
   Asp Ile Gln Met Thr Gln Ser Pro Ser Ser Met Ser Val Ser Val Gly
   1               5                   10                  15
   Asp Arg Val Thr Ile Thr Cys His Ser Ser Gln Asp Ile Thr Tyr Asn
               20                  25                  30
   Val Gly Trp Leu Gln Gln Lys Pro Gly Lys Ser Phe Lys Gly Leu Ile
           35                  40                  45
   Tyr His Gly Ser Asn Leu Asp His Gly Val Pro Ser Arg Phe Ser Gly
       50                  55                  60
   Ser Gly Ser Gly Thr Asp Tyr Thr Leu Thr Ile Ser Ser Leu Gln Pro
   65                  70                  75                  80
   Glu Asp Phe Ala Thr Tyr Tyr Cys Val Gln Tyr Asp Asp Phe Pro Trp
                   85                  90                  95
   Thr Phe Gly Gly Gly Thr Lys Leu Glu Ile Lys
               100                 105
<210> 78
   <211> 116
   <212> PRT
   <213> Artificial Sequence
<220>
   <221> source
   <223> /note="Description of Artificial Sequence: Synthetic polypeptide"
<400> 78
   Glu Val Gln Leu Gln Glu Ser Gly Pro Gly Leu Val Lys Pro Ser Gln
   1 5 10 15
   Thr Leu Ser Leu Thr Cys Thr Val Ser Gly Tyr Ser Ile Ser His Asp
   20 25 30
   Phe Ala Trp Asn Trp Ile Arg Gln Pro Pro Gly Lys Gly Leu Glu Trp
   35 40 45
   Met Gly Tyr Ile Ser Tyr Ser Gly Asn Thr Arg Tyr Gln Pro Ser Leu
   50 55 60
   Lys Ser Arg Ile Thr Ile Ser Arg Asp Thr Ser Lys Asn Gln Phe Phe
   65 70 75 80
   Leu Lys Leu Asn Ser Val Thr Ala Ala Asp Thr Ala Thr Tyr Tyr Cys
   85 90 95
   Val Thr Ala Ser Trp Gly Leu Pro Trp Trp Gly Gln Gly Thr Leu Val
   100 105 110
   Thr Val Ser Ser
   115
<210> 79
   <211> 107
   <212> PRT
   <213> Artificial Sequence
<220>
   <221> source
   <223> /note="Description of Artificial Sequence: Synthetic polypeptide"
<400> 79
   Asp Ile Gln Met Thr Gln Ser Pro Ser Ser Met Ser Val Ser Val Gly
   1 5 10 15
   Asp Arg Val Thr Ile Thr Cys His Ser Ser Gln Asp Ile Asn Met Asn
   20 25 30
   Val Gly Trp Leu Gln Gln Lys Pro Gly Lys Ser Phe Lys Gly Leu Ile
   35 40 45
   Tyr His Gly Ala Ile Leu Asp Asp Gly Val Pro Ser Arg Phe Ser Gly
   50 55 60
   Ser Gly Ser Gly Thr Asp Tyr Thr Leu Thr Ile Ser Ser Leu Gln Pro
   65 70 75 80
   Glu Asp Phe Ala Thr Tyr Tyr Cys Val Gln Tyr Ala Glu Phe Pro Trp
   85 90 95
   Thr Phe Gly Gly Gly Thr Lys Leu Glu Ile Lys
   100 105
<210> 80
   <211> 11
   <212> PRT
   <213> Artificial Sequence
<220>
   <221> source
   <223> /note="Description of Artificial Sequence: Synthetic peptide"
<400> 80
   Gly Tyr Ser Ile Gly Lys Asp Phe Ala Trp Asn
   1 5 10
<210> 81
   <211> 11
   <212> PRT
   <213> Artificial Sequence
<220>
   <221> source
   <223> /note="Description of Artificial Sequence: Synthetic peptide"
<400> 81
   Gly Tyr Ser Ile Ser His Asp Phe Ala Trp Asn
   1 5 10
<210> 82
   <211> 11
   <212> PRT
   <213> Artificial Sequence
<220>
   <221> source
   <223> /note="Description of Artificial Sequence: Synthetic peptide"
<400> 82
   His Ser Ser Gln Asp Ile Asn Ser Asn Leu Gly
   1 5 10
<210> 83
   <211> 7
   <212> PRT
   <213> Artificial Sequence
<220>
   <221> source
   <223> /note="Description of Artificial Sequence: Synthetic peptide"
<400> 83
   His Gly Ala Asn Leu His Asp
   1 5
<210> 84
   <211> 9
   <212> PRT
   <213> Artificial Sequence
<220>
   <221> source
   <223> /note="Description of Artificial Sequence: Synthetic peptide"
<400> 84
   Val Gln Tyr Glu Gln Phe Pro Trp Thr
   1 5
<210> 85
   <211> 9
   <212> PRT
   <213> Artificial Sequence
<220>
   <221> source
   <223> /note="Description of Artificial Sequence: Synthetic peptide"
<400> 85
   Val Gln Tyr Asp Gln Phe Pro Trp Thr
   1 5
<210> 86
   <211> 1338
   <212> DNA
   <213> Artificial Sequence
<220>
   <221> source
   <223> /note="Description of Artificial Sequence: Synthetic polynucleotide"
<400> 86
   gaggtgcaac tccaagagag cgggcccggc ctcgtgaagc cctctcagac tctgtccctg 60
   acttgcactg tgagcgggta ttccatcagc agagacttcg catggaactg gatccgccag 120
   cctcccggta agggactgga gtggatgggg tacatcagct acaacggtaa tacacgctat 180
   cagccctccc tgaagtctcg cattaccatt agtcgcgata cctccaagaa ccagttcttt 240
   ctgaaactca acagcgtgac agccgctgac accgccacct actactgcgt gaccgccagc 300
   agggggttcc cttactgggg ccagggcact ctggtcaccg tttcttctgc gtcgaccaag 360
   ggcccatcgg tcttccccct ggcaccctcc tccaagagca cctctggggg cacagcggcc 420
   ctgggctgcc tggtcaagga ctacttcccc gaaccggtga cggtgtcgtg gaactcaggc 480
   gccctgacca gcggcgtgca caccttcccg gctgtcctac agtcctcagg actctactcc 540
   ctcagcagcg tggtgaccgt gccctccagc agcttgggca cccagaccta catctgcaac 600
   gtgaatcaca agcccagcaa caccaaggtg gacaagaaag ttgagcccaa atcttgtgac 660
   aaaactcaca catgcccacc gtgcccagca cctgaactcc tggggggacc gtcagtcttc 720
   ctcttccccc caaaacccaa ggacaccctc atgatctccc ggacccctga ggtcacatgc 780
   gtggtggtgg acgtgagcca cgaagaccct gaggtcaagt tcaactggta cgtggacggc 840
   gtggaggtgc ataatgccaa gacaaagccg cgggaggagc agtacaacag cacgtaccgt 900
   gtggtcagcg tcctcaccgt cctgcaccag gactggctga atggcaagga gtacaagtgc 960
   aaggtctcca acaaagccct cccagccccc atcgagaaaa ccatctccaa agccaaaggg 1020
   cagccccgag aaccacaggt gtacaccctg cccccatccc gcgaggagat gaccaagaac 1080
   caggtcagcc tgacctgcct ggtcaaaggc ttctatccca gcgacatcgc cgtggagtgg 1140
   gagagcaatg ggcagccgga gaacaactac aagaccacgc ctcccgtgct ggactccgac 1200
   ggctccttct tcctctacag caagctcacc gtggacaaga gcaggtggca gcaggggaac 1260
   gtcttctcat gctccgtgat gcatgaggct ctgcacaacc actacacgca gaagagcctc 1320
   tccctgtctc cgggtaaa 1338
<210> 87
   <211> 642
   <212> DNA
   <213> Artificial Sequence
<220>
   <221> source
   <223> /note="Description of Artificial Sequence: Synthetic polynucleotide"
<400> 87
   gacatccaga tgacccagtc cccctccagt atgtctgtgt ctgtgggcga ccgtgtgacc      60
   attacctgcc actcctccca ggacatcaat agcaatatcg gttggttgca acagaagcca     120
   ggcaagtcct tcaaagggct gatttaccat ggtaccaacc tggacgacgg ggttcctagt     180
   cgtttcagcg gctccgggtc cggaaccgat tacactctga ccatcagcag tttgcagcct     240
   gaggactttg ctacctatta ttgtgtgcag tacgctcagt tcccatggac tttcggcggg     300
   ggcaccaaac tggagatcaa acgtacggtg gctgcaccat ctgtcttcat cttcccgcca     360
   tctgatgagc agttgaaatc tggaactgcc tctgttgtgt gcctgctgaa taacttctat     420
   cccagagagg ccaaagtaca gtggaaggtg gataacgccc tccaatcggg taactcccag     480
   gagagtgtca cagagcagga cagcaaggac agcacctaca gcctcagcag caccctgacg     540
   ctgagcaaag cagactacga gaaacacaaa gtctacgcct gcgaagtcac ccatcagggc     600
   ctgagctcgc ccgtcacaaa gagcttcaac aggggagagt gt                        642
<210> 88
   <211> 19
   <212> PRT
   <213> Artificial Sequence
<220>
   <221> source
   <223> /note="Description of Artificial Sequence: Synthetic peptide"
<400> 88
   Met Glu Phe Gly Leu Ser Trp Leu Phe Leu Val Ala Ile Leu Lys Gly
   1               5                   10                  15
   Val Gln Cys
<210> 89
   <211> 20
   <212> PRT
   <213> Artificial Sequence
<220>
   <221> source
   <223> /note="Description of Artificial Sequence: Synthetic peptide"
<400> 89
   Met Arg Val Pro Ala Gln Leu Leu Gly Leu Leu Leu Leu Trp Phe Pro
   1               5                   10                  15
   Gly Ser Arg Cys
               20
<210> 90
   <211> 446
   <212> PRT
   <213> Artificial Sequence
<220>
   <221> source
   <223> /note="Description of Artificial Sequence: Synthetic polypeptide"
<400> 90
   Glu Val Gln Leu Gln Glu Ser Gly Pro Gly Leu Val Lys Pro Ser Gln
   1               5                   10                  15
   Thr Leu Ser Leu Thr Cys Thr Val Ser Gly Tyr Ser Ile Ser Asn Asp
               20                  25                  30
   Phe Ala Trp Asn Trp Ile Arg Gln Pro Pro Gly Lys Gly Leu Glu Trp
           35                  40                  45
   Met Gly Tyr Ile Ser Tyr Lys Gly Asn Thr Arg Tyr Gln Pro Ser Leu
       50                  55                  60
   Lys Ser Arg Ile Thr Ile Ser Arg Asp Thr Ser Lys Asn Gln Phe Phe
   65                  70                  75                  80
   Leu Lys Leu Asn Ser Val Thr Ala Ala Asp Thr Ala Thr Tyr Tyr Cys
                   85                  90                  95
   Val Thr Ala Ser Arg Gly Phe Pro Trp Trp Gly Gln Gly Thr Leu Val
               100                 105                 110
   Thr Val Ser Ser Ala Ser Thr Lys Gly Pro Ser Val Phe Pro Leu Ala
           115                 120                 125
   Pro Ser Ser Lys Ser Thr Ser Gly Gly Thr Ala Ala Leu Gly Cys Leu
       130                 135                 140
   Val Lys Asp Tyr Phe Pro Glu Pro Val Thr Val Ser Trp Asn Ser Gly
   145                 150                 155                 160
   Ala Leu Thr Ser Gly Val His Thr Phe Pro Ala Val Leu Gln Ser Ser
                   165                 170                 175
   Gly Leu Tyr Ser Leu Ser Ser Val Val Thr Val Pro Ser Ser Ser Leu
               180                 185                 190
   Gly Thr Gln Thr Tyr Ile Cys Asn Val Asn His Lys Pro Ser Asn Thr
           195                 200                 205
   Lys Val Asp Lys Lys Val Glu Pro Lys Ser Cys Asp Lys Thr His Thr
       210                 215                 220
   Cys Pro Pro Cys Pro Ala Pro Glu Leu Leu Gly Gly Pro Ser Val Phe
   225                 230                 235                 240
   Leu Phe Pro Pro Lys Pro Lys Asp Thr Leu Met Ile Ser Arg Thr Pro
   245 250 255
   Glu Val Thr Cys Val Val Val Asp Val Ser His Glu Asp Pro Glu Val
   260 265 270
   Lys Phe Asn Trp Tyr Val Asp Gly Val Glu Val His Asn Ala Lys Thr
   275 280 285
   Lys Pro Arg Glu Glu Gln Tyr Asn Ser Thr Tyr Arg Val Val Ser Val
   290 295 300
   Leu Thr Val Leu His Gln Asp Trp Leu Asn Gly Lys Glu Tyr Lys Cys
   305 310 315 320
   Lys Val Ser Asn Lys Ala Leu Pro Ala Pro Ile Glu Lys Thr Ile Ser
   325 330 335
   Lys Ala Lys Gly Gln Pro Arg Glu Pro Gln Val Tyr Thr Leu Pro Pro
   340 345 350
   Ser Arg Glu Glu Met Thr Lys Asn Gln Val Ser Leu Thr Cys Leu Val
   355 360 365
   Lys Gly Phe Tyr Pro Ser Asp Ile Ala Val Glu Trp Glu Ser Asn Gly
   370 375 380
   Gln Pro Glu Asn Asn Tyr Lys Thr Thr Pro Pro Val Leu Asp Ser Asp
   385 390 395 400
   Gly Ser Phe Phe Leu Tyr Ser Lys Leu Thr Val Asp Lys Ser Arg Trp
   405 410 415
   Gln Gln Gly Asn Val Phe Ser Cys Ser Val Met His Glu Ala Leu His
   420 425 430
   Asn His Tyr Thr Gln Lys Ser Leu Ser Leu Ser Pro Gly Lys
   435 440 445
<210> 91
   <211> 446
   <212> PRT
   <213> Artificial Sequence
<220>
   <221> source
   <223> /note="Description of Artificial Sequence: Synthetic polypeptide"
<400> 91
   Glu Val Gln Leu Gln Glu Ser Gly Pro Gly Leu Val Lys Pro Ser Gln
   1               5                   10                  15
   Thr Leu Ser Leu Thr Cys Thr Val Ser Gly Tyr Ser Ile Ser Asn Asp
               20                  25                  30
   Phe Ala Trp Asn Trp Ile Arg Gln Pro Pro Gly Lys Gly Leu Glu Trp
           35                  40                  45
   Met Gly Tyr Ile Ser Tyr Lys Gly Asn Thr Arg Tyr Gln Pro Ser Leu
       50                  55                  60
   Lys Ser Arg Ile Thr Ile Ser Arg Asp Thr Ser Lys Asn Gln Phe Phe
   65                  70                  75                  80
   Leu Lys Leu Asn Ser Val Thr Ala Ala Asp Thr Ala Thr Tyr Tyr Cys
                   85                  90                  95
   Val Thr Ala Ser Arg Gly Phe Pro Trp Trp Gly Gln Gly Thr Leu Val
               100                 105                 110
   Thr Val Ser Ser Ala Ser Thr Lys Gly Pro Ser Val Phe Pro Leu Ala
           115                 120                 125
   Pro Ser Ser Lys Ser Thr Ser Gly Gly Thr Ala Ala Leu Gly Cys Leu
       130                 135                 140
   Val Lys Asp Tyr Phe Pro Glu Pro Val Thr Val Ser Trp Asn Ser Gly
   145                 150                 155                 160
   Ala Leu Thr Ser Gly Val His Thr Phe Pro Ala Val Leu Gln Ser Ser
                   165                 170                 175
   Gly Leu Tyr Ser Leu Ser Ser Val Val Thr Val Pro Ser Ser Ser Leu
               180                 185                 190
   Gly Thr Gln Thr Tyr Ile Cys Asn Val Asn His Lys Pro Ser Asn Thr
           195                 200                 205
   Lys Val Asp Lys Lys Val Glu Pro Lys Ser Cys Asp Lys Thr His Thr
       210                 215                 220
   Cys Pro Pro Cys Pro Ala Pro Glu Ala Ala Gly Gly Pro Ser Val Phe
   225                 230                 235                 240
   Leu Phe Pro Pro Lys Pro Lys Asp Thr Leu Met Ile Ser Arg Thr Pro
   245 250 255
   Glu Val Thr Cys Val Val Val Asp Val Ser His Glu Asp Pro Glu Val
   260 265 270
   Lys Phe Asn Trp Tyr Val Asp Gly Val Glu Val His Asn Ala Lys Thr
   275 280 285
   Lys Pro Arg Glu Glu Gln Tyr Asn Ser Thr Tyr Arg Val Val Ser Val
   290 295 300
   Leu Thr Val Leu His Gln Asp Trp Leu Asn Gly Lys Glu Tyr Lys Cys
   305 310 315 320
   Lys Val Ser Asn Lys Ala Leu Pro Ala Pro Ile Glu Lys Thr Ile Ser
   325 330 335
   Lys Ala Lys Gly Gln Pro Arg Glu Pro Gln Val Tyr Thr Leu Pro Pro
   340 345 350
   Ser Arg Glu Glu Met Thr Lys Asn Gln Val Ser Leu Thr Cys Leu Val
   355 360 365
   Lys Gly Phe Tyr Pro Ser Asp Ile Ala Val Glu Trp Glu Ser Asn Gly
   370 375 380
   Gln Pro Glu Asn Asn Tyr Lys Thr Thr Pro Pro Val Leu Asp Ser Asp
   385 390 395 400
   Gly Ser Phe Phe Leu Tyr Ser Lys Leu Thr Val Asp Lys Ser Arg Trp
   405 410 415
   Gln Gln Gly Asn Val Phe Ser Cys Ser Val Met His Glu Ala Leu His
   420 425 430
   Asn His Tyr Thr Gln Lys Ser Leu Ser Leu Ser Pro Gly Lys
   435 440 445
<210> 92
   <211> 214
   <212> PRT
   <213> Artificial Sequence
<220>
   <221> source
   <223> /note="Description of Artificial Sequence: Synthetic polypeptide"
<400> 92
   Asp Ile Gln Met Thr Gln Ser Pro Ser Ser Met Ser Val Ser Val Gly
   1               5                   10                  15
   Asp Arg Val Thr Ile Thr Cys His Ser Ser Gln Asp Ile Asn Ser Asn
               20                  25                  30
   Ile Gly Trp Leu Gln Gln Lys Pro Gly Lys Ser Phe Lys Gly Leu Ile
           35                  40                  45
   Tyr His Gly Thr Asn Leu Asp Asp Gly Val Pro Ser Arg Phe Ser Gly
       50                  55                  60
   Ser Gly Ser Gly Thr Asp Tyr Thr Leu Thr Ile Ser Ser Leu Gln Pro
   65                  70                  75                  80
   Glu Asp Phe Ala Thr Tyr Tyr Cys Val Gln Tyr Ala Gln Phe Pro Trp
                   85                  90                  95
   Thr Phe Gly Gly Gly Thr Lys Leu Glu Ile Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys
       210
<210> 93
   <211> 446
   <212> PRT
   <213> Artificial Sequence
<220>
   <221> source
   <223> /note="Description of Artificial Sequence: Synthetic polypeptide"
<400> 93
   Glu Val Gln Leu Gln Glu Ser Gly Pro Gly Leu Val Lys Pro Ser Gln
   1               5                   10                  15
   Thr Leu Ser Leu Thr Cys Thr Val Ser Gly Tyr Ser Ile Ser Asn Asp
               20                  25                  30
   Phe Ala Trp Asn Trp Ile Arg Gln Leu Pro Gly Lys Gly Leu Glu Trp
           35                  40                  45
   Met Gly Tyr Ile Ser Tyr Lys Gly Asn Thr Arg Tyr Gln Pro Ser Leu
       50                  55                  60
   Lys Ser Arg Ile Thr Ile Ser Arg Asp Thr Ser Lys Asn Gln Phe Phe
   65                  70                  75                  80
   Leu Lys Leu Asn Ser Val Thr Ala Ala Asp Thr Ala Thr Tyr Tyr Cys
                   85                  90                  95
   Val Thr Ala Ser Arg Gly Leu Pro Tyr Trp Gly Gln Gly Thr Leu Val
               100                 105                 110
   Thr Val Ser Ser Ala Ser Thr Lys Gly Pro Ser Val Phe Pro Leu Ala
           115                 120                 125
   Pro Ser Ser Lys Ser Thr Ser Gly Gly Thr Ala Ala Leu Gly Cys Leu
       130                 135                 140
   Val Lys Asp Tyr Phe Pro Glu Pro Val Thr Val Ser Trp Asn Ser Gly
   145                 150                 155                 160
   Ala Leu Thr Ser Gly Val His Thr Phe Pro Ala Val Leu Gln Ser Ser
                   165                 170                 175
   Gly Leu Tyr Ser Leu Ser Ser Val Val Thr Val Pro Ser Ser Ser Leu
               180                 185                 190
   Gly Thr Gln Thr Tyr Ile Cys Asn Val Asn His Lys Pro Ser Asn Thr
           195                 200                 205
   Lys Val Asp Lys Lys Val Glu Pro Lys Ser Cys Asp Lys Thr His Thr
       210                 215                 220
   Cys Pro Pro Cys Pro Ala Pro Glu Leu Leu Gly Gly Pro Ser Val Phe
   225                 230                 235                 240
   Leu Phe Pro Pro Lys Pro Lys Asp Thr Leu Met Ile Ser Arg Thr Pro
   245 250 255
   Glu Val Thr Cys Val Val Val Asp Val Ser His Glu Asp Pro Glu Val
   260 265 270
   Lys Phe Asn Trp Tyr Val Asp Gly Val Glu Val His Asn Ala Lys Thr
   275 280 285
   Lys Pro Arg Glu Glu Gln Tyr Asn Ser Thr Tyr Arg Val Val Ser Val
   290 295 300
   Leu Thr Val Leu His Gln Asp Trp Leu Asn Gly Lys Glu Tyr Lys Cys
   305 310 315 320
   Lys Val Ser Asn Lys Ala Leu Pro Ala Pro Ile Glu Lys Thr Ile Ser
   325 330 335
   Lys Ala Lys Gly Gln Pro Arg Glu Pro Gln Val Tyr Thr Leu Pro Pro
   340 345 350
   Ser Arg Glu Glu Met Thr Lys Asn Gln Val Ser Leu Thr Cys Leu Val
   355 360 365
   Lys Gly Phe Tyr Pro Ser Asp Ile Ala Val Glu Trp Glu Ser Asn Gly
   370 375 380
   Gln Pro Glu Asn Asn Tyr Lys Thr Thr Pro Pro Val Leu Asp Ser Asp
   385 390 395 400
   Gly Ser Phe Phe Leu Tyr Ser Lys Leu Thr Val Asp Lys Ser Arg Trp
   405 410 415
   Gln Gln Gly Asn Val Phe Ser Cys Ser Val Met His Glu Ala Leu His
   420 425 430
   Asn His Tyr Thr Gln Lys Ser Leu Ser Leu Ser Pro Gly Lys
   435 440 445
<210> 94
   <211> 446
   <212> PRT
   <213> Artificial Sequence
<220>
   <221> source
   <223> /note="Description of Artificial Sequence: Synthetic polypeptide"
<400> 94
   Glu Val Gln Leu Gln Glu Ser Gly Pro Gly Leu Val Lys Pro Ser Gln
   1               5                   10                  15
   Thr Leu Ser Leu Thr Cys Thr Val Ser Gly Tyr Ser Ile Ser Asn Asp
               20                  25                  30
   Phe Ala Trp Asn Trp Ile Arg Gln Leu Pro Gly Lys Gly Leu Glu Trp
           35                  40                  45
   Met Gly Tyr Ile Ser Tyr Lys Gly Asn Thr Arg Tyr Gln Pro Ser Leu
       50                  55                  60
   Lys Ser Arg Ile Thr Ile Ser Arg Asp Thr Ser Lys Asn Gln Phe Phe
   65                  70                  75                  80
   Leu Lys Leu Asn Ser Val Thr Ala Ala Asp Thr Ala Thr Tyr Tyr Cys
                   85                  90                  95
   Val Thr Ala Ser Arg Gly Leu Pro Tyr Trp Gly Gln Gly Thr Leu Val
               100                 105                 110
   Thr Val Ser Ser Ala Ser Thr Lys Gly Pro Ser Val Phe Pro Leu Ala
           115                 120                 125
   Pro Ser Ser Lys Ser Thr Ser Gly Gly Thr Ala Ala Leu Gly Cys Leu
       130                 135                 140
   Val Lys Asp Tyr Phe Pro Glu Pro Val Thr Val Ser Trp Asn Ser Gly
   145                 150                 155                 160
   Ala Leu Thr Ser Gly Val His Thr Phe Pro Ala Val Leu Gln Ser Ser
                   165                 170                 175
   Gly Leu Tyr Ser Leu Ser Ser Val Val Thr Val Pro Ser Ser Ser Leu
               180                 185                 190
   Gly Thr Gln Thr Tyr Ile Cys Asn Val Asn His Lys Pro Ser Asn Thr
           195                 200                 205
   Lys Val Asp Lys Lys Val Glu Pro Lys Ser Cys Asp Lys Thr His Thr
       210                 215                 220
   Cys Pro Pro Cys Pro Ala Pro Glu Ala Ala Gly Gly Pro Ser Val Phe
   225                 230                 235                 240
   Leu Phe Pro Pro Lys Pro Lys Asp Thr Leu Met Ile Ser Arg Thr Pro
                   245                 250                 255
   Glu Val Thr Cys Val Val Val Asp Val Ser His Glu Asp Pro Glu Val
   260 265 270
   Lys Phe Asn Trp Tyr Val Asp Gly Val Glu Val His Asn Ala Lys Thr
   275 280 285
   Lys Pro Arg Glu Glu Gln Tyr Asn Ser Thr Tyr Arg Val Val Ser Val
   290 295 300
   Leu Thr Val Leu His Gln Asp Trp Leu Asn Gly Lys Glu Tyr Lys Cys
   305 310 315 320
   Lys Val Ser Asn Lys Ala Leu Pro Ala Pro Ile Glu Lys Thr Ile Ser
   325 330 335
   Lys Ala Lys Gly Gln Pro Arg Glu Pro Gln Val Tyr Thr Leu Pro Pro
   340 345 350
   Ser Arg Glu Glu Met Thr Lys Asn Gln Val Ser Leu Thr Cys Leu Val
   355 360 365
   Lys Gly Phe Tyr Pro Ser Asp Ile Ala Val Glu Trp Glu Ser Asn Gly
   370 375 380
   Gln Pro Glu Asn Asn Tyr Lys Thr Thr Pro Pro Val Leu Asp Ser Asp
   385 390 395 400
   Gly Ser Phe Phe Leu Tyr Ser Lys Leu Thr Val Asp Lys Ser Arg Trp
   405 410 415
   Gln Gln Gly Asn Val Phe Ser Cys Ser Val Met His Glu Ala Leu His
   420 425 430
   Asn His Tyr Thr Gln Lys Ser Leu Ser Leu Ser Pro Gly Lys
   435 440 445
<210> 95
   <211> 214
   <212> PRT
   <213> Artificial Sequence
<220>
   <221> source
   <223> /note="Description of Artificial Sequence: Synthetic polypeptide"
<400> 95
   Asp Ile Gln Met Thr Gln Ser Pro Ser Ser Met Ser Val Ser Val Gly
   1 5 10 15
   Asp Arg Val Thr Ile Thr Cys His Ser Ser Gln Asp Ile Thr Tyr Asn
               20                  25                  30
   Ile Gly Trp Leu Gln Gln Lys Pro Gly Lys Ser Phe Lys Gly Leu Ile
           35                  40                  45
   Tyr His Gly Ala Asn Leu Asp Asp Gly Val Pro Ser Arg Phe Ser Gly
       50                  55                  60
   Ser Gly Ser Gly Thr Asp Tyr Thr Leu Thr Ile Ser Ser Leu Gln Pro
   65                  70                  75                  80
   Glu Asp Phe Ala Thr Tyr Tyr Cys Val Gln Tyr Asp Glu Phe Pro Trp
                   85                  90                  95
   Thr Phe Gly Gly Gly Thr Lys Leu Glu Ile Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys
       210
<210> 96
   <211> 446
   <212> PRT
   <213> Artificial Sequence
<220>
   <221> source
   <223> /note="Description of Artificial Sequence: Synthetic polypeptide"
<400> 96
   Glu Val Gln Leu Gln Glu Ser Gly Pro Gly Leu Val Lys Pro Ser Gln
   1               5                   10                  15
   Thr Leu Ser Leu Thr Cys Thr Val Ser Gly Tyr Ser Ile Ser Arg Asp
               20                  25                  30
   Phe Ala Trp Asn Trp Ile Arg Gln Pro Pro Gly Lys Gly Leu Glu Trp
           35                  40                  45
   Met Gly Tyr Ile Ser Tyr Asn Gly Asn Thr Arg Tyr Gln Pro Ser Leu
       50                  55                  60
   Lys Ser Arg Ile Thr Ile Ser Arg Asp Thr Ser Lys Asn Gln Phe Phe
   65                  70                  75                  80
   Leu Lys Leu Asn Ser Val Thr Ala Ala Asp Thr Ala Thr Tyr Tyr Cys
                   85                  90                  95
   Val Thr Ala Ser Arg Gly Phe Pro Trp Trp Gly Gln Gly Thr Leu Val
               100                 105                 110
   Thr Val Ser Ser Ala Ser Thr Lys Gly Pro Ser Val Phe Pro Leu Ala
           115                 120                 125
   Pro Ser Ser Lys Ser Thr Ser Gly Gly Thr Ala Ala Leu Gly Cys Leu
       130                 135                 140
   Val Lys Asp Tyr Phe Pro Glu Pro Val Thr Val Ser Trp Asn Ser Gly
   145                 150                 155                 160
   Ala Leu Thr Ser Gly Val His Thr Phe Pro Ala Val Leu Gln Ser Ser
                   165                 170                 175
   Gly Leu Tyr Ser Leu Ser Ser Val Val Thr Val Pro Ser Ser Ser Leu
               180                 185                 190
   Gly Thr Gln Thr Tyr Ile Cys Asn Val Asn His Lys Pro Ser Asn Thr
           195                 200                 205
   Lys Val Asp Lys Lys Val Glu Pro Lys Ser Cys Asp Lys Thr His Thr
       210                 215                 220
   Cys Pro Pro Cys Pro Ala Pro Glu Leu Leu Gly Gly Pro Ser Val Phe
   225                 230                 235                 240
   Leu Phe Pro Pro Lys Pro Lys Asp Thr Leu Met Ile Ser Arg Thr Pro
                   245                 250                 255
   Glu Val Thr Cys Val Val Val Asp Val Ser His Glu Asp Pro Glu Val
               260                 265                 270
   Lys Phe Asn Trp Tyr Val Asp Gly Val Glu Val His Asn Ala Lys Thr
           275                 280                 285
   Lys Pro Arg Glu Glu Gln Tyr Asn Ser Thr Tyr Arg Val Val Ser Val
       290                 295                 300
   Leu Thr Val Leu His Gln Asp Trp Leu Asn Gly Lys Glu Tyr Lys Cys
   305                 310                 315                 320
   Lys Val Ser Asn Lys Ala Leu Pro Ala Pro Ile Glu Lys Thr Ile Ser
                   325                 330                 335
   Lys Ala Lys Gly Gln Pro Arg Glu Pro Gln Val Tyr Thr Leu Pro Pro
               340                 345                 350
   Ser Arg Glu Glu Met Thr Lys Asn Gln Val Ser Leu Thr Cys Leu Val
           355                 360                 365
   Lys Gly Phe Tyr Pro Ser Asp Ile Ala Val Glu Trp Glu Ser Asn Gly
       370                 375                 380
   Gln Pro Glu Asn Asn Tyr Lys Thr Thr Pro Pro Val Leu Asp Ser Asp
   385                 390                 395                 400
   Gly Ser Phe Phe Leu Tyr Ser Lys Leu Thr Val Asp Lys Ser Arg Trp
                   405                 410                 415
   Gln Gln Gly Asn Val Phe Ser Cys Ser Val Met His Glu Ala Leu His
               420                 425                 430
   Asn His Tyr Thr Gln Lys Ser Leu Ser Leu Ser Pro Gly Lys
           435                 440                 445
<210> 97
   <211> 446
   <212> PRT
   <213> Artificial Sequence
<220>
   <221> source
   <223> /note="Description of Artificial Sequence: Synthetic polypeptide"
<400> 97
   Glu Val Gln Leu Gln Glu Ser Gly Pro Gly Leu Val Lys Pro Ser Gln
   1               5                   10                  15
   Thr Leu Ser Leu Thr Cys Thr Val Ser Gly Tyr Ser Ile Ser Arg Asp
               20                  25                  30
   Phe Ala Trp Asn Trp Ile Arg Gln Pro Pro Gly Lys Gly Leu Glu Trp
           35                  40                  45
   Met Gly Tyr Ile Ser Tyr Asn Gly Asn Thr Arg Tyr Gln Pro Ser Leu
       50                  55                  60
   Lys Ser Arg Ile Thr Ile Ser Arg Asp Thr Ser Lys Asn Gln Phe Phe
   65                  70                  75                  80
   Leu Lys Leu Asn Ser Val Thr Ala Ala Asp Thr Ala Thr Tyr Tyr Cys
                   85                  90                  95
   Val Thr Ala Ser Arg Gly Phe Pro Trp Trp Gly Gln Gly Thr Leu Val
               100                 105                 110
   Thr Val Ser Ser Ala Ser Thr Lys Gly Pro Ser Val Phe Pro Leu Ala
           115                 120                 125
   Pro Ser Ser Lys Ser Thr Ser Gly Gly Thr Ala Ala Leu Gly Cys Leu
       130                 135                 140
   Val Lys Asp Tyr Phe Pro Glu Pro Val Thr Val Ser Trp Asn Ser Gly
   145                 150                 155                 160
   Ala Leu Thr Ser Gly Val His Thr Phe Pro Ala Val Leu Gln Ser Ser
                   165                 170                 175
   Gly Leu Tyr Ser Leu Ser Ser Val Val Thr Val Pro Ser Ser Ser Leu
               180                 185                 190
   Gly Thr Gln Thr Tyr Ile Cys Asn Val Asn His Lys Pro Ser Asn Thr
           195                 200                 205
   Lys Val Asp Lys Lys Val Glu Pro Lys Ser Cys Asp Lys Thr His Thr
       210                 215                 220
   Cys Pro Pro Cys Pro Ala Pro Glu Ala Ala Gly Gly Pro Ser Val Phe
   225                 230                 235                 240
   Leu Phe Pro Pro Lys Pro Lys Asp Thr Leu Met Ile Ser Arg Thr Pro
                   245                 250                 255
   Glu Val Thr Cys Val Val Val Asp Val Ser His Glu Asp Pro Glu Val
   260 265 270
   Lys Phe Asn Trp Tyr Val Asp Gly Val Glu Val His Asn Ala Lys Thr
   275 280 285
   Lys Pro Arg Glu Glu Gln Tyr Asn Ser Thr Tyr Arg Val Val Ser Val
   290 295 300
   Leu Thr Val Leu His Gln Asp Trp Leu Asn Gly Lys Glu Tyr Lys Cys
   305 310 315 320
   Lys Val Ser Asn Lys Ala Leu Pro Ala Pro Ile Glu Lys Thr Ile Ser
   325 330 335
   Lys Ala Lys Gly Gln Pro Arg Glu Pro Gln Val Tyr Thr Leu Pro Pro
   340 345 350
   Ser Arg Glu Glu Met Thr Lys Asn Gln Val Ser Leu Thr Cys Leu Val
   355 360 365
   Lys Gly Phe Tyr Pro Ser Asp Ile Ala Val Glu Trp Glu Ser Asn Gly
   370 375 380
   Gln Pro Glu Asn Asn Tyr Lys Thr Thr Pro Pro Val Leu Asp Ser Asp
   385 390 395 400
   Gly Ser Phe Phe Leu Tyr Ser Lys Leu Thr Val Asp Lys Ser Arg Trp
   405 410 415
   Gln Gln Gly Asn Val Phe Ser Cys Ser Val Met His Glu Ala Leu His
   420 425 430
   Asn His Tyr Thr Gln Lys Ser Leu Ser Leu Ser Pro Gly Lys
   435 440 445
<210> 98
   <211> 214
   <212> PRT
   <213> Artificial Sequence
<220>
   <221> source
   <223> /note="Description of Artificial Sequence: Synthetic polypeptide"
<400> 98
   Asp Ile Gln Met Thr Gln Ser Pro Ser Ser Met Ser Val Ser Val Gly
   1 5 10 15
   Asp Arg Val Thr Ile Thr Cys His Ser Ser Gln Asp Ile Thr Tyr Asn
   20 25 30
   Val Gly Trp Leu Gln Gln Lys Pro Gly Lys Ser Phe Lys Gly Leu Ile
   35 40 45
   Tyr His Gly Ser Asn Leu Asp His Gly Val Pro Ser Arg Phe Ser Gly
   50 55 60
   Ser Gly Ser Gly Thr Asp Tyr Thr Leu Thr Ile Ser Ser Leu Gln Pro
   65 70 75 80
   Glu Asp Phe Ala Thr Tyr Tyr Cys Val Gln Tyr Asp Asp Phe Pro Trp
   85 90 95
   Thr Phe Gly Gly Gly Thr Lys Leu Glu Ile Lys Arg Thr Val Ala Ala
   100 105 110
   Pro Ser Val Phe Ile Phe Pro Pro Ser Asp Glu Gln Leu Lys Ser Gly
   115 120 125
   Thr Ala Ser Val Val Cys Leu Leu Asn Asn Phe Tyr Pro Arg Glu Ala
   130 135 140
   Lys Val Gln Trp Lys Val Asp Asn Ala Leu Gln Ser Gly Asn Ser Gln
   145 150 155 160
   Glu Ser Val Thr Glu Gln Asp Ser Lys Asp Ser Thr Tyr Ser Leu Ser
   165 170 175
   Ser Thr Leu Thr Leu Ser Lys Ala Asp Tyr Glu Lys His Lys Val Tyr
   180 185 190
   Ala Cys Glu Val Thr His Gln Gly Leu Ser Ser Pro Val Thr Lys Ser
   195 200 205
   Phe Asn Arg Gly Glu Cys
   210
<210> 99
   <211> 4
   <212> PRT
   <213> Artificial Sequence
<220>
   <221> source
   <223> /note="Description of Artificial Sequence: Synthetic peptide"
<400> 99
   Gly Phe Leu Gly
   1
<210> 100
   <211> 4
   <212> PRT
   <213> Artificial Sequence
<220>
   <221> source
   <223> /note="Description of Artificial Sequence: Synthetic peptide"
<400> 100
   Ala Leu Ala Leu
   1
<210> 101
   <211> 5
   <212> PRT
   <213> Artificial Sequence
<220>
   <221> source
   <223> /note="Description of Artificial Sequence: Synthetic peptide"
<400> 101
   Tyr Tyr Tyr Tyr Tyr
   1 5
<210> 102
   <211> 446
   <212> PRT
   <213> Artificial Sequence
<220>
   <221> source
   <223> /note="Description of Artificial Sequence: Synthetic polypeptide"
<400> 102
   Glu Val Gln Leu Gln Glu Ser Gly Pro Gly Leu Val Lys Pro Ser Gln
   1               5                   10                  15
   Thr Leu Ser Leu Thr Cys Thr Val Ser Gly Tyr Ser Ile Ser Arg Asp
               20                  25                  30
   Phe Ala Trp Asn Trp Ile Arg Gln Pro Pro Gly Lys Gly Leu Glu Trp
           35                  40                  45
   Met Gly Tyr Ile Ser Tyr Asn Gly Asn Thr Arg Tyr Gln Pro Ser Leu
       50                  55                  60
   Lys Ser Arg Ile Thr Ile Ser Arg Asp Thr Ser Lys Asn Gln Phe Phe
   65                  70                  75                  80
   Leu Lys Leu Asn Ser Val Thr Ala Ala Asp Thr Ala Thr Tyr Tyr Cys
   85 90 95
   Val Thr Ala Ser Arg Gly Phe Pro Tyr Trp Gly Gln Gly Thr Leu Val
   100 105 110
   Thr Val Ser Ser Ala Ser Thr Lys Gly Pro Ser Val Phe Pro Leu Ala
   115 120 125
   Pro Ser Ser Lys Ser Thr Ser Gly Gly Thr Ala Ala Leu Gly Cys Leu
   130 135 140
   Val Lys Asp Tyr Phe Pro Glu Pro Val Thr Val Ser Trp Asn Ser Gly
   145 150 155 160
   Ala Leu Thr Ser Gly Val His Thr Phe Pro Ala Val Leu Gln Ser Ser
   165 170 175
   Gly Leu Tyr Ser Leu Ser Ser Val Val Thr Val Pro Ser Ser Ser Leu
   180 185 190
   Gly Thr Gln Thr Tyr Ile Cys Asn Val Asn His Lys Pro Ser Asn Thr
   195 200 205
   Lys Val Asp Lys Lys Val Glu Pro Lys Ser Cys Asp Lys Thr His Thr
   210 215 220
   Cys Pro Pro Cys Pro Ala Pro Glu Leu Leu Gly Gly Pro Ser Val Phe
   225 230 235 240
   Leu Phe Pro Pro Lys Pro Lys Asp Thr Leu Met Ile Ser Arg Thr Pro
   245 250 255
   Glu Val Thr Cys Val Val Val Asp Val Ser His Glu Asp Pro Glu Val
   260 265 270
   Lys Phe Asn Trp Tyr Val Asp Gly Val Glu Val His Asn Ala Lys Thr
   275 280 285
   Lys Pro Arg Glu Glu Gln Tyr Asn Ser Thr Tyr Arg Val Val Ser Val
   290 295 300
   Leu Thr Val Leu His Gln Asp Trp Leu Asn Gly Lys Glu Tyr Lys Cys
   305 310 315 320
   Lys Val Ser Asn Lys Ala Leu Pro Ala Pro Ile Glu Lys Thr Ile Ser
   325 330 335
   Lys Ala Lys Gly Gln Pro Arg Glu Pro Gln Val Tyr Thr Leu Pro Pro
               340                 345                 350
   Ser Arg Glu Glu Met Thr Lys Asn Gln Val Ser Leu Thr Cys Leu Val
           355                 360                 365
   Lys Gly Phe Tyr Pro Ser Asp Ile Ala Val Glu Trp Glu Ser Asn Gly
       370                 375                 380
   Gln Pro Glu Asn Asn Tyr Lys Thr Thr Pro Pro Val Leu Asp Ser Asp
   385                 390                 395                 400
   Gly Ser Phe Phe Leu Tyr Ser Lys Leu Thr Val Asp Lys Ser Arg Trp
                   405                 410                 415
   Gln Gln Gly Asn Val Phe Ser Cys Ser Val Met His Glu Ala Leu His
               420                 425                 430
   Asn His Tyr Thr Gln Lys Ser Leu Ser Leu Ser Pro Gly Lys
           435                 440                 445